(19) Europäisches Patentamt
European Patent Office
Office européen des brevets

(11) EP 4 570 792 A1

(12) **EUROPEAN PATENT APPLICATION**
published in accordance with Art. 153(4) EPC

(43) Date of publication:
**18.06.2025 Bulletin 2025/25**

(21) Application number: **23851910.2**

(22) Date of filing: **10.08.2023**

(51) International Patent Classification (IPC):
***C07D 401/04*** (2006.01) ***C07D 401/14*** (2006.01)
***C07D 471/04*** (2006.01) ***C07D 487/04*** (2006.01)
***A61K 31/454*** (2006.01) ***A61K 31/4545*** (2006.01)
***A61P 35/00*** (2006.01)

(52) Cooperative Patent Classification (CPC):
**A61K 31/454; A61K 31/4545; A61P 35/00; C07D 401/04; C07D 401/14; C07D 471/04; C07D 487/04**

(86) International application number:
**PCT/CN2023/112130**

(87) International publication number:
**WO 2024/032689 (15.02.2024 Gazette 2024/07)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **10.08.2022 CN 202210956783**

(71) Applicant: **Gluetacs Therapeutics (Shanghai) Co., Ltd.**
**Shanghai 201306 (CN)**

(72) Inventors:
- **YANG, Xiaobao**
**Shanghai 201306 (CN)**
- **SUN, Renhong**
**Shanghai 201306 (CN)**
- **ZHOU, Yuedong**
**Shanghai 201306 (CN)**
- **ZHAO, Baoyin**
**Shanghai 201306 (CN)**

(74) Representative: **J A Kemp LLP**
**80 Turnmill Street**
**London EC1M 5QU (GB)**

# (54) COMPOUND BASED ON ISOINDOLINE-SUBSTITUTED GLUTARIMIDE BACKBONE AND USE THEREOF

(57) The present disclosure provides a compound of Formula (I) or salts, enantiomers, stereoisomers, solvates, or polymorphs thereof and uses thereof. The present disclosure also provides pharmaceutical compositions comprising, as an active ingredient, the compound of Formula (I) or salts, enantiomers, stereoisomers, solvates, or polymorphs thereof and uses thereof. The series of compounds designed and synthesized in the present disclosure can effectively prevent and/or treat diseases or disorders associated with the cereblon protein.

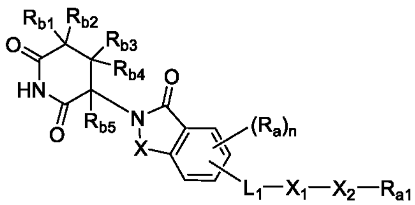

Formula (I)



Processed by Luminess, 75001 PARIS (FR)

## Description

## Technical Field

The present disclosure relates to a compound of Formula (I) or salts, enantiomers, diastereoisomer, isotopically enriched analogs, solvates, prodrugs, or polymorphs thereof and uses thereof, especially their use in the prevention and/or treatment of diseases or disorders associated with cereblon protein (CRBN).

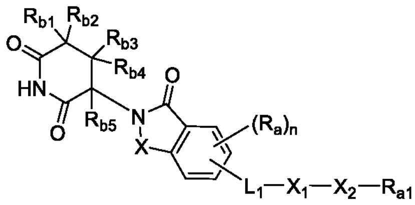

Formula (I)

## Background

Although Thalidomide, lenalidomide and other phthalimide immunomodulatory drugs (IMiDs) have shown significant efficacy in the treatment of multiple myeloma and autoimmune diseases, it was not until 2010 that the E3 ubiquitin ligase cereblon (CRBN) was identified as a direct target of IMiDs. Subsequent research confirmed that these drugs function as molecular glue, inducing the interaction between transcription factors IKZF1/3 and CRBN protein, ultimately leading to their ubiquitination and degradation. Compared with traditional small molecule inhibitors, molecular glue protein degraders have natural mechanism advantages: the former works by occupying the functional domain of the target protein for a long time to inhibit its function, while the protein degraders directly degrade and eliminate the entire target protein, often having a much greater efficacy than traditional small molecule inhibitors. The protein degraders can target "non-drugable" targets and have low requirements for binding force, catalytic capacity and low concentration, which can overcome the clinical drug resistance problem of traditional small molecules. Moreover, molecular glue degraders usually have small molecular weights and desirable druggability, so the research and development of such drugs have received great attention. Based on the CRBN E3 ubiquitin ligase and molecular glue degradation mechanism, a series of compounds have been developed, such as pomalidomide, which has been launched and CC-122 of Bristol-Myers Squibb (BMS), which is undergoing clinical trials, CC-220, CC-90009, CC-99282 and CC-92480, DKY709 from Novartis and CFT7455 from C4 Therapeutics. The degradation substrates of these molecular glues have also expanded from the initially discovered transcription factors IKZF1/3 to include casein kinase 1$\alpha$ (CK1$\alpha$), zinc finger protein 91 (ZFP91) and translation factor GSPT1. The degradation of these protein substrates will enable the molecular glues to exert immune regulatory, antiinflammatory and anti-tumor pharmaceutical activities. Currently, the number of identified molecular glue candidate compounds is quite limited. Moreover, there is a wide variety of pathogenic proteins that can theoretically serve as degradation substrates for the development of molecular glues. Therefore, it is necessary to design and develop more molecular glues through rationalization and diversification approaches to degrade and eliminate more pathogenic proteins, and to apply them to the treatment of diseases associated with these pathogenic proteins.

The present invention develops more novel, highly efficient, low-toxic, stable, and cost-effective molecular glue degraders based on the fused phenyl-glutarimide skeleton through rational design to meet the demands of clinical applications.

## Summary of Invention

In view of the above, the objectives of the present disclosure are to provide novel molecular glue protein degraders, their applications and usage methods.

To achieve the above objectives and other related goals, in one aspect, the present disclosure provides a compound of Formula (I):

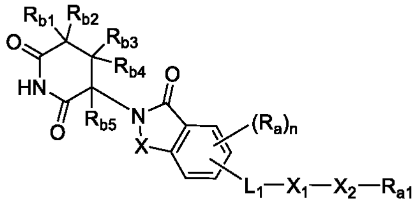

Formula (I)

or salts, stereoisomers (including enantiomers, diastereoisomers), isotopically enriched analogs, solvates, prodrugs, or polymorphs thereof,

$R_{b1}$, $R_{b2}$, $R_{b3}$, $R_{b4}$ and $R_{b5}$ each independently represent H, D, or $C_{1-3}$ alkyl;
X represents C(O) or $CH_2$;
$(R_a)_n$ indicates that benzene ring in Formula (I) is optionally substituted with n $R_a$, where $R_a$ represents deuterium, halogen, hydroxy, mercapto, nitro, amino, cyano, optionally deuterated $C_{1-6}$ alkyl, optionally deuterated $C_{1-6}$ alkoxy, or halogenated $C_{1-6}$ alkyl, and n represents an integer of 0, 1, 2, or 3; and
$L_1$ represents C(O), alkenylene, optionally substituted $C_{1-5}$ alkylene, -CH=, optionally substituted $C_{6-10}$ arylene-$C_{1-5}$ alkylene-*, or $N(R_{c1})$, where $R_{c1}$ represents H or $C_{1-3}$ alkyl, symbol * indicates the point of attachment to $X_1$; or $L_1$ represents $-C(R_{L1}R_{L2})-$, where $R_{L1}$, $R_{L2}$ together with the carbon atom to which they are attached form optionally substituted $C_{3-8}$ cycloalkylene; or $L_1$ represents a bond;
$X_1$ represents optionally substituted cycloalkylene, optionally substituted heterocyclylene or optionally substituted heteroarylene;
$X_2$ represents C(O), optionally substituted $C_{1-5}$ alkylene, optionally substituted $C_{3-8}$ cycloalkylene, optionally substituted $C_{1-2}$ alkylene-$N(R_{c2})$-, -$N(R_{c2})$-optionally substituted $C_{1-2}$ alkylene, or $N(R_{c3})$, wherein $R_{c2}$ and $R_{c3}$ each independently represent H or $C_{1-3}$ alkyl, or $X_2$ represents a bond;
$R_{a1}$ represents the following structure:

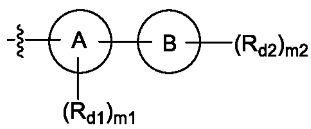

Formula (I-A)

wherein ring A represents heterocyclylene, cycloalkylene, arylene, or heteroarylene, and $(R_{d1})_{m1}$ indicates that ring A is optionally substituted with m1 $R_{d1}$, where m1 represents an integer of 0 to 10, and each $R_{d1}$ independently represents deuterium, hydroxy, amino, mercapto, halogen, cyano, oxo, optionally deuterated $C_{1-6}$ alkyl, optionally deuterated $C_{3-6}$ cycloalkyl, optionally deuterated $C_{1-6}$ alkoxy, halogenated $C_{1-6}$ alkyl, $C_{1-4}$ alkyl-NH-, $C_{1-4}$ alkyl-NHC(O)-, $C_{1-4}$ alkyl-C(O)NH-, or $NH_2$-$C_{1-6}$ alkylene-; and
ring B represents aryl, cycloalkyl, heterocyclyl, or heteroaryl, and $(R_{d2})_{m2}$ indicates that ring B is optionally substituted with m2 $R_{d2}$, where m2 represents an integer of 0 to 10, and each $R_{d2}$ independently represents deuterium, hydroxy, amino, mercapto, halogen, cyano, oxo, optionally deuterated $C_{1-6}$ alkyl, optionally deuterated $C_{3-6}$ cycloalkyl, optionally deuterated $C_{1-6}$ alkoxy, halogenated $C_{1-6}$ alkyl, $C_{1-4}$ alkyl-NH-, $C_{1-4}$ alkyl-NHC(O)-, $C_{1-4}$ alkyl-C(O)NH-, or $NH_2$-$C_{1-6}$ alkylene-;

wherein when $L_1$ represents a bond, $X_1$ represents optionally substituted nitrogen-containing bridged heterocyclylene; and when $X_2$ represents a bond, $L_1$ is not a bond.

In another aspect, the present disclosure provides a pharmaceutical composition comprising the compound of Formula (I) or pharmaceutically acceptable salts, stereoisomers (including enantiomers, diastereoisomers), solvates, isotopically enriched analogs, prodrugs, or polymorphs thereof, and at least one pharmaceutically acceptable carrier.
In a further aspect, the present disclosure further provides a medicine kit or reagent kit comprising: the compound of Formula (I) or a pharmaceutically acceptable salt thereof, or the pharmaceutical composition comprising the same.
In a further aspect, the present disclosure provides the compound of Formula (I) or pharmaceutically acceptable salts, enantiomers, diastereoisomers, solvates, prodrugs, or polymorphs thereof, for use as a medicament.
In a further aspect, the present disclosure provides the compound of Formula (I) or pharmaceutically acceptable

salts, stereoisomers (including enantiomers, diastereoisomers), solvates, isotopically enriched analogs, prodrugs, or polymorphs thereof, or the pharmaceutical composition of the present disclosure for use in the prevention or treatment of diseases or disorders associated with cereblon protein.

In a further aspect, the present disclosure provides use of the compound of Formula (I) or pharmaceutically acceptable salts, stereoisomers (including enantiomers, diastereoisomers), solvates, isotopically enriched analogs, prodrugs, or polymorphs thereof, or the pharmaceutical composition of the present disclosure for the manufacture of a medicament for the prevention or treatment of diseases or disorders associated with cereblon protein.

In a further aspect, the present disclosure provides a method for treating or preventing diseases or disorders associated with cereblon protein in a subject, comprising administering to the subject a therapeutically effective amount of the compound of Formula (I) or pharmaceutically acceptable salts, stereoisomers (including enantiomers, diastereoisomers), solvates, isotopically enriched analogs, prodrugs, or polymorphs thereof, or the pharmaceutical composition.

## Description of Drawings

Figure 1 illustrates the study on the degradation of IKZF1/3 proteins and GSPT1 protein by the compound of the present invention in Human peripheral blood mononuclear cells (hPBMC).

## Detailed Description of the Invention

The following detailed description is provided as exemplary specific embodiments to assist those skilled in the art in understanding and practicing the present disclosure. It should be appreciated, however, that such description is not intended to limit the scope of the present disclosure, and that various modifications and changes may be made to the specific embodiments described in the present disclosure without departing from the spirit and scope of the present disclosure. Such changes and modifications are to be understood as being included within the scope of the present invention as defined by the appended claims.

## I. Compounds

### Compounds of Formula (I)

The present disclosure provides a compound of Formula (I) or salts (including pharmaceutically acceptable salts), stereoisomers (including enantiomers, diastereoisomers), solvates, isotopically enriched analogs, prodrugs, or polymorphs thereof:

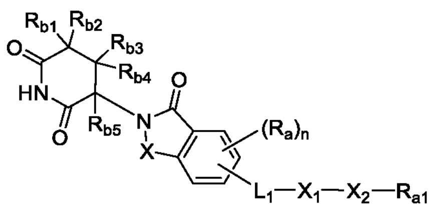

Formula (I)

wherein $R_{b1}$, $R_{b2}$, $R_{b3}$, $R_{b4}$, $R_{b5}$, $(R_a)_n$, X, $L_1$, $X_1$, $X_2$ and $R_{a1}$ are as defined in the compounds of Formula (I) above and its various embodiments herein.

The compounds of Formula (I) or salts (including pharmaceutically acceptable salts), stereoisomers (including enantiomers), solvates, isotopically enriched analogs, prodrugs, or polymorphs thereof of the present disclosure exhibit binding affinity to CRBN, thereby facilitating the recruitment of substrate proteins. The compounds of Formula (I) or salts (including pharmaceutically acceptable salts), stereoisomers (including enantiomers), solvates, isotopically enriched analogs, prodrugs, or polymorphs thereof of the present disclosure are also capable of degrading substrate proteins (e.g., IKZF1/2/3/4 proteins, WEE1 protein, CK1$\alpha$ protein, GSPT1 protein, ZFP91 protein, etc.). The compounds of Formula (I) or salts (including pharmaceutically acceptable salts), stereoisomers (including enantiomers), solvates, isotopically enriched analogs, prodrugs, or polymorphs thereof of the present disclosure demonstrate anti-tumor activity or possess excellent pharmacokinetic properties, making them suitable for use as a therapeutic agent for cancer patients.

In some embodiments of the present disclosure, $R_{b1}$, $R_{b2}$, $R_{b3}$, $R_{b4}$ and $R_{b5}$ are the same or different and each independently represent hydrogen, deuterium, or $C_{1-3}$ alkyl (e.g., methyl, ethyl, or propyl). In some sub-embodiments of the present disclosure, $R_{b1}$, $R_{b2}$, $R_{b3}$, $R_{b4}$ and $R_{b5}$ are the same or different and each independently represent H. In some sub-embodiments of the present disclosure, $R_{b1}$, $R_{b2}$, $R_{b3}$, $R_{b4}$ and $R_{b5}$ are the same or different and each independently

represent D. In some sub-embodiments of the present disclosure, $R_{p1}$, $R_{b2}$, $R_{b3}$ and $R_{b4}$ each independently represent H, and $R_{b5}$ represents H, D or $C_{1-3}$ alkyl (e.g., methyl, ethyl, or propyl).

In some embodiments of the present disclosure, X represents C(O).

In some embodiments of the present disclosure, X represents $CH_2$.

In some embodiments of the present disclosure, $(R_a)_n$ indicates that benzene ring in Formula (I) is optionally substituted with n $R_a$, where $R_a$ represents deuterium, halogen (e.g., fluorine, chlorine, bromine, or iodine), hydroxy, mercapto, nitro, amino, cyano, optionally deuterated $C_{1-6}$ alkyl (e.g., optionally deuterated $C_{1-4}$ alkyl or optionally deuterated $C_{1-3}$ alkyl, such as methyl, $CD_3$, ethyl, propyl, isopropyl, butyl, sec-butyl or tert-butyl), optionally deuterated $C_{1-6}$ alkoxy (e.g., optionally deuterated $C_{1-4}$ alkoxy or optionally deuterated $C_{1-3}$ alkoxy, such as methoxy, ethoxy, or propoxy), or halogenated $C_{1-6}$ alkyl (e.g., halogenated $C_{1-4}$ alkyl or halogenated $C_{1-3}$ alkyl, such as $F_3C$-, $FCH_2$-, $F_2CH$-, $ClCH_2$-, $Cl_2CH$-, $CF_3CF_2$-, $CF_3CHF$-, $CHF_2CF_2$-, $CHF_2CHF$-, $CF_3CH_2$- or $CH_2ClCH_2$-), and n represents an integer of 0, 1, 2 or 3. In some sub-embodiments of the present disclosure, n represents an integer of 0. In some sub-embodiments of the present disclosure, n represents an integer of 1. In some sub-embodiments of the present disclosure, n represents an integer of 2. In some sub-embodiments of the present disclosure, n represents an integer of 3.

In some embodiments of the present disclosure, (a) $L_1$ represents C(O), alkenylene (e.g., $C_{2-8}$ alkenylene, $C_{2-6}$ alkenylene, $C_{2-4}$ alkenylene or $C_{2-3}$ alkenylene), optionally substituted $C_{1-5}$ alkylene (e.g., optionally substituted $C_{1-4}$ alkylene or optionally substituted $C_{1-3}$ alkylene), -CH=, optionally substituted $C_{6-10}$ arylene-$C_{1-5}$ alkylene-* (e.g., optionally substituted $C_{6-10}$ arylene-$C_{1-3}$ alkylene-*), or $N(R_{c1})$, where $R_{c1}$ represents H or $C_{1-3}$ alkyl (e.g., methyl, ethyl, or propyl), and symbol * indicates the point of attachment to $X_1$; or $L_1$ represents -$C(R_{L1}R_{L2})$-, where $R_{L1}$, $R_{L2}$ together with the carbon atom to which they are attached form optionally substituted $C_{3-8}$ cycloalkylene (e.g., optionally substituted $C_{3-7}$ cycloalkylene); and/or (b) $X_1$ represents optionally substituted cycloalkylene (e.g., optionally substituted $C_{3-20}$ cycloalkylene or optionally substituted $C_{3-15}$ cycloalkylene), optionally substituted heterocyclylene (e.g., optionally substituted 4- to 20-membered heterocyclylene or optionally substituted 4- to 15-membered heterocyclylene) or optionally substituted heteroarylene (e.g., optionally substituted 5- to 20-membered heteroarylene or optionally substituted 5- to 15-membered heteroarylene).

In some embodiments of the present disclosure, (a1) $L_1$ represents C(O), $C_{2-6}$ alkenylene (e.g., $C_{2-5}$ alkenylene, $C_{2-4}$ alkenylene or $C_{2-3}$ alkenylene), optionally substituted $C_{1-5}$ alkylene (e.g., optionally substituted $C_{1-4}$ alkylene or optionally substituted $C_{1-3}$ alkylene), -CH=, optionally substituted $C_{6-10}$ arylene-$C_{1-5}$ alkylene-* (e.g., optionally substituted $C_{6-10}$ arylene-$C_{1-3}$ alkylene-*), or $N(R_{c1})$, where $R_{c1}$ represents H or $C_{1-3}$ alkyl (e.g., methyl, ethyl, or propyl), and symbol * indicates the point of attachment to $X_1$, wherein the $C_{1-5}$ alkylene and the $C_{6-10}$ arylene-$C_{1-5}$ alkylene are each independently optionally substituted with 1-10 (e.g., 1-9, 1-8, 1-7, 1-6, 1-5, 1-4, 1-3 or 1-2, or 1) substituents selected from the group consisting of D, optionally deuterated $C_{1-4}$ alkyl (e.g., optionally deuterated $C_{1-3}$ alkyl, such as methyl, $CD_3$, ethyl, propyl, isopropyl, butyl, sec-butyl or tert-butyl), hydroxy, amino, mercapto, halogen (e.g., fluorine, chlorine, bromine, or iodine), cyano, $C_{1-4}$ alkoxy (e.g., $C_{1-3}$ alkoxy, such as methoxy, ethoxy, or propoxy), $C_{1-4}$ alkyl-NH- (e.g., $C_{1-3}$ alkyl-NH-, such as $CH_3NH$-, $CH_3CH_2NH$- or $CH_3CH_2CH_2NH$-), halogenated $C_{1-4}$ alkyl (e.g., halogenated $C_{1-3}$ alkyl, such as $F_3C$-, $FCH_2$-, $F_2CH$-, $ClCH_2$-, $Cl_2CH$-, $CF_3CF_2$-, $CF_3CHF$-, $CHF_2CF_2$-, $CHF_2CHF$-, $CF_3CH_2$- or $CH_2ClCH_2$-), $NH_2$-$C_{1-4}$ alkylene- (e.g., $NH_2$-$C_{1-3}$ alkylene-, such as $NH_2CH_2$-, $NH_2CH_2CH_2$- or $NH_2CH_2CH_2CH_2$-), $C_{1-4}$ alkyl-NHC(O)- (e.g., $C_{1-3}$ alkyl-NHC(O)- , such as $CH_3$-NHC(O)-, $CH_3CH_2$-NHC(O)-, or $CH_3CH_2CH_2$-NHC(O)-), $C_{1-4}$ alkyl-C(O)NH- (e.g., $C_{1-3}$ alkyl-C(O) NH-, such as $CH_3$-C(O)NH-, $CH_3CH_2$-C(O)NH-, or $CH_3CH_2CH_2$-C(O)NH-) and any combination thereof; or $L_1$ represents -$C(R_{L1}R_{L2})$-, where $R_{L1}$, $R_{L2}$ together with the carbon atom to which they are attached form optionally substituted $C_{3-8}$ cycloalkylene (e.g., optionally substituted $C_{3-7}$ cycloalkylene); and/or

(b1) $X_1$ represents optionally substituted $C_{3-20}$ cycloalkylene, optionally substituted 4- to 20-membered heterocyclylene or optionally substituted 5- to 20-membered heteroarylene, wherein the $C_{3-20}$ cycloalkylene and the 4- to 20-membered heterocyclylene are each independently optionally substituted with one or more (e.g., 1-15, 1-10, 1-9, 1-8, 1-7, 1-6, 1-5, 1-4, 1-3 or 1-2, or 1) substituents selected from the group consisting of D, optionally deuterated $C_{1-4}$ alkyl (e.g., optionally deuterated $C_{1-3}$ alkyl, such as methyl, $CD_3$, ethyl, propyl, isopropyl, butyl, sec-butyl or tert-butyl), hydroxy, amino, mercapto, halogen (e.g., fluorine, chlorine, bromine, or iodine), cyano, oxo, $C_{1-4}$ alkoxy (e.g., $C_{1-3}$ alkoxy, such as methoxy, ethoxy, or propoxy), $C_{1-4}$ alkyl-NH-(e.g., $C_{1-3}$ alkyl-NH-, such as $CH_3NH$-, $CH_3CH_2NH$- or $CH_3CH_2CH_2NH$-), halogenated $C_{1-4}$ alkyl (e.g., halogenated $C_{1-3}$ alkyl, such as $F_3C$-, $FCH_2$-, $F_2CH$-, $ClCH_2$-, $Cl_2CH$-, $CF_3CF_2$-, $CF_3CHF$-, $CHF_2CF_2$-, $CHF_2CHF$-, $CF_3CH_2$- or $CH_2ClCH_2$-), $NH_2$-$C_{1-4}$ alkylene (e.g., $NH_2$-$C_{1-3}$ alkylene-, such as $NH_2CH_2$-, $NH_2CH_2CH_2$- or $NH_2CH_2CH_2CH_2$-), $C_{1-4}$ alkyl-NHC(O)- (e.g., $C_{1-3}$ alkyl-NHC(O)-, such as $CH_3$-NHC(O)-, $CH_3CH_2$-NHC(O)-, or $CH_3CH_2CH_2$-NHC(O)-), $C_{1-4}$ alkyl-C(O)NH- (e.g., $C_{1-3}$ alkyl-C(O)NH-, such as $CH_3$-C(O)NH-, $CH_3CH_2$-C(O)NH-, or $CH_3CH_2CH_2$-C(O)NH-) and any combination thereof, and the 5- to 20-membered heteroarylene is optionally substituted with one or more (e.g., 1-15, 1-10, 1-9, 1-8, 1-7, 1-6, 1-5, 1-4, 1-3 or 1-2, or 1) substituents selected from the group consisting of D, optionally deuterated $C_{1-4}$ alkyl (e.g., optionally deuterated $C_{1-3}$ alkyl, such as methyl, $CD_3$, ethyl, propyl, isopropyl, butyl, sec-butyl or tert-butyl), hydroxy, amino, mercapto, halogen (e.g., fluorine, chlorine, bromine, or iodine), cyano, $C_{1-4}$ alkoxy (e.g., $C_{1-3}$ alkoxy, such as methoxy, ethoxy, or propoxy), $C_{1-4}$ alkyl-NH- (e.g., $C_{1-3}$ alkyl-NH-, such as $CH_3NH$-, $CH_3CH_2NH$- or $CH_3CH_2CH_2NH$-), halogenated $C_{1-4}$ alkyl (e.g., halogenated $C_{1-3}$ alkyl, such as $F_3C$-,

$FCH_2$-, $F_2CH$-, $ClCH_2$-, $Cl_2CH$-, $CF_3CF_2$-, $CF_3CHF$-, $CHF_2CF_2$-, $CHF_2CHF$-, $CF_3CH_2$- or $CH_2ClCH_2$-), $NH_2$-$C_{1-4}$ alkylene- (e.g., $NH_2$-$C_{1-3}$ alkylene-, such as $NH_2CH_2$-, $NH_2CH_2CH_2$- or $NH_2CH_2CH_2CH_2$-), $C_{1-4}$ alkyl-NHC(O)- (e.g., $C_{1-3}$ alkyl-NHC(O)-, such as $CH_3$-NHC(O)-, $CH_3CH_2$-NHC(O)-, or $CH_3CH_2CH_2$-NHC(O)-), $C_{1-4}$ alkyl-C(O)NH- (e.g., $C_{1-3}$ alkyl-C(O)NH-, such as $CH_3$-C(O)NH-, $CH_3CH_2$-C(O)NH-, or $CH_3CH_2CH_2$-C(O)NH-) and any combination thereof.

In some embodiments of the present disclosure, (a2) $L_1$ represents C(O), vinylene (i.e., - CH=CH-), optionally substituted $C_{1-5}$ alkylene (e.g., optionally substituted -$CH_2$-, -$(CH_2)_2$- and -$(CH_2)_3$-, such as -CH(OH)-, -$CH_2$-, -$(CH_2)_2$-, -$(CH_2)_3$-, -$CHF_2$- and -$CH_2F$-), NH, -CH=, or optionally substituted $C_{6-10}$ arylene-$C_{1-3}$ alkylene-* (e.g., optionally substituted arylene-$C_{1-3}$ alkylene-*, or optionally substituted naphthylene-$C_{1-3}$ alkylene-*, such as optionally substituted arylene-$CH_2$-*), wherein symbol * indicates the point of attachment to $X_1$, wherein the $C_{1-5}$ alkylene and $C_{6-10}$ arylene-$C_{1-3}$ alkylene are each independently optionally substituted with 1-4 (e.g., 1-3 or 1-2, or 1) substituents selected from the group consisting of D, optionally deuterated $C_{1-4}$ alkyl (e.g., optionally deuterated $C_{1-3}$ alkyl, such as methyl, $CD_3$, ethyl, propyl, isopropyl, butyl, sec-butyl or tert-butyl), hydroxy, amino, mercapto, halogen (e.g., fluorine, chlorine, bromine, or iodine), cyano, $C_{1-4}$ alkoxy (e.g., $C_{1-3}$ alkoxy, such as methoxy, ethoxy, or propoxy), $C_{1-4}$ alkyl-NH- (e.g., $C_{1-3}$ alkyl-NH-, such as $CH_3NH$-, $CH_3CH_2NH$- or $CH_3CH_2CH_2NH$-), halogenated $C_{1-4}$ alkyl (e.g., halogenated $C_{1-3}$ alkyl, such as $F_3C$-, $FCH_2$-, $F_2CH$-, $ClCH_2$-, $Cl_2CH$-, $CF_3CF_2$-, $CF_3CHF$-, $CHF_2CF_2$-, $CHF_2CHF$-, $CF_3CH_2$- or $CH_2ClCH_2$-), $NH_2$-$C_{1-4}$ alkylene- (e.g., $NH_2$-$C_{1-3}$ alkylene-, such as $NH_2CH_2$-, $NH_2CH_2CH_2$- or $NH_2CH_2CH_2CH_2$-), $C_{1-4}$ alkyl-NHC(O)- (e.g., $C_{1-3}$ alkyl-NHC(O)-, such as $CH_3$-NHC(O)-, $CH_3CH_2$-NHC(O)-, or $CH_3CH_2CH_2$-NHC(O)-), $C_{1-4}$ alkyl-C(O)NH- (e.g., $C_{1-3}$ alkyl-C(O)NH-, such as $CH_3$-C(O)NH-, $CH_3CH_2$-C(O)NH-, or $CH_3CH_2CH_2$-C(O)NH-) and any combination thereof; or $L_1$ represents -$C(R_{L1}R_{L2})$-, where $R_{L1}$, $R_{L2}$ together with the carbon atom to which they are attached form optionally substituted $C_{3-8}$ cycloalkylene (e.g., optionally substituted cyclopropylene, optionally substituted cyclobutylene, optionally substituted cyclopentylene, optionally substituted cyclohexylene and optionally substituted cycloheptylene); and/or

(b2) $X_1$ represents optionally substituted $C_{3-20}$ cycloalkylene, optionally substituted 4- to 20-membered heterocyclylene or optionally substituted 5- to 20-membered heteroarylene, wherein the $C_{3-20}$ cycloalkylene and the 4- to 20-membered heterocyclylene are each independently optionally substituted with one or more (e.g., 1-15, 1-10, 1-9, 1-8, 1-7, 1-6, 1-5, 1-4, 1-3 or 1-2, or 1) substituents selected from the group consisting of D, optionally deuterated $C_{1-4}$ alkyl (e.g., optionally deuterated $C_{1-3}$ alkyl, such as methyl, $CD_3$, ethyl, propyl, isopropyl, butyl, sec-butyl or tert-butyl), hydroxy, amino, mercapto, halogen (e.g., fluorine, chlorine, bromine, or iodine), cyano, oxo, $C_{1-4}$ alkoxy (e.g., $C_{1-3}$ alkoxy, such as methoxy, ethoxy, or propoxy), $C_{1-4}$ alkyl-NH-(e.g., $C_{1-3}$ alkyl-NH-, such as $CH_3NH$-, $CH_3CH_2NH$- or $CH_3CH_2CH_2NH$-), halogenated $C_{1-4}$ alkyl (e.g., halogenated $C_{1-3}$ alkyl, such as $F_3C$-, $FCH_2$-, $F_2CH$-, $ClCH_2$-, $Cl_2CH$-, $CF_3CF_2$-, $CF_3CHF$-, $CHF_2CF_2$-, $CHF_2CHF$-, $CF_3CH_2$- or $CH_2ClCH_2$-), $NH_2$-$C_{1-4}$ alkylene (e.g., $NH_2$-$C_{1-3}$ alkylene-, such as $NH_2CH_2$-, $NH_2CH_2CH_2$- or $NH_2CH_2CH_2CH_2$-), $C_{1-4}$ alkyl-NHC(O)- (e.g., $C_{1-3}$ alkyl-NHC(O)-, such as $CH_3$-NHC(O)-, $CH_3CH_2$-NHC(O)-, or $CH_3CH_2CH_2$-NHC(O)-), $C_{1-4}$ alkyl-C(O)NH- (e.g., $C_{1-3}$ alkyl-C(O)NH-, such as $CH_3$-C(O)NH-, $CH_3CH_2$-C(O)NH-, or $CH_3CH_2CH_2$-C(O)NH-) and any combination thereof, and the 5- to 20-membered heteroarylene is optionally substituted with one or more (e.g., 1-15, 1-10, 1-9, 1-8, 1-7, 1-6, 1-5, 1-4, 1-3 or 1-2, or 1) substituents selected from the group consisting of D, optionally deuterated $C_{1-4}$ alkyl (e.g., optionally deuterated $C_{1-3}$ alkyl, such as methyl, $CD_3$, ethyl, propyl, isopropyl, butyl, sec-butyl or tert-butyl), hydroxy, amino, mercapto, halogen (e.g., fluorine, chlorine, bromine, or iodine), cyano, $C_{1-4}$ alkoxy (e.g., $C_{1-3}$ alkoxy, such as methoxy, ethoxy, or propoxy), $C_{1-4}$ alkyl-NH- (e.g., $C_{1-3}$ alkyl-NH-, such as $CH_3NH$-, $CH_3CH_2NH$- or $CH_3CH_2CH_2NH$-), halogenated $C_{1-4}$ alkyl (e.g., halogenated $C_{1-3}$ alkyl, such as $F_3C$-, $FCH_2$-, $F_2CH$-, $ClCH_2$-, $Cl_2CH$-, $CF_3CF_2$-, $CF_3CHF$-, $CHF_2CF_2$-, $CHF_2CHF$-, $CF_3CH_2$- or $CH_2ClCH_2$-), $NH_2$-$C_{1-4}$ alkylene- (e.g., $NH_2$-$C_{1-3}$ alkylene-, such as $NH_2CH_2$-, $NH_2CH_2CH_2$- or $NH_2CH_2CH_2CH_2$-), $C_{1-4}$ alkyl-NHC(O)- (e.g., $C_{1-3}$ alkyl-NHC(O)-, such as $CH_3$-NHC(O)-, $CH_3CH_2$-NHC(O)-, or $CH_3CH_2CH_2$-NHC(O)-), $C_{1-4}$ alkyl-C(O)NH- (e.g., $C_{1-3}$ alkyl-C(O)NH-, such as $CH_3$-C(O)NH-, $CH_3CH_2$-C(O)NH-, or $CH_3CH_2CH_2$-C(O)NH-) and any combination thereof.

In embodiments of the present disclosure, the number of substituents is not limited in principle, or is automatically limited by the size of the building units.

In some embodiments of the present disclosure, (a3) $L_1$ represents C(O), vinylene, optionally substituted $C_{1-5}$ alkylene (e.g., optionally substituted $C_{1-4}$ alkylene or optionally substituted $C_{1-3}$ alkylene, such as -CH(OH)-, -$CH_2$-, -$(CH_2)_2$-, -$(CH_2)_3$-, -$CHF_2$-and -$CH_2F$-), -CH=, optionally substituted arylene-$C_{1-5}$ alkylene-* (e.g., optionally substituted arylene-$C_{1-3}$ alkylene-*, such as optionally substituted arylene-$CH_2$-*), optionally substituted naphthylene-$C_{1-5}$ alkylene-* (e.g., optionally substituted naphthylene-$C_{1-3}$ alkylene-*), or $N(R_{c1})$, where $R_{c1}$ represents H or $C_{1-3}$ alkyl (e.g., methyl, ethyl, or propyl), and symbol * indicates the point of attachment to $X_1$, wherein the $C_{1-5}$ alkylene, the arylene-$C_{1-5}$ alkylene and the naphthylene-$C_{1-5}$ alkylene are each independently optionally substituted with 1-10 (e.g., 1-9, 1-8, 1-7, 1-6, 1-5, 1-4, 1-3 or 1-2, or 1) substituents selected from the group consisting of D, optionally deuterated $C_{1-4}$ alkyl, hydroxy, amino, mercapto, halogen, cyano, $C_{1-4}$ alkoxy, $C_{1-4}$ alkyl-NH-, halogenated $C_{1-4}$ alkyl, $NH_2$-$C_{1-4}$ alkylene-, $C_{1-4}$ alkyl-NHC(O)-, $C_{1-4}$ alkyl-C(O)NH- and any combination thereof; or $L_1$ represents -$C(R_{L1}R_{L2})$-, where $R_{L1}$, $R_{L2}$ together with the carbon atom to which they are attached form optionally substituted $C_{3-8}$ cycloalkylene (e.g., optionally substituted cyclopropylene, optionally substituted cyclobutylene, optionally substituted cyclopentylene, optionally substituted cyclohexylene and optionally substituted cycloheptylene), and/or

(b3) $X_1$ represents the following divalent groups:

cyclopropylene, cyclobutylene, cyclopentylene, cyclopentenylene, cyclohexylene, cyclohexenylene, cycloheptylene, cyclooctylene, decalinylene, octahydropentalenylene, octahydro-1H-indenylene, spiro[3.3]heptylene, spiro[2.5]octylene, spiro[3.5]nonylene, spiro[4.4]nonylene, spiro[4.5]decylene, spiro[5.5]undecylene, p-menthanylene, m-menthanylene, quinuclidinylene, adamantanylene, noradamantanylene, norbomylene, bicyclo[2.2.1]heptylene, 2-oxobicyclo[2.2.1]heptylene, or bicyclo[2.2.1]heptentylene, with each group being optionally substituted with one or more (e.g., 1-15, 1-10, 1-9, 1-8, 1-7, 1-6, 1-5, 1-4, 1-3 or 1-2, or 1) substituents selected from the group consisting of D, optionally deuterated $C_{1-4}$ alkyl, hydroxy, amino, mercapto, halogen, cyano, oxo, $C_{1-4}$ alkoxy, $C_{1-4}$ alkyl-NH-, halogenated $C_{1-4}$ alkyl, $NH_2$-$C_{1-4}$ alkylene-, $C_{1-4}$ alkyl-NHC(O)-, $C_{1-4}$ alkyl-C(O)NH- and any combination thereof; or azetidinylene, pyrrolidinylene, imidazolidylene, pyrazolidylene, oxazolidinylene, thiazolidinylene, piperidinylene, piperazinylene, morpholinylene, thiomorpholinylene, azepanylene, azacyclooctylene, diazacycloheptanylene, diazacyclooctylene, 3-azabicyclo[3.1.0]hexylene, 3-azabicyclo[3.1.1]heptanylene, 2-azabicyclo[2.2.1]heptanylene, 6-azabicyclo[3.1.1]heptanylene, 2-azabicyclo[2.2.2]octylene, 2,5-diazabicyclo[2.2.1]heptanylene, 3,6-diazabicyclo[3.1.1]heptanylene, 3-azabicyclo[3.2.1]octylene, 3,8-diazabicyclo[3.2.1]octylene, 2,5-diazabicyclo[2.2.2]octylene, 2,6-diazaspiro[3.3]heptanylene, 2,7-diazaspiro[3.5]nonylene, 3-azaspiro[5.5]undecylene, 7-azaspiro[3.5]nonylene, or octahydropyrrolo[3,4-c]pyrrolylene, with each group being optionally substituted with one or more (e.g., 1-15, 1-10, 1-9, 1-8, 1-7, 1-6, 1-5, 1-4, 1-3 or 1-2, or 1) substituents selected from the group consisting of D, optionally deuterated $C_{1-4}$ alkyl, hydroxy, amino, mercapto, halogen, cyano, oxo, $C_{1-4}$ alkoxy, $C_{1-4}$ alkyl-NH-, halogenated $C_{1-4}$ alkyl, $NH_2$-$C_{1-4}$ alkylene-, $C_{1-4}$ alkyl-NHC(O)-, $C_{1-4}$ alkyl-C(O)NH- and any combination thereof; or furanylene, oxazolylene, isoxazolylene, oxadiazolylene, thienylene, thiazolylene, isothiazolylene, thiadiazolylene, pyrrolylene, imidazolylene, pyrazolylene, triazolylene, pyridylene, pyrimidinylene, pyridazinylene, pyrazinylene, indolylene, isoindolylene, benzofuranylene, isobenzofuranylene, benzothienylene, indazolylene, benzimidazolylene, benzoxazolylene, benzisoxazolylene, benzothiazolylene, benzisothiazolylene, benzotriazolylene, benzo[2,1,3]oxadiazolylene, benzo[2,1,3]thiadiazolylene, benzo[1,2,3]thiadiazolylene, quinolinylene, isoquinolinylene, naphthyridinylene, cinnolinylene, quinazolinylene, quinoxalinylene, phthalazinylene, pyrazolo[1,5-a]pyridylene, pyrazolo[1,5-a]pyrimidinylene, imidazo[1,2-a]pyridylene, 1H-pyrrolo[3,2-b]pyridylene, 1H-pyrrolo[2,3-b]pyridylene, 4H-fluoro[3,2-b]pyrrolylene, pyrrolo[2,1-b]thiazolylene, or imidazo[2,1-b]thiazolylene, with each group being optionally substituted with one or more (e.g., 1-15, 1-10, 1-9, 1-8, 1-7, 1-6, 1-5, 1-4, 1-3 or 1-2, or 1) substituents selected from the group consisting of D, optionally deuterated $C_{1-4}$ alkyl, hydroxy, amino, mercapto, halogen, cyano, $C_{1-4}$ alkoxy, $C_{1-4}$ alkyl-NH-, halogenated $C_{1-4}$ alkyl, $NH_2$-$C_{1-4}$ alkylene-, $C_{1-4}$ alkyl-NHC(O)-, $C_{1-4}$ alkyl-C(O)NH- and any combination thereof.

In some embodiments of the present disclosure, (a4) $L_1$ represents the following groups: C(O), $-CH_2-$, $-(CH_2)_2-$, $-(CH_2)_3-$, -CH(OH)-, $-CHF_2-$, $-CH_2F-$, NH, -CH=CH-, -CH=, or optionally substituted arylene-$CH_2$-*, where symbol * indicates the point of attachment to $X_1$, wherein the arylene is optionally substituted with 1-4 (e.g., 1-3 or 1-2, or 1) substituents selected from the group consisting of D, optionally deuterated $C_{1-4}$ alkyl, hydroxy, amino, mercapto, halogen, cyano, $C_{1-4}$ alkoxy, $C_{1-4}$ alkyl-NH-, halogenated $C_{1-4}$ alkyl, $NH_2$-$C_{1-4}$ alkylene-, $C_{1-4}$ alkyl-NHC(O)-, $C_{1-4}$ alkyl-C(O)NH- and any combination thereof; or $L_1$ represents $-C(R_{L1}R_{L2})-$, where $R_{L1}$, $R_{L2}$ together with the carbon atom to which they are attached form cyclopropylene, cyclobutylene, cyclopentylene, cyclohexylene or cycloheptylene, which are each independently optionally substituted with one or more (e.g., 1-10, 1-8, 1-6, 1-4, 1-3, 1-2 or 1) substituents selected from the group consisting of D, optionally deuterated $C_{1-4}$ alkyl, hydroxy, amino, mercapto, halogen, cyano, oxo, $C_{1-4}$ alkoxy, $C_{1-4}$ alkyl-NH-, halogenated $C_{1-4}$ alkyl, $NH_2$-$C_{1-4}$ alkylene-, $C_{1-4}$ alkyl-NHC(O)-, $C_{1-4}$ alkyl-C(O)NH- and any combination thereof.

In some embodiments of the present disclosure, (b4) $X_1$ represents the following divalent groups:

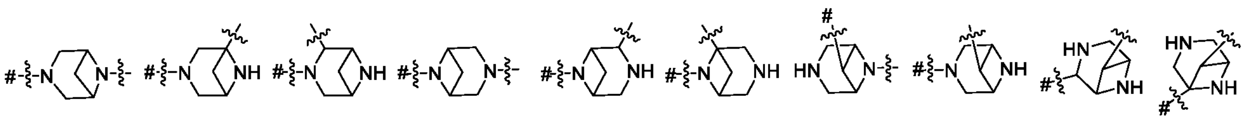

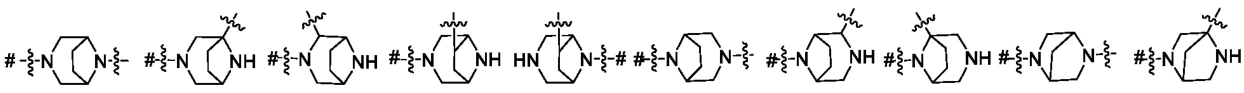

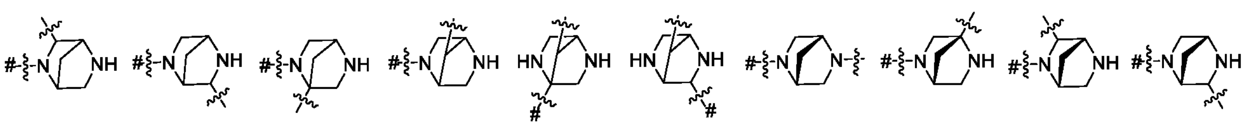

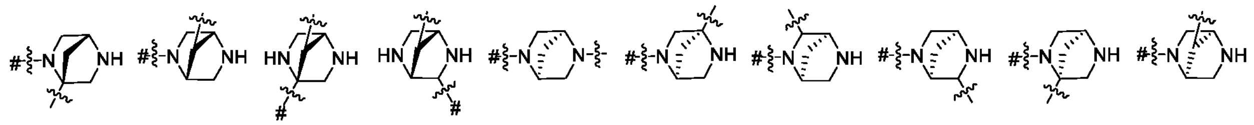

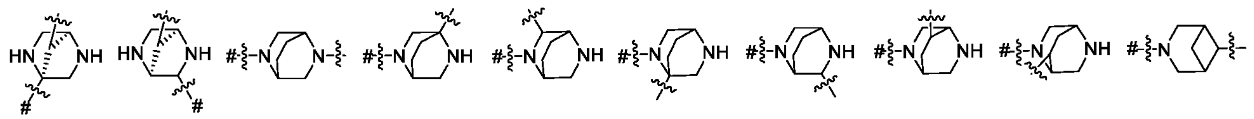

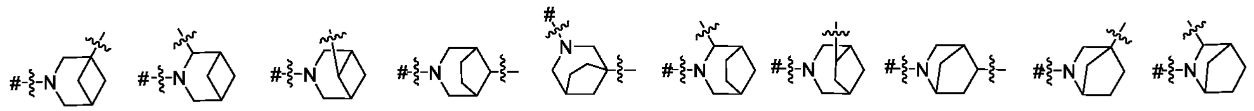

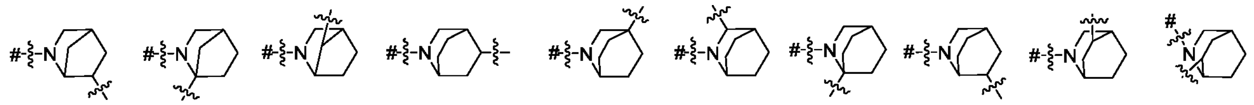

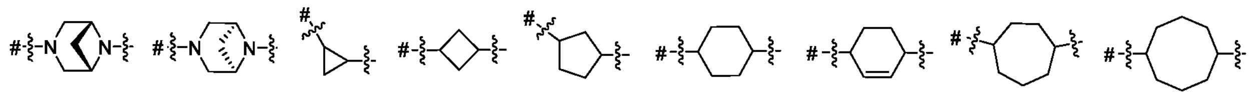

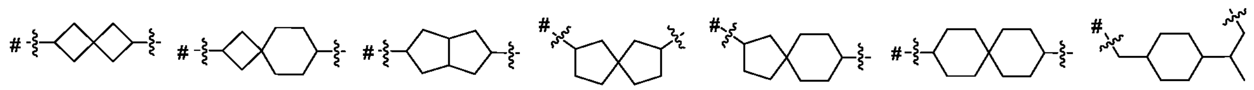

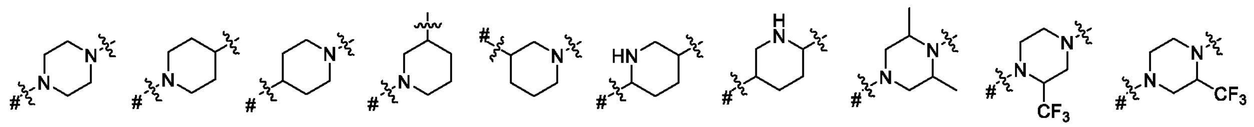

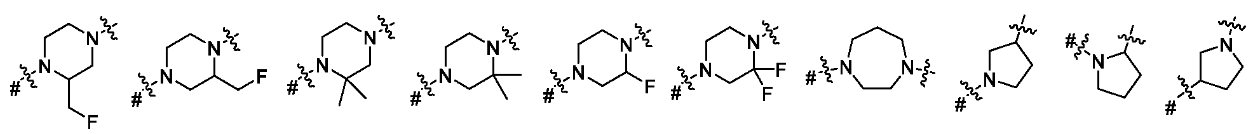

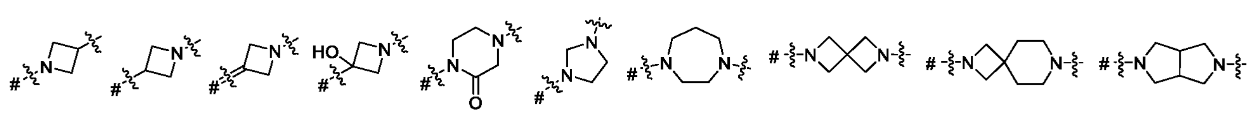

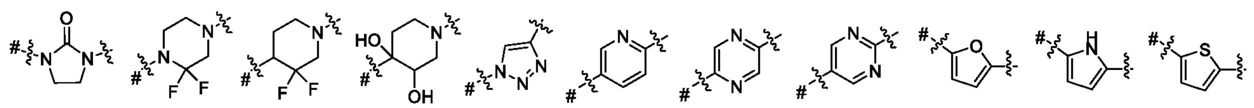

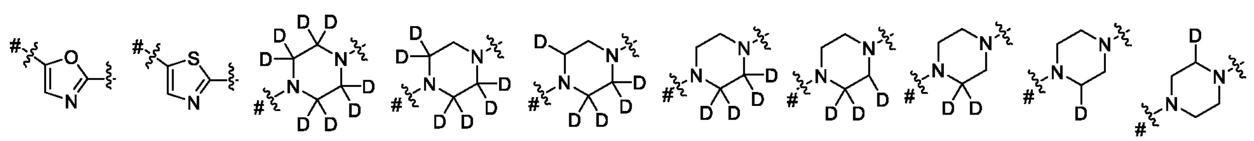

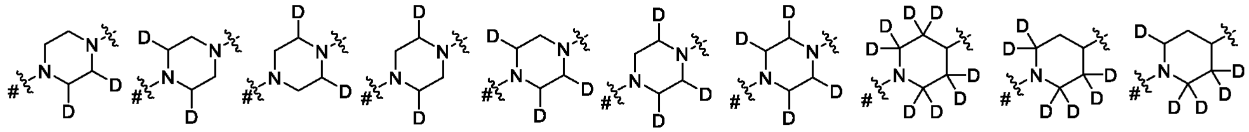

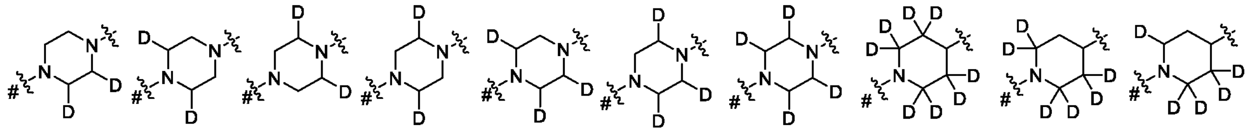

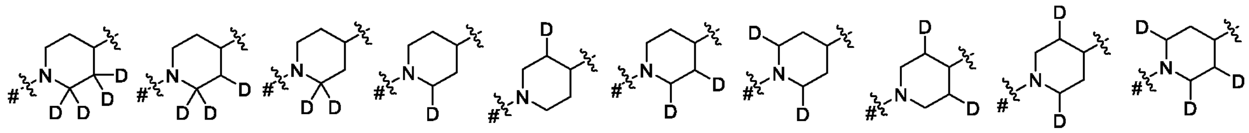

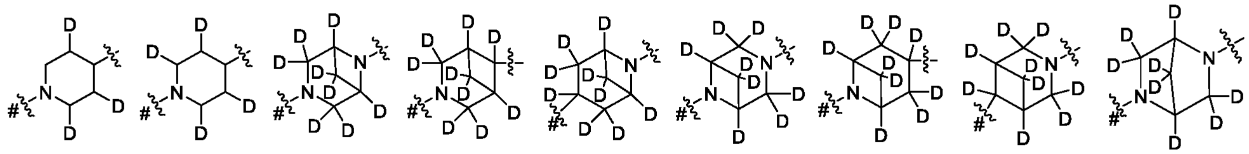

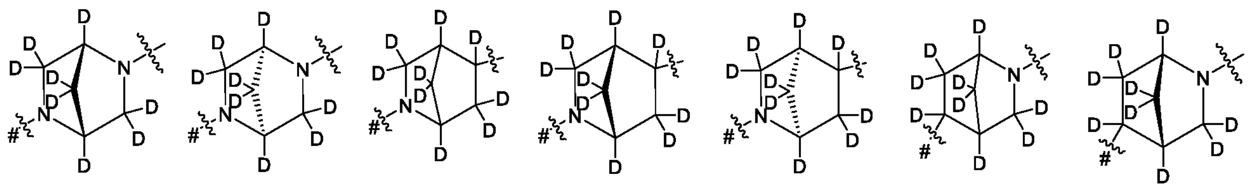

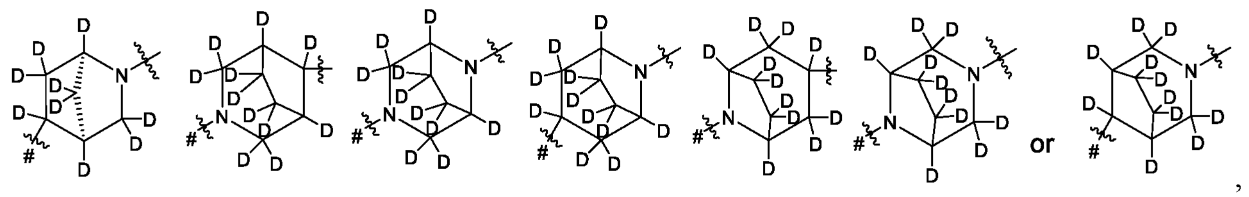

or ,

wherein symbol # indicates the point of attachment to $L_1$.

In some embodiments of the present disclosure, (c) $L_1$ represents a bond, and $X_1$ represents optionally substituted nitrogen-containing bridged heterocyclylene (e.g., optionally substituted 5- to 20-membered nitrogen-containing bridged heterocyclylene or optionally substituted 5- to 15-membered nitrogen-containing bridged heterocyclylene).

In some embodiments of the present disclosure, (c1) $L_1$ represents a bond, and $X_1$ represents optionally substituted 5- to 20-membered nitrogen-containing bridged heterocyclylene, wherein the 5-to 20-membered nitrogen-containing bridged heterocyclylene is optionally substituted with one or more (e.g., 1-15, 1-10, 1-9, 1-8, 1-7, 1-6, 1-5, 1-4, 1-3 or 1-2, or 1) substituents selected from the group consisting of D, optionally deuterated $C_{1-4}$ alkyl (e.g., optionally deuterated $C_{1-3}$ alkyl, such as methyl, $CD_3$, ethyl, propyl, isopropyl, butyl, sec-butyl or tert-butyl), hydroxy, amino, mercapto, halogen (e.g., fluorine, chlorine, bromine, or iodine), cyano, oxo, $C_{1-4}$ alkoxy (e.g., $C_{1-3}$ alkoxy, such as methoxy, ethoxy, or propoxy), $C_{1-4}$ alkyl-NH- (e.g., $C_{1-3}$ alkyl-NH-, such as $CH_3NH$-, $CH_3CH_2NH$- or $CH_3CH_2CH_2NH$-), halogenated $C_{1-4}$ alkyl (e.g., halogenated $C_{1-3}$ alkyl, such as $F_3C$-, $FCH_2$-, $F_2CH$-, $ClCH_2$-, $Cl_2CH$-, $CF_3CF_2$-, $CF_3CHF$-, $CHF_2CF_2$-, $CHF_2CHF$-, $CF_3CH_2$- or $CH_2ClCH_2$-), $NH_2$-$C_{1-4}$ alkylene- (e.g., $NH_2$-$C_{1-3}$ alkylene-, such as $NH_2CH_2$-, $NH_2CH_2CH_2$- or $NH_2CH_2CH_2CH_2$-), $C_{1-4}$ alkyl-NHC(O)- (e.g., $C_{1-3}$ alkyl-NHC(O)-, such as $CH_3$-NHC(O)-, $CH_3CH_2$-NHC(O)-, or $CH_3CH_2CH_2$-NHC(O)-), $C_{1-4}$ alkyl-C(O)NH- (e.g., $C_{1-3}$ alkyl-C(O)NH-, such as $CH_3$-C(O)NH-, $CH_3CH_2$-C(O)NH-, or $CH_3CH_2CH_2$-C(O)NH-) and any combination thereof.

In some embodiments of the present disclosure, (c2) $L_1$ represents a bond, and $X_1$ represents the following divalent groups:

3-azabicyclo[3.1.0]hexylene, 3-azabicyclo[3.1.1]heptanylene, 2-azabicyclo[2.2.1]heptanylene, 6-azabicyclo[3.1.1]heptanylene, 2-azabicyclo[2.2.2]octylene, 2,5-diazabicyclo[2.2.1]heptanylene, 3,6-diazabicyclo[3.1.1]heptanylene, 3-azabicyclo[3.2.1]octylene, 3,8-diazabicyclo[3.2.1]octylene, or 2,5-diazabicyclo[2.2.2]octylene, which are optionally substituted with one or more (e.g., 1-10, 1-9, 1-8, 1-7, 1-6, 1-5, 1-4, 1-3 or 1-2, or 1) substituents selected from the group consisting of D, optionally deuterated $C_{1-4}$ alkyl, hydroxy, amino, mercapto, halogen, cyano, oxo, $C_{1-4}$ alkoxy, $C_{1-4}$ alkyl-NH-, halogenated $C_{1-4}$ alkyl, $NH_2$-$C_{1-4}$ alkylene-, $C_{1-4}$ alkyl-NHC(O)-, $C_{1-4}$ alkyl-C(O)NH- and any combination thereof.

In some embodiments of the present disclosure, (c3) $L_1$ represents a bond, and $X_1$ represents the following divalent groups:

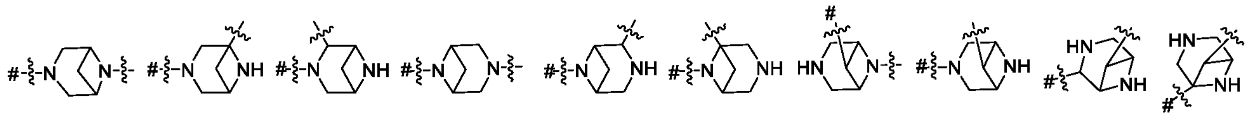

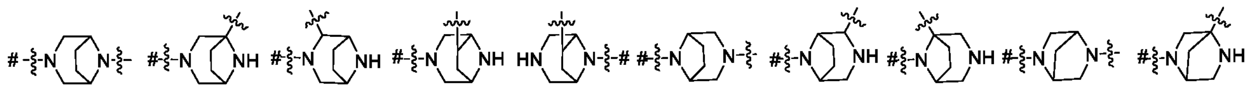

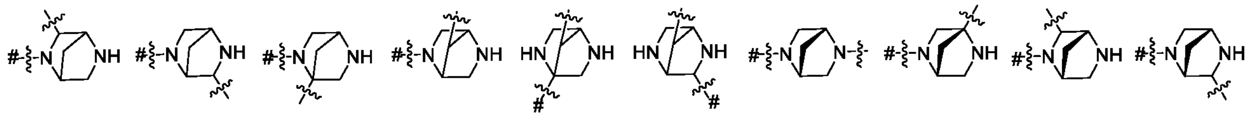

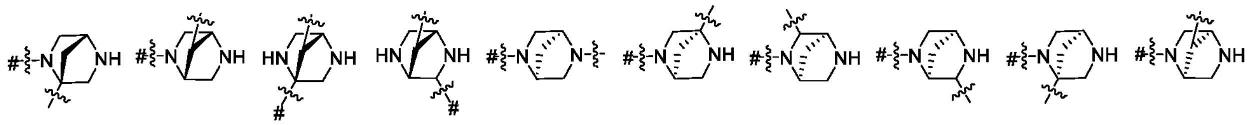

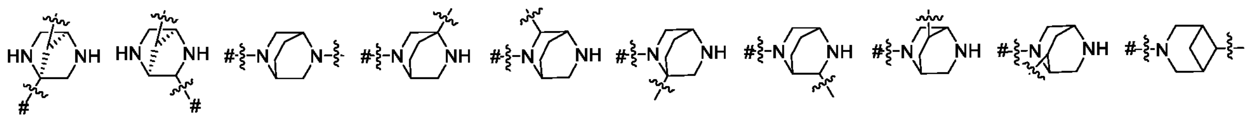

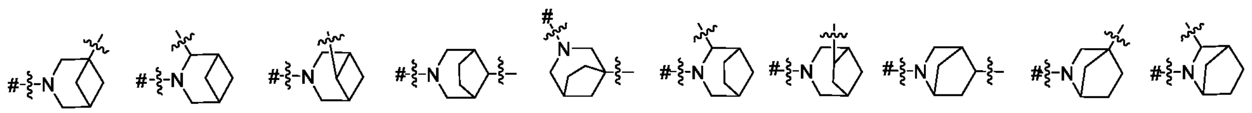

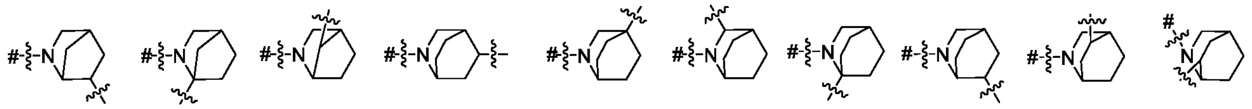

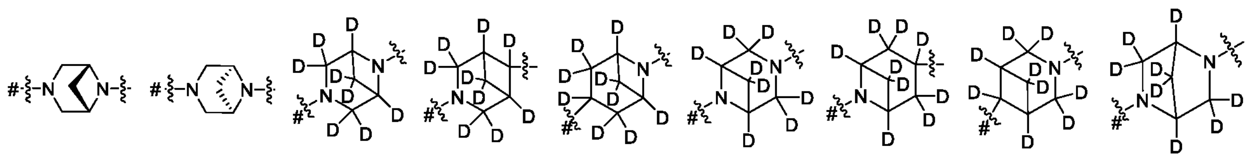

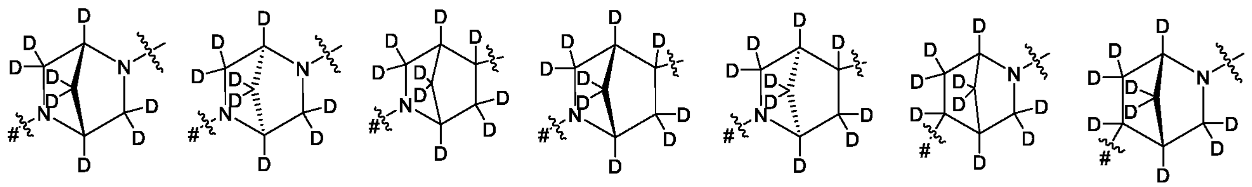

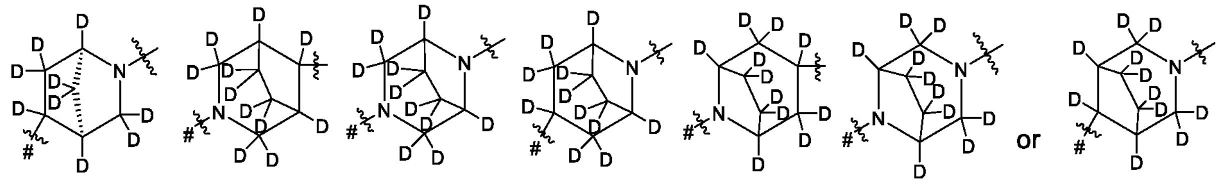

or

,

wherein symbol # indicates the point of attachment to $L_1$.

In some embodiments of the present disclosure, $X_2$ represents a bond, and when $X_2$ represents a bond, $L_1$ is not a bond.

In some embodiments of the present disclosure, $X_2$ represents the following groups:

C(O), optionally substituted $C_{1-5}$ alkylene (e.g., optionally substituted $C_{1-4}$ alkylene or optionally substituted $C_{1-3}$ alkylene), optionally substituted $C_{3-8}$ cycloalkylene, optionally substituted $C_{1-2}$ alkylene-N($R_{c2}$)-, -N($R_{c2}$)-optionally substituted $C_{1-2}$ alkylene, or N($R_{c3}$), where $R_{c2}$ and $R_{c3}$ each independently represent H or $C_{1-3}$ alkyl (e.g., methyl, ethyl, or propyl),

wherein the $C_{1-5}$ alkylene and the $C_{3-8}$ cycloalkylene are each independently optionally substituted with 1-10 (e.g., 1-9, 1-8, 1-7, 1-6, 1-5, 1-4, 1-3 or 1-2, or 1) substituents selected from the group consisting of D, optionally deuterated $C_{1-4}$ alkyl (e.g., optionally deuterated $C_{1-3}$ alkyl, such as methyl, $CD_3$, ethyl, propyl, isopropyl, butyl, sec-butyl or tert-butyl), hydroxy, amino, mercapto, halogen (e.g., fluorine, chlorine, bromine, or iodine), cyano, oxo, $C_{1-4}$ alkoxy (e.g., $C_{1-3}$ alkoxy, such as methoxy, ethoxy, or propoxy), $C_{3-8}$ cycloalkyl (e.g., $C_{3-7}$ cycloalkyl, $C_{3-6}$ cycloalkyl or $C_{3-5}$ cycloalkyl), $C_{1-4}$ alkyl-NH- (e.g., $C_{1-3}$ alkyl-NH-, such as $CH_3NH$-, $CH_3CH_2NH$- or $CH_3CH_2CH_2NH$-), halogenated $C_{1-4}$ alkyl (e.g., halogenated $C_{1-3}$ alkyl, such as $F_3C$-, $FCH_2$-, $F_2CH$-, $ClCH_2$-, $Cl_2CH$-, $CF_3CF_2$-, $CF_3CHF$-, $CHF_2CF_2$-, $CHF_2CHF$-, $CF_3CH_2$- or $CH_2ClCH_2$-), $NH_2$-$C_{1-4}$ alkylene (e.g., $NH_2$-$C_{1-3}$ alkylene-, such as $NH_2CH_2$-, $NH_2CH_2CH_2$- or $NH_2CH_2CH_2CH_2$-), $C_{1-4}$ alkyl-NHC(O)- (e.g., $C_{1-3}$ alkyl-NHC(O)-, such as $CH_3$-NHC(O)-, $CH_3CH_2$-NHC(O)-, or $CH_3CH_2CH_2$-NHC(O)-), $C_{1-4}$ alkyl-C(O)NH- (e.g., $C_{1-3}$ alkyl-C(O)NH-, such as $CH_3$-C(O)NH-, $CH_3CH_2$-C(O)NH-, or $CH_3CH_2CH_2$-C(O)NH-) and any combination thereof, and

the $C_{1-2}$ alkylene is optionally substituted with 1-4 (e.g., 1-3 or 1-2, or 1) substituents selected from the group consisting of D, optionally deuterated $C_{1-4}$ alkyl (e.g., optionally deuterated $C_{1-3}$ alkyl, such as methyl, $CD_3$, ethyl, propyl, isopropyl, butyl, sec-butyl or tert-butyl), hydroxy, amino, mercapto, halogen (e.g., fluorine, chlorine, bromine, or iodine), cyano, oxo, $C_{1-4}$ alkoxy (e.g., $C_{1-3}$ alkoxy, such as methoxy, ethoxy, or propoxy), $C_{3-8}$ cycloalkyl (e.g., $C_{3-7}$ cycloalkyl, $C_{3-6}$ cycloalkyl or $C_{3-5}$ cycloalkyl), $C_{1-4}$ alkyl-NH- (e.g., $C_{1-3}$ alkyl-NH-, such as $CH_3NH$-, $CH_3CH_2NH$- or $CH_3CH_2CH_2NH$-), halogenated $C_{1-4}$ alkyl (e.g., halogenated $C_{1-3}$ alkyl, such as $F_3C$-, $FCH_2$-, $F_2CH$-, $ClCH_2$-, $Cl_2CH$-, $CF_3CF_2$-, $CF_3CHF$-, $CHF_2CF_2$-, $CHF_2CHF$-, $CF_3CH_2$- or $CH_2ClCH_2$-), $NH_2$-$C_{1-4}$ alkylene (e.g., $NH_2$-$C_{1-3}$ alkylene-, such as $NH_2CH_2$-, $NH_2CH_2CH_2$- or $NH_2CH_2CH_2CH_2$-), $C_{1-4}$ alkyl-NHC(O)- (e.g., $C_{1-3}$ alkyl-NHC(O)-, such as $CH_3$-NHC(O)-, $CH_3CH_2$-NHC(O)-, or $CH_3CH_2CH_2$-NHC(O)-), $C_{1-4}$ alkyl-C(O)NH-(e.g., $C_{1-3}$ alkyl-C(O)NH-, such as $CH_3$-C(O)NH-, $CH_3CH_2$-C(O)NH-, or $CH_3CH_2CH_2$-C(O)NH-) and any combination thereof.

In some embodiments of the present disclosure, $X_2$ represents C(O), -$CH_2$-, -$(CH_2)_2$-, -$(CH_2)_3$-, 1,1-cyclopropylene, NH, -$CH_2$-NH-, -$(CH_2)_2$-NH-, -NH-$(CH_2)_2$-, or -NH-$CH_2$-.

In some embodiments of the present disclosure, $R_{a1}$ represents the following structure:

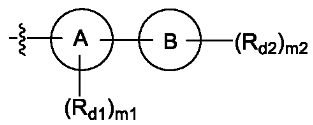

Formula (I-A)

wherein ring A represents heterocyclylene (e.g., 4- to 20-membered heterocyclylene, or 4- to 15-membered heterocyclylene), cycloalkylene (e.g., $C_{3-20}$ cycloalkylene or $C_{3-15}$ cycloalkylene), arylene (e.g., $C_{6-20}$ arylene, or $C_{6-10}$ arylene), or heteroarylene (e.g., 5- to 20-membered heteroarylene, or 5- to 15-membered heteroarylene), $(R_{d1})_{m1}$ indicates that ring A is optionally substituted with m1 $R_{d1}$, where m1 represents an integer of 0 to 10 (e.g., 1-10, 1-9, 1-8, 1-7, 1-6, 1-5, 1-4, 1-3 or 1-2, or 1), and each $R_{d1}$ independently represents deuterium, hydroxy, amino, mercapto, halogen (e.g., fluorine, chlorine, bromine, or iodine), cyano, oxo, optionally deuterated $C_{1-6}$ alkyl (e.g., optionally deuterated $C_{1-4}$ alkyl, or optionally deuterated $C_{1-3}$ alkyl, such as methyl, $CD_3$, ethyl, propyl, isopropyl, butyl, sec-butyl or tert-butyl), optionally deuterated $C_{3-6}$ cycloalkyl (e.g., cyclopropyl, cyclobutyl, cyclopentyl or cyclohexyl), optionally deuterated $C_{1-6}$ alkoxy (e.g., optionally deuterated $C_{1-5}$ alkoxy, or optionally deuterated $C_{1-3}$ alkoxy, such as methoxy, ethoxy, or propoxy), halogenated $C_{1-6}$ alkyl (e.g., halogenated $C_{1-4}$ alkyl or halogenated $C_{1-3}$ alkyl, such as

$F_3C$-, $FCH_2$-, $F_2CH$-, $ClCH_2$-, $Cl_2CH$-, $CF_3CF_2$-, $CF_3CHF$-, $CHF_2CF_2$-, $CHF_2CHF$-, $CF_3CH_2$- or $CH_2ClCH_2$-), $C_{1-4}$ alkyl-NH-(e.g., $C_{1-3}$ alkyl-NH-, such as $CH_3NH$-, $CH_3CH_2NH$- or $CH_3CH_2CH_2NH$-), $C_{1-4}$ alkyl-NHC(O)-(e.g., $C_{1-3}$ alkyl-NHC(O)-, such as $CH_3$-NHC(O)-, $CH_3CH_2$-NHC(O)-, or $CH_3CH_2CH_2$-NHC(O)-), $C_{1-4}$ alkyl-C(O)NH- (e.g., $C_{1-3}$ alkyl-C(O)NH-, such as $CH_3$-C(O)NH-, $CH_3CH_2$-C(O)NH-, or $CH_3CH_2CH_2$-C(O)NH-), or $NH_2$-$C_{1-6}$ alkylene- (e.g., $NH_2$-$C_{1-4}$ alkylene- or $NH_2$-$C_{1-3}$ alkylene-, such as $NH_2CH_2$-, $NH_2CH_2CH_2$- or $NH_2CH_2CH_2CH_2$-); and

ring B represents aryl (e.g., $C_{6-20}$ aryl or $C_{6-10}$ aryl), cycloalkyl (e.g., $C_{3-20}$ cycloalkyl or $C_{3-15}$ cycloalkyl), heterocyclyl (e.g., 4- to 20-membered heterocyclyl or 4- to 15-membered heterocyclyl), or heteroaryl (e.g., 5- to 20-membered heteroaryl or 5- to 15-membered heteroaryl),

$(R_{d2})_{m2}$ indicates that ring B is optionally substituted with m2 $R_{d2}$, where m2 represents an integer of 0 to 10 (e.g., 1-10, 1-9, 1-8, 1-7, 1-6, 1-5, 1-4, 1-3 or 1-2, or 1), and each $R_{d2}$ independently represents deuterium, hydroxy, amino, mercapto, halogen (e.g., fluorine, chlorine, bromine, or iodine), cyano, oxo, optionally deuterated $C_{1-6}$ alkyl (e.g., optionally deuterated $C_{1-4}$ alkyl, or optionally deuterated $C_{1-3}$ alkyl, such as methyl, $CD_3$, ethyl, propyl, isopropyl, butyl, sec-butyl or tert-butyl), optionally deuterated $C_{3-6}$ cycloalkyl (e.g., cyclopropyl, cyclobutyl, cyclopentyl or cyclohexyl), optionally deuterated $C_{1-6}$ alkoxy (e.g., optionally deuterated $C_{1-5}$ alkoxy, or optionally deuterated $C_{1-3}$ alkoxy, such as methoxy, ethoxy, or propoxy), halogenated $C_{1-6}$ alkyl (e.g., halogenated $C_{1-4}$ alkyl or halogenated $C_{1-3}$ alkyl, such as $F_3C$-, $FCH_2$-, $F_2CH$-, $ClCH_2$-, $Cl_2CH$-, $CF_3CF_2$-, $CF_3CHF$-, $CHF_2CF_2$-, $CHF_2CHF$-, $CF_3CH_2$- or $CH_2ClCH_2$-), $C_{1-4}$ alkyl-NH-(e.g., $C_{1-3}$ alkyl-NH-, such as $CH_3NH$-, $CH_3CH_2NH$- or $CH_3CH_2CH_2NH$-), $C_{1-4}$ alkyl-NHC(O)-(e.g., $C_{1-3}$ alkyl-NHC(O)-, such as $CH_3$-NHC(O)-, $CH_3CH_2$-NHC(O)-, or $CH_3CH_2CH_2$-NHC(O)-), $C_{1-4}$ alkyl-C(O)NH- (e.g., $C_{1-3}$ alkyl-C(O)NH-, such as $CH_3$-C(O)NH-, $CH_3CH_2$-C(O)NH-, or $CH_3CH_2CH_2$-C(O)NH-), or $NH_2$-$C_{1-6}$ alkylene- (e.g., $NH_2$-$C_{1-4}$ alkylene- or $NH_2$-$C_{1-3}$ alkylene-, such as $NH_2CH_2$-, $NH_2CH_2CH_2$- or $NH_2CH_2CH_2CH_2$-).

In some embodiments of the present disclosure, $R_{a1}$ represents the following structure:

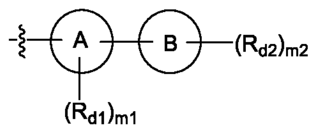

Formula (I-A)

wherein ring A represents 4- to 20-membered heterocyclylene, $C_{3-20}$ cycloalkylene, $C_{6-20}$ arylene, or 5-to 20-membered heteroarylene, and $(R_{d1})_{m1}$ indicates that ring A is optionally substituted with m1 $R_{d1}$, where m1 represents an integer of 0 to 10 (e.g., 1-10, 1-9, 1-8, 1-7, 1-6, 1-5, 1-4, 1-3 or 1-2, or 1), and each $R_{d1}$ independently represents deuterium, hydroxy, amino, mercapto, halogen, cyano, oxo, optionally deuterated $C_{1-6}$ alkyl, optionally deuterated $C_{3-6}$ cycloalkyl, optionally deuterated $C_{1-6}$ alkoxy, halogenated $C_{1-6}$ alkyl, $C_{1-4}$ alkyl-NH-, $C_{1-4}$ alkyl-NHC(O)-, $C_{1-4}$ alkyl-C(O)NH-, or $NH_2$-$C_{1-6}$ alkylene-; and/or

ring B represents $C_{6-20}$ aryl, $C_{3-20}$ cycloalkyl, 4- to 20-membered heterocyclyl, or 5- to 20-membered heteroaryl, and $(R_{d2})_{m2}$ indicates that ring B is optionally substituted with m2 $R_{d2}$, where m2 represents an integer of 0 to 10 (e.g., 1-10, 1-9, 1-8, 1-7, 1-6, 1-5, 1-4, 1-3 or 1-2, or 1), and each $R_{d2}$ independently represents deuterium, hydroxy, amino, mercapto, halogen, cyano, oxo, optionally deuterated $C_{1-6}$ alkyl, optionally deuterated $C_{3-6}$ cycloalkyl, optionally deuterated $C_{1-6}$ alkoxy, halogenated $C_{1-6}$ alkyl, $C_{1-4}$ alkyl-NH-, $C_{1-4}$ alkyl-NHC(O)-, $C_{1-4}$ alkyl-C(O)NH-, or $NH_2$-$C_{1-6}$ alkylene-.

In some embodiments of the present disclosure, ring A represents the following divalent groups: cyclopropylene, cyclobutylene, cyclopentylene, cyclopentenylene, cyclohexylene, cyclohexenylene, cycloheptylene, cyclooctylene, decalinylene, octahydropentalenylene, octahydro-1H-indenylene, spiro[3.3]heptylene, spiro[2.5]octylene, spiro[3.5]nonylene, spiro[4.4]nonylene, spiro[4.5]decylene, spiro[5.5]undecylene, p-menthanylene, m-menthanylene, quinuclidinylene, adamantanylene, noradamantanylene, norbomylene, bicyclo[2.2.1]heptylene, 2-oxobicyclo[2.2.1]heptylene, or bicyclo[2.2.1]heptentylene, with each group being optionally substituted with one or more (e.g., 1-10, 1-9, 1-8, 1-7, 1-6, 1-5, 1-4, 1-3 or 1-2, or 1) substituents selected from the group consisting of D, hydroxy, amino, mercapto, halogen, cyano, oxo, optionally deuterated $C_{1-6}$ alkyl, optionally deuterated $C_{3-6}$ cycloalkyl, optionally deuterated $C_{1-6}$ alkoxy, halogenated $C_{1-6}$ alkyl, $C_{1-4}$ alkyl-NH-, $C_{1-4}$ alkyl-NHC(O)-, $C_{1-4}$ alkyl-C(O)NH-, $NH_2$-$C_{1-6}$ alkylene- and any combination thereof;

arylene or naphthylene, with each group being optionally substituted with one or more (e.g., 1-6, 1-5, 1-4, 1-3 or 1-2, or 1) substituents selected from the group consisting of D, hydroxy, amino, mercapto, halogen, cyano, optionally deuterated $C_{1-6}$ alkyl, optionally deuterated $C_{3-6}$ cycloalkyl, optionally deuterated $C_{1-6}$ alkoxy, halogenated $C_{1-6}$ alkyl, $C_{1-4}$ alkyl-NH-, $C_{1-4}$ alkyl-NHC(O)-, $C_{1-4}$ alkyl-C(O)NH-, $NH_2$-$C_{1-6}$ alkylene- and any combination thereof;

azetidinylene, pyrrolidinylene, imidazolidylene, pyrazolidylene, oxazolidinylene, thiazolidinylene, piperidinylene,

piperazinylene, morpholinylene, thiomorpholinylene, azepanylene, azacyclooctylene, diazacycloheptanylene, diazacyclooctylene, 3-azabicyclo[3.1.0]hexylene, 3-azabicyclo[3.1.1]heptanylene, 2-azabicyclo[2.2.1]heptanylene, 6-azabicyclo[3.1.1]heptanylene, 2-azabicyclo[2.2.2]octylene, 2,5-diazabicyclo[2.2.1]heptanylene, 3,6-diazabicyclo[3.1.1]heptanylene, 3-azabicyclo[3.2.1]octylene, 3,8-diazabicyclo[3.2.1]octylene, 2,5-diazabicyclo[2.2.2]octylene, 2,6-diazaspiro[3.3]heptanylene, 2,7-diazaspiro[3.5]nonylene, 3-azaspiro[5.5]undecylene, 7-azaspiro[3.5]nonylene, or octahydropyrrolo[3,4-c]pyrrolylene, with each group being optionally substituted with one or more (e.g., 1-10, 1-9, 1-8, 1-7, 1-6, 1-5, 1-4, 1-3 or 1-2, or 1) substituents selected from the group consisting of D, hydroxy, amino, mercapto, halogen, cyano, oxo, optionally deuterated $C_{1-6}$ alkyl, optionally deuterated $C_{3-6}$ cycloalkyl, optionally deuterated $C_{1-6}$ alkoxy, halogenated $C_{1-6}$ alkyl, $C_{1-4}$ alkyl-NH-, $C_{1-4}$ alkyl-NHC(O)-, $C_{1-4}$ alkyl-C(O)NH-, $NH_2$-$C_{1-6}$ alkylene- and any combination thereof; or

furanylene, oxazolylene, isoxazolylene, oxadiazolylene, thienylene, thiazolylene, isothiazolylene, thiadiazolylene, pyrrolylene, imidazolylene, pyrazolylene, triazolylene, pyridylene, pyrimidinylene, pyridazinylene, pyrazinylene, indolylene, isoindolylene, benzofuranylene, isobenzofuranylene, benzothienylene, indazolylene, benzimidazolylene, benzoxazolylene, benzisoxazolylene, benzothiazolylene, benzisothiazolylene, benzotriazolylene, benzo[2,1,3]oxadiazolylene, benzo[2,1,3]thiadiazolylene, benzo[1,2,3]thiadiazolylene, quinolinylene, isoquinolinylene, naphthyridinylene, cinnolinylene, quinazolinylene, quinoxalinylene, phthalazinylene, pyrazolo[1,5-a]pyridylene, pyrazolo[1,5-a]pyrimidinylene, imidazo[1,2-a]pyridylene, 1H-pyrrolo[3,2-b]pyridylene, 1H-pyrrolo[2,3-b]pyridylene, 4H-fluoro[3,2-b]pyrrolylene, pyrrolo[2,1-b]thiazolylene, or imidazo[2,1-b]thiazolylene, with each group being optionally substituted with one or more (e.g., 1-6, 1-5, 1-4, 1-3 or 1-2, or 1) substituents selected from the group consisting of D, hydroxy, amino, mercapto, halogen, cyano, optionally deuterated $C_{1-6}$ alkyl, optionally deuterated $C_{3-6}$ cycloalkyl, optionally deuterated $C_{1-6}$ alkoxy, halogenated $C_{1-6}$ alkyl, $C_{1-4}$ alkyl-NH-, $C_{1-4}$ alkyl-NHC(O)-, $C_{1-4}$ alkyl-C(O)NH-, $NH_2$-$C_{1-6}$ alkylene- and any combination thereof. In embodiments of the present disclosure, the number of substituents is not limited in principle, or is automatically limited by the size of the building units.

In some embodiments of the present disclosure, ring B represents the following groups: cyclopropyl, cyclobutyl, cyclopentyl, cyclopentenyl, cyclohexyl, cyclohexenyl, cycloheptyl, cyclooctyl, decalinyl, octahydropentalenyl, octahydro-1H-indenyl, spiro[3.3]heptanyl, spiro[2.5]octyl, spiro[3.5]nonyl, spiro[4.4]nonyl, spiro[4.5]decyl, spiro[5.5]undecyl, *p*-menthanyl, m-menthanyl, quinuclidinyl, adamantanyl, noradamantanyl, bornyl, bicyclo[2.2.1]heptanyl, 2-oxobicyclo[2.2.1]heptyl, or bicyclo[2.2.1]heptenyl, with each group being optionally substituted with one or more (e.g., 1-10, 1-9, 1-8, 1-7, 1-6, 1-5, 1-4, 1-3 or 1-2, or 1) substituents selected from the group consisting of D, hydroxy, amino, mercapto, halogen, cyano, oxo, optionally deuterated $C_{1-6}$ alkyl, optionally deuterated $C_{3-6}$ cycloalkyl, optionally deuterated $C_{1-6}$ alkoxy, halogenated $C_{1-6}$ alkyl, $C_{1-4}$ alkyl-NH-, $C_{1-4}$ alkyl-NHC(O)-, $C_{1-4}$ alkyl-C(O)NH-, $NH_2$-$C_{1-6}$ alkylene- and any combination thereof;

phenyl or naphthyl, with each group being optionally substituted with one or more (e.g., 1-6, 1-5, 1-4, 1-3 or 1-2, or 1) substituents selected from the group consisting of D, hydroxy, amino, mercapto, halogen, cyano, optionally deuterated $C_{1-6}$ alkyl, optionally deuterated $C_{3-6}$ cycloalkyl, optionally deuterated $C_{1-6}$ alkoxy, halogenated $C_{1-6}$ alkyl, $C_{1-4}$ alkyl-NH-, $C_{1-4}$ alkyl-NHC(O)-, $C_{1-4}$ alkyl-C(O)NH-, $NH_2$-$C_{1-6}$ alkylene- and any combination thereof;

azetidinyl, pyrrolidinyl, imidazolidinyl, pyrazolidyl, oxazolidinyl, thiazolidinyl, piperidinyl, piperazinyl, morpholinyl, thiomorpholinyl, azacycloheptyl, azacyclooctyl, diazacycloheptanyl, diazacyclooctyl, 3-azabicyclo[3.1.0]hexyl, 3-azabicyclo[3.1.1]heptanyl, 2-azabicyclo[2.2.1]heptanyl, 6-azabicyclo[3.1.1]heptanyl, 2-azabicyclo[2.2.2]octyl, 2,5-diazabicyclo[2.2.1]heptanyl, 3,6-diazabicyclo[3.1.1]heptanyl, 3-azabicyclo[3.2.1]octyl, 3,8-diazabicyclo[3.2.1]octyl, 2,5-diazabicyclo[2.2.2]octyl, 2,6-diazaspiro[3.3]heptanyl, 2,7-diazaspiro[3.5]nonyl, 3-azaspiro[5.5]undecyl, 7-azaspiro[3.5]nonyl, or octahydropyrrolo[3,4-c]pyrrolyl, with each group being optionally substituted with one or more (e.g., 1-10, 1-9, 1-8, 1-7, 1-6, 1-5, 1-4, 1-3 or 1-2, or 1) substituents selected from the group consisting of D, hydroxy, amino, mercapto, halogen, cyano, oxo, optionally deuterated $C_{1-6}$ alkyl, optionally deuterated $C_{3-6}$ cycloalkyl, optionally deuterated $C_{1-6}$ alkoxy, halogenated $C_{1-6}$ alkyl, $C_{1-4}$ alkyl-NH-, $C_{1-4}$ alkyl-NHC(O)-, $C_{1-4}$ alkyl-C(O)NH-, $NH_2$-$C_{1-6}$ alkylene- and any combination thereof; or

furanyl, oxazolyl, isoxazolyl, oxadiazolyl, thienyl, thiazolyl, isothiazolyl, thiadiazolyl, pyrrolyl, imidazolyl, pyrazolyl, triazolyl, pyridyl, pyrimidinyl, pyridazinyl, pyrazinyl, indolyl, isoindolyl, benzofuranyl, isobenzofuranyl, benzothienyl, indazolyl, benzimidazolyl, benzoxazolyl, benzisoxazolyl, benzothiazolyl, benzisothiazolyl, benzotriazolyl, benzo[2,1,3]oxadiazolyl, benzo[2,1,3]thiadiazolyl, benzo[1,2,3]thiadiazolyl, quinolinyl, isoquinolinyl, naphthyridinyl, cinnolinyl, quinazolinyl, quinoxalinyl, phthalazinyl, pyrazolo[1,5-a]pyridyl, pyrazolo[1,5-a]pyrimidinyl, imidazo[1,2-a]pyridyl, 1H-pyrrolo[3,2-b]pyridyl, 1H-pyrrolo[2,3-b]pyridyl, 4H-fluoro[3,2-b]pyrrolyl, pyrrolo[2,1-b]thiazolyl or imidazo[2,1-b]thiazolyl, with each group being optionally substituted with one or more (e.g., 1-6, 1-5, 1-4, 1-3 or 1-2, or 1) substituents selected from the group consisting of D, hydroxy, amino, mercapto, halogen, cyano, optionally deuterated $C_{1-6}$ alkyl, optionally deuterated $C_{3-6}$ cycloalkyl, optionally deuterated $C_{1-6}$ alkoxy, halogenated $C_{1-6}$ alkyl, $C_{1-4}$ alkyl-NH-, $C_{1-4}$ alkyl-NHC(O)-, $C_{1-4}$ alkyl-C(O)NH-, $NH_2$-$C_{1-6}$ alkylene- and any combination thereof. In

embodiments of the present disclosure, the number of substituents is not limited in principle, or is automatically limited by the size of the building units.

In some embodiments of the present disclosure, ring A represents the following divalent groups:

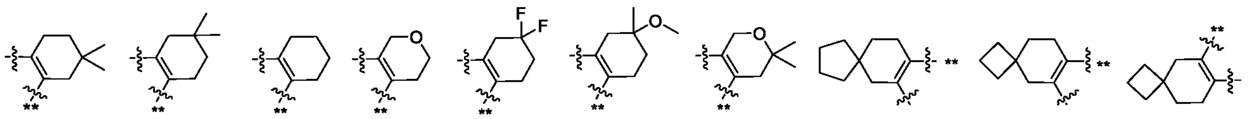

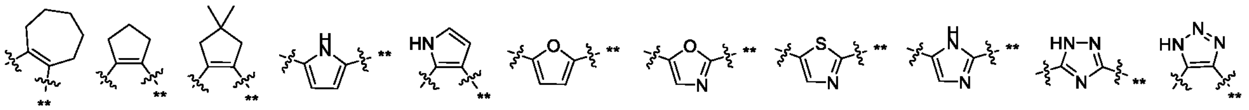

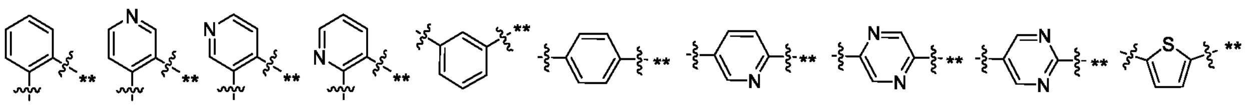

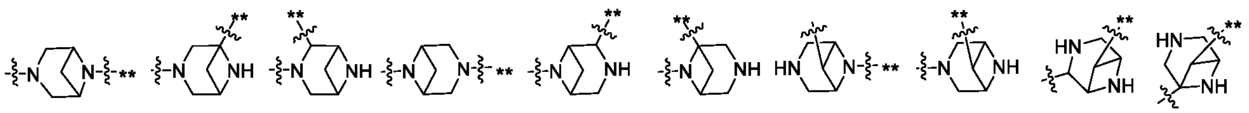

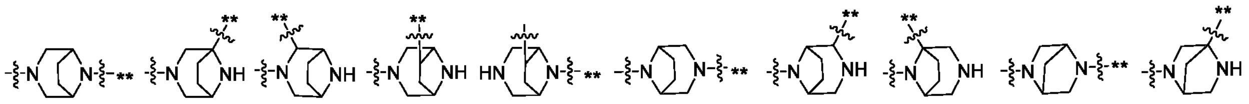

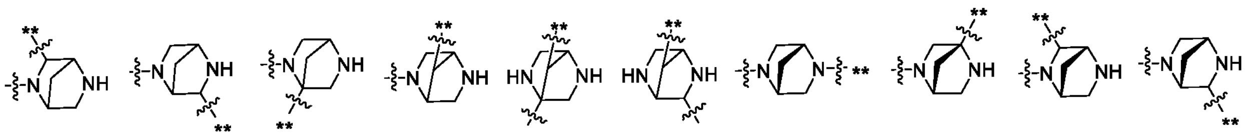

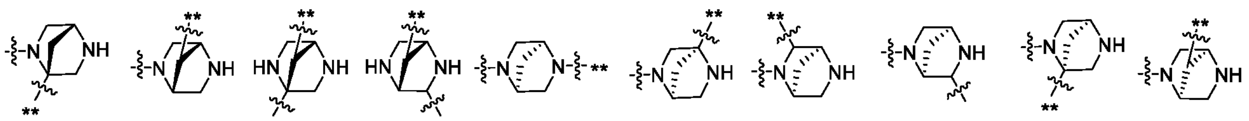

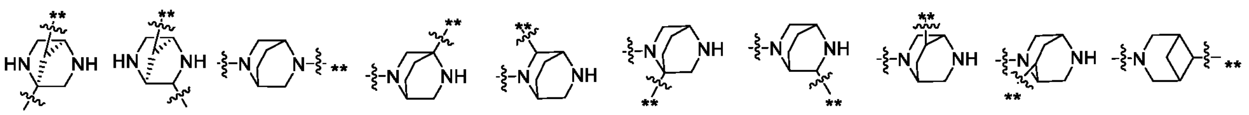

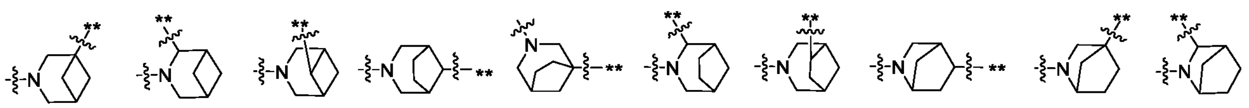

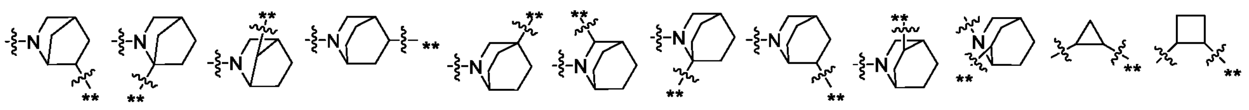

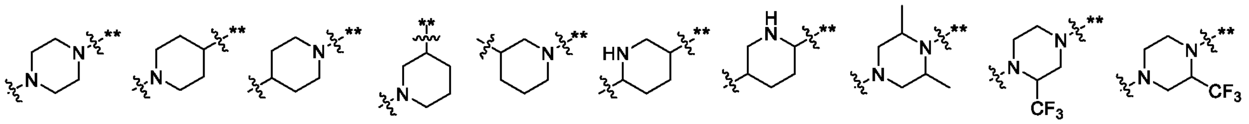

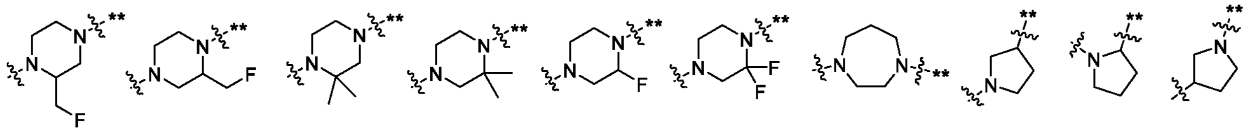

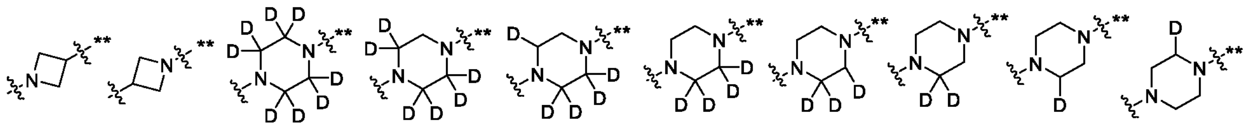

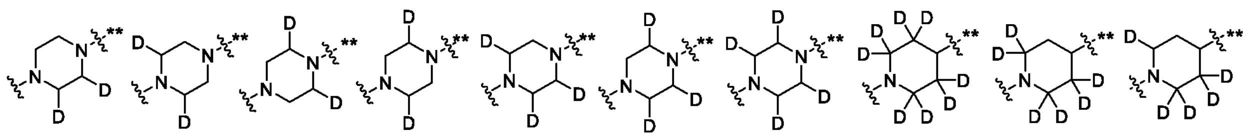

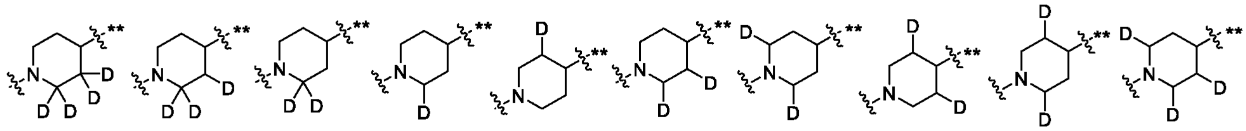

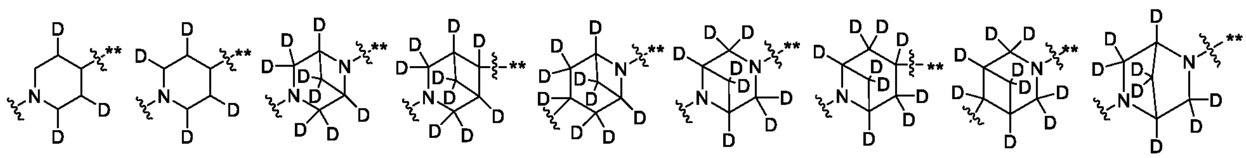

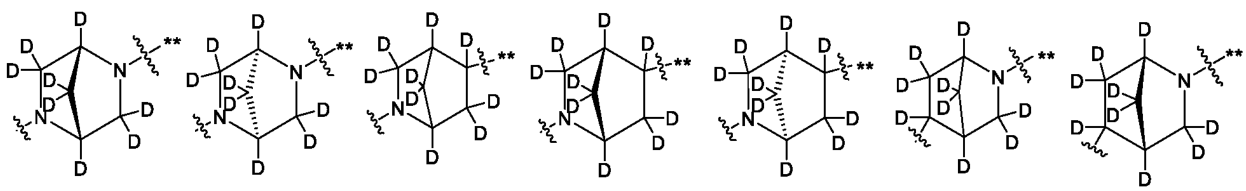

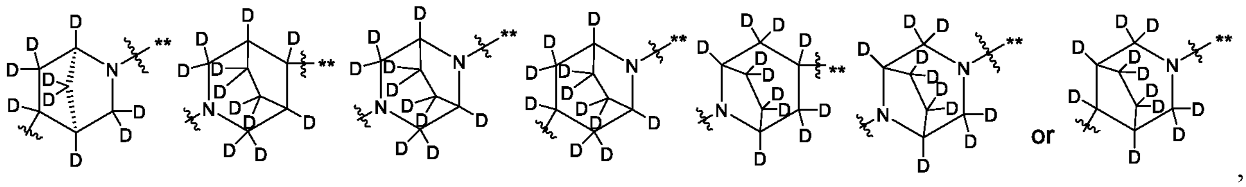

or

,

wherein symbol ** indicates the point of attachment to ring B.

In some embodiments of the present disclosure, ring B represents the following groups:

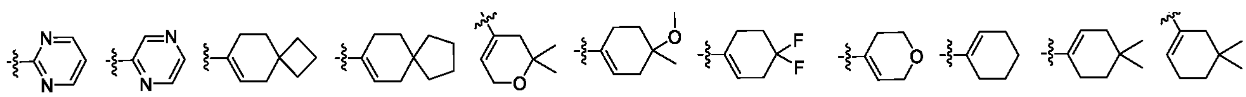

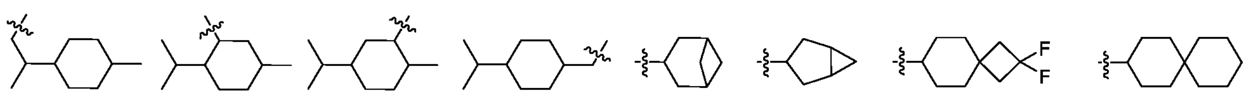

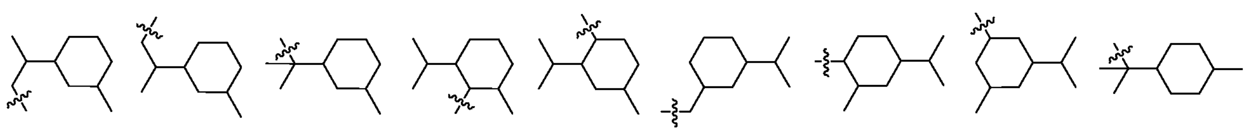

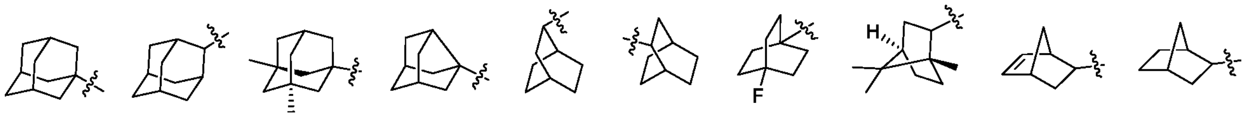

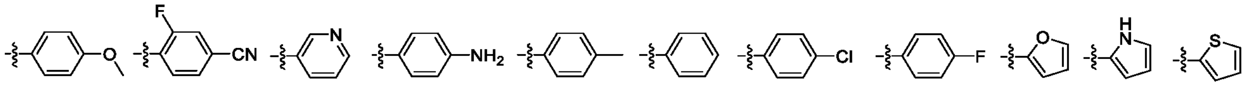

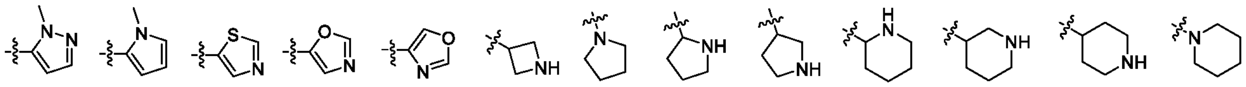

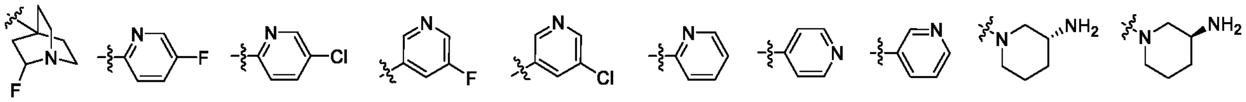

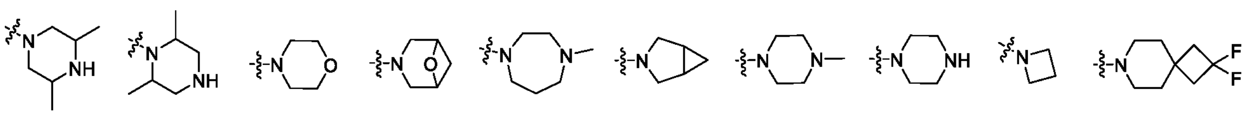

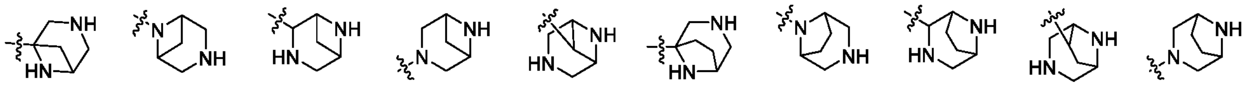

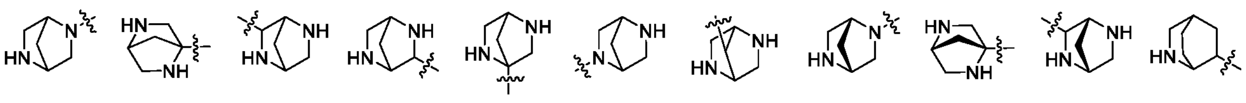

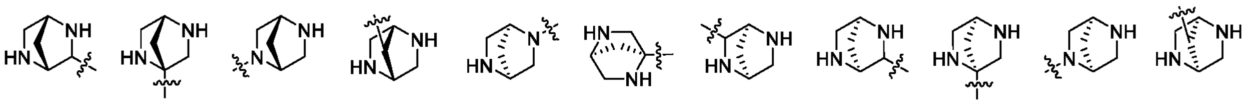

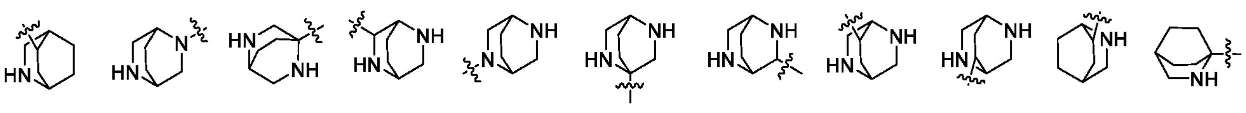

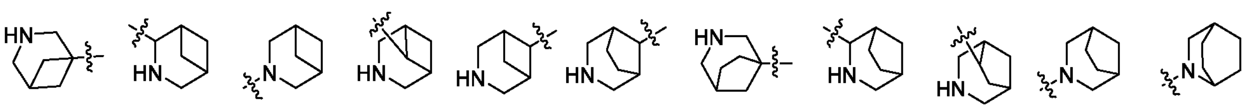

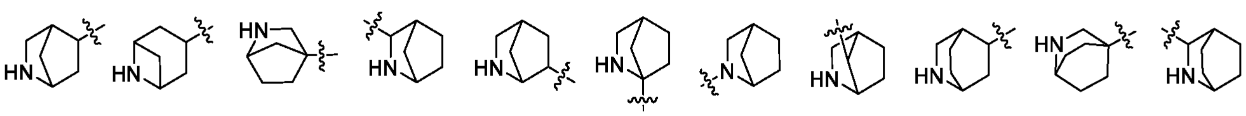

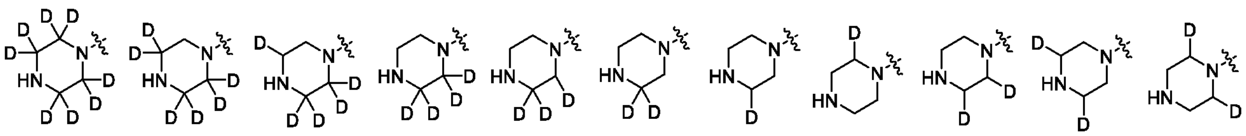

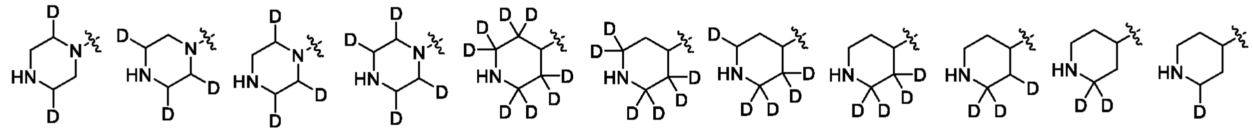

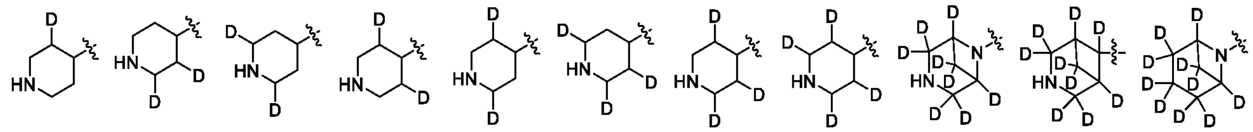

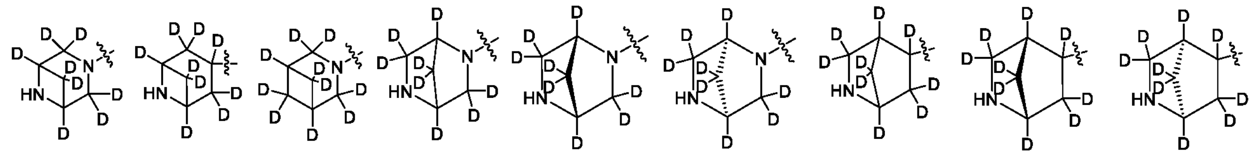

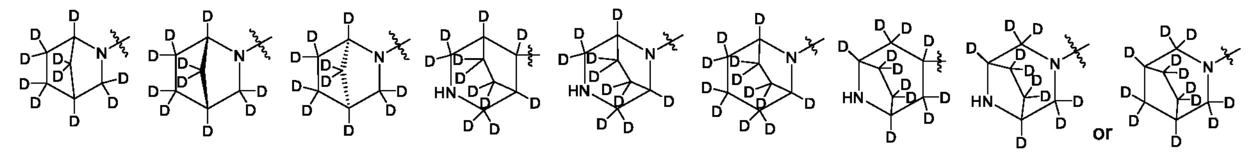

or

In some embodiments of the present disclosure, $R_{a1}$ represents the following structures:

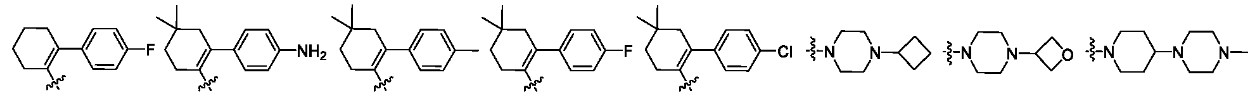

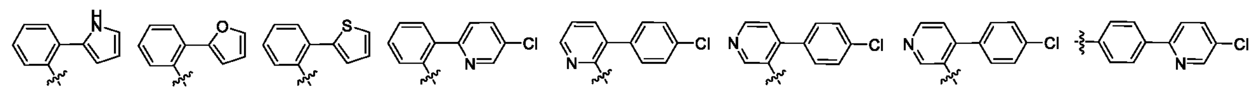

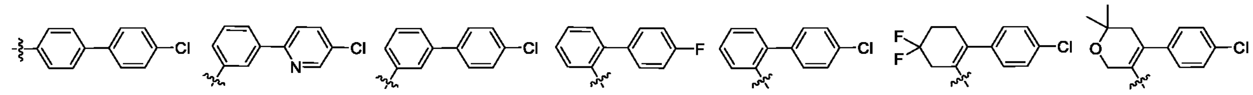

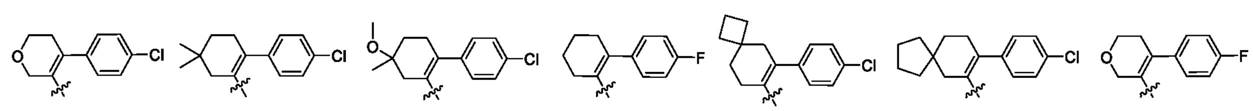

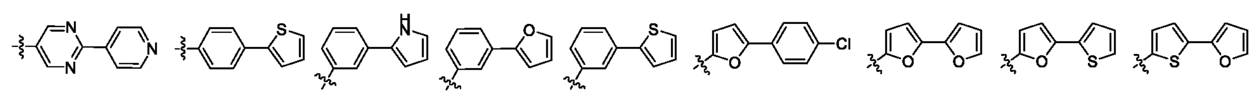

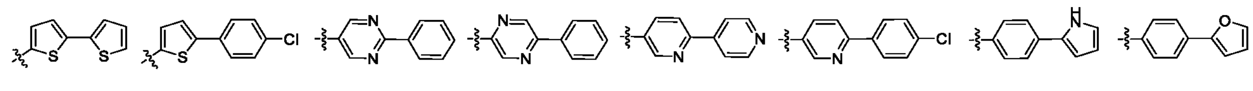

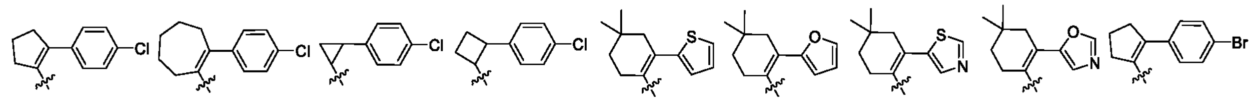

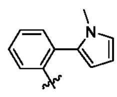 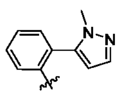 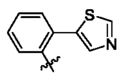 or 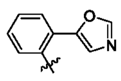 .

In some embodiments of the present disclosure, $-X_1-X_2-R_{a1}$ in the compounds of Formula (I) represents the following structures:

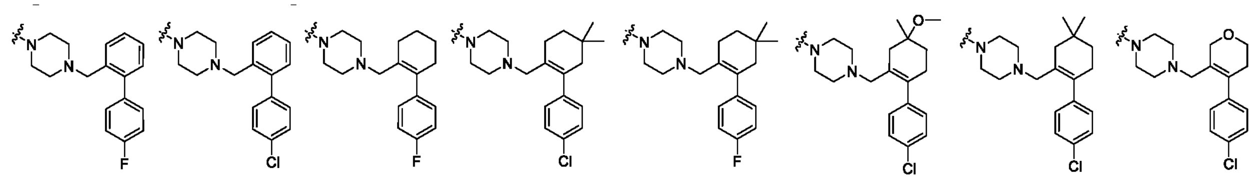

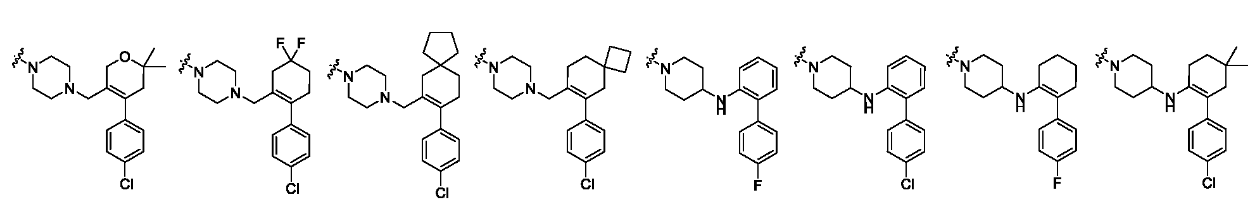

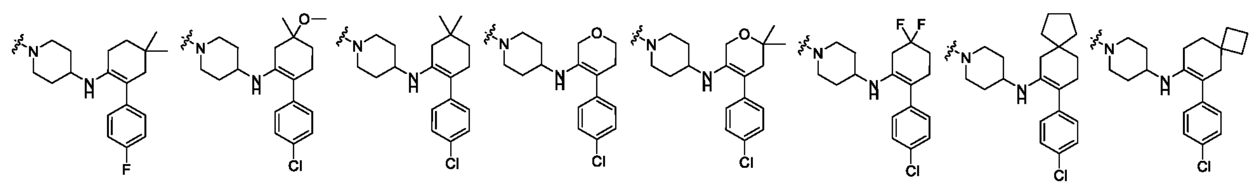

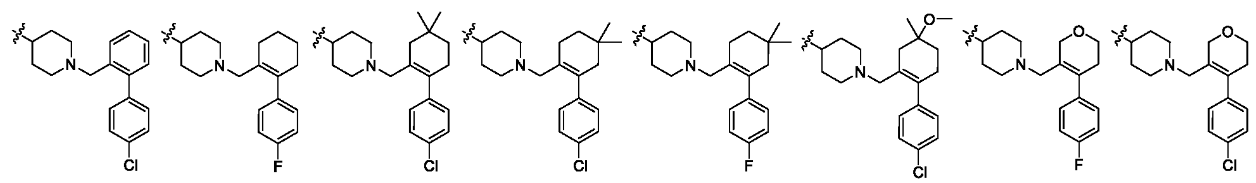

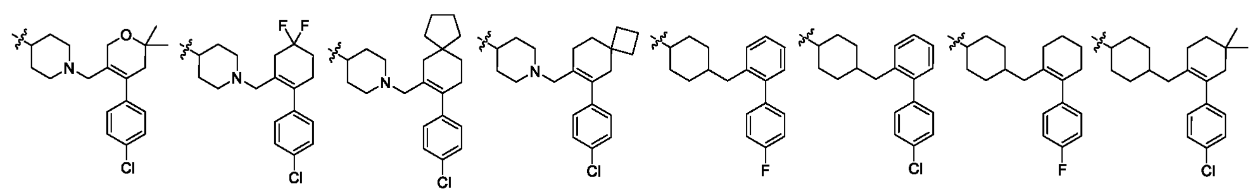

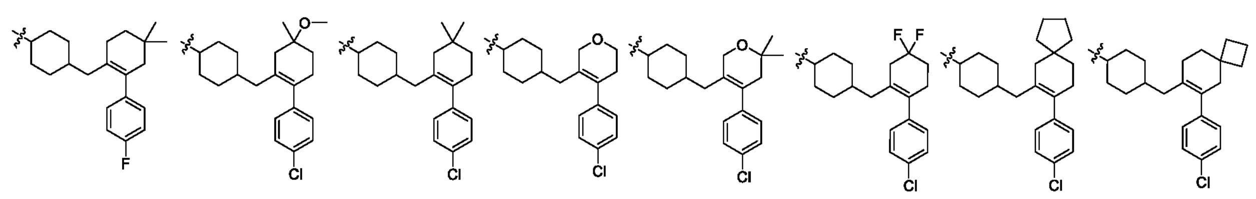

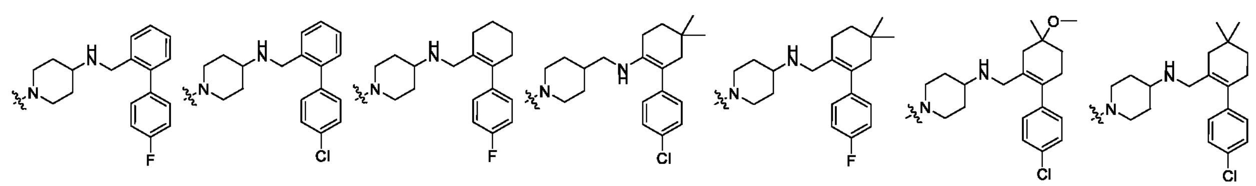

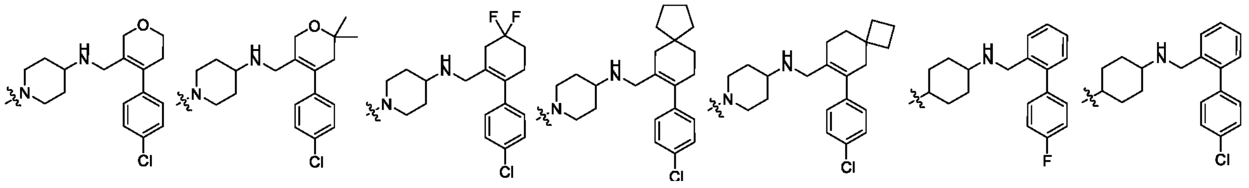

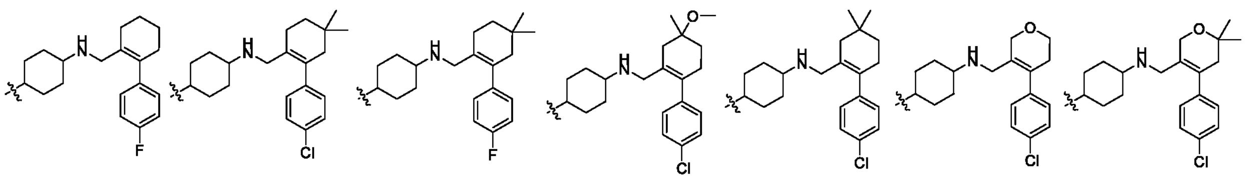

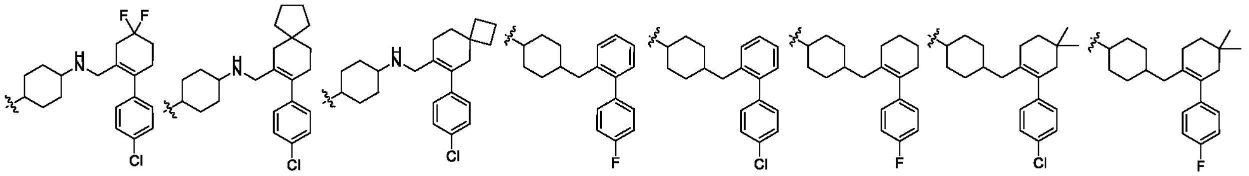

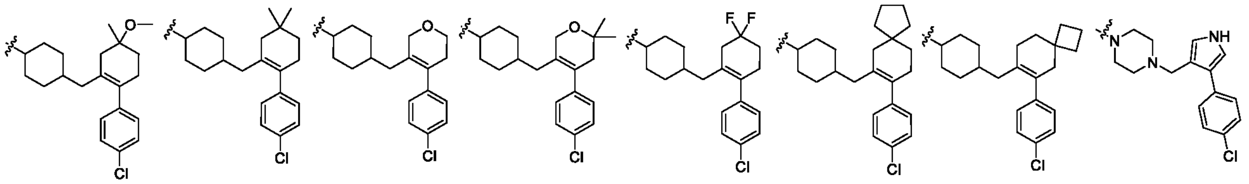

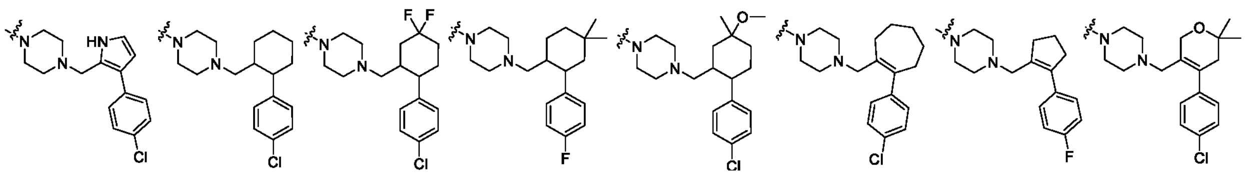

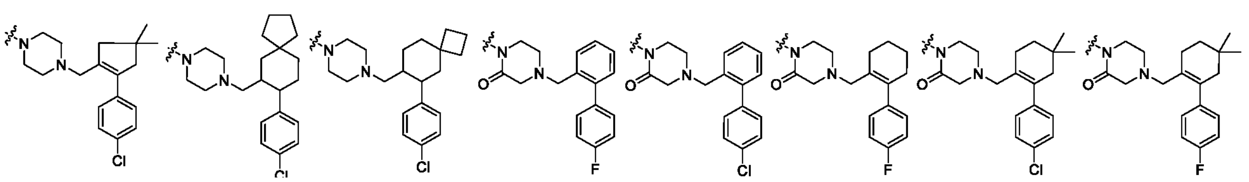

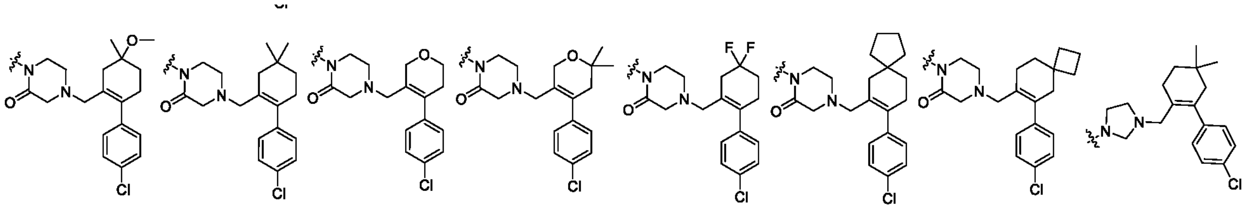

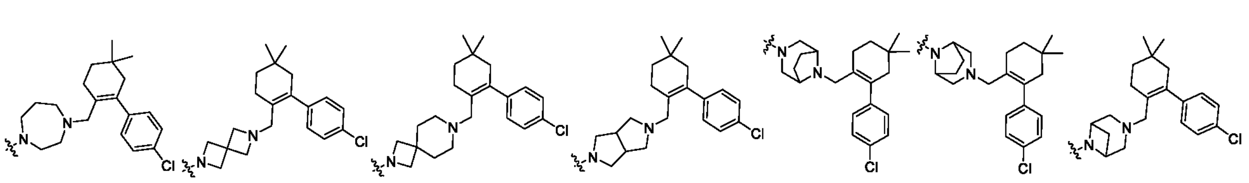

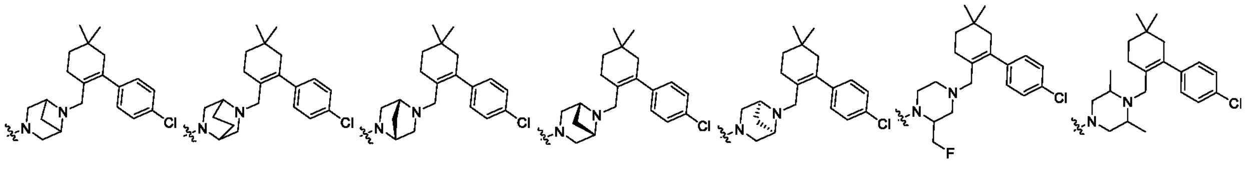

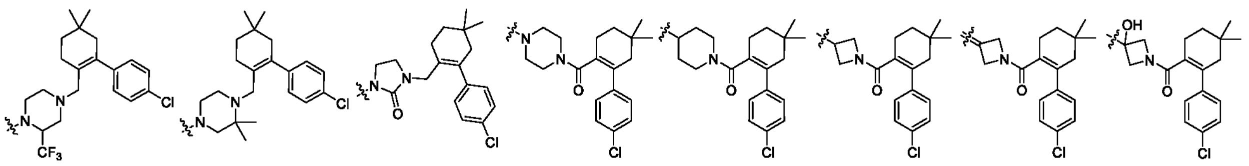

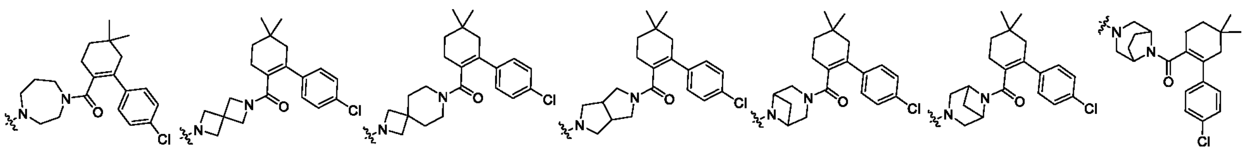

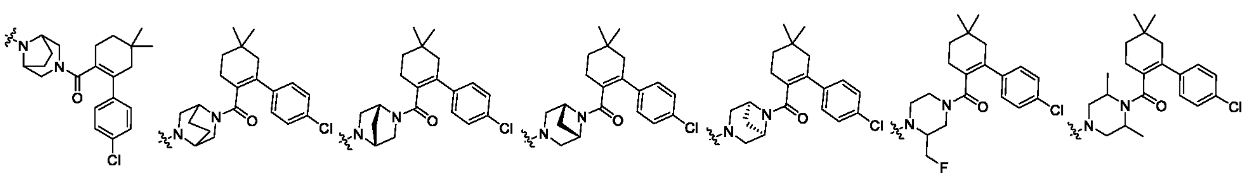

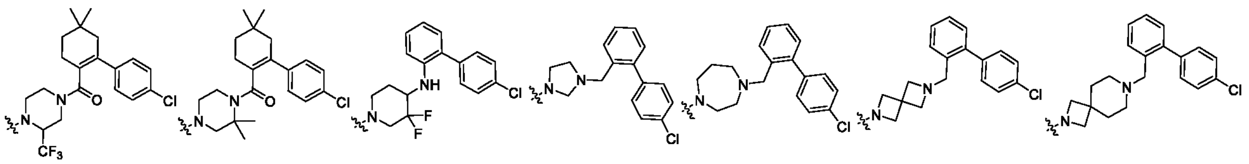

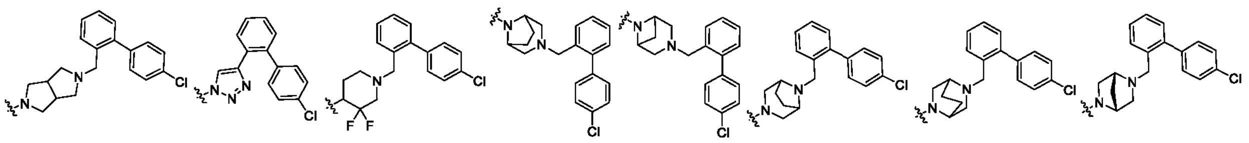

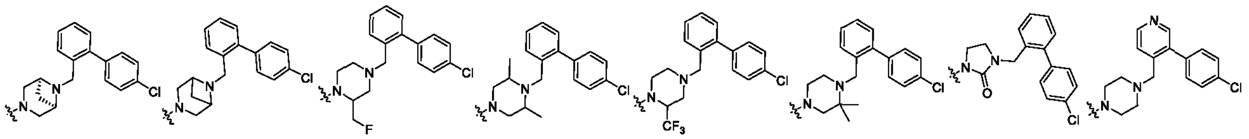

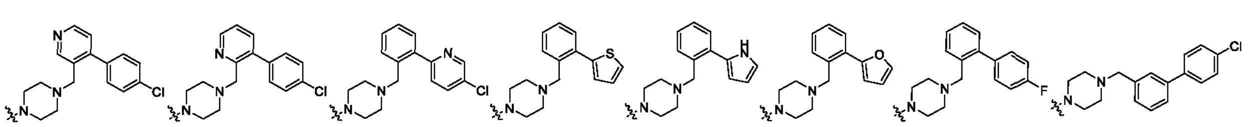

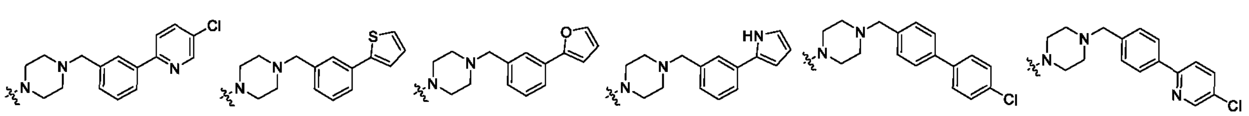

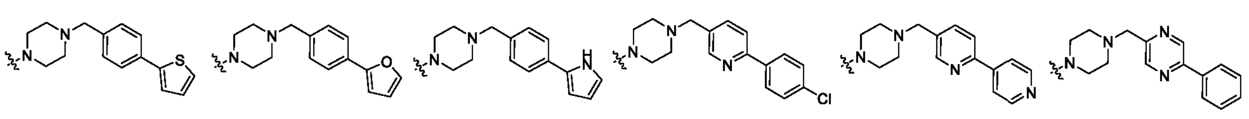

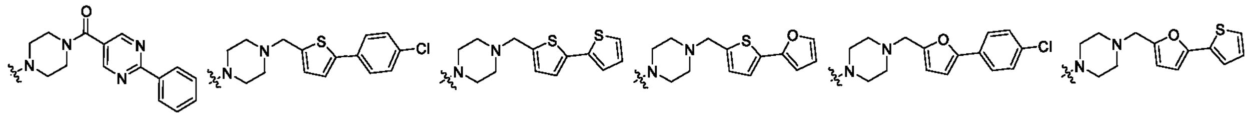

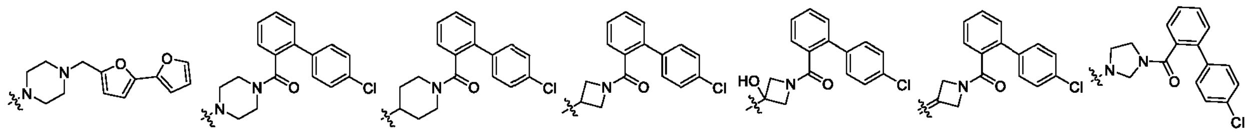

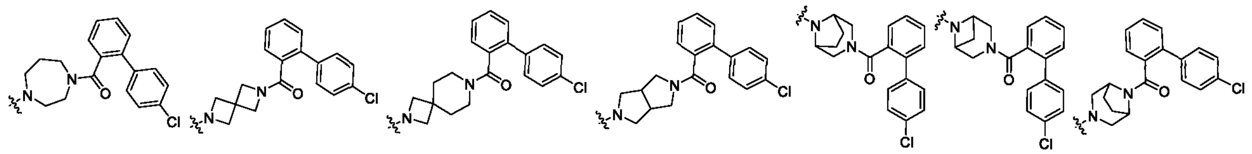

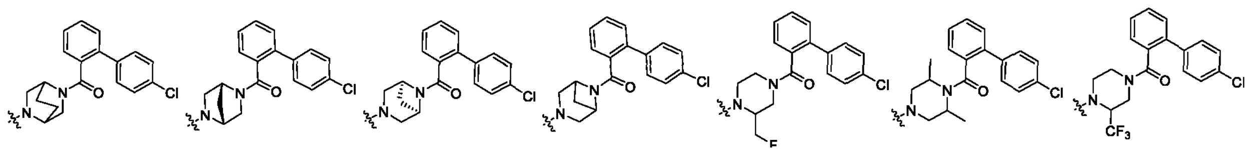

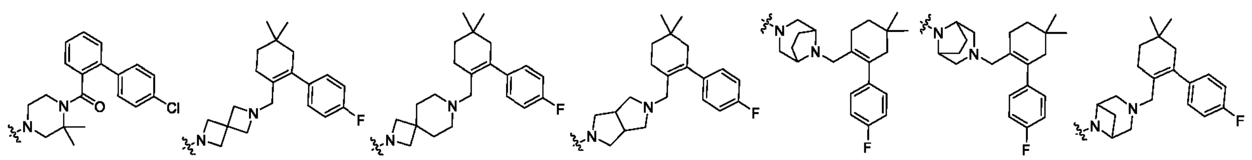

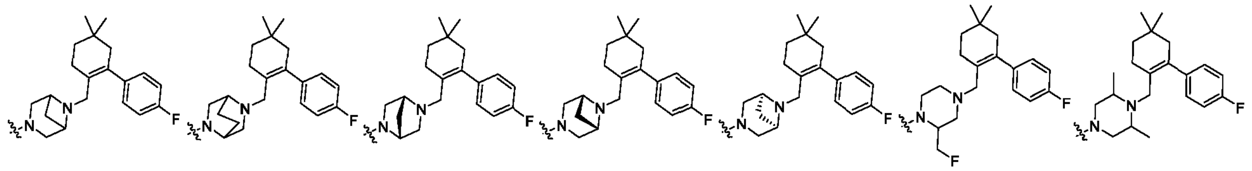

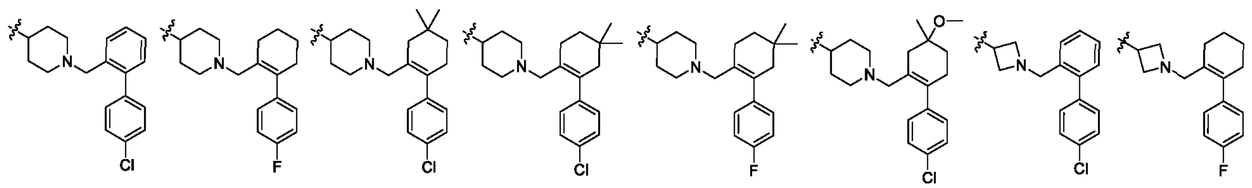

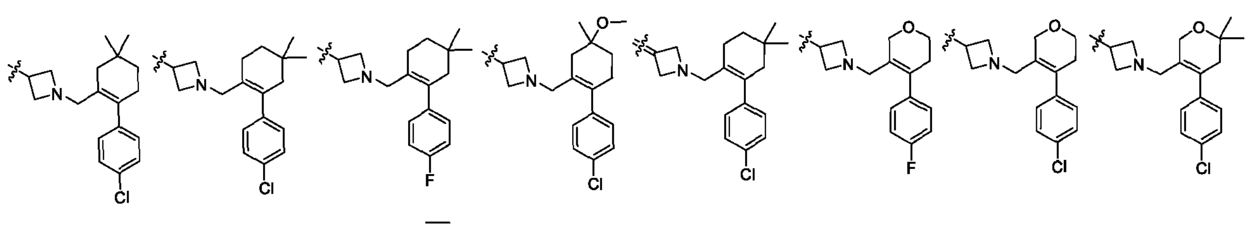

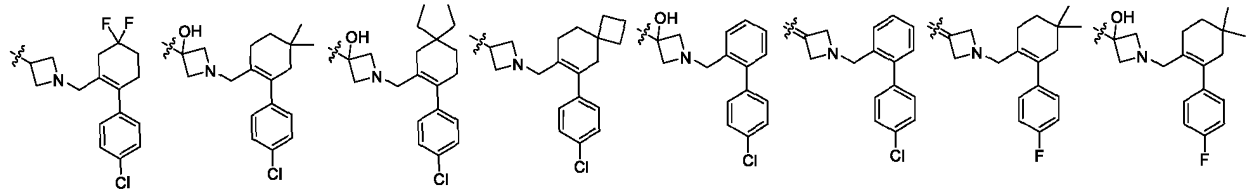

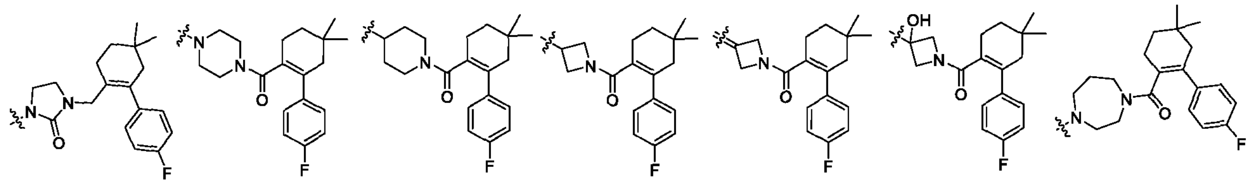

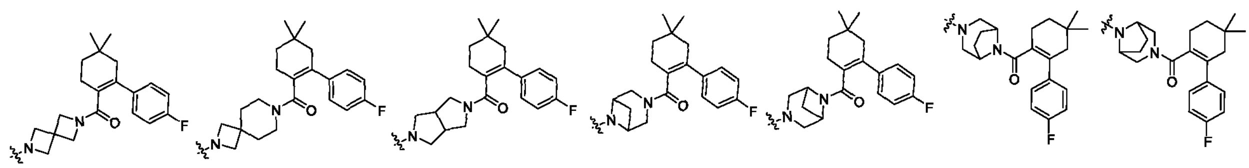

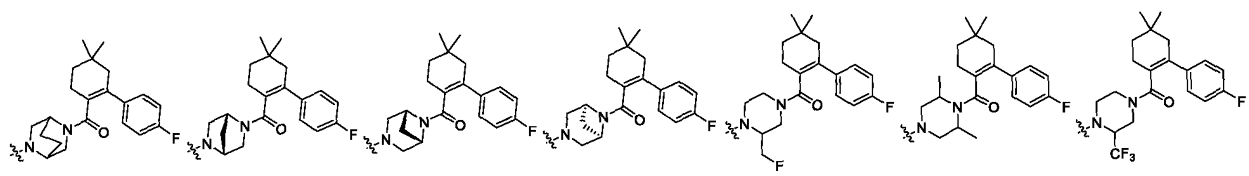

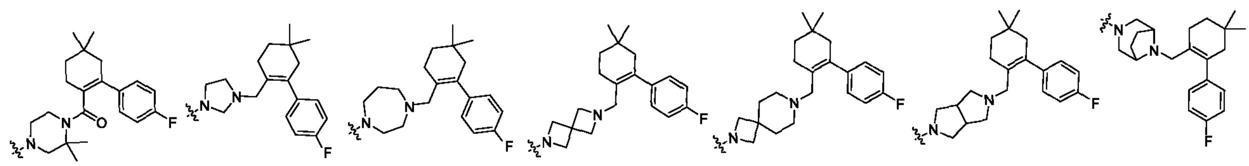

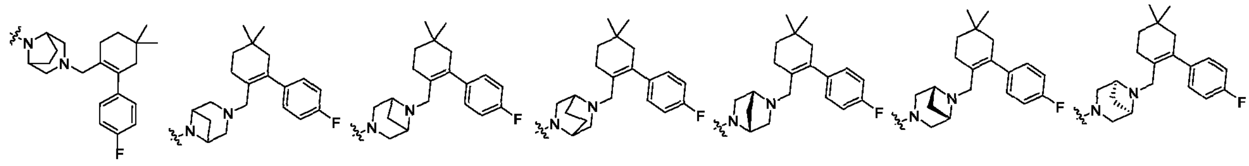

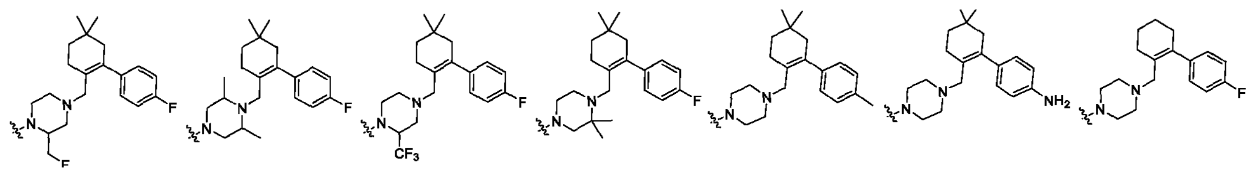

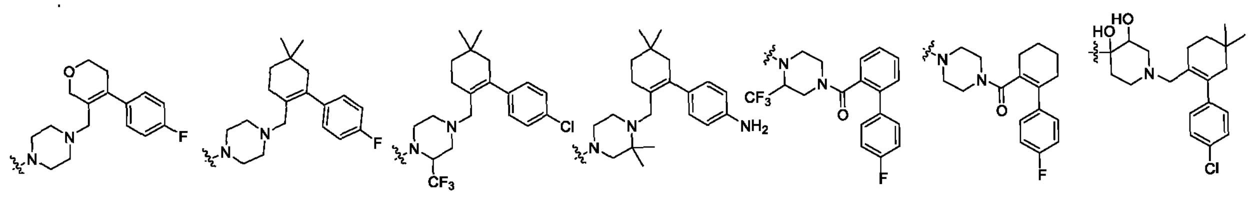

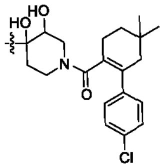 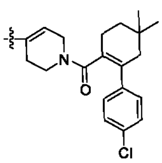 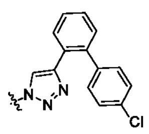 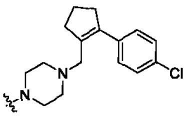 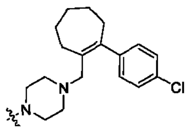 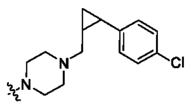 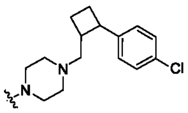

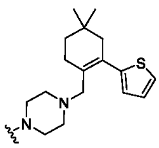 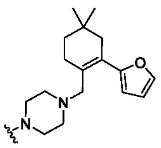 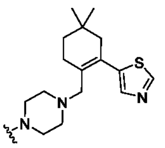 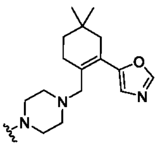 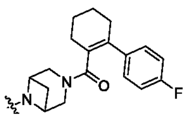 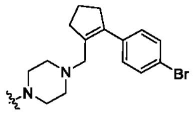 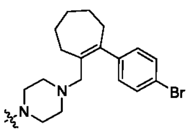

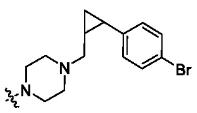 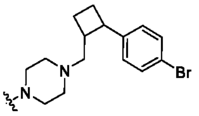 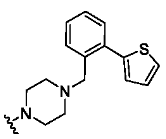 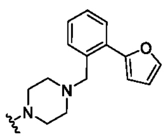 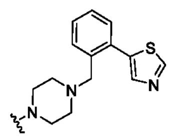 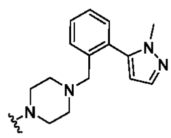 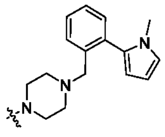

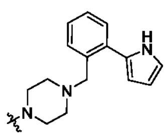 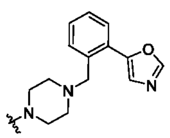 or 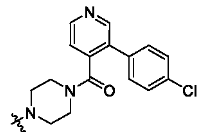 .

In some embodiments of the present disclosure, the compound of Formula (I) is also of Formula (II) or Formula (III):

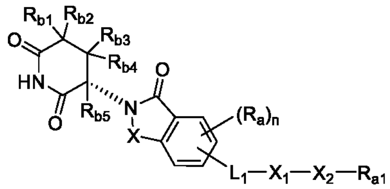
Formula (II)

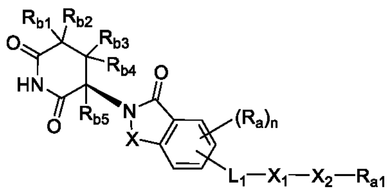
Formula (III)

wherein $R_{b1}$, $R_{b2}$, $R_{b3}$, $R_{b4}$, $R_{b5}$, $(R_a)_n$, X, $L_1$, $X_1$, $X_2$ and $R_{a1}$ are as defined in the compounds of Formula (I) above and its various embodiments herein.

In some embodiments of the present disclosure, the compound of Formula (I) is also of Formula (Ia), Formula (Ib), Formula (Ic), Formula (Id), Formula (IIa), Formula (IIb), Formula (IIc), Formula (IId), Formula (IIIa), Formula (IIIb), Formula (IIIc), or Formula (IIId):

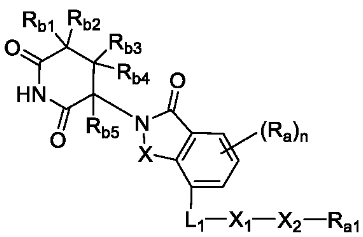
Formula (Ia)

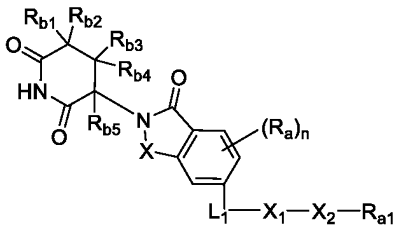
Formula (Ib)

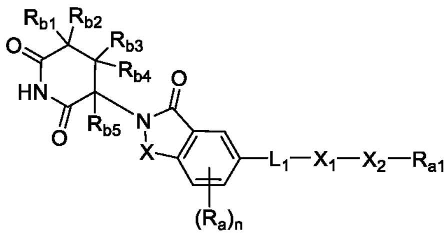
Formula (Ic)

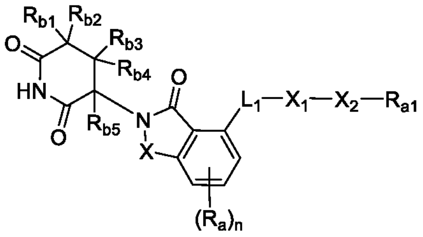
Formula (Id)

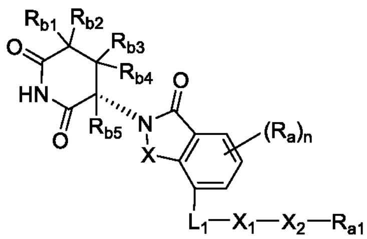
Formula (IIa)

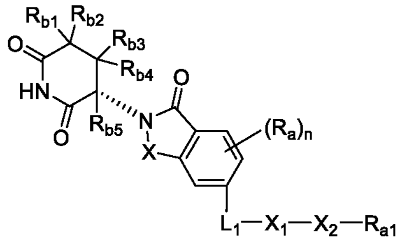
Formula (IIb)

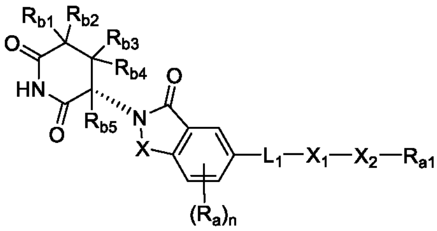
Formula (IIc)

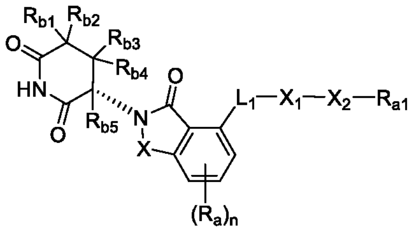
Formula (IId)

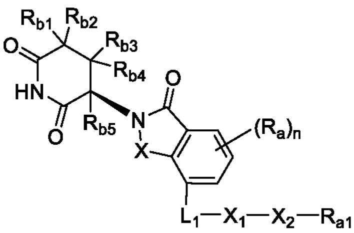
Formula (IIIa)

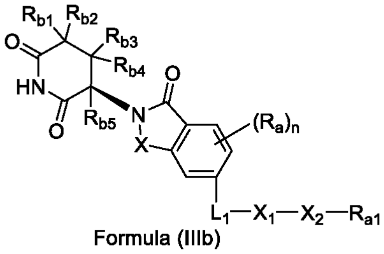
Formula (IIIb)

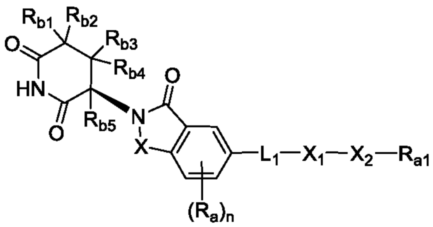
Formula (IIIc)

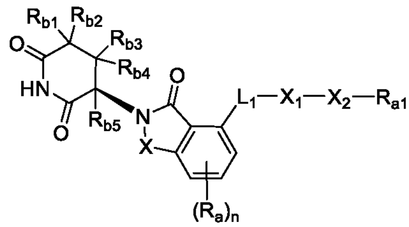
Formula (IIId)

wherein $R_{b1}$, $R_{b2}$, $R_{b3}$, $R_{b4}$, $R_{b5}$, $(R_a)_n$, X, $L_1$, $X_1$, $X_2$ and $R_{a1}$ are as defined in the compounds of Formula (I) above and its various embodiments herein.

Preferably the compounds of the present invention and their salts (especially pharmaceutically acceptable salts, such as hydrochloride, etc.), enantiomers, diastereomers, solvates, or polymorphs thereof in Table 1 below are provided.

Table 1. The compounds of the present invention

| Compound No. | Structure of the compounds | The compounds' name | Chinese translation of the compounds' name |
|---|---|---|---|
| GT-02785 | 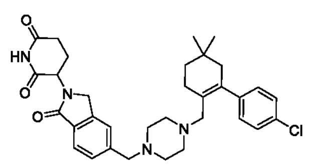 | 3-(5-((4-((4'-chloro-5,5-dimethyl-3,4,5,6-tetrahydro-[1,1'-biphenyl]-2-yl)methyl)piperazin-1-yl)methyl)-1-oxoisoindolin-2-yl)piperidine-2,6-dione | 3-(5-((4-((4'-chloro-5,5-dimethyl-3,4,5,6-tetrahydro-[1,1'-biphenyl]-2-yl)methyl)piperazin-1-yl)methyl)-1-oxoisoindolin-2-yl)piperidine-2,6-dione |
| GT-03468 | 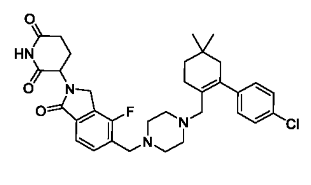 | 3-(5-((4-((4'-chloro-5,5-dimethyl-3,4,5,6-tetrahydro-[1,1'-biphenyl]-2-yl)methyl)piperazin-1-yl)methyl)-4-fluoro-1-oxoisoindolin-2-yl)piperidine-2,6-dione | 3-(5-((4-((4'-chloro-5,5-dimethyl-3,4,5,6-tetrahydro-[1,1'-biphenyl]-2-yl)methyl)piperazin-1-yl)methyl)-4-fluoro-1-oxoisoindolin-2-yl)piperidine-2,6-dione |
| GT-03578 | 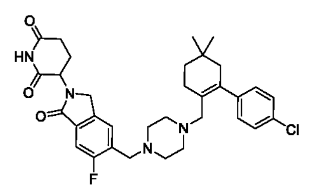 | 3-(5-((4-((4'-chloro-5,5-dimethyl-3,4,5,6-tetrahydro-[1,1'-biphenyl]-2-yl)methyl)piperazin-1-yl)methyl)-6-fluoro-1-oxoisoindolin-2-yl)piperidine-2,6-dione | 3-(5-((4-((4'-chloro-5,5-dimethyl-3,4,5,6-tetrahydro-[1,1'-biphenyl]-2-yl)methyl)piperazin-1-yl)methyl)-6-fluoro-1-oxoisoindolin-2-yl)piperidine-2,6-dione |

(continued)

| Compound No. | Structure of the compounds | The compounds' name | Chinese translation of the compounds' name |
|---|---|---|---|
| GT-03469 | 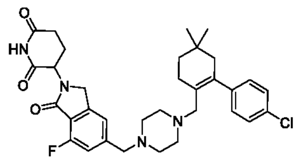 | 3-(5-((4-((4'-chloro-5,5-dimethyl-3,4,5,6-tetrahydro-[1,1'-biphenyl]-2-yl)methyl)piperazin-1-yl)methyl)-7-fluoro-1-oxoisoindolin-2-yl)piperidine-2,6-dione | 3-(5-((4-((4'-chloro-5,5-dimethyl-3,4,5,6-tetrahydro-[1,1'-biphenyl]-2-yl)methyl)piperazin-1-yl)methyl)-7-fluoro-1-oxoisoindolin-2-yl)piperidine-2,6-dione |
| GT-03649 | 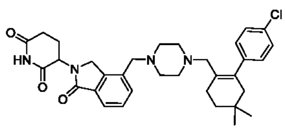 | 3-(4-((4-((4'-chloro-5,5-dimethyl-3,4,5,6-tetrahydro-[1,1'-biphenyl]-2-yl)methyl)piperazin-1-yl)methyl)-1-oxoisoindolin-2-yl)piperidine-2,6-dione | 3-(4-((4-((4'-chloro-5,5-dimethyl-3,4,5,6-tetrahydro-[1,1'-biphenyl]-2-yl)methyl)piperazin-1-yl)methyl)-1-oxoisoindolin-2-yl)piperidine-2,6-dione |
| GT-05363 | 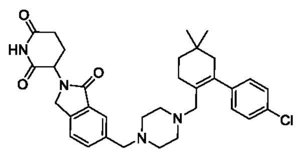 | 3-(6-((4-((4'-chloro-5,5-dimethyl-3,4,5,6-tetrahydro-[1,1'-biphenyl]-2-yl)methyl)piperazin-1-yl)methyl)-1-oxoisoindolin-2-yl)piperidine-2,6-dione | 3-(6-((4-((4'-chloro-5,5-dimethyl-3,4,5,6-tetrahydro-[1,1'-biphenyl]-2-yl)methyl)piperazin-1-yl)methyl)-1-oxoisoindolin-2-yl)piperidine-2,6-dione |
| GT-05360 | 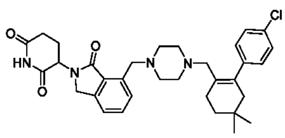 | 3-(7-((4-((4'-chloro-5,5-dimethyl-3,4,5,6-tetrahydro-[1,1'-biphenyl]-2-yl)methyl)piperazin-1-yl)methyl)-1-oxoisoindolin-2-yl)piperidine-2,6-dione | 3-(7-((4-((4'-chloro-5,5-dimethyl-3,4,5,6-tetrahydro-[1,1'-biphenyl]-2-yl)methyl)piperazin-1-yl)methyl)-1-oxoisoindolin-2-yl)piperidine-2,6-dione |
| GT-05361 | 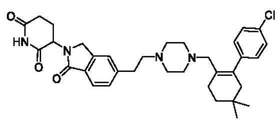 | 3-(5-(2-(4-((4'-chloro-5,5-dimethyl-3,4,5,6-tetrahydro-[1,1'-biphenyl]-2-yl)methyl)piperazin-1-yl)ethyl)-1-oxoisoindolin-2-yl)piperidine-2,6-dione | 3-(5-(2-(4-((4'-chloro-5,5-dimethyl-3,4,5,6-tetrahydro-[1,1'-biphenyl]-2-yl)methyl)piperazin-1-yl)ethyl)-1-oxoisoindolin-2-yl)piperidine-2,6-dione |
| GT-05362 | 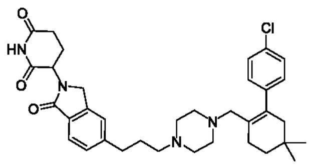 | 3-(5-(3-(4-((4'-chloro-5,5-dimethyl-3,4,5,6-tetrahydro-[1,1'-biphenyl]-2-yl)methyl)piperazin-1-yl)propyl)-1-oxoisoindolin-2-yl)piperidine-2,6-dione | 3-(5-(3-(4-((4'-chloro-5,5-dimethyl-3,4,5,6-tetrahydro-[1,1'-biphenyl]-2-yl)methyl)piperazin-1-yl)propyl)-1-oxoisoindolin-2-yl)piperidine-2,6-dione |
| | 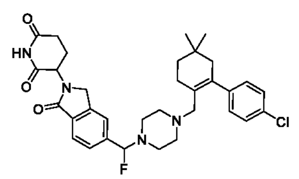 | 3-(5-((4-((4'-chloro-5,5-dimethyl-3,4,5,6-tetrahydro-[1,1'-biphenyl]-2-yl)methyl)piperazin-1-yl)fluoromethyl)-1-oxoisoindolin-2-yl)piperidine-2,6-dione | 3-(5-((4-((4'-chloro-5,5-dimethyl-3,4,5,6-tetrahydro-[1,1'-biphenyl]-2-yl)methyl)piperazin-1-yl)fluoromethyl)-1-oxoisoindolin-2-yl)piperidine-2,6-dione |
| | 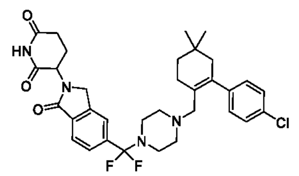 | 3-(5-((4-((4'-chloro-5,5-dimethyl-3,4,5,6-tetrahydro-[1,1'-biphenyl]-2-yl)methyl)piperazin-1-yl)difluoromethyl)-1-oxoisoindolin-2-yl)piperidine-2,6-dione | 3-(5-((4-((4'-chloro-5,5-dimethyl-3,4,5,6-tetrahydro-[1,1'-biphenyl]-2-yl)methyl)piperazin-1-yl)difluoromethyl)-1-oxoisoindolin-2-yl)piperidine-2,6-dione |
| | 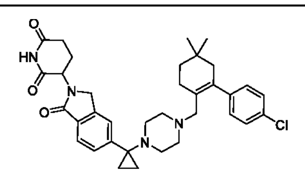 | 3-(5-(1-(4-((4'-chloro-5,5-dimethyl-3,4,5,6-tetrahydro-[1,1'-biphenyl]-2-yl)methyl)piperazin-1-yl)cyclopropyl)-1-oxoisoindolin-2-yl)piperidine-2,6-dione | 3-(5-(1-(4-((4'-chloro-5,5-dimethyl-3,4,5,6-tetrahydro-[1,1'-biphenyl]-2-yl)methyl)piperazin-1-yl)cyclopropyl)-1-oxoisoindolin-2-yl)piperidine-2,6-dione |

(continued)

| Compo und No. | Structure of the compounds | The compounds' name | Chinese translation of the compounds' name |
|---|---|---|---|
| | 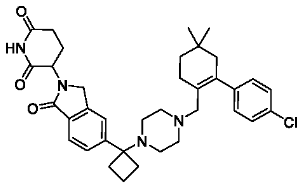 | 3-(5-(1-(4-((4'-chloro-5,5-dimethyl-3,4,5,6-tetrahydro-[1,1'-biphenyl]-2-yl)methyl)piperazin-1-yl)cyclobutyl)-1-oxoisoindolin-2-yl)piperidine-2,6-dione | 3-(5-(1-(4-((4'-chloro-5,5-dimethyl-3,4,5,6-tetrahydro-[1,1'-biphenyl]-2-yl)methyl)piperazin-1-yl)cyclobutyl)-1-oxoisoindolin-2-yl)piperidine-2,6-dione |
| | 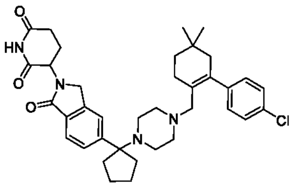 | 3-(5-(1-(4-((4'-chloro-5,5-dimethyl-3,4,5,6-tetrahydro-[1,1'-biphenyl]-2-yl)methyl)piperazin-1-yl)cyclopentyl)-1-oxoisoindolin-2-yl)piperidine-2,6-dione | 3-(5-(1-(4-((4'-chloro-5,5-dimethyl-3,4,5,6-tetrahydro-[1,1'-biphenyl]-2-yl)methyl)piperazin-1-yl)cyclopentyl)-1-oxoisoindolin-2-yl)piperidine-2,6-dione |
| | 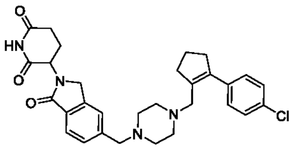 | 3-(5-((4-((2-(4-chlorophenyl)cyclopent-1-en-1-yl)methyl)piperazin-1-yl)methyl)-1-oxoisoindolin-2-yl)piperidine-2,6-dione | 3-(5-((4-((2-(4-chlorophenyl)cyclopent-1-en-1-yl)methyl)piperazin-1-yl)methyl)-1-oxoisoindolin-2-yl)piperidine-2,6-dione |
| | 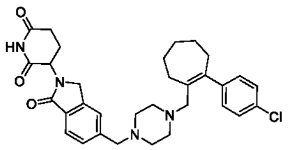 | 3-(5-((4-((2-(4-chlorophenyl)cyclohept-1-en-1-yl)methyl)piperazin-1-yl)methyl)-1-oxoisoindolin-2-yl)piperidine-2,6-dione | 3-(5-((4-((2-(4-chlorophenyl)cyclohept-1-en-1-yl)methyl)piperazin-1-yl)methyl)-1-oxoisoindolin-2-yl)piperidine-2,6-dione |
| | 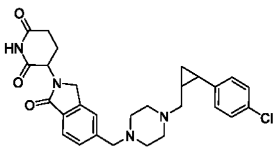 | 3-(5-((4-((2-(4-chlorophenyl)cyclopropyl)m ethyl)piperazin-1-yl)methyl)-1-oxoisoindolin-2-yl) piperidine-2,6-dione | 3-(5-((4-((2-(4-chlorophenyl)cyclopropyl)me thyl)piperazin-1-yl)methyl)-1-oxoisoindolin-2-yl) piperidine-2,6-dione |
| | 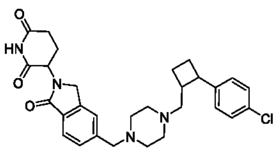 | 3-(5-((4-((2-(4-chlorophenyl)cyclobutyl)me thyl)piperazin-1-yl) methyl)-1-oxoisoindolin-2-yl)piperidine-2,6-dione | 3-(5-((4-((2-(4-chlorophenyl)cyclobutyl)met hyl)piperazin-1-yl) methyl)-1-oxoisoindolin-2-yl)piperidine-2,6-dione |
| GT-05847 | 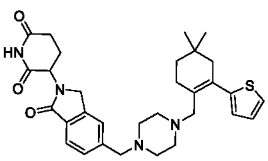 | 3-(5-((4-((4,4-dimethyl-2-(thiophen-2-yl)cyclohex-1-en-1-yl) methyl)piperazin-1-yl) methyl)-1-oxoisoindolin-2-yl)piperidine-2,6-dione | 3-(5-((4-((4,4-dimethyl-2-(thiophen-2-yl)cyclohex-1-en-1-yl) methyl)piperazin-1-yl) methyl)-1-oxoisoindolin-2-yl)piperidine-2,6-dione |
| GT-05790 | 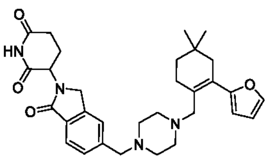 | 3-(5-((4-((2-(furan-2-yl)-4,4-dimethylcyclohex-1-en-1-yl) methyl)piperazin-1-yl) methyl)-1-oxoisoindolin-2-yl)piperidine-2,6-dione | 3-(5-((4-((2-(furan-2-yl)-4,4-dimethylcyclohex-1-en-1-yl) methyl)piperazin-1-yl) methyl)-1-oxoisoindolin-2-yl)piperidine-2,6-dione |
| GT-05791 | 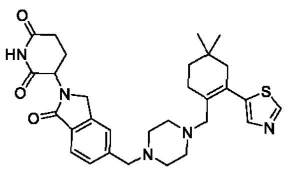 | 3-(5-((4-((4,4-dimethyl-2-(thiazol-5-yl)cyclohex-1-en-1-yl) methyl)piperazin-1-yl) methyl)-1-oxoisoindolin-2-yl)piperidine-2,6-dione | 3-(5-((4-((4,4-dimethyl-2-(thiazol-5-yl)cyclohex-1-en-1-yl) methyl)piperazin-1-yl) methyl)-1-oxoisoindolin-2-ylpiperidine-2,6-dione |

(continued)

| Compo und No. | Structure of the compounds | The compounds' name | Chinese translation of the compounds' name |
|---|---|---|---|
| GT-05848 | 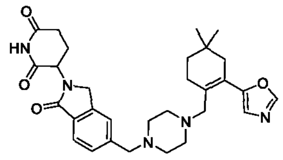 | 3-(5-((4-((4,4-dimethyl-2-(oxazol-5-yl)cyclohex-1-en-1-yl) methyl)piperazin-1-yl) methyl)-1-oxoisoindolin-2-yl)piperidine-2,6-dione | 3-(5-((4-((4,4-dimethyl-2-(oxazol-5-yl)cyclohex-1-en-1-yl) methyl)piperazin-1-yl) methyl)-1-oxoisoindolin-2-yl)piperidine-2,6-dione |
| | 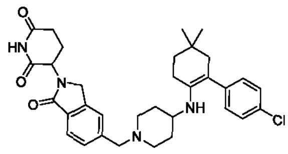 | 3-(5-((4-((4'-chloro-5,5-dimethyl-3,4,5,6-tetrahydro-[1,1'-biphenyl]-2-yl)amino)piperidin-1-yl)methyl)-1-oxoisoindolin-2-yl)piperidine-2,6-dione | 3-(5-((4-((4'-chloro-5,5-dimethyl-3,4,5,6-tetrahydro-[1,1'-biphenyl]-2-yl)amino)piperidin-1-yl)methyl)-1-oxoisoindolin-2-yl)piperidine-2,6-dione |
| GT-03368 | 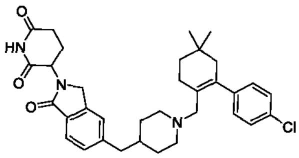 | 3-(5-((1-((4'-chloro-5,5-dimethyl-3,4,5,6-tetrahydro-[1,1'-biphenyl]-2-yl)methyl)piperidin-4-yl)methyl)-1-oxoisoindolin-2-yl)piperidine-2,6-dione | 3-(5-((1-((4'-chloro-5,5-dimethyl-3,4,5,6-tetrahydro-[1,1'-biphenyl]-2-yl)methyl)piperidin-4-yl)methyl)-1-oxoisoindolin-2-yl)piperidine-2,6-dione |
| GT-05782 | 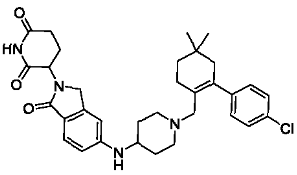 | 3-(5-((1-((4'-chloro-5,5-dimethyl-3,4,5,6-tetrahydro-[1,1'-biphenyl]-2-yl)methyl)piperidin-4-yl)amino)-1-oxoisoindolin-2-yl)piperidine-2,6-dione | 3-(5-((1-((4'-chloro-5,5-dimethyl-3,4,5,6-tetrahydro-[1,1'-biphenyl]-2-yl)methyl)piperidin-4-yl)amino)-1-oxoisoindolin-2-yl)piperidine-2,6-dione |
| GT-05428 | 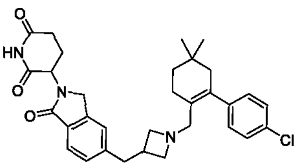 | 3-(5-((1-((4'-chloro-5,5-dimethyl-3,4,5,6-tetrahydro-[1,1'-biphenyl]-2-yl)methyl)azetidin-3-yl)methyl)-1-oxoisoindolin-2-yl)piperidine-2,6-dione | 3-(5-((1-((4'-chloro-5,5-dimethyl-3,4,5,6-tetrahydro-[1,1'-biphenyl]-2-yl)methyl)azetidin-3-yl)methyl)-1-oxoisoindolin-2-yl)piperidine-2,6-dione |
| | 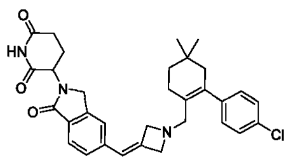 | 3-(5-((1-((4'-chloro-5,5-dimethyl-3,4,5,6-tetrahydro-[1,1'-biphenyl]-2-yl)methyl)azetidin-3-ylidene)methyl)-1-oxoisoindolin-2-yl)piperidine-2,6-dione | 3-(5-((1-((4'-chloro-5,5-dimethyl-3,4,5,6-tetrahydro-[1,1'-biphenyl]-2-yl)methyl)azetidin-3-ylidene)methyl)-1-oxoisoindolin-2-yl)piperidine-2,6-dione |
| GT-05427 | 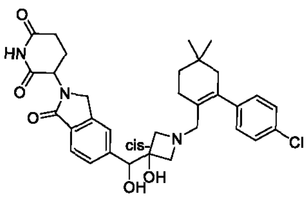 | 3-(5-((1-((4'-chloro-5,5-dimethyl-3,4,5,6-tetrahydro-[1,1'-biphenyl]-2-yl)methyl)-3-hydroxyazetidin-3-yl)(hydroxy) methyl)-1-oxoisoindolin-2-yl)piperidine-2,6-dione | 3-(5-((1-((4'-chloro-5,5-dimethyl-3,4,5,6-tetrahydro-[1,1 '-biphenyl]-2-yl)methyl)-3-hydroxyazetidin-3-yl)(hydroxy) methyl)-1-oxoisoindolin-2-yl)piperidine-2,6-dione |
| GT-03367 | 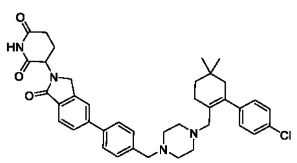 | 3-(5-(4-((4-((4'-chloro-5,5-dimethyl-3,4,5,6-tetrahydro-[1,1'-biphenyl]-2-yl)methyl)piperazin-1-yl)methyl)phenyl)-1-oxoisoindolin-2-yl)piperidine-2,6-dione | 3-(5-(4-((4-((4'-chloro-5,5-dimethyl-3,4,5,6-tetrahydro-[1,1'-biphenyl]-2-yl)methyl)piperazin-1-yl)methyl)phenyl)-1-oxoisoindolin-2-yl)piperidine-2,6-dione |
| GT-04252 | 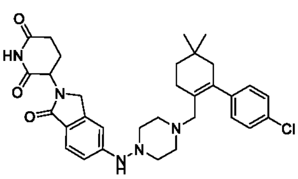 | 3-(5-((4-((4'-chloro-5,5-dimethyl-3,4,5,6-tetrahydro-[1,1'-biphenyl]-2-yl)methyl)piperazin-1-yl)amino)-1-oxoisoindolin-2-yl)piperidine-2,6-dione | 3-(5-((4-((4'-chloro-5,5-dimethyl-3,4,5,6-tetrahydro-[1,1'-biphenyl]-2-yl)methyl)piperazin-1-yl)amino)-1-oxoisoindolin-2-yl)piperidine-2,6-dione |

(continued)

| Compo und No. | Structure of the compounds | The compounds' name | Chinese translation of the compounds' name |
|---|---|---|---|
| | 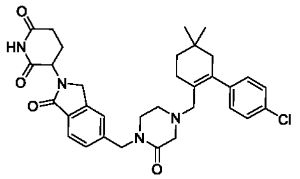 | 3-(5-((4-((4'-chloro-5,5-dimethyl-3,4,5,6-tetrahydro-[1,1'-biphenyl]-2-yl)methyl)-2-oxopiperazin-1-yl)methyl)-1-oxoisoindolin-2-yl)piperidine-2,6-dione | 3-(5-((4-((4'-chloro-5,5-dimethyl-3,4,5,6-tetrahydro-[1,1'-biphenyl]-2-yl)methyl)-2-oxopiperazin-1-yl)methyl)-1-oxoisoindolin-2-yl)piperidine-2,6-dione |
| | 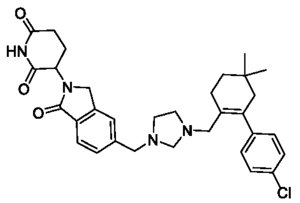 | 3-(5-((3-((4'-chloro-5,5-dimethyl-3,4,5,6-tetrahydro-[1,1'-biphenyl]-2-yl)methyl)imidazolidin-1-yl)methyl)-1-oxoisoindolin-2-yl)piperidine-2,6-dione | 3-(5-((3-((4'-chloro-5,5-dimethyl-3,4,5,6-tetrahydro-[1,1'-biphenyl]-2-yl)methyl)imidazolidin-1-yl)methyl)-1-oxoisoindolin-2-yl)piperidine-2,6-dione |
| GT-05396 | 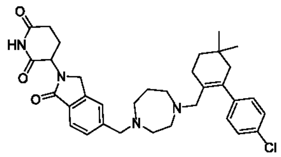 | 3-(5-((4-((4'-chloro-5,5-dimethyl-3,4,5,6-tetrahydro-[1,1'-biphenyl]-2-yl)methyl)-1,4-diazepan-1-yl)methyl)-1-oxoisoindolin-2-yl)piperidine-2,6-dione | 3-(5-((4-((4'-chloro-5,5-dimethyl-3,4,5,6-tetrahydro-[1,1'-biphenyl]-2-yl)methyl)-1,4-diazepan-1-yl)methyl)-1-oxoisoindolin-2-yl)piperidine-2,6-dione |
| GT-05685 | 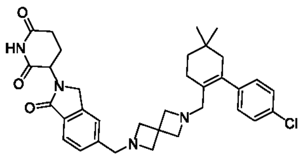 | 3-(5-((6-((4'-chloro-5,5-dimethyl-3,4,5,6-tetrahydro-[1,1'-biphenyl]-2-yl)methyl)-2,6-diazaspiro[3.3]heptan-2-yl) methyl)-1-oxoisoindolin-2-yl)piperidine-2,6-dione | 3-(5-((6-((4'-chloro-5,5-dimethyl-3,4,5,6-tetrahydro-[1,1'-biphenyl]-2-yl)methyl)-2,6-diazaspiro[3.3]heptan-2-yl) methyl)-1-oxoisoindolin-2-yl)piperidine-2,6-dione |
| GT-05677 | 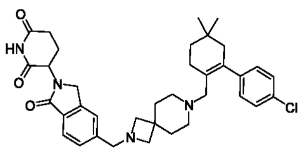 | 3-(5-((7-((4'-chloro-5,5-dimethyl-3,4,5,6-tetrahydro-[1,1'-biphenyl]-2-yl)methyl)-2,7-diazaspiro[3.5]nonan-2-yl) methyl)-1-oxoisoindolin-2-yl)piperidine-2,6-dione | 3-(5-((7-((4'-chloro-5,5-dimethyl-3,4,5,6-tetrahydro-[1,1'-biphenyl]-2-yl)methyl)-2,7-diazaspiro[3.5]nonan-2-yl) methyl)-1-oxoisoindolin-2-yl)piperidine-2,6-dione |
| GT-05653 | 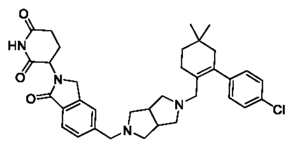 | 3-(5-((5-((4'-chloro-5,5-dimethyl-3,4,5,6-tetrahydro-[1,1'-biphenyl]-2-yl)methyl)hexahydropyrrolo [3,4-c]pyrrol-2(1H)-yl)methyl)-1-oxoisoindolin-2-yl)piperidine-2,6-dione | 3-(5-((5-((4'-chloro-5,5-dimethyl-3,4,5,6-tetrahydro-[1,1'-biphenyl]-2-yl)methyl)hexahydropyrrolo[ 3,4-c]pyrrol-2(1H)-yl)methyl)-1-oxoisoindolin-2-yl)piperidine-2,6-dione |
| GT-05429 | 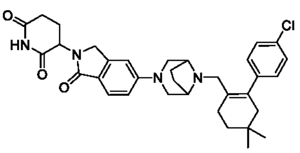 | 3-(5-(8-((4'-chloro-5,5-dimethyl-3,4,5,6-tetrahydro-[1,1'-biphenyl]-2-yl)methyl)-3,8-diazabicyclo[3.2.1]octan-3-yl)-1-oxoisoindolin-2-yl)piperidine-2,6-dione | 3-(5-(8-((4'-chloro-5,5-dimethyl-3,4,5,6-tetrahydro-[1,1'-biphenyl]-2-yl)methyl)-3,8-diazabicyclo[3.2.1]octan-3-yl)-1-oxoisoindolin-2-yl)piperidine-2,6-dione |
| GT-05430 | 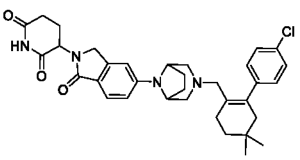 | 3-(5-(3-((4'-chloro-5,5-dimethyl-3,4,5,6-tetrahydro-[1,1'-biphenyl]-2-yl)methyl)-3,8-diazabicyclo[3.2.1]octan-8-yl)-1-oxoisoindolin-2-yl)piperidine-2,6-dione | 3-(5-(3-((4'-chloro-5,5-dimethyl-3,4,5,6-tetrahydro-[1,1'-biphenyl]-2-yl)methyl)-3,8-diazabicyclo[3.2.1]octan-8-yl)-1-oxoisoindolin-2-yl)piperidine-2,6-dione |

(continued)

| Compound No. | Structure of the compounds | The compounds' name | Chinese translation of the compounds' name |
|---|---|---|---|
| GT-03492 | 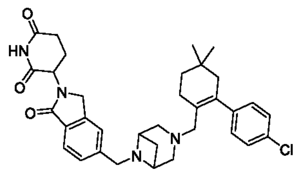 | 3-(5-((3-((4'-chloro-5,5-dimethyl-3,4,5,6-tetrahydro-[1,1'-biphenyl]-2-yl)methyl)-3,6-diazabicyclo[3.1.1]heptan-6-yl)methyl)-1-oxoisoindolin-2-yl)piperidine-2,6-dione | 3-(5-((3-((4'-chloro-5,5-dimethyl-3,4,5,6-tetrahydro-[1,1'-biphenyl]-2-yl)methyl)-3,6-diazabicyclo[3.1.1]heptan-6-yl)methyl)-1-oxoisoindolin-2-yl)piperidine-2,6-dione |
| GT-03995 | 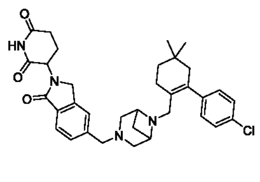 | 3-(5-((6-((4'-chloro-5,5-dimethyl-3,4,5,6-tetrahydro-[1,1'-biphenyl]-2-yl)methyl)-3,6-diazabicyclo[3.1.1]heptan-3-yl)methyl)-1-oxoisoindolin-2-yl)piperidine-2,6-dione | 3-(5-((6-((4'-chloro-5,5-dimethyl-3,4,5,6-tetrahydro-[1,1'-biphenyl]-2-yl)methyl)-3,6-diazabicyclo[3.1.1]heptan-3-yl)methyl)-1-oxoisoindolin-2-yl)piperidine-2,6-dione |
| GT-06194 | 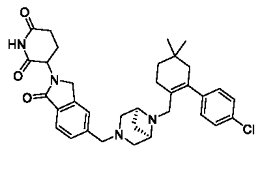 | 3-(5-(((1R,5S)-6-((4'-chloro-5,5-dimethyl-3,4,5,6-tetrahydro-[1,1'-biphenyl]-2-yl)methyl)-3,6-diazabicyclo[3.1.1]heptan-3-yl)methyl)-1-oxoisoindolin-2-yl)piperidine-2,6-dione | 3-(5-(((1R,5S)-6-((4'-chloro-5,5-dimethyl-3,4,5,6-tetrahydro-[1,1'-biphenyl]-2-yl)methyl)-3,6-diazabicyclo[3.1.1]heptan-3-yl)methyl)-1-oxoisoindolin-2-yl)piperidine-2,6-dione |
| GT-03889 | 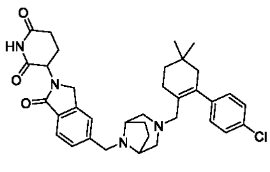 | 3-(5-((3-((4'-chloro-5,5-dimethyl-3,4,5,6-tetrahydro-[1,1'-biphenyl]-2-yl)methyl)-3,8-diazabicyclo[3.2.1]octan-8-yl)methyl)-1-oxoisoindolin-2-yl)piperidine-2,6-dione | 3-(5-((3-((4'-chloro-5,5-dimethyl-3,4,5,6-tetrahydro-[1,1'-biphenyl]-2-yl)methyl)-3,8-diazabicyclo[3.2.1]octan-8-yl)methyl)-1-oxoisoindolin-2-yl)piperidine-2,6-dione |
| GT-03996 | 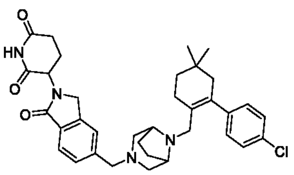 | 3-(5-((8-((4'-chloro-5,5-dimethyl-3,4,5,6-tetrahydro-[1,1'-biphenyl]-2-yl)methyl)-3,8-diazabicyclo[3.2.1]octan-3-yl)methyl)-1-oxoisoindolin-2-yl)piperidine-2,6-dione | 3-(5-((8-((4'-chloro-5,5-dimethyl-3,4,5,6-tetrahydro-[1,1'-biphenyl]-2-yl)methyl)-3,8-diazabicyclo[3.2.1]octan-3-yl)methyl)-1-oxoisoindolin-2-yl)piperidine-2,6-dione |
| GT-04167 | 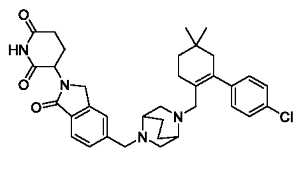 | 3-(5-((5-((4'-chloro-5,5-dimethyl-3,4,5,6-tetrahydro-[1,1'-biphenyl]-2-yl)methyl)-2,5-diazabicyclo[2.2.2]octan-2-yl)methyl)-1-oxoisoindolin-2-yl)piperidine-2,6-dione | 3-(5-((5-((4'-chloro-5,5-dimethyl-3,4,5,6-tetrahydro-[1,1'-biphenyl]-2-yl)methyl)-2,5-diazabicyclo[2.2.2]octan-2-yl)methyl)-1-oxoisoindolin-2-yl)piperidine-2,6-dione |
| GT-03887 | 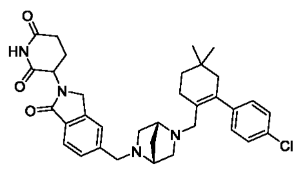 | 3-(5-(((1R,4R)-5-((4'-chloro-5,5-dimethyl-3,4,5,6-tetrahydro-[1,1'-biphenyl]-2-yl)methyl)-2,5-diazabicyclo[2.2.1]heptan-2-yl)methyl)-1-oxoisoindolin-2-yl)piperidine-2,6-dione | 3-(5-(((1R,4R)-5-((4'-chloro-5,5-dimethyl-3,4,5,6-tetrahydro-[1,1'-biphenyl]-2-yl)methyl)-2,5-diazabicyclo[2.2.1]heptan-2-yl)methyl)-1-oxoisoindolin-2-yl)piperidine-2,6-dione |
| | 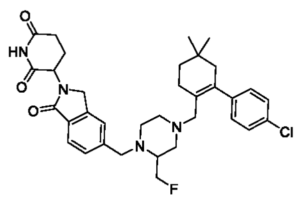 | 3-(5-((4-((4'-chloro-5,5-dimethyl-3,4,5,6-tetrahydro-[1,1'-biphenyl]-2-yl)methyl)-2-(fluoromethyl)piperazin-1-yl)methyl)-1-oxoisoindolin-2-yl)piperidine-2,6-dione | 3-(5-((4-((4'-chloro-5,5-dimethyl-3,4,5,6-tetrahydro-[1,1'-biphenyl]-2-yl)methyl)-2-(fluoromethyl)piperazin-1-yl)methyl)-1-oxoisoindolin-2-yl)piperidine-2,6-dione |

(continued)

| Compo und No. | Structure of the compounds | The compounds' name | Chinese translation of the compounds' name |
|---|---|---|---|
| | 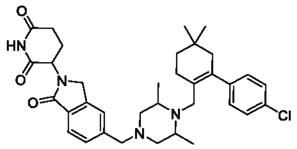 | 3-(5-((4-((4'-chloro-5,5-dimethyl-3,4,5,6-tetrahydro-[1,1'-biphenyl]-2-yl)methyl)-3,5-dimethylpiperazin-1-yl)methyl)-1-oxoisoindolin-2-yl)piperidine-2,6-dione | 3-(5-((4-((4'-chloro-5,5-dimethyl-3,4,5,6-tetrahydro-[1,1'-biphenyl]-2-yl)methyl)-3,5-dimethylpiperazin-1-yl)methyl)-1-oxoisoindolin-2-yl)piperidine-2,6-dione |
| GT-05796 | 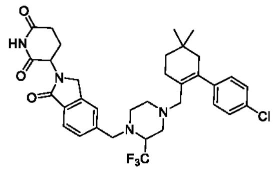 | 3-(5-((4-((4'-chloro-5,5-dimethyl-3,4,5,6-tetrahydro-[1,1'-biphenyl]-2-yl)methyl)-2-(trifluoromethyl)piperazin-1-yl) methyl)-1-oxoisoindolin-2-yl)piperidine-2,6-dione | 3-(5-((4-((4'-chloro-5,5-dimethyl-3,4,5,6-tetrahydro-[1,1'-biphenyl]-2-yl)methyl)-2-(trifluoromethyl)piperazin-1-yl) methyl)-1-oxoisoindolin-2-yl)piperidine-2,6-dione |
| | 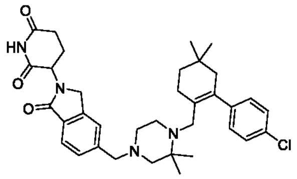 | 3-(5-((4-((4'-chloro-5,5-dimethyl-3,4,5,6-tetrahydro-[1,1'-biphenyl]-2-yl)methyl)-3,3-dimethylpiperazin-1-yl)methyl)-1-oxoisoindolin-2-yl)piperidine-2,6-dione | 3-(5-((4-((4'-chloro-5,5-dimethyl-3,4,5,6-tetrahydro-[1,1'-biphenyl]-2-yl)methyl)-3,3-dimethylpiperazin-1-yl)methyl)-1-oxoisoindolin-2-yl)piperidine-2,6-dione |
| GT-03493 | 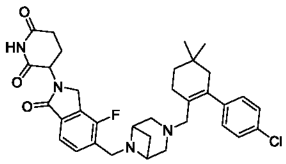 | 3-(5-((3-((4'-chloro-5,5-dimethyl-3,4,5,6-tetrahydro-[1,1'-biphenyl]-2-yl)methyl)-3,6-diazabicyclo[3.1.1]heptan-6-yl) methyl)-4-fluoro-1-oxoisoindolin-2-yl)piperidine-2,6-dione | 3-(5-((3-((4'-chloro-5,5-dimethyl-3,4,5,6-tetrahydro-[1,1'-biphenyl]-2-yl)methyl)-3,6-diazabicyclo[3.1.1]heptan-6-yl) methyl)-4-fluoro-1-oxoisoindolin-2-yl)piperidine-2,6-dione |
| GT-05529 | 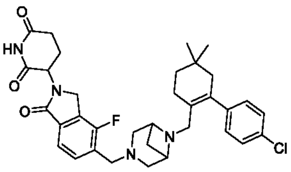 | 3-(5-((6-((4'-chloro-5,5-dimethyl-3,4,5,6-tetrahydro-[1,1'-biphenyl]-2-yl)methyl)-3,6-diazabicyclo[3.1.1]heptan-3-yl) methyl)-4-fluoro-1-oxoisoindolin-2-yl)piperidine-2,6-dione | 3-(5-((6-((4'-chloro-5,5-dimethyl-3,4,5,6-tetrahydro-[1,1'-biphenyl]-2-yl)methyl)-3,6-diazabicyclo[3.1.1]heptan-3-yl) methyl)-4-fluoro-1-oxoisoindolin-2-yl)piperidine-2,6-dione |
| GT-03890 | 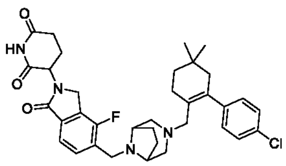 | 3-(5-((3-((4'-chloro-5,5-dimethyl-3,4,5,6-tetrahydro-[1,1'-biphenyl]-2-yl)methyl)-3,8-diazabicyclo[3.2.1]octan-8-yl) methyl)-4-fluoro-1-oxoisoindolin-2-yl)piperidine-2,6-dione | 3-(5-((3-((4'-chloro-5,5-dimethyl-3,4,5,6-tetrahydro-[1,1'-biphenyl]-2-yl)methyl)-3,8-diazabicyclo[3.2.1]octan-8-yl) methyl)-4-fluoro-1-oxoisoindolin-2-yl)piperidine-2,6-dione |
| GT-05534 | 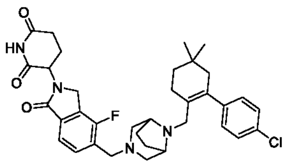 | 3-(5-((8-((4'-chloro-5,5-dimethyl-3,4,5,6-tetrahydro-[1,1'-biphenyl]-2-yl)methyl)-3,8-diazabicyclo[3.2.1]octan-3-yl) methyl)-4-fluoro-1-oxoisoindolin-2-yl)piperidine-2,6-dione | 3-(5-((8-((4'-chloro-5,5-dimethyl-3,4,5,6-tetrahydro-[1,1'-biphenyl]-2-yl)methyl)-3,8-diazabicyclo[3.2.1]octan-3-yl) methyl)-4-fluoro-1-oxoisoindolin-2-yl)piperidine-2,6-dione |
| GT-04168 | 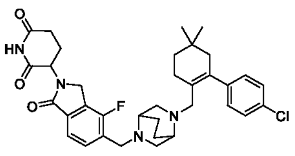 | 3-(5-((5-((4'-chloro-5,5-dimethyl-3,4,5,6-tetrahydro-[1,1'-biphenyl]-2-yl)methyl)-2,5-diazabicyclo[2.2.2]octan-2-yl) methyl)-4-fluoro-1-oxoisoindolin-2-yl)piperidine-2,6-dione | 3-(5-((5-((4'-chloro-5,5-dimethyl-3,4,5,6-tetrahydro-[1,1'-biphenyl]-2-yl)methyl)-2,5-diazabicyclo[2.2.2]octan-2-yl) methyl)-4-fluoro-1-oxoisoindolin-2-yl)piperidine-2,6-dione |

(continued)

| Compo und No. | Structure of the compounds | The compounds' name | Chinese translation of the compounds' name |
|---|---|---|---|
| GT-03888 | 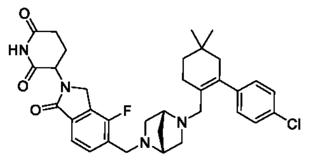 | 3-(5-(((1R,4R)-5-((4'-chloro-5,5-dimethyl-3,4,5,6-tetrahydro-[1,1'-biphenyl]-2-yl)methyl)-2,5-diazabicyclo[2.2.1]heptan-2-yl)methyl)-4-fluoro-1-oxoisoindolin-2-yl)piperidine-2,6-dione | 3-(5-(((1R,4R)-5-((4'-chloro-5,5-dimethyl-3,4,5,6-tetrahydro-[1,1'-biphenyl]-2-yl)methyl)-2,5-diazabicyclo[2.2.1]heptan-2-yl)methyl)-4-fluoro-1-oxoisoindolin-2-yl)piperidine-2,6-dione |
| | 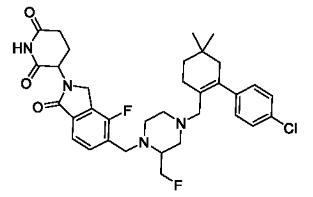 | 3-(5-((4-((4'-chloro-5,5-dimethyl-3,4,5,6-tetrahydro-[1,1'-biphenyl]-2-yl)methyl)-2-(fluoromethyl)piperazin-1-yl)methyl)-4-fluoro-1-oxoisoindolin-2-yl)piperidine-2,6-dione | 3-(5-((4-((4'-chloro-5,5-dimethyl-3,4,5,6-tetrahydro-[1,1'-biphenyl]-2-yl)methyl)-2-(fluoromethyl)piperazin-1-yl)methyl)-4-fluoro-1-oxoisoindolin-2-yl)piperidine-2,6-dione |
| | 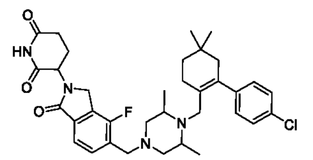 | 3-(5-((4-((4'-chloro-5,5-dimethyl-3,4,5,6-tetrahydro-[1,1'-biphenyl]-2-yl)methyl)-3,5-dimethylpiperazin-1-yl)methyl)-4-fluoro-1-oxoisoindolin-2-yl)piperidine-2,6-dione | 3-(5-((4-((4'-chloro-5,5-dimethyl-3,4,5,6-tetrahydro-[1,1'-biphenyl]-2-yl)methyl)-3,5-dimethylpiperazin-1-yl)methyl)-4-fluoro-1-oxoisoindolin-2-yl)piperidine-2,6-dione |
| GT-06100 | 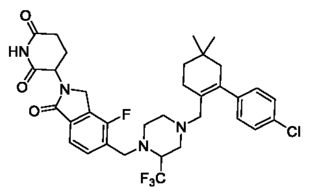 | 3-(5-((4-((4'-chloro-5,5-dimethyl-3,4,5,6-tetrahydro-[1,1'-biphenyl]-2-yl)methyl)-2-(trifluoromethyl)piperazin-1-yl)methyl)-4-fluoro-1-oxoisoindolin-2-yl)piperidine-2,6-dione | 3-(5-((4-((4'-chloro-5,5-dimethyl-3,4,5,6-tetrahydro-[1,1'-biphenyl]-2-yl)methyl)-2-(trifluoromethyl)piperazin-1-yl)methyl)-4-fluoro-1-oxoisoindolin-2-yl)piperidine-2,6-dione |
| | 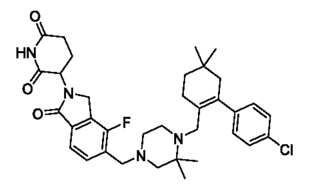 | 3-(5-((4-((4'-chloro-5,5-dimethyl-3,4,5,6-tetrahydro-[1,1'-biphenyl]-2-yl)methyl)-3,3-dimethylpiperazin-1-yl)methyl)-4-fluoro-1-oxoisoindolin-2-yl)piperidine-2,6-dione | 3-(5-((4-((4'-chloro-5,5-dimethyl-3,4,5,6-tetrahydro-[1,1'-biphenyl]-2-yl)methyl)-3,3-dimethylpiperazin-1-yl)methyl)-4-fluoro-1-oxoisoindolin-2-yl)piperidine-2,6-dione |
| GT-05367 | 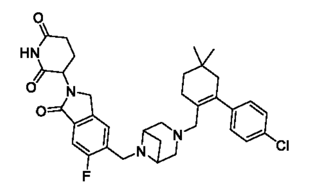 | 3-(5-((3-((4'-chloro-5,5-dimethyl-3,4,5,6-tetrahydro-[1,1'-biphenyl]-2-yl)methyl)-3,6-diazabicyclo[3.1.1]heptan-6-yl)methyl)-6-fluoro-1-oxoisoindolin-2-yl)piperidine-2,6-dione | 3-(5-((3-((4'-chloro-5,5-dimethyl-3,4,5,6-tetrahydro-[1,1'-biphenyl]-2-yl)methyl)-3,6-diazabicyclo[3.1.1]heptan-6-yl)methyl)-6-fluoro-1-oxoisoindolin-2-yl)piperidine-2,6-dione |
| GT-05530 | 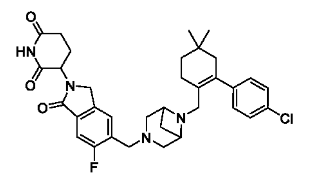 | 3-(5-((6-((4'-chloro-5,5-dimethyl-3,4,5,6-tetrahydro-[1,1'-biphenyl]-2-yl)methyl)-3,6-diazabicyclo[3.1.1]heptan-3-yl)methyl)-6-fluoro-1-oxoisoindolin-2-yl)piperidine-2,6-dione | 3-(5-((6-((4'-chloro-5,5-dimethyl-3,4,5,6-tetrahydro-[1,1'-biphenyl]-2-yl)methyl)-3,6-diazabicyclo[3.1.1]heptan-3-yl)methyl)-6-fluoro-1-oxoisoindolin-2-yl)piperidine-2,6-dione |
| GT-05494 | 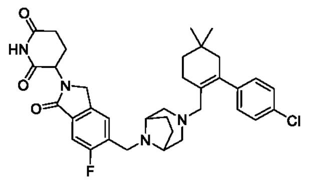 | 3-(5-((3-((4'-chloro-5,5-dimethyl-3,4,5,6-tetrahydro-[1,1'-biphenyl]-2-yl)methyl)-3,8-diazabicyclo[3.2.1]octan-8-yl)methyl)-6-fluoro-1-oxoisoindolin-2-yl)piperidine-2,6-dione | 3-(5-((3-((4'-chloro-5,5-dimethyl-3,4,5,6-tetrahydro-[1,1'-biphenyl]-2-yl)methyl)-3,8-diazabicyclo[3.2.1]octan-8-yl)methyl)-6-fluoro-1-oxoisoindolin-2-yl)piperidine-2,6-dione |

(continued)

| Compound No. | Structure of the compounds | The compounds' name | Chinese translation of the compounds' name |
|---|---|---|---|
| GT-05535 | 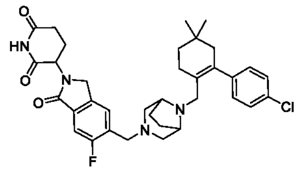 | 3-(5-((8-((4'-chloro-5,5-dimethyl-3,4,5,6-tetrahydro-[1,1'-biphenyl]-2-yl)methyl)-3,8-diazabicyclo[3.2.1]octan-3-yl)methyl)-6-fluoro-1-oxoisoindolin-2-yl)piperidine-2,6-dione | 3-(5-((8-((4'-chloro-5,5-dimethyl-3,4,5,6-tetrahydro-[1,1'-biphenyl]-2-yl)methyl)-3,8-diazabicyclo[3.2.1]octan-3-yl)methyl)-6-fluoro-1-oxoisoindolin-2-yl)piperidine-2,6-dione |
| GT-05460 | 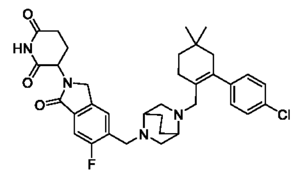 | 3-(5-((5-((4'-chloro-5,5-dimethyl-3,4,5,6-tetrahydro-[1,1'-biphenyl]-2-yl)methyl)-2,5-diazabicyclo[2.2.2]octan-2-yl)methyl)-6-fluoro-1-oxoisoindolin-2-yl)piperidine-2,6-dione | 3-(5-((5-((4'-chloro-5,5-dimethyl-3,4,5,6-tetrahydro-[1,1'-biphenyl]-2-yl)methyl)-2,5-diazabicyclo[2.2.2]octan-2-yl)methyl)-6-fluoro-1-oxoisoindolin-2-yl)piperidine-2,6-dione |
| | 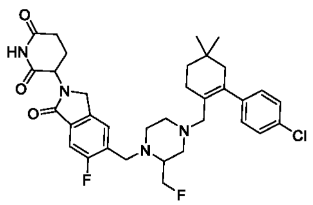 | 3-(5-((4-((4'-chloro-5,5-dimethyl-3,4,5,6-tetrahydro-[1,1'-biphenyl]-2-yl)methyl)-2-(fluoromethyl)piperazin-1-yl)methyl)-6-fluoro-1-oxoisoindolin-2-yl)piperidine-2,6-dione | 3-(5-((4-((4'-chloro-5,5-dimethyl-3,4,5,6-tetrahydro-[1,1'-biphenyl]-2-yl)methyl)-2-(fluoromethyl)piperazin-1-yl)methyl)-6-fluoro-1-oxoisoindolin-2-yl)piperidine-2,6-dione |
| | 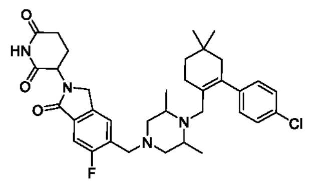 | 3-(5-((4-((4'-chloro-5,5-dimethyl-3,4,5,6-tetrahydro-[1,1'-biphenyl]-2-yl)methyl)-3,5-dimethylpiperazin-1-yl)methyl)-6-fluoro-1-oxoisoindolin-2-yl)piperidine-2,6-dione | 3-(5-((4-((4'-chloro-5,5-dimethyl-3,4,5,6-tetrahydro-[1,1'-biphenyl]-2-yl)methyl)-3,5-dimethylpiperazin-1-yl)methyl)-6-fluoro-1-oxoisoindolin-2-yl)piperidine-2,6-dione |
| GT-06101 | 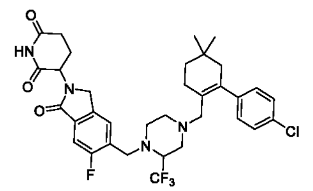 | 3-(5-((4-((4'-chloro-5,5-dimethyl-3,4,5,6-tetrahydro-[1,1'-biphenyl]-2-yl)methyl)-2-(trifluoromethyl)piperazin-1-yl)methyl)-6-fluoro-1-oxoisoindolin-2-yl)piperidine-2,6-dione | 3-(5-((4-((4'-chloro-5,5-dimethyl-3,4,5,6-tetrahydro-[1,1'-biphenyl]-2-yl)methyl)-2-(trifluoromethyl)piperazin-1-yl)methyl)-6-fluoro-1-oxoisoindolin-2-yl)piperidine-2,6-dione |
| | 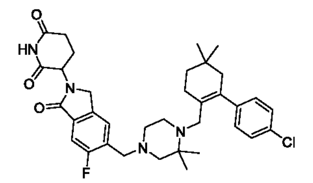 | 3-(5-((4-((4'-chloro-5,5-dimethyl-3,4,5,6-tetrahydro-[1,1'-biphenyl]-2-yl)methyl)-3,3-dimethylpiperazin-1-yl)methyl)-6-fluoro-1-oxoisoindolin-2-yl)piperidine-2,6-dione | 3-(5-((4-((4'-chloro-5,5-dimethyl-3,4,5,6-tetrahydro-[1,1'-biphenyl]-2-yl)methyl)-3,3-dimethylpiperazin-1-yl)methyl)-6-fluoro-1-oxoisoindolin-2-yl)piperidine-2,6-dione |
| GT-05383 | 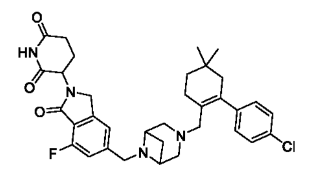 | 3-(5-((3-((4'-chloro-5,5-dimethyl-3,4,5,6-tetrahydro-[1,1'-biphenyl]-2-yl)methyl)-3,6-diazabicyclo[3.1.1]heptan-6-yl)methyl)-7-fluoro-1-oxoisoindolin-2-yl)piperidine-2,6-dione | 3-(5-((3-((4'-chloro-5,5-dimethyl-3,4,5,6-tetrahydro-[1,1'-biphenyl]-2-yl)methyl)-3,6-diazabicyclo[3.1.1]heptan-6-yl)methyl)-7-fluoro-1-oxoisoindolin-2-yl)piperidine-2,6-dione |
| GT-05531 | 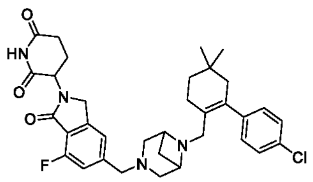 | 3-(5-((6-((4'-chloro-5,5-dimethyl-3,4,5,6-tetrahydro-[1,1'-biphenyl]-2-yl)methyl)-3,6-diazabicyclo[3.1.1]heptan-3-yl)methyl)-7-fluoro-1-oxoisoindolin-2-yl)piperidine-2,6-dione | 3-(5-((6-((4'-chloro-5,5-dimethyl-3,4,5,6-tetrahydro-[1,1'-biphenyl]-2-yl)methyl)-3,6-diazabicyclo[3.1.1]heptan-3-yl)methyl)-7-fluoro-1-oxoisoindolin-2-yl)piperidine-2,6-dione |

(continued)

| Compound No. | Structure of the compounds | The compounds' name | Chinese translation of the compounds' name |
|---|---|---|---|
| GT-05495 | 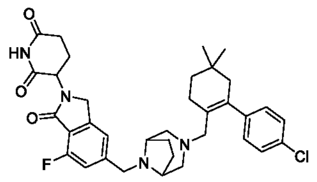 | 3-(5-((3-((4'-chloro-5,5-dimethyl-3,4,5,6-tetrahydro-[1,1'-biphenyl]-2-yl)methyl)-3,8-diazabicyclo[3.2.1]octan-8-yl)methyl)-7-fluoro-1-oxoisoindolin-2-yl)piperidine-2,6-dione | 3-(5-((3-((4'-chloro-5,5-dimethyl-3,4,5,6-tetrahydro-[1,1'-biphenyl]-2-yl)methyl)-3,8-diazabicyclo[3.2.1]octan-8-yl)methyl)-7-fluoro-1-oxoisoindolin-2-yl)piperidine-2,6-dione |
| GT-05536 | 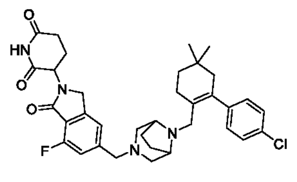 | 3-(5-((8-((4'-chloro-5,5-dimethyl-3,4,5,6-tetrahydro-[1,1'-biphenyl]-2-yl)methyl)-3,8-diazabicyclo[3.2.1]octan-3-yl)methyl)-7-fluoro-1-oxoisoindolin-2-yl)piperidine-2,6-dione | 3-(5-((8-((4'-chloro-5,5-dimethyl-3,4,5,6-tetrahydro-[1,1'-biphenyl]-2-yl)methyl)-3,8-diazabicyclo[3.2.1]octan-3-yl)methyl)-7-fluoro-1-oxoisoindolin-2-yl)piperidine-2,6-dione |
| GT-05781 | 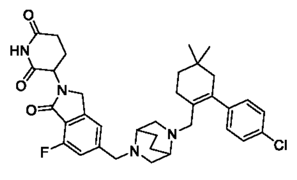 | 3-(5-((5-((4'-chloro-5,5-dimethyl-3,4,5,6-tetrahydro-[1,1'-biphenyl]-2-yl)methyl)-2,5-diazabicyclo[2.2.2]octan-2-yl)methyl)-7-fluoro-1-oxoisoindolin-2-yl)piperidine-2,6-dione | 3-(5-((5-((4'-chloro-5,5-dimethyl-3,4,5,6-tetrahydro-[1,1'-biphenyl]-2-yl)methyl)-2,5-diazabicyclo[2.2.2]octan-2-yl)methyl)-7-fluoro-1-oxoisoindolin-2-yl)piperidine-2,6-dione |
|  | 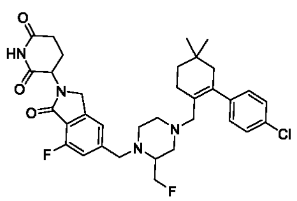 | 3-(5-((4-((4'-chloro-5,5-dimethyl-3,4,5,6-tetrahydro-[1,1'-biphenyl]-2-yl)methyl)-2-(fluoromethyl)piperazin-1-yl)methyl)-7-fluoro-1-oxoisoindolin-2-yl)piperidine-2,6-dione | 3-(5-((4-((4'-chloro-5,5-dimethyl-3,4,5,6-tetrahydro-[1,1'-biphenyl]-2-yl)methyl)-2-(fluoromethyl)piperazin-1-yl)methyl)-7-fluoro-1-oxoisoindolin-2-yl)piperidine-2,6-dione |
|  | 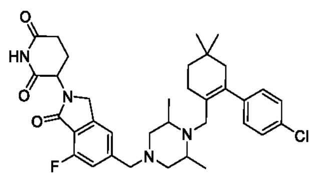 | 3-(5-((4-((4'-chloro-5,5-dimethyl-3,4,5,6-tetrahydro-[1,1'-biphenyl]-2-yl)methyl)-3,5-dimethylpiperazin-1-yl)methyl)-7-fluoro-1-oxoisoindolin-2-yl)piperidine-2,6-dione | 3-(5-((4-((4'-chloro-5,5-dimethyl-3,4,5,6-tetrahydro-[1,1'-biphenyl]-2-yl)methyl)-3,5-dimethylpiperazin-1-yl)methyl)-7-fluoro-1-oxoisoindolin-2-yl)piperidine-2,6-dione |
| GT-06102 | 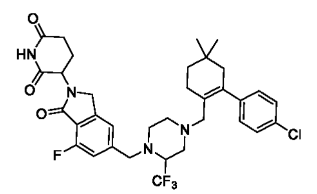 | 3-(5-((4-((4'-chloro-5,5-dimethyl-3,4,5,6-tetrahydro-[1,1'-biphenyl]-2-yl)methyl)-2-(trifluoromethyl)piperazin-1-yl)methyl)-7-fluoro-1-oxoisoindolin-2-yl)piperidine-2,6-dione | 3-(5-((4-((4'-chloro-5,5-dimethyl-3,4,5,6-tetrahydro-[1,1'-biphenyl]-2-yl)methyl)-2-(trifluoromethyl)piperazin-1-yl)methyl)-7-fluoro-1-oxoisoindolin-2-yl)piperidine-2,6-dione |
|  | 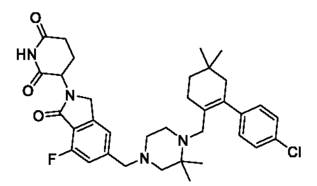 | 3-(5-((4-((4'-chloro-5,5-dimethyl-3,4,5,6-tetrahydro-[1,1'-biphenyl]-2-yl)methyl)-3,3-dimethylpiperazin-1-yl)methyl)-7-fluoro-1-oxoisoindolin-2-yl)piperidine-2,6-dione | 3-(5-((4-((4'-chloro-5,5-dimethyl-3,4,5,6-tetrahydro-[1,1'-biphenyl]-2-yl)methyl)-3,3-dimethylpiperazin-1-yl)methyl)-7-fluoro-1-oxoisoindolin-2-yl)piperidine-2,6-dione |
|  | 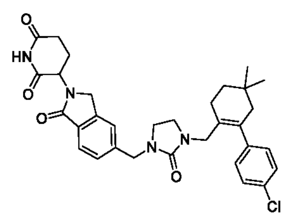 | 3-(5-((3-((4'-chloro-5,5-dimethyl-3,4,5,6-tetrahydro-[1,1'-biphenyl]-2-yl)methyl)-2-oxoimidazolidin-1-yl)methyl)-1-oxoisoindolin-2-yl)piperidine-2,6-dione | 3-(5-((3-((4'-chloro-5,5-dimethyl-3,4,5,6-tetrahydro-[1,1'-biphenyl]-2-yl)methyl)-2-oxoimidazolidin-1-yl)methyl)-1-oxoisoindolin-2-yl)piperidine-2,6-dione |

(continued)

| Compound No. | Structure of the compounds | The compounds' name | Chinese translation of the compounds' name |
|---|---|---|---|
| | 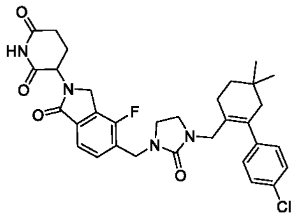 | 3-(5-((3-((4'-chloro-5,5-dimethyl-3,4,5,6-tetrahydro-[1,1'-biphenyl]-2-yl)methyl)-2-oxoimidazolidin-1-yl)methyl)-4-fluoro-1-oxoisoindolin-2-yl)piperidine-2,6-dione | 3-(5-((3-((4'-chloro-5,5-dimethyl-3,4,5,6-tetrahydro-[1,1'-biphenyl]-2-yl)methyl)-2-oxoimidazolidin-1-yl)methyl)-4-fluoro-1-oxoisoindolin-2-yl)piperidine-2,6-dione |
| | 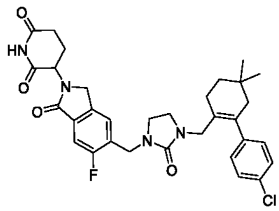 | 3-(5-((3-((4'-chloro-5,5-dimethyl-3,4,5,6-tetrahydro-[1,1'-biphenyl]-2-yl)methyl)-2-oxoimidazolidin-1-yl)methyl)-6-fluoro-1-oxoisoindolin-2-yl)piperidine-2,6-dione | 3-(5-((3-((4'-chloro-5,5-dimethyl-3,4,5,6-tetrahydro-[1,1'-biphenyl]-2-yl)methyl)-2-oxoimidazolidin-1-yl)methyl)-6-fluoro-1-oxoisoindolin-2-yl)piperidine-2,6-dione |
| | 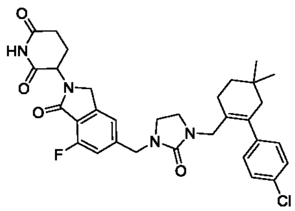 | 3-(5-((3-((4'-chloro-5,5-dimethyl-3,4,5,6-tetrahydro-[1,1'-biphenyl]-2-yl)methyl)-2-oxoimidazolidin-1-yl)methyl)-7-fluoro-1-oxoisoindolin-2-yl)piperidine-2,6-dione | 3-(5-((3-((4'-chloro-5,5-dimethyl-3,4,5,6-tetrahydro-[1,1'-biphenyl]-2-yl)methyl)-2-oxoimidazolidin-1-yl)methyl)-7-fluoro-1-oxoisoindolin-2-yl)piperidine-2,6-dione |
| | 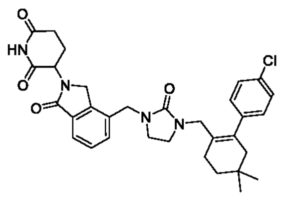 | 3-(4-((3-((4'-chloro-5,5-dimethyl-3,4,5,6-tetrahydro-[1,1'-biphenyl]-2-yl)methyl)-2-oxoimidazolidin-1-yl)methyl)-1-oxoisoindolin-2-yl)piperidine-2,6-dione | 3-(4-((3-((4'-chloro-5,5-dimethyl-3,4,5,6-tetrahydro-[1,1'-biphenyl]-2-yl)methyl)-2-oxoimidazolidin-1-yl)methyl)-1-oxoisoindolin-2-yl)piperidine-2,6-dione |
| GT-05672 | 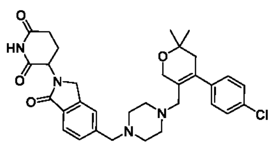 | 3-(5-((4-((4-(4-chlorophenyl)-6,6-dimethyl-5,6-dihydro-2H-pyran-3-yl)methyl)piperazin-1-yl)methyl)-1-oxoisoindolin-2-yl)piperidine-2,6-dione | 3-(5-((4-((4-(4-chlorophenyl)-6,6-dimethyl-5,6-dihydro-2H-pyran-3-yl)methyl)piperazin-1-yl)methyl)-1-oxoisoindolin-2-yl)piperidine-2,6-dione |
| GT-04224 | 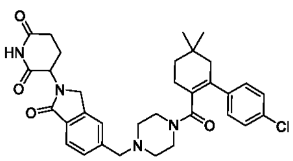 | 3-(5-((4-(4'-chloro-5,5-dimethyl-3,4,5,6-tetrahydro-[1,1'-biphenyl]-2-carbonyl)piperazin-1-yl)methyl)-1-oxoisoindolin-2-yl)piperidine-2,6-dione | 3-(5-((4-(4'-chloro-5,5-dimethyl-3,4,5,6-tetrahydro-[1,1'-biphenyl]-2-carbonyl)piperazin-1-yl)methyl)-1-oxoisoindolin-2-yl)piperidine-2,6-dione |
| GT-05525 | 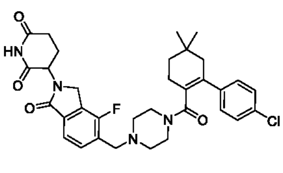 | 3-(5-((4-(4'-chloro-5,5-dimethyl-3,4,5,6-tetrahydro-[1,1'-biphenyl]-2-carbonyl)piperazin-1-yl)methyl)-4-fluoro-1-oxoisoindolin-2-yl)piperidine-2,6-dione | 3-(5-((4-(4'-chloro-5,5-dimethyl-3,4,5,6-tetrahydro-[1,1'-biphenyl]-2-carbonyl)piperazin-1-yl)methyl)-4-fluoro-1-oxoisoindolin-2-yl)piperidine-2,6-dione |
| GT-05526 | 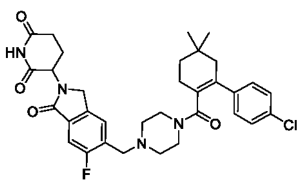 | 3-(5-((4-(4'-chloro-5,5-dimethyl-3,4,5,6-tetrahydro-[1,1'-biphenyl]-2-carbonyl)piperazin-1-yl)methyl)-6-fluoro-1-oxoisoindolin-2-yl)piperidine-2,6-dione | 3-(5-((4-(4'-chloro-5,5-dimethyl-3,4,5,6-tetrahydro-[1,1'-biphenyl]-2-carbonyl)piperazin-1-yl)methyl)-6-fluoro-1-oxoisoindolin-2-yl)piperidine-2,6-dione |

(continued)

| Compound No. | Structure of the compounds | The compounds' name | Chinese translation of the compounds' name |
|---|---|---|---|
| GT-05527 | 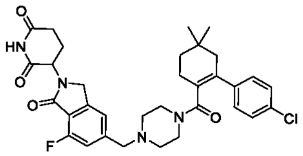 | 3-(5-((4-(4'-chloro-5,5-dimethyl-3,4,5,6-tetrahydro-[1,1'-biphenyl]-2-carbonyl)piperazin-1-yl)methyl)-7-fluoro-1-oxoisoindolin-2-yl)piperidine-2,6-dione | 3-(5-((4-(4'-chloro-5,5-dimethyl-3,4,5,6-tetrahydro-[1,1'-biphenyl]-2-carbonyl)piperazin-1-yl)methyl)-7-fluoro-1-oxoisoindolin-2-yl)piperidine-2,6-dione |
| GT-05528 | 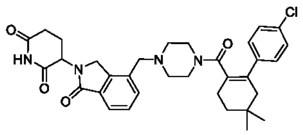 | 3-(4-((4-(4'-chloro-5,5-dimethyl-3,4,5,6-tetrahydro-[1,1'-biphenyl]-2-carbonyl)piperazin-1-yl)methyl)-1-oxoisoindolin-2-yl)piperidine-2,6-dione | 3-(4-((4-(4'-chloro-5,5-dimethyl-3,4,5,6-tetrahydro-[1,1'-biphenyl]-2-carbonyl)piperazin-1-yl)methyl)-1-oxoisoindolin-2-yl)piperidine-2,6-dione |
| GT-05417 | 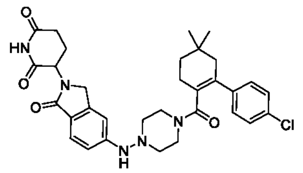 | 3-(5-((4-(4'-chloro-5,5-dimethyl-3,4,5,6-tetrahydro-[1,1'-biphenyl]-2-carbonyl)piperazin-1-yl)amino)-1-oxoisoindolin-2-yl)piperidine-2,6-dione | 3-(5-((4-(4'-chloro-5,5-dimethyl-3,4,5,6-tetrahydro-[1,1'-biphenyl]-2-carbonyl)piperazin-1-yl)amino)-1-oxoisoindolin-2-yl)piperidine-2,6-dione |
| GT-05418 | 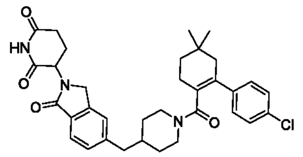 | 3-(5-((1-(4'-chloro-5,5-dimethyl-3,4,5,6-tetrahydro-[1,1'-biphenyl]-2-carbonyl)piperidin-4-yl)methyl)-1-oxoisoindolin-2-yl)piperidine-2,6-dione | 3-(5-((1-(4'-chloro-5,5-dimethyl-3,4,5,6-tetrahydro-[1,1'-biphenyl]-2-carbonyl)piperidin-4-yl)methyl)-1-oxoisoindolin-2-yl)piperidine-2,6-dione |
| GT-05783 | 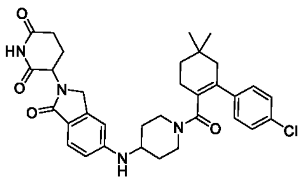 | 3-(5-((1-(4'-chloro-5,5-dimethyl-3,4,5,6-tetrahydro-[1,1'-biphenyl]-2-carbonyl)piperidin-4-yl)amino)-1-oxoisoindolin-2-yl)piperidine-2,6-dione | 3-(5-((1-(4'-chloro-5,5-dimethyl-3,4,5,6-tetrahydro-[1,1'-biphenyl]-2-carbonyl)piperidin-4-yl)amino)-1-oxoisoindolin-2-yl)piperidine-2,6-dione |
| GT-05419 | 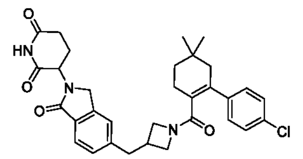 | 3-(5-((1-(4'-chloro-5,5-dimethyl-3,4,5,6-tetrahydro-[1,1'-biphenyl]-2-carbonyl)azetidin-3-yl)methyl)-1-oxoisoindolin-2-yl)piperidine-2,6-dione | 3-(5-((1-(4'-chloro-5,5-dimethyl-3,4,5,6-tetrahydro-[1,1'-biphenyl]-2-carbonyl)azetidin-3-yl)methyl)-1-oxoisoindolin-2-yl) piperidine-2,6-dione |
| GT-05420 | 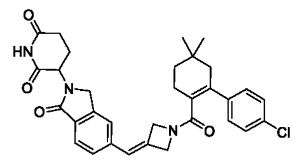 | 3-(5-((1-(4'-chloro-5,5-dimethyl-3,4,5,6-tetrahydro-[1,1'-biphenyl]-2-carbonyl)azetidin-3-ylidene)methyl)-1-oxoisoindolin-2-yl)piperidine-2,6-dione | 3-(5-((1-(4'-chloro-5,5-dimethyl-3,4,5,6-tetrahydro-[1,1'-biphenyl]-2-carbonyl)azetidin-3-ylidene)methyl)-1-oxoisoindolin-2-yl)piperidine-2,6-dione |
| GT-05426 | 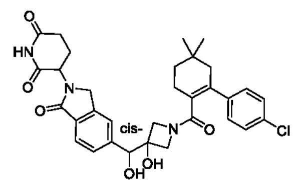 | 3-(5-((1-(4'-chloro-5,5-dimethyl-3,4,5,6-tetrahydro-[1,1'-biphenyl]-2-carbonyl)-3-hydroxyazetidin-3-yl)(hydroxy)methyl)-1-oxoisoindolin-2-yl)piperidine-2,6-dione | 3-(5-((1-(4'-chloro-5,5-dimethyl-3,4,5,6-tetrahydro-[1,1'-biphenyl]-2-carbonyl)-3-hydroxyazetidin-3-yl)(hydroxy)methyl)-1-oxoisoindolin-2-yl)piperidine-2,6-dione |

(continued)

| Compound No. | Structure of the compounds | The compounds' name | Chinese translation of the compounds' name |
|---|---|---|---|
| | 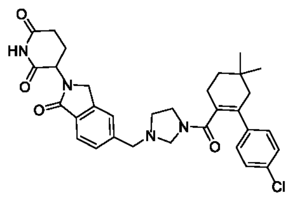 | 3-(5-((3-(4'-chloro-5,5-dimethyl-3,4,5,6-tetrahydro-[1,1'-biphenyl]-2-carbonyl)imidazolidin-1-yl)methyl)-1-oxoisoindolin-2-yl)piperidine-2,6-dione | 3-(5-((3-(4'-chloro-5,5-dimethyl-3,4,5,6-tetrahydro-[1,1'-biphenyl]-2-carbonyl)imidazolidin-1-yl)methyl)-1-oxoisoindolin-2-yl)piperidine-2,6-dione |
| GT-05680 | 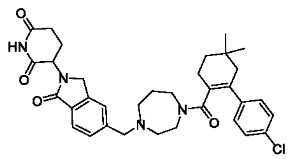 | 3-(5-((4-(4'-chloro-5,5-dimethyl-3,4,5,6-tetrahydro-[1,1'-biphenyl]-2-carbonyl)-1,4-diazepan-1-yl)methyl)-1-oxoisoindolin-2-yl)piperidine-2,6-dione | 3-(5-((4-(4'-chloro-5,5-dimethyl-3,4,5,6-tetrahydro-[1,1'-biphenyl]-2-carbonyl)-1,4-diazepan-1-yl)methyl)-1-oxoisoindolin-2-yl)piperidine-2,6-dione |
| GT-05656 | 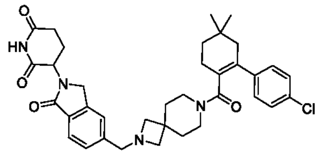 | 3-(5-((7-(4'-chloro-5,5-dimethyl-3,4,5,6-tetrahydro-[1,1'-biphenyl]-2-carbonyl)-2,7-diazaspiro[3.5]nonan-2-yl)methyl)-1-oxoisoindolin-2-yl)piperidine-2,6-dione | 3-(5-((7-(4'-chloro-5,5-dimethyl-3,4,5,6-tetrahydro-[1,1'-biphenyl]-2-carbonyl)-2,7-diazaspiro[3.5]nonan-2-yl)methyl)-1-oxoisoindolin-2-yl)piperidine-2,6-dione |
| GT-05681 | 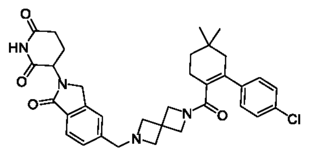 | 3-(5-((6-(4'-chloro-5,5-dimethyl-3,4,5,6-tetrahydro-[1,1'-biphenyl]-2-carbonyl)-2,6-diazaspiro[3.3]heptan-2-yl)methyl)-1-oxoisoindolin-2-yl)piperidine-2,6-dione | 3-(5-((6-(4'-chloro-5,5-dimethyl-3,4,5,6-tetrahydro-[1,1'-biphenyl]-2-carbonyl)-2,6-diazaspiro[3.3]heptan-2-yl)methyl)-1-oxoisoindolin-2-yl)piperidine-2,6-dione |
| GT-05658 | 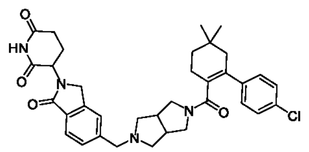 | 3-(5-((5-(4'-chloro-5,5-dimethyl-3,4,5,6-tetrahydro-[1,1'-biphenyl]-2-carbonyl)hexahydropyrrolo[ 3,4-c]pyrrol-2(1H)-yl)methyl)-1-oxoisoindolin-2-yl)piperidine-2,6-dione | 3-(5-((5-(4'-chloro-5,5-dimethyl-3,4,5,6-tetrahydro-[1,1'-biphenyl]-2-carbonyl)hexahydropyrrolo[3 ,4-c]pyrrol-2(1H)-yl)methyl)-1-oxoisoindolin-2-yl)piperidine-2,6-dione |
| GT-05359 | 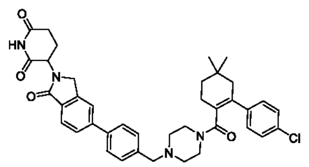 | 3-(5-(4-((4-(4'-chloro-5,5-dimethyl-3,4,5,6-tetrahydro-[1,1'-biphenyl]-2-carbonyl)piperazin-1-yl)methyl)phenyl)-1-oxoisoindolin-2-yl)piperidine-2,6-dione | 3-(5-(4-((4-(4'-chloro-5,5-dimethyl-3,4,5,6-tetrahydro-[1,1'-biphenyl]-2-carbonyl)piperazin-1-yl)methyl)phenyl)-1-oxoisoindolin-2-yl)piperidine-2,6-dione |
| GT-05521 | 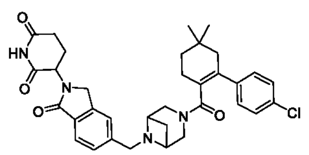 | 3-(5-((3-(4'-chloro-5,5-dimethyl-3,4,5,6-tetrahydro-[1,1'-biphenyl]-2-carbonyl)-3,6-diazabicyclo[3.1.1]heptan-6-yl)methyl)-1-oxoisoindolin-2-yl)piperidine-2,6-dione | 3-(5-((3-(4'-chloro-5,5-dimethyl-3,4,5,6-tetrahydro-[1,1'-biphenyl]-2-carbonyl)-3,6-diazabicyclo[3.1.1]heptan-6-yl)methyl)-1-oxoisoindolin-2-yl)piperidine-2,6-dione |
| GT-05660 | 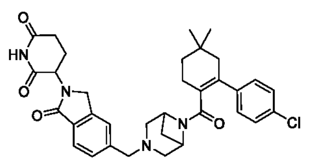 | 3-(5-((6-(4'-chloro-5,5-dimethyl-3,4,5,6-tetrahydro-[1,1'-biphenyl]-2-carbonyl)-3,6-diazabicyclo[3.1.1]heptan-3-yl)methyl)-1-oxoisoindolin-2-yl)piperidine-2,6-dione | 3-(5-((6-(4'-chloro-5,5-dimethyl-3,4,5,6-tetrahydro-[1,1'-biphenyl]-2-carbonyl)-3,6-diazabicyclo[3.1.1]heptan-3-yl)methyl)-1-oxoisoindolin-2-yl)piperidine-2,6-dione |

(continued)

| Compound No. | Structure of the compounds | The compounds' name | Chinese translation of the compounds' name |
|---|---|---|---|
| GT-05522 | 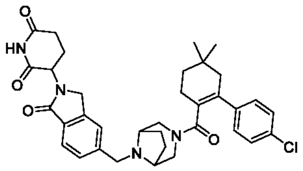 | 3-(5-((3-(4'-chloro-5,5-dimethyl-3,4,5,6-tetrahydro-[1,1'-biphenyl]-2-carbonyl)-3,8-diazabicyclo[3.2.1]octan-8-yl)methyl)-1-oxoisoindolin-2-yl)piperidine-2,6-dione | 3-(5-((3-(4'-chloro-5,5-dimethyl-3,4,5,6-tetrahydro-[1,1'-biphenyl]-2-carbonyl)-3,8-diazabicyclo[3.2.1]octan-8-yl)methyl)-1-oxoisoindolin-2-yl)piperidine-2,6-dione |
| GT-05523 | 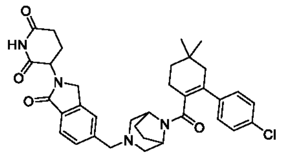 | 3-(5-((8-(4'-chloro-5,5-dimethyl-3,4,5,6-tetrahydro-[1,1'-biphenyl]-2-carbonyl)-3,8-diazabicyclo[3.2.1]octan-3-yl)methyl)-1-oxoisoindolin-2-yl)piperidine-2,6-dione | 3-(5-((8-(4'-chloro-5,5-dimethyl-3,4,5,6-tetrahydro-[1,1'-biphenyl]-2-carbonyl)-3,8-diazabicyclo[3.2.1]octan-3-yl)methyl)-1-oxoisoindolin-2-yl)piperidine-2,6-dione |
| GT-05662 | 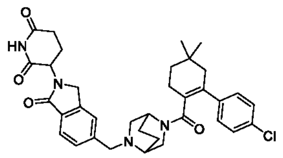 | 3-(5-((5-(4'-chloro-5,5-dimethyl-3,4,5,6-tetrahydro-[1,1'-biphenyl]-2-carbonyl)-2,5-diazabicyclo[2.2.2]octan-2-yl)methyl)-1-oxoisoindolin-2-yl)piperidine-2,6-dione | 3-(5-((5-(4'-chloro-5,5-dimethyl-3,4,5,6-tetrahydro-[1,1'-biphenyl]-2-carbonyl)-2,5-diazabicyclo[2.2.2]octan-2-yl)methyl)-1-oxoisoindolin-2-yl)piperidine-2,6-dione |
| | 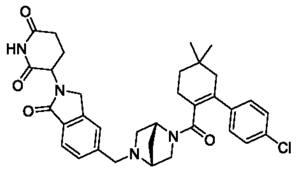 | 3-(5-(((1R,4R)-5-(4'-chloro-5,5-dimethyl-3,4,5,6-tetrahydro-[1,1'-biphenyl]-2-carbonyl)-2,5-diazabicyclo[2.2.1]heptan-2-yl)methyl)-1-oxoisoindolin-2-yl)piperidine-2,6-dione | 3-(5-(((1R,4R)-5-(4'-chloro-5,5-dimethyl-3,4,5,6-tetrahydro-[1,1'-biphenyl]-2-carbonyl)-2,5-diazabicyclo[2.2.1]heptan-2-yl)methyl)-1-oxoisoindolin-2-yl)piperidine-2,6-dione |
| | 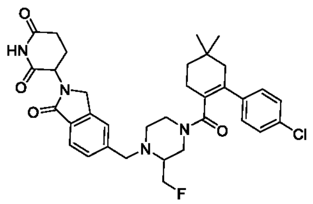 | 3-(5-((4-(4'-chloro-5,5-dimethyl-3,4,5,6-tetrahydro-[1,1'-biphenyl]-2-carbonyl)-2-(fluoromethyl)piperazin-1-yl)methyl)-1-oxoisoindolin-2-yl)piperidine-2,6-dione | 3-(5-((4-(4'-chloro-5,5-dimethyl-3,4,5,6-tetrahydro-[1,1'-biphenyl]-2-carbonyl)-2-(fluoromethyl)piperazin-1-yl)methyl)-1-oxoisoindolin-2-yl)piperidine-2,6-dione |
| | 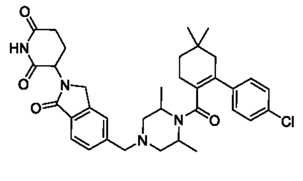 | 3-(5-((4-(4'-chloro-5,5-dimethyl-3,4,5,6-tetrahydro-[1,1'-biphenyl]-2-carbonyl)-3,5-dimethylpiperazin-1-yl)methyl)-1-oxoisoindolin-2-yl)piperidine-2,6-dione | 3-(5-((4-(4'-chloro-5,5-dimethyl-3,4,5,6-tetrahydro-[1,1'-biphenyl]-2-carbonyl)-3,5-dimethylpiperazin-1-yl)methyl)-1-oxoisoindolin-2-yl)piperidine-2,6-dione |
| GT-05845 | 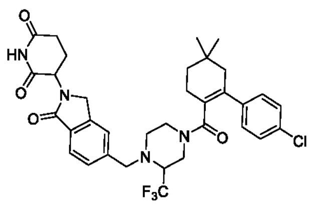 | 3-(5-((4-(4'-chloro-5,5-dimethyl-3,4,5,6-tetrahydro-[1,1'-biphenyl]-2-carbonyl)-2-(trifluoromethyl)piperazin-1-yl)methyl)-1-oxoisoindolin-2-yl)piperidine-2,6-dione | 3-(5-((4-(4'-chloro-5,5-dimethyl-3,4,5,6-tetrahydro-[1,1'-biphenyl]-2-carbonyl)-2-(trifluoromethyl)piperazin-1-yl)methyl)-1-oxoisoindolin-2-yl)piperidine-2,6-dione |
| | 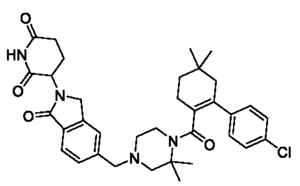 | 3-(5-((4-(4'-chloro-5,5-dimethyl-3,4,5,6-tetrahydro-[1,1'-biphenyl]-2-carbonyl)-3,3-dimethylpiperazin-1-yl)methyl)-1-oxoisoindolin-2-yl)piperidine-2,6-dione | 3-(5-((4-(4'-chloro-5,5-dimethyl-3,4,5,6-tetrahydro-[1,1'-biphenyl]-2-carbonyl)-3,3-dimethylpiperazin-1-yl)methyl)-1-oxoisoindolin-2-yl)piperidine-2,6-dione |

(continued)

| Compo und No. | Structure of the compounds | The compounds' name | Chinese translation of the compounds' name |
|---|---|---|---|
| GT-05445 | 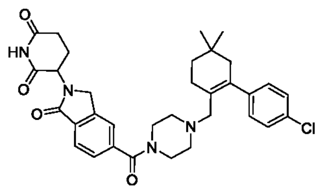 | 3-(5-(4-((4'-chloro-5,5-dimethyl-3,4,5,6-tetrahydro-[1,1'-biphenyl]-2-yl)methyl)piperazine-1-carbonyl)-1-oxoisoindolin-2-yl)piperidine-2,6-dione | 3-(5-(4-((4'-chloro-5,5-dimethyl-3,4,5,6-tetrahydro-[1,1'-biphenyl]-2-yl)methyl)piperazine-1-carbonyl)-1-oxoisoindolin-2-yl)piperidine-2,6-dione |
| | 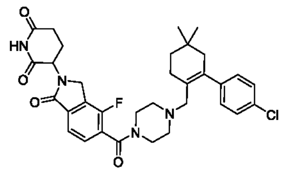 | 3-(5-(4-((4'-chloro-5,5-dimethyl-3,4,5,6-tetrahydro-[1,1'-biphenyl]-2-yl)methyl)piperazine-1-carbonyl)-4-fluoro-1-oxoisoindolin-2-yl)piperidine-2,6-dione | 3-(5-(4-((4'-chloro-5,5-dimethyl-3,4,5,6-tetrahydro-[1,1'-biphenyl]-2-yl)methyl)piperazine-1-carbonyl)-4-fluoro-1-oxoisoindolin-2-yl)piperidine-2,6-dione |
| | 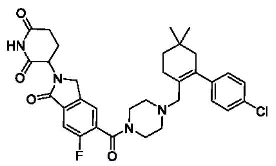 | 3-(5-(4-((4'-chloro-5,5-dimethyl-3,4,5,6-tetrahydro-[1,1'-biphenyl]-2-yl)methyl)piperazine-1-carbonyl)-6-fluoro-1-oxoisoindolin-2-yl)piperidine-2,6-dione | 3-(5-(4-((4'-chloro-5,5-dimethyl-3,4,5,6-tetrahydro-[1,1'-biphenyl]-2-yl)methyl)piperazine-1-carbonyl)-6-fluoro-1-oxoisoindolin-2-yl)piperidine-2,6-dione |
| | 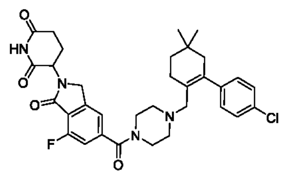 | 3-(5-(4-((4'-chloro-5,5-dimethyl-3,4,5,6-tetrahydro-[1,1'-biphenyl]-2-yl)methyl)piperazine-1-carbonyl)-7-fluoro-1-oxoisoindolin-2-yl)piperidine-2,6-dione | 3-(5-(4-((4'-chloro-5,5-dimethyl-3,4,5,6-tetrahydro-[1,1'-biphenyl]-2-yl)methyl)piperazine-1-carbonyl)-7-fluoro-1-oxoisoindolin-2-yl)piperidine-2,6-dione |
| | 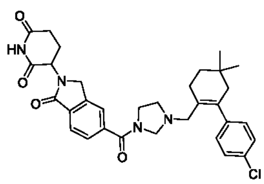 | 3-(5-(3-((4'-chloro-5,5-dimethyl-3,4,5,6-tetrahydro-[1,1'-biphenyl]-2-yl)methyl)imidazolidine-1-carbonyl)-1-oxoisoindolin-2-yl)piperidine-2,6-dione | 3-(5-(3-((4'-chloro-5,5-dimethyl-3,4,5,6-tetrahydro-[1,1'-biphenyl]-2-yl)methyl)imidazolidine-1-carbonyl)-1-oxoisoindolin-2-yl)piperidine-2,6-dione |
| GT-05453 | 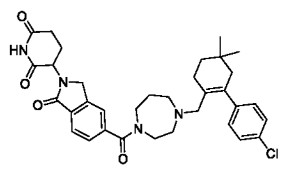 | 3-(5-(4-((4'-chloro-5,5-dimethyl-3,4,5,6-tetrahydro-[1,1'-biphenyl]-2-yl)methyl)-1,4-diazepane-1-carbonyl)-1-oxoisoindolin-2-yl)piperidine-2,6-dione | 3-(5-(4-((4'-chloro-5,5-dimethyl-3,4,5,6-tetrahydro-[1,1'-biphenyl]-2-yl)methyl)-1,4-diazepane-1-carbonyl)-1-oxoisoindolin-2-yl)piperidine-2,6-dione |
| | 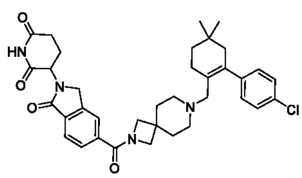 | 3-(5-(7-((4'-chloro-5,5-dimethyl-3,4,5,6-tetrahydro-[1,1'-biphenyl]-2-yl)methyl)-2,7-diazaspiro[3.5]nonane-2-carbonyl)-1-oxoisoindolin-2-yl)piperidine-2,6-dione | 3-(5-(7-((4'-chloro-5,5-dimethyl-3,4,5,6-tetrahydro-[1,1'-biphenyl]-2-yl)methyl)-2,7-diazaspiro[3.5]nonane-2-carbonyl)-1-oxoisoindolin-2-yl)piperidine-2,6-dione |
| | 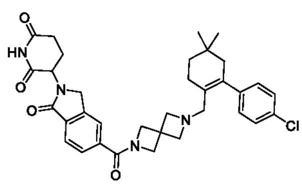 | 3-(5-(6-((4'-chloro-5,5-dimethyl-3,4,5,6-tetrahydro-[1,1'-biphenyl]-2-yl)methyl)-2,6-diazaspiro[3.3]heptane-2-carbonyl)-1-oxoisoindolin-2-yl)piperidine-2,6-dione | 3-(5-(6-((4'-chloro-5,5-dimethyl-3,4,5,6-tetrahydro-[1,1'-biphenyl]-2-yl)methyl)-2,6-diazaspiro[3.3]heptane-2-carbonyl)-1-oxoisoindolin-2-yl)piperidine-2,6-dione |

(continued)

| Compo und No. | Structure of the compounds | The compounds' name | Chinese translation of the compounds' name |
|---|---|---|---|
| | 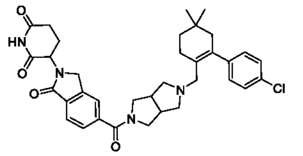 | 3-(5-(5-((4'-chloro-5,5-dimethyl-3,4,5,6-tetrahydro-[1,1'-biphenyl]-2-yl)methyl)octahydropyrrolo[ 3,4-c]pyrrole-2-carbonyl)-1-oxoisoindolin-2-yl)piperidine-2,6-dione | 3-(5-(5-((4'-chloro-5,5-dimethyl-3,4,5,6-tetrahydro-[1,1'-biphenyl]-2-yl)methyl)octahydropyrrolo[3 ,4-c]pyrrole-2-carbonyl)-1-oxoisoindolin-2-yl)piperidine-2,6-dione |
| GT-05440 | 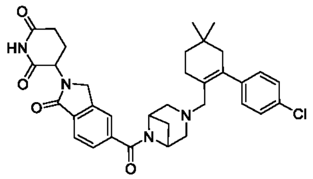 | 3-(5-(3-((4'-chloro-5,5-dimethyl-3,4,5,6-tetrahydro-[1,1'-biphenyl]-2-yl)methyl)-3,6-diazabicyclo[3.1.1]heptane-6-carbonyl)-1-oxoisoindolin-2-yl)piperidine-2,6-dione | 3-(5-(3-((4'-chloro-5,5-dimethyl-3,4,5,6-tetrahydro-[1,1'-biphenyl]-2-yl)methyl)-3,6-diazabicyclo[3.1.1]heptane-6-carbonyl)-1-oxoisoindolin-2-yl)piperidine-2,6-dione |
| GT-05457 | 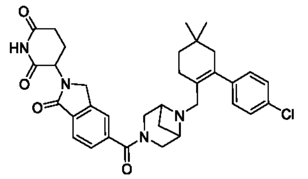 | 3-(5-(6-((4'-chloro-5,5-dimethyl-3,4,5,6-tetrahydro-[1,1'-biphenyl]-2-yl)methyl)-3,6-diazabicyclo[3.1.1]heptane-3-carbonyl)-1-oxoisoindolin-2-yl)piperidine-2,6-dione | 3-(5-(6-((4'-chloro-5,5-dimethyl-3,4,5,6-tetrahydro-[1,1'-biphenyl]-2-yl)methyl)-3,6-diazabicyclo[3.1.1]heptane-3-carbonyl)-1-oxoisoindolin-2-yl)piperidine-2,6-dione |
| GT-05458 | 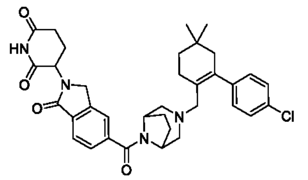 | 3-(5-(3-((4'-chloro-5,5-dimethyl-3,4,5,6-tetrahydro-[1,1'-biphenyl]-2-yl)methyl)-3,8-diazabicyclo[3.2.1]octane-8-carbonyl)-1-oxoisoindolin-2-yl)piperidine-2,6-dione | 3-(5-(3-((4'-chloro-5,5-dimethyl-3,4,5,6-tetrahydro-[1,1'-biphenyl]-2-yl)methyl)-3,8-diazabicyclo[3.2.1]octane-8-carbonyl)-1-oxoisoindolin-2-yl)piperidine-2,6-dione |
| GT-05446 | 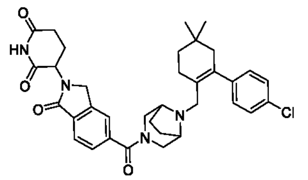 | 3-(5-(8-((4'-chloro-5,5-dimethyl-3,4,5,6-tetrahydro-[1,1'-biphenyl]-2-yl)methyl)-3,8-diazabicyclo[3.2.1]octane-3-carbonyl)-1-oxoisoindolin-2-yl)piperidine-2,6-dione | 3-(5-(8-((4'-chloro-5,5-dimethyl-3,4,5,6-tetrahydro-[1,1'-biphenyl]-2-yl)methyl)-3,8-diazabicyclo[3.2.1]octane-3-carbonyl)-1-oxoisoindolin-2-yl)piperidine-2,6-dione |
| GT-05795 | 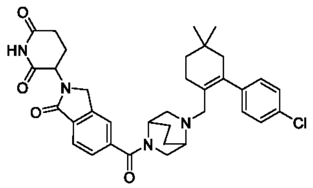 | 3-(5-(5-((4'-chloro-5,5-dimethyl-3,4,5,6-tetrahydro-[1,1'-biphenyl]-2-yl)methyl)-2,5-diazabicyclo[2.2.2]octane-2-carbonyl)-1-oxoisoindolin-2-yl)piperidine-2,6-dione | 3-(5-(5-((4'-chloro-5,5-dimethyl-3,4,5,6-tetrahydro-[1,1'-biphenyl]-2-yl)methyl)-2,5-diazabicyclo[2.2.2]octane-2-carbonyl)-1-oxoisoindolin-2-yl)piperidine-2,6-dione |
| GT-05441 | 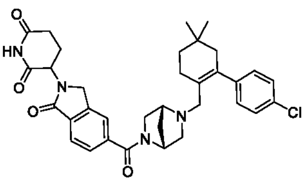 | 3-(5-((1R,4R)-5-((4'-chloro-5,5-dimethyl-3,4,5,6-tetrahydro-[1,1'-biphenyl]-2-yl) methyl)-2,5-diazabicyclo[2.2.1] heptane-2-carbonyl)-1-oxoisoindolin-2-yl)piperidine-2,6-dione | 3-(5-((1R,4R)-5-((4'-chloro-5,5-dimethyl-3,4,5,6-tetrahydro-[1,1'-biphenyl]-2-yl) methyl)-2,5-diazabicyclo[2.2.1] heptane-2-carbonyl)-1-oxoisoindolin-2-yl)piperidine-2,6-dione |
| | 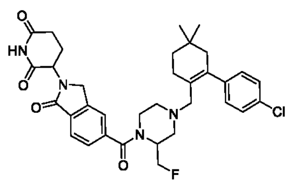 | 3-(5-(4-((4'-chloro-5,5-dimethyl-3,4,5,6-tetrahydro-[1,1'-biphenyl]-2-yl)methyl)-2-(fluoromethyl)piperazine-1-carbonyl)-1-oxoisoindolin-2-yl)piperidine-2,6-dione | 3-(5-(4-((4'-chloro-5,5-dimethyl-3,4,5,6-tetrahydro-[1,1'-biphenyl]-2-yl)methyl)-2-(fluoromethyl)piperazine-1-carbonyl)-1-oxoisoindolin-2-yl)piperidine-2,6-dione |

(continued)

| Compo und No. | Structure of the compounds | The compounds' name | Chinese translation of the compounds' name |
|---|---|---|---|
| | 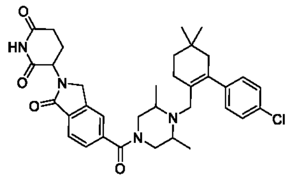 | 3-(5-(4-((4'-chloro-5,5-dimethyl-3,4,5,6-tetrahydro-[1,1'-biphenyl]-2-yl)methyl)-3,5-dimethylpiperazine-1-carbonyl)-1-oxoisoindolin-2-yl)piperidine-2,6-dione | 3-(5-(4-((4'-chloro-5,5-dimethyl-3,4,5,6-tetrahydro-[1,1'-biphenyl]-2-yl)methyl)-3,5-dimethylpiperazine-1-carbonyl)-1-oxoisoindolin-2-yl)piperidine-2,6-dione |
| GT-05706 | 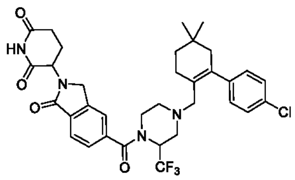 | 3-(5-(4-((4'-chloro-5,5-dimethyl-3,4,5,6-tetrahydro-[1,1'-biphenyl]-2-yl)methyl)-2-(trifluoromethyl)piperazine-1-carbonyl)-1-oxoisoindolin-2-yl)piperidine-2,6-dione | 3-(5-(4-((4'-chloro-5,5-dimethyl-3,4,5,6-tetrahydro-[1,1'-biphenyl]-2-yl)methyl)-2-(trifluoromethyl)piperazine-1-carbonyl)-1-oxoisoindolin-2-yl)piperidine-2,6-dione |
| | 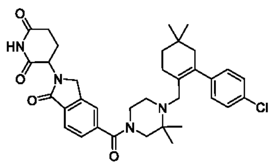 | 3-(5-(4-((4'-chloro-5,5-dimethyl-3,4,5,6-tetrahydro-[1,1'-biphenyl]-2-yl)methyl)-3,3-dimethylpiperazine-1-carbonyl)-1-oxoisoindolin-2-yl)piperidine-2,6-dione | 3-(5-(4-((4'-chloro-5,5-dimethyl-3,4,5,6-tetrahydro-[1,1'-biphenyl]-2-yl)methyl)-3,3-dimethylpiperazine-1-carbonyl)-1-oxoisoindolin-2-yl)piperidine-2,6-dione |
| | 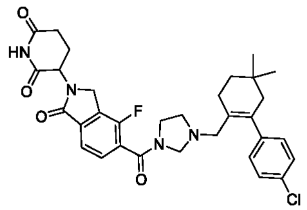 | 3-(5-(3-((4'-chloro-5,5-dimethyl-3,4,5,6-tetrahydro-[1,1'-biphenyl]-2-yl)methyl)imidazolidine-1-carbonyl)-4-fluoro-1-oxoisoindolin-2-yl)piperidine-2,6-dione | 3-(5-(3-((4'-chloro-5,5-dimethyl-3,4,5,6-tetrahydro-[1,1'-biphenyl]-2-yl)methyl)imidazolidine-1-carbonyl)-4-fluoro-1-oxoisoindolin-2-yl)piperidine-2,6-dione |
| | 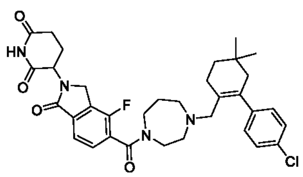 | 3-(5-(4-((4'-chloro-5,5-dimethyl-3,4,5,6-tetrahydro-[1,1'-biphenyl]-2-yl)methyl)-1,4-diazepane-1-carbonyl)-4-fluoro-1-oxoisoindolin-2-yl)piperidine-2,6-dione | 3-(5-(4-((4'-chloro-5,5-dimethyl-3,4,5,6-tetrahydro-[1,1'-biphenyl]-2-yl)methyl)-1,4-diazepane-1-carbonyl)-4-fluoro-1-oxoisoindolin-2-yl)piperidine-2,6-dione |
| | 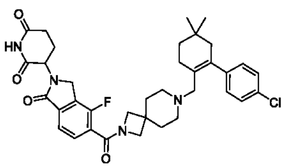 | 3-(5-(7-((4'-chloro-5,5-dimethyl-3,4,5,6-tetrahydro-[1,1'-biphenyl]-2-yl)methyl)-2,7-diazaspiro[3.5]nonane-2-carbonyl)-4-fluoro-1-oxoisoindolin-2-yl)piperidine-2,6-dione | 3-(5-(7-((4'-chloro-5,5-dimethyl-3,4,5,6-tetrahydro-[1,1'-biphenyl]-2-yl)methyl)-2,7-diazaspiro[3.5]nonane-2-carbonyl)-4-fluoro-1-oxoisoindolin-2-yl)piperidine-2,6-dione |
| | 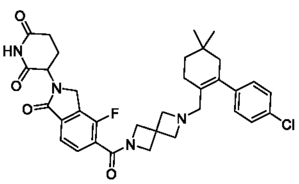 | 3-(5-(6-((4'-chloro-5,5-dimethyl-3,4,5,6-tetrahydro-[1,1'-biphenyl]-2-yl)methyl)-2,6-diazaspiro[3.3]heptane-2-carbonyl)-4-fluoro-1-oxoisoindolin-2-yl)piperidine-2,6-dione | 3-(5-(6-((4'-chloro-5,5-dimethyl-3,4,5,6-tetrahydro-[1,1'-biphenyl]-2-yl)methyl)-2,6-diazaspiro[3.3]heptane-2-carbonyl)-4-fluoro-1-oxoisoindolin-2-yl)piperidine-2,6-dione |
| | 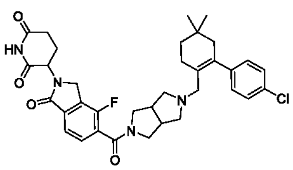 | 3-(5-(5-((4'-chloro-5,5-dimethyl-3,4,5,6-tetrahydro-[1,1'-biphenyl]-2-yl)methyl)octahydropyrrolo[ 3,4-c]pyrrole-2-carbonyl)-4-fluoro-1-oxoisoindolin-2-yl)piperidine-2,6-dione | 3-(5-(5-((4'-chloro-5,5-dimethyl-3,4,5,6-tetrahydro-[1,1'-biphenyl]-2-yl)methyl)octahydropyrrolo[3 ,4-c]pyrrole-2-carbonyl)-4-fluoro-1-oxoisoindolin-2-yl)piperidine-2,6-dione |

(continued)

| Compound No. | Structure of the compounds | The compounds' name | Chinese translation of the compounds' name |
|---|---|---|---|
| | 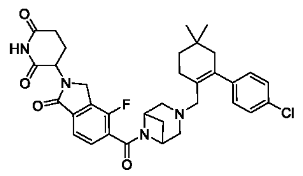 | 3-(5-(3-((4'-chloro-5,5-dimethyl-3,4,5,6-tetrahydro-[1,1'-biphenyl]-2-yl)methyl)-3,6-diazabicyclo[3.1.1]heptane-6-carbonyl)-4-fluoro-1-oxoisoindolin-2-yl)piperidine-2,6-dione | 3-(5-(3-((4'-chloro-5,5-dimethyl-3,4,5,6-tetrahydro-[1,1'-biphenyl]-2-yl)methyl)-3,6-diazabicyclo[3.1.1]heptane-6-carbonyl)-4-fluoro-1-oxoisoindolin-2-yl)piperidine-2,6-dione |
| | 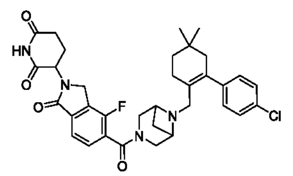 | 3-(5-(6-((4'-chloro-5,5-dimethyl-3,4,5,6-tetrahydro-[1,1'-biphenyl]-2-yl)methyl)-3,6-diazabicyclo[3.1.1]heptane-3-carbonyl)-4-fluoro-1-oxoisoindolin-2-yl)piperidine-2,6-dione | 3-(5-(6-((4'-chloro-5,5-dimethyl-3,4,5,6-tetrahydro-[1,1'-biphenyl]-2-yl)methyl)-3,6-diazabicyclo[3.1.1]heptane-3-carbonyl)-4-fluoro-1-oxoisoindolin-2-yl)piperidine-2,6-dione |
| | 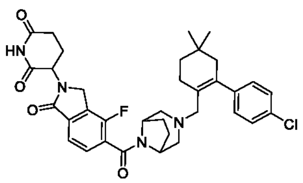 | 3-(5-(3-((4'-chloro-5,5-dimethyl-3,4,5,6-tetrahydro-[1,1'-biphenyl]-2-yl)methyl)-3,8-diazabicyclo[3.2.1]octane-8-carbonyl)-4-fluoro-1-oxoisoindolin-2-yl)piperidine-2,6-dione | 3-(5-(3-((4'-chloro-5,5-dimethyl-3,4,5,6-tetrahydro-[1,1'-biphenyl]-2-yl)methyl)-3,8-diazabicyclo[3.2.1]octane-8-carbonyl)-4-fluoro-1-oxoisoindolin-2-yl)piperidine-2,6-dione |
| | 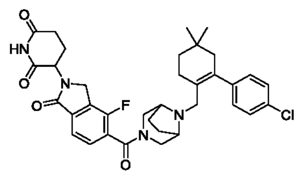 | 3-(5-(8-((4'-chloro-5,5-dimethyl-3,4,5,6-tetrahydro-[1,1'-biphenyl]-2-yl)methyl)-3,8-diazabicyclo[3.2.1]octane-3-carbonyl)-4-fluoro-1-oxoisoindolin-2-yl)piperidine-2,6-dione | 3-(5-(8-((4'-chloro-5,5-dimethyl-3,4,5,6-tetrahydro-[1,1'-biphenyl]-2-yl)methyl)-3,8-diazabicyclo[3.2.1]octane-3-carbonyl)-4-fluoro-1-oxoisoindolin-2-yl)piperidine-2,6-dione |
| | 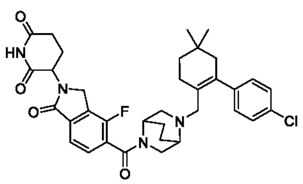 | 3-(5-(5-((4'-chloro-5,5-dimethyl-3,4,5,6-tetrahydro-[1,1'-biphenyl]-2-yl)methyl)-2,5-diazabicyclo[2.2.2]octane-2-carbonyl)-4-fluoro-1-oxoisoindolin-2-yl)piperidine-2,6-dione | 3-(5-(5-((4'-chloro-5,5-dimethyl-3,4,5,6-tetrahydro-[1,1'-biphenyl]-2-yl)methyl)-2,5-diazabicyclo[2.2.2]octane-2-carbonyl)-4-fluoro-1-oxoisoindolin-2-yl)piperidine-2,6-dione |
| | 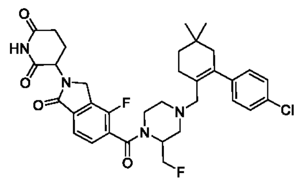 | 3-(5-(4-((4'-chloro-5,5-dimethyl-3,4,5,6-tetrahydro-[1,1'-biphenyl]-2-yl)methyl)-2-(fluoromethyl)piperazine-1-carbonyl)-4-fluoro-1-oxoisoindolin-2-yl)piperidine-2,6-dione | 3-(5-(4-((4'-chloro-5,5-dimethyl-3,4,5,6-tetrahydro-[1,1'-biphenyl]-2-yl)methyl)-2-(fluoromethyl)piperazine-1-carbonyl)-4-fluoro-1-oxoisoindolin-2-yl)piperidine-2,6-dione |
| | 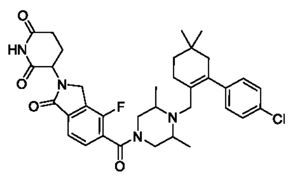 | 3-(5-(4-((4'-chloro-5,5-dimethyl-3,4,5,6-tetrahydro-[1,1'-biphenyl]-2-yl)methyl)-3,5-dimethylpiperazine-1-carbonyl)-4-fluoro-1-oxoisoindolin-2-yl)piperidine-2,6-dione | 3-(5-(4-((4'-chloro-5,5-dimethyl-3,4,5,6-tetrahydro-[1,1'-biphenyl]-2-yl)methyl)-3,5-dimethylpiperazine-1-carbonyl)-4-fluoro-1-oxoisoindolin-2-yl)piperidine-2,6-dione |
| | 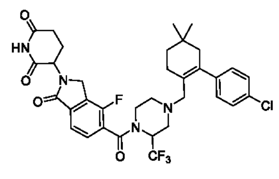 | 3-(5-(4-((4'-chloro-5,5-dimethyl-3,4,5,6-tetrahydro-[1,1'-biphenyl]-2-yl)methyl)-2-(trifluoromethyl)piperazine-1-carbonyl)-4-fluoro-1-oxoisoindolin-2-yl)piperidine-2,6-dione | 3-(5-(4-((4'-chloro-5,5-dimethyl-3,4,5,6-tetrahydro-[1,1'-biphenyl]-2-yl)methyl)-2-(trifluoromethyl)piperazine-1-carbonyl)-4-fluoro-1-oxoisoindolin-2-yl)piperidine-2,6-dione |

(continued)

| Compo und No. | Structure of the compounds | The compounds' name | Chinese translation of the compounds' name |
|---|---|---|---|
| | 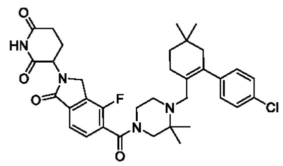 | 3-(5-(4-((4'-chloro-5,5-dimethyl-3,4,5,6-tetrahydro-[1,1'-biphenyl]-2-yl)methyl)-3,3-dimethylpiperazine-1-carbonyl)-4-fluoro-1-oxoisoindolin-2-yl)piperidine-2,6-dione | 3-(5-(4-((4'-chloro-5,5-dimethyl-3,4,5,6-tetrahydro-[1,1'-biphenyl]-2-yl)methyl)-3,3-dimethylpiperazine-1-carbonyl)-4-fluoro-1-oxoisoindolin-2-yl)piperidine-2,6-dione |
| | 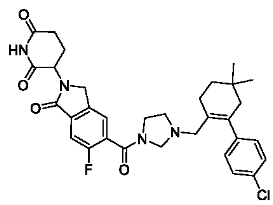 | 3-(5-(3-((4'-chloro-5,5-dimethyl-3,4,5,6-tetrahydro-[1,1'-biphenyl]-2-yl)methyl)imidazolidine-1-carbonyl)-6-fluoro-1-oxoisoindolin-2-yl)piperidine-2,6-dione | 3-(5-(3-((4'-chloro-5,5-dimethyl-3,4,5,6-tetrahydro-[1,1'-biphenyl]-2-yl)methyl)imidazolidine-1-carbonyl)-6-fluoro-1-oxoisoindolin-2-yl)piperidine-2,6-dione |
| | 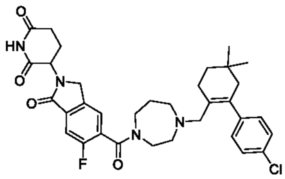 | 3-(5-(4-((4'-chloro-5,5-dimethyl-3,4,5,6-tetrahydro-[1,1'-biphenyl]-2-yl)methyl)-1,4-diazepane-1-carbonyl)-6-fluoro-1-oxoisoindolin-2-yl)piperidine-2,6-dione | 3-(5-(4-((4'-chloro-5,5-dimethyl-3,4,5,6-tetrahydro-[1,1'-biphenyl]-2-yl)methyl)-1,4-diazepane-1-carbonyl)-6-fluoro-1-oxoisoindolin-2-yl)piperidine-2,6-dione |
| | 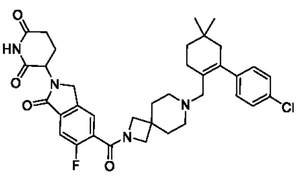 | 3-(5-(7-((4'-chloro-5,5-dimethyl-3,4,5,6-tetrahydro-[1,1'-biphenyl]-2-yl)methyl)-2,7-diazaspiro[3.5]nonane-2-carbonyl)-6-fluoro-1-oxoisoindolin-2-yl)piperidine-2,6-dione | 3-(5-(7-((4'-chloro-5,5-dimethyl-3,4,5,6-tetrahydro-[1,1'-biphenyl]-2-yl)methyl)-2,7-diazaspiro[3.5]nonane-2-carbonyl)-6-fluoro-1-oxoisoindolin-2-yl)piperidine-2,6-dione |
| | 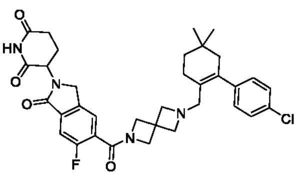 | 3-(5-(6-((4'-chloro-5,5-dimethyl-3,4,5,6-tetrahydro-[1,1'-biphenyl]-2-yl)methyl)-2,6-diazaspiro[3.3]heptane-2-carbonyl)-6-fluoro-1-oxoisoindolin-2-yl)piperidine-2,6-dione | 3-(5-(6-((4'-chloro-5,5-dimethyl-3,4,5,6-tetrahydro-[1,1'-biphenyl]-2-yl)methyl)-2,6-diazaspiro[3.3]heptane-2-carbonyl)-6-fluoro-1-oxoisoindolin-2-yl)piperidine-2,6-dione |
| | 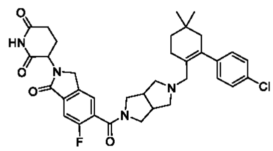 | 3-(5-(5-((4'-chloro-5,5-dimethyl-3,4,5,6-tetrahydro-[1,1'-biphenyl]-2-yl)methyl)octahydropyrrolo[ 3,4-c]pyrrole-2-carbonyl)-6-fluoro-1-oxoisoindolin-2-yl)piperidine-2,6-dione | 3-(5-(5-((4'-chloro-5,5-dimethyl-3,4,5,6-tetrahydro-[1,1'-biphenyl]-2-yl)methyl)octahydropyrrolo[3 ,4-c]pyrrole-2-carbonyl)-6-fluoro-1-oxoisoindolin-2-yl)piperidine-2,6-dione |
| | 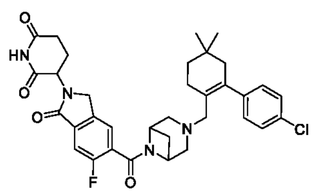 | 3-(5-(3-((4'-chloro-5,5-dimethyl-3,4,5,6-tetrahydro-[1,1'-biphenyl]-2-yl)methyl)-3,6-diazabicyclo[3.1.1]heptane-6-carbonyl)-6-fluoro-1-oxoisoindolin-2-yl)piperidine-2,6-dione | 3-(5-(3-((4'-chloro-5,5-dimethyl-3,4,5,6-tetrahydro-[1,1'-biphenyl]-2-yl)methyl)-3,6-diazabicyclo[3.1.1]heptane-6-carbonyl)-6-fluoro-1-oxoisoindolin-2-yl)piperidine-2,6-dione |
| | 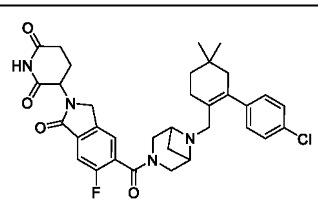 | 3-(5-(6-((4'-chloro-5,5-dimethyl-3,4,5,6-tetrahydro-[1,1'-biphenyl]-2-yl)methyl)-3,6-diazabicyclo[3.1.1]heptane-3-carbonyl)-6-fluoro-1-oxoisoindolin-2-yl)piperidine-2,6-dione | 3-(5-(6-((4'-chloro-5,5-dimethyl-3,4,5,6-tetrahydro-[1,1'-biphenyl]-2-yl)methyl)-3,6-diazabicyclo[3.1.1]heptane-3-carbonyl)-6-fluoro-1-oxoisoindolin-2-yl)piperidine-2,6-dione |

(continued)

| Compound No. | Structure of the compounds | The compounds' name | Chinese translation of the compounds' name |
|---|---|---|---|
| | 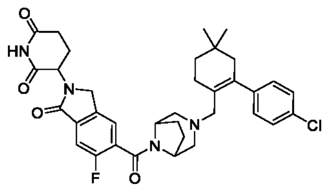 | 3-(5-(3-((4'-chloro-5,5-dimethyl-3,4,5,6-tetrahydro-[1,1'-biphenyl]-2-yl)methyl)-3,8-diazabicyclo[3.2.1]octane-8-carbonyl)-6-fluoro-1-oxoisoindolin-2-yl)piperidine-2,6-dione | 3-(5-(3-((4'-chloro-5,5-dimethyl-3,4,5,6-tetrahydro-[1,1'-biphenyl]-2-yl)methyl)-3,8-diazabicyclo[3.2.1]octane-8-carbonyl)-6-fluoro-1-oxoisoindolin-2-yl)piperidine-2,6-dione |
| | 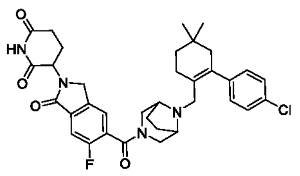 | 3-(5-(8-((4'-chloro-5,5-dimethyl-3,4,5,6-tetrahydro-[1,1'-biphenyl]-2-yl)methyl)-3,8-diazabicyclo[3.2.1]octane-3-carbonyl)-6-fluoro-1-oxoisoindolin-2-yl)piperidine-2,6-dione | 3-(5-(8-((4'-chloro-5,5-dimethyl-3,4,5,6-tetrahydro-[1,1'-biphenyl]-2-yl)methyl)-3,8-diazabicyclo[3.2.1]octane-3-carbonyl)-6-fluoro-1-oxoisoindolin-2-yl)piperidine-2,6-dione |
| | 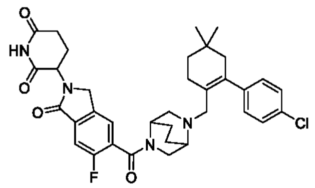 | 3-(5-(5-((4'-chloro-5,5-dimethyl-3,4,5,6-tetrahydro-[1,1'-biphenyl]-2-yl)methyl)-2,5-diazabicyclo[2.2.2]octane-2-carbonyl)-6-fluoro-1-oxoisoindolin-2-yl)piperidine-2,6-dione | 3-(5-(5-((4'-chloro-5,5-dimethyl-3,4,5,6-tetrahydro-[1,1'-biphenyl]-2-yl)methyl)-2,5-diazabicyclo[2.2.2]octane-2-carbonyl)-6-fluoro-1-oxoisoindolin-2-yl)piperidine-2,6-dione |
| | 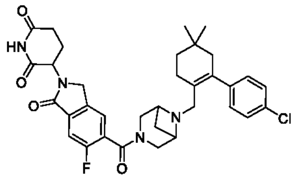 | 3-(5-(6-((4'-chloro-5,5-dimethyl-3,4,5,6-tetrahydro-[1,1'-biphenyl]-2-yl)methyl)-3,6-diazabicyclo[3.1.1]heptane-3-carbonyl)-6-fluoro-1-oxoisoindolin-2-yl)piperidine-2,6-dione | 3-(5-(6-((4'-chloro-5,5-dimethyl-3,4,5,6-tetrahydro-[1,1'-biphenyl]-2-yl)methyl)-3,6-diazabicyclo[3.1.1]heptane-3-carbonyl)-6-fluoro-1-oxoisoindolin-2-yl)piperidine-2,6-dione |
| | 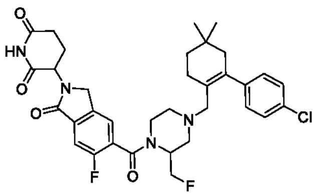 | 3-(5-(4-((4'-chloro-5,5-dimethyl-3,4,5,6-tetrahydro-[1,1'-biphenyl]-2-yl)methyl)-2-(fluoromethyl)piperazine-1-carbonyl)-6-fluoro-1-oxoisoindolin-2-yl)piperidine-2,6-dione | 3-(5-(4-((4'-chloro-5,5-dimethyl-3,4,5,6-tetrahydro-[1,1'-biphenyl]-2-yl)methyl)-2-(fluoromethyl)piperazine-1-carbonyl)-6-fluoro-1-oxoisoindolin-2-yl)piperidine-2,6-dione |
| | 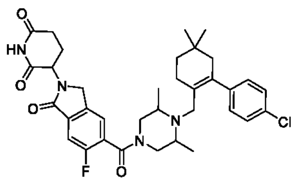 | 3-(5-(4-((4'-chloro-5,5-dimethyl-3,4,5,6-tetrahydro-[1,1'-biphenyl]-2-yl)methyl)-3,5-dimethylpiperazine-1-carbonyl)-6-fluoro-1-oxoisoindolin-2-yl)piperidine-2,6-dione | 3-(5-(4-((4'-chloro-5,5-dimethyl-3,4,5,6-tetrahydro-[1,1'-biphenyl]-2-yl)methyl)-3,5-dimethylpiperazine-1-carbonyl)-6-fluoro-1-oxoisoindolin-2-yl)piperidine-2,6-dione |
| | 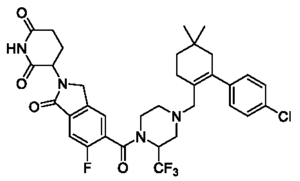 | 3-(5-(4-((4'-chloro-5,5-dimethyl-3,4,5,6-tetrahydro-[1,1'-biphenyl]-2-yl)methyl)-2-(trifluoromethyl)piperazine-1-carbonyl)-6-fluoro-1-oxoisoindolin-2-yl)piperidine-2,6-dione | 3-(5-(4-((4'-chloro-5,5-dimethyl-3,4,5,6-tetrahydro-[1,1'-biphenyl]-2-yl)methyl)-2-(trifluoromethyl)piperazine-1-carbonyl)-6-fluoro-1-oxoisoindolin-2-yl)piperidine-2,6-dione |
| | 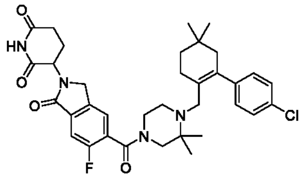 | 3-(5-(4-((4'-chloro-5,5-dimethyl-3,4,5,6-tetrahydro-[1,1'-biphenyl]-2-yl)methyl)-3,3-dimethylpiperazine-1-carbonyl)-6-fluoro-1-oxoisoindolin-2-yl)piperidine-2,6-dione | 3-(5-(4-((4'-chloro-5,5-dimethyl-3,4,5,6-tetrahydro-[1,1'-biphenyl]-2-yl)methyl)-3,3-dimethylpiperazine-1-carbonyl)-6-fluoro-1-oxoisoindolin-2-yl)piperidine-2,6-dione |

(continued)

| Compo und No. | Structure of the compounds | The compounds' name | Chinese translation of the compounds' name |
|---|---|---|---|
| | 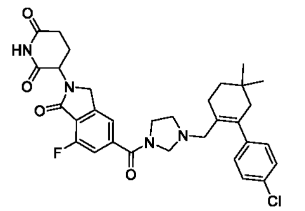 | 3-(5-(3-((4'-chloro-5,5-dimethyl-3,4,5,6-tetrahydro-[1,1'-biphenyl]-2-yl)methyl)imidazolidine-1-carbonyl)-7-fluoro-1-oxoisoindolin-2-yl)piperidine-2,6-dione | 3-(5-(3-((4'-chloro-5,5-dimethyl-3,4,5,6-tetrahydro-[1,1'-biphenyl]-2-yl)methyl)imidazolidine-1-carbonyl)-7-fluoro-1-oxoisoindolin-2-yl)piperidine-2,6-dione |
| | 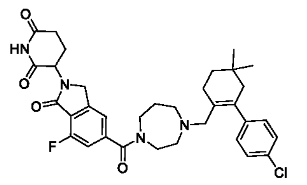 | 3-(5-(4-((4'-chloro-5,5-dimethyl-3,4,5,6-tetrahydro-[1,1'-biphenyl]-2-yl)methyl)-1,4-diazepane-1-carbonyl)-7-fluoro-1-oxoisoindolin-2-yl)piperidine-2,6-dione | 3-(5-(4-((4'-chloro-5,5-dimethyl-3,4,5,6-tetrahydro-[1,1'-biphenyl]-2-yl)methyl)-1,4-diazepane-1-carbonyl)-7-fluoro-1-oxoisoindolin-2-yl)piperidine-2,6-dione |
| | 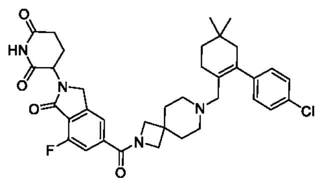 | 3-(5-(7-((4'-chloro-5,5-dimethyl-3,4,5,6-tetrahydro-[1,1'-biphenyl]-2-yl)methyl)-2,7-diazaspiro[3.5]nonane-2-carbonyl)-7-fluoro-1-oxoisoindolin-2-yl)piperidine-2,6-dione | 3-(5-(7-((4'-chloro-5,5-dimethyl-3,4,5,6-tetrahydro-[1,1'-biphenyl]-2-yl)methyl)-2,7-diazaspiro[3.5]nonane-2-carbonyl)-7-fluoro-1-oxoisoindolin-2-yl)piperidine-2,6-dione |
| | 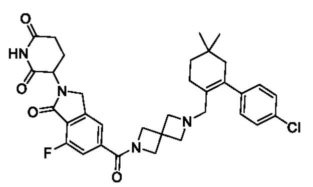 | 3-(5-(6-((4'-chloro-5,5-dimethyl-3,4,5,6-tetrahydro-[1,1'-biphenyl]-2-yl)methyl)-2,6-diazaspiro[3.3]heptane-2-carbonyl)-7-fluoro-1-oxoisoindolin-2-yl)piperidine-2,6-dione | 3-(5-(6-((4'-chloro-5,5-dimethyl-3,4,5,6-tetrahydro-[1,1'-biphenyl]-2-yl)methyl)-2,6-diazaspiro[3.3]heptane-2-carbonyl)-7-fluoro-1-oxoisoindolin-2-yl)piperidine-2,6-dione |
| | 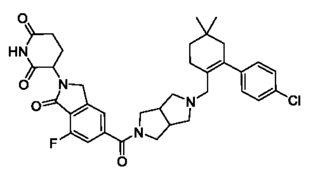 | 3-(5-(5-((4'-chloro-5,5-dimethyl-3,4,5,6-tetrahydro-[1,1'-biphenyl]-2-yl)methyl)octahydropyrrolo[ 3,4-c]pyrrole-2-carbonyl)-7-fluoro-1-oxoisoindolin-2-yl)piperidine-2,6-dione | 3-(5-(5-((4'-chloro-5,5-dimethyl-3,4,5,6-tetrahydro-[1,1'-biphenyl]-2-yl)methyl)octahydropyrrolo[3 ,4-c]pyrrole-2-carbonyl)-7-fluoro-1-oxoisoindolin-2-yl)piperidine-2,6-dione |
| | 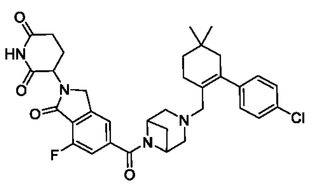 | 3-(5-(3-((4'-chloro-5,5-dimethyl-3,4,5,6-tetrahydro-[1,1'-biphenyl]-2-yl)methyl)-3,6-diazabicyclo[3.1.1]heptane-6-carbonyl)-7-fluoro-1-oxoisoindolin-2-yl)piperidine-2,6-dione | 3-(5-(3-((4'-chloro-5,5-dimethyl-3,4,5,6-tetrahydro-[1,1'-biphenyl]-2-yl)methyl)-3,6-diazabicyclo[3.1.1]heptane-6-carbonyl)-7-fluoro-1-oxoisoindolin-2-yl)piperidine-2,6-dione |
| | 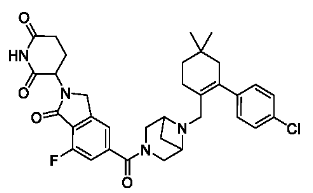 | 3-(5-(6-((4'-chloro-5,5-dimethyl-3,4,5,6-tetrahydro-[1,1'-biphenyl]-2-yl)methyl)-3,6-diazabicyclo[3.1.1]heptane-3-carbonyl)-7-fluoro-1-oxoisoindolin-2-yl)piperidine-2,6-dione | 3-(5-(6-((4'-chloro-5,5-dimethyl-3,4,5,6-tetrahydro-[1,1'-biphenyl]-2-yl)methyl)-3,6-diazabicyclo[3.1.1]heptane-3-carbonyl)-7-fluoro-1-oxoisoindolin-2-yl)piperidine-2,6-dione |
| | 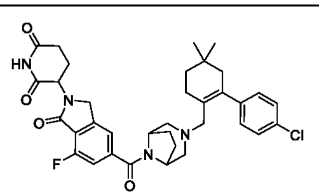 | 3-(5-(3-((4'-chloro-5,5-dimethyl-3,4,5,6-tetrahydro-[1,1'-biphenyl]-2-yl)methyl)-3,8-diazabicyclo[3.2.1]octane-8-carbonyl)-7-fluoro-1-oxoisoindolin-2-yl)piperidine-2,6-dione | 3-(5-(3-((4'-chloro-5,5-dimethyl-3,4,5,6-tetrahydro-[1,1'-biphenyl]-2-yl)methyl)-3,8-diazabicyclo[3.2.1]octane-8-carbonyl)-7-fluoro-1-oxoisoindolin-2-yl)piperidine-2,6-dione |

(continued)

| Compo und No. | Structure of the compounds | The compounds' name | Chinese translation of the compounds' name |
|---|---|---|---|
| | 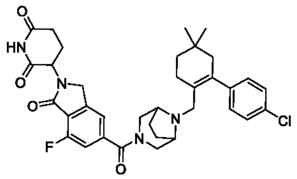 | 3-(5-(8-((4'-chloro-5,5-dimethyl-3,4,5,6-tetrahydro-[1,1'-biphenyl]-2-yl)methyl)-3,8-diazabicyclo[3.2.1]octane-3-carbonyl)-7-fluoro-1-oxoisoindolin-2-yl)piperidine-2,6-dione | 3-(5-(8-((4'-chloro-5,5-dimethyl-3,4,5,6-tetrahydro-[1,1'-biphenyl]-2-yl)methyl)-3,8-diazabicyclo[3.2.1]octane-3-carbonyl)-7-fluoro-1-oxoisoindolin-2-yl)piperidine-2,6-dione |
| | 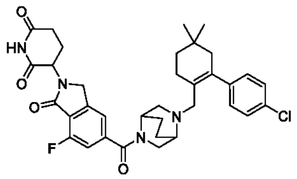 | 3-(5-(5-((4'-chloro-5,5-dimethyl-3,4,5,6-tetrahydro-[1,1'-biphenyl]-2-yl)methyl)-2,5-diazabicyclo[2.2.2]octane-2-carbonyl)-7-fluoro-1-oxoisoindolin-2-yl)piperidine-2,6-dione | 3-(5-(5-((4'-chloro-5,5-dimethyl-3,4,5,6-tetrahydro-[1,1'-biphenyl]-2-yl)methyl)-2,5-diazabicyclo[2.2.2]octane-2-carbonyl)-7-fluoro-1-oxoisoindolin-2-yl)piperidine-2,6-dione |
| | 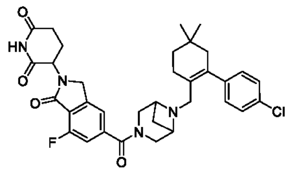 | 3-(5-(6-((4'-chloro-5,5-dimethyl-3,4,5,6-tetrahydro-[1,1'-biphenyl]-2-yl)methyl)-3,6-diazabicyclo[3.1.1]heptane-3-carbonyl)-7-fluoro-1-oxoisoindolin-2-yl)piperidine-2,6-dione | 3-(5-(6-((4'-chloro-5,5-dimethyl-3,4,5,6-tetrahydro-[1,1'-biphenyl]-2-yl)methyl)-3,6-diazabicyclo[3.1.1]heptane-3-carbonyl)-7-fluoro-1-oxoisoindolin-2-yl)piperidine-2,6-dione |
| | 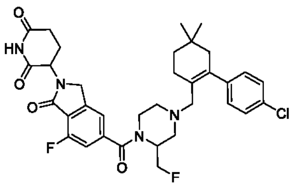 | 3-(5-(4-((4'-chloro-5,5-dimethyl-3,4,5,6-tetrahydro-[1,1'-biphenyl]-2-yl)methyl)-2-(fluoromethyl)piperazine-1-carbonyl)-7-fluoro-1-oxoisoindolin-2-yl)piperidine-2,6-dione | 3-(5-(4-((4'-chloro-5,5-dimethyl-3,4,5,6-tetrahydro-[1,1'-biphenyl]-2-yl)methyl)-2-(fluoromethyl)piperazine-1-carbonyl)-7-fluoro-1-oxoisoindolin-2-yl)piperidine-2,6-dione |
| | 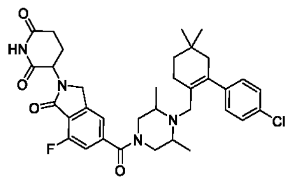 | 3-(5-(4-((4'-chloro-5,5-dimethyl-3,4,5,6-tetrahydro-[1,1'-biphenyl]-2-yl)methyl)-3,5-dimethylpiperazine-1-carbonyl)-7-fluoro-1-oxoisoindolin-2-yl)piperidine-2,6-dione | 3-(5-(4-((4'-chloro-5,5-dimethyl-3,4,5,6-tetrahydro-[1,1'-biphenyl]-2-yl)methyl)-3,5-dimethylpiperazine-1-carbonyl)-7-fluoro-1-oxoisoindolin-2-yl)piperidine-2,6-dione |
| | 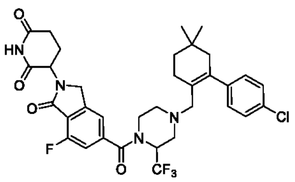 | 3-(5-(4-((4'-chloro-5,5-dimethyl-3,4,5,6-tetrahydro-[1,1'-biphenyl]-2-yl)methyl)-2-(trifluoromethyl)piperazine-1-carbonyl)-7-fluoro-1-oxoisoindolin-2-yl)piperidine-2,6-dione | 3-(5-(4-((4'-chloro-5,5-dimethyl-3,4,5,6-tetrahydro-[1,1'-biphenyl]-2-yl)methyl)-2-(trifluoromethyl)piperazine-1-carbonyl)-7-fluoro-1-oxoisoindolin-2-yl)piperidine-2,6-dione |
| | 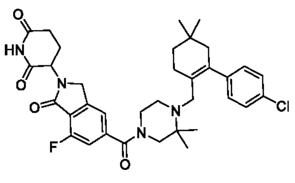 | 3-(5-(4-((4'-chloro-5,5-dimethyl-3,4,5,6-tetrahydro-[1,1'-biphenyl]-2-yl)methyl)-3,3-dimethylpiperazine-1-carbonyl)-7-fluoro-1-oxoisoindolin-2-yl)piperidine-2,6-dione | 3-(5-(4-((4'-chloro-5,5-dimethyl-3,4,5,6-tetrahydro-[1,1'-biphenyl]-2-yl)methyl)-3,3-dimethylpiperazine-1-carbonyl)-7-fluoro-1-oxoisoindolin-2-yl)piperidine-2,6-dione |
| GT-03355 | 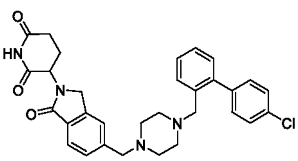 | 3-(5-((4-((4'-chloro-[1,1'-biphenyl]-2-yl)methyl)piperazin-1-yl)methyl)-1-oxoisoindolin-2-yl)piperidine-2,6-dione | 3-(5-((4-((4'-chloro-[1,1'-biphenyl]-2-yl)methyl)piperazin-1-yl)methyl)-1-oxoisoindolin-2-yl)piperidine-2,6-dione |

(continued)

| Compo und No. | Structure of the compounds | The compounds’ name | Chinese translation of the compounds’ name |
|---|---|---|---|
| GT-03694 | 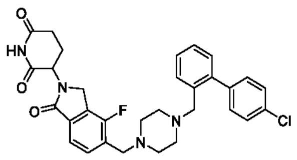 | 3-(5-((4-((4’-chloro-[1,1’-biphenyl]-2-yl)methyl)piperazin-1-yl)methyl)-4-fluoro-1-oxoisoindolin-2-yl)piperidine-2,6-dione | 3-(5-((4-((4’-chloro-[1,1’-biphenyl]-2-yl)methyl)piperazin-1-yl)methyl)-4-fluoro-1-oxoisoindolin-2-yl)piperidine-2,6-dione |
| GT-03695 | 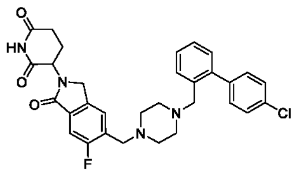 | 3-(5-((4-((4’-chloro-[1,1’-biphenyl]-2-yl)methyl)piperazin-1-yl)methyl)-6-fluoro-1-oxoisoindolin-2-yl)piperidine-2,6-dione | 3-(5-((4-((4’-chloro-[1,1’-biphenyl]-2-yl)methyl)piperazin-1-yl)methyl)-6-fluoro-1-oxoisoindolin-2-yl)piperidine-2,6-dione |
| GT-03696 | 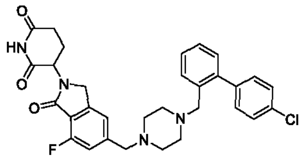 | 3-(5-((4-((4’-chloro-[1,1’-biphenyl]-2-yl)methyl)piperazin-1-yl)methyl)-7-fluoro-1-oxoisoindolin-2-yl)piperidine-2,6-dione | 3-(5-((4-((4’-chloro-[1,1’-biphenyl]-2-yl)methyl)piperazin-1-yl)methyl)-7-fluoro-1-oxoisoindolin-2-yl)piperidine-2,6-dione |
| GT-03876 | 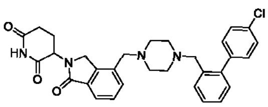 | 3-(4-((4-((4’-chloro-[1,1’-biphenyl]-2-yl)methyl)piperazin-1-yl)methyl)-1-oxoisoindolin-2-yl)piperidine-2,6-dione | 3-(4-((4-((4’-chloro-[1,1’-biphenyl]-2-yl)methyl)piperazin-1-yl)methyl)-1-oxoisoindolin-2-yl)piperidine-2,6-dione |
| GT-05876 | 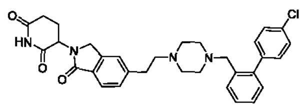 | 3-(5-(2-(4-((4’-chloro-[1,1’-biphenyl]-2-yl)methyl)piperazin-1-yl)ethyl)-1-oxoisoindolin-2-yl)piperidine-2,6-dione | 3-(5-(2-(4-((4’-chloro-[1,1’-biphenyl]-2-yl)methyl)piperazin-1-yl)ethyl)-1-oxoisoindolin-2-yl)piperidine-2,6-dione |
| GT-05893 | 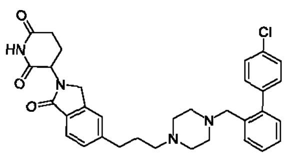 | 3-(5-(3-(4-((4’-chloro-[1,1’-biphenyl]-2-yl)methyl)piperazin-1-yl)propyl)-1-oxoisoindolin-2-yl)piperidine-2,6-dione | 3-(5-(3-(4-((4’-chloro-[1,1’-biphenyl]-2-yl)methyl)piperazin-1-yl)propyl)-1-oxoisoindolin-2-yl)piperidine-2,6-dione |
| GT-03877 | 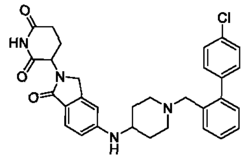 | 3-(5-((1-((4’-chloro-[1,1’-biphenyl]-2-yl)methyl)piperidin-4-yl)amino)-1-oxoisoindolin-2-yl)piperidine-2,6-dione | 3-(5-((1-((4’-chloro-[1,1’-biphenyl]-2-yl)methyl)piperidin-4-yl)amino)-1-oxoisoindolin-2-yl)piperidine-2,6-dione |
| GT-04196 | 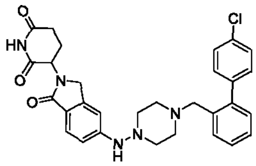 | 3-(5-((4-((4’-chloro-[1,1’-biphenyl]-2-yl)methyl)piperazin-1-yl)amino)-1-oxoisoindolin-2-yl)piperidine-2,6-dione | 3-(5-((4-((4’-chloro-[1,1’-biphenyl]-2-yl)methyl)piperazin-1-yl)amino)-1-oxoisoindolin-2-yl)piperidine-2,6-dione |
| GT-04256 | 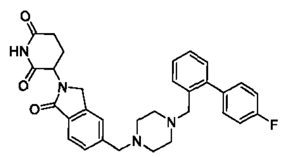 | 3-(5-((4-((4’-fluoro-[1,1’-biphenyl]-2-yl)methyl)piperazin-1-yl)methyl)-1-oxoisoindolin-2-yl)piperidine-2,6-dione | 3-(5-((4-((4’-fluoro-[1,1’-biphenyl]-2-yl)methyl)piperazin-1-yl)methyl)-1-oxoisoindolin-2-yl)piperidine-2,6-dione |

(continued)

| Compo und No. | Structure of the compounds | The compounds' name | Chinese translation of the compounds' name |
|---|---|---|---|
| GT-05602 | 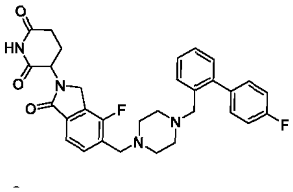 | 3-(4-fluoro-5-((4-((4'-fluoro-[1,1'-biphenyl]-2-yl)methyl)piperazin-1-yl)methyl)-1-oxoisoindolin-2-yl)piperidine-2,6-dione | 3-(4-fluoro-5-((4-((4'-fluoro-[1,1'-biphenyl]-2-yl)methyl)piperazin-1-yl)methyl)-1-oxoisoindolin-2-yl)piperidine-2,6-dione |
| GT-05603 | 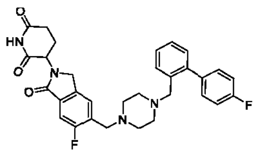 | 3-(6-fluoro-5-((4-((4'-fluoro-[1,1'-biphenyl]-2-yl)methyl)piperazin-1-yl)methyl)-1-oxoisoindolin-2-yl)piperidine-2,6-dione | 3-(6-fluoro-5-((4-((4'-fluoro-[1,1'-biphenyl]-2-yl)methyl)piperazin-1-yl)methyl)-1-oxoisoindolin-2-yl)piperidine-2,6-dione |
| GT-05604 | 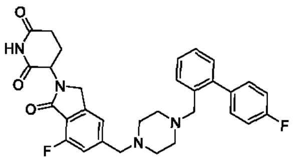 | 3-(7-fluoro-5-((4-((4'-fluoro-[1,1'-biphenyl]-2-yl)methyl)piperazin-1-yl)methyl)-1-oxoisoindolin-2-yl)piperidine-2,6-dione | 3-(7-fluoro-5-((4-((4'-fluoro-[1,1'-biphenyl]-2-yl)methyl)piperazin-1-yl)methyl)-1-oxoisoindolin-2-yl)piperidine-2,6-dione |
| GT-05605 | 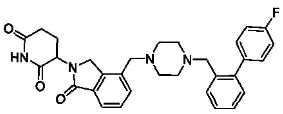 | 3-(4-((4-((4'-fluoro-[1,1'-biphenyl]-2-yl)methyl)piperazin-1-yl)methyl)-1-oxoisoindolin-2-yl)piperidine-2,6-dione | 3-(4-((4-((4'-fluoro-[1,1'-biphenyl]-2-yl)methyl)piperazin-1-yl)methyl)-1-oxoisoindolin-2-yl)piperidine-2,6-dione |
| GT-05606 | 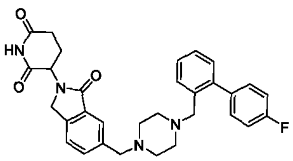 | 3-(6-((4-((4'-fluoro-[1,1'-biphenyl]-2-yl)methyl)piperazin-1-yl)methyl)-1-oxoisoindolin-2-yl)piperidine-2,6-dione | 3-(6-((4-((4'-fluoro-[1,1'-biphenyl]-2-yl)methyl)piperazin-1-yl)methyl)-1-oxoisoindolin-2-yl)piperidine-2,6-dione |
| GT-04194 | 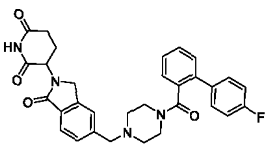 | 3-(5-((4-(4'-fluoro-[1,1'-biphenyl]-2-carbonyl)piperazin-1-yl)methyl)-1-oxoisoindolin-2-yl)piperidine-2,6-dione | 3-(5-((4-(4'-fluoro-[1,1'-biphenyl]-2-carbonyl)piperazin-1-yl)methyl)-1-oxoisoindolin-2-yl)piperidine-2,6-dione |
| GT-04193 | 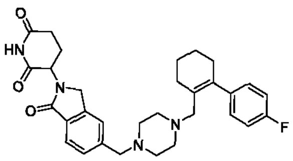 | 3-(5-((4-((4'-fluoro-3,4,5,6-tetrahydro-[1,1'-biphenyl]-2-yl)methyl)piperazin-1-yl)methyl)-1-oxoisoindolin-2-yl)piperidine-2,6-dione | 3-(5-((4-((4'-fluoro-3,4,5,6-tetrahydro-[1,1'-biphenyl]-2-yl)methyl)piperazin-1-yl)methyl)-1-oxoisoindolin-2-yl)piperidine-2,6-dione |
| GT-04170 | 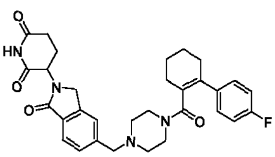 | 3-(5-((4-(4'-fluoro-3,4,5,6-tetrahydro-[1,1'-biphenyl]-2-carbonyl)piperazin-1-yl)methyl)-1-oxoisoindolin-2-yl)piperidine-2,6-dione | 3-(5-((4-(4'-fluoro-3,4,5,6-tetrahydro-[1,1'-biphenyl]-2-carbonyl)piperazin-1-yl)methyl)-1-oxoisoindolin-2-yl)piperidine-2,6-dione |
| GT-04169 | 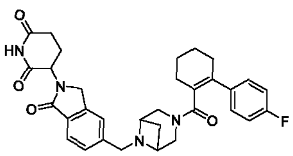 | 3-(5-((3-(4'-fluoro-3,4,5,6-tetrahydro-[1,1'-biphenyl]-2-carbonyl)-3,6-diazabicyclo[3.1.1]heptan-6-yl)methyl)-1-oxoisoindolin-2-yl)piperidine-2,6-dione | 3-(5-((3-(4'-fluoro-3,4,5,6-tetrahydro-[1,1'-biphenyl]-2-carbonyl)-3,6-diazabicyclo[3.1.1]heptan-6-yl)methyl)-1-oxoisoindolin-2-yl)piperidine-2,6-dione |

(continued)

| Compo und No. | Structure of the compounds | The compounds' name | Chinese translation of the compounds' name |
|---|---|---|---|
| | 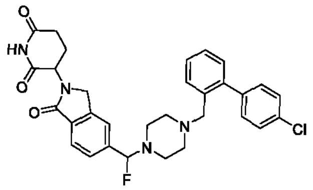 | 3-(5-((4-((4'-chloro-[1,1'-biphenyl]-2-yl)methyl)piperazin-1-yl) fluoromethyl)-1-oxoisoindolin-2-yl)piperidine-2,6-dione | 3-(5-((4-((4'-chloro-[1,1'-biphenyl]-2-yl)methyl)piperazin-1-yl) fluoromethyl)-1-oxoisoindolin-2-yl)piperidine-2,6-dione |
| | 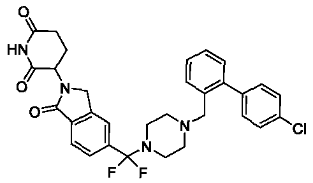 | 3-(5-((4-((4'-chloro-[1,1'-biphenyl]-2-yl)methyl)piperazin-1-yl) difluoromethyl)-1-oxoisoindolin-2-yl)piperidine-2,6-dione | 3-(5-((4-((4'-chloro-[1,1'-biphenyl]-2-yl)methyl)piperazin-1-yl) difluoromethyl)-1-oxoisoindolin-2-yl)piperidine-2,6-dione |
| | 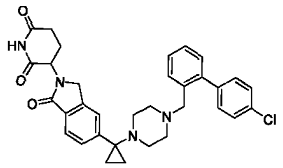 | 3-(5-(1-(4-((4'-chloro-[1,1'-biphenyl]-2-yl)methyl)piperazin-1-yl)cyclopropyl)-1-oxoisoindolin-2-yl)piperidine-2,6-dione | 3-(5-(1-(4-((4'-chloro-[1,1'-biphenyl]-2-yl)methyl)piperazin-1-yl)cyclopropyl)-1-oxoisoindolin-2-yl)piperidine-2,6-dione |
| | 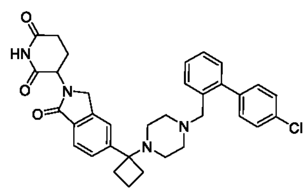 | 3-(5-(1-(4-((4'-chloro-[1,1'-biphenyl]-2-yl)methyl)piperazin-1-yl)cyclobutyl)-1-oxoisoindolin-2-yl)piperidine-2,6-dione | 3-(5-(1-(4-((4'-chloro-[1,1'-biphenyl]-2-yl)methyl)piperazin-1-yl)cyclobutyl)-1-oxoisoindolin-2-yl)piperidine-2,6-dione |
| | 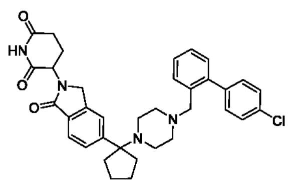 | 3-(5-(1-(4-((4'-chloro-[1,1'-biphenyl]-2-yl)methyl)piperazin-1-yl)cyclopentyl)-1-oxoisoindolin-2-yl)piperidine-2,6-dione | 3-(5-(1-(4-((4'-chloro-[1,1'-biphenyl]-2-yl)methyl)piperazin-1-yl)cyclopentyl)-1-oxoisoindolin-2-yl)piperidine-2,6-dione |
| | 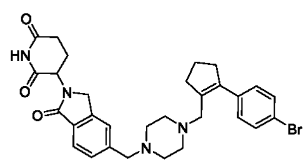 | 3-(5-((4-((2-(4-bromophenyl)cyclopent-1-en-1-yl)methyl)piperazin-1-yl)methyl)-1-oxoisoindolin-2-yl)piperidine-2,6-dione | 3-(5-((4-((2-(4-bromophenyl)cyclopent-1-en-1-yl)methyl)piperazin-1-yl)methyl)-1-oxoisoindolin-2-yl)piperidine-2,6-dione |
| | 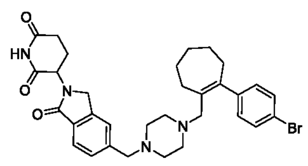 | 3-(5-((4-((2-(4-bromophenyl)cyclohept-1-en-1-yl)methyl)piperazin-1-yl)methyl)-1-oxoisoindolin-2-yl)piperidine-2,6-dione | 3-(5-((4-((2-(4-bromophenyl)cyclohept-1-en-1-yl)methyl)piperazin-1-yl)methyl)-1-oxoisoindolin-2-yl)piperidine-2,6-dione |
| | 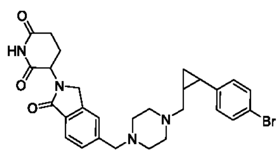 | 3-(5-((4-((2-(4-bromophenyl)cyclopropyl) methyl)piperazin-1-yl)methyl)-1-oxoisoindolin-2-yl) piperidine-2,6-dione | 3-(5-((4-((2-(4-bromophenyl)cyclopropyl)me thyl)piperazin-1-yl)methyl)-1-oxoisoindolin-2-yl) piperidine-2,6-dione |

(continued)

| Compound No. | Structure of the compounds | The compounds' name | Chinese translation of the compounds' name |
|---|---|---|---|
| | 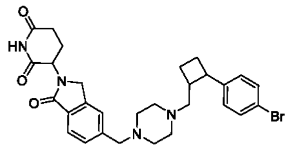 | 3-(5-((4-((2-(4-bromophenyl)cyclobutyl)me thyl)piperazin-1-yl)methyl)-1-oxoisoindolin-2-yl)piperidine-2,6-dione | 3-(5-((4-((2-(4-bromophenyl)cyclobutyl)met hyl)piperazin-1-yl)methyl)-1-oxoisoindolin-2-yl)piperidine-2,6-dione |
| GT-05519 | 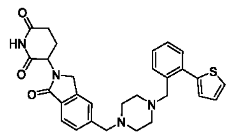 | 3-(1-oxo-5-((4-(2-(thiophen-2-yl)benzyl)piperazin-1-yl)methyl)isoindolin-2-yl)piperidine-2,6-dione | 3-(1-oxo-5-((4-(2-(thiophen-2-yl)benzyl)piperazin-1-yl)methyl)isoindolin-2-yl)piperidine-2,6-dione |
| GT-05779 | 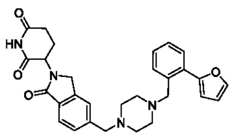 | 3-(5-((4-(2-(furan-2-yl)benzyl)piperazin-1-yl)methyl)-1-oxoisoindolin-2-yl)piperidine-2,6-dione | 3-(5-((4-(2-(furan-2-yl)benzyl)piperazin-1-yl)methyl)-1-oxoisoindolin-2-yl)piperidine-2,6-dione |
| | 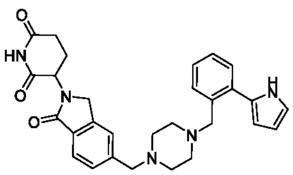 | 3-(5-((4-(2-(1H-pyrrol-2-yl)benzyl)piperazin-1-yl)methyl)-1-oxoisoindolin-2-yl)piperidine-2,6-dione | 3-(5-((4-(2-(1H-pyrrol-2-yl)benzyl)piperazin-1-yl)methyl)-1-oxoisoindolin-2-yl)piperidine-2,6-dione |
| GT-05683 | 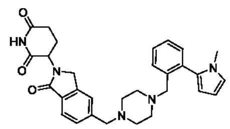 | 3-(5-((4-(2-(1-methyl-1H-pyrrol-2-yl)benzyl)piperazin-1-yl)methyl)-1-oxoisoindolin-2-yl)piperidine-2,6-dione | 3-(5-((4-(2-(1-methyl-1H-pyrrol-2-yl)benzyl)piperazin-1-yl)methyl)-1-oxoisoindolin-2-yl)piperidine-2,6-dione |
| GT-05702 | 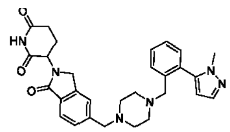 | 3-(5-((4-(2-(1-methyl-1H-pyrazol-5-yl)benzyl)piperazin-1-yl)methyl)-1-oxoisoindolin-2-yl)piperidine-2,6-dione | 3-(5-((4-(2-(1-methyl-1H-pyrazol-5-yl)benzyl)piperazin-1-yl)methyl)-1-oxoisoindolin-2-yl)piperidine-2,6-dione |
| GT-05684 | 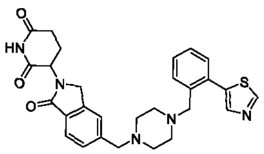 | 3-(1-oxo-5-((4-(2-(thiazol-5-yl)benzyl)piperazin-1-yl)methyl)isoindolin-2-yl)piperidine-2,6-dione | 3-(1-oxo-5-((4-(2-(thiazol-5-yl)benzyl)piperazin-1-yl)methyl)isoindolin-2-yl)piperidine-2,6-dione |
| GT-05849 | 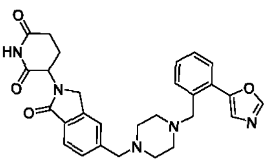 | 3-(5-((4-(2-(oxazol-5-yl)benzyl)piperazin-1-yl)methyl)-1-oxoisoindolin-2-yl)piperidine-2,6-dione | 3-(5-((4-(2-(oxazol-5-yl)benzyl)piperazin-1-yl)methyl)-1-oxoisoindolin-2-yl)piperidine-2,6-dione |
| GT-03880 | 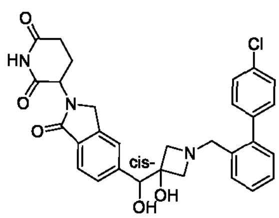 | 3-(5-((1-((4'-chloro-[1,1'-biphenyl]-2-yl)methyl)-3-hydroxyazetidin-3-yl)(hydroxy)methyl)-1-oxoisoindolin-2-yl)piperidine-2,6-dione | 3-(5-((1-((4'-chloro-[1,1'-biphenyl]-2-yl)methyl)-3-hydroxyazetidin-3-yl)(hydroxy)methyl)-1-oxoisoindolin-2-yl)piperidine-2,6-dione |

(continued)

| Compo und No. | Structure of the compounds | The compounds' name | Chinese translation of the compounds' name |
|---|---|---|---|
| GT-03881 | 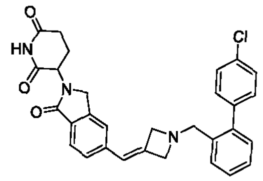 | 3-(5-((1-((4'-chloro-[1,1'-biphenyl]-2-yl)methyl)azetidin-3-ylidene)methyl)-1-oxoisoindolin-2-yl)piperidine-2,6-dione | 3-(5-((1-((4'-chloro-[1,1'-biphenyl]-2-yl)methyl)azetidin-3-ylidene)methyl)-1-oxoisoindolin-2-yl)piperidine-2,6-dione |
| GT-05382 | 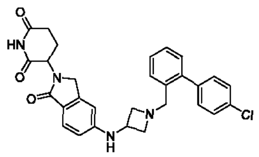 | 3-(5-((1-((4'-chloro-[1,1'-biphenyl]-2-yl)methyl)azetidin-3-yl) amino)-1-oxoisoindolin-2-yl)piperidine-2,6-dione | 3-(5-((1-((4'-chloro-[1,1'-biphenyl]-2-yl)methyl)azetidin-3-yl) amino)-1-oxoisoindolin-2-yl)piperidine-2,6-dione |
| GT-04204 | 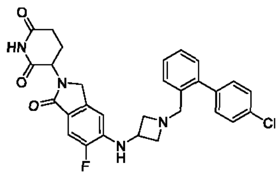 | 3-(5-((1-((4'-chloro-[1,1'-biphenyl]-2-yl)methyl)azetidin-3-yl) amino)-6-fluoro-1-oxoisoindolin-2-yl)piperidine-2,6-dione | 3-(5-((1-((4'-chloro-[1,1'-biphenyl]-2-yl)methyl)azetidin-3-yl) amino)-6-fluoro-1-oxoisoindolin-2-yl)piperidine-2,6-dione |
| GT-03923 | 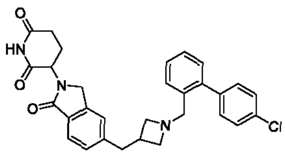 | 3-(5-((1-((4'-chloro-[1,1'-biphenyl]-2-yl)methyl)azetidin-3-yl) methyl)-1-oxoisoindolin-2-yl)piperidine-2,6-dione | 3-(5-((1-((4'-chloro-[1,1'-biphenyl]-2-yl)methyl)azetidin-3-yl) methyl)-1-oxoisoindolin-2-yl)piperidine-2,6-dione |
| GT-03356 | 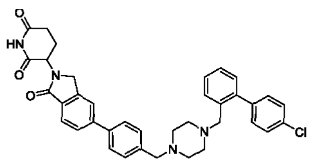 | 3-(5-(4-((4-((4'-chloro-[1,1'-biphenyl]-2-yl)methyl)piperazin-1-yl)methyl)phenyl)-1-oxoisoindolin-2-yl)piperidine-2,6-dione | 3-(5-(4-((4-((4'-chloro-[1,1'-biphenyl]-2-yl)methyl)piperazin-1-yl)methyl)phenyl)-1-oxoisoindolin-2-yl)piperidine-2,6-dione |
| GT-03991 | 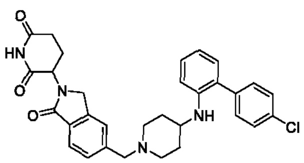 | 3-(5-((4-((4'-chloro-[1,1'-biphenyl]-2-yl)amino)piperidin-1-yl) methyl)-1-oxoisoindolin-2-yl)piperidine-2,6-dione | 3-(5-((4-((4'-chloro-[1,1'-biphenyl]-2-yl)amino)piperidin-1-yl) methyl)-1-oxoisoindolin-2-yl)piperidine-2,6-dione |
| | 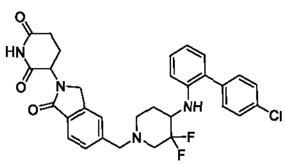 | 3-(5-((4-((4'-chloro-[1,1'-biphenyl]-2-yl)amino)-3,3-difluoropiperidin-1-yl)methyl)-1-oxoisoindolin-2-yl)piperidine-2,6-dione | 3-(5-((4-((4'-chloro-[1,1'-biphenyl]-2-yl)amino)-3,3-difluoropiperidin-1-yl)methyl)-1-oxoisoindolin-2-yl)piperidine-2,6-dione |
| GT-03361 | 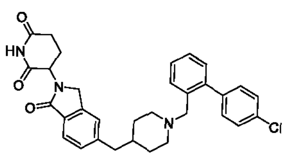 | 3-(5-((1-((4'-chloro-[1,1'-biphenyl]-2-yl)methyl)piperidin-4-yl) methyl)-1-oxoisoindolin-2-yl)piperidine-2,6-dione | 3-(5-((1-((4'-chloro-[1,1'-biphenyl]-2-yl)methyl)piperidin-4-yl) methyl)-1-oxoisoindolin-2-yl)piperidine-2,6-dione |
| | 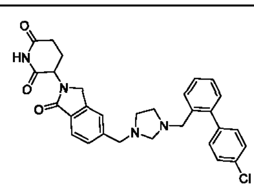 | 3-(5-((3-((4'-chloro-[1,1'-biphenyl]-2-yl)methyl)imidazolidin-1-yl)methyl)-1-oxoisoindolin-2-yl) piperidine-2,6-dione | 3-(5-((3-((4'-chloro-[1,1'-biphenyl]-2-yl)methyl)imidazolidin-1-yl)methyl)-1-oxoisoindolin-2-yl) piperidine-2,6-dione |

(continued)

| Compound No. | Structure of the compounds | The compounds' name | Chinese translation of the compounds' name |
|---|---|---|---|
| GT-05611 | 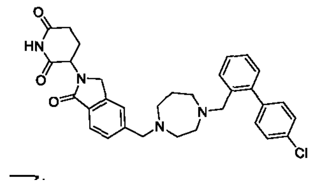 | 3-(5-((4-((4'-chloro-[1,1'-biphenyl]-2-yl)methyl)-1,4-diazepan-1-yl)methyl)-1-oxoisoindolin-2-yl)piperidine-2,6-dione | 3-(5-((4-((4'-chloro-[1,1'-biphenyl]-2-yl)methyl)-1,4-diazepan-1-yl)methyl)-1-oxoisoindolin-2-yl)piperidine-2,6-dione |
| | 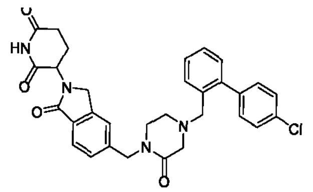 | 3-(5-((4-((4'-chloro-[1,1'-biphenyl]-2-yl)methyl)-2-oxopiperazin-1-yl)methyl)-1-oxoisoindolin-2-yl)piperidine-2,6-dione | 3-(5-((4-((4'-chloro-[1,1'-biphenyl]-2-yl)methyl)-2-oxopiperazin-1-yl)methyl)-1-oxoisoindolin-2-yl)piperidine-2,6-dione |
| GT-05612 | 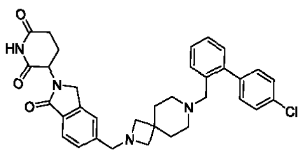 | 3-(5-((7-((4'-chloro-[1,1'-biphenyl]-2-yl)methyl)-2,7-diazaspiro [3.5]nonan-2-yl)methyl)-1-oxoisoindolin-2-yl)piperidine-2,6-dione | 3-(5-((7-((4'-chloro-[1,1'-biphenyl]-2-yl)methyl)-2,7-diazaspiro [3.5]nonan-2-yl)methyl)-1-oxoisoindolin-2-yl)piperidine-2,6-dione |
| GT-05709 | 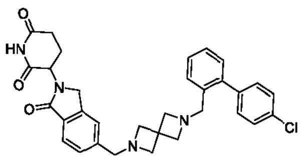 | 3-(5-((6-((4'-chloro-[1,1'-biphenyl]-2-yl)methyl)-2,6-diazaspiro [3.3]heptan-2-yl)methyl)-1-oxoisoindolin-2-yl)piperidine-2,6-dione | 3-(5-((6-((4'-chloro-[1,1'-biphenyl]-2-yl)methyl)-2,6-diazaspiro [3.3]heptan-2-yl)methyl)-1-oxoisoindolin-2-yl)piperidine-2,6-dione |
| GT-05613 | 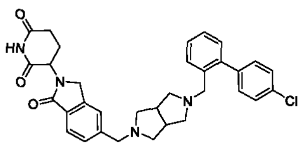 | 3-(5-((5-((4'-chloro-[1,1'-biphenyl]-2-yl)methyl)hexahydropyrrolo [3,4-c]pyrrol-2(1H)-yl) methyl)-1-oxoisoindolin-2-yl)piperidine-2,6-dione | 3-(5-((5-((4'-chloro-[1,1'-biphenyl]-2-yl)methyl)hexahydropyrrolo[ 3,4-c]pyrrol-2(1H)-yl) methyl)-1-oxoisoindolin-2-yl)piperidine-2,6-dione |
| | 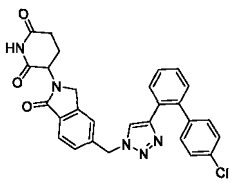 | 3-(5-((4-(4'-chloro-[1,1'-biphenyl]-2-yl)-1H-1,2,3-triazol-1-yl) methyl)-1-oxoisoindolin-2-yl)piperidine-2,6-dione | 3-(5-((4-(4'-chloro-[1,1'-biphenyl]-2-yl)-1H-1,2,3-triazol-1-yl) methyl)-1-oxoisoindolin-2-yl)piperidine-2,6-dione |
| | 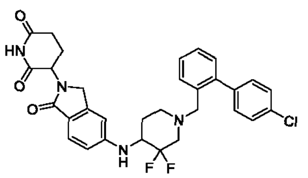 | 3-(5-((1-((4'-chloro-[1,1'-biphenyl]-2-yl)methyl)-3,3-difluoropiperidin-4-yl)amino)-1-oxoisoindolin-2-yl)piperidine-2,6-dione | 3-(5-((1-((4'-chloro-[1,1'-biphenyl]-2-yl)methyl)-3,3-difluoropiperidin-4-yl)amino)-1-oxoisoindolin-2-yl)piperidine-2,6-dione |
| GT-03878 | 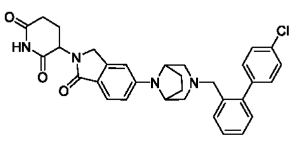 | 3-(5-(3-((4'-chloro-[1,1'-biphenyl]-2-yl)methyl)-3,8-diazabicyclo[3.2.1]octan-8-yl)-1-oxoisoindolin-2-yl)piperidine-2,6-dione | 3-(5-(3-((4'-chloro-[1,1'-biphenyl]-2-yl)methyl)-3,8-diazabicyclo[3.2.1]octan-8-yl)-1-oxoisoindolin-2-yl)piperidine-2,6-dione |
| GT-05607 | 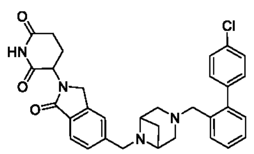 | 3-(5-((3-((4'-chloro-[1,1'-biphenyl]-2-yl)methyl)-3,6-diazabicyclo[3.1.1]heptan-6-yl)methyl)-1-oxoisoindolin-2-yl)piperidine-2,6-dione | 3-(5-((3-((4'-chloro-[1,1'-biphenyl]-2-yl)methyl)-3,6-diazabicyclo[3.1.1]heptan-6-yl)methyl)-1-oxoisoindolin-2-yl)piperidine-2,6-dione |

(continued)

| Compound No. | Structure of the compounds | The compounds' name | Chinese translation of the compounds' name |
|---|---|---|---|
| GT-05608 | 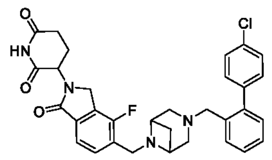 | 3-(5-((3-((4'-chloro-[1,1'-biphenyl]-2-yl)methyl)-3,6-diazabicyclo[3.1.1]heptan-6-yl)methyl)-4-fluoro-1-oxoisoindolin-2-yl)piperidine-2,6-dione | 3-(5-((3-((4'-chloro-[1,1'-biphenyl]-2-yl)methyl)-3,6-diazabicyclo[3.1.1]heptan-6-yl)methyl)-4-fluoro-1-oxoisoindolin-2-yl)piperidine-2,6-dione |
| GT-05609 | 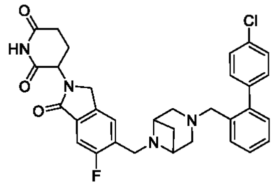 | 3-(5-((3-((4'-chloro-[1,1'-biphenyl]-2-yl)methyl)-3,6-diazabicyclo[3.1.1]heptan-6-yl)methyl)-6-fluoro-1-oxoisoindolin-2-yl)piperidine-2,6-dione | 3-(5-((3-((4'-chloro-[1,1'-biphenyl]-2-yl)methyl)-3,6-diazabicyclo[3.1.1]heptan-6-yl)methyl)-6-fluoro-1-oxoisoindolin-2-yl)piperidine-2,6-dione |
| GT-05610 | 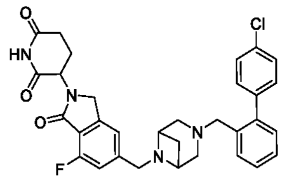 | 3-(5-((3-((4'-chloro-[1,1'-biphenyl]-2-yl)methyl)-3,6-diazabicyclo[3.1.1]heptan-6-yl)methyl)-7-fluoro-1-oxoisoindolin-2-yl)piperidine-2,6-dione | 3-(5-((3-((4'-chloro-[1,1'-biphenyl]-2-yl)methyl)-3,6-diazabicyclo[3.1.1]heptan-6-yl)methyl)-7-fluoro-1-oxoisoindolin-2-yl)piperidine-2,6-dione |
| GT-05704 | 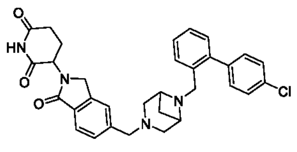 | 3-(5-((6-((4'-chloro-[1,1'-biphenyl]-2-yl)methyl)-3,6-diazabicyclo[3.1.1]heptan-3-yl)methyl)-1-oxoisoindolin-2-yl)piperidine-2,6-dione | 3-(5-((6-((4'-chloro-[1,1'-biphenyl]-2-yl)methyl)-3,6-diazabicyclo[3.1.1]heptan-3-yl)methyl)-1-oxoisoindolin-2-yl)piperidine-2,6-dione |
| GT-05710 | 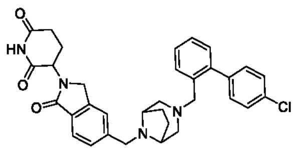 | 3-(5-((3-((4'-chloro-[1,1'-biphenyl]-2-yl)methyl)-3,8-diazabicyclo[3.2.1]octan-8-yl)methyl)-1-oxoisoindolin-2-yl)piperidine-2,6-dione | 3-(5-((3-((4'-chloro-[1,1'-biphenyl]-2-yl)methyl)-3,8-diazabicyclo[3.2.1]octan-8-yl)methyl)-1-oxoisoindolin-2-yl)piperidine-2,6-dione |
| GT-05618 | 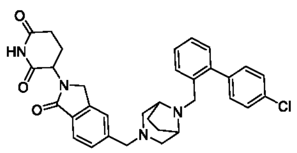 | 3-(5-((8-((4'-chloro-[1,1'-biphenyl]-2-yl)methyl)-3,8-diazabicyclo[3.2.1]octan-3-yl)methyl)-1-oxoisoindolin-2-yl)piperidine-2,6-dione | 3-(5-((8-((4'-chloro-[1,1'-biphenyl]-2-yl)methyl)-3,8-diazabicyclo[3.2.1]octan-3-yl)methyl)-1-oxoisoindolin-2-yl)piperidine-2,6-dione |
| GT-05619 | 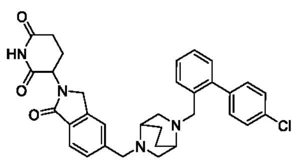 | 3-(5-((5-((4'-chloro-[1,1'-biphenyl]-2-yl)methyl)-2,5-diazabicyclo[2.2.2]octan-2-yl)methyl)-1-oxoisoindolin-2-yl)piperidine-2,6-dione | 3-(5-((5-((4'-chloro-[1,1'-biphenyl]-2-yl)methyl)-2,5-diazabicyclo[2.2.2]octan-2-yl)methyl)-1-oxoisoindolin-2-yl)piperidine-2,6-dione |
| | 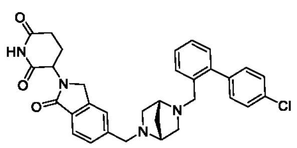 | 3-(5-(((1R,4R)-5-((4'-chloro-[1,1'-biphenyl]-2-yl) methyl)-2,5-diazabicyclo[2.2.1] heptan-2-yl)methyl)-1-oxoisoindolin-2-yl)piperidine-2,6-dione | 3-(5-(((1R,4R)-5-((4'-chloro-[1,1'-biphenyl]-2-yl) methyl)-2,5-diazabicyclo[2.2.1] heptan-2-yl)methyl)-1-oxoisoindolin-2-yl)piperidine-2,6-dione |
| | 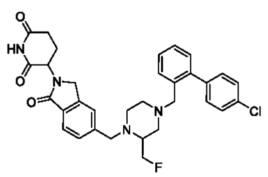 | 3-(5-((4-((4'-chloro-[1,1'-biphenyl]-2-yl)methyl)-2-(fluoromethyl)piperazin-1-yl) methyl)-1-oxoisoindolin-2-yl)piperidine-2,6-dione | 3-(5-((4-((4'-chloro-[1,1'-biphenyl]-2-yl)methyl)-2-(fluoromethyl)piperazin-1-yl) methyl)-1-oxoisoindolin-2-yl)piperidine-2,6-dione |

(continued)

| Compound No. | Structure of the compounds | The compounds' name | Chinese translation of the compounds' name |
|---|---|---|---|
| | 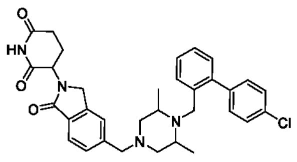 | 3-(5-((4-((4'-chloro-[1,1'-biphenyl]-2-yl)methyl)-3,5-dimethylpiperazin-1-yl)methyl)-1-oxoisoindolin-2-yl)piperidine-2,6-dione | 3-(5-((4-((4'-chloro-[1,1'-biphenyl]-2-yl)methyl)-3,5-dimethylpiperazin-1-yl)methyl)-1-oxoisoindolin-2-yl)piperidine-2,6-dione |
| GT-05711 | 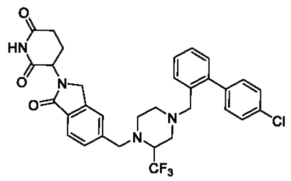 | 3-(5-((4-((4'-chloro-[1,1'-biphenyl]-2-yl)methyl)-2-(trifluoromethyl)piperazin-1-yl) methyl)-1-oxoisoindolin-2-yl)piperidine-2,6-dione | 3-(5-((4-((4'-chloro-[1,1'-biphenyl]-2-yl)methyl)-2-(trifluoromethyl)piperazin-1-yl) methyl)-1-oxoisoindolin-2-yl)piperidine-2,6-dione |
| | 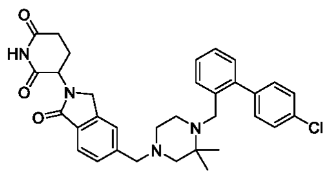 | 3-(5-((4-((4'-chloro-[1,1'-biphenyl]-2-yl)methyl)-3,3-dimethylpiperazin-1-yl)methyl)-1-oxoisoindolin-2-yl)piperidine-2,6-dione | 3-(5-((4-((4'-chloro-[1,1'-biphenyl]-2-yl)methyl)-3,3-dimethylpiperazin-1-yl)methyl)-1-oxoisoindolin-2-yl)piperidine-2,6-dione |
| | 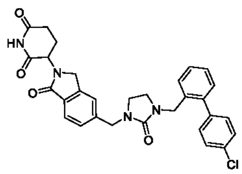 | 3-(5-((3-((4'-chloro-[1,1'-biphenyl]-2-yl)methyl)-2-oxoimidazolidin-1-yl)methyl)-1-oxoisoindolin-2-yl)piperidine-2,6-dione | 3-(5-((3-((4'-chloro-[1,1'-biphenyl]-2-yl)methyl)-2-oxoimidazolidin-1-yl)methyl)-1-oxoisoindolin-2-yl)piperidine-2,6-dione |
| | 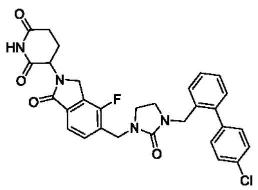 | 3-(5-((3-((4'-chloro-[1,1'-biphenyl]-2-yl)methyl)-2-oxoimidazolidin-1-yl)methyl)-4-fluoro-1-oxoisoindolin-2-yl)piperidine-2,6-dione | 3-(5-((3-((4'-chloro-[1,1'-biphenyl]-2-yl)methyl)-2-oxoimidazolidin-1-yl)methyl)-4-fluoro-1-oxoisoindolin-2-yl)piperidine-2,6-dione |
| | 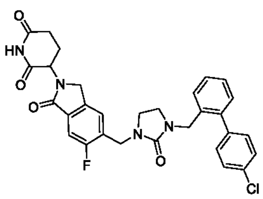 | 3-(5-((3-((4'-chloro-[1,1'-biphenyl]-2-yl)methyl)-2-oxoimidazolidin-1-yl)methyl)-6-fluoro-1-oxoisoindolin-2-yl)piperidine-2,6-dione | 3-(5-((3-((4'-chloro-[1,1'-biphenyl]-2-yl)methyl)-2-oxoimidazolidin-1-yl)methyl)-6-fluoro-1-oxoisoindolin-2-yl)piperidine-2,6-dione |
| | 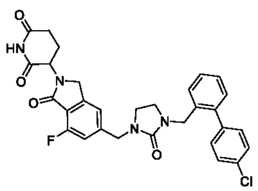 | 3-(5-((3-((4'-chloro-[1,1'-biphenyl]-2-yl)methyl)-2-oxoimidazolidin-1-yl)methyl)-7-fluoro-1-oxoisoindolin-2-yl)piperidine-2,6-dione | 3-(5-((3-((4'-chloro-[1,1'-biphenyl]-2-yl)methyl)-2-oxoimidazolidin-1-yl)methyl)-7-fluoro-1-oxoisoindolin-2-yl)piperidine-2,6-dione |
| | 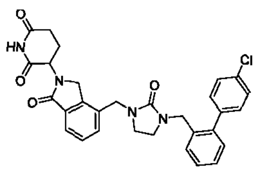 | 3-(4-((3-((4'-chloro-[1,1'-biphenyl]-2-yl)methyl)-2-oxoimidazolidin-1-yl)methyl)-1-oxoisoindolin-2-yl)piperidine-2,6-dione | 3-(4-((3-((4'-chloro-[1,1'-biphenyl]-2-yl)methyl)-2-oxoimidazolidin-1-yl)methyl)-1-oxoisoindolin-2-yl)piperidine-2,6-dione |

(continued)

| Compound No. | Structure of the compounds | The compounds' name | Chinese translation of the compounds' name |
|---|---|---|---|
| GT-04275 | 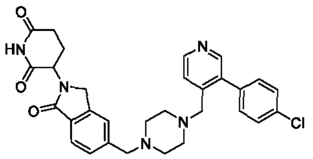 | 3-(5-((4-((3-(4-chlorophenyl) pyridin-4-yl)methyl)piperazin-1-yl)methyl)-1-oxoisoindolin-2-yl) piperidine-2,6-dione | 3-(5-((4-((3-(4-chlorophenyl) pyridin-4-yl)methyl)piperazin-1-yl)methyl)-1-oxoisoindolin-2-yl) piperidine-2,6-dione |
| GT-05703 | 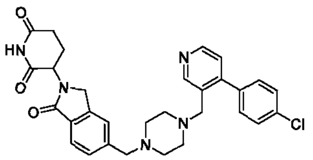 | 3-(5-((4-((4-(4-chlorophenyl) pyridin-3-yl)methyl)piperazin-1-yl)methyl)-1-oxoisoindolin-2-yl) piperidine-2,6-dione | 3-(5-((4-((4-(4-chlorophenyl) pyridin-3 - yl)methyl)piperazin-1-yl)methyl)-1-oxoisoindolin-2-yl)piperidine-2,6-dione |
| GT-05778 | 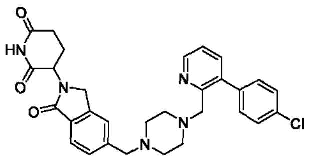 | 3-(5-((4-((3-(4-chlorophenyl) pyridin-2-yl)methyl)piperazin-1-yl)methyl)-1-oxoisoindolin-2-yl) piperidine-2,6-dione | 3-(5-((4-((3-(4-chlorophenyl) pyridin-2-yl)methyl)piperazin-1-yl)methyl)-1-oxoisoindolin-2-yl) piperidine-2,6-dione |
| GT-05860 | 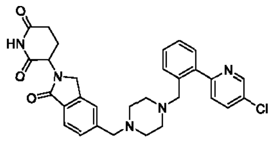 | 3-(5-((4-(2-(5-chloropyridin-2-yl)benzyl)piperazin-1-yl) methyl)-1-oxoisoindolin-2-yl)piperidine-2,6-dione | 3-(5-((4-(2-(5-chloropyridin-2-yl)benzyl)piperazin-1-yl) methyl)-1-oxoisoindolin-2-yl)piperidine-2,6-dione |
| GT-05629 | 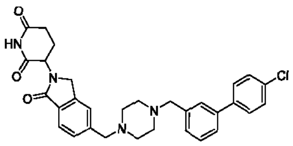 | 3-(5-((4-((4'-chloro-[1,1'-biphenyl]-3-yl)methyl)piperazin-1-yl) methyl)-1-oxoisoindolin-2-yl)piperidine-2,6-dione | 3-(5-((4-((4'-chloro-[1,1'-biphenyl]-3-yl)methyl)piperazin-1-yl) methyl)-1-oxoisoindolin-2-yl)piperidine-2,6-dione |
| GT-05596 | 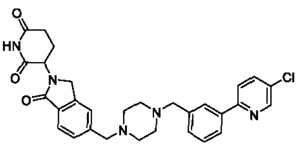 | 3-(5-((4-(3-(5-chloropyridin-2-yl)benzyl)piperazin-1-yl) methyl)-1-oxoisoindolin-2-yl)piperidine-2,6-dione | 3-(5-((4-(3-(5-chloropyridin-2-yl)benzyl)piperazin-1-yl) methyl)-1-oxoisoindolin-2-yl)piperidine-2,6-dione |
| GT-05597 | 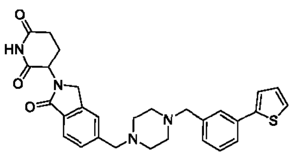 | 3-(1-oxo-5-((4-(3-(thiophen-2-yl)benzyl)piperazin-1-yl)methyl) isoindolin-2-yl)piperidine-2,6-dione | 3-(1-oxo-5-((4-(3-(thiophen-2-yl)benzyl)piperazin-1-yl)methyl) isoindolin-2-yl)piperidine-2,6-dione |
| GT-05692 | 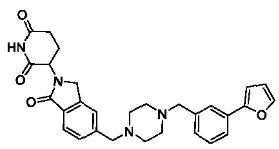 | 3-(5-((4-(3-(furan-2-yl)benzyl) piperazin-1-yl)methyl)-1-oxoisoindolin-2-yl)piperidine-2,6-dione | 3-(5-((4-(3-(furan-2-yl)benzyl) piperazin-1-yl)methyl)-1-oxoisoindolin-2-yl)piperidine-2,6-dione |
| GT-05733 | 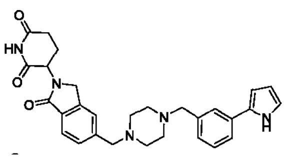 | 3-(5-((4-(3-(1H-pyrrol-2-yl)benzyl)piperazin-1-yl)methyl)-1-oxoisoindolin-2-yl)piperidine-2,6-dione | 3-(5-((4-(3-(1H-pyrrol-2-yl)benzyl)piperazin-1-yl)methyl)-1-oxoisoindolin-2-yl)piperidine-2,6-dione |

(continued)

| Compo und No. | Structure of the compounds | The compounds' name | Chinese translation of the compounds' name |
|---|---|---|---|
| GT-05598 | 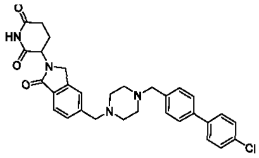 | 3-(5-((4-((4'-chloro-[1,1'-biphenyl]-4-yl)methyl)piperazin-1-yl) methyl)-1-oxoisoindolin-2-yl)piperidine-2,6-dione | 3-(5-((4-((4'-chloro-[1,1'-biphenyl]-4-yl)methyl)piperazin-1-yl) methyl)-1-oxoisoindolin-2-yl)piperidine-2,6-dione |
| GT-05872 | 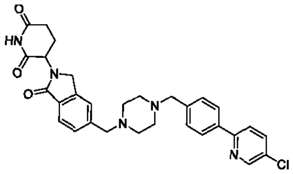 | 3-(5-((4-(4-(5-chloropyridin-2-yl)benzyl)piperazin-1-yl) methyl)-1-oxoisoindolin-2-yl)piperidine-2,6-dione | 3-(5-((4-(4-(5-chloropyridin-2-yl)benzyl)piperazin-1-yl) methyl)-1-oxoisoindolin-2-yl)piperidine-2,6-dione |
| GT-05640 | 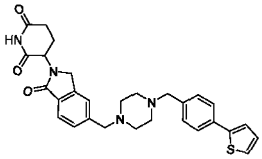 | 3-(1-oxo-5-((4-(4-(thiophen-2-yl)benzyl)piperazin-1-yl)methyl) isoindolin-2-yl)piperidine-2,6-dione | 3-(1-oxo-5-((4-(4-(thiophen-2-yl)benzyl)piperazin-1-yl)methyl) isoindolin-2-yl)piperidine-2,6-dione |
| GT-05693 | 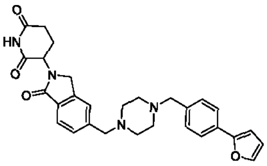 | 3-(5-((4-(4-(furan-2-yl)benzyl) piperazin-1-yl)methyl)-1-oxoisoindolin-2-yl)piperidine-2,6-dione | 3-(5-((4-(4-(furan-2-yl)benzyl) piperazin-1-yl)methyl)-1-oxoisoindolin-2-yl)piperidine-2,6-dione |
| GT-05734 | 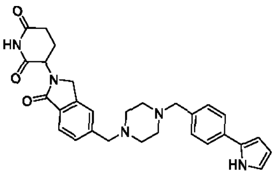 | 3-(5-((4-(4-(1H-pyrrol-2-yl)benzyl)piperazin-1-yl)methyl)-1-oxoisoindolin-2-yl)piperidine-2,6-dione | 3-(5-((4-(4-(1H-pyrrol-2-yl)benzyl)piperazin-1-yl)methyl)-1-oxoisoindolin-2-yl)piperidine-2,6-dione |
| GT-05632 | 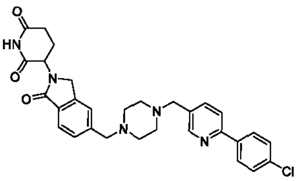 | 3-(5-((4-((6-(4-chlorophenyl) pyridin-3-yl)methyl)piperazin-1-yl)methyl)-1-oxoisoindolin-2-yl) piperidine-2,6-dione | 3-(5-((4-((6-(4-chlorophenyl) pyridin-3-yl)methyl)piperazin-1-yl)methyl)-1-oxoisoindolin-2-yl) piperidine-2,6-dione |
| GT-05633 | 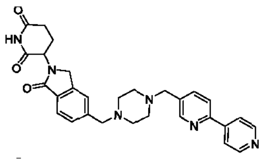 | 3-(5-((4-([2,4'-bipyridin]-5-ylmethyl)piperazin-1-yl) methyl)-1-oxoisoindolin-2-yl)piperidine-2,6-dione | 3-(5-((4-([2,4'-bipyridin]-5-ylmethyl)piperazin-1-yl) methyl)-1-oxoisoindolin-2-yl)piperidine-2,6-dione |
| GT-05544 | 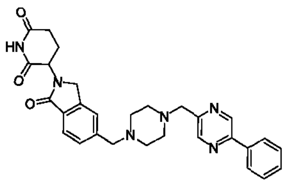 | 3-(1-oxo-5-((4-((5-phenylpyrazin-2-yl)methyl)piperazin-1-yl) methyl)isoindolin-2-yl)piperidine-2,6-dione | 3-(1-oxo-5-((4-((5-phenylpyrazin-2-yl)methyl)piperazin-1-yl) methyl)isoindolin-2-yl)piperidine-2,6-dione |
| | 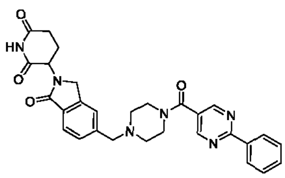 | 3-(1-oxo-5-((4-(2-phenylpyrimidine-5-carbonyl)piperazin-1-yl) methyl)isoindolin-2-yl)piperidine-2,6-dione | 3-(1-oxo-5-((4-(2-phenylpyrimidine-5-carbonyl)piperazin-1-yl) methyl)isoindolin-2-yl)piperidine-2,6-dione |

(continued)

| Compo und No. | Structure of the compounds | The compounds' name | Chinese translation of the compounds' name |
|---|---|---|---|
| GT-05625 | 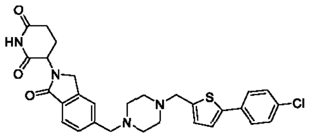 | 3-(5-((4-((5-(4-chlorophenyl) thiophen-2-yl)methyl)piperazin-1-yl)methyl)-1-oxoisoindolin-2-yl)piperidine-2,6-dione | 3-(5-((4-((5-(4-chlorophenyl) thiophen-2-yl)methyl)piperazin-1-yl)methyl)-1-oxoisoindolin-2-yl)piperidine-2,6-dione |
| GT-05624 | 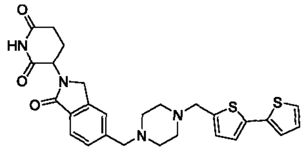 | 3-(5-((4-([2,2'-bithiophen]-5-ylmethyl)piperazin-1-yl) methyl)-1-oxoisoindolin-2-yl)piperidine-2,6-dione | 3-(5-((4-([2,2'-bithiophen]-5-ylmethyl)piperazin-1-yl) methyl)-1-oxoisoindolin-2-yl)piperidine-2,6-dione |
| GT-05780 | 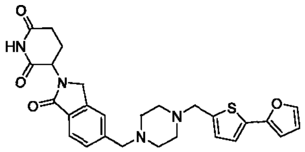 | 3-(5-((4-((5-(furan-2-yl)thiophen-2-yl)methyl)piperazin-1-yl)methyl)-1-oxoisoindolin-2-yl) piperidine-2,6-dione | 3-(5-((4-((5-(furan-2-yl)thiophen-2-yl)methyl)piperazin-1-yl)methyl)-1-oxoisoindolin-2-yl) piperidine-2,6-dione |
| GT-05694 | 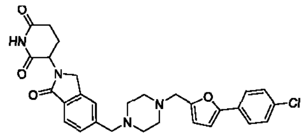 | 3-(5-((4-((5-(4-chlorophenyl)furan-2-yl)methyl)piperazin-1-yl) methyl)-1-oxoisoindolin-2-yl)piperidine-2,6-dione | 3-(5-((4-((5-(4-chlorophenyl)furan-2-yl)methyl)piperazin-1-yl) methyl)-1-oxoisoindolin-2-yl)piperidine-2,6-dione |
| GT-05627 | 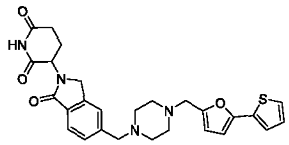 | 3-(1-oxo-5-((4-((5-(thiophen-2-yl)furan-2-yl)methyl)piperazin-1-yl)methyl)isoindolin-2-yl) piperidine-2,6-dione | 3-(1-oxo-5-((4-((5-(thiophen-2-yl)furan-2-yl)methyl)piperazin-1-yl)methyl)isoindolin-2-yl) piperidine-2,6-dione |
| GT-05695 | 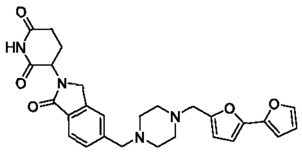 | 3-(5-((4-([2,2'-bifuran]-5-ylmethyl)piperazin-1-yl) methyl)-1-oxoisoindolin-2-yl)piperidine-2,6-dione | 3-(5-((4-([2,2'-bifuran]-5-ylmethyl)piperazin-1-yl) methyl)-1-oxoisoindolin-2-yl)piperidine-2,6-dione |
| GT-03891 | 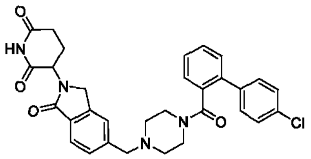 | 3-(5-((4-(4'-chloro-[1,1'-biphenyl]-2-carbonyl)piperazin-1-yl) methyl)-1-oxoisoindolin-2-yl)piperidine-2,6-dione | 3-(5-((4-(4'-chloro-[1,1'-biphenyl]-2-carbonyl)piperazin-1-yl) methyl)-1-oxoisoindolin-2-yl)piperidine-2,6-dione |
| GT-04222 | 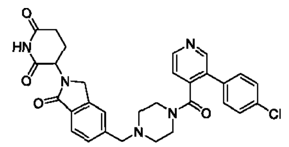 | 3-(5-((4-(3-(4-chlorophenyl)isonicotinoyl) piperazin-1-yl) methyl)-1-oxoisoindolin-2-yl)piperidine-2,6-dione | 3-(5-((4-(3-(4-chlorophenyl)isonicotinoyl)pi perazin-1-yl) methyl)-1-oxoisoindolin-2-yl)piperidine-2,6-dione |
| GT-05649 | 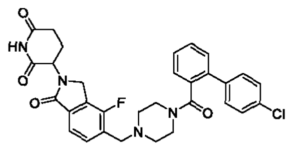 | 3-(5-((4-(4'-chloro-[1,1'-biphenyl]-2-carbonyl)piperazin-1-yl) methyl)-4-fluoro-1-oxoisoindolin-2-yl)piperidine-2,6-dione | 3-(5-((4-(4'-chloro-[1,1'-biphenyl]-2-carbonyl)piperazin-1-yl) methyl)-4-fluoro-1-oxoisoindolin-2-yl)piperidine-2,6-dione |
| GT-05650 | 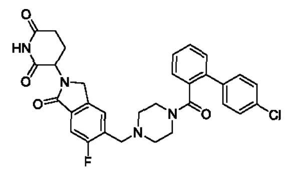 | 3-(5-((4-(4'-chloro-[1,1'-biphenyl]-2-carbonyl)piperazin-1-yl) methyl)-6-fluoro-1-oxoisoindolin-2-yl)piperidine-2,6-dione | 3-(5-((4-(4'-chloro-[1,1'-biphenyl]-2-carbonyl)piperazin-1-yl) methyl)-6-fluoro-1-oxoisoindolin-2-yl)piperidine-2,6-dione |

(continued)

| Compound No. | Structure of the compounds | The compounds' name | Chinese translation of the compounds' name |
|---|---|---|---|
| GT-05700 | 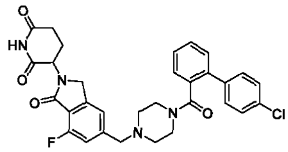 | 3-(5-((4-(4'-chloro-[1,1'-biphenyl]-2-carbonyl)piperazin-1-yl)methyl)-7-fluoro-1-oxoisoindolin-2-yl)piperidine-2,6-dione | 3-(5-((4-(4'-chloro-[1,1'-biphenyl]-2-carbonyl)piperazin-1-yl)methyl)-7-fluoro-1-oxoisoindolin-2-yl)piperidine-2,6-dione |
| GT-05701 | 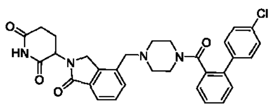 | 3-(4-((4-(4'-chloro-[1,1'-biphenyl]-2-carbonyl)piperazin-1-yl)methyl)-1-oxoisoindolin-2-yl)piperidine-2,6-dione | 3-(4-((4-(4'-chloro-[1,1'-biphenyl]-2-carbonyl)piperazin-1-yl)methyl)-1-oxoisoindolin-2-yl)piperidine-2,6-dione |
| GT-05784 | 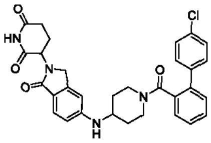 | 3-(5-((1-(4'-chloro-[1,1'-biphenyl]-2-carbonyl)piperidin-4-yl)amino)-1-oxoisoindolin-2-yl)piperidine-2,6-dione | 3-(5-((1-(4'-chloro-[1,1'-biphenyl]-2-carbonyl)piperidin-4-yl)amino)-1-oxoisoindolin-2-yl)piperidine-2,6-dione |
| GT-05467 | 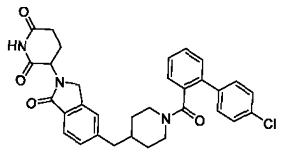 | 3-(5-((1-(4'-chloro-[1,1'-biphenyl]-2-carbonyl)piperidin-4-yl)methyl)-1-oxoisoindolin-2-yl)piperidine-2,6-dione | 3-(5-((1-(4'-chloro-[1,1'-biphenyl]-2-carbonyl)piperidin-4-yl)methyl)-1-oxoisoindolin-2-yl)piperidine-2,6-dione |
| GT-04397 | 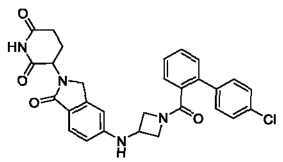 | 3-(5-((1-(4'-chloro-[1,1'-biphenyl]-2-carbonyl)azetidin-3-yl)amino)-1-oxoisoindolin-2-yl)piperidine-2,6-dione | 3-(5-((1-(4'-chloro-[1,1'-biphenyl]-2-carbonyl)azetidin-3-yl)amino)-1-oxoisoindolin-2-yl)piperidine-2,6-dione |
| GT-03922 | 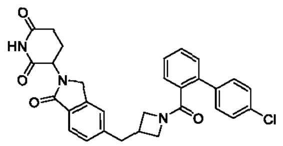 | 3-(5-((1-(4'-chloro-[1,1'-biphenyl]-2-carbonyl)azetidin-3-yl)methyl)-1-oxoisoindolin-2-yl)piperidine-2,6-dione | 3-(5-((1-(4'-chloro-[1,1'-biphenyl]-2-carbonyl)azetidin-3-yl)methyl)-1-oxoisoindolin-2-yl)piperidine-2,6-dione |
| GT-05484 | 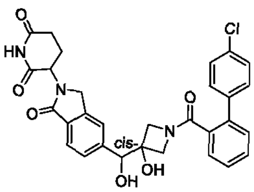 | 3-(5-((1-(4'-chloro-[1,1'-biphenyl]-2-carbonyl)-3-hydroxyazetidin-3-yl)(hydroxy)methyl)-1-oxoisoindolin-2-yl)piperidine-2,6-dione | 3-(5-((1-(4'-chloro-[1,1'-biphenyl]-2-carbonyl)-3-hydroxyazetidin-3-yl)(hydroxy)methyl)-1-oxoisoindolin-2-yl)piperidine-2,6-dione |
| GT-05482 | 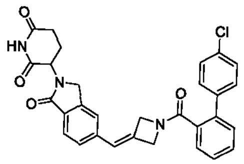 | 3-(5-((1-(4'-chloro-[1,1'-biphenyl]-2-carbonyl)azetidin-3-ylidene)methyl)-1-oxoisoindolin-2-yl)piperidine-2,6-dione | 3-(5-((1-(4'-chloro-[1,1'-biphenyl]-2-carbonyl)azetidin-3-ylidene)methyl)-1-oxoisoindolin-2-yl)piperidine-2,6-dione |
| | 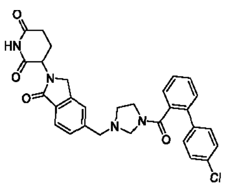 | 3-(5-((3-(4'-chloro-[1,1'-biphenyl]-2-carbonyl)imidazolidin-1-yl)methyl)-1-oxoisoindolin-2-yl)piperidine-2,6-dione | 3-(5-((3-(4'-chloro-[1,1'-biphenyl]-2-carbonyl)imidazolidin-1-yl)methyl)-1-oxoisoindolin-2-yl)piperidine-2,6-dione |

(continued)

| Compound No. | Structure of the compounds | The compounds' name | Chinese translation of the compounds' name |
|---|---|---|---|
| GT-05644 | 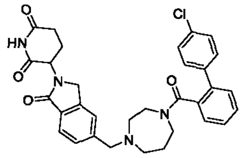 | 3-(5-((4-(4'-chloro-[1,1'-biphenyl]-2-carbonyl)-1,4-diazepan-1-yl)methyl)-1-oxoisoindolin-2-yl)piperidine-2,6-dione | 3-(5-((4-(4'-chloro-[1,1'-biphenyl]-2-carbonyl)-1,4-diazepan-1-yl)methyl)-1-oxoisoindolin-2-yl)piperidine-2,6-dione |
| GT-05874 | 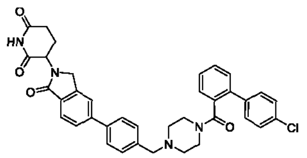 | 3-(5-(4-((4-(4'-chloro-[1,1'-biphenyl]-2-carbonyl)piperazin-1-yl)methyl)phenyl)-1-oxoisoindolin-2-yl)piperidine-2,6-dione | 3-(5-(4-((4-(4'-chloro-[1,1'-biphenyl]-2-carbonyl)piperazin-1-yl)methyl)phenyl)-1-oxoisoindolin-2-yl)piperidine-2,6-dione |
| GT-05620 | 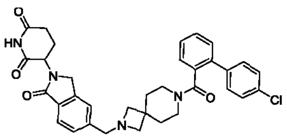 | 3-(5-((7-(4'-chloro-[1,1'-biphenyl]-2-carbonyl)-2,7-diazaspiro [3.5]nonan-2-yl)methyl)-1-oxoisoindolin-2-yl)piperidine-2,6-dione | 3-(5-((7-(4'-chloro-[1,1'-biphenyl]-2-carbonyl)-2,7-diazaspiro [3.5]nonan-2-yl)methyl)-1-oxoisoindolin-2-yl)piperidine-2,6-dione |
| GT-05708 | 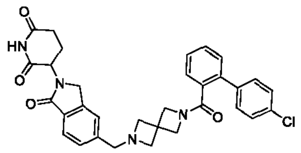 | 3-(5-((6-(4'-chloro-[1,1'-biphenyl]-2-carbonyl)-2,6-diazaspiro [3.3]heptan-2-yl)methyl)-1-oxoisoindolin-2-yl)piperidine-2,6-dione | 3-(5-((6-(4'-chloro-[1,1'-biphenyl]-2-carbonyl)-2,6-diazaspiro [3.3]heptan-2-yl)methyl)-1-oxoisoindolin-2-yl)piperidine-2,6-dione |
| GT-05645 | 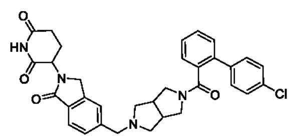 | 3-(5-((5-(4'-chloro-[1,1'-biphenyl]-2-carbonyl)hexahydropyrrolo[ 3,4-c]pyrrol-2(1H)-yl) methyl)-1-oxoisoindolin-2-yl)piperidine-2,6-dione | 3-(5-((5-(4'-chloro-[1,1'-biphenyl]-2-carbonyl)hexahydropyrrolo[3 ,4-c]pyrrol-2(1H)-yl) methyl)-1-oxoisoindolin-2-yl)piperidine-2,6-dione |
| GT-05875 | 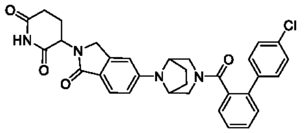 | 3-(5-(3-(4'-chloro-[1,1'-biphenyl]-2-carbonyl)-3,8-diazabicyclo[3.2.1]octan-8-yl)-1-oxoisoindolin-2-yl)piperidine-2,6-dione | 3-(5-(3-(4'-chloro-[1,1'-biphenyl]-2-carbonyl)-3,8-diazabicyclo[3.2.1]octan-8-yl)-1-oxoisoindolin-2-yl)piperidine-2,6-dione |
| GT-05665 | 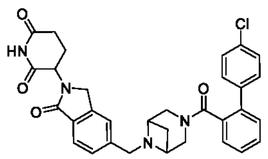 | 3-(5-((3-(4'-chloro-[1,1'-biphenyl]-2-carbonyl)-3,6-diazabicyclo[3.1.1]heptan-6-yl)methyl)-1-oxoisoindolin-2-yl)piperidine-2,6-dione | 3-(5-((3-(4'-chloro-[1,1'-biphenyl]-2-carbonyl)-3,6-diazabicyclo[3.1.1]heptan-6-yl)methyl)-1-oxoisoindolin-2-yl)piperidine-2,6-dione |
| GT-05666 | 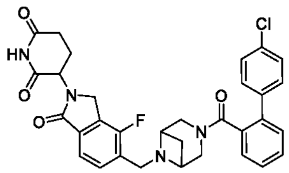 | 3-(5-((3-(4'-chloro-[1,1'-biphenyl]-2-carbonyl)-3,6-diazabicyclo[3.1.1]heptan-6-yl)methyl)-4-fluoro-1-oxoisoindolin-2-yl)piperidine-2,6-dione | 3-(5-((3-(4'-chloro-[1,1'-biphenyl]-2-carbonyl)-3,6-diazabicyclo[3.1.1]heptan-6-yl)methyl)-4-fluoro-1-oxoisoindolin-2-yl)piperidine-2,6-dione |
| GT-05667 | 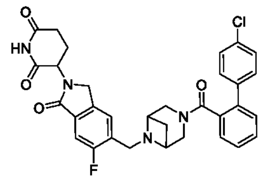 | 3-(5-((3-(4'-chloro-[1,1'-biphenyl]-2-carbonyl)-3,6-diazabicyclo[3.1.1]heptan-6-yl)methyl)-6-fluoro-1-oxoisoindolin-2-yl)piperidine-2,6-dione | 3-(5-((3-(4'-chloro-[1,1'-biphenyl]-2-carbonyl)-3,6-diazabicyclo[3.1.1]heptan-6-yl)methyl)-6-fluoro-1-oxoisoindolin-2-yl)piperidine-2,6-dione |

(continued)

| Compound No. | Structure of the compounds | The compounds' name | Chinese translation of the compounds' name |
|---|---|---|---|
| GT-05668 | 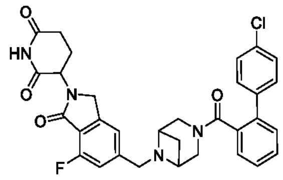 | 3-(5-((3-(4'-chloro-[1,1'-biphenyl]-2-carbonyl)-3,6-diazabicyclo[3.1.1]heptan-6-yl)methyl)-7-fluoro-1-oxoisoindolin-2-yl)piperidine-2,6-dione | 3-(5-((3-(4'-chloro-[1,1'-biphenyl]-2-carbonyl)-3,6-diazabicyclo[3.1.1]heptan-6-yl)methyl)-7-fluoro-1-oxoisoindolin-2-yl)piperidine-2,6-dione |
| GT-05669 | 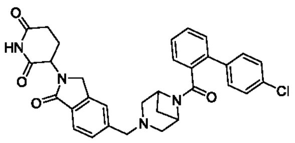 | 3-(5-((6-(4'-chloro-[1,1'-biphenyl]-2-carbonyl)-3,6-diazabicyclo[3.1.1]heptan-3-yl)methyl)-1-oxoisoindolin-2-yl)piperidine-2,6-dione | 3-(5-((6-(4'-chloro-[1,1'-biphenyl]-2-carbonyl)-3,6-diazabicyclo[3.1.1]heptan-3-yl)methyl)-1-oxoisoindolin-2-yl)piperidine-2,6-dione |
| GT-05788 | 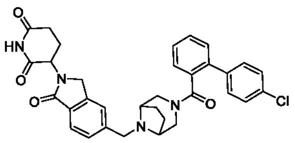 | 3-(5-((3-(4'-chloro-[1,1'-biphenyl]-2-carbonyl)-3,8-diazabicyclo[3.2.1]octan-8-yl)methyl)-1-oxoisoindolin-2-yl)piperidine-2,6-dione | 3-(5-((3-(4'-chloro-[1,1'-biphenyl]-2-carbonyl)-3,8-diazabicyclo[3.2.1]octan-8-yl)methyl)-1-oxoisoindolin-2-yl)piperidine-2,6-dione |
| GT-05542 | 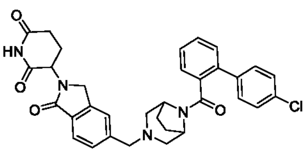 | 3-(5-((8-(4'-chloro-[1,1'-biphenyl]-2-carbonyl)-3,8-diazabicyclo[3.2.1]octan-3-yl)methyl)-1-oxoisoindolin-2-yl)piperidine-2,6-dione | 3-(5-((8-(4'-chloro-[1,1'-biphenyl]-2-carbonyl)-3,8-diazabicyclo[3.2.1]octan-3-yl)methyl)-1-oxoisoindolin-2-yl)piperidine-2,6-dione |
| GT-05543 | 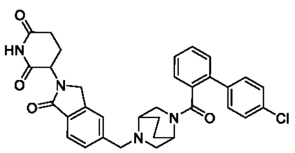 | 3-(5-((5-(4'-chloro-[1,1'-biphenyl]-2-carbonyl)-2,5-diazabicyclo[2.2.2]octan-2-yl)methyl)-1-oxoisoindolin-2-yl)piperidine-2,6-dione | 3-(5-((5-(4'-chloro-[1,1'-biphenyl]-2-carbonyl)-2,5-diazabicyclo[2.2.2]octan-2-yl)methyl)-1-oxoisoindolin-2-yl)piperidine-2,6-dione |
| | 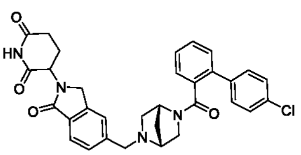 | 3-(5-(((1R,4R)-5-(4'-chloro-[1,1'-biphenyl]-2-carbonyl)-2,5-diazabicyclo[2.2.1]heptan-2-yl)methyl)-1-oxoisoindolin-2-yl)piperidine-2,6-dione | 3-(5-(((1R,4R)-5-(4'-chloro-[1,1'-biphenyl]-2-carbonyl)-2,5-diazabicyclo[2.2.1]heptan-2-yl)methyl)-1-oxoisoindolin-2-yl)piperidine-2,6-dione |
| | 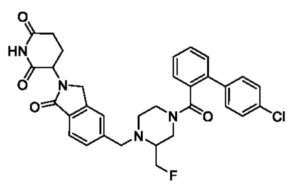 | 3-(5-((4-(4'-chloro-[1,1'-biphenyl]-2-carbonyl)-2-(fluoromethyl)piperazin-1-yl)methyl)-1-oxoisoindolin-2-yl)piperidine-2,6-dione | 3-(5-((4-(4'-chloro-[1,1'-biphenyl]-2-carbonyl)-2-(fluoromethyl)piperazin-1-yl)methyl)-1-oxoisoindolin-2-yl)piperidine-2,6-dione |
| | 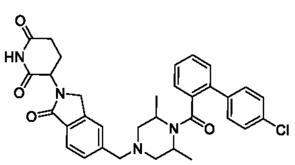 | 3-(5-((4-(4'-chloro-[1,1'-biphenyl]-2-carbonyl)-3,5-dimethylpiperazin-1-yl)methyl)-1-oxoisoindolin-2-yl)piperidine-2,6-dione | 3-(5-((4-(4'-chloro-[1,1'-biphenyl]-2-carbonyl)-3,5-dimethylpiperazin-1-yl)methyl)-1-oxoisoindolin-2-yl)piperidine-2,6-dione |
| GT-05789 | 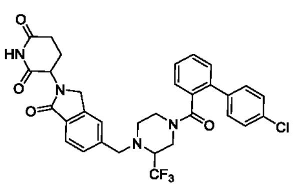 | 3-(5-((4-(4'-chloro-[1,1'-biphenyl]-2-carbonyl)-2-(trifluoromethyl)piperazin-1-yl)methyl)-1-oxoisoindolin-2-yl)piperidine-2,6-dione | 3-(5-((4-(4'-chloro-[1,1'-biphenyl]-2-carbonyl)-2-(trifluoromethyl)piperazin-1-yl)methyl)-1-oxoisoindolin-2-yl)piperidine-2,6-dione |

(continued)

| Compound No. | Structure of the compounds | The compounds' name | Chinese translation of the compounds' name |
|---|---|---|---|
| | 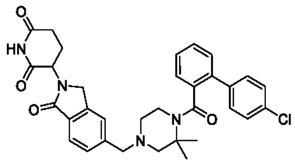 | 3-(5-((4-(4'-chloro-[1,1'-biphenyl]-2-carbonyl)-3,3-dimethylpiperazin-1-yl)methyl)-1-oxoisoindolin-2-yl)piperidine-2,6-dione | 3-(5-((4-(4'-chloro-[1,1'-biphenyl]-2-carbonyl)-3,3-dimethylpiperazin-1-yl)methyl)-1-oxoisoindolin-2-yl)piperidine-2,6-dione |
| GT-05455 | 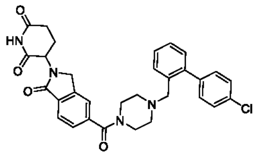 | 3-(5-(4-((4'-chloro-[1,1'-biphenyl]-2-yl)methyl)piperazine-1-carbonyl)-1-oxoisoindolin-2-yl) piperidine-2,6-dione | 3-(5-(4-((4'-chloro-[1,1'-biphenyl]-2-yl)methyl)piperazine-1-carbonyl)-1-oxoisoindolin-2-yl) piperidine-2,6-dione |
| | 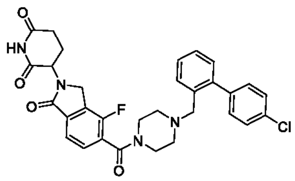 | 3-(5-(4-((4'-chloro-[1,1'-biphenyl]-2-yl)methyl)piperazine-1-carbonyl)-4-fluoro-1-oxoisoindolin-2-yl)piperidine-2,6-dione | 3-(5-(4-((4'-chloro-[1,1'-biphenyl]-2-yl)methyl)piperazine-1-carbonyl)-4-fluoro-1-oxoisoindolin-2-yl)piperidine-2,6-dione |
| | 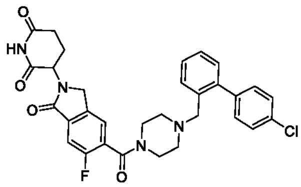 | 3-(5-(4-((4'-chloro-[1,1'-biphenyl]-2-yl)methyl)piperazine-1-carbonyl)-6-fluoro-1-oxoisoindolin-2-yl)piperidine-2,6-dione | 3-(5-(4-((4'-chloro-[1,1'-biphenyl]-2-yl)methyl)piperazine-1-carbonyl)-6-fluoro-1-oxoisoindolin-2-yl)piperidine-2,6-dione |
| | 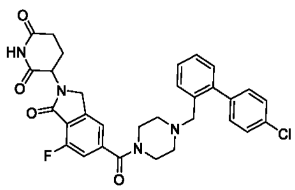 | 3-(5-(4-((4'-chloro-[1,1'-biphenyl]-2-yl)methyl)piperazine-1-carbonyl)-7-fluoro-1-oxoisoindolin-2-yl)piperidine-2,6-dione | 3-(5-(4-((4'-chloro-[1,1'-biphenyl]-2-yl)methyl)piperazine-1-carbonyl)-7-fluoro-1-oxoisoindolin-2-yl)piperidine-2,6-dione |
| GT-05486 | 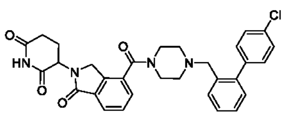 | 3-(4-(4-((4'-chloro-[1,1'-biphenyl]-2-yl)methyl)piperazine-1-carbonyl)-1-oxoisoindolin-2-yl) piperidine-2,6-dione | 3-(4-(4-((4'-chloro-[1,1'-biphenyl]-2-yl)methyl)piperazine-1-carbonyl)-1-oxoisoindolin-2-yl) piperidine-2,6-dione |
| GT-05646 | 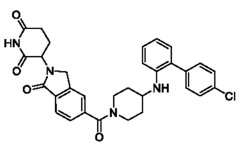 | 3-(5-(4-((4'-chloro-[1,1'-biphenyl]-2-yl)amino)piperidine-1-carbonyl)-1-oxoisoindolin-2-yl) piperidine-2,6-dione | 3-(5-(4-((4'-chloro-[1,1'-biphenyl]-2-yl)amino)piperidine-1-carbonyl)-1-oxoisoindolin-2-yl) piperidine-2,6-dione |
| | 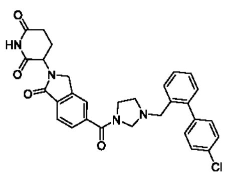 | 3-(5-(3-((4'-chloro-[1,1'-biphenyl]-2-yl)methyl)imidazolidine-1-carbonyl)-1-oxoisoindolin-2-yl)piperidine-2,6-dione | 3-(5-(3-((4'-chloro-[1,1'-biphenyl]-2-yl)methyl)imidazolidine-1-carbonyl)-1-oxoisoindolin-2-yl)piperidine-2,6-dione |
| GT-05616 | 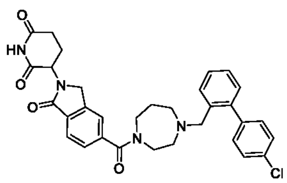 | 3-(5-(4-((4'-chloro-[1,1'-biphenyl]-2-yl)methyl)-1,4-diazepane-1-carbonyl)-1-oxoisoindolin-2-yl)piperidine-2,6-dione | 3-(5-(4-((4'-chloro-[1,1'-biphenyl]-2-yl)methyl)-1,4-diazepane-1-carbonyl)-1-oxoisoindolin-2-yl)piperidine-2,6-dione |

(continued)

| Compo und No. | Structure of the compounds | The compounds' name | Chinese translation of the compounds' name |
|---|---|---|---|
| GT-05641 | 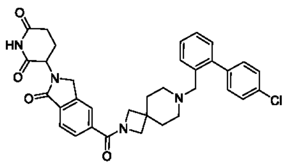 | 3-(5-(7-((4'-chloro-[1,1'-biphenyl]-2-yl)methyl)-2,7-diazaspiro [3.5]nonane-2-carbonyl)-1-oxoisoindolin-2-yl)piperidine-2,6-dione | 3-(5-(7-((4'-chloro-[1,1'-biphenyl]-2-yl)methyl)-2,7-diazaspiro [3.5]nonane-2-carbonyl)-1-oxoisoindolin-2-yl)piperidine-2,6-dione |
| GT-05637 | 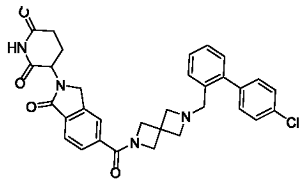 | 3-(5-(6-((4'-chloro-[1,1'-biphenyl]-2-yl)methyl)-2,6-diazaspiro [3.3]heptane-2-carbonyl)-1-oxoisoindolin-2-yl)piperidine-2,6-dione | 3-(5-(6-((4'-chloro-[1,1'-biphenyl]-2-yl)methyl)-2,6-diazaspiro [3.3]heptane-2-carbonyl)-1-oxoisoindolin-2-yl)piperidine-2,6-dione |
| GT-05642 | 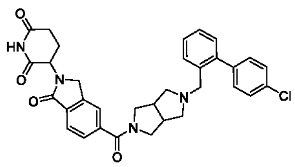 | 3-(5-(5-((4'-chloro-[1,1'-biphenyl]-2-yl)methyl)octahydropyrrolo[ 3,4-c]pyrrole-2-carbonyl)-1-oxoisoindolin-2-yl)piperidine-2,6-dione | 3-(5-(5-((4'-chloro-[1,1'-biphenyl]-2-yl)methyl)octahydropyrrolo[3 ,4-c]pyrrole-2-carbonyl)-1-oxoisoindolin-2-yl)piperidine-2,6-dione |
| GT-05643 | 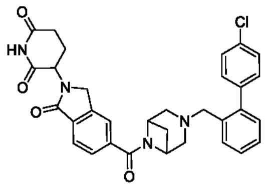 | 3-(5-(3-((4'-chloro-[1,1'-biphenyl]-2-yl)methyl)-3,6-diazabicyclo[3.1.1]heptane-6-carbonyl)-1-oxoisoindolin-2-yl)piperidine-2,6-dione | 3-(5-(3-((4'-chloro-[1,1'-biphenyl]-2-yl)methyl)-3,6-diazabicyclo[3.1.1]heptane-6-carbonyl)-1-oxoisoindolin-2-yl)piperidine-2,6-dione |
| | 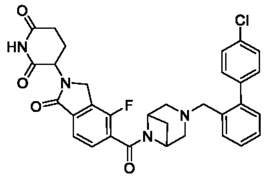 | 3-(5-(3-((4'-chloro-[1,1'-biphenyl]-2-yl)methyl)-3,6-diazabicyclo[3.1.1]heptane-6-carbonyl)-4-fluoro-1-oxoisoindolin-2-yl)piperidine-2,6-dione | 3-(5-(3-((4'-chloro-[1,1'-biphenyl]-2-yl)methyl)-3,6-diazabicyclo[3.1.1]heptane-6-carbonyl)-4-fluoro-1-oxoisoindolin-2-yl)piperidine-2,6-dione |
| | 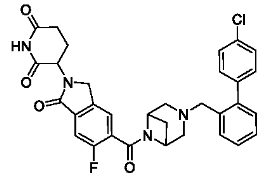 | 3-(5-(3-((4'-chloro-[1,1'-biphenyl]-2-yl)methyl)-3,6-diazabicyclo[3.1.1]heptane-6-carbonyl)-6-fluoro-1-oxoisoindolin-2-yl)piperidine-2,6-dione | 3-(5-(3-((4'-chloro-[1,1'-biphenyl]-2-yl)methyl)-3,6-diazabicyclo[3.1.1]heptane-6-carbonyl)-6-fluoro-1-oxoisoindolin-2-yl)piperidine-2,6-dione |
| | 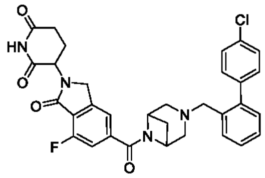 | 3-(5-(3-((4'-chloro-[1,1'-biphenyl]-2-yl)methyl)-3,6-diazabicyclo[3.1.1]heptane-6-carbonyl)-7-fluoro-1-oxoisoindolin-2-yl)piperidine-2,6-dione | 3-(5-(3-((4'-chloro-[1,1'-biphenyl]-2-yl)methyl)-3,6-diazabicyclo[3.1.1]heptane-6-carbonyl)-7-fluoro-1-oxoisoindolin-2-yl)piperidine-2,6-dione |
| GT-05705 | 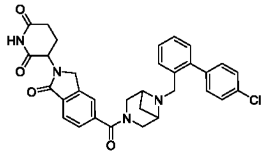 | 3-(5-(6-((4'-chloro-[1,1'-biphenyl]-2-yl)methyl)-3,6-diazabicyclo[3.1.1]heptane-3-carbonyl)-1-oxoisoindolin-2-yl)piperidine-2,6-dione | 3-(5-(6-((4'-chloro-[1,1'-biphenyl]-2-yl)methyl)-3,6-diazabicyclo[3.1.1]heptane-3-carbonyl)-1-oxoisoindolin-2-yl)piperidine-2,6-dione |

(continued)

| Compo und No. | Structure of the compounds | The compounds' name | Chinese translation of the compounds' name |
|---|---|---|---|
| GT-05638 | 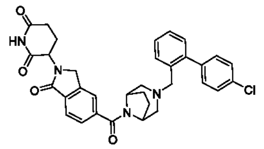 | 3-(5-(3-((4'-chloro-[1,1'-biphenyl]-2-yl)methyl)-3,8-diazabicyclo[3.2.1]octane-8-carbonyl)-1-oxoisoindolin-2-yl)piperidine-2,6-dione | 3-(5-(3-((4'-chloro-[1,1'-biphenyl]-2-yl)methyl)-3,8-diazabicyclo[3.2.1]octane-8-carbonyl)-1-oxoisoindolin-2-yl)piperidine-2,6-dione |
| GT-05621 | 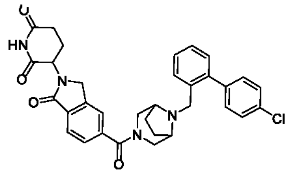 | 3-(5-(8-((4'-chloro-[1,1'-biphenyl]-2-yl)methyl)-3,8-diazabicyclo[3.2.1]octane-3-carbonyl)-1-oxoisoindolin-2-yl)piperidine-2,6-dione | 3-(5-(8-((4'-chloro-[1,1'-biphenyl]-2-yl)methyl)-3,8-diazabicyclo[3.2.1]octane-3-carbonyl)-1-oxoisoindolin-2-yl)piperidine-2,6-dione |
| GT-05622 | 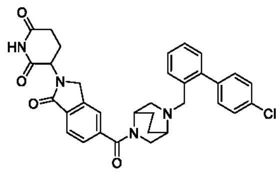 | 3-(5-(5-((4'-chloro-[1,1'-biphenyl]-2-yl)methyl)-2,5-diazabicyclo[2.2.2]octane-2-carbonyl)-1-oxoisoindolin-2-yl)piperidine-2,6-dione | 3-(5-(5-((4'-chloro-[1,1'-biphenyl]-2-yl)methyl)-2,5-diazabicyclo[2.2.2]octane-2-carbonyl)-1-oxoisoindolin-2-yl)piperidine-2,6-dione |
| | 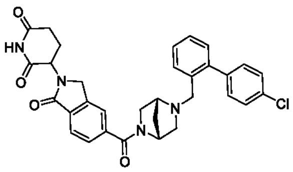 | 3-(5-((1R,4R)-5-((4'-chloro-[1,1'-biphenyl]-2-yl) methyl)-2,5-diazabicyclo[2.2.1] heptane-2-carbonyl)-1-oxoisoindolin-2-yl)piperidine-2,6-dione | 3-(5-((1R,4R)-5-((4'-chloro-[1,1'-biphenyl]-2-yl) methyl)-2,5-diazabicyclo[2.2.1] heptane-2-carbonyl)-1-oxoisoindolin-2-yl)piperidine-2,6-dione |
| | 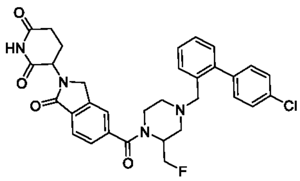 | 3-(5-(4-((4'-chloro-[1,1'-biphenyl]-2-yl)methyl)-2-(fluoromethyl)piperazine-1-carbonyl)-1-oxoisoindolin-2-yl)piperidine-2,6-dione | 3-(5-(4-((4'-chloro-[1,1'-biphenyl]-2-yl)methyl)-2-(fluoromethyl)piperazine-1-carbonyl)-1-oxoisoindolin-2-yl)piperidine-2,6-dione |
| | 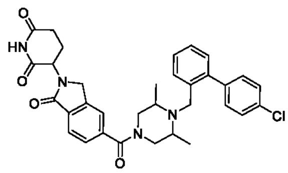 | 3-(5-(4-((4'-chloro-[1,1'-biphenyl]-2-yl)methyl)-3,5-dimethylpiperazine-1-carbonyl)-1-oxoisoindolin-2-yl)piperidine-2,6-dione | 3-(5-(4-((4'-chloro-[1,1'-biphenyl]-2-yl)methyl)-3,5-dimethylpiperazine-1-carbonyl)-1-oxoisoindolin-2-yl)piperidine-2,6-dione |
| | 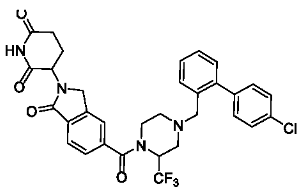 | 3-(5-(4-((4'-chloro-[1,1'-biphenyl]-2-yl)methyl)-2-(trifluoromethyl)piperazine-1-carbonyl)-1-oxoisoindolin-2-yl)piperidine-2,6-dione | 3-(5-(4-((4'-chloro-[1,1'-biphenyl]-2-yl)methyl)-2-(trifluoromethyl)piperazine-1-carbonyl)-1-oxoisoindolin-2-yl)piperidine-2,6-dione |
| | 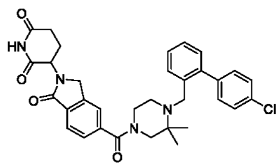 | 3-(5-(4-((4'-chloro-[1,1'-biphenyl]-2-yl)methyl)-3,3-dimethylpiperazine-1-carbonyl)-1-oxoisoindolin-2-yl)piperidine-2,6-dione | 3-(5-(4-((4'-chloro-[1,1'-biphenyl]-2-yl)methyl)-3,3-dimethylpiperazine-1-carbonyl)-1-oxoisoindolin-2-yl)piperidine-2,6-dione |

(continued)

| Compound No. | Structure of the compounds | The compounds' name | Chinese translation of the compounds' name |
|---|---|---|---|
| GT-03454 | 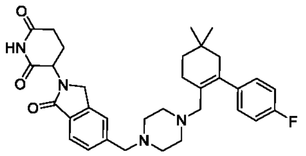 | 3-(5-((4-((4'-fluoro-5,5-dimethyl-3,4,5,6-tetrahydro-[1,1'-biphenyl]-2-yl)methyl)piperazin-1-yl)methyl)-1-oxoisoindolin-2-yl)piperidine-2,6-dione | 3-(5-((4-((4'-fluoro-5,5-dimethyl-3,4,5,6-tetrahydro-[1,1'-biphenyl]-2-yl)methyl)piperazin-1-yl)methyl)-1-oxoisoindolin-2-yl)piperidine-2,6-dione |
| GT-05390 | 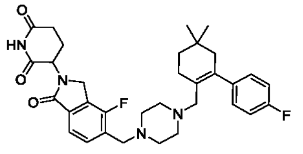 | 3-(4-fluoro-5-((4-((4'-fluoro-5,5-dimethyl-3,4,5,6-tetrahydro-[1,1'-biphenyl]-2-yl)methyl)piperazin-1-yl)methyl)-1-oxoisoindolin-2-yl)piperidine-2,6-dione | 3-(4-fluoro-5-((4-((4'-fluoro-5,5-dimethyl-3,4,5,6-tetrahydro-[1,1'-biphenyl]-2-yl)methyl)piperazin-1-yl)methyl)-1-oxoisoindolin-2-yl)piperidine-2,6-dione |
| GT-05391 | 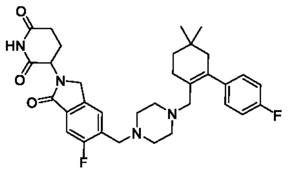 | 3-(6-fluoro-5-((4-((4'-fluoro-5,5-dimethyl-3,4,5,6-tetrahydro-[1,1'-biphenyl]-2-yl)methyl)piperazin-1-yl)methyl)-1-oxoisoindolin-2-yl)piperidine-2,6-dione | 3-(6-fluoro-5-((4-((4'-fluoro-5,5-dimethyl-3,4,5,6-tetrahydro-[1,1'-biphenyl]-2-yl)methyl)piperazin-1-yl)methyl)-1-oxoisoindolin-2-yl)piperidine-2,6-dione |
| GT-05392 | 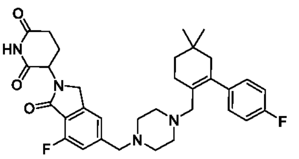 | 3-(7-fluoro-5-((4-((4'-fluoro-5,5-dimethyl-3,4,5,6-tetrahydro-[1,1'-biphenyl]-2-yl)methyl)piperazin-1-yl)methyl)-1-oxoisoindolin-2-yl)piperidine-2,6-dione | 3-(7-fluoro-5-((4-((4'-fluoro-5,5-dimethyl-3,4,5,6-tetrahydro-[1,1'-biphenyl]-2-yl)methyl)piperazin-1-yl)methyl)-1-oxoisoindolin-2-yl)piperidine-2,6-dione |
| GT-05393 | 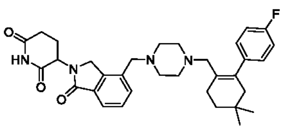 | 3-(4-((4-((4'-fluoro-5,5-dimethyl-3,4,5,6-tetrahydro-[1,1'-biphenyl]-2-yl)methyl)piperazin-1-yl)methyl)-1-oxoisoindolin-2-yl)piperidine-2,6-dione | 3-(4-((4-((4'-fluoro-5,5-dimethyl-3,4,5,6-tetrahydro-[1,1'-biphenyl]-2-yl)methyl)piperazin-1-yl)methyl)-1-oxoisoindolin-2-yl)piperidine-2,6-dione |
| GT-05394 | 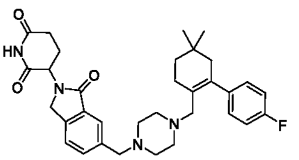 | 3-(6-((4-((4'-fluoro-5,5-dimethyl-3,4,5,6-tetrahydro-[1,1'-biphenyl]-2-yl)methyl)piperazin-1-yl)methyl)-1-oxoisoindolin-2-yl)piperidine-2,6-dione | 3-(6-((4-((4'-fluoro-5,5-dimethyl-3,4,5,6-tetrahydro-[1,1'-biphenyl]-2-yl)methyl)piperazin-1-yl)methyl)-1-oxoisoindolin-2-yl)piperidine-2,6-dione |
| GT-05493 | 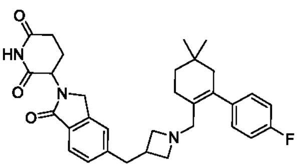 | 3-(5-((1-((4'-fluoro-5,5-dimethyl-3,4,5,6-tetrahydro-[1,1'-biphenyl]-2-yl)methyl)azetidin-3-yl)methyl)-1-oxoisoindolin-2-yl)piperidine-2,6-dione | 3-(5-((1-((4'-fluoro-5,5-dimethyl-3,4,5,6-tetrahydro-[1,1'-biphenyl]-2-yl)methyl)azetidin-3-yl)methyl)-1-oxoisoindolin-2-yl)piperidine-2,6-dione |
| GT-05483 | 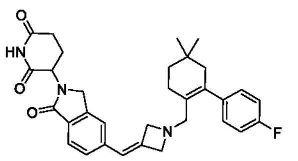 | 3-(5-((1-((4'-fluoro-5,5-dimethyl-3,4,5,6-tetrahydro-[1,1'-biphenyl]-2-yl)methyl)azetidin-3-ylidene)methyl)-1-oxoisoindolin-2-yl)piperidine-2,6-dione | 3-(5-((1-((4'-fluoro-5,5-dimethyl-3,4,5,6-tetrahydro-[1,1'-biphenyl]-2-yl)methyl)azetidin-3-ylidene)methyl)-1-oxoisoindolin-2-yl)piperidine-2,6-dione |

(continued)

| Compound No. | Structure of the compounds | The compounds' name | Chinese translation of the compounds' name |
|---|---|---|---|
| GT-05499 | 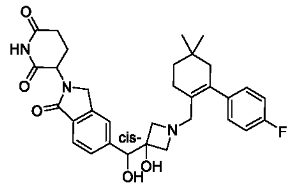 | 3-(5-((1-((4'-fluoro-5,5-dimethyl-3,4,5,6-tetrahydro-[1,1'-biphenyl]-2-yl)methyl)-3-hydroxyazetidin-3-yl)(hydroxy) methyl)-1-oxoisoindolin-2-yl)piperidine-2,6-dione | 3-(5-((1-((4'-fluoro-5,5-dimethyl-3,4,5,6-tetrahydro-[1,1'-biphenyl]-2-yl)methyl)-3-hydroxyazetidin-3-yl)(hydroxy) methyl)-1-oxoisoindolin-2-yl)piperidine-2,6-dione |
| GT-05785 | 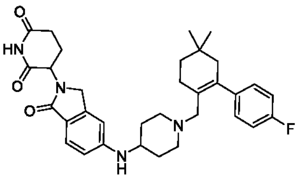 | 3-(5-((1-((4'-fluoro-5,5-dimethyl-3,4,5,6-tetrahydro-[1,1'-biphenyl]-2-yl)methyl)piperidin-4-yl)amino)-1-oxoisoindolin-2-yl)piperidine-2,6-dione | 3-(5-((1-((4'-fluoro-5,5-dimethyl-3,4,5,6-tetrahydro-[1,1'-biphenyl]-2-yl)methyl)piperidin-4-yl)amino)-1-oxoisoindolin-2-yl)piperidine-2,6-dione |
| GT-03455 | 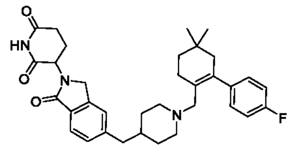 | 3-(5-((1-((4'-fluoro-5,5-dimethyl-3,4,5,6-tetrahydro-[1,1'-biphenyl]-2-yl)methyl)piperidin-4-yl)methyl)-1-oxoisoindolin-2-yl)piperidine-2,6-dione | 3-(5-((1-((4'-fluoro-5,5-dimethyl-3,4,5,6-tetrahydro-[1,1'-biphenyl]-2-yl)methyl)piperidin-4-yl)methyl)-1-oxoisoindolin-2-yl)piperidine-2,6-dione |
| GT-03453 | 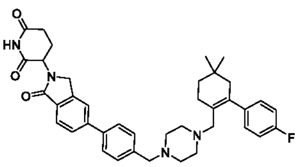 | 3-(5-(4-((4-((4'-fluoro-5,5-dimethyl-3,4,5,6-tetrahydro-[1,1'-biphenyl]-2-yl)methyl)piperazin-1-yl)methyl)phenyl)-1-oxoisoindolin-2-yl)piperidine-2,6-dione | 3-(5-(4-((4-((4'-fluoro-5,5-dimethyl-3,4,5,6-tetrahydro-[1,1'-biphenyl]-2-yl)methyl)piperazin-1-yl)methyl)phenyl)-1-oxoisoindolin-2-yl)piperidine-2,6-dione |
| GT-05506 | 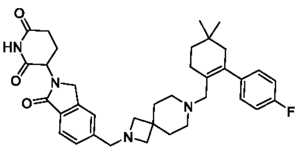 | 3-(5-((7-((4'-fluoro-5,5-dimethyl-3,4,5,6-tetrahydro-[1,1'-biphenyl]-2-yl)methyl)-2,7-diazaspiro[3.5]nonan-2-yl) methyl)-1-oxoisoindolin-2-yl)piperidine-2,6-dione | 3-(5-((7-((4'-fluoro-5,5-dimethyl-3,4,5,6-tetrahydro-[1,1'-biphenyl]-2-yl)methyl)-2,7-diazaspiro[3.5]nonan-2-yl) methyl)-1-oxoisoindolin-2-yl)piperidine-2,6-dione |
| GT-05670 | 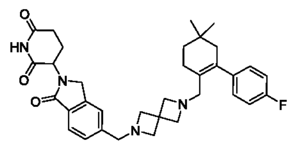 | 3-(5-((6-((4'-fluoro-5,5-dimethyl-3,4,5,6-tetrahydro-[1,1'-biphenyl]-2-yl)methyl)-2,6-diazaspiro[3.3]heptan-2-yl) methyl)-1-oxoisoindolin-2-yl)piperidine-2,6-dione | 3-(5-((6-((4'-fluoro-5,5-dimethyl-3,4,5,6-tetrahydro-[1,1'-biphenyl]-2-yl)methyl)-2,6-diazaspiro[3.3]heptan-2-yl) methyl)-1-oxoisoindolin-2-yl)piperidine-2,6-dione |
| GT-05398 | 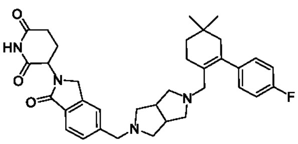 | 3-(5-((5-((4'-fluoro-5,5-dimethyl-3,4,5,6-tetrahydro-[1,1'-biphenyl]-2-yl)methyl)hexahydropyrrolo [3,4-c]pyrrol-2(1H)-yl)methyl)-1-oxoisoindolin-2-yl)piperidine-2,6-dione | 3-(5-((5-((4'-fluoro-5,5-dimethyl-3,4,5,6-tetrahydro-[1,1'-biphenyl]-2-yl)methyl)hexahydropyrrolo[ 3,4-c]pyrrol-2(1H)-yl)methyl)-1-oxoisoindolin-2-yl)piperidine-2,6-dione |
| GT-05399 | 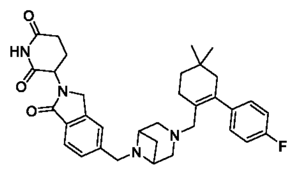 | 3-(5-((3-((4'-fluoro-5,5-dimethyl-3,4,5,6-tetrahydro-[1,1'-biphenyl]-2-yl)methyl)-3,6-diazabicyclo[3.1.1]heptan-6-yl) methyl)-1-oxoisoindolin-2-yl)piperidine-2,6-dione | 3-(5-((3-((4'-fluoro-5,5-dimethyl-3,4,5,6-tetrahydro-[1,1'-biphenyl]-2-yl)methyl)-3,6-diazabicyclo[3.1.1]heptan-6-yl) methyl)-1-oxoisoindolin-2-yl)piperidine-2,6-dione |

(continued)

| Compound No. | Structure of the compounds | The compounds' name | Chinese translation of the compounds' name |
|---|---|---|---|
| GT-05509 | 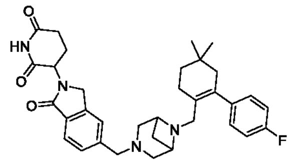 | 3-(5-((6-((4'-fluoro-5,5-dimethyl-3,4,5,6-tetrahydro-[1,1'-biphenyl]-2-yl)methyl)-3,6-diazabicyclo[3.1.1]heptan-3-yl)methyl)-1-oxoisoindolin-2-yl)piperidine-2,6-dione | 3-(5-((6-((4'-fluoro-5,5-dimethyl-3,4,5,6-tetrahydro-[1,1'-biphenyl]-2-yl)methyl)-3,6-diazabicyclo[3.1.1]heptan-3-yl)methyl)-1-oxoisoindolin-2-yl)piperidine-2,6-dione |
| GT-05400 | 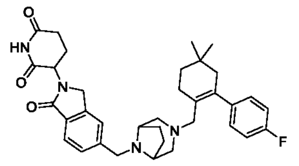 | 3-(5-((3-((4'-fluoro-5,5-dimethyl-3,4,5,6-tetrahydro-[1,1'-biphenyl]-2-yl)methyl)-3,8-diazabicyclo[3.2.1]octan-8-yl)methyl)-1-oxoisoindolin-2-yl)piperidine-2,6-dione | 3-(5-((3-((4'-fluoro-5,5-dimethyl-3,4,5,6-tetrahydro-[1,1'-biphenyl]-2-yl)methyl)-3,8-diazabicyclo[3.2.1]octan-8-yl)methyl)-1-oxoisoindolin-2-yl)piperidine-2,6-dione |
| GT-05510 | 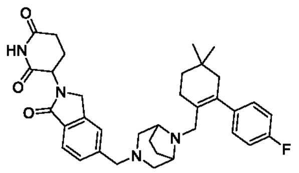 | 3-(5-((8-((4'-fluoro-5,5-dimethyl-3,4,5,6-tetrahydro-[1,1'-biphenyl]-2-yl)methyl)-3,8-diazabicyclo[3.2.1]octan-3-yl)methyl)-1-oxoisoindolin-2-yl)piperidine-2,6-dione | 3-(5-((8-((4'-fluoro-5,5-dimethyl-3,4,5,6-tetrahydro-[1,1'-biphenyl]-2-yl)methyl)-3,8-diazabicyclo[3.2.1]octan-3-yl)methyl)-1-oxoisoindolin-2-yl)piperidine-2,6-dione |
| GT-05512 | 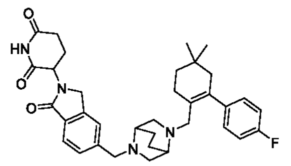 | 3-(5-((5-((4'-fluoro-5,5-dimethyl-3,4,5,6-tetrahydro-[1,1'-biphenyl]-2-yl)methyl)-2,5-diazabicyclo[2.2.2]octan-2-yl)methyl)-1-oxoisoindolin-2-yl)piperidine-2,6-dione | 3-(5-((5-((4'-fluoro-5,5-dimethyl-3,4,5,6-tetrahydro-[1,1'-biphenyl]-2-yl)methyl)-2,5-diazabicyclo[2.2.2]octan-2-yl)methyl)-1-oxoisoindolin-2-yl)piperidine-2,6-dione |
| | 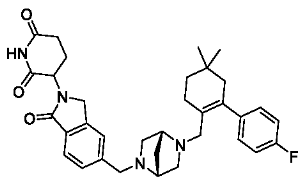 | 3-(5-(((1R,4R)-5-((4'-fluoro-5,5-dimethyl-3,4,5,6-tetrahydro-[1,1'-biphenyl]-2-yl)methyl)-2,5-diazabicyclo[2.2.1]heptan-2-yl)methyl)-1-oxoisoindolin-2-yl)piperidine-2,6-dione | 3-(5-(((1R,4R)-5-((4'-fluoro-5,5-dimethyl-3,4,5,6-tetrahydro-[1,1'-biphenyl]-2-yl)methyl)-2,5-diazabicyclo[2.2.1]heptan-2-yl)methyl)-1-oxoisoindolin-2-yl)piperidine-2,6-dione |
| | 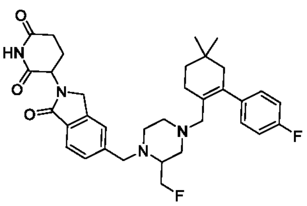 | 3-(5-((4-((4'-fluoro-5,5-dimethyl-3,4,5,6-tetrahydro-[1,1'-biphenyl]-2-yl)methyl)-2-(fluoromethyl)piperazin-1-yl)methyl)-1-oxoisoindolin-2-yl)piperidine-2,6-dione | 3-(5-((4-((4'-fluoro-5,5-dimethyl-3,4,5,6-tetrahydro-[1,1'-biphenyl]-2-yl)methyl)-2-(fluoromethyl)piperazin-1-yl)methyl)-1-oxoisoindolin-2-yl)piperidine-2,6-dione |
| | 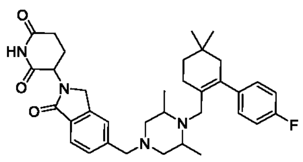 | 3-(5-((4-((4'-fluoro-5,5-dimethyl-3,4,5,6-tetrahydro-[1,1'-biphenyl]-2-yl)methyl)-3,5-dimethylpiperazin-1-yl)methyl)-1-oxoisoindolin-2-yl)piperidine-2,6-dione | 3-(5-((4-((4'-fluoro-5,5-dimethyl-3,4,5,6-tetrahydro-[1,1'-biphenyl]-2-yl)methyl)-3,5-dimethylpiperazin-1-yl)methyl)-1-oxoisoindolin-2-yl)piperidine-2,6-dione |
| GT-05846 | 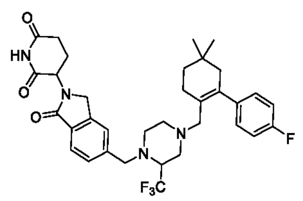 | 3-(5-((4-((4'-fluoro-5,5-dimethyl-3,4,5,6-tetrahydro-[1,1'-biphenyl]-2-yl)methyl)-2-(trifluoromethyl)piperazin-1-yl)methyl)-1-oxoisoindolin-2-yl)piperidine-2,6-dione | 3-(5-((4-((4'-fluoro-5,5-dimethyl-3,4,5,6-tetrahydro-[1,1'-biphenyl]-2-yl)methyl)-2-(trifluoromethyl)piperazin-1-yl)methyl)-1-oxoisoindolin-2-yl)piperidine-2,6-dione |

(continued)

| Compo und No. | Structure of the compounds | The compounds' name | Chinese translation of the compounds' name |
|---|---|---|---|
| | 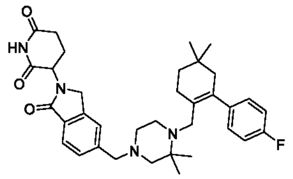 | 3-(5-((4-((4'-fluoro-5,5-dimethyl-3,4,5,6-tetrahydro-[1,1'-biphenyl]-2-yl)methyl)-3,3-dimethylpiperazin-1-yl)methyl)-1-oxoisoindolin-2-yl)piperidine-2,6-dione | 3-(5-((4-((4'-fluoro-5,5-dimethyl-3,4,5,6-tetrahydro-[1,1'-biphenyl]-2-yl)methyl)-3,3-dimethylpiperazin-1-yl)methyl)-1-oxoisoindolin-2-yl)piperidine-2,6-dione |
| | 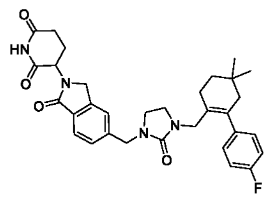 | 3-(5-((3-((4'-fluoro-5,5-dimethyl-3,4,5,6-tetrahydro-[1,1'-biphenyl]-2-yl)methyl)-2-oxoimidazolidin-1-yl)methyl)-1-oxoisoindolin-2-yl)piperidine-2,6-dione | 3-(5-((3-((4'-fluoro-5,5-dimethyl-3,4,5,6-tetrahydro-[1,1'-biphenyl]-2-yl)methyl)-2-oxoimidazolidin-1-yl)methyl)-1-oxoisoindolin-2-yl)piperidine-2,6-dione |
| | 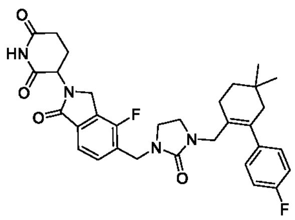 | 3-(4-fluoro-5-((3-((4'-fluoro-5,5-dimethyl-3,4,5,6-tetrahydro-[1,1'-biphenyl]-2-yl)methyl)-2-oxoimidazolidin-1-yl)methyl)-1-oxoisoindolin-2-yl)piperidine-2,6-dione | 3-(4-fluoro-5-((3-((4'-fluoro-5,5-dimethyl-3,4,5,6-tetrahydro-[1,1'-biphenyl]-2-yl)methyl)-2-oxoimidazolidin-1-yl)methyl)-1-oxoisoindolin-2-yl)piperidine-2,6-dione |
| | 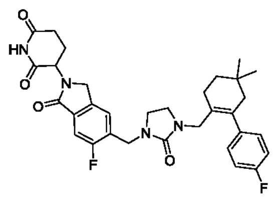 | 3-(6-fluoro-5-((3-((4'-fluoro-5,5-dimethyl-3,4,5,6-tetrahydro-[1,1'-biphenyl]-2-yl)methyl)-2-oxoimidazolidin-1-yl)methyl)-1-oxoisoindolin-2-yl)piperidine-2,6-dione | 3-(6-fluoro-5-((3-((4'-fluoro-5,5-dimethyl-3,4,5,6-tetrahydro-[1,1'-biphenyl]-2-yl)methyl)-2-oxoimidazolidin-1-yl)methyl)-1-oxoisoindolin-2-yl)piperidine-2,6-dione |
| | 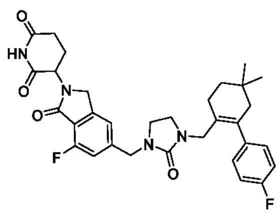 | 3-(7-fluoro-5-((3-((4'-fluoro-5,5-dimethyl-3,4,5,6-tetrahydro-[1,1'-biphenyl]-2-yl)methyl)-2-oxoimidazolidin-1-yl)methyl)-1-oxoisoindolin-2-yl)piperidine-2,6-dione | 3-(7-fluoro-5-((3-((4'-fluoro-5,5-dimethyl-3,4,5,6-tetrahydro-[1,1'-biphenyl]-2-yl)methyl)-2-oxoimidazolidin-1-yl)methyl)-1-oxoisoindolin-2-yl)piperidine-2,6-dione |
| | 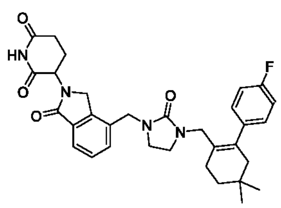 | 3-(4-((3-((4'-fluoro-5,5-dimethyl-3,4,5,6-tetrahydro-[1,1'-biphenyl]-2-yl)methyl)-2-oxoimidazolidin-1-yl)methyl)-1-oxoisoindolin-2-yl)piperidine-2,6-dione | 3-(4-((3-((4'-fluoro-5,5-dimethyl-3,4,5,6-tetrahydro-[1,1'-biphenyl]-2-yl)methyl)-2-oxoimidazolidin-1-yl)methyl)-1-oxoisoindolin-2-yl)piperidine-2,6-dione |
| GT-05500 | 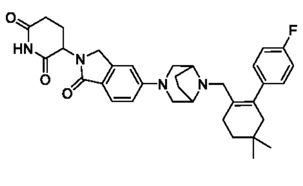 | 3-(5-(8-((4'-fluoro-5,5-dimethyl-3,4,5,6-tetrahydro-[1,1'-biphenyl]-2-yl)methyl)-3,8-diazabicyclo[3.2.1]octan-3-yl)-1-oxoisoindolin-2-yl)piperidine-2,6-dione | 3-(5-(8-((4'-fluoro-5,5-dimethyl-3,4,5,6-tetrahydro-[1,1'-biphenyl]-2-yl)methyl)-3,8-diazabicyclo[3.2.1]octan-3-yl)-1-oxoisoindolin-2-yl)piperidine-2,6-dione |

(continued)

| Compo und No. | Structure of the compounds | The compounds' name | Chinese translation of the compounds' name |
|---|---|---|---|
| GT-05498 | 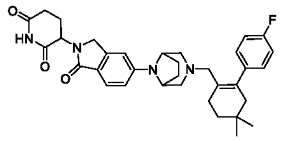 | 3-(5-(3-((4'-fluoro-5,5-dimethyl-3,4,5,6-tetrahydro-[1,1'-biphenyl]-2-yl)methyl)-3,8-diazabicyclo[3.2.1]octan-8-yl)-1-oxoisoindolin-2-yl)piperidine-2,6-dione | 3-(5-(3-((4'-fluoro-5,5-dimethyl-3,4,5,6-tetrahydro-[1,1'-biphenyl]-2-yl)methyl)-3,8-diazabicyclo[3.2.1]octan-8-yl)-1-oxoisoindolin-2-yl)piperidine-2,6-dione |
| GT-05520 | 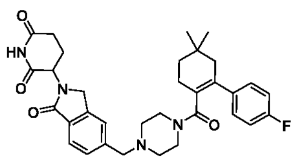 | 3-(5-((4-(4'-fluoro-5,5-dimethyl-3,4,5,6-tetrahydro-[1,1'-biphenyl]-2-carbonyl)piperazin-1-yl)methyl)-1-oxoisoindolin-2-yl)piperidine-2,6-dione | 3-(5-((4-(4'-fluoro-5,5-dimethyl-3,4,5,6-tetrahydro-[1,1'-biphenyl]-2-carbonyl)piperazin-1-yl)methyl)-1-oxoisoindolin-2-yl)piperidine-2,6-dione |
| GT-05487 | 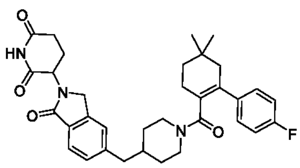 | 3-(5-((1-(4'-fluoro-5,5-dimethyl-3,4,5,6-tetrahydro-[1,1'-biphenyl]-2-carbonyl)piperidin-4-yl)methyl)-1-oxoisoindolin-2-yl)piperidine-2,6-dione | 3-(5-((1-(4'-fluoro-5,5-dimethyl-3,4,5,6-tetrahydro-[1,1'-biphenyl]-2-carbonyl)piperidin-4-yl)methyl)-1-oxoisoindolin-2-yl)piperidine-2,6-dione |
| GT-05488 | 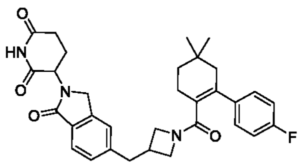 | 3-(5-((1-(4'-fluoro-5,5-dimethyl-3,4,5,6-tetrahydro-[1,1'-biphenyl]-2-carbonyl)azetidin-3-yl)methyl)-1-oxoisoindolin-2-yl)piperidine-2,6-dione | 3-(5-((1-(4'-fluoro-5,5-dimethyl-3,4,5,6-tetrahydro-[1,1'-biphenyl]-2-carbonyl)azetidin-3-yl)methyl)-1-oxoisoindolin-2-yl)piperidine-2,6-dione |
| GT-05489 | 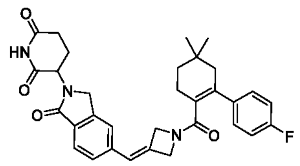 | 3-(5-((1-(4'-fluoro-5,5-dimethyl-3,4,5,6-tetrahydro-[1,1'-biphenyl]-2-carbonyl)azetidin-3-ylidene)methyl)-1-oxoisoindolin-2-yl)piperidine-2,6-dione | 3-(5-((1-(4'-fluoro-5,5-dimethyl-3,4,5,6-tetrahydro-[1,1'-biphenyl]-2-carbonyl)azetidin-3-ylidene)methyl)-1-oxoisoindolin-2-yl)piperidine-2,6-dione |
| GT-05490 | 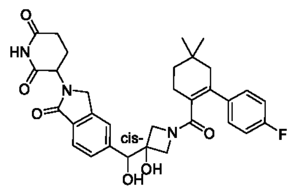 | 3-(5-((1-(4'-fluoro-5,5-dimethyl-3,4,5,6-tetrahydro-[1,1'-biphenyl]-2-carbonyl)-3-hydroxyazetidin-3-yl)(hydroxy)methyl)-1-oxoisoindolin-2-yl)piperidine-2,6-dione | 3-(5-((1-(4'-fluoro-5,5-dimethyl-3,4,5,6-tetrahydro-[1,1'-biphenyl]-2-carbonyl)-3-hydroxyazetidin-3-yl)(hydroxy)methyl)-1-oxoisoindolin-2-yl)piperidine-2,6-dione |
| GT-05786 | 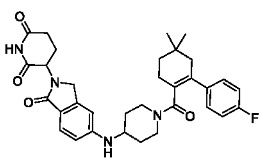 | 3-(5-((1-(4'-fluoro-5,5-dimethyl-3,4,5,6-tetrahydro-[1,1'-biphenyl]-2-carbonyl)piperidin-4-yl)amino)-1-oxoisoindolin-2-yl)piperidine-2,6-dione | 3-(5-((1-(4'-fluoro-5,5-dimethyl-3,4,5,6-tetrahydro-[1,1'-biphenyl]-2-carbonyl)piperidin-4-yl)amino)-1-oxoisoindolin-2-yl)piperidine-2,6-dione |
| GT-05404 | 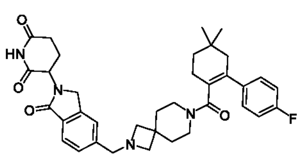 | 3-(5-((7-(4'-fluoro-5,5-dimethyl-3,4,5,6-tetrahydro-[1,1'-biphenyl]-2-carbonyl)-2,7-diazaspiro[3.5]nonan-2-yl)methyl)-1-oxoisoindolin-2-yl)piperidine-2,6-dione | 3-(5-((7-(4'-fluoro-5,5-dimethyl-3,4,5,6-tetrahydro-[1,1'-biphenyl]-2-carbonyl)-2,7-diazaspiro[3.5]nonan-2-yl)methyl)-1-oxoisoindolin-2-yl)piperidine-2,6-dione |

(continued)

| Compound No. | Structure of the compounds | The compounds' name | Chinese translation of the compounds' name |
|---|---|---|---|
| GT-05664 | 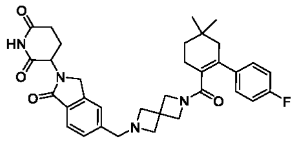 | 3-(5-((6-(4'-fluoro-5,5-dimethyl-3,4,5,6-tetrahydro-[1,1'-biphenyl]-2-carbonyl)-2,6-diazaspiro[3.3]heptan-2-yl)methyl)-1-oxoisoindolin-2-yl)piperidine-2,6-dione | 3-(5-((6-(4'-fluoro-5,5-dimethyl-3,4,5,6-tetrahydro-[1,1'-biphenyl]-2-carbonyl)-2,6-diazaspiro[3.3]heptan-2-yl)methyl)-1-oxoisoindolin-2-yl)piperidine-2,6-dione |
| GT-05405 | 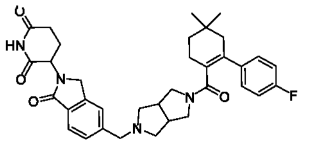 | 3-(5-((5-(4'-fluoro-5,5-dimethyl-3,4,5,6-tetrahydro-[1,1'-biphenyl]-2-carbonyl)hexahydropyrrolo[ 3,4-c]pyrrol-2(1H)-yl)methyl)-1-oxoisoindolin-2-yl)piperidine-2,6-dione | 3-(5-((5-(4'-fluoro-5,5-dimethyl-3,4,5,6-tetrahydro-[1,1'-biphenyl]-2-carbonyl)hexahydropyrrolo[3 ,4-c]pyrrol-2(1H)-yl)methyl)-1-oxoisoindolin-2-yl)piperidine-2,6-dione |
| GT-05514 | 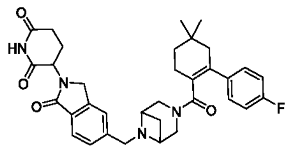 | 3-(5-((3-(4'-fluoro-5,5-dimethyl-3,4,5,6-tetrahydro-[1,1'-biphenyl]-2-carbonyl)-3,6-diazabicyclo[3.1.1]heptan-6-yl)methyl)-1-oxoisoindolin-2-yl)piperidine-2,6-dione | 3-(5-((3-(4'-fluoro-5,5-dimethyl-3,4,5,6-tetrahydro-[1,1'-biphenyl]-2-carbonyl)-3,6-diazabicyclo[3.1.1]heptan-6-yl)methyl)-1-oxoisoindolin-2-yl)piperidine-2,6-dione |
| GT-05515 | 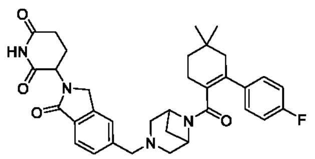 | 3-(5-((6-(4'-fluoro-5,5-dimethyl-3,4,5,6-tetrahydro-[1,1'-biphenyl]-2-carbonyl)-3,6-diazabicyclo[3.1.1]heptan-3-yl)methyl)-1-oxoisoindolin-2-yl)piperidine-2,6-dione | 3-(5-((6-(4'-fluoro-5,5-dimethyl-3,4,5,6-tetrahydro-[1,1'-biphenyl]-2-carbonyl)-3,6-diazabicyclo[3.1.1]heptan-3-yl)methyl)-1-oxoisoindolin-2-yl)piperidine-2,6-dione |
| GT-05516 | 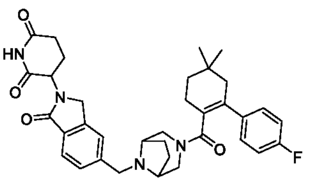 | 3-(5-((3-(4'-fluoro-5,5-dimethyl-3,4,5,6-tetrahydro-[1,1'-biphenyl]-2-carbonyl)-3,8-diazabicyclo[3.2.1]octan-8-yl)methyl)-1-oxoisoindolin-2-yl)piperidine-2,6-dione | 3-(5-((3-(4'-fluoro-5,5-dimethyl-3,4,5,6-tetrahydro-[1,1'-biphenyl]-2-carbonyl)-3,8-diazabicyclo[3.2.1]octan-8-yl)methyl)-1-oxoisoindolin-2-yl)piperidine-2,6-dione |
| GT-05517 | 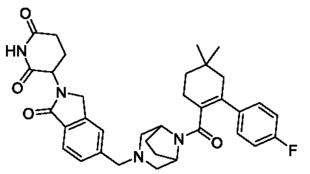 | 3-(5-((8-(4'-fluoro-5,5-dimethyl-3,4,5,6-tetrahydro-[1,1'-biphenyl]-2-carbonyl)-3,8-diazabicyclo[3.2.1]octan-3-yl)methyl)-1-oxoisoindolin-2-yl)piperidine-2,6-dione | 3-(5-((8-(4'-fluoro-5,5-dimethyl-3,4,5,6-tetrahydro-[1,1'-biphenyl]-2-carbonyl)-3,8-diazabicyclo[3.2.1]octan-3-yl)methyl)-1-oxoisoindolin-2-yl)piperidine-2,6-dione |
| GT-05402 | 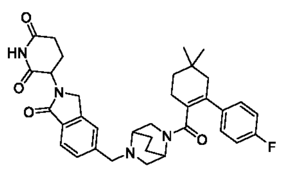 | 3-(5-((5-(4'-fluoro-5,5-dimethyl-3,4,5,6-tetrahydro-[1,1'-biphenyl]-2-carbonyl)-2,5-diazabicyclo[2.2.2]octan-2-yl)methyl)-1-oxoisoindolin-2-yl)piperidine-2,6-dione | 3-(5-((5-(4'-fluoro-5,5-dimethyl-3,4,5,6-tetrahydro-[1,1'-biphenyl]-2-carbonyl)-2,5-diazabicyclo[2.2.2]octan-2-yl)methyl)-1-oxoisoindolin-2-yl)piperidine-2,6-dione |
| | 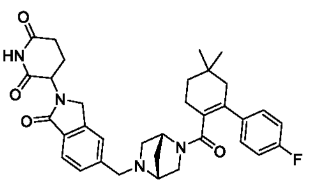 | 3-(5-(((1R,4R)-5-(4'-fluoro-5,5-dimethyl-3,4,5,6-tetrahydro-[1,1'-biphenyl]-2-carbonyl)-2,5-diazabicyclo[2.2.1]heptan-2-yl)methyl)-1-oxoisoindolin-2-yl)piperidine-2,6-dione | 3-(5-(((1R,4R)-5-(4'-fluoro-5,5-dimethyl-3,4,5,6-tetrahydro-[1,1'-biphenyl]-2-carbonyl)-2,5-diazabicyclo[2.2.1]heptan-2-yl)methyl)-1-oxoisoindolin-2-yl)piperidine-2,6-dione |

(continued)

| Compound No. | Structure of the compounds | The compounds' name | Chinese translation of the compounds' name |
|---|---|---|---|
| | 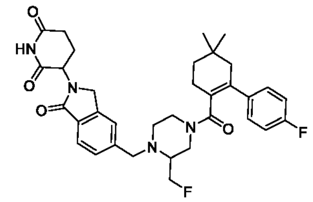 | 3-(5-((4-(4'-fluoro-5,5-dimethyl-3,4,5,6-tetrahydro-[1,1'-biphenyl]-2-carbonyl)-2-(fluoromethyl)piperazin-1-yl) methyl)-1-oxoisoindolin-2-yl)piperidine-2,6-dione | 3-(5-((4-(4'-fluoro-5,5-dimethyl-3,4,5,6-tetrahydro-[1,1'-biphenyl]-2-carbonyl)-2-(fluoromethyl)piperazin-1-yl) methyl)-1-oxoisoindolin-2-yl)piperidine-2,6-dione |
| | 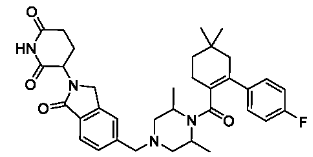 | 3-(5-((4-(4'-fluoro-5,5-dimethyl-3,4,5,6-tetrahydro-[1,1'-biphenyl]-2-carbonyl)-3,5-dimethylpiperazin-1-yl)methyl)-1-oxoisoindolin-2-yl)piperidine-2,6-dione | 3-(5-((4-(4'-fluoro-5,5-dimethyl-3,4,5,6-tetrahydro-[1,1'-biphenyl]-2-carbonyl)-3,5-dimethylpiperazin-1-yl)methyl)-1-oxoisoindolin-2-yl)piperidine-2,6-dione |
| | 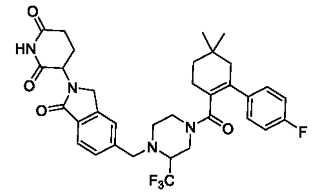 | 3-(5-((4-(4'-fluoro-5,5-dimethyl-3,4,5,6-tetrahydro-[1,1'-biphenyl]-2-carbonyl)-2-(trifluoromethyl)piperazin-1-yl) methyl)-1-oxoisoindolin-2-yl)piperidine-2,6-dione | 3-(5-((4-(4'-fluoro-5,5-dimethyl-3,4,5,6-tetrahydro-[1,1'-biphenyl]-2-carbonyl)-2-(trifluoromethyl)piperazin-1-yl) methyl)-1-oxoisoindolin-2-yl)piperidine-2,6-dione |
| | 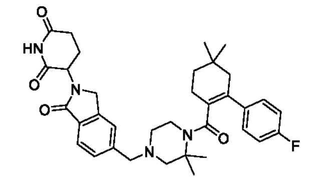 | 3-(5-((4-(4'-fluoro-5,5-dimethyl-3,4,5,6-tetrahydro-[1,1'-biphenyl]-2-carbonyl)-3,3-dimethylpiperazin-1-yl)methyl)-1-oxoisoindolin-2-yl)piperidine-2,6-dione | 3-(5-((4-(4'-fluoro-5,5-dimethyl-3,4,5,6-tetrahydro-[1,1'-biphenyl]-2-carbonyl)-3,3-dimethylpiperazin-1-yl)methyl)-1-oxoisoindolin-2-yl)piperidine-2,6-dione |
| GT-05491 | 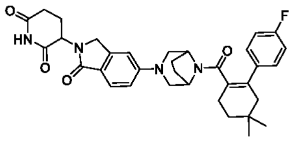 | 3-(5-(8-(4'-fluoro-5,5-dimethyl-3,4,5,6-tetrahydro-[1,1'-biphenyl]-2-carbonyl)-3,8-diazabicyclo[3.2.1]octan-3-yl)-1-oxoisoindolin-2-yl)piperidine-2,6-dione | 3-(5-(8-(4'-fluoro-5,5-dimethyl-3,4,5,6-tetrahydro-[1,1'-biphenyl]-2-carbonyl)-3,8-diazabicyclo[3.2.1]octan-3-yl)-1-oxoisoindolin-2-yl)piperidine-2,6-dione |
| GT-05492 | 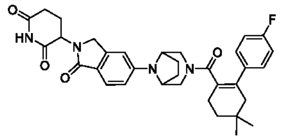 | 3-(5-(3-(4'-fluoro-5,5-dimethyl-3,4,5,6-tetrahydro-[1,1'-biphenyl]-2-carbonyl)-3,8-diazabicyclo[3.2.1]octan-8-yl)-1-oxoisoindolin-2-yl)piperidine-2,6-dione | 3-(5-(3-(4'-fluoro-5,5-dimethyl-3,4,5,6-tetrahydro-[1,1'-biphenyl]-2-carbonyl)-3,8-diazabicyclo[3.2.1]octan-8-yl)-1-oxoisoindolin-2-yl)piperidine-2,6-dione |
| GT-05614 | 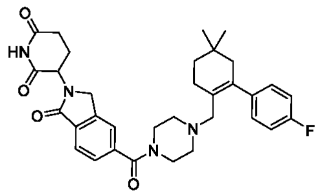 | 3-(5-(4-((4'-fluoro-5,5-dimethyl-3,4,5,6-tetrahydro-[1,1'-biphenyl]-2-yl)methyl)piperazine-1-carbonyl)-1-oxoisoindolin-2-yl)piperidine-2,6-dione | 3-(5-(4-((4'-fluoro-5,5-dimethyl-3,4,5,6-tetrahydro-[1,1'-biphenyl]-2-yl)methyl)piperazine-1-carbonyl)-1-oxoisoindolin-2-yl)piperidine-2,6-dione |
| | 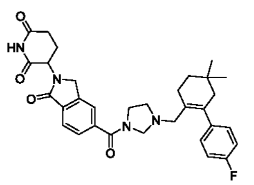 | 3-(5-(3-((4'-fluoro-5,5-dimethyl-3,4,5,6-tetrahydro-[1,1'-biphenyl]-2-yl)methyl)imidazolidine-1-carbonyl)-1-oxoisoindolin-2-yl)piperidine-2,6-dione | 3-(5-(3-((4'-fluoro-5,5-dimethyl-3,4,5,6-tetrahydro-[1,1'-biphenyl]-2-yl)methyl)imidazolidine-1-carbonyl)-1-oxoisoindolin-2-yl)piperidine-2,6-dione |

(continued)

| Compound No. | Structure of the compounds | The compounds' name | Chinese translation of the compounds' name |
|---|---|---|---|
| GT-05505 | 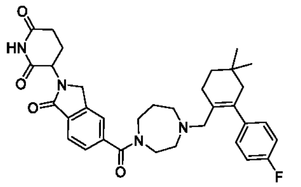 | 3-(5-(4-((4'-fluoro-5,5-dimethyl-3,4,5,6-tetrahydro-[1,1'-biphenyl]-2-yl)methyl)-1,4-diazepane-1-carbonyl)-1-oxoisoindolin-2-yl)piperidine-2,6-dione | 3-(5-(4-((4'-fluoro-5,5-dimethyl-3,4,5,6-tetrahydro-[1,1'-biphenyl]-2-yl)methyl)-1,4-diazepane-1-carbonyl)-1-oxoisoindolin-2-yl)piperidine-2,6-dione |
| GT-05507 | 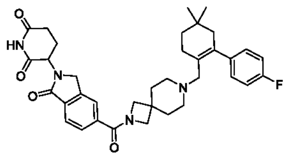 | 3-(5-(7-((4'-fluoro-5,5-dimethyl-3,4,5,6-tetrahydro-[1,1'-biphenyl]-2-yl)methyl)-2,7-diazaspiro[3.5]nonane-2-carbonyl)-1-oxoisoindolin-2-yl)piperidine-2,6-dione | 3-(5-(7-((4'-fluoro-5,5-dimethyl-3,4,5,6-tetrahydro-[1,1'-biphenyl]-2-yl)methyl)-2,7-diazaspiro[3.5]nonane-2-carbonyl)-1-oxoisoindolin-2-yl)piperidine-2,6-dione |
| GT-05508 | 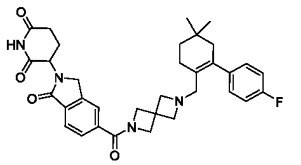 | 3-(5-(6-((4'-fluoro-5,5-dimethyl-3,4,5,6-tetrahydro-[1,1'-biphenyl]-2-yl)methyl)-2,6-diazaspiro[3.3]heptane-2-carbonyl)-1-oxoisoindolin-2-yl)piperidine-2,6-dione | 3-(5-(6-((4'-fluoro-5,5-dimethyl-3,4,5,6-tetrahydro-[1,1'-biphenyl]-2-yl)methyl)-2,6-diazaspiro[3.3]heptane-2-carbonyl)-1-oxoisoindolin-2-yl)piperidine-2,6-dione |
| GT-05447 | 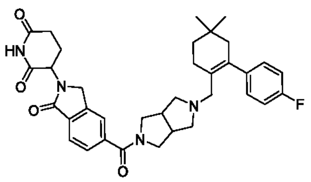 | 3-(5-(5-((4'-fluoro-5,5-dimethyl-3,4,5,6-tetrahydro-[1,1'-biphenyl]-2-yl)methyl)octahydropyrrolo[ 3,4-c]pyrrole-2-carbonyl)-1-oxoisoindolin-2-yl)piperidine-2,6-dione | 3-(5-(5-((4'-fluoro-5,5-dimethyl-3,4,5,6-tetrahydro-[1,1'-biphenyl]-2-yl)methyl)octahydropyrrolo[3 ,4-c]pyrrole-2-carbonyl)-1-oxoisoindolin-2-yl)piperidine-2,6-dione |
| GT-05450 | 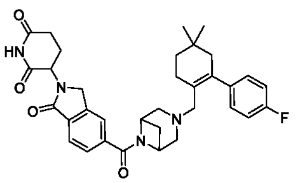 | 3-(5-(3-((4'-fluoro-5,5-dimethyl-3,4,5,6-tetrahydro-[1,1'-biphenyl]-2-yl)methyl)-3,6-diazabicyclo[3.1.1]heptane-6-carbonyl)-1-oxoisoindolin-2-yl)piperidine-2,6-dione | 3-(5-(3-((4'-fluoro-5,5-dimethyl-3,4,5,6-tetrahydro-[1,1'-biphenyl]-2-yl)methyl)-3,6-diazabicyclo[3.1.1]heptane-6-carbonyl)-1-oxoisoindolin-2-yl)piperidine-2,6-dione |
| GT-05671 | 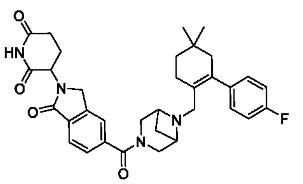 | 3-(5-(6-((4'-fluoro-5,5-dimethyl-3,4,5,6-tetrahydro-[1,1'-biphenyl]-2-yl)methyl)-3,6-diazabicyclo[3.1.1]heptane-3-carbonyl)-1-oxoisoindolin-2-yl)piperidine-2,6-dione | 3-(5-(6-((4'-fluoro-5,5-dimethyl-3,4,5,6-tetrahydro-[1,1'-biphenyl]-2-yl)methyl)-3,6-diazabicyclo[3.1.1]heptane-3-carbonyl)-1-oxoisoindolin-2-yl)piperidine-2,6-dione |
| GT-05451 | 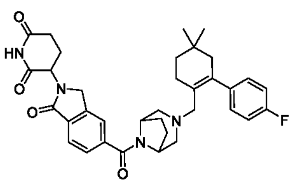 | 3-(5-(3-((4'-fluoro-5,5-dimethyl-3,4,5,6-tetrahydro-[1,1'-biphenyl]-2-yl)methyl)-3,8-diazabicyclo[3.2.1]octane-8-carbonyl)-1-oxoisoindolin-2-yl)piperidine-2,6-dione | 3-(5-(3-((4'-fluoro-5,5-dimethyl-3,4,5,6-tetrahydro-[1,1'-biphenyl]-2-yl)methyl)-3,8-diazabicyclo[3.2.1]octane-8-carbonyl)-1-oxoisoindolin-2-yl)piperidine-2,6-dione |
| GT-05511 | 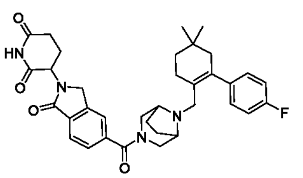 | 3-(5-(8-((4'-fluoro-5,5-dimethyl-3,4,5,6-tetrahydro-[1,1'-biphenyl]-2-yl)methyl)-3,8-diazabicyclo[3.2.1]octane-3-carbonyl)-1-oxoisoindolin-2-yl)piperidine-2,6-dione | 3-(5-(8-((4'-fluoro-5,5-dimethyl-3,4,5,6-tetrahydro-[1,1'-biphenyl]-2-yl)methyl)-3,8-diazabicyclo[3.2.1]octane-3-carbonyl)-1-oxoisoindolin-2-yl)piperidine-2,6-dione |

(continued)

| Compound No. | Structure of the compounds | The compounds' name | Chinese translation of the compounds' name |
|---|---|---|---|
| GT-05513 | 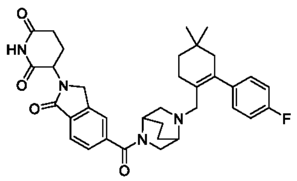 | 3-(5-(5-((4'-fluoro-5,5-dimethyl-3,4,5,6-tetrahydro-[1,1'-biphenyl]-2-yl)methyl)-2,5-diazabicyclo[2.2.2]octane-2-carbonyl)-1-oxoisoindolin-2-yl)piperidine-2,6-dione | 3-(5-(5-((4'-fluoro-5,5-dimethyl-3,4,5,6-tetrahydro-[1,1'-biphenyl]-2-yl)methyl)-2,5-diazabicyclo[2.2.2]octane-2-carbonyl)-1-oxoisoindolin-2-yl)piperidine-2,6-dione |
| | 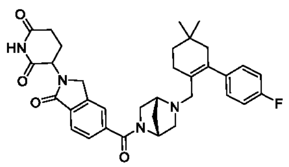 | 3-(5-((1R,4R)-5-((4'-fluoro-5,5-dimethyl-3,4,5,6-tetrahydro-[1,1'-biphenyl]-2-yl)methyl)-2,5-diazabicyclo[2.2.1]heptane-2-carbonyl)-1-oxoisoindolin-2-yl)piperidine-2,6-dione | 3-(5-((1R,4R)-5-((4'-fluoro-5,5-dimethyl-3,4,5,6-tetrahydro-[1,1'-biphenyl]-2-yl)methyl)-2,5-diazabicyclo[2.2.1]heptane-2-carbonyl)-1-oxoisoindolin-2-yl)piperidine-2,6-dione |
| | 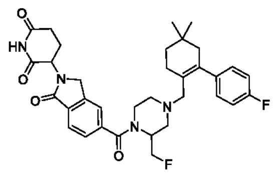 | 3-(5-(4-((4'-fluoro-5,5-dimethyl-3,4,5,6-tetrahydro-[1,1'-biphenyl]-2-yl)methyl)-2-(fluoromethyl)piperazine-1-carbonyl)-1-oxoisoindolin-2-yl)piperidine-2,6-dione | 3-(5-(4-((4'-fluoro-5,5-dimethyl-3,4,5,6-tetrahydro-[1,1'-biphenyl]-2-yl)methyl)-2-(fluoromethyl)piperazine-1-carbonyl)-1-oxoisoindolin-2-yl)piperidine-2,6-dione |
| | 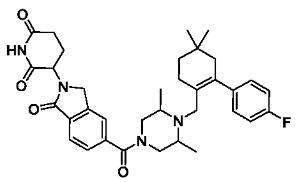 | 3-(5-(4-((4'-fluoro-5,5-dimethyl-3,4,5,6-tetrahydro-[1,1'-biphenyl]-2-yl)methyl)-3,5-dimethylpiperazine-1-carbonyl)-1-oxoisoindolin-2-yl)piperidine-2,6-dione | 3-(5-(4-((4'-fluoro-5,5-dimethyl-3,4,5,6-tetrahydro-[1,1'-biphenyl]-2-yl)methyl)-3,5-dimethylpiperazine-1-carbonyl)-1-oxoisoindolin-2-yl)piperidine-2,6-dione |
| | 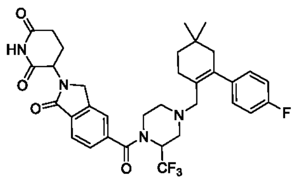 | 3-(5-(4-((4'-fluoro-5,5-dimethyl-3,4,5,6-tetrahydro-[1,1'-biphenyl]-2-yl)methyl)-2-(trifluoromethyl)piperazine-1-carbonyl)-1-oxoisoindolin-2-yl)piperidine-2,6-dione | 3-(5-(4-((4'-fluoro-5,5-dimethyl-3,4,5,6-tetrahydro-[1,1'-biphenyl]-2-yl)methyl)-2-(trifluoromethyl)piperazine-1-carbonyl)-1-oxoisoindolin-2-yl)piperidine-2,6-dione |
| | 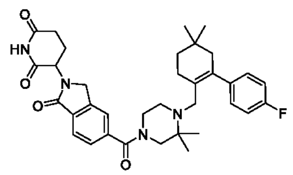 | 3-(5-(4-((4'-fluoro-5,5-dimethyl-3,4,5,6-tetrahydro-[1,1'-biphenyl]-2-yl)methyl)-3,3-dimethylpiperazine-1-carbonyl)-1-oxoisoindolin-2-yl)piperidine-2,6-dione | 3-(5-(4-((4'-fluoro-5,5-dimethyl-3,4,5,6-tetrahydro-[1,1'-biphenyl]-2-yl)methyl)-3,3-dimethylpiperazine-1-carbonyl)-1-oxoisoindolin-2-yl)piperidine-2,6-dione |
| | 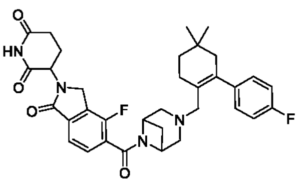 | 3-(4-fluoro-5-(3-((4'-fluoro-5,5-dimethyl-3,4,5,6-tetrahydro-[1,1'-biphenyl]-2-yl)methyl)-3,6-diazabicyclo[3.1.1]heptane-6-carbonyl)-1-oxoisoindolin-2-yl)piperidine-2,6-dione | 3-(4-fluoro-5-(3-((4'-fluoro-5,5-dimethyl-3,4,5,6-tetrahydro-[1,1'-biphenyl]-2-yl)methyl)-3,6-diazabicyclo[3.1.1]heptane-6-carbonyl)-1-oxoisoindolin-2-yl)piperidine-2,6-dione |
| | 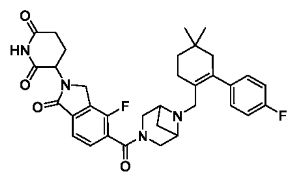 | 3-(4-fluoro-5-(6-((4'-fluoro-5,5-dimethyl-3,4,5,6-tetrahydro-[1,1'-biphenyl]-2-yl)methyl)-3,6-diazabicyclo[3.1.1]heptane-3-carbonyl)-1-oxoisoindolin-2-yl)piperidine-2,6-dione | 3-(4-fluoro-5-(6-((4'-fluoro-5,5-dimethyl-3,4,5,6-tetrahydro-[1,1'-biphenyl]-2-yl)methyl)-3,6-diazabicyclo[3.1.1]heptane-3-carbonyl)-1-oxoisoindolin-2-yl)piperidine-2,6-dione |

(continued)

| Compound No. | Structure of the compounds | The compounds' name | Chinese translation of the compounds' name |
|---|---|---|---|
| | 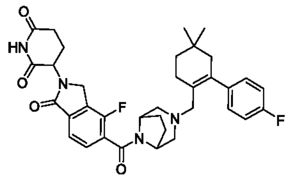 | 3-(4-fluoro-5-(3-((4'-fluoro-5,5-dimethyl-3,4,5,6-tetrahydro-[1,1'-biphenyl]-2-yl) methyl)-3,8-diazabicyclo[3.2.1] octane-8-carbonyl)-1-oxoisoindolin-2-yl)piperidine-2,6-dione | 3-(4-fluoro-5-(3-((4'-fluoro-5,5-dimethyl-3,4,5,6-tetrahydro-[1,1'-biphenyl]-2-yl) methyl)-3,8-diazabicyclo[3.2.1] octane-8-carbonyl)-1-oxoisoindolin-2-yl)piperidine-2,6-dione |
| | 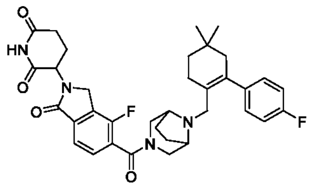 | 3-(4-fluoro-5-(8-((4'-fluoro-5,5-dimethyl-3,4,5,6-tetrahydro-[1,1'-biphenyl]-2-yl) methyl)-3,8-diazabicyclo[3.2.1] octane-3-carbonyl)-1-oxoisoindolin-2-yl)piperidine-2,6-dione | 3-(4-fluoro-5-(8-((4'-fluoro-5,5-dimethyl-3,4,5,6-tetrahydro-[1,1'-biphenyl]-2-yl) methyl)-3,8-diazabicyclo[3.2.1] octane-3-carbonyl)-1-oxoisoindolin-2-yl)piperidine-2,6-dione |
| | 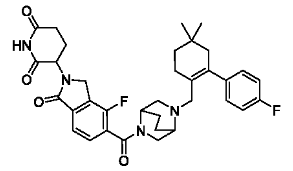 | 3-(4-fluoro-5-(5-((4'-fluoro-5,5-dimethyl-3,4,5,6-tetrahydro-[1,1'-biphenyl]-2-yl) methyl)-2,5-diazabicyclo[2.2.2] octane-2-carbonyl)-1-oxoisoindolin-2-yl)piperidine-2,6-dione | 3-(4-fluoro-5-(5-((4'-fluoro-5,5-dimethyl-3,4,5,6-tetrahydro-[1,1'-biphenyl]-2-yl) methyl)-2,5-diazabicyclo[2.2.2] octane-2-carbonyl)-1-oxoisoindolin-2-yl)piperidine-2,6-dione |
| | 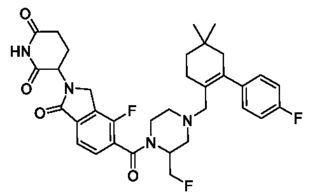 | 3-(4-fluoro-5-(4-((4'-fluoro-5,5-dimethyl-3,4,5,6-tetrahydro-[1,1'-biphenyl]-2-yl) methyl)-2-(fluoromethyl)piperazine-1-carbonyl)-1-oxoisoindolin-2-yl)piperidine-2,6-dione | 3-(4-fluoro-5-(4-((4'-fluoro-5,5-dimethyl-3,4,5,6-tetrahydro-[1,1'-biphenyl]-2-yl) methyl)-2-(fluoromethyl)piperazine-1-carbonyl)-1-oxoisoindolin-2-yl)piperidine-2,6-dione |
| | 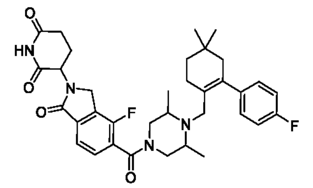 | 3-(4-fluoro-5-(4-((4'-fluoro-5,5-dimethyl-3,4,5,6-tetrahydro-[1,1'-biphenyl]-2-yl) methyl)-3,5-dimethylpiperazine-1-carbonyl)-1-oxoisoindolin-2-yl)piperidine-2,6-dione | 3-(4-fluoro-5-(4-((4'-fluoro-5,5-dimethyl-3,4,5,6-tetrahydro-[1,1'-biphenyl]-2-yl) methyl)-3,5-dimethylpiperazine-1-carbonyl)-1-oxoisoindolin-2-yl)piperidine-2,6-dione |
| | 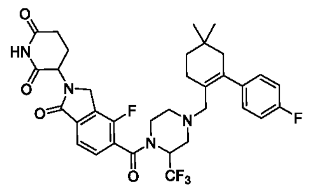 | 3-(4-fluoro-5-(4-((4'-fluoro-5,5-dimethyl-3,4,5,6-tetrahydro-[1,1'-biphenyl]-2-yl) methyl)-2-(trifluoromethyl)piperazine-1-carbonyl)-1-oxoisoindolin-2-yl)piperidine-2,6-dione | 3-(4-fluoro-5-(4-((4'-fluoro-5,5-dimethyl-3,4,5,6-tetrahydro-[1,1'-biphenyl]-2-yl) methyl)-2-(trifluoromethyl)piperazine-1-carbonyl)-1-oxoisoindolin-2-yl)piperidine-2,6-dione |
| | 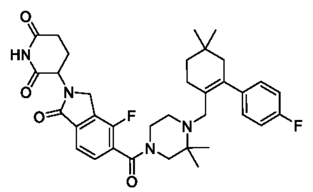 | 3-(4-fluoro-5-(4-((4'-fluoro-5,5-dimethyl-3,4,5,6-tetrahydro-[1,1'-biphenyl]-2-yl) methyl)-3,3-dimethylpiperazine-1-carbonyl)-1-oxoisoindolin-2-yl)piperidine-2,6-dione | 3-(4-fluoro-5-(4-((4'-fluoro-5,5-dimethyl-3,4,5,6-tetrahydro-[1,1'-biphenyl]-2-yl) methyl)-3,3-dimethylpiperazine-1-carbonyl)-1-oxoisoindolin-2-yl)piperidine-2,6-dione |
| | 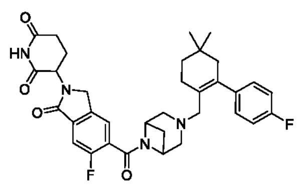 | 3-(6-fluoro-5-(3-((4'-fluoro-5,5-dimethyl-3,4,5,6-tetrahydro-[1,1'-biphenyl]-2-yl) methyl)-3,6-diazabicyclo[3.1.1] heptane-6-carbonyl)-1-oxoisoindolin-2-yl)piperidine-2,6-dione | 3-(6-fluoro-5-(3-((4'-fluoro-5,5-dimethyl-3,4,5,6-tetrahydro-[1,1'-biphenyl]-2-yl) methyl)-3,6-diazabicyclo[3.1.1] heptane-6-carbonyl)-1-oxoisoindolin-2-yl)piperidine-2,6-dione |

(continued)

| Compo und No. | Structure of the compounds | The compounds' name | Chinese translation of the compounds' name |
|---|---|---|---|
| | 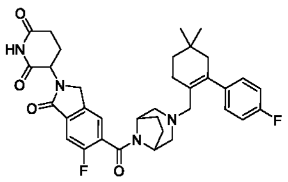 | 3-(6-fluoro-5-(3-((4'-fluoro-5,5-dimethyl-3,4,5,6-tetrahydro-[1,1'-biphenyl]-2-yl) methyl)-3,8-diazabicyclo[3.2.1] octane-8-carbonyl)-1-oxoisoindolin-2-yl)piperidine-2,6-dione | 3-(6-fluoro-5-(3-((4'-fluoro-5,5-dimethyl-3,4,5,6-tetrahydro-[1,1'-biphenyl]-2-yl) methyl)-3,8-diazabicyclo[3.2.1] octane-8-carbonyl)-1-oxoisoindolin-2-yl)piperidine-2,6-dione |
| | 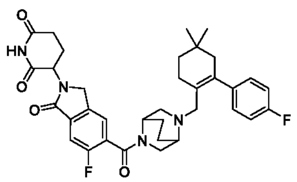 | 3-(6-fluoro-5-(5-((4'-fluoro-5,5-dimethyl-3,4,5,6-tetrahydro-[1,1'-biphenyl]-2-yl) methyl)-2,5-diazabicyclo[2.2.2] octane-2-carbonyl)-1-oxoisoindolin-2-yl)piperidine-2,6-dione | 3-(6-fluoro-5-(5-((4'-fluoro-5,5-dimethyl-3,4,5,6-tetrahydro-[1,1'-biphenyl]-2-yl) methyl)-2,5-diazabicyclo[2.2.2] octane-2-carbonyl)-1-oxoisoindolin-2-yl)piperidine-2,6-dione |
| | 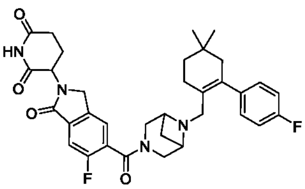 | 3-(6-fluoro-5-(6-((4'-fluoro-5,5-dimethyl-3,4,5,6-tetrahydro-[1,1'-biphenyl]-2-yl) methyl)-3,6-diazabicyclo[3.1.1] heptane-3-carbonyl)-1-oxoisoindolin-2-yl)piperidine-2,6-dione | 3-(6-fluoro-5-(6-((4'-fluoro-5,5-dimethyl-3,4,5,6-tetrahydro-[1,1'-biphenyl]-2-yl) methyl)-3,6-diazabicyclo[3.1.1] heptane-3-carbonyl)-1-oxoisoindolin-2-yl)piperidine-2,6-dione |
| | 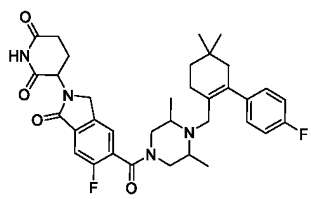 | 3-(6-fluoro-5-(4-((4'-fluoro-5,5-dimethyl-3,4,5,6-tetrahydro-[1,1'-biphenyl]-2-yl) methyl)-3,5-dimethylpiperazine-1-carbonyl)-1-oxoisoindolin-2-yl)piperidine-2,6-dione | 3-(6-fluoro-5-(4-((4'-fluoro-5,5-dimethyl-3,4,5,6-tetrahydro-[1,1'-biphenyl]-2-yl) methyl)-3,5-dimethylpiperazine-1-carbonyl)-1-oxoisoindolin-2-yl)piperidine-2,6-dione |
| | 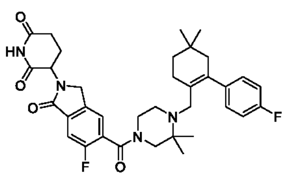 | 3-(6-fluoro-5-(4-((4'-fluoro-5,5-dimethyl-3,4,5,6-tetrahydro-[1,1'-biphenyl]-2-yl) methyl)-3,3-dimethylpiperazine-1-carbonyl)-1-oxoisoindolin-2-yl)piperidine-2,6-dione | 3-(6-fluoro-5-(4-((4'-fluoro-5,5-dimethyl-3,4,5,6-tetrahydro-[1,1'-biphenyl]-2-yl) methyl)-3,3-dimethylpiperazine-1-carbonyl)-1-oxoisoindolin-2-yl)piperidine-2,6-dione |
| | 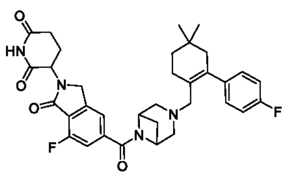 | 3-(7-fluoro-5-(3-((4'-fluoro-5,5-dimethyl-3,4,5,6-tetrahydro-[1,1'-biphenyl]-2-yl) methyl)-3,6-diazabicyclo[3.1.1] heptane-6-carbonyl)-1-oxoisoindolin-2-yl)piperidine-2,6-dione | 3-(7-fluoro-5-(3-((4'-fluoro-5,5-dimethyl-3,4,5,6-tetrahydro-[1,1'-biphenyl]-2-yl) methyl)-3,6-diazabicyclo[3.1.1] heptane-6-carbonyl)-1-oxoisoindolin-2-yl)piperidine-2,6-dione |
| | 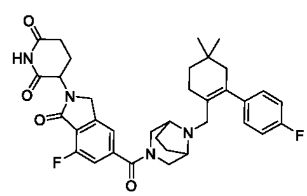 | 3-(7-fluoro-5-(8-((4'-fluoro-5,5-dimethyl-3,4,5,6-tetrahydro-[1,1'-biphenyl]-2-yl) methyl)-3,8-diazabicyclo[3.2.1] octane-3-carbonyl)-1-oxoisoindolin-2-yl)piperidine-2,6-dione | 3-(7-fluoro-5-(8-((4'-fluoro-5,5-dimethyl-3,4,5,6-tetrahydro-[1,1'-biphenyl]-2-yl) methyl)-3,8-diazabicyclo[3.2.1] octane-3-carbonyl)-1-oxoisoindolin-2-yl)piperidine-2,6-dione |
| | 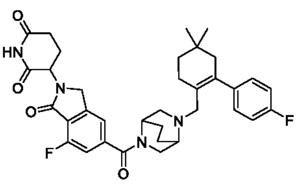 | 3-(7-fluoro-5-(5-((4'-fluoro-5,5-dimethyl-3,4,5,6-tetrahydro-[1,1'-biphenyl]-2-yl) methyl)-2,5-diazabicyclo[2.2.2] octane-2-carbonyl)-1-oxoisoindolin-2-yl)piperidine-2,6-dione | 3-(7-fluoro-5-(5-((4'-fluoro-5,5-dimethyl-3,4,5,6-tetrahydro-[1,1'-biphenyl]-2-yl) methyl)-2,5-diazabicyclo[2.2.2] octane-2-carbonyl)-1-oxoisoindolin-2-yl)piperidine-2,6-dione |

(continued)

| Compo und No. | Structure of the compounds | The compounds' name | Chinese translation of the compounds' name |
|---|---|---|---|
| | 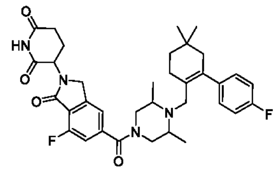 | 3-(7-fluoro-5-(4-((4'-fluoro-5,5-dimethyl-3,4,5,6-tetrahydro-[1,1'-biphenyl]-2-yl) methyl)-3,5-dimethylpipera-zine-1-carbonyl)-1-oxoisoindo-lin-2-yl)piperidine-2,6-dione | 3-(7-fluoro-5-(4-((4'-fluoro-5,5-dimethyl-3,4,5,6-tetrahydro-[1,1'-biphenyl]-2-yl) methyl)-3,5-dimethylpipera-zine-1-carbonyl)-1-oxoisoindo-lin-2-yl)piperidine-2,6-dione |
| | 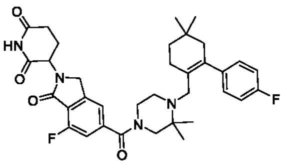 | 3-(7-fluoro-5-(4-((4'-fluoro-5,5-dimethyl-3,4,5,6-tetrahydro-[1,1'-biphenyl]-2-yl) methyl)-3,3-dimethylpipera-zine-1-carbonyl)-1-oxoisoindo-lin-2-yl)piperidine-2,6-dione | 3-(7-fluoro-5-(4-((4'-fluoro-5,5-dimethyl-3,4,5,6-tetrahydro-[1,1'-biphenyl]-2-yl) methyl)-3,3-dimethylpipera-zine-1-carbonyl)-1-oxoisoindo-lin-2-yl)piperidine-2,6-dione |
| GT-05792 | 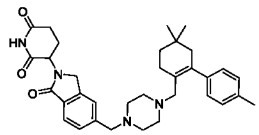 | 3-(1-oxo-5-((4-((4',5,5-trimethyl-3,4,5,6-tetrahydro-[1,1'-biphenyl]-2-yl)methyl)piperazin-1-yl)methyl)isoindolin-2-yl) piperidine-2,6-dione | 3-(1-oxo-5-((4-((4',5,5-trimethyl-3,4,5,6-tetrahydro-[1,1'-biphenyl]-2-yl)methyl)piperazin-1-yl)methyl)isoindolin-2-yl) piperidine-2,6-dione |
| | 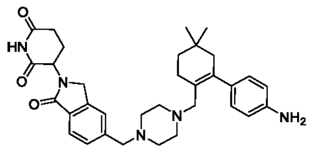 | 3-(5-((4-((4'-amino-5,5-dimethyl-3,4,5,6-tetrahydro-[1,1'-biphenyl]-2-yl)methyl)piperazin-1-yl)methyl)-1-oxoisoindolin-2-yl)piperidine-2,6-dione | 3-(5-((4-((4'-amino-5,5-dimethyl-3,4,5,6-tetrahydro-[1,1'-biphenyl]-2-yl)methyl)piperazin-1-yl)methyl)-1-oxoisoindolin-2-yl)piperidine-2,6-dione |
| | 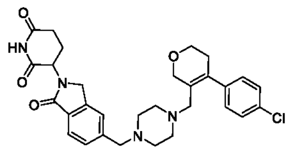 | 3-(5-((4-((4-(4-chlorophenyl)-5,6-dihydro-2H-pyran-3-yl) methyl)piperazin-1-yl) methyl)-1-oxoisoindolin-2-yl)piperidine-2,6-dione | 3-(5-((4-((4-(4-chlorophenyl)-5,6-dihydro-2H-pyran-3-yl) methyl)piperazin-1-yl) methyl)-1-oxoisoindolin-2-yl)pi-peridine-2,6-dione |
| | 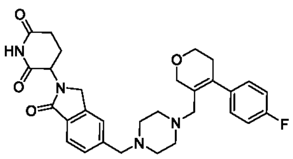 | 3-(5-((4-((4-(4-fluorophenyl)-5,6-dihydro-2H-pyran-3-yl) methyl)piperazin-1-yl) methyl)-1-oxoisoindolin-2-yl)piperidine-2,6-dione | 3-(5-((4-((4-(4-fluorophenyl)-5,6-dihydro-2H-pyran-3-yl) methyl)piperazin-1-yl) methyl)-1-oxoisoindolin-2-yl)pi-peridine-2,6-dione |
| | 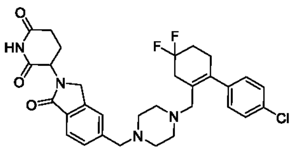 | 3-(5-((4-((4'-chloro-4,4-difluoro-3,4,5,6-tetrahydro-[1,1'-biphenyl]-2-yl)methyl)piperazin-1-yl)methyl)-1-oxoisoindolin-2-yl)piperidine-2,6-dione | 3-(5-((4-((4'-chloro-4,4-difluoro-3,4,5,6-tetrahydro-[1,1'-biphenyl]-2-yl)methyl)piperazin-1-yl)methyl)-1-oxoisoindolin-2-yl)piperidine-2,6-dione |
| | 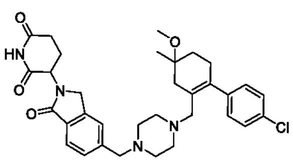 | 3-(5-((4-((4'-chloro-4-methoxy-4-methyl-3,4,5,6-tetrahydro-[1,1'-biphenyl]-2-yl)methyl) piperazin-1-yl)methyl)-1-oxoisoindolin-2-yl)piperidine-2,6-dione | 3-(5-((4-((4'-chloro-4-methoxy-4-methyl-3,4,5,6-tetrahydro-[1,1'-biphenyl]-2-yl)methyl) piperazin-1-yl)methyl)-1-oxoisoindolin-2-yl)piperidine-2,6-dione |
| | 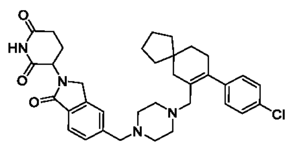 | 3-(5-((4-((8-(4-chlorophenyl) spiro[4.5]dec-7-en-7-yl)methyl) piperazin-1-yl)methyl)-1-oxoisoindolin-2-yl)piperidine-2,6-dione | 3-(5-((4-((8-(4-chlorophenyl) spiro[4.5]dec-7-en-7-yl)methyl) piperazin-1-yl)methyl)-1-oxoisoindolin-2-yl)piperidine-2,6-dione |

(continued)

| Compo und No. | Structure of the compounds | The compounds' name | Chinese translation of the compounds' name |
|---|---|---|---|
| | 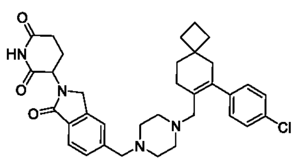 | 3-(5-((4-((6-(4-chlorophenyl) spiro[3.5]non-6-en-7-yl)methyl) piperazin-1-yl)methyl)-1-oxoisoindolin-2-yl)piperidine-2,6-dione | 3-(5-((4-((6-(4-chlorophenyl) spiro[3.5]non-6-en-7-yl)methyl) piperazin-1-yl)methyl)-1-oxoisoindolin-2-yl)piperidine-2,6-dione |
| | 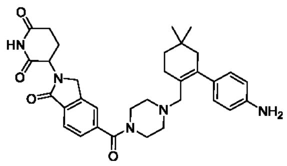 | 3-(5-(4-((4'-amino-5,5-dimethyl-3,4,5,6-tetrahydro-[1,1'-biphenyl]-2-yl)methyl)piperazine-1-carbonyl)-1-oxoisoindolin-2-yl)piperidine-2,6-dione | 3-(5-(4-((4'-amino-5,5-dimethyl-3,4,5,6-tetrahydro-[1,1'-biphenyl]-2-yl)methyl)piperazine-1-carbonyl)-1-oxoisoindolin-2-yl)piperidine-2,6-dione |
| GT-05863 | 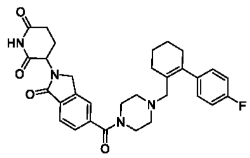 | 3-(5-(4-((4'-fluoro-3,4,5,6-tetrahydro-[1,1'-biphenyl]-2-yl) methyl)piperazine-1-carbonyl)-1-oxoisoindolin-2-yl)piperidine-2,6-dione | 3-(5-(4-((4'-fluoro-3,4,5,6-tetrahydro-[1,1'-biphenyl]-2-yl) methyl)piperazine-1-carbonyl)-1-oxoisoindolin-2-yl)piperidine-2,6-dione |
| | 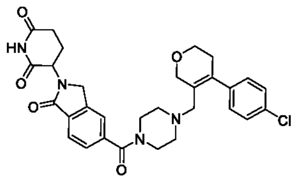 | 3-(5-(4-((4-(4-chlorophenyl)-5,6-dihydro-2H-pyran-3-yl) methyl)piperazine-1-carbonyl)-1-oxoisoindolin-2-yl)piperidine-2,6-dione | 3-(5-(4-((4-(4-chlorophenyl)-5,6-dihydro-2H-pyran-3-yl) methyl)piperazine-1-carbonyl)-1-oxoisoindolin-2-yl)piperidine-2,6-dione |
| | 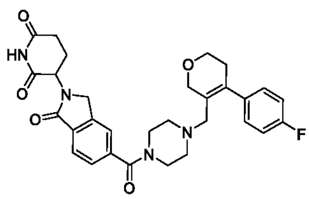 | 3-(5-(4-((4-(4-fluorophenyl)-5,6-dihydro-2H-pyran-3-yl)methyl) piperazine-1-carbonyl)-1-oxoisoindolin-2-yl)piperidine-2,6-dione | 3-(5-(4-((4-(4-fluorophenyl)-5,6-dihydro-2H-pyran-3-yl)methyl) piperazine-1-carbonyl)-1-oxoisoindolin-2-yl)piperidine-2,6-dione |
| | 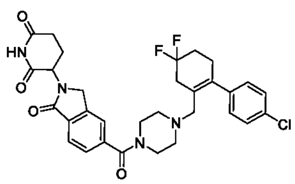 | 3-(5-(4-((4'-chloro-4,4-difluoro-3,4,5,6-tetrahydro-[1,1'-biphenyl]-2-yl)methyl)piperazine-1-carbonyl)-1-oxoisoindolin-2-yl)piperidine-2,6-dione | 3-(5-(4-((4'-chloro-4,4-difluoro-3,4,5,6-tetrahydro-[1,1'-biphenyl]-2-yl)methyl)piperazine-1-carbonyl)-1-oxoisoindolin-2-yl)piperidine-2,6-dione |
| | 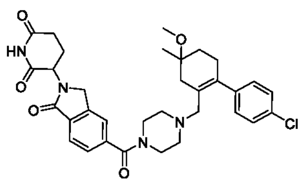 | 3-(5-(4-((4'-chloro-4-methoxy-4-methyl-3,4,5,6-tetrahydro-[1,1'-biphenyl]-2-yl)methyl) piperazine-1-carbonyl)-1-oxoisoindolin-2-yl)piperidine-2,6-dione | 3-(5-(4-((4'-chloro-4-methoxy-4-methyl-3,4,5,6-tetrahydro-[1,1'-biphenyl]-2-yl)methyl) piperazine-1-carbonyl)-1-oxoisoindolin-2-yl)piperidine-2,6-dione |
| GT-05673 | 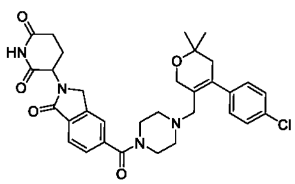 | 3-(5-(4-((4-(4-chlorophenyl)-6,6-dimethyl-5,6-dihydro-2H-pyran-3-yl)methyl)piperazine-1-carbonyl)-1-oxoisoindolin-2-yl)piperidine-2,6-dione | 3-5-(4-((4-(4-chlorophenyl)-6,6-dimethyl-5,6-dihydro-2H-pyran-3-yl)methyl)piperazine-1-carbonyl)-1-oxoisoindolin-2-yl) piperidine-2,6-dione |

(continued)

| Compound No. | Structure of the compounds | The compounds' name | Chinese translation of the compounds' name |
|---|---|---|---|
| | 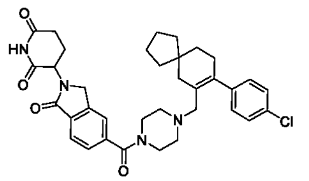 | 3-(5-(4-((8-(4-chlorophenyl) spiro[4.5]dec-7-en-7-yl)methyl) piperazine-1-carbonyl)-1-oxoisoindolin-2-yl)piperidine-2,6-dione | 3-(5-(4-((8-(4-chlorophenyl) spiro[4.5]dec-7-en-7-yl)methyl) piperazine-1-carbonyl)-1-oxoisoindolin-2-yl)piperidine-2,6-dione |
| | 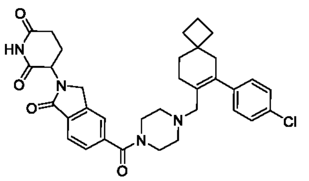 | 3-(5-(4-((6-(4-chlorophenyl) spiro[3.5]non-6-en-7-yl)methyl) piperazine-1-carbonyl)-1-oxoisoindolin-2-yl)piperidine-2,6-dione | 3-(5-(4-((6-(4-chlorophenyl) spiro[3.5]non-6-en-7-yl)methyl) piperazine-1-carbonyl)-1-oxoisoindolin-2-yl)piperidine-2,6-dione |
| GT-05364 | 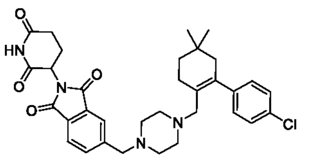 | 5-((4-((4'-chloro-5,5-dimethyl-3,4,5,6-tetrahydro-[1,1'-biphenyl]-2-yl)methyl)piperazin-1-yl)methyl)-2-(2,6-dioxopiperidin-3-yl)isoindoline-1,3-dione | 5-((4-((4'-chloro-5,5-dimethyl-3,4,5,6-tetrahydro-[1,1'-biphenyl]-2-yl)methyl)piperazin-1-yl)methyl)-2-(2,6-dioxopiperidin-3-yl)isoindoline-1,3-dione |
| | 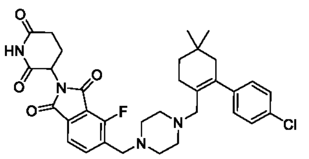 | 5-((4-((4'-chloro-5,5-dimethyl-3,4,5,6-tetrahydro-[1,1'-biphenyl]-2-yl)methyl)piperazin-1-yl)methyl)-2-(2,6-dioxopiperidin-3 -yl)-4-fluoroisoindoline-1,3-dione | 5-((4-((4'-chloro-5,5-dimethyl-3,4,5,6-tetrahydro-[1,1'-biphenyl]-2-yl)methyl)piperazin-1-yl)methyl)-2-(2,6-dioxopiperidin-3-yl)-4-fluoroisoindoline-1,3-dione |
| | 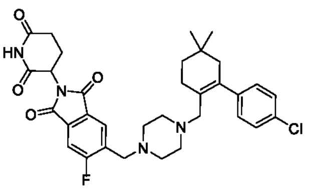 | 5-((4-((4'-chloro-5,5-dimethyl-3,4,5,6-tetrahydro-[1,1'-biphenyl]-2-yl)methyl)piperazin-1-yl)methyl)-2-(2,6-dioxopiperidin-3-yl)-6-fluoroisoindoline-1,3-dione | 5-((4-((4'-chloro-5,5-dimethyl-3,4,5,6-tetrahydro-[1,1'-biphenyl]-2-yl)methyl)piperazin-1-yl)methyl)-2-(2,6-dioxopiperidin-3-yl)-6-fluoroisoindoline-1,3-dione |
| | 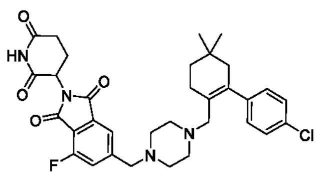 | 6-((4-((4'-chloro-5,5-dimethyl-3,4,5,6-tetrahydro-[1,1'-biphenyl]-2-yl)methyl)piperazin-1-yl)methyl)-2-(2,6-dioxopiperidin-3 -yl)-4-fluoroisoindoline-1,3-dione | 6-((4-((4'-chloro-5,5-dimethyl-3,4,5,6-tetrahydro-[1,1'-biphenyl]-2-yl)methyl)piperazin-1-yl)methyl)-2-(2,6-dioxopiperidin-3-yl)-4-fluoroisoindoline-1,3-dione |
| GT-05365 | 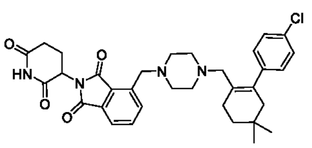 | 4-((4-((4'-chloro-5,5-dimethyl-3,4,5,6-tetrahydro-[1,1'-biphenyl]-2-yl)methyl)piperazin-1-yl)methyl)-2-(2,6-dioxopiperidin-3-yl)isoindoline-1,3-dione | 4-((4-((4'-chloro-5,5-dimethyl-3,4,5,6-tetrahydro-[1,1'-biphenyl]-2-yl)methyl)piperazin-1-yl)methyl)-2-(2,6-dioxopiperidin-3-yl)isoindoline-1,3-dione |
| | 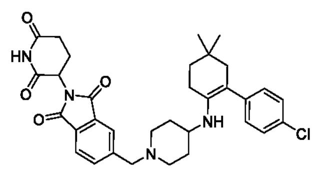 | 5-((4-((4'-chloro-5,5-dimethyl-3,4,5,6-tetrahydro-[1,1'-biphenyl]-2-yl)amino)piperidin-1-yl)methyl)-2-(2,6-dioxopiperidin-3-yl)isoindoline-1,3-dione | 5-((4-((4'-chloro-5,5-dimethyl-3,4,5,6-tetrahydro-[1,1'-biphenyl]-2-yl)amino)piperidin-1-yl)methyl)-2-(2,6-dioxopiperidin-3-yl)isoindoline-1,3-dione |

(continued)

| Compound No. | Structure of the compounds | The compounds' name | Chinese translation of the compounds' name |
|---|---|---|---|
| | 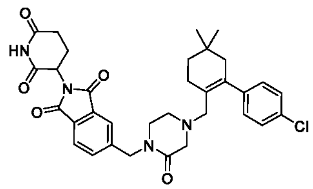 | 5-((4-((4'-chloro-5,5-dimethyl-3,4,5,6-tetrahydro-[1,1'-biphenyl]-2-yl)methyl)-2-oxopiperazin-1-yl)methyl)-2-(2,6-dioxopiperidin-3-yl)isoindoline-1,3-dione | 5-((4-((4'-chloro-5,5-dimethyl-3,4,5,6-tetrahydro-[1,1'-biphenyl]-2-yl)methyl)-2-oxopiperazin-1-yl)methyl)-2-(2,6-dioxopiperidin-3-yl)isoindoline-1,3-dione |
| | 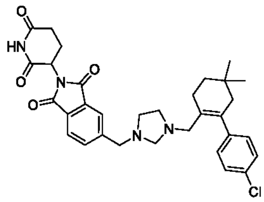 | 5-((3-((4'-chloro-5,5-dimethyl-3,4,5,6-tetrahydro-[1,1'-biphenyl]-2-yl)methyl)imidazolidin-1-yl)methyl)-2-(2,6-dioxopiperidin-3-yl)isoindoline-1,3-dione | 5-((3-((4'-chloro-5,5-dimethyl-3,4,5,6-tetrahydro-[1,1'-biphenyl]-2-yl)methyl)imidazolidin-1-yl)methyl)-2-(2,6-dioxopiperidin-3-yl)isoindoline-1,3-dione |
| GT-05397 | 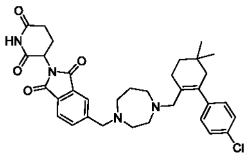 | 5-((4-((4'-chloro-5,5-dimethyl-3,4,5,6-tetrahydro-[1,1'-biphenyl]-2-yl)methyl)-1,4-diazepan-1-yl)methyl)-2-(2,6-dioxopiperidin-3-yl)isoindoline-1,3-dione | 5-((4-((4'-chloro-5,5-dimethyl-3,4,5,6-tetrahydro-[1,1'-biphenyl]-2-yl)methyl)-1,4-diazepan-1-yl)methyl)-2-(2,6-dioxopiperidin-3-yl)isoindoline-1,3-dione |
| GT-05678 | 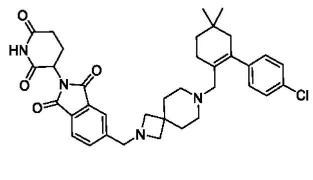 | 5-((7-((4'-chloro-5,5-dimethyl-3,4,5,6-tetrahydro-[1,1'-biphenyl]-2-yl)methyl)-2,7-diazaspiro[3.5]nonan-2-yl) methyl)-2-(2,6-dioxopiperidin-3-yl)isoindoline-1,3-dione | 5-((7-((4'-chloro-5,5-dimethyl-3,4,5,6-tetrahydro-[1,1'-biphenyl]-2-yl)methyl)-2,7-diazaspiro[3.5]nonan-2-yl) methyl)-2-(2,6-dioxopiperidin-3-yl)isoindoline-1,3-dione |
| GT-05675 | 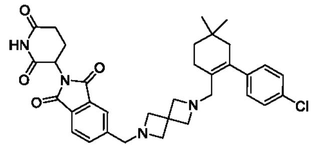 | 5-((6-((4'-chloro-5,5-dimethyl-3,4,5,6-tetrahydro-[1,1'-biphenyl]-2-yl)methyl)-2,6-diazaspiro[3.3]heptan-2-yl) methyl)-2-(2,6-dioxopiperidin-3-yl)isoindoline-1,3-dione | 5-((6-((4'-chloro-5,5-dimethyl-3,4,5,6-tetrahydro-[1,1'-biphenyl]-2-yl)methyl)-2,6-diazaspiro[3.3]heptan-2-yl) methyl)-2-(2,6-dioxopiperidin-3-yl)isoindoline-1,3-dione |
| GT-05654 | 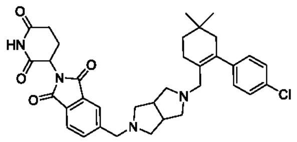 | 5-((5-((4'-chloro-5,5-dimethyl-3,4,5,6-tetrahydro-[1,1'-biphenyl]-2-yl)methyl)hexahydropyrrolo [3,4-c]pyrrol-2(1H)-yl)methyl)-2-(2,6-dioxopiperidin-3-yl)isoindoline-1,3-dione | 5-((5-((4'-chloro-5,5-dimethyl-3,4,5,6-tetrahydro-[1,1'-biphenyl]-2-yl)methyl)hexahydropyrrolo[ 3,4-c]pyrrol-2(1H)-yl)methyl)-2-(2,6-dioxopiperidin-3-yl)isoindoline-1,3-dione |
| GT-05368 | 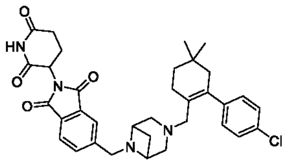 | 5-((3-((4'-chloro-5,5-dimethyl-3,4,5,6-tetrahydro-[1,1'-biphenyl]-2-yl)methyl)-3,6-diazabicyclo[3.1.1]heptan-6-yl) methyl)-2-(2,6-dioxopiperidin-3-yl)isoindoline-1,3-dione | 5-((3-((4'-chloro-5,5-dimethyl-3,4,5,6-tetrahydro-[1,1'-biphenyl]-2-yl)methyl)-3,6-diazabicyclo[3.1.1]heptan-6-yl) methyl)-2-(2,6-dioxopiperidin-3-yl)isoindoline-1,3-dione |
| GT-05532 | 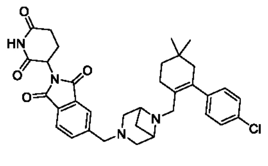 | 5-((6-((4'-chloro-5,5-dimethyl-3,4,5,6-tetrahydro-[1,1'-biphenyl]-2-yl)methyl)-3,6-diazabicyclo[3.1.1]heptan-3-yl) methyl)-2-(2,6-dioxopiperidin-3-yl)isoindoline-1,3-dione | 5-((6-((4'-chloro-5,5-dimethyl-3,4,5,6-tetrahydro-[1,1'-biphenyl]-2-yl)methyl)-3,6-diazabicyclo[3.1.1]heptan-3-yl) methyl)-2-(2,6-dioxopiperidin-3-yl)isoindoline-1,3-dione |

(continued)

| Compound No. | Structure of the compounds | The compounds' name | Chinese translation of the compounds' name |
|---|---|---|---|
| GT-04719 | 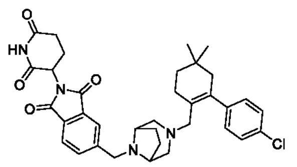 | 5-((3-((4'-chloro-5,5-dimethyl-3,4,5,6-tetrahydro-[1,1'-biphenyl]-2-yl)methyl)-3,8-diazabicyclo[3.2.1]octan-8-yl)methyl)-2-(2,6-dioxopiperidin-3-yl)isoindoline-1,3-dione | 5-((3-((4'-chloro-5,5-dimethyl-3,4,5,6-tetrahydro-[1,1'-biphenyl]-2-yl)methyl)-3,8-diazabicyclo[3.2.1]octan-8-yl)methyl)-2-(2,6-dioxopiperidin-3-yl)isoindoline-1,3-dione |
| GT-05537 | 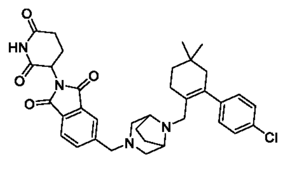 | 5-((8-((4'-chloro-5,5-dimethyl-3,4,5,6-tetrahydro-[1,1'-biphenyl]-2-yl)methyl)-3,8-diazabicyclo[3.2.1]octan-3-yl)methyl)-2-(2,6-dioxopiperidin-3-yl)isoindoline-1,3-dione | 5-((8-((4'-chloro-5,5-dimethyl-3,4,5,6-tetrahydro-[1,1'-biphenyl]-2-yl)methyl)-3,8-diazabicyclo[3.2.1]octan-3-yl)methyl)-2-(2,6-dioxopiperidin-3-yl)isoindoline-1,3-dione |
| GT-05463 | 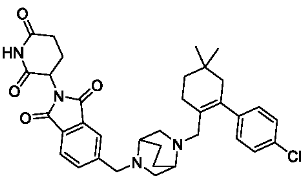 | 5-((5-((4'-chloro-5,5-dimethyl-3,4,5,6-tetrahydro-[1,1'-biphenyl]-2-yl)methyl)-2,5-diazabicyclo[2.2.2]octan-2-yl)methyl)-2-(2,6-dioxopiperidin-3-yl)isoindoline-1,3-dione | 5-((5-((4'-chloro-5,5-dimethyl-3,4,5,6-tetrahydro-[1,1'-biphenyl]-2-yl)methyl)-2,5-diazabicyclo[2.2.2]octan-2-yl)methyl)-2-(2,6-dioxopiperidin-3-yl)isoindoline-1,3-dione |
| | 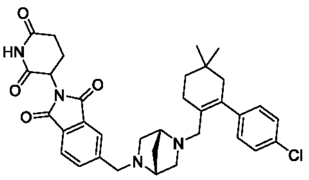 | 5-(((1R,4R)-5-((4'-chloro-5,5-dimethyl-3,4,5,6-tetrahydro-[1,1'-biphenyl]-2-yl)methyl)-2,5-diazabicyclo[2.2.1]heptan-2-yl)methyl)-2-(2,6-dioxopiperidin-3-yl)isoindoline-1,3-dione | 5-(((1R,4R)-5-((4'-chloro-5,5-dimethyl-3,4,5,6-tetrahydro-[1,1'-biphenyl]-2-yl)methyl)-2,5-diazabicyclo[2.2.1]heptan-2-yl)methyl)-2-(2,6-dioxopiperidin-3-yl)isoindoline-1,3-dione |
| | 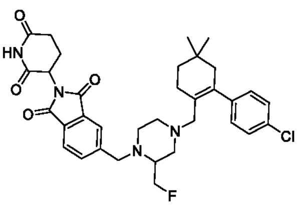 | 5-((4-((4'-chloro-5,5-dimethyl-3,4,5,6-tetrahydro-[1,1'-biphenyl]-2-yl)methyl)-2-(fluoromethyl)piperazin-1-yl)methyl)-2-(2,6-dioxopiperidin-3-yl)isoindoline-1,3-dione | 5-((4-((4'-chloro-5,5-dimethyl-3,4,5,6-tetrahydro-[1,1'-biphenyl]-2-yl)methyl)-2-(fluoromethyl)piperazin-1-yl)methyl)-2-(2,6-dioxopiperidin-3-yl)isoindoline-1,3-dione |
| | 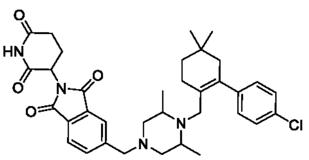 | 5-((4-((4'-chloro-5,5-dimethyl-3,4,5,6-tetrahydro-[1,1'-biphenyl]-2-yl)methyl)-3,5-dimethylpiperazin-1-yl)methyl)-2-(2,6-dioxopiperidin-3-yl)isoindoline-1,3-dione | 5-((4-((4'-chloro-5,5-dimethyl-3,4,5,6-tetrahydro-[1,1'-biphenyl]-2-yl)methyl)-3,5-dimethylpiperazin-1-yl)methyl)-2-(2,6-dioxopiperidin-3-yl)isoindoline-1,3-dione |
| GT-06103 | 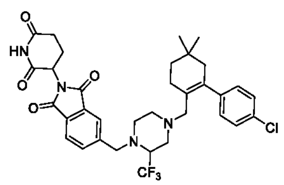 | 5-((4-((4'-chloro-5,5-dimethyl-3,4,5,6-tetrahydro-[1,1'-biphenyl]-2-yl)methyl)-2-(trifluoromethyl)piperazin-1-yl)methyl)-2-(2,6-dioxopiperidin-3-yl)isoindoline-1,3-dione | 5-((4-((4'-chloro-5,5-dimethyl-3,4,5,6-tetrahydro-[1,1'-biphenyl]-2-yl)methyl)-2-(trifluoromethyl)piperazin-1-yl)methyl)-2-(2,6-dioxopiperidin-3-yl)isoindoline-1,3-dione |
| | 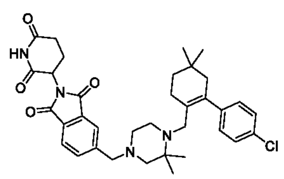 | 5-((4-((4'-chloro-5,5-dimethyl-3,4,5,6-tetrahydro-[1,1'-biphenyl]-2-yl)methyl)-3,3-dimethylpiperazin-1-yl)methyl)-2-(2,6-dioxopiperidin-3-yl)isoindoline-1,3-dione | 5-((4-((4'-chloro-5,5-dimethyl-3,4,5,6-tetrahydro-[1,1'-biphenyl]-2-yl)methyl)-3,3-dimethylpiperazin-1-yl)methyl)-2-(2,6-dioxopiperidin-3-yl)isoindoline-1,3-dione |

(continued)

| Compound No. | Structure of the compounds | The compounds' name | Chinese translation of the compounds' name |
|---|---|---|---|
| | 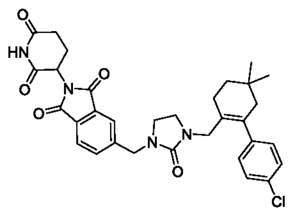 | 5-((3-((4'-chloro-5,5-dimethyl-3,4,5,6-tetrahydro-[1,1'-biphenyl]-2-yl)methyl)-2-oxoimidazolidin-1-yl)methyl)-2-(2,6-dioxopiperidin-3-yl)isoindoline-1,3-dione | 5-((3-((4'-chloro-5,5-dimethyl-3,4,5,6-tetrahydro-[1,1'-biphenyl]-2-yl)methyl)-2-oxoimidazolidin-1-yl)methyl)-2-(2,6-dioxopiperidin-3-yl)isoindoline-1,3-dione |
| GT-05395 | 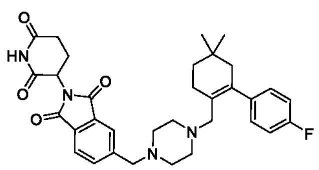 | 2-(2,6-dioxopiperidin-3-yl)-5-((4-((4'-fluoro-5,5-dimethyl-3,4,5,6-tetrahydro-[1,1'-biphenyl]-2-yl)methyl)piperazin-1-yl)methyl)isoindoline-1,3-dione | 2-(2,6-dioxopiperidin-3-yl)-5-((4-((4'-fluoro-5,5-dimethyl-3,4,5,6-tetrahydro-[1,1'-biphenyl]-2-yl)methyl)piperazin-1-yl)methyl)isoindoline-1,3-dione |
| GT-05497 | 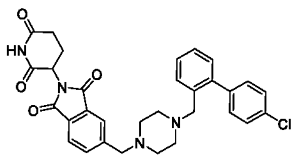 | 5-((4-((4'-chloro-[1,1'-biphenyl]-2-yl)methyl)piperazin-1-yl)methyl)-2-(2,6-dioxopiperidin-3-yl)isoindoline-1,3-dione | 5-((4-((4'-chloro-[1,1'-biphenyl]-2-yl)methyl)piperazin-1-yl)methyl)-2-(2,6-dioxopiperidin-3-yl)isoindoline-1,3-dione |
| GT-05630 | 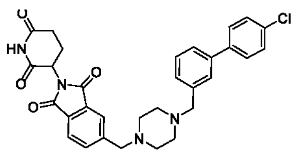 | 5-((4-((4'-chloro-[1,1'-biphenyl]-3-yl)methyl)piperazin-1-yl)methyl)-2-(2,6-dioxopiperidin-3-yl)isoindoline-1,3-dione | 5-((4-((4'-chloro-[1,1'-biphenyl]-3-yl)methyl)piperazin-1-yl)methyl)-2-(2,6-dioxopiperidin-3-yl)isoindoline-1,3-dione |
| GT-05599 | 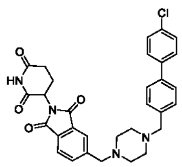 | 5-((4-((4'-chloro-[1,1'-biphenyl]-4-yl)methyl)piperazin-1-yl)methyl)-2-(2,6-dioxopiperidin-3-yl)isoindoline-1,3-dione | 5-((4-((4'-chloro-[1,1'-biphenyl]-4-yl)methyl)piperazin-1-yl)methyl)-2-(2,6-dioxopiperidin-3-yl)isoindoline-1,3-dione |
| GT-05731 | 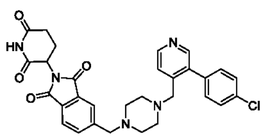 | 5-((4-((3-(4-chlorophenyl)pyridin-4-yl)methyl)piperazin-1-yl)methyl)-2-(2,6-dioxopiperidin-3-yl)isoindoline-1,3-dione | 5-((4-((3-(4-chlorophenyl)pyridin-4-yl)methyl)piperazin-1-yl)methyl)-2-(2,6-dioxopiperidin-3-yl)isoindoline-1,3-dione |
| GT-05634 | 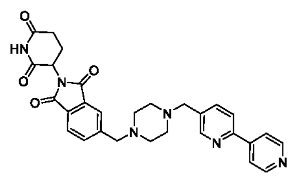 | 5-((4-([2,4'-bipyridin]-5-ylmethyl)piperazin-1-yl)methyl)-2-(2,6-dioxopiperidin-3-yl)isoindoline-1,3-dione | 5-((4-([2,4'-bipyridin]-5-ylmethyl)piperazin-1-yl)methyl)-2-(2,6-dioxopiperidin-3-yl)isoindoline-1,3-dione |
| GT-05545 | 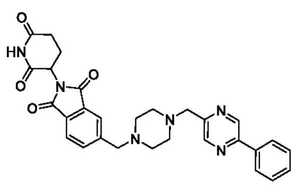 | 2-(2,6-dioxopiperidin-3-yl)-5-((4-((5-phenylpyrazin-2-yl)methyl)piperazin-1-yl)methyl)isoindoline-1,3-dione | 2-(2,6-dioxopiperidin-3-yl)-5-((4-((5-phenylpyrazin-2-yl)methyl)piperazin-1-yl)methyl)isoindoline-1,3-dione |
| GT-05626 | 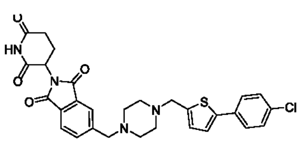 | 5-((4-((5-(4-chlorophenyl)thiophen-2-yl)methyl)piperazin-1-yl)methyl)-2-(2,6-dioxopiperidin-3-yl)isoindoline-1,3-dione | 5-((4-((5-(4-chlorophenyl)thiophen-2-yl)methyl)piperazin-1-yl)methyl)-2-(2,6-dioxopiperidin-3-yl)isoindoline-1,3-dione |

(continued)

| Compound No. | Structure of the compounds | The compounds' name | Chinese translation of the compounds' name |
|---|---|---|---|
| GT-05628 | 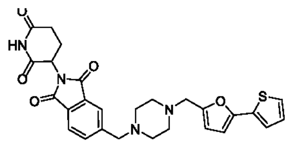 | 2-(2,6-dioxopiperidin-3-yl)-5-((4-((5-(thiophen-2-yl)furan-2-yl)methyl)piperazin-1-yl)methyl)isoindoline-1,3-dione | 2-(2,6-dioxopiperidin-3-yl)-5-((4-((5-(thiophen-2-yl)furan-2-yl)methyl)piperazin-1-yl)methyl)isoindoline-1,3-dione |
| GT-05793 | 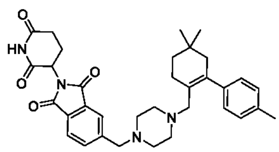 | 2-(2,6-dioxopiperidin-3-yl)-5-((4-((4',5,5-trimethyl-3,4,5,6-tetrahydro-[1,1'-biphenyl]-2-yl)methyl)piperazin-1-yl)methyl)isoindoline-1,3-dione | 2-(2,6-dioxopiperidin-3-yl)-5-((4-((4',5,5-trimethyl-3,4,5,6-tetrahydro-[1,1'-biphenyl]-2-yl)methyl)piperazin-1-yl)methyl)isoindoline-1,3-dione |
| | 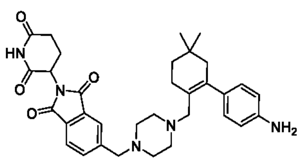 | 5-((4-((4'-amino-5,5-dimethyl-3,4,5,6-tetrahydro-[1,1'-biphenyl]-2-yl)methyl)piperazin-1-yl)methyl)-2-(2,6-dioxopiperidin-3-yl)isoindoline-1,3-dione | 5-((4-((4'-amino-5,5-dimethyl-3,4,5,6-tetrahydro-[1,1'-biphenyl]-2-yl)methyl)piperazin-1-yl)methyl)-2-(2,6-dioxopiperidin-3-yl)isoindoline-1,3-dione |
| GT-05464 | 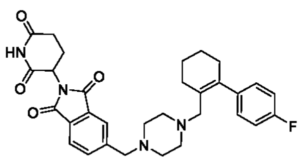 | 2-(2,6-dioxopiperidin-3-yl)-5-((4-((4'-fluoro-3,4,5,6-tetrahydro-[1,1'-biphenyl]-2-yl)methyl)piperazin-1-yl)methyl)isoindoline-1,3-dione | 2-(2,6-dioxopiperidin-3-yl)-5-((4-((4'-fluoro-3,4,5,6-tetrahydro-[1,1'-biphenyl]-2-yl)methyl)piperazin-1-yl)methyl)isoindoline-1,3-dione |
| | 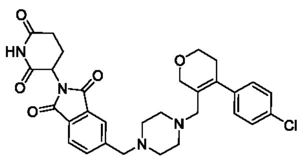 | 5-((4-((4-(4-chlorophenyl)-5,6-dihydro-2H-pyran-3-yl)methyl)piperazin-1-yl)methyl)-2-(2,6-dioxopiperidin-3-yl)isoindoline-1,3-dione | 5-((4-((4-(4-chlorophenyl)-5,6-dihydro-2H-pyran-3-yl)methyl)piperazin-1-yl)methyl)-2-(2,6-dioxopiperidin-3-yl)isoindoline-1,3-dione |
| | 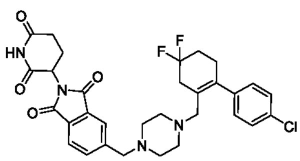 | 5-((4-((4'-chloro-4,4-difluoro-3,4,5,6-tetrahydro-[1,1'-biphenyl]-2-yl)methyl)piperazin-1-yl)methyl)-2-(2,6-dioxopiperidin-3-yl)isoindoline-1,3-dione | 5-((4-((4'-chloro-4,4-difluoro-3,4,5,6-tetrahydro-[1,1'-biphenyl]-2-yl)methyl)piperazin-1-yl)methyl)-2-(2,6-dioxopiperidin-3-yl)isoindoline-1,3-dione |
| | 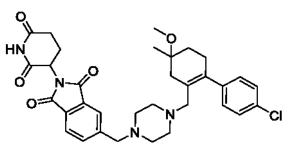 | 5-((4-((4'-chloro-4-methoxy-4-methyl-3,4,5,6-tetrahydro-[1,1'-biphenyl]-2-yl)methyl)piperazin-1-yl)methyl)-2-(2,6-dioxopiperidin-3-yl)isoindoline-1,3-dione | 5-((4-((4'-chloro-4-methoxy-4-methyl-3,4,5,6-tetrahydro-[1,1'-biphenyl]-2-yl)methyl)piperazin-1-yl)methyl)-2-(2,6-dioxopiperidin-3-yl)isoindoline-1,3-dione |
| | 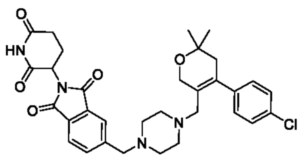 | 5-((4-((4-(4-chlorophenyl)-6,6-dimethyl-5,6-dihydro-2H-pyran-3-yl)methyl)piperazin-1-yl)methyl)-2-(2,6-dioxopiperidin-3-yl)isoindoline-1,3-dione | 5-((4-((4-(4-chlorophenyl)-6,6-dimethyl-5,6-dihydro-2H-pyran-3-yl)methyl)piperazin-1-yl)methyl)-2-(2,6-dioxopiperidin-3-yl)isoindoline-1,3-dione |
| | 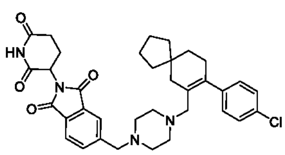 | 5-((4-((8-(4-chlorophenyl)spiro[4.5]dec-7-en-7-yl)methyl)piperazin-1-yl)methyl)-2-(2,6-dioxopiperidin-3-yl)isoindoline-1,3-dione | 5-((4-((8-(4-chlorophenyl)spiro[4.5]dec-7-en-7-yl)methyl)piperazin-1-yl)methyl)-2-(2,6-dioxopiperidin-3-yl)isoindoline-1,3-dione |

(continued)

| Compo und No. | Structure of the compounds | The compounds' name | Chinese translation of the compounds' name |
|---|---|---|---|
| | 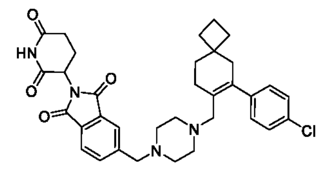 | 5-((4-((6-(4-chlorophenyl)spiro[3.5]non-6-en-7-yl)methyl)piperazin-1-yl)methyl)-2-(2,6-dioxopiperidin-3-yl)isoindoline-1,3-dione | 5-((4-((6-(4-chlorophenyl)spiro[3.5]non-6-en-7-yl)methyl)piperazin-1-yl)methyl)-2-(2,6-dioxopiperidin-3-yl)isoindoline-1,3-dione |
| GT-05366 | 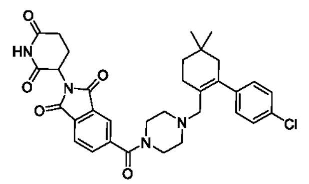 | 5-(4-((4'-chloro-5,5-dimethyl-3,4,5,6-tetrahydro-[1,1'-biphenyl]-2-yl)methyl)piperazine-1-carbonyl)-2-(2,6-dioxopiperidin-3-yl)isoindoline-1,3-dione | 5-(4-((4'-chloro-5,5-dimethyl-3,4,5,6-tetrahydro-[1,1'-biphenyl]-2-yl)methyl)piperazine-1-carbonyl)-2-(2,6-dioxopiperidin-3-yl)isoindoline-1,3-dione |
| | 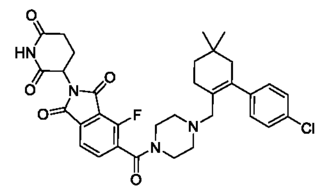 | 5-(4-((4'-chloro-5,5-dimethyl-3,4,5,6-tetrahydro-[1,1'-biphenyl]-2-yl)methyl)piperazine-1-carbonyl)-2-(2,6-dioxopiperidin-3 -yl)-4-fluoroisoindoline-1,3-dione | 5-(4-((4'-chloro-5,5-dimethyl-3,4,5,6-tetrahydro-[1,1'-biphenyl]-2-yl)methyl)piperazine-1-carbonyl)-2-(2,6-dioxopiperidin-3-yl)-4-fluoroisoindoline-1,3-dione |
| | 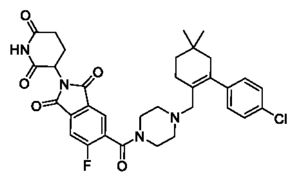 | 5-(4-((4'-chloro-5,5-dimethyl-3,4,5,6-tetrahydro-[1,1'-biphenyl]-2-yl)methyl)piperazine-1-carbonyl)-2-(2,6-dioxopiperidin-3-yl)-6-fluoroisoindoline-1,3-dione | 5-(4-((4'-chloro-5,5-dimethyl-3,4,5,6-tetrahydro-[1,1'-biphenyl]-2-yl)methyl)piperazine-1-carbonyl)-2-(2,6-dioxopiperidin-3-yl)-6-fluoroisoindoline-1,3-dione |
| | 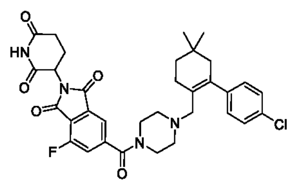 | 6-(4-((4'-chloro-5,5-dimethyl-3,4,5,6-tetrahydro-[1,1'-biphenyl]-2-yl)methyl)piperazine-1-carbonyl)-2-(2,6-dioxopiperidin-3 -yl)-4-fluoroisoindoline-1,3-dione | 6-(4-((4'-chloro-5,5-dimethyl-3,4,5,6-tetrahydro-[1,1'-biphenyl]-2-yl)methyl)piperazine-1-carbonyl)-2-(2,6-dioxopiperidin-3-yl)-4-fluoroisoindoline-1,3-dione |
| | 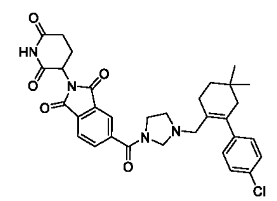 | 5-(3-((4'-chloro-5,5-dimethyl-3,4,5,6-tetrahydro-[1,1'-biphenyl]-2-yl)methyl)imidazolidine-1-carbonyl)-2-(2,6-dioxopiperidin-3-yl)isoindoline-1,3-dione | 5-(3-((4'-chloro-5,5-dimethyl-3,4,5,6-tetrahydro-[1,1'-biphenyl]-2-yl)methyl)imidazolidine-1-carbonyl)-2-(2,6-dioxopiperidin-3-yl)isoindoline-1,3-dione |
| GT-05454 | 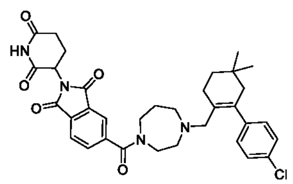 | 5-(4-((4'-chloro-5,5-dimethyl-3,4,5,6-tetrahydro-[1,1'-biphenyl]-2-yl)methyl)-1,4-diazepane-1-carbonyl)-2-(2,6-dioxopiperidin-3-yl)isoindoline-1,3-dione | 5-(4-((4'-chloro-5,5-dimethyl-3,4,5,6-tetrahydro-[1,1'-biphenyl]-2-yl)methyl)-1,4-diazepane-1-carbonyl)-2-(2,6-dioxopiperidin-3-yl)isoindoline-1,3-dione |
| GT-05679 | 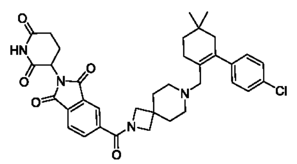 | 5-(7-((4'-chloro-5,5-dimethyl-3,4,5,6-tetrahydro-[1,1'-biphenyl]-2-yl)methyl)-2,7-diazaspiro[3.5]nonane-2-carbonyl)-2-(2,6-dioxopiperidin-3-yl) isoindoline-1,3-dione | 5-(7-((4'-chloro-5,5-dimethyl-3,4,5,6-tetrahydro-[1,1'-biphenyl]-2-yl)methyl)-2,7-diazaspiro[3.5]nonane-2-carbonyl)-2-(2,6-dioxopiperidin-3-yl) isoindoline-1,3-dione |

(continued)

| Compound No. | Structure of the compounds | The compounds' name | Chinese translation of the compounds' name |
|---|---|---|---|
| GT-05676 | 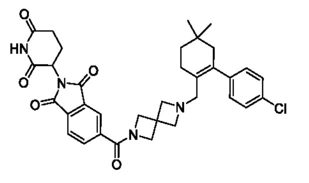 | 5-(6-((4'-chloro-5,5-dimethyl-3,4,5,6-tetrahydro-[1,1'-biphenyl]-2-yl)methyl)-2,6-diazaspiro[3.3]heptane-2-carbonyl)-2-(2,6-dioxopiperidin-3-yl) isoindoline-1,3-dione | 5-(6-((4'-chloro-5,5-dimethyl-3,4,5,6-tetrahydro-[1,1'-biphenyl]-2-yl)methyl)-2,6-diazaspiro[3.3]heptane-2-carbonyl)-2-(2,6-dioxopiperidin-3-yl) isoindoline-1,3-dione |
| GT-05655 | 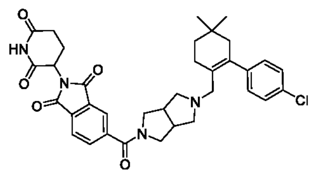 | 5-(5-((4'-chloro-5,5-dimethyl-3,4,5,6-tetrahydro-[1,1'-biphenyl]-2-yl)methyl)octahydropyrrolo[ 3,4-c]pyrrole-2-carbonyl)-2-(2,6-dioxopiperidin-3-yl)isoindoline-1,3-dione | 5-(5-((4'-chloro-5,5-dimethyl-3,4,5,6-tetrahydro-[1,1'-biphenyl]-2-yl)methyl)octahydropyrrolo[3 ,4-c]pyrrole-2-carbonyl)-2-(2,6-dioxopiperidin-3-yl)isoindoline-1,3-dione |
| GT-05369 | 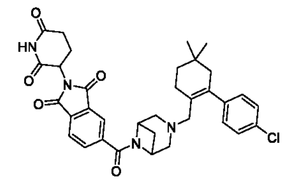 | 5-(3-((4'-chloro-5,5-dimethyl-3,4,5,6-tetrahydro-[1,1'-biphenyl]-2-yl)methyl)-3,6-diazabicyclo[3.1.1]heptane-6-carbonyl)-2-(2,6-dioxopiperidin-3-yl)isoindoline-1,3-dione | 5-(3-((4'-chloro-5,5-dimethyl-3,4,5,6-tetrahydro-[1,1'-biphenyl]-2-yl)methyl)-3,6-diazabicyclo[3.1.1]heptane-6-carbonyl)-2-(2,6-dioxopiperidin-3-yl)isoindoline-1,3-dione |
| GT-05652 | 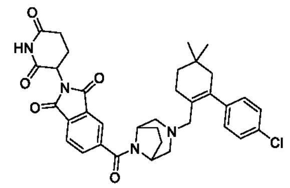 | 5-(3-((4'-chloro-5,5-dimethyl-3,4,5,6-tetrahydro-[1,1'-biphenyl]-2-yl)methyl)-3,8-diazabicyclo[3.2.1]octane-8-carbonyl)-2-(2,6-dioxopiperidin-3-yl) isoindoline-1,3-dione | 5-(3-((4'-chloro-5,5-dimethyl-3,4,5,6-tetrahydro-[1,1'-biphenyl]-2-yl)methyl)-3,8-diazabicyclo[3.2.1]octane-8-carbonyl)-2-(2,6-dioxopiperidin-3-yl) isoindoline-1,3-dione |
| GT-05651 | 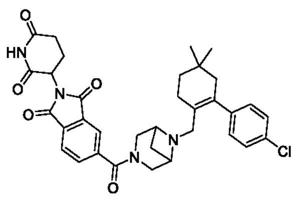 | 5-(6-((4'-chloro-5,5-dimethyl-3,4,5,6-tetrahydro-[1,1'-biphenyl]-2-yl)methyl)-3,6-diazabicyclo[3.1.1]heptane-3-carbonyl)-2-(2,6-dioxopiperidin-3-yl)isoindoline-1,3-dione | 5-(6-((4'-chloro-5,5-dimethyl-3,4,5,6-tetrahydro-[1,1'-biphenyl]-2-yl)methyl)-3,6-diazabicyclo[3.1.1]heptane-3-carbonyl)-2-(2,6-dioxopiperidin-3-yl)isoindoline-1,3-dione |
| GT-05412 | 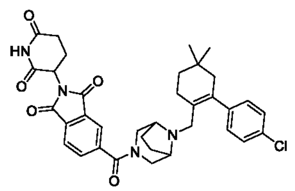 | 5-(8-((4'-chloro-5,5-dimethyl-3,4,5,6-tetrahydro-[1,1'-biphenyl]-2-yl)methyl)-3,8-diazabicyclo[3.2.1]octane-3-carbonyl)-2-(2,6-dioxopiperidin-3-yl) isoindoline-1,3-dione | 5-(8-((4'-chloro-5,5-dimethyl-3,4,5,6-tetrahydro-[1,1'-biphenyl]-2-yl)methyl)-3,8-diazabicyclo[3.2.1]octane-3-carbonyl)-2-(2,6-dioxopiperidin-3-yl) isoindoline-1,3-dione |
| GT-05414 | 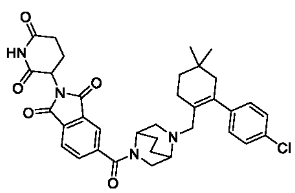 | 5-(5-((4'-chloro-5,5-dimethyl-3,4,5,6-tetrahydro-[1,1'-biphenyl]-2-yl)methyl)-2,5-diazabicyclo[2.2.2]octane-2-carbonyl)-2-(2,6-dioxopiperidin-3-yl) isoindoline-1,3-dione | 5-(5-((4'-chloro-5,5-dimethyl-3,4,5,6-tetrahydro-[1,1'-biphenyl]-2-yl)methyl)-2,5-diazabicyclo[2.2.2]octane-2-carbonyl)-2-(2,6-dioxopiperidin-3-yl) isoindoline-1,3-dione |
| | 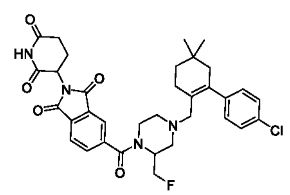 | 5-(4-((4'-chloro-5,5-dimethyl-3,4,5,6-tetrahydro-[1,1'-biphenyl]-2-yl)methyl)-2-(fluoromethyl)piperazine-1-carbonyl)-2-(2,6-dioxopiperidin-3-yl) isoindoline-1,3-dione | 5-(4-((4'-chloro-5,5-dimethyl-3,4,5,6-tetrahydro-[1,1'-biphenyl]-2-yl)methyl)-2-(fluoromethyl)piperazine-1-carbonyl)-2-(2,6-dioxopiperidin-3-yl) isoindoline-1,3-dione |

(continued)

| Compo und No. | Structure of the compounds | The compounds' name | Chinese translation of the compounds' name |
|---|---|---|---|
| GT-05410 | 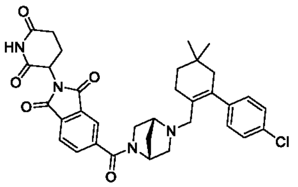 | 5-((1R,4R)-5-((4'-chloro-5,5-dimethyl-3,4,5,6-tetrahydro-[1,1'-biphenyl]-2-yl)methyl)-2,5-diazabicyclo[2.2.1]heptane-2-carbonyl)-2-(2,6-dioxopiperidin-3-yl)isoindoline-1,3-dione | 5-((1R,4R)-5-((4'-chloro-5,5-dimethyl-3,4,5,6-tetrahydro-[1,1'-biphenyl]-2-yl)methyl)-2,5-diazabicyclo[2.2.1]heptane-2-carbonyl)-2-(2,6-dioxopiperidin-3-yl)isoindoline-1,3-dione |
| | 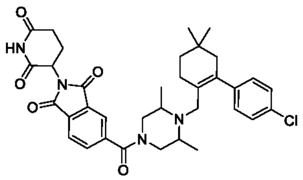 | 5-(4-((4'-chloro-5,5-dimethyl-3,4,5,6-tetrahydro-[1,1'-biphenyl]-2-yl)methyl)-3,5-dimethylpiperazine-1-carbonyl)-2-(2,6-dioxopiperidin-3-yl) isoindoline-1,3-dione | 5-(4-((4'-chloro-5,5-dimethyl-3,4,5,6-tetrahydro-[1,1'-biphenyl]-2-yl)methyl)-3,5-dimethylpiperazine-1-carbonyl)-2-(2,6-dioxopiperidin-3-yl) isoindoline-1,3-dione |
| | 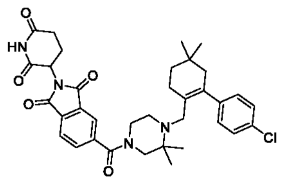 | 5-(4-((4'-chloro-5,5-dimethyl-3,4,5,6-tetrahydro-[1,1'-biphenyl]-2-yl)methyl)-3,3-dimethylpiperazine-1-carbonyl)-2-(2,6-dioxopiperidin-3-yl) isoindoline-1,3-dione | 5-(4-((4'-chloro-5,5-dimethyl-3,4,5,6-tetrahydro-[1,1'-biphenyl]-2-yl)methyl)-3,3-dimethylpiperazine-1-carbonyl)-2-(2,6-dioxopiperidin-3-yl) isoindoline-1,3-dione |
| | 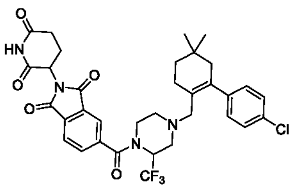 | 5-(4-((4'-chloro-5,5-dimethyl-3,4,5,6-tetrahydro-[1,1'-biphenyl]-2-yl)methyl)-2-(trifluoromethyl)piperazine-1-carbonyl)-2-(2,6-dioxopiperidin-3-yl)isoindoline-1,3-dione | 5-(4-((4'-chloro-5,5-dimethyl-3,4,5,6-tetrahydro-[1,1'-biphenyl]-2-yl)methyl)-2-(trifluoromethyl)piperazine-1-carbonyl)-2-(2,6-dioxopiperidin-3-yl)isoindoline-1,3-dione |
| GT-05615 | 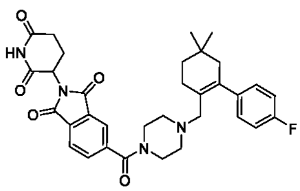 | 2-(2,6-dioxopiperidin-3-yl)-5-(4-((4'-fluoro-5,5-dimethyl-3,4,5,6-tetrahydro-[1,1'-biphenyl]-2-yl)methyl)piperazine-1-carbonyl)isoindoline-1,3-dione | 2-(2,6-dioxopiperidin-3-yl)-5-(4-((4'-fluoro-5,5-dimethyl-3,4,5,6-tetrahydro-[1,1'-biphenyl]-2-yl)methyl)piperazine-1-carbonyl)isoindoline-1,3-dione |
| GT-05456 | 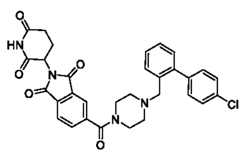 | 5-(4-((4'-chloro-[1,1'-biphenyl]-2-yl)methyl)piperazine-1-carbonyl)-2-(2,6-dioxopiperidin-3-yl)isoindoline-1,3-dione | 5-(4-((4'-chloro-[1,1'-biphenyl]-2-yl)methyl)piperazine-1-carbonyl)-2-(2,6-dioxopiperidin-3-yl)isoindoline-1,3-dione |
| GT-05631 | 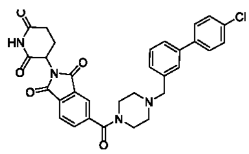 | 5-(4-((4'-chloro-[1,1'-biphenyl]-3-yl)methyl)piperazine-1-carbonyl)-2-(2,6-dioxopiperidin-3-yl)isoindoline-1,3-dione | 5-(4-((4'-chloro-[1,1'-biphenyl]-3-yl)methyl)piperazine-1-carbonyl)-2-(2,6-dioxopiperidin-3-yl)isoindoline-1,3-dione |
| GT-05600 | 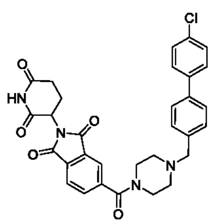 | 5-(4-((4'-chloro-[1,1'-biphenyl]-4-yl)methyl)piperazine-1-carbonyl)-2-(2,6-dioxopiperidin-3-yl)isoindoline-1,3-dione | 5-(4-((4'-chloro-[1,1'-biphenyl]-4-yl)methyl)piperazine-1-carbonyl)-2-(2,6-dioxopiperidin-3-yl)isoindoline-1,3-dione |

(continued)

| Compo und No. | Structure of the compounds | The compounds' name | Chinese translation of the compounds' name |
|---|---|---|---|
| GT-05732 | 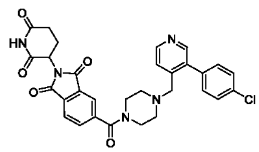 | 5-(4-((3-(4-chlorophenyl)pyridin-4-yl)methyl)piperazine-1-carbonyl)-2-(2,6-dioxopiperidin-3-yl)isoindoline-1,3-dione | 5-(4-((3-(4-chlorophenyl)pyridin-4-yl)methyl)piperazine-1-carbonyl)-2-(2,6-dioxopiperidin-3-yl)isoindoline-1,3-dione |
| GT-05794 | 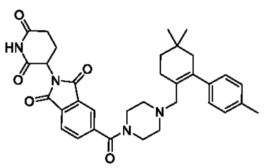 | 2-(2,6-dioxopiperidin-3-yl)-5-(4-((4',5,5-trimethyl-3,4,5,6-tetrahydro-[1,1'-biphenyl]-2-yl)methyl)piperazine-1-carbonyl)isoindoline-1,3-dione | 2-(2,6-dioxopiperidin-3-yl)-5-(4-((4',5,5-trimethyl-3,4,5,6-tetrahydro-[1,1'-biphenyl]-2-yl)methyl)piperazine-1-carbonyl)isoindoline-1,3-dione |
| | 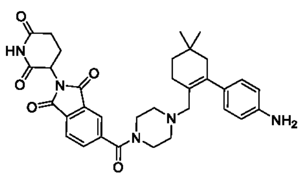 | 5-(4-((4'-amino-5,5-dimethyl-3,4,5,6-tetrahydro-[1,1'-biphenyl]-2-yl)methyl)piperazine-1-carbonyl)-2-(2,6-dioxopiperidin-3-yl)isoindoline-1,3-dione | 5-(4-((4'-amino-5,5-dimethyl-3,4,5,6-tetrahydro-[1,1'-biphenyl]-2-yl)methyl)piperazine-1-carbonyl)-2-(2,6-dioxopiperidin-3-yl)isoindoline-1,3-dione |
| GT-05413 | 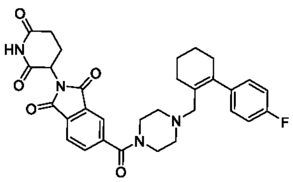 | 2-(2,6-dioxopiperidin-3-yl)-5-(4-((4'-fluoro-3,4,5,6-tetrahydro-[1,1'-biphenyl]-2-yl) methyl)piperazine-1-carbonyl) isoindoline-1,3-dione | 2-(2,6-dioxopiperidin-3-yl)-5-(4-((4'-fluoro-3,4,5,6-tetrahydro-[1,1'-biphenyl]-2-yl) methyl)piperazine-1-carbonyl) isoindoline-1,3-dione |
| | 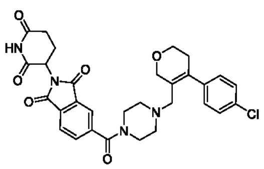 | 5-(4-((4-(4-chlorophenyl)-5,6-dihydro-2H-pyran-3-yl)methyl) piperazine-1-carbonyl)-2-(2,6-dioxopiperidin-3-yl)isoindoline-1,3-dione | 5-(4-((4-(4-chlorophenyl)-5,6-dihydro-2H-pyran-3-yl)methyl) piperazine-1-carbonyl)-2-(2,6-dioxopiperidin-3-yl)isoindoline-1,3-dione |
| | 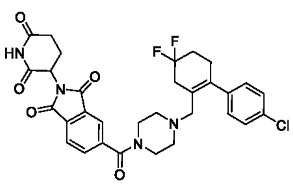 | 5-(4-((4'-chloro-4,4-difluoro-3,4,5,6-tetrahydro-[1,1'-biphenyl]-2-yl)methyl)piperazine-1-carbonyl)-2-(2,6-dioxopiperidin-3-yl)isoindoline-1,3-dione | 5-(4-((4'-chloro-4,4-difluoro-3,4,5,6-tetrahydro-[1,1'-biphenyl]-2-yl)methyl)piperazine-1-carbonyl)-2-(2,6-dioxopiperidin-3-yl)isoindoline-1,3-dione |
| | 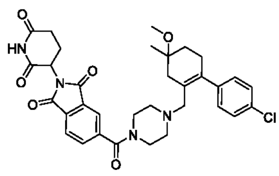 | 5-(4-((4'-chloro-4-methoxy-4-methyl-3,4,5,6-tetrahydro-[1,1'-biphenyl]-2-yl)methyl)piperazine-1-carbonyl)-2-(2,6-dioxopiperidin-3-yl)isoindoline-1,3-dione | 5-(4-((4'-chloro-4-methoxy-4-methyl-3,4,5,6-tetrahydro-[1,1'-biphenyl]-2-yl)methyl)piperazine-1-carbonyl)-2-(2,6-dioxopiperidin-3-yl)isoindoline-1,3-dione |
| GT-05674 | 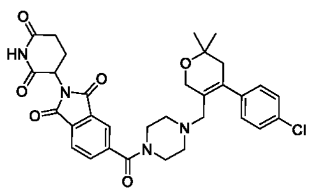 | 5-(4-((4-(4-chlorophenyl)-6,6-dimethyl-5,6-dihydro-2H-pyran-3-yl)methyl)piperazine-1-carbonyl)-2-(2,6-dioxopiperidin-3-yl)isoindoline-1,3-dione | 5-(4-((4-(4-chlorophenyl)-6,6-dimethyl-5,6-dihydro-2H-pyran-3-yl)methyl)piperazine-1-carbonyl)-2-(2,6-dioxopiperidin-3-yl)isoindoline-1,3-dione |

(continued)

| Compound No. | Structure of the compounds | The compounds' name | Chinese translation of the compounds' name |
|---|---|---|---|
| | 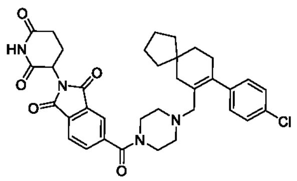 | 5-(4-((8-(4-chlorophenyl)spiro[4.5]dec-7-en-7-yl)methyl)piperazine-1-carbonyl)-2-(2,6-dioxopiperidin-3-yl)isoindoline-1,3-dione | 5-(4-((8-(4-chlorophenyl)spiro[4.5]dec-7-en-7-yl)methyl)piperazine-1-carbonyl)-2-(2,6-dioxopiperidin-3-yl)isoindoline-1,3-dione |
| | 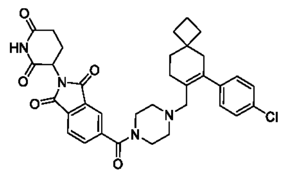 | 5-(4-((6-(4-chlorophenyl)spiro[3.5]non-6-en-7-yl)methyl)piperazine-1-carbonyl)-2-(2,6-dioxopiperidin-3-yl)isoindoline-1,3-dione | 5-(4-((6-(4-chlorophenyl)spiro[3.5]non-6-en-7-yl)methyl)piperazine-1-carbonyl)-2-(2,6-dioxopiperidin-3-yl)isoindoline-1,3-dione |
| GT-05375 | 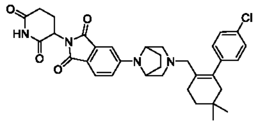 | 5-(3-((4'-chloro-5,5-dimethyl-3,4,5,6-tetrahydro-[1,1'-biphenyl]-2-yl)methyl)-3,8-diazabicyclo[3.2.1]octan-8-yl)-2-(2,6-dioxopiperidin-3-yl) isoindoline-1,3-dione | 5-(3-((4'-chloro-5,5-dimethyl-3,4,5,6-tetrahydro-[1,1'-biphenyl]-2-yl)methyl)-3,8-diazabicyclo[3.2.1]octan-8-yl)-2-(2,6-dioxopiperidin-3-yl) isoindoline-1,3-dione |
| GT-05376 | 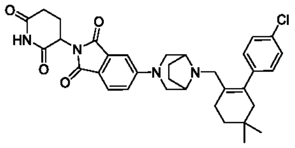 | 5-(8-((4'-chloro-5,5-dimethyl-3,4,5,6-tetrahydro-[1,1'-biphenyl]-2-yl)methyl)-3,8-diazabicyclo[3.2.1]octan-3-yl)-2-(2,6-dioxopiperidin-3-yl) isoindoline-1,3-dione | 5-(8-((4'-chloro-5,5-dimethyl-3,4,5,6-tetrahydro-[1,1'-biphenyl]-2-yl)methyl)-3,8-diazabicyclo[3.2.1]octan-3-yl)-2-(2,6-dioxopiperidin-3-yl) isoindoline-1,3-dione |
| GT-05377 | 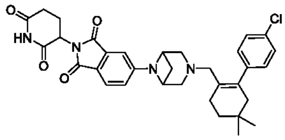 | 5-(3-((4'-chloro-5,5-dimethyl-3,4,5,6-tetrahydro-[1,1'-biphenyl]-2-yl)methyl)-3,6-diazabicyclo[3.1.1]heptan-6-yl)-2-(2,6-dioxopiperidin-3-yl) isoindoline-1,3-dione | 5-(3-((4'-chloro-5,5-dimethyl-3,4,5,6-tetrahydro-[1,1'-biphenyl]-2-yl)methyl)-3,6-diazabicyclo[3.1.1]heptan-6-yl)-2-(2,6-dioxopiperidin-3-yl) isoindoline-1,3-dione |
| GT-05378 | 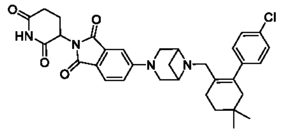 | 5-(6-((4'-chloro-5,5-dimethyl-3,4,5,6-tetrahydro-[1,1'-biphenyl]-2-yl)methyl)-3,6-diazabicyclo[3.1.1]heptan-3-yl)-2-(2,6-dioxopiperidin-3-yl) isoindoline-1,3-dione | 5-(6-((4'-chloro-5,5-dimethyl-3,4,5,6-tetrahydro-[1,1'-biphenyl]-2-yl)methyl)-3,6-diazabicyclo[3.1.1]heptan-3-yl)-2-(2,6-dioxopiperidin-3-yl) isoindoline-1,3-dione |
| GT-05379 | 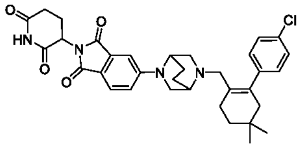 | 5-(5-((4'-chloro-5,5-dimethyl-3,4,5,6-tetrahydro-[1,1'-biphenyl]-2-yl)methyl)-2,5-diazabicyclo[2.2.2]octan-2-yl)-2-(2,6-dioxopiperidin-3-yl) isoindoline-1,3-dione | 5-(5-((4'-chloro-5,5-dimethyl-3,4,5,6-tetrahydro-[1,1'-biphenyl]-2-yl)methyl)-2,5-diazabicyclo[2.2.2]octan-2-yl)-2-(2,6-dioxopiperidin-3-yl) isoindoline-1,3-dione |
| | 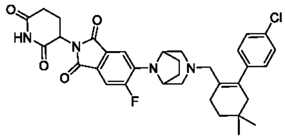 | 5-(3-((4'-chloro-5,5-dimethyl-3,4,5,6-tetrahydro-[1,1'-biphenyl]-2-yl)methyl)-3,8-diazabicyclo[3.2.1]octan-8-yl)-2-(2,6-dioxopiperidin-3-yl)-6-fluoroisoindoline-1,3-dione | 5-(3-((4'-chloro-5,5-dimethyl-3,4,5,6-tetrahydro-[1,1'-biphenyl]-2-yl)methyl)-3,8-diazabicyclo[3.2.1]octan-8-yl)-2-(2,6-dioxopiperidin-3-yl)-6-fluoroisoindoline-1,3-dione |

(continued)

| Compound No. | Structure of the compounds | The compounds' name | Chinese translation of the compounds' name |
|---|---|---|---|
| | 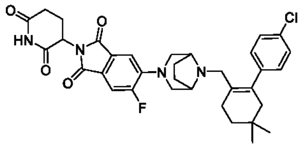 | 5-(8-((4'-chloro-5,5-dimethyl-3,4,5,6-tetrahydro-[1,1'-biphenyl]-2-yl)methyl)-3,8-diazabicyclo[3.2.1]octan-3-yl)-2-(2,6-dioxopiperidin-3-yl)-6-fluoroisoindoline-1,3-dione | 5-(8-((4'-chloro-5,5-dimethyl-3,4,5,6-tetrahydro-[1,1'-biphenyl]-2-yl)methyl)-3,8-diazabicyclo[3.2.1]octan-3-yl)-2-(2,6-dioxopiperidin-3-yl)-6-fluoroisoindoline-1,3-dione |
| | 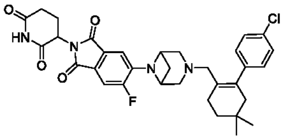 | 5-(3-((4'-chloro-5,5-dimethyl-3,4,5,6-tetrahydro-[1,1'-biphenyl]-2-yl)methyl)-3,6-diazabicyclo[3.1.1]heptan-6-yl)-2-(2,6-dioxopiperidin-3-yl)-6-fluoroisoindoline-1,3-dione | 5-(3-((4'-chloro-5,5-dimethyl-3,4,5,6-tetrahydro-[1,1'-biphenyl]-2-yl)methyl)-3,6-diazabicyclo[3.1.1]heptan-6-yl)-2-(2,6-dioxopiperidin-3-yl)-6-fluoroisoindoline-1,3-dione |
| | 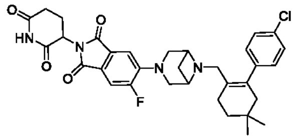 | 5-(6-((4'-chloro-5,5-dimethyl-3,4,5,6-tetrahydro-[1,1'-biphenyl]-2-yl)methyl)-3,6-diazabicyclo[3.1.1]heptan-3-yl)-2-(2,6-dioxopiperidin-3-yl)-6-fluoroisoindoline-1,3-dione | 5-(6-((4'-chloro-5,5-dimethyl-3,4,5,6-tetrahydro-[1,1'-biphenyl]-2-yl)methyl)-3,6-diazabicyclo[3.1.1]heptan-3-yl)-2-(2,6-dioxopiperidin-3-yl)-6-fluoroisoindoline-1,3-dione |
| | 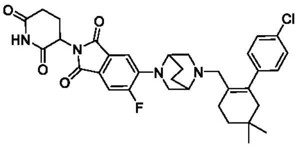 | 5-(5-((4'-chloro-5,5-dimethyl-3,4,5,6-tetrahydro-[1,1'-biphenyl]-2-yl)methyl)-2,5-diazabicyclo[2.2.2]octan-2-yl)-2-(2,6-dioxopiperidin-3-yl)-6-fluoroisoindoline-1,3-dione | 5-(5-((4'-chloro-5,5-dimethyl-3,4,5,6-tetrahydro-[1,1'-biphenyl]-2-yl)methyl)-2,5-diazabicyclo[2.2.2]octan-2-yl)-2-(2,6-dioxopiperidin-3-yl)-6-fluoroisoindoline-1,3-dione |
| GT-05370 | 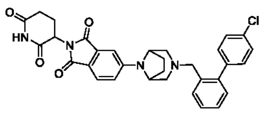 | 5-(3-((4'-chloro-[1,1'-biphenyl]-2-yl)methyl)-3,8-diazabicyclo[3.2.1]octan-8-yl)-2-(2,6-dioxopiperidin-3-yl)isoindoline-1,3-dione | 5-(3-((4'-chloro-[1,1'-biphenyl]-2-yl)methyl)-3,8-diazabicyclo[3.2.1]octan-8-yl)-2-(2,6-dioxopiperidin-3-yl)isoindoline-1,3-dione |
| GT-05371 | 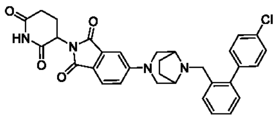 | 5-(8-((4'-chloro-[1,1'-biphenyl]-2-yl)methyl)-3,8-diazabicyclo[3.2.1]octan-3-yl)-2-(2,6-dioxopiperidin-3-yl)isoindoline-1,3-dione | 5-(8-((4'-chloro-[1,1'-biphenyl]-2-yl)methyl)-3,8-diazabicyclo[3.2.1]octan-3-yl)-2-(2,6-dioxopiperidin-3-yl)isoindoline-1,3-dione |
| GT-05372 | 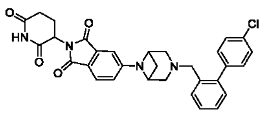 | 5-(3-((4'-chloro-[1,1'-biphenyl]-2-yl)methyl)-3,6-diazabicyclo[3.1.1]heptan-6-yl)-2-(2,6-dioxopiperidin-3-yl)isoindoline-1,3-dione | 5-(3-((4'-chloro-[1,1'-biphenyl]-2-yl)methyl)-3,6-diazabicyclo[3.1.1]heptan-6-yl)-2-(2,6-dioxopiperidin-3-yl)isoindoline-1,3-dione |
| GT-05373 | 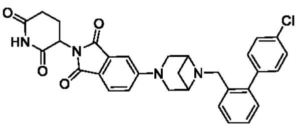 | 5-(6-((4'-chloro-[1,1'-biphenyl]-2-yl)methyl)-3,6-diazabicyclo[3.1.1]heptan-3-yl)-2-(2,6-dioxopiperidin-3-yl)isoindoline-1,3-dione | 5-(6-((4'-chloro-[1,1'-biphenyl]-2-yl)methyl)-3,6-diazabicyclo[3.1.1]heptan-3-yl)-2-(2,6-dioxopiperidin-3-yl)isoindoline-1,3-dione |

(continued)

| Compound No. | Structure of the compounds | The compounds' name | Chinese translation of the compounds' name |
|---|---|---|---|
| GT-05374 | 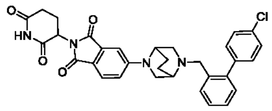 | 5-(5-((4'-chloro-[1,1'-biphenyl]-2-yl)methyl)-2,5-diazabicyclo[2.2.2]octan-2-yl)-2-(2,6-dioxopiperidin-3-yl)isoindoline-1,3-dione | 5-(5-((4'-chloro-[1,1'-biphenyl]-2-yl)methyl)-2,5-diazabicyclo[2.2.2]octan-2-yl)-2-(2,6-dioxopiperidin-3-yl)isoindoline-1,3-dione |
| | 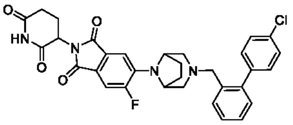 | 5-(3-((4'-chloro-[1,1'-biphenyl]-2-yl)methyl)-3,8-diazabicyclo[3.2.1]octan-8-yl)-2-(2,6-dioxopiperidin-3-yl)-6-fluoroisoindoline-1,3-dione | 5-(3-((4'-chloro-[1,1'-biphenyl]-2-yl)methyl)-3,8-diazabicyclo[3.2.1]octan-8-yl)-2-(2,6-dioxopiperidin-3-yl)-6-fluoroisoindoline-1,3-dione |
| | 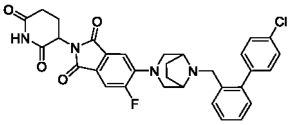 | 5-(8-((4'-chloro-[1,1'-biphenyl]-2-yl)methyl)-3,8-diazabicyclo[3.2.1]octan-3-yl)-2-(2,6-dioxopiperidin-3-yl)-6-fluoroisoindoline-1,3-dione | 5-(8-((4'-chloro-[1,1'-biphenyl]-2-yl)methyl)-3,8-diazabicyclo[3.2.1]octan-3-yl)-2-(2,6-dioxopiperidin-3-yl)-6-fluoroisoindoline-1,3-dione |
| | 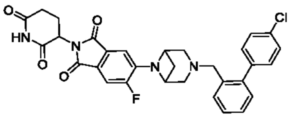 | 5-(3-((4'-chloro-[1,1'-biphenyl]-2-yl)methyl)-3,6-diazabicyclo[3.1.1]heptan-6-yl)-2-(2,6-dioxopiperidin-3-yl)-6-fluoroisoindoline-1,3-dione | 5-(3-((4'-chloro-[1,1'-biphenyl]-2-yl)methyl)-3,6-diazabicyclo[3.1.1]heptan-6-yl)-2-(2,6-dioxopiperidin-3-yl)-6-fluoroisoindoline-1,3-dione |
| | 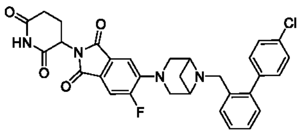 | 5-(6-((4'-chloro-[1,1'-biphenyl]-2-yl)methyl)-3,6-diazabicyclo[3.1.1]heptan-3-yl)-2-(2,6-dioxopiperidin-3-yl)-6-fluoroisoindoline-1,3-dione | 5-(6-((4'-chloro-[1,1'-biphenyl]-2-yl)methyl)-3,6-diazabicyclo[3.1.1]heptan-3-yl)-2-(2,6-dioxopiperidin-3-yl)-6-fluoroisoindoline-1,3-dione |
| | 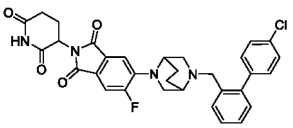 | 5-(5-((4'-chloro-[1,1'-biphenyl]-2-yl)methyl)-2,5-diazabicyclo[2.2.2]octan-2-yl)-2-(2,6-dioxopiperidin-3-yl)-6-fluoroisoindoline-1,3-dione | 5-(5-((4'-chloro-[1,1'-biphenyl]-2-yl)methyl)-2,5-diazabicyclo[2.2.2]octan-2-yl)-2-(2,6-dioxopiperidin-3-yl)-6-fluoroisoindoline-1,3-dione |
| GT-05384 | 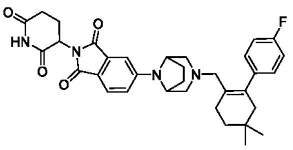 | 2-(2,6-dioxopiperidin-3-yl)-5-(3-((4'-fluoro-5,5-dimethyl-3,4,5,6-tetrahydro-[1,1'-biphenyl]-2-yl)methyl)-3,8-diazabicyclo[3.2.1]octan-8-yl)isoindoline-1,3-dione | 2-(2,6-dioxopiperidin-3-yl)-5-(3-((4'-fluoro-5,5-dimethyl-3,4,5,6-tetrahydro-[1,1'-biphenyl]-2-yl)methyl)-3,8-diazabicyclo[3.2.1]octan-8-yl)isoindoline-1,3-dione |
| GT-05385 | 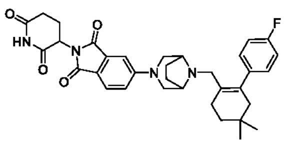 | 2-(2,6-dioxopiperidin-3-yl)-5-(8-((4'-fluoro-5,5-dimethyl-3,4,5,6-tetrahydro-[1,1'-biphenyl]-2-yl)methyl)-3,8-diazabicyclo[3.2.1]octan-3-yl)isoindoline-1,3-dione | 2-(2,6-dioxopiperidin-3-yl)-5-(8-((4'-fluoro-5,5-dimethyl-3,4,5,6-tetrahydro-[1,1'-biphenyl]-2-yl)methyl)-3,8-diazabicyclo[3.2.1]octan-3-yl)isoindoline-1,3-dione |
| GT-05386 | 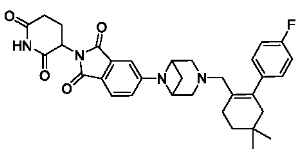 | 2-(2,6-dioxopiperidin-3-yl)-5-(3-((4'-fluoro-5,5-dimethyl-3,4,5,6-tetrahydro-[1,1'-biphenyl]-2-yl)methyl)-3,6-diazabicyclo[3.1.1]heptan-6-yl)isoindoline-1,3-dione | 2-(2,6-dioxopiperidin-3-yl)-5-(3-((4'-fluoro-5,5-dimethyl-3,4,5,6-tetrahydro-[1,1'-biphenyl]-2-yl)methyl)-3,6-diazabicyclo[3.1.1]heptan-6-yl)isoindoline-1,3-dione |

(continued)

| Compound No. | Structure of the compounds | The compounds' name | Chinese translation of the compounds' name |
|---|---|---|---|
| GT-05387 | 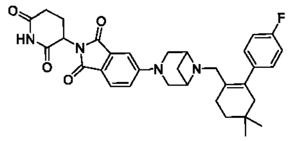 | 2-(2,6-dioxopiperidin-3-yl)-5-(6-((4'-fluoro-5,5-dimethyl-3,4,5,6-tetrahydro-[1,1'-biphenyl]-2-yl)methyl)-3,6-diazabicyclo[3.1.1]heptan-3-yl)isoindoline-1,3-dione | 2-(2,6-dioxopiperidin-3-yl)-5-(6-((4'-fluoro-5,5-dimethyl-3,4,5,6-tetrahydro-[1,1'-biphenyl]-2-yl)methyl)-3,6-diazabicyclo[3.1.1]heptan-3-yl)isoindoline-1,3-dione |
| GT-05388 | 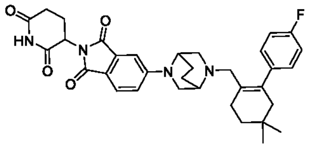 | 2-(2,6-dioxopiperidin-3-yl)-5-(5-((4'-fluoro-5,5-dimethyl-3,4,5,6-tetrahydro-[1,1'-biphenyl]-2-yl)methyl)-2,5-diazabicyclo[2.2.2]octan-2-yl)isoindoline-1,3-dione | 2-(2,6-dioxopiperidin-3-yl)-5-(5-((4'-fluoro-5,5-dimethyl-3,4,5,6-tetrahydro-[1,1'-biphenyl]-2-yl)methyl)-2,5-diazabicyclo[2.2.2]octan-2-yl)isoindoline-1,3-dione |
| | 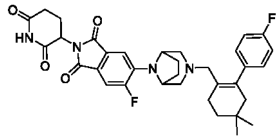 | 2-(2,6-dioxopiperidin-3-yl)-5-fluoro-6-(3-((4'-fluoro-5,5-dimethyl-3,4,5,6-tetrahydro-[1,1'-biphenyl]-2-yl)methyl)-3,8-diazabicyclo[3.2.1]octan-8-yl)isoindoline-1,3-dione | 2-(2,6-dioxopiperidin-3-yl)-5-fluoro-6-(3-((4'-fluoro-5,5-dimethyl-3,4,5,6-tetrahydro-[1,1'-biphenyl]-2-yl)methyl)-3,8-diazabicyclo[3.2.1]octan-8-yl)isoindoline-1,3-dione |
| | 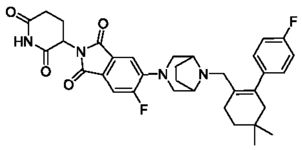 | 2-(2,6-dioxopiperidin-3-yl)-5-fluoro-6-(8-((4'-fluoro-5,5-dimethyl-3,4,5,6-tetrahydro-[1,1'-biphenyl]-2-yl)methyl)-3,8-diazabicyclo[3.2.1]octan-3-yl)isoindoline-1,3-dione | 2-(2,6-dioxopiperidin-3-yl)-5-fluoro-6-(8-((4'-fluoro-5,5-dimethyl-3,4,5,6-tetrahydro-[1,1'-biphenyl]-2-yl)methyl)-3,8-diazabicyclo[3.2.1]octan-3-yl)isoindoline-1,3-dione |
| | 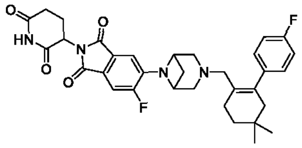 | 2-(2,6-dioxopiperidin-3-yl)-5-fluoro-6-(3-((4'-fluoro-5,5-dimethyl-3,4,5,6-tetrahydro-[1,1'-biphenyl]-2-yl)methyl)-3,6-diazabicyclo[3.1.1]heptan-6-yl)isoindoline-1,3-dione | 2-(2,6-dioxopiperidin-3-yl)-5-fluoro-6-(3 -((4'-fluoro-5,5-dimethyl-3,4,5,6-tetrahydro-[1,1'-biphenyl]-2-yl)methyl)-3,6-diazabicyclo[3.1.1]heptan-6-yl)isoindoline-1,3-dione |
| | 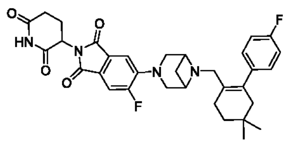 | 2-(2,6-dioxopiperidin-3-yl)-5-fluoro-6-(6-((4'-fluoro-5,5-dimethyl-3,4,5,6-tetrahydro-[1,1'-biphenyl]-2-yl)methyl)-3,6-diazabicyclo[3.1.1]heptan-3-yl)isoindoline-1,3-dione | 2-(2,6-dioxopiperidin-3-yl)-5-fluoro-6-(6-((4'-fluoro-5,5-dimethyl-3,4,5,6-tetrahydro-[1,1'-biphenyl]-2-yl)methyl)-3,6-diazabicyclo[3.1.1]heptan-3-yl)isoindoline-1,3-dione |
| | 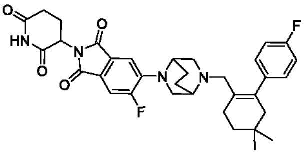 | 2-(2,6-dioxopiperidin-3-yl)-5-fluoro-6-(5-((4'-fluoro-5,5-dimethyl-3,4,5,6-tetrahydro-[1,1'-biphenyl]-2-yl)methyl)-2,5-diazabicyclo[2.2.2]octan-2-yl)isoindoline-1,3-dione | 2-(2,6-dioxopiperidin-3-yl)-5-fluoro-6-(5-((4'-fluoro-5,5-dimethyl-3,4,5,6-tetrahydro-[1,1'-biphenyl]-2-yl)methyl)-2,5-diazabicyclo[2.2.2]octan-2-yl)isoindoline-1,3-dione |
| GT-05459 | 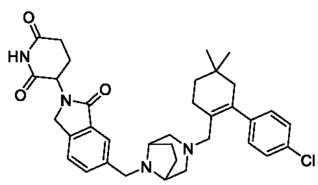 | 3-(6-((3-((4'-chloro-5,5-dimethyl-3,4,5,6-tetrahydro-[1,1'-biphenyl]-2-yl)methyl)-3,8-diazabicyclo[3.2.1]octan-8-yl) methyl)-1-oxoisoindolin-2-yl)piperidine-2,6-dione | 3-(6-((3-((4'-chloro-5,5-dimethyl-3,4,5,6-tetrahydro-[1,1'-biphenyl]-2-yl)methyl)-3,8-diazabicyclo[3.2.1]octan-8-yl) methyl)-1-oxoisoindolin-2-yl)piperidine-2,6-dione |

(continued)

| Compo und No. | Structure of the compounds | The compounds' name | Chinese translation of the compounds' name |
|---|---|---|---|
| GT-05461 | 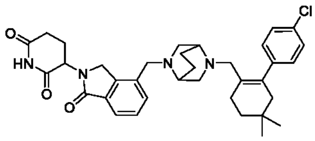 | 3-(4-((5-((4'-chloro-5,5-dimethyl-3,4,5,6-tetrahydro-[1,1'-biphenyl]-2-yl)methyl)-2,5-diazabicyclo[2.2.2]octan-2-yl)methyl)-1-oxoisoindolin-2-yl)piperidine-2,6-dione | 3-(4-((5-((4'-chloro-5,5-dimethyl-3,4,5,6-tetrahydro-[1,1'-biphenyl]-2-yl)methyl)-2,5-diazabicyclo[2.2.2]octan-2-yl)methyl)-1-oxoisoindolin-2-yl)piperidine-2,6-dione |
| GT-05485 | 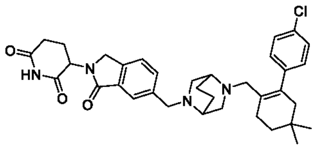 | 3-(6-((5-((4'-chloro-5,5-dimethyl-3,4,5,6-tetrahydro-[1,1'-biphenyl]-2-yl)methyl)-2,5-diazabicyclo[2.2.2]octan-2-yl)methyl)-1-oxoisoindolin-2-yl)piperidine-2,6-dione | 3-(6-((5-((4'-chloro-5,5-dimethyl-3,4,5,6-tetrahydro-[1,1'-biphenyl]-2-yl)methyl)-2,5-diazabicyclo[2.2.2]octan-2-yl)methyl)-1-oxoisoindolin-2-yl)piperidine-2,6-dione |
| GT-05524 | 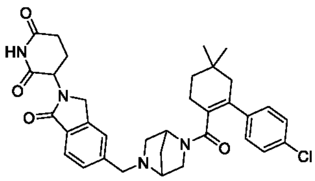 | 3-(5-((5-(4'-chloro-5,5-dimethyl-3,4,5,6-tetrahydro-[1,1'-biphenyl]-2-carbonyl)-2,5-diazabicyclo[2.2.1]heptan-2-yl)methyl)-1-oxoisoindolin-2-yl)piperidine-2,6-dione | 3-(5-((5-(4'-chloro-5,5-dimethyl-3,4,5,6-tetrahydro-[1,1'-biphenyl]-2-carbonyl)-2,5-diazabicyclo[2.2.1]heptan-2-yl)methyl)-1-oxoisoindolin-2-yl)piperidine-2,6-dione |
| GT-05541 | 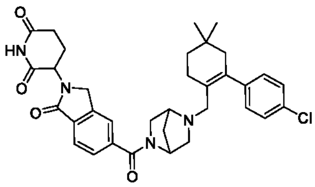 | 3-(5-(5-((4'-chloro-5,5-dimethyl-3,4,5,6-tetrahydro-[1,1'-biphenyl]-2-yl)methyl)-2,5-diazabicyclo[2.2.1]heptane-2-carbonyl)-1-oxoisoindolin-2-yl)piperidine-2,6-dione | 3-(5-(5-((4'-chloro-5,5-dimethyl-3,4,5,6-tetrahydro-[1,1'-biphenyl]-2-yl)methyl)-2,5-diazabicyclo[2.2.1]heptane-2-carbonyl)-1-oxoisoindolin-2-yl)piperidine-2,6-dione |
| GT-05466 | 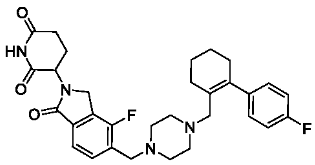 | 3-(4-fluoro-5-((4-((4'-fluoro-3,4,5,6-tetrahydro-[1,1'-biphenyl]-2-yl)methyl)piperazin-1-yl)methyl)-1-oxoisoindolin-2-yl)piperidine-2,6-dione | 3-(4-fluoro-5-((4-((4'-fluoro-3,4,5,6-tetrahydro-[1,1'-biphenyl]-2-yl)methyl)piperazin-1-yl)methyl)-1-oxoisoindolin-2-yl)piperidine-2,6-dione |
| GT-05861 | 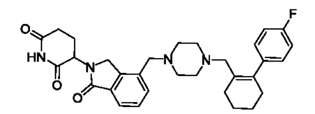 | 3-(4-((4-((4'-fluoro-3,4,5,6-tetrahydro-[1,1'-biphenyl]-2-yl)methyl)piperazin-1-yl)methyl)-1-oxoisoindolin-2-yl)piperidine-2,6-dione | 3-(4-((4-((4'-fluoro-3,4,5,6-tetrahydro-[1,1'-biphenyl]-2-yl)methyl)piperazin-1-yl)methyl)-1-oxoisoindolin-2-yl)piperidine-2,6-dione |
| GT-05465 | 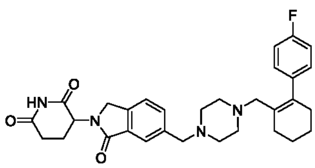 | 3-(6-((4-((4'-fluoro-3,4,5,6-tetrahydro-[1,1'-biphenyl]-2-yl)methyl)piperazin-1-yl)methyl)-1-oxoisoindolin-2-yl)piperidine-2,6-dione | 3-(6-((4-((4'-fluoro-3,4,5,6-tetrahydro-[1,1'-biphenyl]-2-yl)methyl)piperazin-1-yl)methyl)-1-oxoisoindolin-2-yl)piperidine-2,6-dione |
| GT-05472 | 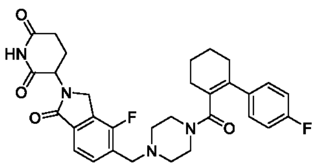 | 3-(4-fluoro-5-((4-(4'-fluoro-3,4,5,6-tetrahydro-[1,1'-biphenyl]-2-carbonyl)piperazin-1-yl)methyl)-1-oxoisoindolin-2-yl)piperidine-2,6-dione | 3-(4-fluoro-5-((4-(4'-fluoro-3,4,5,6-tetrahydro-[1,1'-biphenyl]-2-carbonyl)piperazin-1-yl)methyl)-1-oxoisoindolin-2-yl)piperidine-2,6-dione |

(continued)

| Compo und No. | Structure of the compounds | The compounds' name | Chinese translation of the compounds' name |
|---|---|---|---|
| GT-05473 | 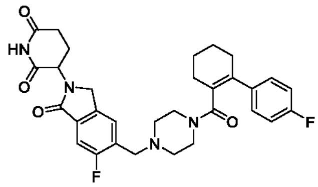 | 3-(6-fluoro-5-((4-(4'-fluoro-3,4,5,6-tetrahydro-[1,1'-biphenyl]-2-carbonyl)piperazin-1-yl)methyl)-1-oxoisoindolin-2-yl)piperidine-2,6-dione | 3-(6-fluoro-5-((4-(4'-fluoro-3,4,5,6-tetrahydro-[1,1'-biphenyl]-2-carbonyl)piperazin-1-yl)methyl)-1-oxoisoindolin-2-yl)piperidine-2,6-dione |
| GT-05474 | 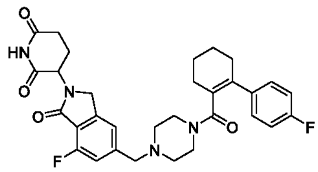 | 3-(7-fluoro-5-((4-(4'-fluoro-3,4,5,6-tetrahydro-[1,1'-biphenyl]-2-carbonyl)piperazin-1-yl)methyl)-1-oxoisoindolin-2-yl)piperidine-2,6-dione | 3-(7-fluoro-5-((4-(4'-fluoro-3,4,5,6-tetrahydro-[1,1'-biphenyl]-2-carbonyl)piperazin-1-yl)methyl)-1-oxoisoindolin-2-yl)piperidine-2,6-dione |
| GT-05471 | 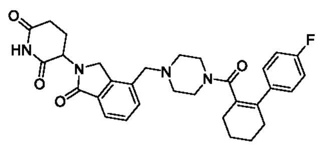 | 3-(4-((4-(4'-fluoro-3,4,5,6-tetrahydro-[1,1'-biphenyl]-2-carbonyl)piperazin-1-yl)methyl)-1-oxoisoindolin-2-yl)piperidine-2,6-dione | 3-(4-((4-(4'-fluoro-3,4,5,6-tetrahydro-[1,1'-biphenyl]-2-carbonyl)piperazin-1-yl)methyl)-1-oxoisoindolin-2-yl)piperidine-2,6-dione |
| GT-05470 | 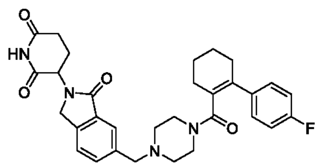 | 3-(6-((4-(4'-fluoro-3,4,5,6-tetrahydro-[1,1'-biphenyl]-2-carbonyl)piperazin-1-yl)methyl)-1-oxoisoindolin-2-yl)piperidine-2,6-dione | 3-(6-((4-(4'-fluoro-3,4,5,6-tetrahydro-[1,1'-biphenyl]-2-carbonyl)piperazin-1-yl)methyl)-1-oxoisoindolin-2-yl)piperidine-2,6-dione |
| GT-05475 | 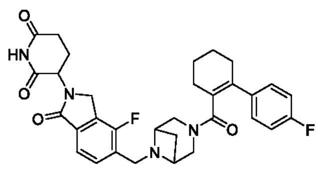 | 3-(4-fluoro-5-((3-(4'-fluoro-3,4,5,6-tetrahydro-[1,1'-biphenyl]-2-carbonyl)-3,6-diazabicyclo[3.1.1]heptan-6-yl)methyl)-1-oxoisoindolin-2-yl)piperidine-2,6-dione | 3-(4-fluoro-5-((3-(4'-fluoro-3,4,5,6-tetrahydro-[1,1'-biphenyl]-2-carbonyl)-3,6-diazabicyclo[3.1.1]heptan-6-yl)methyl)-1-oxoisoindolin-2-yl)piperidine-2,6-dione |
| GT-05477 | 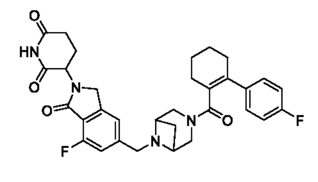 | 3-(7-fluoro-5-((3-(4'-fluoro-3,4,5,6-tetrahydro-[1,1'-biphenyl]-2-carbonyl)-3,6-diazabicyclo[3.1.1]heptan-6-yl)methyl)-1-oxoisoindolin-2-yl)piperidine-2,6-dione | 3-(7-fluoro-5-((3-(4'-fluoro-3,4,5,6-tetrahydro-[1,1'-biphenyl]-2-carbonyl)-3,6-diazabicyclo[3.1.1]heptan-6-yl)methyl)-1-oxoisoindolin-2-yl)piperidine-2,6-dione |
| GT-05478 | 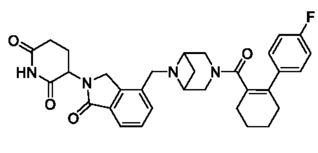 | 3-(4-((3-(4'-fluoro-3,4,5,6-tetrahydro-[1,1'-biphenyl]-2-carbonyl)-3,6-diazabicyclo[3.1.1]heptan-6-yl)methyl)-1-oxoisoindolin-2-yl)piperidine-2,6-dione | 3-(4-((3-(4'-fluoro-3,4,5,6-tetrahydro-[1,1'-biphenyl]-2-carbonyl)-3,6-diazabicyclo[3.1.1]heptan-6-yl)methyl)-1-oxoisoindolin-2-yl)piperidine-2,6-dione |
| GT-05479 | 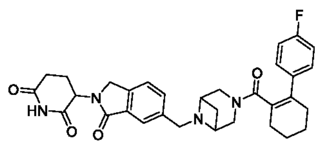 | 3-(6-((3-(4'-fluoro-3,4,5,6-tetrahydro-[1,1'-biphenyl]-2-carbonyl)-3,6-diazabicyclo[3.1.1]heptan-6-yl)methyl)-1-oxoisoindolin-2-yl)piperidine-2,6-dione | 3-(6-((3-(4'-fluoro-3,4,5,6-tetrahydro-[1,1'-biphenyl]-2-carbonyl)-3,6-diazabicyclo[3.1.1]heptan-6-yl)methyl)-1-oxoisoindolin-2-yl)piperidine-2,6-dione |
| GT-05480 | 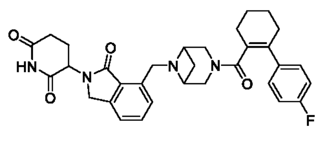 | 3-(7-((3-(4'-fluoro-3,4,5,6-tetrahydro-[1,1'-biphenyl]-2-carbonyl)-3,6-diazabicyclo[3.1.1]heptan-6-yl)methyl)-1-oxoisoindolin-2-yl)piperidine-2,6-dione | 3-(7-((3-(4'-fluoro-3,4,5,6-tetrahydro-[1,1'-biphenyl]-2-carbonyl)-3,6-diazabicyclo[3.1.1]heptan-6-yl)methyl)-1-oxoisoindolin-2-yl)piperidine-2,6-dione |

(continued)

| Compo und No. | Structure of the compounds | The compounds' name | Chinese translation of the compounds' name |
|---|---|---|---|
| GT-04947 | 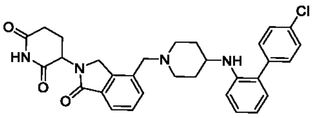 | 3-(4-((4-((4'-chloro-[1,1'-biphenyl]-2-yl)amino)piperidin-1-yl) methyl)-1-oxoisoindolin-2-yl)piperidine-2,6-dione | 3-(4-((4-((4'-chloro-[1,1'-biphenyl]-2-yl)amino)piperidin-1-yl) methyl)-1-oxoisoindolin-2-yl)piperidine-2,6-dione |
| GT-05707 | 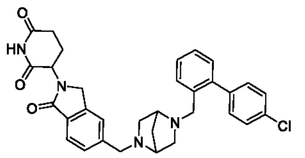 | 3-(5-((5-((4'-chloro-[1,1'-biphenyl]-2-yl)methyl)-2,5-diazabicyclo[2.2.1]heptan-2-yl)methyl)-1-oxoisoindolin-2-yl)piperidine-2,6-dione | 3-(5-((5-((4'-chloro-[1,1'-biphenyl]-2-yl)methyl)-2,5-diazabicyclo[2.2.1]heptan-2-yl)methyl)-1-oxoisoindolin-2-yl)piperidine-2,6-dione |
| GT-05776 | 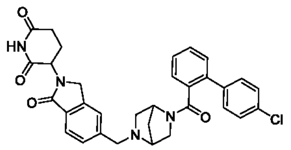 | 3-(5-((5-(4'-chloro-[1,1'-biphenyl]-2-carbonyl)-2,5-diazabicyclo[2.2.1]heptan-2-yl)methyl)-1-oxoisoindolin-2-yl)piperidine-2,6-dione | 3-(5-((5-(4'-chloro-[1,1'-biphenyl]-2-carbonyl)-2,5-diazabicyclo[2.2.1]heptan-2-yl)methyl)-1-oxoisoindolin-2-yl)piperidine-2,6-dione |
| GT-05639 | 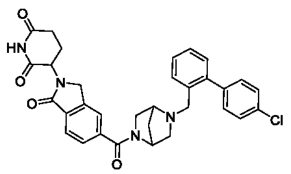 | 3-(5-(5-((4'-chloro-[1,1'-biphenyl]-2-yl)methyl)-2,5-diazabicyclo[2.2.1]heptane-2-carbonyl)-1-oxoisoindolin-2-yl)piperidine-2,6-dione | 3-(5-(5-((4'-chloro-[1,1'-biphenyl]-2-yl)methyl)-2,5-diazabicyclo[2.2.1]heptane-2-carbonyl)-1-oxoisoindolin-2-yl)piperidine-2,6-dione |
| GT-05401 | 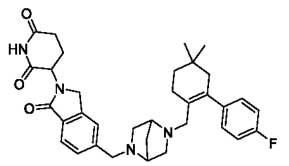 | 3-(5-((5-((4'-fluoro-5,5-dimethyl-3,4,5,6-tetrahydro-[1,1'-biphenyl]-2-yl)methyl)-2,5-diazabicyclo[2.2.1]heptan-2-yl) methyl)-1-oxoisoindolin-2-yl)piperidine-2,6-dione | 3-(5-((5-((4'-fluoro-5,5-dimethyl-3,4,5,6-tetrahydro-[1,1'-biphenyl]-2-yl)methyl)-2,5-diazabicyclo[2.2.1]heptan-2-yl) methyl)-1-oxoisoindolin-2-yl)piperidine-2,6-dione |
| GT-05403 | 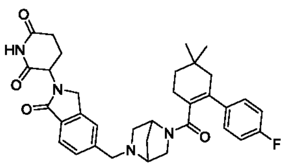 | 3-(5-((5-(4'-fluoro-5,5-dimethyl-3,4,5,6-tetrahydro-[1,1'-biphenyl]-2-carbonyl)-2,5-diazabicyclo[2.2.1]heptan-2-yl) methyl)-1-oxoisoindolin-2-yl)piperidine-2,6-dione | 3-(5-((5-(4'-fluoro-5,5-dimethyl-3,4,5,6-tetrahydro-[1,1'-biphenyl]-2-carbonyl)-2,5-diazabicyclo[2.2.1]heptan-2-yl) methyl)-1-oxoisoindolin-2-yl)piperidine-2,6-dione |
| GT-05452 | 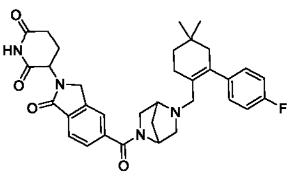 | 3-(5-(5-((4'-fluoro-5,5-dimethyl-3,4,5,6-tetrahydro-[1,1'-biphenyl]-2-yl)methyl)-2,5-diazabicyclo[2.2.1]heptane-2-carbonyl)-1-oxoisoindolin-2-yl)piperidine-2,6-dione | 3-(5-(5-((4'-fluoro-5,5-dimethyl-3,4,5,6-tetrahydro-[1,1'-biphenyl]-2-yl)methyl)-2,5-diazabicyclo[2.2.1]heptane-2-carbonyl)-1-oxoisoindolin-2-yl)piperidine-2,6-dione |
| GT-05448 | 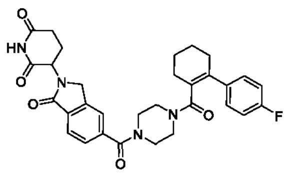 | 3-(5-(4-(4'-fluoro-3,4,5,6-tetrahydro-[1,1'-biphenyl]-2-carbonyl)piperazine-1-carbonyl)-1-oxoisoindolin-2-yl)piperidine-2,6-dione | 3-(5-(4-(4'-fluoro-3,4,5,6-tetrahydro-[1,1'-biphenyl]-2-carbonyl)piperazine-1-carbonyl)-1-oxoisoindolin-2-yl)piperidine-2,6-dione |
| GT-05449 | 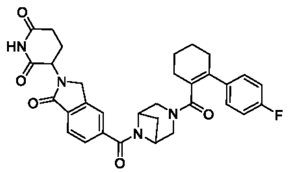 | 3-(5-(3-(4'-fluoro-3,4,5,6-tetrahydro-[1,1'-biphenyl]-2-carbonyl)-3,6-diazabicyclo[3.1.1]heptane-6-carbonyl)-1-oxoisoindolin-2-yl)piperidine-2,6-dione | 3-(5-(3-(4'-fluoro-3,4,5,6-tetrahydro-[1,1'-biphenyl]-2-carbonyl)-3,6-diazabicyclo[3.1.1]heptane-6-carbonyl)-1-oxoisoindolin-2-yl)piperidine-2,6-dione |

(continued)

| Compound No. | Structure of the compounds | The compounds' name | Chinese translation of the compounds' name |
|---|---|---|---|
| GT-05862 | 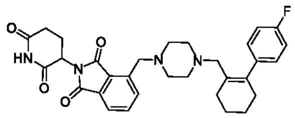 | 2-(2,6-dioxopiperidin-3-yl)-4-((4-((4'-fluoro-3,4,5,6-tetrahydro-[1,1'-biphenyl]-2-yl)methyl)piperazin-1-yl)methyl)isoindoline-1,3-dione | 2-(2,6-dioxopiperidin-3-yl)-4-((4-((4'-fluoro-3,4,5,6-tetrahydro-[1,1'-biphenyl]-2-yl)methyl)piperazin-1-yl)methyl)isoindoline-1,3-dione |
| GT-05533 | 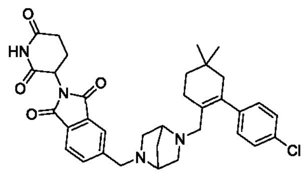 | 5-((5-((4'-chloro-5,5-dimethyl-3,4,5,6-tetrahydro-[1,1'-biphenyl]-2-yl)methyl)-2,5-diazabicyclo[2.2.1]heptan-2-yl)methyl)-2-(2,6-dioxopiperidin-3-yl)isoindoline-1,3-dione | 5-((5-((4'-chloro-5,5-dimethyl-3,4,5,6-tetrahydro-[1,1'-biphenyl]-2-yl)methyl)-2,5-diazabicyclo[2.2.1]heptan-2-yl)methyl)-2-(2,6-dioxopiperidin-3-yl)isoindoline-1,3-dione |
| GT-05468 | 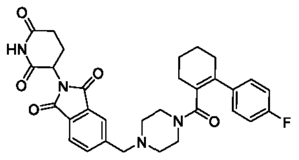 | 2-(2,6-dioxopiperidin-3-yl)-5-((4-(4'-fluoro-3,4,5,6-tetrahydro-[1,1'-biphenyl]-2-carbonyl)piperazin-1-yl)methyl)isoindoline-1,3-dione | 2-(2,6-dioxopiperidin-3-yl)-5-((4-(4'-fluoro-3,4,5,6-tetrahydro-[1,1'-biphenyl]-2-carbonyl)piperazin-1-yl)methyl)isoindoline-1,3-dione |
| GT-05481 | 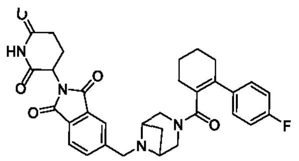 | 2-(2,6-dioxopiperidin-3-yl)-5-((3-(4'-fluoro-3,4,5,6-tetrahydro-[1,1'-biphenyl]-2-carbonyl)-3,6-diazabicyclo[3.1.1]heptan-6-yl)methyl)isoindoline-1,3-dione | 2-(2,6-dioxopiperidin-3-yl)-5-((3-(4'-fluoro-3,4,5,6-tetrahydro-[1,1'-biphenyl]-2-carbonyl)-3,6-diazabicyclo[3.1.1]heptan-6-yl)methyl)isoindoline-1,3-dione |
| GT-05647 | 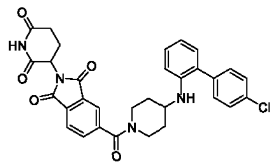 | 5-(4-((4'-chloro-[1,1'-biphenyl]-2-yl)amino)piperidine-1-carbonyl)-2-(2,6-dioxopiperidin-3-yl)isoindoline-1,3-dione | 5-(4-((4'-chloro-[1,1'-biphenyl]-2-yl)amino)piperidine-1-carbonyl)-2-(2,6-dioxopiperidin-3-yl)isoindoline-1,3-dione |
| GT-05415 | 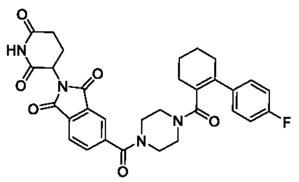 | 2-(2,6-dioxopiperidin-3-yl)-5-(4-(4'-fluoro-3,4,5,6-tetrahydro-[1,1'-biphenyl]-2-carbonyl)piperazine-1-carbonyl)isoindoline-1,3-dione | 2-(2,6-dioxopiperidin-3-yl)-5-(4-(4'-fluoro-3,4,5,6-tetrahydro-[1,1'-biphenyl]-2-carbonyl)piperazine-1-carbonyl)isoindoline-1,3-dione |
| GT-05416 | 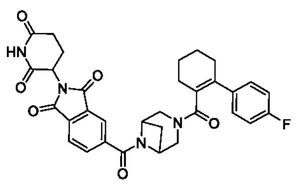 | 2-(2,6-dioxopiperidin-3-yl)-5-(3-(4'-fluoro-3,4,5,6-tetrahydro-[1,1'-biphenyl]-2-carbonyl)-3,6-diazabicyclo[3.1.1]heptane-6-carbonyl)isoindoline-1,3-dione | 2-(2,6-dioxopiperidin-3-yl)-5-(3-(4'-fluoro-3,4,5,6-tetrahydro-[1,1'-biphenyl]-2-carbonyl)-3,6-diazabicyclo[3.1.1]heptane-6-carbonyl)isoindoline-1,3-dione |
| GT-03997 | 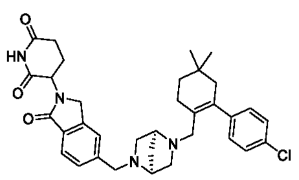 | 3-(5-(((1S,4S)-5-((4'-chloro-5,5-dimethyl-3,4,5,6-tetrahydro-[1,1'-biphenyl]-2-yl)methyl)-2,5-diazabicyclo[2.2.1]heptan-2-yl)methyl)-1-oxoisoindolin-2-yl)piperidine-2,6-dione | 3-(5-(((1S,4S)-5-((4'-chloro-5,5-dimethyl-3,4,5,6-tetrahydro-[1,1'-biphenyl]-2-yl)methyl)-2,5-diazabicyclo[2.2.1]heptan-2-yl)methyl)-1-oxoisoindolin-2-yl)piperidine-2,6-dione |

(continued)

| Compound No. | Structure of the compounds | The compounds' name | Chinese translation of the compounds' name |
|---|---|---|---|
| | 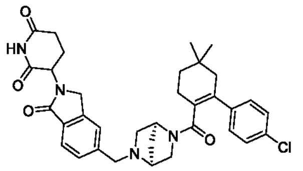 | 3-(5-(((1S,4S)-5-(4'-chloro-5,5-dimethyl-3,4,5,6-tetrahydro-[1,1'-biphenyl]-2-carbonyl)-2,5-diazabicyclo[2.2.1]heptan-2-yl)methyl)-1-oxoisoindolin-2-yl)piperidine-2,6-dione | 3-(5-(((1S,4S)-5-(4'-chloro-5,5-dimethyl-3,4,5,6-tetrahydro-[1,1'-biphenyl]-2-carbonyl)-2,5-diazabicyclo[2.2.1]heptan-2-yl)methyl)-1-oxoisoindolin-2-yl)piperidine-2,6-dione |
| GT-05442 | 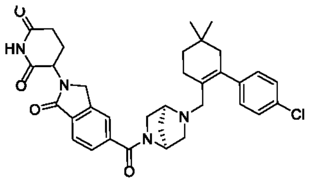 | 3-(5-((1S,4S)-5-((4'-chloro-5,5-dimethyl-3,4,5,6-tetrahydro-[1,1'-biphenyl]-2-yl) methyl)-2,5-diazabicyclo[2.2.1] heptane-2-carbonyl)-1-oxoisoindolin-2-yl)piperidine-2,6-dione | 3-(5-((1S,4S)-5-((4'-chloro-5,5-dimethyl-3,4,5,6-tetrahydro-[1,1'-biphenyl]-2-yl) methyl)-2,5-diazabicyclo[2.2.1] heptane-2-carbonyl)-1-oxoisoindolin-2-yl)piperidine-2,6-dione |
| GT-05657 | 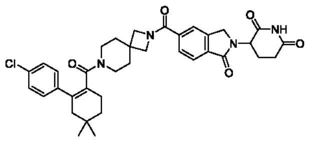 | 3-(5-(7-(4'-chloro-5,5-dimethyl-3,4,5,6-tetrahydro-[1,1'-biphenyl]-2-carbonyl)-2,7-diazaspiro[3.5]nonane-2-carbonyl)-1-oxoisoindolin-2-yl)piperidine-2,6-dione | 3-(5-(7-(4'-chloro-5,5-dimethyl-3,4,5,6-tetrahydro-[1,1'-biphenyl]-2-carbonyl)-2,7-diazaspiro[3.5]nonane-2-carbonyl)-1-oxoisoindolin-2-yl)piperidine-2,6-dione |
| GT-05682 | 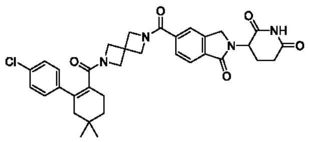 | 3-(5-(6-(4'-chloro-5,5-dimethyl-3,4,5,6-tetrahydro-[1,1'-biphenyl]-2-carbonyl)-2,6-diazaspiro[3.3]heptane-2-carbonyl)-1-oxoisoindolin-2-yl)piperidine-2,6-dione | 3-(5-(6-(4'-chloro-5,5-dimethyl-3,4,5,6-tetrahydro-[1,1'-biphenyl]-2-carbonyl)-2,6-diazaspiro[3.3]heptane-2-carbonyl)-1-oxoisoindolin-2-yl)piperidine-2,6-dione |
| GT-05659 | 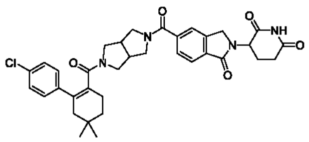 | 3-(5-(5-(4'-chloro-5,5-dimethyl-3,4,5,6-tetrahydro-[1,1'-biphenyl]-2-carbonyl)octahydropyrrolo[ 3,4-c]pyrrole-2-carbonyl)-1-oxoisoindolin-2-yl)piperidine-2,6-dione | 3-(5-(5-(4'-chloro-5,5-dimethyl-3,4,5,6-tetrahydro-[1,1'-biphenyl]-2-carbonyl)octahydropyrrolo[3, 4-c]pyrrole-2-carbonyl)-1-oxoisoindolin-2-yl)piperidine-2,6-dione |
| | 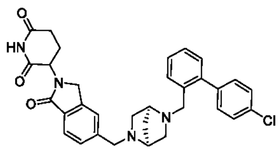 | 3-(5-(((1S,4S)-5-((4'-chloro-[1,1'-biphenyl]-2-yl) methyl)-2,5-diazabicyclo[2.2.1] heptan-2-yl)methyl)-1-oxoisoindolin-2-yl)piperidine-2,6-dione | 3-(5-(((1S,4S)-5-((4'-chloro-[1,1'-biphenyl]-2-yl) methyl)-2,5-diazabicyclo[2.2.1] heptan-2-yl)methyl)-1-oxoisoindolin-2-yl)piperidine-2,6-dione |
| | 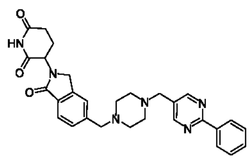 | 3-(1-oxo-5-((4-((2-phenylpyrimidin-5-yl)methyl)piperazin-1-yl)methyl)isoindolin-2-yl)piperidine-2,6-dione | 3-(1-oxo-5-((4-((2-phenylpyrimidin-5-yl)methyl)piperazin-1-yl) methyl)isoindolin-2-yl)piperidine-2,6-dione |
| | 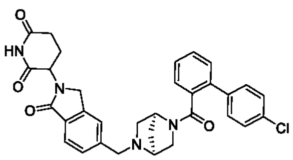 | 3-(5-(((1S,4S)-5-(4'-chloro-[1,1'-biphenyl]-2-carbonyl)-2,5-diazabicyclo[2.2.1]heptan-2-yl)methyl)-1-oxoisoindolin-2-yl)piperidine-2,6-dione | 3-(5-(((1S,4S)-5-(4'-chloro-[1,1'-biphenyl]-2-carbonyl)-2,5-diazabicyclo[2.2.1]heptan-2-yl)methyl)-1-oxoisoindolin-2-yl)piperidine-2,6-dione |

(continued)

| Compound No. | Structure of the compounds | The compounds' name | Chinese translation of the compounds' name |
|---|---|---|---|
| | 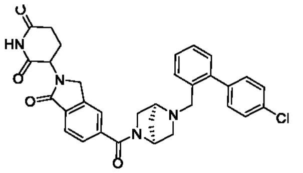 | 3-(5-((1S,4S)-5-((4'-chloro-[1,1'-biphenyl]-2-yl)methyl)-2,5-diazabicyclo[2.2.1]heptane-2-carbonyl)-1-oxoisoindolin-2-yl)piperidine-2,6-dione | 3-(5-((1S,4S)-5-((4'-chloro-[1,1'-biphenyl]-2-yl)methyl)-2,5-diazabicyclo[2.2.1]heptane-2-carbonyl)-1-oxoisoindolin-2-yl)piperidine-2,6-dione |
| | 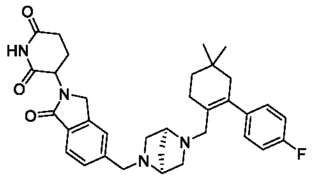 | 3-(5-(((1S,4S)-5-((4'-fluoro-5,5-dimethyl-3,4,5,6-tetrahydro-[1,1'-biphenyl]-2-yl)methyl)-2,5-diazabicyclo[2.2.1]heptan-2-yl)methyl)-1-oxoisoindolin-2-yl)piperidine-2,6-dione | 3-(5-(((1S,4S)-5-((4'-fluoro-5,5-dimethyl-3,4,5,6-tetrahydro-[1,1'-biphenyl]-2-yl)methyl)-2,5-diazabicyclo[2.2.1]heptan-2-yl)methyl)-1-oxoisoindolin-2-yl)piperidine-2,6-dione |
| | 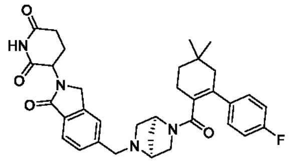 | 3-(5-(((1S,4S)-5-(4'-fluoro-5,5-dimethyl-3,4,5,6-tetrahydro-[1,1'-biphenyl]-2-carbonyl)-2,5-diazabicyclo[2.2.1]heptan-2-yl)methyl)-1-oxoisoindolin-2-yl)piperidine-2,6-dione | 3-(5-(((1S,4S)-5-(4'-fluoro-5,5-dimethyl-3,4,5,6-tetrahydro-[1,1'-biphenyl]-2-carbonyl)-2,5-diazabicyclo[2.2.1]heptan-2-yl)methyl)-1-oxoisoindolin-2-yl)piperidine-2,6-dione |
| | 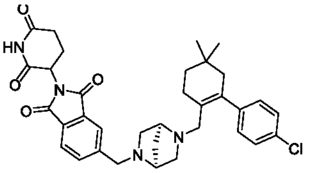 | 5-(((1S,4S)-5-((4'-chloro-5,5-dimethyl-3,4,5,6-tetrahydro-[1,1'-biphenyl]-2-yl)methyl)-2,5-diazabicyclo[2.2.1]heptan-2-yl)methyl)-2-(2,6-dioxopiperidin-3-yl)isoindoline-1,3-dione | 5-(((1S,4S)-5-((4'-chloro-5,5-dimethyl-3,4,5,6-tetrahydro-[1,1'-biphenyl]-2-yl)methyl)-2,5-diazabicyclo[2.2.1]heptan-2-yl)methyl)-2-(2,6-dioxopiperidin-3-yl)isoindoline-1,3-dione |
| GT-05411 | 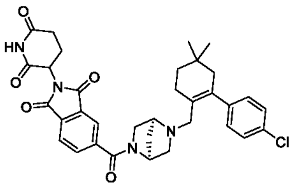 | 5-((1S,4S)-5-((4'-chloro-5,5-dimethyl-3,4,5,6-tetrahydro-[1,1'-biphenyl]-2-yl)methyl)-2,5-diazabicyclo[2.2.1]heptane-2-carbonyl)-2-(2,6-dioxopiperidin-3-yl)isoindoline-1,3-dione | 5-((1S,4S)-5-((4'-chloro-5,5-dimethyl-3,4,5,6-tetrahydro-[1,1'-biphenyl]-2-yl)methyl)-2,5-diazabicyclo[2.2.1]heptane-2-carbonyl)-2-(2,6-dioxopiperidin-3-yl)isoindoline-1,3-dione |
| GT-05476 | 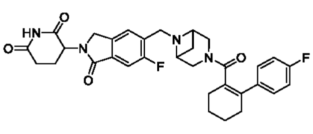 | 3-(6-fluoro-5-((3-(4'-fluoro-3,4,5,6-tetrahydro-[1,1'-biphenyl]-2-carbonyl)-3,6-diazabicyclo[3.1.1]heptan-6-yl)methyl)-1-oxoisoindolin-2-yl)piperidine-2,6-dione | 3-(6-fluoro-5-((3-(4'-fluoro-3,4,5,6-tetrahydro-[1,1'-biphenyl]-2-carbonyl)-3,6-diazabicyclo[3.1.1]heptan-6-yl)methyl)-1-oxoisoindolin-2-yl)piperidine-2,6-dione |
| GT-05504 | 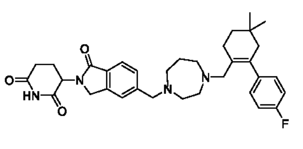 | 3-(5-((4-((4'-fluoro-5,5-dimethyl-3,4,5,6-tetrahydro-[1,1'-biphenyl]-2-yl)methyl)-1,4-diazepan-1-yl)methyl)-1-oxoisoindolin-2-yl)piperidine-2,6-dione | 3-(5-((4-((4'-fluoro-5,5-dimethyl-3,4,5,6-tetrahydro-[1,1'-biphenyl]-2-yl)methyl)-1,4-diazepan-1-yl)methyl)-1-oxoisoindolin-2-yl)piperidine-2,6-dione |
| GT-05661 | 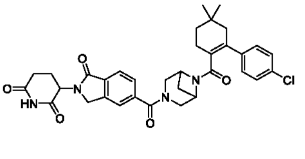 | 3-(5-(6-(4'-chloro-5,5-dimethyl-3,4,5,6-tetrahydro-[1,1'-biphenyl]-2-carbonyl)-3,6-diazabicyclo[3.1.1]heptane-3-carbonyl)-1-oxoisoindolin-2-yl)piperidine-2,6-dione | 3-(5-(6-(4'-chloro-5,5-dimethyl-3,4,5,6-tetrahydro-[1,1'-biphenyl]-2-carbonyl)-3,6-diazabicyclo[3.1.1]heptane-3-carbonyl)-1-oxoisoindolin-2-yl)piperidine-2,6-dione |

(continued)

| Compo und No. | Structure of the compounds | The compounds' name | Chinese translation of the compounds' name |
|---|---|---|---|
| GT-05663 | 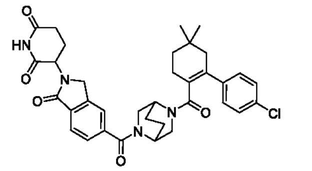 | 3-(5-(5-(4'-chloro-5,5-dimethyl-3,4,5,6-tetrahydro-[1,1'-biphenyl]-2-carbonyl)-2,5-diazabicyclo[2.2.2]octane-2-carbonyl)-1-oxoisoindolin-2-yl)piperidine-2,6-dione | 3-(5-(5-(4'-chloro-5,5-dimethyl-3,4,5,6-tetrahydro-[1,1'-biphenyl]-2-carbonyl)-2,5-diazabicyclo[2.2.2]octane-2-carbonyl)-1-oxoisoindolin-2-yl)piperidine-2,6-dione |
| GT-05462 | 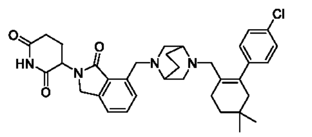 | 3-(7-((5-((4'-chloro-5,5-dimethyl-3,4,5,6-tetrahydro-[1,1'-biphenyl]-2-yl)methyl)-2,5-diazabicyclo[2.2.2]octan-2-yl) methyl)-1-oxoisoindolin-2-yl)piperidine-2,6-dione | 3-(7-((5-((4'-chloro-5,5-dimethyl-3,4,5,6-tetrahydro-[1,1'-biphenyl]-2-yl)methyl)-2,5-diazabicyclo[2.2.2]octan-2-yl) methyl)-1-oxoisoindolin-2-yl)piperidine-2,6-dione |
| GT-05469 | 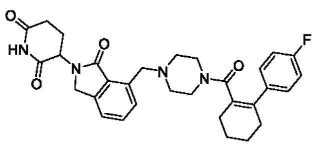 | 3-(7-((4-(4'-fluoro-3,4,5,6-tetrahydro-[1,1'-biphenyl]-2-carbonyl)piperazin-1-yl)methyl)-1-oxoisoindolin-2-yl)piperidine-2,6-dione | 3-(7-((4-(4'-fluoro-3,4,5,6-tetrahydro-[1,1'-biphenyl]-2-carbonyl)piperazin-1-yl)methyl)-1-oxoisoindolin-2-yl)piperidine-2,6-dione |
| GT-05538 | 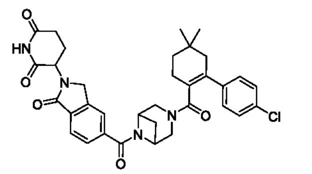 | 3-(5-(3-(4'-chloro-5,5-dimethyl-3,4,5,6-tetrahydro-[1,1'-biphenyl]-2-carbonyl)-3,6-diazabicyclo[3.1.1]heptane-6-carbonyl)-1-oxoisoindolin-2-yl)piperidine-2,6-dione | 3-(5-(3-(4'-chloro-5,5-dimethyl-3,4,5,6-tetrahydro-[1,1'-biphenyl]-2-carbonyl)-3,6-diazabicyclo[3.1.1]heptane-6-carbonyl)-1-oxoisoindolin-2-yl)piperidine-2,6-dione |
| GT-05539 | 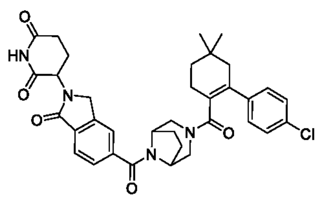 | 3-(5-(3-(4'-chloro-5,5-dimethyl-3,4,5,6-tetrahydro-[1,1'-biphenyl]-2-carbonyl)-3,8-diazabicyclo[3.2.1]octane-8-carbonyl)-1-oxoisoindolin-2-yl)piperidine-2,6-dione | 3-(5-(3-(4'-chloro-5,5-dimethyl-3,4,5,6-tetrahydro-[1,1'-biphenyl]-2-carbonyl)-3,8-diazabicyclo[3.2.1]octane-8-carbonyl)-1-oxoisoindolin-2-yl)piperidine-2,6-dione |
| GT-05540 | 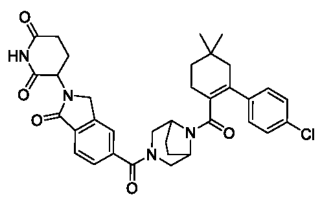 | 3-(5-(8-(4'-chloro-5,5-dimethyl-3,4,5,6-tetrahydro-[1,1'-biphenyl]-2-carbonyl)-3,8-diazabicyclo[3.2.1]octane-3-carbonyl)-1-oxoisoindolin-2-yl)piperidine-2,6-dione | 3-(5-(8-(4'-chloro-5,5-dimethyl-3,4,5,6-tetrahydro-[1,1'-biphenyl]-2-carbonyl)-3,8-diazabicyclo[3.2.1]octane-3-carbonyl)-1-oxoisoindolin-2-yl)piperidine-2,6-dione |
| GT-05635 | 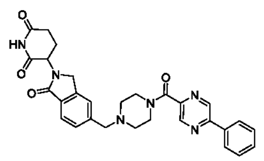 | 3-(1-oxo-5-((4-(5-phenylpyrazine-2-carbonyl)piperazin-1-yl) methyl)isoindolin-2-yl)piperidine-2,6-dione | 3-(1-oxo-5-((4-(5-phenylpyrazine-2-carbonyl)piperazin-1-yl) methyl)isoindolin-2-yl)piperidine-2,6-dione |

**II. Other Forms of Compounds** (including salts, enantiomers, stereoisomers, solvates, isotopically enriched analogs, prodrugs, or polymorphs of compounds)

The compounds of the present disclosure have the structures of any one of Formula (I), Formula (II), Formula (III), Formula (Ia), Formula (Ib), Formula (Ic), Formula (Id), Formula (IIa), Formula (IIb), Formula (IIc), Formula (IId), Formula (IIIa), Formula (IIIb), Formula (IIIc), or Formula (IIId). Unless otherwise specified, all references to the compounds of the present disclosure also include compounds of any one of Formula (I), Formula (II), Formula (III), Formula (Ia), Formula (Ib), Formula (Ic), Formula (Id), Formula (IIa), Formula (IIb), Formula (IIc), Formula (IId), Formula (IIIa), Formula (IIIb), Formula (IIIc), or Formula (IIId) and specific compounds within the scope of these general formulae.

It should be recognized that compounds of the present disclosure (including compounds of Formula (I), Formula (II), Formula (III), Formula (Ia), Formula (Ib), Formula (Ic), Formula (Id), Formula (IIa), Formula (IIb), Formula (IIc), Formula (IId), Formula (IIIa), Formula (IIIb), Formula (IIIc), or Formula (IIId)) may have a stereo-configuration and thus can exist in more than one stereoisomeric form. The present disclosure also relates to optically enriched compounds having a stereo-configuration, e.g., greater than about 90% enantiomeric/diastereomeric excess ("ee"), such as about 95% ee or 97% ee, or greater than 99% ee, and mixtures thereof, including racemic mixtures. As used herein, "optically enriched" means that a mixture of enantiomers consists of a significantly greater proportion of one enantiomer, and can be described by enantiomeric excess (ee%). Purification of isomers and separation of mixtures of isomers can be accomplished by standard techniques known in the art (e.g., column chromatography, preparative TLC, preparative HPLC, asymmetric synthesis (e.g., by using chiral intermediates) and/or or chiral resolution, etc.).

In some embodiments, polymorph forms or salts of the compounds of the present disclosure are also provided. Salts of the compounds of the present disclosure can be pharmaceutically acceptable salts including, but not limited to, hydrochlorides, sulfates, citrates, maleates, sulfonates, citrates, lactates, tartrates, fumarates, phosphates, dihydrophosphates, pyrophosphates, metaphosphates, oxalates, malonates, benzoates, mandelates, succinates, trifluoroacetates, hydroxyacetates, or *p*-toluenesulfonates, etc. The compounds of the present disclosure can exist as non-solvated or solvated forms in pharmaceutically acceptable solvents such as water, ethanol, and the like. In some embodiments, compounds of the present disclosure can be prepared as prodrugs or precursor drugs. Prodrugs can be converted into parent drugs in the body to play their role. In some embodiments, isotopically-labeled compounds of the present disclosure are also provided, examples of which include deuterium (D or $^2$H).

**III. Compositions/Formulations**

In some embodiments, the present disclosure provides a pharmaceutical composition comprising as an active ingredient the compound of the present disclosure or a pharmaceutically acceptable salt, solvate, isotopically enriched analog, polymorph, prodrug, stereoisomer (including enantiomer), or mixture of stereoisomers thereof, and at least one pharmaceutically acceptable carrier.

In some embodiments, pharmaceutically acceptable carriers include, but are not limited to, fillers, stabilizers, dispersants, suspending agents, diluents, excipients, thickeners, colorants, solvents, or encapsulating materials. Each carrier must be "acceptable" in the sense of being compatible with the other ingredients of the formulation (including the compounds useful in the present disclosure) and not injurious to the patient. Some examples of materials that can be used as pharmaceutically acceptable carriers include: sugars such as lactose, glucose, and sucrose; starches such as corn starch and potato starch; cellulose and its derivatives such as sodium carboxymethyl cellulose, ethyl cellulose and cellulose acetate; powdered tragacanth; malt; gelatin; talc; excipients such as cocoa butter and suppository wax; oils such as peanut oil, cottonseed oil, safflower oil, sesame oil, olive oil, corn oil and soybean oil; glycols such as propylene glycol; polyols such as glycerol, sorbitol, mannitol, and polyethylene glycol; esters such as ethyl oleate and ethyl laurate; agar; buffers such as magnesium hydroxide and aluminum hydroxide; surfactant phosphate buffer solution; polyethylene oxide, polyvinylpyrrolidone, polyacrylamide, poloxamer; and other common non-toxic compatible substances used in pharmaceutical formulations.

The pharmaceutical composition of the present disclosure can further comprise at least one second therapeutic agent, e.g., an anticancer agent. The second therapeutic agent may be used in combination with the compounds of Formula (I) of the present disclosure to treat the diseases or disorders as disclosed herein. The second therapeutic agent includes, but is not limited to, chemotherapeutic agents, immunotherapeutic agents, gene therapy agents, and the like.

The pharmaceutical composition of the present disclosure comprising, as an active ingredient, the compounds of Formula (I) of the present disclosure or a pharmaceutically acceptable salt thereof can be formulated into any suitable formulations such as sprays, patches, tablets (such as conventional tablets, dispersible tablets, orally disintegrating tablets), capsules (such as soft capsules, hard capsules, enteric-coated capsules), dragees, troches, powders, granules, powder injections, suppositories, or liquid formulations (such as suspensions (e.g., aqueous or oily suspensions), solutions, emulsions, or syrups), or conventional injection dosage forms such as injectable solutions (e.g., sterile injectable solutions formulated according to methods known in the art using water, Ringer's solution, or isotonic sodium chloride solution or the like as a vehicle or solvent) or lyophilized injectable formulation and the like, depending upon a suitable route of administration (including, but not limited to, nasal administration, inhalation administration, topical administration, oral administration, oral mucosal administration, rectal administration, intrapleural administration, intraperitoneal administration, vaginal administration, intramuscular administration, subcutaneous administration, transdermal administration, epidural administration, intrathecal administration, and intravenous administration). Those skilled in the art can also formulate the compounds of Formula (I) of the present disclosure into conventional, dispersible, chewable, orally disintegrating or rapidly dissolving formulations, or sustained-release capsules or controlled-release capsules as needed.

The compounds of Formula (I) of the present disclosure, as an active ingredient, is contained in the pharmaceutically acceptable carrier or diluent in an amount sufficient to deliver to a subject a therapeutically effective amount for the indication to be treated, without causing serious toxic effects in the subject treated. A dosage of the active compound for all diseases or disorders mentioned herein ranges, for example, from about 5ng/kg to 500mg/kg, from about 10ng/kg to 300mg/kg per day, such as from 0.1 to 100 mg/kg, or from 0.5 to about 25mg per kilogram body weight of the subject per day.

The compounds of Formula (I) of the present disclosure or pharmaceutically acceptable salts thereof can be conveniently administered in any suitable unit dosage form. Suitable unit dosage form specifications include, but are not limited to, less than 1mg, 1mg to 3000mg, 5mg to 1000mg, for example, 5 to 500mg, 25 to 250mg of active ingredient per unit dosage form.

## IV. Kits/Packaged Products

The compounds of Formula (I) or a pharmaceutically acceptable salt, solvate, isotopically enriched analog, polymorph, prodrug, stereoisomer (including enantiomer), or mixture of stereoisomers thereof is used as a medicament. The medicament of the present disclosure or the pharmaceutical composition of the present disclosure may be presented in a kit/packaged product. The kit/packaged product may include a package or container including, but not limited to, ampoules, blister packs, pharmaceutical plastic bottles, vials, pharmaceutical glass bottles, containers, syringes, laminated flexible packaging, co-extruded film infusion containers, test tubes and dispensing devices, and the like. The kit/packaged product may contain instructions for use of the product.

## V. Methods and Uses

The compounds of Formula (I) or a pharmaceutically acceptable salt, solvate, isotopically enriched analog, polymorph, prodrug, stereoisomer (including enantiomer), or mixture of stereoisomers thereof can be used as a medicament. Especially the compounds of Formula (I) or a pharmaceutically acceptable salt, solvate, isotopically enriched analog, polymorph, prodrug, stereoisomer (including enantiomer), or mixture of stereoisomers thereof can be used for the manufacture of a medicament for the prevention and/or treatment of diseases or disorders associated with cereblon protein. The diseases or disorders associated with cereblon protein comprise: tumor, infectious disease, inflammatory disease, autoimmune disease, anemia, hemorrhagic shock, transplant rejection, multiple organ dysfunction syndrome (MODS), sarcoidosis, adult respiratory distress syndrome, cardiovascular disease, Richter syndrome (RS), acute liver failure, or diabetes. In some embodiments, the diseases or disorders associated with cereblon protein include, but are not limited to, tumor, infectious disease, inflammatory disease, autoimmune disease, anemia, hemorrhagic shock, transplant rejection, multiple organ dysfunction syndrome (MODS), sarcoidosis, adult respiratory distress syndrome, cardiovascular disease, Richter syndrome (RS), acute liver failure, or diabetes. In some embodiments, the diseases or disorders associated with cereblon protein include, but are not limited to, myeloma, including multiple myeloma, plasma cell myeloma, smoldering myeloma, smoldering multiple myeloma; myelofibrosis; bone marrow disease; myelodysplastic syndrome (MDS); previously treated myelodysplastic syndrome; transplantation-related cancer; neutropenia; leukemia, including acute myeloid leukemia, chronic myelogenous leukemia, B-cell chronic lymphocytic leukemia, leukemia-associated anemia, acute myeloid leukemia (AML); lymphoma, including diffuse large B-cell lymphoma, non-Hodgkin's lymphoma, Hodgkin's lymphoma, anaplastic lymphoma, anaplastic large cell lymphoma, CD20 positive lymphoma, mantle cell lymphoma, primary lymphoma, B-cell lymphoma, recurrent B-cell non-Hodgkin's lymphoma, recurrent diffuse large B-cell lymphoma, recurrent mediastinal (thymic) large B-cell lymphoma, primary mediastinal (thymic) large B-cell lymphoma, recurrent transformed non-Hodgkin's lymphoma, refractory B-cell non-Hodgkin's lymphoma, refractory diffuse large B-cell lymphoma, refractory primary mediastinal (thymic) large B-cell lymphoma, refractory transformed non-Hodgkin's lymphoma; thyroid cancer; melanoma; lung cancer, including lung adenocarcinoma, lung squamous cell carcinoma; inflammatory myofibroblastoma; colorectal cancer; intestinal cancer; brain glioma; astroblastoma; ovarian cancer; bronchial cancer; prostate cancer; breast cancer, including triple negative breast cancer, sporadic breast cancer and patients with Cowden syndrome; pancreatic cancer; central nervous system tumor; neuroblastoma; neuroglioma; Peripheral neuroepithelioma; extramedullary plasmacytoma; plasmacytoma; gastric cancer; gastrointestinal stromal tumors; esophageal cancer; colorectal adenocarcinoma; esophageal squamous cell carcinoma; liver cancer; renal cell carcinoma; bladder cancer; endometrial cancer; metrocarcinoma; head and neck cancer; brain cancer; oral cancer; sarcoma, including rhabdomyosarcoma, various lipogenic tumors, Ewing's sarcoma/primitive neuroectodermal tumors (Ewing/PNETs), and leiomyosarcoma; urothelial carcinoma; basal cell carcinoma; oral squamous cell carcinoma; cholangiocarcinoma; bone cancer; cervical cancer; skin cancer; Richter syndrome (RS); sepsis syndrome; autoimmune diseases, including rheumatoid arthritis, autoimmune encephalomyelitis, ankylosing spondylitis, psoriasis, systemic lupus erythematosus, multiple sclerosis, recurrent oral ulcers, Kawasaki disease, polymyositis/dermatomyositis, Sjogren's syndrome, and atopic dermatitis; keratoconjunctivitis; inflammatory diseases, including Crohn's disease and ulcerative colitis, pneumonia, osteoarthritis, synovitis, systemic inflammatory response syndrome, airway inflammation,

bronchitis; cerebral malaria; infectious diseases, including viral pneumonia, Acquired immunodeficiency syndrome (AIDS), COVID-19 novel coronavirus infection, gram-negative bacteria infection, gram-positive bacteria infection, tuberculosis, etc.; septic shock; tuberculosis; bacterial meningitis; chronic obstructive pulmonary disease; asthma; hemorrhagic shock; organ (including kidney, heart, lung) or tissue transplantation rejection; diabetes; sarcoidosis; adult respiratory distress syndrome; anemia; pediatric aplastic anemia; cardiovascular diseases (e.g., coronary heart disease, congestive heart failure, myocardial infarction, atherosclerosis); multiple organ dysfunction caused by cachexia and septic shock; or acute liver failure.

The present disclosure provides a method for preventing and/or treating diseases or disorders associated with cereblon protein, comprising administering to a subject a therapeutically effective amount of the compounds of Formula (I) of the present disclosure or a pharmaceutically acceptable salt thereof, or the pharmaceutical composition of the present disclosure comprising as an active ingredient the compounds of Formula (I) of the present disclosure or a pharmaceutically acceptable salt thereof. In some embodiments, the diseases or disorders associated with cereblon protein comprise: tumor, infectious disease, inflammatory disease, autoimmune disease, anemia, hemorrhagic shock, transplant rejection, multiple organ dysfunction syndrome (MODS), sarcoidosis, adult respiratory distress syndrome, cardiovascular disease, Richter syndrome (RS), acute liver failure, or diabetes. In some embodiments, the diseases or disorders associated with cereblon protein include, but are not limited to, myeloma, including multiple myeloma, plasma cell myeloma, smoldering myeloma, smoldering multiple myeloma; myelofibrosis; bone marrow disease; myelodysplastic syndrome (MDS); previously treated myelodysplastic syndrome; transplantation-related cancer; neutropenia; leukemia, including acute myeloid leukemia, chronic myelogenous leukemia, B-cell chronic lymphocytic leukemia, leukemia-associated anemia, acute myeloid leukemia (AML); lymphoma, including diffuse large B-cell lymphoma, non-Hodgkin's lymphoma, Hodgkin's lymphoma, anaplastic lymphoma, anaplastic large cell lymphoma, CD20 positive lymphoma, mantle cell lymphoma, primary lymphoma, B-cell lymphoma, recurrent B-cell non-Hodgkin's lymphoma, recurrent diffuse large B-cell lymphoma, recurrent mediastinal (thymic) large B-cell lymphoma, primary mediastinal (thymic) large B-cell lymphoma, recurrent transformed non-Hodgkin's lymphoma, refractory B-cell non-Hodgkin's lymphoma, refractory diffuse large B-cell lymphoma, refractory primary mediastinal (thymic) large B-cell lymphoma, refractory transformed non-Hodgkin's lymphoma; thyroid cancer; melanoma; lung cancer, including lung adenocarcinoma, lung squamous cell carcinoma; inflammatory myofibroblastoma; colorectal cancer; intestinal cancer; brain glioma; astroblastoma; ovarian cancer; bronchial cancer; prostate cancer; breast cancer, including triple negative breast cancer, sporadic breast cancer and patients with Cowden syndrome; pancreatic cancer; central nervous system tumor; neuroblastoma; neuroglioma; Peripheral neuroepithelioma; extramedullary plasmacytoma; plasmacytoma; gastric cancer; gastrointestinal stromal tumors; esophageal cancer; colorectal adenocarcinoma; esophageal squamous cell carcinoma; liver cancer; renal cell carcinoma; bladder cancer; endometrial cancer; metrocarcinoma; head and neck cancer; brain cancer; oral cancer; sarcoma, including rhabdomyosarcoma, various lipogenic tumors, Ewing's sarcoma/primitive neuroectodermal tumors (Ewing/PNETs), and leiomyosarcoma; urothelial carcinoma; basal cell carcinoma; oral squamous cell carcinoma; cholangiocarcinoma; bone cancer; cervical cancer; skin cancer; Richter syndrome (RS); sepsis syndrome; autoimmune diseases, including rheumatoid arthritis, autoimmune encephalomyelitis, ankylosing spondylitis, psoriasis, systemic lupus erythematosus, multiple sclerosis, recurrent oral ulcers, Kawasaki disease, polymyositis/dermatomyositis, Sjogren's syndrome, and atopic dermatitis; keratoconjunctivitis; inflammatory diseases, including Crohn's disease and ulcerative colitis, pneumonia, osteoarthritis, synovitis, systemic inflammatory response syndrome, airway inflammation, bronchitis; cerebral malaria; infectious diseases, including viral pneumonia, Acquired immunodeficiency syndrome (AIDS), COVID-19 novel coronavirus infection, gram-negative bacteria infection, gram-positive bacteria infection, tuberculosis, etc.; septic shock; tuberculosis; bacterial meningitis; chronic obstructive pulmonary disease; asthma; hemorrhagic shock; organ (including kidney, heart, lung) or tissue transplantation rejection; diabetes; sarcoidosis; adult respiratory distress syndrome; anemia; pediatric aplastic anemia; cardiovascular diseases (e.g., coronary heart disease, congestive heart failure, myocardial infarction, atherosclerosis); multiple organ dysfunction caused by cachexia and septic shock; or acute liver failure.

In the method for preventing and/or treating diseases or disorders associated with cereblon protein, the compound of Formula (I) of the present disclosure or the pharmaceutical composition comprising as an active ingredient the compound of Formula (I) of the present disclosure is administered to the subject through at least one mode of administration selected from the group consisting of nasal administration, inhalation administration, topical administration, oral administration, oral mucosal administration, rectal administration, pleural cavity administration, peritoneal administration, vaginal administration, intramuscular administration, subcutaneous, transdermal, epidural, intrathecal, and intravenous administration.

The term "treatment" or "treating" refers to the administration of the compound of Formula (I) or a pharmaceutically acceptable salt thereof according to the present disclosure, or the pharmaceutical composition containing, as an active ingredient, the compound of Formula (I) or a pharmaceutically acceptable salt thereof, to a subject to mitigate (alleviate) undesirable diseases or conditions, such as the development of a cancer or tumor. The beneficial or desired clinical results of the present disclosure include, but are not limited to: alleviating symptoms, reducing the severity of the disease, stabilizing the state of the disease, slowing down or delaying the progression of the disease, improving or

alleviating the condition, and alleviating the disease.

A "therapeutic effective amount" of the compound of the present disclosure depends on a variety of factors, including the activity of the specific compound used, the metabolic stability of the compound and the duration of its action, the age, sex and weight of the patient, the patient's current medical condition, the route and duration of administration, the excretion rate, the combined administration of additional drugs, and the progression of the diseases or conditions of the patient being treated. Those skilled in the art will be able to determine appropriate dosages based on these and other factors.

It is to be understood that the choice of using one or more active compounds and/or compositions and their dosage depends on the basic situations of the individual (which should generally render the individual situation to achieve the best effect). Dosing and dosing regimens should be within the ability of those skilled in the art, and the appropriate dosage depends on many factors including the knowledge and ability of the physicians, veterinarians or researchers (see e.g., Jun Li (chief editor), "Clinical Pharmacology", 4th edition, People's Public Health Press, 2008).

As used herein, the term "patient" or "subject" to be treated refers to animal, for example mammal, including but not limited to primate (such as human being), cow, sheep, goat, horse, dog, cat, rabbit, guinea pig, rat, mice, etc.

## VI. Definitions

Unless otherwise specified, the following words, phrases and symbols used herein generally have the meanings as described below.

In general, the nomenclature used herein (including the IUPAC nomenclature) and the laboratory procedures described below (including those used in cell culture, organic chemistry, analytical chemistry, and pharmacology, etc.) are those well-known and commonly used in the art. Unless otherwise defined, all scientific and technical terms used herein in connection with the present disclosure described herein have the same meaning as commonly understood by one skill in the art. In addition, the use of the word "a" or "an" when used in conjunction with the term "comprising" or a noun in the claims and/or the specification may mean "one", but it is also consistent with the meaning of "one or more", "at least one", and "one or more than one". Similarly, the terms "another" or "other" can mean at least a second or more.

It should be understood that whenever the term "comprise" or "include" is used herein to describe various aspects, other similar aspects described by "consisting of" and/or "consisting essentially of" are also provided.

As used herein, the term "about" used alone or in combination refers to approximately, roughly, nearly, or around. When the term "about" is used in conjunction with a numerical range, it modifies that range by extending the boundaries above and below the stated numerical value. In general, the term "about" can modify a numerical value above and below the stated value by an upward or downward (increasing or decreasing) variation, e.g., 10%, 5%, 2%, or 1%.

As used herein, the wording "...represents a bond" used alone or in combination means that the referenced group is a bond linker (that is, the referenced group is absent). For example, the wording "$L_1$ represents a bond" means that $L_1$ is a bond linker. In other words, when $L_1$ represents a bond, the group $X_1$ in the structure of Formula (I) is directly connected to benzene ring in the structure of Formula (I). For example, the wording "$X_2$ represents a bond" means that $X_2$ is a bond linker. In other words, when $X_2$ represents a bond, the group $R_{a1}$ in the structure of Formula (I) is directly connected to the group $X_1$ in the compound of Formula (I).

In this document, the term "optionally substituted" used alone or in combination means that the referenced group may be unsubstituted or substituted with one or more substituents as defined here. Herein, the wording "optionally substituted with..." and "unsubstituted or substituted" can be used interchangeably. The term "substituted" generally indicates that one or more hydrogens in the referenced structure are replaced by the same or different specific substituents. The number of substituents is not theoretically limited in any way, or is automatically limited by the size of the building units (i.e., the total number of replaceable hydrogen atoms in the building units), or as clearly defined herein.

Herein, a bond interrupted by a wavy line shows the point of attachment of the depicted group to the rest of the molecule. For example, the group $R_{a1}$ depicted below

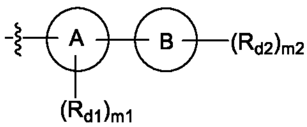

shows the point of attachment of ring A in said group to $X_2$. For example, in the divalent group $X_1$ depicted below:

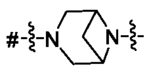

,

the symbol # indicates the point of attachment of the atom N to the group $L_1$, while another atom N is connected to the group $X_2$. Herein, when the attachment point of the group is not specified, e.g., for the group $-CH_2-NH-$ represented by $X_2$, either end of the group (e.g., $CH_2$) can be attached to the group $X_1$ of the compound of Formula (I), while the other end is attached to group $R_{a1}$.

As used herein, the term "halogen atom" or "halogen", used alone or in combination, refers to fluorine, chlorine, bromine, or iodine.

As used herein, the term "alkyl", used alone or in combination, refers to a linear or branched alkyl group. The term "$C_x$-$C_y$ alkyl" or "$C_{x-y}$ alkyl" (x and y each being an integer) refers to a linear or branched alkyl group containing from x to y carbon atoms. The term "$C_{1-6}$ alkyl" used alone or in combination in the present disclosure refers to a linear or branched alkyl group containing from 1 to 6 carbon atoms, examples of which include $C_1$-$C_5$ alkyl, $C_1$-$C_4$ alkyl, and $C_1$-$C_3$ alkyl and the like. The term "$C_{1-4}$ alkyl" used alone or in combination in the present disclosure refers to a linear or branched alkyl group containing from 1 to 4 carbon atoms. Examples of the $C_{1-6}$ alkyl of the present disclosure may include a $C_1$-$C_5$ alkyl, $C_1$-$C_4$ alkyl, $C_1$-$C_3$ alkyl, $C_1$-$C_2$ alkyl and methyl. Representative examples of the term "$C_{1-6}$ alkyl" include methyl, ethyl, n-propyl, isopropyl, n-butyl, isobutyl, sec-butyl, tert-butyl, pentyl, isopentyl, neopentyl, tert-pentyl, and hexyl. The term "$C_{1-4}$ alkyl" used alone or in combination in the present disclosure refers to a linear or branched alkyl group containing from 1 to 4 carbon atoms. Examples of the $C_{1-4}$ alkyl of the present disclosure may include $C_1$-$C_4$ alkyl, $C_1$-$C_3$ alkyl, $C_1$-$C_2$ alkyl and methyl. Representative examples of the term "$C_{1-4}$ alkyl" include methyl, ethyl, n-propyl, isopropyl, n-butyl, isobutyl, sec-butyl, and tert-butyl. The term "$C_{1-3}$ alkyl" or "$C_1$-$C_3$ alkyl" in the present disclosure refers to an alkyl group containing from 1 to 3 carbon atoms, and representative examples of which include methyl, ethyl, n-propyl, and isopropyl. In the present disclosure, the "alkyl" is optionally substituted with one or more substituents optionally selected from the group consisting of D (i.e., deuterium), halogen, hydroxy, cyano, $C_{1-3}$ alkyl, $C_{1-3}$ alkoxy, trifluoromethyl, heterocyclyl (e.g., 4- to 8-membered heterocyclyl), or a combination thereof.

As used herein, the term "halogenated alkyl" or "haloalkyl", used alone or in combination, refers to a linear or branched alkyl group substituted with one or more halogens, wherein one or more hydrogen atom(s) of the alkyl group are replaced with one or more halogens. The term "halogenated $C_{x-Cy}$ alkyl" or "halogenated $C_{x-y}$ alkyl" (x and y are each an integer) refers to a linear or branched alkyl containing from x to y carbon atoms substituted with one or more halogens. The term "halogenated $C_{1-6}$ alkyl" used alone or in combination in the present disclosure refers to a linear or branched alkyl group containing from 1 to 6 carbon atoms substituted with one or more halogens. Examples of the halogenated $C_{1-6}$ alkyl group of the present disclosure include halogenated $C_{1-6}$ alkyl group, halogenated $C_{1-5}$ alkyl group, halogenated $C_{1-4}$ alkyl group, halogenated $C_{1-3}$ alkyl, halogenated $C_{1-2}$ alkyl, and halogenated methyl. Representative examples of the halogenated $C_{1-6}$ alkyl group include halomethyl, haloethyl, halo-n-propyl, haloisopropyl, halo-n-butyl, haloisobutyl, halo-sec-butyl, halo-tert-butyl, halopentyl, haloisoamyl, haloneopentyl, halo-tert-pentyl, and halohexyl, including in particular, for example, $F_3C$-, $FCH_2$-, $F_2CH$-, $ClCH_2$-, $Cl_2CH$-, $CF_3CF_2$-, $CF_3CHF$-, $CHF_2CF_2$-, $CHF_2CHF$-, $CF_3CH_2$- and $CH_2ClCH_2$-. The term "halo-$C_{1-3}$ alkyl" or "halo-$C_1$-$C_3$ alkyl" of the present disclosure refers to an alkyl group containing from 1 to 3 carbon atoms substituted with one or more halogens, and its representative examples include halomethyl, haloethyl, halo-n-propyl and haloisopropyl, including in particular, for example, $F_3C$-, $FCH_2$-, $F_2CH$-, $ClCH_2$-, $Cl_2CH$-, $CF_3CF_2$-, $CF_3CHF$-, $CHF_2CF_2$-, $CHF_2CHF$-, $CF_3CH_2$-and $CH_2ClCH_2$-.

As used herein, the term "deuterated", used alone or in combination, means that one or more hydrogen atom(s) of the referenced group are replaced with deuterium atoms (i.e., D).

As used herein, the term "deuterated $C_{x-Cy}$ alkyl" or "deuterated $C_{x-y}$ alkyl" (x and y are each an integer) refers to a linear or branched alkyl containing from x to y carbon atoms substituted with one or more deuterium atoms. The term "deuterated $C_{1-6}$ alkyl" used alone or in combination in the present disclosure refers to a linear or branched alkyl group containing from 1 to 6 carbon atoms substituted with one or more deuterium atoms. Examples of the deuterated $C_{1-6}$ alkyl group of the present disclosure include deuterated $C_{1-5}$ alkyl group, e.g., deuterated $C_{1-4}$ alkyl group, deuterated $C_{2-5}$ alkyl group, deuterated $C_{1-3}$ alkyl group, deuterated $C_{1-2}$ alkyl, and deuterated methyl. Representative examples of the deuterated $C_{1-6}$ alkyl group include perdeuterated methyl ($CD_3$), perdeuterated ethyl ($CD_3CD_2$), perdeuterated n-propyl, perdeuterated isopropyl, perdeuterated n-butyl, perdeuterated isobutyl, perdeuterated sec-butyl, perdeuterated tert-butyl, perdeuterated pentyl, perdeuterated isopentyl, perdeuterated neopentyl, perdeuterated tert-pentyl, perdeuterated hexyl. The term "deuterated $C_{1-3}$ alkyl" or "deuterated $C_1$-$C_3$ alkyl" of the present disclosure refers to an alkyl group containing from 1 to 3 carbon atoms substituted with one or more deuterium atoms, and its representative examples include perdeuterated methyl ($CD_3$) and perdeuterated ethyl ($CD_3CD_2$).

As used herein, the term "alkylene" (which is used interchangeably with "alkylene chain"), used alone or in combination, refers to a linear or branched divalent saturated hydrocarbon group composed of carbon and hydrogen atoms. The term "$C_x$-$C_y$ alkylene" or "$C_{x-y}$ alkylene" (x and y each being an integer) refers to a linear or branched alkylene group containing from x to y carbon atoms. The term "$C_1$-$C_5$ alkylene" refers to a linear or branched alkylene group containing from 1 to 5 carbon atoms, examples of which include $C_2$-$C_5$ alkylene, $C_1$-$C_4$ alkylene, $C_1$-$C_3$ alkylene, $C_1$-$C_2$ alkylene, and methylene. Representative examples include, but are not limited to, methylene, ethylene, propylene, isopropylene, butylene, isobutylene, sec-butylene, tert-butylene, n-pentylene, isopentylene, neopentylidene, and tert-pentylene. In the present disclosure, the "alkylene" is optionally substituted with one or more (e.g., 1-10, 1-6, 1-5, 1-4, 1-3, 2-3 or 1) substituents optionally selected from D, optionally deuterated $C_{1-4}$ alkyl, $C_{3-6}$ cycloalkyl, hydroxy, amino, mercapto, halogen, cyano, $C_1$-$C_4$ alkoxy, $C_1$-$C_4$ alkylamino, halogenated $C_1$-$C_4$ alkyl, amino$C_{1-4}$ alkylene, $C_{1-4}$ alkyl-NHC(O)-, $C_{1-4}$ alkyl-C(O)NH- and any combination thereof.

As used herein, the term "alkoxy", used alone or in combination, refers to a linear or branched alkoxy group having structural formula of alkyl-O-. Optionally, the alkyl portion of the alkoxy group may contain 1-10 carbon atoms. Representative examples of "alkoxy" include, but are not limited to, methoxy, ethoxy, propoxy, isopropoxy, n-butoxy, isobutoxy, tert-butoxy, pentyloxy, 2-pentyloxy, isopentyloxy, neopentyloxy, hexyloxy, 2-hexyloxy, 3-hexyloxy, 3-methylpentyloxy, etc. The term "$C_1$-$C_4$ alkoxy" or "$C_{1-4}$ alkoxy" refers to a linear or branched alkoxy group containing from 1 to 4 carbon atoms. Representative examples of $C_{1-4}$ alkoxy include, but are not limited to, methoxy, ethoxy, n-propoxy, isopropoxy, n-butoxy, isobutoxy, and tert-butoxy.

As used herein, the term "halogenated alkoxy", used alone or in combination, refers to a alkoxy group substituted with one or more halogen atoms. Optionally, the alkyl portion of the alkoxy group may contain 1-10 carbon atoms. Examples of "halogenated alkoxy" include, but are not limited to, halogenated $C_{1-6}$ alkoxy or halogenated $C_{1-4}$ alkoxy. Representative examples include, but are not limited to, $F_3C$-O-, $FCH_2$-O-, $F_2CH$-O-, $ClCH_2$-O-, $Cl_2CH$-O-, $CF_3CF_2$-O-, $CF_3CHF$-O-, $CHF_2CF_2$-O-, $CHF_2CHF$-O-, $CF_3CH_2$-O-, and $CH_2ClCH_2$-O-.

As used herein, the term "alkyl-NH-", used alone or in combination, refers to a linear or branched alkyl-NH-, wherein alkyl is as defined above. Optionally, the alkyl portion of the alkyl-NH-group may contain 1-6 carbon atoms, i.e., $C_{1-6}$ alkyl-NH-. Representative examples of "alkyl-NH-" include, but are not limited to, methyl-NH-, ethyl-NH-, n-propyl-NH-, isopropyl-NH-, n-butyl-NH-, isobutyl-NH-, tert-butyl-NH-, pentyl-NH-, and hexyl-NH-, etc. The term "$C_1$-$C_4$ alkyl-NH-" or "$C_{1-4}$ alkyl-NH-" refers to a linear or branched alkyl-NH- group containing from 1 to 4 carbon atoms. Representative examples of $C_{1-4}$ alkyl-NH- include, but are not limited to, methyl-NH-, ethyl-NH-, n-propyl-NH-, isopropyl-NH-, n-butyl-NH-, sec-butyl-NH- and tert-butyl-NH-.

As used herein, the term "$NH_2$-alkylene", used alone or in combination, refers to a linear or branched alkylene group substituted with amino, wherein alkylene is as defined above. Optionally, the alkylene portion of the "$NH_2$-alkylene" group may contain 1-6 carbon atoms. The term "$NH_2$-$C_{1-4}$ alkylene" or "amino-$C_{1-4}$ alkylene-" refers to a linear or branched alkylene group containing from 1 to 4 carbon atoms which is substituted with amino. Representative examples of $NH_2$-$C_{1-4}$ alkylene include, but are not limited to, $NH_2$-$CH_2$-, $NH_2$-$CH_2CH_2$-, and $NH_2$-$CH_2CH_2CH_2$-.

As used herein, the term "alkyl-NHC(O)-", used alone or in combination, refers to a linear or branched alkyl-NHC(O)- group, wherein alkyl is as defined above. Optionally, the alkyl portion of the alkyl-NHC(O)- group may contain 1-6 carbon atoms. The term "$C_{1-4}$ alkyl-NHC(O)-" or "$C_1$-$C_4$ alkyl-NHC(O)-" refers to a linear or branched alkyl-NHC(O)- group containing from 1 to 4 carbon atoms. Representative examples of $C_{1-4}$ alkyl-NHC(O)- include, but are not limited to, $CH_3$-NHC(O)-, $CH_3CH_2$-NHC(O)-, and $CH_3CH_2CH_2$-NHC(O)-.

As used herein, the term "alkyl-C(O)NH-", used alone or in combination, refers to a linear or branched alkyl-C(O)NH- group, wherein alkyl is as defined above. Optionally, the alkyl portion of the alkyl-C(O)NH- group may contain 1-6 carbon atoms. The term "$C_{1-4}$ alkyl-C(O)NH-" or "$C_1$-$C_4$ alkyl-C(O)NH-" refers to a linear or branched alkyl-C(O)NH- group containing from 1 to 4 carbon atoms. Representative examples of $C_{1-4}$ alkyl-C(O)NH- include, but are not limited to, $CH_3$-C(O)NH-, $CH_3CH_2$-C(O)NH-, and $CH_3CH_2CH_2$-C(O)NH-.

As used herein, the term "heteroaryl", used alone or in combination, refers to a 5- to 20-membered (e.g., 5- to 15-membered, 5- to 12-membered, 5- to 11-membered, 5- to 10-membered, 5- to 9-membered, 5- to 8-membered, 5- to 7-membered, 5- to 6-membered, 6- to 15-membered, or 6- to 9-membered) monocyclic, bicyclic, or polycyclic cyclic radical containing at least one aromatic ring having one or more (e.g., from 1 to 6, or from 1 to 5, or from 1 to 4, or from 1 to 3) heteroatoms independently selected from the group consisting of oxygen, nitrogen, and sulfur. Bicyclic or polycyclic heteroaryl groups include bicyclic, tricyclic or tetracyclic heteroaryl groups, which contain one aromatic ring having one or more heteroatoms independently selected from O, S and N, and the remaining rings which may be a saturated, partially unsaturated or aromatic ring and can be carbocyclic ring or contain one or more heteroatoms independently selected from O, S and N. Examples of monocyclic heteroaryl groups include, but are not limited to, furanyl, oxazolyl, isoxazolyl, oxadiazolyl, thienyl, thiazolyl, isothiazolyl, thiadiazolyl, pyrrolyl, imidazolyl, pyrazolyl, triazolyl, pyridyl, pyrimidinyl, pyridazinyl, pyrazinyl, tetrazolyl, and triazinyl. Examples of bicyclic heteroaryl groups include, but are not limited to, indolyl, isoindolyl, isoindolinyl, benzofuranyl, isobenzofuranyl, benzothienyl, indazolyl, benzimidazolyl, benzoxazolyl, benzisoxazolyl, benzothiazolyl, benzisothiazolyl, benzotriazolyl, benzo[2,1,3]oxadiazolyl, benzo[2,1,3]thiadiazolyl, benzo[1,2,3]thiadiazolyl, quinolinyl, isoquinolinyl, naphthyridinyl, cinnolinyl, quinazolinyl, quinoxalinyl, phthalazinyl, oxazolopyridyl, furopyridyl, pteridyl, purinyl, pyridopyridyl, pyrazolo[1,5-a]pyridyl, pyrazolo[1,5-a]pyrimidinyl, imidazo[1,2-a]pyridyl, 1H-pyrrolo[3,2-b]pyridyl, 1H-pyrrolo[2,3-b]pyridyl, pyrrolo[2,1-b]thiazolyl and imidazo[2,1-b]thiazolyl. Examples of tricyclic or polycyclic heteroaryl groups include, but are not limited to, acridinyl, benzindolyl, carbazolyl, dibenzofuranyl, and xanthyl. The heteroaryl group may be unsubstituted or substituted. A substituted heteroaryl refers to heteroaryl substituted one or more times (e.g., 1-4, 1-3, or 1-2 times) by a substituent(s) optionally selected from the group consisting of $C_{1-3}$ alkyl, $C_{3-6}$ cycloalkyl, hydroxy, amino, mercapto, halogen, $C_{1-3}$ alkoxy, $C_{1-3}$ alkylamino, halogenated $C_{1-3}$ alkyl,

amino-substituted $C_{1-3}$ alkylene, $C_{1-3}$ alkyl-NHC(O)-, $C_{1-3}$ alkyl-C(O)NH-, cyano, or any combination thereof.

As used herein, the term "heteroarylene", used alone or in combination, refers to a 5- to 20-membered (e.g., 5- to 15-membered, 5- to 12-membered, 5- to 11-membered, 5- to 10-membered, 5- to 9-membered, 5- to 8-membered, 5- to 7-membered, 5- to 6-membered, 6- to 15-membered, or 6- to 9-membered) bivalent monocyclic, bicyclic, or polycyclic cyclic aromatic ring radical containing at least one aromatic ring having one or more (e.g., from 1 to 6, or from 1 to 5, or from 1 to 4, or from 1 to 3) heteroatoms independently selected from the group consisting of oxygen, nitrogen, and sulfur. Bicyclic or polycyclic heteroarylene groups include bicyclic, tricyclic or tetracyclic heteroarylene groups, which contain one aromatic ring having one or more heteroatoms independently selected from O, S and N, and the remaining ring(s) which may be a saturated, partially unsaturated or aromatic ring and can be carbocyclic ring or contain one or more heteroatoms independently selected from O, S and N. Examples of monocyclic heteroarylene groups include, but are not limited to, furanylene, oxazolylene, isoxazolylene, oxadiazolylene, thienylene, thiazolylene, isothiazolylene, thiadiazolylene, pyrrolylene, imidazolylene, pyrazolylene, triazolylene, pyridylene, pyrimidinylene, pyridazinylene, pyrazinylene, tetrazolylene, and triazinylene. Examples of bicyclic heteroarylene groups include, but are not limited to, indolylene, isoindolylene, isoindolinylene, benzofuranylene, isobenzofuranylene, benzothienylene, indazolylene, benzimidazolylene, benzoxazolylene, benzisoxazolylene, benzothiazolylene, benzisothiazolylene, benzotriazolylene, benzo[2,1,3]oxadiazolylene, benzo[2,1,3]thiadiazolylene, benzo[1,2,3]thiadiazolylene, quinolinylene, isoquinolinylene, naphthyridinylene, cinnolinylene, quinazolinylene, quinoxalinylene, phthalazinylene, oxazolopyridylene, furopyridylene, pteridylene, purinylene, pyridopyridylene, pyrazolo[1,5-a]pyridylene, pyrazolo[1,5-a]pyrimidinylene, imidazo[1,2-a]pyridylene, 1H-pyrrolo[3,2-b]pyridylene, 1H-pyrrolo[2,3-b]pyridylene, pyrrolo[2,1-b]thiazolylene, and imidazo[2,1-b]thiazolylene. Examples of tricyclic or polycyclic heteroarylene groups include, but are not limited to, acridinylene, benzindolylene, carbazolylene, dibenzofuranylene, and xanthylene. The heteroarylene group may be unsubstituted or substituted. A substituted heteroarylene refers to heteroarylene substituted one or more times (e.g., 1-4, 1-3, or 1-2 times) by a substituent(s) optionally selected from the group consisting of $C_{1-3}$ alkyl, $C_{3-6}$ cycloalkyl, hydroxy, amino, mercapto, halogen, $C_{1-3}$ alkoxy, $C_{1-3}$ alkylamino, halogenated $C_{1-3}$ alkyl, amino-substituted $C_{1-3}$ alkylene, $C_{1-3}$ alkyl-NHC(O)-, $C_{1-3}$ alkyl-C(O)NH-, cyano, or any combination thereof.

As used herein, the term "aryl", used alone or in combination, refers to a monovalent aromatic hydrocarbon group containing from 5 to 14 carbon atoms and optionally one or more fused rings, such as phenyl group, naphthyl group, or fluorenyl group. As used herein, the "aryl" is optionally substituted. A substituted aryl group refers to an aryl group substituted one or more times (e.g., 1-4, 1-3, or 1-2 times) with a substituent(s). For example, aryl is mono-, di-, or tri-substituted with a substituent(s) optionally selected from e.g., optionally deuterated $C_1$-$C_6$ alkyl, optionally deuterated $C_{3-6}$ cycloalkyl, hydroxy, amino, mercapto, halogen, cyano, oxo, optionally deuterated $C_1$-$C_6$ alkoxy, $C_1$-$C_4$ alkylamino, halogenated $C_1$-$C_6$ alkyl, amino-substituted $C_{1-6}$ alkylene, $C_{1-4}$ alkyl-NHC(O)-, $C_{1-4}$ alkyl-C(O)NH- and any combination thereof.

As used herein, the term "arylene", used alone or in combination, refers to a divalent aromatic hydrocarbon group containing from 5 to 14 carbon atoms and optionally one or more fused rings, such as phenylene, naphthylene, or fluorenylene. As used herein, the "arylene" is optionally substituted. A substituted arylene refers to an arylene group optionally substituted one or more times (e.g., 1-4, 1-3, or 1-2 times) with a substituent(s). For example, arylene is mono-, di-, or tri-substituted with a substituent(s) optionally selected from e.g., deuterium, optionally deuterated $C_1$-$C_6$ alkyl, optionally deuterated $C_{3-6}$ cycloalkyl, hydroxy, amino, mercapto, halogen, cyano, oxo, optionally deuterated $C_1$-$C_6$ alkoxy, $C_1$-$C_4$ alkylamino, halogenated $C_1$-$C_6$ alkyl, amino-substituted $C_{1-6}$ alkylene, $C_{1-4}$ alkyl-NHC(O)-, $C_{1-4}$ alkyl-C(O)NH- and any combination thereof.

As used herein, the term "cycloalkyl", used alone or in combination, refers to a saturated or partially unsaturated (i.e., containing one or more double bonds, but not having a fully conjugated $\pi$-electron system) monocyclic or bicyclic or polycyclic cyclic hydrocarbon radical, which in some embodiments contains from 3 to 20 carbon atoms (i.e., $C_{3-20}$ cycloalkyl), or from 3 to 15 carbon atoms (i.e., $C_{3-15}$ cycloalkyl), or from 3 to 12 carbon atoms (i.e., $C_{3-12}$ cycloalkyl), or from 3 to 11 carbon atoms (i.e., $C_{3-11}$ cycloalkyl), or from 3 to 10 carbon atoms (i.e., $C_{3-10}$ cycloalkyl), or from 3 to 8 carbon atoms ( i.e., $C_{3-8}$ cycloalkyl), or from 3 to 7 carbon atoms (i.e., $C_{3-7}$ cycloalkyl), or from 3 to 6 carbon atoms (i.e., $C_{3-6}$ cycloalkyl). The term "cycloalkyl" includes monocyclic, bicyclic, tricyclic or polycyclic cyclic hydrocarbon radical having from 3 to 20 carbon atoms. Representative examples of monocyclic cycloalkyl groups include, but are not limited to, cyclopropyl, cyclobutyl, cyclopentyl, cyclopentenyl, cyclohexyl, cyclohexenyl, cycloheptyl, and cyclooctyl. Bicyclic, tricyclic and polycyclic cycloalkyl groups include bridged cycloalkyl (e.g., $C_{5-20}$ bridged cycloalkyl, $C_{5-15}$ bridged cycloalkyl and $C_{7-15}$ bridged cycloalkyl), fused cycloalkyl (e.g., $C_{5-20}$ fused cycloalkyl, $C_{5-15}$ fused cycloalkyl, $C_{6-20}$ fused cycloalkyl, $C_{6-15}$ fused cycloalkyl, $C_{7-20}$ fused cycloalkyl, $C_{7-15}$ fused cycloalkyl and $C_{8-15}$ fused cycloalkyl) and spiro-cycloalkyl groups (e.g., $C_{5-20}$ spiro-cycloalkyl, $C_{5-15}$ spiro-cycloalkyl, $C_{6-20}$ spiro-cycloalkyl, $C_{6-15}$ spiro-cycloalkyl, $C_{7-20}$ spiro-cycloalkyl, $C_{7-15}$ spiro-cycloalkyl and $C_{8-15}$ spiro-cycloalkyl), representative examples of which include, but not limited to, decalinyl, octahydropentalenyl, octahydro-1H-indenyl, $C_{5-20}$ spiro-cycloalkyl, $C_{5-15}$ spiro-cycloalkyl, adamantanyl, nor-adamantanyl, bornyl, norbornyl (also named as bicyclo[2.2.1]heptyl by the IUPAC system). As used herein, the "cycloalkyl" is optionally mono- or poly-substituted, such as, but not limited to, 2,2-, 2,3-, 2,4-, 2,5-, or 2,6-disubstituted

cyclohexyl. The substituents of the substituted "cycloalkyl" can be optionally one or more (e.g., 1-5, 1-4, 1-3, 1-2, or 1) substituents selected from the group consisting of deuterium, optionally deuterated $C_1$-$C_6$ alkyl, optionally deuterated $C_{3-6}$ cycloalkyl, hydroxy, amino, mercapto, halogen, cyano, oxo, optionally deuterated $C_1$-$C_6$ alkoxy, $C_1$-$C_4$ alkylamino, halogenated $C_1$-$C_6$ alkyl, amino-substituted $C_{1-6}$ alkylene, $C_{1-4}$ alkyl-NHC(O)-, $C_{1-4}$ alkyl-C(O)NH- and any combination thereof. Examples of "$C_{3-6}$ cycloalkyl" include, but are not limited to, cyclopropyl, cyclobutyl, cyclopentyl, cyclopentenyl, cyclohexenyl, and cyclohexyl.

As used herein, the term "cycloalkylene", used alone or in combination, refers to a saturated or partially unsaturated (i.e., containing one or more double bonds, but not having a fully conjugated π-electron system) monocyclic, bicyclic, tricyclic or polycyclic divalent cyclic hydrocarbon radical containing from 3 to 20 carbon atoms (e.g., 3-15, 3-12, 3-11, 3-10, 3-8, 3-7, 3-6 carbon atoms). The term "cycloalkylene" includes monocyclic, bicyclic, tricyclic or polycyclic divalent cyclic hydrocarbon radical having from 3 to 20 (e.g., 3-15 or 3-12) carbon atoms. Representative examples of monocyclic cycloalkylene groups include, but are not limited to, cyclopropylene, cyclobutylene, cyclopentylene, cyclopentenylene, cyclohexylene, cyclohexenylene, cycloheptylene, and cyclooctylene. Bicyclic, tricyclic and polycyclic cycloalkylene groups include bridged cycloalkylene (e.g., $C_{5-20}$ bridged cycloalkylene, $C_{5-15}$ bridged cycloalkylene and $C_{7-15}$ bridged cycloalkylene), fused cycloalkylene (e.g., $C_{5-20}$ fused cycloalkylene, $C_{5-15}$ fused cycloalkylene, $C_{6-20}$ fused cycloalkylene, $C_{6-15}$ fused cycloalkylene, $C_{7-20}$ fused cycloalkylene, $C_{7-15}$ fused cycloalkylene and $C_{8-15}$ fused cycloalkylene) and spiro-cycloalkylene groups (e.g., $C_{5-20}$ spiro-cycloalkylene, $C_{5-15}$ spiro-cycloalkylene, $C_{6-20}$ spiro-cycloalkylene, $C_{6-15}$ spiro-cycloalkylene, $C_{7-20}$ spiro-cycloalkylene, $C_{7-15}$ spiro-cycloalkylene and $C_{8-15}$ spiro-cycloalkylene), representative examples of which include, but not limited to, decalinylene, octahydropentalenylene, octahydro-1H-indenylene, 2,3-dihydro-1H-indenylene, $C_{5-20}$ spiro-cycloalkylene, $C_{5-15}$ spiro-cycloalkylene, adamantanylene, noradamantanylene, and norbomylene (also named as bicyclo[2.2.1]heptylene by the IUPAC system). As used herein, the "cycloalkylene" is optionally mono- or poly-substituted, such as, but not limited to, 2,2-, 2,3-, 2,4-, 2,5-, or 2,6-disubstituted cyclohexylene. The substituents of the substituted "cycloalkylene" can be optionally one or more substituents selected from the group consisting of deuterium, optionally deuterated $C_1$-$C_6$ alkyl, optionally deuterated $C_{3-6}$ cycloalkyl, hydroxy, amino, mercapto, halogen, cyano, oxo, optionally deuterated $C_1$-$C_6$ alkoxy, $C_1$-$C_4$ alkylamino, halogenated $C_1$-$C_6$ alkyl, amino-substituted $C_{1-6}$ alkylene, $C_{1-4}$ alkyl-NHC(O)-, $C_{1-4}$ alkyl-C(O)NH- and any combination thereof.

As used herein, the term "$C_{x-y}$ spiro-cycloalkylene" (x and y each being an integer), used alone or in combination, refers to a spiro-cycloalkylene group containing from x to y carbon atoms. As used herein, the term "$C_{7-11}$ spiro-cycloalkylene", used alone or in combination, refers to a spiro-cycloalkylene group containing from 7 to 11 carbon atoms (e.g., 7-10, 7-9 carbon atoms). The term "$C_{5-20}$ spiro-cycloalkylene" includes "$C_{5-15}$ spiro-cycloalkylene", "$C_{7-15}$ spiro-cycloalkylene" and "$C_{7-20}$ spiro-cycloalkylene". Representative examples of "$C_{7-11}$ spiro-cycloalkylene" include, but are not limited to, spiro[3.3]heptylene, spiro[2.5]octylene, spiro[3.5]nonylene, spiro[4.4]nonylene, spiro[4.5]decylene, or spiro [5.5]undecylene. The term "$C_{7-11}$ spiro-cycloalkylene" is optionally substituted with one or more substituents selected from the group consisting of deuterium, optionally deuterated $C_1$-$C_6$ alkyl, optionally deuterated $C_{3-6}$ cycloalkyl, hydroxy, amino, mercapto, halogen, cyano, oxo, optionally deuterated $C_1$-$C_6$ alkoxy, $C_1$-$C_4$ alkylamino, halogenated $C_1$-$C_6$ alkyl, amino-substituted $C_{1-6}$ alkylene, $C_{1-4}$ alkyl-NHC(O)-, $C_{1-4}$ alkyl-C(O)NH- and any combination thereof.

As used herein, the term "heterocyclyl" or "heterocyclic group", used alone or in combination, refers to a 3- to 20-membered saturated or partially unsaturated (i.e., containing one or more double bonds, but not having a fully conjugated π-electron system) monocyclic, bicyclic, tricyclic or polycyclic cyclic hydrocarbon group containing one or more (e.g., from 1 to 5, or from 1 to 4, from 1 to 3, from 1 to 2, or 1) heteroatoms independently selected from sulfur, oxygen, and nitrogen. In some embodiments, "heterocyclyl" may refer to a 3- to 15-membered (e.g., 3- to 14-membered, 3- to 12-membered, 3- to 11-membered, 3- to 10-membered, 3- to 9-membered, 3- to 8-membered, 3- to 7-membered, 3- to 6-membered, 3- to 5-membered, or 4- to 9-membered) saturated or partially unsaturated (i.e., containing one or more double bonds, but not having a fully conjugated π-electron system) monocyclic cyclic hydrocarbon group containing one or more (e.g., from 1 to 5, or from 1 to 4, from 1 to 3, from 1 to 2, or 1) heteroatoms independently selected from sulfur, oxygen, and nitrogen. Examples of the heterocyclyl groups include, but not limited to, monocyclic heterocyclyl (e.g., 4- to 20-membered monocyclic heterocyclyl, and 4- to 15-membered monocyclic heterocyclyl), bridged heterocyclyl (e.g., 5- to 20-membered bridged heterocyclyl, 5- to 15-membered bridged heterocyclyl, 7- to 20-membered bridged heterocyclyl, and 7- to 15-membered bridged heterocyclyl), fused heterocyclyl (e.g., 5- to 20-membered fused heterocyclyl, and 5- to 15-membered fused heterocyclyl), spiro-heterocyclyl groups (e.g., 5- to 20-membered spiro-heterocyclyl, and 5- to 15-membered spiro-heterocyclyl), cyclic ester group (i.e., (Lactone), such as 4- to 15-membered, 4- to 14-membered, 4- to 13-membered, 4- to 12-membered, 4- to 11-membered, 4- to 10-membered, 4- to 9-membered, 4- to 8-membered, 5- to 15-membered, 5- to 10-membered, 5- to 8-membered, 6- to 15-membered, or 6- to 10-membered cyclic ester group), and cyclic amide groups (i.e. lactams group, such as 4- to 15-membered, 4- to 14-membered, 4- to 13-membered, 4- to 12-membered, 4- to 11-membered, 4- to 10-membered, 4- to 9-membered, 4- to 8-membered, 5- to 15-membered, 5- to 10-membered, 5- to 8-membered, 6- to 15-membered, or 6- to 10-membered cyclic amide groups). Representative examples of the monocyclic heterocyclyl include, but are not limited to, azetidinyl, oxetanyl, pyrrolidinyl, imidazolidinyl, pyrazolidyl, tetrahydrofuranyl,

tetrahydropyranyl, tetrahydrothienyl, tetrahydrothiopyranyl, oxazolidinyl, thiazolidinyl, piperidinyl, piperazinyl, morpholinyl, thiomorpholinyl, dioxacyclohexyl, azacycloheptyl, azacyclooctyl, diazacycloheptanyl (e.g., 1,4-diazacycloheptan-1-yl) and diazacyclooctyl. Bicyclic, tricyclic and polycyclic heterocyclyl groups include bridged heterocyclyl, fused heterocyclyl and spiro-heterocyclyl groups, representative examples of which include, but not limited to, 6-azabicyclo[3.1.1]heptan-3-yl, 2,5-diazabicyclo[2.2.1]heptan-2-yl, 3,6-diazabicyclo[3.1.1]heptan-3-yl, 3-azabicyclo[3.2.1]octan-8-yl, 3,8-diazabicyclo[3.2.1]octan-8-yl, 3,8-diazabicyclo[3.2.1]octan-3-yl, 2,5-diazabicyclo[2.2.2]octan-2-yl, and azaspirocycloalkyl (e.g., 5- to 20-membered azaspirocycloalkyl, such as 3-azaspiro[5.5]undecan-3-yl). The heterocyclyl may be unsubstituted or substituted as explicitly defined (e.g., mono-, di-, tri-, or poly-substituted) by a substituent(s) optionally selected from the group consisting of deuterium, hydroxy, amino, mercapto, nitro, halogen, cyano, oxo, optionally deuterated $C_{1-6}$ alkyl, halogenated $C_{1-6}$ alkyl, optionally deuterated $C_{3-6}$ cycloalkyl, optionally deuterated $C_{1-6}$ alkoxy, $C_{1-4}$ alkyl-NH-, $NH_2$-$C_{1-6}$ alkylene, $C_{1-4}$ alkyl-NHC(O)-, $C_{1-4}$ alkyl-C(O)NH- and any combination thereof.

As used herein, the term "nitrogen-containing monocyclic heterocyclyl", used alone or in combination, refers to 3- to 20-membered (e.g., 3- to 15-membered, 3- to 14-membered, 3- to 12-membered, 3- to 11-membered, 3- to 10-membered, 3- to 9-membered, 3- to 8-membered, 3- to 7-membered, 3- to 6-membered, 3- to 5-membered, or 4- to 9-membered) saturated or partially unsaturated (i.e., containing one or more double bonds, but not having a fully conjugated $\pi$-electron system) monocyclic monovalent cyclic hydrocarbon group containing one nitrogen atom and optionally one or more (e.g., from 1 to 5, or from 1 to 4, from 1 to 3, from 1 to 2, or 1) heteroatoms independently selected from sulfur, oxygen, and nitrogen. Representative examples of the nitrogen-containing monocyclic heterocyclyl include, but are not limited to, azetidinyl, pyrrolidinyl, imidazolidinyl, pyrazolidyl, oxazolidinyl, thiazolidinyl, piperidinyl, piperazinyl, morpholinyl, thiomorpholinyl, azacycloheptyl, azacyclooctyl, diazacycloheptanyl (e.g., 1,4-diazacycloheptan-1-yl), and diazacyclooctyl. The nitrogen-containing monocyclic heterocyclyl may be unsubstituted or substituted as explicitly defined (e.g., mono-, di-, tri-, or poly-substituted) by a substituent(s) optionally selected from the group consisting of deuterium, hydroxy, amino, mercapto, nitro, halogen, cyano, oxo, optionally deuterated $C_{1-6}$ alkyl, halogenated $C_{1-6}$ alkyl, optionally deuterated $C_{3-6}$ cycloalkyl, optionally deuterated $C_{1-6}$ alkoxy, $C_{1-4}$ alkyl-NH-, $NH_2$-$C_{1-6}$ alkylene, $C_{1-4}$ alkyl-NHC(O)-, $C_{1-4}$ alkyl-C(O)NH- and any combination thereof.

As used herein, the term "nitrogen-containing bridged heterocyclyl", used alone or in combination, refers to 3- to 20-membered (e.g., 3- to 15-membered, 3- to 14-membered, 3- to 12-membered, 3- to 11-membered, 3- to 10-membered, 3- to 9-membered, 3- to 8-membered, 3- to 7-membered, 3- to 6-membered, 3- to 5-membered, or 4- to 9-membered) saturated or partially unsaturated (i.e., containing one or more double bonds, but not having a fully conjugated $\pi$-electron system) monovalent tricyclic cyclic hydrocarbon group containing one nitrogen atom and optionally one or more (e.g., from 1 to 5, or from 1 to 4, from 1 to 3, from 1 to 2, or 1) heteroatoms independently selected from sulfur, oxygen, and nitrogen. Tricyclic heterocyclyl includes bridged heterocyclyl, e.g., but not limited to, 6-azabicyclo[3.1.1]hept-3-yl, 2,5-diazabicyclo[2.2.1]hept-2-yl, 3,6-diazabicyclo[3.1.1]hept-3-yl, 3-azabicyclo[3.2.1]octan-8-yl, 3,8-diazabicyclo[3.2.1]octan-8-yl, 3,8-diazabicyclo[3.2.1]octan-3-yl and 2,5-diazabicyclo[2.2.2]octan-2-yl. The nitrogen-containing bridged heterocyclyl may be unsubstituted or substituted as explicitly defined (e.g., mono-, di-, tri-, or poly-substituted) by a substituent(s) optionally selected from the group consisting of deuterium, hydroxy, amino, mercapto, nitro, halogen, cyano, oxo, optionally deuterated $C_{1-6}$ alkyl, halogenated $C_{1-6}$ alkyl, optionally deuterated $C_{3-6}$ cycloalkyl, optionally deuterated $C_{1-6}$ alkoxy, $C_{1-4}$ alkyl-NH-, $NH_2$-$C_{1-6}$ alkylene, $C_{1-4}$ alkyl-NHC(O)-, $C_{1-4}$ alkyl-C(O)NH- and any combination thereof.

As used herein, the term "heterocyclylene", used alone or in combination, refers to a 3- to 20-membered saturated or partially unsaturated (i.e., containing one or more double bonds, but not having a fully conjugated $\pi$-electron system) monocyclic, bicyclic, tricyclic or polycyclic bivalent cyclic hydrocarbon group containing one or more (e.g., from 1 to 5, or from 1 to 4, from 1 to 3, from 1 to 2, or 1) heteroatoms independently selected from sulfur, oxygen, and nitrogen. In some embodiments, "heterocyclylene" may refer to e.g., a 3- to 15-membered (optionally 3- to 14-membered, 3- to 12-membered, 3- to 11-membered, 3- to 10-membered, 3- to 9-membered, 3- to 8-membered, 3- to 7-membered, 3- to 6-membered, 3- to 5-membered, or 4- to 9-membered) saturated or partially unsaturated (i.e., containing one or more double bonds, but not having a fully conjugated $\pi$-electron system) monocyclic bivalent cyclic hydrocarbon group containing one or more (e.g., from 1 to 5, or from 1 to 4, from 1 to 3, from 1 to 2, or 1) heteroatoms independently selected from sulfur, oxygen, and nitrogen. Examples of the heterocyclylene groups include, but not limited to, monocyclic heterocyclylene (e.g., 4- to 20-membered monocyclic heterocyclylene, and 4- to 15-membered monocyclic heterocyclylene), bridged heterocyclylene (e.g., 5- to 20-membered bridged heterocyclylene, 5- to 15-membered bridged heterocyclylene, 7- to 20-membered bridged heterocyclylene, and 7- to 15-membered bridged heterocyclylene), fused heterocyclylene (e.g., 5- to 20-membered fused heterocyclylene, and 5- to 15-membered fused heterocyclylene), spiro-heterocyclylene groups (e.g., 5- to 20-membered spiro-heterocyclylene, and 5- to 15-membered spiro-heterocyclylene), bivalent cyclic ester group (i.e., (Lactone), such as 4- to 15-membered, 4- to 14-membered, 4- to 13-membered, 4- to 12-membered, 4- to 11-membered, 4- to 10-membered, 4- to 9-membered, 4- to 8-membered, 5- to 15-membered, 5- to 10-membered, 5- to 8-membered, 6- to 15-membered, or 6- to 10-membered bivalent cyclic ester group), and bivalent cyclic amide groups (i.e. bivalent lactams group, such as 4- to 15-membered, 4- to 14-membered, 4- to 13-membered, 4- to 12-membered, 4- to 11-membered, 4- to 10-membered, 4- to 9-membered, 4- to 8-membered, 5- to 15-membered, 5- to 10-membered, 5- to 8-membered, 6- to 15-membered, or 6- to 10-membered bivalent cyclic amide groups). Representative examples of the monocyclic heterocyclylene include, but are not limited to, azetidinylene, oxetanylene, pyrrolidinylene, imidazolidinylene, pyrazolidylene, tetrahydrofuranylene, tetrahydropyranylene, tetrahydrothienylene, tetrahydrothiopyranylene, oxazolidinylene, thiazolidinylene, piperidinylene, piperazinylene, morpholinylene, thiomorpholinylene, dioxacyclohexylene, and diazacycloheptanylene (e.g., 1,4-diazacycloheptanylene, 4,5-diazacycloheptanylene, 1,3-diazacycloheptanylene). Bicyclic, tricyclic and polycyclic heterocyclylene groups include bridged heterocyclylene, fused heterocyclylene and spiro-heterocyclylene groups, representative examples of which include, but not limited to, 6-azabicyclo[3.1.1]heptanylene, 2,5-diazabicyclo[2.2.1]heptanylene, 3,6-diazabicyclo[3.1.1]heptanylene, 3-azabicyclo[3.2.1]octanylene, 3,8-diazabicyclo[3.2.1]octanylene, 3,8-diazabicyclo[3.2.1]octanylene, 2,5-diazabicyclo[2.2.2]octanylene, and azaspirocycloalkylene (e.g., 5- to 20-membered azaspirocycloalkylene, such as 3-azaspiro[5.5]undecanylene). The heterocyclylene may be unsubstituted or substituted as explicitly defined (e.g., mono-, di-, tri-, or poly-substituted) by a substituent(s) optionally selected from the group consisting of deuterium, hydroxy, amino, mercapto, nitro, halogen, cyano, oxo, optionally deuterated $C_{1-6}$ alkyl, halogenated $C_{1-6}$ alkyl, optionally deuterated $C_{3-6}$ cycloalkyl, optionally deuterated $C_{1-6}$ alkoxy, $C_{1-4}$ alkyl-NH-, $NH_2$-$C_{1-6}$ alkylene, $C_{1-4}$ alkyl-NHC(O)-, $C_{1-4}$ alkyl-C(O)NH- and any combination thereof.

As used herein, the term "nitrogen-containing monocyclic heterocyclylene", used alone or in combination, refers to 3- to 20-membered (e.g., 3- to 15-membered, 3- to 14-membered, 3- to 12-membered, 3- to 11-membered, 3- to 10-membered, 3- to 9-membered, 3- to 8-membered, 3- to 7-membered, 3- to 6-membered, 3- to 5-membered, or 4- to 9-membered) saturated or partially unsaturated (i.e., containing one or more double bonds, but not having a fully conjugated $\pi$-electron system) monocyclic bivalent cyclic hydrocarbon group containing one nitrogen atom and optionally one or more (e.g., from 1 to 5, or from 1 to 4, from 1 to 3, from 1 to 2, or 1) heteroatoms independently selected from sulfur, oxygen, and nitrogen. Representative examples of the nitrogen-containing monocyclic heterocyclylene include, but are not limited to, piperidinylene, piperazinylene, morpholinylene, azetidinylene, pyrrolidinylene, imidazolidinylene, pyrazolidylene, oxazolidinylene, thiazolidinylene, thiomorpholinylene, azacycloheptylene, diazacycloheptanylene, azacyclooctylene, and diazacyclooctylene. The nitrogen-containing monocyclic heterocyclylene may be unsubstituted or substituted as explicitly defined (e.g., mono-, di-, tri-, or poly-substituted) by a substituent(s) optionally selected from the group consisting of deuterium, hydroxy, amino, mercapto, nitro, halogen, cyano, oxo, optionally deuterated $C_{1-6}$ alkyl, halogenated $C_{1-6}$ alkyl, optionally deuterated $C_{3-6}$ cycloalkyl, optionally deuterated $C_{1-6}$ alkoxy, $C_{1-4}$ alkyl-NH-, $NH_2$-$C_{1-6}$ alkylene, $C_{1-4}$ alkyl-NHC(O)-, $C_{1-4}$ alkyl-C(O)NH- and any combination thereof.

As used herein, the term "nitrogen-containing bridged heterocyclylene", used alone or in combination, refers to 3- to 20-membered (optionally 3- to 15-membered, 3- to 14-membered, 3- to 12-membered, 3- to 11-membered, 3- to 10-membered, 3- to 9-membered, 3- to 8-membered, 3- to 7-membered, 3- to 6-membered, 3- to 5-membered, or 4- to 9-membered) saturated or partially unsaturated (i.e., containing one or more double bonds, but not having a fully conjugated $\pi$-electron system) tricyclic bivalent cyclic hydrocarbon group containing one nitrogen atom and optionally one or more (e.g., from 1 to 5, or from 1 to 4, from 1 to 3, from 1 to 2, or 1) heteroatoms independently selected from sulfur, oxygen, and nitrogen. Tricyclic heterocyclylene groups include bridged heterocyclylene, such as but not limited to, 6-azabicyclo[3.1.1]heptanylene, 2,5-diazabicyclo[2.2.1]heptanylene, 3,6-diazabicyclo[3.1.1]heptanylene, 3-azabicyclo[3.2.1]octanylene, 3,8-diazabicyclo[3.2.1]octanylene, 3,8-diazabicyclo[3.2.1]octanylene, and 2,5-diazabicyclo[2.2.2]octanylene. The nitrogen-containing bridged heterocyclylene may be unsubstituted or substituted as explicitly defined (e.g., mono-, di-, tri-, or poly-substituted) by a substituent(s) optionally selected from the group consisting of deuterium, hydroxy, amino, mercapto, nitro, halogen, cyano, oxo, optionally deuterated $C_{1-6}$ alkyl, halogenated $C_{1-6}$ alkyl, optionally deuterated $C_{3-6}$ cycloalkyl, optionally deuterated $C_{1-6}$ alkoxy, $C_{1-4}$ alkyl-NH-, $NH_2$-$C_{1-6}$ alkylene, $C_{1-4}$ alkyl-NHC(O)-, $C_{1-4}$ alkyl-C(O)NH- and any combination thereof.

As used herein, the term "alkenylene", used alone or in combination, refers to a linear or branched divalent hydrocarbon group containing from 2 to 8 (e.g., from 2 to 6, from 2 to 5, from 2 to 4, from 2 to 3, or 2) carbon atoms and having one or more (e.g., from 1 to 3, from 1 to 2, or 1) carbon-carbon double bonds. Examples of alkenylene groups include, but are not limited to, vinylene (e.g., - CH=CH-), 1-propenylene, allylidene, 1-butenylene, 2-butenylene, 3-butenylene, isobutenylene, pentenylene, n-pent-2,4-dienylene, 1-methyl-but-1-enylene, 2-methyl-but-1-enylene, 3-methyl-but-1-enylene, 1-methyl-but-2-enylene, 2-methyl-but-2-enylene, 3-methyl-but-2-enylene, 1-methyl-but-3-enylene, 2-methyl-but-3-enylene, 3-methyl-but-3-enylene, and hexenylene.

As used herein, "bornylane" or "bornane" (also known as 1,7,7-trimethylbicyclo[2.2.1]heptane; camphane; bornylane) has a definition known to those skilled in the art. As used herein, "camphanyl" or "bornyl" refers to a monovalent group of bornane, i.e., the group remaining after any one of the hydrogens in bornane is removed. Representative examples of "bornyl" include, but are not limited to, 1,7,7-trimethylbicyclo[2.2.1]heptan-2-yl, 1,7,7-trimethylbicyclo[2.2.1]heptan-3-yl, 1,7,7-trimethylbicyclo[2.2.1]heptan-4-yl, 1,7,7-trimethylbicyclo[2.2.1]heptan-5-yl, 1,7,7-trimethylbicyclo[2.2.1]heptan-6-yl,

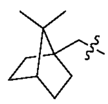 or 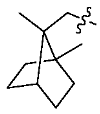 .

As used herein, "bicyclo[2.2.1]heptane" also known as "norbomane", has a definition known to those skilled in the art. As used herein, "bicyclo[2.2.1]heptyl" or "norbornyl" refers to a monovalent group of bicyclo[2.2.1]heptane, i.e., the group remaining after any hydrogen in bicyclo[2.2.1]heptane is removed. Representative examples of "bicyclo[2.2.1]heptyl" include, but are not limited to, bicyclo[2.2.1]heptan-2-yl, bicyclo[2.2.1]heptan-3-yl, bicyclo[2.2.1]heptan-4-yl, bicyclo[2.2.1]heptan-5-yl, or bicyclo[2.2.1]heptan-6-yl.

As used herein, the term "bicyclo[2.2.1]heptene" has a definition known to those skilled in the art. As used herein, "bicyclo[2.2.1]heptenyl" refers to a monovalent group of bicyclo[2.2.1]heptene, i.e., the group remaining after any hydrogen in bicyclo[2.2.1]heptene is removed. Representative examples of "bicyclo[2.2.1]heptenyl" include, but are not limited to, bicyclo[2.2.1]hept-5-en-2-yl, bicyclo[2.2.1]hept-5-en-3-yl, or bicyclo[2.2.1]hept-5-en-7-yl.

As used herein, "adamantane" (also known as tricyclo[3.3.1.1$^{3,7}$]decane) has a definition known to those skilled in the art, and its structural formula is e.g., as follows:

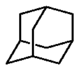 .

As used herein, "adamantanyl" refers to a monovalent group of adamantane, that is, the group remaining after any hydrogen in adamantane is removed. Representative examples of "adamantanyl" include, but are not limited to, 1-adamantanyl, 2-adamantanyl, 3-adamantanyl, 4-adamantanyl, 5-adamantanyl, 6-adamantanyl, 7-adamantanyl, 8-adamantanyl, 9-adamantanyl, or 10-adamantanyl.

As used herein, "noradamantane" (also known as octahydro-2,5-methanopentalen) has a definition known to those skilled in the art, and its structural formula is e.g., as follows:

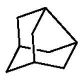 or 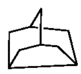 .

As used herein, "noradamantanyl" refers to a monovalent group of noradamantane, that is, the group remaining after any hydrogen in noradamantane is removed. Representative examples of "noradamantanyl" include, but are not limited to, 1-noradamantanyl, 2-noradamantanyl, 3-noradamantanyl, 4-noradamantanyl, 5-noradamantanyl, 6-noradamantanyl, 7-noradamantanyl, 8-noradamantanyl or 9-noradamantanyl.

Salts or pharmaceutically acceptable salts, enantiomers, stereoisomers, solvates, polymorphs of the compounds of Formula (I), Formula (II) or Formula (III) of the present disclosure are also encompassed within the scope of the present disclosure.

In all embodiments of the present disclosure, the salts or pharmaceutically acceptable salts of the compounds of Formula (I), Formula (II) or Formula (III) refer to non-toxic inorganic or organic acid and/or base addition salts. Examples include: sulfates, hydrohalides (including hydrochlorides and hydrobromates), maleates, sulfonates, citrates, lactates, lactobionates, L-tartrates, fumarates, L-malates, L-lactates, α-ketoglutarates, hippurates, D-glucuronates, D-gluconates, α-D-glucoheptates, glycolates, mucates, L-ascorbates, orotates, picrates, glycinates, alaninates, argininates, cinnamates, laurates, pamoates, sebacates, benzenesulfonates, methanesulfonates, ethanesulfonates, ethanedisulfonates, formates, acetates, 2,2-dichloroacetates, trimethylacetates, propionates, valerates, palmitates, triphenylacetates, 2-ethyl-butanedioates, iodates, nicotiniates, L-pyroglutamates, L-prolinates, ferulates, 2-hydroxyethanesulfonates, nitrates, gentisates, cholates, salicylate, terephthalates, glutarates, adipates, stearates, oleoates, undecylenates, camphorates, camphorsulfonates, dodecyl sulfonates, phosphates, thiocyanates, dihydrophosphates, pyrophosphates, metaphosphates, oxalates, carbonates, malonates, benzoates, mandelates, succinates, pyruvates, p-chlorobenzenesulfonates, 1,5-naphthalenedisulfonates, 3-hydroxy-2-naphthoates, 1-hydroxy-2-naphthoates, naphthalenesulfonates, glycolates, or p-toluenesulfonates, etc.

"Pharmaceutically acceptable carrier" refers to a pharmaceutically acceptable material, such as a filler, stabilizer, dispersant, suspending agent, diluent, excipient, thickener, solvent, or encapsulating material, with which the useful compounds according to the present disclosure are carried or transported into or administered to a patient so that they can perform their intended function. Generally, such constructs are carried or transported from one organ or part of the body to another organ or part of the body. The carrier is compatible with the other ingredients of the formulation, including the compounds useful in the present disclosure, and is not harmful to the patient, and the carrier must be "acceptable". Some examples of materials that can be used as pharmaceutically acceptable carriers include sugars such as lactose, glucose,

and sucrose; starches such as corn starch and potato starch; cellulose and its derivatives such as sodium carboxymethyl cellulose, ethyl cellulose and cellulose acetate; powdered tragacanth; malt; gelatin; talc; excipients such as cocoa butter and suppository wax; oils such as peanut oil, cottonseed oil, safflower oil, sesame oil, olive oil, corn oil and soybean oil; glycols such as propylene glycol; polyols such as glycerol, sorbitol, mannitol, and polyethylene glycol; esters such as ethyl oleate and ethyl laurate; agar; buffers such as magnesium hydroxide and aluminum hydroxide; surfactant phosphate buffer solution; and other common non-toxic compatible substances used in pharmaceutical formulations.

As used herein, the term "room temperature" refers to the ambient temperature, such as 20-30°C.

As used herein, "stereoisomer" refers to a compound with the same chemical structural formula, but a different arrangement of atoms or groups in space. Stereoisomers include enantiomers, diastereomers, conformational isomers (rotational isomers), geometric isomers (cis/trans isomers), atropisomers, and so on.

As used herein, the term "solvate" refers to an association or complex formed by the interaction between one or more solvent molecules and compounds of the present invention. Examples of solvents include water, isopropanol, ethanol, methanol, DMSO, ethyl acetate, acetic acid and ethanolamine. The term "hydrate" means that a complex formed with water.

As used herein, the term "chiral" refers to a molecule that is nonsuperimposable on its mirror image; whereas "achiral" refers to a molecule that can be superimposed on its mirror image.

As used herein, the term "enantiomers" refers to two isomers of a compound that are nonsuperimposable mirror images.

As used herein, the term "diastereomers" refers to stereoisomers that have two or more chiral centers but are not mirror images of each other. The diastereomers have different physical properties, such as melting point, boiling point, spectral properties and reactivity. The diastereomeric mixture can be separated by high-resolution analytical operations such as electrophoresis and chromatography, such as HPLC.

As used herein, the term "oxo", used alone or in combination, refers to = O.

As used herein, the term "C(O)" or "C(=O)" or "C=O", used alone or in combination, refers to carbonyl.

As used herein, the term "$C_{x-y}$ spiro-cycloalkyl" (x and y each being an integer), used alone or in combination, refers to a spiro-cycloalkyl group containing from x to y carbon atoms. As used herein, the term "$C_{7-11}$ spiro-cycloalkyl", used alone or in combination, refers to a spiro-cycloalkyl group containing from 7 to 11 carbon atoms. The term "$C_{5-15}$ spiro-cycloalkyl" includes "$C_{7-11}$ spiro-cycloalkyl", representative examples of which include, but are not limited to, spiro[3.3]heptyl, spiro[2.5]octyl, spiro[3.5]nonyl, spiro[4.4]nonyl, spiro[4.5]decyl, or spiro[5.5]undecyl. The "$C_{7-11}$ spiro-cycloalkyl" is optionally further substituted with one or more substituents selected from the group consisting of $C_{1-3}$ alkyl, halogenated $C_{1-3}$ alkyl, $C_{1-3}$ alkoxy, deuterated $C_{1-3}$ alkoxy, $C_{1-3}$ alkylamino, amino, oxo, halogen, hydroxy, cyano, or any combination thereof.

As used herein, the term "$C_{x-y}$ bridged cycloalkyl" (x and y each being an integer), used alone or in combination, refers to a bridged cycloalkyl group containing from x to y carbon atoms. As used herein, the term "$C_{7-11}$ bridged cycloalkyl", used alone or in combination, refers to a bridged cycloalkyl group containing from 7 to 11 carbon atoms. Representative examples of "$C_{7-11}$ bridged cycloalkyl" include, but are not limited to, adamantanyl, noradamantanyl, norbornyl (also named as bicyclo[2.2.1]heptyl by the IUPAC system), and cubanyl. The "bridged cycloalkyl" is optionally substituted with 1-10 substituents selected from the group consisting of $C_{1-3}$ alkyl, deuterated $C_{1-3}$ alkyl, $C_{1-3}$ alkoxy, halogen, halogenated $C_{1-3}$ alkyl, $C_{1-3}$ alkylamino, amino, hydroxy, cyano, oxo, or any combination thereof.

As used herein, "*p*-menthane" has the definitions known to those skilled in the art, and its structural formula is e.g., as follows:

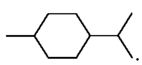

As used herein, "*p*-menthanyl" refers to a monovalent group of *p*-menthane, that is, the group remaining after any hydrogen in *p*-menthane is removed. Representative examples of "*p*-menthanyl" include, but are not limited to,

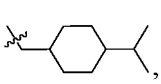 , 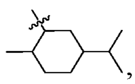 ,

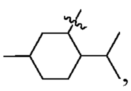 , 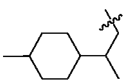 or 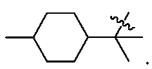 .

As used herein, "*m*-menthane" has the definitions known to those skilled in the art, and its structural formula is e.g., as follows:

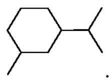.

As used herein, "*m*-menthanyl" refers to a monovalent group of *m*-menthane, that is, the group remaining after any hydrogen in *m*-menthane is removed. Representative examples of "*m*-menthanyl" include, but are not limited to,

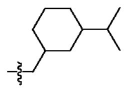, 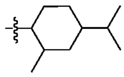,

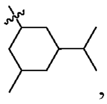, 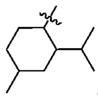, 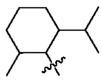, 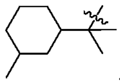, 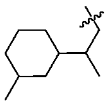 or 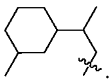.

As used herein, "Quinuclidine" (also known as 1-azabicyclo[2.2.2]octane) has the definitions known to those skilled in the art, and its structural formula is e.g., as follows:

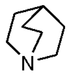.

As used herein, "quinuclidinyl" refers to a monovalent group of Quinuclidine, that is, the group remaining after any hydrogen in Quinuclidine is removed. Representative examples of "quinuclidinyl" include, but are not limited to,

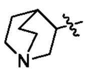, 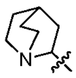, 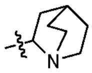, 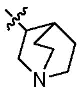, 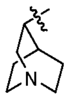, 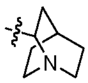, or 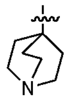.

## Examples

In the following description, numerous specific details are set forth in order to provide a thorough understanding of the present disclosure. The present disclosure may be practiced without some or all of these specific details. In other cases, well-known process operations have not been described in detail in order not to unnecessarily obscure the present disclosure. Although the present disclosure will be described in conjunction with specific embodiments, it should be understood that this is not intended to limit the present disclosure to these embodiments.

The following abbreviations are used throughout the specification and examples:

| | |
|---|---|
| AcOH | acetic acid |
| aq. | aqueous solution |
| Boc | t-Butyloxy carbonyl |
| BINAP | 2,2'-Bis(diphenylphosphino)-1,1'-binaphthalene |
| BPO | dibenzoyl peroxide |
| CAS number | CAS No.; or Chemical Abstracts Service (CAS) Registry Number |
| Con. | Concentration |
| DCM | dichloromethane |
| DIAD | Diisopropyl azodicarboxylate |
| DIEA or DIPEA | N, N-diisopropylethylamine |
| DMF | N,N-dimethylformamide |
| DMSO | dimethyl sulfoxide |
| EA | Ethyl acetate |
| EDCI | 1-(3-dimethylaminopropyl)-3-ethylcarbodiimide hydrochloride |
| ESI | electrospray ionization |
| equiv | equivalent |
| EtOH | ethanol |
| EtOAc | Ethyl acetate |
| HATU | 2-(7-Azabenzotriazol-1-yl)-N,N,N',N'-tetramethyluronium hexafluorophosphate |

| | |
|---|---|
| HPLC | high performance liquid chromatography |
| HRMS | high resolution mass spectrometry |
| LC-MS | liquid chromatography-mass spectrometry |
| LRMS | low resolution mass spectrometry |
| LC | liquid chromatography |
| Me | methyl |
| MeCN | acetonitrile |
| MeOH | Methanol |
| MS | mass spectrometry |
| NBS | N-Bromosuccinimide |
| NMO | 4-Methylmorpholine N-oxide |
| $^1$H NMR | Proton nuclear magnetic resonance |
| MeO- | methoxy |
| PE | petroleum ether |
| rt | room temperature |
| tBu | tert-butyl |
| TEA | triethylamine |
| TFA | trifluoroacetic acid |
| TLC | thin layer chromatography |
| TMS | tetramethylsilane |
| -OMs | methanesulfonyloxy |
| -ONs | 4-Nitrobenzenesulfonyl |
| -$OT_f$ | Trifluoromethanesulfonyloxy |
| -OTs | 4-Methylbenzenesulfonyloxy |

In the present disclosure, the $^1$H NMR spectrum was recorded on a Bruker-500MHz nuclear magnetic resonance instrument, by using, as a solvent, $CD_3OD$ ($\delta$ = 3.31 ppm) containing 0.1% TMS (as an internal standard); or using, as a solvent, $CDCl_3$ ($\delta$ = 7.26 ppm) containing 0.1% TMS (as an internal standard); or using, as a solvent, DMSO-$d_6$ ($\delta$ = 2.50 ppm) containing 0.03% TMS (as an internal standard). LC-MS spectrum was recorded on a Sciex API 2000 mass spectrometer equipped with an Agilent 1100 binary pump and DAD, as well as an ELSD, or on an Agilent 1260-6125B single quadrupole liquid-mass spectrometer equipped with an Agilent 1260 quadrupole pump, DAD, and ELSD; HPLC preparation was measured on a SHIMADZU LC-20AP type instrument, and HPLC purity was measured on a SHIMADZU LC-30AP or Waters 1525 type instrument. Unless otherwise specified, all reactions were performed in the air atmosphere. The reactions were monitored via TLC or LC-MS.

Solvents and reagents are processed as follows:
the solvents used in the reactions such as DCM, DMF, anhydrous EtOH, and anhydrous MeOH were all purchased from Sinopharm Group; HPLC preparation uses preparation grade $CH_3CN$ and deionized water; Other reaction substrates, reagents and drugs can be commercially available without special instructions, or can be synthesized using or according to methods known in the art.

**General synthesis methods**

Compounds and/or pharmaceutically acceptable salts thereof of the present disclosure can be synthesized using commercially available raw materials by synthetic techniques known in the art. The synthetic schemes described below illustrate the preparation of most compounds. The starting materials or reagents used in each scheme can be purchased from commercial sources or prepared by methods known to those skilled in the art. One skilled in the art can prepare the salts, racemates, enantiomers, phosphates, sulfates, hydrochlorides and prodrug forms of the compounds of Formula (I) of the present disclosure according to routine techniques in the art.

**Scheme 1:**

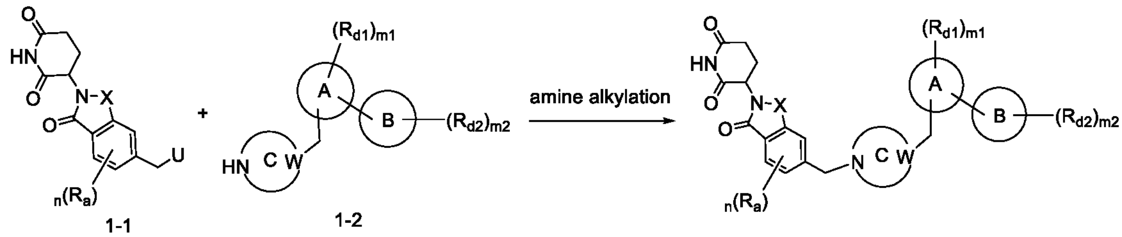


Scheme 1

In Scheme 1, ring A, $(F_{d1})_{m1}$, ring B, $(Ra_2)_{m2}$, $(R_a)_n$, and X are as defined in the compound of Formula (I) of the present disclosure above and its various sub-embodiments herein, wherein ring A preferably represents cycloalkylene, heterocyclylene, heteroarylene or arylene. The group U of substrate 1-1 represents a group capable of undergoing amine alkylation with an amino group, such as Br, Cl, I, -OMs, -OTs, or -ONs. Ring C of substrate 1-2 represents a nitrogen-containing heterocyclyl, and the W of Ring C represents CH, -CH=, or N.

The amine alkylation reaction in Scheme 1 can be carried out using conventional techniques and methods well known to those skilled in the art. For example, the amine alkylation can be carried out in the presence of DIEA and sodium iodide, or triethylamine and sodium iodide at room temperature to 80°C.

For example, the procedure of Scheme 1 can be as follows:

To a solution of substrate 1-1 (1.3 eq.) and substrate 1-2 (1.0 eq.) in DMF were added triethylamine (3.0 eq.) and sodium iodide (1.0 eq.). The reaction solution was stirred at 50 °C for 0.5-24 hours. After monitoring the reaction via LCMS and confirming its completion, the reaction solution was filtered. The filtrate was subjected to a preparative HPLC for separation and purification, yielding the target compound.

**Scheme 2:**

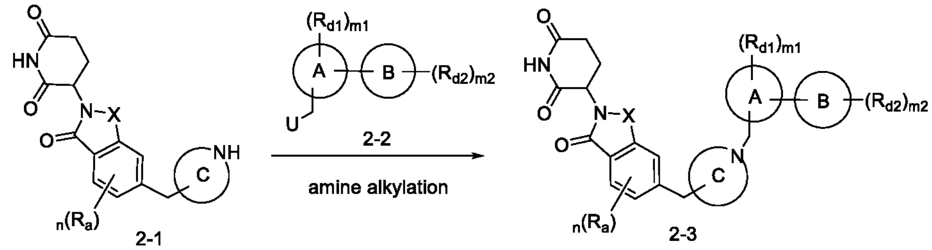


Scheme 2

In Scheme 2, ring A, $(F_{d1})_{m1}$, ring B, $(R_{d2})_{m2}$, $(R_a)_n$, and X are as defined in the compound of Formula (I) of the present disclosure above and its various sub-embodiments herein. The ring C of substrate 2-1 corresponds to the optionally substituted nitrogen-containing heterocyclyl represented by $X_1$ in the compound of formula (I) of the present disclosure. The group U of substrate 2-2 represents a group capable of undergoing amine alkylation with an amino group, such as Br, Cl, I, OMs, OTs, or ONs.

The amine alkylation reaction in Scheme 2 can be carried out using conventional techniques and methods well known to those skilled in the art. For example, the amine alkylation can be carried out in the presence of DIEA and sodium iodide, or triethylamine and sodium iodide at room temperature to 80°C.

For example, the procedure of Scheme 2 can be as follows:

To a solution of substrate 2-2 (1.3 eq.) and substrate 2-1 (1.0 eq.) in DMF were added triethylamine (3.0 eq.) and sodium iodide (1.0 eq.). The reaction solution was stirred at 50 °C for 0.5-24 hours. After monitoring the reaction via LCMS and confirming its completion, the reaction solution was filtered. The filtrate was subjected to a preparative HPLC for separation and purification, yielding the target compound.

**Scheme 3:**

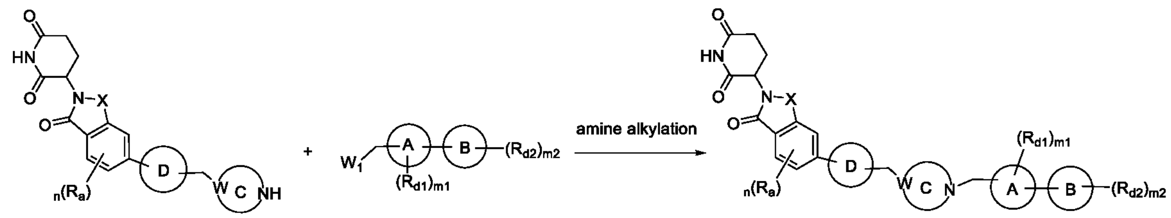


3-1 3-2 3-3

Scheme 3

In Scheme 3, ring A, $(F_{d1})_{m1}$, ring B, $(Ra_2)_{m2}$, $(R_a)_n$, and X are as defined in the compound of Formula (I) of the present disclosure above and its various sub-embodiments herein. The ring D of compound 2-1 corresponds to the optionally substituted $C_{6-10}$ arylene represented by $L_1$ of the compound of Formula (I) of the present disclosure, and the ring C corresponds to the optionally substituted nitrogen-containing heterocyclyl represented by $X_1$ of the compound of Formula (I) of the present disclosure. The W of ring C represents CH, -CH=, or N. The group $W_1$ of substrate 3-2 represents a group capable of undergoing amine alkylation with an amino group, such as Br, Cl, I, OMs, OTs, or ONs.

The amine alkylation reaction in Scheme 3 can be carried out using conventional techniques and methods well known to those skilled in the art. For example, the amine alkylation can be carried out in the presence of DIEA and sodium iodide, or triethylamine and sodium iodide at room temperature to 80°C.

For example, the procedure of Scheme 3 can be as follows:

To a solution of substrate 3-2 (1.3 eq.) and substrate 3-1 (1.0 eq.) in DMF were added triethylamine (3.0 eq.) and sodium iodide (1.0 eq.). The reaction solution was stirred at 50 °C for 0.5-24 hours. After monitoring the reaction via LCMS and confirming its completion, the reaction solution was filtered. The filtrate was subjected to a preparative HPLC for separation and purification, yielding the target compound.

**Scheme 4:**

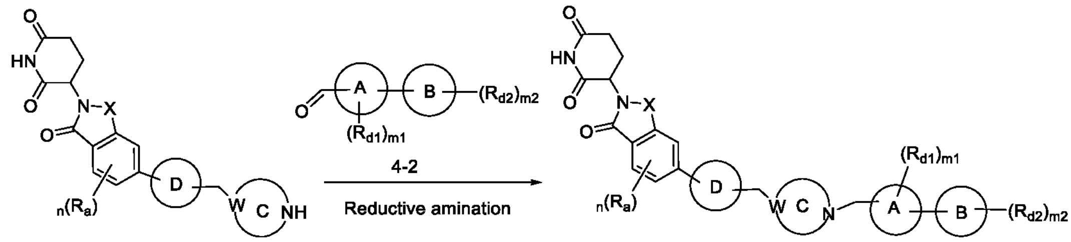


4-1 4-3

Scheme 4

In Scheme 4, ring A, $(R_{d1})_{m1}$, ring B, $(Ra_2)_{m2}$, $(R_a)_n$, and X are as defined in the compound of Formula (I) of the present disclosure above and its various sub-embodiments herein. The ring D of compound 4-1 corresponds to the optionally substituted $C_{6-10}$ arylene represented by $L_1$ of the compound of Formula (I) of the present disclosure, and the ring C corresponds to the optionally substituted nitrogen-containing heterocyclyl represented by $X_1$ of the compound of Formula (I) of the present disclosure. The W of ring C represents CH, -CH=, or N.

Depending on the desired target compounds, the reaction substrates, reaction conditions (including reaction dosage, temperature, duration, etc.), and work up in Scheme 4 can be appropriately modified and adjusted using techniques and methods well known to those skilled in the art to obtain the target compounds. For example, depending on the desired target compounds, ring D may be absent. The reductive amination reaction in Scheme 4 can be a conventional technique and method well known to those skilled in the art. For example, the reductive amination reaction can be carried out in the presence of sodium triacetoxyborohydride and 1,2-dichloroethane, or sodium cyanoborohydride and 1,2-dichloroethane at room temperature to 80 °C.

For example, the procedure of scheme 4 can be as follows:

To a solution of the aldehyde substrate 4-2 (1.3 eq.) and the substrate 4-1 (1.0 eq.) in anhydrous 1,2-dichloroethane was added acetic acid (1 drop). The reaction solution was stirred at 50 °C for 1 hour, followed by the addition of sodium borohydride acetate (2.0 eq.) to the solution. The reaction solution was stirred for another 1-24 hours. After monitoring the reaction via LCMS and confirming its completion, the reaction solution was filtered. The filtrate was subjected to a preparative HPLC for separation and purification, yielding the target compound.

**Scheme 5:**

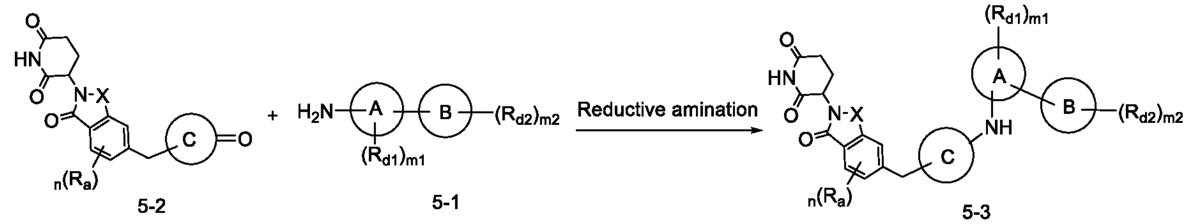


5-2 5-1 5-3

Scheme 5

In Scheme 5, ring A, $(R_{d1})_{m1}$, ring B, $(Ra_2)_{m2}$, $(R_a)_n$, and X are as defined in the compound of Formula (I) of the present disclosure above and its various sub-embodiments herein, wherein ring A is preferably a cyclic hydrocarbon group having unsaturated double bonds, such as heteroaryl, aryl, or cycloalkyl with unsaturated double bonds (e.g., cyclohexenyl). The ring C of substrate 5-2 corresponds to the optionally substituted heterocyclyl or cycloalkyl with a carbonyl group represented by $X_1$ of the compound of Formula (I) of the present disclosure.

The reductive amination reaction in Scheme 5 can be a conventional technique and method well known to those skilled in the art. For example, the reductive amination reaction can be carried out in the presence of sodium triacetoxyborohydride and 1,2-dichloroethane, or sodium cyanoborohydride and 1,2-dichloroethane at room temperature to 80 °C.

**Scheme 6:**

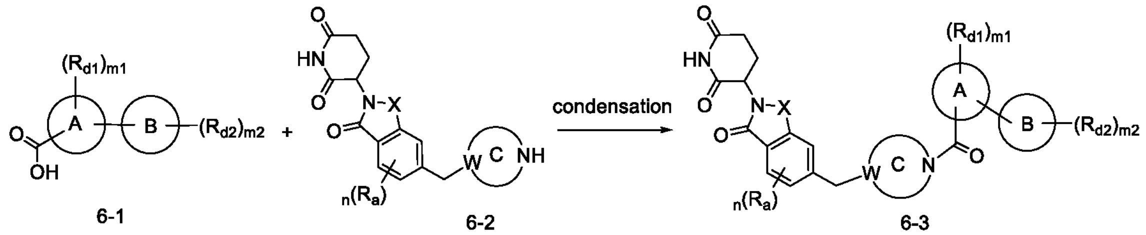


6-1 6-2 6-3

Scheme 6

In Scheme 6, ring A, $(R_{d1})_{m1}$, ring B, $(Ra_2)_{m2}$, $(R_a)_n$, and X are as defined in the compound of Formula (I) of the present disclosure above and its various sub-embodiments herein, wherein ring A is preferably a cyclic hydrocarbon group having unsaturated double bonds, such as heteroaryl, aryl, or cycloalkyl with unsaturated double bonds (e.g., cyclohexenyl). The ring C of substrate 6-2 corresponds to the optionally substituted nitrogen-containing heterocyclyl represented by $X_1$ of the compound of Formula (I) of the present disclosure. The W of ring C represents CH, -CH=, or N. the substrate 6-2 can be in the form of a free base or its salt (e.g., hydrochloride, trifluoroacetate).

The amide condensation reaction in Scheme 6 can be a conventional technique and method well-known to those skilled in the art. For example, the amide condensation reaction can be carried out in the presence of HATU, DIEA, and DMF, or HOAt, EDCI, TEA, and DCM at room temperature to 80°C.

For example, the procedure of scheme 6 can be as follows:
To a solution of the acid substrate 6-1 (1.05 eq.) and the substrate 6-2 (1.0 eq.) in DMF were added HATU (1.5 eq.) and triethylamine (3.0 eq.). The reaction solution was stirred at 50 °C for 1-24 hours. After monitoring the reaction via LCMS and confirming its completion, the reaction solution was filtered. The filtrate was subjected to a preparative HPLC for separation and purification, yielding the target compound.

**Scheme 7:**

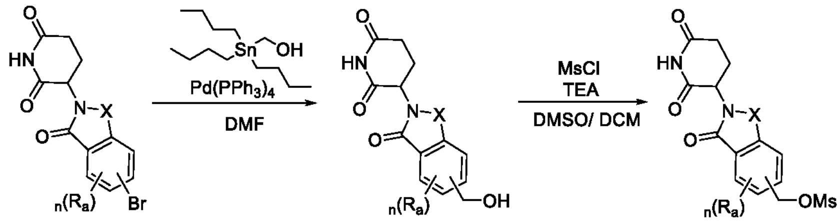


7-1 7-2 7-3

Scheme 7

In Scheme 7, Br can be located at the 4-, 5-, 6-, or 7-position on the phenyl ring of the isoindolinyl moiety, and the hydroxymethyl group of the resulting product is correspondingly located at the 4-, 5-, 6-, or 7-position on the phenyl ring of the isoindolinyl moiety. A is as defined in the compounds of Formula (I) herein. $(R_a)_n$ indicates that the phenyl ring is substituted with n $R_a$ groups, where each $R_a$ is the same or different and each independently represents deuterium, fluorine, hydroxy protected with a protective group, thiol protected with a protective group, nitro, amino protected with a protective group, cyano, optionally deuterated $C_{1-6}$ alkyl, optionally deuterated $C_{1-6}$ alkoxy, halogenated $C_{1-6}$ alkyl, optionally substituted $C_{3-6}$ cycloalkyl, $C_{2-6}$ alkenyl, or $C_{2-6}$ alkynyl; and n represents an integer of 0, 1, 2, or 3. X is as defined in the compound of Formula (I) of the present disclosure above and its various sub-embodiments herein.

The coupling reaction in Step 1 of Scheme 7 can be a conventional technique and method well-known to those skilled in the art. For example, the coupling reaction can be carried out in the presence of tetrakis(triphenylphosphine) palladium and DMF at 40°C to 100°C. The esterification reaction in Step 2 of Scheme 7 can also be a conventional technique and method well-known to those skilled in the art. Alternatively, the reactant MsCl in Step 2 can be replaced with TsCl or NsCl as needed, and the compound 7-2 can undergo esterification with TsCl or NsCl to produce the corresponding target compound with an OTs or ONs group.

For example, the procedure of Scheme 7 can be as follows:

Step 1: to a solution of the brominated substrate 7-1 (1.0 eq.) and (tributylstannyl)methanol (1.5 eq.) in DMF was added tetrakis(triphenylphosphine)palladium (0.05 eq.). Under an $N_2$ atmosphere, the reaction solution was heated to 100°C and stirred for 48 hours. After monitoring the reaction via TLC and confirming its completion, the reaction solution was cooled to 60°C, and filtered while still hot to remove the black solid. The filtrate was then concentrated under reduced pressure. To the residue was added dichloromethane, and the resulting mixture was filtered to yield the solid intermediate compound 7-2.

Step 2: to a solution of the solid intermediate compound 7-2 (1.0 eq.) in dimethyl sulfoxide/dichloromethane (1/9) were added sequentially triethylamine (30. eq.) and methanesulfonyl chloride (1.5 eq.). The reaction solution was stirred at room temperature for 1 hour. After monitoring the reaction via TLC and confirming its completion, water was added to the reaction solution. The resulting reaction mixture was filtered, and the filter cake was washed with water and dried to obtain the target compound.

**Scheme 8:**

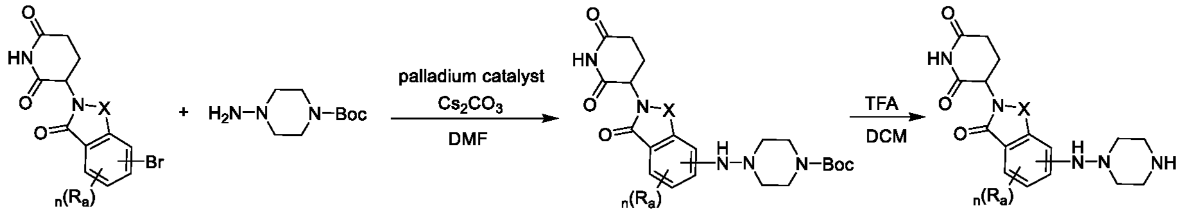


8-1 8-2 8-3 8-4

Scheme 8

In Scheme 8, Br can be located at the 4-, 5-, 6-, or 7-position on the phenyl ring of the isoindolinyl moiety. $(R_a)_n$ indicates that the phenyl ring is substituted with n $R_a$ groups, where each $R_a$ is the same or different and each independently represents deuterium, fluorine, hydroxy protected with a protective group, thiol protected with a protective group, nitro, amino protected with a protective group, cyano, optionally deuterated $C_{1-6}$ alkyl, optionally deuterated $C_{1-6}$ alkoxy, halogenated $C_{1-6}$ alkyl, optionally substituted $C_{3-6}$ cycloalkyl, $C_{2-6}$ alkenyl, or $C_{2-6}$ alkynyl; and n represents an integer of 0, 1, 2, or 3. X is as defined in the compound of Formula (I) of the present disclosure above and its various sub-embodiments herein.

The coupling reaction in Step 1 of Scheme 8 can be a conventional technique and method well-known to those skilled in the art. For example, the coupling reaction can be carried out in the presence of palladium catalyst and cesium carbonate at 40°C to 100°C.

For example, the procedure of Scheme 8 can be as follows:

Step 1: to a solution of the brominated substrate 8-1 (1.0 eq.) and substrate 8-2 (1.5 eq.) in DMF were added palladium catalyst (0.05 eq.) and cesium carbonate (2,0 eq.). Under an $N_2$ atmosphere, the reaction solution was heated to 100°C and stirred for 1-24 hours. After monitoring the reaction via TLC and confirming its completion, the reaction solution was cooled to room temperature, washed with water, and extracted with dichloromethane. The organic phases were combined, dried, filtered, and concentrated. The residue was subjected to a silica gel column chromatography for purification to obtain the intermediate product 8-3.

Step 2: to a solution of the intermediate product 8-3 obtained from the previous step in dichloromethane was added

trifluoroacetic acid. The reaction solution was stirred at room temperature for 2 hours. After monitoring the reaction via LCMS and confirming its completion, the reaction solution was concentrated under reduced pressure to obtain the target product 8-4.

## Scheme 9:

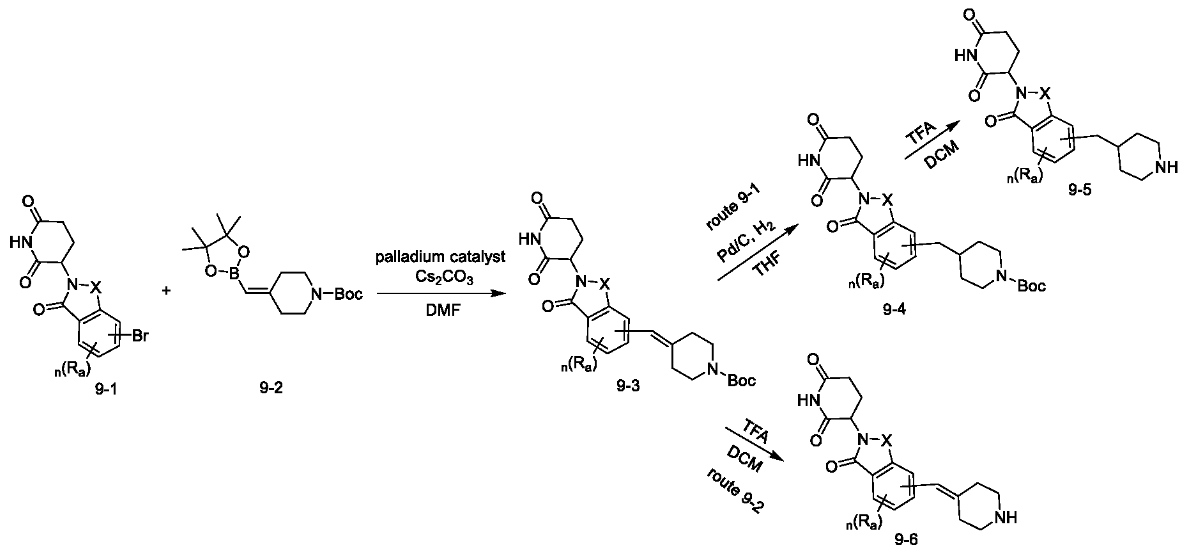


Scheme 9

In Scheme 9, Br can be located at the 4-, 5-, 6-, or 7-position on the phenyl ring of the isoindolinyl moiety. $(R_a)_n$ indicates that the phenyl ring is substituted with n $R_a$ groups, where each $R_a$ is the same or different and each independently represents deuterium, fluorine, hydroxy protected with a protective group, thiol protected with a protective group, nitro, amino protected with a protective group, cyano, optionally deuterated $C_{1-6}$ alkyl, optionally deuterated $C_{1-6}$ alkoxy, halogenated $C_{1-6}$ alkyl, optionally substituted $C_{3-6}$ cycloalkyl, $C_{2-6}$ alkenyl, or $C_{2-6}$ alkynyl; and n represents an integer of 0, 1, 2, or 3. X is as defined in the compound of Formula (I) of the present disclosure above and its various sub-embodiments herein. The Suzuki coupling reaction and hydrogenation reduction reaction in steps 1-3 of Scheme 9 can be conventional techniques and methods well-known to those skilled in the art.

For example, the procedure of Scheme 9 can be as follows:

Step 1: to a solution of the brominated substrate 9-1 (1.0 eq.) and substrate 9-2 (1.5 eq.) in DMF were added palladium catalyst (0.05 eq.) and cesium carbonate (2,0 eq.). Under an $N_2$ atmosphere, the reaction solution was heated to 100°C and stirred for 1-24 hours. After monitoring the reaction via TLC and confirming its completion, the reaction solution was cooled to room temperature, washed with water, and extracted with dichloromethane. The organic phases were combined, dried, filtered, and concentrated. The residue was subjected to a silica gel column chromatography for purification to obtain the intermediate product 9-3.

Step 2 of Route 9-1: to a solution of the intermediate product 9-3 obtained from the previous step in tetrahydrofuran was added palladium on carbon. The reaction flask was purged with hydrogen gas three times. Under a hydrogen atmosphere (1 atm), the reaction solution was heated to 50 °C and stirred for 2 hours. After monitoring the reaction via LCMS and confirming its completion, the reaction solution was filtered, and concentrated under reduced pressure to obtain the target product 9-4.

Step 3 of Route 9-1: to a solution of the intermediate product 9-4 obtained from the previous step in dichloromethane was added trifluoroacetic acid. The reaction solution was stirred at room temperature for 2 hours. After monitoring the reaction via LCMS and confirming its completion, the reaction solution was concentrated under reduced pressure to obtain the target product 9-5.

Step 2 of Route 9-2: to a solution of the intermediate product 9-3 obtained from the previous step in dichloromethane was added trifluoroacetic acid. The reaction solution was stirred at room temperature for 2 hours. After monitoring the reaction via LCMS and confirming its completion, the reaction solution was concentrated under reduced pressure to obtain the target product 9-6.

**Scheme 10:**

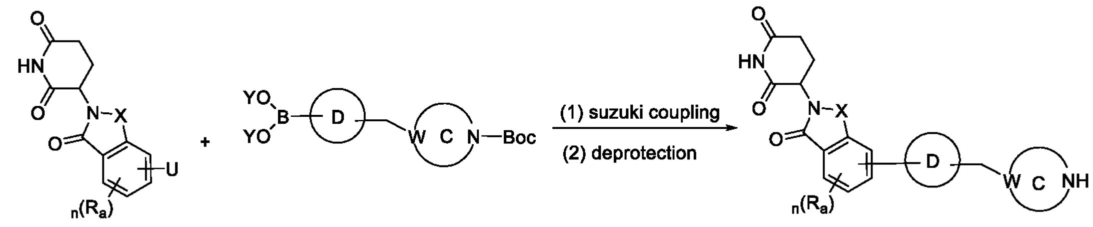


10-1 10-2 10-3

Scheme 10

In Scheme 10, $(R_a)_n$ and X are as defined in the compound of Formula (I) of the present disclosure above and its various sub-embodiments herein. The group U in the substrate 10-1 represents a group capable of undergoing Suzuki coupling with the boronic acid (ester) substrate 10-2, such as Br, Cl, I, or $OT_f$. The group Y in the boronic acid (ester) substrate 10-2 represents H or alkyl. Ring D corresponds to the optionally substituted $C_{6-10}$ arylene represented by $L_1$ in the compound of Formula (I) of the present disclosure, and ring C corresponds to the optionally substituted nitrogen-containing heterocyclyl represented by $X_1$ in the compound of Formula (I) of the present disclosure. W of the ring C represents CH, -CH=, or N. The Suzuki coupling reaction can be carried out using conventional techniques and methods well known to those skilled in the art, for example, in the presence of $Pd(dppf)Cl_2$ and $K_2CO_3$ at 60°C to 80°C.

**Scheme 11:**

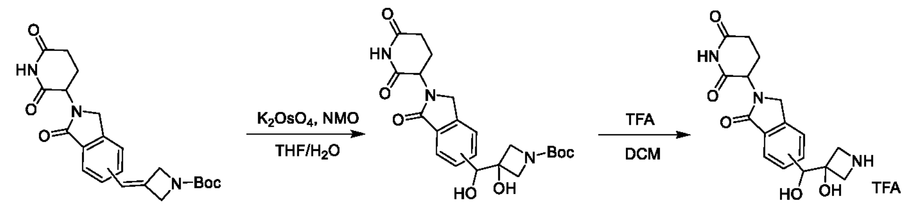


Scheme 11

**Scheme 12:**

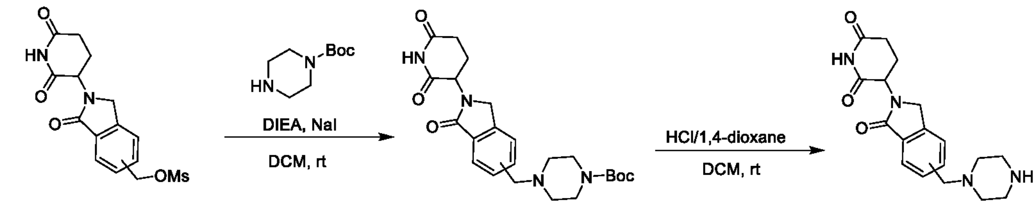


Scheme 12

**Scheme 13:**

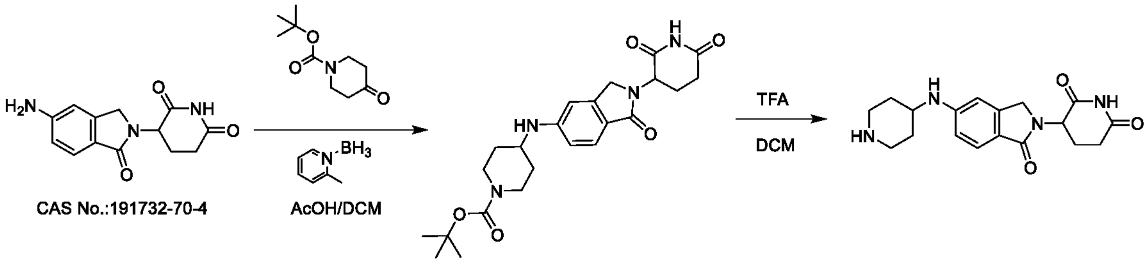


Scheme 13

**Scheme 14:**

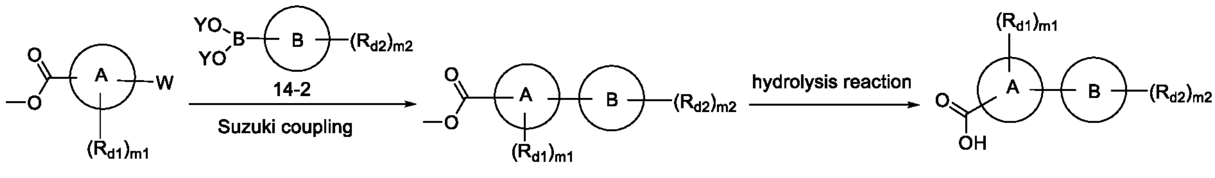

14-1 14-3 14-4

Scheme 14

In Scheme 14, ring A, $(F_{d1})_{m1}$, ring B, and $(R_{a2})_{m2}$ are as defined in the compound of Formula (I) of the present disclosure above and its various sub-embodiments herein, wherein ring A is preferably a cyclic hydrocarbon group having unsaturated double bonds, such as heteroaryl, aryl, or cycloalkyl with unsaturated double bonds (e.g., cyclohexenyl). The group W of ester substrate 14-1 represents a group capable of undergoing Suzuki coupling with the boronic acid (ester) substrate 14-2, such as Br, Cl, I, or $OT_f$. The group Y of the boronic acid (ester) substrate 14-2 represents H or alkyl.

**Scheme 15:**

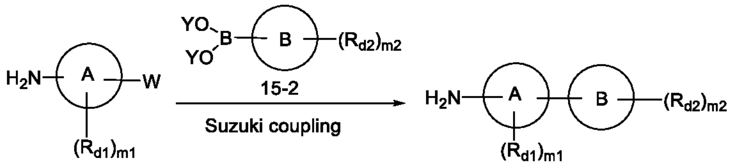

15-1 15-3

Scheme 15

In Scheme 15, ring A, $(R_{d1})_{m1}$, ring B, and $(R_{a2})_{m2}$ are as defined in the compound of Formula (I) of the present disclosure above and its various sub-embodiments herein, wherein ring A is preferably a cyclic hydrocarbon group having unsaturated double bonds, such as heteroaryl, aryl, or cycloalkyl with unsaturated double bonds (e.g., cyclohexenyl). The group W of ester substrate 15-1 represents a group capable of undergoing Suzuki coupling with the boronic acid (ester) substrate 15-2, such as Br, Cl, I, or $OT_f$. The group Y of the boronic acid (ester) substrate 15-2 represents H or alkyl.

In some embodiments, the amino group of the amine substrate 15-1 can be protected with a protecting group (e.g., Boc), and the resulting product 15-3 can also be further deprotected. Deprotection can be carried out under conditions of HCl and ethyl acetate, or TFA and DCM.

**Scheme 16:**

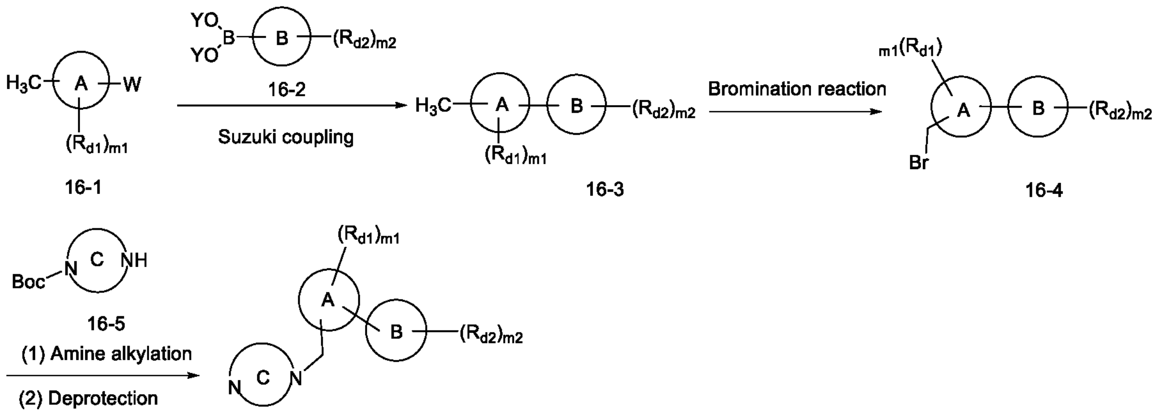

16-1 16-3 16-4 16-6

Scheme 16

In Scheme 16, ring A, $(R_{d1})_{m1}$, ring B, and $(R_{a2})_{m2}$ are as defined in the compound of Formula (I) of the present disclosure above and its various sub-embodiments herein, wherein ring A preferably represents cycloalkylene, heterocyclylene, heteroarylene or arylene. The group W of the substrate 16-1 represents a group capable of undergoing Suzuki coupling with the boronic acid (ester) substrate 16-2, such as Br, Cl, I, or $OT_f$. The group Y of the boronic acid (ester)

substrate 16-2 represents H or alkyl. The ring C of substrate 16-5 represents the optionally substituted nitrogen-containing heterocyclyl, and corresponds to the group $X_1$ in the compound of Formula (I) of the present disclosure.

**Scheme 17:**

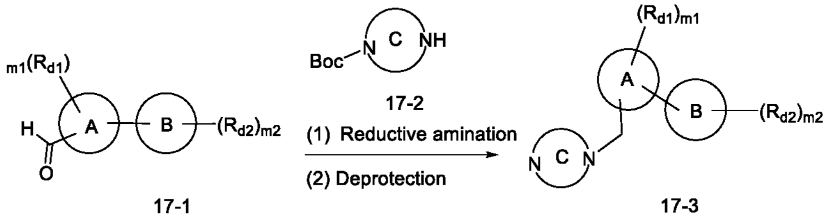


Scheme 17

In Scheme 17, ring A, $(R_{d1})_{m1}$, ring B, and $(R_{a2})_{m2}$ are as defined in the compound of Formula (I) of the present disclosure above and its various sub-embodiments herein, wherein ring A preferably represents cycloalkylene, heterocyclylene, heteroarylene or arylene. The ring C of substrate 17-3 represents the optionally substituted nitrogen-containing heterocyclyl, and corresponds to the group $X_1$ in the compound of Formula (I) of the present disclosure.

The reductive amination reaction in step 1 and deprotection in step 2 of Scheme 17 can be a conventional technique and method well-known to those skilled in the art. For example, the reductive amination reaction can be carried out in the presence of sodium triacetoxyborohydride and 1,2-dichloroethane at room temperature to 80 °C. Deprotection can be carried out in the presence of TFA and DCM, or HCl and ethyl acetate.

For example, the procedure of Scheme 17 can be as follows:

Step 1: to a solution of the aldehyde substrate 17-1 (1.0 eq.) and the corresponding amine substrate 17-2 (1.0 eq.) in 1,2-dichloroethane was added sodium triacetoxyborohydride (2.0 eq.). The reaction solution was heated to 80 °C and stirred for 2 hours. After monitoring the reaction via TLC and confirming its completion, the reaction solution was cooled to room temperature. The reaction was quenched with water, and the reaction mixture was extracted with dichloromethane. The organic phases were combined, dried over anhydrous sodium sulfate, and filtered. The filtrate was concentrated, and the residue was subjected to a silica gel column chromatography for purification to obtain the product.

Step 2: to a solution of the product obtained from the previous step in dichloromethane was added trifluoroacetic acid. The reaction solution was stirred at room temperature for 2 hours. After monitoring the reaction via LCMS and confirming its completion, the reaction solution was concentrated under reduced pressure to obtain the target product.

**Scheme 18:**

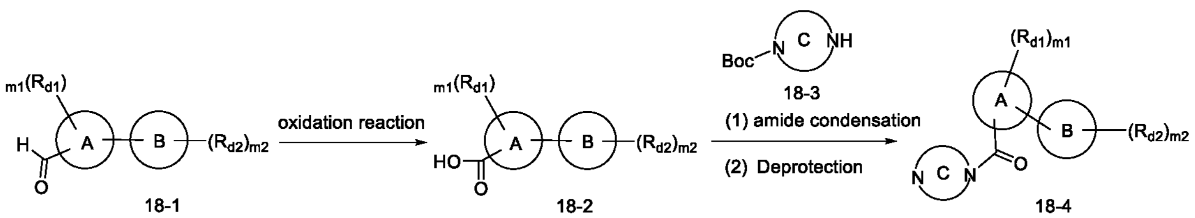


Scheme 18

In Scheme 18, ring A, $(R_{d1})_{m1}$, ring B, and $(R_{d2})_{m2}$ are as defined in the compound of Formula (I) of the present disclosure above and its various sub-embodiments herein, wherein ring A preferably represents cycloalkylene, heterocyclylene, heteroarylene or arylene. The ring C of the substrate 18-3 represents the optionally substituted nitrogen-containing heterocyclyl, and corresponds to the group $X_1$ in the compound of Formula (I) of the present disclosure.

The oxidation reaction in Step 1 of Scheme 18 can be carried out using conventional techniques and methods well known to those skilled in the art. For example, the oxidation reaction can be performed in the presence of sodium hypochlorite, sodium dihydrogen phosphate dihydrate, and hydrogen peroxide. The amide condensation reaction and deprotection in Step 2 of Scheme 18 can also be carried out using conventional techniques and methods well known to those skilled in the art. For instance, the amide condensation reaction can be conducted in the presence of HATU and DIEA at room temperature to 80°C. The deprotection can be carried out in the presence of TFA and DCM.

For example, the procedure of Scheme 18 can be as follows:

Step 1: to a solution of the aldehyde substrate 18-1 (1.0 eq.) in acetonitrile and water were added sequentially sodium hypochlorite (1.5 eq.), sodium dihydrogen phosphate dihydrate (0.2 eq.), and hydrogen peroxide (10 eq.). The reaction solution was stirred at room temperature for 16 hours. After monitoring the reaction via TLC and confirming its completion, saturated sodium carbonate solution was added to the reaction solution, and the resulting mixture was extracted with petroleum ether. The aqueous phase was adjusted to pH 6 with dilute hydrochloric acid, and then extracted with dichloromethane. The organic phases were combined, dried over anhydrous sodium sulfate, and filtered. The filtrate was concentrated to obtain the intermediate acid product 18-2.

Step 2(1), amide condensation reaction: to a solution of the acid 18-2 obtained from Step 1 (1.0 eq.) and the amine substrate 18-3 (1.2 eq.) in N,N-dimethylformamide were added triethylamine (3.0 eq.) and HATU (1.5 eq.). The reaction solution was stirred at room temperature for 3 hours. After monitoring the reaction via TLC and confirming its completion, the reaction solution was washed with water, and extracted with dichloromethane. The organic phases were combined, dried over anhydrous sodium sulfate, and filtered. The filtrate was concentrated under reduced pressure to obtain the intermediate product.

Step 2(2), deprotection: to a solution of the intermediate product obtained from Step 2(1) in dichloromethane was added trifluoroacetic acid. The reaction solution was stirred at room temperature for 2 hours. After monitoring the reaction via LCMS and confirming its completion, the reaction solution was concentrated under reduced pressure to obtain the target product 18-4.

Scheme 19:

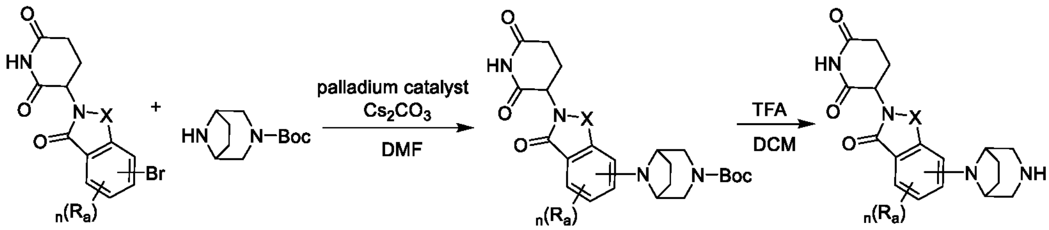


19-1 19-2 19-3

Scheme 19

In Scheme 19, Br can be located at the 4-, 5-, 6-, or 7-position on the phenyl ring of the isoindolinyl moiety. $(R_a)_n$ indicates that the phenyl ring is substituted with n $R_a$ groups, where each $R_a$ is the same or different and each independently represents deuterium, fluorine, hydroxy protected with a protective group, thiol protected with a protective group, nitro, amino protected with a protective group, cyano, optionally deuterated $C_{1-6}$ alkyl, optionally deuterated $C_{1-6}$ alkoxy, halogenated $C_{1-6}$ alkyl, optionally substituted $C_{3-6}$ cycloalkyl, $C_{2-6}$ alkenyl, or $C_{2-6}$ alkynyl; and n represents an integer of 0, 1, 2, or 3. X is as defined in the compound of Formula (I) of the present disclosure above and its various sub-embodiments herein. The coupling reaction and deprotection reaction in Steps 1-2 of Scheme 19 can be a conventional technique and method well-known to those skilled in the art.

For example, the procedure of Scheme 19 can be as follows:

Step 1: to a solution of the brominated substrate 19-1 (1.0 eq.) and tert-butyl 3,8-diazabicyclo[3.2.1]octane-3-carboxylate (1.5 eq.) in DMF were added palladium catalyst (0.05 eq.) and cesium carbonate (2,0 eq.). Under an $N_2$ atmosphere, the reaction solution was heated to 100°C and stirred for 1-24 hours. After monitoring the reaction via TLC and confirming its completion, the reaction solution was cooled to room temperature, washed with water, and extracted with dichloromethane. The organic phases were combined, dried, filtered, and concentrated. The residue was subjected to a silica gel column chromatography for purification to obtain the intermediate product 19-2.

Step 2: to a solution of the intermediate product 19-2 obtained from the previous step in dichloromethane was added trifluoroacetic acid. The reaction solution was stirred at room temperature for 2 hours. After monitoring the reaction via LCMS and confirming its completion, the reaction solution was concentrated under reduced pressure to obtain the target product 19-3.

Scheme 20:

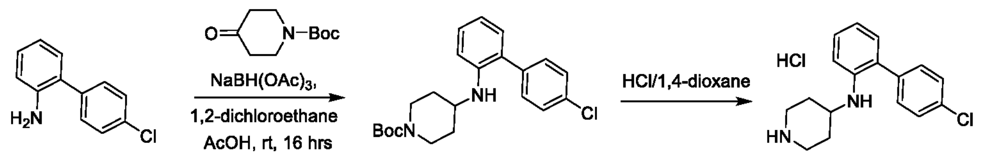


Scheme 20

Scheme 21:

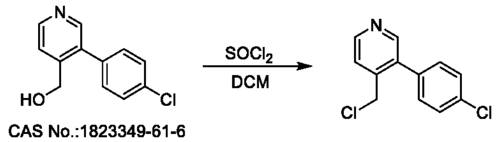


Scheme 21

Depending upon the target compounds, the various schemes mentioned above and their reaction substrates, reaction conditions (including reaction dosage, temperature, duration, etc.), work up, etc. can be appropriately modified and adjusted by techniques and methods well known to those skilled in the art to obtain the desired target compounds. The obtained target compounds can be further modified by the substituents and the like to obtain subsequent target compounds through methods well known to those skilled in the art.

## Examples

### Intermediate example 1: Preparation of 3-(5-(bromomethyl)-1-oxoisoindolin-2-yl)piperidine-2,6-dione (GT-03234)

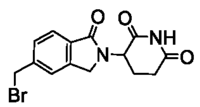

To a solution of methyl 2,5-dimethylbenzoate (3.4 g, 20.6 mmol) in 100 mL of carbon tetrachloride were added sequentially N-bromosuccinimide (8.0 g, 45 mmol) and benzoyl peroxide (0.31 g, 1.26 mmol). The reaction mixture was refluxed at 80°C for 12 hours, then cooled to room temperature, and diluted with 100 mL of petroleum ether. The resulting mixture was washed twice with water and once with saturated saline solution. The organic phase was dried over anhydrous sodium sulfate, and distilled under reduced pressure to remove organic solvent. The residue was subjected to a silica gel column chromatography (eluent (v/v): EA/PE = 0/1 - 1/20) for purification to yield the intermediate compound methyl 2,5-bis(bromomethyl)benzoate as a white solid (5.0 g, yield: 76%).

To a mixed solvent of 50 mL of 1,2-dichloroethane and 5 mL of hexafluoroisopropanol was added Cesium carbonate (1.6 g, 5 mmol). The reaction mixture was stirred at 65°C for 15 minutes, followed by sequential addition of 3-amino-2,6-piperidinedione hydrochloride (8.21 g, 5 mmol) and methyl 2,5-bis(bromomethyl)benzoate (1.6 g, 5 mmol) and continued stirring at 65°C for 12 hours. After the reaction was completed, the reaction mixture was cooled to room temperature and washed with water. The organic phase was separated, and distilled under reduced pressure to remove organic solvent. The residue was subjected to a silica gel column chromatography (eluent (v/v): MeOH/DCM = 0/1 - 1/10) for separation. The obtained product was slurried in acetonitrile for further purification to yield a white powder (455mg, yield: 27%). $^1$H NMR (500 MHz, DMSO-$d_6$) $\delta$ 11.00 (s, 1H), 7.78 - 7.66 (m, 2H), 7.59 (dd, $J$ = 7.9, 1.5 Hz, 1H), 5.11 (dd, $J$ = 13.3, 5.1 Hz, 1H), 4.83 (s, 2H), 4.47 (d, $J$ = 17.3 Hz, 1H), 4.34 (d, $J$ = 17.4 Hz, 1H), 2.91 (ddd, $J$ = 17.3, 13.6, 5.4 Hz, 1H), 2.68 - 2.55 (m, 1H), 2.39 (qd, $J$ = 13.3, 4.5 Hz, 1H), 2.01 (dtd, $J$ = 12.7, 5.3, 4.7, 1.9 Hz, 1H). LCMS (ESI) m/z: calcd for $C_{14}H_{14}BrN_2O_3^+$ [M+H]$^+$, 337.0; found, 337.3.

### Intermediate example 2: Preparation of 4'-fluoro-5,5-dimethyl-3,4,5,6-tetrahydro-[1,1'-biphenyl]-2-carbaldehyde (GTC00947)

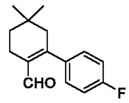

Step 1: 2-chloro-4,4-dimethylcyclohex-1-ene-1-carbaldehyde

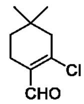

To a solution of DMF (4.9 mL, 63.4 mmol) in DCM (100 mL) was added dropwise $POCl_3$ (5.5 mL, 59.4 mmol) at -10 °C. After the addition was complete, the reaction solution was allowed to warm to room temperature, followed by addition dropwise of 3,3-dimethylcyclohex-1-one (5.5 mL, 39.6 mmol). The reaction solution was refluxed for 18 hours. After monitoring the reaction via TLC and confirming its completion, the reaction solution was cooled to room temperature. The reaction was quenched with saturated $NaHCO_3$ solution. The reaction solution was washed with water (100 mL) and extracted with DCM (100 mL × 3). The combined organic phases were washed with saturated saline solution (50 mL × 1), dried over anhydrous sodium sulfate, and filtered. The filtrate was concentrated under reduced pressure, and the residue was subjected to a column chromatography (eluent: petroleum ether/ethyl acetate = 50/1) for purification to yield the colorless oily compound 2-chloro-4,4-dimethylcyclohex-1-en-1-carbaldehyde (5.9 g, yield: 86%). LCMS (ESI): calcd for $C_9H_{14}ClO^+$ $[M+H]^+$, 173.07, found, 173.3.

Step 2: Preparation of 4'-fluoro-5,5-dimethyl-3,4,5,6-tetrahydro-[1,1'-biphenyl]-2-carbaldehyde

To a solution of the 2-chloro-4,4-dimethylcyclohex-1-en-1-carbaldehyde (1.5 g, 8.7 mmol) obtained from Step 1 and 4-fluorophenylboronic acid (1.8 g, 13.1 mmol) in dioxane (30 mL) and $H_2O$ (3 mL) were added sequentially Pd(dppf)$Cl_2$ (0.32 g, 0.43 mmol) and $K_2CO_3$ (2.4 g, 17.4 mmol). The reaction flask was purged with $N_2$ three times, and the reaction mixture was heated to 90 °C and stirred for 18 hours. After monitoring the reaction via TLC and confirming its completion, the reaction solution was concentrated, and the residue was washed with water (100 mL) and extracted with ethyl acetate (100 mL × 3). The combined organic phases were washed with saturated saline solution (50 mL × 1), dried over anhydrous sodium sulfate, and filtered. The filtrate was concentrated under reduced pressure, and the resulting residue was subjected to a column chromatography (eluent: petroleum ether) for purification to yield the pale yellow oily compound 4'-fluoro-5,5-dimethyl-3,4,5,6-tetrahydro-[1,1'-biphenyl]-2-carbaldehyde (GTC00947) (1.2 g, yield: 59%). $^1H$ NMR (400 MHz, DMSO) δ 9.39 (s, 1H), 7.39 - 7.31 (m, 2H), 7.25 (ddd, *J* = 8.8, 5.6, 2.4 Hz, 2H), 2.32 (d, *J* = 2.0 Hz, 2H), 2.30 - 2.23 (m, 2H), 1.44 (t, *J* = 6.4 Hz, 2H), 0.97 (s, 6H). LCMS (ESI): calcd for $C_{15}H_{18}FO^+$ $[M+H]^+$, 233.13, found, 233.1.

**Intermediate example 3: Preparation of 1-((4'-chloro-[1,1'-biphenyl]-2-yl)methyl)piperazine**

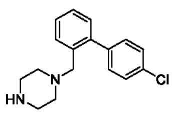

1-((4'-chloro-[1,1'-biphenyl]-2-yl)methyl)piperazine was prepared by referring to the method of Scheme 7.

Step 1: Preparation of tert-butyl 4-((4'-chloro-[1,1'-biphenyl]-2-yl)methyl)piperazine-1-carboxylate

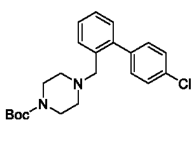

To a solution of 2-(bromomethyl)-4'-chloro-1,1'-biphenyl (CAS No.: 1001754-57-9) (0.8 g, 2.8 mmol) and N-Boc-piperazine (0.8 g, 4.3 mmol) in DCM (20 mL) was added TEA (1 mL, 5.7 mmol). The reaction mixture was stirred at room temperature for 2 hours. After monitoring the reaction via TLC and confirming its completion, the reaction solution was washed with water (50 mL) and extracted with DCM (50 mL × 3). The combined organic phases were washed with saturated saline solution (50 mL × 1), dried over anhydrous sodium sulfate, and filtered. The filtrate was concentrated under reduced pressure, and the residue was subjected to column chromatography (eluent: petroleum ether/ethyl acetate = 4/1) for purification to afford the white solid compound tert-butyl 4-((4'-chloro-[1,1'-biphenyl]-2-yl)methyl)piperazine-1-carboxylate (780 mg, yield: 71%). LC/MS (ESI) m/z: calcd for $C_{22}H_{28}ClN_2O_2^+$ [M+H]$^+$, 387.2; found, 387.2.

Step 2: Preparation of 1-((4'-chloro-[1,1'-biphenyl]-2-yl)methyl)piperazine

To a solution of tert-butyl 4-((4'-chloro-[1,1'-biphenyl]-2-yl)methyl)piperazine-1-carboxylate (780 mg, 2.0 mmol), obtained from Step 3, in dichloromethane (20 mL) was added HCl/dioxane (5 mL, 4M). The reaction mixture was stirred at room temperature for 2 hours. After monitoring the reaction via LCMS and confirming its completion, the reaction solution was concentrated under reduced pressure to afford the white solid compound 1-((4'-chloro-[1,1'-biphenyl]-2-yl)methyl) piperazine (650 mg, yield: 100%). LC/MS (ESI) m/z: calcd for $C_{17}H_{20}ClN_2^+$ [M+H]$^+$, 287.2; found, 287.2.

**Intermediate example 4: Preparation of 3-(1-oxo-5-(4-(piperazin-1-ylmethyl)phenyl)isoindolin-2-yl)piperidine-2,6-dione**

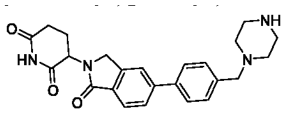

3-(1-oxo-5-(4-(piperazin-1-ylmethyl)phenyl)isoindolin-2-yl)piperidine-2,6-dione was prepared by referring to the method of step 1 of Scheme 9.

Step 1: Preparation of tert-butyl 4-(4-(2-(2,6-dioxopiperidin-3-yl)-1-oxoisoindolin-5-yl)benzyl)piperazine-1-carboxylate

To a solution of 3-(5-bromo-1-oxoisoindolin-2-yl)piperidine-2,6-dione (CAS No.: 1010100-26-1) (1 g, 3.1 mmol) and (4-((4-(tert-butoxycarbonyl)piperazin-1-yl)methyl)phenyl)boronic acid (CAS No.: 1190095-10-3) (1 g, 3.1 mmol) in dioxane (20 mL) were added sequentially Pd(dppf)$Cl_2$ (0.23 g, 0.31 mmol) and $K_2CO_3$ (0.86 g, 6.2 mmol). The reaction flask was purged with $N_2$ three times, and the reaction solution was heated to 90 °C and stirred for 18 hours. After monitoring the reaction via TLC and confirming its completion, the reaction solution was filtered. The filtrate was concentrated under reduced pressure, and the residue was washed with water (100 mL) and extract with DCM (100 mL × 3). The combined organic phases were washed with saturated saline solution (50 mL × 1), dried over anhydrous sodium sulfate, and filtered. The filtrate was concentrated under reduced pressure, and the resulting residue was subjected to column chromatography (eluent: DCM/MeOH = 20/1) for purification to obtain the pale yellow solid compound tert-butyl 4-(4-(2-(2,6-dioxopiperidin-3-yl)-1-oxoisoindolin-5-yl)benzyl)piperazine-1-carboxylate (810 mg, yield: 51%). LC/MS (ESI) m/z: calcd for $C_{29}H_{35}N_4O_5^+$ [M+H]$^+$, 519.3;found, 519.3.

Step 2: Preparation of 3-(1-oxo-5-(4-(piperazin-1-ylmethyl)phenyl)isoindolin-2-yl)piperidine-2,6-dione

To a solution of tert-butyl 4-(4-(2-(2,6-dioxopiperidin-3-yl)-1-oxoisoindolin-5-yl)benzyl)piperazine-1-carboxylate (810 mg, 1.56 mmol), obtained from Step 1, in dichloromethane (20 mL) was added a solution of HCl in dioxane (4 mL, 4M). The reaction solution was stirred at room temperature for 2 hours. After monitoring the reaction via LCMS and confirming its completion, the reaction solution was concentrated under reduced pressure to obtain the white solid compound 3-(1-oxo-5-(4-(piperazin-1-ylmethyl)phenyl)isoindolin-2-yl)piperidine-2,6-dione (740 mg, yield: 104%). LC/MS (ESI) m/z: calcd for $C_{24}H_{27}N_4O_3^+$ [M+H]$^+$, 419.2; found, 419.2.

**Intermediate example 5: Preparation of (2-(2,6-dioxopiperidin-3-yl)-1-oxoisoindolin-5-yl)methyl methanesulfonate**

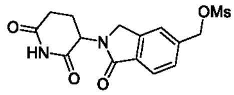

Referring to the method of Scheme 7, (2-(2,6-dioxopiperidin-3-yl)-1-oxoisoindolin-5-yl)methyl methanesulfonate was prepared as grayish white solid (20.0 g, yield: 86%). LC/MS (ESI) m/z: calcd for $C_{15}H_{17}N_2O_6S^+$ [M+H]$^+$, 353.08; found, 353.1.

**Intermediate example 6: Preparation of (2-(2,6-dioxopiperidin-3-yl)-4-fluoro-1-oxoisoindolin-5-yl)methyl methanesulfonate**

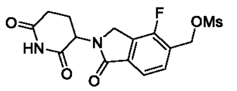

Referring to the method of Scheme 7, (2-(2,6-dioxopiperidin-3-yl)-4-fluoro-1-oxoisoindolin-5-yl)methyl methanesulfonate was prepared as pale yellow solid (1.1 g, yield: 86%). LC/MS (ESI) m/z: calcd for $C_{15}H_{16}FN_2O_6S^+$ [M+H]$^+$, 371.07; found, 371.1.

**Intermediate example 7: Preparation of (2-(2,6-dioxopiperidin-3-yl)-6-fluoro-1-oxoisoindolin-5-yl)methyl methanesulfonate**

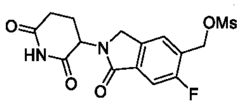

Referring to the method of Scheme 7, (2-(2,6-dioxopiperidin-3-yl)-6-fluoro-1-oxoisoindolin-5-yl)methyl methanesulfonate was prepared as pale yellow solid (1.1 g, yield: 86%). LC/MS (ESI) m/z: calcd for $C_{15}H_{16}FN_2O_6S^+$ [M+H]$^+$, 371.07; found, 371.1.

**Intermediate example 8: Preparation of (2-(2,6-dioxopiperidin-3-yl)-7-fluoro-1-oxoisoindolin-5-yl)methyl methanesulfonate**

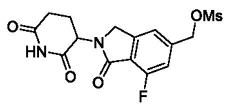

Referring to the method of Scheme 7, (2-(2,6-dioxopiperidin-3-yl)-7-fluoro-1-oxoisoindolin-5-yl)methyl methanesulfonate was prepared as grayish white solid (6.5 g, yield: 82%). LC/MS (ESI) m/z: calcd for $C_{15}H_{16}FN_2O_6S^+$ [M+H]$^+$, 371.07; found, 371.1.

**Intermediate example 9: Preparation of (2-(2,6-dioxopiperidin-3-yl)-1-oxoisoindolin-4-yl)methyl methanesulfonate**

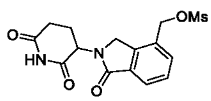

Referring to the method of Scheme 7, (2-(2,6-dioxopiperidin-3-yl)-1-oxoisoindolin-4-yl)methyl methanesulfonate was prepared as grayish white solid (4.5 g, yield: 85%). LC/MS (ESI) m/z: calcd for $C_{15}H_{17}N_2O_6S^+$ [M+H]$^+$, 353.08; found, 353.1.

**Intermediate example 10: Preparation of 3-(1-oxo-5-(piperidin-4-ylmethyl)isoindolin-2-yl)piperidine-2,6-dione (GT-3423)**

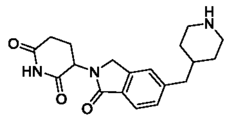

Referring to the method of Scheme 9, 3-(1-oxo-5-(piperidin-4-ylmethyl)isoindolin-2-yl)piperidine-2,6-dione was prepared as brown solid (3.2 g, yield: 83%). $^1$H NMR (400 MHz, DMSO) δ 10.98 (s, 1H), 9.01 - 8.55 (m, 2H), 7.73 - 7.60 (m, 1H), 7.51 - 7.41 (m, 1H), 7.35 (t, J = 7.4 Hz, 1H), 5.11 (dd, J = 13.2, 5.1 Hz, 1H), 4.38 (dt, J = 32.5, 16.6 Hz, 2H), 3.21 (d, J = 12.4 Hz, 2H), 2.92 (dt, J = 13.9, 6.4 Hz, 1H), 2.77 (dd, J = 22.7, 11.4 Hz, 2H), 2.64 (dd, J = 26.0, 11.0 Hz, 3H), 2.46 - 2.36 (m, 1H), 2.07 - 1.77 (m, 3H), 1.76 - 1.64 (m, 2H), 1.48 - 1.28 (m, 2H). LC/MS (ESI) m/z: calcd for $C_{19}H_{23}N_3O_3^+$ [M+H]$^+$, 342.17; found, 342.2.

**Intermediate example 11: Preparation of 3-(1-oxo-5-(piperazin-1-ylamino)isoindolin-2-yl)piperidine-2,6-dione**

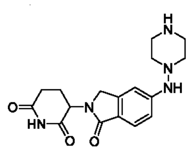

Referring to the method of Scheme 8, 3-(1-oxo-5-(piperazin-1-ylamino)isoindolin-2-yl)piperidine-2,6-dione was prepared as light brown solid (1.6 g, yield: 91%). $^1$H NMR (400 MHz, DMSO) δ 11.09 (s, 1H), 8.90 (s, 2H), 7.37 (d, *J* = 6.5 Hz, 2H), 5.09 (dd, *J* = 13.0, 5.3 Hz, 1H), 3.77 (s, 4H), 3.44 (s, 4H), 3.31 (s, 4H), 3.21 (s, 4H), 2.89 (ddd, *J* = 17.5, 14.2, 5.4 Hz, 1H), 2.64 - 2.52 (m, 2H), 2.01 (dd, *J* = 10.8, 5.7 Hz, 1H). LC/MS (ESI) m/z: calcd for $C_{17}H_{21}N_5O_3^+$ [M+H]$^+$, 344.2; found, 344.2.

**Intermediate example 12: Preparation of 3-(5-(azetidin-3-ylamino)-6-fluoro-1-oxoisoindolin-2-yl)piperidine-2,6-dione**

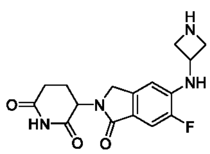

Referring to the method of Scheme 8, 3-(5-(azetidin-3-ylamino)-6-fluoro-1-oxoisoindolin-2-yl)piperidine-2,6-dione was prepared as light brown solid (2.5 g, yield: 97%). $^1$H NMR (400 MHz, DMSO) δ 10.95 (s, 1H), 8.81 (d, *J* = 40.1 Hz, 2H), 7.39 (d, *J* = 10.5 Hz, 1H), 6.92 (d, *J* = 4.8 Hz, 1H), 6.79 (d, *J* = 7.5 Hz, 1H), 5.04 (dd, *J*= 13.3, 5.1 Hz, 1H), 4.48 (dd, *J* = 13.7, 6.9 Hz, 1H), 4.30 (dd, *J* = 10.0, 6.7 Hz, 3H), 4.18 (d, *J* = 16.9 Hz, 1H), 4.06 - 3.97 (m, 2H), 2.94 - 2.84 (m, 1H), 2.59 (d, *J* = 16.7 Hz, 1H), 2.36 (dt, *J* = 13.3, 8.8 Hz, 1H), 1.97 (dd, *J* = 10.4, 5.0 Hz, 1H). LC/MS (ESI) m/z: calcd for $C_{16}H_{18}FN_4O_3^+$ [M+H]$^+$, 333.2; found, 333.1.

**Intermediate example 13: Preparation of 3-(5-(azetidin-3-ylidenemethyl)-1-oxoisoindolin-2-yl)piperidine-2,6-dione**

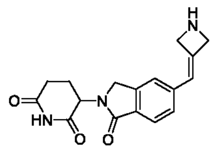

Referring to the method of route 9-2 of Scheme 9, 3-(5-(azetidin-3-ylidenemethyl)-1-oxoisoindolin-2-yl)piperidine-2,6-dione was prepared as brown solid (730 mg, yield: 80%). $^1$H NMR (400 MHz, DMSO) δ 10.99 (s, 1H), 9.08 (s, 2H), 7.72 (d, J = 7.9 Hz, 1H), 7.42 (s, 1H), 7.33 (d, J = 7.7 Hz, 1H), 6.56 (s, 1H), 5.11 (dd, J = 13.3, 5.0 Hz, 1H), 5.04 (s, 1H), 4.80 (s, 2H), 4.39 (dd, J = 48.0, 17.4 Hz, 2H), 2.92 (ddd, J = 18.6, 13.6, 5.4 Hz, 1H), 2.61 (dd, J = 32.9, 14.6 Hz, 2H), 2.46 - 2.28 (m, 1H), 2.06 - 1.95 (m, 1H). LC/MS (ESI) m/z: calcd for $C_{17}H_{19}N_3O_3^+$ [M+H]$^+$, 312.13; found, 312.2.

**Intermediate example 14: Preparation of 3-(5-(azetidin-3-ylmethyl)-1-oxoisoindolin-2-yl)piperidine-2,6-dione**

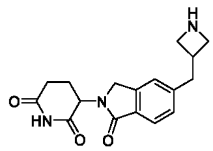

Referring to the method of route 9-1 of Scheme 9, 3-(5-(azetidin-3-ylmethyl)-1-oxoisoindolin-2-yl)piperidine-2,6-dione was prepared as brown solid (1.3 g, yield: 69%). $^1$H NMR (400 MHz, DMSO) δ 10.98 (s, 1H), 8.60 (d, J = 36.3 Hz, 2H), 7.68 (d, J = 7.8 Hz, 1H), 7.44 (s, 1H), 7.35 (d, J = 8.1 Hz, 1H), 5.10 (dd, J = 13.3, 5.0 Hz, 1H), 4.47 - 4.26 (m, 2H), 3.96 (s, 2H), 3.74 (s, 2H), 3.10 (dd, J = 15.3, 7.4 Hz, 1H), 3.04 (d, J = 7.7 Hz, 2H), 2.98 - 2.84 (m, 1H), 2.60 (d, J = 17.8 Hz, 1H), 2.40 (dt, J = 13.6, 9.0 Hz, 2H), 2.01 (s, 1H). LC/MS (ESI) m/z: calcd for $C_{17}H_{20}N_3O_3^+$ [M+H]$^+$, 314.15; found, 314.2.

**Intermediate example 15: Preparation of 3-(5-(hydroxy(3-hydroxyazetidin-3-yl)methyl)-1-oxoisoindolin-2-yl)piperidine-2,6-dione**

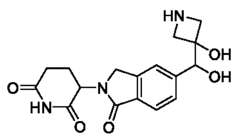

3-(5-(hydroxy(3-hydroxyazetidin-3-yl)methyl)-1-oxoisoindolin-2-yl)piperidine-2,6-dione was prepared by referring to the method of Scheme 11.

Step 1:

To a solution of tert-butyl 3-((2-(2,6-dioxopiperidin-3-yl)-1-oxoisoindolin-5-yl)methylene)azetidine-1-carboxylate (2 g, 4.86 mmol) in THF (10 mL) and $H_2O$ (10 mL) were added potassium osmate (180 mg, 0.49 mmol) and N-methylmorpholine N-oxide (1.14 g, 9.72 mmol). The reaction solution was stirred at room temperature for 18 hours. After monitoring the reaction via TLC and confirming its completion, the reaction solution was quenched with sodium metabisulfite (50 mL) and extracted with ethyl acetate (50 mL × 3). The combined organic phases were dried over anhydrous sodium sulfate and filtered. The filtrate was concentrated under reduced pressure to afford tert-butyl 3-((2-(2,6-dioxopiperidin-3-yl)-1-oxoisoindolin-5-yl)(hydroxy)methyl)-3-hydroxyazetidine-1-carboxylate as a brown solid (1.5 g, yield: 69%). LC/MS (ESI) m/z: calcd for $C_{22}H_{28}N_3O_7^+$ [M+H]$^+$, 446.19; found, [M+H-56]$^+$ 390.2.

Step 2:

To a solution of tert-butyl 3-((2-(2,6-dioxopiperidin-3-yl)-1-oxoisoindolin-5-yl)(hydroxy)methyl)-3-hydroxyazetidine-1-carboxylate (1.5 g, 3.37 mmol) in dichloromethane (30 mL) was added TFA (10 mL). The reaction mixture was stirred at room temperature for 18 hours. After monitoring the reaction via LCMS and confirming its completion, the reaction solution was concentrated under reduced pressure, and the residue was treated with ethyl acetate/petroleum

ether (20 mL, 1:1 volume ratio), causing solids to precipitate.

The suspension was filtered, and the target compound was collected as a brown solid (1 g, yield: 67%). $^1H$ NMR (400 MHz, DMSO) δ 10.99 (s, 1H), 8.66 (d, J = 68.6 Hz, 2H), 7.69 (d, J = 7.9 Hz, 1H), 7.60 (d, J = 6.5 Hz, 1H), 7.55 - 7.46 (m, 1H), 6.45 (s, 1H), 6.26 (s, 1H), 5.12 (dd, J = 8.6, 3.4 Hz, 1H), 4.38 (dd, J = 53.5, 17.2 Hz, 2H), 4.19 (d, J = 31.0 Hz, 2H), 3.79 (s, 1H), 3.57 (s, 1H), 2.91 (dd, J = 21.6, 9.5 Hz, 1H), 2.63 (t, J = 16.5 Hz, 1H), 2.45 - 2.30 (m, 3H), 2.01 (s, 1H). LC/MS (ESI) m/z: calcd for $C_{17}H_{20}N_3O_5^+$ $[M+H]^+$, 346.14; found, 346.2.

**Intermediate example 16: Preparation of 3-(1-oxo-5-(4-(piperazin-1-ylmethyl)phenyl)isoindolin-2-yl)piperidine-2,6-dione**

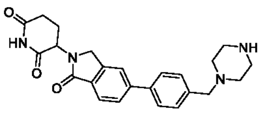

Referring to the method of Scheme 10, 3-(1-oxo-5-(4-(piperazin-1-ylmethyl)phenyl)isoindolin-2-yl)piperidine-2,6-dione was prepared as white solid (740 mg, yield: 104%). LC/MS (ESI) m/z: calcd for $C_{24}H_{27}N_4O_3^+$ $[M+H]^+$, 419.26; found, 419.3.

**Intermediate example 17: Preparation of 3-(5-(3,8-diazabicyclo[3.2.1]octan-8-yl)-1-oxoisoindolin-2-yl)piperidine-2,6-dione**

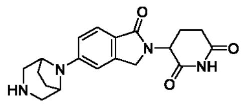

Referring to the method of Scheme 19, 3-(5-(3,8-diazabicyclo[3.2.1]octan-8-yl)-1-oxoisoindolin-2-yl)piperidine-2,6-dione was prepared as light brown solid (0.76 g, yield: 82%). $^1H$ NMR (400 MHz, DMSO) δ 10.95 (s, 1H), 9.25 (d, J = 47.8 Hz, 2H), 7.58 (d, J = 8.3 Hz, 1H), 7.24 - 6.91 (m, 2H), 5.05 (dd, J = 13.0, 4.5 Hz, 1H), 4.54 (s, 2H), 4.27 (dt, J = 26.0, 13.0 Hz, 2H), 3.08 (s, 4H), 2.90 (t, J = 12.8 Hz, 1H), 2.59 (d, J = 17.0 Hz, 1H), 2.44 - 2.31 (m, 1H), 2.19 - 1.87 (m, 5H). LC/MS (ESI) m/z: calcd for $C_{19}H_{22}N_4O_3^+$ $[M+H]^+$, 355.17; found, 355.2.

**Intermediate example 18: Preparation of 3-(1-oxo-5-(piperazin-1-ylmethyl)isoindolin-2-yl)piperidine-2,6-dione**

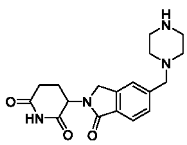

3-(1-oxo-5-(piperazin-1-ylmethyl)isoindolin-2-yl)piperidine-2,6-dione was prepared by referring to the method of Scheme 12.

Step 1:

To a solution of (2-(2,6-Dioxopiperidin-3-yl)-1-oxoisoindolin-5-yl)methyl methanesulfonate (1.0 g, 2.84 mmol) and N-Boc-piperazine (1.06 g, 5.68 mmol) in anhydrous DCM (40 mL) were added diisopropylethylamine (1.18 mL, 8.5 mmol) and sodium iodide (0.43 g, 2.84 mmol). The reaction solution was stirred at room temperature for 18 hours. After monitoring the reaction via TLC and confirming its completion, the reaction solution was washed with water (50 mL) and extracted with DCM (50 mL × 3). The combined organic phases were washed with saturated saline solution (50 mL × 1), dried over anhydrous sodium sulfate, and filtered. The filtrate was concentrated under reduced pressure, and the residue was subjected to column chromatography for purification to obtain the compound tert-butyl 4-((2-(2,6-dioxopiperidin-3-yl)-1-oxoisoindolin-5-yl)methyl)piperazine-1-carboxylate as a white solid (780 mg, yield: 71%). LC/MS (ESI) m/z: calcd for $C_{23}H_{31}N_4O_5^+$ [M+H]$^+$, 443.2; found, 443.2.

Step 2:

To a solution of tert-butyl 4-((2-(2,6-dioxopiperidin-3-yl)-1-oxoisoindolin-5-yl)methyl)piperazine-1-carboxylate (780 mg, 2.0 mmol) in dichloromethane (20 mL) was added a hydrochloric acid/dioxane solution (5.2 mL, 4M). The reaction solution was stirred at room temperature for 2 hours. After monitoring the reaction via LCMS and confirming its completion, the reaction solution was concentrated under reduced pressure to obtain the compound 3-(1-oxo-5-(piperazin-1-ylmethyl) isoindolin-2-yl)piperidine-2,6-dione as a white solid (910 mg, yield: 105%). LC/MS (ESI) m/z: calcd for $C_{18}H_{23}N_4O_3^+$ [M+H]$^+$, 343.2; found, 343.2.

**Intermediate example 19: Preparation of 3-(1-oxo-5-(piperidin-4-ylamino)isoindolin-2-yl)piperidine-2,6-dione**

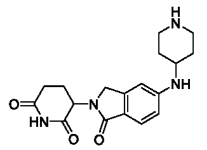

3-(1-oxo-5-(piperidin-4-ylamino)isoindolin-2-yl)piperidine-2,6-dione was prepared by referring to the method of Scheme 13.

Step 1:

To a solution of 3-(5-amino-1-oxoisoindolin-2-yl)piperidine-2,6-dione (CAS No.: 191732-70-4) (2 g, 7.71 mmol) and tert-butyl 4-oxopiperidine-1-carboxylate (3.1 g, 15.43 mmol) in dichloromethane (30 mL) was added acetic acid (10 mL) at 25°C. The reaction solution was then cooled to 0°C and stirred for 2 hours. Subsequently, 2-methylpyridine borane complex (1.64 g, 15.43 mmol) was added to the reaction solution, followed by continued stirring for 48 hours. After monitoring the reaction via TLC and confirming its completion, the reaction solution was quenched with saturated sodium bicarbonate solution (30 mL), and adjusted to pH 8, resulting in the precipitation of a black solid. The resulting mixture was filtered, and the filter cake was washed with ethyl acetate. The solid was collected to obtain the intermediate product tert-butyl 4-((2-(2,6-dioxopiperidin-3-yl)-1-oxoisoindolin-5-yl)amino)piperidine-1-carboxylate as black solid (2.4 g, yield: 70%). $^1$H NMR (400 MHz, DMSO) δ 10.93 (s, 1H), 7.39 (d, J = 8.2 Hz, 1H), 6.69 (s, 2H), 6.27 (d, J = 7.5 Hz, 1H), 5.02 (d, J = 8.8 Hz, 1H), 4.21 (dd, J = 51.6, 16.5 Hz, 2H), 3.89 (d, J = 11.4 Hz, 2H), 3.52 (s, 2H), 2.92 (d, J = 12.2 Hz, 4H), 2.59 (d, J = 17.2 Hz, 1H), 2.35 (d, J = 12.0 Hz, 1H), 1.94 - 1.77 (m, 3H), 1.41 (s, 9H). LCMS (ESI)m/z: calcd for $C_{23}H_{31}N_4O_5^+$ [M+H]$^+$, 443.22; found, 443.3.

Step 2:

To a solution of tert-butyl 4-((2-(2,6-dioxopiperidin-3-yl)-1-oxoisoindolin-5-yl)amino)piperidine-1-carboxylate (2.3 g, 3.64 mmol) in dichloromethane (30 mL) was added TFA (10 mL). The reaction solution was stirred at room temperature for 1 hour. After monitoring the reaction via LCMS and confirming its completion, the reaction solution was concentrated under reduced pressure, and the residue was purified by reverse-phase column chromatography to obtain 3-(1-oxo-5-(piperidin-4-ylamino)isoindolin-2-yl)piperidine-2,6-dione (229 mg, yield: 14%). $^1$H NMR (400 MHz, DMSO) δ 10.93 (s, 1H), 8.70 - 8.32 (m, 2H), 7.48 - 7.33 (m, 1H), 6.80 - 6.61 (m, 2H), 5.02 (dd, J = 13.3, 5.1 Hz, 1H), 4.22 (d, J = 34.3 Hz, 2H), 3.64 (t, J = 9.9 Hz, 1H), 3.33 (d, J = 12.7 Hz, 2H), 3.03 (dd, J = 21.7, 11.5 Hz, 2H), 2.95 - 2.82 (m, 1H), 2.59 (d, J = 16.8 Hz, 1H), 2.35 (dt, J = 13.2, 8.8 Hz, 1H), 2.08 (d, J = 13.1 Hz, 2H), 1.99 - 1.90 (m, 1H), 1.57 (dd, J = 21.5, 10.5 Hz, 2H). LC/MS (ESI) m/z: calcd for $C_{18}H_{23}N_4O_3^+$ [M+H]$^+$, 343.18; found, 343.10.

**Intermediate example 20: Preparation of 2-(2,6-dioxopiperidin-3-yl)-1-oxoisoindoline-5-carboxylic acid (GTC00260)**

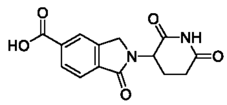

At room temperature, a 250 mL single-necked flask was charged sequentially with 3-(5-bromo-1-oxoisoindolin-2-yl)piperidine-2,6-dione (9.0 g, 27.851 mmol), palladium acetate (0.19 g, 0.836 mmol), ligand Xantphos (16.12 g, 27.851 mmol), formic acid (3.85 g, 83.553 mmol), and N,N'-dicyclohexylcarbodiimide (1.15 g, 5.570 mmol). The reaction mixture was stirred and reacted for one minute, followed by addition of triethylamine (7.764 mL, 55.702 mmol). The reaction flask was purged with nitrogen three times, and the reaction solution was heated to 100°C and stirred for 3 hours. After monitoring the reaction via TLC and confirming its completion, the reaction solution was filtered to remove solids, and the filtrate was concentrated to obtain the crude product. The crude product was slurried in dichloromethane, filtered, washed with water, and dried under vacuum to obtain GTC00260 as white solid (5.96 g, yield 74.24%). $^1$H NMR (400 MHz, DMSO) δ 13.33 (s, 1H), 11.01 (s, 1H), 8.17 (s, 1H), 8.08 (dd, J = 7.9, 1.2 Hz, 1H), 7.83 (d, J = 7.9 Hz, 1H), 5.15 (dd, J = 13.3, 5.1 Hz, 1H), 4.48 (dd, J = 50.2, 17.7 Hz, 2H), 3.01 - 2.89 (m, 1H), 2.61 (d, J = 16.6 Hz, 1H), 2.41 (qd, J = 13.2, 4.4 Hz, 1H), 2.03 (ddd, J = 10.2, 5.2, 3.1 Hz, 1H). LCMS (ESI): calcd for $C_{14}H_{13}N_2O_5^+$ [M+H]$^+$: 289.07; found, 289.1.

**Intermediate example 21: Preparation of 2-(2,6-dioxopiperidin-3-yl)-1,3-dioxoisoindoline-5-carboxylic acid (GTC00923)**

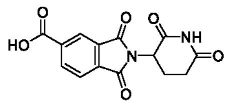

At room temperature, a 250 mL single-necked flask was charged sequentially with 1,3-dioxo-1,3-dihydroisobenzofuran-5-carboxylic acid (9.0 g, 46.843 mmol), 3-aminopiperidine-2,6-dione hydrochloride (6.00 g, 46.843 mmol), sodium acetate (11.53 g, 140.530 mmol), and 100 mL of acetic acid. The reaction mixture was heated to 90°C and stirred for 4 hours. After monitoring the reaction via TLC and confirming its completion, the reaction mixture was concentrated to obtain the crude product. The crude product was slurried in water to precipitate the solid, which was then filtered, washed with water, and dried under vacuum to obtain GTC00923 as blue solid (12.5 g, yield 88.29%). $^1$H NMR (400 MHz, DMSO) δ 13.74 (s, 1H), 11.15 (s, 1H), 8.40 (dd, J = 7.8, 1.4 Hz, 1H), 8.27 (d, J = 0.5 Hz, 1H), 8.04 (d, J = 7.8 Hz, 1H), 5.20 (dd, J = 12.8, 5.4 Hz, 1H), 2.99 - 2.84 (m, 1H), 2.69 - 2.59 (m, 1H), 2.54 (dd, J = 13.3, 4.3 Hz, 1H), 2.15 - 2.03 (m, 1H). LCMS (ESI): calcd for $C_{14}H_{11}N_2O_6^+$ [M+H]$^+$, 303.05; found, 303.1.

**Intermediate example 22: Preparation of 4'-fluoro-5,5-dimethyl-3,4,5,6-tetrahydro-[1,1'-biphenyl]-2-carboxylic acid (GTC00948)**

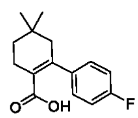

4'-fluoro-5,5-dimethyl-3,4,5,6-tetrahydro-[1,1'-biphenyl]-2-carboxylic acid was prepared according to the method of Step 1 of Scheme 18.

At room temperature, a 500 mL single-necked flask was charged sequentially with a solution of 4'-fluoro-5,5-dimethyl-3,4,5,6-tetrahydro-[1,1'-biphenyl]-2-carbaldehyde (GTC00947) (25 g, 107.619 mmol) in acetonitrile (100 mL), sodium dihydrogen phosphate (3.36 g, 27.981 mmol), water (140 mL), hydrogen peroxide (24.39 g, 215.239 mmol), and an aqueous solution of sodium hypochlorite (13.63 g, 150.667 mmol). The reaction mixture was stirred at room temperature overnight. After monitoring the reaction via TLC and confirming its completion, the reaction solution was washed with water (400 mL) and extracted with ethyl acetate (500 mL × 3). The combined organic phases were dried over anhydrous sodium sulfate, filtered, and the filtrate was concentrated under reduced pressure to afford compound GTC00948 as white solid (20 g, yield 74.84%). $^1$H NMR (400 MHz, DMSO) δ 12.03 (s, 1H), 7.34 - 7.00 (m, 4H), 2.35 (t, *J* = 6.4 Hz, 2H), 2.09 (s, 2H), 1.42 (t, *J* = 6.4 Hz, 2H), 0.96 (s, 6H). LCMS (ESI): calcd for $C_{17}H_{21}FNO_2^+$ [M+H+$CH_3CN$]$^+$,

290.12, found, 290.1.

**Intermediate example 23: Preparation of (4'-chloro-5,5-dimethyl-3,4,5,6-tetrahydro-[1,1'-biphenyl]-2-yl)(piperazin-1-yl)methanone (GTC00935)**

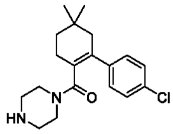

(4'-Chloro-5,5-dimethyl-3,4,5,6-tetrahydro-[1,1'-biphenyl]-2-yl)(piperazin-1-yl)methanone was prepared according to the method of Step 2 of Scheme 18.

Step 2(1): At room temperature, a 100 mL single-necked flask was charged with a solution of 4'-chloro-5,5-dimethyl-3,4,5,6-tetrahydro-[1,1'-biphenyl]-2-carboxylic acid (1.1 g, 4.16 mmol) and tert-butyl piperazine-1-carboxylate (0.85 g, 4.57 mmol) in N,N-dimethylformamide (20 mL), followed by sequential addition of benzotriazol-1-yl-oxytripyrrolidinophosphonium hexafluorophosphate (2.37 g, 4.57 mmol) and N,N-diisopropylethylamine (1.61 g, 12.46 mmol). The flask was purged with nitrogen gas three times, and the reaction solution was stirred at room temperature overnight. After monitoring the reaction via TLC and confirming its completion, the reaction solution was washed with water (40 mL) and extracted with ethyl acetate (100 mL × 3). The combined organic phases were dried over anhydrous sodium sulfate, and filtered. The filtrate was concentrated under reduced pressure, and the residue was subjected to column chromatography (petroleum ether/ethyl acetate = 10/1 ~ 2/1) for purification to afford tert-butyl 4-(4'-chloro-5,5-dimethyl-3,4,5,6-tetrahydro-[1,1'-biphenyl]-2-carbonyl)piperazine-1-carboxylate as colorless oil (1.4 g, yield 85.37%). LCMS (ESI): calcd for $C_{24}H_{33}ClN_2NaO_3^+$ $[M+Na]^+$, 455.21, found, 455.2.

Step 2(2): At room temperature, a 100 mL single-necked flask was charged with a solution of tert-butyl 4-(4'-chloro-5,5-dimethyl-3,4,5,6-tetrahydro-[1,1'-biphenyl]-2-carbonyl)piperazine-1-carboxylate (1.3 g, 3.00 mmol) in ethyl acetate (20 mL) and a solution of hydrochloric acid in ethyl acetate (40 mL, 3M). The flask was purged with nitrogen gas once, and the reaction solution was stirred at room temperature for 3 hours. After monitoring the reaction via LC/MS and confirming its completion, the reaction solution was concentrated under reduced pressure to afford compound GTC00935 as white solid (956 mg, yield 95.7%). $^1H$ NMR (400 MHz, DMSO) δ 7.38 (d, $J$ = 8.4 Hz, 2H), 7.25 (d, $J$ = 8.4 Hz, 2H), 3.25 (s, 1H), 3.11 (d, $J$ = 38.0 Hz, 2H), 2.88 (s, 1H), 2.53 (s, 1H), 2.47 - 2.31 (m, 3H), 2.22 (s, 1H), 2.07 (d, $J$ = 17.6 Hz, 1H), 1.90 (d, $J$ = 17.2 Hz, 1H), 1.78 (s, 1H), 1.43 (dd, $J$ = 14.0, 6.4 Hz, 2H), 0.98 (d, $J$ = 10.4 Hz, 6H). LCMS (ESI) calcd for $C_{19}H_{26}ClN_2O^+$ $[M+H]^+$: 333.17, found, 333.2.

**Intermediate examples 24-62**

The following intermediate compounds were prepared by referring to the methods of Scheme 18 or Step 2 of Scheme 18 or Intermediate examples 22-23.

Table 2. Intermediate examples 24-62 compounds

| Compound No. | Structure of the compound | The compound's name | Chinese translation of the compound's name | Data of nuclear magnetic resonance hydrogen spectrum ($^1H$ NMR) | Data of liquid chromatography-mass spectrometry (LC-MS) |
|---|---|---|---|---|---|
| | 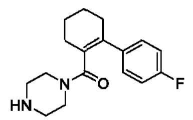 | (4'-fluoro-3,4,5,6-tetrahydro-[1,1'-biphenyl]-2-yl)(piperazin-1-yl) methanone | (4'-fluoro-3,4,5,6-tetrahydro-[1,1'-biphenyl]-2-yl)(piperazin-1-yl) methanone | $^1H$ NMR (400 MHz, MeOD) δ 7.33 (dd, *J* = 7.9, 5.6 Hz, 2H), 7.11 (t, *J* = 8.5 Hz,2H), 3.69 (s, 1H), 3.60 - 3.46 (m, 2H), 3.34 (d, *J* = 10.9 Hz, 1H), 3.12 (s, 1H), 3.00 (d, *J* = 10.9 Hz, 1H), 2.68 (s, 2H), 2.50 (d, *J* = 16.7 Hz, 1H), 2.11 (dd, *J* = 57.7, 10.6 Hz, 3H), 1.91 - 1.69 (m, 4H). | LC/MS (ESI) m/z: cal cd for $C_{17}H_{22}FN_2O^+$ $[M+H]^+$, 289.17; found, 289.2. |
| | 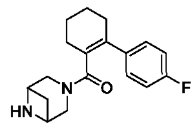 | (3,6-diazabicyclo[ 3.1.1] heptan-3-yl)(4'-fluoro-3,4,5,6-tetrahydro-[1,1'-biphenyl]-2-yl) methanone | (3,6-diazabicyclo[ 3.1.1] heptan-3-yl)(4'-fluoro-3,4,5,6-tetrahydro-[1,1'-biphenyl]-2-yl) methanone | $^1H$ NMR (400 MHz, MeOD) δ 7.34 (dd, *J* = 8.6, 5.4 Hz, 2H), 7.07 (t, *J* = 8.7 Hz, 2H), 4.22 (d, *J* = 26.8 Hz, 2H), 3.78 (d, *J* = 44.7 Hz, 4H), 2.50 (dd, *J* = 127.9, 55.0 Hz, 6H), 1.83 (d, *J* = 4.5 Hz, 5H), 0.54 - 0.35 (m, 1H). | LC/MS (ESI) m/z: calcd for $C_{18}H_{22}FN_2O^+$ $[M+H]^+$, 301.17; found, 301.2. |
| GTC 0093 6 | 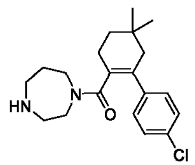 | (4'-chloro-5,5-dimethyl-3,4,5,6-tetrahydro-[1,1'-biphenyl]-2-yl) (1,4-diazepan-1-yl) methanone | (4'-chloro-5,5-dimethyl-3,4,5,6-tetrahydro-[1,1'-biphenyl]-2-yl) (1,4-diazepan-1-yl) methanone | $^1H$NMR (400 MHz, MeOD-d4) δ 7.38 (d, *J* = 7.6 Hz, 2H), 7.31 (d, *J* = 8.0 Hz, 2H), 3.60 (ddd, *J* = 25.2, 20.4, 10.4 Hz, 3H), 3.24 (d, *J*= 6.4 Hz, 2H), 3.06 (d, *J* = 7.2 Hz, 1H), 2.89 (s, 1H), 2.67 - 2.41 (m, 2H), 2.27 (d, *J* = 9.6 Hz, 2H), 1.98 (dd, *J* = 23.2, 14.8 Hz, 3H), 1.57 (dd, *J* = 15.2, 6.4 Hz, 2H), 1.07 (d, *J* = 12.4 Hz, 6H). | LCMS (ESI) calcd for $C_{20}H_{28}ClN_2O^+$ $[M+H]^+$: 347.18; found, 347.2. |
| GTC 0093 7 | 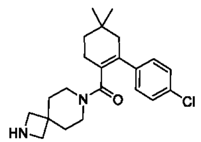 | (4'-chloro-5,5-dimethyl-3,4,5,6-tetrahydro-[1,1'-biphenyl]-2-yl) (2,7-diazaspiro[3. 5] nonan-7-yl)methanone | (4'-chloro-5,5-dimethyl-3,4,5,6-tetrahydro-[1,1'-biphenyl]-2-yl) (2,7-diazaspiro[3. 5] nonan-7-yl)methanone | $^1H$ NMR (400 MHz, DMSO) δ 7.40 - 7.32 (m, 2H), 7.29 - 7.20 (m, 2H), 3.41 - 3.23 (m, 4H), 3.21 - 3.03 (m, 3H), 2.97 (s, 1H), 2.36 (d, *J* = 16.8 Hz, 2H), 2.05 (d, *J*= 18.4 Hz, 1H), 1.91 (d, *J* = 17.2 Hz, 1H), 1.56 (s, 1H), 1.44 (dd, *J* = 15.6, 6.4 Hz, 3H), 1.24 (s, 1H), 0.98 (d, *J* = 14.0 Hz, 6H), 0.74 (s, 1H). | LCMS (ESI) calcd for $C_{22}H_{30}ClN_2O^+$ $[M+H]^+$: 373.20; found, 373.2. |
| GTC 0093 8 | 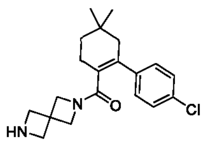 | (4'-chloro-5,5-dimethyl-3,4,5,6-tetrahydro-[1,1'-biphenyl]-2-yl) (2,6-diazaspiro[3. 3] heptan-2- | (4'-chloro-5,5-dimethyl-3,4,5,6-tetrahydro-[1,1'-biphenyl]-2-yl) (2,6-diazaspiro[3. 3] heptan-2- | $^1H$ NMR (400 MHz, MeOD-d4) δ 7.39 (d, *J* = 8.4 Hz, 2H), 7.30 (d, *J* = 8.4 Hz, 2H), 4.04 (d, *J* = 11.6 Hz, 2H), 4.00 - 3.88 (m, 4H), 3.80 (s, 2H), 2.37 (t,*J* = 6.4 Hz, 2H), 2.21 (s, 2H), 1.52 (t, *J* = 6.4 Hz, 2H), 1.06 (s, 6H). | LCMS (ESI) calcd for $C_{20}H_{26}ClN_2O^+$ $[M+H]^+$: 345.17; found, 345.2. |

(continued)

| Compound No. | Structure of the compound | The compound's name | Chinese translation of the compound's name | Data of nuclear magnetic resonance hydrogen spectrum ($^1$H NMR) | Data of liquid chromatography-mass spectrometry (LC-MS) |
|---|---|---|---|---|---|
| | | yl)methanone | yl)methanone | | |
| GTC 0093 9 | 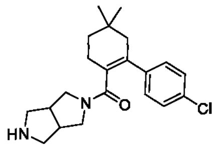 | (4'-chloro-5,5-dimethyl-3,4,5,6-tetrahydro-[1,1'-biphenyl]-2-yl)(hexahydr opyrrolo[3,4-c]pyrrol-2(1H)-yl)methanone | (4'-chloro-5,5-dimethyl-3,4,5,6-tetrahydro-[1,1'-biphenyl]-2-yl)(hexahydr opyrrolo[3,4-c]pyrrol-2(1H)-yl)methanone | $^1$HNMR (400 MHz, MeOD-d4) δ 7.38 (d, *J* = 8.4 Hz, 2H), 7.30 (d, *J* = 8.4 Hz, 2H), 3.46 (dt, *J* = 24.0, 12.0 Hz, 4H), 3.22 (s, 2H), 3.05 - 2.76 (m, 4H), 2.30 (d, *J* = 82.8 Hz, 4H), 1.56 (t, *J* = 6.4 Hz, 2H), 1.07 (d, *J* = 2.4 Hz, 6H). | LCMS (ESI) calcd for $C_{21}H_{28}ClN_2O^+$ [M+H]$^+$: 359.18; found, 359.2. |
| GTC 0094 0 | 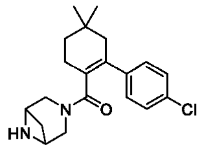 | (3,6-diazabicyclo[ 3.1.1] heptan-3-yl)(4'-chloro-5,5-dimethyl-3,4,5,6-tetrahydro-[1,1'-biphenyl]-2-yl) methanone | (3,6-diazabicyclo[ 3.1.1] heptan-3-yl)(4'-chloro-5,5-dimethyl-3,4,5,6-tetrahydro-[1,1'-biphenyl]-2-yl) methanone | $^1$H NMR (400 MHz, MeOD) δ 7.35 (d,*J* = 8.4 Hz, 2H), 7.29 (d, *J* = 8.8 Hz, 2H), 4.24 (d, *J* = 26.0 Hz, 2H), 3.96 - 3.57 (m, 3H), 3.54 - 3.32 (m, 1H), 2.88 - 2.25 (m, 4H), 2.23 - 2.07 (m, 1H), 2.00 (d, *J* = 8.0 Hz, 2H), 1.56 (s, 2H), 1.06 (d, *J* = 6.8 Hz, 6H). | LCMS (ESI) calcd for $C_{20}H_{24}ClN_2O^+$ [M+H]$^+$: 345.17; found, 345.2. |
| GTC 0094 1 | 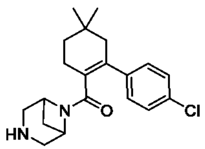 | (3,6-diazabicyclo[ 3.1.1] heptan-6-yl)(4'-chloro-5,5-dimethyl-3,4,5,6-tetrahydro-[1,1'-biphenyl]-2-yl) methanone | (3,6-diazabicyclo[ 3.1.1] heptan-6-yl)(4'-chloro-5,5-dimethyl-3,4,5,6-tetrahydro-[1,1'-biphenyl]-2-yl) methanone | $^1$H NMR (400 MHz, MeOD-d4) δ 7.34 - 7.25 (m, 2H), 7.23 - 7.15 (m, 2H), 4.14 (s, 1H), 3.89 (s, 1H), 3.48 (d, *J* = 12.4 Hz, 1H), 3.33 (t, *J* = 12.8 Hz, 2H), 3.06 (d, *J* = 12.8 Hz, 1H), 2.47 (d, *J*= 18.0 Hz, 1H), 2.23 (dd, *J* = 29.2, 18.4 Hz, 2H), 2.02 - 1.87 (m, 1H), 1.78 - 1.67 (m, 1H), 1.55 (d, *J* = 10.0 Hz, 1H), 1.49 - 1.37 (m, 2H), 0.93 (s, 6H). | LCMS (ESI) calcd for $C_{20}H_{24}ClN_2O^+$ [M+H]$^+$: 345.17; found, 345.1. |
| GTC 0094 2 | 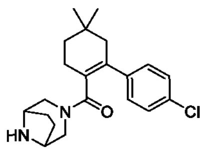 | (3,8-diazabicyclo[ 3.2.1] octan-3-yl)(4'-chloro-5,5-dimethyl-3,4,5,6-tetrahydro-[1,1'-biphenyl]-2-yl) methanone | (3,8-diazabicyclo[ 3.2.1] octan-3-yl)(4'-chloro-5,5-dimethyl-3,4,5,6-tetrahydro-[1,1'-biphenyl]-2-yl) methanone | $^1$H NMR (400 MHz, MeOD) δ 7.49 - 7.23 (m, 3H), 7.21 (d, *J* = 8.0 Hz, 1H), 4.29 (dd, *J* = 80.4, 14.4 Hz, 1H), 4.00 (*d, J*= 6.0 Hz, 1H), 3.95 - 3.81 (m, 1H), 3.70 - 3.54 (m, 1H), 3.47 (d, *J* = 13.6 Hz, 1H), 3.08 (d, *J* = 14.4 Hz, 1H), 2.71 - 2.36 (m, 3H), 2.25 - 2.03 (m, 2H), 1.96 (d, *J* = 20.4 Hz, 2H), 1.86 - 1.45 (m, 4H), 1.05 (d, *J* = 16.8 Hz, 6H). | LCMS (ESI) calcd for $C_{21}H_{28}ClN_2O^+$ [M+H]$^+$: 359.18; found, 359.2. |

(continued)

| Compound No. | Structure of the compound | The compound's name | Chinese translation of the compound's name | Data of nuclear magnetic resonance hydrogen spectrum ($^1$H NMR) | Data of liquid chromatography-mass spectrometry (LC-MS) |
|---|---|---|---|---|---|
| GTC 0094 3 | 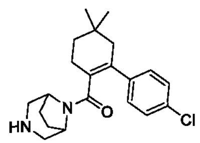 | (3,8-diazabicyclo[ 3.2.1] octan-8-yl)(4'-chloro-5,5-dimethyl-3,4,5,6-tetrahydro-[1,1'-biphenyl]-2-yl) methanone | (3,8-diazabicyclo[ 3.2.1] octan-8-yl)(4'-chloro-5,5-dimethyl-3,4,5,6-tetrahydro-[1,1'-biphenyl]-2-yl) methanone | $^1$H NMR (400 MHz, MeOD) δ 7.37 (d, *J* = 8.4 Hz, 2H), 7.30 (d, *J* = 8.0 Hz, 2H), 4.67 (s, 1H), 4.10 (q, *J* = 7.2 Hz, 1H), 3.20 (d, *J* = 11.6 Hz, 1H), 3.08 (s, 2H), 2.65 - 2.06 (m, 4H), 2.00 (d, *J* = 8.4 Hz, 2H), 1.88 - 1.50 (m, 5H), 1.05 (d, *J*= 2.4 Hz, 6H). | LCMS (ESI) calcd for $C_{21}H_{28}ClN_2O^+$ [M+H]$^+$: 359.18; found, 359.2. |
| GTC 0094 4 | 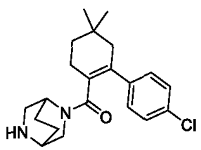 | (2,5-diazabicyclo[ 2.2.2] octan-2-yl)(4'-chloro-5,5-dimethyl-3,4,5,6-tetrahydro-[1,1'-biphenyl]-2-yl) methanone | (2,5-diazabicyclo[ 2.2.2] octan-2-yl)(4'-chloro-5,5-dimethyl-3,4,5,6-tetrahydro-[1,1'-biphenyl]-2-yl) methanone | $^1$H NMR (400 MHz, MeOD-d4) δ 7.40 (d, *J* = 8.4 Hz, 2H), 7.31 (dd, *J* = 16.4, 8.4 Hz, 2H), 4.52 (s, 1H), 3.75 (dd, *J* = 71.2, 51.2 Hz, 3H), 3.34 (s, 1H), 3.10 (s, 1H), 2.75 - 2.41 (m, 2H), 2.23 (s, 2H), 1.94 (dt, *J* = 99.6, 40.4 Hz, 4H), 1.57 (s, 3H), 1.08 (d, *J* = 6.4 Hz, 6H). | LCMS (ESI) calcd for $C_{21}H_{28}ClN_2O^+$ [M+H]$^+$: 359.18; found, 359.2. |
| GTC 0094 5 | 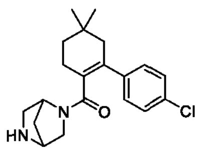 | (2,5-diazabicyclo[ 2.2. 1] heptan-2-yl)(4'-chloro-5,5-dimethyl-3,4,5,6-tetrahydro-[1,1'-biphenyl]-2-yl) methanone | (2,5-diazabicyclo[ 2.2.1] heptan-2-yl)(4'-chloro-5,5-dimethyl-3,4,5,6-tetrahydro-[1,1'-biphenyl]-2-yl) methanone | $^1$H NMR (400 MHz, MeOD) δ 7.33 (d, *J* = 8.4 Hz, 2H), 7.29 - 7.23 (m, 2H), 4.81 (s, 1H), 4.31 (d, *J* = 153.2 Hz, 1H), 3.55 (d, *J* = 30.8 Hz, 1H), 3.03 (dd, *J* = 29.2, 19.2 Hz, 2H), 2.91 - 2.48 (m, 2H), 2.48 - 1.99 (m, 4H), 1.53 (dd, *J* = 10.4, 6.4 Hz, 2H), 1.44 (d, *J* = 17.2 Hz, 1H), 1.10 - 0.99 (m, 6H). | LCMS (ESI) calcd for $C_{20}H_{26}ClN_2O^+$ [M+H]$^+$: 345.20; found, 345.2. |
| GTC 0094 6 | 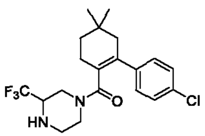 | (4'-chloro-5,5-dimethyl-3,4,5,6-tetrahydro-[1,1'-biphenyl]-2-yl) (3-(trifluoromet hyl)piperazin -1-yl)methanone | (4'-chloro-5,5-dimethyl-3,4,5,6-tetrahydro-[1,1'-biphenyl]-2-yl) (3-(trifluoromet hyl)piperazin -1-yl)methanone | $^1$H NMR (400 MHz, MeOD-d4) δ 7.40 - 7.06 (m, 4H), 4.70 - 4.33 (m, 1H), 4.21 - 3.86 (m, 1H), 3.82 - 3.50 (m, 1H), 3.27 (d, *J* = 13.2 Hz, 1H), 2.99 (s, 1H), 2.41 (dd, *J* = 33.2, 15.6 Hz, 3H), 2.10 (d,*J* = 15.2 Hz, 1H), 2.01 - 1.34 (m, 4H), 0.96 (s, 6H). | LCMS (ESI) calcd for $C_{20}H_{25}ClF_3N_2O^+$ [M+H]$^+$: 401.15; found, 401.5. |
| GTC 0096 3 | 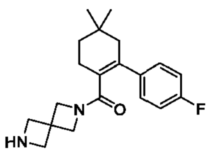 | (4'-fluoro-5,5-dimethyl-3,4,5,6-tetrahydro-[1,1'-biphenyl]-2-yl) (2,6-diazaspiro[3. 3] heptan-2-yl)methanone | (4'-fluoro-5,5-dimethyl-3,4,5,6-tetrahydro-[1,1'-biphenyl]-2-yl) (2,6-diazaspiro[3. 3] heptan-2-yl)methanone | $^1$H NMR (400 MHz, MeOD) δ 7.39 - 7.26 (m, 2H), 7.09 (t, J = 8.5 Hz, 2H), 4.01 (d, J = 11.4 Hz, 2H), 3.89 (d, J = 14.5 Hz, 4H), 3.77 (s, 2H), 2.34 (s, 2H), 2.19 (s, 2H), 1.49 (t, J = 6.2 Hz, 2H), 1.03 (s, 6H). | LCMS (ESI) calcd for $C_{20}H_{25}FN_2O^+$ [M+H]$^+$: 329.20, found, 329.2. |

(continued)

| Compound No. | Structure of the compound | The compound's name | Chinese translation of the compound's name | Data of nuclear magnetic resonance hydrogen spectrum ($^1H$ NMR) | Data of liquid chromatography-mass spectrometry (LC-MS) |
|---|---|---|---|---|---|
| GTC 0096 2 | 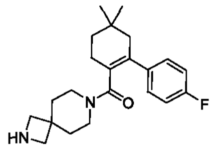 | (4'-fluoro-5,5-dimethyl-3,4,5,6-tetrahydro-[1,1'-biphenyl]-2-yl)(2,7-diazaspiro[3. 5]nonan-7-yl)methanone | (4'-fluoro-5,5-dimethyl-3,4,5,6-tetrahydro-[1,1'-biphenyl]-2-yl)(2,7-diazaspiro[3. 5]nonan-7-yl)methanone | $^1H$ NMR (400 MHz, MeOD) δ 7.33 - 7.24 (m, 2H), 7.04 (ddd, J = 10.9, 6.0, 2.6 Hz, 2H), 3.80 (s, 2H), 3.70 (d, J = 10.7 Hz, 1H), 3.65 - 3.59 (m, 1H), 3.51 - 3.41 (m, 1H), 3.30 - 3.20 (m, 2H), 3.16 - 3.06 (m, 1H), 2.55 - 2.36 (m, 2H), 2.20 - 2.08 (m, 1H), 1.98 (dd, J = 13.0, 9.9 Hz, 1H), 1.83 - 1.71 (m, 1H), 1.70 - 1.60 (m, 1H), 1.52 (dd, J = 13.1, 6.5 Hz, 2H), 1.47 - 1.37 (m, 1H), 1.04 (d, J = 12.9 Hz, 6H), 0.95 (ddd, J = 12.7, 8.7, 3.7 Hz, 1H). | LCMS (ESI) calcd for $C_{22}H_{29}FN_2O^+$ $[M+H]^+$: 357.23, found, 357.2. |
| GTC 0096 4 | 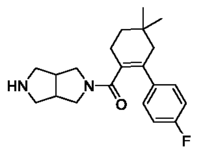 | (4'-fluoro-5,5-dimethyl-3,4,5,6-tetrahydro-[1,1'-biphenyl]-2-yl)(hexahydr opyrrolo[3,4-c]pyrrol-2(1H)-yl)methanone | (4'-fluoro-5,5-dimethyl-3,4,5,6-tetrahydro-[1,1'-biphenyl]-2-yl)(hexahydr opyrrolo[3,4-c]pyrrol-2(1H)-yl)methanone | $^1H$ NMR (400 MHz, MeOD) δ 7.32 (dd, J = 8.5, 5.5 Hz, 2H), 7.09 (t, J = 8.7 Hz, 2H), 3.65 - 3.31 (m, 4H), 3.18 (s, 2H), 3.06 - 2.50 (m, 4H), 2.38 (s, 2H), 2.11 (d, J = 60.3 Hz, 2H), 1.53 (t, J = 6.4 Hz, 2H), 1.06 (d, J = 8.7 Hz, 6H). | LCMS (ESI) calcd for $C_{21}H_{27}FN_2O^+$ $[M+H]^+$: 343.21; found, 343.2. |
| GTC 0096 6 | 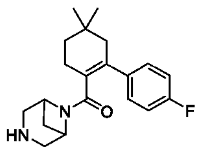 | (3,6-diazabicyclo[ 3.1.1]heptan-6-yl)(4'-fluoro-5,5-dimethyl-3,4,5,6-tetrahydro-[1,1'-biphenyl]-2-yl)methanone | (3,6-diazabicyclo[ 3.1.1]heptan-6-yl)(4'-fluoro-5,5-dimethyl-3,4,5,6-tetrahydro-[1,1'-biphenyl]-2-yl)methanone | $^1H$ NMR (400 MHz, MeOD) δ 7.40 - 7.26 (m, 2H), 7.13 (d, J = 8.7 Hz, 2H), 4.24 (s, 1H), 4.00 (s, 1H), 3.59 (d, J = 12.6 Hz, 1H), 3.47 (t, J = 11.4 Hz, 2H), 3.16 (d, J = 12.8 Hz, 1H), 2.56 (d, J = 18.1 Hz, 1H), 2.34 (t, J = 18.8 Hz, 2H), 2.04 (d, J = 15.6 Hz, 1H), 1.83 (d, J = 9.8 Hz, 1H), 1.70 (d, J = 10.0 Hz, 1H), 1.62 - 1.41 (m, 2H), 1.03 (d, J = 4.2 Hz, 6H). | LCMS (ESI) calcd for $C_{20}H_{25}FN_2O^+$ $[M+H]^+$: 329.20; found, 329.1. |
| GTC 0096 7 | 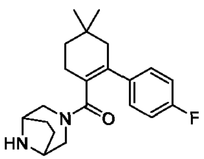 | (3,8-diazabicyclo[ 3.2.1]octan-3-yl)(4'-fluoro-5,5-dimethyl-3,4,5,6-tetrahydro-[1,1'-biphenyl]-2-yl)methanone | (3,8-diazabicyclo[ 3.2.1]octan-3-yl)(4'-fluoro-5,5-dimethyl-3,4,5,6-tetrahydro-[1,1'-biphenyl]-2-yl)methanone | $^1H$ NMR (400 MHz, D2O) δ 7.12 (dd, J = 8.3, 5.6 Hz, 1H), 7.02 - 6.81 (m, 3H), 4.06 - 3.64 (m, 3H), 3.44 (d, J = 13.8 Hz, 1H), 3.12 (dd, J = 155.1, 14.0 Hz, 1H), 2.45 (dd, J = 14.1, 8.7 Hz, 1H), 2.16 (d, J = 16.4 Hz, 2H), 2.02 - 1.89 (m, 1H), 1.89 - 1.54 (m, 4H), 1.37 - 0.85 (m, 3H), 0.84 - 0.71 (m, 6H). | LCMS (ESI) calcd for $C_{21}H_{27}FN_2O^+$ $[M+H]^+$: 343.21; found, 343.4. |
| GTC 0096 8 | 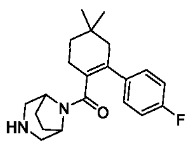 | (3,8-diazabicyclo[ 3.2.1]octan-8-yl)(4'-fluoro-5,5-dimethyl-3,4,5,6-tetrahydro-[1,1'-biphenyl]-2-yl)methanone | (3,8-diazabicyclo[ 3.2.1]octan-8-yl)(4'-fluoro-5,5-dimethyl-3,4,5,6-tetrahydro-[1,1'-biphenyl]-2-yl)methanone | $^1H$ NMR (400 MHz, DMSO) δ 9.76 (s, 1H), 9.29 (s, 1H), 7.28 (s, 2H), 7.18 (d, J = 8.8 Hz, 2H), 4.50 (s, 1H), 3.88 (d, J = 5.8 Hz, 1H), 3.05 (d, J = 12.1 Hz, 1H), 2.93 (s, 2H), 2.30 (s, 4H), 1.89 (d, J = 20.8 Hz, 2H), 1.73 (s, 1H), 1.61 - 1.07 (m, 4H), 0.98 (d, J = 3.7 Hz, 6H). | LCMS (ESI) calcd for $C_{21}H_{27}FN_2O^+$ $[M+H]^+$: 343.21; found, 343.2. |

(continued)

| Compound No. | Structure of the compound | The compound's name | Chinese translation of the compound's name | Data of nuclear magnetic resonance hydrogen spectrum ($^1H$ NMR) | Data of liquid chromatography-mass spectrometry (LC-MS) |
|---|---|---|---|---|---|
| GTC 0096 9 | 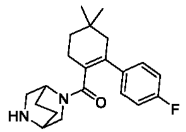 | (2,5-diazabicyclo[ 2.2.2] octan-2-yl)(4'-fluoro-5,5-dimethyl-3,4,5,6-tetrahydro-[1,1'-biphenyl]-2-yl)methanone | (2,5-diazabicyclo[ 2.2.2] octan-2-yl)(4'-fluoro-5,5-dimethyl-3,4,5,6-tetrahydro-[1,1'-biphenyl]-2-yl)methanone | $^1H$ NMR (400 MHz, D2O) δ 7.23 (dd, J = 8.3, 5.7 Hz, 2H), 7.19 - 6.97 (m, 2H), 3.89 (d, J = 50.4 Hz, 2H), 3.67 (d, J = 11.8 Hz, 1H), 3.50 - 3.22 (m, 2H), 3.03 (d, J = 12.6 Hz, 1H), 2.60 - 2.31 (m, 2H), 2.11 (d, J = 40.7 Hz, 2H), 1.99 - 1.74 (m, 3H), 1.47 (s, 3H), 0.94 (d, J = 9.8 Hz, 6H). | LCMS (ESI) calcd for $C_{21}H_{27}FN_2O^+$ $[M+H]^+$: 343.21; found, 343.2. |
| GTC 0097 0 | 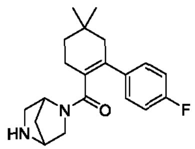 | (2,5-diazabicyclo[ 2.2.1] heptan-2-yl)(4'-fluoro-5,5-dimethyl-3,4,5,6-tetrahydro-[1,1'-biphenyl]-2-yl) methanone | (2,5-diazabicyclo[ 2.2.1] heptan-2-yl)(4'-fluoro-5,5-dimethyl-3,4,5,6-tetrahydro-[1,1'-biphenyl]-2-yl) methanone | $^1H$ NMR (400 MHz, DMSO) δ 9.81- 8.92 (m, 2H), 7.32 - 7.22 (m, 2H), 7.16 (dt, J = 14.0, 8.9 Hz, 2H), 4.55 - 4.05 (m, 2H), 3.76 (s, 1H), 3.63 - 3.24 (m, 1H), 3.19 - 2.83 (m, 2H), 2.71 (d, J = 10.1 Hz, 1H), 2.44 - 2.04 (m, 3H), 1.66 (s, 1H), 1.56 (d, J = 7.4 Hz, 1H), 1.50 - 1.35 (m, 2H), 1.13 - 1.04 (m, 1H), 0.98 (d, J = 11.3 Hz, 6H). | LCMS (ESI) calcd for $C_{20}H_{25}FN_2O^+$ $[M+H]^+$: 329.20; found, 329.2. |
| GTC 0096 1 | 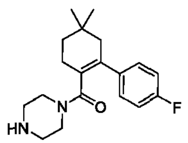 | (4'-fluoro-5,5-dimethyl-3,4,5,6-tetrahydro-[1,1'-biphenyl]-2-yl) (piperazin-1-yl)methanone | (4'-fluoro-5,5-dimethyl-3,4,5,6-tetrahydro-[1,1'-biphenyl]-2-yl) (piperazin-1-yl)methanone | $^1H$ NMR (400 MHz, MeOD) δ 7.32 (dd, J = 8.6, 5.4 Hz, 2H), 7.12 (t, J = 8.7 Hz, 2H), 3.76 (s, 1H), 3.58 (dd, J = 13.9, 8.5 Hz, 2H), 3.37 (s, 1H), 3.15 (s, 1H), 3.04 (d, J = 17.5 Hz, 1H), 2.68 (s, 1H), 2.48 (d, J = 18.6 Hz, 2H), 2.20 (d, J = 17.7 Hz, 1H), 2.00 (dd, J = 25.8, 13.4 Hz, 2H), 1.64 - 1.45 (m, 2H), 1.05 (d, J = 15.9 Hz, 6H). | LCMS (ESI) calcd for $C_{19}H_{25}FN_2O^+$ $[M+H]^+$: 317.2, found, 317.7. |
| GTC 0096 5 | 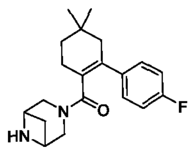 | (3,6-diazabicyclo[ 3.1.1] heptan-3-yl)(4'-fluoro-5,5-dimethyl-3,4,5,6-tetrahydro-[1,1'-biphenyl]-2-yl) methanone | (3,6-diazabicyclo[ 3.1.1] heptan-3-yl)(4'-fluoro-5,5-dimethyl-3,4,5,6-tetrahydro-[1,1'-biphenyl]-2-yl) methanone | $^1H$ NMR (400 MHz, MeOD) δ 7.42 - 7.27 (m, 2H), 7.19 - 6.97 (m, 2H), 4.24 (d, J = 24.6 Hz, 2H), 4.00 - 3.57 (m, 3H), 2.68 (d, J = 8.8 Hz, 1H), 2.59 - 2.20 (m, 3H), 2.00 (d, J = 3.6 Hz, 2H), 1.58 (t, J = 8.5 Hz, 2H), 1.04 (t, J = 8.9 Hz, 6H), 0.44 (s, 1H). | LCMS (ESI) calcd for $C_{20}H_{25}FN_2O^+$ $[M+H]^+$: 329.2; found, 329.6. |
| GTC 0097 1 | 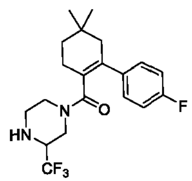 | (4'-fluoro-5,5-dimethyl-3,4,5,6-tetrahydro-[1,1'-biphenyl]-2-yl) (3-(trifluoromet hyl)piperazin -1-yl)methanone | (4'-fluoro-5,5-dimethyl-3,4,5,6-tetrahydro-[1,1'-biphenyl]-2-yl) (3-(trifluoromet hyl)piperazin -1-yl)methanone | $^1H$ NMR (400 MHz, MeOD) δ 7.30 (d, J = 34.3 Hz, 2H), 7.22 - 7.00 (m, 2H), 4.62 (s, 1H), 4.27 (d, J = 118.1 Hz, 1H), 3.86 (d, J = 11.4 Hz, 1H), 3.60 -3.38 (m, 1H), 3.16 (d, J = 24.8 Hz, 1H), 2.49 (dd, J = 33.0, 16.9 Hz, 3H), 2.20 (d, J = 15.1 Hz, 1H), 2.00 (d, J = 8.7 Hz, 2H), 1.55 (d, J = 4.0 Hz, 2H), 1.06 (s, 6H). | LCMS (ESI) calcd for $C_{20}H_{24}F_4N_2O^+$ $[M+H]^+$: 385.18; found, 385.6. |

(continued)

| Compound No. | Structure of the compound | The compound's name | Chinese translation of the compound's name | Data of nuclear magnetic resonance hydrogen spectrum ($^1$H NMR) | Data of liquid chromatography-mass spectrometry (LC-MS) |
|---|---|---|---|---|---|
| GTC 0097 2 | 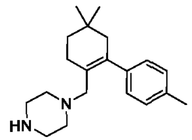 | 1-((4',5,5-trimethyl-3,4,5,6-tetrahydro-[1,1'-biphenyl]-2-yl) methyl)pip erazine | 1-((4',5,5-trimethyl-3,4,5,6-tetrahydro-[1,1'-biphenyl]-2-yl) methyl)pip erazine | $^1$H NMR (400 MHz, MeOD-d4) δ 7.26 (d, J = 7.8 Hz, 2H), 7.05 (d, J = 8.0 Hz, 2H), 3.80 (s, 2H), 3.62 (s, 4H), 3.33 (dt, J = 3.2, 1.6 Hz, 2H), 3.10 (d, J = 42.4 Hz, 2H), 2.44 (s, 2H), 2.37 (s, 3H), 2.17 (s, 2H), 1.61 (t, J = 6.4 Hz, 2H), 1.05 (s, 6H). | LCMS (ESI) calcd for $C_{20}H_{30}N_2^+$ $[M+H]^+$: 299.24; found, 299.6. |
| GTC 0099 0 | 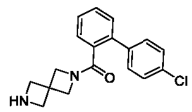 | (4'-chloro-[1,1'-biphenyl]-2-yl)(2,6-diazaspiro [3. 3]heptan-2-yl)methanone | (4'-chloro-[1,1'-biphenyl]-2-yl)(2,6-diazaspiro [3. 3]heptan-2-yl)metha-none | $^1$H NMR (400 MHz, MeOD-d4) δ 7.62 - 7.57 (m, 1H), 7.53 - 7.45 (m, 7H), 4.14 (s, 3H), 4.10 (s, 1H), 3.97 (s, 1H), 3.95 (s, 1H), 3.80 (s, 2H). | LCMS (ESI) calcd for $C_{18}H_{17}ClN_2O^+$ $[M+H]^+$: 313.1; found, 313.1. |
| GTC 0099 7 | 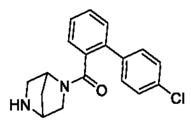 | (2,5-diazabicyclo[ 2.2.1] heptan-2-yl)(4'-chloro-[1,1'-biphenyl]-2-yl)methanone | (2,5-diazabicyclo[ 2.2.1] heptan-2-yl)(4'-chloro-[1,1'-biphenyl]-2-yl)methanone | $^1$H NMR (400 MHz, MeOD) δ 7.66 - 7.59 (m, 1H), 7.53 (tdd, J = 13.0, 6.6, 3.2 Hz, 7H), 4.47 - 3.94 (m, 2H), 3.51 (s, 1H), 3.43 - 3.34 (m, 1H), 3.28 - 2.75 (m, 2H), 2.00 - 1.41 (m, 2H). | LCMS (ESI) calcd for $C_{18}H_{17}ClN_2O^+$ $[M+H]^+$: 313.1; found, 313.2. |
| GTC 0098 6 | 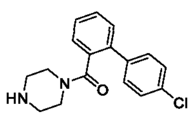 | (4'-chloro-[1,1'-biphenyl]-2-yl)(piperazin-1-yl) methanone | (4'-chloro-[1,1'-biphenyl]-2-yl)(piperazin-1-yl) methanone | $^1$H NMR(400 MHz, MeOD) δ 7.60 (td,J = 7.4, 1.6 Hz, 1H), 7.52 (dd,J = 8.8, 7.2 Hz, 4H), 7.49 - 7.44(m, 3H), 3.89 - 3.73 (m, 2H), 3.39 - 3.32 (m, 1H), 3.28 - 3.20 (m, 1H), 3.03 (dd,J = 21.6, 7.2 Hz, 2H),2.92 - 2.83 (m, 1H), 2.28 - 2.16 (m, 1H). | LCMS (ESI) calcd for $C_{17}H_{17}ClN_2O^+$ $[M+H]^+$: 301.1, found, 301.2. |
| GTC 0098 7 | 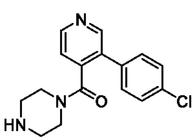 | (3-(4-chlorophenyl) pyri-din-4-yl)(piperazin-1-yl) methanone | (3-(4-chlorophenyl )pyridin-4-yl)(piperazin-1-yl) methanone | $^1$H NMR(400 MHz, MeOD-d4) δ 9.13 (s, 1H), 9.03 (d,J = 6.0 Hz, 1H), 8.29 (d,J = 5.6 Hz, 1H), 7.69 -7.60 (m, 4H), 4.04 - 3.91 (m, 1H), 3.81 - 3.69 (m, 1H), 3.54 - 3.43 (m, 1H), 3.32 (s, 1H), 3.14 (d,J = 10.8Hz, 2H), 3.04 - 2.94 (m, 1H), 2.47 - 2.34 (m, 1H). | LCMS (ESI) calcd for $C_{16}H_{16}ClN_3O^+$ $[M+H]^+$: 302.1; found, 302.1. |

(continued)

| Compound No. | Structure of the compound | The compound's name | Chinese translation of the compound's name | Data of nuclear magnetic resonance hydrogen spectrum ($^1$H NMR) | Data of liquid chromatography-mass spectrometry (LC-MS) |
|---|---|---|---|---|---|
| GTC 0098 8 | 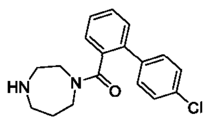 | (4'-chloro-[1,1'-biphenyl]-2-yl)(1,4-diazepan-1-yl)methanone | (4'-chloro-[1,1'-biphenyl]-2-yl)(1,4-diazepan-1-yl)methanone | $^1$H NMR(400 MHz, MeOD) δ 7.58 (ddd,J = 7.6, 6.0, 1.6 Hz, 1H), 7.54 - 7.41 (m, 7H), 4.11 - 3.59 (m,2H), 3.50 - 3.35 (m, 1H), 3.26 (d,J = 5.6 Hz, 1H), 3.19 - 3.03 (m, 2H), 2.99 - 2.45 (m, 2H), 2.16 - 1.70(m, 2H). | LCMS (ESI) calcd for $C_{18}H_{19}ClN_2O^+$ $[M+H]^+$: 315.12; found, 315.2. |
| GTC 0098 9 | 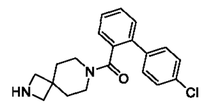 | (4'-chloro-[1,1'-biphenyl]-2-yl)(2,7-diazaspiro[3. 5]nonan-7-yl)methanone | (4'-chloro-[1,1'-biphenyl]-2-yl)(2,7-diazaspiro[3. 5]nonan-7-yl)methanone | $^1$H NMR(400 MHz, MeOD) δ 7.57 - 7.52 (m, 1H), 7.51 - 7.46 (m, 2H), 7.42 (d,J = 9.6 Hz, 4H), 7.38 -7.35 (m, 1H), 3.86 - 3.73 (m, 3H), 3.66 (d,J = 10.8 Hz, 1H), 3.53 (dd,J = 12.8, 7.6 Hz, 2H), 3.10 - 2.98(m, 1H), 2.83 (ddd,J = 13.6, 7.2, 3.6 Hz, 1H), 1.90 - 1.79 (m, 1H), 1.60 (dt,J = 10.4, 4.5 Hz, 2H), 1.06 - 0.95 (m, 1H). | LCMS (ESI) calcd for $C_{20}H_{21}ClN_2O^+$ $[M+H]^+$: 341.13; found, 341.2. |
| GTC 0099 1 | 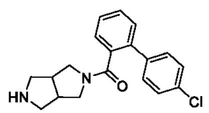 | (4'-chloro-[1,1'-biphenyl]-2-yl)(hexahydropyrrolo[3,4-c]pyrrol-2(1H)-yl)methanone | (4'-chloro-[1,1'-biphenyl]-2-yl)(hexahydropyrrolo[3,4-c]pyrrol-2(1H)-yl)methanone | $^1$H NMR(400 MHz, MeOD) δ 7.56 (dd,J = 7.6, 1.6 Hz, 1H), 7.53 - 7.44 (m, 7H), 3.63 (dd,J = 19.6,12.4 Hz, 1H), 3.49 (d,J = 4.4 Hz, 2H), 3.37 (dd,J = 12.0, 8.0 Hz, 1H), 3.17 (s, 1H), 3.00 (s, 3H), 2.84 (s,1H), 2.51 (dd,J = 39.2, 31.6 Hz, 1H). | LCMS (ESI) calcd for $C_{19}H_{19}ClN_2O^+$ $[M+H]^+$: 327.12; found, 327.2. |
| GTC 0099 2 | 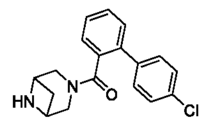 | (3,6-diazabicyclo[ 3.1.1]heptan-3-yl)(4'-chloro-[1,1'-biphenyl]-2-yl)methanone | (3,6-diazabicyclo[ 3.1.1]heptan-3-yl)(4'-chloro-[1,1'-biphenyl]-2-yl)methanone | $^1$H NMR(400 MHz, MeOD-d4) δ 7.60 (t,J = 7.6 Hz, 1H), 7.57 - 7.51 (m, 2H), 7.48 (s, 5H), 4.85 (s,3H), 4.35 (s, 1H), 4.13 (s, 1H), 3.93 (s, 1H), 3.65 (s, 1H), 2.74 (s, 1H). | LCMS (ESI) calcd for $C_{18}H_{17}ClN_2O^+$ $[M+H]^+$: 313.1; found, 313.2. |
| GTC 0099 3 | 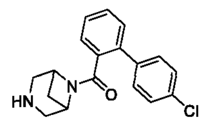 | (3,6-diazabicyclo[ 3.1.1]heptan-6-yl)(4'-chloro-[1,1'-biphenyl]-2-yl)methanone | (3,6-diazabicyclo[ 3.1.1]heptan-6-yl)(4'-chloro-[1,1'-biphenyl]-2-yl)methanone | $^1$H NMR(400 MHz, MeOD-d4) δ 7.62 (dd,J = 11.2, 4.0 Hz, 2H), 7.56 - 7.45 (m, 6H), 4.41 (s, 1H), 3.85(s, 1H), 3.77 (d,J = 12.4 Hz, 1H), 3.50 (d,J = 12.8 Hz, 1H), 3.33 (d,J = 6.0 Hz, 1H), 3.08 (d,J = 13.2Hz, 1H), 2.08 (dt,J = 13.2, 6.4 Hz, 1H), 1.71 (d,J = 10.0 Hz, 1H). | LCMS (ESI) calcd for $C_{18}H_{17}ClN_2O^+$ $[M+H]^+$: 313.1; found, 313.2. |

(continued)

| Compound No. | Structure of the compound | The compound's name | Chinese translation of the compound's name | Data of nuclear magnetic resonance hydrogen spectrum ($^1H$ NMR) | Data of liquid chromatography-mass spectrometry (LC-MS) |
|---|---|---|---|---|---|
| GTC 0099 4 | 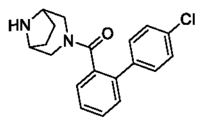 | (3,8-diazabicyclo[ 3.2.1] octan-3-yl)(4'-chloro-[1,1'-biphenyl]-2-yl)methanone | (3,8-diazabicyclo[ 3.2.1] octan-3-yl)(4'-chloro-[1,1'-biphenyl]-2-yl)methanone | $^1H$ NMR(400 MHz, MeOD-d4) δ 7.65 - 7.33 (m, 8H), 4.49 (dd,J = 91.6, 14.4 Hz, 1H), 4.09 (d,J = 6.8Hz, 1H), 3.80 (t,J = 8.4 Hz, 1H), 3.37 (d,J = 14.0 Hz, 1H), 3.30 - 3.07 (m, 1H), 2.73 (dd,J = 41.6, 14.3Hz, 1H), 2.15 - 1.72 (m, 3H), 1.68 - 1.24 (m, 1H), 0.53 - 0.40 (m, 1H). | LCMS (ESI) calcd for $C_{19}H_{19}ClN_2O^+$ $[M+H]^+$: 327.12; found, 327.5. |
| GTC 0099 5 | 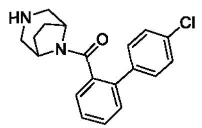 | (3,8-diazabicyclo[ 3.2.1] octan-8-yl)(4'-chloro-[1,1'-biphenyl]-2-yl)methanone | (3,8-diazabicyclo[ 3.2.1] octan-8-yl)(4'-chloro-[1,1'-biphenyl]-2-yl)methanone | $^1H$ NMR(400 MHz, MeOD-d4) δ 7.80 - 7.40 (m, 8H), 4.86 - 4.73 (m, 2H), 3.68 (d,J = 7.2 Hz, 1H),3.26 (d,J = 11.6 Hz, 1H), 3.18 - 2.71 (m, 2H), 1.81 (d,J = 74.4 Hz, 3H), 1.34 - 0.57 (m, 1H). | LCMS (ESI) calcd for $C_{19}H_{19}ClN_2O^+$ $[M+H]^+$: 327.12; found, 327.2. |
| GTC 0099 6 | 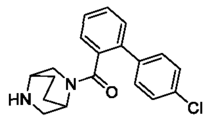 | (2,5-diazabicyclo[ 2.2.2] octan-2-yl)(4'-chloro-[1,1'-biphenyl]-2-yl)methanone | (2,5-diazabicyclo[ 2.2.2] octan-2-yl)(4'-chloro-[1,1'-biphenyl]-2-yl)methanone | $^1H$ NMR(400 MHz, MeOD-d4) δ 7.61 (t,J = 7.6 Hz, 1H), 7.58 - 7.45 (m, 7H), 4.70 (s, 1H), 4.09 - 3.77(m, 2H), 3.48 (dd,J = 46.0, 20.8 Hz, 2H), 3.32 - 2.86 (m, 2H), 2.15 - 1.47 (m, 4H). | LCMS (ESI) calcd for $C_{19}H_{19}ClN_2O^+$ $[M+H]^+$: 327.12; found, 327.2. |
| GTC 0099 8 | 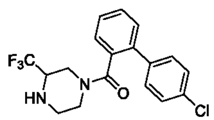 | (4'-chloro-[1,1'-biphenyl]-2-yl)(3-(trifluoromethyl)piperazin -1-yl) methanone | (4'-chloro-[1,1'-biphenyl]-2-yl)(3-(trifluoromethyl)piperazin -1-yl) methanone | $^1H$ NMR(400 MHz, MeOD-d4) δ 7.81- 7.20 (m, 8H), 4.72 (dd,J = 41.6, 14.4 Hz, 1H), 4.39 (dd,J =52.4, 11.2 Hz, 1H), 3.66 (dt,J = 30.8, 6.4 Hz, 1H), 3.56 - 3.33 (m, 2H), 3.27 - 3.05 (m, 1H), 2.92 - 2.65(m, 1H). | LCMS (ESI) calcd for $C_{18}H_{16}ClF_3N_2O^+$ $[M+H]^+$ : 369.09; found, 369.3. |
| GTC 0100 8 | 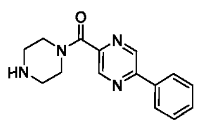 | (5-phenylpyrazi n-2-yl) (piperazin-1-yl)methanone | (5-phenylpyrazi n-2-yl) (piperazin-1-yl)methanone | $^1H$ NMR (400 MHz, D2O) δ 8.88 (d,J=1.2 Hz, 1H), 8.73 (d, J = 1.2 Hz, 1H), 7.85 - 7.76 (m, 2H), 7.50 - 7.39 (m, 3H), 3.94 (s, 2H), 3.78 (s, 2H), 3.34 (s, 2H), 3.25 (d, J = 4.6 Hz, 2H). | LCMS (ESI): calcd for $C_{15}H_{16}N_4O^+$ $[M+H]^+$: 269.13, found, 269.1. |

**Intermediate example 63: Preparation of N-(4'-chloro-[1,1'-biphenyl]-2-yl)piperidin-4-amine hydrochloride**

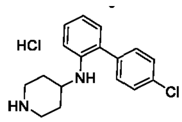

N-(4'-chloro-[1,1'-biphenyl]-2-yl)piperidin-4-amine hydrochloride was prepared by referring to the method of Scheme 20.

Step 1:

To a solution of 4'-chloro-[1,1'-biphenyl]-2-amine (980 mg, 4.81 mmol) and tert-butyl 4-oxopiperidine-1-carboxylate (1150 mg, 5.77 mmol) in 1,2-dichloroethane (10 mL) was added acetic acid (0.5 mL) at room temperature. The reaction solution was stirred for 1 hour, followed by addition of sodium triacetoxyborohydride (2039 mg, 9.62 mmol) and continued stirring for 15 hours. After monitoring the reaction via LCMS and confirming its completion, the reaction solution was washed with water (50 mL) and extracted with DCM (50 mL × 3). The combined organic phases were washed with saturated saline solution (50 mL × 1), dried over anhydrous sodium sulfate, and filtered. The filtrate was concentrated under reduced pressure, and the residue was slurried with petroleum ether/ethyl acetate (5:1, 100 mL) and filtered to obtain the yellow solid intermediate product, tert-butyl 4-((4'-chloro-[1,1'-biphenyl]-2-yl)amino)piperidine-1-carboxylate (1.9 g, yield: 94%). LCMS (ESI): calcd for $C_{22}H_{27}ClN_2O_2^+$ $[M+H]^+$, 387.18; found, 387.2.

Step 2:

To a solution of tert-butyl 4-((4'-chloro-[1,1'-biphenyl]-2-yl)amino)piperidine-1-carboxylate (1.9 g, 3.83 mmol) in dichloromethane was added a hydrochloric acid/dioxane solution (15 mL, 4M) at room temperature. The reaction solution was stirred at room temperature for 1 hour. After monitoring the reaction via TLC and confirming its completion, the reaction solution was concentrated under reduced pressure, and the residue was slurried with petroleum ether/ethyl acetate (5:1, 100 mL) and filtered to obtain the white solid product, N-(4'-chloro-[1,1'-biphenyl]-2-yl)piperidine-4-amine hydrochloride (500 mg, yield: 45%). $^1H$ NMR (400 MHz, DMSO) δ 9.35 - 8.75 (m, 2H), 7.47 (dd, J = 28.8, 8.3 Hz, 4H), 7.24 (t, J = 7.7 Hz, 1H), 7.04 (d, J = 7.4 Hz, 1H), 6.94 (d, J = 8.1 Hz, 1H), 6.81 (t, J = 7.3 Hz, 1H), 3.59 - 3.46 (m, 1H), 3.21 (d, J = 12.7 Hz, 2H), 2.92 (dd, J = 22.1, 11.4 Hz, 2H), 1.99 (d, J = 11.9 Hz, 2H), 1.59 (q, J = 10.0 Hz, 2H). LCMS (ESI): calcd for $C_{22}H_{27}ClN_2O_2^+$ $[M+H]^+$, 287.12; found, 287.2.

**Intermediate example 64: Preparation of 4-(chloromethyl)-3-(4-chlorophenyl)pyridine**

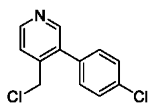

4-(chloromethyl)-3-(4-chlorophenyl)pyridine was prepared by referring to the method of Scheme 21.

At room temperature, (3-(4-chlorophenyl)pyridin-4-yl)methanol (490 mg, 2.23 mmol) was dissolved in dichloromethane (10 mL). The reaction solution was cooled to 0°C, followed by addition dropwise of thionyl chloride (1.5 mL, 20.68 mmol). After the addition was complete, the reaction mixture was slowly warmed to room temperature and stirred for 1.5 hours. After monitoring the reaction via LCMS and confirming its completion, the reaction solution was concentrated under reduced pressure to yield 4-(chloromethyl)-3-(4-chlorophenyl)pyridine (415 mg, yield 78.1%, brown solid). $^1H$ NMR (400 MHz, DMSO) δ8.79 (d, J = 4.8 Hz, 1H), 8.69 (s, 1H), 7.92 (s, 1H), 7.59 (dd, J = 33.2, 8.2 Hz, 4H), 4.78 (s, 2H). LCMS (ESI) calcd for $C_{12}H_9Cl_2N^+$, $[M+H]^+$, 238.01; found, 238.0.

**Intermediate example 65: Preparation of 1-(2-(5-chloropyridin-2-yl)benzyl)piperazine hydrochloride (GTC01002)**

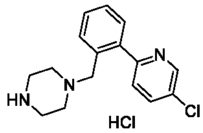

1-(2-(5-Chloropyridin-2-yl)benzyl)piperazine hydrochloride was prepared by referring to the method of Scheme 15.

At room temperature, in a 30 mL sealed tube, (2-((4-(tert-butoxycarbonyl)piperazin-1-yl)methylphenyl)boronic acid (CAS No.: 1012785-48-6) (300 mg, 0.746 mmol) and 2-bromo-5-chloropyridine (250 mg, 1.299 mmol) were dissolved in ethanol (3 mL), toluene (3 mL), and water (3 mL). To the resulting solution were added tetrakis(triphenylphosphine) palladium (62.55 mg, 0.054 mmol) and sodium carbonate (229.49 mg, 2.165 mmol). The reaction vessel was purged with nitrogen gas, and the reaction mixture was subjected to microwave irradiation at 130°C for 15 minutes. Thinlayer chromatography indicated completion of the reaction. The reaction was repeated three times with fresh charges of reactants. The reaction mixtures were then combined, concentrated under reduced pressure, washed with water, extracted with ethyl acetate, dried over anhydrous sodium sulfate, and concentrated. The residue was purified by column chromatography (petroleum ether/ethyl acetate = 1/0 ~ 10/1) to yield tert-butyl 4-(2-(5-chloropyridin-2-yl)benzyl)piperazine-1-carboxylate (yellow solid, 110 mg, yield 12.1%). LCMS (ESI): calcd for C21H26ClN3O2 + [M+H]+, 388.17, found, 388.2.

At room temperature, a 100 mL single-necked flask was charged with tert-butyl 4-(2-(5-chloropyridin-2-yl) benzyl)piperazine-1-carboxylate (110 mg, 0.284 mmol) and a solution of hydrochloric acid in ethyl acetate (5 mL, 3M). The reaction mixture was stirred at room temperature for 1 hour. After monitoring the reaction via LC/MS and confirming its completion, the reaction solution was filtered and dried to yield compound GTC01002 (white solid, 68 mg, yield 73.84%). $^1$H NMR (400 MHz, MeOD-d4) δ 8.92 (d,J = 2.0 Hz, 1H), 8.15 (dd,J = 8.6, 2.4 Hz, 1H), 7.95 - 7.87 (m, 2H), 7.73 (ddd,J = 9.2, 8.0, 1.2 Hz, 2H), 7.64 (td,J = 7.6, 1.2 Hz, 1H), 4.55 (s, 2H), 3.75 (d,J = 5.2 Hz, 4H), 3.72 (d,J = 5.0 Hz, 4H), 2.02 (d,J = 3.6 Hz, 1H). LCMS (ESI) calcd for $C_{16}H_{18}ClN_3^+$[M+H]$^+$: 288.12, found, 288.2.

**Intermediate examples 66-79**

The following intermediate compounds were prepared by referring to the methods of Scheme 15 or Intermediate example 65.

Table 3. Intermediate examples 66-79 compounds

| Compound No. | Structure of the compound | The compound 's name | Chinese translation of the compound 's name | Data of nuclear magnetic resonance hydrogen spectrum ($^1$H NMR) | Data of liquid chromatography-mass spectrometry (LC-MS) |
|---|---|---|---|---|---|
| GTC 0100 3 | 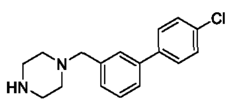 | 1-((4'-chloro-[1,1'-biphenyl]-3-yl) methyl) piperazine | 1-((4'-chloro-[1,1'-biphenyl]-3-yl) methyl) piperazine | $^1$H NMR (400 MHz, DMSO-d6) δ 12.41 (s, 1H), 9.85 (s, 2H), 8.08 (s, 1H), 7.79 (dd, J = 12.0, 8.7 Hz, 3H), 7.64 (s, 1H), 7.56 (dd, J = 7.9, 4.6 Hz, 3H), 4.46 (s, 2H), 3.59 (s, 4H), 3.49 (s, 4H). | LCMS (ESI): calcd for $C_{17}H_{19}ClN_2^+$ [M+H]$^+$: 287.12, found, 259.1. |

(continued)

| Compound No. | Structure of the compound | The compound 's name | Chinese translation of the compound 's name | Data of nuclear magnetic resonance hydrogen spectrum ($^1H$ NMR) | Data of liquid chromatography-mass spectrometry (LC-MS) |
|---|---|---|---|---|---|
| GTC 0100 4 | 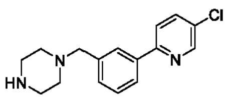 | 1-(3-(5-chloro-pyri din-2-yl)ben-zyl) piperazine | 1-(3-(5-chloro-pyri din-2-yl)ben-zyl) piperazine | $^1H$ NMR (400 MHz, $D_2O$) δ 8.75 (d, J = 2.1 Hz, 1H), 8.37 (dd, J = 8.7, 2.3 Hz, 1H), 8.07 (d, J = 8.8 Hz, 1H), 7.92 (s, 2H), 7.79 - 7.59 (m, 2H), 4.54 (s, 2H), 3.58 (d, J = 26.4 Hz, 8H). | LCMS (ESI): calcd for $C_{16}H_{18}ClN_3^+$ $[M+H]^+$: 288.12; found, 288.1. |
| GTC 0101 6 | 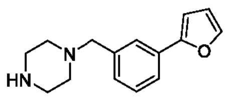 | 1-(3-(furan-2-yl) benzyl) piperazine | 1-(3-(furan-2-yl) benzyl) pipera-zine | $^1H$ NMR (400 MHz, DMSO-d6) δ 12.32 (s, 1H), 9.88 (s, 2H), 8.06 (s, 1H), 7.78 (dd, J = 8.4, 4.5 Hz, 2H), 7.57 (d, J = 7.6 Hz, 1H), 7.50 (t, J = 7.7 Hz, 1H), 6.99 (dd, J = 3.3, 0.5 Hz, 1H), 6.63 (dd, J = 3.4, 1.8 Hz, 1H), 4.43 (s, 2H), 3.56 (s, 3H), 3.47 (s, 5H). | LCMS (ESI): calcd for $C_{15}H_{18}N_2O^+$ $[M+H]^+$: 243.14; found, 243.1. |
| GTC 0102 2 | 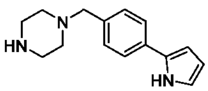 | 1-(4-(1H-pyr-rol-2-yl)benzyl) piperazine | 1-(4-(1H-pyrrol-2-yl)benzyl) piperazine | $^1H$ NMR (400 MHz, $D_2O$) δ 7.64 (d, *J* = 8.2 Hz, 2H), 7.44 (d, *J* = 8.2 Hz, 2H), 6.95 (d, *J* = 2.4 Hz, 1H), 6.62 (d, *J* = 2.3 Hz, 1H), 6.27 (d, *J* = 2.5 Hz, 1H), 4.37 (s, 2H), 3.53 (s, 8H). | LCMS (ESI): calcd for $C_{15}H_{19}N_3^+$ $[M+H]^+$: 242.16; found, 242.2. |
| GTC 0100 5 | 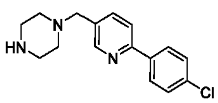 | 1-((6-(4-chloro-phen yl)pyri-din-3-yl)methyl) piperazine | 1-((6-(4-chloro-phen yl)pyri-din-3-yl)methyl) piperazine | $^1H$ NMR (400 MHz, $D_2O$) δ 8.87 (s, 1H), 8.61 (d, J = 8.5 Hz, 1H), 8.25 (d, J = 8.5 Hz, 1H), 7.93 - 7.73 (m, 2H), 7.65 - 7.52 (m, 2H), 4.53 (s, 2H), 3.51 (s, 8H). | LCMS (ESI) calcd for $C_{16}H_{18}ClN_3^+$ $[M+H]^+$: 288.12, found, 288.2. |
| GTC 0100 6 | 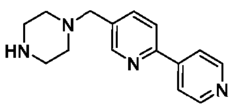 | 5-(piperazin-1-ylmethyl)-2,4'-bipyridine | 5-(piperazin-1-ylmethyl)-2,4'-bi-pyridine | $^1H$ NMR (400 MHz, $D_2O$) δ 8.55 - 8.42 (m, 2H), 8.39 (s, 1H), 7.77 (d, J = 8.2 Hz, 1H), 7.71 (d, J = 8.1 Hz, 1H), 7.67 - 7.57 (m, 2H), 3.59 (s, 2H), 3.20 - 3.12 (m, 4H), 2.68 (s, 4H). | LCMS (ESI): calcd for $C_{15}H_{18}N_4^+$ $[M+H]^+$: 255.15, found, 255.1. |

(continued)

| Compound No. | Structure of the compound | The compound 's name | Chinese translation of the compound 's name | Data of nuclear magnetic resonance hydrogen spectrum ($^1H$ NMR) | Data of liquid chromatography-mass spectrometry (LC-MS) |
|---|---|---|---|---|---|
| GTC 0100 7 | 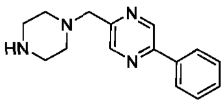 | 2-phenyl-5-(piperazin-1-ylmethyl)p yrazine | 2-phenyl-5-(piperazin-1-yl-methyl)p yrazine | $^1H$ NMR (400 MHz, $D_2O$) δ 9.01 (d, J = 1.2 Hz, 1H), 8.64 (d, J = 1.2 Hz, 1H), 7.84 (dd, J = 6.7, 2.9 Hz, 2H), 7.48 (dd, J = 4.1, 2.4 Hz, 3H), 4.65 (s, 2H), 3.70 (d, J = 5.4 Hz, 4H), 3.60 (d, J = 5.5 Hz, 4H). | LCMS (ESI): calcd for $C_{15}H_{18}N_4^+$ $[M+H]^+$: 255.15, found, 255.3. |
| GTC 0101 5 | 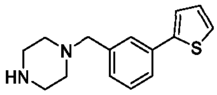 | 1-(3-(thiophen-2-yl)benzyl) piperazine | 1-(3-(thiophen-2-yl)benzyl) piperazine | $^1H$ NMR (400 MHz, $D_2O$) δ 7.84 - 7.64 (m, 2H), 7.47 (dt, J = 8.7, 6.4 Hz, 3H), 7.38 (d, J = 7.7 Hz, 1H), 7.13 (dd, J = 5.0, 3.7 Hz, 1H), 4.43 (s, 2H), 3.57 (s, 8H). | LCMS (ESI): calcd for $C_{15}H_{18}N_2S^+$ $[M+H]^+$: 259.12, found, 259.1. |
| GTC 0101 9 | 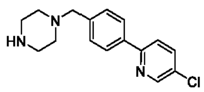 | 1-(4-(5-chloropyri din-2-yl)benzyl) piperazine | 1-(4-(5-chloropyri din-2-yl)benzyl) piperazine | $^1H$ NMR (400 MHz, $D_2O$) δ 8.83 (dd, J = 2.4, 0.5 Hz, 1H), 8.43 (dd, J = 8.7, 2.4 Hz, 1H), 8.16 (dd, J = 8.8, 0.5 Hz, 1H), 8.02 - 7.96 (m, 2H), 7.81 - 7.75 (m, 2H), 4.62 (s, 2H), 3.68 (d, J = 25.1 Hz, 8H). | LCMS (ESI): calcd for $C_{16}H_{18}ClN_3^+$ $[M+H]^+$: 288.12; found, 288.1. |
| GTC 0102 0 | 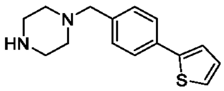 | 1-(4-(thiophen-2-yl)benzyl) piperazine | 1-(4-(thiophen-2-yl)benzyl) piperazine | $^1H$ NMR (400 MHz, $D_2O$) δ 7.79 (d, J = 8.1 Hz, 2H), 7.53 (dd, J = 7.6, 5.3 Hz, 4H), 7.24 - 7.18 (m, 1H), 4.48 (s, 2H), 3.65 (s, 8H). | LCMS (ESI): calcd for $C_{15}H_{18}N_2S^+$ $[M+H]^+$: 259.12; found, 259.1. |
| GTC 0101 7 | 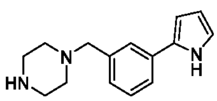 | 1-(3-(1H-pyrrol-2-yl)benzyl) piperazine | 1-(3-(1H-pyrrol-2-yl)benzyl) piperazine | $^1H$ NMR (400 MHz, DMSO) δ 11.26 (d, J = 48.9 Hz, 1H), 7.64 - 7.46 (m, 2H), 7.30 (t, J = 7.6 Hz, 1H), 7.10 (t, J = 12.3 Hz, 1H), 6.96 - 6.75 (m, 1H), 6.46 (d, J = 30.7 Hz, 1H), 6.11 (dd, J = 5.6, 2.5 Hz, 1H), 3.53 (s, 2H), 3.12 - 3.03 (m, 4H), 2.57 (s, 4H). | LCMS (ESI): calcd for $C_{15}H_{19}N_3^+$ $[M+H]^+$: 242.16, found, 242.1. |

(continued)

| Comp ound No. | Structure of the compound | The compound 's name | Chinese translation of the compound 's name | Data of nuclear magnetic resonance hydrogen spectrum ($^1H$ NMR) | Data of liquid chromatograp hy-mass spectrometry (LC-MS) |
|---|---|---|---|---|---|
| GTC 0102 3 | 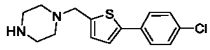 | 1-((5-(4-chloro-phen yl)thiophe n-2-yl)methyl) pi-perazine | 1-((5-(4-chloro-phen yl)thiophe n-2-yl)methyl) pi-perazine | $^1H$ NMR(400 MHz, MeOD-d4) δ 7.65 (d,J = 7.6 Hz, 2H), 7.41 (d,J = 20.0 Hz, 4H), 4.67 (d,J = 17.2Hz, 2H), 3.57 (d,J = 12.0 Hz, 8H). | LCMS (ESI) calcd for $C_{15}H_{17}ClN_2S^+$ $[M+H]^+$: 293.08, found, 293.1. |
| GTC 0102 4 | 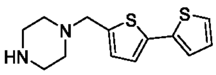 | 1-([2,2'-bithio-phen ]-5-yl-methyl)p ipera-zine | 1-([2,2'-bithio-phen ]-5-yl-methyl)p ipera-zine | $^1H$ NMR(400 MHz, MeOD-d4) δ 7.75 - 6.94 (m, 5H), 4.71 (d,J = 18.0 Hz, 2H), 3.61 (s, 8H). | LCMS (ESI) calcd for $C_{13}H_{16}N_2S_2^+$ $[M+H]^+$: 265.08; found, 265.1. |
| GTC 0102 5 | 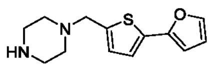 | 1-((5-(furan-2-yl) thiophe n-2-yl) methyl) piperazine | 1-((5-(furan-2-yl) thiophe n-2-yl) methyl) pipera-zine | $^1H$ NMR(400 MHz, MeOD-d4) δ 7.67 (d,J = 4.4 Hz, 1H), 7.55 (d,J = 1.2 Hz, 1H), 7.47 (d,J = 3.6 Hz,1H), 7.39 (d,J = 3.6 Hz, 1H), 7.31 (d,J = 3.6 Hz, 1H), 7.25 (d,J = 3.8 Hz, 1H), 7.18 (d,J = 3.8 Hz, 1H),6.71 (d,J = 3.4 Hz, 1H), 6.53 (dd,J = 3.4, 1.8 Hz, 1H), 4.73 (s, 2H), 3.64 (s, 8H). | LCMS (ESI) calcd for $C_{13}H_{16}N_2OS^+$ $[M+H]^+$: 249.1; found, 249.1. |

**Intermediate example 80: Preparation of 1-((4,4-dimethyl-2-(thiophen-2-yl)cyclohex-1-en-1-yl)methyl)piperazine (GTC00924)**

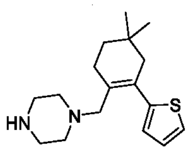

1-((4,4-dimethyl-2-(thiophen-2-yl)cyclohex-1-en-1-yl)methyl)piperazine was prepared by referring to the methods of step 1 of Scheme 16 and Scheme 17.

1. Preparation of 4,4-dimethyl-2-(thiophen-2-yl)cyclohex-1-en-1-carbaldehyde by referring to the method of Step 1 of Scheme 16:

At room temperature, a 100 mL single-neck flask was charged sequentially with 2-bromo-4,4-dimethylcyclohex-1-en-1-carbaldehyde (1.0 g, 4.63 mmol), thiophen-2-ylboronic acid (712 mg, 5.56 mmol), potassium carbonate (1.6 g,

11.56 mmol), tetrabutylammonium bromide (75 mg, 0.232 mmol), and water/chloroform (12 mL, 5:1). Then, to the reaction mixture was added 1,1'-bis(diphenylphosphino)ferrocene palladium(II)dichloride (170 mg, 0.232 mmol). The reaction vessel was purged with nitrogen gas for three times, and the reaction mixture was slowly heated to 110°C and stirred for 40 minutes. After monitoring the reaction via TLC and confirming its completion, the reaction was quenched with water. The reaction mixture was extracted with ethyl acetate, and the organic phase was dried over anhydrous sodium sulfate, filtered, and the filtrate was concentrated under reduced pressure. The residue was purified by column chromatography (petroleum ether/ethyl acetate = 10/1 ~ 2/1) to afford 4,4-dimethyl-2-(thiophen-2-yl)cyclohex-1-en-1-carbaldehyde (white solid, 660 mg, yield 65.8%). LCMS (ESI): calcd for $C_{13}H_{16}OS^+$ [M+H]$^+$, 221.09, found, 221.1.

2. Preparation of 1-((4,4-dimethyl-2-(thiophen-2-yl)cyclohex-1-en-1-yl)methyl)piperazine by referring to the method of Scheme 17:

At room temperature, a 50 mL single-neck flask was charged sequentially with 4,4-dimethyl-2-(thiophen-2-yl) cyclohex-1-en-1-carbaldehyde (560 mg, 2.55 mmol), tert-butyl piperazine-1-carboxylate (710 mg, 3.82 mmol), and 1,2-dichloroethane (10 mL). The reaction mixture was stirred at room temperature for 20 minutes, followed by addition of sodium triacetoxyborohydride (1.08 g, 5.09 mmol). The reaction mixture was stirred at room temperature for 3 hours. After monitoring the reaction via TLC and confirming its completion, the reaction was quenched with water. The reaction mixture was extracted with ethyl acetate, and the organic phase was dried over anhydrous sodium sulfate, filtered, and the filtrate was concentrated under reduced pressure. The residue was purified by column chromatography (petroleum ether/ethyl acetate = 10/1 ~ 2/1) to afford tert-butyl 4-((4,4-dimethyl-2-(thiophen-2-yl)cyclohex-1-en-1-yl)methyl)piperazine-1-carboxylate (white solid, 430 mg, yield 36.70%). LCMS (ESI): calcd for $C_{22}H_{34}N_2O_2S^+$ [M+H]$^+$, 391.23, found, 391.5.

At room temperature, a 50 mL single-neck flask was charged sequentially with a solution of tert-butyl 4-((4,4-dimethyl-2-(thiophen-2-yl)cyclohex-1-en-1-yl)methyl)piperazine-1-carboxylate (430 mg, 1.102 mmol) in ethyl acetate (8 mL), and a solution of hydrochloric acid in ethyl acetate (16 mL, 3M). The reaction solution was stirred at room temperature for 2 hours. After monitoring the reaction via LC/MS and confirming its completion, the reaction solution was concentrated under reduced pressure to afford compound GTC00924 (white solid, 345 mg, yield 97%). $^1$HNMR (400 MHz, D2O) δ 7.42 (d, J = 5.1 Hz, 1H), 7.06 - 7.01 (m, 1H), 6.87 (d, J = 3.4 Hz, 1H), 3.97 (s, 2H), 3.47 (d, J = 4.5 Hz, 4H), 3.36 (s, 4H), 2.24 - 2.14 (m, 4H), 1.45 (t, J = 6.2 Hz, 2H), 0.90 (s, 6H). LCMS (ESI) calcd for $C_{17}H_{26}N_2S^+$ [M+H]$^+$: 291.18, found, 291.2.

**Intermediate examples 81-91**

The following intermediate compounds were prepared by referring to the method of Intermediate example 80.

Table 4. Intermediate examples 81-91 compounds

| Comp ound No. | Structure of the compound | The compound's name | Chinese translation of the compound's name | Data of nuclear magnetic resonance hydrogen spectrum ($^1$H NMR) | Data of liquid chromatograph y-mass spectrometry (LC-MS) |
|---|---|---|---|---|---|
| GTCO 0926 | 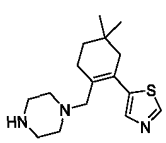 | 5-(5,5-di-methyl-2-(pipera-zin-1-ylmethyl) cyc lohex-1-en-1-yl)thiazole | 5-(5,5-di-methyl-2-(pipera-zin-1-ylmethyl)cy clohex-1-en-1-yl) thiazole | $^1$H NMR (400 MHz, $D_2O$) δ 9.56 (s, 1H), 7.92 (s, 1H), 3.94 (s, 2H), 3.53 (t, J = 5.0 Hz, 4H), 3.40 (s, 4H), 2.25 (s, 2H), 2.16 (s, 2H), 1.48 (t, J = 6.3 Hz, 2H), 0.90 (s, 6H). | LCMS (ESI) calcd for $C_{16}H_{25}N_3S^+$ [M+H]$^+$: 292.18; found, 292.2. |

(continued)

| Comp ound No. | Structure of the compound | The compound's name | Chinese translation of the compound's name | Data of nuclear magnetic resonance hydrogen spectrum ($^1$H NMR) | Data of liquid chromatograph y-mass spectrometry (LC-MS) |
|---|---|---|---|---|---|
| GTCO 1021 | 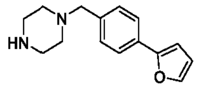 | 1-(4-(furan-2-yl) benzyl)pip erazine | 1-(4-(furan-2-yl) benzyl)pi pera-zine | $^1$H NMR (400 MHz, $D_2O$) δ 7.74 (d, *J* = 8.4 Hz, 2H), 7.55 (d, *J* = 1.2 Hz, 1H), 7.46 (d, *J* = 8.4 Hz, 2H), 6.90 - 6.80 (m, 1H), 6.53 (dd, *J* = 3.4, 1.8 Hz, 1H), 4.39 (s, 2H), 3.54 (s, 8H). | LCMS (ESI): calcd for $C_{15}H_{18}N_2O^+$ [M+H]$^+$: 243.14; found, 243.1. |
| GTCO 1027 | 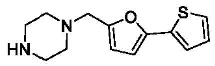 | 1-((5-(thio-phen-2-yl)fur-an-2-yl)methyl)pi perazine | 1-((5-(thio-phen-2-yl)fur-an-2-yl)methyl)pi perazine | $^1$H NMR (400 MHz, D2O) δ 7.38 (dd, J = 12.8, 4.3 Hz, 2H), 7.13 - 6.99 (m, 1H), 6.76 (d, J = 3.5 Hz, 1H), 6.65 (d, J = 3.5 Hz, 1H), 4.50 (s, 2H), 3.59 (d, J = 5.1 Hz, 4H), 3.56 (d, J = 4.0 Hz, 4H). | LCMS (ESI): calcd for $C_{13}H_{16}N_2OS^+$ [M+H]$^+$: 249.10; found, 249.1. |
| GTCO 1028 | 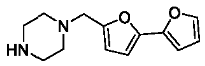 | 1-([2,2'-bifur-an]-5-ylmethyl) pip erazine | 1-([2,2'-bifuran]-5-ylmethyl) pip erazine | $^1$H NMR (400 MHz, D2O) δ 7.47 (d, J = 1.4 Hz, 1H), 6.74 (d, J = 3.5 Hz, 1H), 6.66 (d, J = 3.4 Hz, 1H), 6.60 (d, J = 3.5 Hz, 1H), 6.48 (dd, J = 3.4, 1.8 Hz, 1H), 4.47 (s, 2H), 3.61 - 3.49 (m, 8H). | LCMS (ESI): calcd for $C_{13}H_{16}N_2O_2^+$ [M+H]$^+$: 233.12; found, 233.1. |
| GTCO 1010 | 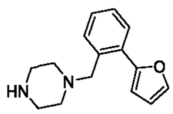 | 1-(2-(furan-2-yl) benzyl)pip erazine | 1-(2-(furan-2-yl) benzyl)pi pera-zine | $^1$H NMR (400 MHz, MeOD-d4) δ 7.88 (d, J = 1.7 Hz, 1H), 7.82 (dd, J = 7.9, 1.0 Hz, 1H), 7.79 - 7.71 (m, 1H), 7.60 (td, J = 7.7, 1.3 Hz, 1H), 7.52 (dd, J = 7.6, 1.2 Hz, 1H), 6.94 (d, J = 3.4 Hz, 1H), 6.68 (dd, J = 3.4, 1.9 Hz, 1H), 4.80 (s, 2H), 3.70 (d, J = 31.7 Hz, 8H). | LCMS (ESI) calcd for $C_{15}H_{18}N_2O^+$ [M+H]$^+$: 303.22, found, 303.3. |

(continued)

| Comp ound No. | Structure of the compound | The compound's name | Chinese translation of the compound's name | Data of nuclear magnetic resonance hydrogen spectrum ($^1H$ NMR) | Data of liquid chromatograph y-mass spectrometry (LC-MS) |
|---|---|---|---|---|---|
| GTCO 0999 | 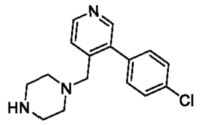 | 1-((3-(4-chloro-phenyl )pyridin-4-yl)methyl)pi pera-zine | 1-((3-(4-chloro-pheny 1)pyri-din-4-yl)methyl)pi perazine | $^1H$ NMR (400 MHz, MeOD-d4) δ 8.76 (d, J = 6.0 Hz, 1H), 8.72 (s, 1H), 8.36 (d, J = 6.0 Hz, 1H), 7.52 (d, J = 8.4 Hz, 2H), 7.42 (d, J = 8.4 Hz, 2H), 3.84 (s, 2H), 3.20 - 3.15 (m, 4H), 2.68 (s, 4H). | LCMS (ESI) calcd for $C_{16}H_{18}ClN_3^+$ [M+H]+: 288.12; found, 288.2. |
| GTCO 1000 | 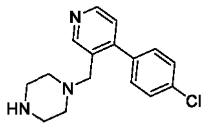 | 1-((4-(4-chloro-phenyl )pyridin-3-yl)methyl)pi pera-zine | 1-((4-(4-chloro-pheny 1)pyri-din-3-yl)methyl)pi perazine | $^1H$ NMR (400 MHz, MeOD) δ 9.46 (s, 1H), 8.98 (d, J = 6.0 Hz, 1H), 8.12 (d, J = 6.1 Hz, 1H), 7.68 (d, J = 8.6 Hz, 2H), 7.62 (d, J = 8.6 Hz, 2H), 4.50 (s, 2H), 3.48 (t, J = 5.2 Hz, 4H), 3.24 (s, 4H). | LCMS (ESI): calcd for $C_{16}H_{18}ClN_3^+$ [M+H]+: 288.12; found, 288.2. |
| GTCO 1011 | 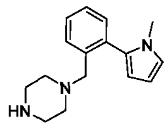 | 1-(2-(1-methyl-1H-pyrrol-2-yl)benzyl) pip erazine | 1-(2-(1-methyl-1H-pyrrol-2-yl)benzyl)pi perazine | $^1H$ NMR (400 MHz, DMSO-d6) δ 12.28 (s, 1H), 9.70 (s, 2H), 8.06 (s, 1H), 7.57 - 7.45 (m, 2H), 7.43 - 7.32 (m, 1H), 6.96 - 6.85 (m, 1H), 6.18 - 6.04 (m, 2H), 4.27 (s, 2H), 3.77 (s, 2H), 3.40 (s, 4H), 3.39 - 3.34 (m, 3H), 2.95 (d, J = 45.3 | LCMS (ESI): calcd for $C_{16}H_{21}N_3^+$ [M+H]+: 256.17; found, 256.5. |
| | | | | Hz, 2H). | |
| GTC0 1013 | 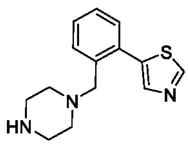 | 5-(2-(piperazin-1-ylmethyl)phe nyl) thiazole | 5-(2-(piperazin-1-ylmethyl)ph enyl) thiazole | $^1H$ NMR (400 MHz, MeOD-d4) δ 10.12 (s, 1H), 8.54 (s, 1H), 8.10 (d, J = 7.5 Hz, 1H), 7.74 (td, J = 7.5, 1.5 Hz, 1H), 7.71 - 7.66 (m, 1H), 7.64 (dd, J = 7.6,1.5 Hz, 1H), 4.66 (s, 2H), 3.65 (d, J = 4.7 Hz, 4H), 3.61 (d, J = 6.9 Hz, 4H). | LCMS (ESI) calcd for $C_{14}H_{17}N_3S^+$ [M+H]+: 260.11; found, 260.2. |

(continued)

| Comp ound No. | Structure of the compound | The compound's name | Chinese translation of the compound's name | Data of nuclear magnetic resonance hydrogen spectrum ($^1$H NMR) | Data of liquid chromatograph y-mass spectrometry (LC-MS) |
|---|---|---|---|---|---|
| GTC0 1014 | 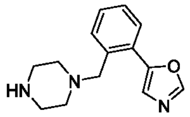 | 5-(2-(piperazin-1-ylmethyl)phe nyl) oxazole | 5-(2-(piperazin-1-ylmethyl)ph enyl) oxazole | $^1$H NMR (400 MHz, MeOD-d4) δ 8.59 (s, 1H), 7.87 (d, J = 7.5 Hz, 2H), 7.70 (s, 1H), 7.64 (dtd, J = 18.6, 7.5, 1.4 Hz, 2H), 4.79 (s, 2H), 3.73 (d, J = 21.7 Hz, 4H), 3.69 (d, J = 4.1 Hz, 4H). | LCMS (ESI): calcd for $C_{14}H_{17}N_3O^+$ $[M+H]^+$: 244.14; found, 244.5. |
| GTC0 1012 | 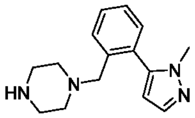 | 1-(2-(1-methyl-1H-pyrazol-5-yl)benzyl) pip erazine | 1-(2-(1-methyl-1H-pyrazol-5-yl)benzyl)pi perazine | $^1$H NMR (400 MHz, MeOD) δ 8.34 (d, J = 2.7 Hz, 1H), 8.18 (dd, J = 7.7, 1.0 Hz, 1H), 7.78 (dd, J = 23.0, 1.3 Hz, 2H), 7.61 (dd, J = 7.6, 1.3 Hz, 1H), 7.01 (d, J = 2.7 Hz, 1H), 4.58 (s, 2H), 3.96 (s, 3H), 3.69 (d, J = 4.6 Hz, 4H), 3.62 (t, J = 7.0 Hz, 4H). | LCMS (ESI) calcd for $C_{15}H_{20}N_4^+$ $[M+H]^+$: 257.17; found, 257.5. |

**Intermediate example 92: Preparation of 1-((4'-chloro-[1,1'-biphenyl]-4-yl)methyl)piperazine hydrochloride (GTC01018)**

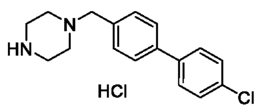

1-((4'-chloro-[1,1'-biphenyl]-4-yl)methyl)piperazine hydrochloride was prepared by referring to the method of Scheme 17.

At room temperature, in a 100 mL single-neck flask, 4'-chloro-[1,1'-biphenyl]-4-carbaldehyde (1.1 g, 5.09 mmol) and tert-butyl piperazine-1-carboxylate (1.14 g, 6.11 mmol) were dissolved in 16 mL of dichloroethane. To the solution was added sodium triacetoxyborohydride (1.62 g, 7.64 mmol). The reaction mixture was stirred at room temperature for 3 hours. After monitoring the reaction via TLC and confirming its completion, the reaction solution was quenched with water (20 mL) and extracted with ethyl acetate (20 mL × 3). The resulting organic phase was dried over anhydrous sodium sulfate, filtered, and the filtrate was concentrated under reduced pressure. The residue was purified by column chromatography (petroleum ether/ethyl acetate = 10/1 ~ 2/1) to obtain tert-butyl 4-((4'-chloro-[1,1'-biphenyl]-4-yl)methyl)piperazine-1-carboxylate (white solid, 1.4 g, yield 71.2%). LCMS (ESI): calcd for $C_{22}H_{28}ClN_2O_2^+$ $[M+H]^+$, 387.18; found, 387.3.

At room temperature, a 100 mL single-neck flask was charged with a solution of tert-butyl 4-((4'-chloro-[1,1'-biphenyl]-4-yl)methyl)piperazine-1-carboxylate (1.2 g, 3.11 mmol) in 10 mL of ethyl acetate, and a 3M solution of hydrochloric acid in ethyl acetate (10 mL). The reaction solution was stirred at room temperature for 2 hours. After monitoring the reaction via LC/MS and confirming its completion, the reaction solution was concentrated under reduced

pressure to obtain compound GTC01018 (white solid, 1.09 g, yield 98.32%). $^1$H NMR (400 MHz, $D_2O$) δ 7.71 (d, J = 8.2 Hz, 2H), 7.60 (t, J = 8.3 Hz, 4H), 7.47 (d, J = 8.5 Hz, 2H), 4.52 (s, 2H), 3.65 (s, 8H). LCMS (ESI): calcd $[M+H]^+$, 287.12; found, 287.2.

**Intermediate examples 93-137**

The following intermediate compounds were prepared by referring to the methods of Scheme 17 or Intermediate example 92.

Table 5. Intermediate examples 93-137 compounds

| Compound No. | Structure of the compound | The compound's name | Chinese translation of the compound's name | Data of nuclear magnetic resonance hydrogen spectrum ($^1H$ NMR) | Data of liquid chromatography-mass spectrometry (LC-MS) |
|---|---|---|---|---|---|
| GTC 0020 3 | 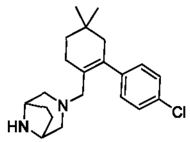 | 3-((4'-chloro-5,5-dimethyl-3,4,5,6-tetrahydro-[1,1'-biphenyl]-2-yl) methyl)-3,8-diazabicyclo[ 3.2.1]octane | 3-((4'-chloro-5,5-dimethyl-3,4,5,6-tetrahydro-[1,1'-biphenyl]-2-yl) methyl)-3,8-diazabicyclo[3 .2.1]octane | $^1H$ NMR (400 MHz, MeOD) δ 7.43 (d, *J* = 8.4 Hz, 2H), 7.14 (d, *J* = 8.4 Hz, 2H), 4.27 (d, *J* = 2.1 Hz, 2H), 3.77 (s, 2H), 3.56 (d, *J* = 13.4 Hz, 2H), 3.21 (d, *J* = 13.6 Hz, 2H), 2.53 - 2.37 (m, 4H), 2.24 (dd, *J* = 9.8, 4.8 Hz, 2H), 2.13 (s, 2H), 1.59 (t, *J* = 6.4 Hz, 2H), 1.02 (s, 6H). | LC/MS (ESI) m/z: calcd for $C_{21}H_{30}ClN_2^+$ $[M+H]^+$, 345.2; found, 345.2. |
| | 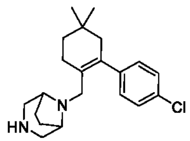 | 8-((4'-chloro-5,5-dimethyl-3,4,5,6-tetrahydro-[1,1'-biphenyl]-2-yl) methyl)-3,8-diazabicyclo[ 3.2.1]octane | 8-((4'-chloro-5,5-dimethyl-3,4,5,6-tetrahydro-[1,1'-biphenyl]-2-yl) methyl)-3,8-diazabicyclo[3 .2.1]octane | $^1H$ NMR (400 MHz, $CD_3OD$) δ 7.38 (d, *J* = 8.3 Hz, 2H), 7.13 (d, *J* = 8.3 Hz, 2H), 3.70 (s, 2H), 3.47 (d, *J* = 12.9 Hz, 2H), 3.37 (s, 2H), 3.25 (s, 2H), 2.36 (t, *J* = 6.3 Hz, 2H), 2.11 (s, 2H), 1.82 (d, *J* = 8.9 Hz, 2H), 1.66 - 1.58 (m, 2H), 1.53 (t, *J* = 6.4 Hz, 2H), 1.01 (s, 6H). | LC/MS (ESI) m/z: calcd for $C_{21}H_{30}ClN_2^+$ $[M+H]^+$, 345.2; found, 345.2. |
| GTC 0024 7 | 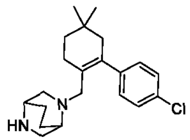 | 2-((4'-chloro-5,5-dimethyl-3,4,5,6-tetrahydro-[1,1'-biphenyl]-2-yl) methyl)-2,5-diazabicyclo[ 2.2.2]octane | 2-((4'-chloro-5,5-dimethyl-3,4,5,6-tetrahydro-[1,1'-biphenyl]-2-yl) methyl)-2,5-diazabicyclo[2 .2.2]octane | $^1H$ NMR (400 MHz, MeOD) δ 7.40 (d, *J* = 8.4 Hz, 2H), 7.11 (d, *J* = 8.3 Hz, 2H), 3.64 - 3.59 (m, 1H), 3.53 - 3.46 (m, 2H), 3.26 - 3.13 (m, 4H), 2.31 - 2.25 (m, 2H), 2.09 (s, 2H), 1.83 - 1.69 (m, 2H), 1.53 (t, *J* = 6.4 Hz, 2H), 1.37 - 1.25 (m, 3H), 1.01 (s, 6H). | LC/MS (ESI) m/z: calcd for $C_{21}H_{30}ClN_2^+$ $[M+H]^+$, 345.2; found, 345.2. |
| | 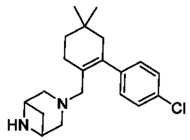 | 3-((4'-chloro-5,5-dimethyl-3,4,5,6-tetrahydro-[1,1'-biphenyl]-2-yl) methyl)-3,6-diazabicyclo[ | 3-((4'-chloro-5,5-dimethyl-3,4,5,6-tetrahydro-[1,1'-biphenyl]-2-yl) methyl)-3,6-diazabicyclo[3 | $^1H$ NMR (400 MHz, MeOD) δ 7.45 (d, *J* = 8.4 Hz, 2H), 7.19 (d, *J* = 8.4 Hz, 2H), 4.22 (d, *J* = 6.3 Hz, 2H), 3.84 (d, *J* = 43.6 Hz, 2H), 2.73 - 2.55 (m, 2H), 2.20 (d, *J* = 6.3 Hz, 2H), 2.13 (s, 3H), 2.00 (d, *J* = 12.4 Hz, 2H), 1.55 (t, *J* = 6.4 Hz, 2H), 1.36 - 1.25 (m, 1H), 1.01 (s, 6H). | LC/MS (ESI) m/z: c alcd for $C_{20}H_{28}ClN_2^+$ $[M+H]^+$, 331.19; found, 331.2. |
| | | 3.1.1]heptane | .1.1]heptane | | |
| GTC 0019 4 | 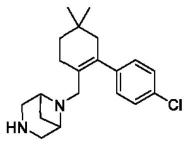 | 6-((4'-chloro-5,5-dimethyl-3,4,5,6-tetrahydro-[1,1'-biphenyl]-2-yl) methyl)-3,6-diazabicyclo[ 3.1.1]heptane | 6-((4'-chloro-5,5-dimethyl-3,4,5,6-tetrahydro-[1,1'-biphenyl]-2-yl) methyl)-3,6-diazabicyclo[3 .1.1]heptane | $^1H$ NMR (400 MHz, MeOD) δ 7.45 (d, *J* = 8.4 Hz, 2H), 7.19 (d, *J* = 8.4 Hz, 2H), 4.22 (d, *J* = 6.3 Hz, 2H), 3.84 (d, *J* = 43.6 Hz, 6H), 2.73 - 2.55 (m, 1H), 2.20 (d, *J*= 6.3 Hz, 2H), 2.13 (s, 2H), 2.00 (d, *J* = 12.4 Hz, 1H), 1.55 (t, *J* = 6.4 Hz, 2H), 1.36 - 1.25 (m, 1H), 1.01 (s, 6H). | LC/MS (ESI) m/z: calcd for $C_{20}H_{28}ClN_2^+$ $[M+H]^+$, 331.19; found, 331.2. |

(continued)

| Comp ound No. | Structure of the compound | The compound's name | Chinese translation of the compound's name | Data of nuclear magnetic resonance hydrogen spectrum ($^1$H NMR) | Data of liquid chromatogra phy-mass spectrometr y (LC-MS) |
|---|---|---|---|---|---|
| | 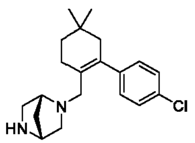 | (1R,4R)-2-((4'-chloro-5,5-dimethyl-3,4,5,6-tetrahydro-[1,1'-biphenyl]-2-yl) methyl)-2,5-diazabicyclo[ 2.2.1]heptane | (1R,4R)-2-((4'-chloro-5,5-dimethyl-3,4,5,6-tetrahydro-[1,1'-biphenyl]-2-yl) methyl)-2,5-diazabicyclo[2 .2.1]heptane | $^1$H NMR (400 MHz, MeOD) δ 7.33 (d, *J* = 8.2 Hz, 1H), 7.06 (d, *J* = 8.2 Hz, 1H), 4.06 (s, 1H), 3.48 (s, 1H), 3.06 (dd, *J* = 25.0, 12.7 Hz, 1H), 2.99 (s, 1H), 2.79 - 2.56 (m, 1H), 2.28 (d, *J* = 1.5 Hz, 1H), 2.03 (s, 1H), 1.70 (dd, *J* = 53.3, 11.2 Hz, 1H), 1.48 (t, *J* = 6.5 Hz, 1H), 0.99 (d, *J* = 1.2 Hz, 3H). | LC/MS (ESI) m/z: calcd for $C_{20}H_{28}ClN_2^+$ $[M+H]^+$, 331.19; found, 331.2. |
| GTC 0092 9 | 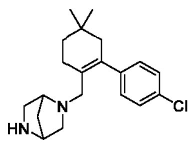 | 2-((4'-chloro-5,5-dimethyl-3,4,5,6-tetrahydro-[1,1'-biphenyl]-2-yl) methyl)-2,5-diazabicyclo[ 2.2.1]heptane | 2-((4'-chloro-5,5-dimethyl-3,4,5,6-tetrahydro-[1,1'-biphenyl]-2-yl) methyl)-2,5-diazabicyclo[2 .2.1]heptane | $^1$H NMR (400 MHz, DMSO) δ 11.85 (d, *J* = 170.8 Hz, 1H), 10.34 (d, *J* = 81.6 Hz, 1H), 9.77 (d, *J* = 77.2 Hz, 1H), 7.52 (d, *J* = 7.6 Hz, 2H), 7.28 (t, *J* = 17.6 Hz, 2H), 4.33 (t, *J* = 40.4 Hz, 2H), 4.17 - 3.78 (m, 2H), 3.62 (s, 2H), 3.28 (dd, *J* = 46.4, 10.8 Hz, 2H), 2.97 (d, *J* = 67.6 Hz, 1H), 2.63 (s, 1H), 2.24 - 1.92 (m, 4H), 1.52 (d, *J*= 5.2 Hz, 2H), 1.02 (d, *J* = 8.4 Hz, 6H). | LCMS (ESI) calcd for $C_{20}H_{28}ClN_2^+$ $[M+H]^+$: 331.19; found, 331.1. |
| | 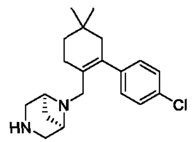 | (1R,5S)-6-((4'-chloro-5,5-dimethyl-3,4,5,6-tetrahydro-[1,1'-biphenyl]-2-yl) methyl)-3,6-diazabicyclo[ 3.1.1]heptane | (1R,5S)-6-((4'-chloro-5,5-dimethyl-3,4,5,6-tetrahydro-[1,1'-biphenyl]-2-yl) methyl)-3,6-diazabicyclo [3 .1.1]heptane | $^1$H NMR (400 MHz, MeOD) δ 7.32 (t, *J* = 9.9 Hz, 1H), 7.06 (d, *J* = 8.2 Hz, 1H), 4.06 (s, 1H), 3.48 (s, 1H), 3.13 - 2.96 (m, 2H), 2.68 (dd, *J* = 56.4, 11.3 Hz, 1H), 2.28 (s, 1H), 2.10 - 1.93 (m, 1H), 1.70 (dd, *J* = 50.7, 11.2 Hz, 1H), 1.48 (t, *J* = 6.4 Hz, 1H), 0.99 (s, 3H). | LC/MS (ESI) m/z: c alcd for $C_{20}H_{28}ClN_2^+$ $[M+H]^+$, 331.19; found, 331.2. |
| | 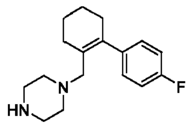 | 1-((4'-fluoro-3,4,5,6-tetrahydro-[1,1'-biphenyl]-2-yl)methyl)pip erazine | 1-((4'-fluoro-3,4,5,6-tetrahydro-[1,1'-biphenyl]-2-yl)methyl)pipe razine | $^1$H NMR (400 MHz, MeOD) δ 7.19 - 7.07 (m, 4H), 3.48 (s, 2H), 3.41 (t, *J* = 5.3 Hz, 4H), 3.06 (s, 4H), 2.36 - 2.19 (m, 4H), 1.78 (dd, *J* = 5.8, 3.0 Hz, 4H). | LC/MS (ESI) m/z: c alcd for $C_{17}H_{24}FN_2^+$ $[M+H]^+$, 275.19; found, 275.2. |
| GTC 0093 0 | 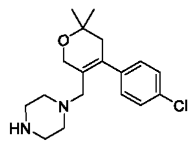 | 1-((4-(4-chlorophenyl) -6,6-dimethyl-5,6-dihydro-2H-pyran-3- | 1-((4-(4-chlorophenyl)-6,6-dimethyl-5,6-dihydro-2H-pyran-3- | $^1$H NMR (400 MHz, MeOD-d4) δ 7.37 *(d, J*= 8.4 Hz, 2H), 7.14 (d, *J* = 8.4 Hz, 2H), 4.39 (s, 2H), 3.71 (s, 2H), 3.51 (d, *J* = 5.2 Hz, 4H), 3.21 (dt, *J* = 3.2, 1.6 Hz, | LC/MS (ESI) m/z: c alcd for $C_{18}H_{26}ClN_2O^+$ $[M+H]^+$, |
| | | yl)methyl)pip erazine | yl)methyl)pipe razine | 3H), 2.26 (s, 2H), 1.93 (s, 1H), 1.21 (s, 6H). | 321.17; found, 321.2. |

(continued)

| Compound No. | Structure of the compound | The compound's name | Chinese translation of the compound's name | Data of nuclear magnetic resonance hydrogen spectrum ($^1$H NMR) | Data of liquid chromatography-mass spectrometry (LC-MS) |
|---|---|---|---|---|---|
| GTC 0093 1 | 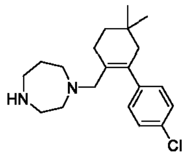 | 1-((4'-chloro-5,5-dimethyl-3,4,5,6-tetrahydro-[1,1'-biphenyl]-2-yl)methyl)-1,4-diazepane | 1-((4'-chloro-5,5-dimethyl-3,4,5,6-tetrahydro-[1,1'-biphenyl]-2-yl)methyl)-1,4-diazepane | $^1$H NMR (400 MHz, DMSO) δ 11.04 (s, 1H), 9.71 (s, 1H), 9.56 (s, 1H), 7.45 (d, *J* = 8.4 Hz, 2H), 7.16 (d, *J* = 8.4 Hz, 2H), 3.57 (s, 4H), 3.25 (dd, *J* = 27.2, 10.8 Hz, 4H), 3.13 - 2.94 (m, 2H), 2.46 - 2.29 (m, 2H), 2.23 (dd, *J* = 17.2, 8.8 Hz, 1H), 2.01 (d, *J* = 21.6 Hz, 2H), 1.97 - 1.84 (m, 1H), 1.46 (t, *J* = 6.4 Hz, 2H), 0.96 (s, 6H). | LCMS (ESI): calcd for $C_{20}H_{30}ClN_2^+$ [M+H]+: 333.25, found, 333.3. |
| GTC 0019 4 | 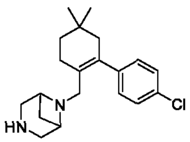 | 6-((4'-chloro-5,5-dimethyl-3,4,5,6-tetrahydro-[1,1'-biphenyl]-2-yl)methyl)-3,6-diazabicyclo[ 3.1.1]heptane | 6-((4'-chloro-5,5-dimethyl-3,4,5,6-tetrahydro-[1,1'-biphenyl]-2-yl)methyl)-3,6-diazabicyclo[3 .1.1]heptane | $^1$H NMR (400 MHz, DMSO) δ 7.34 (d, *J* = 8.4 Hz, 2H), 7.11 (d, *J* = 8.4 Hz, 2H), 3.35 (d, *J* = 5.6 Hz, 2H), 3.10 (q, *J* = 12.4 Hz, 4H), 2.81 (s, 2H), 2.31 (dd, *J* = 13.6, 6.4 Hz, 1H), 2.19 (d, *J* = 6.0 Hz, 2H), 1.95 (s, 2H), 1.38 (t, *J* = 6.4 Hz, 2H), 1.28 (d, *J* = 8.4 Hz, 1H), 0.93 (s, 6H). | LCMS (ESI) calcd for $C_{20}H_{28}ClN_2^+$ [M+H]+: 331.19; found, 331.2. |
| GTC 0020 9 | 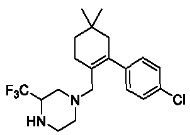 | 1-((4'-chloro-5,5-dimethyl-3,4,5,6-tetrahydro-[1,1'-biphenyl]-2-yl)methyl)-3-(trifluoromethyl)piperazine | 1-((4'-chloro-5,5-dimethyl-3,4,5,6-tetrahydro-[1,1'-biphenyl]-2-yl)methyl)-3-(trifluoromethyl)piperazine | $^1$H NMR (400 MHz, MeOD-d4) δ 7.30 (d, *J* = 8.4 Hz, 2H), 7.00 (d, *J* = 8.4 Hz, 2H), 3.97 (s, 1H), 3.40 (s, 2H), 3.28 (d, *J* = 12.8 Hz, 1H), 3.15 (d, *J* = 12.8 Hz, 1H), 3.06 (t, *J* = 12.4 Hz, 1H), 2.52 - 2.30 (m, 2H), 2.23 (d, *J* = 4.4 Hz, 2H), 2.02 (s, 2H), 1.46 (t, *J* = 6.4 Hz, 2H), 1.22 (d, *J* = 20.0 Hz, 1H), 0.92 (d, *J* = 3.2 Hz, 6H). | LCMS (ESI) calcd for $C_{20}H_{27}ClF_3N_2^+$ [M+H]+: 389.17; found, 388.9. |
| GTC 0093 2 | 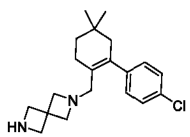 | 2-((4'-chloro-5,5-dimethyl-3,4,5,6-tetrahydro-[1,1'-biphenyl]-2-yl)methyl)-2,6-diazaspiro [3.3 ]heptane | 2-((4'-chloro-5,5-dimethyl-3,4,5,6-tetrahydro-[1,1'-biphenyl]-2-yl)methyl)-2,6-diazaspiro [3.3 ]heptane | $^1$H NMR (400 MHz, MeOD-d4) δ 7.44 (d, *J* = 8.4 Hz, 2H), 7.15 (d, *J* = 8.4 Hz, 2H), 4.21 (s, 8H), 3.72 (s, 2H), 2.18 (t, *J* = 6.0 Hz, 2H), 2.13 (s, 2H), 1.55 (t, *J* = 6.4 Hz, 2H), 1.03 (s, 6H). | LCMS (ESI) calcd for $C_{20}H_{28}ClN_2^+$ [M+H]+: 331.19; found, 331.2. |
| GTC 0093 3 | 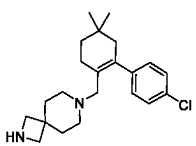 | 7-((4'-chloro-5,5-dimethyl-3,4,5,6-tetrahydro-[1,1'-biphenyl]-2-yl)methyl)-2,7-diazaspiro [3.5 ]nonane | 7-((4'-chloro-5,5-dimethyl-3,4,5,6-tetrahydro-[1,1'-biphenyl]-2-yl)methyl)-2,7-diazaspiro [3.5 ]nonane | $^1$H NMR (400 MHz, MeOD-d4) δ 7.49 - 7.36 (m, 2H), 7.21 - 7.08 (m, 2H), 3.90 (s, 4H), 3.66 (s, 2H), 3.38 (s, 1H), 2.72 (s, 1H), 2.35 (t, *J* = 6.4 Hz, 2H), 2.28 - 2.08 (m, 6H), 2.01 (s, 2H), 1.61 (t, *J* = 6.4 Hz, 2H), 1.05 (s, 6H). | LCMS (ESI) calcd for $C_{22}H_{32}ClN_2^+$ [M+H]+: 359.22; found, 359.6. |

(continued)

| Compound No. | Structure of the compound | The compound's name | Chinese translation of the compound's name | Data of nuclear magnetic resonance hydrogen spectrum ($^1$H NMR) | Data of liquid chromatography-mass spectrometry (LC-MS) |
|---|---|---|---|---|---|
| GTC 0093 4 | 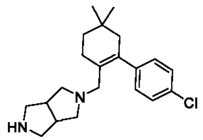 | 2-((4'-chloro-5,5-dimethyl-3,4,5,6-tetrahydro-[1,1'-biphenyl]-2-yl) methyl)oct ahydropyrrolo [3,4-c]pyrrole | 2-((4'-chloro-5,5-dimethyl-3,4,5,6-tetrahydro-[1,1'-biphenyl]-2-yl) methyl)octa hydropyrrolo[ 3,4-c]pyrrole | $^1$H NMR (400 MHz, DMSO) δ 11.05 (s, 1H), 9.74 (d, *J* = 122.4 Hz, 2H), 7.43 (t, *J* = 8.4 Hz, 2H), 7.16 (d, *J* = 8.4 Hz, 2H), 3.61 *(d, J*= 8.0 Hz, 3H), 3.44 *(d, J*= 12.4 Hz, 2H), 3.28 (s, 1H), 3.21 - 3.09 (m, 3H), 3.02 (s, 2H), 2.84 (d, *J* = 8.8 Hz, 1H), 2.37 (d, *J* = 19.2 Hz, 2H), 2.02 (s, 2H), 1.45 (t, *J* = 5.6 Hz, 2H), 0.96 (s, 6H). | LCMS (ESI) calcd for $C_{21}H_{30}ClN_2O^+$ $[M+H]^+$: 345.20; found, 345.2. |
| GTC 0095 2 | 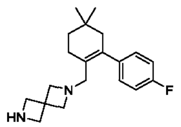 | 2-((4'-fluoro-5,5-dimethyl-3,4,5,6-tetrahydro-[1,1'-biphenyl]-2-yl) methyl)-2,6-diazaspiro [3.3 Theptane | 2-((4'-fluoro-5,5-dimethyl-3,4,5,6-tetrahydro-[1,1'-biphenyl]-2-yl) methyl)-2,6-diazaspiro [3.3 ]heptane | $^1$H NMR (400 MHz, MeOD) δ 7.13 (dd, J = 11.0, 5.0 Hz, 4H), 4.32 (d, J = 44.3 Hz, 4H), 4.17 - 3.86 (m, 4H), 3.71 (s, 2H), 2.14 (dd, J = 17.1, 10.9 Hz, 4H), 1.52 (t, J = 6.4 Hz, 2H), 1.00 (s, 6H). | LCMS (ESI) calcd for $C_{19}H_{26}ClN_2O^+$ $[M+H]^+$: 315.22, found, 315.4. |
| GTC 0095 1 | 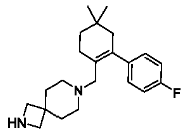 | 7-((4'-fluoro-5,5-dimethyl-3,4,5,6-tetrahydro-[1,1'-biphenyl]-2-yl) methyl)-2,7-diazaspiro [3.5 ]nonane | 7-((4'-fluoro-5,5-dimethyl-3,4,5,6-tetrahydro-[1,1'-biphenyl]-2-yl) methyl)-2,7-diazaspiro [3.5 ]nonane | $^1$H NMR (400 MHz, MeOD) δ 7.13 (dd, J = 9.7, 7.6 Hz, 4H), 3.87 (d, J = 5.5 Hz, 4H), 3.64 (s, 2H), 3.38 (d, J = 13.0 Hz, 2H), 2.66 (t, J = 11.4 Hz, 2H), 2.32 (d, J = 5.9 Hz, 2H), 2.20 (t, J = 13.7 Hz, 3H), 2.12 (d, J = 8.8 Hz, 3H), 1.57 (t, J = 6.3 Hz, 2H), 1.01 (s, 6H). | LCMS (ESI) calcd for $C_{22}H_{32}ClFN_2^+$ $[M+H]^+$: 343.25, found, 343.3. |
| GTC 0095 3 | 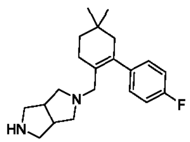 | 2-((4'-fluoro-5,5-dimethyl-3,4,5,6-tetrahydro-[1,1'-biphenyl]-2 yl) methyl)oct ahydropyrrolo [3,4-c]pyrrole | 2-((4'-fluoro-5,5-dimethyl-3,4,5,6-tetrahydro-[1,1'-biphenyl]-2 yl) methyl)octa hydropyrrolo[ 3,4-c]pyrrole | $^1$H NMR (400 MHz, MeOD) δ 7.23 - 7.07 (m, 4H), 3.83 (d, J = 10.9 Hz, 2H), 3.73 (s, 1H), 3.64 - 3.57 (m, 1H), 3.53 (d, J = 12.0 Hz, 1H), 3.50 - 3.43 (m, 1H), 3.35 (s, 3H), 3.18 (d, J = 7.6 Hz, 2H), 2.88 (s, 1H), 2.43 (t, J = 6.2 Hz, 1H), 2.35 (s, 1H), 2.12 (s, 2H), 1.58 (dd, J = 6.4, 5.0 Hz, 2H), 1.03 (s, 6H). | LCMS (ESI) calcd for $C_{21}H_{29}FN_2^+$ $[M+H]^+$: 329.23; found, 329.3. |
| GTC 0095 6 | 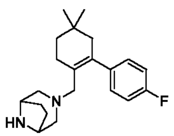 | 3-((4'-fluoro-5,5-dimethyl-3,4,5,6-tetrahydro-[1,1'-biphenyl]-2-yl) methyl)-3,8-diazabicyclo[ 3.2.1]octane | 3-((4'-fluoro-5,5-dimethyl-3,4,5,6-tetrahydro-[1,1'-biphenyl]-2-yl) methyl)-3,8-diazabicyclo[3 .2.1]octane | $^1$H NMR (400 MHz, MeOD) δ 7.14 (d, J = 7.0 Hz, 4H), 4.23 (s, 2H), 3.71 (s, 2H), 3.48 (d, J = 1.6 Hz, 2H), 3.25 - 2.91 (m, 2H), 2.38 (s, 4H), 2.22 (s, 2H), 2.12 (s, 2H), 1.58 (t, J = 6.3 Hz, 2H), 1.02 (s, 6H). | LCMS (ESI) calcd for $C_{21}H_{30}ClFN_2^+$ $[M+H]^+$: 329.23; found, 329.3. |

(continued)

| Compound No. | Structure of the compound | The compound's name | Chinese translation of the compound's name | Data of nuclear magnetic resonance hydrogen spectrum ($^1$H NMR) | Data of liquid chromatography-mass spectrometry (LC-MS) |
|---|---|---|---|---|---|
| GTC 0095 7 | 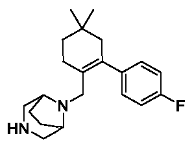 | 8-((4'-fluoro-5,5-dimethyl-3,4,5,6-tetrahydro-[1,1'-biphenyl]-2-yl)methyl)-3,8- | 8-((4'-fluoro-5,5-dimethyl-3,4,5,6-tetrahydro-[1,1'-biphenyl]-2-yl)methyl)-3,8- | $^1$H NMR (400 MHz, MeOD) δ 7.34 - 7.21 (m, 2H), 7.17 (d, J = 8.7 Hz, 2H), 4.12 (d, J = 14.5 Hz, 4H), 3.74 (s, 2H), 3.49 (d, J = 13.3 Hz, 2H), 2.52 (t, J = 6.0 Hz, 2H), 2.25 - 1.99 (m, 4H), 1.60 (t, J = 6.3 Hz, 4H), 1.03 (s, 6H). | LCMS (ESI) calcd for $C_{21}H_{30}ClFN_2^+$ [M+H]$^+$: 329.23; found, 329.2. |
| | | diazabicyclo[ 3.2.1]octane | diazabicyclo [3 .2.1]octane | | |
| GTC 0095 9 | 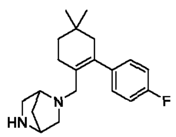 | 2-((4'-fluoro-5,5-dimethyl-3,4,5,6-tetrahydro-[1,1'-biphenyl]-2-yl)methyl)-2,5-diazabicyclo[ 2.2.1]heptane | 2-((4'-fluoro-5,5-dimethyl-3,4,5,6-tetrahydro-[1,1'-biphenyl]-2-yl)methyl)-2,5-diazabicyclo[2 .2.1]heptane | $^1$H NMR (400 MHz, MeOD) δ 7.32 - 7.10 (m, 4H), 4.45 (s, 1H), 4.37 (s, 1H), 3.86 (d, J = 12.9 Hz, 2H), 3.76 (d, J = 15.7 Hz, 1H), 3.59 - 3.32 (m, 2H), 2.38 (d, J = 36.9 Hz, 2H), 2.16 (d, J = 12.7 Hz, 2H), 2.01 (d, J = 3.6 Hz, 1H), 1.99 (s, 2H), 1.59 (t, J = 6.3 Hz, 2H), 1.03 (d, J = 5.3 Hz, 6H). | LCMS (ESI) calcd for $C_{20}H_{28}ClFN_2^+$ [M+H]$^+$: 315.22; found, 315.3. |
| GTC 0094 9 | 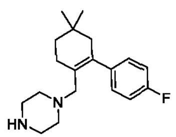 | 1-((4'-fluoro-5,5-dimethyl-3,4,5,6-tetrahydro-[1,1'-biphenyl]-2-yl)methyl)pip erazine | 1-((4'-fluoro-5,5-dimethyl-3,4,5,6-tetrahydro-[1,1'-biphenyl]-2-yl)methyl)pipe razine | $^1$H NMR (400 MHz, MeOD) δ 7.12 (d, J = 7.1 Hz, 4H), 3.52 (s, 2H), 3.42 (t, J = 4.6 Hz, 4H), 3.08 (s, 4H), 2.28 (s, 2H), 2.12 (s, 2H), 1.54 (t, J = 6.3 Hz, 2H), 1.02 (s, 6H). | LCMS (ESI) calcd for $C_{19}H_{27}FN_2^+$ [M+H]$^+$: 303.22, found, 303.3. |
| GTC 0095 0 | 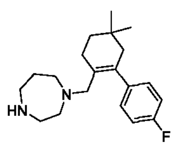 | 1-((4'-fluoro-5,5-dimethyl-3,4,5,6-tetrahydro-[1,1'-biphenyl]-2-yl)methyl)-1,4-diazepane | 1-((4'-fluoro-5,5-dimethyl-3,4,5,6-tetrahydro-[1,1'-biphenyl]-2-yl)methyl)-1,4-diazepane | $^1$H NMR (400 MHz, MeOD) δ 7.36 - 7.04 (m, 4H), 3.78 (s, 2H), 3.68(s, 2H), 3.52 (s, 1H), 3.38 (s, 2H), 3.31 - 3.01 (m, 2H), 2.38 (t, $J$ = 5.9 Hz, 2H), 2.26 (d, $J$ = 36.3 Hz, 1H), 2.16 (s, 3H), 1.98 (s, 1H), 1.58 (t, $J$ = 6.3 Hz, 2H), 1.04 (s, 6H). | LCMS (ESI) calcd for $C_{20}H_{29}FN_2^+$ [M+H]$^+$: 317.23; found, 317.7. |

(continued)

| Compound No. | Structure of the compound | The compound's name | Chinese translation of the compound's name | Data of nuclear magnetic resonance hydrogen spectrum ($^1H$ NMR) | Data of liquid chromatography-mass spectrometr y (LC-MS) |
|---|---|---|---|---|---|
| GTC 0095 5 | 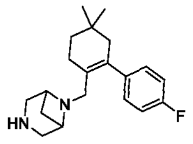 | 6-((4'-fluoro-5,5-dimethyl-3,4,5,6-tetrahydro-[1,1'-biphenyl]-2-yl) methyl)-3,6-diazabicyclo[ 3.1.1]heptane | 6-((4'-fluoro-5,5-dimethyl-3,4,5,6-tetrahydro-[1,1'-biphenyl]-2-yl) methyl)-3,6-diazabicyclo[3 .1.1]heptane | $^1H$ NMR (400 MHz, MeOD) δ 7.30 - 7.23 (m, 2H), 7.18 (t, J = 8.9 Hz, 2H), 4.16 (dd, J = 64.2, 6.7 Hz, 3H), 3.94 - 3.73 (m, 3H), 3.52 (dd, J = 27.1, 25.0 Hz, 2H), 3.18 (dd, J = 25.5, 14.1 Hz, 1H), 2.64 (s, 1H), 2.34 - 2.27 (m, 2H), 2.16 (d, J = 17.8 Hz, 3H), 1.56 (t, J = 6.3 Hz, 2H), 1.06 - 0.99 (m, 6H). | LCMS (ESI) calcd for $C_{20}H_{27}FN_2^+$ $[M+H]^+$: 315.22; found, 315.7. |
| GTC 0095 4 | 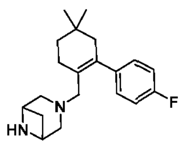 | 3-((4'-fluoro-5,5-dimethyl-3,4,5,6-tetrahydro-[1,1'-biphenyl]-2-yl) methyl)-3,6-diazabicyclo[ 3.1.1]heptane | 3-((4'-fluoro-5,5-dimethyl-3,4,5,6-tetrahydro-[1,1'-biphenyl]-2-yl) methyl)-3,6-diazabicyclo[3 .1.1]heptane | $^1H$ NMR (400 MHz, MeOD) δ 7.18 (dq, *J* = 17.2, 8.5 Hz, 4H), 4.44 (d, *J* = 6.1 Hz, 2H), 4.12 (d, *J* = 13.0 Hz, 2H), 4.01 (s, 2H), 3.61 (s, 2H), 3.15 - 3.04 (m, 1H), 2.80 (t, *J* = 20.3 Hz, 1H), 2.46 (s, 2H), 2.14 (s, 2H), 1.59 (t, *J* = 6.3 Hz, 2H), 1.03 (s, 6H). | LCMS (ESI) calcd for $C_{20}H_{27}FN_2^+$ $[M+H]^+$: 315.22; found, 315.2. |
| GTC 0095 8 | 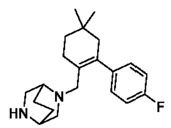 | 2-((4'-fluoro-5,5-dimethyl-3,4,5,6-tetrahydro-[1,1'-biphenyl]-2-yl) methyl)-2,5-diazabicyclo[ 2.2.2]octane | 2-((4'-fluoro-5,5-dimethyl-3,4,5,6-tetrahydro-[1,1'-biphenyl]-2-yl) methyl)-2,5-diazabicyclo[2 .2.2]octane | $^1H$ NMR (400 MHz, MeOD) δ 7.28 - 7.13 (m, 4H), 4.02 - 3.76 (m, 4H), 3.68 (s, 1H), 3.12 (s, 2H), 2.48 (s, 1H), 2.40 (s, 2H), 2.18 (d, *J* = 19.0 Hz, 3H), 2.02 (d, *J* = 3.6 Hz, 1H), 1.98 (s, 2H), 1.58 (t, *J* = 6.1 Hz, 2H), 1.04 (s, 6H). | LCMS (ESI) calcd for $C_{21}H_{29}FN_2^+$ $[M+H]^+$: 329.23; found, 329.3. |
| GTC 0096 0 | 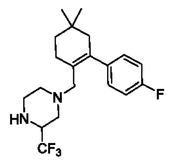 | 1-((4'-fluoro-5,5-dimethyl-3,4,5,6-tetrahydro-[1,1'-biphenyl]-2-yl) methyl)-3-(trifluoromethyl)piperazine | 1-((4'-fluoro-5,5-dimethyl-3,4,5,6-tetrahydro-[1,1'-biphenyl]-2-yl) methyl)-3-(trifluoromethyl)piperazine | $^1H$ NMR (400 MHz, MeOD) δ 14.16 - 14.12 (m, 1H), 7.04 (d, *J* = 7.1 Hz, 4H), 4.44 (s, 1H), 3.58 (dt, *J* = 12.9, 9.3 Hz, 3H), 3.40 (s, 3H), 2.76 (t, *J* = 12.2 Hz, 1H), 2.64 (s, 1H), 2.44 - 2.20 (m, 2H), 2.04 (d, *J* = 8.2 Hz, 2H), 1.48 (t, *J* = 6.3 Hz, 2H), 0.94 (d, *J* = 4.8 Hz, 6H). | LCMS (ESI) calcd for $C_{20}H_{26}F_4N_2^+$ $[M+H]^+$: 371.2; found, 371.7. |
| GTC 0097 6 | 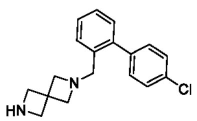 | 2-((4'-chloro-[1,1'-biphenyl]-2-yl)methyl)-2,6-diazaspiro[3.3 ]heptane | 2-((4'-chloro-[1,1'-biphenyl]-2-yl)methyl)-2,6-diazaspiro[3.3 ]heptane | $^1H$ NMR (400 MHz, MeOD-d4) δ 7.56 - 7.50 (m, 5H), 7.39 - 7.33 (m, 3H), 4.44 (s, 2H), 4.20 (d, J = 17.2 Hz, 8H). | LCMS (ESI) calcd for $C_{18}H_{19}ClN_2^+$ $[M+H]^+$: 299.12; found, 299.0. |

(continued)

| Comp ound No. | Structure of the compound | The compound's name | Chinese translation of the compound's name | Data of nuclear magnetic resonance hydrogen spectrum ($^1$H NMR) | Data of liquid chromatogra phy-mass spectrometr y (LC-MS) |
|---|---|---|---|---|---|
| GTC 0098 4 | 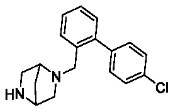 | 2-((4'-chloro-[1,1'-biphenyl]-2-yl)methyl)-2,5-diazabicyclo[ 2.2.1]heptane | 2-((4'-chloro-[1,1'-biphenyl]-2-yl)methyl)-2,5-diazabicyclo[2 .2.1]hep-tane | $^1$H NMR (400 MHz, MeOD-d4) δ 7.99 (d, J = 4.6 Hz, 1H), 7.62 - 7.56 (m, 4H), 7.47 - 7.41 (m, 3H), 4.58 (s, 2H), 4.50 (s, 1H), 4.20 (s, 1H), 3.90 (s, 2H), 3.38 (d, J = 35.7 Hz, 2H), 2.02 (t, J = 6.0 Hz, 1H), 1.62 (s, 1H). | LCMS (ESI) calcd for $C_{18}H_{19}ClN_2^+$ $[M+H]^+$: 299.12; found, 300.8. |
| GTC 0097 3 | 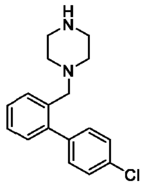 | 1-((4'-chloro-[1,1'-biphenyl]-2-yl)methyl)pip erazine | 1-((4'-chloro-[1,1'-biphenyl]-2-yl)methyl)pipe ra-zine | $^1$H NMR(400 MHz, MeOD) δ 7.94 - 7.85 (m, 1H), 7.63 - 7.49 (m, 4H), 7.40 (dd,J = 12.0, 6.4 Hz, 3H),4.84 - 4.80 (m, 4H), 4.46 (s, 2H), 3.51 (t,J = 4.8 Hz, 4H). | LCMS (ESI) calcd for $C_{17}H_{19}ClN_2^+$ $[M+H]^+$: 287.12, found, 287.2. |
| GTC 0097 4 | 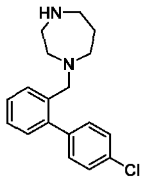 | 1-((4'-chloro-[1,1'-biphenyl]-2-yl)methyl)-1,4-diazepane | 1-((4'-chloro-[1,1'-biphenyl]-2-yl)methyl)-1,4-diazepane | $^1$H NMR(400 MHz, MeOD) δ 7.95 - 7.90 (m, 1H), 7.60 - 7.52 (m, 4H), 7.43 - 7.35 (m, 3H), 4.50 (s,2H), 3.58 (s, 4H), 3.33 (t,J = 13.2 Hz, 5H), 3.09 (s, 1H), 2.11 (s, 2H). | LCMS (ESI) calcd for $C_{18}H_{21}ClN_2^+$ $[M+H]^+$: 301.14; found, 301.2. |
| GTC 0097 5 | 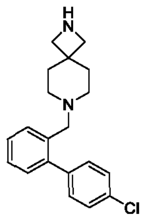 | 7-((4'-chloro-[1,1'-biphenyl]-2-yl)methyl)-2,7-diazaspiro[3.5 ]nonane | 7-((4'-chloro-[1,1'-biphenyl]-2-yl)methyl)-2,7-diazaspiro[3.5 ]nonane | $^1$H NMR(400 MHz, MeOD) δ 7.85 (dd,J = 5.2, 3.6 Hz, 1H), 7.59 - 7.50 (m, 4H), 7.41 - 7.32 (m, 3H),4.37 (s, 2H), 3.89 (s, 2H), 3.84 (s, 2H), 3.32 (d,J = 9.2 Hz, 2H), 2.75 (t,J = 12.0 Hz, 2H), 2.20 (d,J =14.4 Hz, 2H), 2.05 (dd,J = 18.4, 5.6 Hz, 2H). | LCMS (ESI) calcd for $C_{20}H_{23}ClN_2^+$ $[M+H]^+$: 327.15; found, 327.2. |

(continued)

| Compound No. | Structure of the compound | The compound's name | Chinese translation of the compound's name | Data of nuclear magnetic resonance hydrogen spectrum ($^1$H NMR) | Data of liquid chromatography-mass spectrometry (LC-MS) |
|---|---|---|---|---|---|
| GTC 0097 7 | 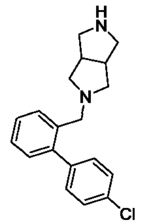 | 2-((4'-chloro-[1,1'-biphenyl]-2-yl)methyl)octahydropyrrolo [3,4-c]pyrrole | 2-((4'-chloro-[1,1'-biphenyl]-2-yl)methyl)octahydropyrrolo[ 3,4-c]pyrrole | $^1$H NMR(400 MHz, MeOD) δ 8.07 - 7.76 (m, 1H), 7.54 (dd,J = 8.8, 5.6 Hz, 4H), 7.38 (d,J = 8.0 Hz,3H), 4.51 (d,J = 24.8 Hz, 2H), 3.76 (d,J = 5.6 Hz, 1H), 3.51 (dd,J = 14.0, 10.4 Hz, 3H), 3.41 - 3.32 (m,3H), 3.25 (s, 1H), 3.18 - 3.06 (m, 2H). | LCMS (ESI) calcd for $C_{19}H_{21}ClN_2^+$ [M+H]$^+$: 313.14; found, 313.2. |
| GTC 0097 8 | 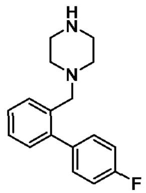 | 1-((4'-fluoro-[1,1'-biphenyl]-2-yl)methyl)piperazine | 1-((4'-fluoro-[1,1'-biphenyl]-2-yl)methyl)piperazine | $^1$H NMR(400 MHz, MeOD) δ 7.93 - 7.85 (m, 1H), 7.60 - 7.53 (m, 2H), 7.41 (dd,J = 8.0, 4.8 Hz, 3H),7.27 (t,J = 8.8 Hz, 2H), 4.84 (s, 4H), 4.49 (s, 2H), 3.52 (t,J = 5.2 Hz, 4H). | LCMS (ESI) calcd for $C_{17}H_{19}FN_2^+$ [M+H]$^+$: 271.15; found, 271.5. |
| GTC 0097 9 | 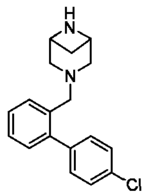 | 3-((4'-chloro-[1,1'-biphenyl]-2-yl)methyl)-3,6-diazabicyclo[ 3.1.1]heptane | 3-((4'-chloro-[1,1'-biphenyl]-2-yl)methyl)-3,6-diazabicyclo[3 .1.1]heptane | $^1$H NMR(400 MHz, MeOD) δ 8.12 (dd,J = 5.6, 3.6 Hz, 1H), 7.59 - 7.51 (m, 4H), 7.40 (dd,J = 8.8, 2.4Hz, 3H), 4.69 (s, 2H), 4.40 (d,J = 6.4 Hz, 2H), 3.98 (d,J = 13.6 Hz, 2H), 3.64 (d,J = 13.6 Hz, 2H), 3.03(s, 1H), 2.65 (d,J = 10.8 Hz, 1H). | LCMS (ESI) calcd for $C_{18}H_{19}ClN_2^+$ [M+H]$^+$: 299.12; found, 299.2. |
| GTC 0098 0 | 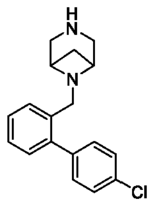 | 6-((4'-chloro-[1,1'-biphenyl]-2-yl)methyl)-3,6-diazabicyclo[ 3.1.1]heptane | 6-((4'-chloro-[1,1'-biphenyl]-2-yl)methyl)-3,6-diazabicyclo[3 .1.1]heptane | $^1$H NMR(400 MHz, MeOD-d4) δ 7.83 (s, 1H), 7.61 - 7.51 (m, 4H), 7.47 - 7.37 (m, 3H), 4.85 - 4.83 (m,1H), 4.71 (s, 2H), 4.21 (s, 3H), 3.84 (s, 2H), 2.65 (s, 1H), 2.14 (s, 1H). | LCMS (ESI) calcd for $C_{18}H_{19}ClN_2^+$ [M+H]$^+$: 299.12; found, 299.2. |

(continued)

| Compound No. | Structure of the compound | The compound's name | Chinese translation of the compound's name | Data of nuclear magnetic resonance hydrogen spectrum ($^1H$ NMR) | Data of liquid chromatography-mass spectrometry (LC-MS) |
|---|---|---|---|---|---|
| GTC 0098 1 | 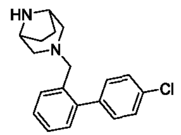 | 3-((4'-chloro-[1,1'-biphenyl]-2-yl)methyl)-3,8-diazabicyclo[ 3.2.1]octane | 3-((4'-chloro-[1,1'-biphenyl]-2-yl)methyl)-3,8-diazabicyclo[3 .2.1]octane | $^1H$ NMR(400 MHz, MeOD-d4) δ 7.93 (s, 1H), 7.59 - 7.49 (m, 4H), 7.42 - 7.35 (m, 3H), 4.37 (s, 2H),4.15 (s, 2H), 3.66 (s, 2H), 3.07 (d,J = 10.8 Hz, 2H), 2.30 (s, 2H), 2.20 - 2.07 (m, 2H). | LCMS (ESI) calcd for $C_{19}H_{21}ClN_2^+$ $[M+H]^+$: 313.14; found, 313.2. |
| GTC 0098 2 | 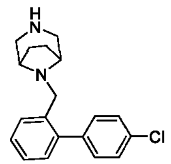 | (1R,5S)-8-((4'-chloro-[1,1'-biphenyl]-2-yl)methyl)-3,8-diazabicyclo[ 3.2.1]octane | (1R,5S)-8-((4'-chloro-[1,1'-biphenyl]-2-yl)methyl)-3,8-diazabicyclo[3 .2.1]octane | $^1H$ NMR(400 MHz, MeOD) δ 8.12 (s, 1H), 7.57 (dd,J = 10.8, 6.4 Hz, 4H), 7.43 (dd,J = 8.4, 6.8 Hz,3H), 4.45 (s, 2H), 4.02 (s, 2H), 3.90 (d,J = 13.6 Hz, 2H), 3.44 (d,J = 13.6 Hz, 2H), 2.08 (d,J = 9.6 Hz,2H), 1.63 (d,J = 25.2 Hz, 2H). | LCMS (ESI): calcd for $C_{19}H_{21}ClN_2^+$ $[M+H]^+$: 313.14; found, 313.2. |
| GTC 0098 3 | 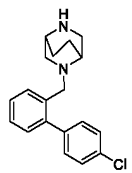 | 2-((4'-chloro-[1,1'-biphenyl]-2-yl)methyl)-2,5-diazabicyclo[ 2.2.2]octane | 2-((4'-chloro-[1,1'-biphenyl]-2-yl)methyl)-2,5-diazabicyclo[2 .2.2]octane | $^1H$ NMR(400 MHz, MeOD-d4) δ 8.04 (dd,J = 6.4, 2.8 Hz, 1H), 7.61 - 7.54 (m, 4H), 7.45 - 7.38 (m,3H), 4.64 (s, 2H), 3.88 (s, 2H), 3.54 (d,J = 46.8 Hz, 2H), 2.96 (d,J = 182.2 Hz, 2H), 2.16 (s, 2H), 2.01 (s,2H). | LCMS (ESI) calcd for $C_{19}H_{21}ClN_2^+$ $[M+H]^+$ : 313.14; found, 313.2. |
| GTC 0098 5 | 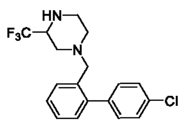 | 1-((4'-chloro-[1,1'-biphenyl]-2-yl)methyl)-3-(trifluorometh yl)piperazine | 1-((4'-chloro-[1,1'-biphenyl]-2-yl)methyl)-3-(trifluorometh yl)piperazine | $^1H$ NMR(400 MHz, MeOD-d4) δ 7.75 (dd,J = 5.6, 3.2 Hz, 1H), 7.55 - 7.46 (m, 4H), 7.41 - 7.33 (m,3H), 4.43 - 4.31 (m, 1H), 4.16 (dd,J = 36.4, 13.6 Hz, 2H), 3.44 (d,J = 13.2 Hz, 2H), 3.30 - 3.25 (m, 1H),3.19 (d,J = 13.2 Hz, 1H), 2.82 - 2.59 (m, 2H). | LCMS (ESI) calcd for $C_{18}H_{18}ClF_3 + N_2$ $[M+H]^+$: 355.11; found, 355.2. |
| GTC 0100 1 | 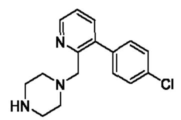 | 1-((3-(4-chlorophenyl) pyridin-2-yl)methyl)piperazine | 1-((3-(4-chlorophenyl) pyridin-2-yl)methyl)piperazine | $^1H$ NMR(400 MHz, MeOD-d4) δ 8.90 (d,J = 5.2 Hz, 1H), 8.41 (d,J = 7.8 Hz, 1H), 8.01 (dd,J = 7.6,5.6 Hz, 1H), 7.60 (d,J = 8.2 Hz, 2H), 7.52 (d,J = 8.2 Hz, 2H), 4.25 (s, 2H), 3.50 - 3.42 (m, | LCMS (ESI) calcd for $C_{16}H_{18}ClN_3^+$ $[M+H]^+$: 288.12; found, |
| | | | | 4H), 3.14 -3.03 (m, 4H). | 288.1. |

(continued)

| Compound No. | Structure of the compound | The compound's name | Chinese translation of the compound's name | Data of nuclear magnetic resonance hydrogen spectrum ($^1$H NMR) | Data of liquid chromatography-mass spectrometry (LC-MS) |
|---|---|---|---|---|---|
| GTC 0102 6 | 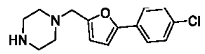 | 1-((5-(4-chlorophenyl) furan-2-yl)methyl)piperazine | 1-((5-(4-chlorophenyl)furan-2-yl)methyl)piperazine | $^1$H NMR (400 MHz, MeOD-d4) δ 7.90 - 7.71 (m, 2H), 7.55 - 7.40 (m, 2H), 7.05 - 6.81 (m, 2H), 4.66 (s, 2H), 3.66 (s, 8H). | LCMS (ESI): calcd for $C_{15}H_{18}N_4^+$ $[M+H]^+$: 277.10, found, 277.1. |
| GTC 0092 5 | 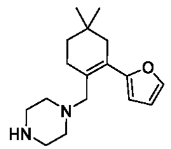 | 1-((2-(furan-2-yl)-4,4-dimethylcyclo hex-1-en-1-yl)methyl)piperazine | 1-((2-(furan-2-yl)-4,4-dimethylcyclo hex-1-en-1-yl)methyl)piperazine | $^1$H NMR (400 MHz, MeOD-d4) δ 7.59 (d, *J* = 1.6 Hz, 1H), 6.50 (d, *J* = 3.6 Hz, 1H), 6.43 (dd, *J* = 3.6, 1.6 Hz, 1H), 4.20 (s, 2H), 3.57 (s, 8H), 2.37 (s, 2H), 2.18 (s, 2H), 1.45 (d, *J* = 6.4 Hz, 2H), 0.92 (s, 6H). | LCMS (ESI): calcd for $C_{17}H_{27}N_2O^+$ $[M+H]^+$, 275.20, found, 275.1. |
| GTC 0092 7 | 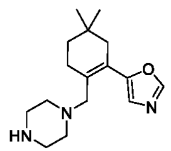 | 5-(5,5-dimethyl-2-(piperazin-1-ylmethyl)cyclohex-1-en-1-yl)oxazole | 5-(5,5-dimethyl-2-(piperazin-1-ylmethyl)cyclohex-1-en-1-yl)oxazole | $^1$H NMR (400 MHz, MeOD-d4) δ 8.89 (s, 1H), 7.64 (s, 1H), 4.32 (s, 2H), 3.79 (s, 8H), 2.60 (s, 2H), 2.32 (s, 2H), 1.60 (s, 2H), 1.05 (s, 6H). | LCMS (ESI): calcd for $C_{16}H_{26}N_3O^+$ $[M+H]^+$, 276.20, found, 276.2. |

**Example 1: Preparation of 3-(5-((4-((4'-chloro-[1,1'-biphenyl]-2-yl)methyl)piperazin-1-yl)methyl)-1-oxoisoindolin-2-yl)piperidine-2,6-dione (GT-03355)**

The target product GT-03355 was prepared according to the synthetic method described in Scheme 1.

To a solution of 1-((4'-chloro-[1,1'-biphenyl]-2-yl)methyl)piperazine (306 mg, 0.85 mmol), prepared from Intermediate Example 3, and 3-(5-(bromomethyl)-1-oxoisoindolin-2-yl)piperidine-2,6-dione (GT-03234) (286 mg, 0.85 mmol) in DMF (8 mL), were added TEA (0.56 mL, 3.4 mmol) and NaI (128 mg, 0.85 mmol). The reaction mixture was stirred at 50 °C for 18 hours. The reaction was monitored by LC-MS, and upon completion, the reaction mixture was filtered, and the filtrate was purified by high-performance liquid chromatography to afford the white solid compound GT-03355 (349 mg, yield: 75%). $^1H$ NMR (400 MHz, DMSO) $\delta$ 10.94 (s, 1H), 7.73-7.68 (m, 2H), 7.65-7.55 (m, 2H), 7.45-7.43 (m, 2H), 7.38-7.30 (m, 4H), 7.24-7.20 (m, 1H), 5.08-5.04 (m, 1H), 4.41 (d, *J* = 17.6 Hz, 1H), 4.28 (d, *J* = 17.6 Hz, 1H), 3.26-3.15 (m, 6H), 3.03-2.81 (m, 5H), 2.56-2.50 (m, 2H), 2.37-2.33 (m, 2H), 1.95-1.92 (m, 1H). LC/MS (ESI) m/z: calcd for $C_{31}H_{32}ClN_4O_3^+$ $[M+H]^+$, 543.22; found, 543.2.

**Example 2: Preparation of 3-(5-(4-((4-((4'-chloro-[1,1'-biphenyl]-2-yl)methyl)piperazin-1-yl)methyl)phenyl)-1-oxoisoindolin-2-yl)piperidine-2,6-dione (GT-03356)**

The target product GT-03356 was prepared according to the synthetic method described in Scheme 3.

To a solution of 3-(1-oxo-5-(4-(piperazin-1-ylmethyl)phenyl)isoindolin-2-yl)piperidine-2,6-dione (30 mg, 0.066 mmol), prepared according to Intermediate Example 16, and 2-(bromomethyl)-4'-chloro-1,1'-biphenyl (CAS No.: 1001754-57-9) (19 mg, 0.066 mmol) in DMF (2 mL), was added TEA (33 μL, 0.2 mmol). The reaction mixture was stirred at 50 °C for 2 hours. The reaction was monitored by LC-MS, and upon completion, the reaction mixture was filtered, and the filtrate was purified by high-performance liquid chromatography to afford the white solid compound GT-03356 (18 mg, yield: 44%). $^1H$ NMR (400 MHz, MeOD) $\delta$ 7.81-7.77 (m, 1H), 7.74-7.70 (m, 1H), 7.56-7.49 (m, 2H), 7.39-7.34 (m, 4H), 7.26-7.18 (m, 5H), 6.78-6.74 (m, 2H), 5.11-5.06 (m, 1H), 4.67-4.56 (m, 2H), 4.55-4.48 (m, 1H), 4.24-4.21 (m, 1H), 4.12 (s, 2H), 3.95-3.79 (m, 2H), 3.43-3.30 (m, 5H), 2.99-2.65 (m, 4H), 2.63-2.34 (m, 2H). LC/MS (ESI) m/z: calcd for $C_{37}H_{36}ClN_4O_3^+$ $[M+H]^+$, 619.25; found, 619.3.

**Example 3: Preparation of 3-(5-((1-((4'-chloro-[1,1'-biphenyl]-2-yl)methyl)piperidin-4-yl)methyl)-1-oxoisoindolin-2-yl)piperidine-2,6-dione (GT-03361)**

Referring to the method described in Scheme 3, the target compound GT-03361 was prepared using 3-(1-oxo-5-(piperidin-4-ylmethyl)isoindolin-2-yl)piperidine-2,6-dione (GT-3423) and 2-(bromomethyl)-4'-chloro-1,1'-biphenyl (CAS No.: 1001754-57-9) as starting materials. The compound (GT-03361) was obtained as white solid (17 mg, yield: 40%). $^1H$ NMR (400 MHz, MeOD) $\delta$ 7.86-7.71 (m, 2H), 7.58-7.51 (m, 4H), 7.46-7.32 (m, 5H), 5.17-5.13 (m, 1H), 4.53-4.41 (m, 3H), 4.34 (s, 2H), 3.44-3.37 (m, 1H), 3.35-3.30 (m, 1H), 3.08-2.88 (m, 2H), 2.82-2.78 (m, 1H), 2.71-2.60 (m, 3H), 2.52-2.49 (m, 1H), 2.19-2.16 (m, 1H), 1.92-1.77 (m, 2H), 1.54-1.47 (m, 1H), 1.41-1.38 (m, 1H). LC/MS (ESI) m/z: calcd for $C_{32}H_{33}ClN_3O_3^+$ $[M+H]^+$, 542.22; found, 542.2.

**Example** 4: **Preparation of 3-(5-(4-((4-((4'-chloro-5,5-dimethyl-3,4,5,6-tetrahydro-[1,1'-biphenyl]-2-yl)methyl)piperazin-1-yl)methyl)phenyl)-1-oxoisoindolin-2-yl)piperidine-2,6-dione (GT-03367)**

The target product GT-03367 was prepared according to the synthetic method described in Scheme 4.

To a solution of 3-(1-oxo-5-(4-(piperazin-1-ylmethyl)phenyl)isoindolin-2-yl)piperidine-2,6-dione (30 mg, 66 μmol), prepared according to Intermediate Example 16, and 4'-chloro-5,5-dimethyl-3,4,5,6-tetrahydro-[1,1'-biphenyl]-2-carbaldehyde (CAS No.: 1228837-05-5) (20 mg, 79 μmol) in DMF (2 mL), was added AcOH (1 drop). The reaction mixture was stirred at 50 °C for 17 hours. To the reaction mixture was added $NaBH_3CN$ (9 mg, 0.13 mmol), followed by continued stirring for 2 hours. The reaction was monitored by LC-MS, and upon completion, the reaction mixture was filtered, and the filtrate was purified by high-performance liquid chromatography to afford the white solid compound GT-03367 (18 mg, yield: 42%). $^1H$ NMR (400 MHz, MeOD) $\delta$ 7.80-7.75 (m, 2H), 7.72-7.68 (m, 3H), 7.47 (d, *J* = 7.8 Hz, 2H), 7.28 (d, *J* = 8.2 Hz, 2H), 6.98 (d, *J* = 8.2 Hz, 2H), 5.11-5.07 (m, 1H), 4.53-4.42 (m, 2H), 4.16-3.98 (m, 2H), 3.38-3.26 (m, 3H), 3.17-2.86 (m, 5H), 2.86-2.59 (m, 4H), 2.45-2.40 (m, 1H), 2.23-2.17 (m, 2H), 2.14-2.08 (m, 1H), 2.00 (brs, 2H), 1.45 (t, *J* = 6.3 Hz, 2H), 0.91 (s, 6H). LC/MS (ESI) m/z: calcd for $C_{39}H_{44}ClN_4O_3^+$ $[M+H]^+$, 651.31; found, 651.3.

**Example 5: Preparation of 3-(5-((1-((4'-chloro-5,5-dimethyl-3,4,5,6-tetrahydro-[1,1'-biphenyl]-2-yl)methyl)piperidin-4-yl)methyl)-1-oxoisoindolin-2-yl)piperidine-2,6-dione (GT-03368)**

Referring to the methods described in Scheme 4 or Example 4, the target compound GT-03368 was prepared

using 3-(1-oxo-5-(piperidin-4-ylmethyl)isoindolin-2-yl)piperidine-2,6-dione (GT-3423) and 4'-chloro-5,5-dimethyl-3,4,5,6-tetrahydro-[1,1'-biphenyl]-2-carbaldehyde as starting materials. The compound (GT-03368) was obtained as white solid (8 mg, yield: 16%). $^1$H NMR (400 MHz, MeOD) $\delta$ 7.63 *(d, J=* 7.8 Hz, 1H), 7.33-7.27(m, 3H), 7.24 *(d, J=* 7.8 Hz, 1H), 7.02-6.97 (m, 2H), 5.07-5.02 (m, 1H), 4.41-4.31 (m, 2H), 3.54-3.49 (m, 2H), 3.30-3.26 (m, 2H), 3.03-2.98 (m, 1H), 2.81-2.77 (m, 1H), 2.71 (brs, 1H), 2.63 (d, *J* = 6.4 Hz, 2H), 2.50-2.36 (m, 3H), 2.20-2.13 (m, 3H), 2.12-1.99 (m, 4H), 1.76-1.72 (m, 2H), 1.48 (t, *J=* 6.2 Hz, 3H), 0.92 (s, 6H). LC/MS (ESI) m/z: calcd for $C_{34}H_{41}ClN_3O_3^+$ [M+H]$^+$, 574.28; found, 574.3.

**Example 6: Preparation of 3-(5-(4-((4-((4'-fluoro-5,5-dimethyl-3,4,5,6-tetrahydro-[1,1'-biphenyl]-2-yl)methyl)piperazin-1-yl)methyl)phenyl)-1-oxoisoindolin-2-yl)piperidine-2,6-dione (GT-03453)**

The target product GT-03453 was prepared according to the synthetic method described in Scheme 4.

To a solution of 3-(1-oxo-5-(4-(piperazin-1-ylmethyl)phenyl)isoindolin-2-yl)piperidine-2,6-dione (30 mg, 66 $\mu$mol), prepared according to Intermediate Example 16, and 4'-fluoro-5,5-dimethyl-3,4,5,6-tetrahydro-[1,1'-biphenyl]-2-carbaldehyde (21 mg, 92 $\mu$mol), prepared according to Intermediate Example 2, in DMF (2 mL), was added DIEA (22 $\mu$L, 0.13 mmol). The reaction mixture was stirred at 50 °C for 2 hours. To the reaction mixture was added $NaBH_3CN$ (8 mg, 0.13 mmol), followed by continued stirring for 16 hours. The reaction was monitored by LC-MS, and upon completion, the reaction mixture was filtered, and the filtrate was purified by high-performance liquid chromatography to afford the white solid compound GT-03453 (10 mg, yield: 14%). $^1$H NMR (400 MHz, MeOD) $\delta$ 7.80-7.75 (m, 2H), 7.72-7.68 (m, 3H), 7.49 -7.44 (m, 2H), 7.30-7.25 (m, 2H), 7.01 - 6.96 (m, 2H), 5.11-5.07 (m, 1H), 4.53-4.42 (m, 2H), 4.16-3.98 (m, 2H), 3.38-3.26 (m, 3H), 3.17-2.86 (m, 5H), 2.86-2.59 (m, 4H), 2.45-2.40 (m, 1H), 2.23-2.17 (m, 2H), 2.14-2.08 (m, 1H), 2.00 (brs, 2H), 1.45 (t, *J* = 6.3 Hz, 2H), 0.91 (s, 6H). LC/MS (ESI) m/z: calcd for $C_{39}H_{44}FN_4O_3^+$ [M+H]$^+$, 635.34; found, 635.3.

**Example 7: Preparation of 3-(5-((4-((4'-fluoro-5,5-dimethyl-3,4,5,6-tetrahydro-[1,1'-biphenyl]-2-yl)methyl)piperazin-1-yl)methyl)-1-oxoisoindolin-2-yl)piperidine-2,6-dione (GT-03454)**

Referring to the methods described in Scheme 4 or Example 4, the target compound GT-03454 was prepared using 3-(1-oxo-5-(piperazin-1-ylmethyl)isoindolin-2-yl)piperidine-2,6-dione, prepared according to Intermediate Example 18, and 4'-fluoro-5,5-dimethyl-3,4,5,6-tetrahydro-[1,1'-biphenyl]-2-carbaldehyde, prepared according to Intermediate Example 2, as the starting materials. The compound (GT-03454) was obtained as white solid (6 mg, yield: 12%). $^1$H NMR (400 MHz, DMSO-d6) $\delta$ 11.01 (s, 1H), 7.87 - 7.77 (m, 2H), 7.75 - 7.66 (m, 1H), 7.21 - 7.12 (m, 4H), 5.14 (dd, *J* = 13.2, 5.1 Hz, 1H), 4.51 - 4.33 (m, 4H), 3.48 - 3.23 (m, 8H), 2.98 - 2.84 (m, 2H), 2.61 (d, *J* = 16.6 Hz, 2H), 2.43 (dd, *J* = 13.0, 4.3 Hz, 1H), 2.35 (brs, 2H), 2.02 (s, 3H), 1.44 (t, *J* = 6.0 Hz, 2H), 0.95 (s, 6H). LC/MS (ESI) m/z: calcd for $C_{33}H_{40}FN_4O_3^+$ [M+H]$^+$, 559.31; found, 559.3.

**Example 7: Preparation of 3-(5-((1-((4'-fluoro-5,5-dimethyl-3,4,5,6-tetrahydro-[1,1'-biphenyl]-2-yl)methyl)piperidin-4-yl)methyl)-1-oxoisoindolin-2-yl)piperidine-2,6-dione (GT-03455)**

Referring to the methods described in Scheme 4 or Example 4, the target compound GT-03455 was prepared using 3-(1-oxo-5-(piperidin-4-ylmethyl)isoindolin-2-yl)piperidine-2,6-dione and 4'-fluoro-5,5-dimethyl-3,4,5,6-tetrahydro-[1,1'-biphenyl]-2-carbaldehyde, prepared according to Intermediate Example 2, as the starting materials. The compound (GT-03455) was obtained as white solid (4 mg, yield: 10%). $^1$H NMR (400 MHz, MeOD) $\delta$ 7.75 (d, *J* = 8.0 Hz, 1H), 7.42 (s, 1H), 7.36 (d, *J= 8.0* Hz, 1H), 7.15-7.12 (m, 4H), 5.19-5.14 (m, 1H), 4.53-4.46 (m, 2H), 3.61 (s, 2H), 3.42 - 3.39 (m, 2H), 3.18 - 3.06 (m, 1H), 2.99 - 2.87 (m, 1H), 2.84 - 2.81 (m, 1H), 2.76 - 2.70 (m, 2H), 2.60 -2.48 (m, 3H), 2.34 - 2.23 (m, 2H), 2.23 - 2.10 (m, 3H), 1.94 - 1.79 (m, 2H), 1.66 -1.46 (m, 4H), 1.04 (s, 6H). LC/MS (ESI) m/z: calcd for $C_{34}H_{41}FN_3O_3^+$ [M+H]$^+$, 558.31; found, 558.3.

**Example 8: Preparation of 3-(5-((4-((4'-chloro-5,5-dimethyl-3,4,5,6-tetrahydro-[1,1'-biphenyl]-2-yl)methyl)piperazin-1-yl)methyl)-4-fluoro-1-oxoisoindolin-2-yl)piperidine-2,6-dione (GT-03468)**

The target product GT-03468 was prepared by referring to the synthetic methods of Scheme 1 or Example 1.

To a solution of (2-(2,6-dioxopiperidin-3-yl)-4-fluoro-1-oxoisoindolin-5-yl)methyl methanesulfonate (20 mg, 0.054 mmol), prepared according to Intermediate Example 6, and 1-((4'-chloro-5,5-dimethyl-3,4,5,6-tetrahydro-[1,1'-biphenyl]-2-yl)methyl)piperazine (CAS No.: 1228780-72-0) (17 mg, 0.054 mmol) in DMF (2 mL), was added TEA (18 $\mu$L, 0.11 mmol). The reaction mixture was stirred at 50 °C for 2 hours. The reaction was monitored by LC-MS, and upon completion, the reaction mixture was filtered, and the filtrate was purified by high-performance liquid chromatography to afford the white solid compound GT-03468 (20 mg, yield: 62%). $^1$H NMR (400 MHz, DMSO) $\delta$ 11.03 (s, 1H), 7.64 (brs, 2H), 7.43 (d, *J* = 8.2 Hz, 2H), 7.12 (d, *J* = 8.2 Hz, 2H), 5.16-5.11 (m, 1H), 4.57 (d, *J* = 17.6 Hz, 1H), 4.41 (d, *J* = 17.6 Hz, 1H), 3.79

(brs, 2H), 3.69-3.49 (m, 4H), 3.31-3.19 (m, 4H), 2.96-2.88 (m, 3H), 2.69-2.59 (m, 3H), 2.50-2.40 (m, 1H), 2.30-2.22 (m, 2H), 2.05-1.98 (m, 3H), 1.51-1.42 (m, 2H), 0.96 (s, 6H). LC/MS (ESI) m/z: calcd for $C_{33}H_{39}ClFN_4O_3^+$ $[M+H]^+$, 593.27; found, 593.3.

**Example 9: Preparation of 3-(5-((4-((4'-chloro-5,5-dimethyl-3,4,5,6-tetrahydro-[1,1'-biphenyl]-2-yl)methyl)piperazin-1-yl)methyl)-7-fluoro-1-oxoisoindolin-2-yl)piperidine-2,6-dione (GT-03469)**

Referring to the methods of Scheme 1 or Example 1, the target compound GT-03469 was prepared using (2-(2,6-dioxopiperidin-3-yl)-7-fluoro-1-oxoisoindolin-5-yl)methyl methanesulfonate, obtained according to Intermediate Example 8, and 1-((4'-chloro-5,5-dimethyl-3,4,5,6-tetrahydro-[1,1'-biphenyl]-2-yl)methyl)piperazine (CAS No.: 1228780-72-0) as the starting materials. The compound (GT-03469) was obtained as white solid (18 mg, yield: 56%). $^1H$ NMR (400 MHz, MeOD) $\delta$ 7.42 (s, 1H), 7.31 (d, $J$ = 8.2 Hz, 2H), 7.25 (d, $J$ = 10.0 Hz, 1H), 7.01 (d, $J$ = 8.2 Hz, 2H), 5.06-5.01 (m, 1H), 4.48-4.37 (m, 2H), 4.06 (brs, 2H), 3.57 (brs, 2H), 3.17-3.06 (m, 5H), 2.86-2.67 (m, 4H), 2.44-2.33 (m, 1H), 2.26-2.23(m 3H), 2.09-2.03 (m, 4H), 1.50-1.47 (m, 2H), 0.92 (s, 6H). LC/MS (ESI) m/z: calcd for $C_{33}H_{39}ClFN_4O_3^+$ $[M+H]^+$, 593.27; found, 593.3.

**Example 10: Preparation of 3-(5-((3-((4'-chloro-5,5-dimethyl-3,4,5,6-tetrahydro-[1,1'-biphenyl]-2-yl)methyl)-3,6-diazabicyclo[3.1.1]heptan-6-yl)methyl)-1-oxoisoindolin-2-yl)piperidine-2,6-dione (GT-03492)**

Referring to the methods of Scheme 1 or Example 1, the target compound GT-03492 was prepared using (2-(2,6-dioxopiperidin-3-yl)-1-oxoisoindolin-5-yl)methyl methanesulfonate, obtained according to Intermediate Example 5, and 3-((4'-chloro-5,5-dimethyl-3,4,5,6-tetrahydro-[1,1'-biphenyl]-2-yl)methyl)-3,6-diazabicyclo[3.1.1]heptane, obtained according to Intermediate Example 13, as the starting materials. The compound (GT-03492) was obtained as white solid (8 mg, yield: 24%). $^1H$ NMR (400 MHz, MeOD) $\delta$ 7.81 (d, $J$ = 8.0Hz, 1H), 7.61 (s, 1H), 7.56-7.54 (m, 1H), 7.23-7.20 (m, 2H), 6.96-6.94 (m, 2H), 5.11-5.06 (m, 1H), 4.54-4.44 (m, 2H), 4.32-4.24 (m, 2H), 3.67-3.53 (m, 1H), 2.86-2.64 (m, 5H), 2.47-2.06 (m, 9H), 1.95-1.91 (m, 3H), 1.42 (d, $J$ = 6.4 Hz, 2H), 0.91 (s, 6H). LC/MS (ESI) m/z: calcd for $C_{34}H_{40}ClN_4O_3^+$ $[M+H]^+$, 587.28; found, 587.3.

**Example 11: Preparation of 3-(5-((3-((4'-chloro-5,5-dimethyl-3,4,5,6-tetrahydro-[1,1'-biphenyl]-2-yl)methyl)-3,6-diazabicyclo[3.1.1]heptan-6-yl)methyl)-4-fluoro-1-oxoisoindolin-2-yl)piperidine-2,6-dione (GT-03493)**

Referring to the methods of Scheme 1 or Example 1, the target product GT-03493 was prepared using (2-(2,6-dioxopiperidin-3-yl)-4-fluoro-1-oxoisoindolin-5-yl)methyl methanesulfonate, obtained according to Intermediate Example 6, and 3-((4'-chloro-5,5-dimethyl-3,4,5,6-tetrahydro-[1,1'-biphenyl]-2-yl)methyl)-3,6-diazabicyclo[3.1.1]heptane, obtained according to Intermediate Example 13, as the starting materials. The compound (GT-03493) was obtained as white solid (8 mg, yield: 24%). $^1H$ NMR (400 MHz, MeOD) $\delta$ 7.66-762 (m, 2H), 7.24-7.22 (m, 2H), 6.97-6.95 (m, 2H), 5.11-5.06 (m, 1H), 4.59-4.47 (m, 2H), 4.38-4.25 (m, 2H), 3.67-3.54 (m, 1H), 3.06-3.03 (m, 1H), 2.93 (d, $J$ = 11.9 Hz, 1H), 2.87 - 2.75 (m, 2H), 2.75 - 2.64 (m, 2H), 2.44 (dd, $J$ = 27.1, 14.1 Hz, 2H), 2.25 (s, 2H), 2.19 (d, $J$ = 12.5 Hz, 2H), 2.11 (s, 2H), 2.00 - 1.91 (m, 2H), 1.41 (t, $J$ = 6.4 Hz, 2H), 0.91 (s, 6H). LC/MS (ESI) m/z: calcd for $C_{34}H_{39}ClFN_4O_3^+$ $[M+H]^+$, 605.27; found, 605.3.

**Example 12: Preparation of 3-(5-((4-((4'-chloro-5,5-dimethyl-3,4,5,6-tetrahydro-[1,1'-biphenyl]-2-yl)methyl)piperazin-1-yl)methyl)-6-fluoro-1-oxoisoindolin-2-yl)piperidine-2,6-dione (GT-03578)**

Referring to the methods of Scheme 1 or Example 1, the target product GT-03578 was prepared using (2-(2,6-dioxopiperidin-3-yl)-6-fluoro-1-oxoisoindolin-5-yl)methyl methanesulfonate, obtained according to Intermediate Example 7, and 1-((4'-chloro-5,5-dimethyl-3,4,5,6-tetrahydro-[1,1'-biphenyl]-2-yl)methyl)piperazine (CAS No.: 1228780-72-0) as the starting materials. The compound (GT-03578) was obtained as white solid (36 mg, yield: 45%). $^1H$ NMR (400 MHz, DMSO) $\delta$ 11.02 (s, 1H), 7.73 (brs, 1H), 7.60 (d, $J$ = 8.6 Hz, 1H), 7.43 (d, $J$ = 8.2 Hz, 2H), 7.12 (d, $J$ = 8.2 Hz, 2H), 5.16-5.11 (m, 1H), 4.44-4.31 (m, 2H), 4.14-3.75 (m, 2H), 3.69-3.49 (m, 4H), 3.33-3.16 (m, 4H), 2.96-2.88 (m, 2H), 2.68-2.59 (m, 2H), 2.46-2.40 (m, 1H), 2.27 (brs, 2H), 2.02 (brs, 3H), 1.47-1.44 (m, 2H), 1.30-1.23 (m, 1H), 0.96 (s, 6H). LC/MS (ESI) m/z: calcd for $C_{33}H_{39}ClFN_4O_3^+$ $[M+H]^+$, 593.27; found, 593.3.

**Example 13: Preparation of 3-(4-((4-((4'-chloro-5,5-dimethyl-3,4,5,6-tetrahydro-[1,1'-biphenyl]-2-yl)methyl)piperazin-1-yl)methyl)-1-oxoisoindolin-2-yl)piperidine-2,6-dione (GT-03649)**

Referring to the methods of Scheme 1 or Example 1, the target product GT-03649 was prepared using (2-(2,6-dioxopiperidin-3-yl)-1-oxoisoindolin-4-yl)methyl methanesulfonate, obtained according to Intermediate Example 9, and

1-((4'-chloro-5,5-dimethyl-3,4,5,6-tetrahydro-[1,1'-biphenyl]-2-yl)methyl)piperazine (CAS No.: 1228780-72-0) as the starting materials. The compound (GT-03649) was obtained as white solid (41 mg, yield: 45%). $^1$H NMR (400 MHz, DMSO) δ 11.04 (s, 1H), 7.79-7.76 (m, 2H), 7.58 (t, *J* = 7.2 Hz, 1H), 7.42 (d, *J* = 8.2 Hz, 2H), 7.12 (d, *J* = 8.2 Hz, 2H), 5.18-5.13 (m, 1H), 4.81-4.68 (m, 1H), 4.47-4.40 (m, 1H), 3.67-3.47 (m, 4H), 3.33-3.17(m, 4H), 3.01-2.92 (m, 2H), 2.85-2.77 (m, 2H), 2.67-2.62 (m, 2H), 2.41-2.31 (m, 3H), 2.02-1.99 (m, 3H), 1.47-1.44 (m, 2H), 0.96 (s, 6H). LC/MS (ESI) m/z: calcd for $C_{33}H_{40}ClN_4O_3^+$ [M+H]$^+$, 575.28; found, 575.3.

**Example 14: Preparation of 3-(5-((4-((4'-chloro-[1,1'-biphenyl]-2-yl)methyl)piperazin-1-yl)methyl)-4-fluoro-1-oxoisoindolin-2-yl)piperidine-2,6-dione (GT-03694)**

Referring to the methods described in Scheme 1 or Example 1 the target product GT-03694 was prepared using (2-(2,6-dioxopiperidin-3-yl)-4-fluoro-1-oxoisoindolin-5-yl)methyl methanesulfonate, obtained according to Intermediate Example 6, and 1-((4'-chloro-[1,1'-biphenyl]-2-yl)methyl)piperazine (CAS No.: 870812-97-8), obtained according to Intermediate Example 3, as the starting materials. The compound (GT-03694) was obtained as white solid (40 mg, yield: 76%). $^1$H NMR (400 MHz, DMSO) δ 11.02 (s, 1H), 7.80-7.75 (m, 1H), 7.65-7.63 (m, 1H), 7.52-7.47 (m, 5H), 7.40-7.38 (m, 2H), 7.30-7.28 (m, 1H), 5.16-5.11(m, 1H), 4.57 (d, *J* = 17.6 Hz, 1H), 4.41 (d, *J*= 17.6 Hz, 1H), 3.33-3.05 (m, 5H), 2.93-2.88 (m, 3H), 2.63-2.58 (m, 3H), 2.42-2.32 (m, 1H), 2.03-1.97 (m, 2H), 1.30-1.23 (m, 2H). LC/MS (ESI) m/z: calcd for $C_{31}H_{31}ClFN_4O_3^+$ [M+H]$^+$, 561.21;found, 561.2.

**Example 15: Preparation of 3-(5-((4-((4'-chloro-[1,1'-biphenyl]-2-yl)methyl)piperazin-1-yl)methyl)-6-fluoro-1-oxoisoindolin-2-yl)piperidine-2,6-dione (GT-03695)**

Referring to the methods of Scheme 1 or Example 1, the target product GT-03695 was prepared using (2-(2,6-dioxopiperidin-3-yl)-6-fluoro-1-oxoisoindolin-5-yl)methyl methanesulfonate, obtained according to Intermediate Example 7, and 1-((4'-chloro-[1,1'-biphenyl]-2-yl)methyl)piperazine as the starting materials. The compound (GT-03695) was obtained as white solid (39 mg, yield: 83%). $^1$H NMR (400 MHz, DMSO) δ 11.02 (s, 1H), 7.86-7.71 (m, 1H), 7.66-7.58 (m, 1H), 7.56-7.47 (m, 5H), 7.43-7.37 (m, 2H), 7.34-7.25 (m, 1H), 5.14-5.11 (m, 1H), 4.45 (d, *J* = 17.2 Hz, 2H), 4.33 (d, *J* = 17.2 Hz, 1H), 3.31-3.04 (m, 3H), 2.96-2.88 (m, 2H), 2.74-2.58 (m, 3H), 2.44-2.33 (m, 2H), 2.07-1.94 (m, 2H), 1.33-1.23 (m, 4H). LC/MS (ESI) m/z: calcd for $C_{31}H_{31}ClFN_4O_3^+$ [M+H]$^+$, 561.21; found, 561.2.

**Example 16: Preparation of 3-(5-((4-((4'-chloro-[1,1'-biphenyl]-2-yl)methyl)piperazin-1-yl)methyl)-7-fluoro-1-oxoisoindolin-2-yl)piperidine-2,6-dione (GT-03696)**

Referring to the methods of Scheme 1 or Example 1, the target product GT-03696 was prepared using (2-(2,6-dioxopiperidin-3-yl)-7-fluoro-1-oxoisoindolin-5-yl)methyl methanesulfonate, obtained according to Intermediate Example 8, and 1-((4'-chloro-[1,1'-biphenyl]-2-yl)methyl)piperazine as the starting materials. The compound (GT-03696) was obtained as white solid (39 mg, yield: 74%). $^1$H NMR (400 MHz, DMSO) δ 11.01 (s, 1H), 7.98-7.71 (m, 1H), 7.62-7.49 (m, 4H), 7.48-7.43 (m, 2H), 7.41-7.35 (m, 2H), 7.30-7.27 (m, 1H), 5.11-5.07 (m, 1H), 4.49 (d, *J* = 17.6 Hz, 1H), 4.36 (d, *J* = 17.6 Hz, 1H), 4.30- 4.05 (m, 2H), 3.33-3.06 (m, 5H), 3.02-2.84 (m, 3H), 2.60-2.58 (m, 2H), 2.46-2.30 (m, 1H), 2.08-1.91 (m, 1H), 1.30-1.24 (m, 1H). LC/MS (ESI) m/z: calcd for $C_{31}H_{31}ClFN_4O_3^+$ [M+H]$^+$, 561.21; found, 561.2.

**Example 17: Preparation of 3-(4-((4-((4'-chloro-[1,1'-biphenyl]-2-yl)methyl)piperazin-1-yl)methyl)-1-oxoisoindolin-2-yl)piperidine-2,6-dione (GT-03876)**

Referring to the methods of Scheme 1 or Example 1, the target product GT-03876 was prepared using (2-(2,6-dioxopiperidin-3-yl)-1-oxoisoindolin-4-yl)methyl methanesulfonate, obtained according to Intermediate Example 9, and 1-((4'-chloro-[1,1'-biphenyl]-2-yl)methyl)piperazine as the starting materials. The compound (GT-03876) was obtained as white solid (16 mg, yield: 24%). $^1$H NMR (400 MHz, MeOD) δ 7.84 (d, *J* = 8.0 Hz, 1H), 7.76-7.70 (m, 1H), 7.68-7.66 (m, 1H), 7.61-7.57 (m, 1H), 7.56-7.51 (m, 4H), 7.42-7.32 (m, 3H), 5.21-5.17 (m, 1H), 4.66-4.53 (m, 2H), 4.27 (brs, 2H), 4.02 (brs, 2H), 3.22-2.88 (m, 8H), 2.87-2.73 (m, 2H), 2.57-2.46 (m, 1H), 2.29-2.12 (m, 1H). LC/MS (ESI) m/z: calcd for $C_{31}H_{32}ClN_4O_3^+$ [M+H]$^+$, 543.22; found, 543.2.

**Example 18: Preparation of 3-(5-((1-((4'-chloro-[1,1'-biphenyl]-2-yl)methyl)piperidin-4-yl)amino)-1-oxoisoindolin-2-yl)piperidine-2,6-dione (GT-03877)**

Referring to the method of Scheme 1, the target product GT-03877 was prepared using 3-(1-oxo-5-(piperidin-4-ylamino)isoindolin-2-yl)piperidine-2,6-dione, obtained according to Intermediate Example 19, and 2-(bromomethyl)-4'-chloro-1,1'-biphenyl (CAS No.: 1001754-57-9) as the starting materials. The compound (GT-03877) was obtained as

white solid (30 mg, yield: 52%). $^1$H NMR (400 MHz, MeOD) δ 7.78-7.76 (m, 1H), 7.64-7.48 (m, 5H), 7.44-7.43 (m, 1H), 7.40-7.38(m, 2H), 6.74-6.71 (m, 2H), 5.10-5.06 (m, 1H), 4.50-4.31 (m, 4H), 3.62-3.57 (m, 1H), 3.44-3.39 (m, 2H), 2.99-2.70 (m, 4H), 2.54-2.38 (m, 1H), 2.26-2.00 (m, 3H), 1.67-1.62 (m, 2H). LC/MS (ESI) m/z: calcd for $C_{31}H_{32}ClN_4O_3^+$ [M+H]+, 543.22; found, 543.2.

**Example 19: Preparation of 3-(5-(3-((4'-chloro-[1,1'-biphenyl]-2-yl)methyl)-3,8-diazabicyclo[3.2.1]octan-8-yl)-1-oxoisoindolin-2-yl)piperidine-2,6-dione (GT-03878)**

Referring to the method of Scheme 1, the target product GT-03878 was prepared using 3-(5-(3,8-diazabicyclo[3.2.1]octan-8-yl)-1-oxoisoindolin-2-yl)piperidine-2,6-dione, obtained according to Intermediate Example 17, and 2-(bromomethyl)-4'-chloro-1,1'-biphenyl as the starting materials. The compound (GT-03878) was obtained as white solid (22 mg, yield: 37%). $^1$H NMR (400 MHz, MeOD) δ 7.73-7.72 (m, 1H), 7.68 (d, *J* = 8.0 Hz, 1H), 7.53-7.44 (m, 2H), 7.42-7.40 (m, 2H), 7.36-7.34 (m, 1H), 7.26-7.24 (m, 2H), 6.92-6.87 (m, 2H), 5.18-5.14 (m, 1H), 4.48-4.41 (m, 4H), 4.33 (brs, 2H), 3.06-2.99 (m, 2H), 2.98-2.77 (m, 4H), 2.58-2.47 (m, 1H), 2.32-2.14 (m, 3H), 2.10-2.00 (m, 2H). LC/MS (ESI) m/z: calcd for $C_{32}H_{32}ClN_4O_3^+$ [M+H]+, 555.22; found, 555.2.

**Example 20: Preparation of 3-(5-((1-((4'-chloro-[1,1'-biphenyl]-2-yl)methyl)-3-hydroxyazetidin-3-yl)(hydroxy)methyl)-1-oxoisoindolin-2-yl)piperidine-2,6-dione (GT-03880)**

Referring to the method of Scheme 1, the target product GT-03880 was prepared using 3-(5-(hydroxy(3-hydroxyazetidin-3-yl)methyl)-1-oxoisoindolin-2-yl)piperidine-2,6-dione, obtained according to Intermediate Example 15, and 2-(bromomethyl)-4'-chloro-1,1'-biphenyl (CAS No.: 1001754-57-9) as the starting materials. The compound (GT-03880) was obtained as white solid (13 mg, yield: 22%). $^1$H NMR (400 MHz, MeOD) δ 7.78 (dd, *J* = 8.0, 1.6 Hz, 1H), 7.67-7.52 (m, 6H), 7.46-7.34 (m, 4H), 5.20-5.15 (m, 1H), 4.77-4.68 (m, 1H), 4.59-4.42 (m, 5H), 4.37-4.21 (m, 1H), 4.09-3.85 (m, 1H), 3.60-3.46 (m, 1H), 3.01-2.86 (m, 1H), 2.83-2.73 (m, 1H), 2.52-2.50 (m, 1H), 2.28-2.08 (m, 1H). LC/MS (ESI) m/z: calcd for $C_{30}H_{29}ClN_3O_5^+$ [M+H]+, 546.18; found, 546.2.

**Example 21: Preparation of 3-(5-((1-((4'-chloro-[1,1'-biphenyl]-2-yl)methyl)azetidin-3-ylidene)methyl)-1-oxoisoindolin-2-yl)piperidine-2,6-dione (GT-03881)**

Referring to the method of Scheme 1, the target product GT-03881 was prepared using 3-(5-(azetidin-3-ylidenemethyl)-1-oxoisoindolin-2-yl)piperidine-2,6-dione, obtained according to Intermediate Example 13, and 2-(bromomethyl)-4'-chloro-1,1'-biphenyl (CAS No.: 1001754-57-9) as the starting materials. The compound (GT-03881) was obtained as white solid (11 mg, yield: 20%). $^1$H NMR (400 MHz, MeOD) δ 7.81 (d, *J* = 8.0 Hz, 1H), 7.63-7.54 (m, 3H), 7.49 (d, *J* = 8.4 Hz, 2H), 7.45-7.42 (m, 1H), 7.39 (d, *J* = 8.4 Hz, 2H), 7.32 (s, 1H), 7.25 (d, *J* = 8.0 Hz, 1H), 6.60 (s, 1H), 5.20-5.16 (m, 1H), 4.95-4.88 (m, 2H), 4.64 (s, 2H), 4.57-4.47 (m, 2H), 2.99-2.88 (m, 1H), 2.84-2.78 (m, 1H), 2.59-2.45 (m, 1H), 2.24-2.17 (m, 1H). LC/MS (ESI) m/z: calcd for $C_{30}H_{27}ClN_3O_3^+$ [M+H]+, 512.17; found, 512.2.

**Example 22: Preparation of 3-(5-(((1R,4R)-5-((4'-chloro-5,5-dimethyl-3,4,5,6-tetrahydro-[1,1'-biphenyl]-2-yl)methyl)-2,5-diazabicyclo[2.2.1]heptan-2-yl)methyl)-1-oxoisoindolin-2-yl)piperidine-2,6-dione (GT-03887)**

Referring to the methods of Scheme 1 or Example 1, the target product GT-03887 was prepared using (2-(2,6-dioxopiperidin-3-yl)-1-oxoisoindolin-5-yl)methyl methanesulfonate, obtained according to Intermediate Example 5, and (1R,4R)-2-((4'-chloro-5,5-dimethyl-3,4,5,6-tetrahydro-[1,1'-biphenyl]-2-yl)methyl)-2,5-diazabicyclo[2.2.1]heptane as the starting materials. The compound (GT-03887) was obtained as white solid (42 mg, yield: 63%). $^1$H NMR (400 MHz, MeOD) δ 7.89 (d, *J* = 8.0 Hz, 1H), 7.82-7.75 (m, 1H), 7.70-7.63 (m, 1H), 7.44 *(d, J* = 8.4 Hz, 2H), 7.17 (d, *J* = 8.4 Hz, 2H), 5.22-5.17 (m, 1H), 5.04-4.93 (m, 4H), 4.64-4.50 (m, 2H), 4.33-4.19 (m, 2H), 3.86-3.83 (m, 1H), 3.76-3.70 (m, 1H), 3.50-3.40 (m, 3H), 3.02-2.88 (m, 1H), 2.83-2.79 (m, 1H), 2.61-2.47 (m, 1H), 2.46-2.29 (m, 3H), 2.23-2.17 m, 3H), 1.61 (t, *J* = 6.4 Hz, 2H), 1.05 (s, 6H). LC/MS (ESI) m/z: calcd for $C_{34}H_{40}ClN_4O_3^+$ [M+H]+, 587.28; found, 587.3.

**Example 23: Preparation of 3-(5-(((1R,4R)-5-((4'-chloro-5,5-dimethyl-3,4,5,6-tetrahydro-[1,1'-biphenyl]-2-yl)methyl)-2,5-diazabicyclo[2.2.1]heptan-2-yl)methyl)-4-fluoro-1-oxoisoindolin-2-yl)piperidine-2,6-dione (GT-03888)**

Referring to the methods of Scheme 1 or Example 1, the target product GT-03888 was prepared using (2-(2,6-dioxopiperidin-3-yl)-4-fluoro-1-oxoisoindolin-5-yl)methyl methanesulfonate, obtained according to Intermediate Example 6, and (1R,4R)-2-((4'-chloro-5,5-dimethyl-3,4,5,6-tetrahydro-[1,1'-biphenyl]-2-yl)methyl)-2,5-diazabicyclo[2.2.1]heptane as the starting materials. The compound (GT-03888) was obtained as white solid (43 mg, yield: 66%). $^1$H NMR

(400 MHz, MeOD) δ 7.83-7.71 (m, 2H), 7.45 (d, *J* = 8.4 Hz, 2H), 7.18 (d, *J* = 8.4 Hz, 2H), 5.21-5.17 m, 1H), 5.04-4.97 (m, 2H), 4.67-4.49 (m, 2H), 4.34-4.27 (m, 3H), 4.16-4.05 (m, 1H), 3.86-3.83 (m, 1H), 3.76-3.70 (m, 1H), 3.51-3.36 (m, 3H), 3.00- 2.88 (m, 1H), 2.83-2.79 (m, 1H), 2.59-2.50 (m, 1H), 2.44-2.28 (m, 3H), 2.22-2.17 (m, 3H), 1.61 (t, *J* = 6.4 Hz, 2H), 1.05 (s, 6H). LC/MS (ESI) m/z: calcd for $C_{34}H_{39}ClFN_4O_3^+$ [M+H]+, 605.27; found, 605.3.

**Example 24: Preparation of 3-(5-((3-((4'-chloro-5,5-dimethyl-3,4,5,6-tetrahydro-[1,1'-biphenyl]-2-yl)methyl)-3,8-diazabicyclo [3.2.1] octan-8-yl)methyl)-1-oxoisoindolin-2-yl)piperidine-2,6-dione (GT-03889)**

Referring to the methods of Scheme 1 or Example 1, the target product GT-03889 was prepared using (2-(2,6-dioxopiperidin-3-yl)-1-oxoisoindolin-5-yl)methyl methylsulfonate, obtained according to Intermediate Example 5, and 3-((4'-chloro-5,5-dimethyl-3,4,5,6-tetrahydro-[1,1'-biphenyl]-2-yl)methyl)-3,8-diazabicyclo[3.2.1]octane, obtained according to Intermediate Example 93, as the starting materials. The compound (GT-03889) was obtained as white solid (26 mg, yield: 38%). $^1$H NMR (400 MHz, MeOD) δ 7.80 (d, *J* = 8.0 Hz, 1H), 7.66 (s, 1H), 7.57 (d, *J* = 8.0 Hz, 1H), 7.25-7.19 (m, 2H), 6.97-6.92 (m, 2H), 5.11-5.06 (m, 1H), 4.57-4.35 (m, 2H), 4.22 (brs, 2H), 3.80 (brs, 2H), 2.87-2.64 (m, 6H), 2.52-2.35 (m, 1H), 2.30-2.18 (m, 9H), 1.93 (brs, 2H), 1.42-1.39 (m, 2H), 0.90 (s, 6H). LC/MS (ESI) m/z: calcd for $C_{35}H_{42}ClN_4O_3^+$ [M+H]+, 601.29; found, 601.3.

**Example 25: Preparation of 3-(5-((3-((4'-chloro-5,5-dimethyl-3,4,5,6-tetrahydro-[1,1'-biphenyl]-2-yl)methyl)-3,8-diazabicyclo [3.2.1] octan-8-yl)methyl)-4-fluoro-1-oxoisoindolin-2-yl)piperidine-2,6-dione (GT-03890)**

Referring to the methods of Scheme 1 or Example 1, the target product GT-03890 was prepared using (2-(2,6-dioxopiperidin-3-yl)-4-fluoro-1-oxoisoindolin-5-yl)methyl methylsulfonate, obtained according to Intermediate Example 6, and 3-((4'-chloro-5,5-dimethyl-3,4,5,6-tetrahydro-[1,1'-biphenyl]-2-yl)methyl)-3,8-diazabicyclo[3.2.1]octane, obtained according to Intermediate Example 93, as the starting materials. The compound (GT-03890) was obtained as white solid (21 mg, yield: 31%). $^1$H NMR (400 MHz, MeOD) δ 7.71-7.61 (m, 2H), 7.21 (d, *J* = 8.4 Hz, 2H), 6.94 (d, *J* = 8.4 Hz, 2H), 5.10-5.06 (m, 1H), 4.52 (q, *J* = 17.6 Hz, 2H), 4.29 (brs, 2H), 3.92 (brs, 2H), 3.00-2.91 (m, 2H), 2.88-2.77 (m, 3H), 2.74-2.66 (m, 1H), 2.45-2.42 (m, 2H), 2.37-2.25 (m, 3H), 2.21-2.17 (m, 2H), 2.16-2.06 (m, 3H), 1.95 (brs, 2H), 1.42 (t, *J* = 6.6 Hz, 2H), 0.9 (s, 6H). LC/MS (ESI) m/z: calcd for $C_{35}H_{41}ClFN_4O_3^+$ [M+H]+, 619.28; found, 619.3.

**Example 26: Preparation of 3-(5-((4-(4'-chloro-[1,1'-biphenyl]-2-carbonyl)piperazin-1-yl)methyl)-1-oxoisoindolin-2-yl)piperidine-2,6-dione (GT-03891)**

The target product GT-03891 was prepared by referring to the synthetic method of Scheme 6.

To a solution of 3-(1-oxo-5-(piperazin-1-ylmethyl)isoindolin-2-yl)piperidine-2,6-dione (45 mg, 0.13 mmol), obtained according to Intermediate Example 18, and 4'-chloro-[1,1'-biphenyl]-2-carboxylic acid (CAS No.: 7079-15-4) (40 mg, 0.17 mmol) in DMF (2 mL), were added TEA (55 μL, 0.39 mmol) and HATU (75 mg, 0.19 mmol). The reaction mixture was stirred at 50 °C for 2 hours. The reaction was monitored by LC-MS, and upon completion, the reaction mixture was filtered, and the filtrate was purified by high-performance liquid chromatography to afford the white solid compound GT-03891 (26 mg, yield: 35%). $^1$H NMR (400 MHz, MeOD) δ 7.92 (d, *J* = 7.6 Hz, 1H), 7.71 (s, 1H), 7.65-7.58 (m, 2H), 7.56-7.46 (m, 7H), 5.21-5.17 (m, 1H), 5.02-4.92 (m, 3H), 4.57 (q, *J* = 17.2 Hz, 2H), 4.39-4.35 (m, 2H), 3.51-3.40 (m, 4H), 3.13-3.03 (m, 1H), 2.97-2.92 (m, 1H), 2.86-2.77 (m, 1H), 2.61-2.47 (m, 1H), 2.29-2.14 (m, 1H). LC/MS (ESI) m/z: calcd for $C_{31}H_{30}ClN_4O_4^+$ [M+H]+, 557.20; found, 557.2.

**Example 27: Preparation of 3-(5-((4-((4'-chloro-[1,1'-biphenyl]-2-yl)amino)piperidin-1-yl)methyl)-1-oxoisoindolin-2-yl)piperidine-2,6-dione (GT-03991)**

Referring to the methods of Scheme 1 or Example 1, 3-(5-(bromomethyl)-1-oxoisoindolin-2-yl)piperidine-2,6-dione (GT-03234) and N-(4'-chloro-[1,1'-biphenyl]-2-yl)piperidin-4-amine hydrochloride, obtained according to Intermediate Example 63, were used as the starting materials to obtain the target product GT-03991 (17 mg, yield: 24%). $^1$H NMR (400 MHz, MeOD) δ 7.92 (d, *J* = 8.0 Hz, 1H), 7.82 (s, 1H), 7.71 (d, *J* = 8.0 Hz, 1H), 7.50 (d, *J* = 8.0 Hz, 2H), 7.40 (d, *J* = 8.0 Hz, 2H), 7.35-7.31 (m, 1H), 7.15 (d, *J* = 8.0 Hz, 1H), 7.06 (d, *J* = 8.0 Hz, 1H), 7.00 (t, *J* = 8.0 Hz, 1H), 5.22-5.17 (m, 1H), 4.64-4.53 (m, 2H), 4.47 (s, 2H), 3.68-3.62 (m, 1H), 3.57-3.54 (m, 2H), 3.17 (t, *J* = 12.4 Hz, 2H), 2.94-2.89 (m, 1H), 2.86-2.73 (m, 1H), 2.59-2.51 (m, 1H), 2.25-2.19 (m, 3H), 1.75-1.67 (m, 2H). LC/MS (ESI) m/z: calcd for $C_{31}H_{32}ClN_4O_3^+$ [M+H]+, 543.22; found, 543.2.

**Example 28: Preparation of 3-(5-((1-(4'-chloro-[1,1'-biphenyl]-2-carbonyl)azetidin-3-yl)methyl)-1-oxoisoindolin-2-yl)piperidine-2,6-dione (GT-03922)**

Referring to the method of Scheme 6, the target product GT-03922 was prepared using 3-(5-(azetidin-3-ylmethyl)-1-oxoisoindolin-2-yl)piperidine-2,6-dione, obtained according to Intermediate Example 14, and 4'-chloro-[1,1'-biphenyl]-2-carboxylic acid (CAS No.: 7079-15-4), as the starting materials. The compound (GT-03922) was obtained as white solid (35 mg, yield: 57%). $^1$H NMR (400 MHz, MeOD) δ 7.60 (d, *J* = 7.6 Hz, 1H), 7.47-7.26 (m, 8H), 7.17 (s, 1H), 7.11 (d, *J* = 7.6 Hz, 1H), 5.07-5.01 (m, 1H), 4.43-4.29 (m, 2H), 3.99-3.88 (m, 1H), 3.59-3.55 (m, 1H), 3.53-3.45 (m, 1H), 3.08-3.04 (m, 1H), 2.87-2.76 (m, 1H), 2.74-2.63 (m, 2H), 2.58-2.52 (m, 2H), 2.46-2.30 (m, 1H), 2.11-2.01 (m, 1H). LC/MS (ESI) m/z: calcd for $C_{30}H_{27}ClN_3O_4^+$ [M+H]$^+$, 528.17; found, 528.2.

**Example 29: Preparation of 3-(5-((1-((4'-chloro-[1,1'-biphenyl]-2-yl)methyl)azetidin-3-yl)methyl)-1-oxoisoindolin-2-yl)piperidine-2,6-dione (GT-03923)**

Referring to the method of Scheme 2, the target product GT-03923 was prepared using 3-(5-(azetidin-3-ylmethyl)-1-oxoisoindolin-2-yl)piperidine-2,6-dione prepared according to Intermediate Example 14 and 2-(bromomethyl)-4'-chloro-1,1'-biphenyl (CAS No.: 1001754-57-9) as the starting materials. The compound (GT-03923) was obtained as white solid (38 mg, yield: 63%). $^1$H NMR (400 MHz, MeOD) δ 7.65-7.63 (m, 1H), 7.47-7.39 (m, 5H), 7.31-7.18 (m, 3H), 7.12 (d, *J* = 7.6 Hz, 1H), 5.08-5.02 (m, 1H), 4.74-4.69 (m, 3H), 4.46-4.34 (m, 2H), 4.32 (s, 1H), 3.96-3.91 (m, 1H), 3.83-3.80 (m, 1H), 3.58-3.53 (m, 1H), 3.06-2.99 (m, 1H), 2.85-2.75 (m, 2H), 2.74-2.64 (m, 1H), 2.41-2.34 (m, 1H), 2.14-2.00 (m, 1H). LC/MS (ESI) m/z: calcd for $C_{30}H_{29}ClN_3O_3^+$ [M+H]$^+$, 514.19; found, 514.2.

**Example 30: Preparation of 3-(5-((6-((4'-chloro-5,5-dimethyl-3,4,5,6-tetrahydro-[1,1'-biphenyl]-2-yl)methyl)-3,6-diazabicyclo[3.1.1]heptan-3-yl)methyl)-1-oxoisoindolin-2-yl)piperidine-2,6-dione (GT-03995)**

Referring to the methods of Scheme 1 or Example 1, the target product GT-03995 was prepared using (2-(2,6-dioxopiperidin-3-yl)-1-oxoisoindolin-5-yl)methyl methanesulfonate prepared according to Intermediate Example 5 and 6-((4'-chloro-5,5-dimethyl-3,4,5,6-tetrahydro-[1,1'-biphenyl]-2-yl)methyl)-3,6-diazabicyclo[3.1.1]heptane prepared according to Intermediate Example 14 as the starting materials. The compound (GT-03995) was obtained as white solid (8 mg, yield: 12%). $^1$H NMR (400 MHz, MeOD) δ 7.82 (d, *J* = 8.0 Hz, 1H), 7.68 (s, 1H), 7.58 (d, *J* = 8.0 Hz, 1H), 7.25-7.19 (m, 2H), 6.97-6.92 (m, 2H), 5.12-5.07 (m, 1H), 4.58-4.37 (m, 2H), 3.80 (brs, 2H), 2.88-2.65 (m, 6H), 2.54-2.37 (m, 1H), 2.31-2.19 (m, 9H), 1.95-1.90 (m, 2H), 1.42-1.39 (m, 2H), 0.90 (s, 6H). LC/MS (ESI) m/z: calcd for $C_{34}H_{40}ClN_4O_3^+$ [M+H]$^+$, 587.28; found, 587.3.

**Example 31: Preparation of 3-(5-((8-((4'-chloro-5,5-dimethyl-3,4,5,6-tetrahydro-[1,1'-biphenyl]-2-yl)methyl)-3,8-diazabicyclo [3.2.1] octan-3-yl)methyl)-1-oxoisoindolin-2-yl)piperidine-2,6-dione (GT-03996)**

Referring to the methods of Scheme 1 or Example 1, the target product GT-03996 was prepared using (2-(2,6-dioxopiperidin-3-yl)-1-oxoisoindolin-5-yl)methyl methanesulfonate and 8-((4'-chloro-5,5-dimethyl-3,4,5,6-tetrahydro-[1,1'-biphenyl]-2-yl)methyl)-3,8-diazabicyclo[3.2.1]octane prepared according to Intermediate Example 94 as the starting materials. The compound (GT-03996) was obtained as white solid (32 mg, yield: 42%). $^1$H NMR (400 MHz, MeOD) δ 7.67 (d, *J* = 8.0 Hz, 1H), 7.46 (s, 1H), 7.40 (d, *J* = 8.0 Hz, 1H), 7.32 (d, *J* = 8.0 Hz, 2H), 7.07 (d, *J* = 8.0 Hz, 2H), 5.08-5.03 (m, 1H), 4.47-4.27 (m, 2H), 3.67 (brs, 4H), 3.52 (brs, 2H), 2.89-2.73 (m, 3H), 2.72-2.70 (m, 1H), 2.69-2.55 (m, 2H), 2.43-2.35 (m, 1H), 2.25-2.20 (m, 2H), 2.13-2.02 (m, 3H), 1.90-1.84 (m, 2H), 1.49 (t, *J*= 8.0 Hz, 2H), 1.33-1.24 (m, 2H), 0.93 (s, 6H). LC/MS (ESI) m/z: calcd for $C_{35}H_{42}ClN_4O_3^+$ [M+H]$^+$, 601.29; found, 601.3.

**Example 32: Preparation of 3-(5-(((1R,5S)-6-((4'-chloro-5,5-dimethyl-3,4,5,6-tetrahydro-[1,1'-biphenyl]-2-yl) methyl)-3,6-diazabicyclo[3.1.1]heptan-3-yl)methyl)-1-oxoisoindolin-2-yl)piperidine-2,6-dione (GT-06194)**

Referring to the methods of Scheme 1 or Example 1, the target product GT-06194 was prepared as white solid (8 mg, yield: 12%). $^1$H NMR (400 MHz, MeOD) δ 7.82 (d, *J* = 8.0 Hz, 1H), 7.68 (s, 1H), 7.58 (d, *J*= 8.0 Hz, 1H), 7.25-7.19 (m, 2H), 6.97-6.92 (m, 2H), 5.12-5.07 (m, 1H), 4.58-4.37 (m, 2H), 3.80 (brs, 2H), 2.88-2.65 (m, 6H), 2.54-2.37 (m, 1H), 2.31-2.19 (m, 9H), 1.95-1.90 (m, 2H), 1.42-1.39 (m, 2H), 0.90 (s, 6H). LC/MS (ESI) m/z: calcd for $C_{34}H_{40}ClN_4O_3^+$ [M+H]$^+$, 587.28; found, 587.3.

**Example 33: Preparation of 3-(5-(((1S,4S)-5-((4'-chloro-5,5-dimethyl-3,4,5,6-tetrahydro-[1,1'-biphenyl]-2-yl)methyl)-2,5-diazabicyclo[2.2.1]heptan-2-yl)methyl)-1-oxoisoindolin-2-yl)piperidine-2,6-dione (GT-03997)**

Referring to the methods of Scheme 1 or Example 1, the target product GT-03997 was prepared as white solid (36 mg, yield: 48%). $^1$H NMR (400 MHz, MeOD) δ 7.89 (d, *J* = 8.0 Hz, 1H), 7.82-7.77 (m, 1H), 7.72-7.64 (m, 1H), 7.44 (d, *J= 8.0* Hz, 2H), 7.18 (d, *J* = 8.0 Hz, 2H), 5.22-5.17 (m, 1H), 4.62-4.51 (m, 2H), 4.44-4.16 (m, 2H), 3.91-3.68 (m, 2H), 2.99-2.87 (m, 1H), 2.83-2.77 (m, 1H), 2.58-2.49 (m, 1H), 2.42-2.39 (m, 2H), 2.26-2.10 (m, 3H), 1.61 (t, *J* = 6.4 Hz, 2H), 1.05 (s, 6H). LC/MS (ESI) m/z: calcd for $C_{34}H_{40}ClN_4O_3^+$ [M+H]$^+$, 587.28; found, 587.3.

**Example 34: Preparation of 3-(5-((4-((4'-chloro-5,5-dimethyl-3,4,5,6-tetrahydro-[1,1'-biphenyl]-2-yl)methyl)piperazin-1-yl)methyl)-1-oxoisoindolin-2-yl)piperidine-2,6-dione (GT-02785)**

Referring to the method of Scheme 1, the target product GT-02785 was prepared using (2-(2,6-dioxopiperidin-3-yl)-1-oxoisoindolin-5-yl)methyl methanesulfonate prepared according to Intermediate example 5 and 1-((4'-chloro-5,5-dimethyl-3,4,5,6-tetrahydro-[1,1'-biphenyl]-2-yl)methyl)piperazine (CAS No.: 1228780-72-0) as starting materials. The target product GT-02785 was prepared as white solid (20 mg, yield: 62%). $^1$H NMR (400 MHz, MeOD) δ 7.87 (d, *J* = 7.8 Hz, 1H), 7.73 (s, 1H), 7.63 (d, *J* = 7.8 Hz, 1H), 7.42 (d, *J* = 8.4 Hz, 2H), 7.12 (d, *J* = 8.4 Hz, 2H), 5.21-5.16 (m, 1H), 4.60-4.49 (m, 2H), 4.22 (brs, 2H), 3.61 (brs, 2H), 3.32 - 3.28 (m, 2H), 3.26 - 3,12 (m, 5H), 3.02 - 2.87 (m, 2H), 2.87 - 2.76 (m, 1H), 2.52 - 2.42 (m, 1H), 2.38 - 2.32 (m, 2H), 2.25 - 2.16 (m, 1H), 2.14 (s, 2H), 1.59 (t, *J* = 6.4 Hz, 2H), 1.04 (s, 6H). LC/MS (ESI) m/z: calcd for $C_{33}H_{40}ClN_4O_3^+$ [M+H]$^+$, 575.28; found, 575.3.

**Example 35: Preparation of 3-(5-((4-((4'-chloro-5,5-dimethyl-3,4,5,6-tetrahydro-[1,1'-biphenyl]-2-yl)methyl)piperazin-1-yl)amino)-1-oxoisoindolin-2-yl)piperidine-2,6-dione (GT-04252)**

Referring to the method of Scheme 4, the target product GT-04252 was prepared using 3-(1-oxo-5-(piperazin-1-ylamino)isoindolin-2-yl)piperidine-2,6-dione prepared according to Intermediate example 11 and 4'-chloro-5,5-dimethyl-3,4,5,6-tetrahydro-[1,1'-biphenyl]-2-carbaldehyde (CAS No.: 1228837-05-5) as starting materials. The target product GT-04252 was prepared as white solid (15 mg, yield: 17%). $^1$H NMR (400 MHz, MeOD) δ 7.44 (d, *J* = 8.4 Hz, 1H), 7.39 - 7.33 (m, 2H), 7.09 - 7.02 (m, 2H), 6.90 (s, 1H), 6.81 (dd, *J* = 8.4, 1.8 Hz, 1H), 5.00 - 4.95 (m, 1H), 4.35 - 4.16 (m, 2H), 3.62 (s, 2H), 3.46 - 3.29 (m, 2H), 3.10 - 2.95 (m, 2H), 2.90 - 2.75 (m, 6H), 2.72 - 2.60 (m, 1H), 2.40 - 2.29 (m, 1H), 2.21 - 2.18 (m, 2H), 2.05 (brs, 3H), 1.51 (t, *J* = 6.4 Hz, 2H), 0.94 (s, 6H). LC/MS (ESI) m/z: calcd for $C_{32}H_{39}ClN_5O_3^+$ [M+H]$^+$, 576.27; found, 576.3.

**Example 36: Preparation of 3-(5-((5-((4'-chloro-5,5-dimethyl-3,4,5,6-tetrahydro-[1,1'-biphenyl]-2-yl)methyl)-2,5-diazabicyclo[2.2.2]octan-2-yl)methyl)-1-oxoisoindolin-2-yl)piperidine-2,6-dione (GT-04167)**

Referring to the method of Scheme 1, the target product GT-04167 was prepared using (2-(2,6-dioxopiperidin-3-yl)-1-oxoisoindolin-5-yl)methyl methanesulfonate prepared according to Intermediate example 5 and 2-((4'-chloro-5,5-dimethyl-3,4,5,6-tetrahydro-[1,1'-biphenyl]-2-yl)methyl)-2,5-diazabicyclo[2.2.2]octane (GTC00247) as starting materials. The target product GT-04167 was prepared as white solid (29 mg, yield: 42%). $^1$H NMR (400 MHz, MeOD) δ 7.78 (d, *J* = 7.8 Hz, 1H), 7.69 - 7.45 (m, 2H), 7.34 (d, *J* = 7.4 Hz, 2H), 7.08 (d, *J* = 7.4 Hz, 2H), 5.11 - 5.05 (m, 1H), 4.53 - 4.37 (m, 2H), 4.21 - 4.01 (m, 4H), 3.88 - 3.72 (m, 2H), 3.61 - 3.32 (m, 2H), 2.87 - 2.78 (m, 1H), 2.75 - 2.61 (m, 1H), 2.48 - 2.37 (m, 1H), 2.32 - 2.18 (m, 3H), 2.15 - 1.96 (m, 3H), 1.96 - 1.62 (m, 2H), 1.49 (t, *J* = 6.4 Hz, 2H), 0.93 (s, 6H). LC/MS (ESI) m/z: calcd for $C_{35}H_{42}ClFN_4O_3^+$ [M+H]$^+$, 601.29; found, 601.3.

**Example 37: Preparation of 3-(5-((5-((4'-chloro-5,5-dimethyl-3,4,5,6-tetrahydro-[1,1'-biphenyl]-2-yl)methyl)-2,5-diazabicyclo [2.2.2] octan-2-yl)methyl)-4-fluoro-1-oxoisoindolin-2-yl)piperidine-2,6-dione (GT-04168)**

Referring to the method of Scheme 1, the target product GT-04168 was prepared using (2-(2,6-dioxopiperidin-3-yl)-4-fluoro-1-oxoisoindolin-5-yl)methyl methanesulfonate prepared according to Intermediate example 6 and 2-((4'-chloro-5,5-dimethyl-3,4,5,6-tetrahydro-[1,1'-biphenyl]-2-yl)methyl)-2,5-diazabicyclo[2.2.2]octane as starting materials. The target product GT-04168 was prepared as white solid (26 mg, yield: 37%). $^1$H NMR (400 MHz, MeOD) δ 7.61 (d, *J* = 6.4 Hz, 1H), 7.56 - 7.49 (m, 1H), 7.34 (d, *J* = 8.4 Hz, 2H), 7.08 (d, *J* = 8.4 Hz, 2H), 5.11 - 5.06 (m, 1H), 4.57 - 4.44 (m, 2H), 4.38 - 3.98 (m, 4H), 3.91 - 3.72 (m, 2H), 3.59 - 3.33 (m, 3H), 2.90 - 2.76 (m, 1H), 2.73 - 2.71 (m, 1H), 2.45 - 2.27 (m, 1H), 2.27 (brs, 2H), 2.21 - 1.96 (m, 6H), 1.94 - 1.6 (m, 2H), 1.49 (t, *J* = 6.5 Hz, 2H), 0.93 (s, 6H). LC/MS (ESI) m/z: calcd for $C_{35}H_{41}ClFN_4O_3^+$ [M+H]$^+$, 619.28; found, 619.3.

**Example 38: Preparation of 3-(5-((4-(4'-chloro-5,5-dimethyl-3,4,5,6-tetrahydro-[1,1'-biphenyl]-2-carbonyl)piperazin-1-yl)methyl)-1-oxoisoindolin-2-yl)piperidine-2,6-dione (GT-04224)**

Referring to the method of Scheme 6, the target product GT-04224 was prepared using 3-(1-oxo-5-(piperazin-1-ylmethyl)isoindolin-2-yl)piperidine-2,6-dione prepared according to Intermediate example 18 and 4'-chloro-5,5-dimethyl-3,4,5,6-tetrahydro-[1,1'-biphenyl]-2-carboxylic acid (CAS No.: 2716235-85-5) as starting materials. The target product GT-04224 was prepared as white solid (27 mg, yield: 43%). $^1$H NMR (400 MHz, MeOD) δ 7.93 *(d, J*= 7.8 Hz, 1H), 7.70 (s, 1H), 7.61 *(d, J*= 7.8 Hz, 1H), 7.47 - 7.34 (m, 2H), 7.27 - 7.21 (m, 2H), 5.22 - 5.18 (m, 1H), 4.69 - 4.50 (m, 2H), 4.36 (brs, 2H), 3.16 - 3.10 (m, 4H), 3.04 - 2.88 (m, 2H), 2.87 - 2.73 (m, 2H), 2.62 - 2.45 (m, 4H), 2.34 - 2.11 (m, 2H), 2.05 - 1.97 (m, 1H), 1.59 - 1.55 (m, 2H), 1.06 (s, 6H). LC/MS (ESI) m/z: calcd for $C_{33}H_{38}ClN_4O_4^+$ [M+H]$^+$, 589.26; found, 589.3.

**Example 39: Preparation of 3-(5-((4-((4'-chloro-[1,1'-biphenyl]-2-yl)methyl)piperazin-1-yl)amino)-1-oxoisoindolin-2-yl)piperidine-2,6-dione (GT-04196)**

Referring to the method of Scheme 2, the target product GT-04196 was prepared using 3-(1-oxo-5-(piperazin-1-ylamino)isoindolin-2-yl)piperidine-2,6-dione prepared according to Intermediate example 11 and 2-(bromomethyl)-4'-chloro-1,1'-biphenyl (CAS No.: 1001754-57-9) as starting materials. The target product GT-04196 was prepared as white solid (34 mg, yield: 56%). $^1$H NMR (400 MHz, MeOD) δ 7.71 - 7.63 (m, 1H), 7.53 - 7.40 (m, 5H), 7.35 - 7.23 (m, 3H), 6.88 (s, 1H), 6.78 (dd, *J* = 8.4, 1.8 Hz, 1H), 5.02 - 4.95 (m, 1H), 4.36 (s, 2H), 4.31 - 4.16 (m, 2H), 3.31 - 3.27 (m, 2H), 3.08 - 2.86 (m, 4H), 2.86 - 2.60 (m, 4H), 2.40 - 2.21 (m, 1H), 2.09 - 1.92 (m, 1H). LC/MS (ESI) m/z: calcd for $C_{30}H_{31}ClN_5O_3^+$ [M+H]$^+$, 544.21; found, 544.2.

**Example 40: Preparation of 3-(5-((1-((4'-chloro-[1,1'-biphenyl]-2-yl)methyl)azetidin-3-yl)amino)-6-fluoro-1-oxoisoindolin-2-yl)piperidine-2,6-dione (GT-04204)**

Referring to the method of Scheme 2, the target product GT-04204 was prepared using 3-(5-(azetidin-3-ylamino)-6-fluoro-1-oxoisoindolin-2-yl)piperidine-2,6-dione prepared according to Intermediate example 12 and 2-(bromomethyl)-4'-chloro-1,1'-biphenyl (CAS No.: 1001754-57-9) as starting materials. The target product GT-04204 was prepared as white solid (10 mg, yield: 19%). $^1$H NMR (400 MHz, MeOD) δ 7.54 - 7.33 (m, 5H), 7.35 - 7.25 (m, 4H), 6.59 - 6.39 (m, 1H), 5.01 - 4.96 (m, 1H), 4.47 (s, 2H), 4.44 - 4.29 (m, 3H), 4.24 (t, *J* = 11.2 Hz, 2H), 3.87 - 3.59 (m, 2H), 2.84 - 2.76 (m, 1H), 2.70 - 2.64 (m, 1H), 2.40 -2.21 (m, 1H), 2.12 - 1.92 (m, 1H). LC/MS (ESI) m/z: calcd for $C_{29}H_{27}ClFN_4O_3^+$ [M+H]$^+$, 533.18; found, 533.2.

**Example 41: Preparation of 3-(5-((4-((3-(4-chlorophenyl)pyridin-4-yl)methyl)piperazin-1-yl)methyl)-1-oxoisoindolin-2-yl)piperidine-2,6-dione (GT-04275)**

Referring to the method of Scheme 2, the target product GT-04275 was prepared using 3-(1-oxo-5-(piperazin-1-ylmethyl)isoindolin-2-yl)piperidine-2,6-dione and 4-(chloromethyl)-3-(4-chlorophenyl)pyridine prepared according to Intermediate example 64 as starting materials. The target product GT-04275 was prepared as white solid (36 mg, yield: 49%). $^1$H NMR (400 MHz, MeOD) δ 8.89 (d, *J* = 6.0 Hz, 1H), 8.81 (s, 1H), 8.47 (d, *J* = 6.0 Hz, 1H), 7.91 (d, *J* = 7.8 Hz, 1H), 7.88 (s, 1H), 7.75 (d, *J* = 7.8 Hz, 1H), 7.64 - 7.57 (m, 2H), 7.54 - 7.48 (m, 2H), 5.21 - 5.17 (m, 1H), 4.59 (d, *J* = 10.0 Hz, 2H), 4.55 (s, 2H), 3.93 (s, 2H), 3.40 (brs, 4H), 2.97 - 2.78 (m, 4H), 2.71 (brs, 1H), 2.59 - 2.48 (m, 1H), 2.27 - 2.11 (m, 1H). LC/MS (ESI) m/z: calcd for $C_{30}H_{31}ClN_5O_3^+$ [M+H]$^+$, 544.21; found, 544.2.

**Example 42: Preparation of 3-(5-((4-(3-(4-chlorophenyl)isonicotinoyl)piperazin-1-yl)methyl)-1-oxoisoindolin-2-yl)piperidine-2,6-dione (GT-04222)**

Referring to the method of Scheme 6, the target product GT-04222 was prepared using 3-(1-oxo-5-(piperazin-1-ylmethyl)isoindolin-2-yl)piperidine-2,6-dione prepared according to Intermediate example 18 and 3-(4-chlorophenyl) isonicotinic acid (CAS No.: 883107-39-9) as starting materials. The target product GT-04222 was prepared as white solid (23 mg, yield: 36%). $^1$H NMR (400 MHz, MeOD) δ 8.75 (s, 1H), 8.69 (d, *J* = 5.6 Hz, 1H), 7.78 (d, *J* = 5.6 Hz, 1H), 7.70 (d, *J* = 7.8 Hz, 1H), 7.58 (s, 1H), 7.45 (d, *J* = 7.8 Hz, 1H), 7.40 (d, *J* = 8.0 Hz, 2H), 7.33 (d, *J* = 8.0 Hz, 2H), 5.01 - 4.95 (m, 1H), 4.37 (q, *J* = 17.2 Hz, 2H), 4.24 (s, 2H), 3.43 - 3.26 (m, 2H), 3.26 - 3.12 (m, 3H), 3.06 - 2.96 (m, 2H), 2.77 - 2.68 (m, 2H), 2.63 - 2.57 (m, 1H), 2.36 - 2.27 (m, 1H), 2.07 - 1.91 (m, 1H). LC/MS (ESI) m/z: calcd for $C_{30}H_{29}ClN_5O_4^+$ [M+H]$^+$, 558.19; found, 558.2.

**Example 43: Preparation of 3-(5-((4-((4'-fluoro-[1,1'-biphenyl]-2-yl)methyl)piperazin-1-yl)methyl)-1-oxoisoindolin-2-yl)piperidine-2,6-dione (GT-04256)**

Referring to the method of Scheme 2, the target product GT-04256 was prepared using 3-(1-oxo-5-(piperazin-1-ylmethyl)isoindolin-2-yl)piperidine-2,6-dione prepared according to Intermediate example 18 and 2-(bromomethyl)-4'-chloro-1,1'-biphenyl (CAS No.: 1001754-57-9) as starting materials. The target product GT-04256 was prepared as white solid (28 mg, yield: 44%). $^1$H NMR (400 MHz, MeOD) δ 7.75 (d, *J* = 7.8 Hz, 1H), 7.58 - 7.54 (m, 2H), 7.49 (d, *J= 8.0* Hz, 1H), 7.39 - 7.36 (m, 2H), 7.29 - 7.22 (m, 3H), 7.11 (t, *J* = 8.8 Hz, 2H), 5.09 - 5.04 (m, 1H), 4.51 - 4.33 (m, 2H), 4.10 (brs, 2H), 3.97 (brs, 1H), 3.03 (brs, 4H), 2.92 - 2.64 (m, 6H), 2.46 - 2.35 (m, 1H), 2.16 - 1.97 (m, 1H). LC/MS (ESI) m/z: calcd for $C_{31}H_{32}FN_4O_3^+$ [M+H]$^+$, 527.25; found, 527.3.

**Example 44: Preparation of 3-(5-((4-(4'-fluoro-[1,1'-biphenyl]-2-carbonyl)piperazin-1-yl)methyl)-1-oxoisoindolin-2-yl)piperidine-2,6-dione (GT-04194)**

Referring to the method of Scheme 6, the target product GT-04194 was prepared using 3-(1-oxo-5-(piperazin-1-ylmethyl)isoindolin-2-yl)piperidine-2,6-dione prepared according to Intermediate example 18 and 4'-fluoro-[1,1'-biphenyl]-2-carboxylic acid (CAS No.: 1841-57-2) as starting materials. The target product GT-04194 was prepared as white solid (10 mg, yield: 16%). $^1$H NMR (400 MHz, MeOD) δ 7.92 (d, *J* = 7.8 Hz, 1H), 7.72 (s, 1H), 7.65 - 7.57 (m, 2H), 7.57 - 7.41 (m, 5H), 7.21 - 7.19 (m, 2H), 5.21 - 5.19 (m, 1H), 4.58 (q, *J* = 17.2 Hz, 2H), 4.41 (brs, 2H), 3.59 - 3.33 (m, 4H), 3.15 - 3.08 (m, 2H), 3.02 - 2.88 (m, 2H), 2.87 - 2.75 (m, 2H), 2.57 - 2.43 (m, 1H), 2.29 - 2.13 (m, 1H). LC/MS (ESI) m/z: calcd for $C_{31}H_{30}FN_4O_4^+$ [M+H]$^+$, 541.22; found, 541.3.

**Example 45: Preparation of 3-(5-((4-((4'-fluoro-3,4,5,6-tetrahydro-[1,1'-biphenyl]-2-yl)methyl)piperazin-1-yl)methyl)-1-oxoisoindolin-2-yl)piperidine-2,6-dione (GT-04193)**

Referring to the method of Scheme 1, the target product GT-04193 was prepared using (2-(2,6-dioxopiperidin-3-yl)-1-oxoisoindolin-5-yl)methyl methanesulfonate prepared according to Intermediate example 5 and 1-((4'-fluoro-3,4,5,6-tetrahydro-[1,1'-biphenyl]-2-yl)methyl)piperazine as starting materials. The target product GT-04193 was prepared as white solid (30 mg, yield: 46%). $^1$H NMR (400 MHz, MeOD) δ 7.89 (d, *J* = 7.8 Hz, 1H), 7.78 (s, 1H), 7.67 (d, *J* = 7.8 Hz, 1H), 7.24 - 7.07 (m, 4H), 5.21 - 5.17 (m, 1H), 4.56 (q, *J* = 17.2 Hz, 2H), 4.36 (brs, 2H), 3.68 (brs, 2H), 3.39 (brs, 8H), 3.00 - 2.87 (m, 1H), 2.86 - 2.72 (m, 1H), 2.61 - 2.42 (m, 1H), 2.37-2.34 (m, 4H), 2.27 - 2.12 (m, 1H), 1.82 (s, 4H). LC/MS (ESI) m/z: calcd for $C_{31}H_{36}FN_4O_3^+$ [M+H]$^+$, 531.28; found, 531.3.

**Example 46: Preparation of 3-(5-((4-(4'-fluoro-3,4,5,6-tetrahydro-[1,1'-biphenyl]-2-carbonyl)piperazin-1-yl)methyl)-1-oxoisoindolin-2-yl)piperidine-2,6-dione (GT-04170)**

Referring to the method of Scheme 1, the target product GT-04170 was prepared using (2-(2,6-dioxopiperidin-3-yl)-1-oxoisoindolin-5-yl)methyl methanesulfonate prepared according to Intermediate example 5 and (4'-fluoro-3,4,5,6-tetrahydro-[1,1'-biphenyl]-2-yl)(piperazin-1-yl)methanone prepared according to Intermediate example 24 as starting materials. The target product GT-04170 was prepared as white solid (42 mg, yield: 63%). $^1$H NMR (400 MHz, MeOD) δ 7.91 (d, *J* = 7.6 Hz, 1H), 7.74 (brs, 1H), 7.63 (brs, 1H), 7.32 - 7.23 (m, 2H), 7.17 - 7.00 (m, 2H), 5.30 - 5.12 (m, 1H), 4.69 - 4.51 (m, 3H), 4.35 - 4.20 (m, 2H), 4.15 - 3.79 (m, 1H), 3.51 - 3.41 (m, 2H), 3.27 - 3.03 (m, 2H), 2.99 - 2.91 (m, 1H), 2.87 - 2.77 (m, 1H), 2.76 - 2.61 (m, 2H), 2.61 - 2.43 (m, 2H), 2.29 - 2.09 (m, 3H), 1.95 - 1.60 (m, 4H). LC/MS (ESI) m/z: calcd for $C_{31}H_{34}FN_4O_4^+$ [M+H]$^+$, 545.26; found, 545.3.

**Example 47: Preparation of 3-(5-((3-(4'-fluoro-3,4,5,6-tetrahydro-[1,1'-biphenyl]-2-carbonyl)-3,6-diazabicyclo[3.1.1]heptan-6-yl)methyl)-1-oxoisoindolin-2-yl)piperidine-2,6-dione (GT-04169)**

Referring to the method of Scheme 1, the target product GT-04169 was prepared using (2-(2,6-dioxopiperidin-3-yl)-1-oxoisoindolin-5-yl)methyl methanesulfonate prepared according to Intermediate example 5 and (3,6-diazabicyclo[3.1.1]heptan-3-yl)(4'-fluoro-3,4,5,6-tetrahydro-[1,1'-biphenyl]-2-yl)methanone prepared according to Intermediate example 25 as starting materials. The target product GT-04169 was prepared as white solid (37 mg, yield: 54%). $^1$H NMR (400 MHz, MeOD) δ 7.87 (d, *J* = 7.8 Hz, 1H), 7.79 (s, 1H), 7.70 - 7.65 (m, 1H), 7.41 - 7.23 (m, 2H), 7.12 - 7.05 (m, 2H), 5.21 - 5.16 (m, 1H), 4.71 (s, 1H), 4.66 - 4.50 (m, 2H), 4.46 - 4.37 (m, 1H), 4.35 - 4.18 (m, 1H), 4.02 - 3.96 (m, 1H), 3.80 - 3.71 (m, 2H), 3.23 - 3.04 (m, 1H), 3.02 - 2.87 (m, 1H), 2.87 - 2.76 (m, 1H), 2.71 - 2.65 (m, 1H), 2.57 - 2.52 (m, 3H), 2.41 - 2.09 (m, 4H), 1.89 - 1.84 (m, 4H). LC/MS (ESI) m/z: calcd for $C_{32}H_{34}FN_4O_4^+$ [M+H]$^+$, 557.26; found, 557.3.

**Example 48: Preparation of 3-(5-((4-((4-(4-chlorophenyl)-6,6-dimethyl-5,6-dihydro-2H-pyran-3-yl)methyl)piperazin-1-yl)methyl)-1-oxoisoindolin-2-yl)piperidine-2,6-dione (GT-05672)**

Referring to the method of Scheme 1, the target product (GT-05672) was prepared as white solid (15 mg, yield 20.85%). 1H NMR (400 MHz, DMSO-d6) δ 11.01 (s, 1H), 7.87 - 7.78 (m, 2H), 7.72 (dd, J = 16.4, 8.2 Hz, 1H), 7.44 (d, J = 8.4 Hz, 2H), 7.20 (d, J = 8.3 Hz, 2H), 5.14 (dd, J = 13.2, 5.1 Hz, 1H), 4.42 (dd, J = 51.5, 17.8 Hz, 6H), 3.42 - 3.12 (m, 10H), 2.95 - 2.87 (m, 1H), 2.61 (d, J = 17.2 Hz, 1H), 2.46 - 2.36 (m, 1H), 2.20 (s, 2H), 2.01 (dd, J = 9.0, 3.6 Hz, 1H), 1.21 (s, 6H). LCMS (ESI) calcd for $C_{32}H_{38}ClN_4O_4^+$ $[M+H]^+$: 577.26, found, 577.3

**Example 49: Preparation of 3-(6-((4-((4'-chloro-5,5-dimethyl-3,4,5,6-tetrahydro-[1,1'-biphenyl]-2-yl)methyl)piperazin-1-yl)methyl)-1-oxoisoindolin-2-yl)piperidine-2,6-dione (GT-05363)**

Referring to the method of Scheme 1, the target product (GT-05363) was prepared as white solid (10 mg, yield 10%). $^1H$ NMR (400 MHz, DMSO-d6) δ 11.00 (s, 1H), 7.96 - 7.81 (m, 1H), 7.74 - 7.67 (m, 2H), 7.41 (d, *J* = 8.2 Hz, 2H), 7.10 (d, *J* = 7.6 Hz, 2H), 5.12 (dd, *J* = 13.2, 5.0 Hz, 1H), 4.51 - 4.34 (m, 4H), 3.56 - 3.40 (m, 4H), 3.08 - 3.05 (m, 2H), 2.99 - 2.85 (m, 2H), 2.86 - 2.71 (m, 2H), 2.70 - 2.57 (m, 2H), 2.47 - 2.36 (m, 1H), 2.29 - 2.21 (m, 2H), 2.09 - 1.93 (m, 3H), 1.53 - 1.36 (m, 2H), 0.96 (s, 6H). LCMS (ESI) calcd for $C_{33}H_{40}ClN_4O_3^+$ $[M+H]^+$: 575.28, found, 575.3.

**Example 50: Preparation of 3-(7-((4-((4'-chloro-5,5-dimethyl-3,4,5,6-tetrahydro-[1,1'-biphenyl]-2-yl)methyl)piperazin-1-yl)methyl)-1-oxoisoindolin-2-yl)piperidine-2,6-dione (GT-05360)**

Referring to the method of Scheme 1, the target product (GT-05360) was prepared as white solid (31 mg, yield 30%). $^1H$ NMR (400 MHz, DMSO-d6) δ 11.04 (s, 1H), 7.69 (brs, 3H), 7.41 (d, *J* = 8.0 Hz, 2H), 7.11 (d, *J* = 7.9 Hz, 2H), 5.12 (dd, *J* = 13.1, 5.0 Hz, 1H), 4.94 - 4.88(m, 2H), 4.49 (d, *J* = 17.6 Hz, 1H), 4.38 (d, *J* = 17.9 Hz, 1H), 3.67 - 3.61 (m, 4H), 2.99 - 2.86 (m, 2H), 2.73 (s, 1H), 2.62 (d, *J* = 17.2 Hz, 1H), 2.56 - 2.51 (m, 4H), 2.44 - 2.40 (m, 1H), 2.32 - 2.24 (m, 2H), 2.01 (brs, 3H), 1.46 - 1.43 (m, 2H), 0.95 (s, 6H). LCMS (ESI) calcd for $C_{33}H_{40}ClN_4O_3^+$ $[M+H]^+$: 575.28, found, 575.3.

**Example 51: Preparation of 3-(5-(2-(4-((4'-chloro-5,5-dimethyl-3,4,5,6-tetrahydro-[1,1'-biphenyl]-2-yl)methyl)piperazin-1-yl)ethyl)-1-oxoisoindolin-2-yl)piperidine-2,6-dione (GT-05361)**

Referring to the method of Scheme 1, the target product (GT-05361) was prepared as white solid (28 mg, yield 26%). $^1H$ NMR (400 MHz, DMSO-d6) δ 10.98 (s, 1H), 7.70 (d, *J* = 8.0 Hz, 1H), 7.50 (s, 1H), 7.44 - 7.40 (m, 3H), 7.12 (d, *J* = 6.5 Hz, 2H), 5.11 (dd, *J* = 13.4, 4.8 Hz, 1H), 4.37 (dd, *J* = 51.1, 17.5 Hz, 2H), 3.62 - 3.49 (m, 3H), 3.14 - 3.05 (m, 3H), 2.97 - 2.85 (m, 3H), 2.69 - 2.54 (m, 4H), 2.46 - 2.29 (m, 3H), 2.26 - 2.19 (m, 2H), 2.15 - 1.94 (m, 4H), 1.54 - 1.38 (m, 2H), 0.96 (s, 6H). LCMS (ESI) calcd for $C_{34}H_{42}ClN_4O_3^+$ $[M+H]^+$: 589.29, found, 589.3.

**Example 52: Preparation of 3-(5-(3-(4-((4'-chloro-5,5-dimethyl-3,4,5,6-tetrahydro-[1,1'-biphenyl]-2-yl)methyl)piperazin-1-yl)propyl)-1-oxoisoindolin-2-yl)piperidine-2,6-dione (GT-05362)**

Referring to the method of Scheme 1, the target product (GT-05362) was prepared as white solid (28 mg, yield 33%). $^1H$ NMR (400 MHz, DMSO-d6) δ 10.98 (s, 1H), 7.67 (d, *J* = 7.8 Hz, 1H), 7.47 (s, 1H), 7.41 - 7.37 (m, 3H), 7.12 (d, *J* = 7.9 Hz, 2H), 5.11 (dd, *J* = 13.3, 5.1 Hz, 1H), 4.43 (d, *J* = 17.3 Hz, 1H), 4.30 (d, *J* = 17.3 Hz, 1H), 3.77 - 3.55 (m, 4H), 3.39 -3.31 (m, 4H), 3.13 - 3.02 (m, 3H), 2.99 - 2.85 (m, 2H), 2.77 - 2.70 (m, 2H), 2.60 (d, *J* = 16.4 Hz, 1H), 2.42 - 2.38 (m, 1H), 2.35 - 2.23 (m, 2H), 2.09 - 1.91 (m, 5H), 1.45 - 1.41(m, 2H), 0.96 (s, 6H). LCMS (ESI) calcd for $C_{35}H_{44}ClN_4O_3^+$ $[M+H]^+$: 603.31, found, 603.3.

**Example 53: Preparation of 3-(4-fluoro-5-((4-((4'-fluoro-5,5-dimethyl-3,4,5,6-tetrahydro-[1,1'-biphenyl]-2-yl)methyl)piperazin-1-yl)methyl)-1-oxoisoindolin-2-yl)piperidine-2,6-dione (GT-05390)**

Referring to the method of Scheme 1, the target product (GT-05390) was prepared as white solid (29 mg, yield 32%). $^1H$ NMR (400 MHz, DMSO-d6) δ 11.02 (s, 1H), 7.71 - 7.63 (m, 2H), 7.21 - 7.11 (m, 4H), 5.13 (dd, *J* = 13.3, 5.0 Hz, 1H), 4.59 - 4.38(m, 2H), 4.16 - 3.90 (m, 2H), 3.43 - 3.28 (m, 5H), 3.15 - 3.03(m, 2H), 2.98 - 2.87 (m, 2H), 2.81 - 2.72 (m, 1H), 2.61 (d, J= 17.6 Hz, 2H), 2.45 (dd, *J* = 13.2, 4.9 Hz, 2H), 2.28 (brs, 2H), 2.02 (brs, 3H), 1.45 (t, *J* = 6.2 Hz, 2H), 0.95 (s, 6H). LCMS (ESI) calcd for $C_{33}H_{39}F_2N_4O_3^+$ $[M+H]^+$: 577.30, found, 577.3.

**Example 54: Preparation of 3-(6-fluoro-5-((4-((4'-fluoro-5,5-dimethyl-3,4,5,6-tetrahydro-[1,1'-biphenyl]-2-yl) methyl)piperazin-1-yl)methyl)-1-oxoisoindolin-2-yl)piperidine-2,6-dione (GT-05391)**

Referring to the method of Scheme 1, the target product (GT-05391) was prepared as white solid (34 mg, yield 38%). $^1$H NMR (400 MHz, DMSO-d6) δ 11.02 (s, 1H), 7.78 - 7.70 (m, 1H), 7.59 (d, *J* = 8.1 Hz, 1H), 7.19 (t, *J* = 8.8 Hz, 2H), 7.13 (t, *J* = 6.9 Hz, 2H), 5.13 (dd, *J* = 13.3, 5.1 Hz, 1H), 4.39 (dd, *J* = 49.9, 17.4 Hz, 2H), 4.10 - 3.98 (m, 1H), 3.30 - 3.20 (m, 5H), 2.94 - 2.87(m, 5H), 2.72 - 2.63 (m, 3H), 2.41 (dd, *J* = 13.3, 4.7 Hz, 1H), 2.29 (brs, 2H), 2.02 (brs, 3H), 1.45 (t, *J* = 6.2 Hz, 2H), 0.95 (s, 6H). LCMS (ESI) calcd for $C_{33}H_{39}F_2N_4O_3^+$ $[M+H]^+$: 577.30, found, 577.3.

**Example 55: Preparation of 3-(7-fluoro-5-((4-((4'-fluoro-5,5-dimethyl-3,4,5,6-tetrahydro-[1,1'-biphenyl]-2-yl) methyl)piperazin-1-yl)methyl)-1-oxoisoindolin-2-yl)piperidine-2,6-dione (GT-05392)**

Referring to the method of Scheme 1, the target product (GT-05392) was prepared as white solid (7 mg, yield 8%). $^1$H NMR (400 MHz, DMSO-d6) δ 11.01 (s, 1H), 7.62 - 7.35 (m, 2H), 7.24 - 7.09 (m, 4H), 5.09 (dd, *J* = 13.2, 5.1 Hz, 1H), 4.56 - 4.29 (m, 3H), 3.37 - 3.27 (m, 4H), 3.30 - 3.00 (m, 4H), 2.98 - 2.85 (m, 2H), 2.83 - 2.72 (m, 1H), 2.60 (d, *J* = 17.5 Hz, 2H), 2.39 (dd, *J* = 13.2, 4.7 Hz, 1H), 2.35 - 2.30 (m, 2H), 2.11 - 1.91 (m, 3H), 1.45 (t, *J* = 6.1 Hz, 2H), 0.95 (s, 6H). LCMS (ESI) calcd for $C_{33}H_{39}F_2N_4O_3^+$ $[M+H]^+$: 577.30, found, 577.3.

**Example 56: Preparation of 3-(4-((4-((4'-fluoro-5,5-dimethyl-3,4,5,6-tetrahydro-[1,1'-biphenyl]-2-yl)methyl)piperazin-1-yl)methyl)-1-oxoisoindolin-2-yl)piperidine-2,6-dione (GT-05393)**

Referring to the method of Scheme 1, the target product (GT-05393) was prepared as white solid (10 mg, yield 11%). $^1$H NMR (400 MHz, DMSO-d6) δ 11.01 (s, 1H), 7.74 - 7.70 (m, 2H), 7.61 - 7.54 (m, 2H), 7.20 - 7.10 (m, 4H), 5.16 (dd, *J* = 13.3, 5.0 Hz, 2H), 4.91 (s, 2H), 4.69 - 4.26 (m, 5H), 3.00 - 2.87 (m, 3H), 2.71 - 2.56 (m, 4H), 2.49 - 2.44 (m, 2H), 2.25 - 2.21 (m, 2H), 2.07 - 2.02 (m, 4H), 1.46 (t, *J*= 6.1 Hz, 2H), 0.95 (s, 6H). LCMS (ESI) calcd for $C_{33}H_{40}FN_4O_3^+$ $[M+H]^+$: 559.31, found, 559.3.

**Example 57: Preparation of 3-(6-((4-((4'-fluoro-5,5-dimethyl-3,4,5,6-tetrahydro-[1,1'-biphenyl]-2-yl)methyl)piperazin-1-yl)methyl)-1-oxoisoindolin-2-yl)piperidine-2,6-dione (GT-05394)**

Referring to the method of Scheme 1, the target product (GT-05394) was prepared as white solid (15 mg, yield 16%). $^1$H NMR (400 MHz, DMSO-d6) δ 11.01 (s, 1H), 7.90 (brs, 1H), 7.83 - 7.54 (m, 3H), 7.19 - 7.12 (m, 3H), 5.12 (dd, *J* = 13.2, 4.9 Hz, 1H), 4.53 - 4.30 (m, 4H), 3.32 - 3.29 (m, 4H), 3.26 - 3.10 (m, 4H), 2.98 - 2.86 (m, 2H), 2.61 (d, *J* = 17.7 Hz, 2H), 2.46 - 2.37 (m, 1H), 2.33 - 2.27 (m, 2H), 2.01 (brs, 3H), 1.44 (t, *J* = 6.4 Hz, 2H), 0.95 (s, 6H). LCMS (ESI) calcd for $C_{33}H_{40}FN_4O_3^+$ $[M+H]^+$: 559.31, found, 559.3.

**Example 58: Preparation of 3-(5-((4-((4'-chloro-5,5-dimethyl-3,4,5,6-tetrahydro-[1,1'-biphenyl]-2-yl)methyl)-1,4-diazepan-1-yl)methyl)-1-oxoisoindolin-2-yl)piperidine-2,6-dione (GT-05396)**

Referring to the method of Scheme 1, the target product (GT-05396) was prepared as white solid (14 mg, yield 17%). $^1$H NMR (400 MHz, DMSO-d6) δ 11.01 (s, 1H), 7.89 - 7.86 (m, 1H), 7.82 - 7.62 (m, 2H), 7.46 - 7.42 (m, 2H), 7.14 (d, *J* = 7.6 Hz, 2H), 5.14 (dd, *J* = 10.9, 2.6 Hz, 1H), 4.67 - 4.24 (m, 4H), 3.82 - 3.59 (m, 4H), 3.10 - 2.89 (m, 4H), 2.61 (d, *J* = 17.2 Hz, 1H), 2.49 - 2.42 (m, 1H), 2.28 - 2.22 (m, 2H), 2.07 (s, 4H), 1.45 (t, *J* = 6.0 Hz, 2H), 0.95 (s, 6H). LCMS (ESI) calcd for $C_{34}H_{42}ClN_4O_3^+$ $[M+H]^+$: 589.29, found, 589.3.

**Example 59: Preparation of 3-(5-((6-((4'-chloro-5,5-dimethyl-3,4,5,6-tetrahydro-[1,1'-biphenyl]-2-yl)methyl)-3,6-diazabicyclo[3.1.1]heptan-3-yl)methyl)-4-fluoro-1-oxoisoindolin-2-yl)piperidine-2,6-dione (GT-05529)**

Referring to the method of Scheme 1, the target product (GT-05529) was prepared as white solid (23 mg, yield 26%). $^1$H NMR (400 MHz, DMSO-d6) δ 11.01 (s, 1H), 7.59 - 7.55 (m, 2H), 7.49 - 7.46 (m, 1H), 7.39 (d, *J* = 8.4 Hz, 1H), 7.26 - 7.14 (m, 2H), 5.17 - 5.05 (m, 1H), 4.63 - 4.19 (m, 3H), 4.05 - 4.00 (m, 1H), 3.84 - 3.65 (m, 2H), 3.31 - 3.13 (m, 4H), 2.95 - 2.85 (m, 1H), 2.77 - 2.62 (m, 3H), 2.45 - 2.42 (m, 1H), 2.33 - 2.15 (m, 2H), 2.12 - 1.90 (m, 5H), 1.44 (t, *J* = 6.1 Hz, 2H), 0.94 (s, 6H). LCMS (ESI) calcd for $C_{34}H_{39}ClFN_4O_3^+$ $[M+H]^+$: 605.27, found, 605.3.

**Example 60: Preparation of 3-(5-((8-((4'-chloro-5,5-dimethyl-3,4,5,6-tetrahydro-[1,1'-biphenyl]-2-yl)methyl)-3,8-diazabicyclo [3.2.1] octan-3-yl)methyl)-4-fluoro-1-oxoisoindolin-2-yl)piperidine-2,6-dione (GT-05534)**

Referring to the method of Scheme 1, the target product (GT-05534) was prepared as white solid (13 mg, yield

45%). $^1H$ NMR (400 MHz, DMSO-d6) δ 11.01 (s, 1H), 7.64 - 7.53 (m, 2H), 7.44 (d, *J* = 8.4 Hz, 2H), 7.19 (d, *J* = 8.4 Hz, 2H), 5.12 (dd, *J* = 13.3, 5.1 Hz, 1H), 4.55 (d, *J* = 17.5 Hz, 1H), 4.38 (d, *J* = 17.5 Hz, 1H), 3.75 (d, *J* = 21.7 Hz, 4H), 3.47 - 3.43(m, 2H), 3.00 - 2.79 (m, 3H), 2.73 - 2.68 (m, 2H), 2.61 (d, *J* = 17.2 Hz, 1H), 2.45 - 2.42 (m, 1H), 2.29 (s, 2H), 2.07 - 1.99 (m, 2H), 2.04 - 1.94 (m, 1H), 1.76 (d, *J* = 8.3 Hz, 2H), 1.46 (t, *J* = 6.1 Hz, 2H), 1.19 (t, *J* = 7.3 Hz, 2H), 0.96 (s, 6H). LCMS (ESI) calcd for $C_{35}H_{41}ClFN_4O_3^+$ $[M+H]^+$: 619.28, found, 619.3.

**Example 61: Preparation of 3-(5-((3-((4'-chloro-5,5-dimethyl-3,4,5,6-tetrahydro-[1,1'-biphenyl]-2-yl)methyl)-3,6-diazabicyclo[3.1.1]heptan-6-yl)methyl)-6-fluoro-1-oxoisoindolin-2-yl)piperidine-2,6-dione (GT-05367)**

Referring to the method of Scheme 1, the target product (GT-05367) was prepared as white solid (11 mg, yield 13%). $^1H$ NMR (400 MHz, DMSO-d6) δ 11.02 (s, 1H), 7.68 (d, *J* = 8.5 Hz, 1H), 7.60 (d, *J* = 8.8 Hz, 1H), 7.39 (d, *J* = 8.3 Hz, 2H), 7.09 (d, *J* = 8.2 Hz, 2H), 5.17 - 5.12 (m, 2H), 4.52 - 4.47 (m, 2H), 4.38 - 4.32 (m, 3H), 2.95 - 2.87 (m, 4H), 2.63 - 2.57 (m, 2H), 2.45 - 2.36 (m, 2H), 2.35 - 2.14 (m, 4H), 2.08 - 2.00 (m, 5H), 1.44 - 1.41 (m, 2H), 0.96 (s, 6H). LCMS (ESI) calcd for $C_{34}H_{30}ClFN_4O_3^+$ $[M+H]^+$: 605.27, found, 605.3.

**Example 62: Preparation of 3-(5-((6-((4'-chloro-5,5-dimethyl-3,4,5,6-tetrahydro-[1,1'-biphenyl]-2-yl)methyl)-3,6-diazabicyclo[3.1.1]heptan-3-yl)methyl)-6-fluoro-1-oxoisoindolin-2-yl)piperidine-2,6-dione (GT-05530)**

Referring to the method of Scheme 1, the target product (GT-05530) was prepared as white solid (25 mg, yield 29%). $^1H$ NMR (400 MHz, DMSO-d6) δ 11.01 (s, 1H), 9.02 (brs, 1H), 7.72 (dd, *J*= 9.9, 6.1 Hz, 1H), 7.54 - 7.51 (m, 1H), 7.50 - 7.47 (m, 1H), 7.36 (t, *J*= 7.9 Hz, 1H), 7.28 - 7.12 (m, 2H), 5.18 - 5.08 (m, 1H), 4.44 - 4.42 (m, 1H), 4.38 - 4.22 (m, 1H), 4.12 - 3.92 (m, 1H), 3.79 - 3.68 (m, 2H), 3.52 - 3.40 (m, 3H), 3.32 - 3.15 (m, 2H), 3.08 - 2.83 (m, 2H), 2.77 - 2.56 (m, 3H), 2.46 - 2.35 (m, 1H), 2.30 - 2.13 (m, 3H), 2.11 - 2.02 (m, 4H), 1.46 - 1.43 (m, 2H), 0.95 (s, 6H). LCMS (ESI) calcd for $C_{34}H_{39}ClFN_4O_3^+$ $[M+H]^+$: 605.27, found, 605.3.

**Example 63: Preparation of 3-(5-((8-((4'-chloro-5,5-dimethyl-3,4,5,6-tetrahydro-[1,1'-biphenyl]-2-yl)methyl)-3,8-diazabicyclo [3.2.1] octan-3-yl)methyl)-6-fluoro-1-oxoisoindolin-2-yl)piperidine-2,6-dione (GT-05535)**

Referring to the method of Scheme 1, the target product (GT-05535) was prepared as white solid (11 mg, yield 38%). $^1H$ NMR (400 MHz, DMSO-d6) δ 11.01 (s, 1H), 7.75 (s, 1H), 7.54 (d, *J* = 8.7 Hz, 1H), 7.44 (d, *J* = 8.4 Hz, 2H), 7.20 (d, *J* = 8.4 Hz, 2H), 5.12 (dd, *J* = 13.3, 5.1 Hz, 1H), 4.44 (d, *J* = 17.4 Hz, 1H), 4.31 *(d, J*= 17.4 Hz, 1H), 3.83 (brs, 2H), 3.75 (brs, 2H), 3.49 - 3.41 (m, 3H), 3.24 - 3.09 (m, 2H), 2.99 - 2.86 (m, 1H), 2.80 (brs, 1H), 2.61 (d, *J* = 16.9 Hz, 1H), 2.47 - 2.31 (m, 3H), 2.07 (s, 2H), 2.05 - 1.96 (m, 1H), 1.85 (brs, 2H), 1.46 (t, *J* = 6.2 Hz, 2H), 1.21 - 1.15 (m, 2H), 0.96 (s, 6H). LCMS (ESI) calcd for $C_{35}H_{41}ClFN_4O_3^+$ $[M+H]^+$: 619.28, found, 619.3.

**Example 64: Preparation of 3-(5-((3-((4'-chloro-5,5-dimethyl-3,4,5,6-tetrahydro-[1,1'-biphenyl]-2-yl)methyl)-3,6-diazabicyclo[3.1.1]heptan-6-yl)methyl)-7-fluoro-1-oxoisoindolin-2-yl)piperidine-2,6-dione (GT-05383)**

Referring to the method of Scheme 1, the target product (GT-05383) was prepared as white solid (30 mg, yield 30%). $^1H$ NMR (400 MHz, DMSO-d6) δ 11.02 (s, 1H), 7.59 - 7.55 (m, 1H), 7.53 - 7.49 (m, 1H), 7.39 (d, *J* = 8.4 Hz, 2H), 7.09 (d, *J* = 8.2 Hz, 2H), 5.15 - 5.06 (m, 1H), 4.64 - 4.25 (m, 6H), 2.95 - 2.83 (m, 4H), 2.67 - 2.57 (m, 2H), 2.33 - 2.23 (m, 5H), 2.02 - 1.91 (m, 5H), 1.42 (t, *J* = 6.7 Hz, 2H), 0.96 (d, *J* = 3.0 Hz, 6H). LCMS (ESI) calcd for $C_{34}H_{39}ClFN_4O_3^+$ $[M+H]^+$: 605.27, found, 605.3.

**Example 65: Preparation of 3-(5-((6-((4'-chloro-5,5-dimethyl-3,4,5,6-tetrahydro-[1,1'-biphenyl]-2-yl)methyl)-3,6-diazabicyclo[3.1.1]heptan-3-yl)methyl)-7-fluoro-1-oxoisoindolin-2-yl)piperidine-2,6-dione (GT-05531)**

Referring to the method of Scheme 1, the target product (GT-05531) was prepared as white solid (22 mg, yield 25%). $^1H$ NMR (400 MHz, DMSO-d6) δ 11.01 (s, 1H), 7.53 - 7.47 (m, 1H), 7.38 - 7.35 (m, 2H), 7.26 - 7.21 (m, 2H), 7.18 - 7.15 (m, 1H), 5.08 (dd, *J* = 13.3, 5.0 Hz, 1H), 4.53 - 4.50 (m, 1H), 4.49 - 4.45 (m, 1H), 4.41 - 4.29 (m, 1H), 3.84 - 3.79 (m, 1H), 3.63 (s, 1H), 3.31 - 3.14 (m, 3H), 3.12 - 3.01 (m, 1H), 2.98 - 2.78 (m, 2H), 2.62 - 2.51 (m, 3H), 2.46 - 2.35 (m, 1H), 2.30 - 2.19 (m, 2H), 2.13 - 1.89 (m, 5H), 1.45 (d, *J* = 5.8 Hz, 2H), 0.97 (s, 6H). LCMS (ESI) calcd for $C_{34}H_{39}ClFN_4O_3^+$ $[M+H]^+$: 605.27, found, 605.3.

**Example 66: Preparation of 3-(5-((8-((4'-chloro-5,5-dimethyl-3,4,5,6-tetrahydro-[1,1'-biphenyl]-2-yl)methyl)-3,8-diazabicyclo [3.2.1] octan-3-yl)methyl)-7-fluoro-1-oxoisoindolin-2-yl)piperidine-2,6-dione (GT-05536)**

Referring to the method of Scheme 1, the target product (GT-05536) was prepared as white solid (12 mg, yield

42%). $^{1}$H NMR (400 MHz, DMSO-d6) δ 11.00 (s, 1H), 7.45 (d, *J* = 8.4 Hz, 2H), 7.39 (s, 1H), 7.27 - 7.23 (m, 1H), 7.21 (t, *d* = 7.5 Hz, 2H), 5.07 (dd, *J* = 13.3, 5.1 Hz, 1H), 4.45 (d, *J* = 17.8 Hz, 1H), 4.32 *(d, J*= 17.9 Hz, 1H), 3.72 (s, 4H), 3.49 - 3.47 (m, 2H), 2.99 - 2.82 (m, 3H), 2.73 - 2.62 (m, 2H), 2.60 (d, *J* = 16.7 Hz, 1H), 2.40 - 2.33 (m, 3H), 2.08 (s, 2H), 2.01 - 1.98 (m, 1H), 1.83 (brs, 2H), 1.47 (t, *J* = 6.2 Hz, 2H), 1.19 (t, *J* = 7.3 Hz, 2H), 0.96 (s, 6H). LCMS (ESI) calcd for $C_{35}H_{41}ClFN_4O_3^+$ [M+H]$^+$: 619.29, found, 619.3.

**Example 67: Preparation of 3-(5-((4-(4'-chloro-5,5-dimethyl-3,4,5,6-tetrahydro-[1,1'-biphenyl]-2-carbonyl)piperazin-1-yl)methyl)-4-fluoro-1-oxoisoindolin-2-yl)piperidine-2,6-dione (GT-05525)**

Referring to the method of Scheme 1, the target product (GT-05525) was prepared as white solid (6 mg, yield 7%). $^{1}$H NMR (400 MHz, DMSO-d6) δ 11.03 (s, 1H), 7.75 (s, 1H), 7.68 (s, 1H), 7.43 - 7.36 (m, 2H), 7.29 - 7.10 (m, 2H), 5.14 (dd, *J* = 13.2, 4.7 Hz, 1H), 4.60 - 4.41 (m, 4H), 3.78 - 3.54 (m, 1H), 3.29 - 3.18 (m, 3H), 3.15 - 2.85 (m, 4H), 2.61 (d, *J* = 16.8 Hz, 1H), 2.48 - 2.28 (m, 4H), 2.22 - 2.11 (m, 1H), 2.06 - 1.97 (m, 1H), 1.92 - 1.88 (m, 1H), 1.56 - 1.34 (m, 2H), 0.98 (s, 6H). LCMS (ESI) calcd for $C_{33}H_{37}ClFN_4O_4^+$ [M+H]$^+$: 607.25, found, 607.3.

**Example 68: Preparation of 3-(5-((4-(4'-chloro-5,5-dimethyl-3,4,5,6-tetrahydro-[1,1'-biphenyl]-2-carbonyl)piperazin-1-yl)methyl)-6-fluoro-1-oxoisoindolin-2-yl)piperidine-2,6-dione (GT-05526)**

Referring to the method of Scheme 1, the target product (GT-05526) was prepared as white solid (11 mg, yield 13%). $^{1}$H NMR (400 MHz, DMSO-d6) δ 11.02 (s, 1H), 7.87 (s, 1H), 7.65 (s, 1H), 7.38 (d, *J* = 8.5 Hz, 2H), 7.20 (s, 2H), 5.14 (dd, *J* = 13.2, 5.0 Hz, 1H), 4.49 - 4.32 (m, 4H), 3.65 (brs, 1H), 3.30 - 3.18 (m, 5H), 3.09 - 3.05 (m, 1H), 2.98 - 2.87 (m, 1H), 2.61 (d, *J* = 16.8 Hz, 1H), 2.48 - 2.27 (m, 4H), 2.22- 2.16 (m, 1H), 2.07 - 1.96 (m, 1H), 1.92 - 1.87 (m, 1H), 1.43 (brs, 2H), 0.96 (s, 6H). LCMS (ESI) calcd for $C_{33}H_{37}ClFN_4O_4^+$ [M+H]$^+$: 607.25, found, 607.3.

**Example 69: Preparation of 3-(5-((4-(4'-chloro-5,5-dimethyl-3,4,5,6-tetrahydro-[1,1'-biphenyl]-2-carbonyl)piperazin-1-yl)methyl)-7-fluoro-1-oxoisoindolin-2-yl)piperidine-2,6-dione (GT-05527)**

Referring to the method of Scheme 1, the target product (GT-05527) was prepared as white solid (5 mg, yield 6%). $^{1}$H NMR (400 MHz, DMSO-d6) δ 11.02 (s, 1H), 7.56 - 7.49 (m, 2H), 7.38 (d, *J* = 8.5 Hz, 2H), 7.29 - 7.10 (m, 2H), 5.10 (dd, *J* = 13.1, 4.9 Hz, 1H), 4.63 - 4.18 (m, 4H), 3.77 - 3.49 (m, 2H), 3.21 - 3.07 (m, 1H), 3.03 - 2.81 (m, 3H), 2.60 (d, *J* = 16.3 Hz, 2H), 2.47 - 2.26 (m, 4H), 2.20 - 2.09 (m, 2H), 2.05 - 1.96 (m, 1H), 1.96 - 1.82 (m, 1H), 1.50 - 1.44 (m, 2H), 0.97 (s, 6H). LCMS (ESI) calcd for $C_{33}H_{37}ClFN_4O_4^+$ [M+H]$^+$: 607.25, found, 607.3.

**Example 70: Preparation of 3-(4-((4-(4'-chloro-5,5-dimethyl-3,4,5,6-tetrahydro-[1,1'-biphenyl]-2-carbonyl)piperazin-1-yl)methyl)-1-oxoisoindolin-2-yl)piperidine-2,6-dione (GT-05528)**

Referring to the method of Scheme 1, the target product (GT-05528) was prepared as white solid (9 mg, yield 11%). $^{1}$H NMR (400 MHz, DMSO-d6) δ 11.06 (s, 1H), 7.97 - 7.69 (m, 2H), 7.65 - 7.59 (m, 1H), 7.43 - 7.38 (m, 2H), 7.29 - 7.15 (m, 2H), 5.26 - 5.09 (m, 1H), 4.74 - 4.62 (m, 1H), 4.54 - 4.20 (m, 3H), 3.74 - 3.63 (m, 1H), 3.33 - 3.18 (m, 4H), 3.07 - 3.88 (m, 3H), 2.64 (d, *J* = 17.8 Hz, 1H), 2.41 - 2.27 (m, 4H), 2.27 - 2.11 (m, 1H), 2.09 - 2.01 (m, 2H), 1.92 - 1.88 (m, 1H), 1.60 - 1.36 (m, 2H), 0.97 (s, 6H). LCMS (ESI) calcd for $C_{33}H_{38}ClN_4O_4^+$ [M+H]$^+$: 589.26, found, 589.3.

**Example 71: Preparation of 3-(5-(4-((4-(4'-chloro-5,5-dimethyl-3,4,5,6-tetrahydro-[1,1'-biphenyl]-2-carbonyl)piperazin-1-yl)methyl)phenyl)-1-oxoisoindolin-2-yl)piperidine-2,6-dione (GT-05359)**

Referring to the method of Scheme 6, the target product (GT-05359) was prepared as white solid (11 mg, yield 16%). $^{1}$H NMR (400 MHz, DMSO-d6) δ 11.01 (s, 1H), 7.93 (s, 1H), 7.87 - 7.83 (m, 1H), 7.57 - 7.50 (m, 2H), 7.44 - 7.38 (m, 2H), 7.23 - 7.15 (m, 4H), 6.81 (d, *J* = 7.9 Hz, 1H), 5.15 (dd, *J* = 13.2, 5.1 Hz, 1H), 4.53 (d, *J* = 17.5 Hz, 1H), 4.40 (d, *J* = 17.5 Hz, 1H), 4.36 - 4.22 (m, 1H), 4.13 - 4.08 (m, 1H), 3.14 - 3.02 (m, 4H), 2.95 - 2.89 (m, 2H), 2.67 - 2.58 (m, 1H), 2.43 - 2.30 (m, 4H), 2.22 - 2.08 (m, 2H), 2.07 - 2.00 (m, 1H), 1.95 - 1.87 (m, 1H), 1.51 - 1.40 (m, 3H), 0.97 (s, 6H). LCMS (ESI) calcd for $C_{39}H_{42}ClN_4O_4^+$ [M+H]$^+$: 665.29, found, 665.3.

**Example 72: Preparation of 3-(5-((3-(4'-chloro-5,5-dimethyl-3,4,5,6-tetrahydro-[1,1'-biphenyl]-2-carbonyl)-3,6-diazabicyclo[3.1.1]heptan-6-yl)methyl)-1-oxoisoindolin-2-yl)piperidine-2,6-dione (GT-05521)**

Referring to the method of Scheme 1, the target product (GT-05521) was prepared as white solid (10 mg, yield 12%). $^{1}$H NMR (400 MHz, DMSO-d6) δ 11.00 (s, 1H), 7.86 - 7.73 (m, 1H), 7.72 - 7.61 (m, 1H), 7.50 - 7.33 (m, 3H), 7.28 - 7.16 (m, 2H), 5.13 (dd, *J* = 13.3, 5.1 Hz, 1H), 4.62 - 4.58 (m, 1H), 4.51 - 4.43 (m, 1H), 4.37 - 4.19 (m, 2H), 4.17 - 4.10 (m, 1H), 3.80 -

3.63 (m, 1H), 3.50 - 3.39 (m, 1H), 3.25 - 3.13 (m, 2H), 2.95 - 2.89 (m, 1H), 2.61 (d, *J = 17.6* Hz, 2H), 2.49 - 2.33 (m, 3H), 2.26 - 2.17 (m, 1H), 2.12 - 1.96 (m, 2H), 1.93 - 1.89 (m, 1H), 1.59 - 1.33 (m, 2H), 0.99 (d, *J* = 6.6 Hz, 6H). LCMS (ESI) calcd for $C_{34}H_{38}ClN_4O_4^+$ [M+H]$^+$: 601.26, found, 601.3.

**Example 73: Preparation of 3-(5-((3-(4'-chloro-5,5-dimethyl-3,4,5,6-tetrahydro-[1,1'-biphenyl]-2-carbonyl)-3,8-diazabicyclo[3.2.1]octan-8-yl)methyl)-1-oxoisoindolin-2-yl)piperidine-2,6-dione (GT-05522)**

Referring to the method of Scheme 1, the target product (GT-05522) was prepared as white solid (12 mg, yield 15%). $^1$H NMR (400 MHz, DMSO-d6) δ 11.01 (s, 1H), 7.97 - 7.64 (m, 3H), 7.45 - 7.36 (m, 3H), 7.15 (d, *J* = 7.7 Hz, 1H), 5.14 (dd, *J* = 12.7, 4.7 Hz, 1H), 4.51 - 4.46 (m, 2H), 4.26 - 4.16 (m, 2H), 4.16 - 4.04 (m, 1H), 3.93 - 3.80 (m, 1H), 3.76 (brs, 1H), 3.69 - 3.52 (m, 1H), 3.27 - 3.18 (m, 2H), 3.00 - 2.85 (m, 1H), 2.75 - 2.68 (m, 1H), 2.61 (d, *J* = 17.4 Hz, 1H), 2.47 - 2.21 (m, 4H), 2.16 - 2.10 (m, 1H), 2.02 - 1.99 (m, 2H), 1.90 - 1.88 (m, 1H), 1.65 - 1.63 (m, 1H), 1.52 - 1.43 (m, 2H), 0.95 (s, 6H). LCMS (ESI) calcd for $C_{35}H_{40}ClN_40_4^+$ [M+H]$^+$: 615.27, found, 615.3.

**Example 74: Preparation of 3-(5-((8-(4'-chloro-5,5-dimethyl-3,4,5,6-tetrahydro-[1,1'-biphenyl]-2-carbonyl)-3,8-diazabicyclo[3.2.1]octan-3-yl)methyl)-1-oxoisoindolin-2-yl)piperidine-2,6-dione (GT-05523)**

Referring to the method of Scheme 1, the target product (GT-05523) was prepared as white solid (12 mg, yield 15%). $^1$H NMR (400 MHz, DMSO-d6) δ 11.00 (s, 1H), 7.90 - 7.86 (m, 1H), 7.79 - 7.69 (m, 2H), 7.41 (d, *J* = 7.7 Hz, 2H), 7.26 (d, *J* = 7.7 Hz, 2H), 5.20 - 5.04 (m, 1H), 4.62 - 4.13 (m, 4H), 3.91 (brs, 1H), 3.41 - 3.28 (m, 4H), 3.21 - 3.07 (m, 2H), 3.03 - 2.82 (m, 2H), 2.63 (t, *J* = 17.3 Hz, 2H), 2.47 - 2.21 (m, 4H), 2.07 - 1.94 (m, 2H), 1.91 - 1.76 (m, 2H), 1.56 - 1.37 (m, 2H), 0.97 (s, 6H). LCMS (ESI) calcd for $C_{35}H_{40}ClN_4O_4^+$ [M+H]$^+$: 615.27, found, 615.3.

**Example 75: Preparation of 3-(5-((5-(4'-chloro-5,5-dimethyl-3,4,5,6-tetrahydro-[1,1'-biphenyl]-2-carbonyl)-2,5-diazabicyclo[2.2.1]heptan-2-yl)methyl)-1-oxoisoindolin-2-yl)piperidine-2,6-dione (GT-05524)**

Referring to the method of Scheme 1, the target product (GT-05524) was prepared as white solid (10 mg, yield 12%). $^1$H NMR (400 MHz, DMSO-d6) δ 11.01 (s, 1H), 7.85 - 7.75 (m, 2H), 7.74 - 7.65 (m, 1H), 7.51 - 7.33 (m, 2H), 7.32 - 7.18 (m, 2H), 5.14 (dd, *J* = 13.1, 5.1 Hz, 1H), 4.66 - 4.09 (m, 6H), 3.73 - 3.60 (m, 1H), 3.27 - 3.22 (m, 2H), 3.15 - 3.13 (m, 1H), 3.05 - 2.84 (m, 2H), 2.61 (d, *J* = 16.1 Hz, 1H), 2.43 (dd, *J* = 13.1, 3.8 Hz, 1H), 2.39 - 2.10 (m, 4H), 2.03 - 1.99 (m, 2H), 1.53 - 1.34 (m, 2H), 0.97 (s, 6H). LCMS (ESI) calcd for $C_{34}H_{38}ClN_40_4^+$ [M+H]$^+$: 601.26, found, 601.3.

**Example 76: Preparation of 3-(5-(5-((4'-chloro-5,5-dimethyl-3,4,5,6-tetrahydro-[1,1'-biphenyl]-2-yl)methyl)-2,5-diazabicyclo[2.2.1]heptane-2-carbonyl)-1-oxoisoindolin-2-yl)piperidine-2,6-dione (GT-05541)**

Referring to the method of Scheme 6, the target compound (GT-05541) (51 mg, yield 60%) was prepared by undergoing an amide condensation reaction between the starting materials 2-(2,6-dioxopiperidin-3-yl)-1-oxoisoindoline-5-carboxylic acid and 2-((4'-chloro-5,5-dimethyl-3,4,5,6-tetrahydro-[1,1'-biphenyl]-2-yl)methyl)-2,5-diazabicyclo [2.2.1]heptane. (GT-05541) $^1$H NMR (400 MHz, DMSO-d6) δ 11.01 (s, 1H), 7.80 - 7.74 (m, 1H), 7.64 (d, *J* = 29.1 Hz, 1H), 7.57 - 7.52 (m, 1H), 7.42 - 7.35 (m, 1H), 7.34 - 7.15 (m, 3H), 5.14 (dt, *J* = 11.3, 5.6 Hz, 1H), 4.71 - 4.67 (m, 1H), 4.55 - 4.36 (m, 2H), 4.17 (t, *J* = 28.0 Hz, 1H), 3.47 - 3.41 (m, 1H), 3.27 - 3.00 (m, 2H), 3.00 - 2.85 (m, 2H), 2.61 (d, *J* = 16.9 Hz, 1H), 2.46 - 2.35 (m, 1H), 2.30 - 2.23 (m, 3H), 2.07 - 2.00 (m, 2H), 1.83 - 1.75 (m, 1H), 1.63 - 1.60 (m, 1H), 1.47 - 1.42 (m, 2H), 0.97 (s, 6H). LCMS (ESI) calcd for $C_{34}H_{36}ClN_4O_5^+$ [M+H]$^+$: 615.24, found, 615.3.

**Example 77: Preparation of 3-(5-(2-(4-((4'-chloro-[1,1'-biphenyl]-2-yl)methyl)piperazin-1-yl)ethyl)-1-oxoisoindolin-2-yl)piperidine-2,6-dione (GT-05876)**

Referring to the method of Scheme 1, the target product (GT-05876) was prepared as white solid (37 mg, yield 50%). $^1$H NMR (400 MHz, DMSO-d6) δ 10.99 (s, 1H), 7.69 (d, *J* = 7.8 Hz, 1H), 7.58 - 7.51 (m, 3H), 7.49 - 7.47(m, 2H), 7.45 - 7.38 (m, 3H), 7.35 - 7.26 (m, 1H), 5.11 (dd, *J* = 13.3, 5.1 Hz, 1H), 4.48 - 4.37 (m, 2H), 4.09 - 3.82 (m, 2H), 3.37 - 3.25 (m, 7H), 3.24 - 3.04 (m, 4H), 3.00 - 2.84 (m, 2H), 2.60 (d, *J* = 16.2 Hz, 1H), 2.48 - 2.32 (m, 1H), 2.11 - 1.91 (m, 1H). LCMS (ESI) calcd for $C_{32}H_{34}ClN_4O_3^+$ [M+H]$^+$: 557.23, found, 557.3.

**Example 78: Preparation of 3-(5-(3-(4-((4'-chloro-[1,1'-biphenyl]-2-yl)methyl)piperazin-1-yl)propyl)-1-oxoisoindolin-2-yl)piperidine-2,6-dione (GT-05893)**

Referring to the method of Scheme 1, the target product (GT-05893) was prepared as white solid (38 mg, yield 48%). $^1$H NMR (400 MHz, DMSO-d6) δ 11.01 (s, 1H), 7.79 (brs, 1H), 7.66 (d, *J* = 7.8 Hz, 1H), 7.52 (d, *J* = 8.4 Hz, 2H), 7.49 -

7.35 (m, 6H), 7.34 - 7.26 (m, 1H), 5.10 (dd, *J* = 13.3, 5.0 Hz, 1H), 4.45 - 4.27 (m, 3H), 3.45 - 3.30 (m, 4H), 3.18 - 3.06 (m, 4H), 2.98 - 2.85 (m, 2H), 2.77 - 2.73 (m, 3H), 2.60 (d, *J* = 17.0 Hz, 2H), 2.46 - 2.32 (m, 1H), 2.05 - 1.97 (m, 3H). LCMS (ESI) calcd for $C_{33}H_{36}ClN_4O_3^+$ [M+H]$^+$: 571.25, found, 571.3.

**Example 79: Preparation of 3-(4-((4-((4'-fluoro-3,4,5,6-tetrahydro-[1,1'-biphenyl]-2-yl)methyl)piperazin-1-yl)methyl)-1-oxoisoindolin-2-yl)piperidine-2,6-dione (GT-05861)**

Referring to the method of Scheme 1, the target product (GT-05861) was prepared as white solid (41 mg, yield 45%). $^1$H NMR (400 MHz, DMSO-d6) δ 11.03 (s, 1H), 7.75 (brs, 2H), 7.57 (brs, 1H), 7.29 - 7.03 (m, 4H), 5.15 (dd, *J* = 13.2, 4.9 Hz, 1H), 4.71 (brs, 1H), 4.44 - 4.40 (m, 1H), 3.39 - 3.33 (m, 4H), 3.30 - 3.10 (m, 4H), 3.03 - 2.85 (m, 3H), 2.73 (brs, 1H), 2.63 (d, *J* = 17.0 Hz, 2H), 2.43 - 2.15 (m, 5H), 2.08 - 1.94 (m, 1H), 1.68 (s, 4H). LCMS (ESI) calcd for $C_{31}H_{36}FN_4O_3^+$ [M+H]$^+$: 531.28, found, 531.3.

**Example 80: Preparation of 3-(5-((1-((4'-chloro-[1,1'-biphenyl]-2-yl)methyl)azetidin-3-yl)amino)-1-oxoisoindolin-2-yl)piperidine-2,6-dione (GT-05382)**

Referring to the method of Scheme 2, the target product (GT-05382) was prepared as white solid (12 mg, yield 19%). $^1$H NMR (400 MHz, DMSO-d6) δ 10.93 (s, 1H), 7.73 - 7.62 (m, 1H), 7.58 - 7.56(m, 1H), 7.53 - 7.47 (m, 3H), 7.46 - 7.43 (m, 2H), 7.41 - 4.37 (m, 1H), 7.37 - 7.27 (m, 1H), 6.60 (brs, 1H), 6.58 (brs, 1H), 5.02 (dd, *J* = 13.3, 5.1 Hz, 1H), 4.56 - 4.38 (m, 3H), 4.28 (d, *J* = 17.0 Hz, 2H), 4.16 (d, *J* = 16.9 Hz, 2H), 4.05 - 3.96 (m, 1H), 3.78 - 3.71 (m, 1H), 2.98 - 2.93 (m, 1H), 2.60 - 2.51 (m, 1H), 2.39 - 2.33 (m, 1H), 1.98 - 1.90 (m, 1H). LCMS (ESI) calcd for $C_{29}H_{28}ClN_4O_3^+$ [M+H]$^+$: 515.18, found, 515.2.

**Example 81: Preparation of 3-(4-((4-((4'-chloro-[1,1'-biphenyl]-2-yl)amino)piperidin-1-yl)methyl)-1-oxoisoindolin-2-yl)piperidine-2,6-dione (GT-04947)**

Referring to the method of Scheme 1, the target product (GT-04947) was prepared as white solid (39 mg, yield 49%). $^1$H NMR (400 MHz, DMSO) δ 11.07 (s, 1H), 7.88 (d, *J* = 8.4 Hz, 1H), 7.84 (d, *J* = 7.7 Hz, 1H), 7.65 (t, *J* = 7.5 Hz, 1H), 7.53 - 7.45 (m, 3H), 7.40 (d, *J* = 8.5 Hz, 1H), 7.22 - 7.17 (m, 1H), 7.01 - 6.93 (m, 1H), 6.81 (d, *J* = 8.0 Hz, 1H), 6.73 (t, *J* = 7.4 Hz, 1H), 5.19 (dd, *J* = 12.8, 4.7 Hz, 1H), 4.73 - 4.68 (m, 1H), 4.56 - 4.44 (m, 2H), 4.35 (d, *J* = 4.5 Hz, 2H), 3.23 - 3.04 (m, 2H), 3.04 - 2.88 (m, 2H), 2.65 (d, *J* = 15.8 Hz, 1H), 2.36 - 2.32 (m, 1H), 2.17 - 1.94 (m, 4H), 1.67 - 1.59 (m, 1H). LCMS (ESI) calcd for $C_{31}H_{32}ClN_4O_3^+$ [M+H]$^+$: 543.22, found, 543.3.

**Example 82: Preparation of 3-(5-((8-((4'-chloro-[1,1'-biphenyl]-2-yl)methyl)-3,8-diazabicyclo[3.2.1]octan-3-yl)methyl)-1-oxoisoindolin-2-yl)piperidine-2,6-dione (GT-05618)**

Referring to the method of Scheme 1, the target product (GT-05618) was prepared as white solid (54 mg, yield 62%). $^1$H NMR (400 MHz, DMSO-d6) δ 10.99 (s, 1H), 8.04 (brs, 1H), 7.71 - 7.68 (m, 1H), 7.64 - 7.49 (m, 4H), 7.46 - 7.41 (m, 3H), 7.37 - 7.31 (m, 1H), 5.10 (dd, *J* = 13.3, 5.2 Hz, 1H), 4.36 (dd, *J* = 51.8, 17.7 Hz, 2H), 4.19 (brs, 1H), 3.84 - 3.54 (m, 4H), 3.54 - 3.47 (m, 4H), 2.96 - 2.89 (m, 2H), 2.80 - 2.54 (m, 3H), 2.46 - 2.28 (m, 1H), 2.08 - 1.94 (m, 1H), 1.94 - 1.76 (m, 1H), 1.35 - 1.26 (m, 1H). LCMS (ESI) calcd for $C_{33}H_{34}ClN_4O_3^+$ [M+H]$^+$: 569.23, found, 569.3.

**Example 83: Preparation of 3-(5-((5-((4'-chloro-[1,1'-biphenyl]-2-yl)methyl)-2,5-diazabicyclo[2.2.2]octan-2-yl)methyl)-1-oxoisoindolin-2-yl)piperidine-2,6-dione (GT-05619)**

Referring to the method of Scheme 1, the target product (GT-05619) was prepared as white solid (40 mg, yield 46%). $^1$H NMR (400 MHz, DMSO-d6) δ 11.01 (s, 1H), 8.17 (brs, 1H), 7.90 (brs, 1H), 7.80 (d, *J* = 4.8 Hz, 2H), 7.64 - 7.36 (m, 6H), 7.29 (brs, 1H), 5.14 (dd, *J* = 12.9, 5.0 Hz, 1H), 4.70 - 4.22 (m, 5H), 4.01 - 3.55 (m, 3H), 3.78 - 3.56 (m, 2H), 3.32 - 3.14 (m, 3H), 3.00 - 2.87 (m, 1H), 2.68 - 2.57 (m, 1H), 2.49 - 2.46 (m, 1H), 2.33 - 2.09 (m, 1H), 2.07 - 1.95 (m, 1H), 1.90 - 1.43 (m, 2H). LCMS (ESI) calcd for $C_{33}H_{34}ClN_4O_3^+$ [M+H]$^+$: 569.23, found, 569.2.

**Example 84: Preparation of 3-(5-((4-(2-(5-chloropyridin-2-yl)benzyl)piperazin-1-yl)methyl)-1-oxoisoindolin-2-yl)piperidine-2,6-dione (GT-05860)**

Referring to the method of Scheme 1, the target product (GT-05860) was prepared as white solid (32 mg, yield 59%). $^1$H NMR (400 MHz, DMSO-d6) δ 11.01 (s, 1H), 8.90 (s, 1H), 8.15 (d, *J* = 7.5 Hz, 1H), 7.96 - 7.67 (m, 6H), 7.65 - 7.53 (m, 2H), 5.14 (dd, *J* = 13.2, 5.1 Hz, 1H), 4.69 - 4.06 (m, 7H), 3.30 - 3.01 (m, 5H), 2.98 - 2.83 (m, 2H), 2.61 (d, *J* = 16.7 Hz, 2H), 2.43 (dd, *J* = 13.0, 4.6 Hz, 1H), 2.03 - 1.99 (m, 1H). LCMS (ESI) calcd for $C_{30}H_{31}ClN_5O_3^+$ [M+H]$^+$: 544.21, found, 544.3.

**Example 85: Preparation of 3-(5-((4-((4'-chloro-[1,1'-biphenyl]-3-yl)methyl)piperazin-1-yl)methyl)-1-oxoisoindolin-2-yl)piperidine-2,6-dione (GT-05629)**

Referring to the method of Scheme 1, the target product (GT-05629) was prepared as white solid (33 mg, yield 37%). $^1$H NMR (400 MHz, DMSO-d6) δ 11.00 (s, 1H), 7.93 (s, 1H), 7.81 - 7.67 (m, 5H), 7.59 - 7.49 (m, 5H), 5.12 (dd, *J* = 13.4, 5.0 Hz, 1H), 4.48 - 4.31 (m, 4H), 3.90 - 3.62 (m, 2H), 3.57 - 3.41 (m, 4H), 3.16 - 3.09 (m, 2H), 3.00 - 2.81 (m, 2H), 2.67 - 2.51 (m, 2H), 2.45 - 2.33 (m, 1H), 2.09 - 1.88 (m, 1H). LCMS (ESI) calcd for $C_{31}H_{32}ClN_4O_3^+$ [M+H]$^+$: 543.22, found, 543.3.

**Example 86: Preparation of 3-(5-((4-(3-(5-chloropyridin-2-yl)benzyl)piperazin-1-yl)methyl)-1-oxoisoindolin-2-yl) piperidine-2,6-dione (GT-05596)**

Referring to the method of Scheme 1, the target product (GT-05596) was prepared as white solid (9 mg, yield 10%). $^1$H NMR (400 MHz, DMSO-d6) δ 11.00 (s, 1H), 8.73 (t, *J* = 1.6 Hz, 1H), 8.33 (s, 1H), 8.15 (d, *J* = 7.2 Hz, 1H), 8.08 (d, *J*= 1.5 Hz, 2H), 7.79 (d, *J* = 7.9 Hz, 2H), 7.74 - 7.64 (m, 2H), 7.61 - 7.57 (m, 1H), 5.21 - 5.11 (m, 1H), 4.69 - 4.17 (m, 8H), 3.38 - 3.19 (m, 5H), 2.95 - 2.89 (m, 2H), 2.60 (d, *J* = 17.1 Hz, 1H), 2.46 - 2.38 (m, 1H), 2.05 - 1.95 (m, 1H). LCMS (ESI) calcd for $C_{30}H_{31}ClN_5O_3^+$ [M+H]$^+$: 544.21, found, 544.2.

**Example 87: Preparation of 3-(1-oxo-5-((4-(3-(thiophen-2-yl)benzyl)piperazin-1-yl)methyl)isoindolin-2-yl)piperidine-2,6-dione (GT-05597)**

Referring to the method of Scheme 1, the target product (GT-05597) was prepared as white solid (52 mg, yield 56%). $^1$H NMR (400 MHz, DMSO-d6) δ 11.00 (s, 1H), 7.97 - 7.90 (m, 1H), 7.85 - 7.75 (m, 2H), 7.74 - 7.64 (m, 2H), 7.59 (d, *J* = 5.1 Hz, 1H), 7.56 (d, *J* = 3.5 Hz, 1H), 7.48 (d, *J* = 4.0 Hz, 2H), 7.17 (dd, *J* = 5.0, 3.7 Hz, 1H), 5.13 (dd, *J* = 13.3, 5.1 Hz, 1H), 4.50 - 4.33 (m, 5H), 3.62 - 3.54 (m, 4H), 3.34 - 3.24 (m, 4H), 3.12 - 3.05 (m, 1H), 2.99 - 2.86 (m, 1H), 2.63 - 2.58 (m, 1H), 2.46 - 2.37 (m, 1H), 2.03 - 1.98 (m, 1H). LCMS (ESI) calcd for $C_{29}H_{31}N_4O_3S^+$ [M+H]$^+$: 515.21, found, 515.2.

**Example 88: Preparation of 3-(5-((4-(3-(furan-2-yl)benzyl)piperazin-1-yl)methyl)-1-oxoisoindolin-2-yl)piperidine-2,6-dione (GT-05692)**

Referring to the method of Scheme 1, the target product (GT-05692) was prepared as white solid (34 mg, yield 36%). $^1$H NMR (400 MHz, DMSO-d6) δ 11.00 (s, 1H), 7.93 (t, *J* = 11.8 Hz, 1H), 7.83 - 7.71 (m, 4H), 7.71 - 7.58 (m, 1H), 7.51 - 7.44 (m, 2H), 6.98 (d, *J* = 3.3 Hz, 1H), 6.63 (dd, *J* = 3.3, 1.7 Hz, 1H), 5.13 (dd, *J* = 13.3, 4.7 Hz, 1H), 4.59 - 4.16 (m, 7H), 3.31 - 3.20 (m, 4H), 2.95 - 2.89 (m, 2H), 2.67 - 2.57 (m, 3H), 2.41 (dd, *J* = 12.5, 4.2 Hz, 1H), 2.04 - 1.99 (m, 1H). LCMS (ESI) calcd for $C_{29}H_{31}N_4O_4^+$ [M+H]$^+$: 499.23, found, 499.2.

**Example 89: Preparation of 3-(5-((4-(3-(1H-pyrrol-2-yl)benzyl)piperazin-1-yl)methyl)-1-oxoisoindolin-2-yl)piperidine-2,6-dione (GT-05733)**

Referring to the method of Scheme 1, the target product (GT-05733) was prepared as white solid (31 mg, yield 32%). $^1$H NMR (400 MHz, DMSO-d6) δ 11.34 (s, 1H), 11.00 (s, 1H), 7.92 - 7.76 (m, 3H), 7.66 (d, *J* = 7.2 Hz, 2H), 7.41 (t, *J* = 7.7 Hz, 1H), 7.32 - 7.29 (m, 1H), 6.88 (d, *J* = 1.3 Hz, 1H), 6.54 (s, 1H), 6.13 (dd, *J* = 5.6, 2.5 Hz, 1H), 5.13 (dd, *J* = 13.3, 5.0 Hz, 1H), 4.61 - 4.16 (m, 7H), 3.22 - 3.06 (m, 5H), 3.00 - 2.86 (m, 3H), 2.60 (d, *J* = 17.4 Hz, 3H), 2.41 (dd, *J* = 13.2, 4.7 Hz, 1H), 2.05 - 1.93 (m, 1H). LCMS (ESI) calcd for $C_{29}H_{32}N_5O_3^+$ [M+H]$^+$: 498.25, found, 498.3.

**Example 90: Preparation of 3-(5-((4-((4'-chloro-[1,1'-biphenyl]-4-yl)methyl)piperazin-1-yl)methyl)-1-oxoisoindolin-2-yl)piperidine-2,6-dione (GT-05598)**

Referring to the method of Scheme 1, the target product (GT-05598) was prepared as white solid (45 mg, yield 50%). $^1$H NMR (400 MHz, DMSO-d6) δ 11.00 (s, 1H), 7.80 - 7.70 (m, 9H), 7.58 - 7.48 (m, 2H), 5.13 (dd, *J* = 13.3, 5.0 Hz, 1H), 4.50 - 4.33 (m, 5H), 3.76- 3.59 (m, 4H), 3.37 - 3.13 (m, 5H), 2.99 - 2.85 (m, 1H), 2.61 (d, *J* = 16.7 Hz, 1H), 2.42 (dd, *J* = 13.2, 4.5 Hz, 1H), 2.07 - 1.91 (m, 1H). LCMS (ESI) calcd for $C_{31}H_{32}ClN_4O_3^+$ [M+H]$^+$: 543.22, found, 543.2.

**Example 91: Preparation of 3-(5-((4-(4-(5-chloropyridin-2-yl)benzyl)piperazin-1-yl)methyl)-1-oxoisoindolin-2-yl) piperidine-2,6-dione (GT-05872)**

Referring to the method of Scheme 1, the target product (GT-05872) was prepared as white solid (39 mg, yield 44%). $^1$H NMR (400 MHz, DMSO-d6) δ 11.00 (s, 1H), 8.73 (dd, *J* = 2.3, 0.8 Hz, 1H), 8.15 (d, *J* = 8.2 Hz, 2H), 8.10 - 8.01 (m, 2H), 7.79 (d, *J* = 7.8 Hz, 2H), 7.70 (d, *J* = 5.7 Hz, 3H), 5.13 (dd, *J* = 13.3, 5.1 Hz, 1H), 4.58 - 4.20 (m, 6H), 3.41 - 3.32 (m, 4H),

3.33 - 3.08 (m, 4H), 2.98 - 2.86 (m, 1H), 2.61 (d, *J* = 16.7 Hz, 1H), 2.42 (dd, *J* = 13.1, 4.4 Hz, 1H), 2.06 - 1.95 (m, 1H). LCMS (ESI) calcd for $C_{30}H_{31}ClN_5O_3^+$ [M+H]$^+$: 544.21, found, 544.2.

**Example 92: Preparation of 3-(1-oxo-5-((4-(4-(thiophen-2-yl)benzyl)piperazin-1-yl)methyl)isoindolin-2-yl)piperidine-2,6-dione (GT-05640)**

Referring to the method of Scheme 1, the target product (GT-05640) was prepared as white solid (9 mg, yield 10%). $^1$H NMR (400 MHz, DMSO-d6) δ 11.00 (s, 1H), 7.81 - 7.66 (m, 4H), 7.65 - 7.43 (m, 5H), 7.16 (dd, *J* = 5.0, 3.7 Hz, 1H), 5.13 (dd, *J* = 13.4, 4.8 Hz, 1H), 4.49 - 4.43 (m, 5H), 3.42 - 3.35 (m, 4H), 3.24 - 3.04 (m, 4H), 2.94 - 2.88 (m, 1H), 2.69 - 2.57 (m, 2H), 2.41 (dd, *J* = 15.2, 2.4 Hz, 1H), 2.08 - 1.94 (m, 1H). LCMS (ESI) calcd for $C_{29}H_{31}N_4O_3S^+$ [M+H]$^+$: 515.21, found, 515.2.

**Example 93: Preparation of 3-(5-((4-(4-(furan-2-yl)benzyl)piperazin-1-yl)methyl)-1-oxoisoindolin-2-yl)piperidine-2,6-dione (GT-05693)**

Referring to the method of Scheme 1, the target product (GT-05693) was prepared as white solid (58 mg, yield 61%). $^1$H NMR (400 MHz, DMSO-d6) δ 11.00 (s, 1H), 7.79 - 7.75 (m, 5H), 7.72 - 7.66 (m, 1H), 7.63 - 7.61 (m, 2H), 7.04 (d, *J* = 3.3 Hz, 1H), 6.62 (dd, *J* = 3.4, 1.8 Hz, 1H), 5.13 (dd, *J* = 13.3, 5.0 Hz, 1H), 4.60 - 4.07 (m, 7H), 3.29 - 3.14 (m, 4H), 3.07 - 2.95 (m, 1H), 2.99 - 2.84 (m, 2H), 2.68 - 2.56 (m, 2H), 2.44 - 2.40 (m, 1H), 2.04 - 1.99 (m, 1H). LCMS (ESI) calcd for $C_{29}H_{31}N_4O_4^+$ [M+H]$^+$: 499.23, found, 499.3.

**Example 94: Preparation of 3-(5-((4-(4-(1H-pyrrol-2-yl)benzyl)piperazin-1-yl)methyl)-1-oxoisoindolin-2-yl)piperidine-2,6-dione (GT-05734)**

Referring to the method of Scheme 1, the target product (GT-05734) was prepared as white solid (32 mg, yield 33%). $^1$H NMR (400 MHz, DMSO-d6) δ 11.39 (s, 1H), 11.00 (s, 1H), 7.83 - 7.73 (m, 2H), 7.69 - 7.64 (m, 3H), 7.53 - 7.51 (m, 2H), 6.88 (s, 1H), 6.59 (s, 1H), 6.13 (dd, *J* = 5.6, 2.5 Hz, 1H), 5.13 (dd, *J* = 13.3, 5.1 Hz, 1H), 4.64 - 4.04 (m, 7H), 3.25 - 3.06 (m, 5H), 2.99 - 2.86 (m, 2H), 2.60 (d, *J* = 16.8 Hz, 2H), 2.45 - 2.34 (m, 1H), 2.06 - 1.90 (m, 1H). LCMS (ESI) calcd for $C_{29}H_{32}N_5O_3^+$ [M+H]$^+$: 498.25, found, 498.2.

**Example 95: Preparation of 3-(5-((4-((6-(4-chlorophenyl)pyridin-3-yl)methyl)piperazin-1-yl)methyl)-1-oxoisoindolin-2-yl)piperidine-2,6-dione (GT-05632)**

Referring to the method of Scheme 1, the target product (GT-05632) was prepared as white solid (49 mg, yield 55%). $^1$H NMR (400 MHz, DMSO-d6) δ 11.00 (s, 1H), 8.81 (s, 1H), 8.15 (d, *J* = 8.6 Hz, 2H), 8.10 (d, *J* = 7.7 Hz, 2H), 7.80 (d, *J* = 7.8 Hz, 2H), 7.72 - 7.69(m, 1H), 7.58 (d, *J* = 8.7 Hz, 2H), 5.13 (dd, *J* = 13.2, 5.0 Hz, 1H), 4.51 - 4.34 (m, 6H), 3.49 - 3.42 (m, 4H), 3.39 - 3.32 (m, 4H), 2.99 - 2.84 (m, 2H), 2.66 - 2.54 (m, 1H), 2.44 - 2.40 (m, 1H), 2.07 - 1.89 (m, 1H). LCMS (ESI) calcd for $C_{30}H_{31}ClN_5O_3^+$ [M+H]$^+$: 544.21, found, 544.2.

**Example 96: Preparation of 3-(5-((4-([2,4'-bipyridin]-5-ylmethyl)piperazin-1-yl)methyl)-1-oxoisoindolin-2-yl)piperidine-2,6-dione (GT-05633)**

Referring to the method of Scheme 1, the target product (GT-05633) was prepared as white solid (32 mg, yield 34%). $^1$H NMR (400 MHz, DMSO-d6) δ 11.01 (s, 1H), 9.09 (s, 1H), 9.04 (d, *J* = 6.6 Hz, 2H), 8.71 (d, *J* = 6.7 Hz, 2H), 8.51 (d, *J* = 8.2 Hz, 1H), 8.42 (d, *J* = 8.3 Hz, 1H), 7.94 (s, 1H), 7.81 (q, *J* = 8.1 Hz, 2H), 5.14 (dd, *J* = 13.2, 5.1 Hz, 1H), 4.71 - 4.33 (m, 10H), 3.94 - 3.83 (m, 4H), 2.99 - 2.87 (m, 1H), 2.61 (d, *J* = 16.9 Hz, 1H), 2.43 (dd, *J* = 13.2, 4.6 Hz, 1H), 2.03 - 2.00 (m, 1H). LCMS (ESI) calcd for $C_{29}H_{31}N_6O_3^+$ [M+H]$^+$: 511.25, found, 511.2.

**Example 97: Preparation of 3-(1-oxo-5-((4-((5-phenylpyrazin-2-yl)methyl)piperazin-1-yl)methyl)isoindolin-2-yl) piperidine-2,6-dione (GT-05544)**

Referring to the method of Scheme 1, the target product (GT-05544) was prepared as white solid (32 mg, yield 34%). $^1$H NMR (400 MHz, DMSO-d6) δ 11.01 (s, 1H), 9.30 (s, 1H), 8.90 (s, 1H), 8.18 (dd, *J* = 7.9, 1.6 Hz, 2H), 7.90 - 7.70 (m, 3H), 7.64 - 7.51 (m, 3H), 5.14 (dd, *J* = 13.3, 5.1 Hz, 1H), 4.60 - 4.29 (m, 6H), 3.45 - 3.41 (m, 4H), 3.33 - 3.23 (m, 4H), 2.99 - 2.85 (m, 1H), 2.61 (d, *J* = 16.6 Hz, 1H), 2.45 - 2.40 (m, 1H), 2.06 - 1.93 (m, 1H). LCMS (ESI) calcd for $C_{29}H_{31}N_6O_3^+$ [M+H]$^+$: 511.25, found, 511.2.

**Example 98: Preparation of 3-(5-((4-((5-(4-chlorophenyl)thiophen-2-yl)methyl)piperazin-1-yl)methyl)-1-oxoisoindolin-2-yl)piperidine-2,6-dione (GT-05625)**

Referring to the method of Scheme 1, the target product (GT-05625) was prepared as white solid (24 mg, yield 27%). $^1$H NMR (400 MHz, DMSO-d6) δ 11.00 (s, 1H), 7.80 (d, *J* = 7.7 Hz, 2H), 7.70 - 7.62 (m, 3H), 7.49 (d, *J* = 8.6 Hz, 3H), 7.24 (brs, 1H), 5.13 (dd, *J* = 13.2, 5.2 Hz, 1H), 4.60 - 4.17 (m, 6H), 3.35 - 3.26 (m, 4H), 3.17 - 3.00 (m, 4H), 2.91 - 2.88 (m, 2H), 2.61 (d, *J* = 16.7 Hz, 1H), 2.44 - 2.39 (m, 1H), 2.04 - 1.95 (m, 1H). LCMS (ESI) calcd for $C_{29}H_{30}ClN_4O_3S^+$ [M+H]$^+$: 549.17, found, 549.2.

**Example 99: Preparation of 3-(5-((4-([2,2'-bithiophen]-5-ylmethyl)piperazin-1-yl)methyl)-1-oxoisoindolin-2-yl) piperidine-2,6-dione (GT-05624)**

Referring to the method of Scheme 1, the target product (GT-05624) was prepared as white solid (29 mg, yield 31%). $^1$H NMR (400 MHz, DMSO-d6) δ 11.00 (s, 1H), 7.80 (d, *J* = 7.7 Hz, 2H), 7.70 (d, *J* = 6.9 Hz, 1H), 7.54 (d, *J* = 4.5 Hz, 1H), 7.31 (d, *J* = 3.0 Hz, 1H), 7.25 (brs, 1H), 7.21 (brs, 1H), 7.10 (dd, *J* = 5.1, 3.6 Hz, 1H), 5.13 (dd, *J* = 13.2, 5.1 Hz, 1H), 4.51 - 4.34 (m, 6H), 3.40 - 3.32 (m, 4H), 3.24 - 2.96 (m, 4H), 2.96 - 2.86 (m, 1H), 2.61 (d, *J* = 16.4 Hz, 1H), 2.42 (dd, *J* = 13.0, 4.4 Hz, 1H), 2.05 - 1.94 (m, 1H). LCMS (ESI) calcd for $C_{27}H_{29}N_4O_3S_2^+$ [M+H]$^+$: 521.17, found, 521.2.

**Example 100: Preparation of 3-(5-((4-((5-(4-chlorophenyl)furan-2-yl)methyl)piperazin-1-yl)methyl)-1-oxoisoindolin-2-yl)piperidine-2,6-dione (GT-05694)**

Referring to the method of Scheme 1, the target product (GT-05694) was prepared as white solid (34 mg, yield 37%). $^1$H NMR (400 MHz, DMSO-d6) δ 11.00 (s, 1H), 7.84 - 7.66 (m, 5H), 7.51 (d, *J* = 8.6 Hz, 2H), 7.05 (d, *J* = 3.2 Hz, 1H), 6.75 (s, 1H), 5.13 (dd, *J* = 13.1, 5.1 Hz, 1H), 4.64 - 4.21 (m, 7H), 3.27 - 3.14 (m, 5H), 2.99 - 2.83 (m, 2H), 2.63 (t, *J* = 18.5 Hz, 2H), 2.42 (dd, *J* = 13.2, 4.5 Hz, 1H), 2.02 - 1.91 (m, 1H). LCMS (ESI) calcd for $C_{29}H_{30}ClN_4O_4^+$ [M+H]$^+$: 533.20, found, 533.3.

**Example 101: Preparation of 3-(1-oxo-5-((4-((5-(thiophen-2-yl)furan-2-yl)methyl)piperazin-1-yl)methyl)isoindolin-2-yl)piperidine-2,6-dione (GT-05627)**

Referring to the method of Scheme 1, the target product (GT-05627) was prepared as white solid (27 mg, yield 28%). $^1$H NMR (400 MHz, DMSO-d6) δ 11.00 (s, 1H), 7.87 - 7.76 (m, 2H), 7.71 (d, *J* = 7.1 Hz, 1H), 7.57 (d, *J* = 5.0 Hz, 1H), 7.42 (d, *J* = 3.2 Hz, 1H), 7.13 (dd, *J* = 4.9, 3.7 Hz, 1H), 6.79 (d, *J* = 3.2 Hz, 1H), 6.74 (s, 1H), 5.13 (dd, *J* = 13.3, 5.1 Hz, 1H), 4.61 - 4.17 (m, 6H), 3.39 - 3.28 (m, 4H), 3.24 - 3.02 (m, 4H), 2.97 - 2.87 (m, 1H), 2.60 (d, *J* = 16.9 Hz, 1H), 2.46 - 2.32 (m, 1H), 2.07 - 1.95 (m, 1H). LCMS (ESI) calcd for $C_{27}H_{29}N_4O_4S^+$ [M+H]$^+$: 505.19, found, 505.2.

**Example 102: Preparation of 3-(5-((4-([2,2'-bifuran]-5-ylmethyl)piperazin-1-yl)methyl)-1-oxoisoindolin-2-yl)piperidine-2,6-dione (GT-05695)**

Referring to the method of Scheme 1, the target product (GT-05695) was prepared as white solid (37 mg, yield 38%). $^1$H NMR (400 MHz, DMSO-d6) δ 11.00 (s, 1H), 7.85 - 7.66 (m, 4H), 6.73 (brs, 3H), 6.64 - 6.59 (m, 1H), 5.13 (dd, *J* = 13.2, 5.1 Hz, 1H), 4.61 - 4.17 (m, 7H), 3.29 - 3.01 (m, 5H), 2.99 - 2.86 (m, 2H), 2.63 (t, *J* = 16.7 Hz, 2H), 2.42 (dd, *J* = 13.1, 4.5 Hz, 1H), 2.09 - 1.88 (m, 1H). LCMS (ESI) calcd for $C_{27}H_{29}N_4O_5^+$ [M+H]$^+$: 489.21, found, 489.2.

**Example 103: Preparation of 3-(5-((1-(4'-chloro-[1,1'-biphenyl]-2-carbonyl)azetidin-3-yl)amino)-1-oxoisoindolin-2-yl)piperidine-2,6-dione (GT-04397)**

Referring to the method of Scheme 6, the target product (GT-04397) was prepared as white solid (24 mg, yield 36%). $^1$H NMR (400 MHz, MeOD) δ 7.49 - 7.33 (m, 9H), 6.51 - 6.44 (m, 1H), 6.39 (s, 1H), 4.97 (dd, *J* = 13.3, 5.2 Hz, 1H), 4.30 - 4.19 (m, 3H), 4.06 - 4.01 (m, 1H), 3.94 - 3.85 (m, 1H), 3.69 (dd, *J* = 10.8, 4.7 Hz, 1H), 3.33 - 3.26 (m, 1H), 2.86 - 2.73 (m, 1H), 2.69 - 2.63 (m, 1H), 2.31 - 2.21 (m, 1H), 2.07 -1.97 (m, 1H). LCMS (ESI) calcd for $C_{29}H_{26}ClN_4O_4^+$ [M+H]$^+$: 529.16, found, 529.2.

**Example 104: Preparation of 3-(5-(4-((4-(4'-chloro-[1,1'-biphenyl]-2-carbonyl)piperazin-1-yl)methyl)phenyl)-1-oxoisoindolin-2-yl)piperidine-2,6-dione (GT-05874)**

Referring to the method of Scheme 6, the target product (GT-05874) was prepared as white solid (16 mg, yield 27%). $^1$H NMR (400 MHz, DMSO-d6) δ 11.01 (s, 1H), 7.93 (s, 1H), 7.83 (brs, 4H), 7.72 - 7.36 (m, 10H), 5.15 (dd, *J* = 13.2,

5.1 Hz, 1H), 4.55 - 4.38 (m, 4H), 4.32 (brs, 1H), 3.30 - 3.23 (m, 4H), 3.07 (brs, 2H), 3.02 - 2.86 (m, 2H), 2.62 (d, *J* = 16.4 Hz, 2H), 2.44 (dd, *J* = 13.3, 4.4 Hz, 1H), 2.04 - 1.97 (m, 1H). LCMS (ESI) calcd for $C_{37}H_{34}ClN_4O_4^+$ $[M+H]^+$: 633.23, found, 633.3.

**Example 105: Preparation of 3-(5-((7-(4'-chloro-[1,1'-biphenyl]-2-carbonyl)-2,7-diazaspiro[3.5]nonan-2-yl) methyl)-1-oxoisoindolin-2-yl)piperidine-2,6-dione (GT-05620)**

Referring to the method of Scheme 1, the target product (GT-05620) was prepared as white solid (52 mg, yield 62%). $^1H$ NMR (400 MHz, DMSO-d6) δ 11.43 - 11.20 (m, 1H), 11.00 (s, 1H), 7.78 (d, *J* = 7.7 Hz, 2H), 7.67 (d, *J* = 7.4 Hz, 1H), 7.53 - 7.39 (m, 7H), 7.36 - 7.26 (m, 1H), 5.13 (dd, *J* = 13.3, 5.3 Hz, 1H), 4.50 - 4.32 (m, 4H), 3.88 - 3.74 (m, 4H), 3.66 - 3.54 (m, 2H), 3.06 - 2.86 (m, 2H), 2.85 - 2.70 (m, 1H), 2.61 (d, *J* = 17.4 Hz, 1H), 2.46 - 2.34 (m, 1H), 2.08 - 1.93 (m, 1H), 1.94 - 1.71 (m, 1H), 1.70 - 1.43 (m, 2H), 1.06 - 0.94 (m, 1H). LCMS (ESI) calcd for $C_{34}H_{34}ClN_4O_4^+$ $[M+H]^+$: 597.23, found, 597.3.

**Example 106: Preparation of 3-(5-(3-(4'-chloro-[1,1'-biphenyl]-2-carbonyl)-3,8-diazabicyclo[3.2.1]octan-8-yl)-1-oxoisoindolin-2-yl)piperidine-2,6-dione (GT-05875)**

Referring to the method of Scheme 6, the target product (GT-05875) was prepared as white solid (34 mg, yield 52%). $^1H$ NMR (400 MHz, DMSO-d6) δ 10.93 (s, 1H), 7.58 (s, 2H), 7.54 - 7.41 (m, 4H), 7.36 - 7.33 (m, 1H), 7.28 (d, *J* = 8.4 Hz, 1H), 7.24 (dd, *J* = 7.6, 2.3 Hz, 1H), 6.98 - 6.89 (m, 1H), 6.87 - 6.81 (m, 1H), 5.10 - 4.94 (m, 1H), 4.45 - 4.41 (m, 1H), 4.35 - 4.23 (m, 1H), 4.23 - 4.08 (m, 2H), 3.92 (d, *J* = 13.2 Hz, 1H), 3.12 (dd, *J* = 50.6, 12.2 Hz, 2H), 2.99 - 2.84 (m, 1H), 2.71 - 2.54 (m, 3H), 2.40 - 2.34 (m, 1H), 2.05 - 1.83 (m, 2H), 1.83 - 1.68 (m, 1H), 1.65 - 1.42 (m, 1H), 1.17 - 1.12 (m, 1H). LCMS (ESI) calcd for $C_{32}H_{30}ClN_4O_4^+$ $[M+H]^+$: 569.20, found, 569.2.

**Example 107: Preparation of 3-(5-((8-(4'-chloro-[1,1'-biphenyl]-2-carbonyl)-3,8-diazabicyclo[3.2.1]octan-3-yl) methyl)-1-oxoisoindolin-2-yl)piperidine-2,6-dione (GT-05542)**

Referring to the method of Scheme 1, the target product (GT-05542) was prepared as white solid (60 mg, yield 70%). $^1H$ NMR (400 MHz, DMSO-d6) δ 11.00 (s, 1H), 7.95 - 7.91 (m, 1H), 7.82 - 7.69 (m, 2H), 7.59 - 7.48 (m, 6H), 7.41 - 7.36 (m, 2H), 5.12 (dd, *J* = 12.9, 3.5 Hz, 1H), 4.69 - 4.59 (m, 1H), 4.55 - 4.21 (m, 3H), 3.67 - 3.61 (m, 1H), 3.56 - 3.41(m, 4H), 3.24 - 3.09 (m, 1H), 3.13 - 2.99 (m, 1H), 2.96 - 2.92 (m, 2H), 2.68 - 2.56 (m, 1H), 2.44 - 2.35 (m, 1H), 2.07 - 2.00 (m, 2H), 1.89 - 1.78 (m, 1H), 1.56 - 1.39 (m, 1H). LCMS (ESI) calcd for $C_{33}H_{32}ClN_4O_4^+$ $[M+H]^+$: 583.21, found, 583.2.

**Example 108: Preparation of 3-(5-((5-(4'-chloro-[1,1'-biphenyl]-2-carbonyl)-2,5-diazabicyclo[2.2.2]octan-2-yl) methyl)-1-oxoisoindolin-2-yl)piperidine-2,6-dione (GT-05543)**

Referring to the method of Scheme 1, the target product (GT-05543) was prepared as white solid (51 mg, yield 60%). $^1H$ NMR (400 MHz, DMSO-d6) δ 11.01 (s, 1H), 8.02 - 7.88 (m, 1H), 7.85 - 7.76 (m, 1H), 7.68 - 7.34 (m, 9H), 5.14 (dd, *J* = 12.9, 4.7 Hz, 1H), 4.71 - 4.42 (m, 3H), 4.42 - 4.28 (m, 1H), 4.25 - 4.16 (m, 1H), 3.97 - 3.56 (m, 2H), 3.45 - 3.41 (m, 2H), 3.27 - 3.08 (m, 1H), 2.96 - 2.89 (m, 2H), 2.61 (d, *J* = 16.4 Hz, 1H), 2.47 - 2.36 (m, 1H), 2.27 - 2.18 (m, 1H), 2.10 - 1.94 (m, 1H), 1.80 - 1.67 (m, 1H), 1.58 - 1.48 (m, 1H). LCMS (ESI) calcd for $C_{33}H_{32}ClN_4O_4^+$ $[M+H]^+$: 583.21, found, 583.2.

**Example 109: Preparation of 3-(5-(6-((4'-chloro-[1,1'-biphenyl]-2-yl)methyl)-2,6-diazaspiro[3.3]heptane-2-carbonyl)-1-oxoisoindolin-2-yl)piperidine-2,6-dione (GT-05637)**

Referring to the methods of Scheme 6 and Example 76, the target product (GT-05637) was prepared as white solid (21 mg, yield 29%). $^1H$ NMR (400 MHz, DMSO-d6) δ 11.01 (s, 1H), 7.85 - 7.79 (m, 2H), 7.74 - 7.63 (m, 1H), 7.64 - 7.45 (m, 5H), 7.45 - 7.36 (m, 2H), 7.36 - 7.29 (m, 1H), 5.13 (dd, *J* = 13.3, 5.2 Hz, 1H), 4.52 - 4.35 (m, 4H), 4.24 (brs, 3H), 4.04 (brs, 3H), 3.64 - 3.47 (m, 2H), 3.00 - 2.82 (m, 1H), 2.63 (t, *J* = 16.6 Hz, 2H), 2.46 - 2.28 (m, 1H), 2.13 - 1.93 (m, 1H). LCMS (ESI) calcd for $C_{32}H_{30}ClN_4O_4^+$ $[M+H]^+$: 569.20, found, 569.3.

**Example 110: Preparation of 3-(5-(3-((4'-chloro-[1,1'-biphenyl]-2-yl)methyl)-3,8-diazabicyclo[3.2.1]octane-8-carbonyl)-1-oxoisoindolin-2-yl)piperidine-2,6-dione (GT-05638)**

Referring to the methods of Scheme 6 and Example 76, the target product (GT-05638) was prepared as white solid (42 mg, yield 47%). $^1H$ NMR (400 MHz, DMSO-d6) δ 11.02 (s, 1H), 7.80 (d, *J* = 7.7 Hz, 1H), 7.56 (d, *J* = 7.8 Hz, 3H), 7.53 - 7.35 (m, 7H), 5.15 (dd, *J* = 13.2, 5.1 Hz, 1H), 4.67 (brs, 1H), 4.54 - 4.29 (m, 4H), 4.02 (brs, 1H), 3.87 (brs, 1H), 3.03 - 2.86 (m, 2H), 2.64 (t, *J* = 21.1 Hz, 2H), 2.43 (dd, *J* = 13.1, 4.2 Hz, 1H), 2.33 - 2.15 (m, 2H), 2.08 - 1.98 (m, 1H), 1.97 - 1.71 (m, 3H) LCMS (ESI) calcd for $C_{33}H_{32}ClN_4O_4^+$ $[M+H]^+$: 583.21, found, 583.3.

**Example 111: Preparation of 3-(5-(8-((4'-chloro-[1,1'-biphenyl]-2-yl)methyl)-3,8-diazabicyclo[3.2.1]octane-3-carbonyl)-1-oxoisoindolin-2-yl)piperidine-2,6-dione (GT-05621)**

Referring to the methods of Scheme 6 and Example 76, the target product (GT-05621) was prepared as white solid (31 mg, yield 35%). $^1$H NMR (400 MHz, DMSO-d6) δ 11.00 (s, 1H), 10.66 (s, 1H), 8.06 (s, 1H), 7.78 (d, *J* = 7.7 Hz, 1H), 7.65 (s, 1H), 7.55 - 7.50 (m, 5H), 7.42 (d, *J* = 8.2 Hz, 2H), 7.36 - 7.35 (m, 1H), 5.13 (dd, *J* = 13.3, 5.0 Hz, 1H), 4.50 - 4.33 (m, 3H), 4.22 (brs, 2H), 3.96 - 3.73 (m, 2H), 3.64 - 3.60 (m, 1H), 3.53 - 3.45 (m, 2H), 2.98 - 2.82 (m, 1H), 2.60 (d, *J* = 18.0 Hz, 1H), 2.46 - 2.31 (m, 1H), 2.04 - 1.99 (m, 1H), 1.68 - 1.58 (m, 1H), 1.51 - 1.42 (m, 1H), 1.24 - 1.17 (m, 2H). LCMS (ESI) calcd for $C_{33}H_{32}ClN_4O_4^+$ [M+H]$^+$: 583.21, found, 583.2.

**Example 112: Preparation of 3-(5-(5-((4'-chloro-[1,1'-biphenyl]-2-yl)methyl)-2,5-diazabicyclo[2.2.2]octane-2-carbonyl)-1-oxoisoindolin-2-yl)piperidine-2,6-dione (GT-05622)**

Referring to the methods of Scheme 6 and Example 76, the target product (GT-05622) was prepared as white solid (27 mg, yield 30%). $^1$H NMR (400 MHz, DMSO-d6) δ 11.01 (s, 1H), 10.39 (s, 1H), 8.00 - 7.93 (m, 1H), 7.85 - 7.73 (m, 1H), 7.68 - 7.27 (m, 9H), 5.14 (dd, *J* = 13.0, 4.5 Hz, 1H), 4.60 - 4.27 (m, 4H), 3.75 (brs, 1H), 3.60 - 3.37 (m, 3H), 3.18 - 3.14 (m, 1H), 3.07 - 2.83 (m, 2H), 2.61 (d, *J* = 16.4 Hz, 1H), 2.41 - 2.33 (m, 1H), 2.07 - 1.96 (m, 2H), 1.85 - 1.79(m, 1H), 1.69 - 1.66 (m, 1H), 1.45 - 1.31 (m, 1H). LCMS (ESI) calcd for $C_{33}H_{32}ClN_4O_4^+$ [M+H]$^+$: 583.21, found, 583.2.

**Example 113: Preparation of 3-(5-(5-((4'-chloro-[1,1'-biphenyl]-2-yl)methyl)-2,5-diazabicyclo[2.2.1]heptane-2-carbonyl)-1-oxoisoindolin-2-yl)piperidine-2,6-dione (GT-05639)**

Referring to the methods of Scheme 6 and Example 76, the target product (GT-05639) was prepared as white solid (30 mg, yield 33%). $^1$H NMR (400 MHz, DMSO-d6) δ 11.01 (s, 1H), 8.00 - 7.84 (m, 1H), 7.79 (d, *J* = 6.9 Hz, 1H), 7.69 - 7.66 (m, 1H), 7.58 - 7.49 (m, 5H), 7.40 - 7.29 (m, 3H), 5.14 (dd, *J* = 13.1, 4.6 Hz, 1H), 4.52 - 4.26 (m, 5H), 3.89 - 3.67 (m, 2H), 3.60 - 3.47 (m, 2H), 2.95 - 2.87 (m, 2H), 2.61 (d, *J* = 15.9 Hz, 2H), 2.47 - 2.34 (m, 1H), 2.06 - 1.97 (m, 2H). LCMS (ESI) calcd for $C_{32}H_{30}ClN_4O_4^+$ [M+H]$^+$: 569.20, found, 569.2.

**Example 114: Preparation of 3-(5-((5-((4'-fluoro-5,5-dimethyl-3,4,5,6-tetrahydro-[1,1'-biphenyl]-2-yl)methyl)hexahydropyrrolo[3,4-c]pyrrol-2(1H)-yl)methyl)-1-oxoisoindolin-2-yl)piperidine-2,6-dione (GT-05398)**

Referring to the method of Scheme 1, the target product (GT-05398) was prepared as white solid (22 mg, yield 26%). $^1$H NMR (400 MHz, DMSO-d6) δ 11.01 (s, 1H), 7.91 - 7.81 (m, 1H), 7.80 - 7.71 (m, 1H), 7.25 - 7.11 (m, 5H), 5.14 (dd, *J* = 13.2, 5.1 Hz, 1H), 4.55 - 4.33 (m, 4H), 3.63 - 3.57 (m, 1H), 3.50 - 3.45 (m, 5H), 3.22 - 3.11 (m, 3H), 3.09 - 3.06 (m, 2H), 2.98 - 2.80 (m, 2H), 2.61 (d, *J* = 17.1 Hz, 1H), 2.47 - 2.36 (m, 2H), 2.32 - 2.27 (m, 2H), 2.03 (s, 3H), 1.55 - 1.37 (m, 2H), 0.95 (s, 6H). LCMS (ESI) calcd for $C_{35}H_{42}FN_4O_3^+$ [M+H]$^+$: 585.32, found, 585.3.

**Example 115: Preparation of 3-(5-((3-((4'-fluoro-5,5-dimethyl-3,4,5,6-tetrahydro-[1,1'-biphenyl]-2-yl)methyl)-3,6-diazabicyclo[3.1.1]heptan-6-yl)methyl)-1-oxoisoindolin-2-yl)piperidine-2,6-dione (GT-05399)**

Referring to the method of Scheme 1, the target product (GT-05399) was prepared as white solid (10 mg, yield 12%). $^1$H NMR (400 MHz, DMSO-d6) δ 11.00 (s, 1H), 7.84 - 7.79 (m, 1H), 7.75 - 7.59 (m, 2H), 7.18 - 7.07 (m, 4H), 5.19 - 5.08 (m, 1H), 4.62 - 4.50 (m, 1H), 4.49 - 4.46 (m, 1H), 4.38 - 4.32 (m, 3H), 3.13 - 2.80 (m, 5H), 2.61 (d, *J* = 16.6 Hz, 2H), 2.40 (dd, *J* = 17.8, 8.7 Hz, 2H), 2.28 - 2.18 (m, 4H), 2.05 - 2.00 (m, 4H), 1.42 (t, *J* = 6.4 Hz, 2H), 0.96 (d, *J* = 2.7 Hz, 6H). LCMS (ESI) calcd for $C_{35}H_{40}FN_4O_3^+$ [M+H]$^+$: 571.31, found, 571.3.

**Example 116: Preparation of 3-(5-((3-((4'-fluoro-5,5-dimethyl-3,4,5,6-tetrahydro-[1,1'-biphenyl]-2-yl)methyl)-3,8-diazabicyclo [3.2.1] octan-8-yl)methyl)-1-oxoisoindolin-2-yl)piperidine-2,6-dione (GT-05400)**

Referring to the method of Scheme 1, the target product (GT-05400) was prepared as white solid (9 mg, yield 11%). $^1$H NMR (400 MHz, DMSO-d6) δ 11.00 (s, 1H), 7.84 (d, *J* = 9.3 Hz, 1H), 7.74 (d, *J* = 7.9 Hz, 1H), 7.16 - 7.04 (m, 5H), 5.14 (dd, *J* = 13.1, 5.2 Hz, 1H), 4.51 - 4.47 (m, 1H), 4.42 - 4.33 (m, 1H), 4.26 (d, *J* = 5.2 Hz, 1H), 3.79 (s, 2H), 2.98 - 2.85 (m, 2H), 2.83 - 2.79 (m, 2H), 2.61 (d, *J* = 15.1 Hz, 3H), 2.47 - 2.39 (m, 1H), 2.26 - 2.14 (m, 6H), 2.08 - 1.85 (m, 6H), 1.43 - 1.40 (m, 2H), 0.94 (s, 6H). LCMS (ESI) calcd for $C_{35}H_{42}FN_4O_3^+$ [M+H]$^+$: 585.32, found, 585.3.

**Example 117: Preparation of 3-(5-((5-((4'-fluoro-5,5-dimethyl-3,4,5,6-tetrahydro-[1,1'-biphenyl]-2-yl)methyl)-2,5-diazabicyclo[2.2.1]heptan-2-yl)methyl)-1-oxoisoindolin-2-yl)piperidine-2,6-dione (GT-05401)**

Referring to the method of Scheme 1, the target product (GT-05401) was prepared as white solid (9 mg, yield 11%). $^1$H NMR (400 MHz, DMSO-d6) δ 11.01 (s, 1H), 8.01 - 7.54 (m, 3H), 7.26 - 7.18 (m, 4H), 5.18 - 5.09 (m, 1H), 4.50 - 4.33 (m, 4H), 4.22 - 4.09 (m, 2H), 3.67 - 3.53 (m, 2H), 3.24 - 3.10 (m, 4H), 2.99 - 2.85 (m, 2H), 2.61 (d, *J* = 16.7 Hz, 1H), 2.41 - 2.26(m, 2H), 2.26 - 2.16 (m, 1H), 2.14 - 1.95 (m, 4H), 1.49 - 1.45 (m, 2H), 0.96 (s, 6H). LCMS (ESI) calcd for $C_{34}H_{40}FN_4O_3^+$ [M+H]$^+$: 571.31, found, 571.3.

**Example 118: Preparation of 3-(5-((4-(4'-fluoro-5,5-dimethyl-3,4,5,6-tetrahydro-[1,1'-biphenyl]-2-carbonyl)piperazin-1-yl)methyl)-1-oxoisoindolin-2-yl)piperidine-2,6-dione (GT-**05520)

Referring to the method of Scheme 1, the target product (GT-05520) was prepared as white solid (11 mg, yield 13%). $^1$H NMR (400 MHz, DMSO-d6) δ 11.01 (s, 1H), 7.80 (d, *J* = 7.7 Hz, 1H), 7.78 - 7.51 (m, 2H), 7.19 - 7.11 (m, 4H), 5.14 (dd, *J* = 12.9, 4.5 Hz, 1H), 4.51- 4.11 (m, 5H), 3.66 (d, *J* = 13.3 Hz, 1H), 3.47 - 3.45 (m, 2H), 3.22 (brs, 1H), 3.10 - 3.05 (m, 1H), 2.96 - 2.88 (m, 2H), 2.61 (d, *J* = 16.3 Hz, 2H), 2.49 - 2.33 (m, 3H), 2.15 (brs, 1H), 2.07 - 1.95 (m, 1H), 1.89 (d, *J* = 17.7 Hz, 1H), 1.43 (brs, 2H), 0.96 (s, 6H). LCMS (ESI) calcd for $C_{33}H_{38}FN_4O_4^+$ [M+H]$^+$: 573.29, found, 573.3.

**Example 119: Preparation of 3-(5-((7-(4'-fluoro-5,5-dimethyl-3,4,5,6-tetrahydro-[1,1'-biphenyl]-2-carbonyl)-2,7-diazaspiro[3.5]nonan-2-yl)methyl)-1-oxoisoindolin-2-yl)piperidine-2,6-dione (GT-05404)**

Referring to the method of Scheme 1, the target product (GT-05404) was prepared as white solid (15 mg, yield 18%). $^1$H NMR (400 MHz, DMSO-d6) δ 11.01 (s, 1H), 7.79 - 7.76 (m, 1H), 7.71 - 7.62 (m, 1H), 7.30 - 7.21 (m, 2H), 7.15 - 7.09 (m, 3H), 5.13 (dd, *J* = 13.2, 5.1 Hz, 1H), 4.55 - 4.24 (m, 4H), 3.80 - 3.67(m, 3H), 3.62 - 3.47 (m, 1H), 3.17 - 3.06 (m, 2H), 3.03 - 2.77 (m, 3H), 2.61 (d, *J* = 18.1 Hz, 1H), 2.40 - 2.28 (m, 4H), 2.07 - 1.99 (m, 2H), 1.90 (d, *J* = 17.0 Hz, 1H), 1.75 - 1.66 (m, 1H), 1.55 - 1.42 (m, 3H), 1.27 - 1.20 (m, 1H), 0.98 (d, *J* = 14.6 Hz, 6H). LCMS (ESI) calcd for $C_{36}H_{42}FN_4O_4^+$ [M+H]$^+$: 613.32, found, 613.3.

**Example 120: Preparation of 3-(5-((5-(4'-fluoro-5,5-dimethyl-3,4,5,6-tetrahydro-[1,1'-biphenyl]-2-carbonyl)hexahydropyrrolo[3,4-c]pyrrol- 2(1H)-yl)methyl)-1-oxoisoindolin-2-yl)piperidine-2,6-dione (GT-05405)**

Referring to the method of Scheme 1, the target product (GT-05405) was prepared as white solid (12 mg, yield 14%). $^1$H NMR (400 MHz, DMSO-d6) δ 11.01 (s, 1H), 7.92 - 7.74 (m, 2H), 7.72 - 7.66 (m, 1H), 7.31 (brs, 1H), 7.28 - 7.08 (m, 3H), 5.14 (dd, *J* = 13.2, 4.9 Hz, 1H), 4.52 - 4.35 (m, 4H), 3.56 - 3.43 (m, 2H), 3.21 - 3.17 (m, 2H), 3.08 (d, *J* = 10.7 Hz, 1H), 2.99 - 2.86 (m, 2H), 2.76 - 2.63 (m, 2H), 2.61 (d, *J* = 17.4 Hz, 2H), 2.45 - 2.41 (m, 1H), 2.33 - 2.19 (m, 3H), 2.03 - 1.99 (m, 2H), 1.45 - 1.42 (m, 2H), 0.99 (s, 6H). LCMS (ESI) calcd for $C_{35}H_{40}FN_4O_4^+$ [M+H]$^+$: 599.30, found, 599.3.

**Example 121: Preparation of 3-(5-((5-(4'-fluoro-5,5-dimethyl-3,4,5,6-tetrahydro-[1,1'-biphenyl]-2-carbonyl)-2,5-diazabicyclo[2.2.2]octan-2-yl)methyl)-1-oxoisoindolin-2-yl)piperidine-2,6-dione (GT-05402)**

Referring to the method of Scheme 1, the target product (GT-05402) was prepared as white solid (13 mg, yield 16%). $^1$H NMR (400 MHz, DMSO-d6) δ 11.01 (s, 1H), 7.82 - 7.71 (m, 2H), 7.41 - 7.09 (m, 5H), 5.16 - 5.12 (m, 1H), 4.51 - 4.27 (m, 5H), 3.78 - 3.66 (m, 2H), 3.12 - 3.08 (m, 1H), 2.96 - 2.89 (m, 2H), 2.61 (d, *J* = 15.5 Hz, 2H), 2.43 - 2.31 (m, 5H), 2.06 - 1.98 (m, 2H), 1.91 - 1.73 (m, 2H), 1.57 - 1.34 (m, 3H), 0.97 (s, 6H). LCMS (ESI) calcd for $C_{35}H_{40}FN_4O_4^+$ [M+H]$^+$: 599.30, found, 599.3.

**Example 122: Preparation of 3-(5-((5-(4'-fluoro-5,5-dimethyl-3,4,5,6-tetrahydro-[1,1'-biphenyl]-2-carbonyl)-2,5-diazabicyclo[2.2.1]heptan-2-yl)methyl)-1-oxoisoindolin-2-yl)piperidine-2,6-dione (GT-05403)**

Referring to the method of Scheme 1, the target product (GT-05403) was prepared as white solid (13 mg, yield 16%). $^1$H NMR (400 MHz, DMSO-d6) δ 11.01 (s, 1H), 7.81 - 7.77 (M, 2H), 7.74 - 7.65 (m, 1H), 7.34 - 7.09 (m, 4H), 5.14 (dd, *J* = 13.2, 4.9 Hz, 1H), 4.53 - 4.14 (m, 6H), 3.27 - 3.23 (m, 2H), 3.17 - 3.13 (m, 2H), 3.05 - 2.83 (m, 2H), 2.61 (d, *J* = 14.6 Hz, 1H), 2.42 (dd, *J* = 13.1, 4.4 Hz, 1H), 2.39 - 2.12 (m, 4H), 2.09 - 1.94 (m, 2H), 1.51 - 1.33 (m, 2H), 0.97 (s, 6H). LCMS (ESI) calcd for $C_{34}H_{38}FN_4O_4^+$ [M+H]$^+$: 585.29, found, 585.3.

**Example 123: Preparation of 3-(5-(4-((4'-fluoro-3,4,5,6-tetrahydro-[1,1'-biphenyl]-2-yl)methyl)piperazine-1-carbonyl)-1-oxoisoindolin-2-yl)piperidine-2,6-dione (GT-05863)**

Referring to the methods of Scheme 6 and Example 76, the target product (GT-05863) was prepared as white solid (25 mg, yield 27%). $^{1}H$ NMR (400 MHz, DMSO-d6) δ 11.01 (s, 1H), 10.12 (brs, 1H), 7.79 (d, *J* = 7.8 Hz, 1H), 7.65 (s, 1H), 7.54 (d, *J* = 7.6 Hz, 1H), 7.25 - 7.19 (m, 4H), 5.14 (dd, *J* = 13.3, 5.0 Hz, 1H), 4.51 - 4.35 (m, 3H), 3.76 - 3.58 (m, 8H), 2.92 - 2.89 (m, 2H), 2.68 - 2.57 (m, 2H), 2.42 (dd, *J* = 13.0, 4.2 Hz, 1H), 2.25 - 2.20 (m, 3H), 2.06 - 1.96 (m, 1H), 1.69 (brs, 4H). LCMS (ESI) calcd for $C_{31}H_{34}FN_4O_4^+$ $[M+H]^+$: 545.26, found, 545.3.

**Example 124: Preparation of 5-((4-((4'-chloro-5,5-dimethyl-3,4,5,6-tetrahydro-[1,1'-biphenyl]-2-yl)methyl)piperazin-1-yl)methyl)-2-(2,6-dioxopiperidin-3-yl)isoindoline-1,3-dione (GT-05364)**

Referring to the method of Scheme 1, the target product (GT-05364) was prepared as white solid (24 mg, yield 26%). $^{1}H$ NMR (400 MHz, DMSO-d6) δ 11.14 (s, 1H), 8.10 - 7.81 (m, 3H), 7.42 (d, *J* = 7.9 Hz, 2H), 7.11 (d, *J* = 7.8 Hz, 2H), 5.16 (dd, *J* = 12.9, 5.3 Hz, 1H), 3.31 - 3.18(m, 4H), 2.96 - 2.87 (m, 4H), 2.71 - 2.54 (m, 5H), 2.26 (brs, 3H), 2.09 - 2.01 (m, 4H), 1.44 (t, *J* = 6.2 Hz, 2H), 0.95 (s, 6H). LCMS (ESI) calcd for $C_{33}H_{38}ClN_4O_4^+$ $[M+H]^+$: 589.26, found, 589.3.

**Example 125: Preparation of 4-((4-((4'-chloro-5,5-dimethyl-3,4,5,6-tetrahydro-[1,1'-biphenyl]-2-yl)methyl)piperazin-1-yl)methyl)-2-(2,6-dioxopiperidin-3-yl)isoindoline-1,3-dione (GT-05365)**

Referring to the method of Scheme 1, the target product (GT-05365) was prepared as white solid (21 mg, yield 24%). $^{1}H$ NMR (400 MHz, DMSO-d6) δ 11.14 (s, 1H), 7.87 (brs, 3H), 7.42 (d, *J* = 8.1 Hz, 2H), 7.12 (d, *J* = 8.4 Hz, 2H), 5.14 (dd, *J* = 12.5, 5.4 Hz, 1H), 4.12 - 3.99 (m, 1H), 3.67 - 3.51 (m, 2H), 3.40 - 3.20 (m, 4H), 2.89 - 2.85 (m, 3H), 2.82 - 2.68 (m, 2H), 2.63 - 2.59 (m, 2H), 2.26 (brs, 2H), 2.12 - 1.90 (m, 4H), 1.46 (t, *J* = 6.0 Hz, 2H), 0.95 (s, 6H). LCMS (ESI) calcd for $C_{33}H_{38}ClN_4O_4^+$ $[M+H]^+$: 589.26, found, 589.3.

**Example 126: Preparation of 2-(2,6-dioxopiperidin-3-yl)-4-((4-((4'-fluoro-3,4,5,6-tetrahydro-[1,1'-biphenyl]-2-yl)methyl)piperazin-1-yl)methyl)isoindoline-1,3-dione (GT-05862)**

Referring to the method of Scheme 1, the target product (GT-05862) was prepared as white solid (50 mg, yield 53%). $^{1}H$ NMR (400 MHz, DMSO-d6) δ 11.14 (s, 1H), 8.06 - 7.82 (m, 3H), 7.26 - 7.12 (m, 4H), 5.15 (dd, *J* = 12.8, 5.3 Hz, 1H), 4.49 - 4.14 (m, 2H), 3.33 - 3.24 (m, 4H), 3.22 - 2.97 (m, 3H), 2.97 - 2.83 (m, 2H), 2.83 - 2.68 (m, 2H), 2.66 - 2.56 (m, 1H), 2.54 - 2.51 (m, 1H), 2.34 - 2.20 (m, 4H), 2.14 - 1.98 (m, 1H), 1.68 (s, 4H). LCMS (ESI) calcd for $C_{31}H_{34}FN_4O_4^+$ $[M+H]^+$: 545.26, found, 545.3.

**Example 127: Preparation of 5-((4-((4'-chloro-5,5-dimethyl-3,4,5,6-tetrahydro-[1,1'-biphenyl]-2-yl)methyl)-1,4-diazepan-1-yl)methyl)-2-(2,6-dioxopiperidin-3-yl)isoindoline-1,3-dione (GT-05397)**

Referring to the method of Scheme 1, the target product (GT-05397) was prepared as white solid (8 mg, yield 10%). $^{1}H$ NMR (400 MHz, DMSO-d6) δ 11.15 (s, 1H), 8.22 - 7.95 (m, 3H), 7.45 (brs, 2H), 7.14 (d, *J* = 8.2 Hz, 2H), 5.27 - 5.12 (m, 1H), 4.65 - 4.23 (m, 1H), 3.86 - 3.74 (m, 1H), 3.67 - 3.43 (m, 4H), 3.29 - 3.20 (m, 4H), 3.17 - 2.99 (m, 2H), 2.97 - 2.77 (m, 2H), 2.62 (d, *J* = 17.8 Hz, 1H), 2.58 - 2.52 (m, 1H), 2.37 - 2.17 (m, 3H), 2.14 - 1.93 (m, 4H), 1.45 (t, *J* = 6.1 Hz, 2H), 0.95 (s, 6H). LCMS (ESI) calcd for $C_{34}H_{40}ClN_4O_4^+$ $[M+H]^+$: 603.27, found, 603.3.

**Example 128: Preparation of 5-((3-((4'-chloro-5,5-dimethyl-3,4,5,6-tetrahydro-[1,1'-biphenyl]-2-yl)methyl)-3,6-diazabicyclo[3.1.1]heptan-6-yl)methyl)-2-(2,6-dioxopiperidin-3-yl)isoindoline-1,3-dione (GT-05368)**

Referring to the method of Scheme 1, the target product (GT-05368) was prepared as white solid (8 mg, yield 10%). $^{1}H$ NMR (400 MHz, DMSO-d6) δ 11.14 (s, 1H), 8.16 (brs, 1H), 8.03 (s, 1H), 8.01 (brs, 1H), 7.39 (d, *J* = 8.3 Hz, 2H), 7.10 (t, *J* = 8.3 Hz, 2H), 5.23 - 5.14 (m, 1H), 4.49 - 4.33 (m, 1H), 4.17 - 4.15 (m, 1H), 3.60 - 3.47 (m, 2H), 3.13 - 3.07 (m, 2H), 2.90 - 2.86 (m, 3H), 2.67 - 2.58 (m, 2H), 2.57 - 2.55 (m, 2H), 2.30 - 2.15 (m, 3H), 2.14 - 2.03 (m, 2H), 2.00 - 1.97 (m, 2H), 1.44 - 1.40 (m, 2H), 0.95 (s, 6H). LCMS (ESI) calcd for $C_{34}H_{38}ClN_4O_4^+$ $[M+H]^+$: 601.26, found, 601.3.

**Example 129: Preparation of 5-((6-((4'-chloro-5,5-dimethyl-3,4,5,6-tetrahydro-[1,1'-biphenyl]-2-yl)methyl)-3,6-diazabicyclo[3.1.1]heptan-3-yl)methyl)-2-(2,6-dioxopiperidin-3-yl)isoindoline-1,3-dione (GT-05532)**

Referring to the method of Scheme 1, the target product (GT-05532) was prepared as white solid (22 mg, yield 25%). $^{1}H$ NMR (400 MHz, DMSO-d6) δ 11.13 (s, 1H), 7.93 - 7.90 (m, 2H), 7.83 - 7.63 (m, 1H), 7.53 - 7.47 (m, 1H), 7.38 - 7.36

(m, 1H), 7.29 - 7.12 (m, 2H), 5.17 (dd, *J* = 12.9, 3.3 Hz, 1H), 4.09 (brs, 1H), 3.79 - 3.73 (m, 2H), 3.60 - 3.48 (m, 4H), 3.07 - 2.80 (m, 2H), 2.67 - 2.56 (m, 3H), 2.31 - 2.10 (m, 3H), 2.11 - 1.88 (m, 4H), 1.46 - 1.42 (m, 2H), 0.96 (s, 6H). LCMS (ESI) calcd for $C_{34}H_{38}ClN_4O_4^+$ [M+H]$^+$: 601.26, found, 601.3.

**Example 130: Preparation of 5-((8-((4'-chloro-5,5-dimethyl-3,4,5,6-tetrahydro-[1,1'-biphenyl]-2-yl)methyl)-3,8-diazabicyclo[3.2.1]octan-3-yl)methyl)-2-(2,6-dioxopiperidin-3-yl)isoindoline-1,3-dione (GT-05537)**

Referring to the method of Scheme 1, the target product (GT-05537) was prepared as white solid (10 mg, yield 35%). $^1$H NMR (400 MHz, DMSO-d6) δ 11.13 (s, 1H), 7.98 - 7.77 (m, 3H), 7.45 (d, *J* = 8.4 Hz, 2H), 7.19 (d, *J* = 8.4 Hz, 2H), 5.15 (dd, *J* = 12.8, 5.4 Hz, 1H), 3.74 (d, *J* = 15.0 Hz, 4H), 3.48 (d, *J* = 4.9 Hz, 3H), 2.96 - 2.78 (m, 3H), 2.75 - 2.66 (m, 2H), 2.58 - 2.51 (m, 2H), 2.34 - 2.31 (m, 2H), 2.07 (s, 3H), 1.79 (d, *J* = 8.5 Hz, 2H), 1.47 (t, *J* = 6.2 Hz, 2H), 1.21 - 1.17 (m, 2H), 0.96 (s, 6H). LCMS (ESI) calcd for $C_{35}H_{40}ClN_4O_4^+$ [M+H]$^+$: 615.27, found, 615.3.

**Example 131: Preparation of 5-((5-((4'-chloro-5,5-dimethyl-3,4,5,6-tetrahydro-[1,1'-biphenyl]-2-yl)methyl)-2,5-diazabicyclo[2.2.1]heptan-2-yl)methyl)-2-(2,6-dioxopiperidin-3-yl)isoindoline-1,3-dione (GT-05533)**

Referring to the method of Scheme 1, the target product (GT-05533) was prepared as white solid (36 mg, yield 42%). $^1$H NMR (400 MHz, DMSO-d6) δ 11.14 (s, 1H), 8.30 - 7.73 (m, 3H), 7.47 - 7.30 (m, 2H), 7.18 (d, *J* = 8.2 Hz, 2H), 5.23 - 5.12 (m, 1H), 4.45 - 3.90 (m, 4H), 3.71 - 3.43 (m, 6H), 3.26 - 3.02 (m, 2H), 2.95 - 2.87 (m, 1H), 2.64 - 2.59 (m, 2H), 2.44 - 2.40(m, 1H), 2.31 - 2.14 (m, 1H), 2.12 - 1.98 (m, 3H), 1.57 - 1.37 (m, 2H), 0.96 (s, 6H). LCMS (ESI) calcd for $C_{34}H_{38}ClN_4O_4^+$ [M+H]$^+$: 601.26, found, 601.3.

**Example 132: Preparation of 2-(2,6-dioxopiperidin-3-yl)-5-((4-((4'-fluoro-5,5-dimethyl-3,4,5,6-tetrahydro-[1,1'-biphenyl]-2-yl)methyl)piperazin-1-yl)methyl)isoindoline-1,3-dione (GT-05395)**

Referring to the method of Scheme 1, the target product (GT-05395) was prepared as white solid (30 mg, yield 31%). $^1$H NMR (400 MHz, DMSO-d6) δ 11.14 (s, 1H), 7.99 - 7.82 (m, 3H), 7.26 - 7.04 (m, 4H), 5.16 (dd, *J* = 12.7, 5.8 Hz, 1H), 3.62 - 3.47 (m, 5H), 2.98 - 2.83 (m, 4H), 2.82 - 2.70 (m, 2H), 2.67 - 2.55 (m, 3H), 2.27 - 2.22 (m, 2H), 2.15 - 1.95 (m, 4H), 1.45 (t, *J* = 5.0 Hz, 2H), 0.95 (s, 6H). LCMS (ESI) calcd for $C_{33}H_{38}FN_4O_4^+$ [M+H]$^+$: 573.29, found, 573.3.

**Example 133: Preparation of 5-((4-((4'-chloro-[1,1'-biphenyl]-3-yl)methyl)piperazin-1-yl)methyl)-2-(2,6-dioxopiperidin-3-yl)isoindoline-1,3-dione (GT-05630)**

Referring to the method of Scheme 1, the target product (GT-05630) was prepared as white solid (40 mg, yield 43%). $^1$H NMR (400 MHz, DMSO-d6) δ 11.14 (s, 1H), 8.03 (brs, 1H), 7.97 - 7.91 (m, 3H), 7.76 - 7.73 (m, 3H), 7.61 - 7.50 (m, 4H), 5.16 (dd, *J* = 12.8, 5.3 Hz, 1H), 4.47 - 4.27 (m, 2H), 3.37 - 3.29 (m, 4H), 3.25 - 3.10 (m, 4H), 2.94 - 2.86 (m, 4H), 2.70 - 2.52 (m, 3H), 2.06 - 1.99 (m, 1H). LCMS (ESI) calcd for $C_{31}H_{30}ClN_4O_4^+$ [M+H]$^+$: 557.20, found, 557.2.

**Example 134: Preparation of 5-((4-((4'-chloro-[1,1'-biphenyl]-4-yl)methyl)piperazin-1-yl)methyl)-2-(2,6-dioxopiperidin-3-yl)isoindoline-1,3-dione (GT-05599)**

Referring to the method of Scheme 1, the target product (GT-05599) was prepared as white solid (42 mg, yield 46%). $^1$H NMR (400 MHz, DMSO-d6) δ 11.14 (s, 1H), 8.09 (brs, 1H), 7.98 (brs, 2H), 7.78 - 7.73 (m, 4H), 7.68 (d, *J* = 7.5 Hz, 2H), 7.54 (d, *J* = 8.6 Hz, 2H), 5.17 (dd, *J* = 12.8, 5.4 Hz, 1H), 4.52 - 4.00 (m, 4H), 3.43 - 3.34 (m, 4H), 3.31 - 3.10(m, 4H), 2.96 - 2.84 (m, 1H), 2.61 (d, *J* = 18.2 Hz, 1H), 2.52 - 2.49 (m, 1H), 2.12 - 1.99 (m, 1H). LCMS (ESI) calcd for $C_{31}H_{30}ClN_4O_4^+$ [M+H]$^+$: 557.20, found, 557.2.

**Example 135: Preparation of 5-((4-((3-(4-chlorophenyl)pyridin-4-yl)methyl)piperazin-1-yl)methyl)-2-(2,6-dioxopiperidin-3-yl)isoindoline-1,3-dione (GT-05731)**

Referring to the method of Scheme 1, the target product (GT-05731) was prepared as white solid (8 mg, yield 14%). $^1$H NMR (400 MHz, DMSO-d6) δ 11.15 (s, 1H), 8.80 (d, *J* = 5.5 Hz, 1H), 8.69 (s, 1H), 8.22 (s, 1H), 8.11 (d, *J* = 7.6 Hz, 1H), 8.02 (d, *J* = 7.6 Hz, 2H), 7.59 (d, *J* = 8.3 Hz, 2H), 7.52 (d, *J* = 8.2 Hz, 2H), 5.18 (dd, *J* = 12.7, 5.4 Hz, 1H), 4.48 (s, 2H), 3.26 (brs, 4H), 3.07 (brs, 2H), 2.96 - 2.77 (m, 4H), 2.66 - 2.55 (m, 3H), 2.09 - 2.05 (m, 1H). LCMS (ESI) calcd for $C_{30}H_{29}ClN_5O_4^+$ [M+H]$^+$: 558.19, found, 558.2.

**Example 136: Preparation of 5-((4-([2,4'-bipyridin]-5-ylmethyl)piperazin-1-yl)methyl)-2-(2,6-dioxopiperidin-3-yl) isoindoline-1,3-dione (GT-05634)**

Referring to the method of Scheme 1, the target product (GT-05634) was prepared as white solid (35 mg, yield 36%). $^1$H NMR (400 MHz, DMSO-d6) δ 11.15 (s, 1H), 9.11 (s, 1H), 9.06 (d, *J* = 5.6 Hz, 2H), 8.74 (d, *J* = 5.6 Hz, 2H), 8.53 (d, *J* = 8.1 Hz, 1H), 8.45 (d, *J* = 8.1 Hz, 1H), 8.30 (s, 1H), 8.18 (d, *J* = 7.6 Hz, 1H), 8.02 (d, *J* = 7.6 Hz, 1H), 5.18 (dd, *J* = 12.6, 5.3 Hz, 2H), 4.61 (brs, 8H), 4.31 (brs, 4H), 2.94 - 2.87 (m, 1H), 2.65 - 2.55 (m, 2H), 2.11 - 2.03 (m, 1H). LCMS (ESI) calcd for $C_{29}H_{29}N_6O_4^+$ [M+H]$^+$: 525.22, found, 525.2.

**Example 137: Preparation of 2-(2,6-dioxopiperidin-3-yl)-5-((4-((5-phenylpyrazin-2-yl)methyl)piperazin-1-yl) methyl)isoindoline-1,3-dione (GT-05545)**

Referring to the method of Scheme 1, the target product (GT-05545) was prepared as white solid (31 mg, yield 32%). $^1$H NMR (400 MHz, DMSO-d6) δ 11.14 (s, 1H), 9.30 (s, 1H), 8.88 (s, 1H), 8.18 (dd, *J* = 7.9, 1.6 Hz, 2H), 8.12 (brs, 1H), 8.00 (brs, 2H), 7.62 - 7.49 (m, 3H), 5.17 (dd, *J* = 12.8, 5.4 Hz, 1H), 4.37 (brs, 4H), 3.34 - 3.23 (m, 4H), 3.21 - 3.04 (m, 4H), 2.95 - 2.83 (m, 1H), 2.69 - 2.52 (m, 2H), 2.09 - 2.05 (m, 1H). LCMS (ESI) calcd for $C_{29}H_{29}N_6O_4^+$ [M+H]$^+$: 525.22, found, 525.2.

**Example 138: Preparation of 5-((4-((5-(4-chlorophenyl)thiophen-2-yl)methyl)piperazin-1-yl)methyl)-2-(2,6-dioxopiperidin-3-yl)isoindoline-1,3-dione (GT-05626)**

Referring to the method of Scheme 1, the target product (GT-05626) was prepared as white solid (38 mg, yield 42%). $^1$H NMR (400 MHz, DMSO-d6) δ 11.14 (s, 1H), 8.06 (brs, 1H), 7.98 (brs, 2H), 7.67 (d, *J* = 8.5 Hz, 2H), 7.50 - 7.48 (m, 3H), 7.24 (brs, 1H), 5.17 (dd, *J* = 12.9, 5.4 Hz, 1H), 4.72 - 3.95 (m, 4H), 3.41 - 3.32 (m, 4H), 3.26 - 3.17 (m, 4H), 3.12 - 3.05 (m, 1H), 2.91 - 2.87 (m, 1H), 2.63 - 2.55 (m, 1H), 2.12 - 2.02 (m, 1H). LCMS (ESI) calcd for $C_{29}H_{28}ClN_4O_4S^+$ [M+H]$^+$: 563.15, found, 563.2.

**Example 139: Preparation of 2-(2,6-dioxopiperidin-3-yl)-5-((4-((5-(thiophen-2-yl)furan-2-yl)methyl)piperazin-1-yl)methyl)isoindoline-1,3-dione (GT-05628)**

Referring to the method of Scheme 1, the target product (GT-05628) was prepared as white solid (31 mg, yield 32%). $^1$H NMR (400 MHz, DMSO-d6) δ 11.14 (s, 1H), 8.08 (brs, 1H), 7.98 (s, 2H), 7.57 (d, *J* = 4.7 Hz, 1H), 7.41 (d, *J* = 3.3 Hz, 1H), 7.13 (dd, *J* = 4.9, 3.7 Hz, 1H), 6.79 (d, *J* = 3.0 Hz, 1H), 6.72 (s, 1H), 5.17 (dd, *J* = 12.8, 5.4 Hz, 1H), 4.54 - 4.01 (m, 4H), 3.30 - 3.23 (m, 4H), 3.09 - 3.04 (m, 3H), 2.94 - 2.85 (m, 2H), 2.67 - 2.57 (m, 1H), 2.57 - 2.51 (m, 1H), 2.12 - 1.99 (m, 1H). LCMS (ESI) calcd for $C_{27}H_{26}N_4NaO_5S^+$ [M+Na]$^+$: 541.15, found, 541.2.

**Example 140: Preparation of 5-(4-((4'-chloro-[1,1'-biphenyl]-3-yl)methyl)piperazine-1-carbonyl)-2-(2,6-dioxopiperidin-3-yl)isoindoline-1,3-dione (GT-05631)**

Referring to the methods of Scheme 6 and Example 76, the target product (GT-05631) was prepared as white solid (38 mg, yield 40%). $^1$H NMR (400 MHz, DMSO-d6) δ 11.32 (s, 1H), 11.15 (s, 1H), 8.04 - 7.97 (m, 3H), 7.93 (d, *J* = 7.6 Hz, 1H), 7.77 (d, *J* = 8.4 Hz, 3H), 7.61 - 7.52 (m, 4H), 5.19 (dd, *J* = 12.8, 5.4 Hz, 1H), 4.57 (brs, 1H), 4.40 (s, 2H), 3.72 - 3.59 (m, 2H), 3.55 - 3.41 (m, 2H), 3.25 - 3.15 (m, 3H), 2.91 - 2.87 (m, 1H), 2.62 - 2.59 (m, 1H), 2.57 - 2.52 (m, 1H), 2.13 - 1.99 (m, 1H). LCMS (ESI) calcd for $C_{31}H_{28}ClN_4O_5^+$ [M+H]$^+$: 571.17, found, 571.2.

**Example 141: Preparation of 5-(4-((4'-chloro-[1,1'-biphenyl]-4-yl)methyl)piperazine-1-carbonyl)-2-(2,6-dioxopiperidin-3-yl)isoindoline-1,3-dione (GT-05600)**

Referring to the methods of Scheme 6 and Example 76, the target product (GT-05600) was prepared as white solid (39 mg, yield 41%). $^1$H NMR (400 MHz, DMSO-d6) δ 11.15 (s, 1H), 8.04 - 7.97 (m, 2H), 7.92 (d, *J* = 7.7 Hz, 1H), 7.79 (d, *J* = 7.2 Hz, 2H), 7.74 (d, *J* = 8.5 Hz, 2H), 7.67 (d, *J* = 6.9 Hz, 2H), 7.55 (d, *J* = 8.5 Hz, 2H), 5.19 (dd, *J* = 12.8, 5.4 Hz, 1H), 4.58 (brs, 1H), 4.38 (brs, 2H), 3.76 - 3.47 (m, 3H), 3.27 - 3.12 (m, 4H), 2.96 - 2.84 (m, 1H), 2.62 (d, *J* = 18.8 Hz, 1H), 2.57 - 2.52 (m, 1H), 2.14 - 2.00 (m, 1H). LCMS (ESI) calcd for $C_{31}H_{28}ClN_4O_5^+$ [M+H]$^+$: 571.17, found, 571.2.

**Example 142: Preparation of 5-(4-((3-(4-chlorophenyl)pyridin-4-yl)methyl)piperazine-1-carbonyl)-2-(2,6-dioxopiperidin-3-yl)isoindoline-1,3-dione (GT-05732)**

Referring to the methods of Scheme 6 and Example 76, the target product (GT-05732) was prepared as white

solid (25 mg, yield 44%). $^1$H NMR (400 MHz, DMSO-d6) δ 11.14 (s, 1H), 8.75 (d, *J* = 5.2 Hz, 1H), 8.60 (s, 1H), 8.12 (s, 1H), 7.99 (d, *J* = 7.7 Hz, 1H), 7.93 (s, 1H), 7.86 (d, *J* = 7.6 Hz, 1H), 7.62 (d, *J* = 7.9 Hz, 2H), 7.48 (d, *J* = 8.1 Hz, 2H), 5.18 (dd, *J* = 12.6, 5.3 Hz, 1H), 4.53 - 4.12 (m, 3H), 3.32 - 3.22 (m, 4H), 2.96 - 2.84 (m, 2H), 2.67 - 2.53 (m, 4H), 2.09 - 2.04 (m, 1H). LCMS (ESI) calcd for $C_{30}H_{27}ClN_5O_5^+$ [M+H]$^+$: 572.17, found, 572.2.

**Example 143: Preparation of 5-(3-((4'-chloro-5,5-dimethyl-3,4,5,6-tetrahydro-[1,1'-biphenyl]-2-yl)methyl)-3,8-diazabicyclo[3.2.1]octan-8-yl)-2-(2,6-dioxopiperidin-3-yl)isoindoline-1,3-dione (GT-05375)**

Referring to the method of Scheme 4, the target product (GT-05375) was prepared as white solid (8 mg, yield 12%). $^1$H NMR (400 MHz, DMSO-d6) δ 11.10 (s, 1H), 7.76 (d, *J* = 8.2 Hz, 1H), 7.34 (brs, 1H), 7.25 (d, *J* = 8.2 Hz, 2H), 7.19 - 7.17 (m, 1H), 7.06 - 7.02 (m, 2H), 5.13 (dd, *J* = 13.4, 9.5 Hz, 1H), 4.74 - 4.60 (m, 2H), 4.51 - 4.47 (m, 1H), 3.12 - 3.06 (m, 2H), 2.92 - 2.87 (m, 2H), 2.76 - 2.72 (m, 2H), 2.67 - 2.64 (m, 1H), 2.60 - 2.57(m, 1H), 2.24 - 2.16 (m, 4H), 2.10 - 2.02 (m, 3H), 1.92 - 1.85 (m, 2H), 1.46 - 1.35 (m, 3H), 0.88 (s, 6H). LCMS (ESI) calcd for $C_{34}H_{38}ClN_4O_4^+$ [M+H]$^+$: 601.26, found, 601.3.

**Example 144: Preparation of 5-(8-((4'-chloro-5,5-dimethyl-3,4,5,6-tetrahydro-[1,1'-biphenyl]-2-yl)methyl)-3,8-diazabicyclo[3.2.1]octan-3-yl)-2-(2,6-dioxopiperidin-3-yl)isoindoline-1,3-dione (GT-05376)**

Referring to the method of Scheme 4, the target product (GT-05376) was prepared as white solid (3 mg, yield 5%). $^1$H NMR (400 MHz, DMSO-d6) δ 11.09 (s, 1H), 7.74 (d, *J* = 8.8 Hz, 1H), 7.48 (d, *J* = 8.3 Hz, 2H), 7.34 - 7.33 (m, 1H), 7.24 (d, *J* = 8.3 Hz, 2H), 7.22 - 7.17 (m, 1H), 5.08 (dd, *J* = 12.0, 4.7 Hz, 1H), 4.01 - 3.88 (m, 3H), 3.62 - 3.60 (m, 1H), 3.50 - 3.47 (m, 1H), 2.89 - 2.83 (m, 3H), 2.60 - 2.51 (m, 2H), 2.39 - 2.32 (m, 3H), 2.05 - 1.96 (m, 3H), 1.79 - 1.75 (m, 2H), 1.52 - 1.50 (m, 2H), 1.33 - 1.29 (m, 2H), 1.00 (s, 6H). LCMS (ESI) calcd for $C_{34}H_{38}ClN_4O_4^+$ [M+H]$^+$: 601.26, found, 601.3.

**Example 145: Preparation of 5-(3-((4'-chloro-5,5-dimethyl-3,4,5,6-tetrahydro-[1,1'-biphenyl]-2-yl)methyl)-3,6-diazabicyclo[3.1.1]heptan-6-yl)-2-(2,6-dioxopiperidin-3-yl)isoindoline-1,3-dione (GT-05377)**

Referring to the method of Scheme 4, the target product (GT-05377) was prepared as white solid (11 mg, yield 16%). $^1$H NMR (400 MHz, DMSO-d6) δ 11.11 (s, 1H), 7.71 (d, *J* = 8.3 Hz, 1H), 7.18 (d, *J* = 7.4 Hz, 2H), 7.04 (s, 1H), 6.95 (d, *J* = 8.2 Hz, 2H), 6.78 (d, *J* = 6.7 Hz, 1H), 5.15 (dd, *J* = 12.6, 5.7 Hz, 1H), 4.61 - 4.47 (m, 2H), 3.55 - 3.50 (m, 2H), 3.14 - 3.06 (m, 2H), 2.98 - 2.85 (m, 2H), 2.76 - 2.71 (m, 1H), 2.70 - 2.56 (m, 3H), 2.44 (d, *J* = 9.7 Hz, 2H), 2.24 - 2.19 (m, 2H), 2.11 - 2.02 (m, 2H), 1.41 - 1.38 (m, 2H), 0.92 (s, 6H). LCMS (ESI) calcd for $C_{33}H_{36}ClN_4O_4^+$ [M+H]$^+$: 587.24, found, 587.3.

**Example 146: Preparation of 5-(6-((4'-chloro-5,5-dimethyl-3,4,5,6-tetrahydro-[1,1'-biphenyl]-2-yl)methyl)-3,6-diazabicyclo[3.1.1]heptan-3-yl)-2-(2,6-dioxopiperidin-3-yl)isoindoline-1,3-dione (GT-05378)**

Referring to the method of Scheme 4, the target product (GT-05378) was prepared as white solid (3 mg, yield 5%). $^1$H NMR (400 MHz, DMSO-d6) δ 11.09 (s, 1H), 7.79 (d, *J* = 8.5 Hz, 1H), 7.73 (d, *J* = 4.5 Hz, 1H), 7.51 (d, *J* = 8.4 Hz, 1H), 7.25 (d, *J* = 8.2 Hz, 1H), 7.20 - 7.18 (m, 1H), 7.05 - 7.02 (m, 1H), 6.83 - 6.81 (m, 1H), 5.13 - 5.08 (m, 1H), 4.57 - 4.49 (m, 1H), 4.02 - 3.95 (m, 1H), 3.92 - 3.88 (m, 1H), 3.82 - 3.72 (m, 1H), 3.51 - 3.49 (m, 1H), 3.02 - 2.87 (m, 3H), 2.57 - 2.51 (m, 3H), 2.28 - 2.23 (m, 2H), 2.07 - 2.01 (m, 3H), 1.91 - 1.88 (m, 1H), 1.51 - 1.38 (m, 3H), 0.96 (s, 6H). LCMS (ESI) calcd for $C_{33}H_{36}ClN_4O_4^+$ [M+H]$^+$: 587.24, found, 587.3.

**Example 147: Preparation of 5-(5-((4'-chloro-5,5-dimethyl-3,4,5,6-tetrahydro-[1,1'-biphenyl]-2-yl)methyl)-2,5-diazabicyclo[2.2.2]octan-2-yl)-2-(2,6-dioxopiperidin-3-yl)isoindoline-1,3-dione (GT-05379)**

Referring to the method of Scheme 4, the target product (GT-05379) was prepared as white solid (3 mg, yield 5%). $^1$H NMR (400 MHz, DMSO-d6) δ 11.07 (s, 1H), 7.67 (d, *J* = 8.7 Hz, 1H), 7.50 (d, *J* = 8.3 Hz, 1H), 7.22 - 7.16 (m, 3H), 7.07 - 7.05 (m, 2H), 5.15 - 5.03 (m, 1H), 4.50 - 4.39 (m, 1H), 3.71 - 3.67 (m, 3H), 3.55 - 3.53 (m, 2H), 3.14 - 3.09 (m, 1H), 2.98 - 2.84 (m, 3H), 2.62 - 2.58 (m, 2H), 2.30 - 2.23 (m, 2H), 2.05 - 2.00 (m, 3H), 1.94 - 1.85 (m, 2H), 1.54 - 1.43 (m, 3H), 0.96 (s, 6H). LCMS (ESI) calcd for $C_{34}H_{38}ClN_4O_4^+$ [M+H]$^+$: 601.26, found, 601.3.

**Example 148: Preparation of 5-(3-((4'-chloro-[1,1'-biphenyl]-2-yl)methyl)-3,8-diazabicyclo[3.2.1]octan-8-yl)-2-(2,6-dioxopiperidin-3-yl)isoindoline-1,3-dione (GT-05370)**

Referring to the method of Scheme 2, the target product (GT-05370) was prepared as white solid (5 mg, yield 8%). $^1$H NMR (400 MHz, DMSO-d6) δ 11.07 (s, 1H), 7.64 - 7.60 (m, 1H), 7.48 - 7.40 (m, 3H), 7.43 - 7.31 (m, 3H), 7.31 - 7.15 (m, 3H), 7.11 - 7.04 (m, 1H), 5.18 - 5.00 (m, 1H), 4.63 - 4.58 (m, 1H), 4.49 - 4.45 (m, 1H), 4.20 - 4.16 (m, 1H), 3.34 - 3.29 (m, 2H), 3.06 - 3.01 (m, 1H), 2.95 - 2.82 (m, 1H), 2.71 - 2.55 (m, 2H), 2.35 - 2.31 (m, 1H), 2.23 - 2.17 (m, 2H), 2.12 - 1.94 (m, 2H),

1.83 - 1.78 (m, 1H), 1.68 - 1.63 (m, 1H). LCMS (ESI) calcd for $C_{32}H_{30}ClN_4O_4^+$ [M+H]$^+$: 569.20, found, 569.2.

**Example 149: Preparation of 5-(8-((4'-chloro-[1,1'-biphenyl]-2-yl)methyl)-3,8-diazabicyclo[3.2.1]octan-3-yl)-2-(2,6-dioxopiperidin-3-yl)isoindoline-1,3-dione (GT-05371)**

Referring to the method of Scheme 2, the target product (GT-05371) was prepared as white solid (8 mg, yield 13%). $^1$H NMR (400 MHz, DMSO-d6) δ 11.08 (s, 1H), 8.10 - 8.08 (m, 1H), 7.71 (d, *J* = 8.6 Hz, 1H), 7.60 - 7.56 (m, 4H), 7.52 - 7.45 (m, 2H), 7.43 - 7.36 (m, 1H), 7.31 (s, 1H), 7.19 - 7.12 (m, 1H), 5.09 - 5.05 (m, 1H), 4.30 (d, *J* = 5.1 Hz, 2H), 3.91 - 3.88 (m, 3H), 3.50 - 3.47 (m, 2H), 3.01 - 2.81 (m, 2H), 2.62 - 2.51 (m, 2H), 2.07 - 1.95 (m, 1H), 1.77 (d, *J* = 8.4 Hz, 2H), 1.40 - 1.35 (m, 2H). LCMS (ESI) calcd for $C_{32}H_{30}ClN_4O_4^+$ [M+H]$^+$: 569.20, found, 569.2.

**Example 150: Preparation of 5-(3-((4'-chloro-[1,1'-biphenyl]-2-yl)methyl)-3,6-diazabicyclo[3.1.1]heptan-6-yl)-2-(2,6-dioxopiperidin-3-yl)isoindoline-1,3-dione (GT-05372)**

Referring to the method of Scheme 2, the target product (GT-05372) was prepared as white solid (11 mg, yield 17%). $^1$H NMR (400 MHz, DMSO-d6) δ 11.14 (s, 2H), 7.87 - 7.77 (m, 1H), 7.64 (d, *J* = 8.2 Hz, 1H), 7.42 (d, *J* = 7.6 Hz, 2H), 7.29 - 7.21 (m, 4H), 7.15 (d, *J* = 7.5 Hz, 1H), 6.90 (s, 1H), 6.72 - 6.60 (m, 1H), 5.21 - 5.16 (m, 1H), 4.51 - 4.42 (m, 2H), 4.19 - 4.02 (m, 2H), 3.29 - 3.19 (m, 2H), 3.15 - 3.09 (m, 2H), 3.02 - 2.85 (m, 2H), 2.72 - 2.66 (m, 2H), 2.38 (d, *J* = 9.5 Hz, 1H), 2.15 - 2.02 (m, 1H). LCMS (ESI) calcd for $C_{31}H_{28}ClN_4O_4^+$ [M+H]$^+$: 555.18, found, 555.2.

**Example 151: Preparation of 5-(6-((4'-chloro-[1,1'-biphenyl]-2-yl)methyl)-3,6-diazabicyclo[3.1.1]heptan-3-yl)-2-(2,6-dioxopiperidin-3-yl)isoindoline-1,3-dione (GT-05373)**

Referring to the method of Scheme 2, the target product (GT-05373) was prepared as white solid (11 mg, yield 17%). $^1$H NMR (400 MHz, DMSO-d6) δ 11.10 (s, 1H), 8.01 - 7.99 (m, 1H), 7.73 - 7.68 (m, 1H), 7.55 - 7.50 (m, 2H), 7.37 - 7.34 (m, 1H), 7.25 (d, *J* = 8.4 Hz, 2H), 7.13 (d, *J* = 8.3 Hz, 2H), 6.81 (brs, 2H), 5.14 (dd, *J* = 12.5, 5.4 Hz, 1H), 4.49 - 4.44 (m, 2H), 4.13 - 4.09 (m, 2H), 3.95 (dd, *J* = 35.8, 12.3 Hz, 1H), 3.76 (d, *J* = 12.9 Hz, 2H), 3.05 - 2.82 (m, 3H), 2.60 - 2.51 (m, 2H), 2.06 - 2.01 (m, 1H), 1.93 - 1.87 (m, 1H). LCMS (ESI) calcd for $C_{31}H_{28}ClN_4O_4^+$ [M+H]$^+$: 555.18, found, 555.2.

**Example 152: Preparation of 5-(5-((4'-chloro-[1,1'-biphenyl]-2-yl)methyl)-2,5-diazabicyclo[2.2.2]octan-2-yl)-2-(2,6-dioxopiperidin-3-yl)isoindoline-1,3-dione (GT-05374)**

Referring to the method of Scheme 2, the target product (GT-05374) was prepared as white solid (5 mg, yield 8%). $^1$H NMR (400 MHz, DMSO-d6) δ 11.08 (s, 1H), 7.72 - 7.65 (m, 1H), 7.62 - 7.58 (m, 2H), 7.57 - 7.53 (m, 2H), 7.49 - 7.41 (m, 2H), 7.41 - 7.30 (m, 3H), 6.95 - 6.92 (m, 1H), 5.12 - 5.05 (m, 1H), 4.50 - 4.31 (m, 2H), 3.55 - 3.53 (m, 1H), 3.48 - 3.42 (m, 2H), 3.03 - 2.80 (m, 4H), 2.67 - 2.53 (m, 2H), 2.06 - 1.94 (m, 1H), 1.94 - 1.69 (m, 2H), 0.96 - 0.92 (m, 2H). LCMS (ESI) calcd for $C_{32}H_{30}ClN_4O_4^+$ [M+H]$^+$: 569.20, found, 569.2.

**Example 153: Preparation of 2-(2,6-dioxopiperidin-3-yl)-5-(3-((4'-fluoro-5,5-dimethyl-3,4,5,6-tetrahydro-[1,1'-biphenyl]-2-yl)methyl)-3,8-diazabicyclo[3.2.1]octan-8-yl)isoindoline-1,3-dione (GT-05384)**

Referring to the method of Scheme 4, the target product (GT-05384) was prepared as white solid (10 mg, yield 15%). $^1$H NMR (400 MHz, DMSO-d6) δ 11.11 (s, 1H), 7.75 (d, *J* = 8.6 Hz, 1H), 7.34 (s, 1H), 7.18 (d, *J* = 8.2 Hz, 1H), 7.07 - 7.02 (m, 4H), 5.14 (dd, *J* = 12.8, 5.3 Hz, 1H), 4.66 (brs, 2H), 4.49 (brs, 1H), 3.11 - 3.02 (m, 2H), 2.90 - 2.85 (m, 2H), 2.77 - 2.72 (m, 2H), 2.68 - 2.63 (m, 1H), 2.62 - 2.55 (m, 2H), 2.21 - 2.14 (m, 3H), 2.09 - 2.04 (m, 2H), 1.89 - 1.86 (m, 2H), 1.43 - 1.36 (m, 3H), 0.87 (s, 6H). LCMS (ESI) calcd for $C_{34}H_{38}FN_4O_4^+$ [M+H]$^+$: 585.29, found, 585.3.

**Example 154: Preparation of 2-(2,6-dioxopiperidin-3-yl)-5-(8-((4'-fluoro-5,5-dimethyl-3,4,5,6-tetrahydro-[1,1'-biphenyl]-2-yl)methyl)-3,8-diazabicyclo[3.2.1]octan-3-yl)isoindoline-1,3-dione (GT-05385)**

Referring to the method of Scheme 4, the target product (GT-05385) was prepared as white solid (6 mg, yield 9%). $^1$H NMR (400 MHz, DMSO-d6) δ 11.09 (s, 1H), 7.77 - 7.72 (m, 1H), 7.34 - 7.33 (m, 1H), 7.26 - 7.24 (m, 3H), 7.21 - 7.19 (m, 2H), 5.09 (dd, *J* = 13.3, 6.4 Hz, 1H), 3.97 - 3.90 (m, 3H), 3.60 - 3.54(m, 1H), 3.53 - 3.50 (m, 2H), 2.91 - 2.85 (m, 2H), 2.62 - 2.57 (m, 2H), 2.41 - 2.33 (m, 2H), 2.15 - 2.11 (m, 2H), 2.05 - 1.95 (m, 4H), 1.81 - 1.71 (m, 2H), 1.53 - 1.50 (m, 2H), 1.32 - 1.26 (m, 2H), 1.00 (s, 6H). LCMS (ESI) calcd for $C_{34}H_{38}FN_4O_4^+$ [M+H]$^+$: 585.29, found, 585.3.

**Example 155: Preparation of 2-(2,6-dioxopiperidin-3-yl)-5-(3-((4'-fluoro-5,5-dimethyl-3,4,5,6-tetrahydro-[1,1'-biphenyl]-2-yl)methyl)-3,6-diazabicyclo[3.1.1]heptan-6-yl)isoindoline-1,3-dione (GT-05386)**

Referring to the method of Scheme 4, the target product (GT-05386) was prepared as white solid (12 mg, yield 19%). $^1$H NMR (400 MHz, DMSO-d6) δ 11.12 (s, 1H), 7.66 (d, *J* = 8.1 Hz, 1H), 7.03 - 6.93 (m, 5H), 6.76 (dd, *J* = 8.3, 1.4 Hz, 1H), 5.14 (d, *J* = 3.4 Hz, 1H), 4.61 - 4.50 (m, 2H), 3.59 - 3.49 (m, 2H), 3.15 - 3.01 (m, 3H), 2.93 - 2.88 (m, 2H), 2.78 - 2.72 (m, 1H), 2.67 - 2.62 (m, 1H), 2.40 - 2.33 (m, 2H), 2.23 - 2.18 (m, 2H), 2.14 - 2.02 (m, 2H), 1.98 (brs, 2H), 1.44 - 1.36 (m, 2H), 0.93 (s, 6H). LCMS (ESI) calcd for $C_{33}H_{36}FN_4O_4^+$ [M+H]$^+$: 571.27, found, 571.3.

**Example 156: Preparation of 2-(2,6-dioxopiperidin-3-yl)-5-(6-((4'-fluoro-5,5-dimethyl-3,4,5,6-tetrahydro-[1,1'-biphenyl]-2-yl)methyl)-3,6-diazabicyclo[3.1.1]heptan-3-yl)isoindoline-1,3-dione (GT-05387)**

Referring to the method of Scheme 4, the target product (GT-05387) was prepared as white solid (10 mg, yield 16%). $^1$H NMR (400 MHz, DMSO-d6) δ 11.09 (s, 1H), 7.28 - 7.09 (m, 5H), 6.92 - 6.74 (m, 2H), 5.10 (dd, *J* = 14.8, 10.2 Hz, 1H), 4.59 - 4.48 (m, 1H), 4.05 - 3.73 (m, 3H), 2.99 - 2.87 (m, 4H), 2.60 - 2.55 (m, 1H), 2.31 - 2.24 (m, 2H), 2.09 - 1.82 (m, 6H), 1.51 - 1.42 (m, 3H), 0.97 (s, 6H). LCMS (ESI) calcd for $C_{33}H_{36}FN_4O_4^+$ [M+H]$^+$: 571.27, found, 571.3.

**Example 157: Preparation of 2-(2,6-dioxopiperidin-3-yl)-5-(5-((4'-fluoro-5,5-dimethyl-3,4,5,6-tetrahydro-[1,1'-biphenyl]-2-yl)methyl)-2,5-diazabicyclo[2.2.2]octan-2-yl)isoindoline-1,3-dione (GT-05388)**

Referring to the method of Scheme 4, the target product (GT-05388) was prepared as white solid (3 mg, yield 5%). $^1$H NMR (400 MHz, DMSO-d6) δ 11.08 (s, 1H), 7.72 - 7.65 (m, 1H), 7.30 - 7.16 (m, 2H), 7.10 - 7.07 (m, 2H), 7.01 - 6.92 (m, 2H), 5.13 - 5.04 (m, 1H), 4.46 - 4.42 (m, 1H), 3.76 - 3.60 (m, 3H), 3.29 - 3.20 (m, 3H), 3.13 - 3.03 (m, 1H), 3.00 - 2.83 (m, 2H), 2.65 - 2.53 (m, 3H), 2.33 - 2.25 (m, 2H), 2.10 - 1.96 (m, 3H), 1.92 - 1.86 (m, 2H), 1.49 - 1.44 (m, 2H), 0.96 (s, 6H). LCMS (ESI) calcd for $C_{34}H_{38}FN_4O_4^+$ [M+H]$^+$: 585.29, found, 585.3.

**Example 158: Preparation of 3-(5-((3-((4'-chloro-5,5-dimethyl-3,4,5,6-tetrahydro-[1,1'-biphenyl]-2-yl) methyl)-3,8-diazabicyclo [3.2.1] octan-8-yl)methyl)-6-fluoro-1-oxoisoindolin-2-yl)piperidine-2,6-dione** (GT-05494)

Referring to the method of Scheme 1, the target product (GT-05494) was prepared as white solid (34 mg, yield 37.88 %). $^1$H NMR (400 MHz, DMSO-d6) δ 11.03 (s, 1H), 10.21 (s, 1H), 8.07 (d, J = 5.7 Hz, 1H), 7.66 (d, J = 8.6 Hz, 1H), 7.38 (t, J = 8.4 Hz, 2H), 7.07 (d, J = 7.9 Hz, 2H), 5.14 (dd, J = 13.3, 5.1 Hz, 1H), 4.55 - 4.19 (m, 4H), 3.94 (s, 2H), 3.63 (s, 3H), 3.59 - 3.53 (m, 2H), 3.00 - 2.86 (m, 2H), 2.61 (d, J = 16.5 Hz, 2H), 2.46 - 2.39 (m, 1H), 2.26 (s, 4H), 2.01 (dd, J = 15.1, 8.3 Hz, 4H), 1.43 (t, J = 6.2 Hz, 2H), 0.94 (s, 6H). LCMS (ESI) calcd for $C_{35}H_{41}ClFN_4O_3^+$ [M+H]$^+$: , 619.29, found, 619.3.

**Example 159: Preparation of 3-(5-((3-((4'-chloro-5,5-dimethyl-3,4,5,6-tetrahydro-[1,1'-biphenyl]-2-yl) methyl)-3,8-diazabicyclo [3.2.1] octan-8-yl)methyl)-7-fluoro-1-oxoisoindolin-2-yl)piperidine-2,6-dione (GT-05495)**

Referring to the method of Scheme 1, the target product (GT-05495) was prepared as white solid (21 mg, yield 23.40 %). $^1$H NMR (400 MHz, DMSO-d6) δ 11.02 (s, 1H), 10.33 (s, 1H), 7.73 (s, 2H), 7.37 (d, J = 8.2 Hz, 2H), 7.08 (s, 2H), 5.10 (dd, J = 13.3, 5.1 Hz, 1H), 4.45 (d, J = 33.3 Hz, 2H), 4.25 (s, 2H), 3.77 (d, J = 34.5 Hz, 2H), 3.64 (s, 3H), 2.99 - 2.71 (m, 3H), 2.63 (t, J = 17.6 Hz, 2H), 2.41 (ddd, J = 26.0, 13.1, 4.2 Hz, 2H), 2.22 (s, 4H), 1.97 (s, 4H), 1.42 (s, 2H), 0.94 (s, 6H). LCMS (ESI) calcd for $C_{35}H_{41}ClFN_4O_3^+$ [M+H]$^+$: 619.29, found, 619.3.

**Example 160: Preparation of 3-(6-((3-((4'-chloro-5,5-dimethyl-3,4,5,6-tetrahydro-[1,1'-biphenyl]-2-yl) methyl)-3,8-diazabicyclo [3.2.1] octan-8-yl)methyl)-1-oxoisoindolin-2-yl)piperidine-2,6-dione (GT-05459)**

Referring to the method of Scheme 1, the target product (GT-05459) was prepared as white solid (15 mg, yield GT-05459 %). $^1$H NMR (400 MHz, DMSO) δ 11.01 (s, 1H), 9.90 (s, 1H), 8.00 (d, J = 12.8 Hz, 1H), 7.90 (d, J = 7.7 Hz, 1H), 7.70 (d, J = 7.8 Hz, 1H), 7.38 (t, J = 10.4 Hz, 2H), 7.07 (t, J = 10.6 Hz, 2H), 5.13 (dd, J = 13.3, 5.1 Hz, 1H), 4.44 (dd, J = 51.7, 17.7 Hz, 2H), 4.27 (s, 2H), 3.77 (s, 2H), 3.44 (d, J = 7.1 Hz, 2H), 2.90 (ddd, J = 38.4, 21.8, 15.1 Hz, 3H), 2.61 (d, J = 17.1 Hz, 2H), 2.42 (dd, J = 13.2, 4.7 Hz, 1H), 2.22 (d, J = 26.8 Hz, 4H), 1.99 (dd, J = 14.1, 7.2 Hz, 4H), 1.42 (t, J = 6.2 Hz, 2H), 0.94 (d, J = 5.6 Hz, 6H). LCMS (ESI) calcd for $C_{35}H_{42}ClN_4O_3^+$ [M+H]$^+$: 601.29, found, 601.3.

**Example 161: Preparation of 5-((3-((4'-chloro-5,5-dimethyl-3,4,5,6-tetrahydro-[1,1'-biphenyl]-2-yl)methyl)-3,8-diazabicyclo[3.2.1]octan-8-yl)methyl)-2-(2,6-dioxopiperidin-3-yl)isoindoline-1,3-dione (GT-04719)**

Referring to the method of Scheme 1, the target product (GT-04719) was prepared as white solid (21 mg, yield 58.87 %). $^1$H NMR (400 MHz, DMSO) δ 11.14 (s, 1H), 10.17 (s, 1H), 8.25 (s, 1H), 8.15 (d, J = 7.7 Hz, 1H), 8.02 (d, J = 7.7 Hz, 1H), 7.37 (d, J = 8.2 Hz, 2H), 7.05 (d, J = 8.1 Hz, 2H), 5.18 (dd, J = 12.7, 5.3 Hz, 1H), 4.35 (s, 2H), 3.79 (s, 2H), 3.45 (s, 2H), 2.97 - 2.75 (m, 3H), 2.60 (t, J = 15.5 Hz, 3H), 2.35 (d, J = 16.3 Hz, 2H), 2.21 (d, J = 20.3 Hz, 4H), 2.10 - 2.04 (m, 1H), 1.97 (s, 3H), 1.42 (t, J = 6.3 Hz, 2H), 0.94 (s, 6H). LCMS (ESI) calcd for $C_{35}H_{40}ClN_4O_4^+$ [M+H]$^+$: 615.27, found, 615.3.

**Example 162: Preparation of 3-(5-((5-((4'-chloro-5,5-dimethyl-3,4,5,6-tetrahydro-[1,1'-biphenyl]-2-yl) methyl)-2,5-diazabicyclo [2.2.2] octan-2-yl)methyl)-6-fluoro-1-oxoisoindolin-2-yl)piperidine-2,6-dione (GT-05460)**

Referring to the method of Scheme 1, the target product (GT-05460) was prepared as white solid (8 mg, yield 17.83%). $^1$H NMR (400 MHz, DMSO) δ 11.02 (s, 1H), 8.04 - 7.35 (m, 4H), 7.19 (d, J = 7.6 Hz, 2H), 5.14 (dd, J = 12.8, 4.5 Hz, 1H), 4.56 - 4.35 (m, 2H), 3.75 (s, 7H), 3.17 (s, 2H), 2.91 (dd, J = 21.6, 8.9 Hz, 2H), 2.61 (d, J = 16.7 Hz, 1H), 2.37 (d, J = 33.4 Hz, 5H), 2.05 (d, J = 20.6 Hz, 4H), 1.80 (d, J = 36.2 Hz, 1H), 1.46 (s, 3H), 0.96 (s, 6H). LCMS (ESI) calcd for $C_{35}H_{41}ClFN_4O_3^+$ [M+H]$^+$: 619.29, found, 619.3.

**Example 163: Preparation of 3-(5-((5-((4'-chloro-5,5-dimethyl-3,4,5,6-tetrahydro-[1,1'-biphenyl]-2-yl) methyl)-2,5-diazabicyclo [2.2.2] octan-2-yl)methyl)-7-fluoro-1-oxoisoindolin-2-yl)piperidine-2,6-dione (GT-05781)**

Referring to the method of Scheme 1, the target product (GT-05781) was prepared as white solid (28 mg, yield 39.00%). $^1$H NMR (400 MHz, DMSO-d6) δ 11.02 (s, 1H), 7.37 (dd, J = 139.8, 48.1 Hz, 6H), 5.10 (dd, J = 13.2, 4.6 Hz, 1H), 4.55 - 4.37 (m, 2H), 3.75 (s, 10H), 3.04 - 2.87 (m, 2H), 2.61 (d, J = 17.8 Hz, 1H), 2.46 - 2.23 (m, 4H), 2.05 (d, *J* = 17.5 Hz, 4H), 1.64 - 1.30 (m, 3H), 0.96 (s, 6H). LCMS (ESI) calcd for $C_{35}H_{41}ClFN_4O_3^+$ [M+H]$^+$: 619.29, found, 619.3.

**Example 164: Preparation of 3-(4-((5-((4'-chloro-5,5-dimethyl-3,4,5,6-tetrahydro-[1,1'-biphenyl]-2-yl) methyl)-2,5-diazabicyclo [2.2.2] octan-2-yl)methyl)-1-oxoisoindolin-2-yl)piperidine-2,6-dione (GT-05461)**

Referring to the method of Scheme 1, the target product (GT-05461) was prepared as white solid (18 mg, yield 41.31%). $^1$H NMR (400 MHz, DMSO) δ 11.04 (s, 1H), 7.75 (s, 1H), 7.58 (s, 1H), 7.49 - 7.08 (m, 5H), 5.17 (s, 1H), 4.48 (s, 2H), 3.88 - 3.43 (m, 9H), 2.93 (dd, J = 21.6, 9.1 Hz, 2H), 2.70 - 2.57 (m, 2H), 2.33 (s, 4H), 2.02 (s, 4H), 1.46 (s, 3H), 0.96 (s, 6H). LCMS (ESI) calcd for $C_{35}H_{42}ClN_4O_3^+$ [M+H]$^+$: 601.29, found, 601.3.

**Example 165: Preparation of 3-(6-((5-((4'-chloro-5,5-dimethyl-3,4,5,6-tetrahydro-[1,1'-biphenyl]-2-yl) methyl)-2,5-diazabicyclo [2.2.2] octan-2-yl)methyl)-1-oxoisoindolin-2-yl)piperidine-2,6-dione (GT-05485)**

Referring to the method of Scheme 1, the target product (GT-05485) was prepared as white solid (5 mg, yield 9.56%). $^1$H NMR (400 MHz, DMSO) δ 11.00 (s, 1H), 8.10 - 7.60 (m, 3H), 7.43 (s, 2H), 7.18 (d, J = 7.4 Hz, 2H), 5.12 (d, J = 8.1 Hz, 1H), 4.61 - 4.29 (m, 4H), 3.45 (d, J = 7.0 Hz, 9H), 2.91 (s, 2H), 2.61 (d, J = 17.1 Hz, 1H), 2.42 (d, J = 13.0 Hz, 1H), 2.26 (s, 2H), 2.12 - 1.86 (m, 4H), 1.45 (s, 3H), 0.95 (s, 6H). LCMS (ESI) calcd for $C_{35}H_{42}ClN_4O_3^+$ [M+H]$^+$: 601.29, found, 602.3.

**Example 166: Preparation of 5-((5-((4'-chloro-5,5-dimethyl-3,4,5,6-tetrahydro-[1,1'-biphenyl]-2-yl)methyl)-2,5-diazabicyclo[2.2.2]octan-2-yl)methyl)-2-(2,6-dioxopiperidin-3-yl)isoindoline-1,3-dione (GT-05463)**

Referring to the method of Scheme 1, the target product (GT-05463) was prepared as white solid (15 mg, yield 33.64%). $^1$H NMR (400 MHz, DMSO) δ 11.15 (s, 1H), 8.41 - 8.07 (m, 1H), 7.97 (s, 1H), 7.90 - 7.74 (m, 1H), 7.42 (d, J = 20.2 Hz, 2H), 7.18 (d, J = 7.7 Hz, 2H), 5.28 - 5.13 (m, 1H), 4.69 (s, 2H), 3.94 - 3.45 (m, 7H), 2.90 (dd, J = 21.9, 9.7 Hz, 2H), 2.59 (dd, J = 24.5, 14.6 Hz, 3H), 2.35 - 1.91 (m, 7H), 1.46 (s, 3H), 0.96 (s, 6H). LCMS (ESI) calcd for $C_{35}H_{40}ClN_4O_4^+$ [M+H]$^+$: 615.27, found, 615.3.

**Example 167: Preparation of 3-(4-fluoro-5-((4-((4'-fluoro-3,4,5,6-tetrahydro-[1,1'-biphenyl]-2-yl)methyl)piperazin-1-yl)methyl)-1-oxoisoindolin-2-yl)piperidine-2,6-dione (GT-**05466)

Referring to the method of Scheme 1, the target product (GT-05466) was prepared as white solid (18 mg, yield 36.01%). $^1$H NMR (400 MHz, DMSO) δ 11.03 (s, 1H), 7.75 (s, 1H), 7.69 - 7.62 (m, 1H), 7.29 - 7.11 (m, 4H), 5.14 (dd, J = 13.3,

5.1 Hz, 1H), 4.59 - 4.39 (m, 2H), 4.23 (s, 2H), 3.56 (s, 3H), 3.33 - 3.06 (m, 5H), 2.91 (ddd, J = 32.4, 18.9, 12.0 Hz, 3H), 2.61 (d, J = 17.4 Hz, 1H), 2.46 (dd, J = 13.2, 4.6 Hz, 1H), 2.27 (d, J = 26.9 Hz, 4H), 2.06 - 1.96 (m, 1H), 1.68 (s, 4H). LCMS (ESI) calcd for $C_{31}H_{35}F_2N_4O_3^+$ $[M+H]^+$: 549.27, found, 549.3.

**Example 168: Preparation of 3-(6-((4-((4'-fluoro-3,4,5,6-tetrahydro-[1,1'-biphenyl]-2-yl)methyl)piperazin-1-yl)methyl)-1-oxoisoindolin-2-yl)piperidine-2,6-dione (GT-05465)**

Referring to the method of Scheme 1, the target product (GT-05465) was prepared as white solid (13 mg, yield 26.89%). $^1$H NMR (400 MHz, DMSO) δ 11.01 (s, 1H), 7.95 (s, 1H), 7.82 (d, J = 7.7 Hz, 1H), 7.70 (d, J = 7.8 Hz, 1H), 7.18 (p, J = 8.8 Hz, 4H), 5.13 (dd, J = 13.3, 5.0 Hz, 1H), 4.44 (dd, J = 52.5, 17.8 Hz, 4H), 3.60 (dd, J = 8.6, 4.7 Hz, 7H), 3.01 - 2.79 (m, 3H), 2.61 (d, J = 17.4 Hz, 1H), 2.55 - 2.52 (m, 1H), 2.42 (dd, J = 13.1, 4.7 Hz, 1H), 2.26 (d, J = 25.5 Hz, 4H), 2.09 - 1.94 (m, 1H), 1.67 (s, 4H). LCMS (ESI) calcd for $C_{31}H_{36}FN_4O_3^+$ $[M+H]^+$: 531.28, found, 531.3.

**Example 169: Preparation of 2-(2,6-dioxopiperidin-3-yl)-5-((4-((4'-fluoro-3,4,5,6-tetrahydro-[1,1'-biphenyl]-2-yl)methyl)piperazin-1-yl)methyl)isoindoline-1,3-dione (GT-05464)**

Referring to the method of Scheme 1, the target product (GT-05464) was prepared as white solid (21 mg, yield 42.32%). $^1$H NMR (400 MHz, DMSO) δ 11.15 (s, 1H), 8.12 (s, 1H), 7.99 (t, J = 6.5 Hz, 2H), 7.24 - 7.12 (m, 4H), 5.18 (dd, J = 12.8, 5.4 Hz, 1H), 4.33 (s, 2H), 3.52 (s, 3H), 3.17 (s, 6H), 2.96 - 2.84 (m, 2H), 2.58 (dd, J = 24.1, 12.4 Hz, 2H), 2.27 (d, J = 26.4 Hz, 4H), 2.11 - 2.03 (m, 1H), 1.68 (s, 4H). LCMS (ESI) calcd for $C_{31}H_{34}FN_4O_4^+$ $[M+H]^+$: 545.26, found, 545.3.

**Example 170: Preparation of 3-(4-fluoro-5-((4-(4'-fluoro-3,4,5,6-tetrahydro-[1,1'-biphenyl]-2-carbonyl)piperazin-1-yl)methyl)-1-oxoisoindolin-2-yl)piperidine-2,6-dione (GT-05472)**

Referring to the method of Scheme 1, the target product (GT-05472) was prepared as white solid (37mg, yield 47.41%). $^1$H NMR (400 MHz, DMSO) δ 11.04 (s, 1H), 7.85 (s, 1H), 7.67 (d, J = 7.7 Hz, 1H), 7.17 (d, J = 45.8 Hz, 4H), 5.16 (dd, J = 12.5, 4.3 Hz, 1H), 4.69 - 4.30 (m, 4H), 3.70 (s, 6H), 3.34 (s, 1H), 3.18 - 2.89 (m, 3H), 2.62 (d, J = 15.7 Hz, 2H), 2.37 (s, 1H), 2.18 - 1.99 (m, 3H), 1.67 (d, J = 30.3 Hz, 4H). LCMS (ESI) calcd for $C_{31}H_{33}F_2N_4O_4^+$ $[M+H]^+$: 563.25, found, 563.3.

**Example 171: Preparation of 3-(6-fluoro-5-((4-(4'-fluoro-3,4,5,6-tetrahydro-[1,1'-biphenyl]-2-carbonyl)piperazin-1-yl)methyl)-1-oxoisoindolin-2-yl)piperidine-2,6-dione (GT-05473)**

Referring to the method of Scheme 1, the target product (GT-05473) was prepared as white solid (25mg, yield 32.03%). $^1$H NMR (400 MHz, DMSO) δ 11.72 (s, 1H), 11.03 (s, 1H), 10.82 (s, 1H), 7.86 (d, J = 30.8 Hz, 1H), 7.66 (d, J = 8.4 Hz, 1H), 7.17 (d, J = 43.3 Hz, 4H), 5.13 (s, 1H), 4.41 (dd, J = 50.4, 17.4 Hz, 5H), 3.74 (s, 1H), 3.38 - 3.24 (m, 4H), 2.92 (s, 4H), 2.61 (d, J = 16.4 Hz, 2H), 2.42 (s, 1H), 2.04 (dd, J = 22.4, 15.1 Hz, 3H), 1.71 (s, 4H). LCMS (ESI) calcd for $C_{31}H_{33}F_2N_4O_4^+$ $[M+H]^+$: 563.25, found, 563.3.

**Example 172: Preparation of 3-(7-fluoro-5-((4-(4'-fluoro-3,4,5,6-tetrahydro-[1,1'-biphenyl]-2-carbonyl)piperazin-1-yl)methyl)-1-oxoisoindolin-2-yl)piperidine-2,6-dione (GT-05474)**

Referring to the method of Scheme 1, the target product (GT-05474) was prepared as white solid (31 mg, yield 39.72%). $^1$H NMR (400 MHz, DMSO) δ 12.12 (s, 1H), 11.53 (s, 1H), 11.03 (s, 1H), 7.57 (d, J = 27.2 Hz, 2H), 7.31 - 6.99 (m, 4H), 5.11 (dd, J = 13.5, 4.3 Hz, 1H), 4.58 - 4.32 (m, 4H), 3.86 - 3.67 (m, 4H), 3.13 (dd, J = 48.6, 14.1 Hz, 2H), 2.97 - 2.87 (m, 2H), 2.71 (d, J = 2.8 Hz, 1H), 2.61 (d, J = 18.3 Hz, 2H), 2.40 (dd, J = 13.7, 6.9 Hz, 1H), 2.23 - 1.95 (m, 4H), 1.68 (d, J = 30.3 Hz, 4H). LCMS (ESI) calcd for $C_{31}H_{33}F_2N_4O_4^+$ $[M+H]^+$: 563.25, found, 563.3.

**Example 173: Preparation of 3-(6-((4-(4'-fluoro-3,4,5,6-tetrahydro-[1,1'-biphenyl]-2-carbonyl)piperazin-1-yl)methyl)-1-oxoisoindolin-2-yl)piperidine-2,6-dione (GT-05470)**

Referring to the method of Scheme 1, the target product (GT-05470) was prepared as white solid (15 mg, yield 19.85%). $^1$H NMR (400 MHz, DMSO) δ 11.87 (s, 1H), 11.13 (s, 1H), 11.01 (s, 1H), 7.99 - 7.67 (m, 3H), 7.14 (dd, J = 45.3, 23.5 Hz, 4H), 5.13 (d, J = 13.0 Hz, 1H), 4.54 - 4.30 (m, 4H), 3.84 - 3.71 (m, 5H), 3.41 (d, J = 70.1 Hz, 1H), 3.12 (d, J = 49.7 Hz, 2H), 2.98 - 2.87 (m, 2H), 2.65 (d, J = 14.2 Hz, 1H), 2.40 (d, J = 19.0 Hz, 1H), 2.19 - 1.97 (m, 4H), 1.67 (d, J = 32.1 Hz, 4H). LCMS (ESI) calcd for $C_{31}H_{34}FN_4O_4^+$ $[M+H]^+$: 545.26, found, 545.3.

**Example 174: Preparation of 3-(4-((4-(4'-fluoro-3,4,5,6-tetrahydro-[1,1'-biphenyl]-2-carbonyl)piperazin-1-yl)methyl)-1-oxoisoindolin-2-yl)piperidine-2,6-dione (GT-05471)**

Referring to the method of Scheme 1, the target product (GT-05471) was prepared as white solid (26 mg, yield 34.41%). $^1$H NMR (400 MHz, DMSO) δ 12.01 (s, 1H), 11.31 (s, 1H), 11.07 (s, 1H), 7.99 - 7.74 (m, 2H), 7.61 (t, J = 7.5 Hz, 1H), 7.30 - 7.07 (m, 4H), 5.16 (dd, J = 8.7, 4.5 Hz, 1H), 4.82 (d, J = 19.6 Hz, 1H), 4.63 - 4.18 (m, 5H), 3.88 (s, 2H), 3.50 (s, 2H), 3.31 - 2.91 (m, 5H), 2.73 - 2.66 (m, 1H), 2.38 - 2.32 (m, 1H), 2.07 (d, J = 21.6 Hz, 3H), 1.68 (d, J = 30.9 Hz, 4H). LCMS (ESI) calcd for $C_{31}H_{34}FN_4O_4^+$ [M+H]$^+$: 545.26, found, 545.3.

**Example 175: Preparation of 2-(2,6-dioxopiperidin-3-yl)-5-((4-(4'-fluoro-3,4,5,6-tetrahydro-[1,1'-biphenyl]-2-carbonyl)piperazin-1-yl)methyl)isoindoline-1,3-dione (GT-05468)**

Referring to the method of Scheme 1, the target product (GT-05468) was prepared as white solid (35 mg, yield 45.17%). $^1$H NMR (400 MHz, DMSO) δ 11.94 (s, 1H), 11.15 (s, 1H), 8.06 (d, J = 42.6 Hz, 3H), 7.17 (d, J = 40.3 Hz, 4H), 5.17 (dd, J = 15.0, 10.4 Hz, 1H), 4.38 (d, J = 70.8 Hz, 3H), 3.75 (s, 1H), 3.41 (s, 2H), 3.12 (d, J = 40.6 Hz, 5H), 2.98 - 2.86 (m, 2H), 2.62 (d, J = 17.4 Hz, 2H), 2.35 (d, J = 15.5 Hz, 1H), 2.13 - 1.95 (m, 3H), 1.67 (d, J = 30.0 Hz, 4H). LCMS (ESI) calcd for $C_{31}H_{32}FN_4O_5^+$ [M+H]$^+$: 559.24, found, 559.3.

**Example 176: Preparation of 3-(4-fluoro-5-((3-(4'-fluoro-3,4,5,6-tetrahydro-[1,1'-biphenyl]-2-carbonyl)-3,6-diazabicyclo[3.1.1]heptan-6-yl)methyl)-1-oxoisoindolin-2-yl)piperidine-2,6-dione (GT-05475)**

Referring to the method of Scheme 1, the target product (GT-05475) was prepared as white solid (mg, yield %). $^1$H NMR (400 MHz, DMSO) δ 11.02 (s, 1H), 7.97 - 7.57 (m, 2H), 7.31 (dd, J = 39.5, 22.3 Hz, 2H), 7.15 (dd, J = 19.0, 10.2 Hz, 2H), 5.18 - 5.09 (m, 1H), 4.83 - 4.02 (m, 6H), 3.90 - 3.41 (m, 3H), 3.33 - 2.87 (m, 4H), 2.61 (d, J = 16.8 Hz, 2H), 2.46 - 2.33 (m, 2H), 2.18 - 2.00 (m, 3H), 1.72 (s, 4H). LCMS (ESI) calcd for $C_{32}H_{33}F_2N_4O_4^+$ [M+H]$^+$: 575.25, found, 575.3.

**Example 177: Preparation of 3-(6-fluoro-5-((3-(4'-fluoro-3,4,5,6-tetrahydro-[1,1'-biphenyl]-2-carbonyl)-3,6-diazabicyclo[3.1.1]heptan-6-yl)methyl)-1-oxoisoindolin-2-yl)piperidine-2,6-dione (GT-05476)**

Referring to the method of Scheme 1, the target product (GT-05476) was prepared as white solid (25 mg, yield 29.04%). $^1$H NMR (400 MHz, DMSO) δ 11.02 (s, 1H), 10.05 (d, J = 219.9 Hz, 1H), 7.37 (qdd, J = 71.3, 62.4, 54.4 Hz, 6H), 5.13 (dd, J = 9.2, 3.8 Hz, 1H), 4.79 - 3.92 (m, 5H), 3.81 - 3.45 (m, 2H), 3.14 (s, 4H), 2.93 (dd, J = 21.8, 8.6 Hz, 1H), 2.61 (d, J = 15.7 Hz, 2H), 2.47 - 2.25 (m, 3H), 2.07 (d, J = 17.1 Hz, 3H), 1.72 (s, 4H). LCMS (ESI) calcd for $C_{32}H_{33}F_2N_4O_4^+$ [M+H]$^+$: 575.25, found, 575.3.

**Example 178: Preparation of 3-(7-fluoro-5-((3-(4'-fluoro-3,4,5,6-tetrahydro-[1,1'-biphenyl]-2-carbonyl)-3,6-diazabicyclo[3.1.1]heptan-6-yl)methyl)-1-oxoisoindolin-2-yl)piperidine-2,6-dione (GT-05477)**

Referring to the method of Scheme 1, the target product (GT-05477) was prepared as white solid (21 mg, yield 27.44%). $^1$H NMR (400 MHz, DMSO) δ 11.62 (s, 1H), 11.02 (s, 1H), 9.74 (s, 1H), 7.79 - 7.43 (m, 2H), 7.38 - 7.02 (m, 4H), 5.09 (dd, J = 13.2, 4.9 Hz, 1H), 4.88 - 4.00 (m, 6H), 3.98 - 3.38 (m, 4H), 3.32 - 2.85 (m, 4H), 2.65 - 2.54 (m, 2H), 2.33 (s, 1H), 2.07 (dd, J = 30.9, 6.6 Hz, 3H), 1.72 (s, 4H). LCMS (ESI) calcd for $C_{32}H_{33}F_2N_4O_4^+$ [M+H]$^+$: 575.25, found, 575.3.

**Example 179: Preparation of 3-(4-((3-(4'-fluoro-3,4,5,6-tetrahydro-[1,1'-biphenyl]-2-carbonyl)-3,6-diazabicyclo[3.1.1]heptan-6-yl)methyl)-1-oxoisoindolin-2-yl)piperidine-2,6-dione (GT-05478)**

Referring to the method of Scheme 1, the target product (GT-05478) was prepared as white solid (32 mg, yield 38.37%). $^1$H NMR (400 MHz, DMSO-d6) δ 11.03 (d, J = 20.3 Hz, 1H), 9.50 (s, 1H), 8.01 - 6.97 (m, 7H), 5.19 (s, 1H), 4.91 - 4.01 (m, 5H), 3.74 (s, 4H), 3.22 - 2.87 (m, 2H), 2.79 - 2.53 (m, 3H), 2.40 (d, J = 15.7 Hz, 3H), 2.07 (d, J = 22.4 Hz, 3H), 1.72 (s, 4H). LCMS (ESI) calcd for $C_{32}H_{34}FN_4O_4^+$ [M+H]$^+$: 557.26, found, 557.3.

**Example 180: Preparation of 3-(6-((3-(4'-fluoro-3,4,5,6-tetrahydro-[1,1'-biphenyl]-2-carbonyl)-3,6-diazabicyclo[3.1.1]heptan-6-yl)methyl)-1-oxoisoindolin-2-yl)piperidine-2,6-dione (GT-05479)**

Referring to the method of Scheme 1, the target product (GT-05479) was prepared as white solid (15 mg, yield 17.96%). $^1$H NMR (400 MHz, DMSO) δ 11.39 (s, 1H), 11.01 (s, 1H), 9.55 (s, 1H), 8.01 - 7.84 (m, 1H), 7.73 - 7.61 (m, 1H), 7.47 (s, 1H), 7.38 - 7.15 (m, 4H), 5.12 (dd, J = 13.3, 5.0 Hz, 1H), 4.84 - 4.11 (m, 6H), 4.12 - 3.78 (m, 2H), 3.66 (d, J = 53.4 Hz, 3H), 3.30 - 3.06 (m, 2H), 2.96 - 2.86 (m, 1H), 2.63 (s, 1H), 2.41 (dd, J = 12.9, 4.3 Hz, 1H), 2.10 (dd, J = 68.6, 38.6 Hz, 4H),

1.72 (s, 4H). LCMS (ESI) calcd for $C_{32}H_{34}FN_4O_4^+$ $[M+H]^+$: 557.26, found, 557.3.

**Example 181: Preparation of 3-(7-((3-(4'-fluoro-3,4,5,6-tetrahydro-[1,1'-biphenyl]-2-carbonyl)-3,6-diazabicyclo [3.1.1]heptan-6-yl)methyl)-1-oxoisoindolin-2-yl)piperidine-2,6-dione (GT-05480)**

Referring to the method of Scheme 1, the target product (GT-05480) was prepared as white solid (22 mg, yield 29.72%). $^1H$ NMR (400 MHz, DMSO) δ 11.04 (s, 1H), 7.95 - 7.58 (m, 3H), 7.28 (d, J = 23.8 Hz, 2H), 7.14 (dd, J = 18.2, 9.8 Hz, 2H), 5.13 (d, J = 14.6 Hz, 1H), 4.95 - 3.99 (m, 6H), 3.94 - 3.39 (m, 4H), 2.92 (s, 5H), 2.63 (d, J = 17.0 Hz, 1H), 2.42 (s, 1H), 2.06 (d, J = 24.3 Hz, 3H), 1.71 (s, 4H). LCMS (ESI) calcd for $C_{32}H_{34}FN_4O_4^+$ $[M+H]^+$: 557.26, found, 557.3.

**Example 182: Preparation of 2-(2,6-dioxopiperidin-3-yl)-5-((3-(4'-fluoro-3,4,5,6-tetrahydro-[1,1'-biphenyl]-2-carbonyl)-3,6-diazabicyclo[3.1.1]heptan-6-yl)methyl)isoindoline-1,3-dione (GT-05481)**

Referring to the method of Scheme 1, the target product (GT-05481) was prepared as white solid (30 mg, yield 31.59%). $^1H$ NMR (400 MHz, DMSO) δ 11.65 - 11.25 (m, 1H), 11.14 (s, 1H), 9.60 (s, 1H), 8.37 - 7.12 (m, 7H), 5.26 - 5.08 (m, 1H), 4.57 (d, J = 107.3 Hz, 3H), 3.60 (s, 6H), 3.17 (s, 2H), 2.95 - 2.82 (m, 1H), 2.61 (d, J = 18.7 Hz, 2H), 2.38 (s, 1H), 2.13 (t, J = 28.9 Hz, 3H), 1.72 (s, 4H). LCMS (ESI) calcd for $C_{32}H_{32}FN_4O_5^+$ $[M+H]^+$: 571.24, found, 571.3.

**Example 183: Preparation of 5-((4-((4'-chloro-[1,1'-biphenyl]-2-yl)methyl)piperazin-1-yl)methyl)-2-(2,6-dioxopiperidin-3-yl)isoindoline-1,3-dione (GT-05497)**

Referring to the method of Scheme 1, the target product (GT-05497) was prepared as white solid (40 mg, yield 45.77%). $^1H$ NMR (400 MHz, DMSO-d6) δ 11.15 (s, 1H), 8.17 (s, 1H), 8.02 (dd, J = 27.5, 7.6 Hz, 2H), 7.94 (d, J = 7.8 Hz, 1H), 7.56 - 7.47 (m, 4H), 7.39 (d, J = 8.4 Hz, 2H), 7.30 (dd, J = 5.6, 3.4 Hz, 1H), 5.22 - 5.12 (m, 1H), 4.61 - 4.08 (m, 4H), 3.29 (s, 9H), 2.59 (s, 1H), 2.54 (s, 1H), 2.07 (dd, J = 9.2, 3.6 Hz, 1H). LCMS (ESI) calcd for $C_{31}H_{30}ClN_4O_4^+$ $[M+H]^+$: 557.20, found, 557.2.

**Example 184: Preparation of 3-(5-(4-((4-(4-chlorophenyl)-6,6-dimethyl-5,6-dihydro-2H-pyran-3-yl)methyl)piperazine-1-carbonyl)-1-oxoisoindolin-2-yl)piperidine-2,6-dione (GT-05673)**

Referring to the methods of Scheme 6 and Example 76, the target product (GT-05673) was prepared as white solid (15 mg, yield 20.36%). $^1H$ NMR (400 MHz, DMSO-d6) δ 11.01 (s, 1H), 7.86 - 7.77 (m, 1H), 7.69 - 7.57 (m, 2H), 7.48 (ddd, J = 17.4, 6.2, 2.9 Hz, 3H), 7.23 (d, J = 8.4 Hz, 1H), 5.15 (dt, J = 8.0, 5.7 Hz, 1H), 4.46 (dt, J = 34.6, 17.7 Hz, 4H), 3.79 (d, J = 125.2 Hz, 6H), 3.23 - 3.07 (m, 2H), 2.91 (dd, J = 20.6, 7.9 Hz, 4H), 2.61 (d, J = 16.5 Hz, 1H), 2.44 - 2.36 (m, 1H), 2.22 (s, 1H), 2.03 (dd, J = 16.2, 9.3 Hz, 1H), 1.47 (d, J = 20.0 Hz, 3H), 1.22 (s, 3H). LCMS (ESI) calcd for $C_{32}H_{36}ClN_4O_5^+$ $[M+H]^+$: 591.24, found, 591.3.

**Example 185: Preparation of 5-(4-((4-(4-chlorophenyl)-6,6-dimethyl-5,6-dihydro-2H-pyran-3-yl)methyl)piperazine-1-carbonyl)-2-(2,6-dioxopiperidin-3-yl)isoindoline-1,3-dione (GT-05674)**

Referring to the methods of Scheme 6 and Example 76, the target product (GT-05674) was prepared as white solid (22 mg, yield 29.17%). $^1H$ NMR (400 MHz, DMSO-d6) δ 11.15 (s, 1H), 10.11 (s, 1H), 8.05 - 7.84 (m, 3H), 7.49 (d, J = 8.0 Hz, 2H), 7.23 (d, J = 8.0 Hz, 2H), 5.19 (dd, J = 12.8, 5.4 Hz, 1H), 4.34 (d, J = 60.7 Hz, 2H), 3.60 (d, J = 40.9 Hz, 5H), 3.33 - 3.17 (m, 4H), 2.99 - 2.85 (m, 2H), 2.58 (dd, J = 23.8, 11.5 Hz, 2H), 2.20 (s, 2H), 2.10 - 2.02 (m, 1H), 1.24 (d, J = 17.4 Hz, 6H). LCMS (ESI) calcd for $C_{32}H_{34}ClN_4O_6^+$ $[M+H]^+$: 605.22, found, 605.3.

**Example 186: Preparation of 3-(5-((1-((4'-chloro-5,5-dimethyl-3,4,5,6-tetrahydro-[1,1'-biphenyl]-2-yl)methyl)piperidin-4-yl)amino)-1-oxoisoindolin-2-yl)piperidine-2,6-dione (GT-05782)**

Referring to the method of Scheme 4, the target product (GT-05782) was prepared as white solid (20 mg, yield 21.62%). $^1H$ NMR (400 MHz, DMSO-d6) δ 10.92 (s, 1H), 9.99 (d, J = 44.0 Hz, 1H), 7.43 (dd, J = 31.8, 8.3 Hz, 3H), 7.16 (d, J = 8.4 Hz, 2H), 6.67 (d, J = 9.4 Hz, 2H), 5.01 (dd, J = 13.3, 5.1 Hz, 1H), 4.19 (d, J = 35.0 Hz, 2H), 3.52 (d, J = 5.1 Hz, 4H), 3.32 (d, J = 11.6 Hz, 4H), 2.89 (td, J = 13.0, 6.7 Hz, 1H), 2.70 (d, J = 11.9 Hz, 1H), 2.62 - 2.54 (m, 1H), 2.34 (d, J = 7.7 Hz, 3H), 2.05 (s, 5H), 1.48 (s, 2H), 0.97 (s, 6H). LCMS (ESI) calcd for $C_{33}H_{40}ClN_4O_3^+$ $[M+H]^+$: 575.28, found, 575.28.

**Example 187: Preparation of 3-(5-((1-(4'-chloro-5,5-dimethyl-3,4,5,6-tetrahydro-[1,1'-biphenyl]-2-carbonyl)piperidin-4-yl)amino)-1-oxoisoindolin-2-yl)piperidine-2,6-dione (GT-05783)**

Referring to the method of Scheme 6, the target product (GT-05783) was prepared as white solid (25 mg, yield 28.17%). $^1$H NMR (400 MHz, DMSO-d6) δ 10.91 (s, 1H), 7.37 (t, J = 8.5 Hz, 3H), 7.26 (dd, J = 16.5, 8.2 Hz, 2H), 6.64 (d, J = 15.3 Hz, 2H), 5.00 (dd, J = 13.3, 4.9 Hz, 1H), 4.31 - 4.02 (m, 3H), 3.50 (s, 4H), 3.35 - 3.21 (m, 2H), 3.07 (s, 1H), 2.93 - 2.79 (m, 1H), 2.57 (d, J = 17.0 Hz, 2H), 2.39 - 2.30 (m, 2H), 2.09 (d, J = 17.3 Hz, 1H), 2.00 - 1.87 (m, 2H), 1.71 (d, J = 48.3 Hz, 2H), 1.45 (d, J = 6.1 Hz, 2H), 1.20 (s, 1H), 0.98 (d, J = 11.4 Hz, 6H). LCMS (ESI) calcd for $C_{33}H_{38}ClN_4O_4^+$ [M+H]$^+$: 589.26, found, 589.3.

**Example 188: Preparation of 3-(5-((1-(4'-chloro-[1,1'-biphenyl]-2-carbonyl)piperidin-4-yl)amino)-1-oxoisoindolin-2-yl)piperidine-2,6-dione (GT-05784)**

Referring to the method of Scheme 6, the target product (GT-05784) was prepared as white solid (25 mg, yield 26.10%). $^1$H NMR (400 MHz, DMSO-d6) δ 10.91 (s, 1H), 7.67 - 7.23 (m, 9H), 6.66 (d, J = 8.8 Hz, 2H), 5.00 (dd, J = 13.3, 5.0 Hz, 1H), 4.22 (s, 3H), 3.56 (s, 2H), 3.20 (d, J = 12.7 Hz, 1H), 3.01 (t, J = 12.1 Hz, 1H), 2.93 - 2.81 (m, 1H), 2.77 - 2.53 (m, 2H), 2.33 (qd, J = 13.2, 4.4 Hz, 1H), 2.02 - 1.80 (m, 2H), 1.61 (dd, J = 47.3, 12.0 Hz, 1H), 1.39 - 1.13 (m, 1H), 1.09 - 0.35 (m, 1H). LCMS (ESI) calcd for $C_{31}H_{30}ClN_4O_4^+$ [M+H]$^+$: 557.20, found, 557.2.

**Example 189: Preparation of 3-(5-((1-((4'-fluoro-5,5-dimethyl-3,4,5,6-tetrahydro-[1,1'-biphenyl]-2-yl)methyl)piperidin-4-yl)amino)-1-oxoisoindolin-2-yl)piperidine-2,6-dione (GT-05785)**

Referring to the method of Scheme 4, the target product (GT-05785) was prepared as white solid (20 mg, yield 20.79%). $^1$H NMR (400 MHz, DMSO-d6) δ 10.92 (s, 1H), 9.99 (d, J = 36.0 Hz, 1H), 7.39 (d, J = 8.2 Hz, 1H), 7.31 - 7.13 (m, 4H), 6.68 (d, J = 8.9 Hz, 2H), 5.01 (dd, J = 13.2, 5.1 Hz, 1H), 4.19 (dd, J = 51.6, 16.8 Hz, 2H), 3.52 (d, J = 4.7 Hz, 4H), 3.32 (d, J = 11.6 Hz, 4H), 2.90 (dd, J = 22.4, 8.8 Hz, 1H), 2.68 (d, J = 12.0 Hz, 3H), 2.34 (d, J = 7.6 Hz, 2H), 2.07 - 1.87 (m, 5H), 1.48 (t, J = 6.0 Hz, 2H), 0.97 (s, 6H). LCMS (ESI) calcd for $C_{33}H_{40}FN_4O_3^+$ [M+H]$^+$: 559.31, found, 559.3.

**Example 190: Preparation of 3-(5-((1-(4'-fluoro-5,5-dimethyl-3,4,5,6-tetrahydro-[1,1'-biphenyl]-2-carbonyl)piperidin-4-yl)amino)-1-oxoisoindolin-2-yl)piperidine-2,6-dione (GT-05786)**

Referring to the method of Scheme 6, the target product (GT-05786) was prepared as white solid (25 mg, yield 27.10%). $^1$H NMR (400 MHz, DMSO-d6) δ 10.92 (s, 1H), 7.36 (d, J = 8.7 Hz, 1H), 7.33 - 7.21 (m, 2H), 7.14 (t, J = 8.8 Hz, 2H), 6.65 (d, J = 18.4 Hz, 2H), 5.00 (dd, J = 13.3, 5.0 Hz, 1H), 4.34 - 4.09 (m, 3H), 3.32 (s, 4H), 3.07 - 2.74 (m, 2H), 2.57 (d, J = 14.1 Hz, 2H), 2.41 - 2.31 (m, 2H), 2.08 (d, J = 17.7 Hz, 1H), 1.92 (dd, J = 14.8, 10.2 Hz, 2H), 1.82 - 1.57 (m, 2H), 1.51 - 1.37 (m, 2H), 1.19 (s, 1H), 0.99 (d, J = 12.1 Hz, 6H). LCMS (ESI) calcd for $C_{33}H_{38}FN_4O_4^+$ [M+H]$^+$: 573.29, found, 573.3.

**Example 191: Preparation of 3-(5-((1-(4'-chloro-[1,1'-biphenyl]-2-carbonyl)piperidin-4-yl)methyl)-1-oxoisoindolin-2-yl)piperidine-2,6-dione (GT-05467)**

Referring to the method of Scheme 6, the target product (GT-05467) was prepared as white solid (24 mg, yield 36.84%). $^1$H NMR (400 MHz, DMSO) δ 10.98 (s, 1H), 7.61 (d, J = 8.0 Hz, 1H), 7.51 - 7.44 (m, 6H), 7.35 (d, J = 8.9 Hz, 2H), 7.28 (d, J = 7.9 Hz, 2H), 5.09 (dd, J = 13.1, 4.9 Hz, 1H), 4.52 - 4.29 (m, 3H), 3.44 (dd, J = 14.0, 7.0 Hz, 2H), 3.19 (s, 2H), 3.06 - 2.86 (m, 2H), 2.64 - 2.53 (m, 2H), 2.38 (d, J = 12.7 Hz, 2H), 2.00 (s, 1H), 1.73 - 1.47 (m, 2H), 1.23 - 0.99 (m, 2H). LCMS (ESI) calcd for $C_{32}H_{31}ClN_3O_4^+$ [M+H]$^+$: 556.20, found, 556.3.

**Example 192: Preparation of 3-(5-((1-(4'-chloro-[1,1'-biphenyl]-2-carbonyl)azetidin-3-ylidene)methyl)-1-oxoisoindolin-2-yl)piperidine-2,6-dione** (GT-05482)

Referring to the method of Scheme 6, the target product (GT-05482) was prepared as white solid (22 mg, yield 32.55%). $^1$H NMR (400 MHz, DMSO) δ 10.98 (d, J = 4.5 Hz, 1H), 7.66 (dd, J = 15.8, 7.9 Hz, 1H), 7.56 - 7.43 (m, 7H), 7.31 (dd, J = 38.8, 31.2 Hz, 2H), 6.48 (d, J = 37.5 Hz, 1H), 5.10 (s, 1H), 4.96 - 4.60 (m, 3H), 4.36 (dd, J = 34.3, 17.3 Hz, 3H), 2.99 - 2.84 (m, 1H), 2.62 (s, 1H), 2.43 - 2.33 (m, 1H), 2.00 (d, J = 5.7 Hz, 1H). LCMS (ESI) calcd for $C_{30}H_{25}ClN_3O_4^+$ [M+H]$^+$: 526.15, found, 526.1.

Example 193: Preparation of 3-(5-((1-(4'-chloro-[1,1'-biphenyl]-2-carbonyl)-3-hydroxyazetidin-3-yl)(hydroxy)methyl)-1-oxoisoindolin-2-yl)piperidine-2,6-dione (GT-05484)

Referring to the method of Scheme 6, the target product (GT-05484) was prepared as white solid (17 mg, yield 26.12%). $^1$H NMR (400 MHz, DMSO) δ 10.99 (d, J = 5.7 Hz, 1H), 7.63 (dd, J = 11.9, 7.9 Hz, 1H), 7.56 - 7.40 (m, 10H), 7.38 - 7.27 (m, 1H), 5.89 (d, J = 44.4 Hz, 2H), 5.15 - 5.02 (m, 1H), 4.54 - 4.38 (m, 2H), 4.35 - 4.24 (m, 1H), 4.12 (d, J = 9.6 Hz, 1H), 3.82 (d, J = 9.4 Hz, 1H), 3.47 - 3.38 (m, 1H), 3.26 (d, J = 9.1 Hz, 1H), 2.91 (d, J = 3.8 Hz, 1H), 2.60 (d, J = 16.0 Hz, 1H), 2.39 (s, 1H), 1.99 (d, J = 7.4 Hz, 1H). LCMS (ESI) calcd for $C_{30}H_{27}ClN_3O_6^+$ [M+H]$^+$: 560.16, found, 560.2.

**Example 194: Preparation of 3-(5-((1-((4'-fluoro-5,5-dimethyl-3,4,5,6-tetrahydro-[1,1'-biphenyl]-2-yl)methyl)azetidin-3-yl)methyl)-1-oxoisoindolin-2-yl)piperidine-2,6-dione (GT-05493)**

Referring to the method of Scheme 4, the target product (GT-05493) was prepared as white solid (18 mg, yield 21.30%). $^1$H NMR (400 MHz, DMSO-d6) δ 11.10 (s, 1H), 10.99 (s, 1H), 10.80 (s, 1H), 7.65 (d, J = 7.9 Hz, 1H), 7.47 - 7.31 (m, 2H), 7.29 - 7.17 (m, 4H), 5.10 (dd, J = 13.1, 4.0 Hz, 1H), 4.36 (dd, J = 51.3, 17.3 Hz, 2H), 4.03 (s, 1H), 3.92 - 3.56 (m, 4H), 3.37 (s, 2H), 3.20 (d, J = 8.0 Hz, 1H), 2.90 (ddd, J = 30.8, 22.4, 10.9 Hz, 3H), 2.60 (d, J = 16.4 Hz, 1H), 2.40 (dd, J = 13.0, 4.0 Hz, 1H), 2.06 (t, J = 41.0 Hz, 5H), 1.42 (d, J = 4.9 Hz, 1H), 1.09 - 0.91 (m, 6H). LCMS (ESI) calcd for $C_{32}H_{37}FN_3O_3^+$ [M+H]$^+$: 530.28, found, 530.3.

**Example 195: Preparation of 3-(5-((1-((4'-fluoro-5,5-dimethyl-3,4,5,6-tetrahydro-[1,1'-biphenyl]-2-yl)methyl)azetidin-3-ylidene)methyl)-1-oxoisoindolin-2-yl)piperidine-2,6-dione (GT-05483)**

Referring to the method of Scheme 4, the target product (GT-05483) was prepared as white solid (5 mg, yield 5.5%). $^1$H NMR (400 MHz, DMSO-d6) δ 10.99 (s, 1H), 10.87 (s, 1H), 7.69 (t, J = 11.5 Hz, 1H), 7.37 - 7.13 (m, 6H), 6.54 (s, 1H), 5.24 - 5.02 (m, 2H), 4.99 - 4.80 (m, 2H), 4.63 - 4.30 (m, 3H), 3.82 (s, 1H), 3.20 - 2.82 (m, 3H), 2.61 (d, J = 15.9 Hz, 1H), 2.40 (dd, J = 12.9, 4.3 Hz, 1H), 2.22 (s, 2H), 2.02 (dd, J = 15.2, 10.0 Hz, 3H), 1.46 (t, J = 6.3 Hz, 2H), 1.07 - 0.92 (m, 6H). LCMS (ESI) calcd for $C_{32}H_{35}FN_3O_3^+$ [M+H]$^+$: 528.27, found, 528.3.

**Example 196: Preparation of 3-(5-(3-((4'-fluoro-5,5-dimethyl-3,4,5,6-tetrahydro-[1,1'-biphenyl]-2-yl)methyl)-3,8-diazabicyclo[3.2.1]octan-8-yl)-1-oxoisoindolin-2-yl)piperidine-2,6-dione (GT-05498)**

Referring to the method of Scheme 4, the target product (GT-05498) was prepared as white solid (32 mg, yield 39.17%). $^1$H NMR (400 MHz, DMSO-d6) δ 10.96 (s, 1H), 10.37 (s, 1H), 7.55 (dd, J = 15.9, 8.4 Hz, 1H), 7.19 - 6.94 (m, 6H), 5.10 (dd, J = 13.1, 5.0 Hz, 1H), 4.50 (s, 2H), 4.31 (dd, J = 35.5, 16.9 Hz, 2H), 3.38 (s, 4H), 3.07 - 2.78 (m, 5H), 2.61 (d, J = 17.3 Hz, 1H), 2.46 - 2.39 (m, 1H), 2.28 (d, J = 7.7 Hz, 2H), 2.13 (s, 2H), 1.99 (d, J = 35.5 Hz, 5H), 1.34 (s, 2H), 0.86 (t, J = 16.5 Hz, 6H). LCMS (ESI) calcd for $C_{34}H_{40}FN_4O_3^+$ [M+H]$^+$: 571.31, found, 571.3.

**Example 197: Preparation of 3-(5-((1-((4'-fluoro-5,5-dimethyl-3,4,5,6-tetrahydro-[1,1'-biphenyl]-2-yl)methyl)-3-hydroxyazetidin-3-yl)(hydroxy)methyl)-1-oxoisoindolin-2-yl)piperidine-2,6-dione (GT-05499)**

Referring to the method of Scheme 4, the target product (GT-05499) was prepared as white solid (18 mg, yield 28.78%). $^1$H NMR (400 MHz, DMSO-d6) δ 10.99 (s, 1H), 10.39 (s, 1H), 7.74 - 7.55 (m, 2H), 7.39 - 7.18 (m, 5H), 6.31 - 6.13 (m, 2H), 5.11 (d, J = 13.3 Hz, 1H), 4.41 (ddd, J = 38.1, 15.8, 8.6 Hz, 3H), 3.97 - 3.59 (m, 3H), 3.54 - 3.35 (m, 4H), 2.92 (t, J = 14.9 Hz, 1H), 2.61 (d, J = 15.8 Hz, 1H), 2.41 (dd, J = 15.4, 11.0 Hz, 1H), 2.22 - 2.01 (m, 4H), 1.43 (d, J = 6.2 Hz, 2H), 1.00 - 0.90 (m, 6H). LCMS (ESI) calcd for $C_{32}H_{37}FN_3O_5^+$ [M+H]$^+$: 562.27, found, 562.3.

**Example 198: Preparation of 3-(5-(8-((4'-fluoro-5,5-dimethyl-3,4,5,6-tetrahydro-[1,1'-biphenyl]-2-yl)methyl)-3,8-diazabicyclo [3.2.1] octan-3-yl)-1-oxoisoindolin-2-yl)piperidine-2,6-dione (GT-05500)**

Referring to the method of Scheme 4, the target product (GT-05500) was prepared as white solid (20 mg, yield 24.84%). $^1$H NMR (400 MHz, DMSO-d6) δ 10.94 (s, 1H), 10.42 (s, 1H), 7.55 (d, J = 8.4 Hz, 1H), 7.31 - 7.22 (m, 4H), 7.00 (d, J = 11.5 Hz, 2H), 5.05 (dd, J = 13.2, 5.0 Hz, 1H), 4.27 (dd, J = 50.0, 17.1 Hz, 3H), 3.90 (s, 2H), 3.74 (d, J = 12.7 Hz, 2H), 3.57 (d, J = 12.3 Hz, 4H), 2.60 (s, 1H), 2.41 (d, J = 16.4 Hz, 2H), 2.13 (s, 2H), 2.01 - 1.90 (m, 2H), 1.74 (d, J = 8.3 Hz, 2H), 1.51 (t, J = 6.0 Hz, 2H), 1.33 - 1.25 (m, 2H), 0.99 (s, 6H). LCMS (ESI) calcd for $C_{34}H_{40}FN_4O_3^+$ [M+H]$^+$: 571.31, found, 571.3.

**Example 199: Preparation of 3-(5-((1-(4'-fluoro-5,5-dimethyl-3,4,5,6-tetrahydro-[1,1'-biphenyl]-2-carbonyl)piperidin-4-yl)methyl)-1-oxoisoindolin-2-yl)piperidine-2,6-dione (GT-05487)**

Referring to the method of Scheme 6, the target product (GT-05487) was prepared as white solid (8 mg, yield 8.69%). $^1$H NMR (400 MHz, DMSO-d6) δ 10.98 (s, 1H), 7.62 (d, J = 7.7 Hz, 1H), 7.38 - 7.27 (m, 2H), 7.26 - 7.11 (m, 4H), 5.10 (dd, J = 13.1, 4.9 Hz, 1H), 4.56 - 4.16 (m, 3H), 3.47 (d, J = 12.8 Hz, 2H), 3.33 - 3.22 (m, 2H), 2.91 (t, J = 12.9 Hz, 1H), 2.63 (dd, J = 39.4, 27.3 Hz, 2H), 2.44 - 2.31 (m, 4H), 2.19 - 1.89 (m, 4H), 1.66 - 1.33 (m, 5H), 0.97 (d, J = 9.3 Hz, 6H). LCMS (ESI) calcd for $C_{34}H_{39}FN_3O_4^+$ [M+H]$^+$: 572.29, found, 572.3.

**Example 200: Preparation of 3-(5-((1-(4'-fluoro-5,5-dimethyl-3,4,5,6-tetrahydro-[1,1'-biphenyl]-2-carbonyl)azetidin-3-yl)methyl)-1-oxoisoindolin-2-yl)piperidine-2,6-dione (GT-05488)**

Referring to the method of Scheme 6, the target product (GT-05488) was prepared as white solid (18 mg, yield 20.56%). $^1$H NMR (400 MHz, DMSO-d6) δ 11.00 (s, 1H), 7.62 (d, J = 7.8 Hz, 1H), 7.32 (dd, J = 8.5, 5.7 Hz, 2H), 7.27 - 7.20 (m, 3H), 7.15 (d, J = 7.8 Hz, 1H), 5.10 (dd, J = 13.2, 4.9 Hz, 1H), 4.35 (dd, J = 55.0, 17.0 Hz, 2H), 3.66 (dt, J = 15.5, 8.1 Hz, 2H), 3.26 (dd, J = 9.7, 4.1 Hz, 1H), 3.01 (d, J = 5.8 Hz, 1H), 2.89 (dd, J = 13.0, 4.6 Hz, 1H), 2.57 (dd, J = 21.4, 12.3 Hz, 3H), 2.41 (ddd, J = 17.9, 12.0, 6.1 Hz, 2H), 2.32 - 2.12 (m, 3H), 2.01 (dd, J = 20.5, 12.1 Hz, 2H), 1.39 (t, J = 6.4 Hz, 2H), 0.95 (d, J = 1.4 Hz, 6H). LCMS (ESI) calcd for $C_{32}H_{35}FN_3O_4^+$ [M+H]$^+$: 544.26, found, 544.3.

**Example 201: Preparation of 3-(5-((1-(4'-fluoro-5,5-dimethyl-3,4,5,6-tetrahydro-[1,1'-biphenyl]-2-carbonyl)azetidin-3-ylidene)methyl)-1-oxoisoindolin-2-yl)piperidine-2,6-dione (GT-05489)**

Referring to the method of Scheme 6, the target product (GT-05489) was prepared as white solid (13 mg, yield 15.00%). $^1$H NMR (400 MHz, DMSO-d6) δ 10.99 (d, J = 6.1 Hz, 1H), 7.66 (dd, J = 11.4, 7.9 Hz, 1H), 7.36 - 7.29 (m, 2H), 7.28 - 7.08 (m, 4H), 6.40 (d, J = 16.6 Hz, 1H), 5.18 - 5.04 (m, 1H), 4.72 - 4.43 (m, 2H), 4.41 - 4.23 (m, 3H), 2.97 - 2.86 (m, 1H), 2.60 (d, J = 16.4 Hz, 1H), 2.39 (dd, J = 28.3, 16.2 Hz, 3H), 2.18 - 1.97 (m, 3H), 1.44 (dd, J = 12.3, 6.1 Hz, 2H), 1.24 - 1.05 (m, 1H), 1.05 - 0.93 (m, 6H). LCMS (ESI) calcd for $C_{32}H_{33}FN_3O_4^+$ [M+H]$^+$: 542.25, found, 542.3.

**Example 202: Preparation of 3-(5-((1-(4'-fluoro-5,5-dimethyl-3,4,5,6-tetrahydro-[1,1'-biphenyl]-2-carbonyl)-3-hydroxyazetidin-3-yl)(hydroxy)methyl)-1-oxoisoindolin-2-yl)piperidine-2,6-dione (GT-05490)**

Referring to the method of Scheme 6, the target product (GT-05490) was prepared as white solid (8 mg, yield 8.60%). $^1$H NMR (400 MHz, DMSO-d6) δ 10.99 (s, 1H), 7.62 (dd, J = 7.8, 2.1 Hz, 1H), 7.48 - 7.33 (m, 2H), 7.29 (ddd, J = 17.8, 8.5, 4.3 Hz, 2H), 7.21 - 7.10 (m, 2H), 5.66 (s, 2H), 5.11 (dd, J = 13.3, 5.0 Hz, 1H), 4.36 (ddd, J = 19.7, 16.2, 8.4 Hz, 3H), 3.92 - 3.77 (m, 1H), 3.66 (d, J = 9.0 Hz, 1H), 3.30 - 3.08 (m, 4H), 2.97 - 2.86 (m, 1H), 2.60 (d, J = 17.1 Hz, 1H), 2.40 (dd, J = 17.9, 8.4 Hz, 1H), 2.08 (dd, J = 60.3, 22.8 Hz, 5H), 1.39 (d, J = 5.6 Hz, 2H), 0.99 - 0.93 (m, 6H). LCMS (ESI) calcd for $C_{32}H_{35}FN_3O_6^+$ [M+H]$^+$: 576.25, found, 576.3.

**Example 203: Preparation of 3-(5-(8-(4'-fluoro-5,5-dimethyl-3,4,5,6-tetrahydro-[1,1'-biphenyl]-2-carbonyl)-3,8-diazabicyclo [3.2.1] octan-3-yl)-1-oxoisoindolin-2-yl)piperidine-2,6-dione (GT-05491)**

Referring to the method of Scheme 6, the target product (GT-05491) was prepared as white solid (14 mg, yield 14.86%). $^1$H NMR (400 MHz, DMSO-d6) δ 10.94 (s, 1H), 7.48 (d, J = 8.5 Hz, 1H), 7.41 - 7.29 (m, 2H), 7.14 (s, 2H), 6.90 (s, 2H), 5.03 (dd, J = 13.3, 5.0 Hz, 1H), 4.54 (s, 1H), 4.23 (dd, J = 50.5, 16.9 Hz, 2H), 3.89 (s, 1H), 3.59 (d, J = 10.4 Hz, 1H), 3.36 (s, 4H), 2.98 - 2.69 (m, 2H), 2.58 (d, J = 16.9 Hz, 1H), 2.35 (ddd, J = 26.4, 13.2, 4.5 Hz, 4H), 2.04 - 1.87 (m, 2H), 1.51 (d, J = 41.9 Hz, 4H), 1.01 (d, J = 3.8 Hz, 6H). LCMS (ESI) calcd for $C_{34}H_{38}FN_4O_4^+$ [M+H]$^+$: 585.29, found, 585.3.

**Example 204: Preparation of 3-(5-(3-(4'-fluoro-5,5-dimethyl-3,4,5,6-tetrahydro-[1,1'-biphenyl]-2-carbonyl)-3,8-diazabicyclo [3.2.1] octan-8-yl)-1-oxoisoindolin-2-yl)piperidine-2,6-dione (GT-05492)**

Referring to the method of Scheme 6, the target product (GT-05492) was prepared as white solid (18 mg, yield 19.11%). $^1$H NMR (400 MHz, DMSO-d6) δ 10.81 (s, 1H), 7.38 - 7.25 (m, 2H), 7.08 (t, J = 8.8 Hz, 1H), 6.99 (d, J = 5.6 Hz, 1H), 6.92 - 6.77 (m, 2H), 6.71 (s, 1H), 4.97 - 4.87 (m, 1H), 4.13 (dt, J = 48.5, 13.4 Hz, 4H), 3.65 (d, J = 13.0 Hz, 1H), 3.26 (s, 2H), 3.09 (dd, J = 59.1, 12.6 Hz, 2H), 2.75 (d, J = 12.7 Hz, 1H), 2.45 (d, J = 17.1 Hz, 1H), 2.35 - 2.17 (m, 3H), 2.03 (s, 1H), 1.93 - 1.75 (m, 3H), 1.37 (d, J = 86.6 Hz, 4H), 0.86 (dd, J = 18.9, 6.3 Hz, 6H). LCMS (ESI) calcd for $C_{34}H_{38}FN_4O_4^+$ [M+H]$^+$: 585.29, found, 585.3.

**Example 205: Preparation of 3-(5-((6-((4'-chloro-5,5-dimethyl-3,4,5,6-tetrahydro-[1,1'-biphenyl]-2-yl)methyl)-2,6-diazaspiro[3.3]heptan-2-yl)methyl)-1-oxoisoindolin-2-yl)piperidine-2,6-dione (GT-05685)**

Referring to the method of Scheme 1, the target product (GT-05685) was prepared as white solid (8 mg, yield 11.27%). $^1H$ NMR (400 MHz, DMSO-d6) δ 11.01 (s, 2H), 7.79 (s, 1H), 7.68 (s, 1H), 7.59 (s, 1H), 7.43 (d, J = 8.3 Hz, 2H), 7.15 (d, J = 8.2 Hz, 2H), 5.13 (dd, J = 13.2, 4.9 Hz, 1H), 4.53 - 4.11 (m, 6H), 3.88 (s, 4H), 3.42 (s, 2H), 2.91 (d, J = 13.7 Hz, 3H), 2.61 (d, J = 17.2 Hz, 1H), 2.41 (d, J = 8.2 Hz, 1H), 2.15 (s, 2H), 2.01 (s, 3H), 1.42 (d, J = 5.7 Hz, 2H), 0.95 (d, J = 10.0 Hz, 6H). LCMS (ESI) calcd for $C_{34}H_{40}ClN_4O_3^+$ $[M+H]^+$: 587.28, found, 587.3.

**Example 206: Preparation of 5-((6-((4'-chloro-5,5-dimethyl-3,4,5,6-tetrahydro-[1,1'-biphenyl]-2-yl)methyl)-2,6-diazaspiro[3.3]heptan-2-yl)methyl)-2-(2,6-dioxopiperidin-3-yl)isoindoline-1,3-dione (GT-05675)**

Referring to the method of Scheme 1, the target product (GT-05675) was prepared as white solid (8 mg, yield 11.17%). $^1H$ NMR (400 MHz, DMSO-d6) δ 11.14 (s, 1H), 7.97 (d, J = 34.8 Hz, 3H), 7.43 (d, J = 8.4 Hz, 2H), 7.32 - 7.13 (m, 2H), 5.17 (dd, J = 12.7, 5.2 Hz, 1H), 4.49 (s, 1H), 4.21 (s, 4H), 3.66 (d, J = 43.9 Hz, 6H), 3.24 - 3.01 (m, 2H), 2.90 (t, J = 12.8 Hz, 1H), 2.61 (d, J = 17.4 Hz, 1H), 2.16 - 2.00 (m, 4H), 1.42 (d, J = 5.9 Hz, 2H), 1.26 (dt, J = 7.4, 5.1 Hz, 1H), 0.96 (d, J = 10.3 Hz, 6H). LCMS (ESI) calcd for $C_{34}H_{38}ClN_4O_4^+$ $[M+H]^+$: 601.26, found, 601.3.

**Example 207: Preparation of 5-(6-((4'-chloro-5,5-dimethyl-3,4,5,6-tetrahydro-[1,1'-biphenyl]-2-yl)methyl)-2,6-diazaspiro[3.3]heptane-2-carbonyl)-2-(2,6-dioxopiperidin-3-yl)isoindoline-1,3-dione (GT-05676)**

Referring to the methods of Scheme 6 and Example 76, the target product (GT-05676) was prepared as white solid (9 mg, yield 12.10%). $^1H$ NMR (400 MHz, DMSO-d6) δ 11.15 (s, 1H), 10.37 (d, J = 84.9 Hz, 1H), 8.15 - 7.94 (m, 3H), 7.43 (d, J = 22.4 Hz, 2H), 7.15 (s, 2H), 5.19 (dd, J = 12.7, 5.4 Hz, 1H), 4.22 (d, J = 17.6 Hz, 3H), 3.93 (d, J = 55.3 Hz, 3H), 3.62 (s, 1H), 3.36 (s, 2H), 3.31 (s, 3H), 2.91 (dd, J = 22.3, 9.0 Hz, 1H), 2.64 - 2.55 (m, 1H), 2.13 - 2.00 (m, 4H), 1.42 (s, 2H), 0.94 (s, 6H). LCMS (ESI) calcd for $C_{34}H_{36}ClN_4O_5^+$ $[M+H]^+$: 615.24, found, 615.3.

**Example 208: Preparation of 3-(5-((7-((4'-chloro-5,5-dimethyl-3,4,5,6-tetrahydro-[1,1'-biphenyl]-2-yl)methyl)-2,7-diazaspiro[3.5]nonan-2-yl)methyl)-1-oxoisoindolin-2-yl)piperidine-2,6-dione (GT-05677)**

Referring to the method of Scheme 1, the target product (GT-05677) was prepared as white solid (10 mg, yield 14.59%). $^1H$ NMR (400 MHz, DMSO-d6) δ 11.66 (s, 1H), 11.01 (s, 1H), 9.73 (s, 1H), 7.86 - 7.68 (m, 3H), 7.43 (dd, J = 10.3, 8.5 Hz, 2H), 7.13 (dd, J = 10.8, 8.4 Hz, 2H), 5.13 (dd, J = 13.4, 4.7 Hz, 1H), 4.56 - 4.25 (m, 4H), 4.00 - 3.61 (m, 4H), 3.48 (s, 2H), 3.22 (dd, J = 35.0, 20.6 Hz, 4H), 2.92 (dd, J = 21.6, 8.9 Hz, 1H), 2.61 (d, J = 16.7 Hz, 2H), 2.43 (dd, J = 12.9, 4.3 Hz, 1H), 2.30 (s, 2H), 2.06 (d, J = 39.1 Hz, 6H), 1.45 (t, J = 6.1 Hz, 2H), 0.96 (d, J = 10.5 Hz, 6H). LCMS (ESI) calcd for $C_{36}H_{44}ClN_4O_3^+$ $[M+H]^+$: 615.31, found, 615.3.

**Example 209: Preparation of 5-((7-((4'-chloro-5,5-dimethyl-3,4,5,6-tetrahydro-[1,1'-biphenyl]-2-yl)methyl)-2,7-diazaspiro[3.5]nonan-2-yl)methyl)-2-(2,6-dioxopiperidin-3-yl)isoindoline-1,3-dione (GT-05678)**

Referring to the method of Scheme 1, the target product (GT-05678) was prepared as white solid (20 mg, yield 28.17%). $^1H$ NMR (400 MHz, DMSO-d6) δ 11.83 (s, 1H), 11.15 (s, 1H), 9.74 (s, 1H), 8.18 (s, 1H), 8.04 (dd, J = 26.5, 7.7 Hz, 2H), 7.42 (t, J = 7.5 Hz, 2H), 7.11 (d, J = 8.3 Hz, 2H), 5.18 (dd, J = 12.8, 5.5 Hz, 1H), 4.69 - 4.44 (m, 2H), 3.87 (dd, J = 64.6, 29.5 Hz, 4H), 3.48 (s, 3H), 3.20 (s, 3H), 2.89 (dd, J = 16.7, 4.9 Hz, 1H), 2.59 (dd, J = 23.5, 11.3 Hz, 3H), 2.31 (s, 3H), 2.15 - 2.01 (m, 6H), 1.45 (t, J = 6.0 Hz, 2H), 0.95 (s, 6H). LCMS (ESI) calcd for $C_{36}H_{42}ClN_4O_4^+$ $[M+H]^+$: 629.29, found, 629.3.

**Example 210: Preparation of 5-(7-((4'-chloro-5,5-dimethyl-3,4,5,6-tetrahydro-[1,1'-biphenyl]-2-yl)methyl)-2,7-diazaspiro[3.5]nonane-2-carbonyl)-2-(2,6-dioxopiperidin-3-yl)isoindoline-1,3-dione (GT-05679)**

Referring to the methods of Scheme 6 and Example 76, the target product (GT-05679) was prepared as white solid (12 mg, yield 16.74%). $^1H$ NMR (400 MHz, DMSO-d6) δ 11.15 (s, 1H), 8.95 (s, 1H), 8.17 - 7.98 (m, 3H), 7.43 (dd, J = 25.3, 8.2 Hz, 2H), 7.12 (dd, J = 24.2, 8.3 Hz, 2H), 5.18 (dd, J = 12.8, 5.4 Hz, 1H), 4.13 - 3.75 (m, 4H), 3.55 (s, 2H), 3.37 (d, J = 2.6 Hz, 2H), 3.31 (dd, J = 5.6, 2.5 Hz, 2H), 3.18 (d, J = 10.8 Hz, 2H), 3.01 - 2.85 (m, 1H), 2.62 (d, J = 18.7 Hz, 2H), 2.24 (s, 2H), 2.00 (d, J = 16.9 Hz, 5H), 1.47 (s, 2H), 0.95 (s, 6H). LCMS (ESI) calcd for $C_{36}H_{40}ClN_4O_5^+$ $[M+H]^+$: 643.27, found, 643.3.

**Example 211: Preparation of 3-(5-((5-((4'-chloro-5,5-dimethyl-3,4,5,6-tetrahydro-[1,1'-biphenyl]-2-yl)methyl) hexahydropyrrolo[3,4-c]pyrrol-2(1H)-yl)methyl)-1-oxoisoindolin-2-yl)piperidine-2,6-dione (GT-05653)**

Referring to the method of Scheme 1, the target product (GT-05653) was prepared as white solid (25 mg, yield 28.69%). $^1$H NMR (400 MHz, DMSO-d6) δ 11.52 (d, J = 60.0 Hz, 1H), 11.01 (s, 1H), 10.76 (d, J = 95.5 Hz, 1H), 7.79 (d, J = 8.2 Hz, 3H), 7.48 - 7.34 (m, 2H), 7.15 (dd, J = 8.1, 5.8 Hz, 2H), 5.14 (dd, J = 13.2, 5.1 Hz, 1H), 4.43 (d, J = 33.9 Hz, 4H), 3.81 (d, J = 8.9 Hz, 1H), 3.55 (d, J = 59.1 Hz, 6H), 3.27 (s, 3H), 3.16 - 3.03 (m, 2H), 3.00 - 2.84 (m, 2H), 2.61 (d, J = 16.9 Hz, 1H), 2.49 - 2.43 (m, 1H), 2.31 (s, 1H), 2.02 (s, 3H), 1.52 - 1.38 (m, 2H), 0.96 (d, J = 8.8 Hz, 6H). LCMS (ESI) calcd for $C_{35}H_{42}ClN_4O_3^+$ [M+H]$^+$: 601.29, found, 601.3.

**Example 212: Preparation of 5-((5-((4'-chloro-5,5-dimethyl-3,4,5,6-tetrahydro-[1,1'-biphenyl]-2-yl)methyl)hexahydropyrrolo[3,4-c]pyrrol-2(1H)-yl)methyl)-2-(2,6-dioxopiperidin-3-yl)isoindoline-1,3-dione (GT-05654)**

Referring to the method of Scheme 1, the target product (GT-05654) was prepared as white solid (50 mg, yield 56.28%). $^1$H NMR (400 MHz, DMSO-d6) δ 11.30 (s, 1H), 11.15 (s, 1H), 10.53 (d, J = 46.7 Hz, 1H), 8.10 (ddd, J = 27.2, 24.3, 7.7 Hz, 3H), 7.41 (dd, J = 18.2, 8.1 Hz, 2H), 7.23 - 7.05 (m, 2H), 5.18 (dd, J = 12.8, 5.3 Hz, 1H), 4.58 (d, J = 39.6 Hz, 2H), 3.86 - 3.48 (m, 6H), 3.37 (s, 3H), 3.15 (d, J = 70.7 Hz, 4H), 2.92 (dd, J = 22.3, 8.7 Hz, 2H), 2.61 (d, J = 17.8 Hz, 1H), 2.48 - 2.43 (m, 1H), 2.30 (d, J = 18.5 Hz, 1H), 2.11 - 2.00 (m, 3H), 1.45 (s, 2H), 0.96 (d, J = 8.4 Hz, 6H). LCMS (ESI) calcd for $C_{35}H_{40}ClN_4O_4^+$ [M+H]$^+$: 615.27, found, 615.3.

**Example 213: Preparation of 5-(5-((4'-chloro-5,5-dimethyl-3,4,5,6-tetrahydro-[1,1'-biphenyl]-2-yl)methyl)octahydropyrrolo[3,4-c]pyrrole-2-carbonyl)-2-(2,6-dioxopiperidin-3-yl)isoindoline-1,3-dione (GT-05655)**

Referring to the methods of Scheme 6 and Example 76, the target product (GT-05655) was prepared as white solid (35 mg, yield 47.97%). $^1$H NMR (400 MHz, DMSO-d6) δ 11.15 (s, 1H), 10.47 (d, J = 72.6 Hz, 1H), 7.95 (ddd, J = 39.3, 19.6, 7.6 Hz, 3H), 7.45 (t, J = 8.7 Hz, 2H), 7.16 (dd, J = 8.3, 3.5 Hz, 2H), 5.20 (dd, J = 12.8, 5.7 Hz, 1H), 3.79 - 3.49 (m, 7H), 3.24 (s, 2H), 2.94 (dd, J = 37.3, 22.8 Hz, 3H), 2.74 - 2.53 (m, 3H), 2.29 (s, 2H), 2.14 - 1.97 (m, 3H), 1.46 (d, J = 5.9 Hz, 2H), 0.96 (s, 6H). LCMS (ESI) calcd for $C_{35}H_{38}ClN_4O_5^+$ [M+H]$^+$: 629.25, found, 629.3.

**Example 214: Preparation of 3-(5-((4-(4'-chloro-5,5-dimethyl-3,4,5,6-tetrahydro-[1,1'-biphenyl]-2-carbonyl)-1,4-diazepan-1-yl)methyl)-1-oxoisoindolin-2-yl)piperidine-2,6-dione (GT-05680)**

Referring to the method of Scheme 1, the target product (GT-05680) was prepared as white solid (35 mg, yield 50.15%). $^1$H NMR (400 MHz, DMSO-d6) δ 11.38 (d, J = 71.0 Hz, 1H), 11.01 (s, 1H), 7.91 - 7.77 (m, 2H), 7.74 (d, J = 7.0 Hz, 1H), 7.53 (s, 1H), 7.36 (d, J = 8.4 Hz, 2H), 7.22 (d, J = 9.1 Hz, 1H), 5.14 (dd, J = 13.2, 5.0 Hz, 1H), 4.58 - 4.34 (m, 3H), 4.08 (s, 2H), 3.80 - 3.42 (m, 2H), 3.19 (s, 4H), 3.09 - 2.92 (m, 2H), 2.61 (d, J = 16.5 Hz, 1H), 2.39 (dd, J = 30.8, 14.5 Hz, 3H), 2.19 (s, 2H), 2.05 - 1.78 (m, 3H), 1.46 (d, J = 5.7 Hz, 2H), 0.98 (t, J = 10.5 Hz, 6H). LCMS (ESI) calcd for $C_{34}H_{40}ClN_4O_4^+$ [M+H]$^+$: 603.27, found, 603.3.

**Example 215: Preparation of 3-(5-((7-(4'-chloro-5,5-dimethyl-3,4,5,6-tetrahydro-[1,1'-biphenyl]-2-carbonyl)-2,7-diazaspiro[3.5]nonan-2-yl)methyl)-1-oxoisoindolin-2-yl)piperidine-2,6-dione (GT-05656)**

Referring to the method of Scheme 1, the target product (GT-05656) was prepared as white solid (23 mg, yield 27.17%). $^1$H NMR (400 MHz, DMSO-d6) δ 11.69 (d, J = 83.0 Hz, 1H), 11.01 (s, 1H), 7.75 (ddd, J = 28.8, 14.8, 10.5 Hz, 3H), 7.35 (d, J = 8.4 Hz, 2H), 7.24 (d, J = 6.8 Hz, 2H), 5.13 (dd, J = 13.2, 4.9 Hz, 1H), 4.41 (dd, J = 53.3, 17.4 Hz, 4H), 3.84 - 3.58 (m, 6H), 3.34 - 3.07 (m, 4H), 3.02 - 2.86 (m, 2H), 2.61 (d, J = 17.3 Hz, 1H), 2.38 (dd, J = 18.5, 10.6 Hz, 2H), 2.11 - 1.97 (m, 2H), 1.81 (dd, J = 68.4, 17.3 Hz, 3H), 1.38 (d, J = 55.4 Hz, 3H), 0.98 (d, J = 14.5 Hz, 6H). LCMS (ESI) calcd for $C_{36}H_{42}ClN_4O_4^+$ [M+H]$^+$: 629.29, found, 629.3.

**Example 216: Preparation of 3-(5-(7-(4'-chloro-5,5-dimethyl-3,4,5,6-tetrahydro-[1,1'-biphenyl]-2-carbonyl)-2,7-diazaspiro[3.5]nonane-2-carbonyl)-1-oxoisoindolin-2-yl)piperidine-2,6-dione (GT-05657)**

Referring to the methods of Scheme 6 and Example 76, the target product (GT-05657) was prepared as white solid (6 mg, yield 8.72%). $^1$H NMR (400 MHz, DMSO-d6) δ 11.00 (s, 1H), 7.82 (s, 1H), 7.74 (dd, J = 23.1, 7.8 Hz, 2H), 7.36 (dd, J = 23.6, 8.0 Hz, 2H), 7.24 (t, J = 10.5 Hz, 2H), 5.12 (dd, J = 13.3, 5.1 Hz, 1H), 4.42 (dd, J = 50.8, 17.6 Hz, 2H), 4.01 - 3.60 (m, 3H), 3.48 (dd, J = 23.6, 8.5 Hz, 4H), 3.21 (dd, J = 21.5, 16.5 Hz, 2H), 3.05 (s, 1H), 2.90 (dd, J = 16.9, 8.5 Hz, 1H), 2.61 (d, J = 16.1 Hz, 1H), 2.44 - 2.31 (m, 3H), 2.14 - 1.87 (m, 3H), 1.52 (d, J = 64.3 Hz, 4H), 0.97 (d, J = 13.1 Hz, 6H). LCMS (ESI) calcd for $C_{36}H_{40}ClN_4O_5^+$ [M+H]$^+$: 643.27, found, 643.27.

**Example 217: Preparation of 3-(5-((6-(4'-chloro-5,5-dimethyl-3,4,5,6-tetrahydro-[1,1'-biphenyl]-2-carbonyl)-2,6-diazaspiro[3.3]heptan-2-yl)methyl)-1-oxoisoindolin-2-yl)piperidine-2,6-dione (GT-05681)**

Referring to the method of Scheme 1, the target product (GT-05681) was prepared as white solid (11 mg, yield 15.17%). $^1H$ NMR (400 MHz, DMSO-d6) δ 11.01 (s, 1H), 10.51 (s, 1H), 7.79 (d, J = 8.1 Hz, 1H), 7.62 (s, 1H), 7.53 (s, 1H), 7.40 (t, J = 8.8 Hz, 2H), 7.25 (dd, J = 14.5, 8.5 Hz, 2H), 5.13 (dd, J = 13.4, 5.0 Hz, 1H), 4.42 (dd, J = 50.6, 17.7 Hz, 3H), 4.00 - 3.49 (m, 8H), 3.26 (dd, J = 24.4, 8.3 Hz, 2H), 2.97 - 2.87 (m, 1H), 2.61 (d, J = 16.5 Hz, 1H), 2.42 (dd, J = 13.0, 4.6 Hz, 1H), 2.23 (s, 2H), 2.12 (s, 1H), 2.06 - 1.97 (m, 1H), 1.40 (d, J = 6.2 Hz, 2H), 0.96 (d, J = 7.6 Hz, 6H). LCMS (ESI) calcd for $C_{34}H_{38}ClN_4O_4^+$ [M+H]$^+$: 601.26, found, 601.3.

**Example 218: Preparation of 3-(5-(6-(4'-chloro-5,5-dimethyl-3,4,5,6-tetrahydro-[1,1'-biphenyl]-2-carbonyl)-2,6-diazaspiro[3.3]heptane-2-carbonyl)-1-oxoisoindolin-2-yl)piperidine-2,6-dione (GT-05682)**

Referring to the methods of Scheme 6 and Example 76, the target product (GT-05682) was prepared as white solid (6 mg, yield 16.82%). $^1H$ NMR (400 MHz, DMSO-d6) δ 11.01 (s, 1H), 7.77 (d, J = 7.4 Hz, 2H), 7.65 (d, J = 8.4 Hz, 1H), 7.40 (d, J = 8.5 Hz, 2H), 7.25 (d, J = 8.5 Hz, 2H), 5.13 (dd, J = 13.3, 5.0 Hz, 1H), 4.43 (d, J = 31.0 Hz, 2H), 4.20 (d, J = 9.0 Hz, 1H), 4.00 (dd, J = 16.8, 10.1 Hz, 2H), 3.79 (d, J = 10.5 Hz, 3H), 3.65 (d, J = 16.6 Hz, 2H), 3.01 - 2.86 (m, 1H), 2.61 (d, J = 16.8 Hz, 1H), 2.44 - 2.32 (m, 1H), 2.24 (s, 2H), 2.12 (s, 2H), 2.06 - 1.97 (m, 1H), 1.40 (t, J = 6.4 Hz, 2H), 0.97 (s, 6H). LCMS (ESI) calcd for $C_{34}H_{36}ClN_4O_5^+$ [M+H]$^+$: 615.24, found, 615.3.

**Example 219: Preparation of 3-(5-((5-(4'-chloro-5,5-dimethyl-3,4,5,6-tetrahydro-[1,1'-biphenyl]-2-carbonyl)hexahydropyrrolo[3,4-c]pyrrol-2(1H)-yl)methyl)-1-oxoisoindolin-2-yl)piperidine-2,6-dione (GT-05658)**

Referring to the method of Scheme 1, the target product (GT-05658) was prepared as white solid (25 mg, yield 36.17%). $^1H$ NMR (400 MHz, DMSO-d6) δ 11.47 (d, J = 58.5 Hz, 1H), 11.01 (s, 1H), 7.82 - 7.68 (m, 3H), 7.46 - 7.23 (m, 4H), 5.14 (dd, J = 13.2, 4.9 Hz, 1H), 4.59 - 4.27 (m, 4H), 3.58 (s, 2H), 3.19 (dd, J = 40.2, 26.4 Hz, 4H), 2.97 - 2.73 (m, 4H), 2.61 (d, J = 16.7 Hz, 1H), 2.43 (dd, J = 13.0, 4.1 Hz, 1H), 2.34 - 2.00 (m, 6H), 1.43 (t, J = 6.0 Hz, 2H), 0.99 (s, 6H). LCMS (ESI) calcd for $C_{35}H_{40}ClN_4O_4^+$ [M+H]$^+$: 615.27, found, 615.3.

**Example 220: Preparation of 3-(5-(5-(4'-chloro-5,5-dimethyl-3,4,5,6-tetrahydro-[1,1'-biphenyl]-2-carbonyl)octahydropyrrolo[3,4-c]pyrrole-2-carbonyl)-1-oxoisoindolin-2-yl)piperidine-2,6-dione (GT-05659)**

Referring to the methods of Scheme 6 and Example 76, the target product (GT-05659) was prepared as white solid (25 mg, yield 35.17%). $^1H$ NMR (400 MHz, DMSO-d6) δ 11.01 (s, 1H), 7.79 (t, J = 8.5 Hz, 1H), 7.64 (s, 1H), 7.51 (s, 1H), 7.38 (dd, J = 11.5, 5.6 Hz, 2H), 7.27 (dd, J = 15.0, 8.3 Hz, 2H), 5.14 (d, J = 13.1 Hz, 1H), 4.55 - 4.34 (m, 2H), 3.73 - 3.43 (m, 3H), 3.32 - 2.89 (m, 5H), 2.79 - 2.58 (m, 4H), 2.42 (d, J = 13.0 Hz, 1H), 2.16 (d, J = 96.6 Hz, 5H), 1.50 - 1.34 (m, 2H), 0.98 (dd, J = 17.6, 2.4 Hz, 6H). LCMS (ESI) calcd for $C_{35}H_{38}ClN_4O_5^+$ [M+H]$^+$: 629.25, found, 629.3.

**Example 221: Preparation of 3-(5-((6-(4'-chloro-5,5-dimethyl-3,4,5,6-tetrahydro-[1,1'-biphenyl]-2-carbonyl)-3,6-diazabicyclo[3.1.1]heptan-3-yl)methyl)-1-oxoisoindolin-2-yl)piperidine-2,6-dione (GT-05660)**

Referring to the method of Scheme 1, the target product (GT-05660) was prepared as white solid (35 mg, yield 50.31%). $^1H$ NMR (400 MHz, DMSO-d6) δ 11.92 (s, 1H), 11.01 (s, 1H), 7.92 (s, 1H), 7.81 (s, 2H), 7.46 (d, J = 8.1 Hz, 2H), 7.27 (d, J = 8.5 Hz, 2H), 5.15 (dd, J = 13.3, 5.0 Hz, 1H), 4.57 - 4.33 (m, 2H), 4.30 - 4.10 (m, 2H), 3.84 (s, 1H), 3.51 (s, 4H), 2.99 - 2.83 (m, 1H), 2.63 (t, J = 16.3 Hz, 2H), 2.45 (dd, J = 13.2, 4.4 Hz, 1H), 2.21 (d, J = 19.1 Hz, 3H), 2.07 - 1.95 (m, 2H), 1.85 (s, 1H), 1.65 (d, J = 6.8 Hz, 1H), 1.36 (d, J = 6.1 Hz, 2H), 0.94 (s, 6H). LCMS (ESI) calcd for $C_{34}H_{38}ClN_4O_4^+$ [M+H]$^+$: 601.26, found, 601.3.

**Example 222: Preparation of 3-(5-(6-(4'-chloro-5,5-dimethyl-3,4,5,6-tetrahydro-[1,1'-biphenyl]-2-carbonyl)-3,6-diazabicyclo[3.1.1]heptane-3-carbonyl)-1-oxoisoindolin-2-yl)piperidine-2,6-dione (GT-05661)**

Referring to the methods of Scheme 6 and Example 76, the target product (GT-05661) was prepared as white solid (38 mg, yield 53.21%). $^1H$ NMR (400 MHz, DMSO-d6) δ 11.01 (s, 1H), 7.80 (dd, J = 10.2, 4.9 Hz, 1H), 7.54 (d, J = 5.1 Hz, 1H), 7.41 (dd, J = 15.1, 6.6 Hz, 3H), 7.28 (dd, J = 21.4, 8.4 Hz, 2H), 5.14 (dd, J = 13.0, 5.0 Hz, 1H), 4.45 (ddd, J = 17.6, 13.6, 7.7 Hz, 2H), 3.97 (d, J = 32.4 Hz, 1H), 3.65 (dt, J = 18.5, 12.0 Hz, 2H), 3.45 (d, J = 13.1 Hz, 1H), 3.30 (s, 2H), 2.93 (dd, J = 19.4, 10.3 Hz, 1H), 2.61 (d, J = 16.5 Hz, 1H), 2.43 (td, J = 13.5, 4.3 Hz, 2H), 2.21 (dd, J = 49.1, 17.6 Hz, 2H), 2.03 (d, J = 17.3 Hz, 2H), 1.83 (d, J = 8.8 Hz, 1H), 1.47 - 1.32 (m, 3H), 1.01 - 0.82 (m, 6H). LCMS (ESI) calcd for $C_{34}H_{36}ClN_4O_5^+$ [M+H]$^+$: 615.24, found, 615.3.

**Example 223: Preparation of 3-(5-((5-(4'-chloro-5,5-dimethyl-3,4,5,6-tetrahydro-[1,1'-biphenyl]-2-carbonyl)-2,5-diazabicyclo[2.2.2]octan-2-yl)methyl)-1-oxoisoindolin-2-yl)piperidine-2,6-dione (GT-05662)**

Referring to the method of Scheme 1, the target product (GT-05662) was prepared as white solid (35 mg, yield 51.32%). $^1$H NMR (400 MHz, DMSO-d6) δ 11.01 (s, 1H), 8.01 - 7.57 (m, 3H), 7.52 - 7.35 (m, 2H), 7.34 - 7.13 (m, 2H), 5.14 (dd, J = 13.1, 4.7 Hz, 1H), 4.44 (dd, J = 38.0, 13.9 Hz, 3H), 4.03 (d, J = 50.0 Hz, 1H), 3.80 - 3.38 (m, 5H), 3.33 - 2.77 (m, 4H), 2.61 (d, J = 17.2 Hz, 1H), 2.48 - 2.19 (m, 4H), 2.02 (t, J = 46.2 Hz, 3H), 1.76 (s, 1H), 1.44 (s, 2H), 1.09 - 0.90 (m, 6H). LCMS (ESI) calcd for $C_{35}H_{40}ClN_4O_4^+$ [M+H]$^+$: 615.27, found, 615.3.

**Example 224: Preparation of 3-(5-(5-(4'-chloro-5,5-dimethyl-3,4,5,6-tetrahydro-[1,1'-biphenyl]-2-carbonyl)-2,5-diazabicyclo [2.2.2] octane-2-carbonyl)-1-oxoisoindolin-2-yl)piperidine-2,6-dione (GT-05663)**

Referring to the methods of Scheme 6 and Example 76, the target product (GT-05663) was prepared as white solid (30 mg, yield 42.45%). $^1$H NMR (400 MHz, DMSO-d6) δ 11.01 (d, J = 4.4 Hz, 1H), 7.83 - 7.72 (m, 1H), 7.67 - 7.13 (m, 6H), 5.13 (dd, J = 12.5, 5.6 Hz, 1H), 4.71 - 4.18 (m, 3H), 3.95 - 3.36 (m, 6H), 3.23 (dd, J = 28.1, 12.4 Hz, 2H), 2.94 (dt, J = 17.2, 14.3 Hz, 2H), 2.41 (dd, J = 26.1, 13.1 Hz, 3H), 2.06 (t, J = 29.0 Hz, 2H), 1.92 - 1.68 (m, 3H), 1.42 (s, 2H), 0.97 (t, J = 15.8 Hz, 6H). LCMS (ESI) calcd for $C_{35}H_{38}ClN_4O_5^+$ [M+H]$^+$: 629.25, found, 629.3.

**Example 225: Preparation of 3-(5-((5-(4'-chloro-[1,1'-biphenyl]-2-carbonyl)-2,5-diazabicyclo[2.2.1]heptan-2-yl) methyl)-1-oxoisoindolin-2-yl)piperidine-2,6-dione (GT-05776)**

Referring to the method of Scheme 1, the target product (GT-05776) was prepared as white solid (25 mg, yield 34.32%). $^1$H NMR (400 MHz, DMSO-d6) δ 11.56 (s, 1H), 11.01 (s, 1H), 10.91 (s, 1H), 7.84 (ddd, J = 27.1, 20.4, 19.8 Hz, 3H), 7.59 - 7.39 (m, 8H), 5.14 (dd, J = 13.2, 5.1 Hz, 1H), 4.42 (dd, J = 50.3, 17.8 Hz, 4H), 3.88 (dd, J = 26.8, 12.8 Hz, 2H), 3.48 (d, J = 5.5 Hz, 2H), 3.25 (d, J = 11.1 Hz, 3H), 3.12 - 2.87 (m, 2H), 2.61 (d, J = 17.2 Hz, 1H), 2.42 (dt, J = 12.7, 6.6 Hz, 1H), 2.07 - 1.94 (m, 1H). LCMS (ESI) calcd for $C_{32}H_{30}ClN_4O_4^+$ [M+H]$^+$: 569.20, found, 569.2.

**Example 226: Preparation of 3-(5-((4-((3-(4-chlorophenyl)pyridin-2-yl)methyl)piperazin-1-yl)methyl)-1-oxoisoindolin-2-yl)piperidine-2,6-dione (GT-05778)**

Referring to the method of Scheme 1, the target product (GT-05778) was prepared as white solid (30 mg, yield 39.17%). $^1$H NMR (400 MHz, DMSO-d6) δ 11.01 (s, 1H), 8.72 (dd, J = 5.0, 1.5 Hz, 1H), 7.96 (dd, J = 7.8, 1.3 Hz, 1H), 7.85 (s, 1H), 7.81 (d, J = 7.8 Hz, 1H), 7.74 (d, J = 7.7 Hz, 1H), 7.66 (dd, J = 7.8, 5.0 Hz, 1H), 7.60 (d, J = 8.5 Hz, 2H), 7.51 (d, J = 8.5 Hz, 2H), 5.14 (dd, J = 13.3, 5.1 Hz, 1H), 4.58 - 4.30 (m, 6H), 3.36 - 3.25 (m, 8H), 2.96 - 2.89 (m, 1H), 2.61 (d, J = 16.9 Hz, 1H), 2.47 - 2.36 (m, 1H), 2.06 - 1.96 (m, 1H). LCMS (ESI) calcd for $C_{30}H_{31}ClN_5O_3^+$ [M+H]$^+$: 544.21, found, 544.3.

**Example 227: Preparation of 3-(5-((4-(2-(furan-2-yl)benzyl)piperazin-1-yl)methyl)-1-oxoisoindolin-2-yl)piperidine-2,6-dione (GT-05779)**

Referring to the method of Scheme 1, the target product (GT-05779) was prepared as white solid (31 mg, yield 37.17%). $^1$H NMR (400 MHz, DMSO-d6) δ 11.01 (s, 1H), 7.94 - 7.67 (m, 6H), 7.57 - 7.35 (m, 2H), 6.93 (s, 1H), 6.66 (dd, J = 3.2, 1.8 Hz, 1H), 5.13 (dd, J = 13.3, 5.1 Hz, 1H), 4.55 - 4.28 (m, 5H), 3.39 - 3.03 (m, 9H), 2.92 (s, 1H), 2.61 (d, J = 17.0 Hz, 1H), 2.42 (s, 1H), 2.00 (s, 1H). LCMS (ESI) calcd for $C_{29}H_{31}N_4O_4^+$ [M+H]$^+$: 499.23, found, 499.3.

**Example 228: Preparation of 3-(5-((4-(2-(1-methyl-1H-pyrrol-2-yl)benzyl)piperazin-1-yl)methyl)-1-oxoisoindolin-2-yl)piperidine-2,6-dione (GT-05683)**

Referring to the method of Scheme 1, the target product (GT-05683) was prepared as white solid (30 mg, yield 37.17%). $^1$H NMR (400 MHz, DMSO-d6) δ 11.01 (s, 1H), 7.94 (s, 1H), 7.88 - 7.69 (m, 3H), 7.48 (dd, J = 5.5, 3.5 Hz, 2H), 7.38 - 7.29 (m, 1H), 6.88 (s, 1H), 6.10 (dd, J = 8.8, 6.0 Hz, 2H), 5.13 (dd, J = 13.3, 5.1 Hz, 1H), 4.51 - 4.32 (m, 4H), 4.17 (s, 2H), 3.33 (d, J = 37.7 Hz, 10H), 2.98 - 2.84 (m, 2H), 2.61 (d, J = 16.8 Hz, 1H), 2.43 (dt, J = 13.3, 8.8 Hz, 1H), 2.07 - 1.95 (m, 1H). LCMS (ESI) calcd for $C_{30}H_{34}N_5O_3^+$ [M+H]$^+$: 512.27, found, 512.3.

**Example 229: Preparation of 3-(1-oxo-5-((4-(2-(thiazol-5-yl)benzyl)piperazin-1-yl)methyl)isoindolin-2-yl)piperidine-2,6-dione (GT-05684)**

Referring to the method of Scheme 1, the target product (GT-05684) was prepared as white solid (14 mg, yield 17.32%). $^1$H NMR (400 MHz, DMSO-d6) δ 11.07 (s, 1H), 9.32 (s, 1H), 8.09 (s, 1H), 7.94 (d, J = 6.3 Hz, 1H), 7.90 (s, 1H), 7.82

(dd, J = 28.1, 7.8 Hz, 2H), 7.63 - 7.49 (m, 3H), 5.17 (dd, J = 13.2, 5.0 Hz, 1H), 4.57 - 4.41 (m, 4H), 4.28 (s, 2H), 3.27 (s, 8H), 2.95 (d, J = 12.8 Hz, 1H), 2.67 (d, J = 16.8 Hz, 1H), 2.48 (dd, J = 13.0, 4.2 Hz, 1H), 2.12 - 1.95 (m, 1H). LCMS (ESI) calcd for $C_{28}H_{30}N_5O_3S^+$ [M+H]$^+$: 516.21, found, 516.3.

**Example 230: Preparation of 3-(5-((4-((5-(furan-2-yl)thiophen-2-yl)methyl)piperazin-1-yl)methyl)-1-oxoisoindolin-2-yl)piperidine-2,6-dione (GT-05780)**

Referring to the method of Scheme 1, the target product (GT-05780) was prepared as white solid (20 mg, yield 24.17%). $^1$H NMR (400 MHz, DMSO-d6) δ 11.01 (s, 1H), 7.80 (d, J = 7.9 Hz, 2H), 7.72 (d, J = 1.3 Hz, 2H), 7.27 (t, J = 14.3 Hz, 2H), 6.78 (d, J = 3.2 Hz, 1H), 6.60 (dd, J = 3.4, 1.8 Hz, 1H), 5.13 (dd, J = 13.2, 5.1 Hz, 1H), 4.43 (dd, J = 51.4, 17.6 Hz, 6H), 3.27 - 2.98 (m, 7H), 2.99 - 2.87 (m, 2H), 2.61 (d, J = 16.9 Hz, 1H), 2.42 (dd, J = 13.1, 4.5 Hz, 1H), 2.05 - 1.96 (m, 1H). LCMS (ESI) calcd for $C_{27}H_{29}N_4O_4S^+$ [M+H]$^+$: 505.19, found, 505.2.

**Example 231: Preparation of 3-(5-((4-((4'-fluoro-5,5-dimethyl-3,4,5,6-tetrahydro-[1,1'-biphenyl]-2-yl)methyl)-1,4-diazepan-1-yl)methyl)-1-oxoisoindolin-2-yl)piperidine-2,6-dione (GT-05504)**

Referring to the method of Scheme 1, the target product (GT-05504) was prepared as white solid (5 mg, yield 5.57%). $^1$H NMR (400 MHz, DMSO-d6) δ 11.59 (s, 1H), 11.01 (s, 1H), 10.51 (s, 1H), 7.75 (dd, J = 45.0, 28.0 Hz, 3H), 7.34 - 7.11 (m, 4H), 5.14 (d, J = 8.9 Hz, 1H), 4.43 (dd, J = 50.7, 18.1 Hz, 4H), 3.55 (s, 7H), 3.32 - 2.74 (m, 5H), 2.61 (d, J = 17.6 Hz, 1H), 2.37 (dd, J = 55.3, 18.4 Hz, 3H), 2.03 (d, J = 7.2 Hz, 4H), 1.45 (t, J = 6.1 Hz, 2H), 0.95 (s, 6H). LCMS (ESI) calcd for $C_{34}H_{42}FN_4O_3^+$ [M+H]$^+$: 573.32, found, 573.3.

**Example 232: Preparation of 3-(5-(4-((4'-fluoro-5,5-dimethyl-3,4,5,6-tetrahydro-[1,1'-biphenyl]-2-yl)methyl)-1,4-diazepane-1-carbonyl)-1-oxoisoindolin-2-yl)piperidine-2,6-dione (GT-05505)**

Referring to the methods of Scheme 6 and Example 76, the target product (GT-05505) was prepared as white solid (25 mg, yield 27.17%). $^1$H NMR (400 MHz, DMSO-d6) δ 11.01 (s, 1H), 10.15 (d, J = 44.9 Hz, 1H), 7.85 - 7.75 (m, 1H), 7.60 (d, J = 16.5 Hz, 1H), 7.51 (t, J = 9.0 Hz, 1H), 7.29 - 7.11 (m, 4H), 5.14 (dd, J = 13.2, 4.8 Hz, 1H), 4.42 (ddd, J = 22.3, 20.7, 11.1 Hz, 2H), 4.12 - 3.39 (m, 7H), 3.29 (s, 2H), 3.06 - 2.88 (m, 3H), 2.61 (d, J = 16.5 Hz, 1H), 2.39 (dt, J = 37.5, 16.4 Hz, 4H), 2.04 (d, J = 14.1 Hz, 3H), 1.47 (dd, J = 14.5, 8.2 Hz, 2H), 0.96 (dd, J = 12.5, 5.3 Hz, 6H). LCMS (ESI) calcd for $C_{34}H_{40}FN_4O_4^+$ [M+H]$^+$: 587.30, found, 587.3.

**Example 233: Preparation of 3-(5-((7-((4'-fluoro-5,5-dimethyl-3,4,5,6-tetrahydro-[1,1'-biphenyl]-2-yl)methyl)-2,7-diazaspiro[3.5]nonan-2-yl)methyl)-1-oxoisoindolin-2-yl)piperidine-2,6-dione (GT-05506)**

Referring to the method of Scheme 1, the target product (GT-05506) was prepared as white solid (20 mg, yield 22.88%). $^1$H NMR (400 MHz, DMSO-d6) δ 11.82 (d, J = 5.3 Hz, 1H), 11.01 (s, 1H), 9.91 (s, 1H), 7.83 (s, 1H), 7.78 (d, J = 7.8 Hz, 1H), 7.72 (t, J = 7.8 Hz, 1H), 7.23 - 7.10 (m, 4H), 5.13 (dd, J = 13.2, 5.0 Hz, 1H), 4.58 - 4.30 (m, 4H), 3.97 - 3.66 (m, 4H), 3.47 (s, 2H), 3.36 (s, 3H), 3.34 - 3.30 (m, 2H), 3.19 (t, J = 13.8 Hz, 2H), 2.91 (d, J = 12.8 Hz, 1H), 2.61 (d, J = 15.9 Hz, 1H), 2.43 (dd, J = 13.2, 4.5 Hz, 1H), 2.35 (d, J = 21.3 Hz, 3H), 2.11 (t, J = 11.9 Hz, 3H), 2.02 (s, 3H), 1.45 (t, J = 6.2 Hz, 2H), 0.95 (s, 6H). LCMS (ESI) calcd for $C_{36}H_{44}FN_4O_3^+$ [M+H]$^+$: 599.34, found, 599.3.

**Example 234: Preparation of 3-(5-(7-((4'-fluoro-5,5-dimethyl-3,4,5,6-tetrahydro-[1,1'-biphenyl]-2-yl)methyl)-2,7-diazaspiro[3.5]nonane-2-carbonyl)-1-oxoisoindolin-2-yl)piperidine-2,6-dione (GT-05507)**

Referring to the methods of Scheme 6 and Example 76, the target product (GT-05507) was prepared as white solid (19 mg, yield 21.17%). $^1$H NMR (400 MHz, DMSO-d6) δ 11.01 (s, 1H), 9.86 (d, J = 32.0 Hz, 1H), 7.84 (d, J = 4.4 Hz, 1H), 7.78 (d, J = 7.9 Hz, 1H), 7.73 (d, J = 13.5 Hz, 1H), 7.24 - 7.08 (m, 4H), 5.24 - 5.03 (m, 1H), 4.42 (dd, J = 33.4, 8.7 Hz, 2H), 4.04 (s, 1H), 3.95 (d, J = 5.9 Hz, 1H), 3.77 (d, J = 7.2 Hz, 2H), 3.51 (d, J = 5.2 Hz, 2H), 3.18 (s, 2H), 2.92 (t, J = 14.0 Hz, 1H), 2.61 (d, J = 15.8 Hz, 3H), 2.33 (s, 3H), 2.20 - 1.89 (m, 8H), 1.46 (s, 2H), 0.96 (d, J = 3.6 Hz, 6H). LCMS (ESI) calcd for $C_{36}H_{42}FN_4O_4^+$ [M+H]$^+$: 613.32, found, 613.4.

**Example 235: Preparation of 3-(5-((6-((4'-fluoro-5,5-dimethyl-3,4,5,6-tetrahydro-[1,1'-biphenyl]-2-yl)methyl)-2,6-diazaspiro[3.3]heptan-2-yl)methyl)-1-oxoisoindolin-2-yl)piperidine-2,6-dione (GT-05670)**

Referring to the method of Scheme 1, the target product (GT-05670) was prepared as white solid (6 mg, yield 11.27%). $^1$H NMR (400 MHz, DMSO-d6) δ 11.43 (s, 1H), 11.11 (s, 1H), 11.00 (s, 1H), 7.92 - 7.51 (m, 3H), 7.23 - 7.14 (m, 4H), 5.13 (dd, J = 13.3, 5.0 Hz, 1H), 4.46 - 4.04 (m, 7H), 3.87 (s, 2H), 3.66 (d, J = 49.0 Hz, 3H), 2.93 (dd, J = 21.6, 9.0 Hz, 2H), 2.61

(d, J = 17.0 Hz, 1H), 2.41 (dd, J = 17.6, 8.8 Hz, 1H), 2.21 (d, J = 31.5 Hz, 2H), 2.03 (d, J = 12.7 Hz, 3H), 1.44 (d, J = 21.9 Hz, 2H), 0.95 (d, J = 10.6 Hz, 6H). LCMS (ESI) calcd for $C_{34}H_{40}FN_4O_3^+$ [M+H]$^+$: 571.31, found, 571.3.

**Example 236: Preparation of 3-(5-(6-((4'-fluoro-5,5-dimethyl-3,4,5,6-tetrahydro-[1,1'-biphenyl]-2-yl)methyl)-2,6-diazaspiro[3.3]heptane-2-carbonyl)-1-oxoisoindolin-2-yl)piperidine-2,6-dione (GT-05508)**

Referring to the methods of Scheme 6 and Example 76, the target product (GT-05508) was prepared as white solid (26 mg, yield 28.17%). $^1$H NMR (400 MHz, DMSO-d6) δ 11.01 (s, 1H), 7.91 - 7.65 (m, 3H), 7.32 - 7.13 (m, 4H), 5.14 (dd, J = 13.2, 5.0 Hz, 1H), 4.62 - 4.36 (m, 3H), 4.06 (d, J = 26.1 Hz, 4H), 3.60 (s, 2H), 3.42 (s, 2H), 3.09 - 2.84 (m, 2H), 2.61 (d, J = 17.1 Hz, 1H), 2.45 - 2.35 (m, 1H), 2.13 (s, 2H), 2.03 (s, 3H), 1.43 (dd, J = 13.4, 7.3 Hz, 2H), 0.96 (d, J = 7.7 Hz, 6H). LCMS (ESI) calcd for $C_{34}H_{38}FN_4O_4^+$ [M+H]$^+$: 585.29, found, 585.3.

**Example 237: Preparation of 3-(5-((6-((4'-fluoro-5,5-dimethyl-3,4,5,6-tetrahydro-[1,1'-biphenyl]-2-yl)methyl)-3,6-diazabicyclo[3.1.1]heptan-3-yl)methyl)-1-oxoisoindolin-2-yl)piperidine-2,6-dione (GT-05509)**

Referring to the method of Scheme 1, the target product (GT-05509) was prepared as white solid (16 mg, yield 17.38%). $^1$H NMR (400 MHz, DMSO-d6) δ 11.00 (s, 1H), 9.12 (s, 1H), 8.12 - 7.37 (m, 3H), 7.33 - 7.07 (m, 4H), 5.13 (dd, J = 13.1, 4.7 Hz, 1H), 4.61 - 4.25 (m, 3H), 3.73 (d, J = 55.5 Hz, 2H), 3.42 (s, 2H), 3.34 - 3.18 (m, 2H), 3.10 - 2.82 (m, 2H), 2.61 (d, J = 16.2 Hz, 2H), 2.42 (d, J = 9.0 Hz, 1H), 2.23 (s, 3H), 2.04 (d, J = 15.1 Hz, 4H), 1.43 (t, J = 13.5 Hz, 2H), 0.96 (d, J = 4.6 Hz, 6H). LCMS (ESI) calcd for $C_{34}H_{40}FN_4O_3^+$ [M+H]$^+$: 571.31, found, 571.3.

**Example 238: Preparation of 3-(5-(6-((4'-fluoro-5,5-dimethyl-3,4,5,6-tetrahydro-[1,1'-biphenyl]-2-yl)methyl)-3,6-diazabicyclo[3.1.1]heptane-3-carbonyl)-1-oxoisoindolin-2-yl)piperidine-2,6-dione (GT-05671)**

Referring to the methods of Scheme 6 and Example 76, the target product (GT-05671) was prepared as white solid (6 mg, yield 10.17%). $^1$H NMR (400 MHz, DMSO-d6) δ 11.14 (s, 1H), 11.02 (s, 1H), 10.05 (s, 1H), 7.81 (dd, J = 19.4, 7.9 Hz, 1H), 7.74 - 7.58 (m, 1H), 7.52 (s, 1H), 7.23 (dd, J = 11.3, 5.5 Hz, 4H), 5.30 - 5.01 (m, 1H), 4.46 (ddd, J = 32.6, 22.2, 14.0 Hz, 3H), 4.13 (t, J = 29.2 Hz, 2H), 3.73 (dd, J = 69.2, 45.2 Hz, 6H), 2.94 (dd, J = 22.3, 9.0 Hz, 2H), 2.62 (d, J = 16.8 Hz, 1H), 2.45 (d, J = 12.8 Hz, 1H), 2.19 (s, 3H), 2.05 (s, 3H), 1.82 (d, J = 8.5 Hz, 1H), 1.44 (s, 2H), 0.95 (d, J = 9.7 Hz, 6H). LCMS (ESI) calcd for $C_{34}H_{38}FN_4O_4^+$ [M+H]$^+$: 585.29, found, 585.3.

**Example 239: Preparation of 3-(5-((8-((4'-fluoro-5,5-dimethyl-3,4,5,6-tetrahydro-[1,1'-biphenyl]-2-yl)methyl)-3,8-diazabicyclo[3.2.1]octan-3-yl)methyl)-1-oxoisoindolin-2-yl)piperidine-2,6-dione (GT-05510)**

Referring to the method of Scheme 1, the target product (GT-05510) was prepared as white solid (35 mg, yield 39.17%). $^1$H NMR (400 MHz, DMSO-d6) δ 11.01 (s, 1H), 7.96 - 7.57 (m, 3H), 7.38 - 7.08 (m, 4H), 5.13 (dd, J = 13.3, 5.1 Hz, 1H), 4.41 (dd, J = 52.1, 17.6 Hz, 2H), 4.14 (s, 2H), 3.88 (s, 3H), 3.52 - 3.43 (m, 3H), 2.94 (ddd, J = 30.5, 23.6, 17.6 Hz, 3H), 2.61 (d, J = 16.6 Hz, 1H), 2.50 - 2.35 (m, 3H), 2.19 - 1.95 (m, 5H), 1.47 (t, J = 6.2 Hz, 2H), 1.23 (s, 2H), 0.96 (s, 6H). LCMS (ESI) calcd for $C_{35}H_{42}FN_4O_3^+$ [M+H]$^+$: 585.32, found, 585.4.

**Example 240: Preparation of 3-(5-(8-((4'-fluoro-5,5-dimethyl-3,4,5,6-tetrahydro-[1,1'-biphenyl]-2-yl)methyl)-3,8-diazabicyclo [3.2.1] octane-3-carbonyl)-1-oxoisoindolin-2-yl)piperidine-2,6-dione (GT-05511)**

Referring to the methods of Scheme 6 and Example 76, the target product (GT-05511) was prepared as white solid (50 mg, yield 54.18%). $^1$H NMR (400 MHz, DMSO-d6) δ 11.01 (s, 1H), 10.39 (s, 1H), 7.80 (d, J = 7.8 Hz, 1H), 7.66 (s, 1H), 7.52 (d, J = 7.6 Hz, 1H), 7.20 (d, J = 7.3 Hz, 4H), 5.14 (d, J = 8.2 Hz, 1H), 4.43 (d, J = 31.5 Hz, 3H), 3.93 (s, 2H), 3.51 (s, 5H), 2.97 - 2.86 (m, 1H), 2.61 (d, J = 17.2 Hz, 1H), 2.40 (d, J = 4.8 Hz, 3H), 2.07 (s, 3H), 1.63 (s, 1H), 1.48 (t, J = 6.0 Hz, 3H), 1.26 (d, J = 19.2 Hz, 1H), 1.13 (s, 1H), 0.96 (s, 6H). LCMS (ESI) calcd for $C_{35}H_{40}FN_4O_4^+$ [M+H]$^+$: 599.30, found, 599.3.

**Example 241: Preparation of 3-(5-((5-((4'-fluoro-5,5-dimethyl-3,4,5,6-tetrahydro-[1,1'-biphenyl]-2-yl)methyl)-2,5-diazabicyclo [2.2.2] octan-2-yl)methyl)-1-oxoisoindolin-2-yl)piperidine-2,6-dione (GT-05512)**

Referring to the method of Scheme 1, the target product (GT-05512) was prepared as white solid (23 mg, yield 25.84%). $^1$H NMR (400 MHz, DMSO-d6) δ 11.45 (d, J = 145.6 Hz, 1H), 11.02 (s, 1H), 7.74 (dd, J = 19.1, 11.3 Hz, 3H), 7.21 (s, 4H), 5.14 (dd, J = 13.2, 4.9 Hz, 1H), 4.44 (ddd, J = 51.7, 23.0, 12.1 Hz, 5H), 3.63 (d, J = 64.0 Hz, 6H), 2.92 (dd, J = 21.7, 9.2 Hz, 3H), 2.62 (d, J = 16.6 Hz, 1H), 2.47 - 2.27 (m, 3H), 2.24 - 1.89 (m, 5H), 1.65 - 1.26 (m, 3H), 0.96 (s, 6H). LCMS (ESI) calcd for $C_{35}H_{42}FN_4O_3^+$ [M+H]$^+$: 585.32, found, 585.3.

**Example 242: Preparation of 3-(5-(5-((4'-fluoro-5,5-dimethyl-3,4,5,6-tetrahydro-[1,1'-biphenyl]-2-yl)methyl)-2,5-diazabicyclo [2.2.2] octane-2-carbonyl)-1-oxoisoindolin-2-yl)piperidine-2,6-dione (GT-05513)**

Referring to the methods of Scheme 6 and Example 76, the target product (GT-05513) was prepared as white solid (14 mg, yield 25.16%). $^1$H NMR (400 MHz, DMSO-d6) δ 11.02 (d, J = 8.8 Hz, 1H), 10.34 (d, J = 115.5 Hz, 1H), 7.79 (dt, J = 14.1, 7.1 Hz, 1H), 7.64 - 7.46 (m, 1H), 7.28 - 7.12 (m, 4H), 5.14 (dd, J = 13.2, 3.2 Hz, 1H), 4.57 - 4.33 (m, 2H), 3.93 - 3.48 (m, 6H), 3.24 - 2.79 (m, 4H), 2.61 (d, J = 17.0 Hz, 1H), 2.44 - 2.13 (m, 4H), 2.08 - 1.92 (m, 3H), 1.88 - 1.66 (m, 2H), 1.47 (dd, J = 13.1, 6.6 Hz, 2H), 0.96 (dd, J = 10.4, 4.0 Hz, 6H). LCMS (ESI) calcd for $C_{35}H_{40}FN_4O_4^+$ [M+H]$^+$: 599.30, found, 599.3.

**Example 243: Preparation of 3-(5-((6-(4'-fluoro-5,5-dimethyl-3,4,5,6-tetrahydro-[1,1'-biphenyl]-2-carbonyl)-2,6-diazaspiro[3.3]heptan-2-yl)methyl)-1-oxoisoindolin-2-yl)piperidine-2,6-dione (GT-05664)**

Referring to the method of Scheme 1, the target product (GT-05664) was prepared as white solid (12 mg, yield 16.85%). $^1$H NMR (400 MHz, DMSO-d6) δ 11.01 (s, 1H), 7.77 (d, J = 7.7 Hz, 1H), 7.66 (s, 1H), 7.56 (s, 1H), 7.27 (ddd, J = 12.2, 8.8, 5.6 Hz, 2H), 7.21 - 7.12 (m, 2H), 5.13 (dd, J = 13.3, 5.0 Hz, 1H), 4.41 (dd, J = 51.5, 17.5 Hz, 4H), 4.07 - 3.53 (m, 6H), 3.36 (s, 2H), 3.33 - 3.30 (m, 1H), 3.05 - 2.86 (m, 1H), 2.61 (d, J = 16.8 Hz, 1H), 2.47 - 2.37 (m, 1H), 2.23 (s, 2H), 2.12 (s, 1H), 2.07 - 1.97 (m, 1H), 1.39 (t, J = 6.3 Hz, 2H), 0.97 (s, 6H). LCMS (ESI) calcd for $C_{34}H_{38}FN_4O_4^+$ [M+H]$^+$: 585.29, found, 585.3.

**Example 244: Preparation of 3-(5-((3-(4'-fluoro-5,5-dimethyl-3,4,5,6-tetrahydro-[1,1'-biphenyl]-2-carbonyl)-3,6-diazabicyclo[3.1.1]heptan-6-yl)methyl)-1-oxoisoindolin-2-yl)piperidine-2,6-dione (GT-05514)**

Referring to the method of Scheme 1, the target product (GT-05514) was prepared as white solid (16 mg, yield 18.00%). $^1$H NMR (400 MHz, DMSO-d6) δ 11.49 (s, 1H), 11.01 (s, 1H), 9.99 (d, J = 268.4 Hz, 1H), 7.93 - 7.69 (m, 2H), 7.49 - 7.05 (m, 5H), 5.13 (dd, J = 13.2, 4.9 Hz, 1H), 4.73 - 4.13 (m, 5H), 4.11 - 3.38 (m, 4H), 3.26 - 2.79 (m, 3H), 2.61 (d, J = 16.7 Hz, 1H), 2.32 (ddd, J = 49.8, 33.9, 19.3 Hz, 5H), 2.07 - 1.87 (m, 2H), 1.45 (d, J = 6.1 Hz, 2H), 1.05 - 0.95 (m, 6H). LCMS (ESI) calcd for $C_{34}H_{38}FN_4O_4^+$ [M+H]$^+$: 585.29, found, 585.3.

**Example 245: Preparation of 3-(5-((6-(4'-fluoro-5,5-dimethyl-3,4,5,6-tetrahydro-[1,1'-biphenyl]-2-carbonyl)-3,6-diazabicyclo[3.1.1]heptan-3-yl)methyl)-1-oxoisoindolin-2-yl)piperidine-2,6-dione (GT-05515)**

Referring to the method of Scheme 1, the target product (GT-05515) was prepared as white solid (13 mg, yield 14.61%). $^1$H NMR (400 MHz, DMSO-d6) δ 11.88 (s, 1H), 11.01 (s, 1H), 7.93 (s, 1H), 7.81 (s, 2H), 7.31 - 7.17 (m, 4H), 5.15 (dd, J = 13.2, 5.0 Hz, 1H), 4.40 (dt, J = 50.3, 25.0 Hz, 4H), 3.97 (d, J = 121.6 Hz, 2H), 3.52 (s, 5H), 2.99 - 2.86 (m, 1H), 2.65 (t, J = 27.7 Hz, 2H), 2.48 - 2.37 (m, 1H), 2.23 (d, J = 16.1 Hz, 2H), 1.99 (t, J = 14.6 Hz, 2H), 1.78 (d, J = 19.3 Hz, 1H), 1.60 (d, J = 8.2 Hz, 1H), 1.44 - 1.33 (m, 2H), 1.00 - 0.90 (m, 6H). LCMS (ESI) calcd for $C_{34}H_{38}FN_4O_4^+$ [M+H]$^+$: 585.29, found, 585.3.

**Example 246: Preparation of 3-(5-((3-(4'-fluoro-5,5-dimethyl-3,4,5,6-tetrahydro-[1,1'-biphenyl]-2-carbonyl)-3,8-diazabicyclo [3.2.1] octan-8-yl)methyl)-1-oxoisoindolin-2-yl)piperidine-2,6-dione (GT-05516)**

Referring to the method of Scheme 1, the target product (GT-05516) was prepared as white solid (30 mg, yield 34.00%). $^1$H NMR (400 MHz, DMSO-d6) δ 11.82 (s, 1H), 11.01 (s, 1H), 10.82 (s, 1H), 7.79 (d, J = 6.4 Hz, 3H), 7.39 (s, 1H), 7.16 (dd, J = 20.7, 15.7 Hz, 3H), 5.13 (dd, J = 13.2, 4.8 Hz, 1H), 4.46 (s, 4H), 3.80 (s, 4H), 3.39 - 3.26 (m, 2H), 3.05 - 2.79 (m, 2H), 2.61 (d, J = 17.0 Hz, 1H), 2.43 (d, J = 8.4 Hz, 4H), 2.06 - 1.83 (m, 3H), 1.43 (d, J = 4.8 Hz, 3H), 0.96 (d, J = 8.0 Hz, 6H). LCMS (ESI) calcd for $C_{35}H_{40}FN_4O_4^+$ [M+H]$^+$: 599.30, found, 599.4.

**Example 247: Preparation of 3-(5-((8-(4'-fluoro-5,5-dimethyl-3,4,5,6-tetrahydro-[1,1'-biphenyl]-2-carbonyl)-3,8-diazabicyclo [3.2.1] octan-3-yl)methyl)-1-oxoisoindolin-2-yl)piperidine-2,6-dione (GT-05517)**

Referring to the method of Scheme 1, the target product (GT-05517) was prepared as white solid (40 mg, yield 45.75%). $^1$H NMR (400 MHz, DMSO-d6) δ 11.01 (s, 1H), 10.87 (s, 1H), 7.86 (d, J = 70.5 Hz, 3H), 7.30 - 7.05 (m, 4H), 5.13 (dd, J = 13.2, 3.3 Hz, 1H), 4.39 (dt, J = 32.3, 16.0 Hz, 5H), 3.91 (s, 1H), 3.60 (s, 2H), 3.19 - 2.83 (m, 4H), 2.61 (d, J = 17.2 Hz, 1H), 2.41 (ddd, J = 39.4, 19.7, 15.4 Hz, 4H), 2.09 - 1.80 (m, 4H), 1.44 (dd, J = 13.2, 6.5 Hz, 2H), 1.30 - 1.08 (m, 1H), 0.98 (d, J = 9.7 Hz, 6H). LCMS (ESI) calcd for $C_{35}H_{40}FN_4O_4^+$ [M+H]$^+$: 599.30, found, 599.3.

**Example 248: Preparation of 3-(5-((3-(4'-chloro-[1,1'-biphenyl]-2-carbonyl)-3,6-diazabicyclo[3.1.1]heptan-6-yl) methyl)-1-oxoisoindolin-2-yl)piperidine-2,6-dione (GT-05665)**

Referring to the method of Scheme 1, the target product (GT-05665) was prepared as white solid (35 mg, yield 48.10%). $^1$H NMR (400 MHz, DMSO-d6) δ 11.00 (s, 1H), 9.78 (s, 1H), 8.01 - 7.13 (m, 11H), 5.24 - 5.08 (m, 1H), 4.70 - 4.15 (m, 4H), 3.87 (s, 2H), 3.60 (s, 2H), 3.33 - 3.11 (m, 2H), 2.92 (ddd, J = 13.4, 11.7, 5.0 Hz, 1H), 2.61 (d, J = 16.2 Hz, 1H), 2.48 - 2.11 (m, 2H), 2.02 (s, 1H). LCMS (ESI) calcd for $C_{32}H_{30}ClN_4O_4^+$ [M+H]$^+$: 569.20, found, 569.2.

**Example 249: Preparation of 3-(5-((3-(4'-chloro-[1,1'-biphenyl]-2-carbonyl)-3,6-diazabicyclo[3.1.1]heptan-6-yl) methyl)-4-fluoro-1-oxoisoindolin-2-yl)piperidine-2,6-dione (GT-05666)**

Referring to the method of Scheme 1, the target product (GT-05666) was prepared as white solid (45 mg, yield 60.12%). $^1$H NMR (400 MHz, DMSO-d6) δ 11.02 (d, J = 4.8 Hz, 1H), 9.83 (s, 1H), 7.64 - 7.22 (m, 10H), 5.14 (dd, J = 13.2, 5.0 Hz, 1H), 4.80 - 4.29 (m, 4H), 3.67 (dd, J = 67.6, 63.5 Hz, 3H), 3.33 - 2.85 (m, 4H), 2.61 (d, J = 16.3 Hz, 1H), 2.42 (dd, J = 30.1, 25.2 Hz, 2H), 2.22 - 1.94 (m, 2H). LCMS (ESI) calcd for $C_{32}H_{29}ClFN_4O_4^+$ [M+H]$^+$: 587.19, found, 587.2.

**Example 250: Preparation of 3-(5-((3-(4'-chloro-[1,1'-biphenyl]-2-carbonyl)-3,6-diazabicyclo[3.1.1]heptan-6-yl) methyl)-6-fluoro-1-oxoisoindolin-2-yl)piperidine-2,6-dione (GT-05667)**

Referring to the method of Scheme 1, the target product (GT-05667) was prepared as white solid (35 mg, yield 46.35%). $^1$H NMR (400 MHz, DMSO-d6) δ 11.02 (d, J = 4.7 Hz, 1H), 9.84 (s, 1H), 7.62 - 7.31 (m, 10H), 5.18 - 5.05 (m, 1H), 4.75 - 4.24 (m, 4H), 4.08 - 3.46 (m, 3H), 3.31 - 2.84 (m, 4H), 2.61 (d, J = 15.9 Hz, 1H), 2.48 - 2.31 (m, 2H), 2.03 (d, J = 5.0 Hz, 2H). LCMS (ESI) calcd for $C_{32}H_{29}ClFN_4O_4^+$ [M+H]$^+$: 587.19, found, 587.2.

**Example 251: Preparation of 3-(5-((3-(4'-chloro-[1,1'-biphenyl]-2-carbonyl)-3,6-diazabicyclo[3.1.1]heptan-6-yl) methyl)-7-fluoro-1-oxoisoindolin-2-yl)piperidine-2,6-dione (GT-05668)**

Referring to the method of Scheme 1, the target product (GT-05668) was prepared as white solid (20 mg, yield 26.64%). $^1$H NMR (400 MHz, DMSO-d6) δ 11.02 (d, J = 2.7 Hz, 1H), 10.72 - 9.49 (m, 1H), 7.68 - 7.35 (m, 10H), 5.19 - 5.02 (m, 1H), 4.81 - 4.31 (m, 4H), 4.16 - 3.42 (m, 4H), 3.33 - 2.86 (m, 4H), 2.61 (d, J = 16.4 Hz, 1H), 2.47 - 2.24 (m, 2H), 2.01 (s, 1H). LCMS (ESI) calcd for $C_{32}H_{29}ClFN_4O_4^+$ [M+H]$^+$: 587.19, found, 587.2.

**Example 252: Preparation of 3-(5-((6-(4'-chloro-[1,1'-biphenyl]-2-carbonyl)-3,6-diazabicyclo[3.1.1]heptan-3-yl) methyl)-1-oxoisoindolin-2-yl)piperidine-2,6-dione (GT-05669)**

Referring to the method of Scheme 1, the target product (GT-05669) was prepared as white solid (40 mg, yield 55.10%). $^1$H NMR (400 MHz, DMSO-d6) δ 11.84 (s, 1H), 11.02 (d, J = 5.8 Hz, 1H), 7.87 (d, J = 38.8 Hz, 3H), 7.57 (d, J = 8.3 Hz, 3H), 7.49 - 7.39 (m, 4H), 7.30 (dd, J = 46.2, 7.2 Hz, 1H), 5.16 (ddd, J = 13.5, 8.8, 5.0 Hz, 1H), 4.57 - 4.26 (m, 5H), 3.64 (d, J = 83.3 Hz, 4H), 2.93 (t, J = 12.6 Hz, 1H), 2.77 (s, 1H), 2.62 (d, J = 17.0 Hz, 1H), 2.45 (d, J = 13.2 Hz, 1H), 2.30 (s, 1H), 2.09 - 1.87 (m, 2H). LCMS (ESI) calcd for $C_{32}H_{30}ClN_4O_4^+$ [M+H]$^+$: 569.20, found, 569.3.

**Example 253: Preparation of 3-(4-(4-((4'-chloro-[1,1'-biphenyl]-2-yl)methyl)piperazine-1-carbonyl)-1-oxoisoindolin-2-yl)piperidine-2,6-dione (GT-05486)**

Referring to the method of Scheme 1, the target product (GT-05486) was prepared as white solid (8 mg, yield 213.32%). $^1$H NMR (400 MHz, DMSO-d6) δ 11.07 (s, 1H), 10.99 (s, 1H), 8.09 (s, 1H), 7.83 (d, J = 7.1 Hz, 1H), 7.66 - 7.29 (m, 9H), 5.13 (dd, J = 13.3, 5.0 Hz, 1H), 4.39 (dd, J = 40.4, 17.8 Hz, 4H), 3.61 (s, 3H), 3.26 (s, 3H), 3.06 - 2.72 (m, 3H), 2.59 (d, J = 17.1 Hz, 1H), 2.38 (dt, J = 17.5, 6.8 Hz, 1H), 1.98 (dd, J = 11.5, 6.2 Hz, 1H). LCMS (ESI) calcd for $C_{31}H_{30}ClN_4O_4^+$ [M+H]$^+$: 557.20, found, 557.2.

**Example 254: Preparation of 5-(4-((4'-chloro-5,5-dimethyl-3,4,5,6-tetrahydro-[1,1'-biphenyl]-2-yl)methyl)piperazine-1-carbonyl)-2-(2,6-dioxopiperidin-3-yl)isoindoline-1,3-dione (GT-05366)**

Referring to the methods of Scheme 6 and Example 76, the target product (GT-05366) was prepared as white solid (13 mg, yield 30.80%). $^1$H NMR (400 MHz, DMSO) δ 11.15 (s, 1H), 10.14 (s, 1H), 8.11 - 7.93 (m, 2H), 7.88 (d, *J* = 7.2 Hz, 1H), 7.47 (d, *J* = 7.7 Hz, 2H), 7.15 (d, *J* = 7.9 Hz, 2H), 5.19 (dd, *J* = 12.7, 5.3 Hz, 1H), 4.46 (s, 1H), 3.59 (s, 4H), 3.21 - 3.07 (m, 1H), 3.03 - 2.85 (m, 2H), 2.75 - 2.56 (m, 3H), 2.55 (d, *J* = 6.9 Hz, 1H), 2.10 - 1.96 (m, 3H), 1.47 (s, 2H), 1.26 (dd, *J* = 14.6, 8.2 Hz, 1H), 0.96 (s, 6H). LCMS (ESI) calcd for $C_{33}H_{36}ClN_4O_5^+$ [M+H]$^+$: 603.24, found, 603.3.

**Example 255: Preparation of 5-(3-((4'-chloro-5,5-dimethyl-3,4,5,6-tetrahydro-[1,1'-biphenyl]-2-yl)methyl)-3,6-diazabicyclo[3.1.1]heptane-6-carbonyl)-2-(2,6-dioxopiperidin-3-yl)isoindoline-1,3-dione (GT-05369)**

Referring to the methods of Scheme 6 and Example 76, the target product (GT-05369) was prepared as white solid (10 mg, yield 23.26%). $^1$H NMR (400 MHz, DMSO) δ 11.16 (s, 1H), 10.83 (s, 1H), 8.01 (d, *J* = 7.0 Hz, 2H), 7.83 (d, *J* = 34.1 Hz, 1H), 7.46 (s, 2H), 7.12 (d, *J* = 6.5 Hz, 2H), 5.21 (s, 1H), 4.49 (s, 2H), 3.62 (s, 3H), 2.90 (d, *J* = 13.4 Hz, 2H), 2.80 (s, 1H), 2.65 (s, 1H), 2.60 (s, 2H), 2.41 (s, 1H), 2.33 (s, 2H), 2.05 (s, 3H), 1.93 (s, 1H), 1.45 (s, 2H), 0.95 (s, 6H), 0.82 (s, 1H). LCMS (ESI) calcd for $C_{34}H_{36}ClN_4O_5^+$ [M+H]$^+$: 615.24, found, 615.3 .

**Example 256: Preparation of 5-((1R,4R)-5-((4'-chloro-5,5-dimethyl-3,4,5,6-tetrahydro-[1,1'-biphenyl]-2-yl) methyl)-2,5-diazabicyclo[2.2.1]heptane-2-carbonyl)-2-(2,6-dioxopiperidin-3-yl)isoindoline-1,3-dione (GT-05410)**

Referring to the methods of Scheme 6 and Example 76, the target product (GT-05410) was prepared as white solid (4.6 mg, yield 10.70%). $^1$H NMR (400 MHz, DMSO) δ 11.15 (s, 1H), 7.99 (d, *J* = 31.7 Hz, 3H), 7.55 - 7.37 (m, 2H), 7.19 (d, *J* = 7.6 Hz, 2H), 5.19 (d, *J* = 8.2 Hz, 1H), 4.30 (s, 1H), 3.89 (s, 1H), 3.74 (s, 2H), 3.62 (s, 2H), 3.13 (s, 1H), 2.89 (d, *J* = 12.4 Hz, 2H), 2.30 (d, *J* = 25.2 Hz, 2H), 2.06 (s, 3H), 1.45 (s, 3H), 1.29 - 1.24 (m, 1H), 0.96 (s, 6H). LCMS (ESI) calcd for $C_{34}H_{36}ClN_4O_5^+$ [M+H]$^+$: 615.24, found, 615.3.

**Example 257: Preparation of 5-((1S,4S)-5-((4'-chloro-5,5-dimethyl-3,4,5,6-tetrahydro-[1,1'-biphenyl]-2-yl) methyl)-2,5-diazabicyclo[2.2.1]heptane-2-carbonyl)-2-(2,6-dioxopiperidin-3-yl)isoindoline-1,3-dione (GT-05411)**

Referring to the methods of Scheme 6 and Example 76, the target product (GT-05411) was prepared as white solid (6.7 mg, yield 31.16%). $^1$H NMR (400 MHz, DMSO) δ 11.15 (s, 1H), 8.04 (d, *J* = 9.1 Hz, 2H), 7.94 (d, *J* = 8.3 Hz, 1H), 7.45 (dd, *J* = 18.3, 8.0 Hz, 2H), 7.17 (dd, *J* = 14.0, 7.8 Hz, 2H), 5.19 (dd, *J* = 12.6, 5.0 Hz, 1H), 3.75 (s, 2H), 3.59 (d, *J* = 22.6 Hz, 2H), 3.11 (d, *J* = 11.2 Hz, 1H), 2.93 (d, *J* = 17.3 Hz, 1H), 2.69 - 2.56 (m, 2H), 2.31 (d, *J* = 11.1 Hz, 2H), 2.05 (d, *J* = 11.7 Hz, 4H), 1.45 (s, 3H), 1.29 - 1.22 (m, 1H), 0.97 (s, 6H). LCMS (ESI) calcd for $C_{34}H_{36}ClN_4O_5^+$ [M+H]$^+$: 615.24, found, 615.3 .

**Example 258: Preparation of 5-(8-((4'-chloro-5,5-dimethyl-3,4,5,6-tetrahydro-[1,1'-biphenyl]-2-yl)methyl)-3,8-diazabicyclo[3.2.1]octane-3-carbonyl)-2-(2,6-dioxopiperidin-3-yl)isoindoline-1,3-dione (GT-05412)**

Referring to the methods of Scheme 6 and Example 76, the target product (GT-05412) was prepared as white solid (13.7 mg, yield 31.19%). $^1$H NMR (400 MHz, DMSO) δ 11.14 (s, 1H), 10.19 (s, 1H), 8.00 (d, *J* = 7.9 Hz, 2H), 7.89 (d, *J* = 7.5 Hz, 1H), 7.44 (d, *J* = *8.2* Hz, 2H), 7.18 (d, *J* = 8.2 Hz, 2H), 5.19 (dd, *J* = 12.8, 5.4 Hz, 1H), 3.92 (s, 2H), 3.62 (s, 1H), 3.51 (s, 1H), 2.96 - 2.85 (m, 1H), 2.59 (dd, *J* = 23.2, 11.4 Hz, 2H), 2.37 (s, 2H), 2.07 (s, 3H), 1.66 (d, *J* = 27.4 Hz, 2H), 1.48 (s, 3H), 1.30 - 1.20 (m, 2H), 1.14 (s, 1H), 0.96 (s, 6H). LCMS (ESI) calcd for $C_{35}H_{38}ClN_4O_5^+$ [M+H]$^+$: 629.25, found, 629.3.

**Example 259: Preparation of 2-(2,6-dioxopiperidin-3-yl)-5-(4-((4'-fluoro-3,4,5,6-tetrahydro-[1,1'-biphenyl]-2-yl) methyl)piperazine-1-carbonyl)isoindoline-1,3-dione (GT-05413)**

Referring to the methods of Scheme 6 and Example 76, the target product (GT-05413) was prepared as white solid (15.1 mg, yield 38.45%). $^1$H NMR (400 MHz, DMSO) δ 11.14 (s, 1H), 9.85 (s, 1H), 8.06 - 7.93 (m, 2H), 7.88 (d, *J* = 7.7 Hz, 1H), 7.21 (dt, *J* = 14.2, 8.4 Hz, 4H), 5.19 (dd, *J* = 12.7, 5.4 Hz, 1H), 4.45 (s, 1H), 3.58 (d, *J* = 18.0 Hz, 4H), 3.38 (s, 1H), 3.32 - 3.16 (m, 2H), 2.98 - 2.81 (m, 2H), 2.76 - 2.53 (m, 3H), 2.28 (d, *J* = 28.8 Hz, 4H), 2.11 - 2.03 (m, 1H), 1.73 (d, *J* = 22.6 Hz, 4H). LCMS (ESI) calcd for $C_{31}H_{32}FN_4O_5^+$ [M+H]$^+$: 559.24, found, 559.3 .

**Example 260: Preparation of 5-(5-((4'-chloro-5,5-dimethyl-3,4,5,6-tetrahydro-[1,1'-biphenyl]-2-yl)methyl)-2,5-diazabicyclo[2.2.2]octane-2-carbonyl)-2-(2,6-dioxopiperidin-3-yl)isoindoline-1,3-dione (GT-05414)**

Referring to the methods of Scheme 6 and Example 76, the target product (GT-05414) was prepared as white solid (10.7 mg, yield 24.36%). $^1$H NMR (400 MHz, DMSO) δ 11.14 (s, 1H), 8.01 (dd, *J* = 29.6, 21.8 Hz, 2H), 7.87 (s, 1H), 7.55 - 7.43 (m, 2H), 7.29 - 7.06 (m, 3H), 5.19 (d, *J* = 13.3 Hz, 1H), 3.72 (d, *J* = 25.5 Hz, 4H), 2.94 (dd, *J* = 44.0, 11.5 Hz, 3H), 2.61 (d, *J* = 18.5 Hz, 2H), 2.34 - 2.16 (m, 3H), 2.12 - 2.02 (m, 3H), 1.91 (s, 1H), 1.62 (d, *J* = 15.3 Hz, 1H), 1.48 (d, *J* = 7.1 Hz, 2H), 1.26 (t, *J* = 6.7 Hz, 1H), 0.95 (s, 6H). LCMS (ESI) calcd for $C_{35}H_{38}ClN_4O_5^+$ [M+H]$^+$: 629.25, found, 629.3 .

**Example 261: Preparation of 2-(2,6-dioxopiperidin-3-yl)-5-(4-(4'-fluoro-3,4,5,6-tetrahydro-[1,1'-biphenyl]-2-carbonyl)piperazine-1-carbonyl)isoindoline-1,3-dione (GT-05415)**

Referring to the methods of Scheme 6 and Example 76, the target product (GT-05415) was prepared as white solid (18 mg, yield 47.63%). $^1$H NMR (400 MHz, DMSO) δ 11.14 (s, 1H), 7.96 (d, *J* = 7.6 Hz, 1H), 7.88 (s, 1H), 7.81 (d, *J* = 7.3 Hz, 1H), 7.29 (s, 2H), 7.16 (s, 2H), 5.18 (dd, *J* = 12.8, 5.3 Hz, 1H), 3.52 (s, 1H), 3.13 (d, *J* = 33.7 Hz, 4H), 2.89 (dd, *J* = 20.0, 10.0 Hz, 1H), 2.68 - 2.54 (m, 3H), 2.33 (s, 1H), 2.14 - 1.96 (m, 3H), 1.65 (d, *J* = 41.0 Hz, 5H), 1.32 - 1.17 (m, 1H), 1.14 - 0.98 (m, 1H). LCMS (ESI) calcd for $C_{31}H_{30}FN_4O_6^+$ [M+H]$^+$: 573.21, found, 573.3 .

**Example 262: Preparation of 2-(2,6-dioxopiperidin-3-yl)-5-(3-(4'-fluoro-3,4,5,6-tetrahydro-[1,1'-biphenyl]-2-carbonyl)-3,6-diazabicyclo[3.1.1]heptane-6-carbonyl)isoindoline-1,3-dione (GT-05416)**

Referring to the methods of Scheme 6 and Example 76, the target product (GT-05416) was prepared as white solid (10 mg, yield 25.85%). $^1$H NMR (400 MHz, DMSO) δ 11.15 (s, 1H), 8.00 (s, 2H), 7.81 - 7.63 (m, 1H), 7.30 (s, 2H), 7.11 (t, *J* = 25.6 Hz, 2H), 5.19 *(d, J* = 12.4 Hz, 1H), 4.57 - 4.24 (m, 2H), 3.41 (s, 1H), 3.31 - 3.24 (m, 2H), 3.08 (s, 1H), 2.89 (d, *J* = 14.0 Hz, 1H), 2.71 - 2.52 (m, 4H), 2.33 (s, 1H), 2.09 (s, 3H), 1.63 (t, *J* = 44.0 Hz, 5H). LCMS (ESI) calcd for $C_{32}H_{30}FN_4O_6^+$ [M+H]$^+$: 585.21, found, 585.3 .

**Example 263: Preparation of 3-(5-((4-(4'-chloro-5,5-dimethyl-3,4,5,6-tetrahydro-[1,1'-biphenyl]-2-carbonyl)piperazin-1-yl)amino)-1-oxoisoindolin-2-yl)piperidine-2,6-dione (GT-05417)**

Referring to the method of Scheme 6, the target product (GT-05417) was prepared as white solid (33 mg, yield 46.61%). $^1$H NMR (400 MHz, DMSO) δ 10.92 (s, 1H), 7.45 - 7.35 (m, 3H), 7.25 (d, *J* = 8.5 Hz, 2H), 6.87 (s, 1H), 6.79 (d, *J* = 8.3 Hz, 1H), 5.01 (dd, *J* = 13.2, 5.1 Hz, 1H), 4.27 (d, *J* = 17.1 Hz, 1H), 4.14 (d, *J* = 16.9 Hz, 1H), 3.50 - 3.33 (m, 4H), 2.91 - 2.82 (m, 1H), 2.57 (d, *J* = 18.2 Hz, 4H), 2.39 (s, 1H), 2.40 - 2.28 (m, 3H), 2.13 (s, 1H), 1.98 - 1.86 (m, 2H), 1.46 (d, *J* = 9.7 Hz, 2H), 0.98 (s, 6H). LCMS (ESI) calcd for $C_{32}H_{37}ClN_5O_4^+$ [M+H]$^+$: 590.25, found, 590.3.

**Example 264: Preparation of 3-(5-((1-(4'-chloro-5,5-dimethyl-3,4,5,6-tetrahydro-[1,1'-biphenyl]-2-carbonyl)piperidin-4-yl)methyl)-1-oxoisoindolin-2-yl)piperidine-2,6-dione** (GT-**05418)**

Referring to the method of Scheme 6, the target product (GT-05418) was prepared as white solid (10 mg, yield 15.01%). $^1$H NMR (400 MHz, DMSO) δ 10.98 (s, 1H), 7.62 (d, *J* = 7.6 Hz, 1H), 7.46 (d, *J* = 8.5 Hz, 1H), 7.41 - 7.19 (m, 4H), 7.19 - 7.14 (m, 1H), 5.10 (dd, *J* = 13.0, 4.9 Hz, 1H), 4.42 (d, *J* = 17.2 Hz, 1H), 4.33 - 4.08 (m, 2H), 3.43 (d, *J* = 13.0 Hz, 2H), 3.32 - 3.28 (m, 1H), 2.92 - 2.86 (m, 1H), 2.75 (d, *J* = 12.6 Hz, 1H), 2.65 - 2.55 (m, 2H), 2.36 (dt, *J* = 31.3, 20.7 Hz, 5H), 2.20 - 2.11 (m, 1H), 1.97 (dd, *J* = 16.6, 11.1 Hz, 2H), 1.58 (s, 1H), 1.42 (d, *J* = 5.3 Hz, 3H), 0.97 (s, 6H). LCMS (ESI) calcd for $C_{34}H_{39}ClN_3O_4^+$ [M+H]$^+$: 588.26, found, 588.3.

**Example 265: Preparation of 3-(5-((1-(4'-chloro-5,5-dimethyl-3,4,5,6-tetrahydro-[1,1'-biphenyl]-2-carbonyl)azetidin-3-yl)methyl)-1-oxoisoindolin-2-yl)piperidine-2,6-dione (GT-05419)**

Referring to the method of Scheme 6, the target product (GT-05419) was prepared as white solid (16 mg, yield 25.28%). $^1$H NMR (400 MHz, DMSO) δ 10.98 (s, 1H), 7.62 (d, *J* = 7.7 Hz, 1H), 7.47 (dd, *J* = 8.6, 2.5 Hz, 2H), 7.30 (d, *J* = 8.5 Hz, 2H), 7.22 (s, 1H), 7.15 (d, *J* = 7.8 Hz, 1H), 5.10 (dd, *J* = 13.1, 4.8 Hz, 1H), 4.46 - 4.19 (m, 2H), 3.78 - 3.60 (m, 2H), 3.26 (dd, *J* = 9.9, 4.4 Hz, 1H), 3.04 - 2.97 (m, 1H), 2.90 (d, *J* = 12.2 Hz, 1H), 2.58 (t, *J* = 13.9 Hz, 3H), 2.54 (s, 1H), 2.46 - 2.37 (m, 2H), 2.27 (s, 1H), 2.21 (d, *J* = 17.5 Hz, 2H), 2.01 (dd, *J* = 19.2, 11.6 Hz, 2H), 1.39 (t, *J* = 6.4 Hz, 2H), 0.95 (s, 6H). LCMS (ESI) calcd for $C_{32}H_{35}ClN_3O_4^+$ [M+H]$^+$: 560.23, found, 560.2.

**Example 266: Preparation of 3-(5-((1-(4'-chloro-5,5-dimethyl-3,4,5,6-tetrahydro-[1,1'-biphenyl]-2-carbonyl)azetidin-3-ylidene)methyl)-1-oxoisoindolin-2-yl)piperidine-2,6-dione (GT-05420)**

Referring to the method of Scheme 6, the target product (GT-05420) was prepared as white solid (18 mg, yield 28.46%). $^1$H NMR (400 MHz, DMSO) δ 10.98 (d, *J* = 5.9 Hz, 1H), 7.66 (dd, *J* = 11.3, 7.9 Hz, 1H), 7.41 - 7.34 (m, 2H), 7.32 - 7.26 (m, 2H), 7.20 (dd, *J* = 20.1, 9.7 Hz, 1H), 6.41 (d, *J* = 16.4 Hz, 1H), 5.11 (dd, *J* = 12.4, 6.8 Hz, 1H), 4.74 - 4.45 (m, 2H), 4.45 - 4.27 (m, 3H), 2.89 (dd, *J* = 11.6, 6.0 Hz, 1H), 2.60 (d, *J* = 16.4 Hz, 3H), 2.42 - 2.29 (m, 3H), 2.14 (s, 2H), 2.00 (d, *J* = 5.0 Hz, 1H), 1.46 - 1.41 (m, 1H), 0.98 (s, 6H). LCMS (ESI) calcd for $C_{32}H_{33}ClN_3O_4^+$ [M+H]$^+$: 558.22, found, 558.3.

**Example 267: Preparation of 3-(5-((1-(4'-chloro-5,5-dimethyl-3,4,5,6-tetrahydro-[1,1'-biphenyl]-2-carbonyl)-3-hydroxyazetidin-3-yl)(hydroxy)methyl)-1-oxoisoindolin-2-yl)piperidine-2,6-dione (GT-05426)**

Referring to the method of Scheme 6, the target product (GT-05426) was prepared as white solid (27 mg, yield 40.24%). $^1$H NMR (400 MHz, DMSO-d6) δ 11.00 (s, 1H), 7.63 (dd, *J* = 7.8, 2.8 Hz, 1H), 7.59 - 7.32 (m, 4H), 7.27 (dd, *J* = 11.8, 8.5 Hz, 2H), 5.72 (s, 1H), 5.11 (dd, *J* = 13.3, 4.9 Hz, 1H), 4.43 (dd, *J* = 17.1, 5.5 Hz, 1H), 4.35 - 4.16 (m, 2H), 3.89 (t, *J* = 10.6 Hz, 1H), 3.65 (d, *J* = 9.2 Hz, 1H), 3.33 - 3.17 (m, 2H), 2.99 - 2.86 (m, 1H), 2.60 (d, *J* = 16.9 Hz, 1H), 2.48 - 2.30 (m, 1H), 2.22 - 1.97 (m, 4H), 1.39 (d, *J*= 5.3 Hz, 2H), 0.79 (s, 6H). LCMS (ESI) calcd for $C_{32}H_{35}ClN_3O_6^+$ [M+H]$^+$: 592.22, found, 592.3.

**Example 268: Preparation of 3-(5-((1-((4'-chloro-5,5-dimethyl-3,4,5,6-tetrahydro-[1,1'-biphenyl]-2-yl)methyl)-3-hydroxyazetidin-3-yl)(hydroxy)methyl)-1-oxoisoindolin-2-yl)piperidine-2,6-dione (GT-05427)**

Referring to the method of Scheme 4, the target product (GT-05427) was prepared as white solid (17 mg, yield 13.76%). $^1$H NMR (400 MHz, DMSO-d6) δ 11.01 (s, 1H), 10.26 (s, 1H), 7.67 (dd, *J* = 7.8, 4.6 Hz, 1H), 7.61 - 7.50 (m, 2H), 7.44 (t, *J* = 7.1 Hz, 1H), 7.24 (d, *J* = 8.3 Hz, 1H), 7.21 - 7.11 (m, 1H), 6.24 - 6.18 (m, 1H), 5.12 (d, *J* = 13.2 Hz, 1H), 4.41 (ddd, *J* = 38.6, 16.2, 9.0 Hz, 3H), 4.03 (dd, *J* = 27.4, 5.4 Hz, 1H), 3.72 (d, *J* = 5.4 Hz, 1H), 3.65 - 3.40 (m, 2H), 2.98 - 2.85 (m, 1H), 2.57 (dd, *J* = 25.9, 10.7 Hz, 2H), 2.40 (d, *J* = 13.4 Hz, 1H), 2.15 (d, *J* = 19.5 Hz, 2H), 2.03 (t, *J* = 20.7 Hz, 3H), 1.43 (d, *J* = 6.4 Hz, 2H), 0.87 (s, 6H). LCMS (ESI) calcd for $C_{32}H_{37}ClN_3O_5^+$ [M+H]$^+$: 578.24, found, 578.2.

**Example 269: Preparation of 3-(5-((1-((4'-chloro-5,5-dimethyl-3,4,5,6-tetrahydro-[1,1'-biphenyl]-2-yl)methyl) azetidin-3-yl)methyl)-1-oxoisoindolin-2-yl)piperidine-2,6-dione (GT-05428)**

Referring to the method of Scheme 4, the target product (GT-05428) was prepared as white solid (4 mg, yield 4.26%). $^1$H NMR (400 MHz, DMSO-d6) δ 11.00 (s, 1H), 7.66 (dd, *J* = 7.7, 2.9 Hz, 1H), 7.50 - 7.40 (m, 3H), 7.33 (d, *J* = 10.9 Hz, 1H), 7.18 (ddd, *J* = 22.2, 15.1, 8.2 Hz, 3H), 5.11 (dd, *J* = 13.2, 5.1 Hz, 1H), 4.43 (d, *J* = 17.4 Hz, 1H), 4.30 (d, *J* = 17.5 Hz, 1H), 4.03 (s, 1H), 3.82 (d, *J* = 6.5 Hz, 1H), 3.64 (s, 2H), 3.58 (d, *J* = 6.5 Hz, 1H), 3.17 (d, *J* = 8.3 Hz, 1H), 3.02 - 2.85 (m, 2H), 2.82 (d, *J* = 7.6 Hz, 1H), 2.62 - 2.54 (m, 2H), 2.45 - 2.34 (m, 1H), 2.16 (s, 2H), 2.03 (d, *J* = 11.2 Hz, 3H), 1.46 - 1.38 (m, 2H), 0.93 (s, 6H). LCMS (ESI) calcd for $C_{32}H_{37}ClN_3O_3^+$ [M+H]$^+$: 546.25, found, 546.3.

**Example 270: Preparation of 3-(5-(8-((4'-chloro-5,5-dimethyl-3,4,5,6-tetrahydro-[1,1'-biphenyl]-2-yl)methyl)-3,8-diazabicyclo [3.2.1] octan-3-yl)-1-oxoisoindolin-2-yl)piperidine-2,6-dione (GT-05429)**

Referring to the method of Scheme 4, the target product (GT-05429) was prepared as white solid (8.5 mg, yield 6.78%). $^1$H NMR (400 MHz, DMSO-d6) δ 10.96 (s, 1H), 10.20 (s, 1H), 7.55 (d, *J* = 8.5 Hz, 1H), 7.46 (dd, *J* = 13.4, 8.5 Hz, 2H), 7.25 (d, *J* = 8.4 Hz, 2H), 7.08 - 6.91 (m, 2H), 5.05 (dd, *J* = 13.2, 5.0 Hz, 1H), 4.27 (dd, *J* = 50.8, 17.0 Hz, 2H), 3.91 (s, 2H), 3.75 (d, *J*= 12.1 Hz, 2H), 3.60 (d, *J* = 5.2 Hz, 1H), 3.51 (d, *J* = 12.2 Hz, 2H), 2.89 (dd, *J* = 21.6, 9.5 Hz, 1H), 2.70 - 2.53 (m, 2H), 2.37 (dd, *J* = 23.6, 9.9 Hz, 3H), 2.12 (s, 2H), 2.02 - 1.82 (m, 2H), 1.76 (d, *J* = 8.3 Hz, 1H), 1.50 (dd, *J* = 15.3, 9.3 Hz, 2H), 1.33 (s, 1H), 0.98 (d, *J* = 12.9 Hz, 6H). LCMS (ESI) calcd for $C_{34}H_{40}ClN_4O_3^+$ [M+H]$^+$: 587.28, found, 587.3.

**Example 271: Preparation of 3-(5-(3-((4'-chloro-5,5-dimethyl-3,4,5,6-tetrahydro-[1,1'-biphenyl]-2-yl)methyl)-3,8-diazabicyclo [3.2.1] octan-8-yl)-1-oxoisoindolin-2-yl)piperidine-2,6-dione (GT-05430)**

Referring to the method of Scheme 4, the target product (GT-05430) was prepared as white solid (21 mg, yield 16.75%). $^1$H NMR (400 MHz, DMSO-d6) δ 10.97 (s, 1H), 10.11 (s, 1H), 7.58 (d, *J* = 8.4 Hz, 1H), 7.27 (d, *J* = 8.3 Hz, 2H), 7.06 - 6.99 (m, 3H), 6.96 (d, *J* = 8.7 Hz, 1H), 5.10 (dd, *J* = 13.2, 4.9 Hz, 1H), 4.50 (s, 2H), 4.30 (dd, *J* = 38.0, 16.9 Hz, 2H), 3.01 (d, *J* = 10.6 Hz, 2H), 2.84 (dd, *J* = 24.4, 12.5 Hz, 2H), 2.60 (d, *J* = 16.8 Hz, 1H), 2.42 (dd, *J* = 13.2, 8.7 Hz, 1H), 2.24 (d, *J* = 8.0 Hz, 2H), 2.08 (d, *J* = 29.8 Hz, 4H), 1.95 (s, 3H), 1.36 (d, *J* = 5.9 Hz, 2H), 0.96 (s, 1H), 0.84 (d, *J* = 3.2 Hz, 6H). LCMS (ESI) calcd for $C_{34}H_{40}ClN_4O_3^+$ [M+H]$^+$: 587.28, found, 587.3.

**Example 272: Preparation of 3-(5-(3-((4'-chloro-5,5-dimethyl-3,4,5,6-tetrahydro-[1,1'-biphenyl]-2-yl)methyl)-3,6-diazabicyclo[3.1.1]heptane-6-carbonyl)-1-oxoisoindolin-2-yl)piperidine-2,6-dione (GT-05440)**

Referring to the methods of Scheme 6 and Example 76, the target product (GT-05440) was prepared as white solid (14 mg, yield 31.82%). $^1$H NMR (400 MHz, DMSO-d6) δ 11.04 (s, 1H), 10.61 (s, 1H), 7.89 - 7.76 (m, 1H), 7.60 (s, 1H), 7.54 - 7.23 (m, 3H), 7.11 (d, *J* = 7.5 Hz, 2H), 5.16 (dd, *J* = 13.3, 4.7 Hz, 1H), 4.47 (dd, *J* = 40.8, 17.2 Hz, 3H), 3.65 (s, 2H), 3.53 (s, 1H), 2.96 - 2.87 (m, 1H), 2.77 (s, 1H), 2.62 (d, *J* = 17.3 Hz, 2H), 2.45 (s, 2H), 2.28 (s, 3H), 2.02 (d, *J* = 24.3 Hz, 3H), 1.45 (s, 2H), 0.96 (s, 6H). LCMS (ESI) calcd for $C_{34}H_{38}ClN_4O_4^+$ [M+H]$^+$: 601.26, found, 601.3.

**Example 273: Preparation of 3-(5-((1R,4R)-5-((4'-chloro-5,5-dimethyl-3,4,5,6-tetrahydro-[1,1'-biphenyl]-2-yl) methyl)-2,5-diazabicyclo[2.2.1]heptane-2-carbonyl)-1-oxoisoindolin-2-yl)piperidine-2,6-dione (GT-05441)**

Referring to the methods of Scheme 6 and Example 76, the target product (GT-05441) was prepared as white solid (23 mg, yield 52.28%). $^1$H NMR (400 MHz, DMSO-d6) δ 11.03 (s, 1H), 9.91 (s, 1H), 7.82 (d, *J* = 7.3 Hz, 2H), 7.71 (d, *J* = 8.9 Hz, 1H), 7.50 - 7.42 (m, 2H), 7.17 (dd, *J* = 12.4, 8.3 Hz, 2H), 5.17 - 5.10 (m, 1H), 4.48 (dd, *J* = 31.3, 18.2 Hz, 2H), 4.39 (s, 1H), 3.77 (d, *J* = 10.9 Hz, 2H), 3.66 (s, 1H), 3.19 - 3.07 (m, 1H), 3.04 - 2.88 (m, 2H), 2.71 - 2.56 (m, 2H), 2.42 (d, *J* = 8.4 Hz, 1H), 2.26 (s, 2H), 2.03 (d, *J* = 8.1 Hz, 3H), 1.46 (d, *J* = 6.6 Hz, 3H), 0.95 (s, 6H). LCMS (ESI) calcd for $C_{34}H_{33}ClN_4O_4^+$ $[M+H]^+$: 601.26, found, 601.3.

**Example 274: Preparation of 3-(5-((1S,4S)-5-((4'-chloro-5,5-dimethyl-3,4,5,6-tetrahydro-[1,1'-biphenyl]-2-yl) methyl)-2,5-diazabicyclo[2.2.1]heptane-2-carbonyl)-1-oxoisoindolin-2-yl)piperidine-2,6-dione (GT-05442)**

Referring to the methods of Scheme 6 and Example 76, the target product (GT-05442) was prepared as white solid (7 mg, yield 15.91%). $^1$H NMR (400 MHz, DMSO-d6) δ 11.03 (s, 1H), 10.21 (d, *J* = 58.5 Hz, 1H), 7.85 - 7.76 (m, 2H), 7.72 (s, 1H), 7.51 - 7.44 (m, 2H), 7.17 (dd, *J* = 11.7, 8.4 Hz, 2H), 5.15 (dd, *J* = 13.4, 5.0 Hz, 1H), 4.55 - 4.36 (m, 3H), 3.68 (d, *J* = *1*5.5 Hz, 3H), 3.15 (dd, *J* = 25.8, 11.4 Hz, 1H), 2.95 (dd, *J* = 32.8, 13.1 Hz, 2H), 2.61 (d, *J* = 15.7 Hz, 2H), 2.41 (s, 1H), 2.30 (d, *J* = 27.9 Hz, 2H), 2.03 (s, 3H), 1.44 (s, 3H), 0.95 (s, 6H). LCMS (ESI) calcd for $C_{34}H_{38}ClN_4O_4^+$ $[M+H]^+$: 601.26, found, 601.3.

**Example 275: Preparation of 3-(5-(4-((4'-chloro-5,5-dimethyl-3,4,5,6-tetrahydro-[1,1'-biphenyl]-2-yl)methyl)piperazine-1-carbonyl)-1-oxoisoindolin-2-yl)piperidine-2,6-dione (GT-05445)**

Referring to the methods of Scheme 6 and Example 76, the target product (GT-05445) was prepared as white solid (25 mg, yield 57.92%). $^1$H NMR (400 MHz, DMSO-d6) δ 11.03 (s, 1H), 9.28 (s, 1H), 7.80 (d, *J* = 7.8 Hz, 1H), 7.65 (s, 1H), 7.54 (d, *J* = 7.6 Hz, 1H), 7.46 (d, *J* = 8.3 Hz, 2H), 7.14 (d, *J* = *8.3* Hz, 2H), 5.14 (dd, *J* = 13.3, 5.1 Hz, 1H), 4.47 (s, 1H), 4.37 (d, *J* = 17.7 Hz, 1H), 3.61 (s, 3H), 3.47 (s, 2H), 3.17 (s, 2H), 2.95 - 2.84 (m, 2H), 2.61 (d, *J* = 16.8 Hz, 1H), 2.42 (dd, *J* = 13.1, 4.2 Hz, 1H), 2.22 (s, 2H), 2.01 (d, *J* = 13.8 Hz, 3H), 1.48 (s, 2H), 0.97 (s, 6H). LCMS (ESI) calcd for $C_{33}H_{38}ClN_4O_4^+$ $[M+H]^+$: 589.26, found, 589.3.

**Example 276: Preparation of 3-(5-(8-((4'-chloro-5,5-dimethyl-3,4,5,6-tetrahydro-[1,1'-biphenyl]-2-yl)methyl)-3,8-diazabicyclo [3.2.1] octane-3-carbonyl)-1-oxoisoindolin-2-yl)piperidine-2,6-dione (GT-05446)**

Referring to the methods of Scheme 6 and Example 76, the target product (GT-05446) was prepared as white solid (21 mg, yield 46.71%). $^1$H NMR (400 MHz, DMSO-d6) δ 11.01 (s, 1H), 9.93 (s, 1H), 7.80 (d, *J* = 7.8 Hz, 1H), 7.67 (s, 1H), 7.53 (d, *J* = 7.5 Hz, 1H), 7.44 (d, *J* = 8.2 Hz, 2H), 7.18 (d, *J* = 8.2 Hz, 2H), 5.14 (dd, *J* = 13.3, 5.1 Hz, 1H), 4.49 (d, *J* = 17.9 Hz, 1H), 4.37 (d, *J* = 17.8 Hz, 1H), 3.88 (d, *J* = 46.0 Hz, 2H), 3.64 (s, 1H), 3.51 (s, 3H), 3.38 (s, 2H), 2.93 (dd, *J* = 22.1, 9.3 Hz, 1H), 2.58 (dd, *J* = 24.1, 11.3 Hz, 2H), 2.42 (dd, *J* = 13.1, 4.5 Hz, 1H), 2.34 (s, 2H), 2.07 (s, 2H), 2.03 - 1.96 (m, 1H), 1.65 (s, 1H), 1.48 (s, 3H), 1.37 - 1.21 (m, 2H), 1.16 (s, 1H), 0.96 (s, 7H). LCMS (ESI) calcd for $C_{35}H_{40}ClN_4O_4^+$ $[M+H]^+$: 615.27, found, 615.3.

**Example 277: Preparation of 3-(5-(5-((4'-fluoro-5,5-dimethyl-3,4,5,6-tetrahydro-[1,1'-biphenyl]-2-yl)methyl)octahydropyrrolo[3,4-c]pyrrole-2-carbonyl)-1-oxoisoindolin-2-yl)piperidine-2,6-dione (GT-05447)**

Referring to the methods of Scheme 6 and Example 76, the target product (GT-05447) was prepared as white solid (32 mg, yield 48.44%). $^1$H NMR (400 MHz, DMSO-d6) δ 11.02 (s, 1H), 9.57 *(d, J* = 62.2 Hz, 1H), 7.80 (d, *J* = 7.8 Hz, 1H), 7.65 - 7.54 (m, 1H), 7.22 (t, *J* = 8.4 Hz, 2H), 7.18 - 7.12 (m, 2H), 5.15 (dd, *J* = 13.3, 4.9 Hz, 1H), 4.51 (d, *J* = 17.6 Hz, 1H), 4.38 (dd, *J* = 17.5, 4.8 Hz, 1H), 3.68 (d, *J* = 5.0 Hz, 2H), 3.55 (d, *J* = 37.8 Hz, 4H), 3.22 - 3.05 (m, 2H), 3.00 - 2.90 (m, 2H), 2.62 (d, *J* = 18.0 Hz, 2H), 2.43 (dd, *J* = 13.1, 5.0 Hz, 1H), 2.21 (s, 2H), 2.03 (s, 3H), 1.47 (d, *J* = 5.5 Hz, 2H), 0.96 (s, 6H). LCMS (ESI) calcd for $C_{35}H_{40}FN_4O_4^+$ $[M+H]^+$: 599.30, found, 599.3.

Example 278: Preparation of 3-(5-(4-(4'-fluoro-3,4,5,6-tetrahydro-[1,1'-biphenyl]-2-carbonyl)piperazine-1-carbonyl)-1-oxoisoindolin-2-yl)piperidine-2,6-dione (GT-05448)

Referring to the methods of Scheme 6 and Example 76, the target product (GT-05448) was prepared as white solid (21 mg, yield 54.18%). $^1$H NMR (400 MHz, DMSO-d6) δ 11.03 (s, 1H), 7.75 (t, *J* = 21.3 Hz, 2H), 7.49 (d, *J* = 22.2 Hz, 1H), 7.30 (s, 2H), 7.14 (s, 2H), 5.15 (d, *J* = 13.2 Hz, 1H), 4.46 (dt, *J* = 27.6, 17.3 Hz, 4H), 4.03 - 3.73 (m, 1H), 3.55 (d, *J* = 12.6 Hz, 1H), 3.22 (dd, *J* = 37.7, 20.5 Hz, 1H), 3.03 *(d, J* = 11.0 Hz, 1H), 2.92 (s, 1H), 2.61 (d, *J* = 17.6 Hz, 3H), 2.33 (s, 2H), 2.14 (s,

1H), 2.03 (s, 2H), 1.73 (s, 2H), 1.63 (s, 2H), 1.51 (d, *J* = 9.0 Hz, 1H). LCMS (ESI) calcd for $C_{31}H_{32}FN_4O_5^+$ [M+H]+: 559.24, found, 559.3.

**Example 279: Preparation of 3-(5-(3-(4'-fluoro-3,4,5,6-tetrahydro-[1,1'-biphenyl]-2-carbonyl)-3,6-diazabicyclo [3.1.1]heptane-6-carbonyl)-1-oxoisoindolin-2-yl)piperidine-2,6-dione (GT-05449)**

Referring to the methods of Scheme 6 and Example 76, the target product (GT-05449) was prepared as white solid (19 mg, yield 47.99%). $^1$H NMR (400 MHz, DMSO-d6) δ 11.02 (s, 1H), 7.76 (d, *J* = 7.8 Hz, 1H), 7.58 (s, 1H), 7.45 (d, *J* = 7.2 Hz, 1H), 7.29 (s, 2H), 7.18 (d, *J* = 7.4 Hz, 2H), 5.13 (dd, *J* = 13.2, 5.0 Hz, 1H), 4.48 (d, *J* = 17.6 Hz, 1H), 4.35 (d, *J* = 17.6 Hz, 1H), 3.61 (d, *J* = 68.7 Hz, 2H), 3.18 (d, *J* = 53.0 Hz, 4H), 3.00 - 2.76 (m, 2H), 2.60 (d, *J* = 17.4 Hz, 2H), 2.47 - 2.20 (m, 3H), 2.02 (dd, *J* = 19.2, 13.6 Hz, 3H), 1.71 (s, 4H). LCMS (ESI) calcd for $C_{32}H_{32}FN_4O_5^+$ [M+H]+: 571.24, found, 571.2.

**Example 280: Preparation of 3-(5-(3-((4'-fluoro-5,5-dimethyl-3,4,5,6-tetrahydro-[1,1'-biphenyl]-2-yl)methyl)-3,6-diazabicyclo[3.1.1]heptane-6-carbonyl)-1-oxoisoindolin-2-yl)piperidine-2,6-dione (GT-05450)**

Referring to the methods of Scheme 6 and Example 76, the target product (GT-05450) was prepared as white solid (14 mg, yield 21.67%). $^1$H NMR (400 MHz, DMSO-d6) δ 11.03 (s, 1H), 10.81 (s, 1H), 7.80 (d, *J* = 7.8 Hz, 1H), 7.60 (s, 1H), 7.44 (s, 1H), 7.20 (d, *J* = 8.1 Hz, 3H), 7.12 (s, 2H), 5.16 (dd, *J* = 13.1, 4.7 Hz, 1H), 4.70 (s, 1H), 4.46 (dd, *J* = 40.5, 17.5 Hz, 3H), 3.64 (s, 4H), 2.99 - 2.84 (m, 2H), 2.68 (dd, *J* = 44.7, 18.4 Hz, 4H), 2.39 - 2.24 (m, 3H), 2.05 (s, 3H), 1.45 (s, 2H), 0.95 (s, 6H). LCMS (ESI) calcd for $C_{34}H_{38}FN_4O_4^+$ [M+H]+: 585.29, found, 585.3.

**Example 281: Preparation of 3-(5-(3-((4'-fluoro-5,5-dimethyl-3,4,5,6-tetrahydro-[1,1'-biphenyl]-2-yl)methyl)-3,8-diazabicyclo [3.2.1] octane-8-carbonyl)-1-oxoisoindolin-2-yl)piperidine-2,6-dione (GT-05451)**

Referring to the methods of Scheme 6 and Example 76, the target product (GT-05451) was prepared as white solid (14 mg, yield 21.19%). $^1$H NMR (400 MHz, DMSO-d6) δ 11.03 (s, 1H), 7.84 (d, *J*= 7.7 Hz, 1H), 7.64 (s, 1H), 7.52 (d, *J*= 7.4 Hz, 1H), 7.31 - 7.21 (m, 2H), 7.16 (s, 3H), 5.16 (d, *J* = 9.1 Hz, 1H), 4.56 - 4.42 (m, 2H), 4.39 (s, 1H), 3.63 (d, *J* = 22.5 Hz, 3H), 3.38 - 3.28 (m, 1H), 3.20 (s, 1H), 2.98 - 2.85 (m, 2H), 2.72 - 2.57 (m, 3H), 2.29 (d, *J* = 30.2 Hz, 3H), 2.10 (s, 2H), 1.80 (s, 2H), 1.47 (s, 3H), 0.96 (s, 7H). LCMS (ESI) calcd for $C_{35}H_{40}FN_4O_4^+$ [M+H]+: 599.30, found, 599.3.

**Example 282: Preparation of 3-(5-(5-((4'-fluoro-5,5-dimethyl-3,4,5,6-tetrahydro-[1,1'-biphenyl]-2-yl)methyl)-2,5-diazabicyclo[2.2.1]heptane-2-carbonyl)-1-oxoisoindolin-2-yl)piperidine-2,6-dione (GT-05452)**

Referring to the methods of Scheme 6 and Example 76, the target product (GT-05452) was prepared as white solid (18 mg, yield 27.86%). $^1$H NMR (400 MHz, DMSO-d6) δ 11.03 (s, 1H), 7.86 - 7.82 (m, 1H), 7.82 (s, 1H), 7.72 (s, 1H), 7.27 (d, *J* = 8.3 Hz, 2H), 7.21 (s, 1H), 7.20 - 7.17 (m, 2H), 5.19 - 5.13 (m, 1H), 4.57 - 4.46 (m, 2H), 4.44 - 4.37 (m, 2H), 3.77 - 3.57 (m, 8H), 2.95 (dd, *J* = 30.1, 11.9 Hz, 3H), 2.63 (t, *J* = 15.9 Hz, 2H), 2.45 - 2.40 (m, 1H), 2.18 (d, *J* = 31.3 Hz, 4H), 2.04 (d, *J* = 9.1 Hz, 5H), 1.50 - 1.43 (m, 3H), 0.95 (s, 6H). LCMS (ESI) calcd for $C_{34}H_{38}FN_4O_4^+$ [M+H]+: 585.29, found, 585.3.

**Example 283: Preparation of 3-(5-(4-((4'-chloro-5,5-dimethyl-3,4,5,6-tetrahydro-[1,1'-biphenyl]-2-yl)methyl)-1,4-diazepane-1-carbonyl)-1-oxoisoindolin-2-yl)piperidine-2,6-dione (GT-05453)**

Referring to the methods of Scheme 6 and Example 76, the target product (GT-05453) was prepared as white solid (32 mg, yield 48.11%). $^1$H NMR (400 MHz, DMSO-d6) δ 11.01 (s, 1H), 8.87 (s, 1H), 7.81 (t, *J* = 9.2 Hz, 1H), 7.59 (d, *J* = 10.2 Hz, 1H), 7.48 (t, *J = 8.9* Hz, 3H), 7.14 (dd, *J* = 13.8, 8.1 Hz, 2H), 5.20 - 5.07 (m, 1H), 4.50 (dd, *J*= 17.2, 7.1 Hz, 1H), 4.39 (d, *J* = 6.2 Hz, 1H), 3.73 - 3.55 (m, 4H), 3.41 (d, *J* = 35.9 Hz, 4H), 3.07 - 2.86 (m, 3H), 2.67 - 2.55 (m, 2H), 2.41 (d, *J* = 10.0 Hz, 1H), 2.20 (d, *J* = 19.6 Hz, 2H), 2.05 (d, *J* = 13.7 Hz, 4H), 1.48 (d, *J* = 17.0 Hz, 2H), 0.96 (s, 6H). LCMS (ESI) calcd for $C_{34}H_{40}ClN_4O_4^+$ [M+H]+: 603.27, found, 603.3.

**Example 284: Preparation of 5-(4-((4'-chloro-5,5-dimethyl-3,4,5,6-tetrahydro-[1,1'-biphenyl]-2-yl)methyl)-1,4-diazepane-1-carbonyl)-2-(2,6-dioxopiperidin-3-yl)isoindoline-1,3-dione (GT-05454)**

Referring to the methods of Scheme 6 and Example 76, the target product (GT-05454) was prepared as white solid (19 mg, yield 29.36%). $^1$H NMR (400 MHz, DMSO-d6) δ 11.15 (s, 1H), 9.90 (s, 1H), 8.01 - 7.96 (m, 1H), 7.90 - 7.81 (m, 1H), 7.52 - 7.36 (m, 2H), 7.14 (dd, *J* = 18.4, 7.1 Hz, 2H), 5.30 - 5.10 (m, 1H), 3.80 - 3.53 (m, 4H), 3.45 (*d, J* = 39.5 Hz, 2H), 3.24 (d, *J* = 13.7 Hz, 1H), 2.91 (dd, *J* = 12.8,9.1 Hz, 3H), 2.58 (dd, *J* = 24.9, 11.7 Hz, 2H), 2.31 (d, *J* = 19.1 Hz, 3H), 2.07 (d, *J* = 12.4 Hz, 3H), 1.46 (dd, *J* = 13.3, 6.1 Hz, 2H), 0.95 (s, 6H). LCMS (ESI) calcd for $C_{34}H_{38}ClN_4O_5^+$ [M+H]+: 617.25, found, 617.3.

**Example 285: Preparation of 3-(5-(4-((4'-chloro-[1,1'-biphenyl]-2-yl)methyl)piperazine-1-carbonyl)-1-oxoisoindolin-2-yl)piperidine-2,6-dione (GT-05455)**

Referring to the methods of Scheme 6 and Example 76, the target product (GT-05455) was prepared as white solid (28 mg, yield 45.36%). $^1$H NMR (400 MHz, DMSO-d6) δ 11.03 (s, 1H), 10.94 (s, 1H), 8.06 (s, 1H), 7.79 (d, *J* = 7.8 Hz, 1H), 7.64 (s, 1H), 7.54 (dd, *J* = 13.0, 7.9 Hz, 5H), 7.39 (d, *J* = 8.2 Hz, 2H), 7.32 (d, *J* = 6.0 Hz, 1H), 5.14 (dd, *J* = 13.3, 5.1 Hz, 1H), 4.43 (dd, *J* = 50.0, 17.6 Hz, 4H), 4.26 (s, 1H), 3.60 (s, 2H), 3.24 (d, *J* = 10.8 Hz, 3H), 3.01 - 2.86 (m, 2H), 2.79 (s, 1H), 2.61 (d, *J* = 16.8 Hz, 1H), 2.41 (tt, *J* = 13.2, 6.5 Hz, 1H), 2.00 (dd, *J* = 15.4, 10.3 Hz, 1H). LCMS (ESI) calcd for $C_{31}H_{30}ClN_4O_4^+$ [M+H]$^+$: 557.20, found, 557.2.

**Example 286: Preparation of 5-(4-((4'-chloro-[1,1'-biphenyl]-2-yl)methyl)piperazine-1-carbonyl)-2-(2,6-dioxopiperidin-3-yl)isoindoline-1,3-dione (GT-05456)**

Referring to the methods of Scheme 6 and Example 76, the target product (GT-05456) was prepared as white solid (25 mg, yield 41.57%). $^1$H NMR (400 MHz, DMSO-d6) δ 11.16 (s, 1H), 10.70 (s, 1H), 8.00 (d, *J* = 7.6 Hz, 1H), 7.94 (d, *J* = 7.7 Hz, 1H), 7.90 - 7.84 (m, 1H), 7.56 (s, 2H), 7.53 (s, 1H), 7.39 (d, *J* = 8.3 Hz, 2H), 7.33 (d, *J* = 6.5 Hz, 1H), 5.19 (dd, *J* = 12.8, 5.4 Hz, 1H), 4.33 (d, *J* = 64.6 Hz, 2H), 3.54 (s, 2H), 3.25 (s, 4H), 3.02 (s, 1H), 2.93 - 2.85 (m, 1H), 2.77 (s, 1H), 2.58 (dd, *J* = 24.7, 12.0 Hz, 2H), 2.11 - 2.00 (m, 1H). LCMS (ESI) calcd for $C_{31}H_{28}ClN_4O_5^+$ [M+H]$^+$: 571.17, found, 571.1.

**Example 287: Preparation of 3-(5-(6-((4'-chloro-5,5-dimethyl-3,4,5,6-tetrahydro-[1,1'-biphenyl]-2-yl)methyl)-3,6-diazabicyclo[3.1.1]heptane-3-carbonyl)-1-oxoisoindolin-2-yl)piperidine-2,6-dione (GT-05457)**

Referring to the methods of Scheme 6 and Example 76, the target product (GT-05457) was prepared as white solid (12 mg, yield 18.10%). $^1$H NMR (400 MHz, DMSO-d6) δ 11.09 (s, 1H), 11.01 (s, 1H), 7.85 - 7.80 (m, 1H), 7.62 (t, *J* = 9.2 Hz, 1H), 7.48 (d, *J* = 8.3 Hz, 1H), 7.43 (d, *J* = 6.6 Hz, 2H), 7.22 (d, *J* = 8.3 Hz, 2H), 5.17 - 5.13 (m, 1H), 4.54 - 4.46 (m, 2H), 4.23 (s, 1H), 4.12 - 3.97 (m, 3H), 3.57 (d, *J* = 16.6 Hz, 3H), 2.92 (dd, *J* = 21.7, 9.0 Hz, 2H), 2.44 (dd, *J* = 17.9, 8.4 Hz, 2H), 2.21 (d, *J* = 30.8 Hz, 4H), 1.44 (s, 3H), 0.96 (s, 6H). LCMS (ESI) calcd for $C_{34}H_{38}ClN_4O_4^+$ [M+H]$^+$: 601.26, found, 601.3.

**Example 288: Preparation of 3-(5-(3-((4'-chloro-5,5-dimethyl-3,4,5,6-tetrahydro-[1,1'-biphenyl]-2-yl)methyl)-3,8-diazabicyclo[3.2.1]octane-8-carbonyl)-1-oxoisoindolin-2-yl)piperidine-2,6-dione (GT-05458)**

Referring to the methods of Scheme 6 and Example 76, the target product (GT-05458) was prepared as white solid (12 mg, yield 17.71%). $^1$H NMR (400 MHz, DMSO-d6) δ 11.02 (s, 1H), 9.72 (s, 1H), 7.79 (d, *J* = 28.3 Hz, 1H), 7.64 (s, 1H), 7.50 (dd, *J* = 14.6, 7.3 Hz, 2H), 7.38 (s, 1H), 7.20 - 7.04 (m, 2H), 5.15 (d, *J* = 8.1 Hz, 1H), 4.47 (dd, *J* = 45.9, 19.3 Hz, 2H), 3.65 (s, 2H), 3.40 - 3.31 (m, 1H), 3.13 (d, *J* = 4.9 Hz, 2H), 2.96 - 2.78 (m, 2H), 2.61 (d, *J* = 17.2 Hz, 2H), 2.31 (dd, *J* = 56.0, 39.7 Hz, 4H), 2.03 - 1.70 (m, 6H), 1.46 (s, 2H), 0.95 (s, 6H). LCMS (ESI) calcd for $C_{35}H_{40}ClN_4O_4^+$ [M+H]$^+$: 615.27, found, 615.3.

**Example 289: Preparation of 3-(4-fluoro-5-((4-((4'-fluoro-[1,1'-biphenyl]-2-yl)methyl)piperazin-1-yl)methyl)-1-oxoisoindolin-2-yl)piperidine-2,6-dione (GT-05602)**

Referring to the method of Scheme 1, the target product (GT-05602) was prepared as white solid (40 mg, yield 49.83%). $^1$H NMR (400 MHz, DMSO-d6) δ 11.02 (s, 1H), 7.86 (s, 1H), 7.75 (s, 1H), 7.64 (d, *J* = 7.7 Hz, 1H), 7.47 (d, *J* = 4.4 Hz, 2H), 7.40 (dd, *J* = 8.4, 5.6 Hz, 2H), 7.29 (t, *J* = 8.7 Hz, 3H), 5.13 (dd, *J* = 13.2, 5.0 Hz, 1H), 4.59 - 4.40 (m, 2H), 4.22 (s, 3H), 3.19 (s, 5H), 2.98 - 2.83 (m, 2H), 2.83 - 2.69 (m, 1H), 2.61 (d, *J* = 17.4 Hz, 1H), 2.48 - 2.39 (m, 1H), 2.07 - 1.95 (m, 1H). LCMS (ESI) calcd for $C_{31}H_{31}F_2N_4O_3^+$ [M+H]$^+$: 545.24, found, 545.3.

**Example 290: Preparation of 3-(6-fluoro-5-((4-((4'-fluoro-[1,1'-biphenyl]-2-yl)methyl)piperazin-1-yl)methyl)-1-oxoisoindolin-2-yl)piperidine-2,6-dione (GT-05603)**

Referring to the method of Scheme 1, the target product (GT-05603) was prepared as white solid (36 mg, yield 44.84%). $^1$H NMR (400 MHz, DMSO-d6) δ 11.02 (s, 1H), 7.84 (s, 2H), 7.62 (d, *J* = 8.5 Hz, 1H), 7.50 - 7.44 (m, 2H), 7.40 (dd, *J* = 8.5, 5.6 Hz, 2H), 7.30 (d, *J* = 8.7 Hz, 3H), 5.13 (dd, *J* = 13.3, 5.1 Hz, 1H), 4.39 (dd, *J* = 50.3, 17.4 Hz, 3H), 4.19 (s, 2H), 3.18 (s, 5H), 2.96 - 2.85 (m, 2H), 2.80 (s, 1H), 2.61 (d, *J* = 16.4 Hz, 1H), 2.45 - 2.35 (m, 1H), 2.05 - 1.95 (m, 1H). LCMS (ESI) calcd for $C_{31}H_{31}F_2N_4O_3^+$ [M+H]$^+$: 545.24, found, 545.3.

**Example 291: Preparation of 3-(7-fluoro-5-((4-((4'-fluoro-[1,1'-biphenyl]-2-yl)methyl)piperazin-1-yl)methyl)-1-oxoisoindolin-2-yl)piperidine-2,6-dione (GT-05604)**

Referring to the method of Scheme 1, the target product (GT-05604) was prepared as white solid (29 mg, yield 36.12%). $^1$H NMR (400 MHz, DMSO-d6) δ 11.01 (s, 1H), 7.84 (s, 1H), 7.57 (s, 1H), 7.47 (s, 2H), 7.41 (dd, *J* = 8.5, 5.6 Hz, 2H), 7.29 (t, *J* = 8.7 Hz, 3H), 5.09 (dd, *J* = 13.3, 5.0 Hz, 1H), 4.42 (dd, *J* = 52.0, 17.9 Hz, 3H), 4.26 (s, 2H), 3.14 (s, 5H), 2.97 - 2.84 (m, 2H), 2.81 - 2.69 (m, 1H), 2.60 (d, *J* = 17.6 Hz, 1H), 2.43 - 2.32 (m, 1H), 2.05 - 1.95 (m, 1H). LCMS (ESI) calcd for $C_{31}H_{31}F_2N_4O_3^+$ [M+H]$^+$: 545.24, found, 545.3.

**Example 292: Preparation of 3-(4-((4-((4'-fluoro-[1,1'-biphenyl]-2-yl)methyl)piperazin-1-yl)methyl)-1-oxoisoindolin-2-yl)piperidine-2,6-dione (GT-05605)**

Referring to the method of Scheme 1, the target product (GT-05605) was prepared as white solid (35 mg, yield 44.91%). $^1$H NMR (400 MHz, DMSO-d6) δ 11.04 (s, 1H), 7.77 (d, *J* = 6.7 Hz, 3H), 7.58 (t, *J* = 7.3 Hz, 1H), 7.46 (s, 2H), 7.41 (dd, *J* = 8.4, 5.6 Hz, 2H), 7.29 (t, *J* = 8.6 Hz, 3H), 5.15 (dd, *J* = 13.2, 5.1 Hz, 1H), 4.70 (s, 1H), 4.43 *(d, J* = 17.9 Hz, 1H), 4.22 (s, 4H), 3.15 (s, 5H), 2.99 - 2.88 (m, 2H), 2.86 - 2.73 (m, 1H), 2.63 (d, *J* = 16.9 Hz, 2H), 2.39 - 2.25 (m, 1H), 2.05 - 1.94 (m, 1H). LCMS (ESI) calcd for $C_{31}H_{32}FN_4O_3^+$ [M+H]$^+$: 527.25, found, 527.3.

**Example 293: Preparation of 3-(6-((4-((4'-fluoro-[1,1'-biphenyl]-2-yl)methyl)piperazin-1-yl)methyl)-1-oxoisoindolin-2-yl)piperidine-2,6-dione (GT-05606)**

Referring to the method of Scheme 1, the target product (GT-05606) was prepared as white solid (30 mg, yield 38.49%). $^1$H NMR (400 MHz, DMSO-d6) δ 11.00 (s, 1H), 7.93 (s, 1H), 7.80 (d, *J* = 7.0 Hz, 1H), 7.68 (d, *J* = 7.7 Hz, 1H), 7.45 (s, 1H), 7.40 (dd, *J* = 8.5, 5.6 Hz, 2H), 7.29 (dd, *J*= 11.3, 6.0 Hz, 3H), 5.12 (dd, *J* = 13.3, 5.1 Hz, 1H), 4.50 (d, *J* = 17.7 Hz, 1H), 4.37 (d, *J* = 17.6 Hz, 2H), 3.70 (s, 2H), 3.51 (s, 2H), 3.35 - 3.22 (m, 3H), 3.15 (s, 3H), 2.94 - 2.86 (m, 1H), 2.63 (t, *J* = 16.9 Hz, 2H), 2.41 (dt, *J* = 13.4, 8.8 Hz, 1H), 2.05 - 1.96 (m, 1H). LCMS (ESI) calcd for $C_{31}H_{32}FN_4O_3^+$ [M+H]$^+$: 527.25, found, 527.3.

**Example 294: Preparation of 3-(5-((3-((4'-chloro-[1,1'-biphenyl]-2-yl)methyl)-3,6-diazabicyclo[3.1.1]heptan-6-yl)methyl)-1-oxoisoindolin-2-yl)piperidine-2,6-dione (GT-05607)**

Referring to the method of Scheme 1, the target product (GT-05607) was prepared as white solid (14 mg, yield 18.73%). $^1$H NMR (400 MHz, DMSO-d6) δ 11.01 (s, 1H), 9.71 (s, 1H), 7.81 - 7.77 (m, 1H), 7.75 - 7.67 (m, 1H), 7.63 (s, 1H), 7.49 (d, *J* = 8.3 Hz, 3H), 7.40 (s, 2H), 7.28 (d, *J* = 6.5 Hz, 1H), 5.13 (dd, *J* = 13.3, 5.0 Hz, 1H), 4.66 (d, *J* = 4.7 Hz, 1H), 4.48 (d, *J* = 17.5 Hz, 1H), 4.35 (d, *J* = 17.6 Hz, 1H), 4.28 (d, *J* = 9.9 Hz, 1H), 4.15 (s, 1H), 3.99 (s, 1H), 3.55 (s, 2H), 3.16 (s, 2H), 2.95 (dd, *J* = 36.7, 24.4 Hz, 2H), 2.67 (s, 1H), 2.59 (s, 1H), 2.50 - 2.46 (m, 1H), 2.46 - 2.36 (m, 1H), 2.33 (s, 1H), 2.01 (d, *J* = 5.8 Hz, 1H). LCMS (ESI) calcd for $C_{32}H_{32}ClN_4O_3^+$ [M+H]$^+$: 555.22, found, 555.3.

**Example 295: Preparation of 3-(5-((3-((4'-chloro-[1,1'-biphenyl]-2-yl)methyl)-3,6-diazabicyclo[3.1.1]heptan-6-yl)methyl)-4-fluoro-1-oxoisoindolin-2-yl)piperidine-2,6-dione (GT-05608)**

Referring to the method of Scheme 1, the target product (GT-05608) was prepared as white solid (24 mg, yield 31.22%). $^1$H NMR (400 MHz, DMSO-d6) δ 11.05 (s, 1H), 9.18 (s, 1H), 7.84 - 7.73 (m, 1H), 7.69 (d, *J* = 7.7 Hz, 1H), 7.62 (dd, *J* = 16.2, 6.8 Hz, 1H), 7.51 - 7.46 (m, 3H), 7.40 (ddd, *J* = 10.5, 6.0, 2.7 Hz, 2H), 7.26 (dd, *J* = 11.6, 5.5 Hz, 1H), 5.19 - 5.09 (m, 1H), 4.76 - 4.54 (m, 2H), 4.46 - 4.37 (m, 1H), 4.33 (d, *J* = 7.1 Hz, 1H), 4.20 (d, *J* = 5.6 Hz, 1H), 3.51 (dd, *J* = 28.7, 15.1 Hz, 4H), 3.17 (d, *J* = 10.0 Hz, 1H), 2.99 (s, 1H), 2.92 (d, *J* = 13.6 Hz, 1H), 2.61 (d, *J* = 16.5 Hz, 2H), 2.48 - 2.26 (m, 2H), 2.06 (dd, *J* = 33.7, 28.5 Hz, 2H). LCMS (ESI) calcd for $C_{32}H_{31}ClFN_4O_3^+$ [M+H]$^+$: 573.21, found, 573.2.

**Example 296: Preparation of 3-(5-((3-((4'-chloro-[1,1'-biphenyl]-2-yl)methyl)-3,6-diazabicyclo[3.1.1]heptan-6-yl)methyl)-6-fluoro-1-oxoisoindolin-2-yl)piperidine-2,6-dione (GT-**05609)

Referring to the method of Scheme 1, the target product (GT-05609) was prepared as white solid (38 mg, yield 49.43%). $^1$H NMR (400 MHz, DMSO-d6) δ 11.02 (s, 1H), 9.13 (s, 1H), 7.91 - 7.79 (m, 1H), 7.67 (t, *J* = 8.5 Hz, 1H), 7.60 (d, *J* = 8.6 Hz, 1H), 7.52 - 7.45 (m, 3H), 7.43 - 7.36 (m, 2H), 7.26 (dd, *J* = 11.4, 5.3 Hz, 1H), 5.19 - 5.05 (m, 1H), 4.68 (d, *J* = 21.2 Hz, 1H), 4.47 (dd, *J* = 17.2, 4.7 Hz, 1H), 4.40 - 4.26 (m, 2H), 4.22 (d, *J* = 5.8 Hz, 1H), 3.72 (s, 1H), 3.52 (d, *J* = 44.4 Hz, 2H), 2.94 (dd, *J* = 31.5, 14.7 Hz, 3H), 2.61 (d, *J* = 17.4 Hz, 3H), 2.39 (dd, *J* = 28.5, 15.5 Hz, 2H), 2.07 (t, *J* = 29.8 Hz, 2H). LCMS (ESI) calcd for $C_{32}H_{31}ClFN_4O_3^+$ [M+H]$^+$: 573.21, found, 573.3.

**Example 297: Preparation of 3-(5-((3-((4'-chloro-[1,1'-biphenyl]-2-yl)methyl)-3,6-diazabicyclo[3.1.1]heptan-6-yl) methyl)-7-fluoro-1-oxoisoindolin-2-yl)piperidine-2,6-dione (GT-**05610)

Referring to the method of Scheme 1, the target product (GT-05610) was prepared as white solid (26 mg, yield 33.82%). $^1$H NMR (400 MHz, DMSO-d6) δ 11.02 (s, 1H), 7.58 (s, 1H), 7.54 (d, *J* = 10.0 Hz, 1H), 7.52 - 7.45 (m, 4H), 7.44 - 7.36 (m, 3H), 7.27 (d, *J* = 8.5 Hz, 1H), 5.10 (d, *J* = 12.9 Hz, 1H), 4.65 (s, 1H), 4.51 (d, *J* = 18.4 Hz, 1H), 4.37 (d, *J* = 18.2 Hz, 1H), 4.29 (d, *J* = 18.1 Hz, 1H), 4.15 (s, 1H), 3.75 (s, 1H), 3.48 (s, 2H), 3.13 (d, *J* = 13.2 Hz, 1H), 3.04 - 2.87 (m, 3H), 2.68 - 2.57 (m, 2H), 2.41 (d, *J* = 13.5 Hz, 1H), 2.33 (s, 1H), 2.16 (s, 1H), 2.02 (s, 1H). LCMS (ESI) calcd for $C_{32}H_{31}ClFN_4O_3^+$ [M+H]$^+$: 573.21, found, 573.2.

**Example 298: Preparation of 3-(5-((4-((4'-chloro-[1,1'-biphenyl]-2-yl)methyl)-1,4-diazepan-1-yl)methyl)-1-oxoisoindolin-2-yl)piperidine-2,6-dione (GT-05611)**

Referring to the method of Scheme 1, the target product (GT-05611) was prepared as white solid (39 mg, yield 46.54%). $^1$H NMR (400 MHz, DMSO-d6) δ 11.62 (d, *J* = 56.5 Hz, 1H), 11.03 (s, 1H), 8.07 (d, *J* = 35.1 Hz, 1H), 7.87 - 7.76 (m, 2H), 7.70 (d, *J* = 35.4 Hz, 1H), 7.53 (d, *J* = 10.5 Hz, 4H), 7.44 - 7.34 (m, 2H), 7.34 - 7.24 (m, 1H), 5.14 (dd, *J* = 13.1, 4.7 Hz, 1H), 4.47 (t, *J* = 15.8 Hz, 2H), 4.36 (d, *J* = 17.3 Hz, 3H), 3.79 (s, 1H), 3.54 (s, 2H), 3.31 (s, 2H), 3.06 (s, 1H), 3.01 - 2.74 (m, 2H), 2.61 (d, *J* = 16.3 Hz, 1H), 2.48 - 2.35 (m, 1H), 2.03 (dd, *J* = 29.9, 24.3 Hz, 3H). LCMS (ESI) calcd for $C_{32}H_{34}ClN_4O_3^+$ [M+H]$^+$: 557.23, found, 557.3.

**Example 299: Preparation of 3-(5-((7-((4'-chloro-[1,1'-biphenyl]-2-yl)methyl)-2,7-diazaspiro[3.5]nonan-2-yl) methyl)-1-oxoisoindolin-2-yl)piperidine-2,6-dione (GT-05612)**

Referring to the method of Scheme 1, the target product (GT-05612) was prepared as white solid (23 mg, yield 31.87%). $^1$H NMR (400 MHz, DMSO-d6) δ 11.54 (s, 1H), 11.01 (s, 1H), 10.38 (s, 1H), 8.14 - 8.05 (m, 1H), 7.82 - 7.74 (m, 2H), 7.69 (t, *J* = 7.0 Hz, 1H), 7.55 - 7.46 (m, 4H), 7.37 (t, *J* = 8.8 Hz, 2H), 7.32 - 7.25 (m, 1H), 5.13 (dd, *J* = 13.1, 4.8 Hz, 1H), 4.55 - 4.41 (m, 3H), 4.33 (dd, *J* = 17.7, 3.8 Hz, 1H), 4.20 (s, 2H), 3.90 (d, *J* = 6.4 Hz, 1H), 3.81 (s, 2H), 3.71 (d, *J* = 5.3 Hz, 1H), 3.16 (dd, *J* = 24.7, 12.1 Hz, 2H), 2.91 (dd, *J* = 22.0, 9.1 Hz, 1H), 2.72 (s, 1H), 2.61 (d, *J* = 16.3 Hz, 2H), 2.47 - 2.37 (m, 1H), 2.31 (d, *J* = 12.8 Hz, 1H), 2.04 (dd, *J* = 24.3, 10.8 Hz, 4H). LCMS (ESI) calcd for $C_{34}H_{36}ClN_4O_3^+$ [M+H]$^+$: 583.25, found, 583.2.

**Example 300: Preparation of 3-(5-((5-((4'-chloro-[1,1'-biphenyl]-2-yl)methyl)hexahydropyrrolo[3,4-c]pyrrol-2(1H)-yl)methyl)-1-oxoisoindolin-2-yl)piperidine-2,6-dione (GT-05613)**

Referring to the method of Scheme 1, the target product (GT-05613) was prepared as white solid (34 mg, yield 46.45%). $^1$H NMR (400 MHz, DMSO-d6) δ 10.96 (d, *J* = 42.9 Hz, 2H), 7.89 (s, 1H), 7.84 - 7.78 (m, 1H), 7.69 (dd, *J* = 42.0, 12.0 Hz, 2H), 7.52 (dd, *J* = 18.0, 7.0 Hz, 4H), 7.38 (d, *J* = 8.2 Hz, 2H), 7.32 (s, 1H), 5.13 (dd, *J* = 13.2, 5.0 Hz, 1H), 4.52 - 4.29 (m, 6H), 3.68 (s, 1H), 3.49 (s, 4H), 3.21 (s, 2H), 3.04 (s, 2H), 2.98 - 2.84 (m, 2H), 2.61 (d, *J* = 16.7 Hz, 1H), 2.42 (d, *J* = 12.9 Hz, 1H), 2.05 - 1.96 (m, 1H). LCMS (ESI) calcd for $C_{33}H_{34}ClN_4O_3^+$ [M+H]$^+$: 569.23, found, 569.2.

**Example 301: Preparation of 3-(5-(4-((4'-fluoro-5,5-dimethyl-3,4,5,6-tetrahydro-[1,1'-biphenyl]-2-yl)methyl)piperazine-1-carbonyl)-1-oxoisoindolin-2-yl)piperidine-2,6-dione (GT-05614)**

Referring to the methods of Scheme 6 and Example 76, the target product (GT-05614) was prepared as white solid (12 mg, yield 14.92%). $^1$H NMR (400 MHz, DMSO-d6) δ 11.03 (s, 1H), 7.80 (d, *J* = 7.8 Hz, 1H), 7.65 (s, 1H), 7.54 (d, *J*= 7.7 Hz, 1H), 7.23 (t, *J* = 8.7 Hz, 2H), 7.15 (t, *J* = 6.8 Hz, 2H), 5.14 (dd, *J*= 13.3, 5.1 Hz, 1H), 4.49 (d, *J* = 17.7 Hz, 2H), 4.37 (d, *J* = 17.6 Hz, 1H), 3.62 (s, 6H), 3.28 - 3.16 (m, 2H), 2.97 - 2.82 (m, 2H), 2.66 (dd, *J* = 36.4, 20.5 Hz, 2H), 2.47 - 2.34 (m, 1H), 2.25 (d, *J* = 21.3 Hz, 2H), 2.10 - 1.96 (m, 3H), 1.48 (s, 2H), 0.97 (s, 6H). LCMS (ESI) calcd for $C_{33}H_{38}FN_4O_4^+$ [M+H]$^+$: 573.29, found, 573.3.

**Example 302: Preparation of 2-(2,6-dioxopiperidin-3-yl)-5-(4-((4'-fluoro-5,5-dimethyl-3,4,5,6-tetrahydro-[1,1'-biphenyl]-2-yl)methyl)piperazine-1-carbonyl)isoindoline-1,3-dione (GT-05615)**

Referring to the methods of Scheme 6 and Example 76, the target product (GT-05615) was prepared as white solid (8.6 mg, yield 10.46%). $^1$H NMR (400 MHz, DMSO-d6) δ 11.15 (s, 1H), 9.26 (s, 1H), 8.01 (d, *J* = 7.6 Hz, 1H), 7.96 (s, 1H), 7.88 (d, *J* = 8.0 Hz, 1H), 7.24 (t, *J* = 8.5 Hz, 2H), 7.16 (d, *J* = 5.4 Hz, 2H), 5.19 (dd, *J* = 12.9, 5.5 Hz, 1H), 4.48 (s, 1H), 3.49 (s, 4H), 3.23 - 3.15 (m, 2H), 2.95 - 2.84 (m, 2H), 2.68 - 2.57 (m, 2H), 2.55 (d, *J* = 4.9 Hz, 2H), 2.21 (s, 2H), 2.06 (d, *J* = 8.5 Hz, 3H), 1.48 (s, 2H), 0.97 (s, 6H). LCMS (ESI) calcd for $C_{33}H_{36}FN_4O_5^+$ [M+H]$^+$: 587.27, found, 587.3.

**Example 303: Preparation of 3-(5-(4-((4'-chloro-[1,1'-biphenyl]-2-yl)methyl)-1,4-diazepane-1-carbonyl)-1-oxoisoindolin-2-yl)piperidine-2,6-dione (GT-05616)**

Referring to the methods of Scheme 6 and Example 76, the target product (GT-05616) was prepared as white solid (36 mg, yield 42.63%). $^1$H NMR (400 MHz, DMSO-d6) δ 11.01 (s, 1H), 9.79 (s, 1H), 8.00 - 7.83 (m, 1H), 7.79 (t, *J* = 7.4 Hz, 1H), 7.62 - 7.56 (m, 3H), 7.55 - 7.48 (m, 2H), 7.43 - 7.37 (m, 2H), 7.35 (d, *J* = 7.9 Hz, 1H), 5.13 (dd, *J* = 13.2, 5.2 Hz, 1H), 4.55 - 4.27 (m, 4H), 3.62 - 3.50 (m, 2H), 3.44 (s, 2H), 3.24 (s, 2H), 3.03 (t, *J* = 26.1 Hz, 2H), 2.61 (d, *J* = 16.1 Hz, 1H), 2.47 - 2.36 (m, 1H), 2.05 (d, *J* = 17.4 Hz, 2H). LCMS (ESI) calcd for $C_{32}H_{32}ClN_4O_4^+$ [M+H]$^+$: 571.21, found, 571.3.

**Example 304: Preparation of 3-(5-(7-((4'-chloro-[1,1'-biphenyl]-2-yl)methyl)-2,7-diazaspiro[3.5]nonane-2-carbonyl)-1-oxoisoindolin-2-yl)piperidine-2,6-dione (GT-05641)**

Referring to the methods of Scheme 6 and Example 76, the target product (GT-05641) was prepared as white solid (33 mg, yield 37.47%). $^1$H NMR (400 MHz, DMSO-d6) δ 11.01 (s, 1H), 9.13 (s, 1H), 7.83 (s, 1H), 7.78 (d, *J* = 7.8 Hz, 2H), 7.71 (t, *J* = 6.7 Hz, 1H), 7.59 - 7.49 (m, 4H), 7.39 (d, *J* = 8.3 Hz, 1H), 7.36 - 7.29 (m, 2H), 5.13 (d, *J* = 13.1 Hz, 1H), 4.48 (dd, *J* = 17.7,4.9 Hz, 1H), 4.36 (t, *J = 8.6* Hz, 1H), 4.28 (d, *J* = 9.1 Hz, 2H), 4.00 (d, *J* = 8.1 Hz, 1H), 3.96 (d, *J* = 7.1 Hz, 1H), 3.76 (s, 2H), 3.16 (s, 2H), 2.98 - 2.89 (m, 1H), 2.78 (d, *J* = 40.7 Hz, 2H), 2.65 (dd, *J* = 32.5, 17.3 Hz, 2H), 2.41 (d, *J* = 13.3 Hz, 1H), 1.99 (d, *J* = 11.8 Hz, 2H), 1.84 (d, *J* = 14.5 Hz, 2H). LCMS (ESI) calcd for $C_{34}H_{34}ClN_4O_4^+$ [M+H]$^+$: 597.23, found, 597.3.

**Example 305: Preparation of 3-(5-(5-((4'-chloro-[1,1'-biphenyl]-2-yl)methyl)octahydropyrrolo[3,4-c]pyrrole-2-carbonyl)-1-oxoisoindolin-2-yl)piperidine-2,6-dione** (GT-05642)

Referring to the methods of Scheme 6 and Example 76, the target product (GT-05642) was prepared as white solid (39 mg, yield 45.29%). $^1$H NMR (400 MHz, DMSO-d6) δ 11.02 (s, 1H), 10.14 (*d, J = 72.4* Hz, 1H), 7.88 - 7.80 (m, 1H), 7.76 (d, *J* = 9.8 Hz, 1H), 7.65 (d, *J* = 24.8 Hz, 1H), 7.54 (t, *J* = 9.1 Hz, 5H), 7.39 (d, *J* = 8.2 Hz, 2H), 7.34 (d, *J* = 7.9 Hz, 1H), 5.15 (dd, *J* = 13.3, 4.9 Hz, 1H), 4.50 (t, *J* = 14.0 Hz, 1H), 4.42 - 4.37 (m, 2H), 3.72 (d, *J* = 47.6 Hz, 2H), 3.49 (s, 3H), 3.28 - 3.14 (m, 2H), 3.01 - 2.89 (m, 3H), 2.82 (d, *J* = 14.5 Hz, 1H), 2.62 (d, *J* = 16.2 Hz, 2H), 2.42 (dd, *J* = 17.5, 8.8 Hz, 1H), 2.02 (d, *J* = 5.3 Hz, 1H). LCMS (ESI) calcd for $C_{33}H_{32}ClN_4O_4^+$ [M+H]$^+$: 583.21, found, 583.2.

**Example 306: Preparation of 3-(5-(3-((4'-chloro-[1,1'-biphenyl]-2-yl)methyl)-3,6-diazabicyclo[3.1.1]heptane-6-carbonyl)-1-oxoisoindolin-2-yl)piperidine-2,6-dione (GT-05643)**

Referring to the methods of Scheme 6 and Example 76, the target product (GT-05643) was prepared as white solid (25 mg, yield 29.70%). $^1$H NMR (400 MHz, DMSO-d6) δ 11.89 (s, 1H), 11.03 (s, 1H), 8.05 (s, 1H), 7.79 (d, *J* = 7.8 Hz, 1H), 7.56 (s, 2H), 7.55 (s, 3H), 7.34 (s, 1H), 7.31 (s, 2H), 5.16 (dd, *J* = 13.2, 4.6 Hz, 1H), 4.62 (s, 1H), 4.50 (t, *J* = 20.1 Hz, 2H), 4.38 (d, *J* = 18.7 Hz, 2H), 4.25 (s, 1H), 3.37 - 3.26 (m, 3H), 2.97 - 2.86 (m, 1H), 2.71 (s, 1H), 2.63 (d, *J* = 17.6 Hz, 2H), 2.45 (s, 1H), 2.38 (s, 1H), 2.08 - 1.98 (m, 1H). LCMS (ESI) calcd for $C_{32}H_{30}ClN_4O_4^+$ [M+H]$^+$: 569.20, found, 569.3.

**Example 307: Preparation of 3-(5-((4-(4'-chloro-[1,1'-biphenyl]-2-carbonyl)-1,4-diazepan-1-yl)methyl)-1-oxoisoindolin-2-yl)piperidine-2,6-dione (GT-05644)**

Referring to the method of Scheme 1, the target product (GT-05644) was prepared as white solid (45 mg, yield 57.87%). $^1$H NMR (400 MHz, DMSO) δ 11.01 (s, 1H), 10.72 (s, 1H), 7.84 (s, 1H), 7.77 (d, *J* = 14.2 Hz, 1H), 7.61 (s, 1H), 7.54 (dd, *J* = 16.7, 9.4 Hz, 3H), 7.49 - 7.33 (m, 4H), 5.15 (dd, *J* = 13.3, 5.0 Hz, 1H), 4.52 (d, *J* = 17.8 Hz, 1H), 4.40 (d, *J* = 17.2 Hz, 2H), 4.18 (d, *J* = 42.9 Hz, 1H), 3.41 (s, 2H), 3.34 - 3.28 (m, 3H), 3.21 (s, 2H), 3.06 (s, 1H), 2.96 - 2.87 (m, 1H), 2.79 (s, 1H), 2.61 (d, *J* = 17.7 Hz, 1H), 2.43 (d, *J* = 9.6 Hz, 1H), 2.14 - 1.93 (m, 2H), 1.80 (s, 1H). LCMS (ESI) calcd for $C_{32}H_{32}ClN_4O_4^+$ [M+H]$^+$: 571.21, found, 571.2.

**Example 308: Preparation of 3-(5-((5-(4'-chloro-[1,1'-biphenyl]-2-carbonyl)hexahydropyrrolo[3,4-c]pyrrol-2(1H)-yl)methyl)-1-oxoisoindolin-2-yl)piperidine-2,6-dione (GT-05645)**

Referring to the method of Scheme 1, the target product (GT-05645) was prepared as white solid (47 mg, yield 59.27%). $^1$H NMR (400 MHz, DMSO-d6) δ 11.07 (d, *J* = 43.1 Hz, 2H), 7.81 (t, *J* = 7.0 Hz, 2H), 7.70 (d, *J* = 8.0 Hz, 1H), 7.60 - 7.55 (m, 2H), 7.49 (d, *J* = 6.4 Hz, 3H), 7.47 - 7.41 (m, 3H), 5.14 (dd, *J* = 13.2, 3.8 Hz, 1H), 4.50 (d, *J* = 17.6 Hz, 1H), 4.47 - 4.35 (m, 3H), 3.66 - 3.54 (m, 2H), 3.33 - 3.20 (m, 3H), 2.99 - 2.83 (m, 4H), 2.75 (s, 1H), 2.63 (t, *J* = 16.6 Hz, 2H), 2.46 - 2.38 (m, 1H), 2.04 - 1.99 (m, 1H). LCMS (ESI) calcd for $C_{33}H_{32}ClN_4O_4^+$ [M+H]$^+$: 583.21, found, 583.2.

**Example 309: Preparation of 3-(1-oxo-5-((4-(2-(thiophen-2-yl)benzyl)piperazin-1-yl)methyl)isoindolin-2-yl)piperidine-2,6-dione (GT-05519)**

Referring to the method of Scheme 1, the target product (GT-05519) was prepared as white solid (30 mg, yield 39.89%). $^1$H NMR (400 MHz, DMSO) δ 11.01 (s, 1H), 7.80 (d, *J* = 7.6 Hz, 2H), 7.71 - 7.64 (m, 2H), 7.45 (s, 3H), 7.26 (d, *J* = 2.8 Hz, 1H), 7.17 (dd, *J* = 5.0, 3.6 Hz, 1H), 5.13 (dd, *J* = 13.3, 5.0 Hz, 1H), 4.39 (t, *J* = 25.6 Hz, 4H), 3.36 - 3.26 (m, 3H), 3.16 (s, 4H), 2.98 - 2.85 (m, 2H), 2.61 (d, *J* = 16.4 Hz, 1H), 2.47 - 2.37 (m, 1H), 2.04 - 1.96 (m, 1H). LCMS (ESI) calcd for $C_{29}H_{31}N_4O_3S^+$ [M+H]$^+$: 515.21, found, 515.3.

**Example 310: Preparation of 3-(5-(4-((4'-chloro-[1,1'-biphenyl]-2-yl)amino)piperidine-1-carbonyl)-1-oxoisoindolin-2-yl)piperidine-2,6-dione (GT-05646)**

Referring to the methods of Scheme 6 and Example 76, the target product (GT-05646) was prepared as white solid (30 mg, yield 43.88%). $^1$H NMR (400 MHz, DMSO-d6) δ 11.00 (s, 1H), 7.77 (d, *J* = 7.8 Hz, 1H), 7.60 (s, 1H), 7.50 (dd, *J* = 12.2, 5.5 Hz, 3H), 7.45 - 7.37 (m, 2H), 7.21 - 7.14 (m, 1H), 6.99 (dd, *J* = 7.5, 1.5 Hz, 1H), 6.84 (d, *J* = 8.2 Hz, 1H), 6.70 (t, *J* = 7.3 Hz, 1H), 5.13 (dd, *J* = 13.3, 5.1 Hz, 1H), 4.49 (d, *J* = 17.7 Hz, 1H), 4.36 (d, *J* = 17.5 Hz, 2H), 3.59 (d, *J* = 10.1 Hz, 2H), 3.15 (s, 1H), 2.97 (d, *J* = 11.6 Hz, 1H), 2.95 - 2.86 (m, 1H), 2.60 (d, *J* = 17.8 Hz, 1H), 2.46 - 2.32 (m, 1H), 2.06 - 1.92 (m, 2H), 1.79 (s, 1H), 1.36 (dd, *J* = 20.5, 9.8 Hz, 2H). LCMS (ESI) calcd for $C_{31}H_{30}ClN_4O_4^+$ [M+H]$^+$: 557.20, found, 557.2.

**Example 311: Preparation of 5-(4-((4'-chloro-[1,1'-biphenyl]-2-yl)amino)piperidine-1-carbonyl)-2-(2,6-dioxopiperidin-3-yl)isoindoline-1,3-dione (GT-05647)**

Referring to the methods of Scheme 6 and Example 76, the target product (GT-05647) was prepared as white solid (35 mg, yield 49.83%). $^1$H NMR (400 MHz, DMSO-d6) δ 11.14 (s, 1H), 7.98 (d, *J* = 7.6 Hz, 1H), 7.90 - 7.82 (m, 2H), 7.52 (d, *J* = 8.4 Hz, 2H), 7.42 (d, *J* = 8.4 Hz, 2H), 7.22 - 7.16 (m, 1H), 6.99 (dd, *J* = 7.5, 1.4 Hz, 1H), 6.85 (d, *J* = 8.2 Hz, 1H), 6.70 (t, *J* = 7.4 Hz, 1H), 5.18 (dd, *J* = 12.7, 5.4 Hz, 1H), 4.36 (d, *J* = 10.2 Hz, 1H), 3.61 (s, 2H), 3.18 (s, 1H), 3.00 (s, 1H), 2.93 - 2.85 (m, 1H), 2.64 - 2.54 (m, 2H), 2.10 - 2.02 (m, 1H), 1.97 (s, 1H), 1.78 (s, 1H), 1.40 (dd, *J* = 20.6, 10.1 Hz, 2H). LCMS (ESI) calcd for $C_{31}H_{28}ClN_4O_5^+$ [M+H]$^+$: 571.17, found, 571.2.

**Example 312: (GT-05649) Preparation of 3-(5-((4-(4'-chloro-[1,1'-biphenyl]-2-carbonyl)piperazin-1-yl)methyl)-4-fluoro-1-oxoisoindolin-2-yl)piperidine-2,6-dione (GT-05649)**

Referring to the method of Scheme 1, the target product (GT-05649) was prepared as white solid (34 mg, yield 47.52%). $^1$H NMR (400 MHz, DMSO-d6) δ 11.03 (s, 1H), 7.80 (s, 1H), 7.67 (d, *J* = 7.7 Hz, 1H), 7.56 (dd, *J* = 13.1, 5.6 Hz, 1H), 7.50 (t, *J* = 8.0 Hz, 4H), 7.46 (s, 1H), 7.42 - 7.32 (m, 2H), 5.14 (dd, *J* = 13.2, 5.1 Hz, 1H), 4.58 (d, *J* = 17.5 Hz, 1H), 4.42 (dd, *J* = 17.3, 10.0 Hz, 3H), 3.39 (s, 3H), 3.34 - 3.24 (m, 2H), 3.14 (s, 2H), 2.97 - 2.89 (m, 1H), 2.79 (s, 1H), 2.61 (d, *J* = 17.4 Hz, 1H), 2.49 - 2.37 (m, 1H), 2.05 - 1.95 (m, 1H). LCMS (ESI) calcd for $C_{31}H_{29}ClFN_4O_4^+$ [M+H]$^+$: 575.19, found, 575.2.

**Example 313: Preparation of 3-(5-((4-(4'-chloro-[1,1'-biphenyl]-2-carbonyl)piperazin-1-yl)methyl)-6-fluoro-1-oxoisoindolin-2-yl)piperidine-2,6-dione (GT-05650)**

Referring to the method of Scheme 1, the target product (GT-05650) was prepared as white solid (31 mg, yield 43.33%). $^1$H NMR (400 MHz, DMSO-d6) δ 11.02 (s, 1H), 7.89 (s, 1H), 7.65 (d, *J* = 8.1 Hz, 1H), 7.59 - 7.54 (m, 1H), 7.50 (t, *J* = 8.3 Hz, 4H), 7.46 (s, 1H), 7.38 (s, 2H), 5.14 (dd, *J* = 13.2, 5.0 Hz, 1H), 4.41 (dd, *J* = 50.2, 17.4 Hz, 4H), 3.34 - 3.20 (m, 3H), 3.17 (s, 2H), 2.99 - 2.84 (m, 2H), 2.83 - 2.68 (m, 1H), 2.61 (d, *J* = 17.5 Hz, 1H), 2.41 (tt, *J* = 28.2, 14.0 Hz, 2H), 2.05 - 1.98 (m, 1H). LCMS (ESI) calcd for $C_{31}H_{29}ClFN_4O_4^+$ [M+H]$^+$: 575.19, found, 575.2.

**Example 314: Preparation of 5-(6-((4'-chloro-5,5-dimethyl-3,4,5,6-tetrahydro-[1,1'-biphenyl]-2-yl)methyl)-3,6-diazabicyclo[3.1.1]heptane-3-carbonyl)-2-(2,6-dioxopiperidin-3-yl)isoindoline-1,3-dione (GT-05651)**

Referring to the methods of Scheme 6 and Example 76, the target product (GT-05651) was prepared as white solid (9.5 mg, yield 11.05%). $^1$H NMR (400 MHz, DMSO-d6) δ 11.15 (s, 1H), 8.09 8.01 (m, 1H), 7.93 (d, *J* = 18.1 Hz, 1H), 7.48 (d, *J* = 7.8 Hz, 1H), 7.41 (d, *J* = 7.6 Hz, 1H), 7.21 (d, *J* = 7.4 Hz, 2H), 5.20 (dd, *J* = 12.7, 5.3 Hz, 1H), 4.60 (s, 1H), 4.12 (t, *J* = 42.1 Hz, 2H), 3.78 (d, *J* = 37.6 Hz, 1H), 3.55 (s, 3H), 3.27 - 3.12 (m, 1H), 3.10 - 2.81 (m, 2H), 2.70 - 2.53 (m, 2H), 2.25 (d, *J* = 63.2 Hz, 3H), 2.12 - 1.82 (m, 4H), 1.44 (s, 2H), 0.97 (s, 6H). LCMS (ESI) calcd for $C_{34}H_{36}ClN_4O_5^+$ [M+H]$^+$: 615.24, found, 615.3.

**Example 315: Preparation of 5-(3-((4'-chloro-5,5-dimethyl-3,4,5,6-tetrahydro-[1,1'-biphenyl]-2-yl)methyl)-3,8-diazabicyclo[3.2.1]octane-8-carbonyl)-2-(2,6-dioxopiperidin-3-yl)isoindoline-1,3-dione (GT-05652)**

Referring to the methods of Scheme 6 and Example 76, the target product (GT-05652) was prepared as white solid (40 mg, yield 45.53%). $^1$H NMR (400 MHz, DMSO-d6) δ 11.16 (s, 1H), 9.80 (s, 1H), 8.03 (s, 1H), 7.88 (dd, *J* = 11.1, 3.5 Hz, 2H), 7.46 (d, *J* = 46.7 Hz, 2H), 7.13 (s, 2H), 5.20 (s, 1H), 4.06 (s, 1H), 3.64 (s, 2H), 3.23 - 2.97 (m, 2H), 2.92 (dd, *J* = 22.3, 8.8 Hz, 2H), 2.68 - 2.52 (m, 3H), 2.28 (s, 2H), 2.00 (s, 4H), 1.76 - 1.67 (m, 1H), 1.61 (dd, *J* = 9.2, 4.2 Hz, 1H), 1.45 (s, 2H), 1.31 - 1.13 (m, 2H), 1.06 (dd, *J* = 16.2, 6.8 Hz, 1H), 0.95 (s, 6H). LCMS (ESI) calcd for $C_{35}H_{38}ClN_4O_5^+$ [M+H]$^+$: 629.25, found, 629.3.

**Example 316: Preparation of 3-(5-((4-(4'-chloro-[1,1'-biphenyl]-2-carbonyl)piperazin-1-yl)methyl)-7-fluoro-1-oxoisoindolin-2-yl)piperidine-2,6-dione (GT-05700)**

Referring to the method of Scheme 1, the target product (GT-05700) was prepared as white solid (30 mg, yield 41.93%). $^1$H NMR (400 MHz, DMSO-d6) δ 11.02 (s, 1H), 7.54 (dt, *J* = 19.2, 9.0 Hz, 6H), 7.45 (d, *J* = 9.6 Hz, 2H), 7.37 (s, 2H), 5.10 (dd, *J* = 13.2, 5.0 Hz, 1H), 4.44 (dd, *J*= 50.5, 17.8 Hz, 4H), 4.32 - 3.96 (m, 1H), 3.32 - 3.20 (m, 2H), 3.06 (s, 2H), 2.97 - 2.84 (m, 2H), 2.83 - 2.70 (m, 1H), 2.63 (t, *J* = 18.1 Hz, 2H), 2.43 - 2.32 (m, 1H), 2.03 - 1.95 (m, 1H). LCMS (ESI) calcd for $C_{31}H_{20}ClFN_4O_4^+$ [M+H]$^+$: 575.19, found, 575.2.

**Example 317: Preparation of 3-(4-((4-(4'-chloro-[1,1'-biphenyl]-2-carbonyl)piperazin-1-yl)methyl)-1-oxoisoindolin-2-yl)piperidine-2,6-dione (GT-05701)**

Referring to the method of Scheme 1, the target product (GT-05701) was prepared as white solid (40 mg, yield 57.60%). $^1$H NMR (400 MHz, DMSO-d6) δ 11.06 (s, 1H), 7.80 (s, 2H), 7.63 - 7.50 (m, 5H), 7.48 (d, *J* = 7.5 Hz, 2H), 7.41 (dd, *J* = 21.1, 12.6 Hz, 3H), 5.17 (d, *J* = 8.4 Hz, 1H), 4.71 (s, 1H), 4.45 (d, *J* = 17.5 Hz, 2H), 4.34 (s, 1H), 3.34 - 3.28 (m, 2H), 3.12 (s, 2H), 3.01 - 2.85 (m, 2H), 2.81 (s, 1H), 2.65 (d, *J* = 17.9 Hz, 1H), 2.33 (s, 1H), 2.05 (s, 1H). LCMS (ESI) calcd for $C_{31}H_{30}ClN_4O_4^+$ [M+H]$^+$: 557.20, found, 557.3.

**Example 318: Preparation of 3-(5-((4-(2-(1-methyl-1H-pyrazol-5-yl)benzyl)piperazin-1-yl)methyl)-1-oxoisoindolin-2-yl)piperidine-2,6-dione (GT-05702)**

Referring to the method of Scheme 1, the target product (GT-05702) was prepared as white solid (40 mg, yield 53.37%). $^1$H NMR (400 MHz, DMSO-d6) δ 11.02 (d, *J* = 8.7 Hz, 1H), 8.03 (d, *J* = 13.5 Hz, 1H), 7.87 (s, 1H), 7.80 (d, *J*= 7.8 Hz, 1H), 7.74 (d, *J* = 8.1 Hz, 1H), 7.62 - 7.50 (m, 3H), 7.45 - 7.35 (m, 1H), 6.41 (d, *J* = 1.4 Hz, 1H), 5.13 (dd, *J* = 13.3, 5.1 Hz, 1H), 4.48 (d, *J* = 17.3 Hz, 3H), 4.12 (s, 2H), 3.61 (s, 3H), 3.44 (s, 4H), 3.30 (s, 3H), 2.97 - 2.87 (m, 1H), 2.61 (d, *J* = 16.2 Hz, 1H), 2.43 (dt, *J* = 13.2, 8.8 Hz, 1H), 2.06 - 1.93 (m, 1H). LCMS (ESI) calcd for $C_{29}H_{33}N_6O_3^+$ [M+H]$^+$: 513.26, found, 513.3.

**Example 319: Preparation of 3-(5-((4-((4-(4-chlorophenyl)pyridin-3-yl)methyl)piperazin-1-yl)methyl)-1-oxoisoindolin-2-yl)piperidine-2,6-dione (GT-05703)**

Referring to the method of Scheme 1, the target product (GT-05703) was prepared as white solid (24 mg, yield 30.39%). $^1$H NMR (400 MHz, DMSO) δ 11.01 (s, 1H), 8.95 (s, 1H), 8.85 (d, *J* = 5.7 Hz, 1H), 7.87 - 7.79 (m, 3H), 7.73 (d, *J* = 8.1 Hz, 1H), 7.61 (q, *J* = 8.7 Hz, 4H), 5.14 (dd, *J* = 13.2, 5.1 Hz, 1H), 4.40 (dd, *J* = 30.3, 21.6 Hz, 4H), 3.26 (s, 3H), 3.07 (s, 3H), 2.94 - 2.82 (m, 3H), 2.61 (d, *J* = 16.0 Hz, 3H), 2.46 - 2.37 (m, 1H), 2.06 - 1.97 (m, 1H). LCMS (ESI) calcd for $C_{30}H_{31}ClN_5O_3^+$ [M+H]$^+$: 544.21, found, 544.2.

**Example 320: Preparation of 3-(5-((6-((4'-chloro-[1,1'-biphenyl]-2-yl)methyl)-3,6-diazabicyclo[3.1.1]heptan-3-yl)methyl)-1-oxoisoindolin-2-yl)piperidine-2,6-dione (GT-05704)**

Referring to the method of Scheme 1, the target product (GT-05704) was prepared as white solid (30 mg, yield 37.32%). $^1$H NMR (400 MHz, DMSO-d6) δ 10.99 (s, 1H), 9.41 (s, 1H), 7.88 (d, *J* = 6.7 Hz, 1H), 7.72 (d, *J* = 7.7 Hz, 1H), 7.58 (d, *J* = 8.4 Hz, 1H), 7.54 - 7.41 (m, 5H), 7.39 (d, *J* = 8.4 Hz, 1H), 7.33 (t, *J* = 8.7 Hz, 1H), 5.19 - 5.06 (m, 1H), 4.54 (s, 1H), 4.46 *(d, J* = 17.4 Hz, 1H), 4.33 (d, *J* = 17.4 Hz, 1H), 4.12 (d, *J* = 14.5 Hz, 2H), 3.66 (s, 1H), 3.44 (s, 2H), 3.03 - 2.77 (m, 2H), 2.76 - 2.53 (m, 4H), 2.41 (d, *J* = 13.1 Hz, 2H), 2.01 (d, *J* = 12.0 Hz, 2H). LCMS (ESI) calcd for $C_{32}H_{32}ClN_4O_3^+$ [M+H]$^+$: 555.22, found, 555.2.

**Example 321: Preparation of 3-(5-(6-((4'-chloro-[1,1'-biphenyl]-2-yl)methyl)-3,6-diazabicyclo[3.1.1]heptane-3-carbonyl)-1-oxoisoindolin-2-yl)piperidine-2,6-dione (GT-05705)**

Referring to the methods of Scheme 6 and Example 76, the target product (GT-05705) was prepared as white solid (32 mg, yield 38.02%). $^1$H NMR (400 MHz, DMSO-d6) δ 11.02 (s, 1H), 7.82 (t, *J* = 8.3 Hz, 1H), 7.71 - 7.62 (m, 1H), 7.59 - 7.55 (m, 2H), 7.52 (s, 3H), 7.43 (d, *J* = 8.3 Hz, 2H), 7.34 (s, 1H), 5.20 - 5.10 (m, 1H), 4.61 - 4.45 (m, 2H), 4.38 (dd, *J* = 17.0, 9.1 Hz, 1H), 4.25 (s, 1H), 4.14 - 3.83 (m, 2H), 3.64 - 3.45 (m, 2H), 3.27 - 3.07 (m, 1H), 3.03 - 2.78 (m, 2H), 2.62 (d, *J* = 17.3 Hz, 2H), 2.47 - 2.36 (m, 1H), 2.13 - 1.76 (m, 2H). LCMS (ESI) calcd for $C_{32}H_{30}ClN_4O_4^+$ [M+H]$^+$: 569.20, found, 569.2.

**Example 322: Preparation of 3-(5-(4-((4'-chloro-5,5-dimethyl-3,4,5,6-tetrahydro-[1,1'-biphenyl]-2-yl) methyl)-2-(trifluoromethyl)piperazine-1-carbonyl)-1-oxoisoindolin-2-yl)piperidine-2,6-dione (GT-05706)**

Referring to the methods of Scheme 6 and Example 76, the target product (GT-05706) was prepared as white solid (3.3 mg, yield 19.82%). $^1$H NMR (400 MHz, DMSO-d6) δ 11.01 (s, 1H), 7.80 (d, *J* = 7.6 Hz, 1H), 7.62 (d, *J* = 15.4 Hz, 1H), 7.49 (d, *J* = 7.4 Hz, 1H), 7.38 (d, *J* = 8.0 Hz, 2H), 7.09 (d, *J* = 8.3 Hz, 2H), 5.13 (dd, *J* = 13.3, 5.1 Hz, 1H), 4.54 - 4.44 (m, 1H), 4.37 (dd, *J* = 17.5, 7.4 Hz, 1H), 3.18 - 3.02 (m, 2H), 2.90 (dd, *J* = 21.8, 9.6 Hz, 2H), 2.60 (d, *J* = 16.5 Hz, 2H), 2.47 - 2.26 (m, 2H), 2.24 - 2.07 (m, 2H), 1.99 (s, 3H), 1.41 (s, 2H), 1.27 - 1.20 (m, 3H), 0.95 (s, 6H). LCMS (ESI) calcd for $C_{34}H_{37}ClF_3N_4O_4^+$ [M+H]$^+$: 657.24, found, 657.3.

**Example 323: Preparation of 3-(5-((5-((4'-chloro-[1,1'-biphenyl]-2-yl)methyl)-2,5-diazabicyclo[2.2.1]heptan-2-yl) methyl)-1-oxoisoindolin-2-yl)piperidine-2,6-dione (GT-05707)**

Referring to the method of Scheme 1, the target product (GT-05707) was prepared as white solid (39 mg, yield 48.52%). $^1$H NMR (400 MHz, DMSO-d6) δ 11.01 (s, 1H), 7.88 (s, 2H), 7.78 (s, 2H), 7.50 (s, 4H), 7.42 (d, *J* = 8.1 Hz, 2H), 7.31 (s, 1H), 5.13 (dd, *J* = 13.3, 5.1 Hz, 1H), 4.49 (d, *J* = 17.7 Hz, 3H), 4.36 (d, *J* = 16.9 Hz, 2H), 4.28 - 4.17 (m, 1H), 4.02 (s, 3H), 3.44 (s, 2H), 3.35 - 3.25 (m, 2H), 2.98 - 2.87 (m, 1H), 2.61 (d, *J* = 17.3 Hz, 2H), 2.48 - 2.35 (m, 1H), 2.05 - 1.97 (m, 1H). LCMS (ESI) calcd for $C_{32}H_{32}ClN_4O_3^+$ [M+H]$^+$: 555.22, found, 555.2.

**Example 324: Preparation of 3-(5-((6-(4'-chloro-[1,1'-biphenyl]-2-carbonyl)-2,6-diazaspiro[3.3]heptan-2-yl) methyl)-1-oxoisoindolin-2-yl)piperidine-2,6-dione (GT-05708)**

Referring to the method of Scheme 1, the target product (GT-05708) was prepared as white solid (31 mg, yield 40.00%). $^1$H NMR (400 MHz, DMSO-d6) δ 11.27 (s, 1H), 11.01 (s, 1H), 7.78 (d, *J* = 7.7 Hz, 1H), 7.68 (s, 1H), 7.62 - 7.51 (m, 2H), 7.51 - 7.38 (m, 6H), 5.13 (dd, *J* = 13.3, 5.0 Hz, 1H), 4.48 (d, *J* = 17.6 Hz, 1H), 4.35 (d, *J* = 17.6 Hz, 2H), 4.20 (s, 1H), 4.08 - 3.82 (m, 4H), 3.80 - 3.54 (m, 2H), 3.36 (s, 1H), 3.13 (s, 1H), 2.99 - 2.84 (m, 1H), 2.61 (d, *J* = 16.3 Hz, 1H), 2.47 - 2.32 (m, 1H), 2.09 - 1.95 (m, 1H). LCMS (ESI) calcd for $C_{32}H_{30}ClN_4O_4^+$ [M+H]$^+$: 569.20, found, 569.2.

**Example 325: Preparation of 3-(5-((6-((4'-chloro-[1,1'-biphenyl]-2-yl)methyl)-2,6-diazaspiro[3.3]heptan-2-yl) methyl)-1-oxoisoindolin-2-yl)piperidine-2,6-dione (GT-05709)**

Referring to the method of Scheme 1, the target product (GT-05709) was prepared as white solid (8 mg, yield 9.95%). $^1$H NMR (400 MHz, DMSO-d6) δ 11.01 (s, 1H), 7.81 (s, 1H), 7.65 (s, 1H), 7.53 (d, *J* = 11.7 Hz, 6H), 7.39 (d, *J*= 7.9 Hz, 3H), 7.35 - 7.31 (m, 1H), 5.22 - 5.07 (m, 1H), 4.35 (s, 4H), 4.16 (s, 4H), 2.98 - 2.91 (m, 2H), 2.67 (s, 3H), 2.33 (s, 4H), 2.01 (s, 1H). LCMS (ESI) calcd for $C_{32}H_{32}ClN_4O_3^+$ [M+H]$^+$: 555.22, found, 555.2.

**Example 326: Preparation of 3-(5-((3-((4'-chloro-[1,1'-biphenyl]-2-yl)methyl)-3,8-diazabicyclo[3.2.1]octan-8-yl) methyl)-1-oxoisoindolin-2-yl)piperidine-2,6-dione (GT-05710)**

Referring to the method of Scheme 1, the target product (GT-05710) was prepared as white solid (35 mg, yield 42.59%). $^1$H NMR (400 MHz, DMSO-d6) δ 11.01 (s, 1H), 10.30 (s, 1H), 7.89 (s, 1H), 7.79 (q, *J* = 8.0 Hz, 2H), 7.50 (d, *J* = 8.5 Hz, 2H), 7.43 (d, *J* = 8.5 Hz, 3H), 7.37 (dd, *J* = 5.5, 3.5 Hz, 2H), 7.25 (dd, *J* = 8.3, 4.8 Hz, 1H), 5.13 (dd, *J* = 13.2, 5.1 Hz, 1H), 4.42 (dd, *J* = 50.7, 17.6 Hz, 2H), 4.24 (d, *J* = 5.8 Hz, 2H), 3.72 (s, 2H), 3.49 (s, 3H), 2.98 - 2.86 (m, 1H), 2.61 (d, *J* = 14.3 Hz, 3H), 2.48 - 2.34 (m, 2H), 2.14 (d, *J* = 9.5 Hz, 2H), 2.06 - 1.94 (m, 1H), 1.67 (s, 2H). LCMS (ESI) calcd for $C_{33}H_{34}ClN_4O_3^+$ [M+H]$^+$: 569.23, found, 569.3.

**Example 327: Preparation of 3-(5-((4-((4'-chloro-[1,1'-biphenyl]-2-yl)methyl)-2-(trifluoromethyl)piperazin-1-yl) methyl)-1-oxoisoindolin-2-yl)piperidine-2,6-dione (GT-05711)**

Referring to the method of Scheme 1, the target product (GT-05711) was prepared as white solid (21 mg, yield 71.96%). $^1$H NMR (400 MHz, DMSO-d6) δ 10.99 (s, 1H), 7.69 *(d, J*= 7.8 Hz, 1H), 7.51 (d, *J* = 13.6 Hz, 4H), 7.44 (d, *J* = 7.4 Hz, 5H), 7.29 (s, 1H), 5.10 (dd, *J* = 13.3, 5.1 Hz, 1H), 4.43 (d, *J* = 17.4 Hz, 1H), 4.33 - 4.24 (m, 1H), 3.98 (s, 2H), 3.46 (s, 2H), 3.06 - 2.84 (m, 3H), 2.82 - 2.70 (m, 1H), 2.62 (t, *J* = 18.5 Hz, 3H), 2.39 (ddd, *J* = 26.9, 13.5, 4.7 Hz, 2H), 2.03 - 1.96 (m, 1H). LCMS (ESI) calcd for $C_{32}H_{31}ClF_3N_4O_3^+$ [M+H]$^+$: 611.20, found, 611.3.

**Example 328: Preparation of 3-(5-((3-(4'-chloro-[1,1'-biphenyl]-2-carbonyl)-3,8-diazabicyclo[3.2.1]octan-8-yl) methyl)-1-oxoisoindolin-2-yl)piperidine-2,6-dione (GT-05788)**

Referring to the method of Scheme 1, the target product (GT-05788) was prepared as white solid (47 mg, yield 59.27%). $^1$H NMR (400 MHz, DMSO) δ 11.00 (s, 1H), 7.79 (t, *J* = 37.4 Hz, 3H), 7.63 - 7.36 (m, 7H), 7.34 (d, *J* = 8.1 Hz, 1H), 5.13 (dd, *J* = 12.9, 4.6 Hz, 1H), 4.35 (dt, *J* = 44.9, 17.1 Hz, 5H), 3.88 (d, *J* = 22.5 Hz, 1H), 3.62 (d, *J* = 51.3 Hz, 3H), 3.12 - 2.83 (m, 3H), 2.61 (d, *J* = 16.8 Hz, 1H), 2.38 (dd, *J* = 31.9, 18.4 Hz, 2H), 1.97 (dd, *J* = 29.5, 23.3 Hz, 2H), 1.24 (ddd, *J* = 49.0, 24.2, 19.6 Hz, 1H). LCMS (ESI) calcd for $C_{33}H_{32}ClN_4O_4^+$ [M+H]$^+$: 583.21, found, 583.2.

**Example 329: Preparation of 3-(5-((4-(4'-chloro-[1,1'-biphenyl]-2-carbonyl)-2-(trifluoromethyl)piperazin-1-yl) methyl)-1-oxoisoindolin-2-yl)piperidine-2,6-dione (GT-05789)**

Referring to the method of Scheme 1, the target product (GT-05789) was prepared as white solid (20 mg, yield 7.85%). $^1$H NMR (400 MHz, DMSO) δ 10.98 (s, 1H), 7.66 (d, *J* = 7.7 Hz, 1H), 7.55 (d, *J* = 8.4 Hz, 2H), 7.49 (d, *J* = 14.3 Hz, 3H), 7.42 (d, *J* = 8.3 Hz, 3H), 7.40 - 7.28 (m, 2H), 5.16 - 5.04 (m, 1H), 4.35 (dd, *J* = 52.7, 17.2 Hz, 3H), 4.08 - 3.84 (m, 2H), 3.67 (s, 1H), 3.27 (s, 1H), 3.10 (s, 1H), 2.91 (t, *J* = 12.8 Hz, 1H), 2.82 - 2.65 (m, 2H), 2.60 (d, *J* = 17.2 Hz, 2H), 2.38 (dd, *J* = 17.5, 8.4 Hz, 2H), 2.00 (s, 1H). LCMS (ESI) calcd for $C_{32}H_{29}ClF_3N_4O_4^+$ [M+H]$^+$: 625.18, found, 625.2.

**Example 330: Preparation of 3-(5-((4-((2-(furan-2-yl)-4,4-dimethylcyclohex-1-en-1-yl)methyl)piperazin-1-yl) methyl)-1-oxoisoindolin-2-yl)piperidine-2,6-dione (GT-05790)**

Referring to the method of Scheme 1, the target product (GT-05790) was prepared as white solid (49 mg, yield 63.42%). $^1$H NMR (400 MHz, DMSO-d6) δ 11.01 (s, 1H), 7.89 - 7.77 (m, 2H), 7.71 (s, 2H), 6.55 (d, *J* = 13.4 Hz, 2H), 5.14 (dd, *J* = 13.3, 5.1 Hz, 1H), 4.43 (dd, *J*= 51.1, 17.6 Hz, 4H), 4.04 (s, 3H), 3.49 (s, 4H), 3.33 - 3.28 (m, 2H), 2.97 - 2.87 (m, 1H), 2.61 (d, *J* = 18.2 Hz, 2H), 2.43 (dd, *J* = 13.3, 4.5 Hz, 1H), 2.36 (s, 2H), 2.16 (s, 2H), 2.04 - 1.95 (m, 1H), 1.40 (t, *J* = 6.2 Hz, 2H), 0.94 (s, 6H). LCMS (ESI) calcd for $C_{31}H_{39}N_4O_4^+$ [M+H]$^+$: 531.30, found, 531.3.

**Example 331: Preparation of 3-(5-((4-((4,4-dimethyl-2-(thiazol-5-yl)cyclohex-1-en-1-yl)methyl)piperazin-1-yl) methyl)-1-oxoisoindolin-2-yl)piperidine-2,6-dione (GT-05791)**

Referring to the method of Scheme 1, the target product (GT-05791)was prepared as white solid (31 mg, yield 39.02%). $^1$H NMR (400 MHz, DMSO) δ 11.01 (s, 1H), 9.15 (s, 1H), 7.86 (s, 1H), 7.81 (d, *J* = 7.8 Hz, 1H), 7.74 (*d, J* = 6.0 Hz, 2H), 5.14 (dd, *J* = 13.3, 5.1 Hz, 1H), 4.49 (*d, J* = 17.3 Hz, 3H), 4.37 (d, *J* = 17.6 Hz, 1H), 3.50 (s, 7H), 3.15 (s, 2H), 2.98 - 2.87 (m, 1H), 2.61 (d, *J* = 17.5 Hz, 2H), 2.43 (d, *J* = 16.9 Hz, 3H), 2.10 (s, 2H), 2.04 - 1.97 (m, 1H), 1.44 (t, *J* = 6.0 Hz, 2H), 0.94 (s, 6H). LCMS (ESI) calcd for $C_{30}H_{38}N_5O_3S^+$ [M+H]$^+$: 548.27, found, 548.3.

**Example 332: Preparation of 3-(1-oxo-5-((4-((4',5,5-trimethyl-3,4,5,6-tetrahydro-[1,1'-biphenyl]-2-yl)methyl)piperazin-1-yl)methyl)isoindolin-2-yl)piperidine-2,6-dione (GT-05792)**

Referring to the method of Scheme 1, the target product (GT-05792) was prepared as white solid (46 mg, yield 57.26%). $^1$H NMR (400 MHz, DMSO) δ 11.01 (s, 1H), 7.79 (d, *J* = 7.7 Hz, 2H), 7.66 (s, 1H), 7.17 (d, *J* = 7.7 Hz, 2H), 6.97 (d, *J* = 7.7 Hz, 2H), 5.13 (dd, *J* = 13.4, 5.1 Hz, 1H), 4.41 (dd, *J* = 51.0, 17.5 Hz, 3H), 4.29 - 4.08 (m, 1H), 3.51 (s, 4H), 3.24 - 3.11 (m, 2H), 2.91 (dd, *J* = 21.7, 9.1 Hz, 2H), 2.61 (d, *J* = 17.7 Hz, 2H), 2.42 (dd, *J* = 17.3, 8.6 Hz, 2H), 2.30 (s, 5H), 2.01 (s, 3H), 1.45 (d, *J* = 5.7 Hz, 2H), 1.28 (dd, *J* = 10.6, 6.6 Hz, 1H), 0.95 (s, 6H). LCMS (ESI) calcd for $C_{34}H_{43}N_4O_3^+$ [M+H]$^+$: 555.33, found, 555.3.

**Example 333: Preparation of 2-(2,6-dioxopiperidin-3-yl)-5-((4-((4',5,5-trimethyl-3,4,5,6-tetrahydro-[1,1'-biphenyl]-2-yl)methyl)piperazin-1-yl)methyl)isoindoline-1,3-dione (GT-05793)**

Referring to the method of Scheme 1, the target product (GT-05793) was prepared as white solid (14 mg, yield 19.14%). $^1$H NMR (400 MHz, DMSO) δ 11.14 (s, 1H), 8.00 (d, *J* = 31.0 Hz, 3H), 7.18 (d, *J* = 7.7 Hz, 2H), 6.97 (d, *J* = 7.6 Hz, 2H), 5.17 (dd, *J* = 12.7, 5.3 Hz, 1H), 4.19 (s, 2H), 3.52 (s, 2H), 3.33 - 3.26 (m, 2H), 3.20 - 2.97 (m, 3H), 2.91 (dd, *J* = 22.1, 9.0 Hz, 2H), 2.80 - 2.70 (m, 1H), 2.58 (dd, *J* = 23.3, 11.2 Hz, 3H), 2.31 (s, 5H), 2.10 - 2.04 (m, 1H), 2.01 (s, 2H), 1.45 (d, *J* = 5.8 Hz, 2H), 0.95 (s, 6H). LCMS (ESI) calcd for $C_{34}H_{41}N_4O_4^+$ [M+H]$^+$: 569.31, found, 569.3.

**Example 334: Preparation of 2-(2,6-dioxopiperidin-3-yl)-5-(4-((4',5,5-trimethyl-3,4,5,6-tetrahydro-[1,1'-biphenyl]-2-yl)methyl)piperazine-1-carbonyl)isoindoline-1,3-dione (GT-05794)**

Referring to the methods of Scheme 6 and Example 76, the target product (GT-05794) was prepared as white solid (25 mg, yield 30.59%). $^1$H NMR (400 MHz, DMSO) δ 11.15 (s, 1H), 10.24 (s, 1H), 8.05 - 7.92 (m, 2H), 7.88 (d, *J* = 7.7 Hz, 1H), 7.21 (d, *J* = 7.6 Hz, 2H), 7.00 (d, *J* = 7.5 Hz, 2H), 5.18 (dd, *J* = 12.8, 5.4 Hz, 1H), 4.45 (s, 1H), 3.62 (s, 4H), 3.47 (s, 2H), 3.23 - 3.16 (m, 1H), 2.96 - 2.82 (m, 2H), 2.67 (s, 1H), 2.65 - 2.55 (m, 2H), 2.41 (s, 1H), 2.31 (d, *J* = 7.4 Hz, 3H), 2.29 - 2.19 (m, 1H), 2.10 - 1.96 (m, 3H), 1.47 (s, 2H), 0.96 (s, 6H). LCMS (ESI) calcd for $C_{34}H_{39}N_4O_5^+$ [M+H]$^+$: 583.29, found, 583.3.

**Example 335: Preparation of 3-(5-(5-((4'-chloro-5,5-dimethyl-3,4,5,6-tetrahydro-[1,1'-biphenyl]-2-yl)methyl)-2,5-diazabicyclo[2.2.2]octane-2-carbonyl)-1-oxoisoindolin-2-yl)piperidine-2,6-dione (GT-05795)**

Referring to the methods of Scheme 6 and Example 76, the target product (GT-05795) was prepared as white solid (32 mg, yield 35.33%). $^1$H NMR (400 MHz, DMSO-d6) δ 11.03 (d, *J* = 12.0 Hz, 1H), 9.89 (s, 1H), 7.81 (dd, *J* = 9.8, 5.5 Hz, 1H), 7.60 (d, *J* = 19.4 Hz, 1H), 7.51 (dd, *J* = 13.5, 5.4 Hz, 1H), 7.46 (d, *J* = 8.1 Hz, 1H), 7.25 - 7.11 (m, 2H), 5.14 (dd, *J* = 12.5, 4.3 Hz, 1H), 4.51 (dd, *J* = 16.6, 7.7 Hz, 1H), 4.38 (dd, *J* = 17.7, 3.4 Hz, 1H), 3.84 - 3.63 (m, 4H), 3.59 - 3.39 (m, 2H), 3.29 - 3.10 (m, 1H), 3.06 - 2.84 (m, 2H), 2.61 (d, *J* = 16.6 Hz, 1H), 2.48 - 2.15 (m, 4H), 2.13 - 1.88 (m, 4H), 1.81 (s, 1H), 1.53 - 1.40 (m, 2H), 1.31 (s, 1H), 0.92 (s, 6H). LCMS (ESI) calcd for $C_{35}H_{40}ClN_4O_4^+$ [M+H]$^+$: 615.27, found, 615.3.

**Example 336: Preparation of 3-(5-((4-((4'-chloro-5,5-dimethyl-3,4,5,6-tetrahydro-[1,1'-biphenyl]-2-yl) methyl)-2-(trifluoromethyl)piperazin-1-yl)methyl)-1-oxoisoindolin-2-yl)piperidine-2,6-dione (GT-05796)**

Referring to the method of Scheme 1, the target product (GT-05796) was prepared as white solid (5.9 mg, yield 23.54%). $^1$H NMR (400 MHz, DMSO) δ 10.99 (s, 1H), 7.69 (d, *J* = 7.7 Hz, 1H), 7.52 (s, 1H), 7.43 (d, *J* = 7.6 Hz, 3H), 7.10 (s, 2H), 5.10 (dd, *J* = 13.3, 5.2 Hz, 1H), 4.37 (dd, *J* = 52.3, 17.3 Hz, 3H), 4.02 (s, 2H), 3.13 - 2.98 (m, 1H), 2.90 (dd, *J* = 22.2, 9.1 Hz, 2H), 2.79 (s, 2H), 2.68 - 2.54 (m, 3H), 2.44 - 2.32 (m, 2H), 2.29 - 2.17 (m, 1H), 2.01 (s, 4H), 1.44 (s, 2H), 0.94 (s, 6H). LCMS (ESI) calcd for $C_{34}H_{39}ClF_3N_4O_3^+$ [M+H]$^+$: 643.27, found, 643.3.

**Example 337: Preparation of 3-(5-((4-((4'-chloro-5,5-dimethyl-3,4,5,6-tetrahydro-[1,1'-biphenyl]-2-yl) methyl)-2-(trifluoromethyl)piperazin-1-yl)methyl)-4-fluoro-1-oxoisoindolin-2-yl)piperidine-2,6-dione (GT-06100)**

Referring to the method of Scheme 1, the target product (GT-06100) was prepared as white solid (14 mg, yield 21.92%). $^1$H NMR (400 MHz, DMSO-d6) δ 11.01 (s, 1H), 10.38 (s, 1H), 7.58 (s, 2H), 7.40 (s, 2H), 7.09 (s, 2H), 5.14 - 5.06 (m, 1H), 4.55 (d, *J* = 17.4 Hz, 1H), 4.37 (d, *J* = 17.4 Hz, 1H), 4.00 (s, 2H), 3.62 (s, 2H), 3.39 (s, 2H), 3.10 (s, 1H), 2.97 - 2.74 (m, 3H), 2.63 (t, *J* = 17.8 Hz, 3H), 2.39 (dd, *J* = 30.6, 16.6 Hz, 2H), 2.22 (s, 1H), 2.01 (s, 3H), 1.44 (s, 2H), 0.95 (s, 6H). LCMS (ESI) calcd for $C_{34}H_{38}ClF_4N_4O_3^+$ [M+H]$^+$: 661.26, found, 661.3.

**Example 338: Preparation of 3-(5-((4-((4'-chloro-5,5-dimethyl-3,4,5,6-tetrahydro-[1,1'-biphenyl]-2-yl) methyl)-2-(trifluoromethyl)piperazin-1-yl)methyl)-6-fluoro-1-oxoisoindolin-2-yl)piperidine-2,6-dione (GT-06101)**

Referring to the method of Scheme 1, the target product (GT-06101) was prepared as white solid (17.6 mg, yield 27.56%). $^1$H NMR (400 MHz, DMSO-d6) δ 11.00 (s, 1H), 10.73 (s, 1H), 7.64 (d, *J* = 6.0 Hz, 1H), 7.50 (d, *J*= 8.7 Hz, 1H), 7.41 (d, *J* = 7.3 Hz, 2H), 7.10 (s, 2H), 5.11 (dd, *J* = 13.1, 4.9 Hz, 1H), 4.37 (dd, *J* = 49.8, 17.4 Hz, 2H), 4.00 (s, 2H), 3.54 (s, 3H), 3.09 (s, 2H), 2.98 - 2.78 (m, 3H), 2.60 (d, *J* = 16.5 Hz, 3H), 2.39 (dt, *J* = 26.8, 13.4 Hz, 2H), 2.25 (s, 1H), 2.01 (s, 3H), 1.44 (s, 2H), 0.95 (s, 6H). LCMS (ESI) calcd for $C_{34}H_{38}ClF_4N_4O_3^+$ [M+H]$^+$: 661.26, found, 661.3.

**Example 339: Preparation of 3-(5-((4-((4'-chloro-5,5-dimethyl-3,4,5,6-tetrahydro-[1,1'-biphenyl]-2-yl)methyl)-2-(trifluoromethyl)piperazin-1-yl)methyl)-7-fluoro-1-oxoisoindolin-2-yl)piperidine-2,6-dione (GT-06102)**

Referring to the method of Scheme 1, the target product (GT-06102) was prepared as white solid (9 mg, yield 14.09%). $^1$H NMR (400 MHz, DMSO-d6) δ 11.00 (s, 1H), 10.54 (s, 1H), 7.42 (s, 2H), 7.35 (s, 1H), 7.20 (d, *J* = 10.3 Hz, 1H), 7.10 (s, 2H), 5.07 (d, *J* = 8.6 Hz, 1H), 4.45 (d, *J* = 17.4 Hz, 1H), 4.32 (d, *J* = 17.4 Hz, 1H), 4.03 (s, 2H), 3.63 (s, 3H), 3.06 (s, 1H), 2.99 - 2.78 (m, 3H), 2.62 (t, *J* = 19.1 Hz, 3H), 2.37 (d, *J* = 12.4 Hz, 2H), 2.27 - 2.12 (m, 1H), 2.01 (s, 4H), 1.44 (s, 2H), 0.95 (s, 6H). LCMS (ESI) calcd for $C_{34}H_{38}ClF_4N_4O_3^+$ [M+H]$^+$: 661.26, found, 661.3.

**Example 340: Preparation of 5-((4-((4'-chloro-5,5-dimethyl-3,4,5,6-tetrahydro-[1,1'-biphenyl]-2-yl)methyl)-2-(trifluoromethyl)piperazin-1-yl)methyl)-2-(2,6-dioxopiperidin-3-yl)isoindoline-1,3-dione (GT-06103)**

Referring to the method of Scheme 1, the target product (GT-06103) was prepared as white solid (9.5 mg, yield 14.96%). $^1$H NMR (400 MHz, DMSO-d6) δ 11.12 (s, 1H), 7.82 (dd, *J* = 27.3, 11.8 Hz, 4H), 7.42 (s, 2H), 7.09 (s, 2H), 5.46 - 4.96 (m, 2H), 4.09 (s, 2H), 3.73 (s, 2H), 3.11 (s, 2H), 2.87 (d, *J* = 13.8 Hz, 3H), 2.72 - 2.53 (m, 4H), 2.33 (s, 2H), 2.01 (s, 4H), 1.44 (s, 2H), 0.95 (s, 6H). LCMS (ESI) calcd for $C_{34}H_{37}ClF_3N_4O_4^+$ [M+H]$^+$: 657.24, found, 657.3.

**Example 341: Preparation of 3-(5-((4-(4'-chloro-5,5-dimethyl-3,4,5,6-tetrahydro-[1,1'-biphenyl]-2-carbonyl)-2-(trifluoromethyl)piperazin-1-yl)methyl)-1-oxoisoindolin-2-yl)piperidine-2,6-dione (GT-05845)**

Referring to the method of Scheme 1, the target product (GT-05845) was prepared as white solid (5.6 mg, yield 17.94%). $^1$H NMR (400 MHz, DMSO-d6) δ 10.98 (s, 1H), 7.67 (d, *J* = 7.7 Hz, 1H), 7.50 (d, *J* = 8.0 Hz, 1H), 7.40 (dd, *J* = 16.1, 8.7 Hz, 3H), 7.22 (dd, *J* = 19.1, 8.1 Hz, 2H), 5.17 - 5.00 (m, 1H), 4.43 (d, *J* = 17.4 Hz, 1H), 4.30 (d, *J* = 17.3 Hz, 1H), 4.00 - 3.65 (m, 3H), 3.54 (s, 1H), 3.37 (s, 2H), 3.19 (s, 1H), 2.90 (dd, *J* = 21.5, 9.5 Hz, 1H), 2.60 (d, *J* = 16.5 Hz, 3H), 2.38 (dd, *J* = 24.9, 15.7 Hz, 3H), 2.17 - 1.87 (m, 3H), 1.44 (s, 2H), 1.09 (s, 6H). LCMS (ESI) calcd for $C_{34}H_{37}ClF_3N_4O_4^+$ [M+H]$^+$: 657.24, found, 657.3.

**Example 342: Preparation of 3-(5-((4-((4'-fluoro-5,5-dimethyl-3,4,5,6-tetrahydro-[1,1'-biphenyl]-2-yl)methyl)-2-(trifluoromethyl)piperazin-1-yl)methyl)-1-oxoisoindolin-2-yl)piperidine-2,6-dione (GT-05846)**

Referring to the method of Scheme 1, the target product (GT-05846) was prepared as white solid (11.3 mg, yield 32.96%). $^1$H NMR (400 MHz, DMSO-d6) δ 10.99 (s, 1H), 7.69 (d, *J* = 7.6 Hz, 1H), 7.52 (s, 1H), 7.43 (d, *J* = 7.9 Hz, 1H), 7.14 (d, *J* = 36.3 Hz, 5H), 5.10 (dd, *J* = 13.2, 5.2 Hz, 1H), 4.43 (d, *J* = 17.2 Hz, 1H), 4.30 (d, *J* = 17.4 Hz, 1H), 4.02 (s, 2H), 2.93 (d, *J* = 13.5 Hz, 2H), 2.78 (s, 2H), 2.60 (d, *J* = 16.*8* Hz, 2H), 2.38 (dd, *J* = 20.3, 11.1 Hz, 3H), 2.28 - 2.14 (m, 2H), 2.01 (s, 4H), 1.44 (s, 2H), 0.94 (s, 6H). LCMS (ESI) calcd for $C_{34}H_{39}F_4N_4O_3^+$ [M+H]$^+$: 627.30, found, 627.3.

**Example 343: Preparation of 3-(5-((4-((4,4-dimethyl-2-(thiophen-2-yl)cyclohex-1-en-1-yl)methyl)piperazin-1-yl)methyl)-1-oxoisoindolin-2-yl)piperidine-2,6-dione (GT-05847)**

Referring to the method of Scheme 1, the target product (GT-05847) was prepared as white solid (41 mg, yield 48.30%). $^1$H NMR (400 MHz, DMSO-d6) δ 11.01 (s, 1H), 7.79 (d, *J* = 7.7 Hz, 2H), 7.70 (s, 1H), 7.54 (d, *J* = 4.8 Hz, 1H), 7.11 - 6.97 (m, 1H), 6.92 (s, 1H), 5.14 (dd, *J* = 13.3, 5.1 Hz, 1H), 4.49 (d, *J* = 17.6 Hz, 1H), 4.36 (d, *J* = 17.5 Hz, 2H), 3.59 (s, 2H), 3.46 (s, 4H), 3.36 - 3.27 (m, 4H), 3.00 - 2.86 (m, 2H), 2.61 (d, *J* = 16.8 Hz, 1H), 2.43 (dd, *J* = 13.1, 4.5 Hz, 1H), 2.34 (s, 2H), 2.11 (s, 2H), 2.04 - 1.95 (m, 1H), 1.43 (t, *J* = 6.1 Hz, 2H), 0.94 (s, 6H). LCMS (ESI) calcd for $C_{31}H_{39}N_4O_3S^+$ [M+H]$^+$: 547.27, found, 547.3.

**Example 344: Preparation of 3-(5-((4-((4,4-dimethyl-2-(oxazol-5-yl)cyclohex-1-en-1-yl)methyl)piperazin-1-yl)methyl)-1-oxoisoindolin-2-yl)piperidine-2,6-dione (GT-05848)**

Referring to the method of Scheme 1, the target product (GT-05848) was prepared as white solid (11.7 mg, yield 28.46%). $^1$H NMR (400 MHz, DMSO) δ 11.01 (s, 1H), 8.35 (s, 1H), 7.85 (s, 1H), 7.80 (d, *J* = 7.7 Hz, 1H), 7.75 (s, 1H), 7.31 (s, 1H), 5.14 (dd, *J* = 13.4, 5.0 Hz, 1H), 4.43 (dd, *J* = 51.3, 17.6 Hz, 5H), 3.94 (s, 3H), 3.58 (s, 3H), 3.00 - 2.84 (m, 2H), 2.61 (d, *J* = 17.9 Hz, 3H), 2.46 - 2.36 (m, 3H), 2.16 (s, 2H), 2.04 - 1.96 (m, 1H), 1.41 (t, *J* = 5.9 Hz, 2H), 0.94 (s, 6H). LCMS (ESI) calcd for $C_{30}H_{38}N_5O_4^+$ [M+H]$^+$: 532.29, found, 532.3.

**Example 345: Preparation of 3-(5-((4-(2-(oxazol-5-yl)benzyl)piperazin-1-yl)methyl)-1-oxoisoindolin-2-yl)piperidine-2,6-dione (GT-05849)**

Referring to the method of Scheme 1, the target product (GT-05849) was prepared as white solid (33 mg, yield 42.08%). $^1$H NMR (400 MHz, DMSO) δ 11.01 (s, 1H), 8.52 (s, 1H), 7.88 (s, 1H), 7.78 (dd, *J* = 18.6, 9.2 Hz, 4H), 7.65 (s, 1H), 7.52 (dt, *J* = 19.6, 7.1 Hz, 2H), 5.14 (dd, *J* = 13.3, 5.1 Hz, 1H), 4.49 (d, *J* = 17.4 Hz, 3H), 4.36 (d, *J* = 17.6 Hz, 3H), 3.41 - 3.19 (m, 6H), 2.99 - 2.84 (m, 1H), 2.61 (d, *J* = 16.9 Hz, 1H), 2.43 (dd, *J* = 13.2, 4.4 Hz, 1H), 2.05 - 1.92 (m, 1H). LCMS (ESI) calcd for $C_{28}H_{30}N_5O_4^+$ $[M+H]^+$: 500.23, found, 500.2.

**Example 346: Preparation of 3-(5-(3-(4'-chloro-5,5-dimethyl-3,4,5,6-tetrahydro-[1,1'-biphenyl]-2-carbonyl)-3,6-diazabicyclo[3.1.1]heptane-6-carbonyl)-1-oxoisoindolin-2-yl)piperidine-2,6-dione (GT-05538)**

Referring to the methods of Scheme 6 and Example 76, the target product (GT-05538) was prepared as white solid (29 mg, yield 34%). $^1$H NMR (400 MHz, DMSO-d6) δ 11.01 (s, 1H), 7.88 - 7.64 (m, 2H), 7.50 (d, *J* = 14.3 Hz, 1H), 7.38 (d, *J* = 6.6 Hz, 2H), 7.32 - 7.22 (m, 2H), 5.16 - 5.13 (m, 1H), 4.66 - 4.22 (m, 4H), 3.58 - 3.51 (m, 1H), 2.92 (t, *J* = 15.5 Hz, 1H), 2.63 - 2.59 (m, 2H), 2.46 - 2.36 (m, 4H), 2.28 - 2.14 (m, 1H), 2.07 - 2.01 (m, 1H), 1.97 - 1.73 (m, 2H), 1.59 - 1.21 (m, 4H), 0.99 - 0.93 (m, 6H). LCMS (ESI) calcd for $C_{34}H_{36}ClN_4O_5^+$ $[M+H]^+$: 615.24, found, 615.3.

**Example 347: Preparation of 3-(5-(3-(4'-chloro-5,5-dimethyl-3,4,5,6-tetrahydro-[1,1'-biphenyl]-2-carbonyl)-3,8-diazabicyclo [3.2.1] octane-8-carbonyl)-1-oxoisoindolin-2-yl)piperidine-2,6-dione (GT-05539)**

Referring to the methods of Scheme 6 and Example 76, the target product (GT-05539) was prepared as white solid (34 mg, yield 41%). $^1$H NMR (400 MHz, DMSO-d6) δ 11.01 (s, 1H), 7.77 (d, *J*= 7.2 Hz, 1H), 7.67 (s, 1H), 7.55 (d, *J* = 7.6 Hz, 1H), 7.41 - 7.35 (m, 3H), 7.21 - 7.14 (m, 1H), 5.13 (dd, *J* = 13.0, 4.9 Hz, 1H), 4.64 - 4.30 (m, 3H), 3.98 - 3.77 (m, 2H), 3.20 (brs, 1H), 2.95 - 2.87 (m, 2H), 2.61 (d, *J* = 16.7 Hz, 1H), 2.49 - 2.40 (m, 4H), 2.28 - 2.24 (m, 1H), 2.17 - 2.11 (m, 1H), 2.16 - 2.08 (m, 1H), 1.93 - 1.85 (m, 2H), 1.61 - 1.27 (m, 4H), 0.96 (s, 6H). LCMS (ESI) calcd for $C_{35}H_{38}ClN_4O_5^+$ $[M+H]^+$: 629.25, found, 629.3.

**Example 348: Preparation of 3-(5-(8-(4'-chloro-5,5-dimethyl-3,4,5,6-tetrahydro-[1,1'-biphenyl]-2-carbonyl)-3,8-diazabicyclo [3.2.1] octane-3-carbonyl)-1-oxoisoindolin-2-yl)piperidine-2,6-dione (GT-05540)**

Referring to the methods of Scheme 6 and Example 76, the target product (GT-05540) was prepared as white solid (29 mg, yield 35%). $^1$H NMR (400 MHz, DMSO-d6) δ 11.00 (s, 1H), 7.76 (d, *J*= 7.7 Hz, 1H), 7.56 (brs, 1H), 7.46 - 7.33 (m, 3H), 7.29 - 7.23 (m, 2H), 5.13 (dd, *J* = 13.3, 5.2 Hz, 1H), 4.56 - 4.13 (m, 4H), 3.88 - 3.67 (m, 1H), 3.25 - 3.20 (m, 1H), 3.17 - 3.13 (m, 1H), 2.95 - 2.87 (m, 1H), 2.50 - 2.45 (m, 2H), 2.46 - 2.35 (m, 2H), 2.30 - 2.18 (m, 2H), 2.07 - 1.94 (m, 2H), 1.53 - 1.33 (m, 5H), 0.99 - 0.94 (m, 6H). LCMS (ESI) calcd for $C_{35}H_{38}ClN_4O_5^+$ $[M+H]^+$: 629.25, found, 629.3.

**Example 349: Preparation of 3-(1-oxo-5-((4-(5-phenylpyrazine-2-carbonyl)piperazin-1-yl)methyl)isoindolin-2-yl)piperidine-2,6-dione (GT-05635)**

Referring to the method of Scheme 6, the target product (GT-05635) was prepared as white solid (58 mg, yield 63%). $^1$H NMR (400 MHz, DMSO-d6) δ 11.50 (s, 1H), 11.01 (s, 1H), 9.29 (d, *J* = 1.4 Hz, 1H), 8.96 (d, *J* = 1.3 Hz, 1H), 8.21 (dd, *J* = 7.6, 1.9 Hz, 2H), 7.91 - 7.80 (m, 2H), 7.75 (d, *J* = 7.7 Hz, 1H), 7.63 - 7.51 (m, 3H), 5.15 (dd, *J* = 13.3, 5.1 Hz, 1H), 4.62 (brs, 1H), 4.56 - 4.34 (m, 4H), 4.23 - 4.19 (m, 1H), 3.68 (t, *J* = 15.4 Hz, 1H), 3.54 - 3.44 (m, 3H), 3.23 - 3.14 (m, 2H), 3.02 - 2.84 (m, 1H), 2.61 (d, *J* = 17.5 Hz, 1H), 2.47 - 2.35 (m, 1H), 2.09 - 1.92 (m, 1H). LCMS (ESI) calcd for $C_{29}H_{29}N_6O_4^+$ $[M+H]^+$: 525.22, found, 525.3.

**Example 350: Preparation of 3-(7-((5-((4'-chloro-5,5-dimethyl-3,4,5,6-tetrahydro-[1,1'-biphenyl]-2-yl)methyl)-2,5-diazabicyclo[2.2.2]octan-2-yl)methyl)-1-oxoisoindolin-2-yl)piperidine-2,6-dione (GT-05462)**

Referring to the method of Scheme 1, the target product GT-04169 was prepared as white solid(GT-05462) (9 mg, yield 20.66%). $^1$H NMR (400 MHz, DMSO) δ 11.69 (s, 1H), 11.05 (s, 1H), 7.94 (s, 1H), 7.72 (s, 2H), 7.52 - 7.30 (m, 2H), 7.21 (s, 2H), 5.01 (d, J = 121.5 Hz, 3H), 4.46 (d, J = 31.8 Hz, 2H), 3.72 (d, J = 31.3 Hz, 7H), 2.97 (dd, J = 50.9, 37.5 Hz, 2H), 2.75 - 2.59 (m, 2H), 2.33 (s, 2H), 2.25 1.83 (m, 6H), 1.39 (d, J = 47.0 Hz, 3H), 0.96 (s, 6H). LCMS (ESI) calcd for $C_{35}H_{42}ClN_4O_3^+$ $[M+H]^+$: 601.29, found, 601.3.

**Example 351: Preparation of 3-(7-((4-(4'-fluoro-3,4,5,6-tetrahydro-[1,1'-biphenyl]-2-carbonyl)piperazin-1-yl) methyl)-1-oxoisoindolin-2-yl)piperidine-2,6-dione (GT-05469)**

Referring to the method of Scheme 6, the target product (GT-05469) was prepared as white solid (22 mg, yield 29.12%). $^1$H NMR (400 MHz, DMSO) δ 11.56 (s, 1H), 11.05 (s, 1H), 10.42 (s, 1H), 7.71 (d, J = 5.8 Hz, 3H), 7.16 (dd, J = 58.3, 30.6 Hz, 4H), 5.12 (s, 1H), 4.89 - 4.23 (m, 6H), 3.73 (d, J = 13.6 Hz, 2H), 3.43 - 2.88 (m, 6H), 2.61 (s, 2H), 2.05 (s, 4H), 1.71 (s, 4H). LCMS (ESI) calcd for $C_{31}H_{34}FN_4O_4^+$ [M+H]$^+$: 545.26, found, 545.3.

**Biological activity assay**

**Reagents and Materials:**

| Reagents and materials | Suppliers or Source |
|---|---|
| Human T cell acute lymphoblastic leukemia cell line: | Dalian Meilun Biotech Co., Ltd. CCRF-CEM |
| Human acute lymphoblastic leukemia cell: Kasumi-1 | ATCC (American Type Culture Collection) |
| Human breast cancer cell: MDA-MB-231 | Dalian Meilun Biotech Co., Ltd. |
| Human mantle cell lymphoma cells: Mino | ATCC |
| Human multiple myeloma cell line resistant to dexamethasone: MM.1R | ATCC |
| Human multiple myeloma cells: MM.1S | ATCC |
| Human B Lymphoma Cell: Ramos | Dalian Meilun Biotech Co., Ltd. |
| Human B lymphoma cell SU-DHL-4 | ATCC |
| Human diffuse large B-cell lymphoma cell: SU-DHL-6 | ATCC |
| Human diffuse large B-cell lymphoma cell line: TMD8 | Kyinno Biotechnology Co., Ltd |
| Human colon cancer cell: SW620 | ATCC |
| Human myelomonocytic leukemia cells: MV-4-11 | ATCC |
| RPMI-1640 Medium | Dalian Meilun Biotech Co., Ltd. |
| DMEM Medium | Dalian Meilun Biotech Co., Ltd. |
| IMDM Medium | Gibco Company |
| L-15 Medium | ATCC |
| Meilun Cell Counting Kit-8 | Dalian Meilun Biotech Co., Ltd. |
| Fetal bovine serum (FBS) | Sunrise Science Products Company |
| Penicillin-Streptomycin | Beijing TransGen Biotech Co., Ltd. |
| Mycoplasma detection kit | Beijing TransGen Biotech Co., Ltd. |
| STR genotype detection | Shanghai Biowing Applied Biotechnology Co. Ltd. |

**Methods:**

**Cell cultures**

The tumor cells used herein were cultured in a cell culture medium listed in table A at 37°C in an incubator with 5% $CO_2$. Prior to experimentation, all cells used were correctly identified by STR profiling, and tested negative for mycoplasma through routine screenings.

Table A. Tumor cells and cell culture medium used in the present disclosure

| Cell line | Cell culture medium used |
|---|---|
| CCRF-CEM | RPMI 1640 culture medium supplemented with 10% FBS, as well as penicillin at a final concentration of 100 U/mL and streptomycin at a final concentration of 100 μg/mL |
| Kasumi-1 | |
| Mino | |
| MM.1R | |

(continued)

| Cell line | Cell culture medium used |
|---|---|
| MM.1S | |
| Ramos | |
| SU-DHL-4 | |
| SU-DHL-6 | |
| TMD8 | |
| MDA-MB-231 | DMEM culture medium supplemented with 10% FBS, as well as penicillin at a final concentration of 100 U/mL and streptomycin at a final concentration of 100 μg/mL |
| SW620 | L-15 culture medium supplemented with 10% FBS, as well as penicillin at a final concentration of 100 U/mL and streptomycin at a final concentration of 100 μg/mL |
| MV-4-11 | IMEM culture medium supplemented with 10% FBS, as well as |
| | penicillin at a final concentration of 100 U/mL and streptomycin at a final concentration of 100 μg/mL |

**I. Determination of half inhibitory concentration ($IC_{50}$) of the compounds against tumor cells::**

$IC_{50}$ values of the compounds of the present disclosure (including the compounds in Table 1 and the Examples compounds) were measured using the Meilun Cell Counting Kit-8 kit from Dalian Meilun Biotech Co., Ltd. Assay details are as follows: tumor cells were seeded in a 96-well plate at a density listed in Table B. After 24h, 100 μL of the inoculated cells were treated with 0.5μL of the compounds of the present disclosure (including the compounds in Table 1 and the Examples compounds) at 10 different successively decreasing concentrations (starting at the highest concentration of 10μM; 5-fold serial dilutions). After the cells were treated with the compounds of the present disclosure for a period of time, the cell viability detection reagent was added to the culture medium for cell viability assessment according to the instructions provided with the CCK-8 kit. DMSO served as the negative control, and corresponding commercial inhibitors (including lenalidomide, (CAS No.: 191732-72-6); pomalidomide, (CAS No.: 19171-19-8)) were used as the positive control, both of which treated the cells using the same protocol as that of the compounds of the present disclosure. The growth inhibition of the compounds of the present disclosure on cells was plotted by Prism Graphpad software, and the $IC_{50}$ values of the compounds of the present disclosure were calculated therefrom.

Table B. Seeding protocol and treatment time for tumor cells

| Cells | Cells seeding protocol | The times for treating cells with the compounds of the present disclosure (hours) |
|---|---|---|
| Kasumi-1 | cells were seeded at a density of 10,000 cells per well in 100 μL of serum-containing RPMI-1640 medium per well | 72 h |
| Mino | | |
| MM.1R | | |
| MM.1S | | |
| Ramos | | |
| SU-DHL-4 | | |
| SU-DHL-6 | | |
| TMD8 | | |
| CCRF-CEM | cells were seeded at a density of 4,000 cells per well in 100 μL of serum-containing RPMI-1640 medium per well | 96 h |
| MDA-MB-231 | cells were seeded at a density of 4,000 cells per well in 100 μL of serum-containing DMEM medium per well | 96 h |

(continued)

| Cells | Cells seeding protocol | The times for treating cells with the compounds of the present disclosure (hours) |
|---|---|---|
| SW620 | cells were seeded at a density of 4,000 cells per well in 100 μL of serum-containing L-15 medium per well | 96 h |
| MV-4-11 | cells were seeded at a density of 10,000 cells per well in 100 μL of serum-containing IMDM medium per well | 96 h |

Results of inhibiting tumor cell proliferation were shown in Table C below.

Table C. $IC_{50}$ values (in nM) of the compounds of the present disclosure for inhibiting the proliferation of tumor cells

| Comp. No. | CCR F-CE M | Kasu mi-1 | MD A-MB-231 | Mi no | MM. 1R | MM. 1S | Ram os | SU-DH L-4 | SU-DH L-6 | TM D8 | SW6 20 | Dau di | M V-4-11 |
|---|---|---|---|---|---|---|---|---|---|---|---|---|---|
| lenalidom ide | F | F | F | F | F | F | F | F | F | F | F | F | F |
| pomalido mide | F | F | F | D | F | B | D | F | F | C | F | E | F |
| GT-02785 | A3 | A4 | A4 | A2 | A3 | A1 | B | - | - | B | A4 | 159. 1 | 24. 0 |
| GT-03468 | A3 | A3 | A3 | B | A3 | A3 | B | E | - | B | A3 | | |
| GT-03578 | A1 | A1 | A1 | - | A2 | A1 | A3 | B | D | A3 | - | | |
| GT-03469 | D | - | B | - | - | - | - | - | - | - | - | | |
| GT-03355 | A1 | A1 | A1 | A1 | A1 | A1 | A2 | B | A3 | A1 | A2 | | |
| GT-03694 | - | - | - | A2 | - | - | - | B | - | - | A1 | | |
| GT-03695 | - | - | - | A2 | - | - | - | A4 | - | - | A2 | | |
| GT-03356 | D | - | - | - | - | - | E | - | B | - | - | | |
| GT-03454 | A3 | - | - | - | B | - | D | - | - | D | - | | |
| GT-05364 | B | | | | | B | | | | | | | |
| GT-05370 | | | | | | A1 | | | | | | | |
| GT-05382 | B | | | A3 | | A3 | | | | | | B | |
| GT-05383 | | | | | | C | | | | | | | |
| GT-05390 | A4 | | | A3 | | A3 | | | | | | | B |
| GT-05391 | A1 | A3 | | A2 | | A2 | | | | | | A3 | A1 |
| GT-05396 | | | | | | B | | | | | | | |
| GT-05403 | | | | | | A2 | | | | | | | |
| GT-05466 | | | | A3 | | A3 | | | | | | A4 | |
| GT-05467 | A2 | A3 | | A3 | | A3 | | | | | | B | A3 |
| GT-05482 | A2 | A3 | | A2 | | A2 | | | | | | A3 | A2 |
| GT-05494 | B | | | | | C | | | | | | | B |
| GT-05497 | A4 | B | | B | | B | | | | | | C | B |
| GT-05640 | | | | A3 | | B | | | | | | | |
| GT-05526 | C | | | | | | | | | | | | |

(continued)

| Comp. No. | CCR F-CE M | Kasu mi-1 | MD A-MB-231 | Mi no | MM. 1R | MM. 1S | Ram os | SU-DH L-4 | SU-DH L-6 | TM D8 | SW6 20 | Dau di | M V-4-11 |
|---|---|---|---|---|---|---|---|---|---|---|---|---|---|
| GT-05529 | | | | C | | B | | | | | | | |
| GT-05542 | A3 | | | A1 | | A3 | | | | | | | |
| GT-05596 | | | | A3 | | | | | | | | | |
| GT-05544 | | | | A2 | | B | | | | | | | |
| GT-05545 | | | | C | | | | | | | | | |
| GT-05597 | | | | B | | | | | | | | | |
| GT-05598 | C | | | A3 | | A4 | | | | | | C | |
| GT-05599 | | | | B | | C | | | | | | | |
| GT-05872 | | | | | | C | | | | | | | |
| GT-05620 | B | | | B | | A3 | | | | | | | |
| GT-05624 | | | | A3 | | B | | | | | | C | |
| GT-05625 | | | | A4 | | B | | | | | | B | |
| GT-05626 | C | | | C | | | | | | | | C | |
| GT-05627 | | | | B | | | | | | | | | |
| GT-05629 | | | | B | | | | | | | | | |
| GT-05632 | | | | A1 | | B | | | | | | A3 | |
| GT-05633 | | | | A1 | | | | | | | | B | |
| GT-05634 | | | | C | | | | | | | | | |
| GT-05692 | | | | B | | | | | | | | | |
| GT-05693 | | | | A3 | | B | | | | | | C | |
| GT-05695 | | | | B | | | | | | | | | |
| GT-05734 | | | | B | | | | | | | | | |
| GT-05860 | A2 | | | A2 | | A1 | | | | | | A3 | |
| GT-05602 | A1 | | | A1 | | A1 | | | | | | A2 | |
| GT-05603 | 2.5 | | | A1 | | A1 | | | | | | A2 | |
| GT-05608 | | | | c | | | | | | | | | |

(continued)

| Comp. No. | CCR F-CE M | Kasu mi-1 | MD A-MB-231 | Mi no | MM. 1R | MM. 1S | Ram os | SU-DH L-4 | SU-DH L-6 | TM D8 | SW6 20 | Dau di | M V-4-11 |
|---|---|---|---|---|---|---|---|---|---|---|---|---|---|
| GT-05642 | A4 | | | | | B | | | | | | | |
| GT-05519 | A1 | | | A1 | | A1 | | | | | | A2 | |
| GT-05646 | B | | | | | B | | | | | | | |
| GT-03891 | | | | B | | B | | | | | | | |
| GT-05649 | | | | B | | B | | | | | | | |
| GT-05650 | A3 | | | A4 | | A3 | | | | | | B | |
| GT-05702 | | | | C | | B | | | | | | | |
| GT-05703 | | | | A3 | | A2 | | | | | | B | |
| GT-05709 | A3 | | | A4 | | A3 | | | | | | | |
| GT-05711 | B | | | | | | | | | | | | |
| GT-05788 | | | | B | | A4 | | | | | | | |
| GT-05789 | A3 | | | A3 | | A3 | | | | | | B | |
| GT-05790 | A4 | | | A3 | | A3 | | | | | | | |
| GT-05791 | | | | A3 | | A3 | | | | | | c | |
| GT-05792 | A2 | | | A2 | | A2 | | | | | | A4 | |
| GT-05793 | B | | | B | | B | | | | | | | |
| GT-05847 | A1 | | | A1 | | A1 | | | | | | A3 | |
| GT-05848 | | | | A4 | | A4 | | | | | | | |
| GT-05849 | | | | B | | B | | | | | | | |
| GT-05656 | | | | | | B | | | | | | | |
| GT-05664 | A3 | | | A3 | | A3 | | | | | | B | |
| GT-05672 | | | | A3 | | A3 | | | | | | c | |
| GT-05677 | | | | | | A4 | | | | | | | |
| GT-05681 | B | | | B | | A4 | | | | | | | |
| GT-05683 | A2 | | | A3 | | A2 | | | | | | A4 | |
| GT-05684 | B | | | A3 | | A4 | | | | | | | |

(continued)

| Comp. No. | CCR F-CE M | Kasu mi-1 | MD A-MB-231 | Mi no | MM. 1R | MM. 1S | Ram os | SU-DH L-4 | SU-DH L-6 | TM D8 | SW6 20 | Dau di | M V-4-11 |
|---|---|---|---|---|---|---|---|---|---|---|---|---|---|
| GT-05685 | | | | B | | | | | | | | | |
| GT-05778 | A1 | | | A1 | | A1 | | | | | | A2 | |
| GT-05779 | A2 | | | A2 | | A2 | | | | | | A4 | |
| GT-05780 | | | | A2 | | c | | | | | | B | |
| GT-05784 | A1 | | | A1 | | A1 | | | | | | A3 | |
| GT-06101 | C | | | | | | | | | | | | |

Note: In Table C, the symbols A1, A2, A3, A4, B, C, D, E and F are defined as follows: 0 nM < A1 ≤ 5 nM; 5 nM < A2 ≤ 10 nM; 10 nM < A3 ≤ 50 nM; 50 nM < A4 ≤ 100 nM; 100 nM < B ≤ 500 nM; 500 nM < C ≤ 1000 nM; 1000 nM < D ≤ 5000 nM; 5000 nM < E ≤ 10000 nM; 10000 nM < F.

The results indicated that the compounds of the present invention (including the compounds listed in Table 1 and the Examples compounds) can inhibit the proliferation of tumor cells (as shown in Table C), with inhibitory effects superior to or comparable to those of the corresponding positive control drugs, and degradation effects superior to those of the corresponding positive control drugs. Furthermore, the results demonstrate that the compounds of the present invention (including the compounds listed in Table 1 and the Examples compounds) significantly inhibit the proliferation of MM.1S cells (human multiple myeloma cells), MM.1R cells (human multiple myeloma cells resistant to dexamethasone), Kasumi-1 cells (human acute lymphoblastic leukemia cells), Daudi cells (human Burkitt's lymphoma cells), TMD8 cells (human diffuse large B-cell lymphoma cells), Ramos cells (human B Lymphoma cells), MDA-MB-231 cells (human breast cancer cells), Mino cells (human mantle cell lymphoma cells), SU-DHL-4 cells (human B-lymphoma cells), SU-DHL-6 cells (human diffuse large B-cell lymphoma cells), SW620 cells (human colon cancer cells), MV-4-11 cells (human myelomonocytic leukemia cells), and CCRF-CEM cells (human T-cell acute lymphoblastic leukemia cells) (as shown in Table C), with inhibitory effects superior to those of the corresponding positive control drugs.

**II. Determination of CRBN-binding affinity of compounds:**

The CEREBLON BINDING kits (specification: 10,000 tests; product number: Catalog # 64BDCRBNPEH; CISBIO company) was used to determine the CRBN binding ability of the compounds to be tested through a HTRF method (homogeneous time-resolved fluorescence). The specific methods are as follows:

**1.** According to the instructions of the CEREBLON BINDING kits, the compounds to be tested of the present invention and lenalidomide were serially diluted using diluent #9 (1X) solution to obtain a final concentration of 2 μM for both the tested compounds and lenalidomide solution.
**2.** 2.5 μL of the above 2 μM of the tested compounds and lenalidomide solution, as well as the same volume of diluent #9 (1X) solution (solvent control group, Std0) were added to each well of a 96-well plate, respectively. Then, 2.5 μL of human Cereblon WT GST-tagged protein solution was added to each well. Finally, 5 μL of the thoroughly mixed Thalidomide-Re reagent and GST Eu antibody working solution were added to each of the aforementioned wells. The final concentration of the tested compounds and lenalidomide in each well is 0.5 μM.
**3.** The blank control wells were sequentially added with 2.5 μL of diluent #9 (1X) solution, 2.5 μL of PROTAC binding buffer, and 5 μL of thoroughly mixed Thalidomide-Re reagent and GST Eu antibody working solution.
**4.** After sealing and incubating the solutions in the aforementioned wells at room temperature for 3 hours, the absorbance values at emission wavelengths of 620 nm and 665 nm were detected by the HTRF method using a Spark microplate reader (V3.1 SP1).

The corresponding CRBN binding inhibition rate was calculated using the following method:

$$R_{compound} = OD\ 665\ nm\ compound / OD\ 620\ nm_{compound} - OD\ 665\ nm_{control} / OD\ 620\ nm\ control\ R_{Std0} = OD\ 665\ nm_{Std0} / OD\ 620\ nm_{Std0} - OD\ 665\ nm_{control} / OD\ 620\ nm_{control}\ \text{inhibition rate (\%)} = (1 - R\ compound / R_{Std0}) * 100$$

Note: OD 665 nm $_{compound}$ is the absorbance value of each well of the compounds to be tested of the present disclosure at an emission wavelength of 665 nm.
OD 620 nm $_{compound}$ is the absorbance value of each well of the compounds to be tested of the present disclosure at an emission wavelength of 620 nm.
OD 665 nm $_{control}$ is the absorbance value of the blank control well at an emission wavelength of 665 nm.
OD 620 nm $_{control}$ is the absorbance value of the blank control well at an emission wavelength of 620 nm.
OD 665 nm $_{Std0}$ is the absorbance value of the solvent control well at an emission wavelength of 665 nm.
OD 620 nm $_{Std0}$ is the absorbance value of the solvent control well at an emission wavelength of 620 nm.

The test results were shown in Table D below.

Table D. HTRF inhibitory activity ($IC_{50}$, μM) of the compounds of the present disclosure (including the compounds in Table 1 and the Examples compounds)

| Comp. No. | HTRF $IC_{50}$ (μM) | Comp. No. | HTRF $IC_{50}$ (μM) | Comp. No. | HTRF $IC_{50}$ (μM) |
|---|---|---|---|---|---|
| lenalidomide | 1.9 | GT-05462 | 1.90 | GT-05860 | 0.56 |
| pomalidomide | 1.76 | GT-02785 | 3.07 | GT-03468 | 2.96 |
| GT-03578 | 1.83 | GT-05465 | 0.83 | GT-05861 | 1.29 |

(continued)

| Comp. No. | HTRF $IC_{50}$ ($\mu M$) | Comp. No. | HTRF $IC_{50}$ ($\mu M$) | Comp. No. | HTRF $IC_{50}$ ($\mu M$) |
|---|---|---|---|---|---|
| GT-03492 | 0.52 | GT-05466 | 0.70 | GT-05863 | 1.02 |
| GT-03493 | 0.75 | GT-05467 | 0.52 | GT-05893 | 1.15 |
| GT-03995 | 0.63 | GT-05468 | 1.50 | GT-05874 | 0.44 |
| GT-03889 | 0.71 | GT-05470 | 0.98 | GT-05875 | 0.51 |
| GT-03890 | 0.51 | GT-05471 | 1.08 | GT-05876 | 0.12 |
| GT-03996 | 1.44 | GT-05472 | 1.12 | GT-05602 | 0.56 |
| GT-03887 | 0.19 | GT-05473 | 1.16 | GT-05603 | 0.94 |
| GT-03888 | 0.24 | GT-05474 | 1.17 | GT-05604 | 0.72 |
| GT-03997 | 0.77 | GT-05475 | 0.91 | GT-05605 | 1.05 |
| GT-03368 | 1.38 | GT-05476 | 1.20 | GT-05606 | 1.18 |
| GT-03454 | 0.41 | GT-05477 | 0.66 | GT-05607 | 1.15 |
| GT-03455 | 0.34 | GT-05478 | 1.12 | GT-05608 | 0.80 |
| GT-03355 | 0.77 | GT-05479 | 0.72 | GT-05609 | 0.54 |
| GT-03694 | 0.54 | GT-05480 | 1.74 | GT-05610 | 0.64 |
| GT-03695 | 1.17 | GT-05482 | 0.33 | GT-05611 | 0.63 |
| GT-03696 | 0.88 | GT-05483 | 0.51 | GT-05612 | 0.65 |
| GT-03891 | 0.23 | GT-05484 | 0.36 | GT-05613 | 0.59 |
| GT-05607 | 0.88 | GT-05485 | 0.74 | GT-05614 | 1.41 |
| GT-03361 | 1.59 | GT-05486 | 1.56 | GT-05616 | 0.90 |
| GT-03991 | 0.47 | GT-05487 | 0.65 | GT-05641 | 1.48 |
| GT-03877 | 1.40 | GT-05488 | 0.60 | GT-05642 | 0.49 |
| GT-03881 | 0.48 | GT-05489 | 0.61 | GT-05643 | 0.59 |
| GT-03880 | 0.23 | GT-05490 | 0.58 | GT-05644 | 0.59 |
| GT-03923 | 0.17 | GT-05491 | 0.97 | GT-05645 | 0.70 |
| GT-03922 | 0.16 | GT-05492 | 0.83 | GT-05519 | 0.41 |
| GT-03469 | 2.12 | GT-05493 | 0.76 | GT-05646 | 1.02 |
| GT-04167 | 0.66 | GT-05498 | 1.52 | GT-05734 | 0.11 |
| GT-04168 | 0.55 | GT-05499 | 0.67 | GT-05649 | 0.68 |
| GT-04224 | 0.43 | GT-05500 | 1.87 | GT-05650 | 0.94 |
| GT-04256 | 1.42 | GT-05504 | 0.73 | GT-05700 | 1.22 |
| GT-04194 | 1.16 | GT-05505 | 0.81 | GT-05701 | 1.80 |
| GT-04193 | 0.46 | GT-05506 | 0.54 | GT-05702 | 0.61 |
| GT-04170 | 1.01 | GT-05507 | 1.24 | GT-05703 | 0.53 |
| GT-04169 | 0.85 | GT-05508 | 0.72 | GT-05704 | 0.68 |
| GT-04204 | 0.52 | GT-05509 | 0.25 | GT-05705 | 0.88 |
| GT-04275 | 0.46 | GT-05510 | 1.22 | GT-05707 | 0.71 |
| GT-05460 | 1.63 | GT-05511 | 1.95 | GT-05708 | 0.55 |
| GT-05359 | 0.97 | GT-05512 | 0.57 | GT-05709 | 0.66 |
| GT-05361 | 0.37 | GT-05513 | 0.97 | GT-05710 | 1.10 |

(continued)

| Comp. No. | HTRF $IC_{50}$ (μM) | Comp. No. | HTRF $IC_{50}$ (μM) | Comp. No. | HTRF $IC_{50}$ (μM) |
|---|---|---|---|---|---|
| GT-05362 | 1.26 | GT-05514 | 0.74 | GT-05711 | 0.49 |
| GT-05366 | 1.44 | GT-05515 | 1.01 | GT-04222 | 0.56 |
| GT-05367 | 0.40 | GT-05516 | 0.60 | GT-05788 | 0.50 |
| GT-05368 | 1.80 | GT-05517 | 0.60 | GT-05789 | 0.52 |
| GT-05373 | 1.49 | GT-05640 | 0.39 | GT-05790 | 0.57 |
| GT-05374 | 0.52 | GT-05520 | 0.35 | GT-05791 | 0.73 |
| GT-05378 | 1.77 | GT-05521 | 0.62 | GT-05792 | 0.66 |
| GT-05379 | 0.11 | GT-05522 | 0.48 | GT-05795 | 0.94 |
| GT-05382 | 0.66 | GT-05523 | 0.86 | GT-05796 | 0.20 |
| GT-05383 | 0.54 | GT-05524 | 0.49 | GT-05845 | 0.76 |
| GT-05386 | 1.46 | GT-05525 | 0.71 | GT-05846 | 0.94 |
| GT-05387 | 0.81 | GT-05526 | 0.69 | GT-05847 | 1.00 |
| GT-05388 | 0.41 | GT-05527 | 0.99 | GT-05848 | 0.71 |
| GT-05390 | 0.50 | GT-05528 | 1.93 | GT-05849 | 0.99 |
| GT-05391 | 0.57 | GT-05529 | 0.35 | GT-05670 | 0.62 |
| GT-05392 | 0.44 | GT-05530 | 1.20 | GT-05671 | 0.30 |
| GT-05393 | 0.34 | GT-05531 | 0.80 | GT-05653 | 1.06 |
| GT-05394 | 0.49 | GT-05534 | 1.17 | GT-05656 | 0.70 |
| GT-05395 | 1.61 | GT-05535 | 1.79 | GT-05657 | 1.17 |
| GT-05396 | 0.59 | GT-05536 | 1.05 | GT-05658 | 0.74 |
| GT-05398 | 1.05 | GT-05538 | 1.02 | GT-05659 | 0.66 |
| GT-05399 | 0.43 | GT-05539 | 1.28 | GT-05660 | 1.04 |
| GT-05400 | 0.84 | GT-05540 | 0.96 | GT-05661 | 0.96 |
| GT-05401 | 0.59 | GT-05541 | 0.60 | GT-05662 | 0.71 |
| GT-05402 | 0.43 | GT-05542 | 0.44 | GT-05663 | 1.09 |
| GT-05403 | 0.55 | GT-05543 | 0.45 | GT-05664 | 0.56 |
| GT-05404 | 0.49 | GT-05596 | 1.34 | GT-05665 | 0.60 |
| GT-05405 | 0.50 | GT-05544 | 0.84 | GT-05666 | 0.69 |
| GT-05417 | 1.48 | GT-05597 | 1.40 | GT-05667 | 0.75 |
| GT-05418 | 0.54 | GT-05598 | 0.68 | GT-05668 | 0.65 |
| GT-05419 | 0.72 | GT-05599 | 1.67 | GT-05669 | 0.63 |
| GT-05420 | 0.73 | GT-05618 | 0.58 | GT-05672 | 0.58 |
| GT-05426 | 0.62 | GT-05619 | 0.59 | GT-05673 | 1.14 |
| GT-05427 | 0.95 | GT-05620 | 0.59 | GT-05677 | 1.23 |
| GT-05428 | 1.08 | GT-05621 | 1.10 | GT-05680 | 0.98 |
| GT-05429 | 1.49 | GT-05622 | 0.91 | GT-05681 | 0.46 |
| GT-05440 | 0.94 | GT-05733 | 0.21 | GT-05682 | 0.57 |
| GT-05441 | 1.57 | GT-05624 | 0.47 | GT-05683 | 0.75 |
| GT-05442 | 0.67 | GT-05625 | 1.67 | GT-05684 | 0.54 |

(continued)

| Comp. No. | HTRF $IC_{50}$ (μM) | Comp. No. | HTRF $IC_{50}$ (μM) | Comp. No. | HTRF $IC_{50}$ (μM) |
|---|---|---|---|---|---|
| GT-05445 | 1.91 | GT-05627 | 0.26 | GT-05685 | 1.16 |
| GT-05446 | 1.87 | GT-05629 | 1.28 | GT-05776 | 0.42 |
| GT-05447 | 1.15 | GT-05631 | 1.75 | GT-05778 | 0.56 |
| GT-05448 | 1.39 | GT-05632 | 0.72 | GT-05779 | 0.43 |
| GT-05449 | 1.69 | GT-05633 | 0.76 | GT-05780 | 0.23 |
| GT-05450 | 0.89 | GT-05635 | 1.00 | GT-05781 | 0.71 |
| GT-05451 | 1.16 | GT-05637 | 1.07 | GT-05782 | 1.76 |
| GT-05452 | 0.44 | GT-05638 | 1.21 | GT-05783 | 0.94 |
| GT-05453 | 1.24 | GT-05639 | 0.78 | GT-05784 | 0.62 |
| GT-05455 | 1.07 | GT-05692 | 0.47 | GT-05785 | 1.25 |
| GT-05457 | 1.26 | GT-05693 | 0.31 | GT-05786 | 1.07 |
| GT-05458 | 1.41 | GT-05695 | 0.25 | GT-04947 | 0.39 |
| GT-05459 | 1.12 | GT-05731 | 1.65 | GT-04397 | 0.2 |
| GT-05461 | 1.58 | | | | |

The results show that the compounds of the present disclosure (including the compounds listed in Table 1 and the Examples compounds) exhibit strong binding affinity to CRBN.

**III. Western-blot assay**

The effect of the compounds of the present disclosure on the expression of target proteins in cells was detected using conventional Western blot analysis.

After culturing $5\times10^5$ hPBMC cells for 24 hours, the cells were treated with the compounds of the present disclosure (including the compounds listed in Table 1 and the Examples compounds) at certain concentrations. The concentrations of the compounds of the present disclosure were (1) 500 nM or (2) a series of diluted concentrations, namely 0.03 nM, 0.1 nM, 0.3 nM, 1 nM, 3 nM, 10 nM, 30 nM, 100 nM, 300 nM, and 1000 nM. DMSO was used as the negative control, and commercial immunomodulatory drugs (lenalidomide and pomalidomide) were used as the positive controls, all of which were administered to the cells following the same protocol as that of the compounds of the present disclosure. After treating the cells with the compounds for 24 hours, the cells were collected and lysed using RIPA protein lysate containing phosphatase inhibitors. The lysate was then placed on ice for 30-60 minutes and centrifuged. The supernatant was aspirated as the extracted total cell protein. The protein concentration of the supernatant was determined using the conventional BCA (Bicinchoninic Acid) assay. The supernatant was then mixed with 4X SDS sample loading buffer, denatured at 95°C for 5 minutes, and then subjected to SDS-PAGE electrophoresis on a polyacrylamide gel, followed by transfer to a nitrocellulose membrane (NC membrane). The membrane was blocked with blocking buffer at room temperature for 1-2 hours, and then antibody incubation and detection were carried out according to the antibody instructions provided by Cell Signaling Technology.

Half-Maximal Degradation Concentration value (the drug concentration required for degrading proteins by 50%, abbreviated as $DC_{50}$) reads method: comparing the gray values of the Western blotting bands for the drug treatment with the gray values of the Western blotting band for the DMSO control, and reading the drug concentration range corresponding to the gray value of the Western blotting bands for the drug treatment which is equal to half of the gray value of the Western blotting band for the DMSO control.

$DC_{50}$ value could also be calculated as follows: using software ImageJ to quantify the gray values of the Western blotting bands for the drug treatment, fitting the relationship curve between drug concentrations and gray values, and from the fitted curve, calculating the drug concentration corresponding to half of the gray value of the Western blotting band for the DMSO control.

The results of the effects of the compounds of the present disclosure (including the compounds listed in Table 1 and the Examples compounds) at a concentration of 500 nM on the expression of substrate proteins in hPBMC cells were listed in Table E below.

Table E: Results of substrate proteins degradation by the compounds of the present invention at a concentration of 500 nM

| Comp. No. (500 nM) | substrate proteins | | | Comp. No. (500 nM) | substrate proteins | | |
|---|---|---|---|---|---|---|---|
| | IKZF1 | IKZF3 | GSPT1 | | IKZF1 | IKZF3 | GSPT1 |
| GT-02785 | A | A | A | GT-05545 | B | A | B |
| GT-03468 | A | A | A | GT-05598 | A | A | B |
| GT-03578 | A | A | A | GT-05599 | A | A | B |
| GT-03493 | A | A | A | GT-05600 | B | B | A |
| GT-03995 | A | A | A | GT-05872 | A | B | B |
| GT-03889 | B | B | A | GT-05620 | A | A | A |
| GT-03890 | B | A | A | GT-05621 | B | B | A |
| GT-03997 | A | A | B | GT-05622 | B | B | A |
| GT-03454 | A | A | A | GT-05596 | A | A | B |
| GT-03355 | A | A | A | GT-05624 | A | A | B |
| GT-03694 | A | A | A | GT-05625 | B | B | B |
| GT-03695 | A | A | A | GT-05626 | B | B | B |
| GT-03696 | B | B | A | GT-05630 | B | A | B |
| GT-03891 | B | A | A | GT-05632 | A | A | B |
| GT-05607 | B | B | A | GT-05633 | A | A | B |
| GT-03991 | A | A | A | GT-05634 | A | A | B |
| GT-03881 | B | B | A | GT-05637 | B | B | A |
| GT-03922 | A | A | A | GT-05693 | B | A | B |
| GT-05533 | A | A | A | GT-05695 | A | A | B |
| GT-04167 | B | B | B | GT-05733 | B | A | A |
| GT-04168 | B | B | A | GT-05734 | B | A | B |
| GT-04256 | A | A | A | GT-05860 | A | A | A |
| GT-04194 | B | A | A | GT-05602 | A | A | A |
| GT-04193 | A | A | A | GT-05603 | A | A | A |
| GT-04204 | B | B | A | GT-05604 | B | B | A |
| GT-04275 | A | A | A | GT-05608 | B | A | A |
| GT-05364 | A | A | A | GT-05609 | B | B | A |
| GT-05367 | B | B | A | GT-05612 | A | A | B |
| GT-05368 | B | A | A | GT-05613 | B | A | B |
| GT-05370 | B | A | A | GT-05642 | B | B | A |
| GT-05382 | A | A | A | GT-05644 | B | B | A |
| GT-05383 | B | B | A | GT-05645 | B | B | B |
| GT-05390 | A | A | A | GT-05519 | A | A | A |
| GT-05391 | A | A | A | GT-05646 | B | B | A |
| GT-05395 | B | A | A | GT-05544 | A | A | B |
| GT-05396 | B | A | A | GT-05649 | B | A | B |
| GT-05399 | B | B | A | GT-05650 | A | A | A |
| GT-05400 | B | B | A | GT-05702 | A | A | A |

(continued)

| Comp. No. (500 nM) | substrate proteins | | | Comp. No. (500 nM) | substrate proteins | | |
|---|---|---|---|---|---|---|---|
| | IKZF1 | IKZF3 | GSPT1 | | IKZF1 | IKZF3 | GSPT1 |
| GT-05401 | B | A | B | GT-05703 | A | A | B |
| GT-05402 | B | A | B | GT-05709 | A | A | A |
| GT-05404 | B | A | B | GT-05710 | B | A | A |
| GT-05366 | B | A | B | GT-05789 | B | B | A |
| GT-05414 | B | A | B | GT-05791 | A | A | B |
| GT-05420 | B | B | A | GT-05792 | A | A | A |
| GT-05426 | B | A | A | GT-05793 | B | A | A |
| GT-05430 | B | B | A | GT-05796 | B | B | A |
| GT-05449 | B | B | A | GT-05845 | B | A | B |
| GT-05450 | B | B | A | GT-05846 | B | B | A |
| GT-05455 | B | B | A | GT-05847 | A | A | A |
| GT-04719 | B | B | A | GT-05848 | A | A | B |
| GT-05459 | B | B | A | GT-05849 | A | A | A |
| GT-05464 | B | A | B | GT-05670 | A | A | A |
| GT-05466 | A | A | A | GT-05656 | B | A | B |
| GT-05467 | B | B | A | GT-05657 | B | B | B |
| GT-05472 | B | B | A | GT-05658 | B | A | B |
| GT-05473 | B | B | A | GT-05662 | B | A | B |
| GT-05482 | B | B | A | GT-05664 | A | A | A |
| GT-05490 | A | B | B | GT-05665 | B | B | A |
| GT-05494 | A | A | A | GT-05672 | A | A | A |
| GT-05497 | B | A | A | GT-05675 | B | B | B |
| GT-05504 | A | A | B | GT-05677 | A | A | B |
| GT-05505 | A | B | B | GT-05680 | B | A | B |
| GT-05512 | B | B | A | GT-05681 | B | A | A |
| GT-05513 | B | B | A | GT-05683 | A | A | A |
| GT-05515 | B | B | A | GT-05684 | A | A | A |
| GT-05640 | A | A | B | GT-05778 | A | A | A |
| GT-05524 | A | A | B | GT-05779 | A | A | A |
| GT-05526 | B | B | A | GT-05780 | A | A | A |
| GT-05529 | A | A | B | GT-06103 | B | B | A |
| In Table E, A represents a residual substrate protein level of <50%, and B represents a residual substrate protein level of >50%. | | | | | | | |

The results show that the compounds of the present disclosure (including the compounds listed in Table 1 and the Examples compounds) can effectively degrade substrate proteins (e.g., IKZF1/3 proteins, GSPT1 protein, etc.) at a concentration of 500 nM.

The inventors further tested the effects of serially diluted concentrations (i.e., 0.03 nM, 0.1 nM, 0.3 nM, 1 nM, 3 nM, 10 nM, 30 nM, 100 nM, 300 nM, and 1000 nM) of the compounds of the present disclosure on the expression of target proteins in hPBMC cells. The Western Blot results were shown in Figure 1. As shown in Figure 1, lenalidomide cannot

degrade IKZF1/3 proteins and GSPT1 protein at concentrations of 50 nM or 500 nM, and pomalidomide cannot degrade IKZF1/3 proteins and GSPT1 protein at a concentration of 50 nM. However, the compounds of the present disclosure can significantly degrade IKZF1/3 proteins and GSPT1 protein (Figure 1). In particular, the compound GT-04194 disclosed herein exhibited significant degradation effects at concentrations below 30 nM, and the compounds GT-05640, GT-05596, GT-05598, and GT-05620 disclosed herein exhibited significant degradation effects at concentrations below 0.1 nM, 0.3 nM, or 3 nM. The Western Blot experimental results indicated that the compounds of the present disclosure have significantly improved activity compared to lenalidomide and pomalidomide in terms of inhibiting tumor cells and inducing substrate proteins degradation.

### IV. Pharmacokinetic Study of the compounds of the present disclosure

The pharmacokinetic properties of the compounds of the present disclosure were evaluated using conventional pharmacokinetic experimental methods.

The test compounds of the present disclosure (including the compounds listed in Table 1 and the Examples compounds) were orally administered to experimental animals to assess their pharmacokinetic properties. The experimental animals used can be those commonly used in pharmacokinetic experiments, e.g., adult and healthy rodents (e.g., mice, rats (such as Sprague-Dawley (SD) rats), guinea pigs) or non-rodents (rabbits, dogs, and monkeys). In the present disclosure, mice were used as the experimental animals.

The experimental animals were divided into two groups: a control group (for collecting blank plasma) and an orally administered group (dosed at 10 mg/kg or 30 mg/kg). Blood samples were collected at predetermined sampling time points after administration, such as 0.25 h, 0.5 h, 1 h, 2 h, 4 h, 8 h, and 24 h post-dosing. Blank plasma was collected from the control group.

Plasma samples were preprocessed before analysis: 10 μL of 50% acetonitrile and 200 μL of internal standard solution (containing 50 ng/mL dexamethasone in acetonitrile) were added to 10 μL of plasma samples. For the blank control group, the same volume of acetonitrile was added instead of the internal standard. The processed samples were vortexed, centrifuged, and the supernatant was taken for LC-MS/MS injection analysis.

After preparing standard curves and quality control (QC) samples, the concentrations of the compounds in the plasma samples were determined by LC-MS/MS analysis. Based on the plasma concentration data obtained from LC-MS/MS analysis, the pharmacokinetic parameters of the test compounds were calculated using the non-compartmental model in the pharmacokinetic calculation software WinNonlin v8.1. The results were listed in Table F below.

Table F: Pharmacokinetic Parameters of the compounds of the present disclosure in Mouse Plasma

| Test Compounds | Route of Administration | $AUC_{0-t}$(h*ng/mL) |
|---|---|---|
| GT-02785 | p.o.(10mg/kg) | A |
| GT-03649 | p.o.(30mg/kg) | A |
| GT-03468 | p.o.(30mg/kg) | A |
| GT-03578 | p.o.(30mg/kg) | A |
| GT-03355 | p.o.(30mg/kg) | A |
| GT-03694 | p.o.(30mg/kg) | A |
| GT-03695 | p.o.(30mg/kg) | A |
| GT-03922 | p.o.(10mg/kg) | B |
| GT-04256 | p.o.(10mg/kg) | B |
| Note: in Table F, p.o. indicates oral administration, A represents >500 h*ng/mL, and B represents ≤500 h*ng/mL. | | |

The results show that the compounds of the present disclosure, administered orally, exhibit good pharmacokinetic (DMPK) properties and can be used as therapeutic agents for cancer patients.

### V. Effects of the compounds of the present disclosure on TNF-α, IL-10, IL-6, IL-1β, IFN-γ, GM-CSF, IL-2, and IL-12p40 release from PBMCs induced by Lipopolysaccharide (LPS) or Phytohemagglutinin (PHA)

The following materials were used in this study:

V.1 Reagents and Consumable materials

| Name | Supplier or Source |
|---|---|
| Human PBMC cell | Shanghai Sai Li |
| Lipopolysaccharide (LPS) | Sigma |
| Phytohemagglutinin (PHA) | MCE |
| RPMI 1640 Medium | Gibco |
| Human TNF-alpha DuoSet ELISA | R&D Systems |
| Human IL-6 DuoSet ELISA | R&D Systems |
| Human IL-10 DuoSet ELISA | R&D Systems |
| Human IFN-gamma DuoSet ELISA | R&D Systems |
| Human IL-2 DuoSet ELISA | R&D Systems |
| Human IL-1 beta ELISA Kit | Abclonal |
| Human GM-CSF ELISA Kit | Abclonal |
| Human IL-12/IL-23 p40 ELISA Kit | Abclonal |

V.2 Methods

V.2.1 Cell recovering and plating

A complete medium was prepared and preheated to 37°C. Cells were retrieved from liquid nitrogen tank, and the frozen vial was held with forceps just below the cap-body junction, immersed in 37°C warm water, and shaken occasionally to speed up thawing. The cell suspension was aspirated and added to a centrifuge tube containing 10 mL of the complete medium, and mixed well. The mixture was centrifuged at 400 g for 10 min at room temperature. The supernatant was discarded. The complete medium was added to the cells, followed by counting the cells and adjusting the cell density. The cells were seeded at 75 μL/well into a 96-well plate to achieve $1\times10^5$ cells per well. The plate was incubated in a high-humidity, 37°C, 5% $CO_2$ incubator for 2 h.

V.2.2 Preparation of Compounds and Addition to Cell Plates

1) 10 mM stock solutions of the test compounds in DMSO were prepared.
2) The test compounds were diluted stepwise 5-fold in DMSO, starting from 1 mM, to obtain nine concentration gradients.
3) The test compounds were diluted in medium to five times the final concentration intended for use.
4) The diluted compounds were added to the corresponding cell wells at 25 μL/well according to the following layout. The final compound concentrations start at 1 μM, with 5-fold dilutions, for a total of nine concentration points.
5) After 1 h, LPS or PHA was added to the cell wells at 25 μL/well to achieve final LPS concentrations of 1 μg/mL(for TNF-α, IL-10, IL-6, GM-CSF, and IL-12p40), 5 μg/mL(for IL-1β), 50 μg/mL (for IFN-γ), or a final PHA concentration of 1 μg/mL(for IL-2).
6) The cell plate was incubated in a high-humidity incubator at 37°C with 5% $CO_2$ for 24 h. Cell plate layout:

<table>
<tr><td></td><td>1</td><td>2</td><td>3</td><td>4</td><td>5</td><td>6</td><td>7</td><td>8</td><td>9</td><td>10</td><td>11</td><td>12</td></tr>
<tr><td>A</td><td>$H_2O$</td><td>$H_2O$</td><td>$H_2O$</td><td>$H_2O$</td><td>$H_2O$</td><td>$H_2O$</td><td>$H_2O$</td><td>$H_2O$</td><td>$H_2O$</td><td>$H_2O$</td><td>$H_2O$</td><td>$H_2O$</td></tr>
<tr><td>B</td><td>$H_2O$</td><td colspan="9" rowspan="2">Cmpd 1# (1 μM Starting, 5-fold dilution, 9 points)</td><td rowspan="4">LPS/PHA</td><td>$H_2O$</td></tr>
<tr><td>C</td><td>$H_2O$</td><td>$H_2O$</td></tr>
<tr><td>D</td><td>$H_2O$</td><td colspan="9" rowspan="2">Cmpd 2# (1 μM Starting, 5-fold dilution, 9 points)</td><td>$H_2O$</td></tr>
<tr><td>E</td><td>$H_2O$</td><td>$H_2O$</td></tr>
<tr><td>F</td><td>$H_2O$</td><td colspan="9" rowspan="2">Cmpd 3# (1 μM Starting, 5-fold dilution, 9 points)</td><td rowspan="2">Blank</td><td>$H_2O$</td></tr>
<tr><td>G</td><td>$H_2O$</td><td>$H_2O$</td></tr>
<tr><td>H</td><td>$H_2O$</td><td>$H_2O$</td><td>$H_2O$</td><td>$H_2O$</td><td>$H_2O$</td><td>$H_2O$</td><td>$H_2O$</td><td>$H_2O$</td><td>$H_2O$</td><td>$H_2O$</td><td>$H_2O$</td><td>$H_2O$</td></tr>
</table>

V.2.3 Detection

(1) The cell plate was centrifuged and the culture supernatant was aspirated. TNF-α, IL-10, IL-6, IL-1β, IFN-γ, GM-CSF, IL-2, and IL-12p40 cytokines in the culture supernatant were detected according to the ELISA kit instructions.

The concentrations of TNF-α, IL-10, IL-6, IL-1β, IFN-y, GM-CSF, IL-2, and IL-12p40 in the samples were calculated based on the standard curve.

$$\text{Inhibition rate\%} = [(\text{Ac-As})/(\text{Ac-Ab})] \times 100\%$$

As: OA of the samples (cells + LPS/PHA + test compound)
Ac: OA of positive cell control (cells + LPS/PHA + DMSO)
Ab: OA of blank control (cells + culture medium + DMSO)

(2) $IC_{50}$ value was calculated as follows: using GraphPad Prism 6 software and applying the calculation formula XY-analysis/Nonlinear regression (curve fit)/Dose response-Inhibition/log (inhibitor) vs. response-Variable slope (four parameters) to perform $IC_{50}$ curve fitting and determine the $IC_{50}$ values.

The experimental results indicated that the compounds of the present disclosure can modulate the production levels of cytokines associated with autoimmune diseases and may be used for the treatment of autoimmune diseases.

**VI. Efficacy experiment of the compounds of the present disclosure on Psoriasis-like skin lesions induced by Imiquimod in Mice**

The compounds of the present disclosure (test compounds) were administered orally via gavage once daily for 7 consecutive days to mice with psoriasis-like skin lesions induced by imiquimod, to investigate their efficacy on such lesions.

Ninety C57BL/6J female mice (purchased from Shanghai SLAC Laboratory Animal Co., Ltd.) were divided into 9 groups based on their body weights, with 10 mice in each group:

| Group | Drug | Number of Animals | Dose (mg/kg) | Volume (mL/kg) | Concentratio n (mg/mL) | Route and Duration |
|---|---|---|---|---|---|---|
| 1 | Control: Vehicle | 10 | NA | 10 | NA | ig. qd. 7 days |
| 2 | Model: Vehicle | 10 | NA | 10 | NA | ig. qd. 7 days |
| 3 | Positive Control | 10 | 3 | 10 | 0.3 | sc. qd. 7 days |

(continued)

| Group | Drug | Number of Animals | Dose (mg/kg) | Volume (mL/kg) | Concentratio n (mg/mL) | Route and Duration |
|---|---|---|---|---|---|---|
| 4 | Low-dose test compounds group | 10 | 3 | 10 | 0.3 | ig. qd. 7 days |
| 5 | Medium-dose test compounds group | 10 | 10 | 10 | 1 | ig. qd. 7 days |
| 6 | High-dose test compounds group | 10 | 30 | 10 | 3 | ig. qd. 7 days |
| Note: ig. = oral gavage; sc. = subcutaneous injection | | | | | | |

After grouping the experimental animals, the backs of all mice were shaved. Except for the Control group, the remaining groups were topically treated with imiquimod cream once daily for 7 consecutive days. Simultaneously, each group was administered with a solvent control, a positive control drug, or the test compounds, according to the above table, once daily for 7 consecutive days.

All clinical symptoms of each animal were observed at the beginning and throughout the experiment. The animals' weights were measured and recorded every 2 days.

Scoring

Gross Observation Scoring of Skin Lesions (PASI Method): during the last 3 days of modeling and drug administration, the changes in skin lesions of mice in each group were observed once daily. PASI Method: the erythema, scaling, and thickening/infiltration of the lesions were scored separately, and the scores were summed to obtain the total score. PASI Scoring Criteria: 0 = none; 1 = mild; 2 = moderate; 3 = severe; 4 = very severe.

The skin lesions were photographed once daily during the last 3 days of modeling and drug administration.

After the experiment, the mice were euthanized by $CO_2$ inhalation. Three different areas of skin from the lesion site on the back were collected using a 6 mm punch, weighed, and recorded, and the average weight was calculated.

The skin tissue from the lesion site was homogenized and the levels of IL-23p19, IL-12p40, IL-17, and TNF-$\alpha$ were determined.

The local lesion tissue was fixed, stained with HE, and subjected to pathological scoring (based on epidermal thickness, degree of epidermal keratinization, thickness of the basal layer, and degree of dermal inflammatory cell infiltration). Scoring Criteria: 0 = none; 1 = mild; 2 = moderate; 3 = severe; 4 = very severe.

Experimental data were presented as Mean $\pm$ SD. Statistical analysis between two groups was performed using SPSS, with $p < 0.05$ considered statistically significant.

The experimental results indicated that the compounds of the present disclosure improve psoriasis-like skin lesions on the back of mice induced by imiquimod and may be used for the treatment of psoriasis.

**VII. Efficacy Experiment of the compounds of the present disclosure on the CIA Model in Rats Induced by Type II Collagen**

The compounds of the present disclosure (test compounds) were administered orally via gavage once daily for 21 consecutive days to Wistar rats with collagen-induced arthritis (CIA) that was induced using bovine type II collagen (Chondrex, Inc.), in order to investigate their efficacy in the rat arthritis model.

Preparation of the Modeling Agent:

10 mg of CII (Immunization Grade Bovine Type II Collagen, purchased from Chondrex, Inc.) was dissolved in 5 mL of 0.05M acetic acid solution, allowing it to be fully dissolved, thereby preparing a solution with a concentration of 2 mg/mL, which was then stored at 4°C in the dark overnight. On the day of the experiment, the 2 mg/mL CII solution was mixed with an equal volume of a 4 mg/mL solution of Complete Freund's Adjuvant (CFA, also purchased from Chondrex, Inc.) on ice, and was thoroughly emulsified into an emulsion.

On day 0, 10 Wistar rats (purchased from Beijing Vital River Laboratory Animal Technology Co., Ltd.) were randomly selected as the blank control group and were not immunized. The remaining 100 Wistar rats were intradermally injected at the base of the tail with an emulsion prepared by mixing equal volumes of CII (2 mg/mL) and CFA (4 mg/mL) for the first immunization. A second booster immunization was administered to them on day 7. Between days 10 and 14, i.e., 3

to 7 days after the second booster immunization, 80 rats with an arthritis index (AI) score ranging from 1 to 2 were selected from the modeled animals and were divided into 8 groups based on their body weight, foot volume, and AI score, with 10 rats per group:

| Group | Drug | Number of Animals | Dose (mg/kg) | Volume (mL/kg) | Concentratio n (mg/mL) | Route and Duration |
|---|---|---|---|---|---|---|
| 1 | Control: Vehicle | 10 | NA | 10 | NA | ig. qd. 21 days |
| 2 | Model: Vehicle | 10 | NA | 10 | NA | ig. qd. 21 days |
| 3 | Positive Control | 10 | 0.1 | 10 | 0.01 | sc. qd. 21 days |
| 4 | Low-dose test compounds group | 10 | 3 | 10 | 0.3 | ig. qd. 21 days |
| 5 | Medium-dose test compounds group | 10 | 10 | 10 | 1 | ig. qd. 21 days |
| 6 | High-dose test compounds group | 10 | 30 | 10 | 3 | ig. qd. 21 days |
| Note: ig. = oral gavage; sc. = subcutaneous injection | | | | | | |

Between 3 and 7 days after the second booster immunization, when the AI score of the affected feet reached 1 to 2, a solvent control, a positive control drug, or the test compounds were administered orally via gavage (or subcutaneously) to the animals in each group, according to the above table, once daily for 21 consecutive days.

Starting 3 days after the second booster immunization, the volume of both hind paws (or toes) was measured twice a week and recorded.

Foot Volume Measurement: Before the measurement, a line was marked on the rat's ankle joint with a marker pen to indicate the position. After clean water was added to the instrument, the instrument's reading was reset to zero in preparation for the measurement. The hind limb of the rat was placed in the water so that the marked line at the ankle joint was at the surface of the liquid. The reading obtained by pressing the foot pedal at this time was the foot volume of the rat. After the measurement was completed, the foot pedal was pressed again to reset the instrument to zero, preparing it for the measurement of the next rat.

Scoring

Arthritis Index (AI): The foot volume was measured twice a week, and the swelling of the four toes was scored simultaneously. Each foot was scored on a scale from 0 to 4, with a maximum score of 16 for each rat.

Arthritis Index (AI) Scoring Criteria:

| Score | Degree |
|---|---|
| 0 | No swelling, normal appearance |
| 1 | Mild swelling or redness of the ankle, wrist, or finger joints |
| 2 | Moderate swelling or redness of the ankle, wrist, or finger joints |
| 3 | Severe swelling or redness of the ankle, wrist, or finger joints |
| 4 | Very severe swelling or redness of the ankle, wrist, or finger joints |

The day of starting drug administration is recorded as D0. Five days after starting drug administration, i.e., 2 hours after D5 administration, blood was collected from the jugular vein of the experimental animals, allowed to stand for more than 30 minutes, centrifuged to separate the serum, which was stored at -80°C. The levels of TNF-$\alpha$, IL-1$\beta$, and IL-6 in

serum were measured by ELISA.

After the experiment, the animals were euthanized by $CO_2$ inhalation. The spleen and thymus of the animals were collected and weighed, and the organ coefficients were calculated.

Pathological Examination: One side of the foot joint tissue was fixed in 10% neutral formalin solution, followed by decalcification and paraffin embedding to prepare pathological sections. The sections were then stained with hematoxylin and eosin (HE) for pathological observation.

Observation Indices:

1. Infiltration of synovial inflammatory cells (lymphocytes, plasma cells);
2. Synovial tissue hyperplasia;
3. Synovial pannus;
4. Fibrinoid necrosis on the synovial surface.

Scoring and Counting Method: 0, 1, 2, 3, and 4 levels, where "0" indicates no observation; "1" is mild; "2" is moderate; "3" is severe; and "4" is extremely severe.

Experimental data were presented as Mean ± SEM. Data between two groups were analyzed using Excel-T test, with $p<0.05$ considered statistically significant. Scoring data were analyzed using the non-parametric Mann-Whitney U test in SPSS, with $p<0.05$ considered statistically significant.

The experimental results indicated that the compounds of the present disclosure can regulate the serum levels of inflammatory cytokines in rats with collagen-induced arthritis (CIA) model that was induced using bovine type II collagen, and significantly improve the swelling of rat limbs, and thus might be used for the treatment of arthritis.

The basic principles, main features and advantages of the present disclosure are shown and described above. Those skilled in the art should understand that the present disclosure is not limited by the foregoing embodiments and they can make various changes, substitutions and alterations herein without departing from the spirit and scope of the present disclosure. These changes, substitutions and alterations fall within the scope of the present disclosure. The claimed scope of the present disclosure is defined by the appended claims and their equivalents.

## Claims

**1.** A compound of Formula (I)

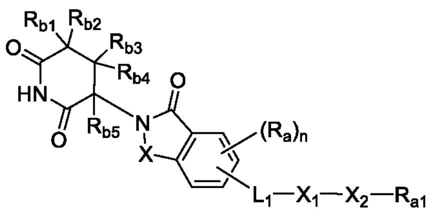

Formula (I)

or salts, enantiomers, diastereoisomer, isotopically enriched analogs, solvates, prodrugs, or polymorphs thereof,

$R_{b1}$, $R_{b2}$, $R_{b3}$, $R_{b4}$ and $R_{b5}$ each independently represent H, D, or $C_{1-3}$ alkyl;
X represents C(O) or $CH_2$;
$(R_a)_n$ indicates that benzene ring in Formula (I) is optionally substituted with n $R_a$, where $R_a$ represents deuterium, halogen, hydroxy, mercapto, nitro, amino, cyano, optionally deuterated $C_{1-6}$ alkyl, optionally deuterated $C_{1-6}$ alkoxy, or halogenated $C_{1-6}$ alkyl, and n represents an integer of 0, 1, 2, or 3; and
$L_1$ represents C(O), alkenylene, optionally substituted $C_{1-5}$ alkylene, -CH=, optionally substituted $C_{6-10}$ arylene-$C_{1-5}$ alkylene-*, or $N(R_{c1})$, where $R_{c1}$ represents H or $C_{1-3}$ alkyl, and symbol * indicates the point of attachment to $X_1$; or $L_1$ represents $-C(R_{L1}R_{L2})-$, where $R_{L1}$, $R_{L2}$ together with the carbon atom to which they are attached form optionally substituted $C_{3-8}$ cycloalkylene; or $L_1$ represents a bond;
$X_1$ represents optionally substituted cycloalkylene, optionally substituted heterocyclylene or optionally substituted heteroarylene;
$X_2$ represents C(O), optionally substituted $C_{1-5}$ alkylene, optionally substituted $C_{3-8}$ cycloalkylene, optionally

substituted $C_{1-2}$ alkylene-N($R_{c2}$)-, -N($R_{c2}$)-optionally substituted $C_{1-2}$ alkylene, or N($R_{c3}$), wherein $R_{c2}$ and $R_{c3}$ each independently represent H or $C_{1-3}$ alkyl; or $X_2$ represents a bond;

$R_{a1}$ represents the following structure:

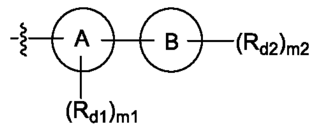

Formula (I-A)

wherein ring A represents heterocyclylene, cycloalkylene, arylene, or heteroarylene, and $(R_{d1})_{m1}$ indicates that ring A is optionally substituted with m1 $R_{d1}$, where m1 represents an integer of 0 to 10, and each $R_{d1}$ independently represents deuterium, hydroxy, amino, mercapto, halogen, cyano, oxo, optionally deuterated $C_{1-6}$ alkyl, optionally deuterated $C_{3-6}$ cycloalkyl, optionally deuterated $C_{1-6}$ alkoxy, halogenated $C_{1-6}$ alkyl, $C_{1-4}$ alkyl-NH-, $C_{1-4}$ alkyl-NHC(O)-, $C_{1-4}$ alkyl-C(O)NH-, or $NH_2$-$C_{1-6}$ alkylene-; and

ring B represents aryl, cycloalkyl, heterocyclyl, or heteroaryl, and $(R_{d2})_{m2}$ indicates that ring B is optionally substituted with m2 $R_{d2}$, where m2 represents an integer of 0 to 10, and each $R_{d2}$ independently represents deuterium, hydroxy, amino, mercapto, halogen, cyano, oxo, optionally deuterated $C_{1-6}$ alkyl, optionally deuterated $C_{3-6}$ cycloalkyl, optionally deuterated $C_{1-6}$ alkoxy, halogenated $C_{1-6}$ alkyl, $C_{1-4}$ alkyl-NH-, $C_{1-4}$ alkyl-NHC(O)-, $C_{1-4}$ alkyl-C(O)NH-, or $NH_2$-$C_{1-6}$ alkylene-;

wherein when $L_1$ represents a bond, $X_1$ represents optionally substituted nitrogen-containing bridged heterocyclylene; and when $X_2$ represents a bond, $L_1$ is not a bond.

**2.** The compound of Formula (I) or salts, enantiomers, diastereoisomer, isotopically enriched analogs, solvates, prodrugs, or polymorphs thereof as claimed in claim 1, wherein the compound of Formula (I) is also of Formula (II) or Formula (III):

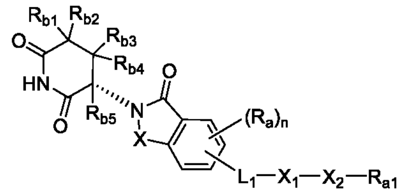

Formula (II)

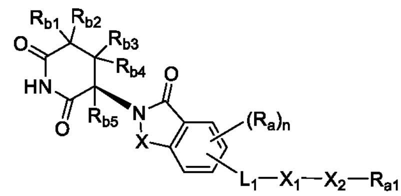

Formula (III)

wherein $R_{b1}$, $R_{b2}$, $R_{b3}$, $R_{b4}$, $R_{b5}$, $(R_a)_n$, X, $L_1$, $X_1$, $X_2$ and $R_{a1}$ are as defined in claim 1.

**3.** The compound of Formula (I) or salts, enantiomers, diastereoisomer, isotopically enriched analogs, solvates, prodrugs, or polymorphs thereof as claimed in claim 1, wherein the compound of Formula (I) is also of Formula (Ia), Formula (Ib), Formula (Ic), Formula (Id), Formula (IIa), Formula (IIb), Formula (IIc), Formula (IId), Formula (IIIa), Formula (IIIb), Formula (IIIc), or Formula (IIId):

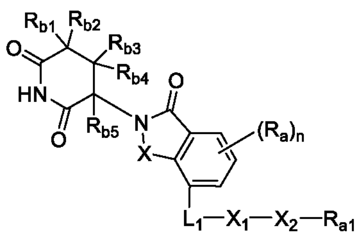

Formula (Ia)

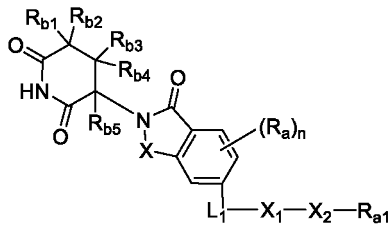

Formula (Ib)

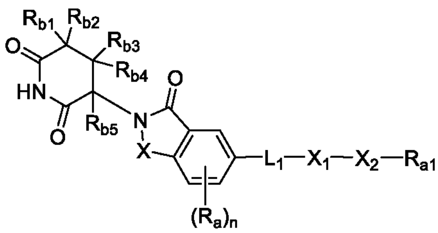

Formula (Ic)

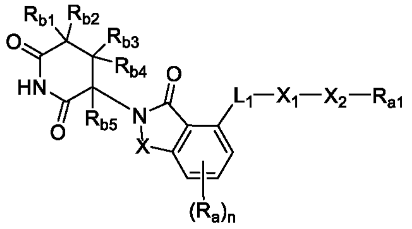
Formula (Id)

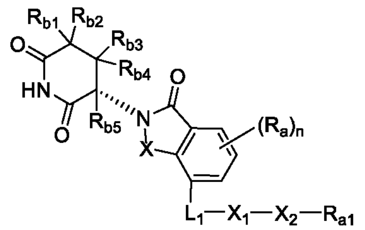
Formula (IIa)

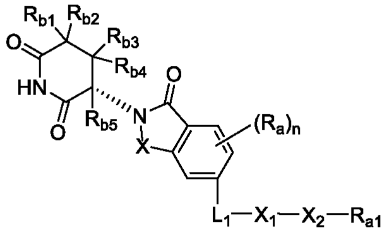
Formula (IIb)

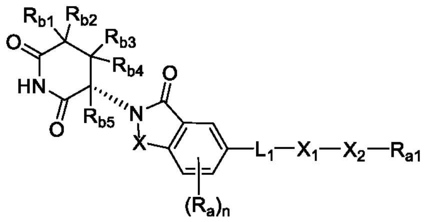
Formula (IIc)

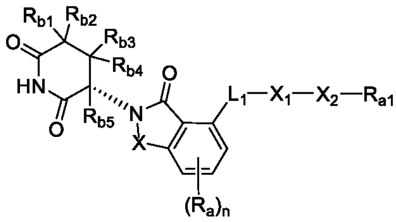
Formula (IId)

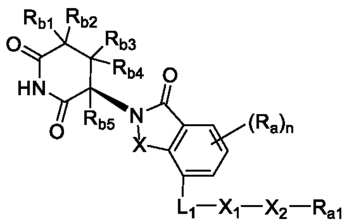
Formula (IIIa)

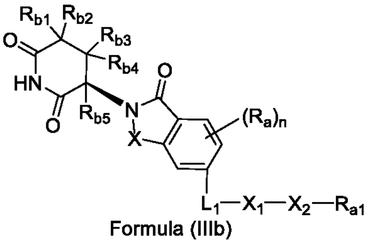
Formula (IIIb)

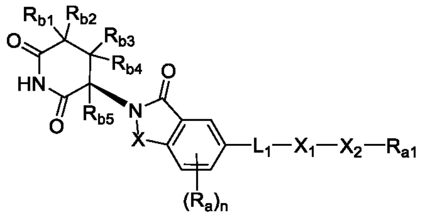
Formula (IIIc)

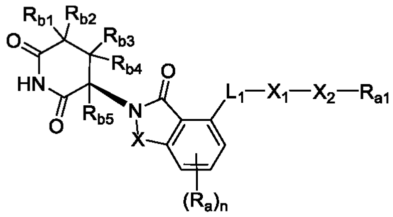
Formula (IIId)

wherein $R_{b1}$, $R_{b2}$, $R_{b3}$, $R_{b4}$, $R_{b5}$, $(R_a)_n$, X, $L_1$, $X_1$, $X_2$ and $R_{a1}$ are as defined in claim 1.

**4.** The compound of Formula (I) or salts, enantiomers, diastereoisomer, isotopically enriched analogs, solvates, prodrugs, or polymorphs thereof as claimed in any one of claims 1-3, wherein $R_{b1}$, $R_{b2}$, $R_{b3}$ and $R_{b4}$ each independently represent H, and $R_{b5}$ represents H to D.

**5.** The compound of Formula (I) or salts, enantiomers, diastereoisomer, isotopically enriched analogs, solvates, prodrugs, or polymorphs thereof as claimed in any one of claims 1-4, wherein X represents C(O).

**6.** The compound of Formula (I) or salts, enantiomers, diastereoisomer, isotopically enriched analogs, solvates, prodrugs, or polymorphs thereof as claimed in any one of claims 1-4, wherein X represents $CH_2$.

**7.** The compound of Formula (I) or salts, enantiomers, diastereoisomer, isotopically enriched analogs, solvates, prodrugs, or polymorphs thereof as claimed in any one of claims 1-6, wherein

$L_1$ represents C(O), $C_{2-6}$ alkenylene, optionally substituted $C_{1-5}$ alkylene, -CH=, optionally substituted $C_{6-10}$ arylene-$C_{1-5}$ alkylene-*, or $N(R_{c1})$, where $R_{c1}$ represents H or $C_{1-3}$ alkyl, and symbol * indicates the point of attachment to $X_1$, wherein the $C_{1-5}$ alkylene and the $C_{6-10}$ arylene-$C_{1-5}$ alkylene are each independently optionally substituted with 1-10 substituents selected from the group consisting of D, optionally deuterated $C_{1-4}$ alkyl, hydroxy, amino, mercapto, halogen, cyano, $C_{1-4}$ alkoxy, $C_{1-4}$ alkyl-NH-, halogenated $C_{1-4}$ alkyl, $NH_2$-$C_{1-4}$ alkylene-, $C_{1-4}$ alkyl-NHC(O)-, $C_{1-4}$ alkyl-C(O)NH- and any combination thereof; or $L_1$ represents -$C(R_{L1}R_{L2})$-, where $R_{L1}$, $R_{L2}$ together with the carbon atom to which they are attached form optionally substituted $C_{3-8}$ cycloalkylene, and/or

$X_1$ represents optionally substituted $C_{3-20}$ cycloalkylene, optionally substituted 4- to 20-membered heterocyclylene or optionally substituted 5- to 20-membered heteroarylene, wherein the $C_{3-20}$ cycloalkylene and the 4- to 20-membered heterocyclylene are each independently optionally substituted with one or more substituents selected from the group consisting of D, optionally deuterated $C_{1-4}$ alkyl, hydroxy, amino, mercapto, halogen, cyano, oxo, $C_{1-4}$ alkoxy, $C_{1-4}$ alkyl-NH-, halogenated $C_{1-4}$ alkyl, $NH_2$-$C_{1-4}$ alkylene, $C_{1-4}$ alkyl-NHC(O)-, $C_{1-4}$ alkyl-C(O)NH- and any combination thereof, and the 5- to 20-membered heteroarylene is optionally substituted with

one or more substituents selected from the group consisting of D, optionally deuterated $C_{1-4}$ alkyl, hydroxy, amino, mercapto, halogen, cyano, $C_{1-4}$ alkoxy, $C_{1-4}$ alkyl-NH-, halogenated $C_{1-4}$ alkyl, $NH_2$-$C_{1-4}$ alkylene-, $C_{1-4}$ alkyl-NHC(O)-, $C_{1-4}$ alkyl-C(O)NH- and any combination thereof.

**8.** The compound of Formula (I) or salts, enantiomers, diastereoisomer, isotopically enriched analogs, solvates, prodrugs, or polymorphs thereof as claimed in claim 7, wherein

$L_1$ represents C(O), vinylene, optionally substituted $C_{1-5}$ alkylene, -CH=, optionally substituted arylene-$C_{1-5}$ alkylene-*, optionally substituted naphthylene-$C_{1-5}$ alkylene-*, or N($R_{c1}$), where $R_{c1}$ represents H or $C_{1-3}$ alkyl, and symbol * indicates the point of attachment to $X_1$, wherein the $C_{1-5}$ alkylene, the arylene-$C_{1-5}$ alkylene and the naphthylene-$C_{1-5}$ alkylene are each independently optionally substituted with 1-10 substituents selected from the group consisting of D, optionally deuterated $C_{1-4}$ alkyl, hydroxy, amino, mercapto, halogen, cyano, $C_{1-4}$ alkoxy, $C_{1-4}$ alkyl-NH-, halogenated $C_{1-4}$ alkyl, $NH_2$-$C_{1-4}$ alkylene-, $C_{1-4}$ alkyl-NHC(O)-, $C_{1-4}$ alkyl-C(O)NH- and any combination thereof; or $L_1$ represents -C($R_{L1}R_{L2}$)-, where $R_{L1}$, $R_{L2}$ together with the carbon atom to which they are attached form optionally substituted $C_{3-8}$ cycloalkylene, wherein the $C_{3-8}$ cycloalkylene is optionally substituted with 1-10 substituents selected from the group consisting of D, optionally deuterated $C_{1-4}$ alkyl, hydroxy, amino, mercapto, halogen, cyano, $C_{1-4}$ alkoxy, $C_{1-4}$ alkyl-NH-, halogenated $C_{1-4}$ alkyl, $NH_2$-$C_{1-4}$ alkylene-, $C_{1-4}$ alkyl-NHC(O)-, $C_{1-4}$ alkyl-C(O)NH- and any combination thereof; and/or
$X_1$ represents the following divalent groups:

cyclopropylene, cyclobutylene, cyclopentylene, cyclopentenylene, cyclohexylene, cyclohexenylene, cycloheptylene, cyclooctylene, decalinylene, octahydropentalenylene, octahydro-1H-indenylene, spiro[3.3]heptylene, spiro[2.5]octylene, spiro[3.5]nonylene, spiro[4.4]nonylene, spiro[4.5]decylene, spiro[5.5]undecylene, p-menthanylene, *m*-menthanylene, quinuclidinylene, adamantanylene, noradamantanylene, norbornylene, bicyclo[2.2.1]heptylene, 2-oxobicyclo[2.2.1]heptylene, or bicyclo[2.2.1]heptentylene, with each group being optionally substituted with one or more substituents selected from the group consisting of D, optionally deuterated $C_{1-4}$ alkyl, hydroxy, amino, mercapto, halogen, cyano, oxo, $C_{1-4}$ alkoxy, $C_{1-4}$ alkyl-NH-, halogenated $C_{1-4}$ alkyl, $NH_2$-$C_{1-4}$ alkylene-, $C_{1-4}$ alkyl-NHC(O)-, $C_{1-4}$ alkyl-C(O)NH- and any combination thereof; or
azetidinylene, pyrrolidinylene, imidazolidylene, pyrazolidylene, oxazolidinylene, thiazolidinylene, piperidinylene, piperazinylene, morpholinylene, thiomorpholinylene, azepanylene, azacyclooctylene, diazacycloheptanylene, diazacyclooctylene, 3-azabicyclo[3.1.0]hexylene, 3-azabicyclo[3.1.1]heptanylene, 2-azabicyclo[2.2.1]heptanylene, 6-azabicyclo[3.1.1]heptanylene, 2-azabicyclo[2.2.2]octylene, 2,5-diazabicyclo [2.2.1]heptanylene, 3,6-diazabicyclo[3.1.1]heptanylene, 3-azabicyclo[3.2.1]octylene, 3,8-diazabicyclo [3.2.1]octylene, 2,5-diazabicyclo[2.2.2]octylene, 2,6-diazaspiro[3.3]heptanylene, 2,7-diazaspiro[3.5]nonylene, 3-azaspiro[5.5]undecylene, 7-azaspiro[3.5]nonylene, or octahydropyrrolo[3,4-c]pyrrolylene, with each group being optionally substituted with one or more substituents selected from the group consisting of D, optionally deuterated $C_{1-4}$ alkyl, hydroxy, amino, mercapto, halogen, cyano, oxo, $C_{1-4}$ alkoxy, $C_{1-4}$ alkyl-NH-, halogenated $C_{1-4}$ alkyl, $NH_2$-$C_{1-4}$ alkylene-, $C_{1-4}$ alkyl-NHC(O)-, $C_{1-4}$ alkyl-C(O)NH- and any combination thereof; or
furanylene, oxazolylene, isoxazolylene, oxadiazolylene, thienylene, thiazolylene, isothiazolylene, thiadiazolylene, pyrrolylene, imidazolylene, pyrazolylene, triazolylene, pyridylene, pyrimidinylene, pyridazinylene, pyrazinylene, indolylene, isoindolylene, benzofuranylene, isobenzofuranylene, benzothienylene, indazolylene, benzimidazolylene, benzoxazolylene, benzisoxazolylene, benzothiazolylene, benzisothiazolylene, benzotriazolylene, benzo[2,1,3]oxadiazolylene, benzo[2,1,3]thiadiazolylene, benzo[1,2,3]thiadiazolylene, quinolinylene, isoquinolinylene, naphthyridinylene, cinnolinylene, quinazolinylene, quinoxalinylene, phthalazinylene, pyrazolo[1,5-a]pyridylene, pyrazolo[1,5-a]pyrimidinylene, imidazo[1,2-a]pyridylene, 1H-pyrrolo [3,2-b]pyridylene, 1H-pyrrolo[2,3-b]pyridylene, 4H-fluoro[3,2-b]pyrrolylene, pyrrolo[2,1-b]thiazolylene, or imidazo[2,1-b]thiazolylene, with each group being optionally substituted with one or more substituents selected from the group consisting of D, optionally deuterated $C_{1-4}$ alkyl, hydroxy, amino, mercapto, halogen, cyano, $C_{1-4}$ alkoxy, $C_{1-4}$ alkyl-NH-, halogenated $C_{1-4}$ alkyl, $NH_2$-$C_{1-4}$ alkylene-, $C_{1-4}$ alkyl-NHC(O)-, $C_{1-4}$ alkyl-C(O)NH- and any combination thereof.

**9.** The compound of Formula (I) or salts, enantiomers, diastereoisomer, isotopically enriched analogs, solvates, prodrugs, or polymorphs thereof as claimed in claim 7, wherein $X_1$ represents the following divalent groups:

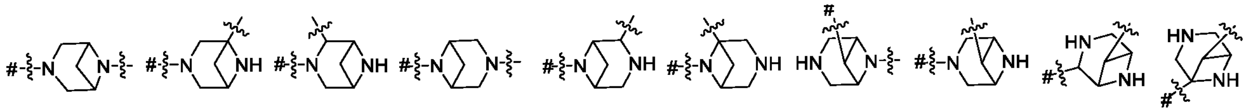

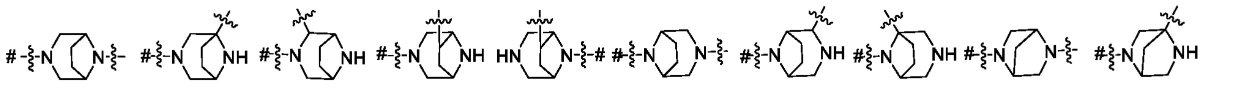

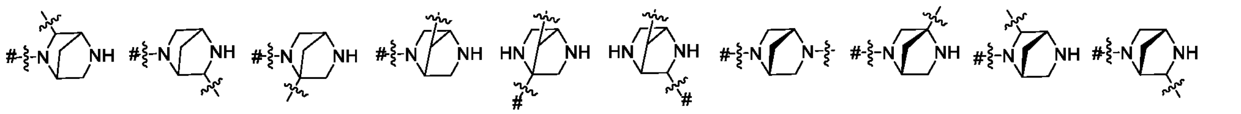

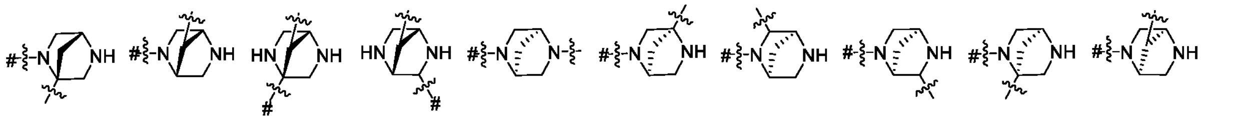

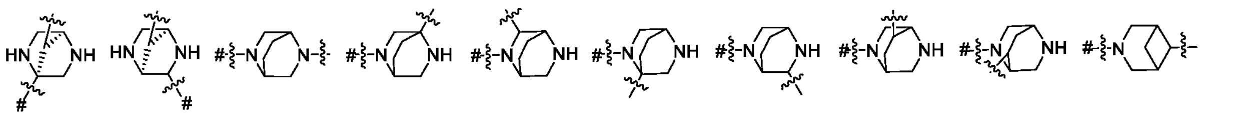

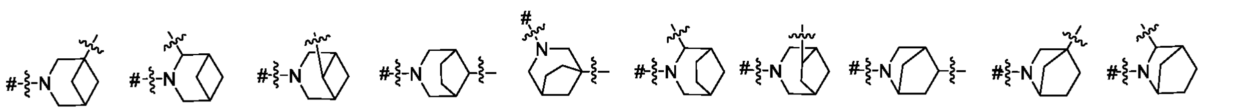

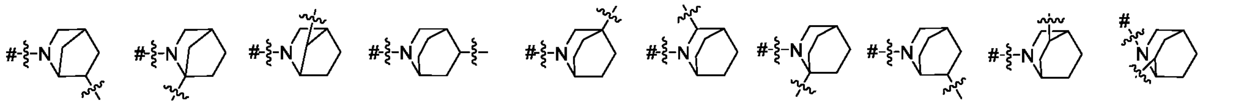

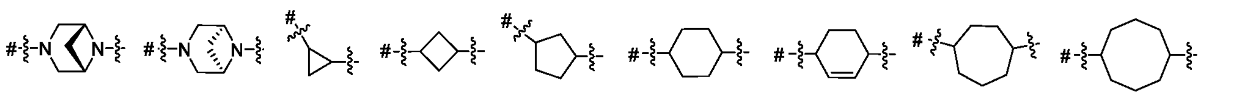

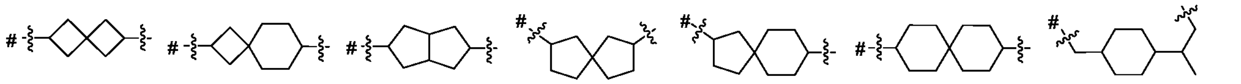

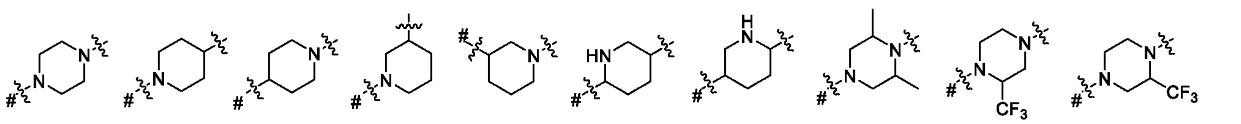

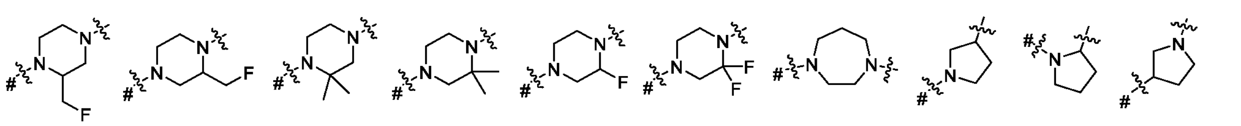

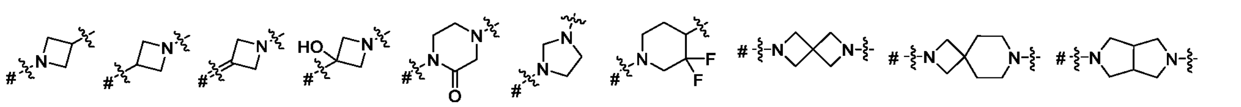

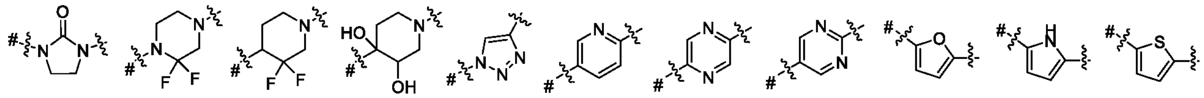

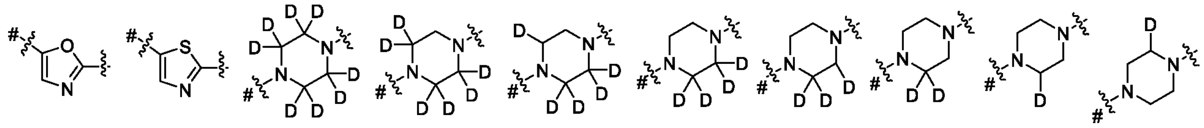

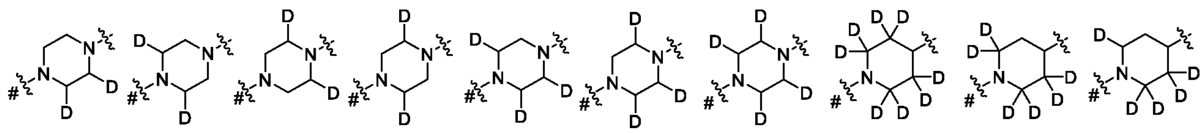

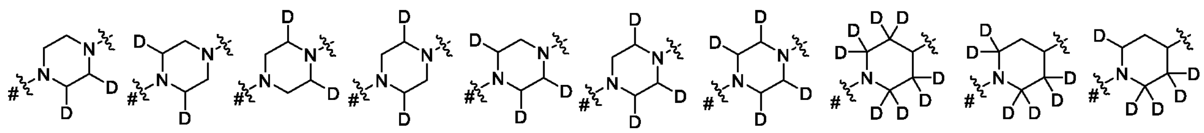

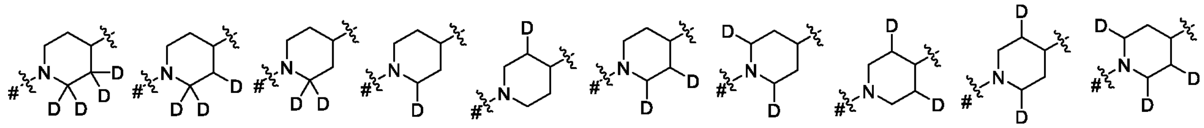

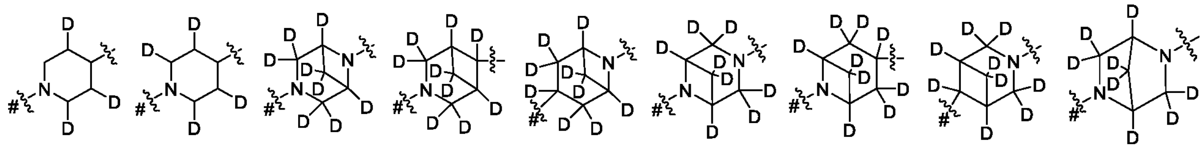

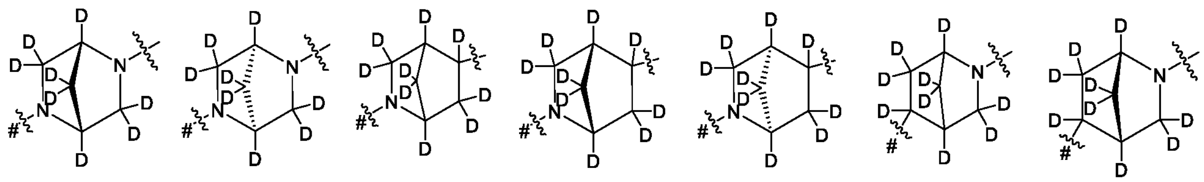

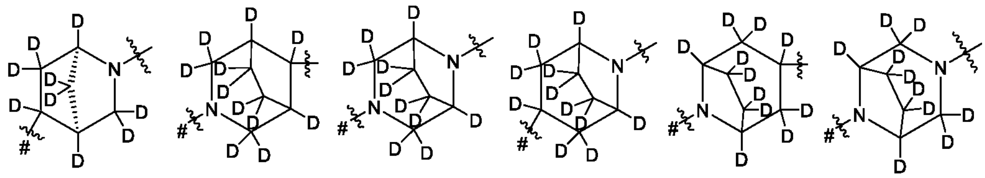 or 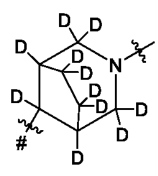,

wherein symbol # indicates the point of attachment to $L_1$.

**10.** The compound of Formula (I) or salts, enantiomers, diastereoisomer, isotopically enriched analogs, solvates, prodrugs, or polymorphs thereof as claimed in claim 7, wherein $L_1$ represents the following groups:

C(O), $-CH_2-$, $-(CH_2)_2-$, $-(CH_2)_3-$, -CH(OH)-, $-CHF_2-$, $-CH_2F-$, NH, -CH=CH-, -CH=, or optionally substituted arylene-$CH_2$-*, where symbol * indicates the point of attachment to $X_1$, wherein the arylene is optionally substituted with 1-4 substituents selected from the group consisting of D, optionally deuterated $C_{1-4}$ alkyl, hydroxy, amino, mercapto, halogen, cyano, $C_{1-4}$ alkoxy, $C_{1-4}$ alkyl-NH-, halogenated $C_{1-4}$ alkyl, $NH_2$-$C_{1-4}$ alkylene-, $C_{1-4}$ alkyl-NHC(O)-, $C_{1-4}$ alkyl-C(O)NH-and any combination thereof, or

$L_1$ represents $-C(R_{L1}R_{L2})-$, where $R_{L1}$, $R_{L2}$ together with the carbon atom to which they are attached form

optionally substituted cyclopropylene, optionally substituted cyclobutylene, optionally substituted cyclopentylene, optionally substituted cyclohexylene, optionally substituted cycloheptylene, or optionally substituted cyclooctylene, wherein the cyclopropylene, cyclobutylene, cyclopentylene, cyclohexylene, cycloheptylene, or cyclooctylene is optionally substituted with 1-10 substituents selected from the group consisting of D, optionally deuterated $C_{1-4}$ alkyl, hydroxy, amino, mercapto, halogen, cyano, $C_{1-4}$ alkoxy, $C_{1-4}$ alkyl-NH-, halogenated $C_{1-4}$ alkyl, $NH_2$-$C_{1-4}$ alkylene-, $C_{1-4}$ alkyl-NHC(O)-, $C_{1-4}$ alkyl-C(O)NH- and any combination thereof.

**11.** The compound of Formula (I) or salts, enantiomers, diastereoisomer, isotopically enriched analogs, solvates, prodrugs, or polymorphs thereof as claimed in any one of claims 1-6, wherein $L_1$ represents a bond, and $X_1$ represents optionally substituted 5- to 20-membered nitrogen-containing bridged heterocyclylene, wherein the 5- to 20-membered nitrogen-containing bridged heterocyclylene is optionally substituted with one or more substituents selected from the group consisting of D, optionally deuterated $C_{1-4}$ alkyl, hydroxy, amino, mercapto, halogen, cyano, oxo, $C_{1-4}$ alkoxy, $C_{1-4}$ alkyl-NH-, halogenated $C_{1-4}$ alkyl, $NH_2$-$C_{1-4}$ alkylene-, $C_{1-4}$ alkyl-NHC(O)-, $C_{1-4}$ alkyl-C(O)NH- and any combination thereof.

**12.** The compound of Formula (I) or salts, enantiomers, diastereoisomer, isotopically enriched analogs, solvates, prodrugs, or polymorphs thereof as claimed in claim 11, wherein $X_1$ represents the following divalent groups: 3-azabicyclo[3.1.0]hexylene, 3-azabicyclo[3.1.1]heptanylene, 2-azabicyclo[2.2.1]heptanylene, 6-azabicyclo[3.1.1]heptanylene, 2-azabicyclo[2.2.2]octylene, 2,5-diazabicyclo[2.2.1]heptanylene, 3,6-diazabicyclo[3.1.1]heptanylene, 3-azabicyclo[3.2.1]octylene, 3,8-diazabicyclo[3.2.1]octylene, or 2,5-diazabicyclo[2.2.2]octylene, with each group being optionally substituted with one or more substituents selected from the group consisting of D, optionally deuterated $C_{1-4}$ alkyl, hydroxy, amino, mercapto, halogen, cyano, oxo, $C_{1-4}$ alkoxy, $C_{1-4}$ alkyl-NH-, halogenated $C_{1-4}$ alkyl, $NH_2$-$C_{1-4}$ alkylene-, $C_{1-4}$ alkyl-NHC(O)-, $C_{1-4}$ alkyl-C(O)NH- and any combination thereof.

**13.** The compound of Formula (I) or salts, enantiomers, diastereoisomer, isotopically enriched analogs, solvates, prodrugs, or polymorphs thereof as claimed in claim 11, wherein

$X_1$ represents the following divalent groups:

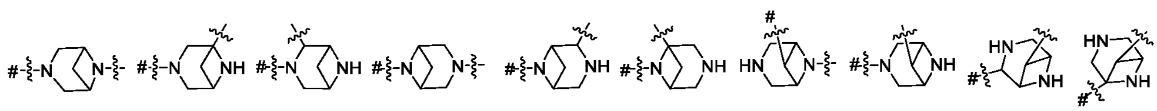

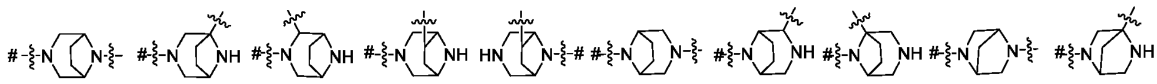

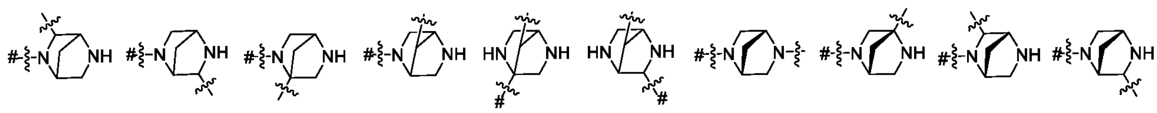

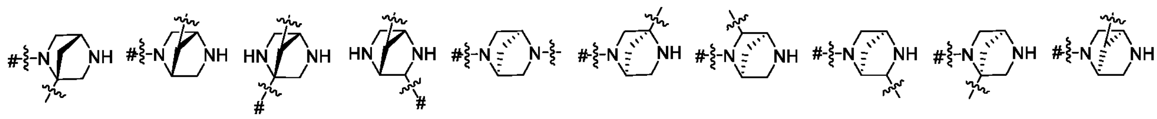

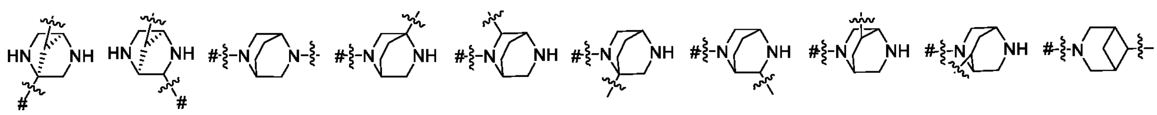

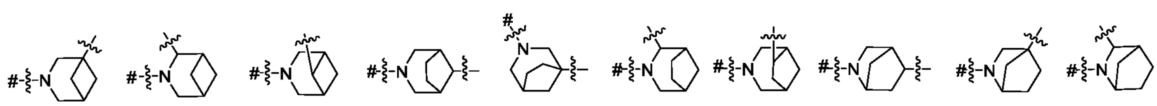

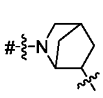 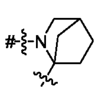 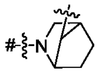 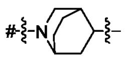 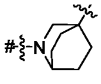 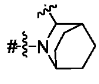 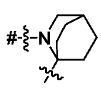 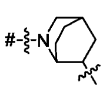 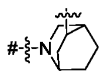 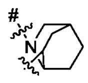

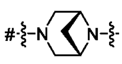 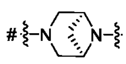 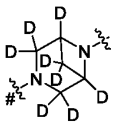 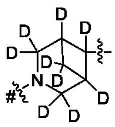 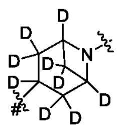 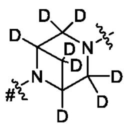 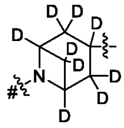 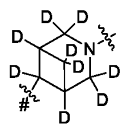 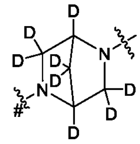

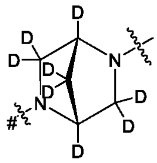 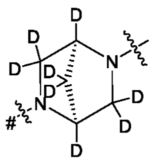 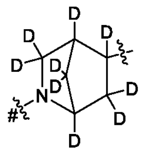 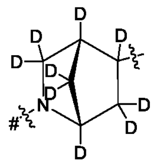 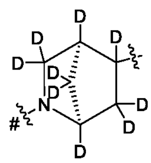 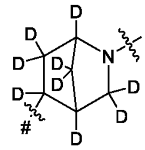 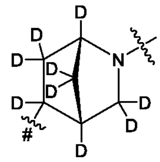

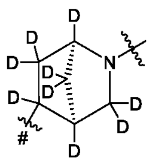 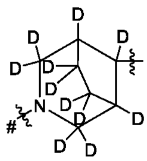 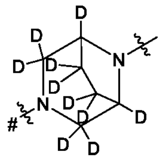 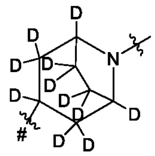 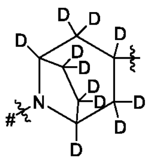 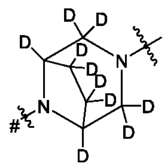 or 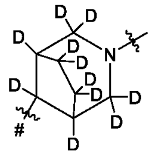 ,

wherein symbol # indicates the point of attachment to $L_1$.

**14.** The compound of Formula (I) or salts, enantiomers, diastereoisomer, isotopically enriched analogs, solvates, prodrugs, or polymorphs thereof as claimed in any one of claims 1-13, wherein $X_2$ represents C(O), optionally substituted $C_{1-5}$ alkylene, optionally substituted $C_{3-8}$ cycloalkylene, optionally substituted $C_{1-2}$ alkylene-N($R_{c2}$)-, -N($R_{c2}$)-optionally substituted $C_{1-2}$ alkylene, or N($R_{c3}$), where $R_{c2}$ and $R_{c3}$ each independently represent H or $C_{1-3}$ alkyl, wherein the $C_{1-5}$ alkylene and $C_{3-8}$ cycloalkylene are each independently optionally substituted with 1-10 substituents selected from the group consisting of D, optionally deuterated $C_{1-4}$ alkyl, hydroxy, amino, mercapto, halogen, cyano, oxo, $C_{1-4}$ alkoxy, $C_{3-8}$ cycloalkyl, $C_{1-4}$ alkyl-NH-, halogenated $C_{1-4}$ alkyl, $NH_2$-$C_{1-4}$ alkylene, $C_{1-4}$ alkyl-NHC(O)-, $C_{1-4}$ alkyl-C(O)NH- and any combination thereof, and the $C_{1-2}$ alkylene is optionally substituted with 1-4 substituents selected from the group consisting of D, optionally deuterated $C_{1-4}$ alkyl, hydroxy, amino, mercapto, halogen, cyano, oxo, $C_{1-4}$ alkoxy, $C_{3-8}$ cycloalkyl, $C_{1-4}$ alkyl-NH-, halogenated $C_{1-4}$ alkyl, $NH_2$-$C_{1-4}$ alkylene, $C_{1-4}$ alkyl-NHC(O)-, $C_{1-4}$ alkyl-C(O)NH- and any combination thereof.

**15.** The compound of Formula (I) or salts, enantiomers, diastereoisomer, isotopically enriched analogs, solvates, prodrugs, or polymorphs thereof as claimed in claim 14, wherein $X_2$ represents C(O), $-CH_2-$, $-(CH_2)_2-$, $-(CH_2)_3-$, NH, $-CH_2$-NH-, $-(CH_2)_2$-NH-, -NH-$(CH_2)_2$-, or -NH-$CH_2$-.

**16.** The compound of Formula (I) or salts, enantiomers, diastereoisomer, isotopically enriched analogs, solvates, prodrugs, or polymorphs thereof as claimed in any one of claims 1-10, wherein $X_2$ represents a bond, and wherein when $X_2$ represents a bond, $L_1$ is not a bond.

**17.** The compound of Formula (I) or salts, enantiomers, diastereoisomer, isotopically enriched analogs, solvates, prodrugs, or polymorphs thereof as claimed in any one of claims 1-16, wherein $R_{a1}$ represents the following structure:

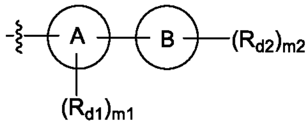

Formula (I-A)

wherein ring A represents 4- to 20-membered heterocyclylene, $C_{3-20}$ cycloalkylene, $C_{6-20}$ arylene, or 5-to 20-membered heteroarylene, and $(R_{d1})_{m1}$ indicates that ring A is optionally substituted with m1 $R_{d1}$, where m1 represents an integer of 0 to 10, and each $R_{d1}$ independently represents deuterium, hydroxy, amino, mercapto, halogen, cyano, oxo, optionally deuterated $C_{1-6}$ alkyl, optionally deuterated $C_{3-6}$ cycloalkyl, optionally deuterated $C_{1-6}$ alkoxy, halogenated $C_{1-6}$ alkyl, $C_{1-4}$ alkyl-NH-, $C_{1-4}$ alkyl-NHC(O)-, $C_{1-4}$ alkyl-C(O)NH-, or $NH_2$-$C_{1-6}$ alkylene-; and/or

ring B represents $C_{6-20}$ aryl, $C_{3-20}$ cycloalkyl, 4- to 20-membered heterocyclyl, or 5- to 20-membered heteroaryl, and $(R_{d2})_{m2}$ indicates that ring B is optionally substituted with m2 $R_{d2}$, where m2 represents an integer of 0 to 10, and each $R_{d2}$ independently represents deuterium, hydroxy, amino, mercapto, halogen, cyano, oxo, optionally deuterated $C_{1-6}$ alkyl, optionally deuterated $C_{3-6}$ cycloalkyl, optionally deuterated $C_{1-6}$ alkoxy, halogenated $C_{1-6}$ alkyl, $C_{1-4}$ alkyl-NH-, $C_{1-4}$ alkyl-NHC(O)-, $C_{1-4}$ alkyl-C(O)NH-, or $NH_2$-$C_{1-6}$ alkylene-.

**18.** The compound of Formula (I) or salts, enantiomers, diastereoisomer, isotopically enriched analogs, solvates, prodrugs, or polymorphs thereof as claimed in claim 17, wherein ring A represents the following divalent groups:

cyclopropylene, cyclobutylene, cyclopentylene, cyclopentenylene, cyclohexylene, cyclohexenylene, cycloheptylene, cycloheptenylene, cyclooctylene, decalinylene, octahydropentalenylene, octahydro-1H-indenylene, spiro[3.3]heptylene, spiro[2.5]octylene, spiro[3.5]nonylene, spiro[4.4]nonylene, spiro[4.5]decylene, spiro[5.5]undecylene, *p*-menthanylene, *m*-menthanylene, quinuclidinylene, adamantanylene, noradamantanylene, norbornylene, bicyclo[2.2.1]heptylene, 2-oxobicyclo[2.2.1]heptylene, or bicyclo[2.2.1]heptentylene, with each group being optionally substituted with one or more substituents selected from the group consisting of D, hydroxy, amino, mercapto, halogen, cyano, oxo, optionally deuterated $C_{1-6}$ alkyl, optionally deuterated $C_{3-6}$ cycloalkyl, optionally deuterated $C_{1-6}$ alkoxy, halogenated $C_{1-6}$ alkyl, $C_{1-4}$ alkyl-NH-, $C_{1-4}$ alkyl-NHC(O)-, $C_{1-4}$ alkyl-C(O)NH-, $NH_2$-$C_{1-6}$ alkylene- and any combination thereof;

arylene or naphthylene, with each group being optionally substituted with one or more substituents selected from the group consisting of D, hydroxy, amino, mercapto, halogen, cyano, optionally deuterated $C_{1-6}$ alkyl, optionally deuterated $C_{3-6}$ cycloalkyl, optionally deuterated $C_{1-6}$ alkoxy, halogenated $C_{1-6}$ alkyl, $C_{1-4}$ alkyl-NH-, $C_{1-4}$ alkyl-NHC(O)-, $C_{1-4}$ alkyl-C(O)NH-, $NH_2$-$C_{1-6}$ alkylene- and any combination thereof;

azetidinylene, pyrrolidinylene, imidazolidylene, pyrazolidylene, oxazolidinylene, thiazolidinylene, piperidinylene, piperazinylene, morpholinylene, thiomorpholinylene, pyranylene, dihydropyranylene, azepanylene, azacyclooctylene, diazacycloheptanylene, diazacyclooctylene, 3-azabicyclo[3.1.0]hexylene, 3-azabicyclo[3.1.1]heptanylene, 2-azabicyclo[2.2.1]heptanylene, 6-azabicyclo[3.1.1]heptanylene, 2-azabicyclo[2.2.2]octylene, 2,5-diazabicyclo[2.2.1]heptanylene, 3,6-diazabicyclo[3.1.1]heptanylene, 3-azabicyclo[3.2.1]octylene, 3,8-diazabicyclo [3.2.1]octylene, 2,5-diazabicyclo[2.2.2]octylene, 2,6-diazaspiro[3.3]heptanylene, 2,7-diazaspiro[3.5]nonylene, 3-azaspiro[5.5]undecylene, 7-azaspiro[3.5]nonylene, or octahydropyrrolo[3,4-c]pyrrolylene, with each group being optionally substituted with one or more substituents selected from the group consisting of D, hydroxy, amino, mercapto, halogen, cyano, oxo, optionally deuterated $C_{1-6}$ alkyl, optionally deuterated $C_{3-6}$ cycloalkyl, optionally deuterated $C_{1-6}$ alkoxy, halogenated $C_{1-6}$ alkyl, $C_{1-4}$ alkyl-NH-, $C_{1-4}$ alkyl-NHC(O)-, $C_{1-4}$ alkyl-C(O)NH-, $NH_2$-$C_{1-6}$ alkylene- and any combination thereof; or

furanylene, oxazolylene, isoxazolylene, oxadiazolylene, thienylene, thiazolylene, isothiazolylene, thiadiazolylene, pyrrolylene, imidazolylene, pyrazolylene, triazolylene, pyridylene, pyrimidinylene, pyridazinylene, pyrazinylene, indolylene, isoindolylene, benzofuranylene, isobenzofuranylene, benzothienylene, indazolylene, benzimidazolylene, benzoxazolylene, benzisoxazolylene, benzothiazolylene, benzisothiazolylene, benzotriazolylene, benzo[2,1,3]oxadiazolylene, benzo[2,1,3]thiadiazolylene, benzo[1,2,3]thiadiazolylene, quinolinylene, isoquinolinylene, naphthyridinylene, cinnolinylene, quinazolinylene, quinoxalinylene, phthalazinylene, pyrazolo [1,5-a]pyridylene, pyrazolo[1,5-a]pyrimidinylene, imidazo[1,2-a]pyridylene, 1H-pyrrolo[3,2-b]pyridylene, 1H-pyrrolo[2,3-b]pyridylene, 4H-fluoro[3,2-b]pyrrolylene, pyrrolo[2,1-b]thiazolylene, or imidazo[2,1-b]thiazolylene, with each group being optionally substituted with one or more substituents selected from the group consisting of D, hydroxy, amino, mercapto, halogen, cyano, optionally deuterated $C_{1-6}$ alkyl, optionally deuterated $C_{3-6}$ cycloalkyl, optionally deuterated $C_{1-6}$ alkoxy, halogenated $C_{1-6}$ alkyl, $C_{1-4}$ alkyl-NH-, $C_{1-4}$ alkyl-NHC(O)-, $C_{1-4}$ alkyl-C(O)NH-, $NH_2$-$C_{1-6}$ alkylene- and any combination thereof;

and/or

ring B represents the following groups:

cyclopropyl, cyclobutyl, cyclopentyl, cyclopentenyl, cyclohexyl, cyclohexenyl, cycloheptyl, cyclooctyl, decalinyl, octahydropentalenyl, octahydro-1H-indenyl, spiro[3.3]heptanyl, spiro[2.5]octyl, spiro[3.5]nonyl, spiro [4.4]nonyl, spiro[4.5]decyl, spiro[5.5]undecyl, *p*-menthanyl, *m*-menthanyl, quinuclidinyl, adamantanyl, noradamantanyl, bornyl, bicyclo[2.2.1]heptanyl, 2-oxobicyclo[2.2.1]heptyl, or bicyclo[2.2.1]heptenyl, with each group being optionally substituted with one or more substituents selected from the group consisting of D,

hydroxy, amino, mercapto, halogen, cyano, oxo, optionally deuterated $C_{1-6}$ alkyl, optionally deuterated $C_{3-6}$ cycloalkyl, optionally deuterated $C_{1-6}$ alkoxy, halogenated $C_{1-6}$ alkyl, $C_{1-4}$ alkyl-NH-, $C_{1-4}$ alkyl-NHC(O)-, $C_{1-4}$ alkyl-C(O)NH-, $NH_2$-$C_{1-6}$ alkylene- and any combination thereof;

phenyl or naphthyl, with each group being optionally substituted with one or more substituents selected from the group consisting of D, hydroxy, amino, mercapto, halogen, cyano, optionally deuterated $C_{1-6}$ alkyl, optionally deuterated $C_{3-6}$ cycloalkyl, optionally deuterated $C_{1-6}$ alkoxy, halogenated $C_{1-6}$ alkyl, $C_{1-4}$ alkyl-NH-, $C_{1-4}$ alkyl-NHC(O)-, $C_{1-4}$ alkyl-C(O)NH-, $NH_2$-$C_{1-6}$ alkylene- and any combination thereof;

azetidinyl, pyrrolidinyl, imidazolidinyl, pyrazolidyl, oxazolidinyl, thiazolidinyl, piperidinyl, piperazinyl, morpholinyl, thiomorpholinyl, azacycloheptyl, azacyclooctyl, diazacycloheptanyl, diazacyclooctyl, 3-azabicyclo[3.1.0]hexyl, 3-azabicyclo[3.1.1]heptanyl, 2-azabicyclo[2.2.1]heptanyl, 6-azabicyclo[3.1.1]heptanyl, 2-azabicyclo[2.2.2]octyl, 2,5-diazabicyclo[2.2.1]heptanyl, 3,6-diazabicyclo[3.1.1]heptanyl, 3-azabicyclo[3.2.1]octyl, 3,8-diazabicyclo[3.2.1]octyl, 2,5-diazabicyclo[2.2.2]octyl, 2,6-diazaspiro[3.3]heptanyl, 2,7-diazaspiro[3.5]nonyl, 3-azaspiro[5.5]undecyl, 7-azaspiro[3.5]nonyl, or octahydropyrrolo[3,4-c]pyrrolyl, with each group being optionally substituted with one or more substituents selected from the group consisting of D, hydroxy, amino, mercapto, halogen, cyano, oxo, optionally deuterated $C_{1-6}$ alkyl, optionally deuterated $C_{3-6}$ cycloalkyl, optionally deuterated $C_{1-6}$ alkoxy, halogenated $C_{1-6}$ alkyl, $C_{1-4}$ alkyl-NH-, $C_{1-4}$ alkyl-NHC(O)-, $C_{1-4}$ alkyl-C(O)NH-, $NH_2$-$C_{1-6}$ alkylene- and any combination thereof; or

furanyl, oxazolyl, isoxazolyl, oxadiazolyl, thienyl, thiazolyl, isothiazolyl, thiadiazolyl, pyrrolyl, imidazolyl, pyrazolyl, triazolyl, pyridyl, pyrimidinyl, pyridazinyl, pyrazinyl, indolyl, isoindolyl, benzofuranyl, isobenzofuranyl, benzothienyl, indazolyl, benzimidazolyl, benzoxazolyl, benzisoxazolyl, benzothiazolyl, benzisothiazolyl, benzotriazolyl, benzo[2,1,3]oxadiazolyl, benzo[2,1,3]thiadiazolyl, benzo[1,2,3]thiadiazolyl, quinolinyl, isoquinolinyl, naphthyridinyl, cinnolinyl, quinazolinyl, quinoxalinyl, phthalazinyl, pyrazolo[1,5-a]pyridyl, pyrazolo[1,5-a]pyrimidinyl, imidazo[1,2-a]pyridyl, 1H-pyrrolo[3,2-b]pyridyl, 1H-pyrrolo[2,3-b]pyridyl, 4H-fluoro[3,2-b]pyrrolyl, pyrrolo[2,1-b]thiazolyl or imidazo[2,1-b]thiazolyl, with each group being optionally substituted with one or more substituents selected from the group consisting of D, hydroxy, amino, mercapto, halogen, cyano, optionally deuterated $C_{1-6}$ alkyl, optionally deuterated $C_{3-6}$ cycloalkyl, optionally deuterated $C_{1-6}$ alkoxy, halogenated $C_{1-6}$ alkyl, $C_{1-4}$ alkyl-NH-, $C_{1-4}$ alkyl-NHC(O)-, $C_{1-4}$ alkyl-C(O)NH-, $NH_2$-$C_{1-6}$ alkylene- and any combination thereof.

**19.** The compound of Formula (I) or salts, enantiomers, diastereoisomer, isotopically enriched analogs, solvates, prodrugs, or polymorphs thereof as claimed in claim 17, wherein ring A represents the following divalent groups:

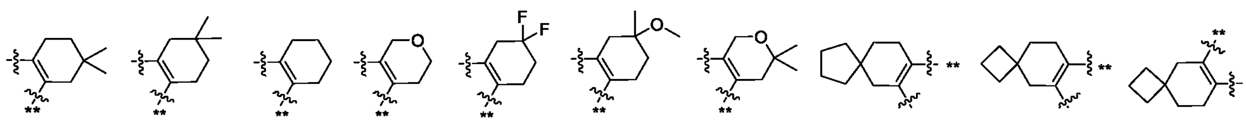

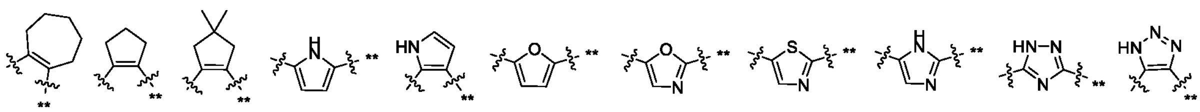

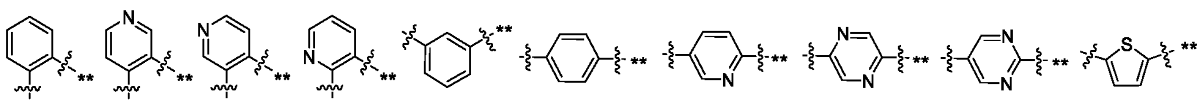

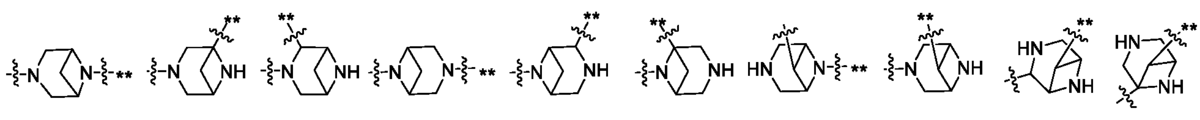

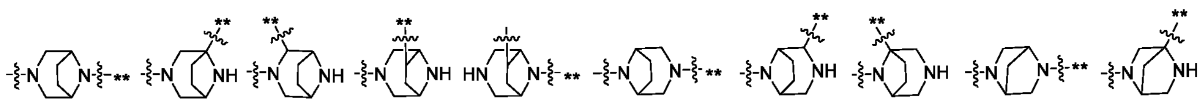

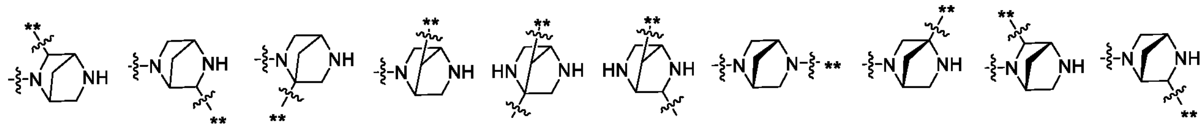

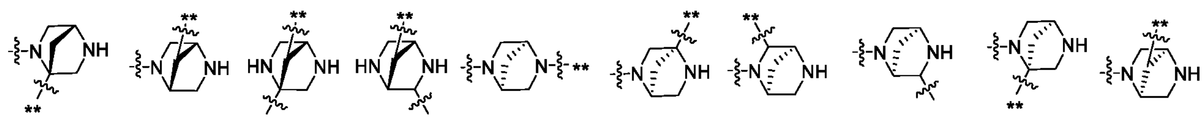

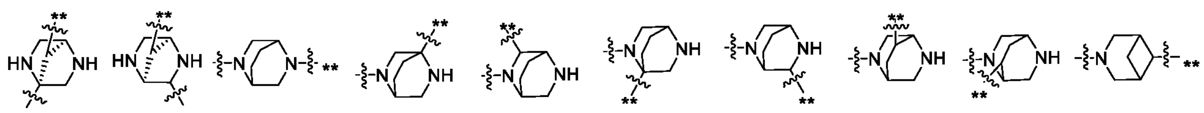

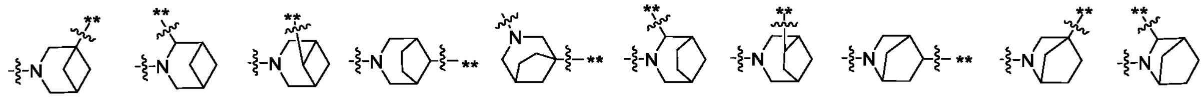

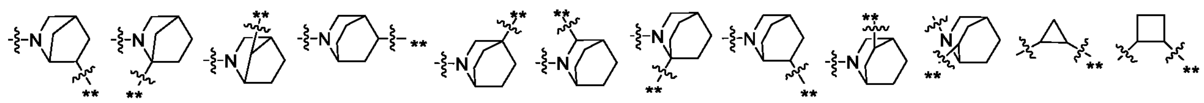

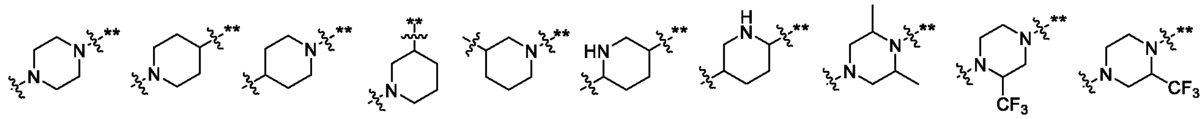

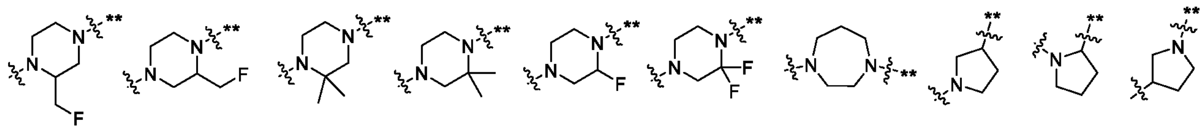

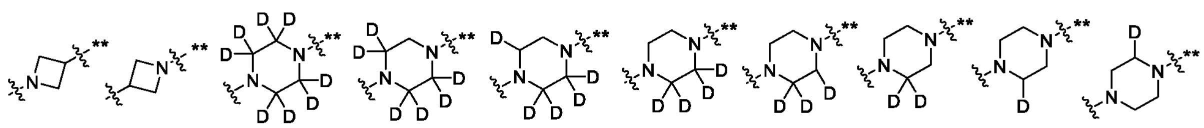

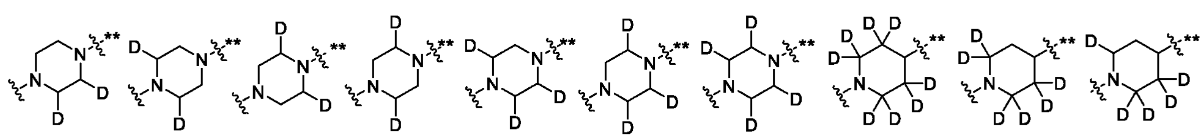

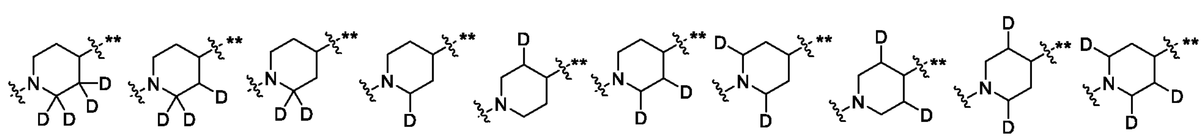

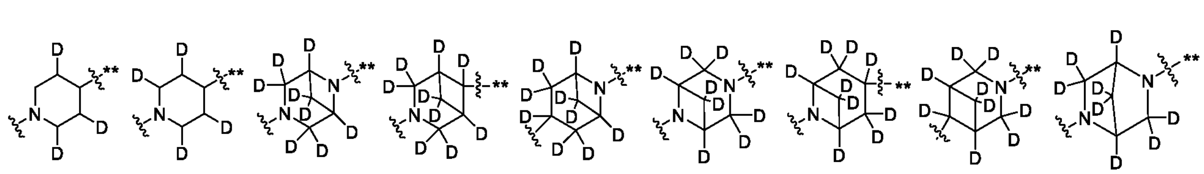

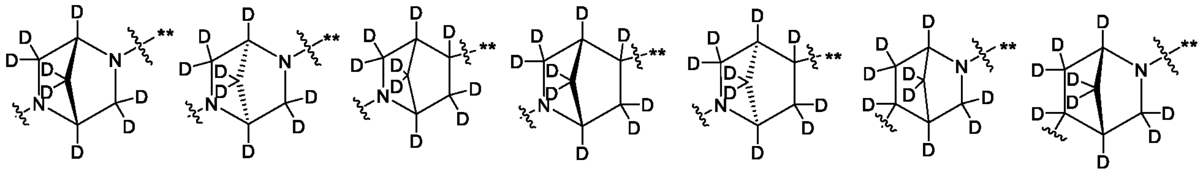

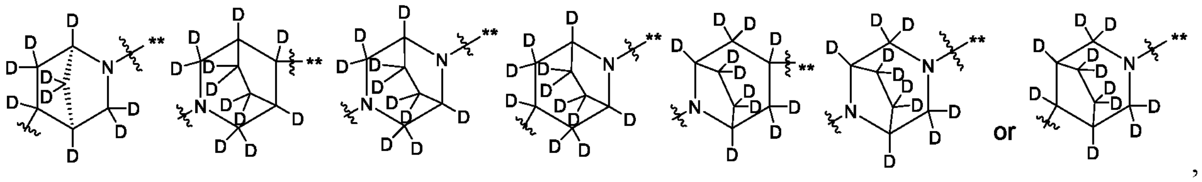

,

wherein symbol ** indicates the point of attachment to ring B; and/or ring B represents the following groups:

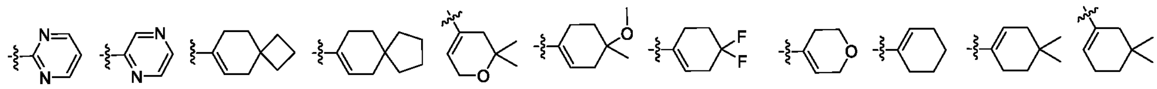

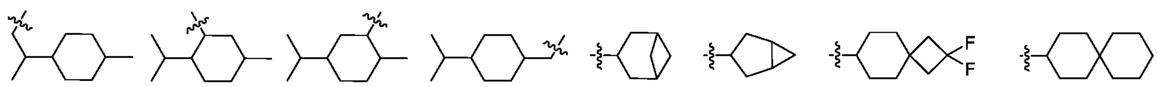

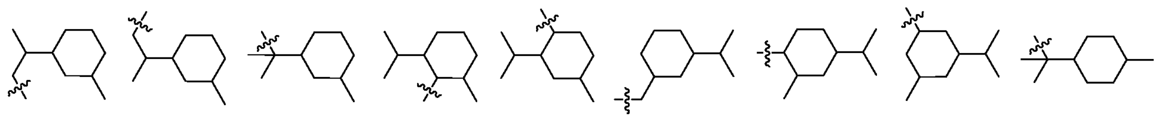

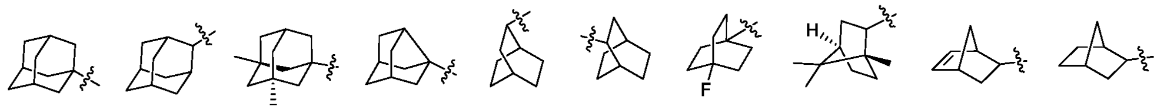

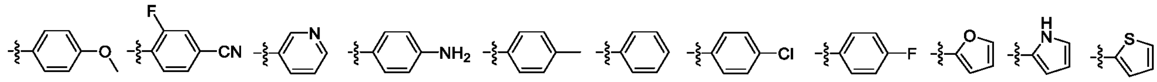

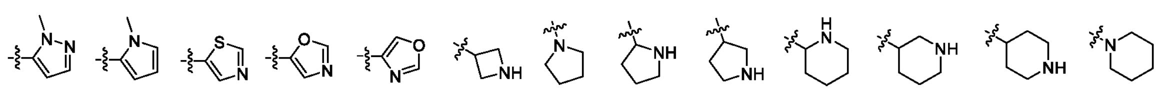

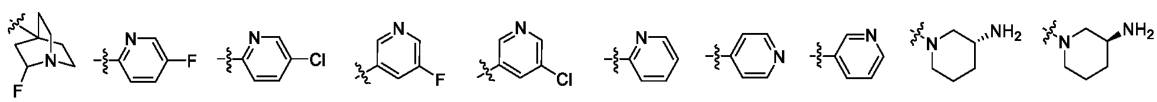

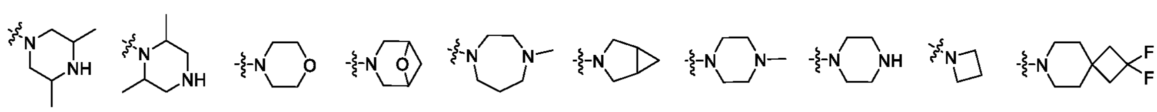

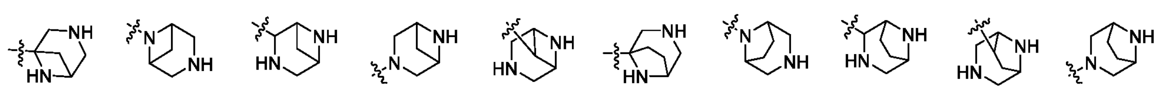

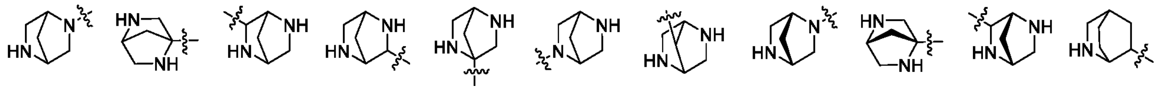

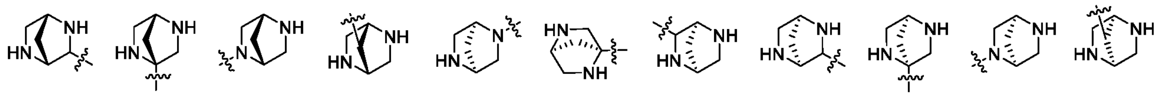

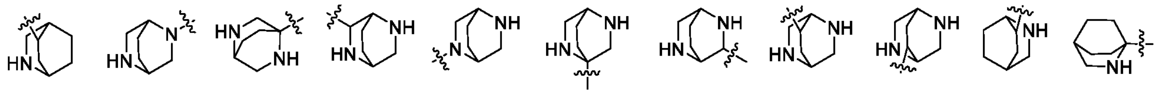

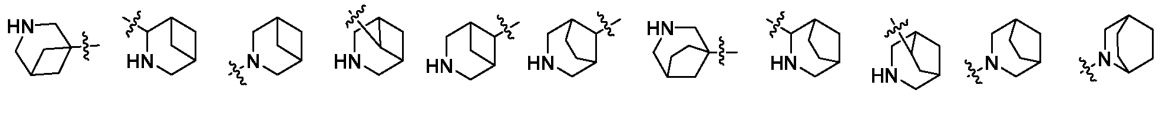

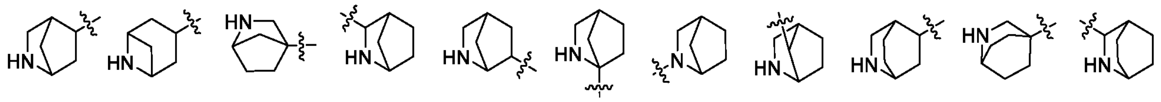

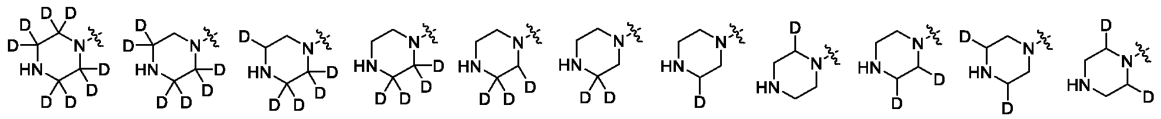

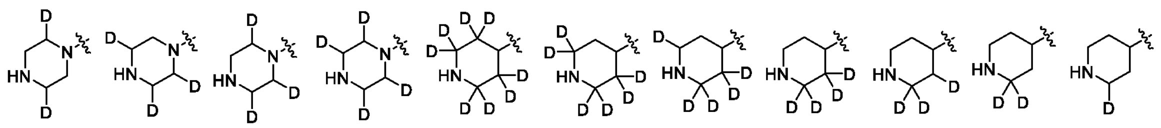

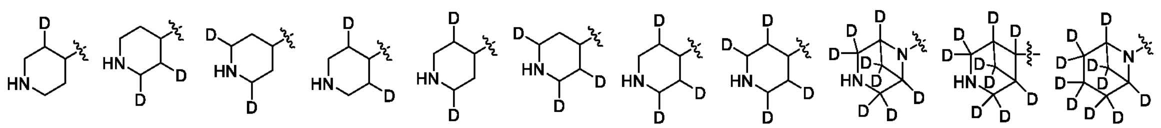

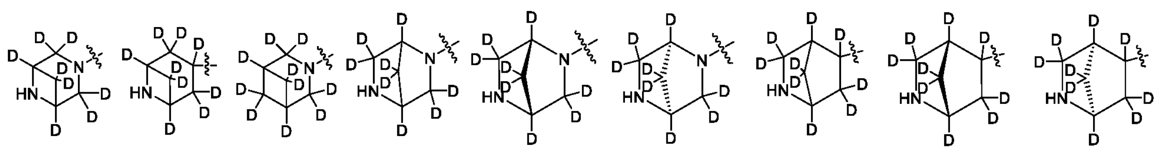

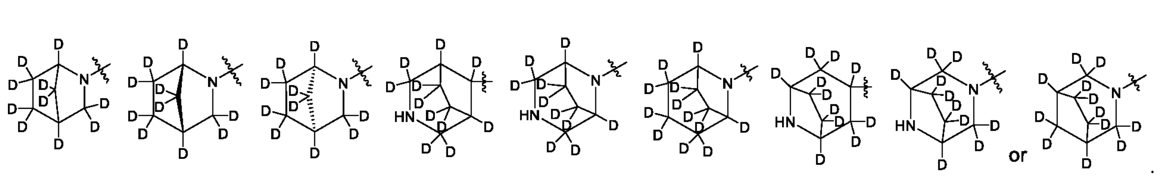

or

.

**20.** The compound of Formula (I) or salts, enantiomers, diastereoisomer, isotopically enriched analogs, solvates, prodrugs, or polymorphs thereof as claimed in any one of claims 1-17, wherein $R_{a1}$ represents the following structures:

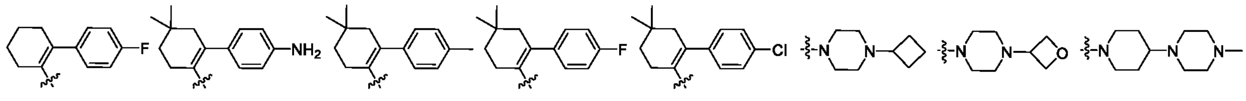

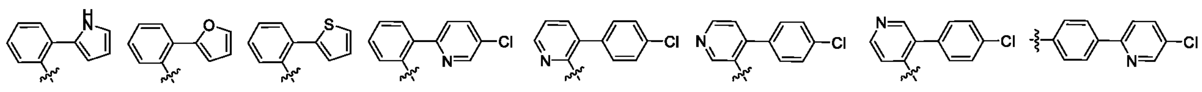

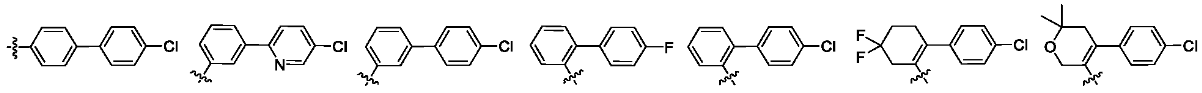

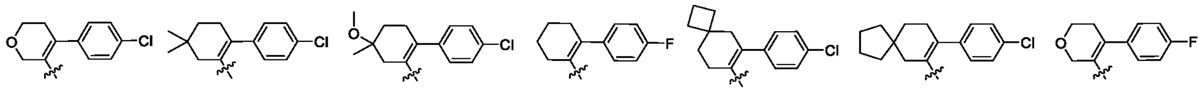

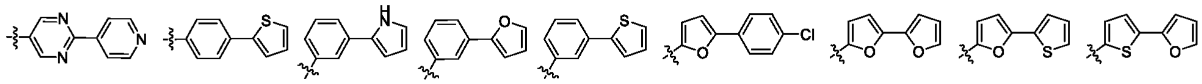

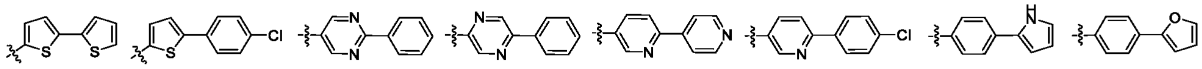

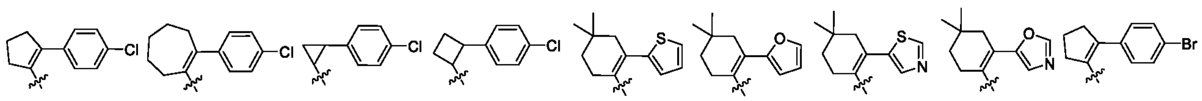

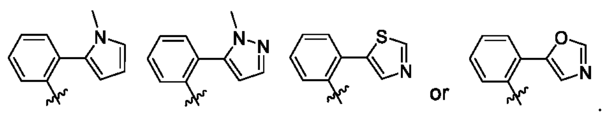
or
.

21. The compound of Formula (I) or salts, enantiomers, diastereoisomer, isotopically enriched analogs, solvates, prodrugs, or polymorphs thereof as claimed in any one of claims 1-17, wherein $-X_1-X_2-R_{a1}$ represents the following structures:

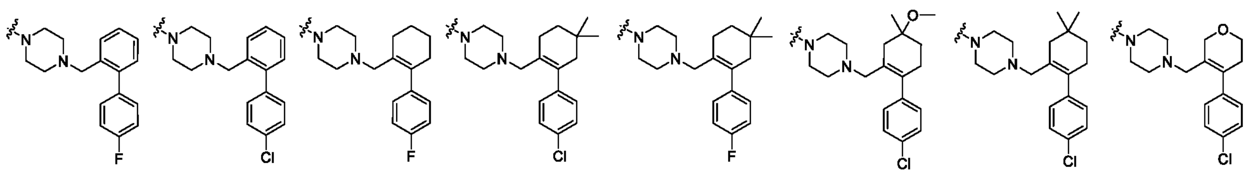

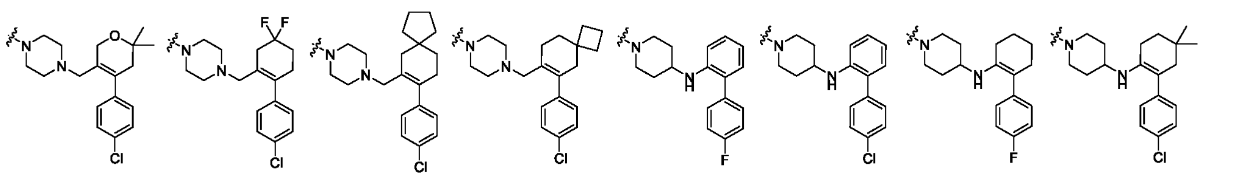

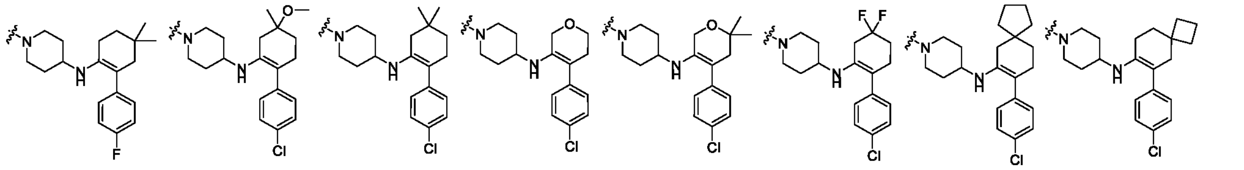

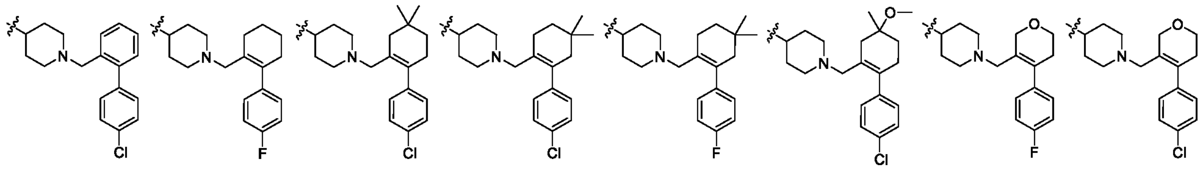

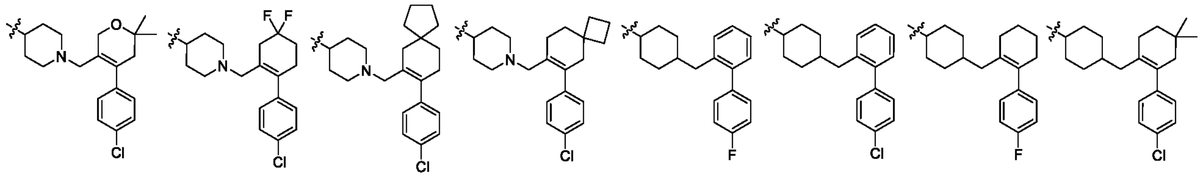

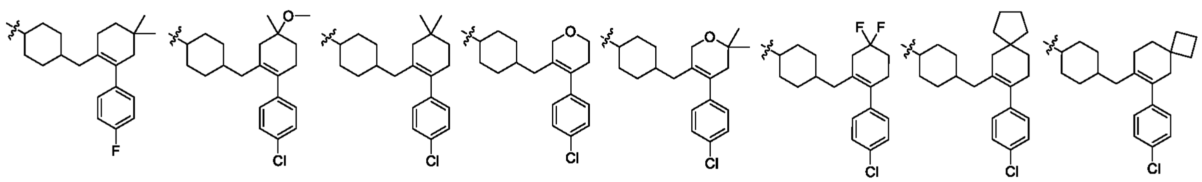

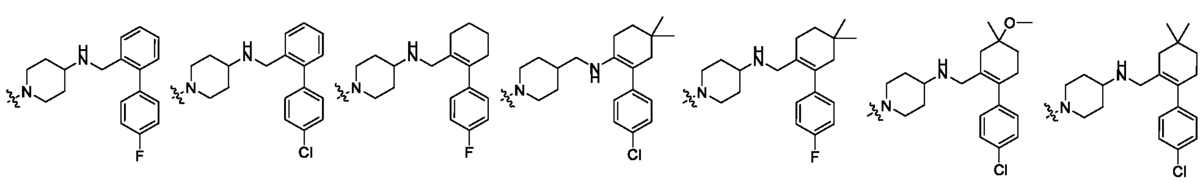

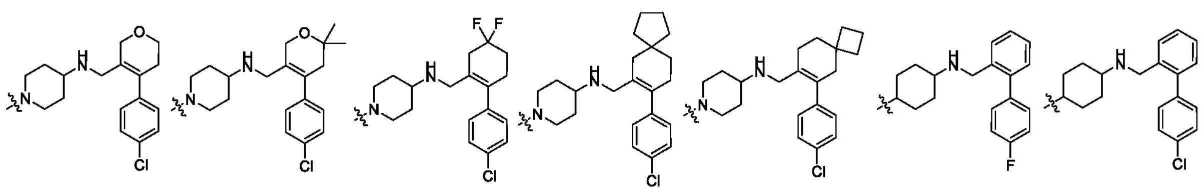

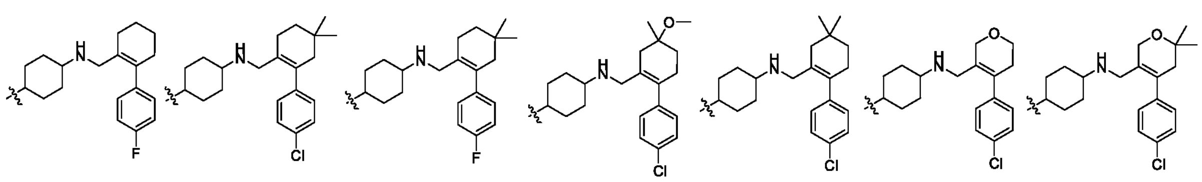

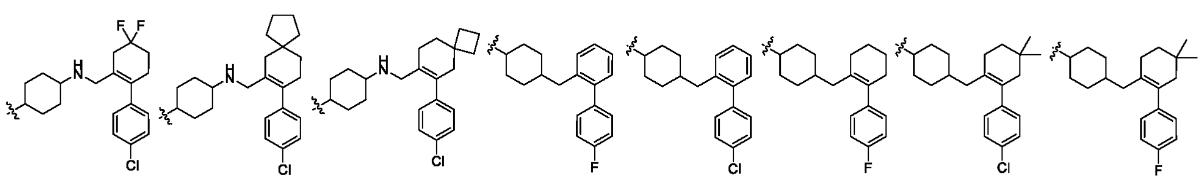

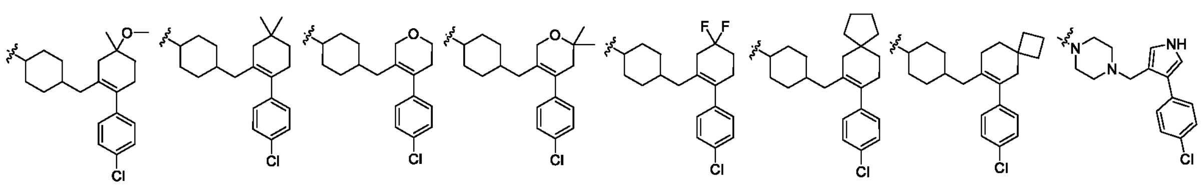

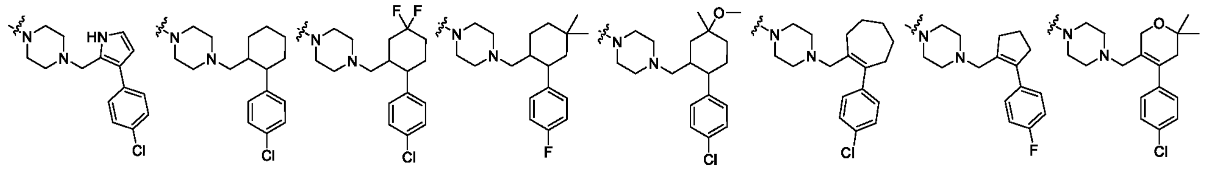

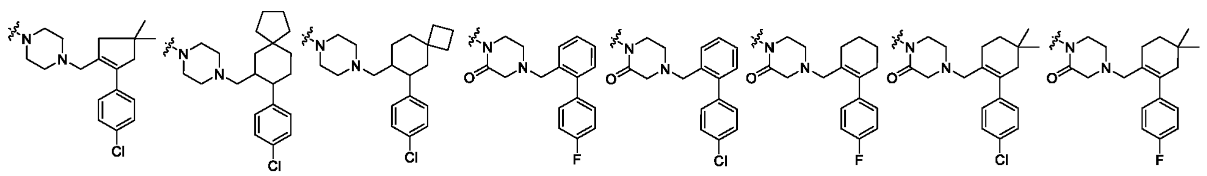

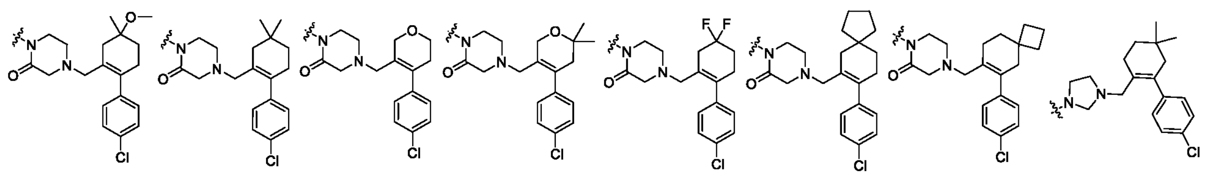

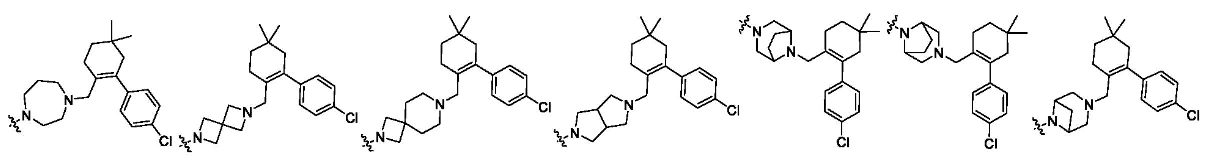

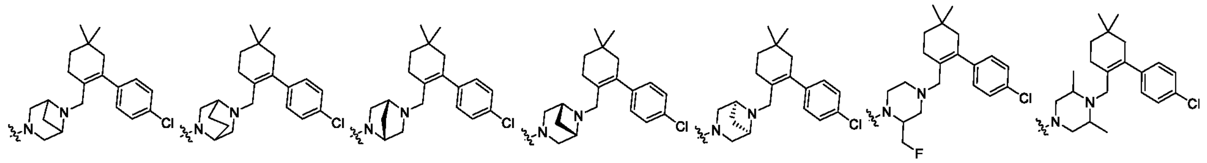

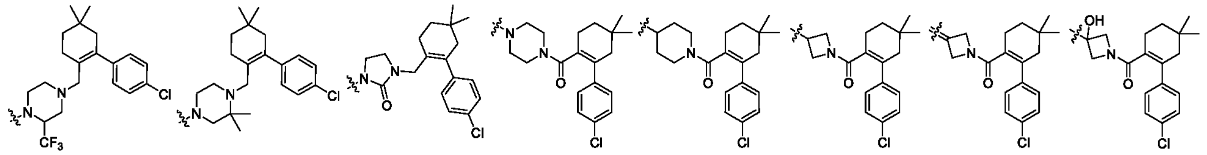

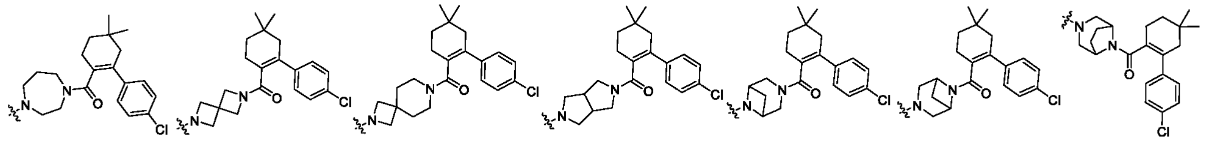

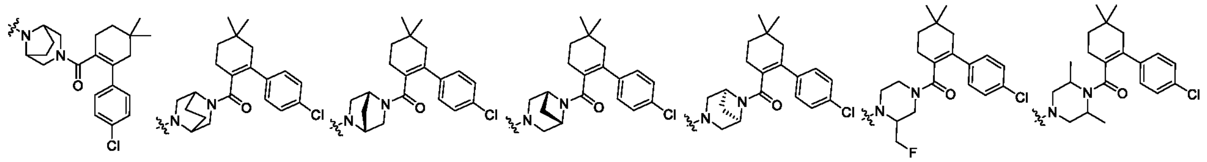

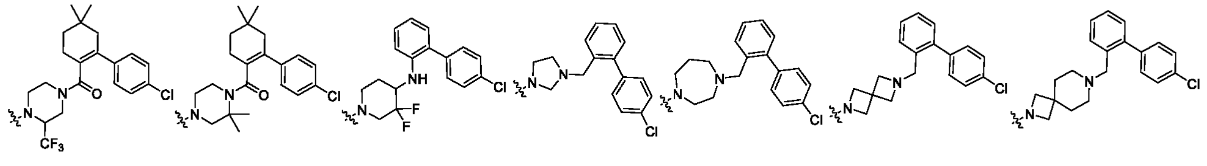

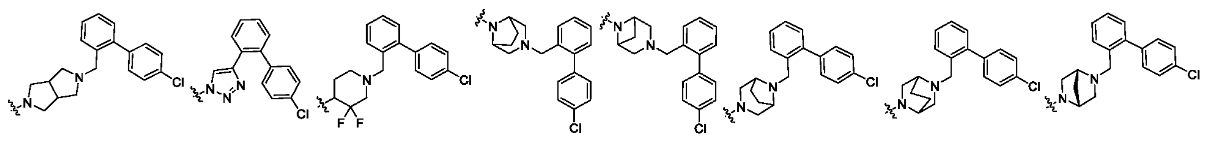

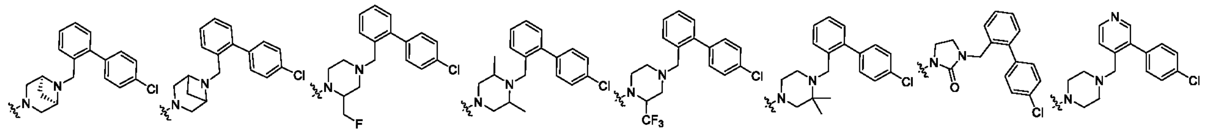

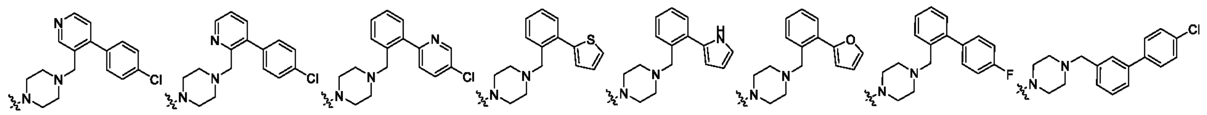

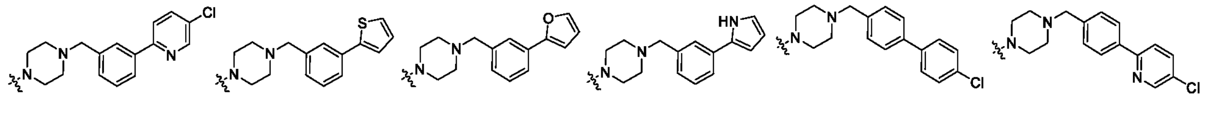

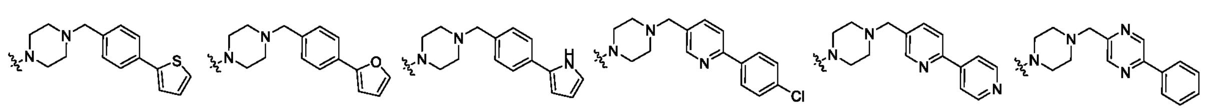

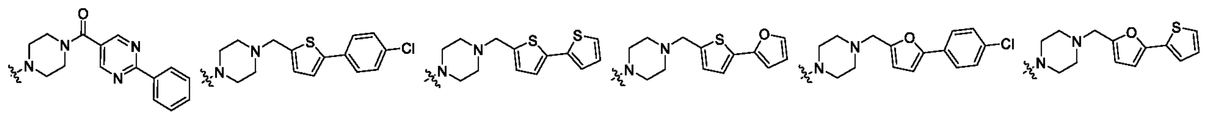

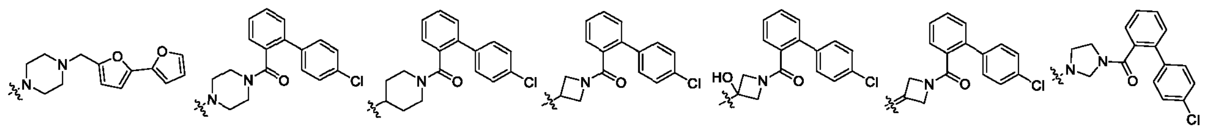

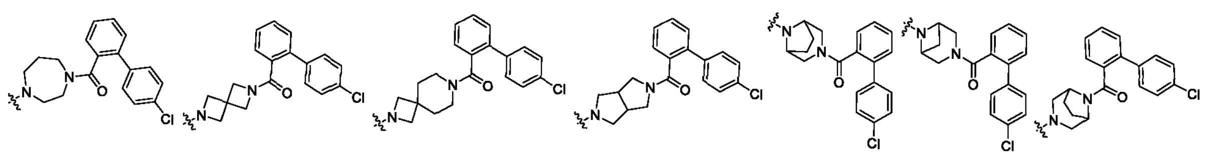

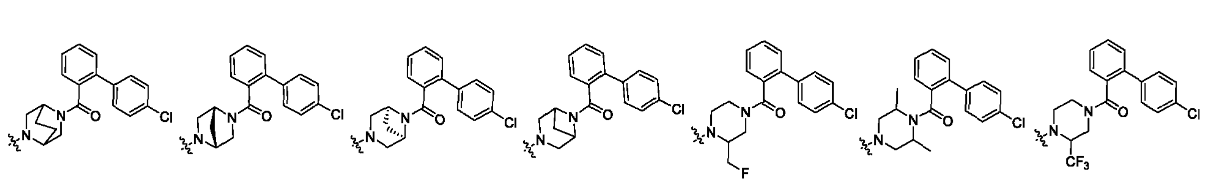

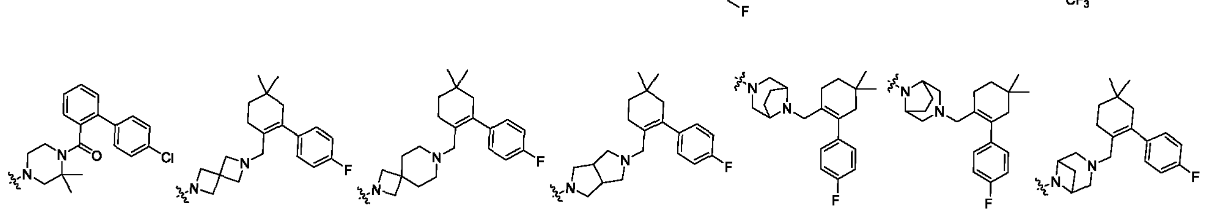

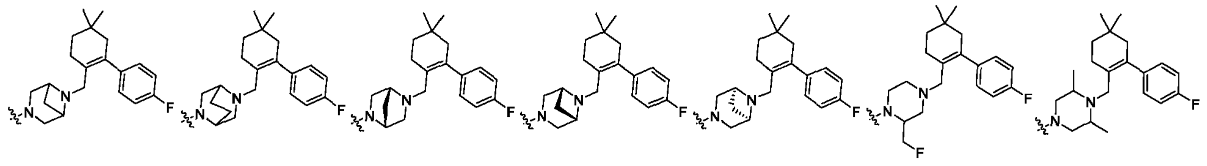

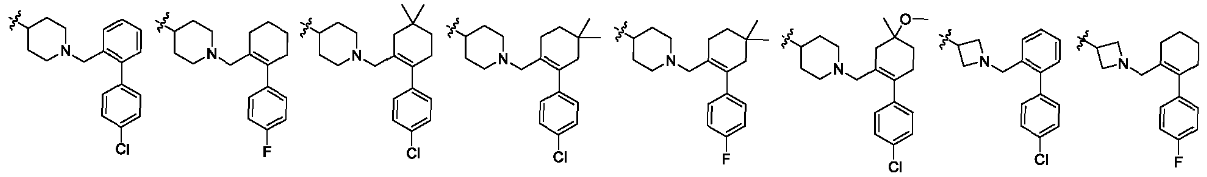

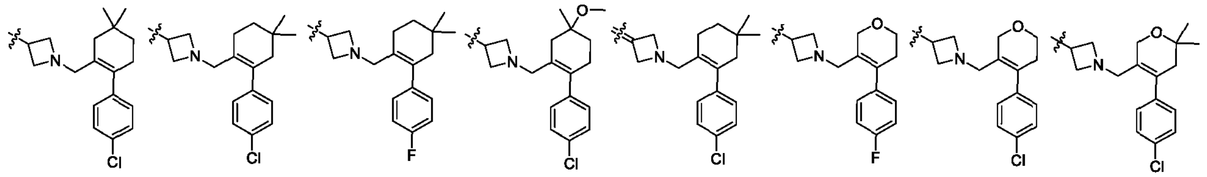

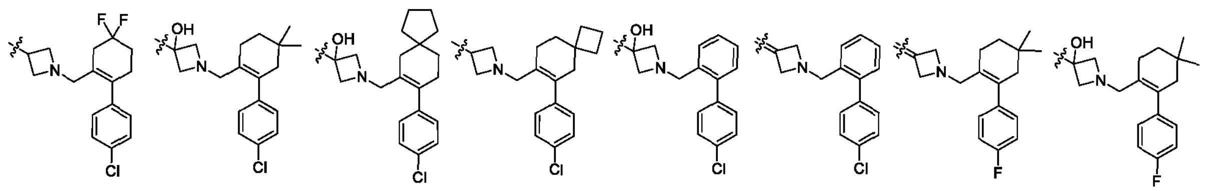

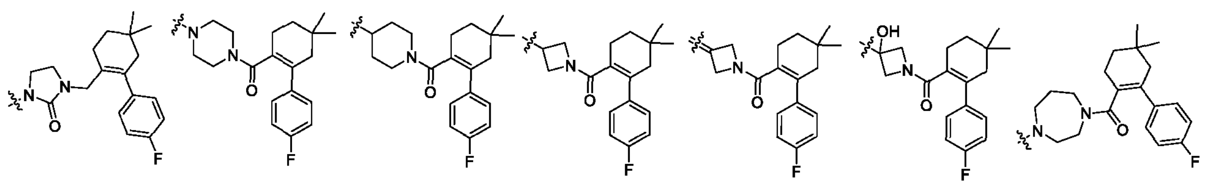

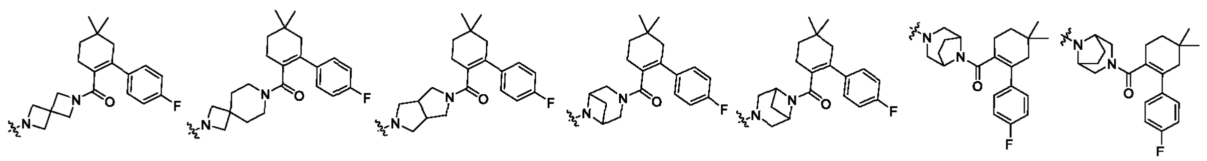

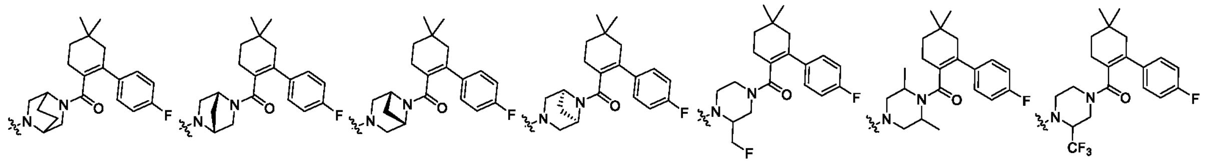

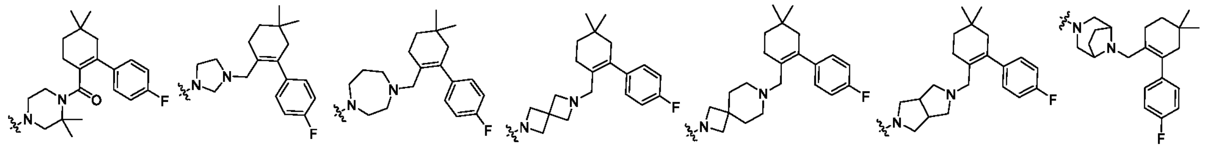

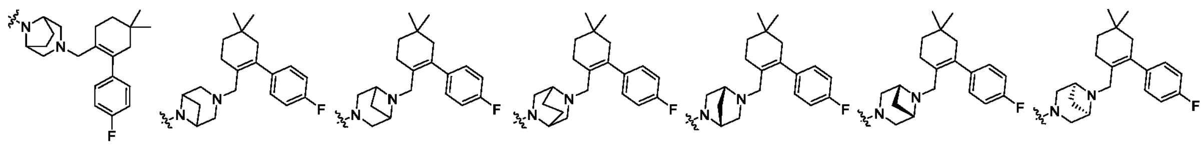

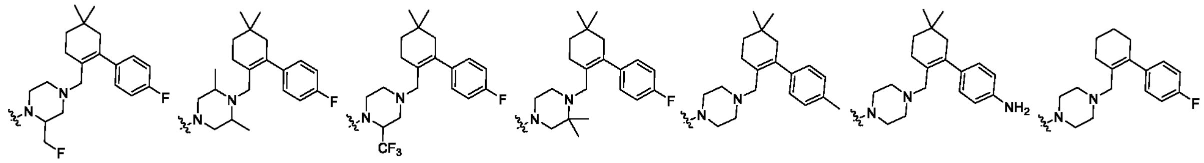

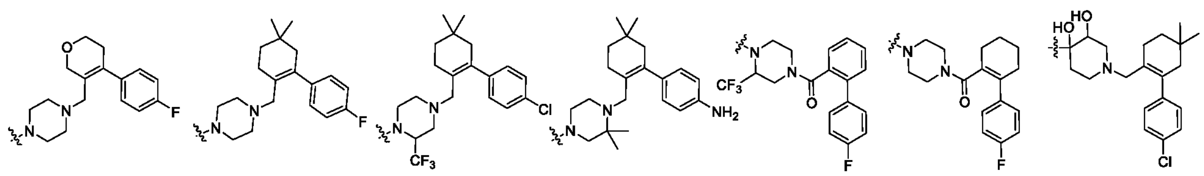

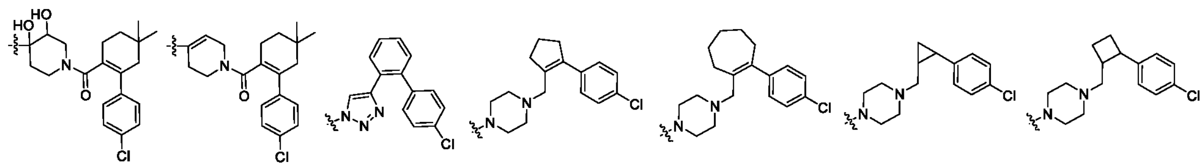

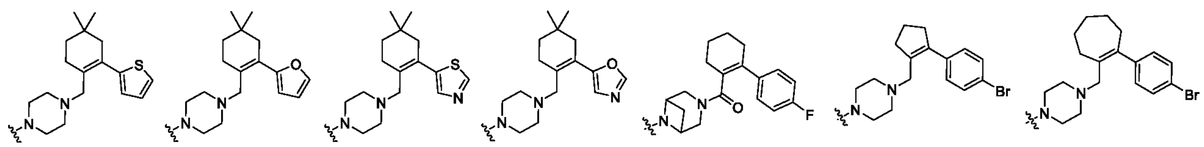

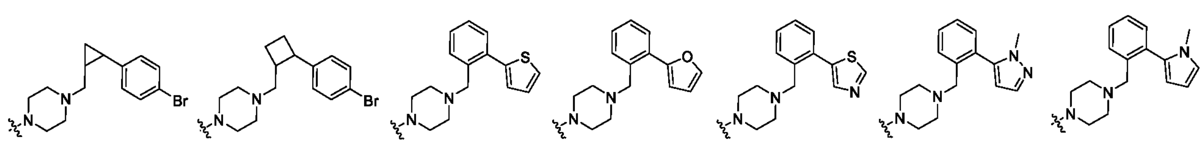

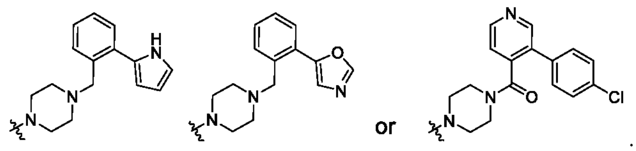

or

22. The compound of Formula (I) or salts, enantiomers, diastereoisomer, isotopically enriched analogs, solvates, prodrugs, or polymorphs thereof as claimed in claim 1, which is selected from the group consisting of:

3-(5-((4-((4'-chloro-5,5-dimethyl-3,4,5,6-tetrahydro-[1,1'-biphenyl]-2-yl)methyl)piperazin-1-yl)methyl)-1-oxoisoindolin-2-yl)piperidine-2,6-dione;
3-(5-((4-((4'-chloro-5,5-dimethyl-3,4,5,6-tetrahydro-[1,1'-biphenyl]-2-yl)methyl)piperazin-1-yl)methyl)-4-fluoro-1-oxoisoindolin-2-yl)piperidine-2,6-dione;
3-(5-((4-((4'-chloro-5,5-dimethyl-3,4,5,6-tetrahydro-[1,1'-biphenyl]-2-yl)methyl)piperazin-1-yl)methyl)-6-fluoro-1-oxoisoindolin-2-yl)piperidine-2,6-dione;
3-(5-((4-((4'-chloro-5,5-dimethyl-3,4,5,6-tetrahydro-[1,1'-biphenyl]-2-yl)methyl)piperazin-1-yl)methyl)-7-fluoro-1-oxoisoindolin-2-yl)piperidine-2,6-dione;
3-(4-((4-((4'-chloro-5,5-dimethyl-3,4,5,6-tetrahydro-[1,1'-biphenyl]-2-yl)methyl)piperazin-1-yl)methyl)-1-oxoisoindolin-2-yl)piperidine-2,6-dione;
3-(6-((4-((4'-chloro-5,5-dimethyl-3,4,5,6-tetrahydro-[1,1'-biphenyl]-2-yl)methyl)piperazin-1-yl)methyl)-1-oxoisoindolin-2-yl)piperidine-2,6-dione;

3-(7-((4-((4'-chloro-5,5-dimethyl-3,4,5,6-tetrahydro-[1,1'-biphenyl]-2-yl)methyl)piperazin-1-yl)methyl)-1-oxoisoindolin-2-yl)piperidine-2,6-dione;
3-(5-(2-(4-((4'-chloro-5,5-dimethyl-3,4,5,6-tetrahydro-[1,1'-biphenyl]-2-yl)methyl)piperazin-1-yl)ethyl)-1-oxoisoindolin-2-yl)piperidine-2,6-dione;
3-(5-(3-(4-((4'-chloro-5,5-dimethyl-3,4,5,6-tetrahydro-[1,1'-biphenyl]-2-yl)methyl)piperazin-1-yl)propyl)-1-oxoisoindolin-2-yl)piperidine-2,6-dione;
3-(5-((4-((4'-chloro-5,5-dimethyl-3,4,5,6-tetrahydro-[1,1'-biphenyl]-2-yl)methyl)piperazin-1-yl)fluoromethyl)-1-oxoisoindolin-2-yl)piperidine-2,6-dione;
3-(5-((4-((4'-chloro-5,5-dimethyl-3,4,5,6-tetrahydro-[1,1'-biphenyl]-2-yl)methyl)piperazin-1-yl)difluoromethyl)-1-oxoisoindolin-2-yl)piperidine-2,6-dione;
3-(5-(1-(4-((4'-chloro-5,5-dimethyl-3,4,5,6-tetrahydro-[1,1'-biphenyl]-2-yl)methyl)piperazin-1-yl)cyclopropyl)-1-oxoisoindolin-2-yl)piperidine-2,6-dione;
3-(5-(1-(4-((4'-chloro-5,5-dimethyl-3,4,5,6-tetrahydro-[1,1'-biphenyl]-2-yl)methyl)piperazin-1-yl)cyclobutyl)-1-oxoisoindolin-2-yl)piperidine-2,6-dione;
3-(5-(1-(4-((4'-chloro-5,5-dimethyl-3,4,5,6-tetrahydro-[1,1'-biphenyl]-2-yl)methyl)piperazin-1-yl)cyclopentyl)-1-oxoisoindolin-2-yl)piperidine-2,6-dione;
3-(5-((4-((2-(4-chlorophenyl)cyclopent-1-en-1-yl)methyl)piperazin-1-yl)methyl)-1-oxoisoindolin-2-yl)piperidine-2,6-dione;
3-(5-((4-((2-(4-chlorophenyl)cyclohept-1-en-1-yl)methyl)piperazin-1-yl)methyl)-1-oxoisoindolin-2-yl)piperidine-2,6-dione;
3-(5-((4-((2-(4-chlorophenyl)cyclopropyl)methyl)piperazin-1-yl)methyl)-1-oxoisoindolin-2-yl)piperidine-2,6-dione;
3-(5-((4-((2-(4-chlorophenyl)cyclobutyl)methyl)piperazin-1-yl)methyl)-1-oxoisoindolin-2-yl)piperidine-2,6-dione;
3-(5-((4-((4,4-dimethyl-2-(thiophen-2-yl)cyclohex-1-en-1-yl)methyl)piperazin-1-yl)methyl)-1-oxoisoindolin-2-yl)piperidine-2,6-dione;
3-(5-((4-((2-(furan-2-yl)-4,4-dimethylcyclohex-1-en-1-yl)methyl)piperazin-1-yl)methyl)-1-oxoisoindolin-2-yl)piperidine-2,6-dione;
3-(5-((4-((4,4-dimethyl-2-(thiazol-5-yl)cyclohex-1-en-1-yl)methyl)piperazin-1-yl)methyl)-1-oxoisoindolin-2-yl)piperidine-2,6-dione;
3-(5-((4-((4,4-dimethyl-2-(oxazol-5-yl)cyclohex-1-en-1-yl)methyl)piperazin-1-yl)methyl)-1-oxoisoindolin-2-yl)piperidine-2,6-dione;
3-(5-((4-((4'-chloro-5,5-dimethyl-3,4,5,6-tetrahydro-[1,1'-biphenyl]-2-yl)amino)piperidin-1-yl)methyl)-1-oxoisoindolin-2-yl)piperidine-2,6-dione;
3-(5-((1-((4'-chloro-5,5-dimethyl-3,4,5,6-tetrahydro-[1,1'-biphenyl]-2-yl)methyl)piperidin-4-yl)methyl)-1-oxoisoindolin-2-yl)piperidine-2,6-dione;
3-(5-((1-((4'-chloro-5,5-dimethyl-3,4,5,6-tetrahydro-[1,1'-biphenyl]-2-yl)methyl)piperidin-4-yl)amino)-1-oxoisoindolin-2-yl)piperidine-2,6-dione;
3-(5-((1-((4'-chloro-5,5-dimethyl-3,4,5,6-tetrahydro-[1,1'-biphenyl]-2-yl)methyl)azetidin-3-yl)methyl)-1-oxoisoindolin-2-yl)piperidine-2,6-dione;
3-(5-((1-((4'-chloro-5,5-dimethyl-3,4,5,6-tetrahydro-[1,1'-biphenyl]-2-yl)methyl)azetidin-3-ylidene)methyl)-1-oxoisoindolin-2-yl)piperidine-2,6-dione;
3-(5-((1-((4'-chloro-5,5-dimethyl-3,4,5,6-tetrahydro-[1,1'-biphenyl]-2-yl)methyl)-3-hydroxyazetidin-3-yl)(hydroxy)methyl)-1-oxoisoindolin-2-yl)piperidine-2,6-dione;
3-(5-(4-((4-((4'-chloro-5,5-dimethyl-3,4,5,6-tetrahydro-[1,1'-biphenyl]-2-yl)methyl)piperazin-1-yl)methyl)phenyl)-1-oxoisoindolin-2-yl)piperidine-2,6-dione;
3-(5-((4-((4'-chloro-5,5-dimethyl-3,4,5,6-tetrahydro-[1,1'-biphenyl]-2-yl)methyl)piperazin-1-yl)amino)-1-oxoisoindolin-2-yl)piperidine-2,6-dione;
3-(5-((4-((4'-chloro-5,5-dimethyl-3,4,5,6-tetrahydro-[1,1'-biphenyl]-2-yl)methyl)-2-oxopiperazin-1-yl)methyl)-1-oxoisoindolin-2-yl)piperidine-2,6-dione;
3-(5-((3-((4'-chloro-5,5-dimethyl-3,4,5,6-tetrahydro-[1,1'-biphenyl]-2-yl)methyl)imidazolidin-1-yl)methyl)-1-oxoisoindolin-2-yl)piperidine-2,6-dione;
3-(5-((4-((4'-chloro-5,5-dimethyl-3,4,5,6-tetrahydro-[1,1'-biphenyl]-2-yl)methyl)-1,4-diazepan-1-yl)methyl)-1-oxoisoindolin-2-yl)piperidine-2,6-dione;
3-(5-((6-((4'-chloro-5,5-dimethyl-3,4,5,6-tetrahydro-[1,1'-biphenyl]-2-yl)methyl)-2,6-diazaspiro[3.3]heptan-2-yl)methyl)-1-oxoisoindolin-2-yl)piperidine-2,6-dione;
3-(5-((7-((4'-chloro-5,5-dimethyl-3,4,5,6-tetrahydro-[1,1'-biphenyl]-2-yl)methyl)-2,7-diazaspiro[3.5]nonan-2-yl)methyl)-1-oxoisoindolin-2-yl)piperidine-2,6-dione;

3-(5-((5-((4'-chloro-5,5-dimethyl-3,4,5,6-tetrahydro-[1,1'-biphenyl]-2-yl)methyl)hexahydropyrrolo[3,4-c]pyrrol-2(1H)-yl)methyl)-1-oxoisoindolin-2-yl)piperidine-2,6-dione;
3-(5-(8-((4'-chloro-5,5-dimethyl-3,4,5,6-tetrahydro-[1,1'-biphenyl]-2-yl)methyl)-3,8-diazabicyclo[3.2.1]octan-3-yl)-1-oxoisoindolin-2-yl)piperidine-2,6-dione;
3-(5-(3-((4'-chloro-5,5-dimethyl-3,4,5,6-tetrahydro-[1,1'-biphenyl]-2-yl)methyl)-3,8-diazabicyclo[3.2.1]octan-8-yl)-1-oxoisoindolin-2-yl)piperidine-2,6-dione;
3-(5-((3-((4'-chloro-5,5-dimethyl-3,4,5,6-tetrahydro-[1,1'-biphenyl]-2-yl)methyl)-3,6-diazabicyclo[3.1.1]heptan-6-yl)methyl)-1-oxoisoindolin-2-yl)piperidine-2,6-dione;
3-(5-((6-((4'-chloro-5,5-dimethyl-3,4,5,6-tetrahydro-[1,1'-biphenyl]-2-yl)methyl)-3,6-diazabicyclo[3.1.1]heptan-3-yl)methyl)-1-oxoisoindolin-2-yl)piperidine-2,6-dione;
3-(5-(((1R,5S)-6-((4'-chloro-5,5-dimethyl-3,4,5,6-tetrahydro-[1,1'-biphenyl]-2-yl)methyl)-3,6-diazabicyclo[3.1.1]heptan-3-yl)methyl)-1-oxoisoindolin-2-yl)piperidine-2,6-dione;
3-(5-((3-((4'-chloro-5,5-dimethyl-3,4,5,6-tetrahydro-[1,1'-biphenyl]-2-yl)methyl)-3,8-diazabicyclo[3.2.1]octan-8-yl)methyl)-1-oxoisoindolin-2-yl)piperidine-2,6-dione;
3-(5-((8-((4'-chloro-5,5-dimethyl-3,4,5,6-tetrahydro-[1,1'-biphenyl]-2-yl)methyl)-3,8-diazabicyclo[3.2.1]octan-3-yl)methyl)-1-oxoisoindolin-2-yl)piperidine-2,6-dione;
3-(5-((5-((4'-chloro-5,5-dimethyl-3,4,5,6-tetrahydro-[1,1'-biphenyl]-2-yl)methyl)-2,5-diazabicyclo[2.2.2]octan-2-yl)methyl)-1-oxoisoindolin-2-yl)piperidine-2,6-dione;
3-(5-(((1R,4R)-5-((4'-chloro-5,5-dimethyl-3,4,5,6-tetrahydro-[1,1'-biphenyl]-2-yl)methyl)-2,5-diazabicyclo[2.2.1]heptan-2-yl)methyl)-1-oxoisoindolin-2-yl)piperidine-2,6-dione;
3-(5-((4-((4'-chloro-5,5-dimethyl-3,4,5,6-tetrahydro-[1,1'-biphenyl]-2-yl)methyl)-2-(fluoromethyl)piperazin-1-yl)methyl)-1-oxoisoindolin-2-yl)piperidine-2,6-dione;
3-(5-((4-((4'-chloro-5,5-dimethyl-3,4,5,6-tetrahydro-[1,1'-biphenyl]-2-yl)methyl)-3,5-dimethylpiperazin-1-yl)methyl)-1-oxoisoindolin-2-yl)piperidine-2,6-dione;
3-(5-((4-((4'-chloro-5,5-dimethyl-3,4,5,6-tetrahydro-[1,1'-biphenyl]-2-yl)methyl)-2-(trifluoromethyl)piperazin-1-yl)methyl)-1-oxoisoindolin-2-yl)piperidine-2,6-dione;
3-(5-((4-((4'-chloro-5,5-dimethyl-3,4,5,6-tetrahydro-[1,1'-biphenyl]-2-yl)methyl)-3,3-dimethylpiperazin-1-yl)methyl)-1-oxoisoindolin-2-yl)piperidine-2,6-dione;
3-(5-((3-((4'-chloro-5,5-dimethyl-3,4,5,6-tetrahydro-[1,1'-biphenyl]-2-yl)methyl)-3,6-diazabicyclo[3.1.1]heptan-6-yl)methyl)-4-fluoro-1-oxoisoindolin-2-yl)piperidine-2,6-dione;
3-(5-((6-((4'-chloro-5,5-dimethyl-3,4,5,6-tetrahydro-[1,1'-biphenyl]-2-yl)methyl)-3,6-diazabicyclo[3.1.1]heptan-3-yl)methyl)-4-fluoro-1-oxoisoindolin-2-yl)piperidine-2,6-dione;
3-(5-((3-((4'-chloro-5,5-dimethyl-3,4,5,6-tetrahydro-[1,1'-biphenyl]-2-yl)methyl)-3,8-diazabicyclo[3.2.1]octan-8-yl)methyl)-4-fluoro-1-oxoisoindolin-2-yl)piperidine-2,6-dione;
3-(5-((8-((4'-chloro-5,5-dimethyl-3,4,5,6-tetrahydro-[1,1'-biphenyl]-2-yl)methyl)-3,8-diazabicyclo[3.2.1]octan-3-yl)methyl)-4-fluoro-1-oxoisoindolin-2-yl)piperidine-2,6-dione;
3-(5-((5-((4'-chloro-5,5-dimethyl-3,4,5,6-tetrahydro-[1,1'-biphenyl]-2-yl)methyl)-2,5-diazabicyclo[2.2.2]octan-2-yl)methyl)-4-fluoro-1-oxoisoindolin-2-yl)piperidine-2,6-dione;
3-(5-(((1R,4R)-5-((4'-chloro-5,5-dimethyl-3,4,5,6-tetrahydro-[1,1'-biphenyl]-2-yl)methyl)-2,5-diazabicyclo[2.2.1]heptan-2-yl)methyl)-4-fluoro-1-oxoisoindolin-2-yl)piperidine-2,6-dione;
3-(5-((4-((4'-chloro-5,5-dimethyl-3,4,5,6-tetrahydro-[1,1'-biphenyl]-2-yl)methyl)-2-(fluoromethyl)piperazin-1-yl)methyl)-4-fluoro-1-oxoisoindolin-2-yl)piperidine-2,6-dione;
3-(5-((4-((4'-chloro-5,5-dimethyl-3,4,5,6-tetrahydro-[1,1'-biphenyl]-2-yl)methyl)-3,5-dimethylpiperazin-1-yl)methyl)-4-fluoro-1-oxoisoindolin-2-yl)piperidine-2,6-dione;
3-(5-((4-((4'-chloro-5,5-dimethyl-3,4,5,6-tetrahydro-[1,1'-biphenyl]-2-yl)methyl)-2-(trifluoromethyl)piperazin-1-yl)methyl)-4-fluoro-1-oxoisoindolin-2-yl)piperidine-2,6-dione;
3-(5-((4-((4'-chloro-5,5-dimethyl-3,4,5,6-tetrahydro-[1,1'-biphenyl]-2-yl)methyl)-3,3-dimethylpiperazin-1-yl)methyl)-4-fluoro-1-oxoisoindolin-2-yl)piperidine-2,6-dione;
3-(5-((3-((4'-chloro-5,5-dimethyl-3,4,5,6-tetrahydro-[1,1'-biphenyl]-2-yl)methyl)-3,6-diazabicyclo[3.1.1]heptan-6-yl)methyl)-6-fluoro-1-oxoisoindolin-2-yl)piperidine-2,6-dione;
3-(5-((6-((4'-chloro-5,5-dimethyl-3,4,5,6-tetrahydro-[1,1'-biphenyl]-2-yl)methyl)-3,6-diazabicyclo[3.1.1]heptan-3-yl)methyl)-6-fluoro-1-oxoisoindolin-2-yl)piperidine-2,6-dione;
3-(5-((3-((4'-chloro-5,5-dimethyl-3,4,5,6-tetrahydro-[1,1'-biphenyl]-2-yl)methyl)-3,8-diazabicyclo[3.2.1]octan-8-yl)methyl)-6-fluoro-1-oxoisoindolin-2-yl)piperidine-2,6-dione;
3-(5-((8-((4'-chloro-5,5-dimethyl-3,4,5,6-tetrahydro-[1,1'-biphenyl]-2-yl)methyl)-3,8-diazabicyclo[3.2.1]octan-3-yl)methyl)-6-fluoro-1-oxoisoindolin-2-yl)piperidine-2,6-dione;
3-(5-((5-((4'-chloro-5,5-dimethyl-3,4,5,6-tetrahydro-[1,1'-biphenyl]-2-yl)methyl)-2,5-diazabicyclo[2.2.2]octan-2-yl)methyl)-6-fluoro-1-oxoisoindolin-2-yl)piperidine-2,6-dione;

3-(5-((4-((4'-chloro-5,5-dimethyl-3,4,5,6-tetrahydro-[1,1'-biphenyl]-2-yl)methyl)-2-(fluoromethyl)piperazin-1-yl)methyl)-6-fluoro-1-oxoisoindolin-2-yl)piperidine-2,6-dione;
3-(5-((4-((4'-chloro-5,5-dimethyl-3,4,5,6-tetrahydro-[1,1'-biphenyl]-2-yl)methyl)-3,5-dimethylpiperazin-1-yl)methyl)-6-fluoro-1-oxoisoindolin-2-yl)piperidine-2,6-dione;
3-(5-((4-((4'-chloro-5,5-dimethyl-3,4,5,6-tetrahydro-[1,1'-biphenyl]-2-yl)methyl)-2-(trifluoromethyl)piperazin-1-yl)methyl)-6-fluoro-1-oxoisoindolin-2-yl)piperidine-2,6-dione;
3-(5-((4-((4'-chloro-5,5-dimethyl-3,4,5,6-tetrahydro-[1,1'-biphenyl]-2-yl)methyl)-3,3-dimethylpiperazin-1-yl)methyl)-6-fluoro-1-oxoisoindolin-2-yl)piperidine-2,6-dione;
3-(5-((3-((4'-chloro-5,5-dimethyl-3,4,5,6-tetrahydro-[1,1'-biphenyl]-2-yl)methyl)-3,6-diazabicyclo[3.1.1]heptan-6-yl)methyl)-7-fluoro-1-oxoisoindolin-2-yl)piperidine-2,6-dione;
3-(5-((6-((4'-chloro-5,5-dimethyl-3,4,5,6-tetrahydro-[1,1'-biphenyl]-2-yl)methyl)-3,6-diazabicyclo[3.1.1]heptan-3-yl)methyl)-7-fluoro-1-oxoisoindolin-2-yl)piperidine-2,6-dione;
3-(5-((3-((4'-chloro-5,5-dimethyl-3,4,5,6-tetrahydro-[1,1'-biphenyl]-2-yl)methyl)-3,8-diazabicyclo[3.2.1]octan-8-yl)methyl)-7-fluoro-1-oxoisoindolin-2-yl)piperidine-2,6-dione;
3-(5-((8-((4'-chloro-5,5-dimethyl-3,4,5,6-tetrahydro-[1,1'-biphenyl]-2-yl)methyl)-3,8-diazabicyclo[3.2.1]octan-3-yl)methyl)-7-fluoro-1-oxoisoindolin-2-yl)piperidine-2,6-dione;
3-(5-((5-((4'-chloro-5,5-dimethyl-3,4,5,6-tetrahydro-[1,1'-biphenyl]-2-yl)methyl)-2,5-diazabicyclo[2.2.2]octan-2-yl)methyl)-7-fluoro-1-oxoisoindolin-2-yl)piperidine-2,6-dione;
3-(5-((4-((4'-chloro-5,5-dimethyl-3,4,5,6-tetrahydro-[1,1'-biphenyl]-2-yl)methyl)-2-(fluoromethyl)piperazin-1-yl)methyl)-7-fluoro-1-oxoisoindolin-2-yl)piperidine-2,6-dione;
3-(5-((4-((4'-chloro-5,5-dimethyl-3,4,5,6-tetrahydro-[1,1'-biphenyl]-2-yl)methyl)-3,5-dimethylpiperazin-1-yl)methyl)-7-fluoro-1-oxoisoindolin-2-yl)piperidine-2,6-dione;
3-(5-((4-((4'-chloro-5,5-dimethyl-3,4,5,6-tetrahydro-[1,1'-biphenyl]-2-yl)methyl)-2-(trifluoromethyl)piperazin-1-yl)methyl)-7-fluoro-1-oxoisoindolin-2-yl)piperidine-2,6-dione;
3-(5-((4-((4'-chloro-5,5-dimethyl-3,4,5,6-tetrahydro-[1,1'-biphenyl]-2-yl)methyl)-3,3-dimethylpiperazin-1-yl)methyl)-7-fluoro-1-oxoisoindolin-2-yl)piperidine-2,6-dione;
3-(5-((3-((4'-chloro-5,5-dimethyl-3,4,5,6-tetrahydro-[1,1'-biphenyl]-2-yl)methyl)-2-oxoimidazolidin-1-yl)methyl)-1-oxoisoindolin-2-yl)piperidine-2,6-dione;
3-(5-((3-((4'-chloro-5,5-dimethyl-3,4,5,6-tetrahydro-[1,1'-biphenyl]-2-yl)methyl)-2-oxoimidazolidin-1-yl)methyl)-4-fluoro-1-oxoisoindolin-2-yl)piperidine-2,6-dione;
3-(5-((3-((4'-chloro-5,5-dimethyl-3,4,5,6-tetrahydro-[1,1'-biphenyl]-2-yl)methyl)-2-oxoimidazolidin-1-yl)methyl)-6-fluoro-1-oxoisoindolin-2-yl)piperidine-2,6-dione;
3-(5-((3-((4'-chloro-5,5-dimethyl-3,4,5,6-tetrahydro-[1,1'-biphenyl]-2-yl)methyl)-2-oxoimidazolidin-1-yl)methyl)-7-fluoro-1-oxoisoindolin-2-yl)piperidine-2,6-dione;
3-(4-((3-((4'-chloro-5,5-dimethyl-3,4,5,6-tetrahydro-[1,1'-biphenyl]-2-yl)methyl)-2-oxoimidazolidin-1-yl)methyl)-1-oxoisoindolin-2-yl)piperidine-2,6-dione;
3-(5-((4-((4-(4-chlorophenyl)-6,6-dimethyl-5,6-dihydro-2H-pyran-3-yl)methyl)piperazin-1-yl)methyl)-1-oxoisoindolin-2-yl)piperidine-2,6-dione;
3-(5-((4-(4'-chloro-5,5-dimethyl-3,4,5,6-tetrahydro-[1,1'-biphenyl]-2-carbonyl)piperazin-1-yl)methyl)-1-oxoisoindolin-2-yl)piperidine-2,6-dione;
3-(5-((4-(4'-chloro-5,5-dimethyl-3,4,5,6-tetrahydro-[1,1'-biphenyl]-2-carbonyl)piperazin-1-yl)methyl)-4-fluoro-1-oxoisoindolin-2-yl)piperidine-2,6-dione;
3-(5-((4-(4'-chloro-5,5-dimethyl-3,4,5,6-tetrahydro-[1,1'-biphenyl]-2-carbonyl)piperazin-1-yl)methyl)-6-fluoro-1-oxoisoindolin-2-yl)piperidine-2,6-dione;
3-(5-((4-(4'-chloro-5,5-dimethyl-3,4,5,6-tetrahydro-[1,1'-biphenyl]-2-carbonyl)piperazin-1-yl)methyl)-7-fluoro-1-oxoisoindolin-2-yl)piperidine-2,6-dione;
3-(4-((4-(4'-chloro-5,5-dimethyl-3,4,5,6-tetrahydro-[1,1'-biphenyl]-2-carbonyl)piperazin-1-yl)methyl)-1-oxoisoindolin-2-yl)piperidine-2,6-dione;
3-(5-((4-(4'-chloro-5,5-dimethyl-3,4,5,6-tetrahydro-[1,1'-biphenyl]-2-carbonyl)piperazin-1-yl)amino)-1-oxoisoindolin-2-yl)piperidine-2,6-dione;
3-(5-((1-(4'-chloro-5,5-dimethyl-3,4,5,6-tetrahydro-[1,1'-biphenyl]-2-carbonyl)piperidin-4-yl)methyl)-1-oxoisoindolin-2-yl)piperidine-2,6-dione;
3-(5-((1-(4'-chloro-5,5-dimethyl-3,4,5,6-tetrahydro-[1,1'-biphenyl]-2-carbonyl)piperidin-4-yl)amino)-1-oxoisoindolin-2-yl)piperidine-2,6-dione;
3-(5-((1-(4'-chloro-5,5-dimethyl-3,4,5,6-tetrahydro-[1,1'-biphenyl]-2-carbonyl)azetidin-3-yl)methyl)-1-oxoisoindolin-2-yl)piperidine-2,6-dione;
3-(5-((1-(4'-chloro-5,5-dimethyl-3,4,5,6-tetrahydro-[1,1'-biphenyl]-2-carbonyl)azetidin-3-ylidene)methyl)-1-oxoisoindolin-2-yl)piperidine-2,6-dione;

3-(5-((1-(4'-chloro-5,5-dimethyl-3,4,5,6-tetrahydro-[1,1'-biphenyl]-2-carbonyl)-3-hydroxyazetidin-3-yl)(hydroxy)methyl)-1-oxoisoindolin-2-yl)piperidine-2,6-dione;
3-(5-((3-(4'-chloro-5,5-dimethyl-3,4,5,6-tetrahydro-[1,1'-biphenyl]-2-carbonyl)imidazolidin-1-yl)methyl)-1-oxoisoindolin-2-yl)piperidine-2,6-dione;
3-(5-((4-(4'-chloro-5,5-dimethyl-3,4,5,6-tetrahydro-[1,1'-biphenyl]-2-carbonyl)-1,4-diazepan-1-yl)methyl)-1-oxoisoindolin-2-yl)piperidine-2,6-dione;
3-(5-((7-(4'-chloro-5,5-dimethyl-3,4,5,6-tetrahydro-[1,1'-biphenyl]-2-carbonyl)-2,7-diazaspiro[3.5]nonan-2-yl)methyl)-1-oxoisoindolin-2-yl)piperidine-2,6-dione;
3-(5-((6-(4'-chloro-5,5-dimethyl-3,4,5,6-tetrahydro-[1,1'-biphenyl]-2-carbonyl)-2,6-diazaspiro[3.3]heptan-2-yl)methyl)-1-oxoisoindolin-2-yl)piperidine-2,6-dione;
3-(5-((5-(4'-chloro-5,5-dimethyl-3,4,5,6-tetrahydro-[1,1'-biphenyl]-2-carbonyl)hexahydropyrrolo[3,4-c]pyrrol-2(1H)-yl)methyl)-1-oxoisoindolin-2-yl)piperidine-2,6-dione;
3-(5-(4-((4-(4'-chloro-5,5-dimethyl-3,4,5,6-tetrahydro-[1,1'-biphenyl]-2-carbonyl)piperazin-1-yl)methyl)phenyl)-1-oxoisoindolin-2-yl)piperidine-2,6-dione;
3-(5-((3-(4'-chloro-5,5-dimethyl-3,4,5,6-tetrahydro-[1,1'-biphenyl]-2-carbonyl)-3,6-diazabicyclo[3.1.1]heptan-6-yl)methyl)-1-oxoisoindolin-2-yl)piperidine-2,6-dione;
3-(5-((6-(4'-chloro-5,5-dimethyl-3,4,5,6-tetrahydro-[1,1'-biphenyl]-2-carbonyl)-3,6-diazabicyclo[3.1.1]heptan-3-yl)methyl)-1-oxoisoindolin-2-yl)piperidine-2,6-dione;
3-(5-((3-(4'-chloro-5,5-dimethyl-3,4,5,6-tetrahydro-[1,1'-biphenyl]-2-carbonyl)-3,8-diazabicyclo[3.2.1]octan-8-yl)methyl)-1-oxoisoindolin-2-yl)piperidine-2,6-dione;
3-(5-((8-(4'-chloro-5,5-dimethyl-3,4,5,6-tetrahydro-[1,1'-biphenyl]-2-carbonyl)-3,8-diazabicyclo[3.2.1]octan-3-yl)methyl)-1-oxoisoindolin-2-yl)piperidine-2,6-dione;
3-(5-((5-(4'-chloro-5,5-dimethyl-3,4,5,6-tetrahydro-[1,1'-biphenyl]-2-carbonyl)-2,5-diazabicyclo[2.2.2]octan-2-yl)methyl)-1-oxoisoindolin-2-yl)piperidine-2,6-dione;
3-(5-(((1R,4R)-5-(4'-chloro-5,5-dimethyl-3,4,5,6-tetrahydro-[1,1'-biphenyl]-2-carbonyl)-2,5-diazabicyclo[2.2.1]heptan-2-yl)methyl)-1-oxoisoindolin-2-yl)piperidine-2,6-dione;
3-(5-((4-(4'-chloro-5,5-dimethyl-3,4,5,6-tetrahydro-[1,1'-biphenyl]-2-carbonyl)-2-(fluoromethyl)piperazin-1-yl)methyl)-1-oxoisoindolin-2-yl)piperidine-2,6-dione;
3-(5-((4-(4'-chloro-5,5-dimethyl-3,4,5,6-tetrahydro-[1,1'-biphenyl]-2-carbonyl)-3,5-dimethylpiperazin-1-yl)methyl)-1-oxoisoindolin-2-yl)piperidine-2,6-dione;
3-(5-((4-(4'-chloro-5,5-dimethyl-3,4,5,6-tetrahydro-[1,1'-biphenyl]-2-carbonyl)-2-(trifluoromethyl)piperazin-1-yl)methyl)-1-oxoisoindolin-2-yl)piperidine-2,6-dione;
3-(5-((4-(4'-chloro-5,5-dimethyl-3,4,5,6-tetrahydro-[1,1'-biphenyl]-2-carbonyl)-3,3-dimethylpiperazin-1-yl)methyl)-1-oxoisoindolin-2-yl)piperidine-2,6-dione;
3-(5-(4-((4'-chloro-5,5-dimethyl-3,4,5,6-tetrahydro-[1,1'-biphenyl]-2-yl)methyl)piperazine-1-carbonyl)-1-oxoisoindolin-2-yl)piperidine-2,6-dione;
3-(5-(4-((4'-chloro-5,5-dimethyl-3,4,5,6-tetrahydro-[1,1'-biphenyl]-2-yl)methyl)piperazine-1-carbonyl)-4-fluoro-1-oxoisoindolin-2-yl)piperidine-2,6-dione;
3-(5-(4-((4'-chloro-5,5-dimethyl-3,4,5,6-tetrahydro-[1,1'-biphenyl]-2-yl)methyl)piperazine-1-carbonyl)-6-fluoro-1-oxoisoindolin-2-yl)piperidine-2,6-dione;
3-(5-(4-((4'-chloro-5,5-dimethyl-3,4,5,6-tetrahydro-[1,1'-biphenyl]-2-yl)methyl)piperazine-1-carbonyl)-7-fluoro-1-oxoisoindolin-2-yl)piperidine-2,6-dione;
3-(5-(3-((4'-chloro-5,5-dimethyl-3,4,5,6-tetrahydro-[1,1'-biphenyl]-2-yl)methyl)imidazolidine-1-carbonyl)-1-oxoisoindolin-2-yl)piperidine-2,6-dione;
3-(5-(4-((4'-chloro-5,5-dimethyl-3,4,5,6-tetrahydro-[1,1'-biphenyl]-2-yl)methyl)-1,4-diazepane-1-carbonyl)-1-oxoisoindolin-2-yl)piperidine-2,6-dione;
3-(5-(7-((4'-chloro-5,5-dimethyl-3,4,5,6-tetrahydro-[1,1'-biphenyl]-2-yl)methyl)-2,7-diazaspiro[3.5]nonane-2-carbonyl)-1-oxoisoindolin-2-yl)piperidine-2,6-dione;
3-(5-(6-((4'-chloro-5,5-dimethyl-3,4,5,6-tetrahydro-[1,1'-biphenyl]-2-yl)methyl)-2,6-diazaspiro[3.3]heptane-2-carbonyl)-1-oxoisoindolin-2-yl)piperidine-2,6-dione;
3-(5-(5-((4'-chloro-5,5-dimethyl-3,4,5,6-tetrahydro-[1,1'-biphenyl]-2-yl)methyl)octahydropyrrolo[3,4-c]pyrrole-2-carbonyl)-1-oxoisoindolin-2-yl)piperidine-2,6-dione;
3-(5-(3-((4'-chloro-5,5-dimethyl-3,4,5,6-tetrahydro-[1,1'-biphenyl]-2-yl)methyl)-3,6-diazabicyclo[3.1.1]heptane-6-carbonyl)-1-oxoisoindolin-2-yl)piperidine-2,6-dione;
3-(5-(6-((4'-chloro-5,5-dimethyl-3,4,5,6-tetrahydro-[1,1'-biphenyl]-2-yl)methyl)-3,6-diazabicyclo[3.1.1]heptane-3-carbonyl)-1-oxoisoindolin-2-yl)piperidine-2,6-dione;
3-(5-(3-((4'-chloro-5,5-dimethyl-3,4,5,6-tetrahydro-[1,1'-biphenyl]-2-yl)methyl)-3,8-diazabicyclo[3.2.1]octane-8-carbonyl)-1-oxoisoindolin-2-yl)piperidine-2,6-dione;

3-(5-(8-((4'-chloro-5,5-dimethyl-3,4,5,6-tetrahydro-[1,1'-biphenyl]-2-yl)methyl)-3,8-diazabicyclo[3.2.1]octane-3-carbonyl)-1-oxoisoindolin-2-yl)piperidine-2,6-dione;
3-(5-(5-((4'-chloro-5,5-dimethyl-3,4,5,6-tetrahydro-[1,1'-biphenyl]-2-yl)methyl)-2,5-diazabicyclo[2.2.2]octane-2-carbonyl)-1-oxoisoindolin-2-yl)piperidine-2,6-dione;
3-(5-((1R,4R)-5-((4'-chloro-5,5-dimethyl-3,4,5,6-tetrahydro-[1,1'-biphenyl]-2-yl)methyl)-2,5-diazabicyclo[2.2.1]heptane-2-carbonyl)-1-oxoisoindolin-2-yl)piperidine-2,6-dione;
3-(5-(4-((4'-chloro-5,5-dimethyl-3,4,5,6-tetrahydro-[1,1'-biphenyl]-2-yl)methyl)-2-(fluoromethyl)piperazine-1-carbonyl)-1-oxoisoindolin-2-yl)piperidine-2,6-dione;
3-(5-(4-((4'-chloro-5,5-dimethyl-3,4,5,6-tetrahydro-[1,1'-biphenyl]-2-yl)methyl)-3,5-dimethylpiperazine-1-carbonyl)-1-oxoisoindolin-2-yl)piperidine-2,6-dione;
3-(5-(4-((4'-chloro-5,5-dimethyl-3,4,5,6-tetrahydro-[1,1'-biphenyl]-2-yl)methyl)-2-(trifluoromethyl)piperazine-1-carbonyl)-1-oxoisoindolin-2-yl)piperidine-2,6-dione;
3-(5-(4-((4'-chloro-5,5-dimethyl-3,4,5,6-tetrahydro-[1,1'-biphenyl]-2-yl)methyl)-3,3-dimethylpiperazine-1-carbonyl)-1-oxoisoindolin-2-yl)piperidine-2,6-dione;
3-(5-(3-((4'-chloro-5,5-dimethyl-3,4,5,6-tetrahydro-[1,1'-biphenyl]-2-yl)methyl)imidazolidine-1-carbonyl)-4-fluoro-1-oxoisoindolin-2-yl)piperidine-2,6-dione;
3-(5-(4-((4'-chloro-5,5-dimethyl-3,4,5,6-tetrahydro-[1,1'-biphenyl]-2-yl)methyl)-1,4-diazepane-1-carbonyl)-4-fluoro-1-oxoisoindolin-2-yl)piperidine-2,6-dione;
3-(5-(7-((4'-chloro-5,5-dimethyl-3,4,5,6-tetrahydro-[1,1'-biphenyl]-2-yl)methyl)-2,7-diazaspiro[3.5]nonane-2-carbonyl)-4-fluoro-1-oxoisoindolin-2-yl)piperidine-2,6-dione;
3-(5-(6-((4'-chloro-5,5-dimethyl-3,4,5,6-tetrahydro-[1,1'-biphenyl]-2-yl)methyl)-2,6-diazaspiro[3.3]heptane-2-carbonyl)-4-fluoro-1-oxoisoindolin-2-yl)piperidine-2,6-dione;
3-(5-(5-((4'-chloro-5,5-dimethyl-3,4,5,6-tetrahydro-[1,1'-biphenyl]-2-yl)methyl)octahydropyrrolo[3,4-c]pyrrole-2-carbonyl)-4-fluoro-1-oxoisoindolin-2-yl)piperidine-2,6-dione;
3-(5-(3-((4'-chloro-5,5-dimethyl-3,4,5,6-tetrahydro-[1,1'-biphenyl]-2-yl)methyl)-3,6-diazabicyclo[3.1.1]heptane-6-carbonyl)-4-fluoro-1-oxoisoindolin-2-yl)piperidine-2,6-dione;
3-(5-(6-((4'-chloro-5,5-dimethyl-3,4,5,6-tetrahydro-[1,1'-biphenyl]-2-yl)methyl)-3,6-diazabicyclo[3.1.1]heptane-3-carbonyl)-4-fluoro-1-oxoisoindolin-2-yl)piperidine-2,6-dione;
3-(5-(3-((4'-chloro-5,5-dimethyl-3,4,5,6-tetrahydro-[1,1'-biphenyl]-2-yl)methyl)-3,8-diazabicyclo[3.2.1]octane-8-carbonyl)-4-fluoro-1-oxoisoindolin-2-yl)piperidine-2,6-dione;
3-(5-(8-((4'-chloro-5,5-dimethyl-3,4,5,6-tetrahydro-[1,1'-biphenyl]-2-yl)methyl)-3,8-diazabicyclo[3.2.1]octane-3-carbonyl)-4-fluoro-1-oxoisoindolin-2-yl)piperidine-2,6-dione;
3-(5-(5-((4'-chloro-5,5-dimethyl-3,4,5,6-tetrahydro-[1,1'-biphenyl]-2-yl)methyl)-2,5-diazabicyclo[2.2.2]octane-2-carbonyl)-4-fluoro-1-oxoisoindolin-2-yl)piperidine-2,6-dione;
3-(5-(4-((4'-chloro-5,5-dimethyl-3,4,5,6-tetrahydro-[1,1'-biphenyl]-2-yl)methyl)-2-(fluoromethyl)piperazine-1-carbonyl)-4-fluoro-1-oxoisoindolin-2-yl)piperidine-2,6-dione;
3-(5-(4-((4'-chloro-5,5-dimethyl-3,4,5,6-tetrahydro-[1,1'-biphenyl]-2-yl)methyl)-3,5-dimethylpiperazine-1-carbonyl)-4-fluoro-1-oxoisoindolin-2-yl)piperidine-2,6-dione;
3-(5-(4-((4'-chloro-5,5-dimethyl-3,4,5,6-tetrahydro-[1,1'-biphenyl]-2-yl)methyl)-2-(trifluoromethyl)piperazine-1-carbonyl)-4-fluoro-1-oxoisoindolin-2-yl)piperidine-2,6-dione;
3-(5-(4-((4'-chloro-5,5-dimethyl-3,4,5,6-tetrahydro-[1,1'-biphenyl]-2-yl)methyl)-3,3-dimethylpiperazine-1-carbonyl)-4-fluoro-1-oxoisoindolin-2-yl)piperidine-2,6-dione;
3-(5-(3-((4'-chloro-5,5-dimethyl-3,4,5,6-tetrahydro-[1,1'-biphenyl]-2-yl)methyl)imidazolidine-1-carbonyl)-6-fluoro-1-oxoisoindolin-2-yl)piperidine-2,6-dione;
3-(5-(4-((4'-chloro-5,5-dimethyl-3,4,5,6-tetrahydro-[1,1'-biphenyl]-2-yl)methyl)-1,4-diazepane-1-carbonyl)-6-fluoro-1-oxoisoindolin-2-yl)piperidine-2,6-dione;
3-(5-(7-((4'-chloro-5,5-dimethyl-3,4,5,6-tetrahydro-[1,1'-biphenyl]-2-yl)methyl)-2,7-diazaspiro[3.5]nonane-2-carbonyl)-6-fluoro-1-oxoisoindolin-2-yl)piperidine-2,6-dione;
3-(5-(6-((4'-chloro-5,5-dimethyl-3,4,5,6-tetrahydro-[1,1'-biphenyl]-2-yl)methyl)-2,6-diazaspiro[3.3]heptane-2-carbonyl)-6-fluoro-1-oxoisoindolin-2-yl)piperidine-2,6-dione;
3-(5-(5-((4'-chloro-5,5-dimethyl-3,4,5,6-tetrahydro-[1,1'-biphenyl]-2-yl)methyl)octahydropyrrolo[3,4-c]pyrrole-2-carbonyl)-6-fluoro-1-oxoisoindolin-2-yl)piperidine-2,6-dione;
3-(5-(3-((4'-chloro-5,5-dimethyl-3,4,5,6-tetrahydro-[1,1'-biphenyl]-2-yl)methyl)-3,6-diazabicyclo[3.1.1]heptane-6-carbonyl)-6-fluoro-1-oxoisoindolin-2-yl)piperidine-2,6-dione;
3-(5-(6-((4'-chloro-5,5-dimethyl-3,4,5,6-tetrahydro-[1,1'-biphenyl]-2-yl)methyl)-3,6-diazabicyclo[3.1.1]heptane-3-carbonyl)-6-fluoro-1-oxoisoindolin-2-yl)piperidine-2,6-dione;
3-(5-(3-((4'-chloro-5,5-dimethyl-3,4,5,6-tetrahydro-[1,1'-biphenyl]-2-yl)methyl)-3,8-diazabicyclo[3.2.1]octane-8-carbonyl)-6-fluoro-1-oxoisoindolin-2-yl)piperidine-2,6-dione;

3-(5-(8-((4'-chloro-5,5-dimethyl-3,4,5,6-tetrahydro-[1,1'-biphenyl]-2-yl)methyl)-3,8-diazabicyclo[3.2.1]octane-3-carbonyl)-6-fluoro-1-oxoisoindolin-2-yl)piperidine-2,6-dione;
3-(5-(5-((4'-chloro-5,5-dimethyl-3,4,5,6-tetrahydro-[1,1'-biphenyl]-2-yl)methyl)-2,5-diazabicyclo[2.2.2]octane-2-carbonyl)-6-fluoro-1-oxoisoindolin-2-yl)piperidine-2,6-dione;
3-(5-(6-((4'-chloro-5,5-dimethyl-3,4,5,6-tetrahydro-[1,1'-biphenyl]-2-yl)methyl)-3,6-diazabicyclo[3.1.1]heptane-3-carbonyl)-6-fluoro-1-oxoisoindolin-2-yl)piperidine-2,6-dione;
3-(5-(4-((4'-chloro-5,5-dimethyl-3,4,5,6-tetrahydro-[1,1'-biphenyl]-2-yl)methyl)-2-(fluoromethyl)piperazine-1-carbonyl)-6-fluoro-1-oxoisoindolin-2-yl)piperidine-2,6-dione;
3-(5-(4-((4'-chloro-5,5-dimethyl-3,4,5,6-tetrahydro-[1,1'-biphenyl]-2-yl)methyl)-3,5-dimethylpiperazine-1-carbonyl)-6-fluoro-1-oxoisoindolin-2-yl)piperidine-2,6-dione;
3-(5-(4-((4'-chloro-5,5-dimethyl-3,4,5,6-tetrahydro-[1,1'-biphenyl]-2-yl)methyl)-2-(trifluoromethyl)piperazine-1-carbonyl)-6-fluoro-1-oxoisoindolin-2-yl)piperidine-2,6-dione;
3-(5-(4-((4'-chloro-5,5-dimethyl-3,4,5,6-tetrahydro-[1,1'-biphenyl]-2-yl)methyl)-3,3-dimethylpiperazine-1-carbonyl)-6-fluoro-1-oxoisoindolin-2-yl)piperidine-2,6-dione;
3-(5-(3-((4'-chloro-5,5-dimethyl-3,4,5,6-tetrahydro-[1,1'-biphenyl]-2-yl)methyl)imidazolidine-1-carbonyl)-7-fluoro-1-oxoisoindolin-2-yl)piperidine-2,6-dione;
3-(5-(4-((4'-chloro-5,5-dimethyl-3,4,5,6-tetrahydro-[1,1'-biphenyl]-2-yl)methyl)-1,4-diazepane-1-carbonyl)-7-fluoro-1-oxoisoindolin-2-yl)piperidine-2,6-dione;
3-(5-(7-((4'-chloro-5,5-dimethyl-3,4,5,6-tetrahydro-[1,1'-biphenyl]-2-yl)methyl)-2,7-diazaspiro[3.5]nonane-2-carbonyl)-7-fluoro-1-oxoisoindolin-2-yl)piperidine-2,6-dione;
3-(5-(6-((4'-chloro-5,5-dimethyl-3,4,5,6-tetrahydro-[1,1'-biphenyl]-2-yl)methyl)-2,6-diazaspiro[3.3]heptane-2-carbonyl)-7-fluoro-1-oxoisoindolin-2-yl)piperidine-2,6-dione;
3-(5-(5-((4'-chloro-5,5-dimethyl-3,4,5,6-tetrahydro-[1,1'-biphenyl]-2-yl)methyl)octahydropyrrolo[3,4-c]pyrrole-2-carbonyl)-7-fluoro-1-oxoisoindolin-2-yl)piperidine-2,6-dione;
3-(5-(3-((4'-chloro-5,5-dimethyl-3,4,5,6-tetrahydro-[1,1'-biphenyl]-2-yl)methyl)-3,6-diazabicyclo[3.1.1]heptane-6-carbonyl)-7-fluoro-1-oxoisoindolin-2-yl)piperidine-2,6-dione;
3-(5-(6-((4'-chloro-5,5-dimethyl-3,4,5,6-tetrahydro-[1,1'-biphenyl]-2-yl)methyl)-3,6-diazabicyclo[3.1.1]heptane-3-carbonyl)-7-fluoro-1-oxoisoindolin-2-yl)piperidine-2,6-dione;
3-(5-(3-((4'-chloro-5,5-dimethyl-3,4,5,6-tetrahydro-[1,1'-biphenyl]-2-yl)methyl)-3,8-diazabicyclo[3.2.1]octane-8-carbonyl)-7-fluoro-1-oxoisoindolin-2-yl)piperidine-2,6-dione;
3-(5-(8-((4'-chloro-5,5-dimethyl-3,4,5,6-tetrahydro-[1,1'-biphenyl]-2-yl)methyl)-3,8-diazabicyclo[3.2.1]octane-3-carbonyl)-7-fluoro-1-oxoisoindolin-2-yl)piperidine-2,6-dione;
3-(5-(5-((4'-chloro-5,5-dimethyl-3,4,5,6-tetrahydro-[1,1'-biphenyl]-2-yl)methyl)-2,5-diazabicyclo[2.2.2]octane-2-carbonyl)-7-fluoro-1-oxoisoindolin-2-yl)piperidine-2,6-dione;
3-(5-(6-((4'-chloro-5,5-dimethyl-3,4,5,6-tetrahydro-[1,1'-biphenyl]-2-yl)methyl)-3,6-diazabicyclo[3.1.1]heptane-3-carbonyl)-7-fluoro-1-oxoisoindolin-2-yl)piperidine-2,6-dione;
3-(5-(4-((4'-chloro-5,5-dimethyl-3,4,5,6-tetrahydro-[1,1'-biphenyl]-2-yl)methyl)-2-(fluoromethyl)piperazine-1-carbonyl)-7-fluoro-1-oxoisoindolin-2-yl)piperidine-2,6-dione;
3-(5-(4-((4'-chloro-5,5-dimethyl-3,4,5,6-tetrahydro-[1,1'-biphenyl]-2-yl)methyl)-3,5-dimethylpiperazine-1-carbonyl)-7-fluoro-1-oxoisoindolin-2-yl)piperidine-2,6-dione;
3-(5-(4-((4'-chloro-5,5-dimethyl-3,4,5,6-tetrahydro-[1,1'-biphenyl]-2-yl)methyl)-2-(trifluoromethyl)piperazine-1-carbonyl)-7-fluoro-1-oxoisoindolin-2-yl)piperidine-2,6-dione;
3-(5-(4-((4'-chloro-5,5-dimethyl-3,4,5,6-tetrahydro-[1,1'-biphenyl]-2-yl)methyl)-3,3-dimethylpiperazine-1-carbonyl)-7-fluoro-1-oxoisoindolin-2-yl)piperidine-2,6-dione;
3-(5-((4-((4'-chloro-[1,1'-biphenyl]-2-yl)methyl)piperazin-1-yl)methyl)-1-oxoisoindolin-2-yl)piperidine-2,6-dione;
3-(5-((4-((4'-chloro-[1,1'-biphenyl]-2-yl)methyl)piperazin-1-yl)methyl)-4-fluoro-1-oxoisoindolin-2-yl)piperidine-2,6-dione;
3-(5-((4-((4'-chloro-[1,1'-biphenyl]-2-yl)methyl)piperazin-1-yl)methyl)-6-fluoro-1-oxoisoindolin-2-yl)piperidine-2,6-dione;
3-(5-((4-((4'-chloro-[1,1'-biphenyl]-2-yl)methyl)piperazin-1-yl)methyl)-7-fluoro-1-oxoisoindolin-2-yl)piperidine-2,6-dione;
3-(4-((4-((4'-chloro-[1,1'-biphenyl]-2-yl)methyl)piperazin-1-yl)methyl)-1-oxoisoindolin-2-yl)piperidine-2,6-dione;
3-(5-(2-(4-((4'-chloro-[1,1'-biphenyl]-2-yl)methyl)piperazin-1-yl)ethyl)-1-oxoisoindolin-2-yl)piperidine-2,6-dione;
3-(5-(3-(4-((4'-chloro-[1,1'-biphenyl]-2-yl)methyl)piperazin-1-yl)propyl)-1-oxoisoindolin-2-yl)piperidine-2,6-dione;

3-(5-((1-((4'-chloro-[1,1'-biphenyl]-2-yl)methyl)piperidin-4-yl)amino)-1-oxoisoindolin-2-yl)piperidine-2,6-dione;
3-(5-((4-((4'-chloro-[1,1'-biphenyl]-2-yl)methyl)piperazin-1-yl)amino)-1-oxoisoindolin-2-yl)piperidine-2,6-dione;
3-(5-((4-((4'-fluoro-[1,1'-biphenyl]-2-yl)methyl)piperazin-1-yl)methyl)-1-oxoisoindolin-2-yl)piperidine-2,6-dione;
3-(4-fluoro-5-((4-((4'-fluoro-[1,1'-biphenyl]-2-yl)methyl)piperazin-1-yl)methyl)-1-oxoisoindolin-2-yl)piperidine-2,6-dione;
3-(6-fluoro-5-((4-((4'-fluoro-[1,1'-biphenyl]-2-yl)methyl)piperazin-1-yl)methyl)-1-oxoisoindolin-2-yl)piperidine-2,6-dione;
3-(7-fluoro-5-((4-((4'-fluoro-[1,1'-biphenyl]-2-yl)methyl)piperazin-1-yl)methyl)-1-oxoisoindolin-2-yl)piperidine-2,6-dione;
3-(4-((4-((4'-fluoro-[1,1'-biphenyl]-2-yl)methyl)piperazin-1-yl)methyl)-1-oxoisoindolin-2-yl)piperidine-2,6-dione;
3-(6-((4-((4'-fluoro-[1,1'-biphenyl]-2-yl)methyl)piperazin-1-yl)methyl)-1-oxoisoindolin-2-yl)piperidine-2,6-dione;
3-(5-((4-(4'-fluoro-[1,1'-biphenyl]-2-carbonyl)piperazin-1-yl)methyl)-1-oxoisoindolin-2-yl)piperidine-2,6-dione;
3-(5-((4-((4'-fluoro-3,4,5,6-tetrahydro-[1,1'-biphenyl]-2-yl)methyl)piperazin-1-yl)methyl)-1-oxoisoindolin-2-yl)piperidine-2,6-dione;
3-(5-((4-(4'-fluoro-3,4,5,6-tetrahydro-[1,1'-biphenyl]-2-carbonyl)piperazin-1-yl)methyl)-1-oxoisoindolin-2-yl)piperidine-2,6-dione;
3-(5-((3-(4'-fluoro-3,4,5,6-tetrahydro-[1,1'-biphenyl]-2-carbonyl)-3,6-diazabicyclo[3.1.1]heptan-6-yl)methyl)-1-oxoisoindolin-2-yl)piperidine-2,6-dione;
3-(5-((4-((4'-chloro-[1,1'-biphenyl]-2-yl)methyl)piperazin-1-yl)fluoromethyl)-1-oxoisoindolin-2-yl)piperidine-2,6-dione;
3-(5-((4-((4'-chloro-[1,1'-biphenyl]-2-yl)methyl)piperazin-1-yl)difluoromethyl)-1-oxoisoindolin-2-yl)piperidine-2,6-dione;
3-(5-(1-(4-((4'-chloro-[1,1'-biphenyl]-2-yl)methyl)piperazin-1-yl)cyclopropyl)-1-oxoisoindolin 2-yl)piperidine-2,6-dione;
3-(5-(1-(4-((4'-chloro-[1,1'-biphenyl]-2-yl)methyl)piperazin-1-yl)cyclobutyl)-1-oxoisoindolin-2-yl)piperidine-2,6-dione;
3-(5-(1-(4-((4'-chloro-[1,1'-biphenyl]-2-yl)methyl)piperazin-1-yl)cyclopentyl)-1-oxoisoindolin 2-yl)piperidine-2,6-dione;
3-(5-((4-((2-(4-bromophenyl)cyclopent-1-en-1-yl)methyl)piperazin-1-yl)methyl)-1-oxoisoindolin-2-yl)piperidine-2,6-dione;
3-(5-((4-((2-(4-bromophenyl)cyclohept-1-en-1-yl)methyl)piperazin-1-yl)methyl)-1-oxoisoindolin-2-yl)piperidine-2,6-dione;
3-(5-((4-((2-(4-bromophenyl)cyclopropyl)methyl)piperazin-1-yl)methyl)-1-oxoisoindolin-2-yl)piperidine-2,6-dione;
3-(5-((4-((2-(4-bromophenyl)cyclobutyl)methyl)piperazin-1-yl)methyl)-1-oxoisoindolin-2-yl)piperidine-2,6-dione;
3-(1-oxo-5-((4-(2-(thiophen-2-yl)benzyl)piperazin-1-yl)methyl)isoindolin-2-yl)piperidine-2,6-dione;
3-(5-((4-(2-(furan-2-yl)benzyl)piperazin-1-yl)methyl)-1-oxoisoindolin-2-yl)piperidine-2,6-dione;
3-(5-((4-(2-(1H-pyrrol-2-yl)benzyl)piperazin-1-yl)methyl)-1-oxoisoindolin-2-yl)piperidine-2,6-dione;
3-(5-((4-(2-(1-methyl-1H-pyrrol-2-yl)benzyl)piperazin-1-yl)methyl)-1-oxoisoindolin-2-yl)piperidine-2,6-dione;
3-(5-((4-(2-(1-methyl-1H-pyrazol-5-yl)benzyl)piperazin-1-yl)methyl)-1-oxoisoindolin-2-yl)piperidine-2,6-dione;
3-(1-oxo-5-((4-(2-(thiazol-5-yl)benzyl)piperazin-1-yl)methyl)isoindolin-2-yl)piperidine-2,6-dione;
3-(5-((4-(2-(oxazol-5-yl)benzyl)piperazin-1-yl)methyl)-1-oxoisoindolin-2-yl)piperidine-2,6-dione;
3-(5-((1-((4'-chloro-[1,1'-biphenyl]-2-yl)methyl)-3-hydroxyazetidin-3-yl)(hydroxy)methyl)-1-oxoisoindolin-2-yl)piperidine-2,6-dione;
3-(5-((1-((4'-chloro-[1,1'-biphenyl]-2-yl)methyl)azetidin-3-ylidene)methyl)-1-oxoisoindolin-2-yl)piperidine-2,6-dione;
3-(5-((1-((4'-chloro-[1,1'-biphenyl]-2-yl)methyl)azetidin-3-yl)amino)-1-oxoisoindolin-2-yl)piperidine-2,6-dione;
3-(5-((1-((4'-chloro-[1,1'-biphenyl]-2-yl)methyl)azetidin-3-yl)amino)-6-fluoro-1-oxoisoindolin-2-yl)piperidine-2,6-dione;
3-(5-((1-((4'-chloro-[1,1'-biphenyl]-2-yl)methyl)azetidin-3-yl)methyl)-1-oxoisoindolin-2-yl)piperidine-2,6-dione;
3-(5-(4-((4-((4'-chloro-[1,1'-biphenyl]-2-yl)methyl)piperazin-1-yl)methyl)phenyl)-1-oxoisoindolin-2-yl)piperidine-2,6-dione;
3-(5-((4-((4'-chloro-[1,1'-biphenyl]-2-yl)amino)piperidin-1-yl)methyl)-1-oxoisoindolin-2-yl)piperidine-2,6-dione;
3-(5-((4-((4'-chloro-[1,1'-biphenyl]-2-yl)amino)-3,3-difluoropiperidin-1-yl)methyl)-1-oxoisoindolin-2-yl)piperidine-2,6-dione;
3-(5-((1-((4'-chloro-[1,1'-biphenyl]-2-yl)methyl)piperidin-4-yl)methyl)-1-oxoisoindolin-2-yl)piperidine-2,6-dione;
3-(5-((3-((4'-chloro-[1,1'-biphenyl]-2-yl)methyl)imidazolidin-1-yl)methyl)-1-oxoisoindolin-2-yl)piperidine-2,6-dione;

3-(5-((4-((4'-chloro-[1,1'-biphenyl]-2-yl)methyl)-1,4-diazepan-1-yl)methyl)-1-oxoisoindolin-2-yl)piperidine-2,6-dione;

3-(5-((4-((4'-chloro-[1,1'-biphenyl]-2-yl)methyl)-2-oxopiperazin-1-yl)methyl)-1-oxoisoindolin-2-yl)piperidine-2,6-dione;

3-(5-((7-((4'-chloro-[1,1'-biphenyl]-2-yl)methyl)-2,7-diazaspiro[3.5]nonan-2-yl)methyl)-1-oxoisoindolin-2-yl)piperidine-2,6-dione;

3-(5-((6-((4'-chloro-[1,1'-biphenyl]-2-yl)methyl)-2,6-diazaspiro[3.3]heptan-2-yl)methyl)-1-oxoisoindolin-2-yl)piperidine-2,6-dione;

3-(5-((5-((4'-chloro-[1,1'-biphenyl]-2-yl)methyl)hexahydropyrrolo[3,4-c]pyrrol-2(1H)-yl)methyl)-1-oxoisoindolin-2-yl)piperidine-2,6-dione;

3-(5-((4-(4'-chloro-[1,1'-biphenyl]-2-yl)-1H-1,2,3-triazol-1-yl)methyl)-1-oxoisoindolin-2-yl)piperidine-2,6-dione;

3-(5-((1-((4'-chloro-[1,1'-biphenyl]-2-yl)methyl)-3,3-difluoropiperidin-4-yl)amino)-1-oxoisoindolin-2-yl)piperidine-2,6-dione;

3-(5-(3-((4'-chloro-[1,1'-biphenyl]-2-yl)methyl)-3,8-diazabicyclo[3.2.1]octan-8-yl)-1-oxoisoindolin-2-yl)piperidine-2,6-dione;

3-(5-((3-((4'-chloro-[1,1'-biphenyl]-2-yl)methyl)-3,6-diazabicyclo[3.1.1]heptan-6-yl)methyl)-1-oxoisoindolin-2-yl)piperidine-2,6-dione;

3-(5-((3-((4'-chloro-[1,1'-biphenyl]-2-yl)methyl)-3,6-diazabicyclo[3.1.1]heptan-6-yl)methyl)-4-fluoro-1-oxoisoindolin-2-yl)piperidine-2,6-dione;

3-(5-((3-((4'-chloro-[1,1'-biphenyl]-2-yl)methyl)-3,6-diazabicyclo[3.1.1]heptan-6-yl)methyl)-6-fluoro-1-oxoisoindolin-2-yl)piperidine-2,6-dione;

3-(5-((3-((4'-chloro-[1,1'-biphenyl]-2-yl)methyl)-3,6-diazabicyclo[3.1.1]heptan-6-yl)methyl)-7-fluoro-1-oxoisoindolin-2-yl)piperidine-2,6-dione;

3-(5-((6-((4'-chloro-[1,1'-biphenyl]-2-yl)methyl)-3,6-diazabicyclo[3.1.1]heptan-3-yl)methyl)-1-oxoisoindolin-2-yl)piperidine-2,6-dione;

3-(5-((3-((4'-chloro-[1,1'-biphenyl]-2-yl)methyl)-3,8-diazabicyclo[3.2.1]octan-8-yl)methyl)-1-oxoisoindolin-2-yl)piperidine-2,6-dione;

3-(5-((8-((4'-chloro-[1,1'-biphenyl]-2-yl)methyl)-3,8-diazabicyclo[3.2.1]octan-3-yl)methyl)-1-oxoisoindolin-2-yl)piperidine-2,6-dione;

3-(5-((5-((4'-chloro-[1,1'-biphenyl]-2-yl)methyl)-2,5-diazabicyclo[2.2.2]octan-2-yl)methyl)-1-oxoisoindolin-2-yl)piperidine-2,6-dione;

3-(5-(((1R,4R)-5-((4'-chloro-[1,1'-biphenyl]-2-yl)methyl)-2,5-diazabicyclo[2.2.1]heptan-2-yl)methyl)-1-oxoisoindolin-2-yl)piperidine-2,6-dione;

3-(5-((4-((4'-chloro-[1,1'-biphenyl]-2-yl)methyl)-2-(fluoromethyl)piperazin-1-yl)methyl)-1-oxoisoindolin-2-yl)piperidine-2,6-dione;

3-(5-((4-((4'-chloro-[1,1'-biphenyl]-2-yl)methyl)-3,5-dimethylpiperazin-1-yl)methyl)-1-oxoisoindolin-2-yl)piperidine-2,6-dione;

3-(5-((4-((4'-chloro-[1,1'-biphenyl]-2-yl)methyl)-2-(trifluoromethyl)piperazin-1-yl)methyl)-1-oxoisoindolin-2-yl)piperidine-2,6-dione;

3-(5-((4-((4'-chloro-[1,1'-biphenyl]-2-yl)methyl)-3,3-dimethylpiperazin-1-yl)methyl)-1-oxoisoindolin-2-yl)piperidine-2,6-dione;

3-(5-((3-((4'-chloro-[1,1'-biphenyl]-2-yl)methyl)-2-oxoimidazolidin-1-yl)methyl)-1-oxoisoindolin-2-yl)piperidine-2,6-dione;

3-(5-((3-((4'-chloro-[1,1'-biphenyl]-2-yl)methyl)-2-oxoimidazolidin-1-yl)methyl)-4-fluoro-1-oxoisoindolin-2-yl)piperidine-2,6-dione;

3-(5-((3-((4'-chloro-[1,1'-biphenyl]-2-yl)methyl)-2-oxoimidazolidin-1-yl)methyl)-6-fluoro-1-oxoisoindolin-2-yl)piperidine-2,6-dione;

3-(5-((3-((4'-chloro-[1,1'-biphenyl]-2-yl)methyl)-2-oxoimidazolidin-1-yl)methyl)-7-fluoro-1-oxoisoindolin-2-yl)piperidine-2,6-dione;

3-(4-((3-((4'-chloro-[1,1'-biphenyl]-2-yl)methyl)-2-oxoimidazolidin-1-yl)methyl)-1-oxoisoindolin-2-yl)piperidine-2,6-dione;

3-(5-((4-((3-(4-chlorophenyl)pyridin-4-yl)methyl)piperazin-1-yl)methyl)-1-oxoisoindolin-2-yl)piperidine-2,6-dione;

3-(5-((4-((4-(4-chlorophenyl)pyridin-3-yl)methyl)piperazin-1-yl)methyl)-1-oxoisoindolin-2-yl)piperidine-2,6-dione;

3-(5-((4-((3-(4-chlorophenyl)pyridin-2-yl)methyl)piperazin-1-yl)methyl)-1-oxoisoindolin-2-yl)piperidine-2,6-dione;

3-(5-((4-(2-(5-chloropyridin-2-yl)benzyl)piperazin-1-yl)methyl)-1-oxoisoindolin-2-yl)piperidine-2,6-dione;
3-(5-((4-((4'-chloro-[1,1'-biphenyl]-3-yl)methyl)piperazin-1-yl)methyl)-1-oxoisoindolin-2-yl)piperidine-2,6-dione;
3-(5-((4-(3-(5-chloropyridin-2-yl)benzyl)piperazin-1-yl)methyl)-1-oxoisoindolin-2-yl)piperidine-2,6-dione;
3-(1-oxo-5-((4-(3-(thiophen-2-yl)benzyl)piperazin-1-yl)methyl)isoindolin-2-yl)piperidine-2,6-dione;
3-(5-((4-(3-(furan-2-yl)benzyl)piperazin-1-yl)methyl)-1-oxoisoindolin-2-yl)piperidine-2,6-dione;
3-(5-((4-(3-(1H-pyrrol-2-yl)benzyl)piperazin-1-yl)methyl)-1-oxoisoindolin-2-yl)piperidine-2,6-dione;
3-(5-((4-((4'-chloro-[1,1'-biphenyl]-4-yl)methyl)piperazin-1-yl)methyl)-1-oxoisoindolin-2-yl)piperidine-2,6-dione;
3-(5-((4-(4-(5-chloropyridin-2-yl)benzyl)piperazin-1-yl)methyl)-1-oxoisoindolin-2-yl)piperidine-2,6-dione;
3-(1-oxo-5-((4-(4-(thiophen-2-yl)benzyl)piperazin-1-yl)methyl)isoindolin-2-yl)piperidine-2,6-dione;
3-(S-((4-(4-(furan-2-yl)benzyl)piperazin-1-yl)methyl)-1-oxoisoindolin-2-yl)piperidine-2,6-dione;
3-(5-((4-(4-(1H-pyrrol-2-yl)benzyl)piperazin-1-yl)methyl)-1-oxoisoindolin-2-yl)piperidine-2,6-dione;
3-(5-((4-((6-(4-chlorophenyl)pyridin-3-yl)methyl)piperazin-1-yl)methyl)-1-oxoisoindolin-2-yl)piperidine-2,6-dione;
3-(5-((4-([2,4'-bipyridin]-5-ylmethyl)piperazin-1-yl)methyl)-1-oxoisoindolin-2-yl)piperidine-2,6-dione;
3-(1-oxo-5-((4-((5-phenylpyrazin-2-yl)methyl)piperazin-1-yl)methyl)isoindolin-2-yl)piperidine-2,6-dione;
3-(1-oxo-5-((4-(2-phenylpyrimidine-5-carbonyl)piperazin-1-yl)methyl)isoindolin-2-yl)piperidine-2,6-dione;
3-(5-((4-((5-(4-chlorophenyl)thiophen-2-yl)methyl)piperazin-1-yl)methyl)-1-oxoisoindolin-2-yl)piperidine-2,6-dione;
3-(5-((4-([2,2'-bithiophen]-5-ylmethyl)piperazin-1-yl)methyl)-1-oxoisoindolin-2-yl)piperidine-2,6-dione;
3-(5-((4-((5-(furan-2-yl)thiophen-2-yl)methyl)piperazin-1-yl)methyl)-1-oxoisoindolin-2-yl)piperidine-2,6-dione;
3-(5-((4-((5-(4-chlorophenyl)furan-2-yl)methyl)piperazin-1-yl)methyl)-1-oxoisoindolin-2-yl)piperidine-2,6-dione;
3-(1-oxo-5-((4-((5-(thiophen-2-yl)furan-2-yl)methyl)piperazin-1-yl)methyl)isoindolin-2-yl)piperidine-2,6-dione;
3-(5-((4-([2,2'-bifuran]-5-ylmethyl)piperazin-1-yl)methyl)-1-oxoisoindolin-2-yl)piperidine-2,6-dione;
3-(5-((4-(4'-chloro-[1,1'-biphenyl]-2-carbonyl)piperazin-1-yl)methyl)-1-oxoisoindolin-2-yl)piperidine-2,6-dione;
3-(5-((4-(3-(4-chlorophenyl)isonicotinoyl)piperazin-1-yl)methyl)-1-oxoisoindolin-2-yl)piperidine-2,6-dione;
3-(5-((4-(4'-chloro-[1,1'-biphenyl]-2-carbonyl)piperazin-1-yl)methyl)-4-fluoro-1-oxoisoindolin-2-yl)piperidine-2,6-dione;
3-(5-((4-(4'-chloro-[1,1'-biphenyl]-2-carbonyl)piperazin-1-yl)methyl)-6-fluoro-1-oxoisoindolin-2-yl)piperidine-2,6-dione;
3-(5-((4-(4'-chloro-[1,1'-biphenyl]-2-carbonyl)piperazin-1-yl)methyl)-7-fluoro-1-oxoisoindolin-2-yl)piperidine-2,6-dione;
3-(4-((4-(4'-chloro-[1,1'-biphenyl]-2-carbonyl)piperazin-1-yl)methyl)-1-oxoisoindolin-2-yl)piperidine-2,6-dione;
3-(5-((1-(4'-chloro-[1,1'-biphenyl]-2-carbonyl)piperidin-4-yl)amino)-1-oxoisoindolin-2-yl)piperidine-2,6-dione;
3-(5-((1-(4'-chloro-[1,1'-biphenyl]-2-carbonyl)piperidin-4-yl)methyl)-1-oxoisoindolin-2-yl)piperidine-2,6-dione;
3-(5-((1-(4'-chloro-[1,1'-biphenyl]-2-carbonyl)azetidin-3-yl)amino)-1-oxoisoindolin-2-yl)piperidine-2,6-dione;
3-(5-((1-(4'-chloro-[1,1'-biphenyl]-2-carbonyl)azetidin-3-yl)methyl)-1-oxoisoindolin-2-yl)piperidine-2,6-dione;
3-(5-((1-(4'-chloro-[1,1'-biphenyl]-2-carbonyl)-3-hydroxyazetidin-3-yl)(hydroxy)methyl)-1-oxoisoindolin-2-yl)piperidine-2,6-dione;
3-(5-((1-(4'-chloro-[1,1'-biphenyl]-2-carbonyl)azetidin-3-ylidene)methyl)-1-oxoisoindolin-2-yl)piperidine-2,6-dione;
3-(5-((3-(4'-chloro-[1,1'-biphenyl]-2-carbonyl)imidazolidin-1-yl)methyl)-1-oxoisoindolin-2-yl)piperidine-2,6-dione;
3-(5-((4-(4'-chloro-[1,1'-biphenyl]-2-carbonyl)-1,4-diazepan-1-yl)methyl)-1-oxoisoindolin-2-yl)piperidine-2,6-dione;
3-(5-(4-((4-(4'-chloro-[1,1'-biphenyl]-2-carbonyl)piperazin-1-yl)methyl)phenyl)-1-oxoisoindolin-2-yl)piperidine-2,6-dione;
3-(5-((7-(4'-chloro-[1,1'-biphenyl]-2-carbonyl)-2,7-diazaspiro[3.5]nonan-2-yl)methyl)-1-oxoisoindolin-2-yl)piperidine-2,6-dione;
3-(5-((6-(4'-chloro-[1,1'-biphenyl]-2-carbonyl)-2,6-diazaspiro[3.3]heptan-2-yl)methyl)-1-oxoisoindolin-2-yl)piperidine-2,6-dione;
3-(5-((5-(4'-chloro-[1,1'-biphenyl]-2-carbonyl)hexahydropyrrolo[3,4-c]pyrrol-2(1H)-yl)methyl)-1-oxoisoindolin-2-yl)piperidine-2,6-dione;
3-(5-(3-(4'-chloro-[1,1'-biphenyl]-2-carbonyl)-3,8-diazabicyclo[3.2.1]octan-8-yl)-1-oxoisoindolin-2-yl)piperidine-2,6-dione;
3-(5-((3-(4'-chloro-[1,1'-biphenyl]-2-carbonyl)-3,6-diazabicyclo[3.1.1]heptan-6-yl)methyl)-1-oxoisoindolin-2-yl)piperidine-2,6-dione;
3-(5-((3-(4'-chloro-[1,1'-biphenyl]-2-carbonyl)-3,6-diazabicyclo[3.1.1]heptan-6-yl)methyl)-4-fluoro-1-oxoisoindolin-2-yl)piperidine-2,6-dione;
3-(5-((3-(4'-chloro-[1,1'-biphenyl]-2-carbonyl)-3,6-diazabicyclo[3.1.1]heptan-6-yl)methyl)-6-fluoro-1-oxoisoindolin-2-yl)piperidine-2,6-dione;
3-(5-((3-(4'-chloro-[1,1'-biphenyl]-2-carbonyl)-3,6-diazabicyclo[3.1.1]heptan-6-yl)methyl)-7-fluoro-1-oxoisoindolin-2-yl)piperidine-2,6-dione;
3-(5-((6-(4'-chloro-[1,1'-biphenyl]-2-carbonyl)-3,6-diazabicyclo[3.1.1]heptan-3-yl)methyl)-1-oxoisoindolin-2-yl)piperidine-2,6-dione;
3-(5-((3-(4'-chloro-[1,1'-biphenyl]-2-carbonyl)-3,8-diazabicyclo[3.2.1]octan-8-yl)methyl)-1-oxoisoindolin-2-yl)piperidine-2,6-dione;
3-(5-((8-(4'-chloro-[1,1'-biphenyl]-2-carbonyl)-3,8-diazabicyclo[3.2.1]octan-3-yl)methyl)-1-oxoisoindolin-2-yl)piperidine-2,6-dione;
3-(5-((5-(4'-chloro-[1,1'-biphenyl]-2-carbonyl)-2,5-diazabicyclo[2.2.2]octan-2-yl)methyl)-1-oxoisoindolin-2-yl)piperidine-2,6-dione;
3-(5-(((1R,4R)-5-(4'-chloro-[1,1'-biphenyl]-2-carbonyl)-2,5-diazabicyclo[2.2.1]heptan-2-yl)methyl)-1-oxoisoindolin-2-yl)piperidine-2,6-dione;
3-(5-((4-(4'-chloro-[1,1'-biphenyl]-2-carbonyl)-2-(fluoromethyl)piperazin-1-yl)methyl)-1-oxoisoindolin-2-yl)piperidine-2,6-dione;
3-(5-((4-(4'-chloro-[1,1'-biphenyl]-2-carbonyl)-3,5-dimethylpiperazin-1-yl)methyl)-1-oxoisoindolin-2-yl)piperidine-2,6-dione;
3-(5-((4-(4'-chloro-[1,1'-biphenyl]-2-carbonyl)-2-(trifluoromethyl)piperazin-1-yl)methyl)-1-oxoisoindolin-2-yl)piperidine-2,6-dione;
3-(5-((4-(4'-chloro-[1,1'-biphenyl]-2-carbonyl)-3,3-dimethylpiperazin-1-yl)methyl)-1-oxoisoindolin-2-yl)piperidine-2,6-dione;
3-(5-(4-((4'-chloro-[1,1'-biphenyl]-2-yl)methyl)piperazine-1-carbonyl)-1-oxoisoindolin-2-yl)piperidine-2,6-dione;
3-(5-(4-((4'-chloro-[1,1'-biphenyl]-2-yl)methyl)piperazine-1-carbonyl)-4-fluoro-1-oxoisoindolin-2-yl)piperidine-2,6-dione;
3-(5-(4-((4'-chloro-[1,1'-biphenyl]-2-yl)methyl)piperazine-1-carbonyl)-6-fluoro-1-oxoisoindolin-2-yl)piperidine-2,6-dione;
3-(5-(4-((4'-chloro-[1,1'-biphenyl]-2-yl)methyl)piperazine-1-carbonyl)-7-fluoro-1-oxoisoindolin-2-yl)piperidine-2,6-dione;
3-(4-(4-((4'-chloro-[1,1'-biphenyl]-2-yl)methyl)piperazine-1-carbonyl)-1-oxoisoindolin-2-yl)piperidine-2,6-dione;
3-(5-(4-((4'-chloro-[1,1'-biphenyl]-2-yl)amino)piperidine-1-carbonyl)-1-oxoisoindolin-2-yl)piperidine-2,6-dione;
3-(5-(3-((4'-chloro-[1,1'-biphenyl]-2-yl)methyl)imidazolidine-1-carbonyl)-1-oxoisoindolin-2-yl)piperidine-2,6-dione;
3-(5-(4-((4'-chloro-[1,1'-biphenyl]-2-yl)methyl)-1,4-diazepane-1-carbonyl)-1-oxoisoindolin-2-yl)piperidine-2,6-dione;
3-(5-(7-((4'-chloro-[1,1'-biphenyl]-2-yl)methyl)-2,7-diazaspiro[3.5]nonane-2-carbonyl)-1-oxoisoindolin-2-yl)piperidine-2,6-dione;
3-(5-(6-((4'-chloro-[1,1'-biphenyl]-2-yl)methyl)-2,6-diazaspiro[3.3]heptane-2-carbonyl)-1-oxoisoindolin-2-yl)piperidine-2,6-dione;
3-(5-(5-((4'-chloro-[1,1'-biphenyl]-2-yl)methyl)octahydropyrrolo[3,4-c]pyrrole-2-carbonyl)-1-oxoisoindolin-2-yl)piperidine-2,6-dione;
3-(5-(3-((4'-chloro-[1,1'-biphenyl]-2-yl)methyl)-3,6-diazabicyclo[3.1.1]heptane-6-carbonyl)-1-oxoisoindolin-2-yl)piperidine-2,6-dione;
3-(5-(3-((4'-chloro-[1,1'-biphenyl]-2-yl)methyl)-3,6-diazabicyclo[3.1.1]heptane-6-carbonyl)-4-fluoro-1-oxoisoindolin-2-yl)piperidine-2,6-dione;
3-(5-(3-((4'-chloro-[1,1'-biphenyl]-2-yl)methyl)-3,6-diazabicyclo[3.1.1]heptane-6-carbonyl)-6-fluoro-1-oxoisoindolin-2-yl)piperidine-2,6-dione;
3-(5-(3-((4'-chloro-[1,1'-biphenyl]-2-yl)methyl)-3,6-diazabicyclo[3.1.1]heptane-6-carbonyl)-7-fluoro-1-oxoisoindolin-2-yl)piperidine-2,6-dione;
3-(5-(6-((4'-chloro-[1,1'-biphenyl]-2-yl)methyl)-3,6-diazabicyclo[3.1.1]heptane-3-carbonyl)-1-oxoisoindolin-2-yl)piperidine-2,6-dione;
3-(5-(3-((4'-chloro-[1,1'-biphenyl]-2-yl)methyl)-3,8-diazabicyclo[3.2.1]octane-8-carbonyl)-1-oxoisoindolin-2-yl)piperidine-2,6-dione;

3-(5-(8-((4'-chloro-[1,1'-biphenyl]-2-yl)methyl)-3,8-diazabicyclo[3.2.1]octane-3-carbonyl)-1-oxoisoindolin-2-yl)piperidine-2,6-dione;
3-(5-(5-((4'-chloro-[1,1'-biphenyl]-2-yl)methyl)-2,5-diazabicyclo[2.2.2]octane-2-carbonyl)-1-oxoisoindolin-2-yl)piperidine-2,6-dione;
3-(5-((1R,4R)-5-((4'-chloro-[1,1'-biphenyl]-2-yl)methyl)-2,5-diazabicyclo[2.2.1]heptane-2-carbonyl)-1-oxoisoindolin-2-yl)piperidine-2,6-dione;
3-(5-(4-((4'-chloro-[1,1'-biphenyl]-2-yl)methyl)-2-(fluoromethyl)piperazine-1-carbonyl)-1-oxoisoindolin-2-yl)piperidine-2,6-dione;
3-(5-(4-((4'-chloro-[1,1'-biphenyl]-2-yl)methyl)-3,5-dimethylpiperazine-1-carbonyl)-1-oxoisoindolin-2-yl)piperidine-2,6-dione;
3-(5-(4-((4'-chloro-[1,1'-biphenyl]-2-yl)methyl)-2-(trifluoromethyl)piperazine-1-carbonyl)-1-oxoisoindolin-2-yl)piperidine-2,6-dione;
3-(5-(4-((4'-chloro-[1,1'-biphenyl]-2-yl)methyl)-3,3-dimethylpiperazine-1-carbonyl)-1-oxoisoindolin-2-yl)piperidine-2,6-dione;
3-(5-((4-((4'-fluoro-5,5-dimethyl-3,4,5,6-tetrahydro-[1,1'-biphenyl]-2-yl)methyl)piperazin-1-yl)methyl)-1-oxoisoindolin-2-yl)piperidine-2,6-dione;
3-(4-fluoro-5-((4-((4'-fluoro-5,5-dimethyl-3,4,5,6-tetrahydro-[1,1'-biphenyl]-2-yl)methyl)piperazin-1-yl)methyl)-1-oxoisoindolin-2-yl)piperidine-2,6-dione;
3-(6-fluoro-5-((4-((4'-fluoro-5,5-dimethyl-3,4,5,6-tetrahydro-[1,1'-biphenyl]-2-yl)methyl)piperazin-1-yl)methyl)-1-oxoisoindolin-2-yl)piperidine-2,6-dione;
3-(7-fluoro-5-((4-((4'-fluoro-5,5-dimethyl-3,4,5,6-tetrahydro-[1,1'-biphenyl]-2-yl)methyl)piperazin-1-yl)methyl)-1-oxoisoindolin-2-yl)piperidine-2,6-dione;
3-(4-((4-((4'-fluoro-5,5-dimethyl-3,4,5,6-tetrahydro-[1,1'-biphenyl]-2-yl)methyl)piperazin-1-yl)methyl)-1-oxoisoindolin-2-yl)piperidine-2,6-dione;
3-(6-((4-((4'-fluoro-5,5-dimethyl-3,4,5,6-tetrahydro-[1,1'-biphenyl]-2-yl)methyl)piperazin-1-yl)methyl)-1-oxoisoindolin-2-yl)piperidine-2,6-dione;
3-(5-((1-((4'-fluoro-5,5-dimethyl-3,4,5,6-tetrahydro-[1,1'-biphenyl]-2-yl)methyl)azetidin-3-yl)methyl)-1-oxoisoindolin-2-yl)piperidine-2,6-dione;
3-(5-((1-((4'-fluoro-5,5-dimethyl-3,4,5,6-tetrahydro-[1,1'-biphenyl]-2-yl)methyl)azetidin-3-ylidene)methyl)-1-oxoisoindolin-2-yl)piperidine-2,6-dione;
3-(5-((1-((4'-fluoro-5,5-dimethyl-3,4,5,6-tetrahydro-[1,1'-biphenyl]-2-yl)methyl)-3-hydroxyazetidin-3-yl)(hydroxy)methyl)-1-oxoisoindolin-2-yl)piperidine-2,6-dione;
3-(5-((1-((4'-fluoro-5,5-dimethyl-3,4,5,6-tetrahydro-[1,1'-biphenyl]-2-yl)methyl)piperidin-4-yl)amino)-1-oxoisoindolin-2-yl)piperidine-2,6-dione;
3-(5-((1-((4'-fluoro-5,5-dimethyl-3,4,5,6-tetrahydro-[1,1'-biphenyl]-2-yl)methyl)piperidin-4-yl)methyl)-1-oxoisoindolin-2-yl)piperidine-2,6-dione;
3-(5-(4-((4-((4'-fluoro-5,5-dimethyl-3,4,5,6-tetrahydro-[1,1'-biphenyl]-2-yl)methyl)piperazin-1-yl)methyl)phenyl)-1-oxoisoindolin-2-yl)piperidine-2,6-dione;
3-(5-((7-((4'-fluoro-5,5-dimethyl-3,4,5,6-tetrahydro-[1,1'-biphenyl]-2-yl)methyl)-2,7-diazaspiro[3.5]nonan-2-yl)methyl)-1-oxoisoindolin-2-yl)piperidine-2,6-dione;
3-(5-((6-((4'-fluoro-5,5-dimethyl-3,4,5,6-tetrahydro-[1,1'-biphenyl]-2-yl)methyl)-2,6-diazaspiro [3.3]heptan-2-yl)methyl)-1-oxoisoindolin-2-yl)piperidine-2,6-dione;
3-(5-((5-((4'-fluoro-5,5-dimethyl-3,4,5,6-tetrahydro-[1,1'-biphenyl]-2-yl)methyl)hexahydropyrrolo[3,4-c]pyrrol-2(1H)-yl)methyl)-1-oxoisoindolin-2-yl)piperidine-2,6-dione;
3-(5-((3-((4'-fluoro-5,5-dimethyl-3,4,5,6-tetrahydro-[1,1'-biphenyl]-2-yl)methyl)-3,6-diazabicyclo[3.1.1]heptan-6-yl)methyl)-1-oxoisoindolin-2-yl)piperidine-2,6-dione;
3-(5-((6-((4'-fluoro-5,5-dimethyl-3,4,5,6-tetrahydro-[1,1'-biphenyl]-2-yl)methyl)-3,6-diazabicyclo[3.1.1]heptan-3-yl)methyl)-1-oxoisoindolin-2-yl)piperidine-2,6-dione;
3-(5-((3-((4'-fluoro-5,5-dimethyl-3,4,5,6-tetrahydro-[1,1'-biphenyl]-2-yl)methyl)-3,8-diazabicyclo[3.2.1]octan-8-yl)methyl)-1-oxoisoindolin-2-yl)piperidine-2,6-dione;
3-(5-((8-((4'-fluoro-5,5-dimethyl-3,4,5,6-tetrahydro-[1,1'-biphenyl]-2-yl)methyl)-3,8-diazabicyclo[3.2.1]octan-3-yl)methyl)-1-oxoisoindolin-2-yl)piperidine-2,6-dione;
3-(5-((5-((4'-fluoro-5,5-dimethyl-3,4,5,6-tetrahydro-[1,1'-biphenyl]-2-yl)methyl)-2,5-diazabicyclo[2.2.2]octan-2-yl)methyl)-1-oxoisoindolin-2-yl)piperidine-2,6-dione;
3-(5-(((1R,4R)-5-((4'-fluoro-5,5-dimethyl-3,4,5,6-tetrahydro-[1,1'-biphenyl]-2-yl)methyl)-2,5-diazabicyclo[2.2.1]heptan-2-yl)methyl)-1-oxoisoindolin-2-yl)piperidine-2,6-dione;
3-(5-((4-((4'-fluoro-5,5-dimethyl-3,4,5,6-tetrahydro-[1,1'-biphenyl]-2-yl)methyl)-2-(fluoromethyl)piperazin-1-yl)methyl)-1-oxoisoindolin-2-yl)piperidine-2,6-dione;

3-(5-((4-((4'-fluoro-5,5-dimethyl-3,4,5,6-tetrahydro-[1,1'-biphenyl]-2-yl)methyl)-3,5-dimethylpiperazin-1-yl)methyl)-1-oxoisoindolin-2-yl)piperidine-2,6-dione;
3-(5-((4-((4'-fluoro-5,5-dimethyl-3,4,5,6-tetrahydro-[1,1'-biphenyl]-2-yl)methyl)-2-(trifluoromethyl)piperazin-1-yl)methyl)-1-oxoisoindolin-2-yl)piperidine-2,6-dione;
3-(5-((4-((4'-fluoro-5,5-dimethyl-3,4,5,6-tetrahydro-[1,1'-biphenyl]-2-yl)methyl)-3,3-dimethylpiperazin-1-yl)methyl)-1-oxoisoindolin-2-yl)piperidine-2,6-dione;
3-(5-((3-((4'-fluoro-5,5-dimethyl-3,4,5,6-tetrahydro-[1,1'-biphenyl]-2-yl)methyl)-2-oxoimidazolidin-1-yl)methyl)-1-oxoisoindolin-2-yl)piperidine-2,6-dione;
3-(4-fluoro-5-((3-((4'-fluoro-5,5-dimethyl-3,4,5,6-tetrahydro-[1,1'-biphenyl]-2-yl)methyl)-2-oxoimidazolidin-1-yl)methyl)-1-oxoisoindolin-2-yl)piperidine-2,6-dione;
3-(6-fluoro-5-((3-((4'-fluoro-5,5-dimethyl-3,4,5,6-tetrahydro-[1,1'-biphenyl]-2-yl)methyl)-2-oxoimidazolidin-1-yl)methyl)-1-oxoisoindolin-2-yl)piperidine-2,6-dione;
3-(7-fluoro-5-((3-((4'-fluoro-5,5-dimethyl-3,4,5,6-tetrahydro-[1,1'-biphenyl]-2-yl)methyl)-2-oxoimidazolidin-1-yl)methyl)-1-oxoisoindolin-2-yl)piperidine-2,6-dione;
3-(4-((3-((4'-fluoro-5,5-dimethyl-3,4,5,6-tetrahydro-[1,1'-biphenyl]-2-yl)methyl)-2-oxoimidazolidin-1-yl)methyl)-1-oxoisoindolin-2-yl)piperidine-2,6-dione;
3-(5-(8-((4'-fluoro-5,5-dimethyl-3,4,5,6-tetrahydro-[1,1'-biphenyl]-2-yl)methyl)-3,8-diazabicyclo[3.2.1]octan-3-yl)-1-oxoisoindolin-2-yl)piperidine-2,6-dione;
3-(5-(3-((4'-fluoro-5,5-dimethyl-3,4,5,6-tetrahydro-[1,1'-biphenyl]-2-yl)methyl)-3,8-diazabicyclo[3.2.1]octan-8-yl)-1-oxoisoindolin-2-yl)piperidine-2,6-dione;
3-(5-((4-(4'-fluoro-5,5-dimethyl-3,4,5,6-tetrahydro-[1,1'-biphenyl]-2-carbonyl)piperazin-1-yl)methyl)-1-oxoisoindolin-2-yl)piperidine-2,6-dione;
3-(5-((1-(4'-fluoro-5,5-dimethyl-3,4,5,6-tetrahydro-[1,1'-biphenyl]-2-carbonyl)piperidin-4-yl)methyl)-1-oxoisoindolin-2-yl)piperidine-2,6-dione;
3-(5-((1-(4'-fluoro-5,5-dimethyl-3,4,5,6-tetrahydro-[1,1'-biphenyl]-2-carbonyl)azetidin-3-yl)methyl)-1-oxoisoindolin-2-yl)piperidine-2,6-dione;
3-(5-((1-(4'-fluoro-5,5-dimethyl-3,4,5,6-tetrahydro-[1,1'-biphenyl]-2-carbonyl)azetidin-3-ylidene)methyl)-1-oxoisoindolin-2-yl)piperidine-2,6-dione;
3-(5-((1-(4'-fluoro-5,5-dimethyl-3,4,5,6-tetrahydro-[1,1'-biphenyl]-2-carbonyl)-3-hydroxyazetidin-3-yl)(hydroxy)methyl)-1-oxoisoindolin-2-yl)piperidine-2,6-dione;
3-(5-((1-(4'-fluoro-5,5-dimethyl-3,4,5,6-tetrahydro-[1,1'-biphenyl]-2-carbonyl)piperidin-4-yl)amino)-1-oxoisoindolin-2-yl)piperidine-2,6-dione;
3-(5-((7-(4'-fluoro-5,5-dimethyl-3,4,5,6-tetrahydro-[1,1'-biphenyl]-2-carbonyl)-2,7-diazaspiro[3.5]nonan-2-yl)methyl)-1-oxoisoindolin-2-yl)piperidine-2,6-dione;
3-(5-((6-(4'-fluoro-5,5-dimethyl-3,4,5,6-tetrahydro-[1,1'-biphenyl]-2-carbonyl)-2,6-diazaspiro[3.3]heptan-2-yl)methyl)-1-oxoisoindolin-2-yl)piperidine-2,6-dione;
3-(5-((5-(4'-fluoro-5,5-dimethyl-3,4,5,6-tetrahydro-[1,1'-biphenyl]-2-carbonyl)hexahydropyrrolo[3,4-c]pyrrol-2(1H)-yl)methyl)-1-oxoisoindolin-2-yl)piperidine-2,6-dione;
3-(5-((3-(4'-fluoro-5,5-dimethyl-3,4,5,6-tetrahydro-[1,1'-biphenyl]-2-carbonyl)-3,6-diazabicyclo[3.1.1]heptan-6-yl)methyl)-1-oxoisoindolin-2-yl)piperidine-2,6-dione;
3-(5-((6-(4'-fluoro-5,5-dimethyl-3,4,5,6-tetrahydro-[1,1'-biphenyl]-2-carbonyl)-3,6-diazabicyclo[3.1.1]heptan-3-yl)methyl)-1-oxoisoindolin-2-yl)piperidine-2,6-dione;
3-(5-((3-(4'-fluoro-5,5-dimethyl-3,4,5,6-tetrahydro-[1,1'-biphenyl]-2-carbonyl)-3,8-diazabicyclo[3.2.1]octan-8-yl)methyl)-1-oxoisoindolin-2-yl)piperidine-2,6-dione;
3-(5-((8-(4'-fluoro-5,5-dimethyl-3,4,5,6-tetrahydro-[1,1'-biphenyl]-2-carbonyl)-3,8-diazabicyclo[3.2.1]octan-3-yl)methyl)-1-oxoisoindolin-2-yl)piperidine-2,6-dione;
3-(5-((5-(4'-fluoro-5,5-dimethyl-3,4,5,6-tetrahydro-[1,1'-biphenyl]-2-carbonyl)-2,5-diazabicyclo[2.2.2]octan-2-yl)methyl)-1-oxoisoindolin-2-yl)piperidine-2,6-dione;
3-(5-(((1R,4R)-5-(4'-fluoro-5,5-dimethyl-3,4,5,6-tetrahydro-[1,1'-biphenyl]-2-carbonyl)-2,5-diazabicyclo[2.2.1]heptan-2-yl)methyl)-1-oxoisoindolin-2-yl)piperidine-2,6-dione;
3-(5-((4-(4'-fluoro-5,5-dimethyl-3,4,5,6-tetrahydro-[1,1'-biphenyl]-2-carbonyl)-2-(fluoromethyl)piperazin-1-yl)methyl)-1-oxoisoindolin-2-yl)piperidine-2,6-dione;
3-(5-((4-(4'-fluoro-5,5-dimethyl-3,4,5,6-tetrahydro-[1,1'-biphenyl]-2-carbonyl)-3,5-dimethylpiperazin-1-yl)methyl)-1-oxoisoindolin-2-yl)piperidine-2,6-dione;
3-(5-((4-(4'-fluoro-5,5-dimethyl-3,4,5,6-tetrahydro-[1,1'-biphenyl]-2-carbonyl)-2-(trifluoromethyl)piperazin-1-yl)methyl)-1-oxoisoindolin-2-yl)piperidine-2,6-dione;
3-(5-((4-(4'-fluoro-5,5-dimethyl-3,4,5,6-tetrahydro-[1,1'-biphenyl]-2-carbonyl)-3,3-dimethylpiperazin-1-yl)methyl)-1-oxoisoindolin-2-yl)piperidine-2,6-dione;

3-(5-(8-(4'-fluoro-5,5-dimethyl-3,4,5,6-tetrahydro-[1,1'-biphenyl]-2-carbonyl)-3,8-diazabicyclo[3.2.1]octan-3-yl)-1-oxoisoindolin-2-yl)piperidine-2,6-dione;
3-(5-(3-(4'-fluoro-5,5-dimethyl-3,4,5,6-tetrahydro-[1,1'-biphenyl]-2-carbonyl)-3,8-diazabicyclo[3.2.1]octan-8-yl)-1-oxoisoindolin-2-yl)piperidine-2,6-dione;
3-(5-(4-((4'-fluoro-5,5-dimethyl-3,4,5,6-tetrahydro-[1,1'-biphenyl]-2-yl)methyl)piperazine-1-carbonyl)-1-oxoisoindolin-2-yl)piperidine-2,6-dione;
3-(5-(3-((4'-fluoro-5,5-dimethyl-3,4,5,6-tetrahydro-[1,1'-biphenyl]-2-yl)methyl)imidazolidine-1-carbonyl)-1-oxoisoindolin-2-yl)piperidine-2,6-dione;
3-(5-(4-((4'-fluoro-5,5-dimethyl-3,4,5,6-tetrahydro-[1,1'-biphenyl]-2-yl)methyl)-1,4-diazepane-1-carbonyl)-1-oxoisoindolin-2-yl)piperidine-2,6-dione;
3-(5-(7-((4'-fluoro-5,5-dimethyl-3,4,5,6-tetrahydro-[1,1'-biphenyl]-2-yl)methyl)-2,7-diazaspiro[3.5]nonane-2-carbonyl)-1-oxoisoindolin-2-yl)piperidine-2,6-dione;
3-(5-(6-((4'-fluoro-5,5-dimethyl-3,4,5,6-tetrahydro-[1,1'-biphenyl]-2-yl)methyl)-2,6-diazaspiro[3.3]heptane-2-carbonyl)-1-oxoisoindolin-2-yl)piperidine-2,6-dione;
3-(5-(5-((4'-fluoro-5,5-dimethyl-3,4,5,6-tetrahydro-[1,1'-biphenyl]-2-yl)methyl)octahydropyrrolo[3,4-c]pyrrole-2-carbonyl)-1-oxoisoindolin-2-yl)piperidine-2,6-dione;
3-(5-(3-((4'-fluoro-5,5-dimethyl-3,4,5,6-tetrahydro-[1,1'-biphenyl]-2-yl)methyl)-3,6-diazabicyclo[3.1.1]heptane-6-carbonyl)-1-oxoisoindolin-2-yl)piperidine-2,6-dione;
3-(5-(6-((4'-fluoro-5,5-dimethyl-3,4,5,6-tetrahydro-[1,1'-biphenyl]-2-yl)methyl)-3,6-diazabicyclo[3.1.1]heptane-3-carbonyl)-1-oxoisoindolin-2-yl)piperidine-2,6-dione;
3-(5-(3-((4'-fluoro-5,5-dimethyl-3,4,5,6-tetrahydro-[1,1'-biphenyl]-2-yl)methyl)-3,8-diazabicyclo[3.2.1]octane-8-carbonyl)-1-oxoisoindolin-2-yl)piperidine-2,6-dione;
3-(5-(8-((4'-fluoro-5,5-dimethyl-3,4,5,6-tetrahydro-[1,1'-biphenyl]-2-yl)methyl)-3,8-diazabicyclo[3.2.1]octane-3-carbonyl)-1-oxoisoindolin-2-yl)piperidine-2,6-dione;
3-(5-(5-((4'-fluoro-5,5-dimethyl-3,4,5,6-tetrahydro-[1,1'-biphenyl]-2-yl)methyl)-2,5-diazabicyclo[2.2.2]octane-2-carbonyl)-1-oxoisoindolin-2-yl)piperidine-2,6-dione;
3-(5-((1R,4R)-5-((4'-fluoro-5,5-dimethyl-3,4,5,6-tetrahydro-[1,1'-biphenyl]-2-yl)methyl)-2,5-diazabicyclo[2.2.1]heptane-2-carbonyl)-1-oxoisoindolin-2-yl)piperidine-2,6-dione;
3-(5-(4-((4'-fluoro-5,5-dimethyl-3,4,5,6-tetrahydro-[1,1'-biphenyl]-2-yl)methyl)-2-(fluoromethyl)piperazine-1-carbonyl)-1-oxoisoindolin-2-yl)piperidine-2,6-dione;
3-(5-(4-((4'-fluoro-5,5-dimethyl-3,4,5,6-tetrahydro-[1,1'-biphenyl]-2-yl)methyl)-3,5-dimethylpiperazine-1-carbonyl)-1-oxoisoindolin-2-yl)piperidine-2,6-dione;
3-(5-(4-((4'-fluoro-5,5-dimethyl-3,4,5,6-tetrahydro-[1,1'-biphenyl]-2-yl)methyl)-2-(trifluoromethyl)piperazine-1-carbonyl)-1-oxoisoindolin-2-yl)piperidine-2,6-dione;
3-(5-(4-((4'-fluoro-5,5-dimethyl-3,4,5,6-tetrahydro-[1,1'-biphenyl]-2-yl)methyl)-3,3-dimethylpiperazine-1-carbonyl)-1-oxoisoindolin-2-yl)piperidine-2,6-dione;
3-(4-fluoro-5-(3-((4'-fluoro-5,5-dimethyl-3,4,5,6-tetrahydro-[1,1'-biphenyl]-2-yl)methyl)-3,6-diazabicyclo[3.1.1]heptane-6-carbonyl)-1-oxoisoindolin-2-yl)piperidine-2,6-dione;
3-(4-fluoro-5-(6-((4'-fluoro-5,5-dimethyl-3,4,5,6-tetrahydro-[1,1'-biphenyl]-2-yl)methyl)-3,6-diazabicyclo[3.1.1]heptane-3-carbonyl)-1-oxoisoindolin-2-yl)piperidine-2,6-dione;
3-(4-fluoro-5-(3-((4'-fluoro-5,5-dimethyl-3,4,5,6-tetrahydro-[1,1'-biphenyl]-2-yl)methyl)-3,8-diazabicyclo[3.2.1]octane-8-carbonyl)-1-oxoisoindolin-2-yl)piperidine-2,6-dione;
3-(4-fluoro-5-(8-((4'-fluoro-5,5-dimethyl-3,4,5,6-tetrahydro-[1,1'-biphenyl]-2-yl)methyl)-3,8-diazabicyclo[3.2.1]octane-3-carbonyl)-1-oxoisoindolin-2-yl)piperidine-2,6-dione;
3-(4-fluoro-5-(5-((4'-fluoro-5,5-dimethyl-3,4,5,6-tetrahydro-[1,1'-biphenyl]-2-yl)methyl)-2,5-diazabicyclo[2.2.2]octane-2-carbonyl)-1-oxoisoindolin-2-yl)piperidine-2,6-dione;
3-(4-fluoro-5-(4-((4'-fluoro-5,5-dimethyl-3,4,5,6-tetrahydro-[1,1'-biphenyl]-2-yl)methyl)-2-(fluoromethyl)piperazine-1-carbonyl)-1-oxoisoindolin-2-yl)piperidine-2,6-dione;
3-(4-fluoro-5-(4-((4'-fluoro-5,5-dimethyl-3,4,5,6-tetrahydro-[1,1'-biphenyl]-2-yl)methyl)-3,5-dimethylpiperazine-1-carbonyl)-1-oxoisoindolin-2-yl)piperidine-2,6-dione;
3-(4-fluoro-5-(4-((4'-fluoro-5,5-dimethyl-3,4,5,6-tetrahydro-[1,1'-biphenyl]-2-yl)methyl)-2-(trifluoromethyl)piperazine-1-carbonyl)-1-oxoisoindolin-2-yl)piperidine-2,6-dione;
3-(4-fluoro-5-(4-((4'-fluoro-5,5-dimethyl-3,4,5,6-tetrahydro-[1,1'-biphenyl]-2-yl)methyl)-3,3-dimethylpiperazine-1-carbonyl)-1-oxoisoindolin-2-yl)piperidine-2,6-dione;
3-(6-fluoro-5-(3-((4'-fluoro-5,5-dimethyl-3,4,5,6-tetrahydro-[1,1'-biphenyl]-2-yl)methyl)-3,6-diazabicyclo[3.1.1]heptane-6-carbonyl)-1-oxoisoindolin-2-yl)piperidine-2,6-dione;
3-(6-fluoro-5-(3-((4'-fluoro-5,5-dimethyl-3,4,5,6-tetrahydro-[1,1'-biphenyl]-2-yl)methyl)-3,8-diazabicyclo[3.2.1]octane-8-carbonyl)-1-oxoisoindolin-2-yl)piperidine-2,6-dione;

3-(6-fluoro-5-(5-((4'-fluoro-5,5-dimethyl-3,4,5,6-tetrahydro-[1,1'-biphenyl]-2-yl)methyl)-2,5-diazabicyclo[2.2.2]octane-2-carbonyl)-1-oxoisoindolin-2-yl)piperidine-2,6-dione;
3-(6-fluoro-5-(6-((4'-fluoro-5,5-dimethyl-3,4,5,6-tetrahydro-[1,1'-biphenyl]-2-yl)methyl)-3,6-diazabicyclo[3.1.1]heptane-3-carbonyl)-1-oxoisoindolin-2-yl)piperidine-2,6-dione;
3-(6-fluoro-5-(4-((4'-fluoro-5,5-dimethyl-3,4,5,6-tetrahydro-[1,1'-biphenyl]-2-yl)methyl)-3,5-dimethylpiperazine-1-carbonyl)-1-oxoisoindolin-2-yl)piperidine-2,6-dione;
3-(6-fluoro-5-(4-((4'-fluoro-5,5-dimethyl-3,4,5,6-tetrahydro-[1,1'-biphenyl]-2-yl)methyl)-3,3-dimethylpiperazine-1-carbonyl)-1-oxoisoindolin-2-yl)piperidine-2,6-dione;
3-(7-fluoro-5-(3-((4'-fluoro-5,5-dimethyl-3,4,5,6-tetrahydro-[1,1'-biphenyl]-2-yl)methyl)-3,6-diazabicyclo[3.1.1]heptane-6-carbonyl)-1-oxoisoindolin-2-yl)piperidine-2,6-dione;
3-(7-fluoro-5-(8-((4'-fluoro-5,5-dimethyl-3,4,5,6-tetrahydro-[1,1'-biphenyl]-2-yl)methyl)-3,8-diazabicyclo[3.2.1]octane-3-carbonyl)-1-oxoisoindolin-2-yl)piperidine-2,6-dione;
3-(7-fluoro-5-(5-((4'-fluoro-5,5-dimethyl-3,4,5,6-tetrahydro-[1,1'-biphenyl]-2-yl)methyl)-2,5-diazabicyclo[2.2.2]octane-2-carbonyl)-1-oxoisoindolin-2-yl)piperidine-2,6-dione;
3-(7-fluoro-5-(4-((4'-fluoro-5,5-dimethyl-3,4,5,6-tetrahydro-[1,1'-biphenyl]-2-yl)methyl)-3,5-dimethylpiperazine-1-carbonyl)-1-oxoisoindolin-2-yl)piperidine-2,6-dione;
3-(7-fluoro-5-(4-((4'-fluoro-5,5-dimethyl-3,4,5,6-tetrahydro-[1,1'-biphenyl]-2-yl)methyl)-3,3-dimethylpiperazine-1-carbonyl)-1-oxoisoindolin-2-yl)piperidine-2,6-dione;
3-(1-oxo-5-((4-((4',5,5-trimethyl-3,4,5,6-tetrahydro-[1,1'-biphenyl]-2-yl)methyl)piperazin-1-yl)methyl)isoindolin-2-yl)piperidine-2,6-dione;
3-(5-((4-((4'-amino-5,5-dimethyl-3,4,5,6-tetrahydro-[1,1'-biphenyl]-2-yl)methyl)piperazin-1-yl)methyl)-1-oxoisoindolin-2-yl)piperidine-2,6-dione;
3-(5-((4-((4-(4-chlorophenyl)-5,6-dihydro-2H-pyran-3-yl)methyl)piperazin-1-yl)methyl)-1-oxoisoindolin-2-yl)piperidine-2,6-dione;
3-(5-((4-((4-(4-fluorophenyl)-5,6-dihydro-2H-pyran-3-yl)methyl)piperazin-1-yl)methyl)-1-oxoisoindolin-2-yl)piperidine-2,6-dione;
3-(5-((4-((4'-chloro-4,4-difluoro-3,4,5,6-tetrahydro-[1,1'-biphenyl]-2-yl)methyl)piperazin-1-yl)methyl)-1-oxoisoindolin-2-yl)piperidine-2,6-dione;
3-(5-((4-((4'-chloro-4-methoxy-4-methyl-3,4,5,6-tetrahydro-[1,1'-biphenyl]-2-yl)methyl)piperazin-1-yl)methyl)-1-oxoisoindolin-2-yl)piperidine-2,6-dione;
3-(5-((4-((8-(4-chlorophenyl)spiro[4.5]dec-7-en-7-yl)methyl)piperazin-1-yl)methyl)-1-oxoisoindolin-2-yl)piperidine-2,6-dione;
3-(5-((4-((6-(4-chlorophenyl)spiro[3.5]non-6-en-7-yl)methyl)piperazin-1-yl)methyl)-1-oxoisoindolin-2-yl)piperidine-2,6-dione;
3-(5-(4-((4'-amino-5,5-dimethyl-3,4,5,6-tetrahydro-[1,1'-biphenyl]-2-yl)methyl)piperazine-1-carbonyl)-1-oxoisoindolin-2-yl)piperidine-2,6-dione;
3-(5-(4-((4'-fluoro-3,4,5,6-tetrahydro-[1,1'-biphenyl]-2-yl)methyl)piperazine-1-carbonyl)-1-oxoisoindolin-2-yl)piperidine-2,6-dione;
3-(5-(4-((4-(4-chlorophenyl)-5,6-dihydro-2H-pyran-3-yl)methyl)piperazine-1-carbonyl)-1-oxoisoindolin-2-yl)piperidine-2,6-dione;
3-(5-(4-((4-(4-fluorophenyl)-5,6-dihydro-2H-pyran-3-yl)methyl)piperazine-1-carbonyl)-1-oxoisoindolin-2-yl)piperidine-2,6-dione;
3-(5-(4-((4'-chloro-4,4-difluoro-3,4,5,6-tetrahydro-[1,1'-biphenyl]-2-yl)methyl)piperazine-1-carbonyl)-1-oxoisoindolin-2-yl)piperidine-2,6-dione;
3-(5-(4-((4'-chloro-4-methoxy-4-methyl-3,4,5,6-tetrahydro-[1,1'-biphenyl]-2-yl)methyl)piperazine-1-carbonyl)-1-oxoisoindolin-2-yl)piperidine-2,6-dione;
3-(5-(4-((4-(4-chlorophenyl)-6,6-dimethyl-5,6-dihydro-2H-pyran-3-yl)methyl)piperazine-1-carbonyl)-1-oxoisoindolin-2-yl)piperidine-2,6-dione;
3-(5-(4-((8-(4-chlorophenyl)spiro[4.5]dec-7-en-7-yl)methyl)piperazine-1-carbonyl)-1-oxoisoindolin-2-yl)piperidine-2,6-dione;
3-(5-(4-((6-(4-chlorophenyl)spiro[3.5]non-6-en-7-yl)methyl)piperazine-1-carbonyl)-1-oxoisoindolin-2-yl)piperidine-2,6-dione;
5-((4-((4'-chloro-5,5-dimethyl-3,4,5,6-tetrahydro-[1,1'-biphenyl]-2-yl)methyl)piperazin-1-yl)methyl)-2-(2,6-dioxopiperidin-3-yl)isoindoline-1,3-dione;
5-((4-((4'-chloro-5,5-dimethyl-3,4,5,6-tetrahydro-[1,1'-biphenyl]-2-yl)methyl)piperazin-1-yl)methyl)-2-(2,6-dioxopiperidin-3-yl)-4-fluoroisoindoline-1,3-dione;
5-((4-((4'-chloro-5,5-dimethyl-3,4,5,6-tetrahydro-[1,1'-biphenyl]-2-yl)methyl)piperazin-1-yl)methyl)-2-(2,6-dioxopiperidin-3-yl)-6-fluoroisoindoline-1,3-dione;

6-((4-((4'-chloro-5,5-dimethyl-3,4,5,6-tetrahydro-[1,1'-biphenyl]-2-yl)methyl)piperazin-1-yl)methyl)-2-(2,6-dioxopiperidin-3-yl)-4-fluoroisoindoline-1,3-dione;
4-((4-((4'-chloro-5,5-dimethyl-3,4,5,6-tetrahydro-[1,1'-biphenyl]-2-yl)methyl)piperazin-1-yl)methyl)-2-(2,6-dioxopiperidin-3-yl)isoindoline-1,3-dione;
5-((4-((4'-chloro-5,5-dimethyl-3,4,5,6-tetrahydro-[1,1'-biphenyl]-2-yl)amino)piperidin-1-yl)methyl)-2-(2,6-dioxopiperidin-3-yl)isoindoline-1,3-dione;
5-((4-((4'-chloro-5,5-dimethyl-3,4,5,6-tetrahydro-[1,1'-biphenyl]-2-yl)methyl)-2-oxopiperazin-1-yl)methyl)-2-(2,6-dioxopiperidin-3-yl)isoindoline-1,3-dione;
5-((3-((4'-chloro-5,5-dimethyl-3,4,5,6-tetrahydro-[1,1'-biphenyl]-2-yl)methyl)imidazolidin-1-yl)methyl)-2-(2,6-dioxopiperidin-3-yl)isoindoline-1,3-dione;
5-((4-((4'-chloro-5,5-dimethyl-3,4,5,6-tetrahydro-[1,1'-biphenyl]-2-yl)methyl)-1,4-diazepan-1-yl)methyl)-2-(2,6-dioxopiperidin-3-yl)isoindoline-1,3-dione;
5-((7-((4'-chloro-5,5-dimethyl-3,4,5,6-tetrahydro-[1,1'-biphenyl]-2-yl)methyl)-2,7-diazaspiro[3.5]nonan-2-yl)methyl)-2-(2,6-dioxopiperidin-3-yl)isoindoline-1,3-dione;
5-((6-((4'-chloro-5,5-dimethyl-3,4,5,6-tetrahydro-[1,1'-biphenyl]-2-yl)methyl)-2,6-diazaspiro[3.3]heptan-2-yl)methyl)-2-(2,6-dioxopiperidin-3-yl)isoindoline-1,3-dione;
5-((5-((4'-chloro-5,5-dimethyl-3,4,5,6-tetrahydro-[1,1'-biphenyl]-2-yl)methyl)hexahydropyrrolo[3,4-c]pyrrol-2(1H)-yl)methyl)-2-(2,6-dioxopiperidin-3-yl)isoindoline-1,3-dione;
5-((3-((4'-chloro-5,5-dimethyl-3,4,5,6-tetrahydro-[1,1'-biphenyl]-2-yl)methyl)-3,6-diazabicyclo[3.1.1]heptan-6-yl)methyl)-2-(2,6-dioxopiperidin-3-yl)isoindoline-1,3-dione;
5-((6-((4'-chloro-5,5-dimethyl-3,4,5,6-tetrahydro-[1,1'-biphenyl]-2-yl)methyl)-3,6-diazabicyclo[3.1.1]heptan-3-yl)methyl)-2-(2,6-dioxopiperidin-3-yl)isoindoline-1,3-dione;
5-((3-((4'-chloro-5,5-dimethyl-3,4,5,6-tetrahydro-[1,1'-biphenyl]-2-yl)methyl)-3,8-diazabicyclo[3.2.1]octan-8-yl)methyl)-2-(2,6-dioxopiperidin-3-yl)isoindoline-1,3-dione;
5-((8-((4'-chloro-5,5-dimethyl-3,4,5,6-tetrahydro-[1,1'-biphenyl]-2-yl)methyl)-3,8-diazabicyclo[3.2.1]octan-3-yl)methyl)-2-(2,6-dioxopiperidin-3-yl)isoindoline-1,3-dione;
5-((5-((4'-chloro-5,5-dimethyl-3,4,5,6-tetrahydro-[1,1'-biphenyl]-2-yl)methyl)-2,5-diazabicyclo[2.2.2]octan-2-yl)methyl)-2-(2,6-dioxopiperidin-3-yl)isoindoline-1,3-dione;
5-(((1R,4R)-5-((4'-chloro-5,5-dimethyl-3,4,5,6-tetrahydro-[1,1'-biphenyl]-2-yl)methyl)-2,5-diazabicyclo[2.2.1]heptan-2-yl)methyl)-2-(2,6-dioxopiperidin-3-yl)isoindoline-1,3-dione;
5-((4-((4'-chloro-5,5-dimethyl-3,4,5,6-tetrahydro-[1,1'-biphenyl]-2-yl)methyl)-2-(£1uoromethyl)piperazin-l-yl)methyl)-2-(2,6-dioxopiperidin-3-yl)isoindoline-l,3-dione;
5-((4-((4'-chloro-5,5-dimethyl-3,4,5,6-tetrahydro-[1,1'-biphenyl]-2-yl)methyl)-3,5-dimethylpiperazin-1-yl)methyl)-2-(2,6-dioxopiperidin-3-yl)isoindoline-1,3-dione;
5-((4-((4'-chloro-5,5-dimethyl-3,4,5,6-tetrahydro-[1,1'-biphenyl]-2-yl)methyl)-2-(trifluoromethyl)piperazin-1-yl)methyl)-2-(2,6-dioxopiperidin-3-yl)isoindoline-1,3-dione;
5-((4-((4'-chloro-5,5-dimethyl-3,4,5,6-tetrahydro-[1,1'-biphenyl]-2-yl)methyl)-3,3-dimethylpiperazin-1-yl)methyl)-2-(2,6-dioxopiperidin-3-yl)isoindoline-1,3-dione;
5-((3-((4'-chloro-5,5-dimethyl-3,4,5,6-tetrahydro-[1,1'-biphenyl]-2-yl)methyl)-2-oxoimidazolidin-1-yl)methyl)-2-(2,6-dioxopiperidin-3-yl)isoindoline-1,3-dione;
2-(2,6-dioxopiperidin-3-yl)-5-((4-((4'-fluoro-5, 5-dimethyl-3,4, 5,6-tetrahydro-[ 1,1'-biphenyl]-2-yl)methyl)piperazin-1-yl)methyl)isoindoline-1,3-dione;
5-((4-((4'-chloro-[1,1'-biphenyl]-2-yl)methyl)piperazin-1-yl)methyl)-2-(2,6-dioxopiperidin-3-yl)isoindoline-1,3-dione;
5-((4-((4'-chloro-[1,1'-biphenyl]-3-yl)methyl)piperazin-1-yl)methyl)-2-(2,6-dioxopiperidin-3-yl)isoindoline-1,3-dione;
5-((4-((4'-chloro-[1,1'-biphenyl]-4-yl)methyl)piperazin-1-yl)methyl)-2-(2,6-dioxopiperidin-3-yl)isoindoline-1,3-dione;
5-((4-((3-(4-chlorophenyl)pyridin-4-yl)methyl)piperazin-1-yl)methyl)-2-(2,6-dioxopiperidin-3-yl)isoindoline-1,3-dione;
5-((4-([2,4'-bipyridin]-5-ylmethyl)piperazin-1-yl)methyl)-2-(2,6-dioxopiperidin-3-yl)isoindoline-1,3-dione;
2-(2,6-dioxopiperidin-3-yl)-5-((4-((5-phenylpyrazin-2-yl)methyl)piperazin-1-yl)methyl)isoindoline-1,3-dione;
5-((4-((5-(4-chlorophenyl)thiophen-2-yl)methyl)piperazin-1-yl)methyl)-2-(2,6-dioxopiperidin-3-yl)isoindoline-1,3-dione;
2-(2,6-dioxopiperidin-3-yl)-5-((4-((5-(thiophen-2-yl)furan-2-yl)methyl)piperazin-1-yl)methyl)isoindoline-1,3-dione;
2-(2,6-dioxopiperidin-3-yl)-5-((4-((4',5,5-trimethyl-3,4,5,6-tetrahydro-[1,1'-biphenyl]-2-yl)methyl)piperazin-1-yl)methyl)isoindoline-1,3-dione;

5-((4-((4'-amino-5,5-dimethyl-3,4,5,6-tetrahydro-[1,1'-biphenyl]-2-yl)methyl)piperazin-1-yl)methyl)-2-(2,6-dioxopiperidin-3-yl)isoindoline-1,3-dione;
2-(2,6-dioxopiperidin-3-yl)-5-((4-((4'-fluoro-3,4,5,6-tetrahydro-[1,1'-biphenyl]-2-yl)methyl)piperazin-1-yl)methyl)isoindoline-1,3-dione;
5-((4-((4-(4-chlorophenyl)-5,6-dihydro-2H-pyran-3-yl)methyl)piperazin-1-yl)methyl)-2-(2,6-dioxopiperidin-3-yl)isoindoline-1,3-dione;
5-((4-((4'-chloro-4,4-difluoro-3,4,5,6-tetrahydro-[1,1'-biphenyl]-2-yl)methyl)piperazin-1-yl)methyl)-2-(2,6-dioxopiperidin-3-yl)isoindoline-1,3-dione;
5-((4-((4'-chloro-4-methoxy-4-methyl-3,4,5,6-tetrahydro-[1,1'-biphenyl]-2-yl)methyl)piperazin-1-yl)methyl)-2-(2,6-dioxopiperidin-3-yl)isoindoline-1,3-dione;
5-((4-((4-(4-chlorophenyl)-6,6-dimethyl-5,6-dihydro-2H-pyran-3-yl)methyl)piperazin-1-yl)methyl)-2-(2,6-dioxopiperidin-3-yl)isoindoline-1,3-dione;
5-((4-((8-(4-chlorophenyl)spiro[4.5]dec-7-en-7-yl)methyl)piperazin-1-yl)methyl)-2-(2,6-dioxopiperidin-3-yl)isoindoline-1,3-dione;
5-((4-((6-(4-chlorophenyl)spiro[3.5]non-6-en-7-yl)methyl)piperazin-1-yl)methyl)-2-(2,6-dioxopiperidin-3-yl)isoindoline-1,3-dione;
5-(4-((4'-chloro-5,5-dimethyl-3,4,5,6-tetrahydro-[1,1'-biphenyl]-2-yl)methyl)piperazine-1-carbonyl)-2-(2,6-dioxopiperidin-3-yl)isoindoline-1,3-dione;
5-(4-((4'-chloro-5,5-dimethyl-3,4,5,6-tetrahydro-[1,1'-biphenyl]-2-yl)methyl)piperazine-1-carbonyl)-2-(2,6-dioxopiperidin-3-yl)-4-fluoroisoindoline-1,3-dione;
5-(4-((4'-chloro-5,5-dimethyl-3,4,5,6-tetrahydro-[1,1'-biphenyl]-2-yl)methyl)piperazine-1-carbonyl)-2-(2,6-dioxopiperidin-3-yl)-6-fluoroisoindoline-1,3-dione;
6-(4-((4'-chloro-5,5-dimethyl-3,4,5,6-tetrahydro-[1,1'-biphenyl]-2-yl)methyl)piperazine-1-carbonyl)-2-(2,6-dioxopiperidin-3-yl)-4-fluoroisoindoline-1,3-dione;
5-(3-((4'-chloro-5,5-dimethyl-3,4,5,6-tetrahydro-[1,1'-biphenyl]-2-yl)methyl)imidazolidine-1-carbonyl)-2-(2,6-dioxopiperidin-3-yl)isoindoline-1,3-dione;
5-(4-((4'-chloro-5,5-dimethyl-3,4,5,6-tetrahydro-[1,1'-biphenyl]-2-yl)methyl)-1,4-diazepane-1-carbonyl)-2-(2,6-dioxopiperidin-3-yl)isoindoline-1,3-dione;
5-(7-((4'-chloro-5,5-dimethyl-3,4,5,6-tetrahydro-[1,1'-biphenyl]-2-yl)methyl)-2,7-diazaspiro[3.5]nonane-2-carbonyl)-2-(2,6-dioxopiperidin-3-yl)isoindoline-1,3-dione;
5-(6-((4'-chloro-5,5-dimethyl-3,4,5,6-tetrahydro-[1,1'-biphenyl]-2-yl)methyl)-2,6-diazaspiro[3.3]heptane-2-carbonyl)-2-(2,6-dioxopiperidin-3-yl)isoindoline-1,3-dione;
5-(5-((4'-chloro-5,5-dimethyl-3,4,5,6-tetrahydro-[1,1'-biphenyl]-2-yl)methyl)octahydropyrrolo[3,4-c]pyrrole-2-carbonyl)-2-(2,6-dioxopiperidin-3-yl)isoindoline-1,3-dione;
5-(3-((4'-chloro-5,5-dimethyl-3,4,5,6-tetrahydro-[1,1'-biphenyl]-2-yl)methyl)-3,6-diazabicyclo[3.1.1]heptane-6-carbonyl)-2-(2,6-dioxopiperidin-3-yl)isoindoline-1,3-dione;
5-(3-((4'-chloro-5,5-dimethyl-3,4,5,6-tetrahydro-[1,1'-biphenyl]-2-yl)methyl)-3,8-diazabicyclo[3.2.1]octane-8-carbonyl)-2-(2,6-dioxopiperidin-3-yl)isoindoline-1,3-dione;
5-(6-((4'-chloro-5,5-dimethyl-3,4,5,6-tetrahydro-[1,1'-biphenyl]-2-yl)methyl)-3,6-diazabicyclo[3.1.1]heptane-3-carbonyl)-2-(2,6-dioxopiperidin-3-yl)isoindoline-1,3-dione;
5-(8-((4'-chloro-5,5-dimethyl-3,4,5,6-tetrahydro-[1,1'-biphenyl]-2-yl)methyl)-3,8-diazabicyclo[3.2.1]octane-3-carbonyl)-2-(2,6-dioxopiperidin-3-yl)isoindoline-1,3-dione;
5-(5-((4'-chloro-5,5-dimethyl-3,4,5,6-tetrahydro-[1,1'-biphenyl]-2-yl)methyl)-2,5-diazabicyclo[2.2.2]octane-2-carbonyl)-2-(2,6-dioxopiperidin-3-yl)isoindoline-1,3-dione;
5-(4-((4'-chloro-5,5-dimethyl-3,4,5,6-tetrahydro-[1,1'-biphenyl]-2-yl)methyl)-2-(fluoromethyl)piperazine-1-carbonyl)-2-(2,6-dioxopiperidin-3-yl)isoindoline-1,3-dione;
5-((1R,4R)-5-((4'-chloro-5,5-dimethyl-3,4,5,6-tetrahydro-[1,1'-biphenyl]-2-yl)methyl)-2,5-diazabicyclo[2.2.1]heptane-2-carbonyl)-2-(2,6-dioxopiperidin-3-yl)isoindoline-1,3-dione;
5-(4-((4'-chloro-5,5-dimethyl-3,4,5,6-tetrahydro-[1,1'-biphenyl]-2-yl)methyl)-3,5-dimethylpiperazine-1-carbonyl)-2-(2,6-dioxopiperidin-3-yl)isoindoline-1,3-dione;
5-(4-((4'-chloro-5,5-dimethyl-3,4,5,6-tetrahydro-[1,1'-biphenyl]-2-yl)methyl)-3,3-dimethylpiperazine-1-carbonyl)-2-(2,6-dioxopiperidin-3-yl)isoindoline-1,3-dione;
5-(4-((4'-chloro-5,5-dimethyl-3,4,5,6-tetrahydro-[1,1'-biphenyl]-2-yl)methyl)-2-(trifluoromethyl)piperazine-1-carbonyl)-2-(2,6-dioxopiperidin-3-yl)isoindoline-1,3-dione;
2-(2,6-dioxopiperidin-3-yl)-5-(4-((4'-fluoro-5,5-dimethyl-3,4,5,6-tetrahydro-[1,1'-biphenyl]-2-yl)methyl)piperazine-1-carbonyl)isoindoline-1,3-dione;
5-(4-((4'-chloro-[1,1'-biphenyl]-2-yl)methyl)piperazine-1-carbonyl)-2-(2,6-dioxopiperidin-3-yl)isoindoline-1,3-dione;

5-(4-((4'-chloro-[1,1'-biphenyl]-3-yl)methyl)piperazine-1-carbonyl)-2-(2,6-dioxopiperidin-3-yl)isoindoline-1,3-dione;
5-(4-((4'-chloro-[1,1'-biphenyl]-4-yl)methyl)piperazine-l-carbonyl)-2-(2,6-dioxopiperidin-3-yl)isoindoline-1,3-dione;
5-(4-((3-(4-chlorophenyl)pyridin-4-yl)methyl)piperazine-1-carbonyl)-2-(2,6-dioxopiperidin-3-yl)isoindoline-1,3-dione;
2-(2,6-dioxopiperidin-3-yl)-5-(4-((4',5,5-trimethyl-3,4,5,6-tetrahydro-[1,1'-biphenyl]-2-yl)methyl)piperazine-1-carbonyl)isoindoline-1,3-dione;
5-(4-((4'-amino-5,5-dimethyl-3,4,5,6-tetrahydro-[1,1'-biphenyl]-2-yl)methyl)piperazine-1-carbonyl)-2-(2,6-dioxopiperidin-3-yl)isoindoline-1,3-dione;
2-(2,6-dioxopiperidin-3-yl)-5-(4-((4'-fluoro-3,4,5,6-tetrahydro-[1,1'-biphenyl]-2-yl)methyl)piperazine-1-carbonyl)isoindoline-1,3-dione;
5-(4-((4-(4-chlorophenyl)-5,6-dihydro-2H-pyran-3-yl)methyl)piperazine-1-carbonyl)-2-(2,6-dioxopiperidin-3-yl) isoindoline-1,3-dione;
5-(4-((4'-chloro-4,4-difluoro-3,4,5,6-tetrahydro-[1,1'-biphenyl]-2-yl)methyl)piperazine-1-carbonyl)-2-(2,6-dioxopiperidin-3-yl)isoindoline-1,3-dione;
5-(4-((4'-chloro-4-methoxy-4-methyl-3,4,5,6-tetrahydro-[1,1'-biphenyl]-2-yl)methyl)piperazine-1-carbonyl)-2-(2,6-dioxopiperidin-3-yl)isoindoline-1,3-dione;
5-(4-((4-(4-chlorophenyl)-6,6-dimethyl-5,6-dihydro-2H-pyran-3-yl)methyl)piperazine-1-carbonyl)-2-(2,6-dioxopiperidin-3-yl)isoindoline-1,3-dione;
5-(4-((8-(4-chlorophenyl)spiro[4.5]dec-7-en-7-yl)methyl)piperazine-1-carbonyl)-2-(2,6-dioxopiperidin-3-yl)isoindoline-1,3-dione;
5-(4-((6-(4-chlorophenyl)spiro[3.5]non-6-en-7-yl)methyl)piperazine-1-carbonyl)-2-(2,6-dioxopiperidin-3-yl)isoindoline-1,3-dione;
5-(3-((4'-chloro-5,5-dimethyl-3,4,5,6-tetrahydro-[1,1'-biphenyl]-2-yl)methyl)-3,8-diazabicyclo[3.2.1]octan-8-yl)-2-(2,6-dioxopiperidin-3-yl)isoindoline-1,3-dione;
5-(8-((4'-chloro-5,5-dimethyl-3,4,5,6-tetrahydro-[1,1'-biphenyl]-2-yl)methyl)-3,8-diazabicyclo[3.2.1]octan-3-yl)-2-(2,6-dioxopiperidin-3-yl)isoindoline-1,3-dione;
5-(3-((4'-chloro-5,5-dimethyl-3,4,5,6-tetrahydro-[1,1'-biphenyl]-2-yl)methyl)-3,6-diazabicyclo[3.1.1]heptan-6-yl)-2-(2,6-dioxopiperidin-3-yl)isoindoline-1,3-dione;
5-(6-((4'-chloro-5,5-dimethyl-3,4,5,6-tetrahydro-[1,1'-biphenyl]-2-yl)methyl)-3,6-diazabicyclo[3.1.1]heptan-3-yl)-2-(2,6-dioxopiperidin-3-yl)isoindoline-1,3-dione;
5-(5-((4'-chloro-5,5-dimethyl-3,4,5,6-tetrahydro-[1,1'-biphenyl]-2-yl)methyl)-2,5-diazabicyclo[2.2.2]octan-2-yl)-2-(2,6-dioxopiperidin-3-yl)isoindoline-1,3-dione;
5-(3-((4'-chloro-5,5-dimethyl-3,4,5,6-tetrahydro-[1,1'-biphenyl]-2-yl)methyl)-3,8-diazabicyclo[3.2.1]octan-8-yl)-2-(2,6-dioxopiperidin-3-yl)-6-fluoroisoindoline-1,3-dione;
5-(8-((4'-chloro-5,5-dimethyl-3,4,5,6-tetrahydro-[1,1'-biphenyl]-2-yl)methyl)-3,8-diazabicyclo[3.2.1]octan-3-yl)-2-(2,6-dioxopiperidin-3-yl)-6-fluoroisoindoline-1,3-dione;
5-(3-((4'-chloro-5,5-dimethyl-3,4,5,6-tetrahydro-[1,1'-biphenyl]-2-yl)methyl)-3,6-diazabicyclo[3.1.1]heptan-6-yl)-2-(2,6-dioxopiperidin-3-yl)-6-fluoroisoindoline-1,3-dione;
5-(6-((4'-chloro-5,5-dimethyl-3,4,5,6-tetrahydro-[1,1'-biphenyl]-2-yl)methyl)-3,6-diazabicyclo[3.1.1]heptan-3-yl)-2-(2,6-dioxopiperidin-3-yl)-6-fluoroisoindoline-1,3-dione;
5-(5-((4'-chloro-5,5-dimethyl-3,4,5,6-tetrahydro-[1,1'-biphenyl]-2-yl)methyl)-2,5-diazabicyclo[2.2.2]octan-2-yl)-2-(2,6-dioxopiperidin-3-yl)-6-fluoroisoindoline-1,3-dione;
5-(3-((4'-chloro-[1,1'-biphenyl]-2-yl)methyl)-3,8-diazabicyclo[3.2.1]octan-8-yl)-2-(2,6-dioxopiperidin-3-yl)isoindoline-1,3-dione;
5-(8-((4'-chloro-[1,1'-biphenyl]-2-yl)methyl)-3,8-diazabicyclo[3.2.1]octan-3-yl)-2-(2,6-dioxopiperidin-3-yl)isoindoline-1,3-dione;
5-(3-((4'-chloro-[1,1'-biphenyl]-2-yl)methyl)-3,6-diazabicyclo[3.1.1]heptan-6-yl)-2-(2,6-dioxopiperidin-3-yl)isoindoline-1,3-dione;
5-(6-((4'-chloro-[1,1'-biphenyl]-2-yl)methyl)-3,6-diazabicyclo[3.1.1]heptan-3-yl)-2-(2,6-dioxopiperidin-3-yl)isoindoline-1,3-dione;
5-(5-((4'-chloro-[1,1'-biphenyl]-2-yl)methyl)-2,5-diazabicyclo[2.2.2]octan-2-yl)-2-(2,6-dioxopiperidin-3-yl)isoindoline-1,3-dione;
5-(3-((4'-chloro-[1,1'-biphenyl]-2-yl)methyl)-3,8-diazabicyclo[3.2.1]octan-8-yl)-2-(2,6-dioxopiperidin-3-yl)-6-fluoroisoindoline-1,3-dione;
5-(8-((4'-chloro-[1,1'-biphenyl]-2-yl)methyl)-3,8-diazabicyclo[3.2.1]octan-3-yl)-2-(2,6-dioxopiperidin-3-yl)-6-fluoroisoindoline-1,3-dione;

5-(3-((4'-chloro-[1,1'-biphenyl]-2-yl)methyl)-3,6-diazabicyclo[3.1.1]heptan-6-yl)-2-(2,6-dioxopiperidin-3-yl)-6-fluoroisoindoline-1,3-dione;
5-(6-((4'-chloro-[1,1'-biphenyl]-2-yl)methyl)-3,6-diazabicyclo[3.1.1]heptan-3-yl)-2-(2,6-dioxopiperidin-3-yl)-6-fluoroisoindoline-1,3-dione;
5-(5-((4'-chloro-[1,1'-biphenyl]-2-yl)methyl)-2,5-diazabicyclo[2.2.2]octan-2-yl)-2-(2,6-dioxopiperidin-3-yl)-6-fluoroisoindoline-1,3-dione;
2-(2,6-dioxopiperidin-3-yl)-5-(3-((4'-fluoro-5,5-dimethyl-3,4,5,6-tetrahydro-[1,1'-biphenyl]-2-yl)methyl)-3,8-diazabicyclo[3.2.1]octan-8-yl)isoindoline-1,3-dione;
2-(2,6-dioxopiperidin-3-yl)-5-(8-((4'-fluoro-5,5-dimethyl-3,4,5,6-tetrahydro-[1,1'-biphenyl]-2-yl)methyl)-3,8-diazabicyclo[3.2.1]octan-3-yl)isoindoline-1,3-dione;
2-(2,6-dioxopiperidin-3-yl)-5-(3-((4'-fluoro-5,5-dimethyl-3,4,5,6-tetrahydro-[1,1'-biphenyl]-2-yl)methyl)-3,6-diazabicyclo[3.1.1]heptan-6-yl)isoindoline-1,3-dione;
2-(2,6-dioxopiperidin-3-yl)-5-(6-((4'-fluoro-5,5-dimethyl-3,4,5,6-tetrahydro-[1,1'-biphenyl]-2-yl)methyl)-3,6-diazabicyclo[3.1.1]heptan-3-yl)isoindoline-1,3-dione;
2-(2,6-dioxopiperidin-3-yl)-5-(5-((4'-fluoro-5,5-dimethyl-3,4,5,6-tetrahydro-[1,1'-biphenyl]-2-yl)methyl)-2,5-diazabicyclo[2.2.2]octan-2-yl)isoindoline-1,3-dione;
2-(2,6-dioxopiperidin-3-yl)-5-fluoro-6-(3-((4'-fluoro-5,5-dimethyl-3,4,5,6-tetrahydro-[1,1'-biphenyl]-2-yl)methyl)-3,8-diazabicyclo[3.2.1]octan-8-yl)isoindoline-1,3-dione;
2-(2,6-dioxopiperidin-3-yl)-5-fluoro-6-(8-((4'-fluoro-5,5-dimethyl-3,4,5,6-tetrahydro-[1,1'-biphenyl]-2-yl)methyl)-3,8-diazabicyclo[3.2.1]octan-3-yl)isoindoline-1,3-dione;
2-(2,6-dioxopiperidin-3-yl)-5-fluoro-6-(3-((4'-fluoro-5,5-dimethyl-3,4,5,6-tetrahydro-[1,1'-biphenyl]-2-yl)methyl)-3,6-diazabicyclo[3.1.1]heptan-6-yl)isoindoline-1,3-dione;
2-(2,6-dioxopiperidin-3-yl)-5-fluoro-6-(6-((4'-fluoro-5,5-dimethyl-3,4,5,6-tetrahydro-[1,1'-biphenyl]-2-yl)methyl)-3,6-diazabicyclo[3.1.1]heptan-3-yl)isoindoline-1,3-dione;
2-(2,6-dioxopiperidin-3-yl)-5-fluoro-6-(5-((4'-fluoro-5,5-dimethyl-3,4,5,6-tetrahydro-[1,1'-biphenyl]-2-yl)methyl)-2,5-diazabicyclo[2.2.2]octan-2-yl)isoindoline-1,3-dione;
3-(6-((3-((4'-chloro-5,5-dimethyl-3,4,5,6-tetrahydro-[1,1'-biphenyl]-2-yl)methyl)-3,8-diazabicyclo[3.2.1]octan-8-yl)methyl)-1-oxoisoindolin-2-yl)piperidine-2,6-dione;
3-(4-((5-((4'-chloro-5,5-dimethyl-3,4,5,6-tetrahydro-[1,1'-biphenyl]-2-yl)methyl)-2,5-diazabicyclo[2.2.2]octan-2-yl)methyl)-1-oxoisoindolin-2-yl)piperidine-2,6-dione;
3-(6-((5-((4'-chloro-5,5-dimethyl-3,4,5,6-tetrahydro-[1,1'-biphenyl]-2-yl)methyl)-2,5-diazabicyclo[2.2.2]octan-2-yl)methyl)-1-oxoisoindolin-2-yl)piperidine-2,6-dione;
3-(5-((5-(4'-chloro-5,5-dimethyl-3,4,5,6-tetrahydro-[1,1'-biphenyl]-2-carbonyl)-2,5-diazabicyclo[2.2.1]heptan-2-yl)methyl)-1-oxoisoindolin-2-yl)piperidine-2,6-dione;
3-(5-(5-((4'-chloro-5,5-dimethyl-3,4,5,6-tetrahydro-[1,1'-biphenyl]-2-yl)methyl)-2,5-diazabicyclo[2.2.1]heptane-2-carbonyl)-1-oxoisoindolin-2-yl)piperidine-2,6-dione;
3-(4-fluoro-5-((4-((4'-fluoro-3,4,5,6-tetrahydro-[1,1'-biphenyl]-2-yl)methyl)piperazin-1-yl)methyl)-1-oxoisoindolin-2-yl)piperidine-2,6-dione;
3-(4-((4-((4'-fluoro-3,4,5,6-tetrahydro-[1,1'-biphenyl]-2-yl)methyl)piperazin-1-yl)methyl)-1-oxoisoindolin-2-yl)piperidine-2,6-dione;
3-(6-((4-((4'-fluoro-3,4,5,6-tetrahydro-[1,1'-biphenyl]-2-yl)methyl)piperazin-1-yl)methyl)-1-oxoisoindolin-2-yl)piperidine-2,6-dione;
3-(4-fluoro-5-((4-(4'-fluoro-3,4,5,6-tetrahydro-[1,1'-biphenyl]-2-carbonyl)piperazin-1-yl)methyl)-1-oxoisoindolin-2-yl)piperidine-2,6-dione;
3-(6-fluoro-5-((4-(4'-fluoro-3,4,5,6-tetrahydro-[1,1'-biphenyl]-2-carbonyl)piperazin-1-yl)methyl)-1-oxoisoindolin-2-yl)piperidine-2,6-dione;
3-(7-fluoro-5-((4-(4'-fluoro-3,4,5,6-tetrahydro-[1,1'-biphenyl]-2-carbonyl)piperazin-1-yl)methyl)-1-oxoisoindolin-2-yl)piperidine-2,6-dione;
3-(4-((4-(4'-fluoro-3,4,5,6-tetrahydro-[1,1'-biphenyl]-2-carbonyl)piperazin-1-yl)methyl)-1-oxoisoindolin-2-yl)piperidine-2,6-dione;
3-(6-((4-(4'-fluoro-3,4,5,6-tetrahydro-[1,1'-biphenyl]-2-carbonyl)piperazin-1-yl)methyl)-1-oxoisoindolin-2-yl)piperidine-2,6-dione;
3-(4-fluoro-5-((3-(4'-fluoro-3,4,5,6-tetrahydro-[1,1'-biphenyl]-2-carbonyl)-3,6-diazabicyclo[3.1.1]heptan-6-yl)methyl)-1-oxoisoindolin-2-yl)piperidine-2,6-dione;
3-(7-fluoro-5-((3-(4'-fluoro-3,4,5,6-tetrahydro-[1,1'-biphenyl]-2-carbonyl)-3,6-diazabicyclo[3.1.1]heptan-6-yl)methyl)-1-oxoisoindolin-2-yl)piperidine-2,6-dione;
3-(4-((3-(4'-fluoro-3,4,5,6-tetrahydro-[1,1'-biphenyl]-2-carbonyl)-3,6-diazabicyclo[3.1.1]heptan-6-yl)methyl)-1-oxoisoindolin-2-yl)piperidine-2,6-dione;

3-(6-((3-(4'-fluoro-3,4,5,6-tetrahydro-[1,1'-biphenyl]-2-carbonyl)-3,6-diazabicyclo[3.1.1]heptan-6-yl)methyl)-1-oxoisoindolin-2-yl)piperidine-2,6-dione;
3-(7-((3-(4'-fluoro-3,4,5,6-tetrahydro-[1,1'-biphenyl]-2-carbonyl)-3,6-diazabicyclo[3.1.1]heptan-6-yl)methyl)-1-oxoisoindolin-2-yl)piperidine-2,6-dione;
3-(4-((4-((4'-chloro-[1,1'-biphenyl]-2-yl)amino)piperidin-1-yl)methyl)-1-oxoisoindolin-2-yl)piperidine-2,6-dione;
3-(5-((5-((4'-chloro-[1,1'-biphenyl]-2-yl)methyl)-2,5-diazabicyclo[2.2.1]heptan-2-yl)methyl)-1-oxoisoindolin-2-yl)piperidine-2,6-dione;
3-(5-((5-(4'-chloro-[1,1'-biphenyl]-2-carbonyl)-2,5-diazabicyclo[2.2.1]heptan-2-yl)methyl)-1-oxoisoindolin-2-yl) piperidine-2,6-dione;
3-(5-(5-((4'-chloro-[1,1'-biphenyl]-2-yl)methyl)-2,5-diazabicyclo[2.2.1]heptane-2-carbonyl)-1-oxoisoindolin-2-yl)piperidine-2,6-dione;
3-(5-((5-((4'-fluoro-5,5-dimethyl-3,4,5,6-tetrahydro-[1,1'-biphenyl]-2-yl)methyl)-2,5-diazabicyclo[2.2.1]heptan-2-yl)methyl)-1-oxoisoindolin-2-yl)piperidine-2,6-dione;
3-(5-((5-(4'-fluoro-5,5-dimethyl-3,4,5,6-tetrahydro-[1,1'-biphenyl]-2-carbonyl)-2,5-diazabicyclo[2.2.1]heptan-2-yl)methyl)-1-oxoisoindolin-2-yl)piperidine-2,6-dione;
3-(5-(5-((4'-fluoro-5,5-dimethyl-3,4,5,6-tetrahydro-[1,1'-biphenyl]-2-yl)methyl)-2,5-diazabicyclo[2.2.1]heptane-2-carbonyl)-1-oxoisoindolin-2-yl)piperidine-2,6-dione;
3-(5-(4-(4'-fluoro-3,4,5,6-tetrahydro-[1,1'-biphenyl]-2-carbonyl)piperazine-1-carbonyl)-1-oxoisoindolin-2-yl)piperidine-2,6-dione;
3-(5-(3-(4'-fluoro-3,4,5,6-tetrahydro-[1,1'-biphenyl]-2-carbonyl)-3,6-diazabicyclo[3.1.1]heptane-6-carbonyl)-1-oxoisoindolin-2-yl)piperidine-2,6-dione;
2-(2,6-dioxopiperidin-3-yl)-4-((4-((4'-fluoro-3,4,5,6-tetrahydro-[1,1'-biphenyl]-2-yl)methyl)piperazin-1-yl) methyl)isoindoline-1,3-dione;
5-((5-((4'-chloro-5,5-dimethyl-3,4,5,6-tetrahydro-[1,1'-biphenyl]-2-yl)methyl)-2,5-diazabicyclo[2.2.1]heptan-2-yl)methyl)-2-(2,6-dioxopiperidin-3-yl)isoindoline-1,3-dione;
2-(2,6-dioxopiperidin-3-yl)-5-((4-(4'-fluoro-3,4,5,6-tetrahydro-[1,1'-biphenyl]-2-carbonyl)piperazin-1-yl)methyl) isoindoline-1,3-dione;
2-(2,6-dioxopiperidin-3-yl)-5-((3-(4'-fluoro-3,4,5,6-tetrahydro-[1,1'-biphenyl]-2-carbonyl)-3,6-diazabicyclo [3.1.1]heptan-6-yl)methyl)isoindoline-1,3-dione;
5-(4-((4'-chloro-[1,1'-biphenyl]-2-yl)amino)piperidine-1-carbonyl)-2-(2,6-dioxopiperidin-3-yl)isoindoline-1,3-dione;
2-(2,6-dioxopiperidin-3-yl)-5-(4-(4'-fluoro-3,4,5,6-tetrahydro-[1,1'-biphenyl]-2-carbonyl)piperazine-1-carbonyl) isoindoline-1,3-dione;
2-(2,6-dioxopiperidin-3-yl)-5-(3-(4'-fluoro-3,4,5,6-tetrahydro-[1,1'-biphenyl]-2-carbonyl)-3,6-diazabicyclo[3.1.1] heptane-6-carbonyl)isoindoline-1,3-dione;
3-(5-(((1S,4S)-5-((4'-chloro-5,5-dimethyl-3,4,5,6-tetrahydro-[1,1'-biphenyl]-2-yl)methyl)-2,5-diazabicyclo [2.2.1]heptan-2-yl)methyl)-1-oxoisoindolin-2-yl)piperidine-2,6-dione;
3-(5-(((1S,4S)-5-(4'-chloro-5,5-dimethyl-3,4,5,6-tetrahydro-[1,1'-biphenyl]-2-carbonyl)-2,5-diazabicyclo[2.2.1] heptan-2-yl)methyl)-1-oxoisoindolin-2-yl)piperidine-2,6-dione;
3-(5-((1S,4S)-5-((4'-chloro-5,5-dimethyl-3,4,5,6-tetrahydro-[1,1'-biphenyl]-2-yl)methyl)-2,5-diazabicyclo[2.2.1] heptane-2-carbonyl)-1-oxoisoindolin-2-yl)piperidine-2,6-dione;
3-(5-(7-(4'-chloro-5,5-dimethyl-3,4,5,6-tetrahydro-[1,1'-biphenyl]-2-carbonyl)-2,7-diazaspiro[3.5]nonane-2-carbonyl)-1-oxoisoindolin-2-yl)piperidine-2,6-dione;
3-(5-(6-(4'-chloro-5,5-dimethyl-3,4,5,6-tetrahydro-[1,1'-biphenyl]-2-carbonyl)-2,6-diazaspiro[3.3]heptane-2-carbonyl)-1-oxoisoindolin-2-yl)piperidine-2,6-dione;
3-(5-(5-(4'-chloro-5,5-dimethyl-3,4,5,6-tetrahydro-[1,1'-biphenyl]-2-carbonyl)octahydropyrrolo[3,4-c]pyrrole-2-carbonyl)-1-oxoisoindolin-2-yl)piperidine-2,6-dione;
3-(5-(((1S,4S)-5-((4'-chloro-[1,1'-biphenyl]-2-yl)methyl)-2,5-diazabicyclo[2.2.1]heptan-2-yl)methyl)-1-oxoisoindolin-2-yl)piperidine-2,6-dione;
3-(1-oxo-5-((4-((2-phenylpyrimidin-5-yl)methyl)piperazin-1-yl)methyl)isoindolin-2-yl)piperidine-2,6-dione;
3-(5-(((1S,4S)-5-(4'-chloro-[1,1'-biphenyl]-2-carbonyl)-2,5-diazabicyclo[2.2.1]heptan-2-yl)methyl)-1-oxoisoindolin-2-yl)piperidine-2,6-dione;
3-(5-((1S,4S)-5-((4'-chloro-[1,1'-biphenyl]-2-yl)methyl)-2,5-diazabicyclo[2.2.1]heptane-2-carbonyl)-1-oxoisoindolin-2-yl)piperidine-2,6-dione;
3-(5-(((1S,4S)-5-((4'-fluoro-5,5-dimethyl-3,4,5,6-tetrahydro-[1,1'-biphenyl]-2-yl)methyl)-2,5-diazabicyclo[2.2.1] heptan-2-yl)methyl)-1-oxoisoindolin-2-yl)piperidine-2,6-dione;
3-(5-(((1S,4S)-5-(4'-fluoro-5,5-dimethyl-3,4,5,6-tetrahydro-[1,1'-biphenyl]-2-carbonyl)-2,5-diazabicyclo[2.2.1] heptan-2-yl)methyl)-1-oxoisoindolin-2-yl)piperidine-2,6-dione;

5-(((1S,4S)-5-((4'-chloro-5,5-dimethyl-3,4,5,6-tetrahydro-[1,1'-biphenyl]-2-yl)methyl)-2,5-diazabicyclo[2.2.1]heptan-2-yl)methyl)-2-(2,6-dioxopiperidin-3-yl)isoindoline-1,3-dione;
5-((1S,4S)-5-((4'-chloro-5,5-dimethyl-3,4,5,6-tetrahydro-[1,1'-biphenyl]-2-yl)methyl)-2,5-diazabicyclo[2.2.1]heptane-2-carbonyl)-2-(2,6-dioxopiperidin-3-yl)isoindoline-1,3-dione;
3-(6-fluoro-5-((3-(4'-fluoro-3,4,5,6-tetrahydro-[1,1'-biphenyl]-2-carbonyl)-3,6-diazabicyclo[3.1.1]heptan-6-yl)methyl)-1-oxoisoindolin-2-yl)piperidine-2,6-dione;
3-(5-((4-((4'-fluoro-5,5-dimethyl-3,4,5,6-tetrahydro-[1,1'-biphenyl]-2-yl)methyl)-1,4-diazepan-1-yl)methyl)-1-oxoisoindolin-2-yl)piperidine-2,6-dione;
3-(5-(6-(4'-chloro-5,5-dimethyl-3,4,5,6-tetrahydro-[1,1'-biphenyl]-2-carbonyl)-3,6-diazabicyclo[3.1.1]heptane-3-carbonyl)-1-oxoisoindolin-2-yl)piperidine-2,6-dione;
3-(5-((4-((4'-fluoro-5,5-dimethyl-3,4,5,6-tetrahydro-[1,1'-biphenyl]-2-yl)methyl)-1,4-diazepan-1-yl)methyl)-1-oxoisoindolin-2-yl)piperidine-2,6-dione;
3-(7-((5-((4'-chloro-5,5-dimethyl-3,4,5,6-tetrahydro-[1,1'-biphenyl]-2-yl)methyl)-2,5-diazabicyclo[2.2.2]octan-2-yl)methyl)-1-oxoisoindolin-2-yl)piperidine-2,6-dione;
3-(7-((4-(4'-fluoro-3,4,5,6-tetrahydro-[1,1'-biphenyl]-2-carbonyl)piperazin-1-yl)methyl)-1-oxoisoindolin-2-yl)piperidine-2,6-dione;
3-(5-(3-(4'-chloro-5,5-dimethyl-3,4,5,6-tetrahydro-[1,1'-biphenyl]-2-carbonyl)-3,6-diazabicyclo[3.1.1]heptane-6-carbonyl)-1-oxoisoindolin-2-yl)piperidine-2,6-dione;
3-(5-(3-(4'-chloro-5,5-dimethyl-3,4,5,6-tetrahydro-[1,1'-biphenyl]-2-carbonyl)-3,8-diazabicyclo[3.2.1]octane-8-carbonyl)-1-oxoisoindolin-2-yl)piperidine-2,6-dione;
3-(5-(8-(4'-chloro-5,5-dimethyl-3,4,5,6-tetrahydro-[1,1'-biphenyl]-2-carbonyl)-3,8-diazabicyclo[3.2.1]octane-3-carbonyl)-1-oxoisoindolin-2-yl)piperidine-2,6-dione; and
3-(1-oxo-5-((4-(5-phenylpyrazine-2-carbonyl)piperazin-1-yl)methyl)isoindolin-2-yl)piperidine-2,6-dione.

**23.** The compound of Formula (I) or salts, enantiomers, diastereoisomer, isotopically enriched analogs, solvates, prodrugs, or polymorphs thereof as claimed in any one of claims 1-22, which is hydrochlorides, sulfates, citrates, maleates, methanesulfonates, citrates, lactates, tartrates, fumarates, phosphates, dihydrophosphates, pyrophosphates, metaphosphates, oxalates, malonates, benzoates, mandelates, succinates, trifluoroacetates, hydroxyacetates, or p-toluenesulfonates of the compound of Formula (I).

**24.** A pharmaceutical composition comprising the compound of Formula (I) or pharmaceutically acceptable salts thereof as claimed in any one of claims 1-23, and at least one pharmaceutically acceptable carrier.

**25.** The pharmaceutical composition as claimed in claim 24, further comprising a second therapeutic agent, e.g., an anticancer agent.

**26.** Use of the compound of Formula (I) or pharmaceutically acceptable salts thereof as claimed in any one of claims 1-23, or the pharmaceutical composition as claimed in claim 24 or 25 for the manufacture of a medicament for the prevention and/or treatment of diseases or disorders associated with cereblon protein.

**27.** The use as claimed in claim 26, wherein the diseases or disorders associated with cereblon protein are selected from the group consisting of: tumor, infectious disease, inflammatory disease, autoimmune disease, anemia, hemorrhagic shock, transplant rejection, multiple organ dysfunction syndrome (MODS), sarcoidosis, adult respiratory distress syndrome, cardiovascular disease, Richter syndrome (RS), acute liver failure, or diabetes.

**28.** The use as claimed in claim 26, wherein the diseases or disorders associated with cereblon protein are selected from the group consisting of: myeloma, including multiple myeloma, plasma cell myeloma, smoldering myeloma, smoldering multiple myeloma; myelofibrosis; bone marrow disease; myelodysplastic syndrome (MDS); previously treated myelodysplastic syndrome; transplantation-related cancer; neutropenia; leukemia, including acute myeloid leukemia, chronic myelogenous leukemia, B-cell chronic lymphocytic leukemia, leukemia-associated anemia, acute myeloid leukemia (AML); lymphoma, including diffuse large B-cell lymphoma, non-Hodgkin's lymphoma, Hodgkin's lymphoma, anaplastic lymphoma, anaplastic large cell lymphoma, CD20 positive lymphoma, mantle cell lymphoma, primary lymphoma, B-cell lymphoma, recurrent B-cell non-Hodgkin's lymphoma, recurrent diffuse large B-cell lymphoma, recurrent mediastinal (thymic) large B-cell lymphoma, primary mediastinal (thymic) large B-cell lymphoma, recurrent transformed non-Hodgkin's lymphoma, refractory B-cell non-Hodgkin's lymphoma, refractory diffuse large B-cell lymphoma, refractory primary mediastinal (thymic) large B-cell lymphoma, refractory transformed non-Hodgkin's lymphoma; thyroid cancer; melanoma; lung cancer, including lung adenocarcinoma, lung squamous cell carcinoma; inflammatory myofibroblastoma; colorectal cancer; intestinal cancer; brain glioma; astroblastoma;

ovarian cancer; bronchial cancer; prostate cancer; breast cancer, including triple negative breast cancer, sporadic breast cancer and patients with Cowden syndrome; pancreatic cancer; central nervous system tumor; neuroblastoma; neuroglioma; Peripheral neuroepithelioma; extramedullary plasmacytoma; plasmacytoma; gastric cancer; gastrointestinal stromal tumors; esophageal cancer; colorectal adenocarcinoma; esophageal squamous cell carcinoma; liver cancer; renal cell carcinoma; bladder cancer; endometrial cancer; metrocarcinoma; head and neck cancer; brain cancer; oral cancer; sarcoma, including rhabdomyosarcoma, various lipogenic tumors, Ewing's sarcoma/primitive neuroectodermal tumors (Ewing/PNETs), and leiomyosarcoma; urothelial carcinoma; basal cell carcinoma; oral squamous cell carcinoma; cholangiocarcinoma; bone cancer; cervical cancer; skin cancer; Richter syndrome (RS); sepsis syndrome; autoimmune diseases, including rheumatoid arthritis, autoimmune encephalomyelitis, ankylosing spondylitis, psoriasis, systemic lupus erythematosus, multiple sclerosis, recurrent oral ulcers, Kawasaki disease, polymyositis/dermatomyositis, Sjogren's syndrome, and atopic dermatitis; keratoconjunctivitis; inflammatory diseases, including Crohn's disease and ulcerative colitis, pneumonia, osteoarthritis, synovitis, systemic inflammatory response syndrome, airway inflammation, bronchitis; cerebral malaria; infectious diseases, including viral pneumonia, Acquired immunodeficiency syndrome (AIDS), COVID-19 novel coronavirus infection, gram-negative bacteria infection, gram-positive bacteria infection, tuberculosis, etc.; septic shock; tuberculosis; bacterial meningitis; chronic obstructive pulmonary disease; asthma; hemorrhagic shock; organ (including kidney, heart, lung) or tissue transplantation rejection; diabetes; sarcoidosis; adult respiratory distress syndrome; anemia; pediatric aplastic anemia; cardiovascular diseases (e.g., coronary heart disease, congestive heart failure, myocardial infarction, atherosclerosis); multiple organ dysfunction caused by cachexia and septic shock; or acute liver failure.

**29.** The compound of Formula (I) or pharmaceutically acceptable salts thereof as claimed in any one of claims 1-23, for use in the prevention and/or treatment of diseases or disorders associated with cereblon protein.

**30.** The compound of Formula (I) or pharmaceutically acceptable salts thereof as claimed in claim 29, wherein the diseases or disorders associated with cereblon protein are selected from the group consisting of: tumor, infectious disease, inflammatory disease, autoimmune disease, anemia, hemorrhagic shock, transplant rejection, multiple organ dysfunction syndrome (MODS), sarcoidosis, adult respiratory distress syndrome, cardiovascular disease, Richter syndrome (RS), acute liver failure, or diabetes.

**31.** The compound of Formula (I) or pharmaceutically acceptable salts thereof as claimed in claim 29, wherein the diseases or disorders associated with cereblon protein are selected from the group consisting of: myeloma, including multiple myeloma, plasma cell myeloma, smoldering myeloma, smoldering multiple myeloma; myelofibrosis; bone marrow disease; myelodysplastic syndrome (MDS); previously treated myelodysplastic syndrome; transplantation-related cancer; neutropenia; leukemia, including acute myeloid leukemia, chronic myelogenous leukemia, B-cell chronic lymphocytic leukemia, leukemia-associated anemia, acute myeloid leukemia (AML); lymphoma, including diffuse large B-cell lymphoma, non-Hodgkin's lymphoma, Hodgkin's lymphoma, anaplastic lymphoma, anaplastic large cell lymphoma, CD20 positive lymphoma, mantle cell lymphoma, primary lymphoma, B-cell lymphoma, recurrent B-cell non-Hodgkin's lymphoma, recurrent diffuse large B-cell lymphoma, recurrent mediastinal (thymic) large B-cell lymphoma, primary mediastinal (thymic) large B-cell lymphoma, recurrent transformed non-Hodgkin's lymphoma, refractory B-cell non-Hodgkin's lymphoma, refractory diffuse large B-cell lymphoma, refractory primary mediastinal (thymic) large B-cell lymphoma, refractory transformed non-Hodgkin's lymphoma; thyroid cancer; melanoma; lung cancer, including lung adenocarcinoma, lung squamous cell carcinoma; inflammatory myofibroblastoma; colorectal cancer; intestinal cancer; brain glioma; astroblastoma; ovarian cancer; bronchial cancer; prostate cancer; breast cancer, including triple negative breast cancer, sporadic breast cancer and patients with Cowden syndrome; pancreatic cancer; central nervous system tumor; neuroblastoma; neuroglioma; Peripheral neuroepithelioma; extramedullary plasmacytoma; plasmacytoma; gastric cancer; gastrointestinal stromal tumors; esophageal cancer; colorectal adenocarcinoma; esophageal squamous cell carcinoma; liver cancer; renal cell carcinoma; bladder cancer; endometrial cancer; metrocarcinoma; head and neck cancer; brain cancer; oral cancer; sarcoma, including rhabdomyosarcoma, various lipogenic tumors, Ewing's sarcoma/primitive neuroectodermal tumors (Ewing/PNETs), and leiomyosarcoma; urothelial carcinoma; basal cell carcinoma; oral squamous cell carcinoma; cholangiocarcinoma; bone cancer; cervical cancer; skin cancer; Richter syndrome (RS); sepsis syndrome; autoimmune diseases, including rheumatoid arthritis, autoimmune encephalomyelitis, ankylosing spondylitis, psoriasis, systemic lupus erythematosus, multiple sclerosis, recurrent oral ulcers, Kawasaki disease, polymyositis/dermatomyositis, Sjogren's syndrome, and atopic dermatitis; keratoconjunctivitis; inflammatory diseases, including Crohn's disease and ulcerative colitis, pneumonia, osteoarthritis, synovitis, systemic inflammatory response syndrome, airway inflammation, bronchitis; cerebral malaria; infectious diseases, including viral pneumonia, Acquired immunodeficiency syndrome (AIDS), COVID-19 novel coronavirus infection, gram-negative bacteria infection, gram-positive bacteria infection, tuberculosis, etc.; septic shock; tuberculosis; bacterial meningitis; chronic obstructive pulmonary disease; asthma; hemorrhagic shock; organ (including kidney, heart, lung) or tissue transplantation rejection; diabetes; sarcoidosis; adult respiratory distress syndrome; anemia; pediatric aplastic anemia; cardiovascular diseases (e.g., coronary heart disease, congestive heart failure, myocardial infarction, atherosclerosis); multiple organ dysfunction caused by cachexia and septic shock; or acute liver failure.

**32.** A method for treating or preventing diseases or disorders associated with cereblon protein, comprising administering to a subject a therapeutically effective amount of the compound of Formula (I) or pharmaceutically acceptable salts thereof as claimed in any one of claims 1-23, or the pharmaceutical composition as claimed in claim 24 or 25.

**33.** The method as claimed in claim 32, wherein the diseases or disorders associated with cereblon protein are selected from the group consisting of: tumor, infectious disease, inflammatory disease, autoimmune disease, anemia, hemorrhagic shock, transplant rejection, multiple organ dysfunction syndrome (MODS), sarcoidosis, adult respiratory distress syndrome, cardiovascular disease, Richter syndrome (RS), acute liver failure, or diabetes.

**34.** The method as claimed in claim 32, wherein the diseases or disorders associated with cereblon protein are selected from the group consisting of: myeloma, including multiple myeloma, plasma cell myeloma, smoldering myeloma, smoldering multiple myeloma; myelofibrosis; bone marrow disease; myelodysplastic syndrome (MDS); previously treated myelodysplastic syndrome; transplantation-related cancer; neutropenia; leukemia, including acute myeloid leukemia, chronic myelogenous leukemia, B-cell chronic lymphocytic leukemia, leukemia-associated anemia, acute myeloid leukemia (AML); lymphoma, including diffuse large B-cell lymphoma, non-Hodgkin’s lymphoma, Hodgkin’s lymphoma, anaplastic lymphoma, anaplastic large cell lymphoma, CD20 positive lymphoma, mantle cell lymphoma, primary lymphoma, B-cell lymphoma, recurrent B-cell non-Hodgkin’s lymphoma, recurrent diffuse large B-cell lymphoma, recurrent mediastinal (thymic) large B-cell lymphoma, primary mediastinal (thymic) large B-cell lymphoma, recurrent transformed non-Hodgkin’s lymphoma, refractory B-cell non-Hodgkin’s lymphoma, refractory diffuse large B-cell lymphoma, refractory primary mediastinal (thymic) large B-cell lymphoma, refractory transformed non-Hodgkin’s lymphoma; thyroid cancer; melanoma; lung cancer, including lung adenocarcinoma, lung squamous cell carcinoma; inflammatory myofibroblastoma; colorectal cancer; intestinal cancer; brain glioma; astroblastoma; ovarian cancer; bronchial cancer; prostate cancer; breast cancer, including triple negative breast cancer, sporadic breast cancer and patients with Cowden syndrome; pancreatic cancer; central nervous system tumor; neuroblastoma; neuroglioma; Peripheral neuroepithelioma; extramedullary plasmacytoma; plasmacytoma; gastric cancer; gastrointestinal stromal tumors; esophageal cancer; colorectal adenocarcinoma; esophageal squamous cell carcinoma; liver cancer; renal cell carcinoma; bladder cancer; endometrial cancer; metrocarcinoma; head and neck cancer; brain cancer; oral cancer; sarcoma, including rhabdomyosarcoma, various lipogenic tumors, Ewing’s sarcoma/primitive neuroectodermal tumors (Ewing/PNETs), and leiomyosarcoma; urothelial carcinoma; basal cell carcinoma; oral squamous cell carcinoma; cholangiocarcinoma; bone cancer; cervical cancer; skin cancer; Richter syndrome (RS); sepsis syndrome; autoimmune diseases, including rheumatoid arthritis, autoimmune encephalomyelitis, ankylosing spondylitis, psoriasis, systemic lupus erythematosus, multiple sclerosis, recurrent oral ulcers, Kawasaki disease, polymyositis/dermatomyositis, Sjogren’s syndrome, and atopic dermatitis; keratoconjunctivitis; inflammatory diseases, including Crohn’s disease and ulcerative colitis, pneumonia, osteoarthritis, synovitis, systemic inflammatory response syndrome, airway inflammation, bronchitis; cerebral malaria; infectious diseases, including viral pneumonia, Acquired immunodeficiency syndrome (AIDS), COVID-19 novel coronavirus infection, gram-negative bacteria infection, gram-positive bacteria infection, tuberculosis, etc.; septic shock; tuberculosis; bacterial meningitis; chronic obstructive pulmonary disease; asthma; hemorrhagic shock; organ (including kidney, heart, lung) or tissue transplantation rejection; diabetes; sarcoidosis; adult respiratory distress syndrome; anemia; pediatric aplastic anemia; cardiovascular diseases (e.g., coronary heart disease, congestive heart failure, myocardial infarction, atherosclerosis); multiple organ dysfunction caused by cachexia and septic shock; or acute liver failure.

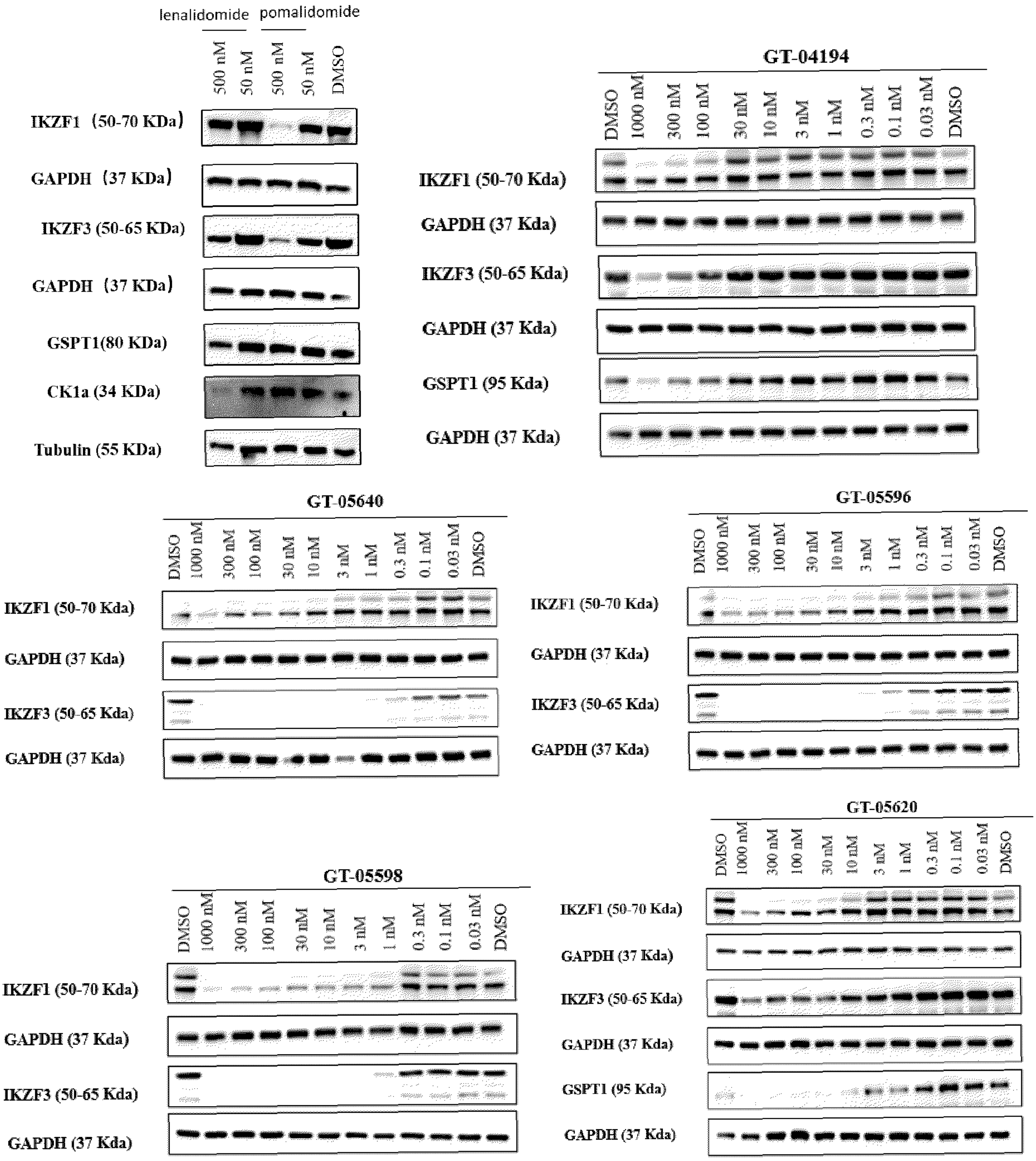
lenalidomide
pomalidomide
500 nM
50 nM
500 nM
50 nM
DMSO
IKZF1 (50-70 KDa)
GAPDH (37 KDa)
IKZF3 (50-65 KDa)
GAPDH (37 KDa)
GSPT1(80 KDa)
CK1a (34 KDa)
Tubulin (55 KDa)
GT-04194
DMSO
1000 nM
300 nM
100 nM
30 nM
10 nM
3 nM
1 nM
0.3 nM
0.1 nM
0.03 nM
DMSO
IKZF1 (50-70 Kda)
GAPDH (37 Kda)
IKZF3 (50-65 Kda)
GAPDH (37 Kda)
GSPT1 (95 Kda)
GAPDH (37 Kda)
GT-05640
IKZF1 (50-70 Kda)
GAPDH (37 Kda)
IKZF3 (50-65 Kda)
GAPDH (37 Kda)
GT-05596
IKZF1 (50-70 Kda)
GAPDH (37 Kda)
IKZF3 (50-65 Kda)
GAPDH (37 Kda)
GT-05598
IKZF1 (50-70 Kda)
GAPDH (37 Kda)
IKZF3 (50-65 Kda)
GAPDH (37 Kda)
GT-05620
IKZF1 (50-70 Kda)
GAPDH (37 Kda)
IKZF3 (50-65 Kda)
GAPDH (37 Kda)
GSPT1 (95 Kda)
GAPDH (37 Kda)

Figure 1

## INTERNATIONAL SEARCH REPORT

International application No.
**PCT/CN2023/112130**

**A. CLASSIFICATION OF SUBJECT MATTER**

C07D 401/04(2006.01)i； C07D 401/14(2006.01)i； C07D471/04(2006.01)i； C07D487/04(2006.01)i； A61K31/454(2006.01)i； A61K31/4545(2006.01)i； A61P35/00(2006.01)i

According to International Patent Classification (IPC) or to both national classification and IPC

**B. FIELDS SEARCHED**

Minimum documentation searched (classification system followed by classification symbols)

C07D A61K A61P

Documentation searched other than minimum documentation to the extent that such documents are included in the fields searched

Electronic data base consulted during the international search (name of data base and, where practicable, search terms used)

CNTXT, CNKI, ENTXT, ISI, CAPLUS(STN), REGISTRY(STN): 标新生物医药, 杨小宝, 孙仁红, 周跃东, 赵宝寅, 异吲哚啉, 异吲哚, 邻苯二甲酰亚胺, 戊二酰亚胺, 哌啶二酮, 泛素连接酶, 癌, 瘤, 结构式检索, search for structural formula, isoindoline, isoindole, phthalimide, glutarimide, cereblon, CRBN, cancer, tumor

**C. DOCUMENTS CONSIDERED TO BE RELEVANT**

| Category* | Citation of document, with indication, where appropriate, of the relevant passages | Relevant to claim No. |
|---|---|---|
| PX | WO 2022232536 A1 (NEOMORPH, INC. et al.) 03 November 2022 (2022-11-03) description, page 3 paragraph 1 to page 69 paragraph 7 | 1-34 |
| X | CN 111051298 A (NOVARTIS AG) 21 April 2020 (2020-04-21) description, paragraphs 10-743 | 1-34 |
| X | CN 111629749 A (NOVARTIS AG) 04 September 2020 (2020-09-04) description, paragraphs 855-1150 | 1-34 |
| X | WO 2021173995 A2 (NOVARTIS AG et al.) 02 September 2021 (2021-09-02) description, page 129 line 1 to page 147 line 31 | 1-34 |
| X | CN 114650868 A (DANA-FARBER CANCER INSTITUTE, INC.) 21 June 2022 (2022-06-21) description, paragraphs 8-340 | 1-34 |
| X | WO 2020160198 A1 (FOGHORN THERAPEUTICS INC.) 06 August 2020 (2020-08-06) description, page 1 line 9 to page 218 line 26 | 1-34 |

☑ Further documents are listed in the continuation of Box C. ☑ See patent family annex.

* Special categories of cited documents:
- "A" document defining the general state of the art which is not considered to be of particular relevance
- "D" document cited by the applicant in the international application
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **02 November 2023** | **10 November 2023** |

| Name and mailing address of the ISA/CN | Authorized officer |
|---|---|
| **China National Intellectual Property Administration (ISA/CN)**<br>**China No. 6, Xitucheng Road, Jimenqiao, Haidian District, Beijing 100088** | Telephone No. |

Form PCT/ISA/210 (second sheet) (July 2022)

INTERNATIONAL SEARCH REPORT

International application No.
PCT/CN2023/112130

C. DOCUMENTS CONSIDERED TO BE RELEVANT

| Category* | Citation of document, with indication, where appropriate, of the relevant passages | Relevant to claim No. |
|---|---|---|
| A | WO 2021022163 A2 (FOGHORN THERAPEUTICS INC.) 04 February 2021 (2021-02-04) description, page 1 line 9 to page 76 line 15 | 1-34 |

Form PCT/ISA/210 (second sheet) (July 2022)

**INTERNATIONAL SEARCH REPORT**

International application No.
**PCT/CN2023/112130**

**Box No. II Observations where certain claims were found unsearchable (Continuation of item 2 of first sheet)**

This international search report has not been established in respect of certain claims under Article 17(2)(a) for the following reasons:

1. ☑ Claims Nos.: **32-34**
because they relate to subject matter not required to be searched by this Authority, namely:

Claims 32-34 relate to a method for treatment or prevention of a disease in a human or animal body. However, the present search report is provided regarding a pharmaceutical use of a compound or composition.

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

**INTERNATIONAL SEARCH REPORT**
**Information on patent family members**

International application No.
**PCT/CN2023/112130**

| Patent document cited in search report | Publication date (day/month/year) | Patent family member(s) | Publication date (day/month/year) |
|---|---|---|---|
| WO 2022232536 A1 | 03 November 2022 | None | |
| CN 111051298 A | 21 April 2020 | CA 3072694 A1 | 28 February 2019 |
| | | UY 37854 A | 29 March 2019 |
| | | AU 2020277231 A1 | 24 December 2020 |
| | | AR 112529 A1 | 06 November 2019 |
| | | EP 4183782 A1 | 24 May 2023 |
| | | US 2021309638 A1 | 07 October 2021 |
| | | PT 3672949 T | 16 March 2023 |
| | | SA 520411325 B1 | 13 June 2022 |
| | | JOP 20200042 A1 | 20 February 2020 |
| | | LT 3672949 T | 11 April 2023 |
| | | US 2019359594 A1 | 28 November 2019 |
| | | US 10647701 B2 | 12 May 2020 |
| | | JP 2020531498 A | 05 November 2020 |
| | | AU 2018319577 A1 | 06 February 2020 |
| | | AU 2018319577 B2 | 15 October 2020 |
| | | AU 2022231670 A1 | 06 October 2022 |
| | | US 2020231569 A1 | 23 July 2020 |
| | | US 11053218 B2 | 06 July 2021 |
| | | MX 2020002060 A | 13 July 2020 |
| | | TW 201920143 A | 01 June 2019 |
| | | TWI 793151 B | 21 February 2023 |
| | | RS 64058 B1 | 28 April 2023 |
| | | RS 64058 B9 | 31 August 2023 |
| | | ZA 202000208 B | 28 July 2021 |
| | | US 2019062309 A1 | 28 February 2019 |
| | | US 10414755 B2 | 17 September 2019 |
| | | KR 20200044038 A | 28 April 2020 |
| | | PL 3672949 T3 | 08 May 2023 |
| | | DK 3672949 T3 | 27 March 2023 |
| | | DK 3672949 T5 | 01 May 2023 |
| | | WO 2019038717 A1 | 28 February 2019 |
| | | IL 272748 A | 30 April 2020 |
| | | IL 272748 B | 01 October 2022 |
| | | IL 272748 B2 | 01 February 2023 |
| | | CR 20200081 A | 14 May 2020 |
| | | PH 12020500125 A1 | 14 September 2020 |
| | | BR 112020003373 A2 | 25 August 2020 |
| | | BR 112020003373 B1 | 09 August 2022 |
| | | RU 2020111556 A | 23 September 2021 |
| | | RU 2020111556 A3 | 17 December 2021 |
| | | EP 3672949 A1 | 01 July 2020 |
| | | EP 3672949 B1 | 21 December 2022 |
| | | EP 3672949 B9 | 05 April 2023 |
| | | MA 49952 A | 28 April 2021 |
| | | MA 49952 B1 | 31 March 2023 |
| | | ES 2940448 T3 | 08 May 2023 |
| | | SG 11202000490 PA | 30 March 2020 |
| | | CO 2020001860 A2 | 29 May 2020 |
| | | HRP 20230244 T1 | 14 April 2023 |

Form PCT/ISA/210 (patent family annex) (July 2022)

**INTERNATIONAL SEARCH REPORT**
**Information on patent family members**

International application No.
**PCT/CN2023/112130**

| Patent document cited in search report | | | Publication date (day/month/year) | Patent family member(s) | | | Publication date (day/month/year) |
|---|---|---|---|---|---|---|---|
| | | | | CU | 20200014 | A7 | 30 November 2020 |
| | | | | EA | 202090553 | A1 | 08 June 2020 |
| | | | | SI | 3672949 | T1 | 28 April 2023 |
| | | | | HUE | 061895 | T2 | 28 August 2023 |
| | | | | US | 2019367483 | A1 | 05 December 2019 |
| | | | | US | 10640489 | B2 | 05 May 2020 |
| | | | | FI | 3672949 | T3 | 23 March 2023 |
| | | | | DOP | 2020000037 | A | 15 August 2020 |
| | | | | ECSP | 20013248 | A | 29 May 2020 |
| | | | | CL | 2020000427 | A1 | 28 August 2020 |
| CN | 111629749 | A | 04 September 2020 | BR | 112020007576 | A2 | 24 September 2020 |
| | | | | SG | 11202003415 | XA | 28 May 2020 |
| | | | | KR | 20200086278 | A | 16 July 2020 |
| | | | | IL | 273948 | A | 31 May 2020 |
| | | | | RU | 2020115889 | A | 18 November 2021 |
| | | | | CA | 3079407 | A1 | 25 April 2019 |
| | | | | MX | 2020004013 | A | 08 January 2021 |
| | | | | AR | 123115 | A1 | 02 November 2022 |
| | | | | TW | 201922799 | A | 16 June 2019 |
| | | | | WO | 2019079569 | A1 | 25 April 2019 |
| | | | | US | 2020339704 | A1 | 29 October 2020 |
| | | | | EP | 3697436 | A1 | 26 August 2020 |
| | | | | AU | 2018351050 | A1 | 07 May 2020 |
| | | | | AU | 2018351050 | A8 | 28 May 2020 |
| | | | | JP | 2021501570 | A | 21 January 2021 |
| WO | 2021173995 | A2 | 02 September 2021 | US | 2023174933 | A1 | 08 June 2023 |
| | | | | WO | 2021173995 | A3 | 25 November 2021 |
| | | | | WO | 2021173995 | A8 | 15 September 2022 |
| | | | | CA | 3173737 | A1 | 02 September 2021 |
| | | | | CL | 2022002340 | A1 | 10 April 2023 |
| | | | | JP | 2023515211 | A | 12 April 2023 |
| | | | | BR | 112022016633 | A2 | 13 December 2022 |
| | | | | IL | 295878 | A | 01 October 2022 |
| | | | | EP | 4110377 | A2 | 04 January 2023 |
| | | | | KR | 20220147109 | A | 02 November 2022 |
| | | | | AU | 2021225949 | A1 | 15 September 2022 |
| CN | 114650868 | A | 21 June 2022 | EP | 4051386 | A1 | 07 September 2022 |
| | | | | DOP | 2022000091 | A | 31 August 2022 |
| | | | | IL | 292173 | A | 01 June 2022 |
| | | | | BR | 112022007867 | A2 | 12 July 2022 |
| | | | | CL | 2022001111 | A1 | 18 November 2022 |
| | | | | AU | 2020374957 | A1 | 28 April 2022 |
| | | | | CR | 20220234 | A | 19 July 2022 |
| | | | | CA | 3154942 | A1 | 06 May 2021 |
| | | | | WO | 2021087093 | A1 | 06 May 2021 |
| | | | | MX | 2022005232 | A | 08 June 2022 |
| | | | | KR | 20220092920 | A | 04 July 2022 |
| | | | | PE | 20221457 | A1 | 21 September 2022 |
| | | | | JP | 2023500611 | A | 10 January 2023 |
| | | | | US | 2023002397 | A1 | 05 January 2023 |

Form PCT/ISA/210 (patent family annex) (July 2022)

**INTERNATIONAL SEARCH REPORT**
**Information on patent family members**

International application No.
**PCT/CN2023/112130**

| Patent document cited in search report | Publication date (day/month/year) | Patent family member(s) | Publication date (day/month/year) |
|---|---|---|---|
| WO 2020160198 A1 | 06 August 2020 | US 2023142883 A1 | 11 May 2023 |
| | | EP 3917517 A1 | 08 December 2021 |
| | | EP 3917517 A4 | 25 January 2023 |
| WO 2021022163 A2 | 04 February 2021 | WO 2021022163 A3 | 04 March 2021 |
| | | US 2022289711 A1 | 15 September 2022 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- Clinical Pharmacology. People's Public Health Press, 2008
- *CHEMICAL ABSTRACTS*, 1001754-57-9
- *CHEMICAL ABSTRACTS*, 1010100-26-1
- *CHEMICAL ABSTRACTS*, 1190095-10-3
- *CHEMICAL ABSTRACTS*, 191732-70-4
- *CHEMICAL ABSTRACTS*, 1228837-05-5
- *CHEMICAL ABSTRACTS*, 1228780-72-0
- *CHEMICAL ABSTRACTS*, 870812-97-8
- *CHEMICAL ABSTRACTS*, 7079-15-4
- *CHEMICAL ABSTRACTS*, 2716235-85-5
- *CHEMICAL ABSTRACTS*, 1841-57-2
- *CHEMICAL ABSTRACTS*, 191732-72-6
- *CHEMICAL ABSTRACTS*, 19171-19-8